(19) Europäisches Patentamt
European Patent Office
Office européen des brevets

(11) EP 4 393 937 A1

(12) EUROPEAN PATENT APPLICATION
published in accordance with Art. 153(4) EPC

(43) Date of publication:
**03.07.2024 Bulletin 2024/27**

(21) Application number: **22860594.5**

(22) Date of filing: **25.08.2022**

(51) International Patent Classification (IPC):
***C07J 71/00*** *(2006.01)* ***C07K 16/28*** *(2006.01)*
***A61K 31/585*** *(2006.01)* ***A61K 47/68*** *(2017.01)*
***A61P 37/00*** *(2006.01)*

(52) Cooperative Patent Classification (CPC):
**A61K 31/585; A61K 47/68; A61P 37/00; C07J 71/00; C07K 16/28**

(86) International application number:
**PCT/CN2022/114855**

(87) International publication number:
**WO 2023/025248 (02.03.2023 Gazette 2023/09)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.08.2021 CN 202110988668**
**23.12.2021 CN 202111592481**

(71) Applicant: **Duality Biologics (Suzhou) Co., Ltd.**
**Suzhou, Jiangsu 215000 (CN)**

(72) Inventors:
- **ZHANG, Yu**
  **Shanghai 201204 (CN)**
- **LI, Bing**
  **Shanghai 201204 (CN)**
- **SHEN, Hongxia**
  **Shanghai 201204 (CN)**
- **LI, Jian**
  **Shanghai 201204 (CN)**
- **LI, Xi**
  **Shanghai 201204 (CN)**
- **HUA, Haiqing**
  **Shanghai 201204 (CN)**
- **ZHU, Zhongyuan**
  **Shanghai 201204 (CN)**

(74) Representative: **Vossius & Partner Patentanwälte Rechtsanwälte mbB**
**Siebertstrasse 3**
**81675 München (DE)**

Remarks:
The complete document including Reference Table(s) and the Sequence Listing(s) can be downloaded from the EPO website

# (54) STEROID COMPOUND AND CONJUGATE THEREOF

(57) The present application relates to a steroid compound and a conjugate thereof, and in particular to a compound and a conjugate thereof, or a tautomer, a mesomer, a racemate, an enantiomer or a diastereoisomer thereof or a mixture thereof, or a pharmaceutically acceptable salt thereof. The present application also relates to a preparation method and use of the compound and the conjugate thereof of the present application.

EP 4 393 937 A1

## Description

### TECHNICAL FIELD

The present application relates to the field of biomedicine, and in particular to a steroid compound and a conjugate thereof.

### BACKGROUND

Inflammation is an adaptive response triggered by a variety of noxious stimuli and conditions, and it underlies many diseases related to the human immune system. Steroid compounds are a class of antiinflammatory drugs that have the potential to influence immune system functions and treat or prevent diseases and/or conditions associated with glucocorticoid receptor signaling. However, some existing steroid compounds are not that potent in anti-inflammation, and others may have many undesirable side effects. Therefore, there is an urgent need to further develop antibody-drug conjugates and steroid compounds formed from various steroids as drugs that can exert better therapeutic effect or have better safety.

### SUMMARY

The present application provides a compound or a tautomer, a mesomer, a racemate, an enantiomer or a diastereoisomer thereof, or a mixture thereof, or a pharmaceutically acceptable salt thereof, which may have one or more effects selected from the group consisting of: (1) having the ability to influence the activity of immune cells; (2) the conjugate thereof having targeting effect; (3) having plasma stability; (4) having biological safety; (5) having the ability to influence cytokine release from immune cells; (6) having the ability to affect transcription of responsive genes of IFN signaling pathway; (7) having the ability to influence the degree of skin fibrosis; (8) having the ability to influence the number and/or proportion of dendritic cells; (9) having the ability to influence the collagen content of the skin; (10) having the ability to influence GRE expression levels; (11) having the ability to influence cytokine release from monocytes; (12) having the ability to influence contact hypersensitivity; (13) having the ability to influence skin swelling and (14) having the ability to influence arthritic symptoms.

The present application provides an immunoconjugate comprising a glucocorticoid receptor agonist linked to a protein (e.g., an antibody or an antigen-binding fragment thereof and a soluble receptor protein). In some embodiments, the antibody or the antigen-binding fragment thereof is human, humanized, chimeric or murine. In some embodiments, the protein (e.g., an antibody, an antigen-binding fragment thereof, or a soluble receptor protein) can bind to a target on the surface of a cell and become internalized.

In one aspect, the present application provides a compound of formula I:

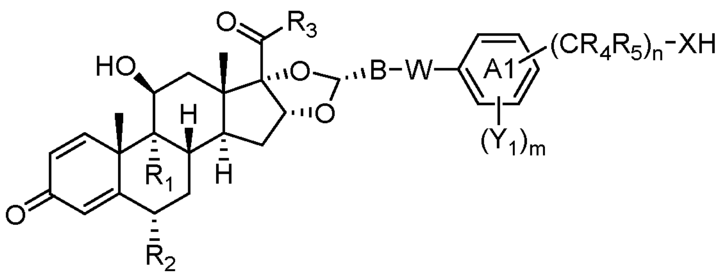

(I)

or a tautomer, a mesomer, a racemate, an enantiomer or a diastereoisomer thereof, or a mixture thereof, or a pharmaceutically acceptable salt thereof, wherein,

$R_1$, $R_2$, $R_3$, $R_4$ and $R_5$ are each independently any group;
B is absent or B is any group;
W is absent or W is any group;
A1 is a substituted benzene ring;
$CR_4R_5$ units are each independently unreplaced or replaced by a group selected from the group consisting of: -O-, -S-, -NR-, -S(O)-, $-S(O)_2-$, -C(=O)-, -C=C- and -C=C-, wherein $R_4$ and $R_5$ can together form optionally substituted cycloalkyl, optionally substituted heterocyclyl, optionally substituted bridged cyclyl, optionally substituted bridged

heterocyclyl, optionally substituted spiro cyclyl and optionally substituted spiro heterocyclyl, and n is any integer from 1 to 20;

X is selected from the group consisting of: -O-, -S- and -NR-;

$Y_1$ is any group, and m is any integer from 0 to 4;

wherein substituents are selected from the group consisting of: protium, deuterium, tritium, halogen, -CN, =O, =N-OH, =N-OR, =N-R, -OR, -C(O)R, -C(O)OR, -OC(O)R, -OC(O)OR, -C(O)NHR, - C(O)$NR_2$, -OC(O)NHR, -OC(O)$NR_2$, -SR, -S(O)R, -S$(O)_2$R, -NHR, -N$(R)_2$, -NHC(O)R, -NRC(O)R, -NHC(O)OR, -NRC(O)OR, -S$(O)_2$NHR, -S$(O)_2$N$(R)_2$, -NHS$(O)_2$$NR_2$, -NRS$(O)_2$$NR_2$, -NHS$(O)_2$R, - NRS$(O)_2$R, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy, wherein each R is independently selected from the group consisting of hydrogen, protium, deuterium, tritium, oxygen, hydroxy, halogen, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy.

In another aspect, the present invention provides a compound of a formula below or a tautomer, a mesomer, a racemate, an enantiomer or a diastereoisomer thereof, or a mixture thereof, or a pharmaceutically acceptable salt thereof, wherein the compound is selected from the group consisting of:

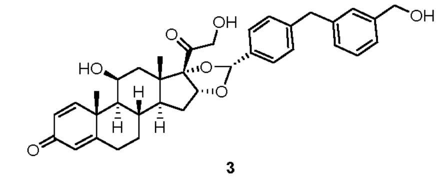

3

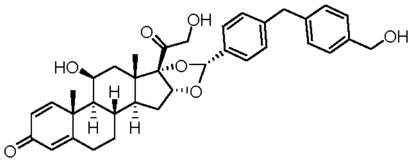

4

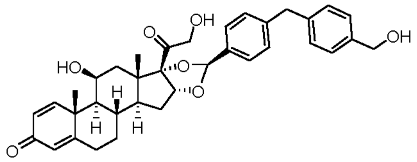

4S

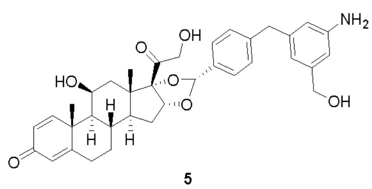

5

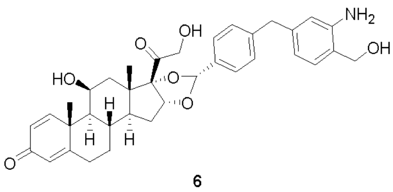

6

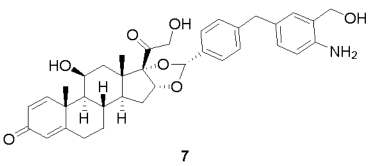

7

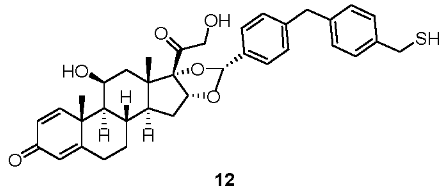

12

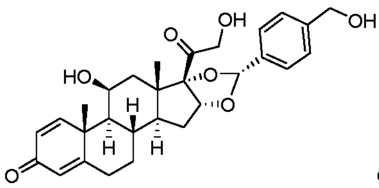

1

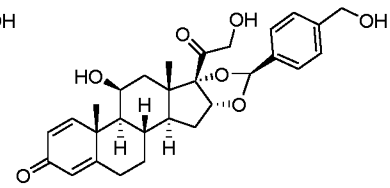

1S

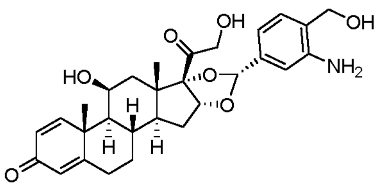

2

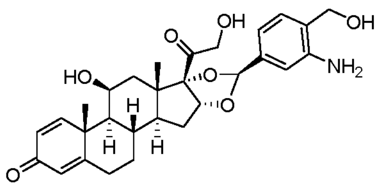

2S

.

In another aspect, the present application provides a compound of formula IIa or IIb:

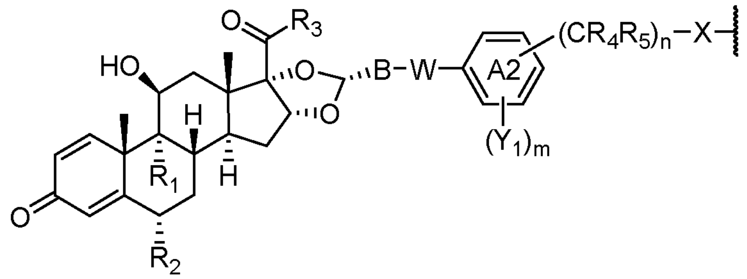

or

(IIa)

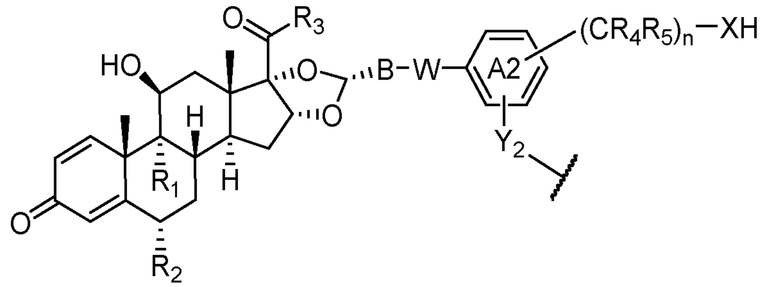

(IIb)

or a tautomer, a mesomer, a racemate, an enantiomer or a diastereoisomer thereof, or a mixture thereof, or a pharmaceutically acceptable salt thereof, wherein,

$R_1$, $R_2$, $R_3$, $R_4$ and $R_5$ are each independently any group;
B is absent or B is any group;
W is absent or W is any group;
A2 is a substituted benzene ring;
$CR_4R_5$ units are each independently unreplaced or replaced by a group selected from the group consisting of: -O-, -S-, -NR-, -S(O)-, $-S(O)_2-$, -C(=O)-, -C=C- and -C=C-, wherein $R_4$ and $R_5$ can together form optionally substituted cycloalkyl, optionally substituted heterocyclyl, optionally substituted bridged cyclyl, optionally substituted bridged heterocyclyl, optionally substituted spiro cyclyl and optionally substituted spiro heterocyclyl, and n is any integer from 1 to 20;
X is selected from the group consisting of: -O-, -S- and -NR-;
$Y_1$ is any group, and m is any integer from 0 to 4;
$Y_2$ is selected from the group consisting of -O-, -S- and -NR-;
wherein substituents are selected from the group consisting of: protium, deuterium, tritium, halogen, -CN, =O, =N-OH, =N-OR, =N-R, -OR, -C(O)R, -C(O)OR, -OC(O)R, -OC(O)OR, -C(O)NHR, - $C(O)NR_2$, -OC(O)NHR, $-OC(O)NR_2$, -SR, -S(O)R, $-S(O)_2R$, -NHR, $-N(R)_2$, -NHC(O)R, -NRC(O)R, -NHC(O)OR, -NRC(O)OR, $-S(O)_2NHR$, $-S(O)_2N(R)_2$, $-NHS(O)_2NR_2$, $-NRS(O)_2NR_2$, $-NHS(O)_2R$, - $NRS(O)_2R$, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy; wherein each R is independently selected from the group consisting of hydrogen, protium, deuterium, tritium, oxygen, halogen, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy.

In another aspect, the present application provides a conjugate comprising the compound or the tautomer, the mesomer, the racemate, the enantiomer or the diastereoisomer thereof, or the mixture thereof, or the pharmaceutically acceptable salt thereof described above.

In another aspect, the present application provides a pharmaceutical composition comprising the compound or the tautomer, the mesomer, the racemate, the enantiomer or the diastereoisomer thereof, or the mixture thereof, or the pharmaceutically acceptable salt thereof described above and/or the conjugate described above, and optionally a pharmaceutically acceptable carrier.

In another aspect, the present application provides a method for influencing immune system functions, which comprises administering to a subject the compound or the tautomer, the mesomer, the racemate, the enantiomer or the

diastereoisomer thereof, or the mixture thereof, or the pharmaceutically acceptable salt thereof described above, the conjugate described above and/or the pharmaceutical composition described above.

Other aspects and advantages of the present application will be readily apparent to those skilled in the art from the following detailed description. Only exemplary embodiments of the present application have been shown and described in the following detailed description. As those skilled in the art will recognize, the content of the present application enables those skilled in the art to make changes to the specific embodiments disclosed without departing from the spirit and scope of the invention to which the present application pertains. Accordingly, descriptions in the specification are only illustrative rather than restrictive.

## DETAILED DESCRIPTION

The embodiments of the present invention are described below with reference to specific examples, and other advantages and effects of the present invention will be readily apparent to those skilled in the art from the disclosure of the present specification.

### Definitions of Terms

In the present application, the term "glucocorticoid" generally refers to naturally occurring steroid hormones or synthetic steroid hormones that interact with the glucocorticoid receptor.

In the present application, the term "halogen" generally refers to fluorine, chlorine, bromine or iodine, and it may be, for example, fluorine or chlorine.

In the present application, the term "alkyl" generally refers to a residue derived from an alkane by removal of a hydrogen atom. Alkyl may be substituted or unsubstituted, or replaced or unreplaced. The term "alkyl" generally refers to a saturated linear or branched aliphatic hydrocarbon group having a residue derived from the parent alkane by removal of hydrogen atoms from the same carbon atom or two different carbon atoms, and it may be a linear or branched group containing 1 to 20 carbon atoms, e.g., 1 to 12 carbon atoms, such as chain alkyl containing 1 to 6 carbon atoms. Non-limiting examples of alkyl include, but are not limited to, methyl, ethyl, propyl, butyl, etc. Alkyl may be substituted or unsubstituted, or replaced or unreplaced. For example, when it is substituted, substitution with a substituent may be performed at any available linking site, and the substituent may be independently optionally selected from the group consisting of one or more of alkyl, alkenyl, alkynyl, alkoxy, alkylthio, alkylamino, halogen, sulfhydryl, hydroxy, nitro, cyano, cycloalkyl, heterocyclyl, aryl, heteroaryl, cycloalkoxy, heterocycloalkoxy, cycloalkylthio, heterocycloalkylthio and oxo, and it may, e.g., be hydrogen, protium, deuterium, tritium, halogen, $-NO_2$, -CN, -OH, -SH, $-NH_2$, -C(O)H, $-CO_2H$, -C(O)C(O)H, $-C(O)CH_2C(O)H$, -S(O)H, $-S(O)_2H$, $-C(O)NH_2$, $-SO_2NH_2$, - OC(O)H, $-N(H)SO_2H$ or a $C_{1-6}$ aliphatic group.

In the present application, the term "alkylene" generally refers to a saturated linear or branched aliphatic hydrocarbon group having 2 residues derived from the parent alkane by removal of two hydrogen atoms from the same carbon atom or two different carbon atoms, and it may be a linear or branched group containing 1 to 20 carbon atoms; for example, the term "methylene" may refer to a residue derived from a one-carbon atom group by removal of two hydrogen atoms. Methylene may be substituted or unsubstituted, or replaced or unreplaced; for example, alkylene contains 1 to 12 carbon atoms, e.g., an alkylene group containing 1 to 6 carbon atoms. Non-limiting examples of alkylene include, but are not limited to, methylene($-CH_2-$), 1,1-ethylidene($-CH(CH_3)-$), 1,2-ethylidene($-CH_2CH_2-$), 1,1-propylidene($-CH(CH_2CH_3)-$), 1,2-propylidene($-CH_2CH(CH_3)-$), 1,3-propylidene($-CH_2CH_2CH_2-$), 1,4-butylidene($-CH_2CH_2CH_2CH_2-$), 1,5-butylidene($-CH_2CH_2CH_2CH_2CH_2-$), etc. Alkylene may be substituted or unsubstituted, or replaced or unreplaced. For example, when it is substituted, substitution with a substituent may be performed at any available linking site, and the substituent may be independently optionally selected from the group consisting of one or more of alkyl, alkenyl, alkynyl, alkoxy, alkylthio, alkylamino, halogen, sulfhydryl, hydroxy, nitro, cyano, cycloalkyl, heterocyclyl, aryl, heteroaryl, cycloalkoxy, heterocycloalkoxy, cycloalkylthio, heterocycloalkylthio, and oxo, and it may, e.g., be hydrogen, protium, deuterium, tritium, halogen, $-NO_2$, -CN, -OH, -SH, $-NH_2$, -C(O)H, $-CO_2H$, -C(O)C(O)H, $-C(O)CH_2C(O)H$, - S(O)H, $-S(O)_2H$, $-C(O)NH_2$, $-SO_2NH_2$, -OC(O)H, $-N(H)SO_2H$ or a $C_{1-6}$ aliphatic group. Methylene or alkylene may be substituted or unsubstituted.

In the present application, the term "alkenyl" generally refers to a linear or branched hydrocarbon group containing one or more double bonds. Examples of alkenyl include allyl, homoallyl, vinyl, crotyl, butenyl, pentenyl, hexenyl, etc. Examples of C2-6 alkenyl containing more than one double bond include butadienyl, pentadienyl, hexadienyl, and hexatrienyl, as well as branched forms thereof. The positions of the unsaturated bonds (double bonds) may be any positions in the carbon chain. Alkenyl may be substituted or unsubstituted.

In the present application, the term "alkenylene" generally refers to a group having two residues derived from an alkene by removal of two hydrogen atoms from a carbon atom. For example, alkenylene may be acrol, vinylene, butenylene, pentenylene, hexenylene, etc. Alkenylene may be substituted or unsubstituted.

In the present application, the term "alkynyl" generally refers to unsaturated linear or branched alkynyl, e.g.,

ethynyl, 1-propynyl, propargyl, or butynyl. Alkynyl may be substituted or unsubstituted. In the present application, the term "alkynylene" generally refers to a group having two residues derived from an alkyne by removal of two hydrogen atoms from a carbon atom. For example, alkynylene may be ethynylene, propynylene, propargylene, butynylene, etc. Alkynylene may be substituted or unsubstituted.

In the present application, the term "aryl" generally refers to a group having a residue derived from an aromatic ring by removal of a hydrogen atom. The term "aromatic ring" may refer to a 6-14 membered all-carbon monocyclic ring or fused polycyclic ring (i.e., rings that share adjacent pairs of carbon atoms) having a conjugated $\pi$-electron system, and it may be 6-10 membered, such as benzene and naphthalene. The aromatic ring can be fused to a heteroaryl, heterocyclyl or cycloalkyl ring, wherein the ring connected to the parent structure is the aryl ring. Aryl may be substituted or unsubstituted, and when it is substituted, the substituent may be one or more of the following groups independently selected from the group consisting of: alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio and heterocycloalkylthio. Aryl may be substituted or unsubstituted.

In the present application, the term "arylene" generally refers to a group having two residues derived from an aromatic ring by removal of two hydrogen atoms from carbon atoms of the ring. For example, arylene may be phenylene and naphthylene. Arylene may be substituted or unsubstituted.

In the present application, the term "heteroaryl" generally refers to a group having a residue derived from a heteroaromatic ring by removal of a hydrogen atom from a carbon atom. The term "heteroaromatic ring" refers to a heteroaromatic system comprising 1 to 4 heteroatoms and 5 to 14 ring atoms, wherein the heteroatoms may be selected from the group consisting of: oxygen, sulfur and nitrogen. Heteroaryl may be 5-10 membered and may be 5- or 6-membered, such as furanyl, thienyl, pyridinyl, pyrrolyl, N-alkylpyrrolyl, pyrimidinyl, pyrazinyl, imidazolyl and tetrazolyl. The heteroaryl ring can be fused to an aryl, heterocyclyl or cycloalkyl ring, wherein the ring connected to the parent structure is the heteroaryl ring. Heteroaryl may be optionally substituted or unsubstituted, and when it is substituted, the substituent may be one or more of the following groups independently selected from the group consisting of: alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio and heterocycloalkylthio. Heteroaryl may be substituted or unsubstituted.

In the present application, the term "heteroarylene" generally refers to a group having two residues derived from a heteroaromatic ring by removal of two hydrogen atoms from carbon atoms of the ring. For example, heteroarylene may be furylene, thienylene, pyridylidene, pyrrolylene, pyrimidinylene, pyrazinylene, imidazolylene, tetrazolylene, etc. Heteroarylene may be substituted or unsubstituted. In the present application, the terms "alcyl" and "cycloalkyl" are used interchangeably, and the term "alcyl" generally refers to a group having a residue derived from an aliphatic ring by removal of a hydrogen atom from the same carbon atom or from a plurality of different carbon atoms. The term "cycloalkane" generally refers to a saturated or partially unsaturated monocyclic or polycyclic cyclic hydrocarbon, and it contains 3 to 20 carbon atoms, may contain 3 to 12 carbon atoms, may contain 3 to 10 carbon atoms, and may contain 3 to 8 carbon atoms. Non-limiting examples of alcyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc. Polycyclic carbon rings may include spiro, fused and bridged carbon rings. Alcyl may be substituted or unsubstituted. In the present application, the term "carbocyclyl" generally refers to a group having a residue derived from a carbon ring by removal of a hydrogen atom from a carbon atom. The term "carbon ring" generally refers to a saturated or partially unsaturated monocyclic or polycyclic cyclic hydrocarbon, and it contains 3 to 20 carbon atoms, may contain 3 to 12 carbon atoms, may contain 3 to 10 carbon atoms, and may contain 3 to 8 carbon atoms. Non-limiting examples of a monocyclic carbon ring include cyclopropane, cyclobutane, cyclopentane, cyclopentene, cyclohexane, cyclohexene, cyclohexadiene, cycloheptane, cycloheptatriene, cyclooctane, etc; polycyclic carbon rings may include spiro, fused and bridged carbon rings. Carbocyclyl may be substituted or unsubstituted. In some instances, alicyclic and carbocyclic rings may be used in place of one another.

In the present application, the term "partially unsaturated" generally means that the cyclic structure contains at least one double or triple bond between the ring molecules. The term "partially unsaturated" encompasses cyclic structures having multiple sites of unsaturation, but is not intended to include aromatic or heteroaromatic rings defined herein. The term "unsaturated" means that the moiety has one or more degrees of unsaturation.

In the present application, the terms "alcylene" and "cycloalkylene" are used interchangeably, and the term "alcylene" generally refers to a group having a residue derived from an alicyclic ring by removal of two hydrogen atoms from a carbon atom. For example, alcylene may be cyclopropylene, cyclobutylene, cyclopentylene, cyclopentenylene, cyclohexylene, cyclohexenylene, cyclohexadienylene, cycloheptylene, cycloheptatrienylene, cyclooctylene, etc; polycyclic carbon rings may include spiro, fused and bridged carbon rings. Alcylene may be substituted or unsubstituted.

In the present application, the terms "aliphatic heterocyclyl" and "heterocyclyl" are used interchangeably, and the term "aliphatic heterocyclyl" generally refers to a 3-7 membered monocyclic carbon ring structure, a fused 7-10 membered bicyclic heterocyclic structure or a bridged 6-10 membered bicyclic heterocyclic structure that is stable and non-aromatic. These cyclic structures may be saturated or partially saturated, and contain one or more heteroatoms in

addition to carbon atoms, wherein the heteroatoms may be selected from the group consisting of: oxygen, sulfur and nitrogen. For example, they contain 1 to 4 heteroatoms as defined above. When used to refer to atoms on an aliphatic heterocyclic cyclic structure, the term "nitrogen" may include nitrogen that undergoes a substitution reaction. For example, the aliphatic heterocyclyl may comprise "heterocycloalkyl" that may refer to a 3-7 membered monocyclic alkyl structure, a fused 7-10 membered bicyclic heterocyclic structure or a bridged 6-10 membered bicyclic heterocyclic structure that is stable and non-aromatic. These structures also contain one or more heteroatoms in addition to carbon atoms, wherein the heteroatoms may be selected from the group consisting of: oxygen, sulfur and nitrogen. For example, they contain 1 to 4 heteroatoms as defined above. Heterocycloalkyl may be substituted or unsubstituted. Aliphatic heterocyclyl may be substituted or unsubstituted.

In the present application, the terms "aliphatic heterocyclylene" and "heterocyclylene" are used interchangeably, and the term "aliphatic heterocyclylene" generally refers to a group having a residue derived from an aliphatic heterocyclic ring by removal of two hydrogen atoms from carbon atoms of the ring. Aliphatic heterocyclylene may be substituted or unsubstituted.

In the present application, in the description of cyclic structures such as cycloalkyl, cycloalkylene, heterocyclyl, heterocyclylene, aryl, arylene, heteroaryl and heteroarylene, the prefix (e.g., $C_3$-$C_{20}$, $C_3$-$C_7$ or $C_5$-$C_6$) is generally intended to refer to the number of ring atoms, or the range of ring atoms, whether carbon atoms or heteroatoms. For example, the term "$C_5$-$C_6$ heterocyclyl" as used herein relates to heterocyclyl groups having 5 or 6 ring atoms. For another example, the term "$C_5$-$C_{10}$ heteroaryl" as described herein relates to heteroaryl groups having 5 to 10 ring atoms.

In the present application, the term "ring-forming atom" or "ring atom" generally refers to an atom contained in a cyclic structure. For example, a ring-forming atom may be a carbon atom in a benzene ring, or may be a nitrogen atom in a pyridine ring. When a hydrogen atom is linked to a ring-forming atom, the ring-forming atom may be substituted or unsubstituted.

In the present application, the term "each independently" generally means that a variable applies in any case irrespective of the presence or absence of variables having the same or different definitions in the same compound. For example, the variable may refer to the type or number of substituents in the compound, the type of atoms in the compound, and so on. For example, where R occurs twice in a compound and R is defined as "independently carbon or nitrogen", both R can be carbon, both R can be nitrogen, or one R can be carbon and the other R is nitrogen.

In the present application, the term "optional" or "optionally" generally means that the event or circumstance subsequently described may, but not necessarily, occur, and that the description includes instances where the event or circumstance occurs or does not occur. For example, "heterocyclyl group optionally substituted with alkyl" means that alkyl may be, but not necessarily, present, and that the description may include instances where the heterocyclyl group is or is not substituted with alkyl. In the present application, the term "substituted" generally means that one or more hydrogen atoms in the group, for example, up to 5 (e.g., 1 to 3) hydrogen atoms, are each independently substituted with a corresponding number of substituents. A substituent is only in its possible chemical position, and those skilled in the art will be able to determine (by experiments or theories) possible or impossible substitution without undue efforts. For example, it may be unstable when amino or hydroxy having a free hydrogen is bound to a carbon atom having an unsaturated (such as olefin) bond.

In the case of substitution, the substituent of an "optionally substituted" group may include, but is not limited to, one or more substituents independently selected from the group consisting of the following groups, alone or in combination, or a specifically specified set of groups: lower alkyl, lower alkenyl, lower alkynyl, lower alkanoyl, lower heteroalkyl, lower heterocycloalkyl, lower haloalkyl, lower haloalkenyl, lower haloalkynyl, lower perhaloalkyl, lower perhaloalkoxy, lower cycloalkyl, phenyl, aryl, aryloxy, lower alkoxy, lower haloalkoxy, oxo, lower acyloxy, carbonyl, carboxyl, lower alkylcarbonyl, lower carboxyl ester, lower formylamino, cyano, halogen, hydroxy, amino, lower alkylamino, arylamino, acylamino, nitro, thiol, lower alkylthio, lower haloalkylthio, lower perhaloalkylthio, arylthio, sulfonate, sulfonic acid, trisubstituted silyl, N3, SH, SCH3, C(O)CH3, CO2CH3, CO2H, nitrile, CF3, cycloalkyl, pyridinyl, thiophene, furanyl, lower carbamate, halophenyl, hydroxyphenyl, haloalkyl and hydroxyalkyl. Two substituents may be linked together to form a 5-, 6- or 7-membered aromatic or nonaromatic carbocyclic or heterocyclic ring containing one to three heteroatoms, for example to form methylenedioxy or ethylenedioxy. Optionally substituted groups may be incompletely substituted (e.g., $-CH_2CH_3$), fully substituted (e.g., $-CF_2CF_3$), monosubstituted ($-CH_2CH_2F$) or substituted at any level between fully substituted and monosubstituted (e.g., $-CH_2CF_3$). When substituents are listed without qualification as to substitution, both substituted and unsubstituted forms are encompassed. When a substituent is qualified as "substituted," the substituted form is specifically intended. Additionally, different sets of optional substituents to a particular moiety may be defined as needed; in these cases, the optional substitution will be as defined, often immediately following the phrase "optionally substituted with". The term "lower" in connection with organic groups or compounds means a compound or group that may be branched or unbranched with up to and including 7 carbon atoms, preferably 1-4 carbon atoms. Lower alkyl represents, for example, methyl, ethyl, *n*-propyl, isopropyl, *n*-butyl, *sec*-butyl, *tert*-butyl, *n*-pentyl and branched pentyl, *n*-hexyl and branched hexyl.

In the present application, the term "0 or more (e.g., 0 or 1 or more, 0 or 1, or 0) methylene units are replaced"

generally means that when the structure comprises one or more methylene units, the one or more methylene units may be unreplaced, or may be replaced by one or more groups that are not methylene (e.g., -NHC(O)-, -C(O)NH-, -C(O)-, -OC(O)-, -C(O)O-, -NH-, -O-, -S-, -SO-, $-SO_2-$, -PH-, -P(=O)H-, $-NHSO_2-$, $-SO_2NH-$, -C(=S)-, -C(=NH)-, -N=N-, -C=N-, -N=C- or $-C(=N_2)-$).

In the present application, the terms such as "alkyl", "alkenyl" and "cycloalkyl" may be preceded by a notation to indicate the number of atoms present in the groups under particular circumstances as in $C_1$-$C_4$ alkyl, $C_3$-$C_7$ cycloalkoxy, and $C_1$-$C_4$ alkylcarbonylamino and the like, as known to those skilled in the art, and the subscript numeral following "C" indicates the number of carbon atoms present in the group. For example, $C_3$ alkyl refers to an alkyl group containing three carbon atoms (e.g., n-propyl, or isopropyl); in $C_{1-10}$, members of the group may contain any number of carbon atoms within the range of 1-10.

One or more hydrogen atoms in the group, for example, up to 5 (e.g., 1 to 3) hydrogen atoms, are each independently substituted with a corresponding number of substituents. A substituent is only in its possible chemical position, and those skilled in the art will be able to determine (by experiments or theories) possible or impossible substitution without undue efforts. For example, it may be unstable when amino or hydroxy having a free hydrogen is bound to a carbon atom having an unsaturated (such as olefin) bond.

In the present application, the term "compound" generally refers to a substance having two or more different elements. For example, the compound disclosed herein may be an organic compound. For example, the compound disclosed herein may be a compound having a molecular weight of no more than 500, a compound having a molecular weight of no more than 1000, a compound having a molecular weight of no less than 1000, or a compound having a molecular weight of no less than 10,000 or no less than 100,000. In the present application, the compound may also refer to a compound that involves linking by a chemical bond, for example, a compound where one or more molecules having a molecular weight of no more than 1000 are linked, by a chemical bond, to a biological macromolecule, wherein the biological macromolecule may be polysaccharide, protein, nucleic acid, polypeptide, and the like. For example, the compound disclosed herein may include a compound where a protein is linked to one or more molecules having a molecular weight of no more than 1000, may include a compound where a protein is linked to one or more molecules having a molecular weight of no more than 10,000, and may include a compound where a protein is linked to one or more molecules having a molecular weight of no more than 100,000.

The pharmaceutical composition may be in the form of a sterile injectable aqueous or oily suspension for intramuscular and subcutaneous administration. The suspension can be prepared according to a known technique using those suitable dispersing agents or wetting agents and suspending agents described above. The sterile injectable formulation may also be a sterile injection or suspension prepared in a parenterally acceptable non-toxic diluent or solvent, e.g., a solution prepared in 1,3-butanediol. In addition, a sterile fixed oil may be conventionally used as a solvent or a suspending medium. For example, any blend fixed oil including synthetic mono- or di-glycerides can be used. In addition, fatty acids such as oleic acid may also be used in the preparation of injections.

In the present application, the compound of the present application includes tautomers, mesomers, racemates, enantiomers and/or diastereoisomers thereof. In the present application, the term "diastereoisomer" generally refers to a stereoisomer that has two or more chiral centers and whose molecules are not mirror images of each other. Diastereoisomers may have different physical properties, e.g., melting points, boiling points, spectral properties, and reactivities. In the present application, the terms "tautomer" and "tautomeric form" are used interchangeably and generally refer to structural isomers of different energies that can be converted into each other by crossing a low energy barrier. For example, proton tautomers (also known as prototropic tautomers) include interconversions by proton migration, such as keto-enol isomerization and imine-enamine isomerization. Valence tautomers include interconversions by recombination of some bonding electrons. In the present application, the term "mesomer" generally means that the molecule contains asymmetric atoms but the total optical rotation is zero due to the presence of symmetric factors. The term "racemate" or "racemic mixture" refers to a composition of two enantiomeric substances in equimolar amounts.

In the present application, certain atoms of the compound of the present application may be present in more than one isotopic form. For example, hydrogen may occur as protium ($^1H$), deuterium ($^2H$) and tritium ($^3H$), and carbon may naturally occur as three different isotopes ($^{12}C$, $^{13}C$ and $^{14}C$). Examples of isotopes that can be incorporated into the compound of the present application also include, but are not limited to, $^{15}N$, $^{18}O$, $^{17}O$, $^{18}F$, $^{32}P$, $^{33}P$, $^{129}I$, $^{131}I$, $^{123}I$, $^{124}I$, $^{125}I$, or similar isotopes. Thus, the compounds of the present application may be enriched with one or more of these isotopes relative to the natural abundance of these isotopes. Such isotopically enriched compounds can be used for a variety of purposes, as known to those skilled in the art. For example, replacement with heavy isotopes such as deuterium ($^2H$) may offer certain therapeutic advantages, possibly due to higher metabolic stability. For example, the natural abundance of deuterium ($^2H$) is about 0.015%. Accordingly, one out of about 6500 hydrogen atoms is a deuterium atom. Accordingly, the deuterium abundance of one or more sites (as the case may be) in the deuterium-containing compound of the present invention is greater than 0.015%. Unless otherwise indicated, the structures described herein may also include compounds that differ only in the presence or absence of one or more isotopically enriched atoms. For example, compounds having a structure identical to the structure of the present application except for the substitution of the

hydrogen atom with deuterium or tritium or the substitution of the carbon atom with carbon 13 or carbon 14 are within the scope of the present application.

In the present application, "isomers" generally refer to different compounds having the same molecular formula. "Stereoisomers" generally refer to isomers that differ only in the way the atoms are arranged in space. In the present application, the term "isomer" includes any and all geometric isomers and stereoisomers. For example, "isomers" include geometric double bond cis- and trans-isomers, also termed E- and Z-isomers; R- and S-enantiomers; diastereoisomers, (d)-isomers and (l)-isomers, racemic mixtures thereof; and other mixtures thereof, as falling within the scope of the present disclosure.

In the present application, "enantiomers" generally refer to a pair of stereoisomers that are non-superimposable mirror images of each other. A 1:1 mixture of a pair of enantiomers is a "racemic" mixture. The term "(±)" is used to designate a racemic mixture where appropriate. "Diastereoisomers" are stereoisomers that have at least two asymmetric atoms, but are not mirror images of each other. The absolute stereochemistry is specified according to the Cahn-Ingold-Prelog R-S system. When the compound is a pure enantiomer, the stereochemistry at each chiral carbon may be specified by either R or S. Resolved compounds whose absolute configuration is unknown are designated (+) or (-) depending on the direction (dextrorotatory or laevorotary) that they rotate the plane of polarized light at the wavelength of the sodium D line. Certain compounds described herein contain one or more asymmetric centers and can therefore give rise to enantiomers, diastereoisomers, and other stereoisomeric forms that can be defined in terms of absolute stereochemistry, such as (R)- or (S)-. The chemical entities, pharmaceutical compositions and methods of the present application are intended to include all such possible isomers, including racemic mixtures, optically pure forms and intermediate mixtures. Optically active (R)- and (S)-isomers can be prepared using chiral synthons or chiral reagents, or resolved using conventional techniques. When the compound described herein contains an olefinic double bond or other centers of geometric asymmetry, and unless specified otherwise, the compound is intended to include both E and Z geometric isomers.

In the present application, the term "enantiomeric purity" generally refers to the relative amount, expressed as a percentage, of the presence of a specific enantiomer relative to the other enantiomer. For example, if a compound that may potentially have an (R)- or an (S)-isomeric configuration is present as a racemic mixture, the enantiomeric purity is about 50% with respect to either the (R)- or (S)-isomer. If that compound has one isomeric form predominant over the other, for example, 80% (S)- and 20% (R)-, the enantiomeric purity of the compound with respect to the (S)-isomeric form is 80%. The enantiomeric purity of a compound may be determined in a number of ways known in the art, including but not limited to chromatography using a chiral support, polarimetric measurement of the rotation of polarized light, nuclear magnetic resonance spectroscopy using chiral shift reagents (including but not limited to lanthanide-containing chiral complexes or Pirkle's alcohol), or derivatization of the compound using a chiral compound such as Mosher's acid followed by chromatography or nuclear magnetic resonance spectroscopy.

In the present application, the term "tautomer" generally refers to a type of isomer that includes two or more interconvertible compounds resulting from at least one formal migration of a hydrogen atom and at least one change in valency (e.g., a single bond to a double bond, a triple bond to a double bond, or a triple bond to a single bond, or vice versa). "Tautomerization" includes prototropic or proton-shift tautomerization, which is considered a subset of acid-base chemistry. "Prototropic tautomerization" or "proton-shift tautomerization" involves the migration of a proton accompanied by changes in bond order. The exact ratio of the tautomers depends on several factors, including temperature, solvent and pH. Where tautomerization is possible (e.g., in solution), a chemical equilibrium of tautomers may be reached. Tautomerization (i.e., the reaction providing a tautomeric pair) may be catalyzed by acids or bases, or can occur without the action or presence of an external agent. Exemplary tautomerizations include, but are not limited to, keto-enol; amide-imide; lactam-lactim; enamine-imine; and enamine-(a different) enamine tautomerizations. A specific example of keto-enol tautomerization is the interconversion of pentane-2,4-dione and 4-hydroxypent-3-en-2-one tautomers. Another example of tautomerization is phenol-keto tautomerization. A specific example of phenol-keto tautomerization is the interconversion of pyridin-4-ol and pyridin-4(1H)-one tautomers.

In the present application, the term "pharmaceutical composition" generally refers to a mixture containing one or more of the compounds described herein or a physiologically/pharmaceutically acceptable salt or pro-drug thereof, and other chemical components, and other components, for example, physiologically/pharmaceutically acceptable carriers and excipients. The pharmaceutical composition may promote the administration to an organism, which facilitates the absorption of the active ingredient, thereby exerting biological activities. For preparation of conventional pharmaceutical compositions, reference can be made to *Chinese Pharmacopoeia.*

In the present application, the term "pharmaceutically acceptable salt" generally refers to a salt of a compound or ligand-drug conjugate of the present application, or a salt of a compound described herein. Such salts may be safe and/or effective when used in mammals and may possess the required biological activity, and the antibody-antibody drug conjugate compound of the present application may form a salt with an acid, and non-limiting examples of pharmaceutically acceptable salts include: hydrochloride, hydrobromide, hydriodate, sulphate, bisulfate, citrate, acetate, succinate, ascorbate, oxalate, nitrate, sorbate, hydrophosphate, dihydrophosphate, salicylate, hydrocitrate, tartrate, maleate, fumarate, formate, benzoate, mesylate, ethanesulfonate, benzenesulphonate and *p*-toluenesulfonate.

In the present application, the term "immunoconjugate", "conjugate", "antibody-drug conjugate" or "ADC" generally refers to a compound or a derivative thereof that is linked to a protein, such as a cell binding agent (e.g., an anti-TNF-α antibody or a fragment thereof), and is defined by the following general formula: (SM-L-Q) n-A, wherein SM is a group derived from a small-molecule glucocorticoid receptor agonist (e.g., glucocorticoid), L is a linker, Q is a heterobifunctional group, a heterotrifunctional group, or absent, A is a protein (e.g., an antibody or an antigen-binding fragment thereof, an anti-TNF protein, an anti-TNF-α antibody or a fragment thereof, a soluble receptor, or a soluble TNF receptor), and n is 1-10. The immunoconjugate may also be defined by the following general formula in reverse order: A-(Q-L-SM)n.

In the present application, the term "linker" generally refers to any chemical moiety capable of linking a protein (e.g., an antibody, an antibody fragment (e.g., an antigen-binding fragment), or a functional equivalent) to a glucocorticoid. The linker may be susceptible to cleavage ("cleavable linker"), thereby facilitating the release of the glucocorticoid. For example, such cleavable linkers may be susceptible to acid-induced cleavage, light-induced cleavage, peptidase-induced cleavage, esterase-induced cleavage and disulfide bond cleavage under conditions that the glucocorticoid and/or antibody retains activity. Alternatively, the linker may be substantially resistant to cleavage ("a non-cleavable linker").

In the present application, the term "drug antibody ratio" or "DAR" refers to the number of SMs (i.e., groups derived from a small-molecule glucocorticoid receptor agonist (e.g., a glucocorticoid)) linked to A (i.e., a protein, e.g., an antibody or an antigen-binding fragment thereof, an anti-TNF protein, an anti-TNF-α antibody or a fragment thereof, a soluble receptor, or a soluble TNF receptor). Thus, in an immunoconjugate having the general formula (SM-L-Q)n-A, the DAR is defined by the variable "n".

When referring to a compound having formula (SM-L-Q)n-A representing an individual immunoconjugate, the DAR generally refers to the number of SMs linked to the individual A (e.g., n is an integer from 1 to 10).

When referring to a compound having formula (SM-L-Q)n-A representing a plurality of immunoconjugates, the DAR generally refers to the average number of SMs linked to the As (e.g., n is an integer or fraction from 1 to 10). Thus, by way of example, a compound having formula (SM-L-Q)n-A comprising a first immunoconjugate with 3 SMs per A and a second immunoconjugate with 4 SMs per A has a DAR (i.e., an "n") of 3.5.

In the present application, a non-cleavable linker generally refers to any chemical moiety capable of linking a glucocorticoid to an antibody in a stable, covalent manner and does not fall off under the categories listed above for cleavable linkers. Thus, non-cleavable linkers are substantially resistant to acid-induced cleavage, light-induced cleavage, peptidase-induced cleavage, esterase-induced cleavage and disulfide bond cleavage. Furthermore, non-cleavable refers to the ability of the chemical bond in the linker or adjoining to the linker to withstand cleavage induced by an acid, a photolabile cleaving agent, a peptidase, an esterase or a chemical or physiological compound that cleaves a disulfide bond, under conditions that the glucocorticoid and/or antibody does not lose its activity. Some cleavable linkers are cleaved by peptidases ("peptidase cleavable linkers"). Only certain peptides are readily cleaved inside or outside cells, see e.g. Trout et al., Proc. Natl. Acad. Sci. USA, 626-629 (1982) and Umemoto et al., Int. J. Cancer, 677-684 (1989). Furthermore, peptides are composed of α-amino acid units and peptide bonds, which chemically are amide bonds between the carboxylic acid of one amino acid and the amino group of a second amino acid. Other amide bonds (such as the bond between the carboxylic acid and the α-amino acid group of lysine) are understood not to be peptide bonds and are considered non-cleavable.

Some linkers are cleaved by esterases ("esterase cleavable linkers"). Only certain esters can be cleaved by esterases present inside or outside of cells. Esters are formed by the condensation of carboxylic acid and alcohol. Simple esters are esters produced with simple alcohols (such as aliphatic alcohols) and small cyclic and small aromatic alcohols.

In some embodiments, the cleavable linker component may comprise a peptide comprising 1 to 10 amino acid residues. In these embodiments, the peptide allows for cleavage of the linker by a protease, thereby facilitating the release of the glucocorticoid upon exposure to intracellular proteases (such as lysosomal enzymes) (Doronina et al., (2003) Nat. Biotechnol. 21: 778-784). Exemplary peptides include, but are not limited to, dipeptides, tripeptides, tetrapeptides and pentapeptides. Exemplary dipeptides include, but are not limited to, alanine-alanine (ala-ala), valine-citrulline (vc or val-cit), alanine-phenylalanine (af or ala-phe); phenylalanine-lysine (fk or phe-lys); phenylalanine-homolysine (phe-homolys); and N-methyl-valine-citrulline (Me-val-cit). Exemplary tripeptides include, but are not limited to, glycine-valine-citrulline (gly-val-cit) and glycine-glycine-glycine (gly-gly-gly).

A peptide may comprise naturally-occurring amino acid residues and/or non-natural amino acid residues. The term "naturally-occurring amino acid" generally refers to Ala, Asp, Cys, Glu, Phe, Gly, His, He, Lys, Leu, Met, Asn, Pro, Gin, Arg, Ser, Thr, Val, Trp and Tyr. By way of non-limiting example, "non-natural amino acids" (i.e., amino acids that do not occur naturally) include homoserine, homoarginine, citrulline, phenylglycine, taurine, iodotyrosine, selenocysteine, norleucine ("Nle"), norvaline ("Nva"), β-alanine, L- or D-naphthalanine, ornithine ("Orn"), and the like. Peptides can be designed and optimized for enzymatic cleavage by a particular enzyme (e.g., a tumor-associated protease, cathepsin B, C and D, or a plasmin protease).

Amino acids may also include the D-forms of natural and non-natural amino acids. "D-" designates an amino acid having the "D" (dextrorotary) configuration, as opposed to the configuration in the naturally occurring ("L-") amino acids. Natural and non-natural amino acids can be commercially available (Sigma Chemical Co., or Advanced Chemtech)

or be synthesized using methods known in the art.

In the present application, the term "pharmaceutically acceptable carrier" generally refers to a carrier for providing therapeutic agents, such as antibodies or polypeptides, genes, and other therapeutic agents. The term refers to any pharmaceutical carrier that does not itself induce the production of antibodies harmful to the individual that receives the composition and can be given without producing undue toxicity. Suitable carriers may be macromolecules that are large and metabolize slowly, such as proteins, polysaccharides, polylactic acids, polyglycolic acids, poly(amino acid)s, amino acid copolymers, lipid aggregates and inactivated virus particles. Such carriers are well known to those skilled in the art. Pharmaceutically acceptable carriers in therapeutic compositions may include liquids such as water, saline, glycerol and ethanol. Auxiliary substances, such as wetting or emulsifying agents, or pH buffering substances, may also be present in these carriers.

In the present application, the term "anti-TNFα protein" generally refers to proteins that are capable of (i) binding to TNFα and (ii) inhibiting binding of soluble TNF-α to cell surface TNF receptors (p55 and/or p75) and/or lysing cells expressing surface TNFα or TNFα receptor *in vitro* in the presence of complement. Anti-TNFα proteins include, for example, anti-TNF antibodies or antigen-binding fragments thereof (e.g., adalimumab or infliximab) as well as soluble TNF receptors (e.g., etanercept). In the present application, the term "antibody" generally refers to an immunoglobulin that is reactive to a specified protein or peptide or a fragment thereof. The antibody can be an antibody of any class, including but not limited to IgG, IgA, IgM, IgD and IgE, and of any subclass (e.g., IgG1, IgG2, IgG3 and IgG4). The antibody may have a heavy chain constant region selected from the group consisting of, for example, IgG1, IgG2, IgG3 and IgG4. The antibody may also have a light chain selected from the group consisting of, for example, kappa **(κ)** and lambda (λ**).** The antibodies of the present application may be derived from any species. The term "antibody" may comprise intact polyclonal antibodies, intact monoclonal antibodies, chimeric antibodies, humanized antibodies, human antibodies, fusion proteins comprising an antibody and any other modified immunoglobulin molecules so long as the antibodies exhibit the desired biological activity.

In the present application, the term "antigen-binding fragment" generally refers to a portion of an antibody molecule that comprises amino acids responsible for specific binding of an antibody to an antigen. The portion of the antigen specifically recognized and bound by the antibody is referred to as the "epitope" described above. As described above, the antigen-binding domain may typically comprise an antibody light chain variable region (VL) and an antibody heavy chain variable region (VH); however, it does not necessarily comprise both. Fd fragment, for example, has two VH regions and typically retains some of the antigen-binding functions of the intact antigen-binding domain. Examples of antigen-binding fragments of antibodies include: (1) a Fab fragment, a monovalent fragment having a VL, a VH, a constant light chain (CL) and a CH1 domain; (2) a F(ab')2 fragment, a bivalent fragment having two Fab fragments linked by a disulfide bridge at the hinge region; (3) an Fd fragment, having two VH and CH1 domains; (4) an Fv fragment, having VL and VH domains of a single arm of an antibody; (5) a dAb fragment (Ward et al., "Binding Activities of a Repertoire of Single Immunoglobulin Variable Domains Secreted From Escherichia coli", Nature 341:544-546 (1989), which is incorporated herein by reference in its entirety), having a VH domain; (6) an isolated complementarity determining region (CDR); (7) a single chain Fv (scFv), e.g., derived from an scFV-library. Although the two domains of the Fv fragment, VL and VH, are encoded by separate genes, they may be joined by a recombinant method using a synthetic linker that allows them to be prepared as a single protein chain in which the VL and VH regions pair to form monovalent molecules (referred to as single chain Fv (scFv)) (see, e.g., Huston et al., "Protein Engineering of Antibody Binding Sites: Recovery of Specific Activity in an Anti-Digoxin Single-Chain Fv Analogue Produced in Escherichia coli", Proc. Natl. Acad. Sci. USA 85: 5879-5883 (1988)); and (8) VHH, which relates to variable antigen-binding domains of heavy chain antibodies from Camelidae (camel, dromedary, llama, alpaca, etc.) (see Nguyen VK. et al., 2000, The EMBO Journal, 19, 921-930; Muyldermans S., 2001, J Biotechnol., 74, 277-302 and a review of Vanl and schoot P. et al., 2011, Antiviral Research 92, 389-407). VHH may also be referred to as nanobody (Nb).

In the present application, the term "variable region" or "variable domain" generally refers to a domain of a heavy chain or light chain of an antibody involved in the binding of the antibody to an antigen. In the present application, the term "variable" generally means that certain portions of the sequences of the variable domains of antibodies vary considerably, resulting in the binding and specificity of various particular antibodies to their particular antigens. Variability is not evenly distributed throughout the variable regions of the antibody. It is concentrated in three segments in each of the light chain and heavy chain variable regions called complementarity determining regions (CDRs) or hypervariable regions (HVRs), which are LCDR1, LCDR2, LCDR3, HCDR1, HCDR2 and HCDR3. The more highly conserved portions of the variable domains are called frameworks (FRs). The variable domains of native heavy and light chains each comprise four FRs (H-FR1, H-FR2, H-FR3, H-FR4, L-FR1, L-FR2, L-FR3 and L-FR4) largely in a β-sheet configuration. The FRs are connected by three CDR structural loop regions. The CDRs in each chain are held in close proximity by the FR regions and form, together with the CDRs from the other chain, the antigen-binding sites of the antibody.

In the art, the variable regions of an antibody can be encoded or the CDRs of an antibody can be divided by various methods, such as Kabat numbering scheme and definition rule based on sequence variability (see Kabat et al., Sequences of Proteins of Immunological Interest, Fifth Edition, National Institutes of Health, Bethesda, Md. (1991)),

Chothia numbering scheme and definition rule based on the regional position of a structural loop (see A1-Lazikani et al., J Mol Biol 273 : 927-48, 1997), IMGT numbering scheme and definition rule based on alignment of amino acid sequences of germline V genes by efranc et al., as well as Honneger's numbering scheme (AHo's), Martin numbering scheme, Gelfand numbering scheme, etc., see Mathieu Dondelinger et al., Understanding the Significance and Implications of Antibody Numbering and Antigen-Binding Surface/Residue Definition, Front. Immunol., October 16, 2018.

In the present application, the term "percent (%) sequence identity" generally refers to the number of matches ("hits") of identical amino acids of two or more aligned amino acid sequences as compared to the number of amino acid residues making up the overall length of the amino acid sequences. In other words, using an alignment, for two or more sequences, the percent of amino acid residues that are the same (e.g., 70%, 75%, 80%, 85%, 90%, 95%, 96%, 97%, 98% or 99% sequence identity) can be determined, when the sequences are compared and aligned for maximum correspondence (as measured using a sequence comparison algorithm known in the art), or when manually aligned and visually inspected. Thus, the sequences which are compared to determine sequence identity may differ by one or more substitutions, additions or deletions of amino acids. Suitable programs for aligning protein sequences are known to those skilled in the art. The percent sequence identity of protein sequences can, for example, be determined with programs such as CLUSTALW, Clustal Omega, FASTA or BLAST, e.g., using the NCBI BLAST algorithm (Altschul SF, et al., (1997), Nucleic Acids Res. 25:3389-3402).

In the present application, the term "antibody analog" is generally used in the broadest sense and specifically covers a molecule that specifically binds to a target molecule with monospecificity and that is structurally different from a native antibody. For example, in the context of describing an anti-PD-1 antibody or an anti-PD-L1 antibody, the term "antibody analog" refers to an antibody that comprises a segment that has substantial identity to a portion of an amino acid sequence and that has at least one of the following properties: (1) specific binding to PD-1 or PD-L1 under suitable binding conditions, and (2) ability to inhibit at least one biological activity of PD-1 or PD-L1. Typically, antibody analogs comprise a conservative amino acid substitution (or insertion or deletion) with respect to the native sequence. Analogs typically are at least 20 or 25 amino acids long, at least 50, 60, 70, 80, 90, 100, 150 or 200 amino acids long or longer, and can generally be as long as a full-length heavy chain or light chain of the antibody. Some examples include antibody analogs with 1, 2, 3, 4, 5, 6, 7, 8, 9, 10, 11, 12, 13, 14, 15, 16 or 17 substitutions as compared to the germline amino acid sequence.

In the present application, the term "effective amount" or "therapeutically effective amount" generally refers to an amount of the compound or pharmaceutical composition described herein sufficient to achieve the intended use described below, including but not limited to disease treatment. The therapeutically effective amount may vary according to: intended use (*in vivo* or *in vitro*); or the subject and disease condition being treated, e.g., the weight and age of the subject, the severity of the disease condition, the mode of administration and the like, which can readily be determined by one of ordinary skill in the art. The term also applies to a dose that will induce a particular response in target cells, which is, for example, platelet adhesion and/or cell migration. The specific dose will vary depending on, for example, the particular compound chosen, the dosing regimen to be followed, whether it is administered in combination with other agents, timing of administration, the tissue to which it is administered, and the physical delivery system in which it is carried.

In the present application, the term "*in vivo*" generally refers to an event that occurs in a subject's body.

In the present application, the term "*in vitro*" generally refers to an event that occurs outside a subject's body. For example, *in vitro* assays include any assay conducted outside of a subject. *In vitro* assays include cell-based assays in which cells, alive or dead, are employed. *In vitro* assays also include a cell-free assay in which no intact cells are employed.

In the present application, the terms "treatment" and "treating" generally refer to a method of achieving beneficial or desired results, including but not limited to therapeutic benefits. Therapeutic benefits include, but are not limited to, eradication, inhibition, reduction, or amelioration of the underlying disorder being treated. In addition, therapeutic benefits are achieved by eradicating, inhibiting, reducing, or ameliorating one or more physiological symptoms associated with the underlying disorder, and thus improvements are observed in the patient, but the patient may still be afflicted with the underlying disorder.

In the present application, the terms "prevention" and "preventing" generally refer to a method of achieving beneficial or desired results, including but not limited to prophylactic benefits. For the purpose of prophylactic benefits, a pharmaceutical composition can be administered to a patient at risk of developing a particular disease, or to a patient who reports they have one or more physiological symptoms of a disease, even though a diagnosis of this disease may not have been made.

In the present application, the term "subject" or "patient" generally refers to a human (i.e., a male or female in any age group, e.g., a pediatric subject (e.g., an infant, a child, or an adolescent) or an adult subject (e.g., a young adult, a middle-aged adult or a senior adult)) and/or other primate (e.g., a cynomolgus monkey or a rhesus monkey); a mammal, including commercially relevant mammals, such as cows, pigs, horses, sheep, goats, cats and/or dogs; and/or birds, including commercially relevant birds such as chickens, ducks, geese, quail and/or turkeys.

In the present application, the term "about" or "approximately" generally refers to an acceptable error of a

particular value as determined by one of ordinary skill in the art, which depends in part on how the value is measured or determined. In certain embodiments, the term "about" or "approximately" generally means within 1, 2, 3 or 4 standard deviations. In certain embodiments, the term "about" or "approximately" generally means within 50%, 20%, 15%, 10%, 9%, 8%, 7%, 6%, 5%, 4%, 3%, 2%, 1%, 0.5%, or 0.05% of a given value or range.

In the present application, the term "comprise" and its variants, including, "comprises", "comprising" and other forms, generally mean being inclusive of other components, elements, integers, steps and the like.

## BRIEF DESCRIPTION OF THE DRAWINGS

FIG. 1 shows the inhibiting effect of the drug conjugate of the present invention on the R848-induced release of IFNα, TNFα, IL-6 and IL-8 from human peripheral blood mononuclear cells, 1a: R848-induced IFNα release from PBMCs; 1b: R848-induced TNFα release from PBMCs; 1c: R848-induced IL6 release from PBMCs, 1d: R848-induced IL8 release from PBMCs.

FIG. 2 shows the determination of biological activity in fluorescein isothiocyanate (FITC)-induced delayed-type IV hypersensitivity mouse models.

FIG. 3 shows the arthritis scores for bovine type II collagen mixed adjuvant-induced DBA/1 mouse arthritis models. 3a: arthritis scores; 3b: AUC for arthritis scores.

## DETAILED DESCRIPTION OF THE INVENTION

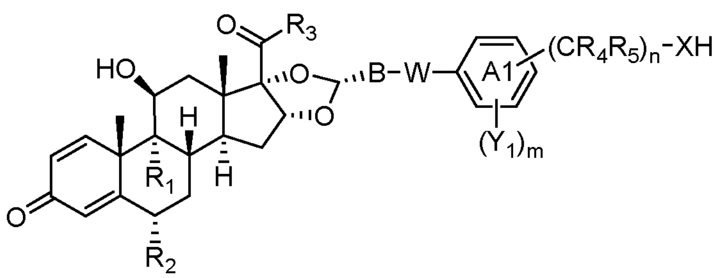

(I)

or a tautomer, a mesomer, a racemate, an enantiomer or a diastereoisomer thereof, or a mixture thereof, or a pharmaceutically acceptable salt thereof, wherein,

$R_1$, $R_2$, $R_3$, $R_4$ and $R_5$ are each independently any group;

B is absent or B is any group;

W is absent or W is any group;

A1 is a substituted benzene ring;

$CR_4R_5$ units are each independently unreplaced or replaced by a group selected from the group consisting of: -O-, -S-, -NR-, -S(O)-, $-S(O)_2-$, -C(=O)-, -C=C- and -C=C-, wherein $R_4$ and $R_5$ can together form optionally substituted cycloalkyl, optionally substituted heterocyclyl, optionally substituted bridged cyclyl, optionally substituted bridged heterocyclyl, optionally substituted spiro cyclyl and optionally substituted spiro heterocyclyl, and n is any integer from 1 to 20;

X is selected from the group consisting of: -O-, -S- and -NR-;

$Y_1$ is any group, and m is any integer from 0 to 4;

wherein substituents are selected from the group consisting of: protium, deuterium, tritium, halogen, -CN, =O, =N-OH, =N-OR, =N-R, -OR, -C(O)R, -C(O)OR, -OC(O)R, -OC(O)OR, -C(O)NHR, - $C(O)NR_2$, -OC(O)NHR, $-OC(O)NR_2$, -SR, -S(O)R, $-S(O)_2R$, -NHR, $-N(R)_2$, -NHC(O)R, -NRC(O)R, -NHC(O)OR, -NRC(O)OR, $-S(O)_2NHR$, $-S(O)_2N(R)_2$, $-NHS(O)_2NR_2$, $-NRS(O)_2NR_2$, $-NHS(O)_2R$, - $NRS(O)_2R$, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy, wherein each R is independently selected from the group consisting of hydrogen, protium, deuterium, tritium, oxygen, hydroxy, halogen, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy.

In certain embodiments, the compound of formula I or the tautomer, the mesomer, the racemate, the enantiomer or the diastereoisomer thereof, or the mixture thereof, or the pharmaceutically acceptable salt thereof is selected from the group consisting of:

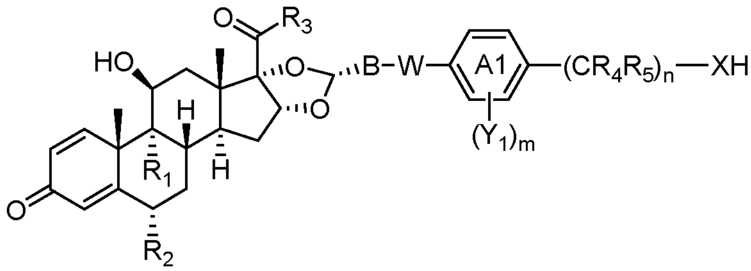

,

(Ia)

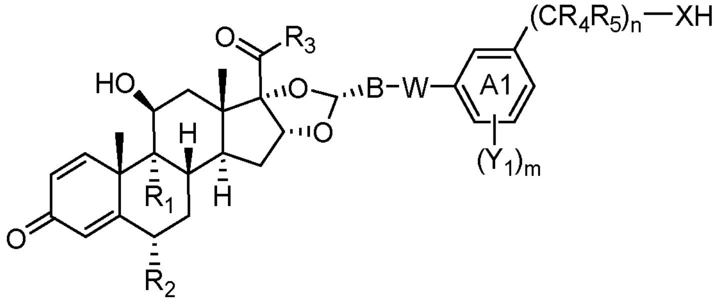

(Ib)

and

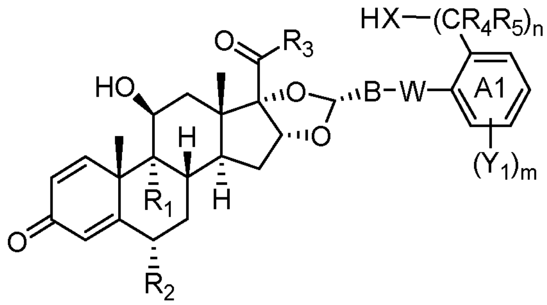

,

(Ic)

wherein,

$R_1$, $R_2$, $R_3$, $R_4$ and $R_5$ are each independently any group;
B is absent or B is any group;
W is absent or W is any group;
A1 is a substituted benzene ring;
$CR_4R_5$ units are each independently unreplaced or replaced by a group selected from the group consisting of: -O-, -S-, -NR-, -S(O)-, $-S(O)_2-$, -C(=O)-, -C=C- and -C=C-, wherein $R_4$ and $R_5$ can together form optionally substituted cycloalkyl, optionally substituted heterocyclyl, optionally substituted bridged cyclyl, optionally substituted bridged heterocyclyl, optionally substituted spiro cyclyl and optionally substituted spiro heterocyclyl, and n is any integer from 1 to 20;

X is selected from the group consisting of: -O-, -S- and -NR-;

$Y_1$ is any group, and m is 0 or 1;

wherein substituents are selected from the group consisting of: protium, deuterium, tritium, halogen, -CN, =O, =N-OH, =N-OR, =N-R, -OR, -C(O)R, -C(O)OR, -OC(O)R, -OC(O)OR, -C(O)NHR, - $C(O)NR_2$, -OC(O)NHR, -OC(O)$NR_2$, -SR, -S(O)R, -$S(O)_2R$, -NHR, -$N(R)_2$, -NHC(O)R, -NRC(O)R, -NHC(O)OR, -NRC(O)OR, -$S(O)_2NHR$, -$S(O)_2N(R)_2$, -$NHS(O)_2NR_2$, -$NRS(O)_2NR_2$, -$NHS(O)_2R$, - $NRS(O)_2R$, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy, wherein each R is independently selected from the group consisting of hydrogen, protium, deuterium, tritium, oxygen, hydroxy, halogen, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy.

In certain preferred embodiments of the present invention, certain groups in the compounds of formulas (I), (Ia), (Ib) and (Ic) or pharmaceutically acceptable salts thereof are defined as follows, and groups not mentioned are as described in any one of the embodiments of the present application ("in certain embodiments" for short).

In certain embodiments, X is -O-, -S- or -NH-.

In certain embodiments, $CR_4R_5$ units are each independently unreplaced or replaced by a group selected from the group consisting of: -O-, -S-and -C(=O)-.

In certain embodiments, $R_4$ and $R_5$ together form optionally substituted cycloalkyl or optionally substituted heterocyclyl.

In certain embodiments, $R_4$ and $R_5$ are each independently selected from the group consisting of: hydrogen, protium, deuterium, tritium, halogen, -$NO_2$, -CN, =O, =S, optionally substituted -S(=O)H, optionally substituted -$S(=O)_2H$, optionally substituted -C(=O)H, optionally substituted -OH, optionally substituted -SH, optionally substituted -$PH_2$, optionally substituted -$P(=O)H_2$, optionally substituted -$NH_2$, optionally substituted alkyl, optionally substituted alkenyl, optionally substituted alkynyl, optionally substituted cycloalkyl, optionally substituted heterocycloalkyl, optionally substituted aryl and optionally substituted heteroaryl; wherein, when $R_4$ and $R_5$ comprise a methylene unit, the methylene units of $R_4$ and $R_5$ are each independently unreplaced, or the methylene units of $R_4$ and $R_5$ are each independently replaced by a group selected from the group consisting of: -S(=O)-, -$S(=O)_2$-, -O-, -S-, -C(=O)-, optionally substituted -PH-, optionally substituted -P(=O)H-, optionally substituted -NH-, optionally substituted alkylene, optionally substituted alkenylene, optionally substituted alkynylene, optionally substituted cycloalkylene, optionally substituted heterocycloalkylene, optionally substituted arylene and optionally substituted heteroarylene.

In certain embodiments, $R_4$ and $R_5$ are each independently selected from the group consisting of: hydrogen, protium, deuterium, tritium, halogen, optionally substituted -C(=O)H, optionally substituted -OH, optionally substituted -SH, optionally substituted -$NH_2$, optionally substituted alkyl, optionally substituted alkenyl, optionally substituted alkynyl, optionally substituted cycloalkyl, optionally substituted heterocycloalkyl, optionally substituted aryl and optionally substituted heteroaryl; wherein, when $R_4$ and $R_5$ comprise a methylene unit, the methylene units of $R_4$ and $R_5$ are each independently unreplaced, or the methylene units of $R_4$ and $R_5$ are each independently replaced by a group selected from the group consisting of: -S(=O)-, -$S(=O)_2$-, -O-, -S-, -C(=O)-, optionally substituted -PH-, optionally substituted -P(=O)H-, optionally substituted -NH-, optionally substituted alkylene, optionally substituted alkenylene, optionally substituted alkynylene, optionally substituted cycloalkylene, optionally substituted heterocycloalkylene, optionally substituted arylene and optionally substituted heteroarylene.

In certain embodiments, provided is the compound or the tautomer, the mesomer, the racemate, the enantiomer or the diastereoisomer thereof, or the mixture thereof, or the pharmaceutically acceptable salt thereof of the present invention, wherein $R_4$ and $R_5$ are each independently selected from the group consisting of: H. F, Cl, -OH, -$NH_2$ and $C_1$-$C_6$ alkyl, or $R_4$ and $R_5$ together form $C_3$-$C_6$ cycloalkyl or 3-6 membered heterocyclyl; and n is selected from the group consisting of 1, 2 and 3.

In certain embodiments, provided is the compound or the tautomer, the mesomer, the racemate, the enantiomer or the diastereoisomer thereof, or the mixture thereof, or the pharmaceutically acceptable salt thereof of the present invention, wherein the $(CR_4R_5)_n$- are each independently selected from the group consisting of -$CH_2$-, -$CH_2CH_2$-,

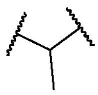, 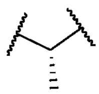, 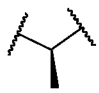 and 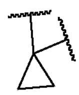.

In certain embodiments, $Y_1$ is absent or selected from the group consisting of: protium, deuterium, tritium, halogen, -OR, -SR, -NHR, -$N(R)_2$, -PHR, -$P(R)_2$, -P(=O)HR, -$P(=O)R_2$, optionally substituted alkyl, optionally substituted alkenyl, optionally substituted alkynyl, optionally substituted cycloalkyl, optionally substituted heterocycloalkyl, optionally substituted aryl and optionally substituted heteroaryl; wherein, each R is independently selected from the group consisting of hydrogen, protium, deuterium, tritium, oxygen, hydroxy, halogen, $C_1$-$C_6$ alkyl, $C_1$-$C_6$ alkoxy, aryl, heteroaryl, cycloalkyl,

heterocycloalkyl, halogenated $C_1$-$C_6$ alkyl and halogenated $C_1$-$C_6$ alkoxy.

In certain embodiments, $Y_1$ is absent or selected from the group consisting of: protium, deuterium, tritium, halogen, -OR, -SR, -NHR, -N(R)$_2$, optionally substituted $C_1$-$C_6$ alkyl and optionally substituted $C_1$-$C_6$ alkoxy; wherein each R is independently selected from the group consisting of hydrogen, protium, deuterium, tritium, hydroxy, halogen, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy.

In certain embodiments, $Y_1$ is absent or selected from the group consisting of: hydroxy, sulfhydryl, amino and optionally substituted $C_1$-$C_6$ alkyl.

For example, A1 (structural unit

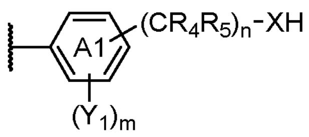

) may be selected from the group consisting of:

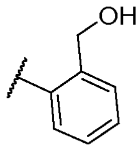 , 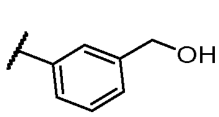 , 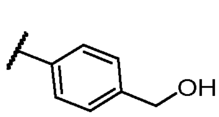 , 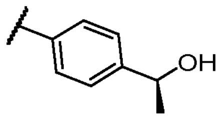 , 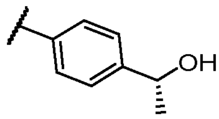 ,

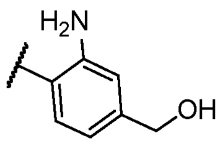 , 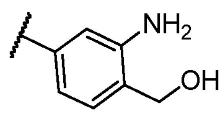 , 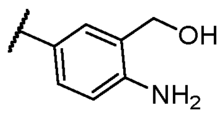 , 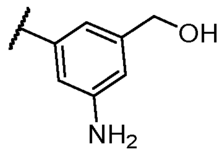 , 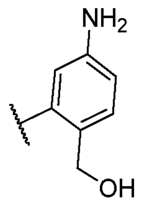 ,

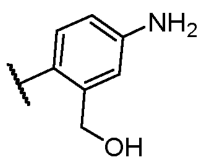 , 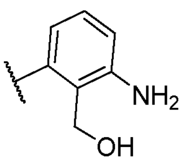 , 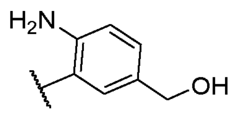 , 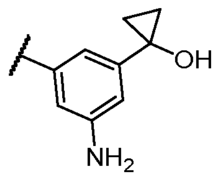 , 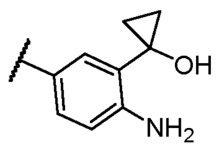 ,

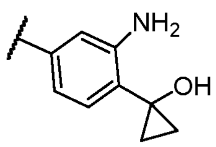 , 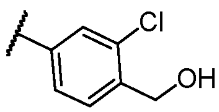 , 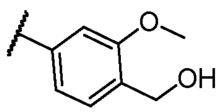 , 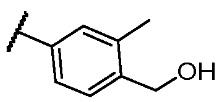 , 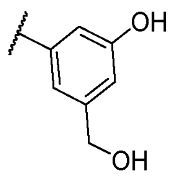 ,

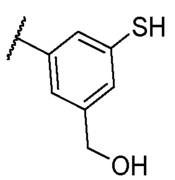 , 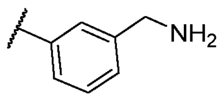 , 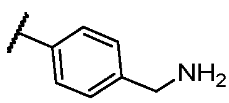 , 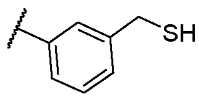 , 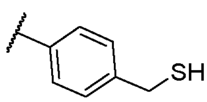 ,

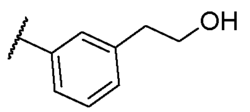 , 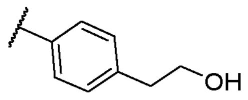 , 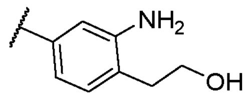 , 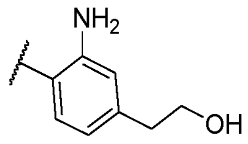 ,

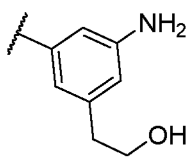 and 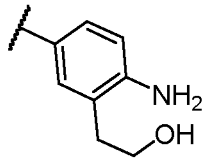 .

In certain embodiments, $R_1$, $R_2$ and $R_3$ are each independently selected from the group consisting of: hydrogen, protium, deuterium, tritium, halogen, $-NO_2$, -CN, =O, =S, optionally substituted -S(=O)H, optionally substituted $-S(=O)_2H$, optionally substituted -C(=O)H, optionally substituted -OH, optionally substituted -SH, optionally substituted $-PH_2$, optionally substituted $-P(=O)H_2$, optionally substituted $-NH_2$, optionally substituted alkyl, optionally substituted alkenyl, optionally substituted alkynyl, optionally substituted cycloalkyl, optionally substituted heterocycloalkyl, optionally substituted aryl and optionally substituted heteroaryl; wherein, when $R_1$, $R_2$ and $R_3$ comprise a methylene unit, the methylene units of $R_1$, $R_2$ and $R_3$ are each independently unreplaced, or the methylene units of $R_1$, $R_2$ and $R_3$ are each independently replaced by a group selected from the group consisting of: -S(=O)-, $-S(=O)_2-$, -O-, -S-, -C(=O)-, optionally substituted -PH-, optionally substituted -P(=O)H-, optionally substituted -NH-, optionally substituted alkylene, optionally substituted alkenylene, optionally substituted alkynylene, optionally substituted cycloalkylene, optionally substituted heterocycloalkylene, optionally substituted arylene and optionally substituted heteroarylene.

In certain embodiments, $R_1$ and $R_2$ are each independently selected from the group consisting of: H, F, Cl, Br and optionally substituted $C_1$-$C_6$ alkyl.

For example, $R_1$ and $R_2$ may each independently be selected from the group consisting of: H, F, Cl, Br and optionally substituted methyl.

In certain embodiments, $R_3$ is selected from the group consisting of: hydrogen, optionally substituted -OH, optionally substituted -SH and optionally substituted $C_1$-$C_6$ alkyl.

In certain embodiments, when $R_3$ is optionally substituted methyl, $R_3$ is substituted with $R_{31}$, and $R_{31}$ is selected from the group consisting of: hydrogen, protium, deuterium, tritium, halogen, $-NO_2$, -CN, =O, =S, optionally substituted -S(=O)H, optionally substituted $-S(=O)_2H$, optionally substituted - C(=O)H, optionally substituted -OH, optionally substituted -SH, optionally substituted $-PH_2$, optionally substituted $-P(=O)H_2$, optionally substituted $-NH_2$, optionally substituted alkyl, optionally substituted alkenyl, optionally substituted alkynyl, optionally substituted cycloalkyl, optionally substituted heterocyclyl, optionally substituted aryl and optionally substituted heteroaryl; wherein, when $R_{31}$ comprises a methylene unit, the methylene unit of $R_{31}$ is each independently unreplaced, or the methylene unit of $R_{31}$ is each independently replaced by a group selected from the group consisting of: -S(=O)-, $-S(=O)_2-$, -O-, -S-, -C(=O)-, optionally substituted -PH-, optionally substituted -P(=O)H-, optionally substituted -NH-, optionally substituted alkylene, optionally substituted alkenylene, optionally substituted alkynylene, optionally substituted cycloalkylene, optionally substituted heterocyclylene, optionally substituted arylene and optionally substituted heteroarylene. In certain embodiments, $R_{31}$ is selected from the group consisting of: hydrogen, halogen, optionally substituted -OH and optionally substituted -SH.

In certain embodiments, when $R_{31}$ is optionally substituted -OH, $R_{31}$ is substituted with $R_{311}$, and $R_{311}$ is selected from the group consisting of: hydrogen, protium, deuterium, tritium, halogen, $-NO_2$, -CN, optionally substituted -S(=O)H, optionally substituted $-S(=O)_2H$, optionally substituted -C(=O)H, optionally substituted -OH, optionally substituted -SH, optionally substituted $-PH_2$, optionally substituted $-P(=O)H_2$, optionally substituted $-NH_2$, optionally substituted alkyl, optionally substituted alkenyl, optionally substituted alkynyl, optionally substituted cycloalkyl, optionally substituted heterocyclyl, optionally substituted aryl and optionally substituted heteroaryl; wherein, when $R_{311}$ comprises a methylene unit, the methylene unit of $R_{311}$ is each independently unreplaced, or the methylene unit of $R_{311}$ is each independently replaced by a group selected from the group consisting of: -S(=O)-, $-S(=O)_2-$, -O-, -S-, -C(=O)-, optionally substituted -PH-, optionally substituted -P(=O)H-, optionally substituted -NH-, optionally substituted alkylene, optionally substituted alkenylene, optionally substituted alkynylene, optionally substituted cycloalkylene, optionally substituted heterocyclylene, optionally substituted arylene and optionally substituted heteroarylene.

In certain embodiments, $R_{311}$ is selected from the group consisting of: hydrogen, optionally substituted $-P(=O)H_2$, optionally substituted -S(=O)H, optionally substituted $-S(=O)_2H$, optionally substituted $C_1$-$C_6$ alkyl and optionally substituted $C_2$-$C_9$ heterocyclylene; wherein, when $R_{311}$ comprises a methylene unit, the methylene unit of $R_{311}$ is each independently unreplaced, or the methylene unit of $R_{311}$ is each independently replaced by a group selected from the group consisting of: -S(=O)-, $-S(=O)_2-$, -O-, -S-, -C(=O)-, optionally substituted -PH-, optionally substituted - $P(=O)H_2-$ and

optionally substituted -NH-; wherein substituents are selected from the group consisting of: protium, deuterium, tritium, halogen, -CN, =O, =N-OH, =N-OR, =N-R, -OR, -C(O)R, -C(O)OR, -OC(O)R, -OC(O)OR, -C(O)NHR, $-C(O)NR_2$, -OC(O)NHR, $-OC(O)NR_2$, -SR, -S(O)R, - $S(O)_2R$, -NHR, $-N(R)_2$, $-N^+(R)_3$, -PHR, $-P(R)_2$, $-P(=O)R_2$, $-OP(=O)R_2$, -NHC(O)R, -NRC(O)R, - NHC(O)OR, -NRC(O)OR, $-S(O)_2NHR$, $-S(O)_2N(R)_2$, $-NHS(O)_2NR_2$, $-NRS(O)_2NR_2$, $-NHS(O)_2R$, - $NRS(O)_2R$, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy, wherein each R is independently selected from the group consisting of hydrogen, protium, deuterium, tritium, oxygen, hydroxy, halogen, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy.

In certain embodiments, $R_{311}$ is selected from the group consisting of: hydrogen, optionally substituted

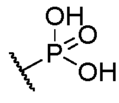

,

optionally substituted

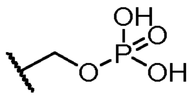

,

optionally substituted

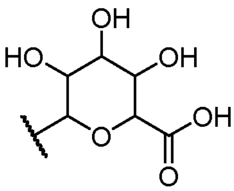

,

optionally substituted

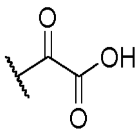

,

optionally substituted

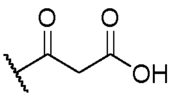

,

optionally substituted

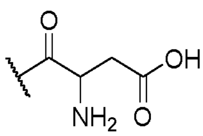

,

optionally substituted

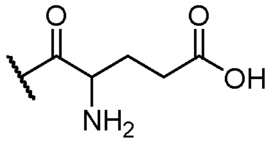

,

optionally substituted

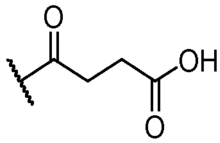

,

optionally substituted

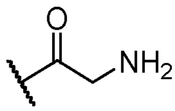

,

optionally substituted

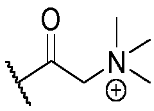

,

optionally substituted

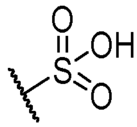

,

optionally substituted

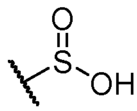

,

optionally substituted

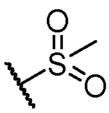

.

In certain embodiments, when $R_3$ is optionally substituted -OH, $R_3$ is substituted with $R_{32}$, and $R_{32}$ is selected from the group consisting of: hydrogen, protium, deuterium, tritium, halogen, $-NO_2$, -CN, optionally substituted -S(=O)H, optionally substituted $-S(=O)_2H$, optionally substituted -C(=O)H, optionally substituted -OH, optionally substituted -SH, optionally substituted $-PH_2$, optionally substituted $-P(=O)H_2$, optionally substituted $-NH_2$, optionally substituted alkyl, optionally substituted alkenyl, optionally substituted alkynyl, optionally substituted cycloalkyl, optionally substituted heterocyclyl, optionally substituted aryl and optionally substituted heteroaryl; wherein, when $R_{32}$ comprises a methylene unit, the methylene unit of $R_{32}$ is each independently unreplaced, or the methylene unit of $R_{32}$ is each independently replaced by a group selected from the group consisting of: -S(=O)-, $-S(=O)_2-$, -O-, -S-, -C(=O)-, optionally substituted

-PH-, optionally substituted -P(=O)H-, optionally substituted -NH-, optionally substituted alkylene, optionally substituted alkenylene, optionally substituted alkynylene, optionally substituted cycloalkylene, optionally substituted heterocyclylene, optionally substituted arylene and optionally substituted heteroarylene.

In certain embodiments, $R_{32}$ is selected from the group consisting of: hydrogen and optionally substituted $C_1$-$C_6$ alkyl.

In certain embodiments, when $R_{32}$ is optionally substituted methyl or optionally substituted ethyl, $R_{32}$ is substituted with $R_{321}$, and $R_{321}$ is selected from the group consisting of: hydrogen, protium, deuterium, tritium, halogen, $-NO_2$, -CN, =O, =S, optionally substituted -S(=O)H, optionally substituted $-S(=O)_2H$, optionally substituted -C(=O)H, optionally substituted -OH, optionally substituted -SH, optionally substituted $-PH_2$, optionally substituted $-P(=O)H_2$, optionally substituted $-NH_2$, optionally substituted alkyl, optionally substituted alkenyl, optionally substituted alkynyl, optionally substituted cycloalkyl, optionally substituted heterocyclyl, optionally substituted aryl and optionally substituted heteroaryl; wherein, when $R_{321}$ comprises a methylene unit, the methylene unit of $R_{321}$ is each independently unreplaced, or the methylene unit of $R_{321}$ is each independently replaced by a group selected from the group consisting of: -S(=O)-, $-S(=O)_2-$, -O-, -S-, -C(=O)-, optionally substituted -PH-, optionally substituted -P(=O)H-, optionally substituted -NH-, optionally substituted alkylene, optionally substituted alkenylene, optionally substituted alkynylene, optionally substituted cycloalkylene, optionally substituted heterocyclylene, optionally substituted arylene and optionally substituted heteroarylene.

In certain embodiments, $R_{321}$ is selected from the group consisting of: hydrogen, halogen, -CN and optionally substituted $C_1$-$C_6$ alkyl.

In certain embodiments, $R_{321}$ is selected from the group consisting of: hydrogen, F, Cl, -CN and optionally substituted methyl.

In certain embodiments, when $R_3$ is optionally substituted -SH, $R_3$ is substituted with $R_{33}$, and $R_{33}$ is selected from the group consisting of: hydrogen, protium, deuterium, tritium, halogen, $-NO_2$, -CN, =O, =S, optionally substituted -S(=O)H, optionally substituted $-S(=O)_2H$, optionally substituted - C(=O)H, optionally substituted -OH, optionally substituted -SH, optionally substituted $-PH_2$, optionally substituted $-P(=O)H_2$, optionally substituted $-NH_2$, optionally substituted alkyl, optionally substituted alkenyl, optionally substituted alkynyl, optionally substituted cycloalkyl, optionally substituted heterocyclyl, optionally substituted aryl and optionally substituted heteroaryl; wherein, when $R_{33}$ comprises a methylene unit, the methylene unit of $R_{33}$ is each independently unreplaced, or the methylene unit of $R_{33}$ is each independently replaced by a group selected from the group consisting of: -S(=O)-, $-S(=O)_2-$, -O-, -S-, -C(=O)-, optionally substituted -PH-, optionally substituted -P(=O)H-, optionally substituted -NH-, optionally substituted alkylene, optionally substituted alkenylene, optionally substituted alkynylene, optionally substituted cycloalkylene, optionally substituted heterocyclylene, optionally substituted arylene and optionally substituted heteroarylene. In certain embodiments, $R_{33}$ is selected from the group consisting of: hydrogen and optionally substituted $C_1$-$C_6$ alkyl.

In certain embodiments, $R_{33}$ is optionally substituted methyl.

In certain embodiments, when $R_{33}$ is optionally substituted methyl, $R_{33}$ is substituted with $R_{331}$, and $R_{331}$ is selected from the group consisting of: hydrogen, protium, deuterium, tritium, halogen, $-NO_2$, -CN, =O, =S, optionally substituted -S(=O)H, optionally substituted $-S(=O)_2H$, optionally substituted -C(=O)H, optionally substituted -OH, optionally substituted -SH, optionally substituted $-PH_2$, optionally substituted $-P(=O)H_2$, optionally substituted $-NH_2$, optionally substituted alkyl, optionally substituted alkenyl, optionally substituted alkynyl, optionally substituted cycloalkyl, optionally substituted heterocyclyl, optionally substituted aryl and optionally substituted heteroaryl; wherein, when $R_{331}$ comprises a methylene unit, the methylene unit of $R_{331}$ is each independently unreplaced, or the methylene unit of $R_{331}$ is each independently replaced by a group selected from the group consisting of: -S(=O)-, $-S(=O)_2-$, -O-, -S-, -C(=O)-, optionally substituted -PH-, optionally substituted -P(=O)H-, optionally substituted -NH-, optionally substituted alkylene, optionally substituted alkenylene, optionally substituted alkynylene, optionally substituted cycloalkylene, optionally substituted heterocyclylene, optionally substituted arylene and optionally substituted heteroarylene. In certain embodiments, $R_{331}$ is selected from the group consisting of: hydrogen, chlorine, fluorine and -CN.

For example, $R_3$ may be selected from the group consisting of: optionally substituted $-CH_2Cl$, optionally substituted $-CH_2SH$, optionally substituted $-CH_2OH$, optionally substituted

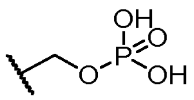

,

optionally substituted

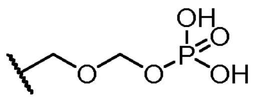,

optionally substituted

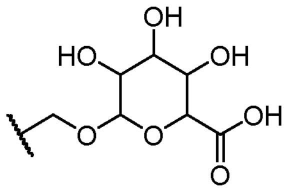,

optionally substituted

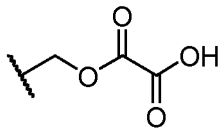,

optionally substituted

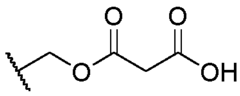,

optionally substituted

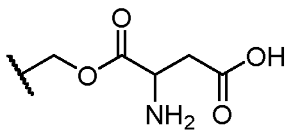,

optionally substituted

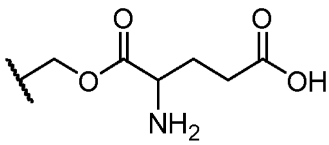,

optionally substituted

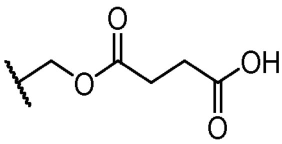,

optionally substituted

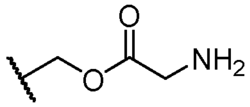

,

optionally substituted

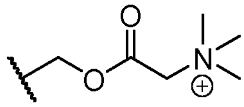

,

optionally substituted

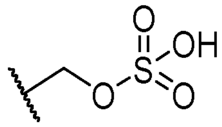

,

optionally substituted

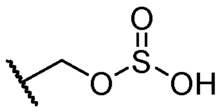

,

optionally substituted

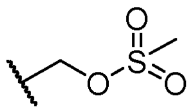

,

optionally substituted -OH, optionally substituted $-OCH_3$, optionally substituted $-OCH_2F$, optionally substituted $-OCH_2Cl$, optionally substituted $-OCH_2CN$, optionally substituted $-OCH_2CH_3$, optionally substituted sulfhydryl, optionally substituted $-SCH_2F$, optionally substituted $-SCH_2Cl$, optionally substituted $-SCH_2CF_3$ and optionally substituted $-SCH_2CN$.

In certain embodiments, provided is the compound or the tautomer, the mesomer, the racemate, the enantiomer or the diastereoisomer thereof, or the mixture thereof, or the pharmaceutically acceptable salt thereof described herein, wherein $R_1$ and $R_2$ are each independently selected from the group consisting of hydrogen, fluorine, chlorine and methyl, and $R_3$ is selected from the group consisting of: $-CH_2Cl$, $-CH_2SH$, $-CH_2OH$,

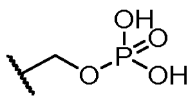

,

$-OCH_3$, $-OCH_2F$, $-OCH_2Cl$, $-OCH_2CN$, $-OCH_2CH_3$, -SH, $-SCH_2F$, $-SCH_2Cl$, $-SCH_2CF_3$ and $-SCH_2CN$.

In certain embodiments, $R_1$ and $R_2$ are each independently selected from the group consisting of hydrogen, protium, deuterium, tritium and halogen, and $R_3$ is selected from the group consisting of $CH_2OH$, $-SCH_2F$,

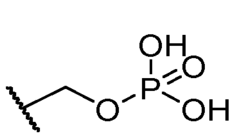

,

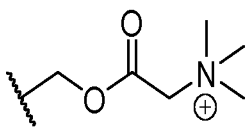

,

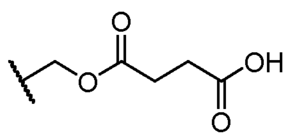

,

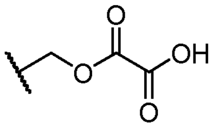 , 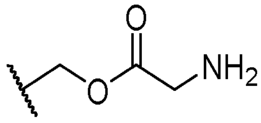 , 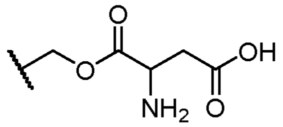 , 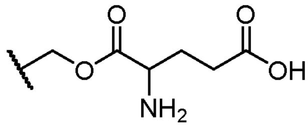 ,

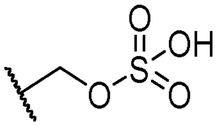 , 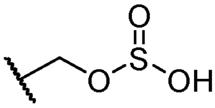 and 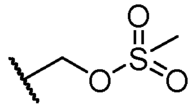 .

In certain embodiments, B is absent or selected from the group consisting of: optionally substituted $C_1$-$C_6$ alkyl, optionally substituted $C_3$-$C_8$ cycloalkyl, optionally substituted $C_3$-$C_8$ heterocyclyl, optionally substituted $C_6$-$C_{10}$ aryl and optionally substituted $C_5$-$C_{10}$ heteroaryl, wherein substituents are selected from the group consisting of: protium, deuterium, tritium, halogen, -CN, =O, =N-OH, =N-OR, =N-R, -OR, -C(O)R, -C(O)OR, -OC(O)R, -OC(O)OR, -C(O)NHR, -C(O)$NR_2$, -OC(O)NHR, -OC(O)$NR_2$, -SR, -S(O)R, -S$(O)_2$R, -NHR, -N$(R)_2$, -NHC(O)R, -NRC(O)R, -NHC(O)OR, -NRC(O)OR, -S$(O)_2$NHR, -S$(O)_2$N$(R)_2$, -NHS$(O)_2$$NR_2$, -NRS$(O)_2$$NR_2$, -NHS$(O)_2$R, -NRS$(O)_2$R, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy; wherein each R is independently selected from the group consisting of hydrogen, protium, deuterium, tritium, oxygen, halogen, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy.

In certain embodiments, B is selected from the group consisting of: optionally substituted

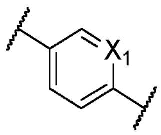 ,

optionally substituted

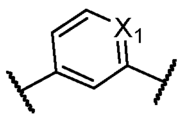

and optionally substituted

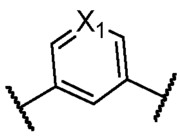 ;

and $X_1$ is selected from the group consisting of: N and optionally substituted CH.

In certain embodiments, when $X_1$ is optionally substituted CH, $X_1$ is substituted with $R_6$, and $R_6$ is selected from the group consisting of: hydrogen, protium, deuterium, tritium, halogen, -$NO_2$, -CN, =O, =S, optionally substituted -S(=O)H, optionally substituted -S$(=O)_2$H, optionally substituted - C(=O)H, optionally substituted -OH, optionally substituted -SH, optionally substituted -$PH_2$, optionally substituted -P(=O)$H_2$, optionally substituted -$NH_2$, optionally substituted alkyl, optionally substituted alkenyl, optionally substituted alkynyl, optionally substituted cycloalkyl, optionally substituted heterocyclyl, optionally substituted aryl and optionally substituted heteroaryl; wherein, when $R_6$ comprises a methylene unit, the methylene unit of $R_6$ is each independently unreplaced, or the methylene unit of $R_6$ is each independently replaced by a group selected from the group consisting of: -S(=O)-, -S$(=O)_2$-, -O-, -S-, -C(=O)-, optionally substituted -PH-, optionally substituted -P(=O)H-, optionally substituted -NH-, optionally substituted alkylene, optionally substituted alkenylene, optionally substituted alkynylene, optionally substituted cycloalkylene, optionally substituted heterocyclylene, optionally substituted arylene and optionally substituted heteroarylene.

In certain embodiments, $R_6$ is selected from the group consisting of: hydrogen and optionally substituted -OH.

In certain embodiments, when $R_6$ is optionally substituted -OH, $R_6$ is substituted with $R_{61}$, and $R_{61}$ is selected from the group consisting of: hydrogen, protium, deuterium, tritium, halogen, $-NO_2$, -CN, =O, =S, optionally substituted -S(=O)H, optionally substituted $-S(=O)_2H$, optionally substituted - C(=O)H, optionally substituted -OH, optionally substituted -SH, optionally substituted $-PH_2$, optionally substituted $-P(=O)H_2$, optionally substituted $-NH_2$, optionally substituted alkyl, optionally substituted alkenyl, optionally substituted alkynyl, optionally substituted cycloalkyl, optionally substituted heterocyclyl, optionally substituted aryl and optionally substituted heteroaryl; wherein, when $R_{61}$ comprises a methylene unit, the methylene unit of $R_{61}$ is each independently unreplaced, or the methylene unit of $R_{61}$ is each independently replaced by a group selected from the group consisting of: -S(=O)-, $-S(=O)_2-$, -O-, -S-, -C(=O)-, optionally substituted -PH-, optionally substituted -P(=O)H-, optionally substituted -NH-, optionally substituted alkylene, optionally substituted alkenylene, optionally substituted alkynylene, optionally substituted cycloalkylene, optionally substituted heterocyclylene, optionally substituted arylene and optionally substituted heteroarylene. In certain embodiments, $R_{61}$ is selected from the group consisting of: H and optionally substituted $C_1$-$C_6$ alkyl.

In certain embodiments, $X_1$ is selected from the group consisting of: CH. C(-O-$CH_3$) and N.

In certain embodiments, B is selected from the group consisting of: optionally substituted

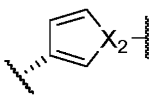
,

optionally substituted

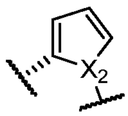
,

optionally substituted

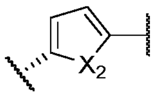
,

optionally substituted

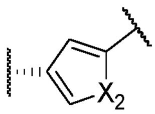
,

optionally substituted

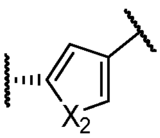
,

optionally substituted

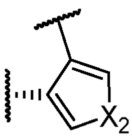
,

optionally substituted

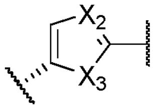

,

optionally substituted

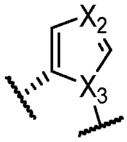

,

optionally substituted

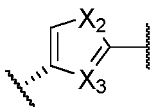

,

optionally substituted

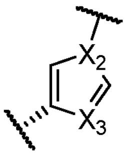

,

optionally substituted

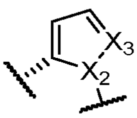

and optionally substituted

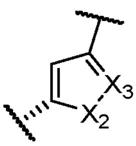

;

and $X_2$ and $X_3$ are each independently selected from the group consisting of: O, S and optionally substituted NH.

In certain embodiments, when $X_2$ and $X_3$ are optionally substituted NH, $X_2$ or $X_3$ is substituted with $R_7$, and $R_7$ is selected from the group consisting of: hydrogen, protium, deuterium, tritium, halogen, $-NO_2$, -CN, =O, =S, optionally substituted -S(=O)H, optionally substituted $-S(=O)_2H$, optionally substituted -C(=O)H, optionally substituted -OH, optionally substituted -SH, optionally substituted - $PH_2$, optionally substituted $-P(=O)H_2$, optionally substituted $-NH_2$, optionally substituted alkyl, optionally substituted alkenyl, optionally substituted alkynyl, optionally substituted cycloalkyl, optionally substituted heterocyclyl, optionally substituted aryl and optionally substituted heteroaryl; wherein, when $R_7$ comprises a methylene unit, the methylene unit of $R_7$ is each independently unreplaced, or the methylene unit of $R_7$ is each independently replaced by a group selected from the group consisting of: -S(=O)-, $-S(=O)_2-$, -O-, -S-, -C(=O)-, optionally substituted -PH-, optionally substituted -P(=O)H-, optionally substituted -NH-, optionally substituted alkylene, optionally substituted alkenylene, optionally substituted alkynylene, optionally substituted cycloalkylene, optionally substituted heterocyclylene, optionally substituted arylene and optionally substituted heteroarylene.

In certain embodiments, $R_7$ is selected from the group consisting of: hydrogen and optionally substituted $C_1$-$C_6$ alkyl; wherein, when $R_7$ comprises a methylene unit, the methylene unit of $R_7$ is each independently unreplaced, or the methylene unit of $R_7$ is each independently replaced by a group selected from the group consisting of: -S(=O)-, $-S(=O)_2-$, -O-, -S-, -C(=O)-, optionally substituted - PH-, optionally substituted -P(=O)H-, optionally substituted -NH-, optionally substituted alkylene, optionally substituted alkenylene, optionally substituted alkynylene, optionally substituted cycloalkylene, optionally substituted heterocyclylene, optionally substituted arylene and optionally substituted heteroarylene.

In certain embodiments, when $R_7$ is optionally substituted $C_1$-$C_6$ alkyl, $R_7$ is substituted with $R_{71}$, and $R_{71}$ is selected from the group consisting of: hydrogen, protium, deuterium, tritium, halogen, $-NO_2$, - CN, =O, =S, optionally substituted -S(=O)H, optionally substituted $-S(=O)_2H$, optionally substituted -C(=O)H, optionally substituted -OH, optionally substituted -SH, optionally substituted $-PH_2$, optionally substituted $-P(=O)H_2$, optionally substituted $-NH_2$, optionally substituted alkyl, optionally substituted alkenyl, optionally substituted alkynyl, optionally substituted cycloalkyl, optionally substituted heterocyclyl, optionally substituted aryl and optionally substituted heteroaryl; wherein, when $R_{71}$ comprises a methylene unit, the methylene unit of $R_{71}$ is each independently unreplaced, or the methylene unit of $R_{71}$ is each independently replaced by a group selected from the group consisting of: -S(=O)-, $-S(=O)_2-$, -O-, -S-, -C(=O)-, optionally substituted -PH-, optionally substituted -P(=O)H-, optionally substituted -NH-, optionally substituted alkylene, optionally substituted alkenylene, optionally substituted alkynylene, optionally substituted cycloalkylene, optionally substituted heterocyclylene, optionally substituted arylene and optionally substituted heteroarylene. In certain embodiments, $R_{71}$ is selected from the group consisting of: H and optionally substituted $C_1$-$C_6$ alkyl.

In certain embodiments, $X_2$ or $X_3$ is selected from the group consisting of: NH, $N(CH_3)$, O and S.

For example, B may be selected from the group consisting of: optionally substituted

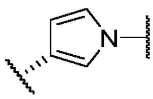,

optionally substituted

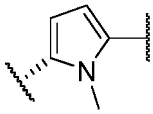,

optionally substituted

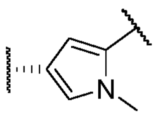,

optionally substituted

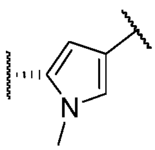,

optionally substituted

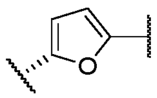,

optionally substituted

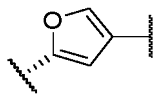

,

optionally substituted

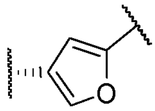

,

optionally substituted

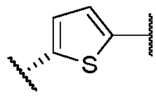

,

optionally substituted

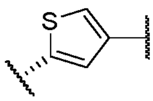

,

optionally substituted

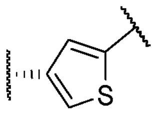

,

optionally substituted

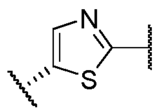

,

optionally substituted

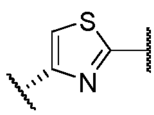

,

optionally substituted

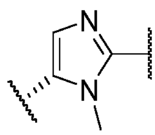

,

optionally substituted

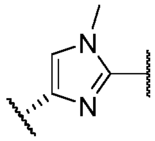,

optionally substituted

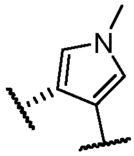,

optionally substituted

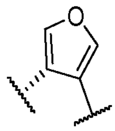,

optionally substituted

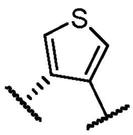,

optionally substituted

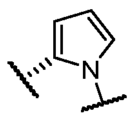,

optionally substituted

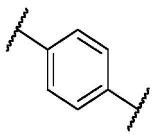

and optionally substituted

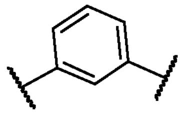,

wherein substituents are selected from the group consisting of: protium, deuterium, tritium, halogen, -CN, =O, =N-OH, =N-OR, =N-R, -OR, - C(O)R, -C(O)OR, -OC(O)R, -OC(O)OR, -C(O)NHR, -C(O)$NR_2$, -OC(O)NHR, -OC(O)$NR_2$, -SR, -S(O)R, -S$(O)_2$R, -NHR, -N$(R)_2$, -NHC(O)R, -NRC(O)R, -NHC(O)OR, -NRC(O)OR, -S$(O)_2$NHR, - S$(O)_2$N$(R)_2$, -NHS$(O)_2$$NR_2$, -NRS$(O)_2$$NR_2$, -NHS$(O)_2$R, -NRS$(O)_2$R, $C_1$-$C_6$ alkyl, $C_1$-$C_6$ alkoxy, aryl, heteroaryl, cycloalkyl, hetero-

cycloalkyl, halogenated $C_1$-$C_6$ alkyl and halogenated $C_1$-$C_6$ alkoxy, wherein each R is independently selected from the group consisting of hydrogen, protium, deuterium, tritium, oxygen, halogen, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy.

In certain embodiments, provided is the compound or the tautomer, the mesomer, the racemate, the enantiomer or the diastereoisomer thereof, or the mixture thereof, or the pharmaceutically acceptable salt thereof of the present invention, wherein B is selected from the group consisting of:

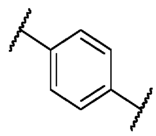,

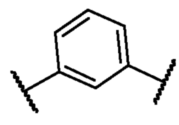 and 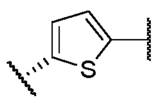.

In certain embodiments, W is absent or W is selected from the group consisting of: -S(=O)-, -S(=O)$_2$-, -O-, -S-, optionally substituted -NHC(=O)-, optionally substituted -C(=O)NH-, optionally substituted -PH-, optionally substituted -P(=O)H-, optionally substituted -NH-, optionally substituted alkylene, optionally substituted alkenylene, optionally substituted alkynylene, optionally substituted cycloalkylene, optionally substituted heterocycloalkylene, optionally substituted arylene and optionally substituted heteroarylene; wherein, when W comprises a methylene unit, the methylene unit of W is each independently unreplaced, or the methylene unit of W is each independently replaced by a group selected from the group consisting of: -S(=O)-, -S(=O)$_2$-, -O-, -S-, -C(=O)-, optionally substituted -PH-, optionally substituted -P(=O)H-, optionally substituted -NH-, optionally substituted alkylene, optionally substituted alkenylene, optionally substituted alkynylene, optionally substituted cycloalkylene, optionally substituted heterocyclylene, optionally substituted arylene and optionally substituted heteroarylene.

In certain embodiments, when W is optionally substituted $C_1$-$C_6$ alkylene, W is substituted with $R_8$, and $R_8$ is selected from the group consisting of: hydrogen, protium, deuterium, tritium, halogen, - $NO_2$, -CN, =O, =S, optionally substituted -S(=O)H, optionally substituted -S(=O)$_2$H, optionally substituted -C(=O)H, optionally substituted -OH, optionally substituted -SH, optionally substituted - $PH_2$, optionally substituted -P(=O)$H_2$, optionally substituted -$NH_2$, optionally substituted alkyl, optionally substituted alkenyl, optionally substituted alkynyl, optionally substituted cycloalkyl, optionally substituted heterocyclyl, optionally substituted aryl and optionally substituted heteroaryl; wherein, when $R_8$ comprises a methylene unit, the methylene unit of $R_8$ is each independently unreplaced, or the methylene unit of $R_8$ is each independently replaced by a group selected from the group consisting of: -S(=O)-, -S(=O)$_2$-, -O-, -S-, -C(=O)-, optionally substituted -PH-, optionally substituted -P(=O)H-, optionally substituted -NH-, optionally substituted alkylene, optionally substituted alkenylene, optionally substituted alkynylene, optionally substituted cycloalkylene, optionally substituted heterocyclylene, optionally substituted arylene and optionally substituted heteroarylene.

In certain embodiments, $R_8$ is selected from the group consisting of: hydrogen, =O, optionally substituted $C_1$-$C_6$ alkyl and optionally substituted cycloalkyl.

In certain embodiments, $R_8$ is optionally substituted methyl.

In certain embodiments, when $R_8$ is optionally substituted methyl, $R_8$ is substituted with $R_{81}$, and $R_{81}$ is selected from the group consisting of: hydrogen, protium, deuterium, tritium, halogen, -$NO_2$, -CN, =O, =S, optionally substituted -S(=O)H, optionally substituted -S(=O)$_2$H, optionally substituted - C(=O)H, optionally substituted -OH, optionally substituted -SH, optionally substituted -$PH_2$, optionally substituted -P(=O)$H_2$, optionally substituted -$NH_2$, optionally substituted alkyl, optionally substituted alkenyl, optionally substituted alkynyl, optionally substituted cycloalkyl, optionally substituted heterocycloalkyl, optionally substituted aryl and optionally substituted heteroaryl.

In certain embodiments, $R_{81}$ is selected from the group consisting of: halogen and optionally substituted cycloalkyl.

In certain embodiments, $R_{81}$ is selected from the group consisting of: F and optionally substituted cyclopropyl.

In certain embodiments, W is absent or W is selected from the group consisting of: -S(=O)-, -S(=O)$_2$-, -O-, -S-, -C(=O)-, optionally substituted -$CH_2$NHC(=O)-, optionally substituted -NHC(=O)$CH_2$-, optionally substituted -C(=O)NH-, optionally substituted -NH-, optionally substituted -CH=CH-, - C=C-, optionally substituted $C_1$-$C_6$ alkylene, optionally substituted -O$CH_2$-, optionally substituted - $CH_2$O-, optionally substituted -S$CH_2$-, optionally substituted -$CH_2$S-, optionally substituted - NHC(=O)-, optionally substituted -C(=O)$CH_2$-, optionally substituted -$CH_2$NH-, optionally substituted -NH$CH_2$-, optionally substituted

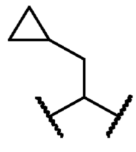,

optionally substituted

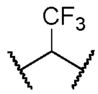,

optionally substituted

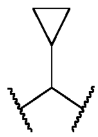,

optionally substituted

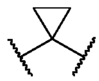

and optionally substituted

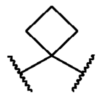.

For example, W may be absent or W is selected from the group consisting of:

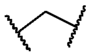, 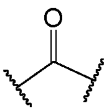, 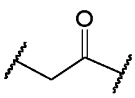, 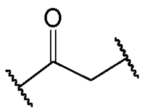, 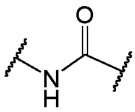, 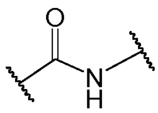, 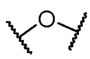, 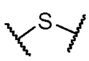, 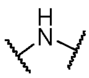,

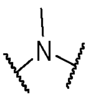, 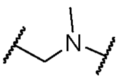, 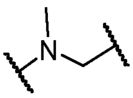, 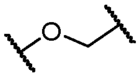, 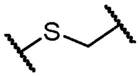, 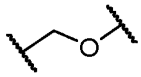, 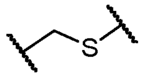, 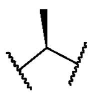,

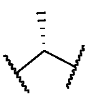, 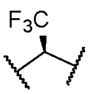, 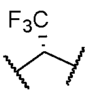, 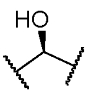, 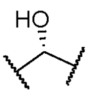, 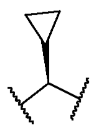, 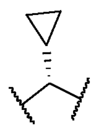, 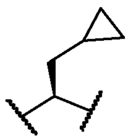, 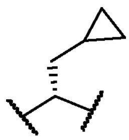, 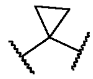,

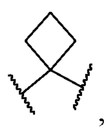, 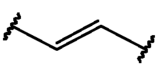 and 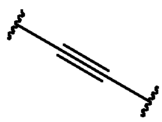.

In certain embodiments, provided is the compound or the tautomer, the mesomer, the racemate, the enantiomer or the diastereoisomer thereof, or the mixture thereof, or the pharmaceutically acceptable salt thereof of the present invention, wherein W is absent or W is

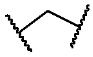.

For another example, B may be absent or selected from the group consisting of:

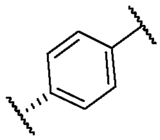,

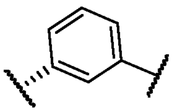, 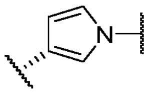, 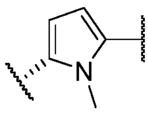, 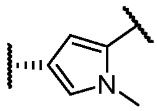, 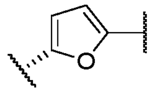, 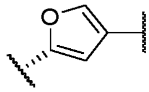, 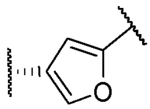,

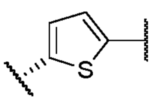, 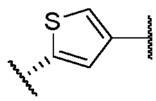, 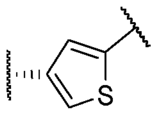, 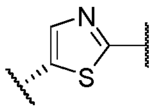 and 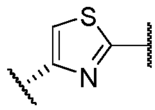;

W may be absent or W is selected from the group consisting of:

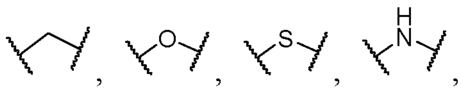,

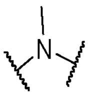, 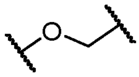, 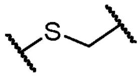, 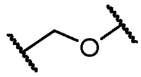, 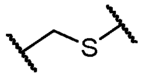, 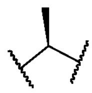, 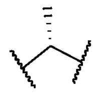, 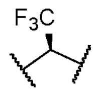, 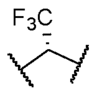,

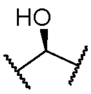, 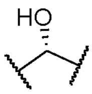, 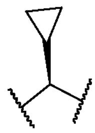, 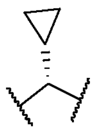, 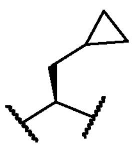, 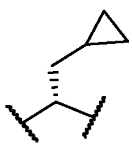, 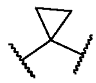 and 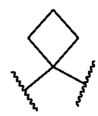;

A1 may be selected from the group consisting of:

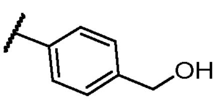, 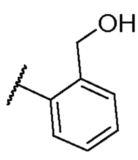, 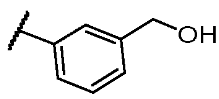, 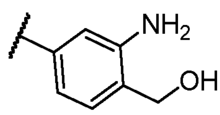, 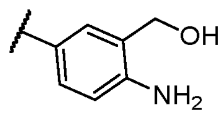,

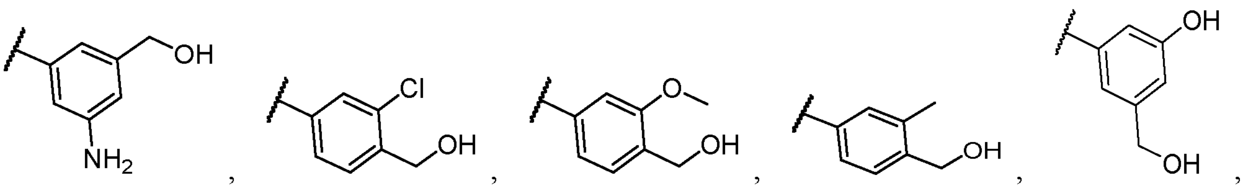,

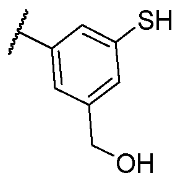, 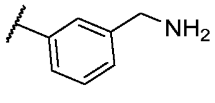, 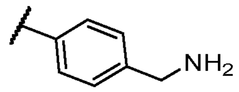, 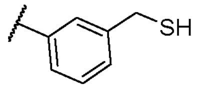, 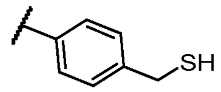,

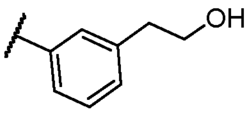 and 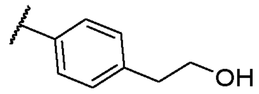.

In certain embodiments, provided is the compound or the tautomer, the mesomer, the racemate, the enantiomer or the diastereoisomer thereof, or the mixture thereof, or the pharmaceutically acceptable salt thereof of the present invention, which is selected from the group consisting of:

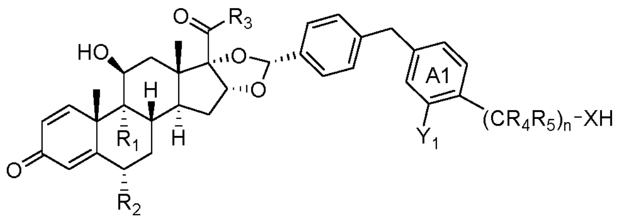

(Ia-1)

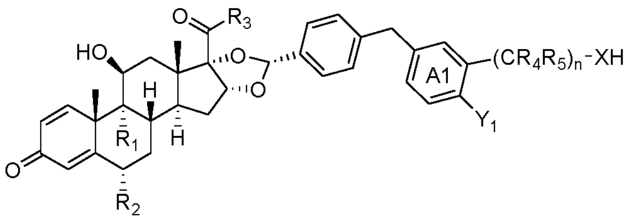

(Ib-1)

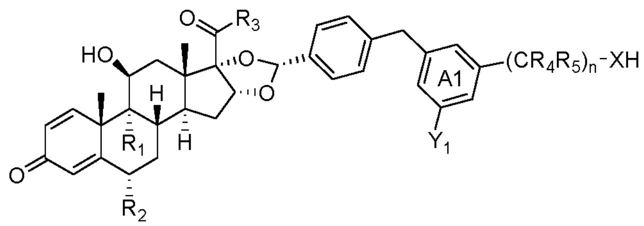

(Ic-1)

,

wherein,

X is selected from the group consisting of -O-, -S- and -NH-;
$R_4$ and $R_5$ are each independently selected from the group consisting of: H, F, Cl, -OH, $-NH_2$ and $C_1$-$C_6$ alkyl;
N is selected from the group consisting of 1, 2 and 3;
$Y_1$ is absent or selected from the group consisting of: hydroxy, sulfhydryl, amino and $C_1$-$C_6$ alkyl;
$R_1$ and $R_2$ are each independently selected from the group consisting of hydrogen, fluorine, chlorine and methyl;
$R_3$ is selected from the group consisting of: $-CH_2Cl$, $-CH_2SH$, $-CH_2OH$,

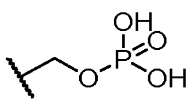,

$-OCH_3$, - $OCH_2F$, $-OCH_2Cl$, $-OCH_2CN$, $-OCH_2CH_3$, -SH, $-SCH_2F$, $-SCH_2Cl$, $-SCH_2CF_3$ and $-SCH_2CN$.

For example, the compound of formula I may be selected from the group consisting of the following structures:

| No. | Structure |
|---|---|
| I-1 | 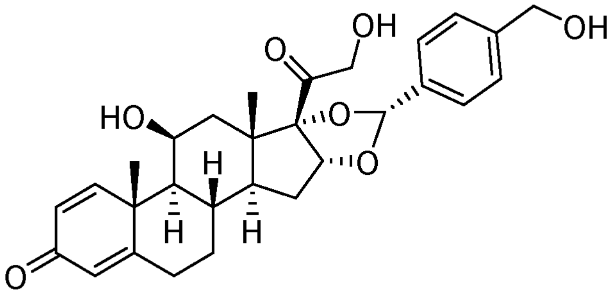 |
| I-2 | 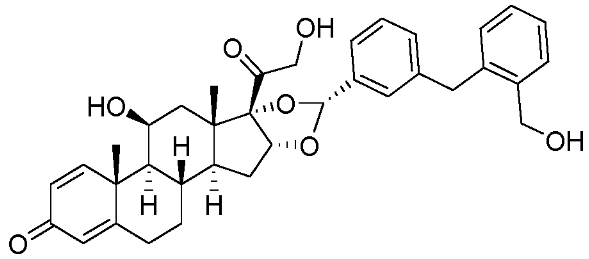 |
| I-3 | 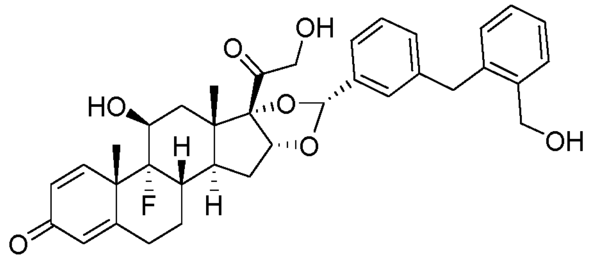 |
| I-4 | 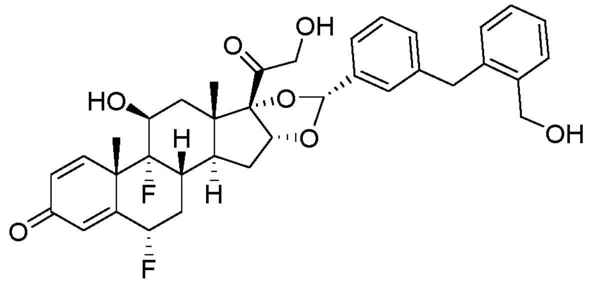 |
| I-5 | 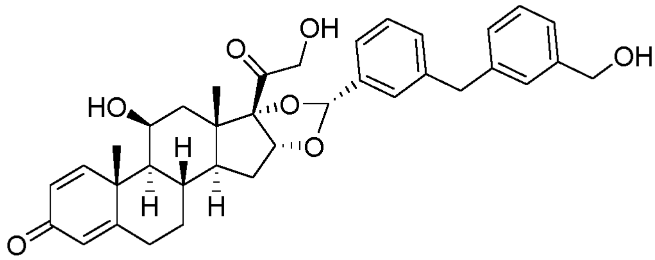 |
| I-6 | 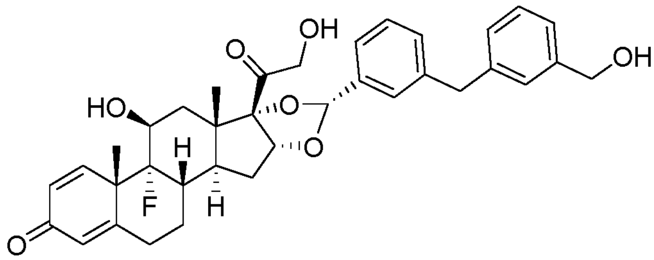 |

(continued)

| No. | Structure |
|---|---|
| I-7 | 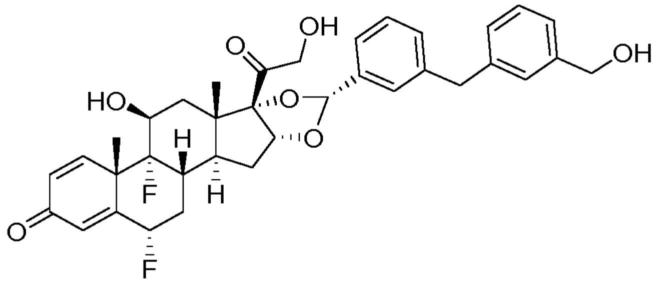 |
| I-8 | 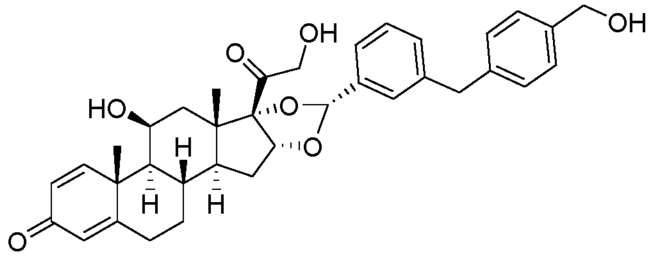 |
| I-9 | 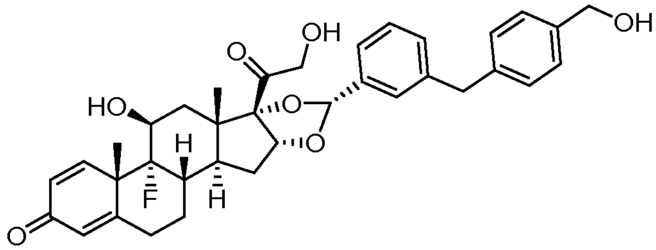 |
| I-10 | 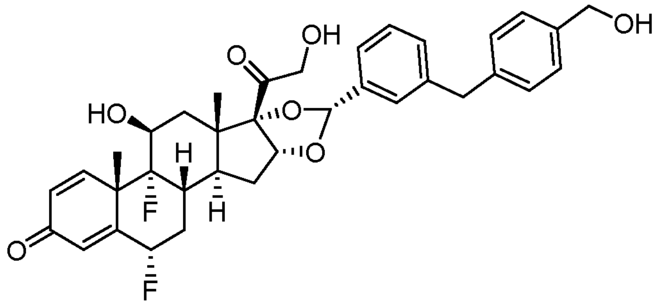 |
| I-11 | 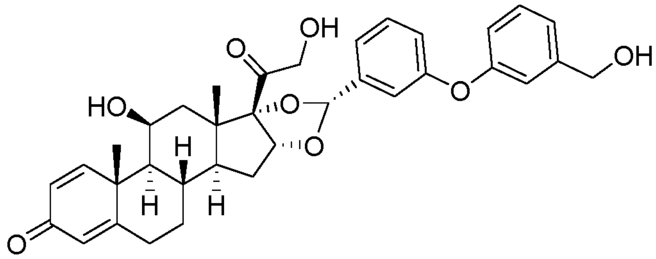 |

(continued)

| No. | Structure |
| --- | --- |
| I-12 | 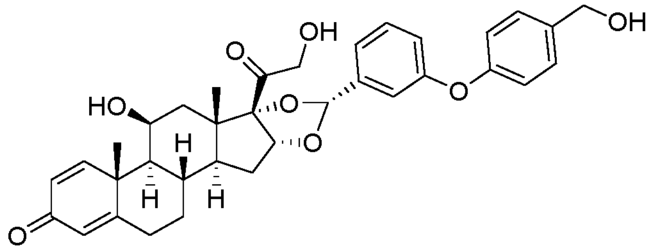 |
| I-13 | 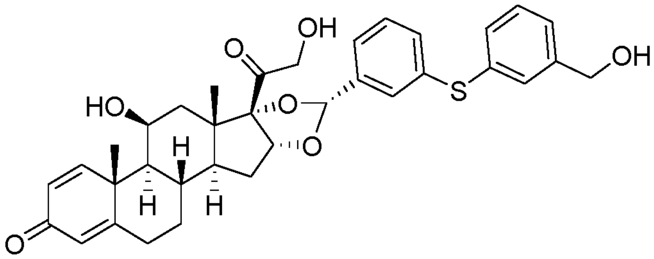 |
| I-14 | 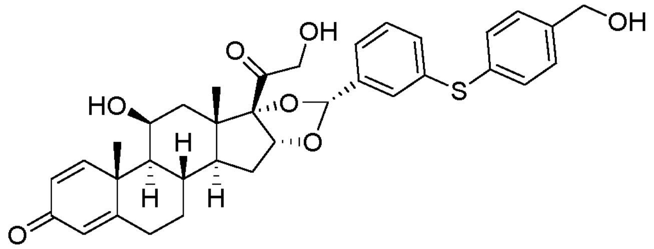 |
| I-15 | 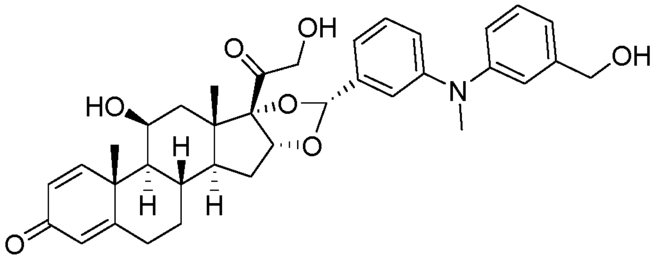 |
| I-16 | 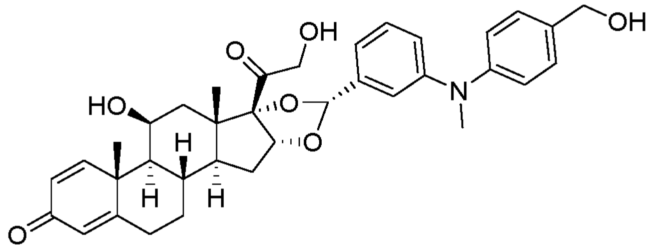 |
| I-17 | 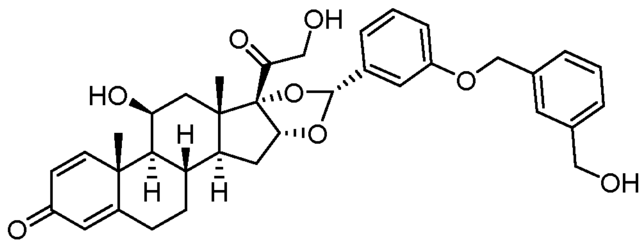 |

(continued)

| No. | Structure |
|---|---|
| I-18 | 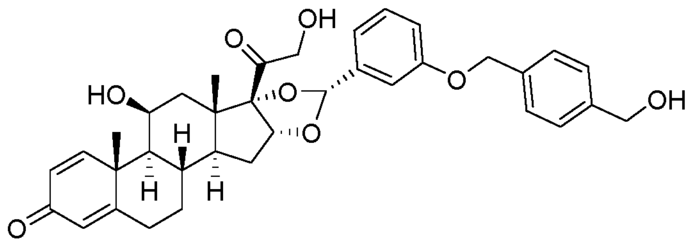 |
| I-19 | 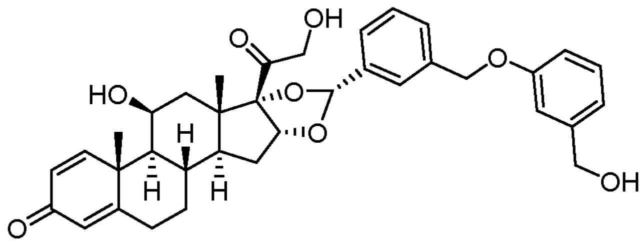 |
| I-20 | 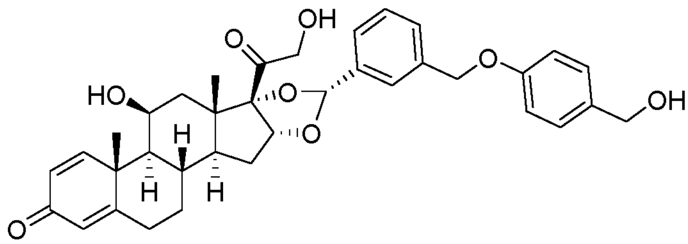 |
| I-21 | 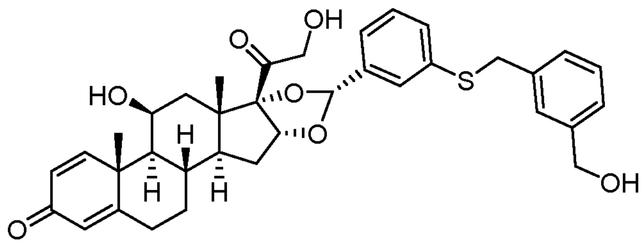 |
| I-22 | 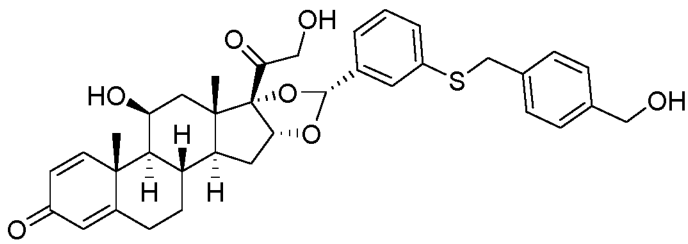 |
| I-23 | 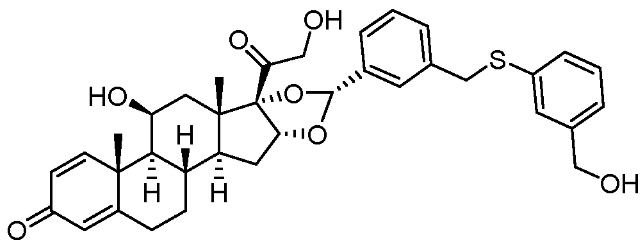 |

(continued)

| No. | Structure |
|---|---|
| I-24 | 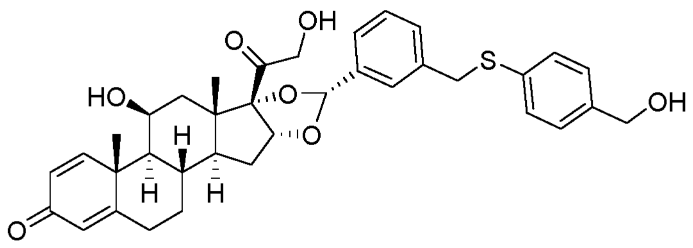 |
| I-25 | 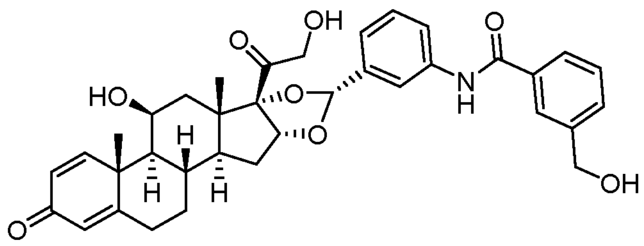 |
| I-26 | 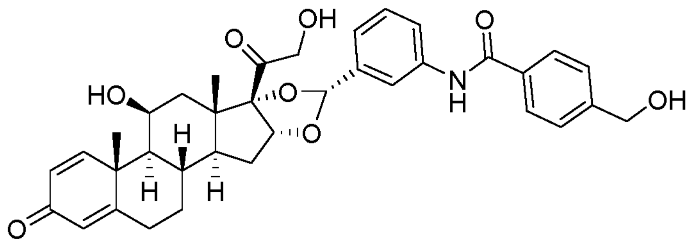 |
| I-27 | 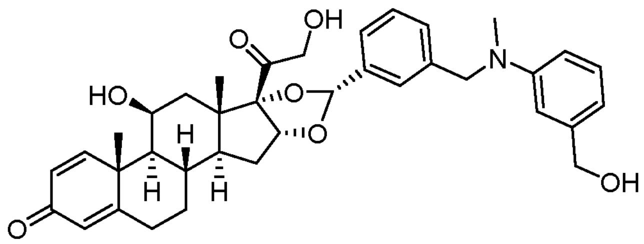 |
| I-28 | 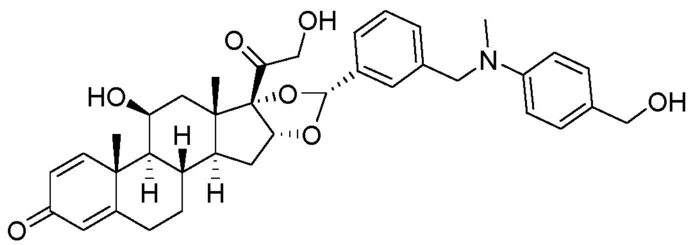 |
| I-29 | 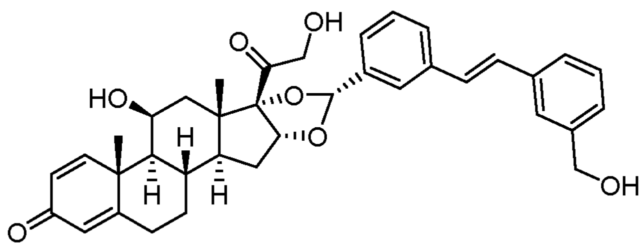 |

(continued)

| No. | Structure |
| --- | --- |
| I-30 | 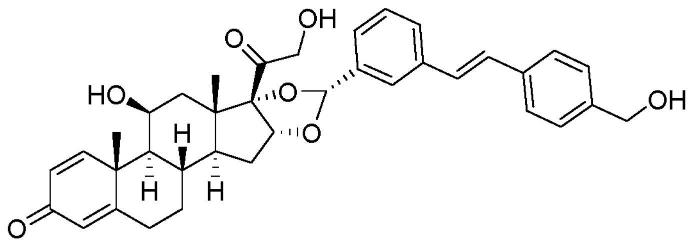 |
| I-31 | 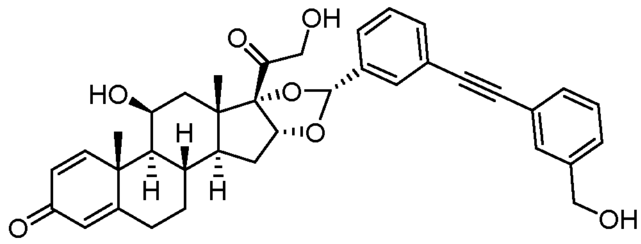 |
| I-32 | 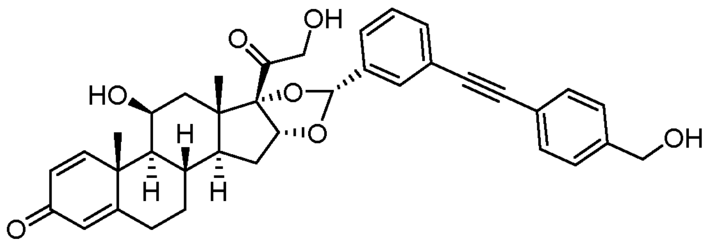 |
| I-33 | 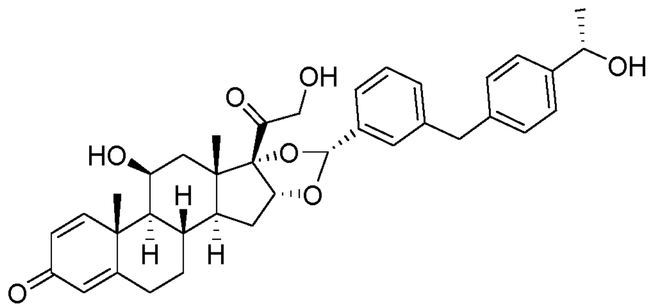 |
| I-34 | 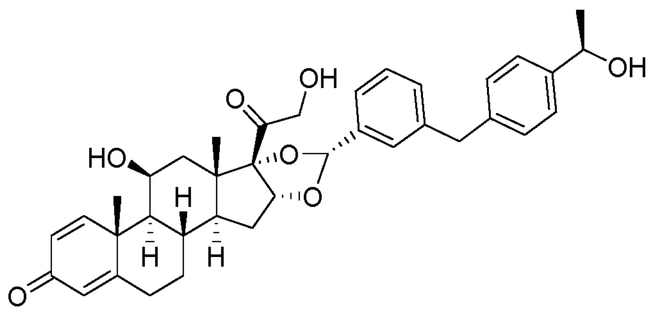 |

(continued)

| No. | Structure |
|---|---|
| I-35 | 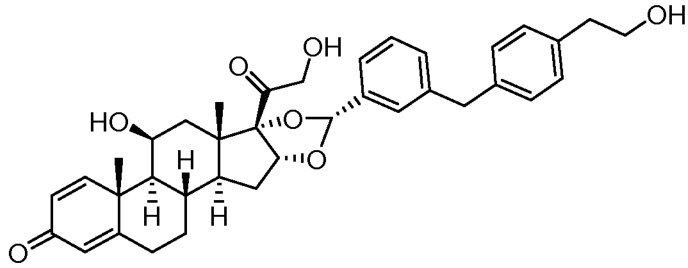 |
| I-36 | 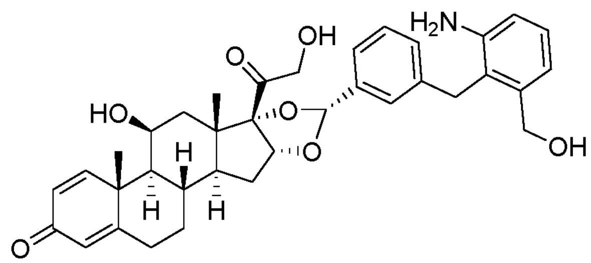 |
| I-37 | 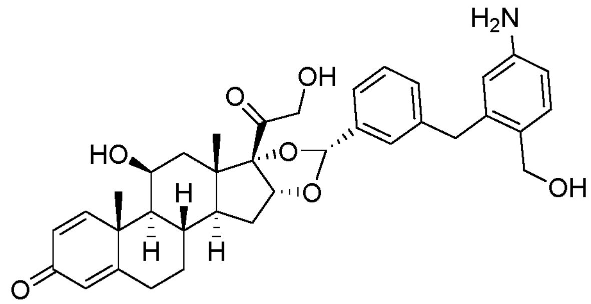 |
| I-38 | 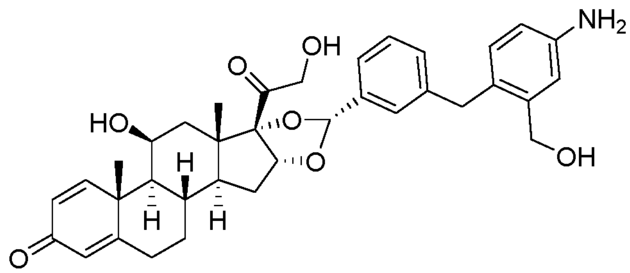 |
| I-39 | 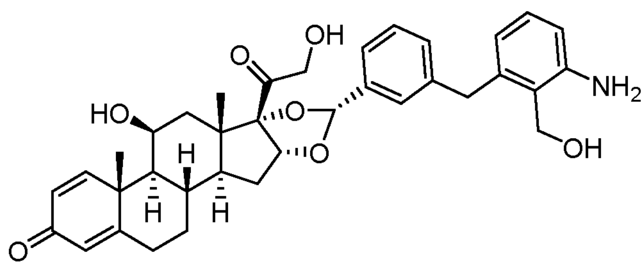 |

(continued)

| No. | Structure |
|---|---|
| I-40 | 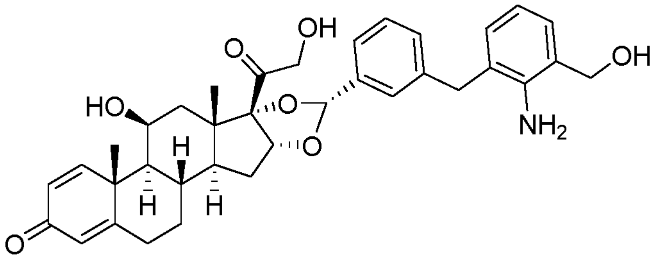 |
| I-41 | 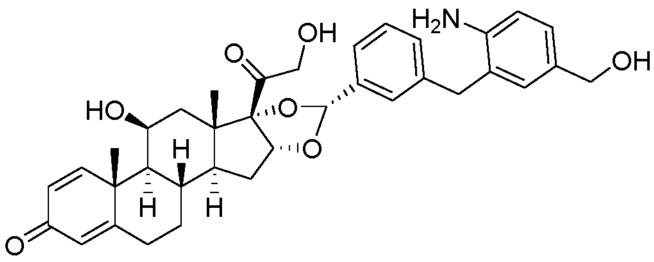 |
| I-42 | 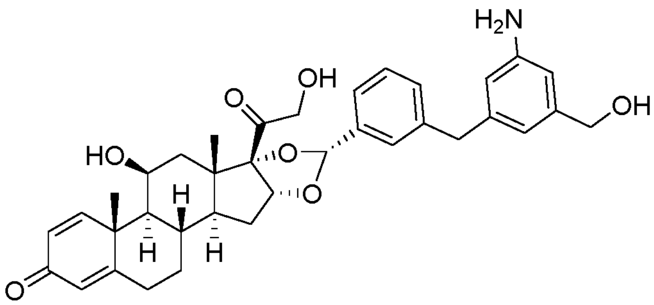 |
| I-43 | 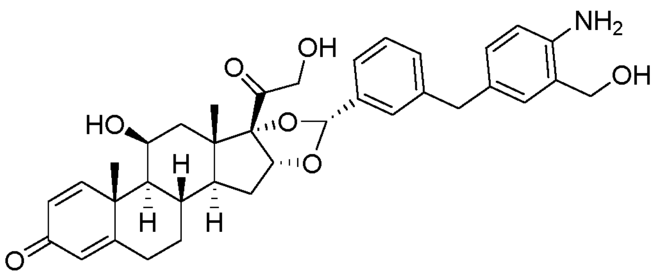 |
| I-44 | 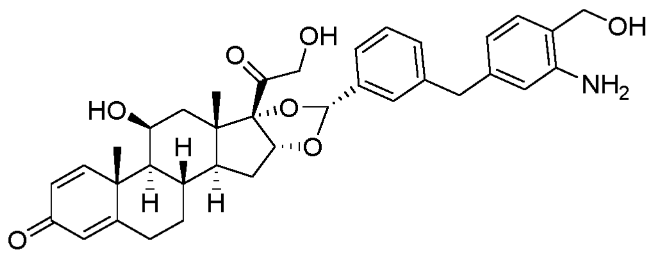 |

(continued)

| No. | Structure |
|---|---|
| I-45 | 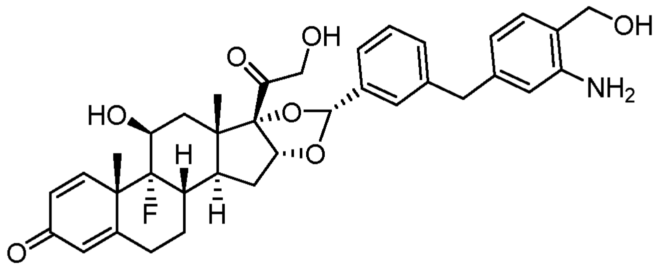 |
| I-46 | 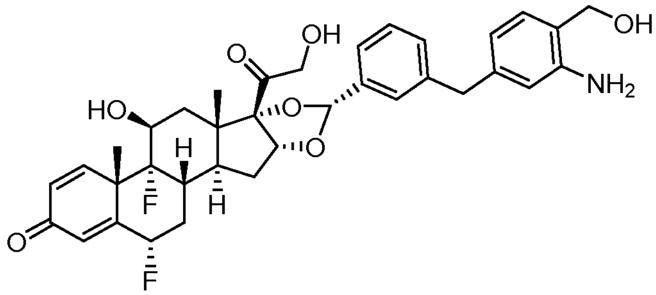 |
| I-47 | 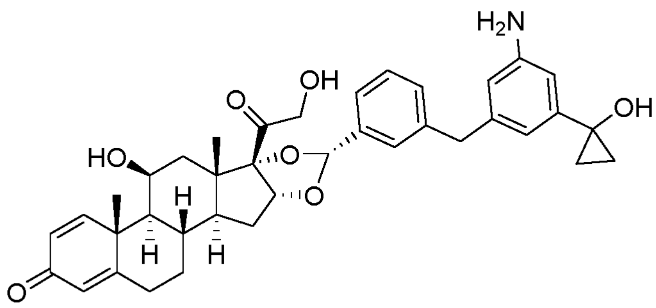 |
| I-48 | 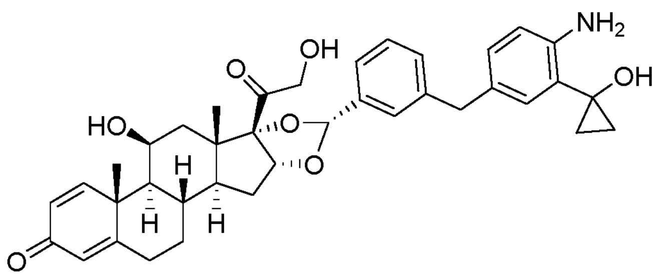 |
| I-49 | 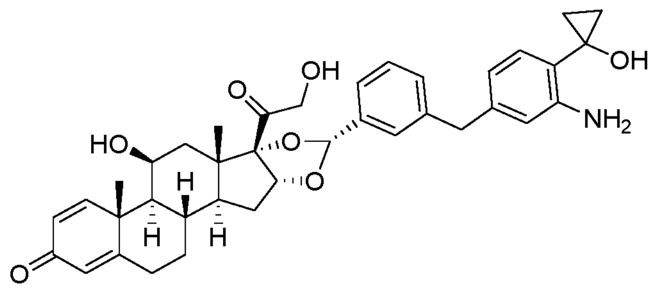 |

(continued)

| No. | Structure |
|---|---|
| I-50 | 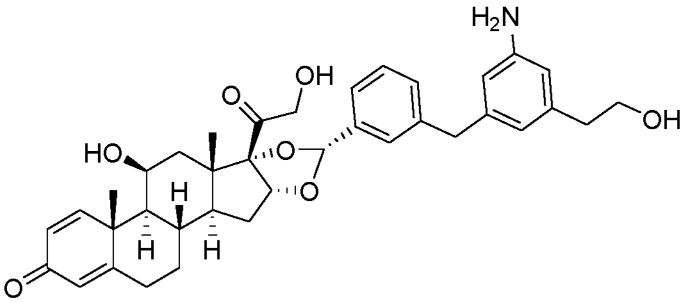 |
| I-51 | 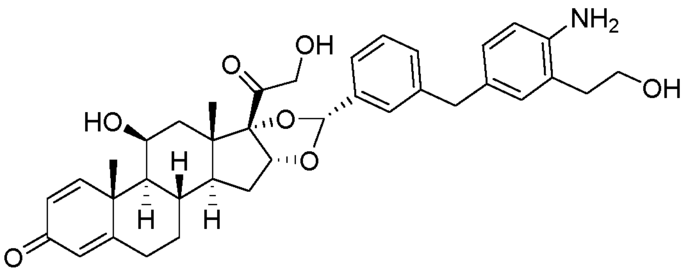 |
| I-52 | 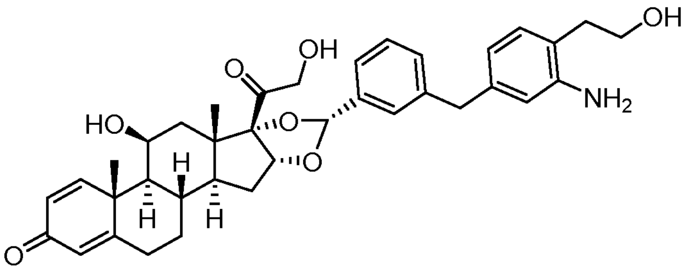 |
| I-53 | 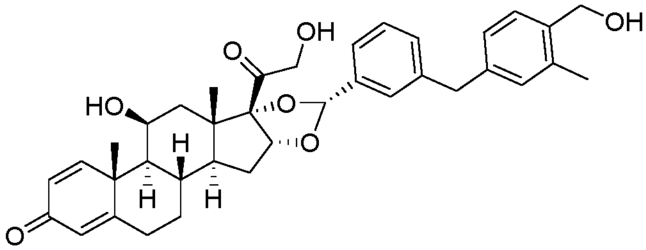 |
| I-54 | 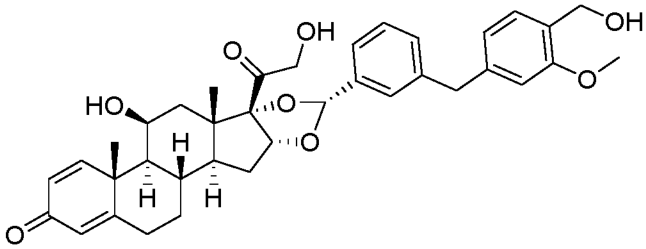 |

(continued)

| No. | Structure |
|---|---|
| I-55 | 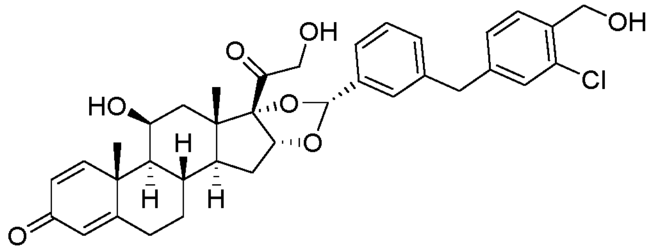 |
| I-56 | 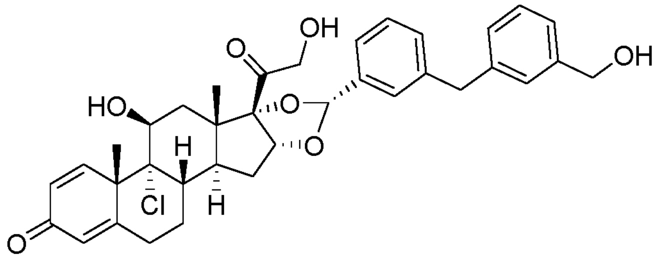 |
| I-57 | 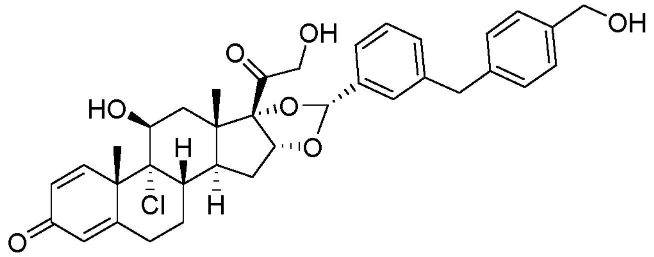 |
| I-58 | 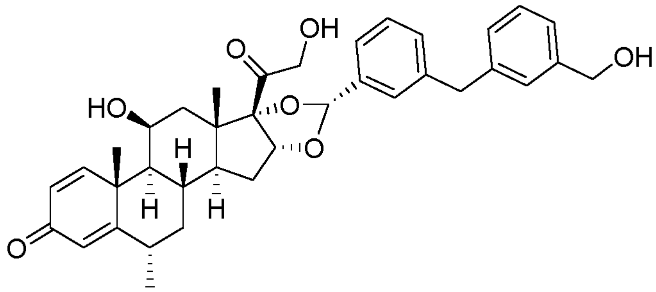 |
| I-59 | 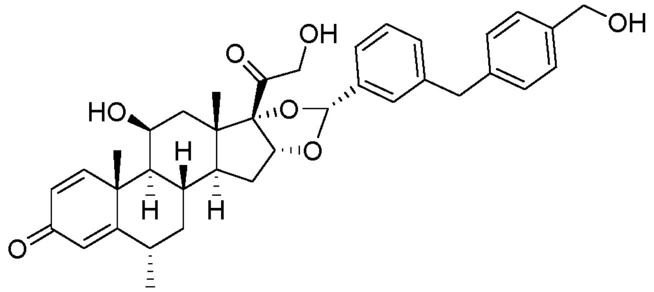 |

(continued)

| No. | Structure |
|---|---|
| I-60 | 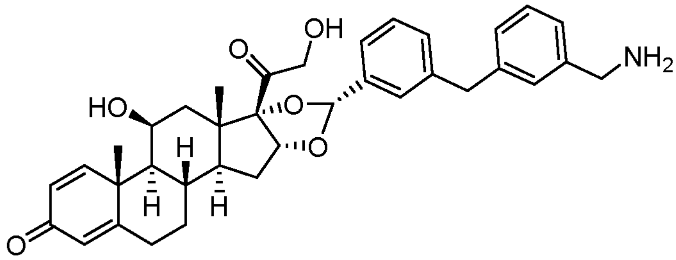 |
| I-61 | 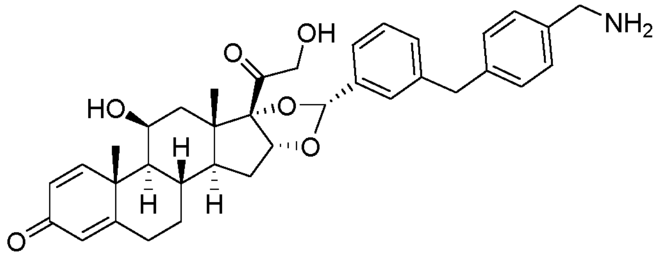 |
| I-62 | 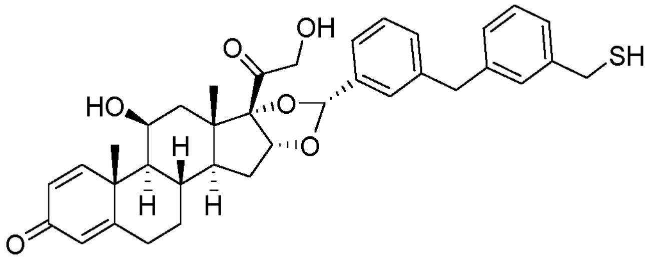 |
| I-63 | 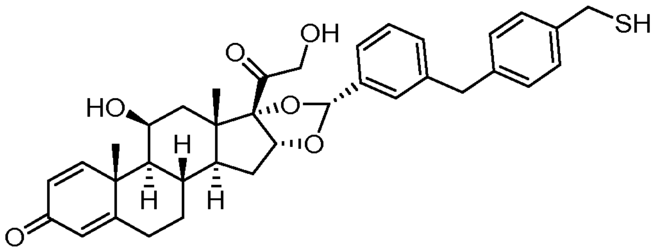 |
| I-64 | 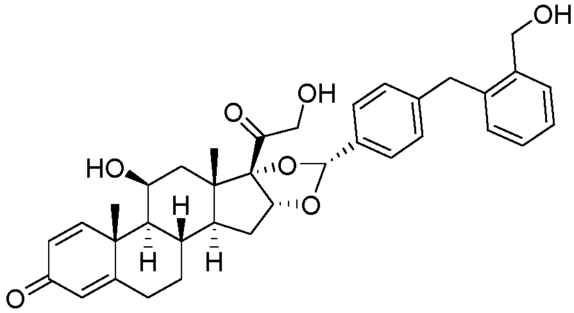 |

(continued)

| No. | Structure |
| --- | --- |
| I-65 | 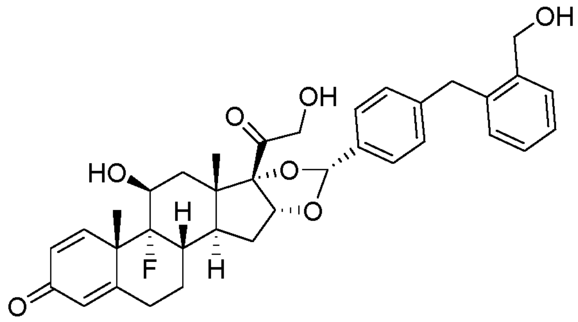 |
| I-66 | 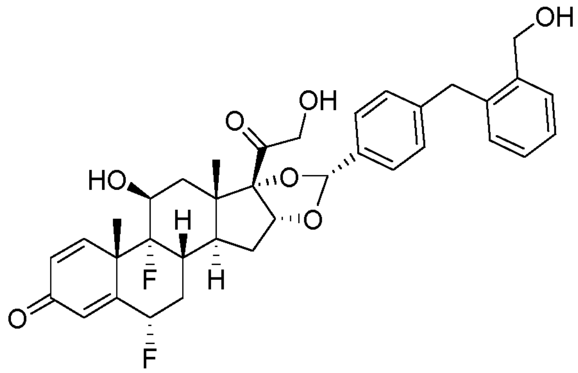 |
| I-67 | 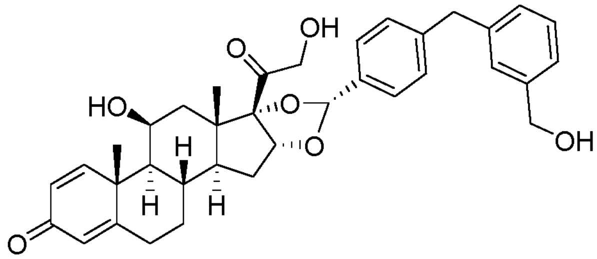 |
| I-68 | 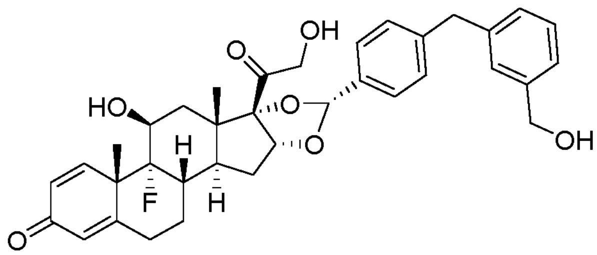 |
| I-69 | 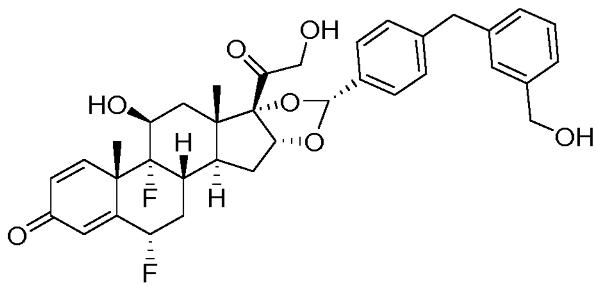 |

(continued)

| No. | Structure |
|---|---|
| I-70 | 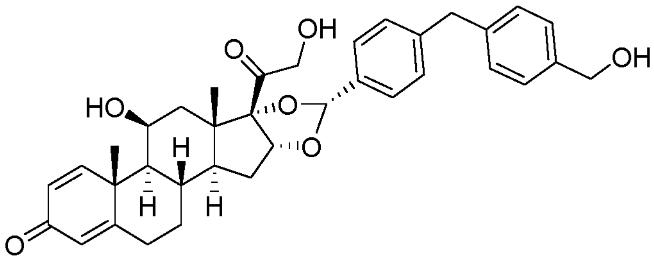 |
| I-71 | 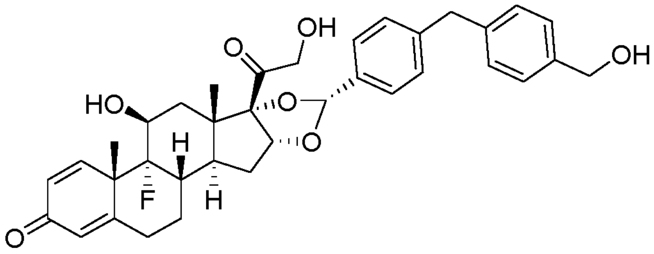 |
| I-72 | 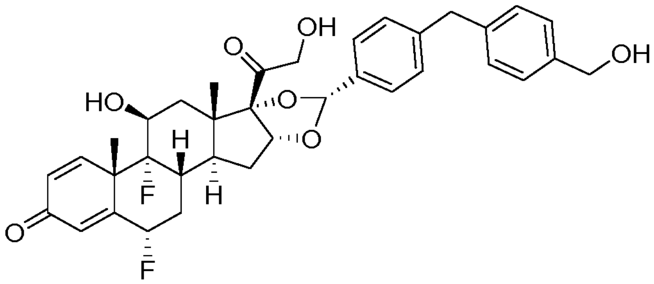 |
| I-73 | 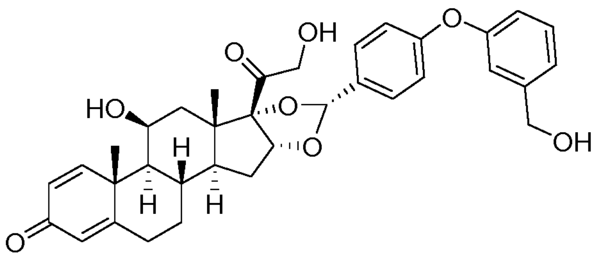 |
| I-74 | 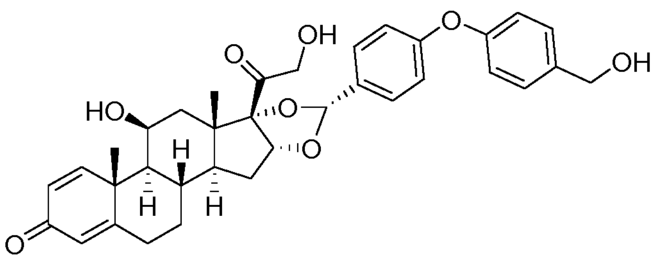 |
| I-75 | 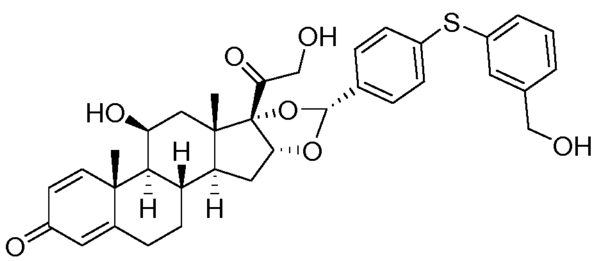 |

(continued)

| No. | Structure |
|---|---|
| I-76 | 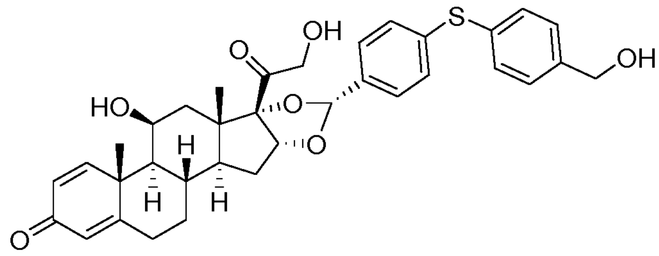 |
| I-77 | 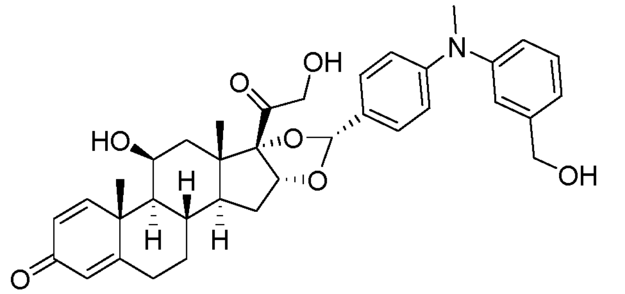 |
| I-78 | 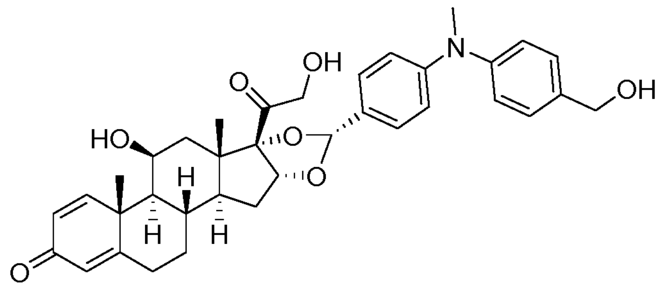 |
| I-79 | 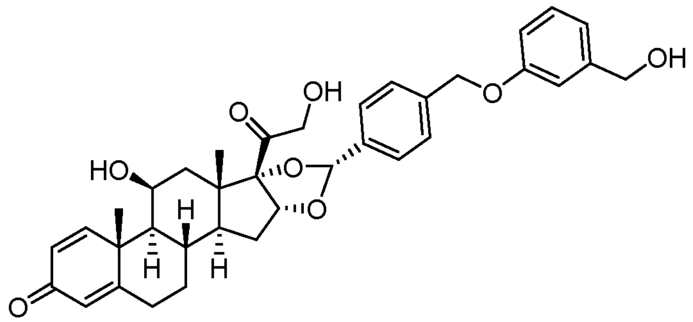 |
| I-80 | 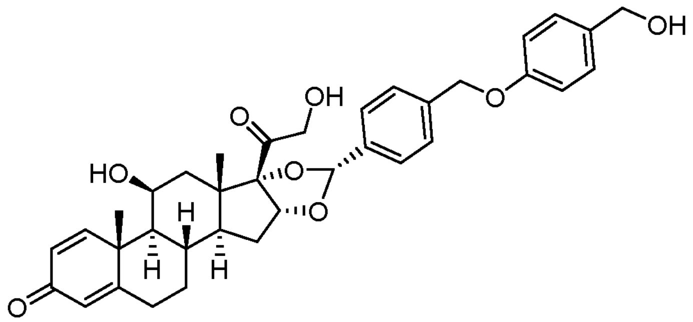 |

(continued)

| No. | Structure |
|---|---|
| I-81 | 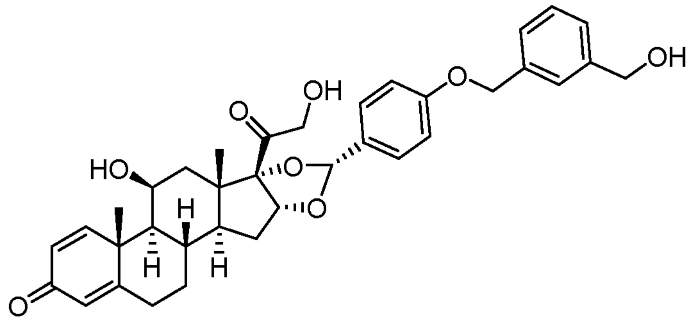 |
| I-82 | 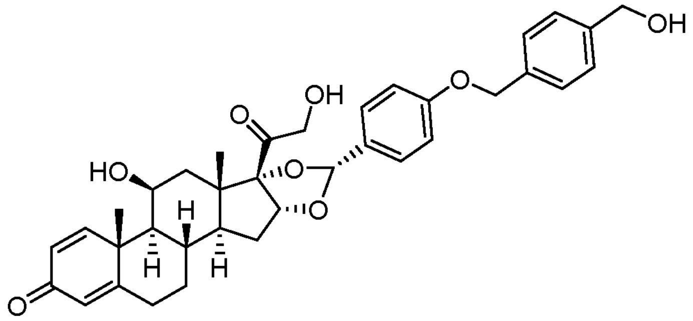 |
| I-83 | 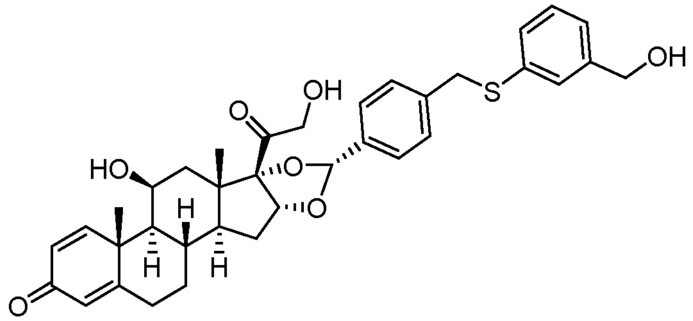 |
| I-84 | 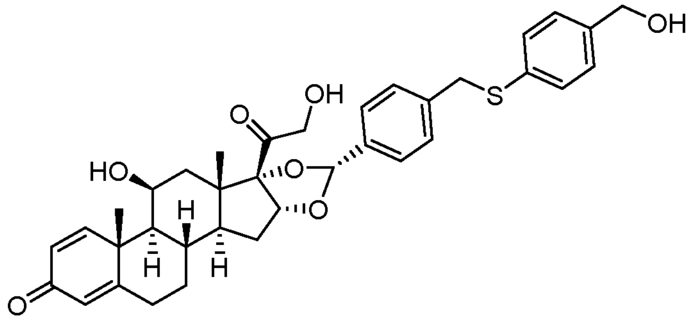 |

(continued)

| No. | Structure |
|---|---|
| I-85 | 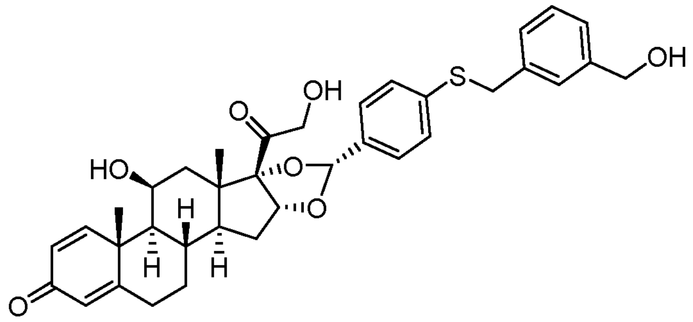 |
| I-86 | 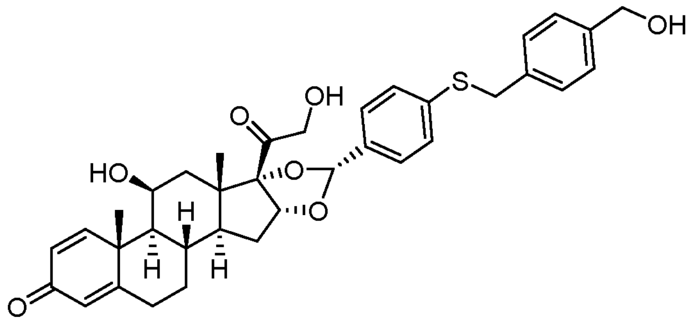 |
| I-87 | 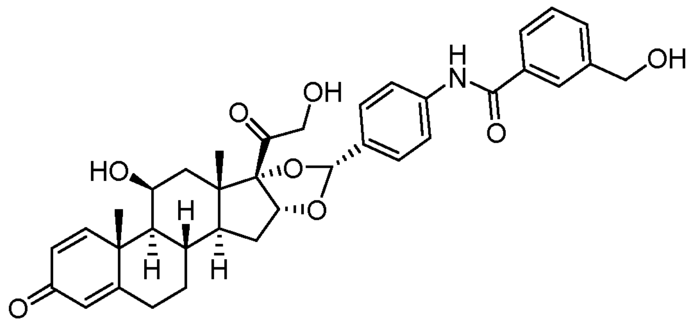 |
| I-88 | 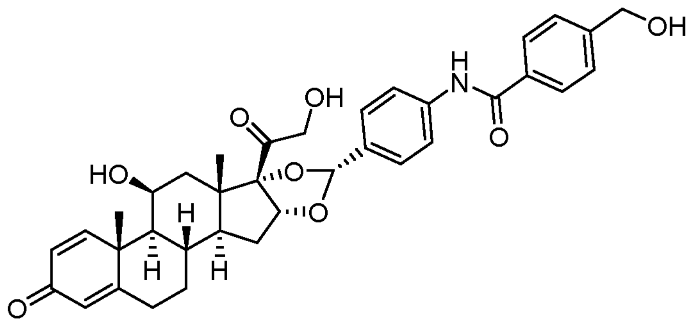 |

(continued)

| No. | Structure |
|---|---|
| I-89 | 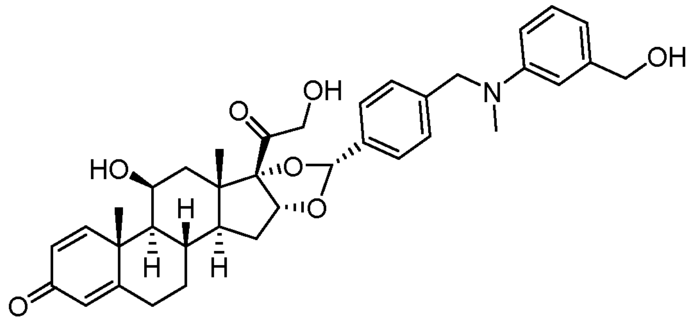 |
| I-90 | 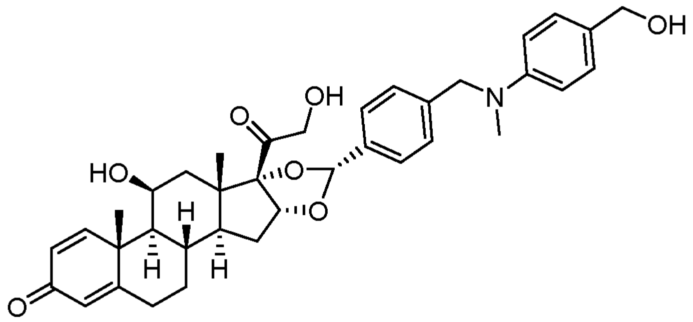 |
| I-91 | 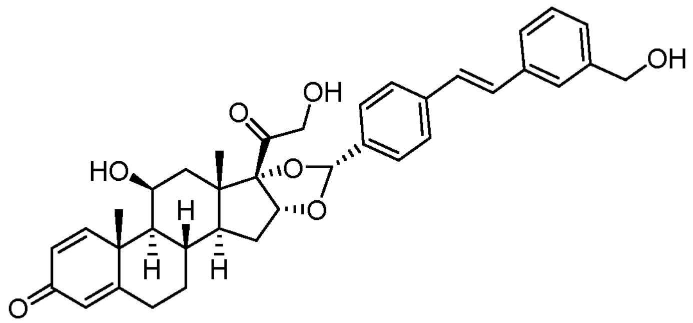 |
| I-92 | 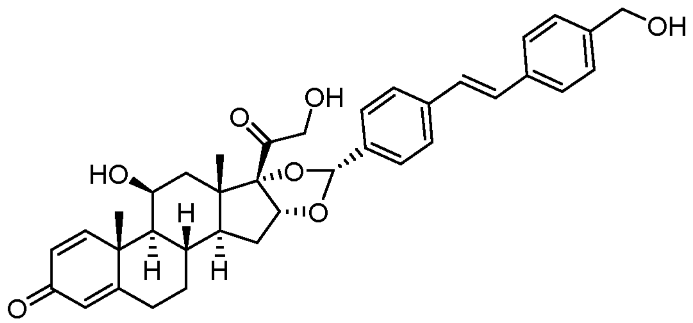 |

(continued)

| No. | Structure |
|---|---|
| I-93 | 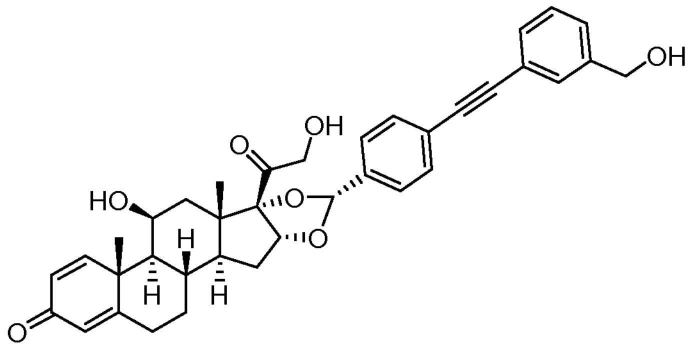 |
| I-94 | 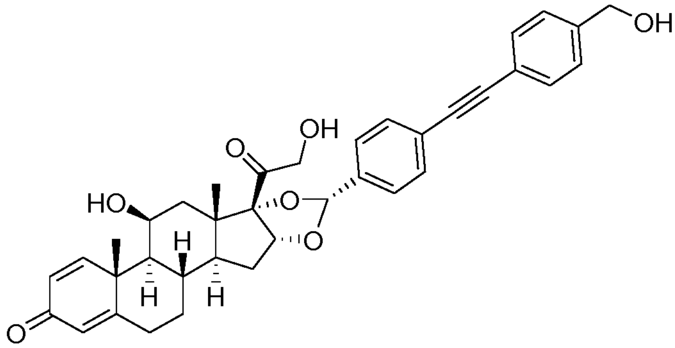 |
| I-95 | 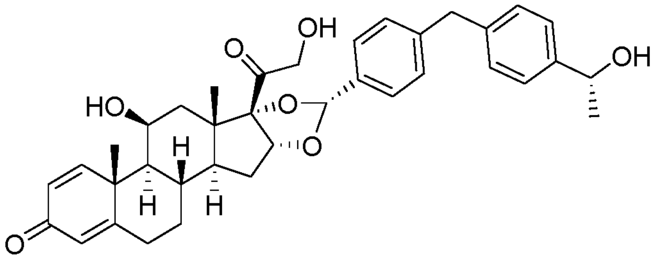 |
| I-96 | 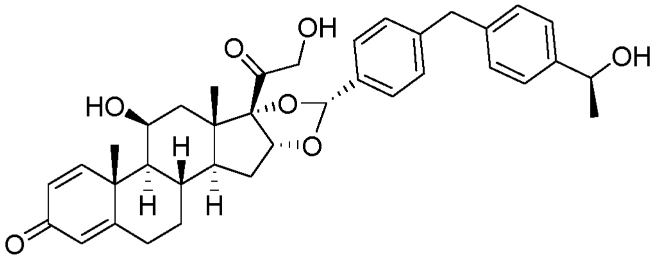 |
| I-97 | 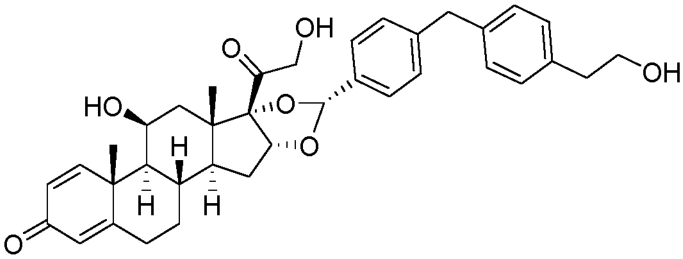 |

(continued)

| No. | Structure |
|---|---|
| I-98 | 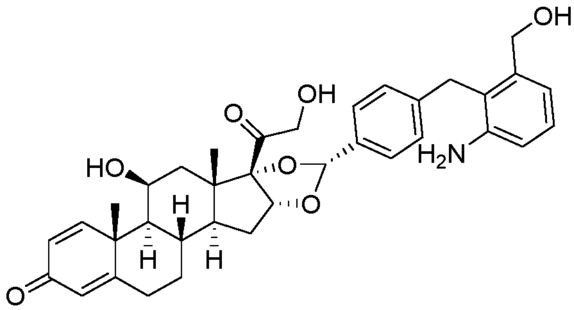 |
| I-99 | 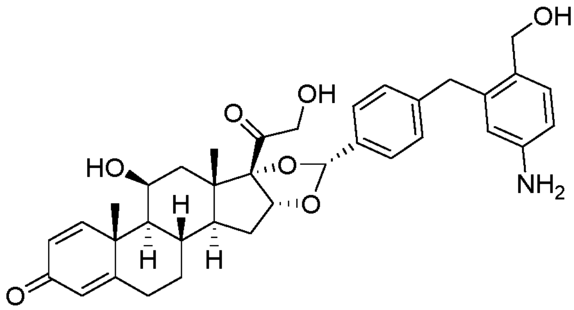 |
| I-100 | 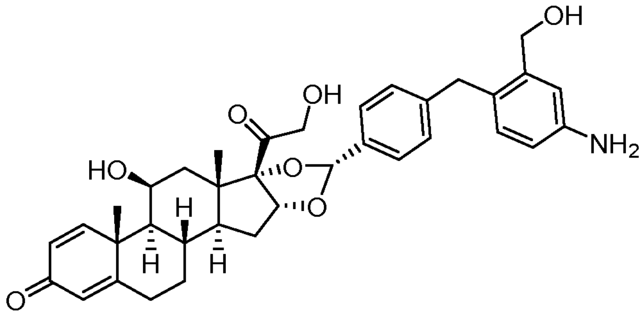 |
| I-101 | 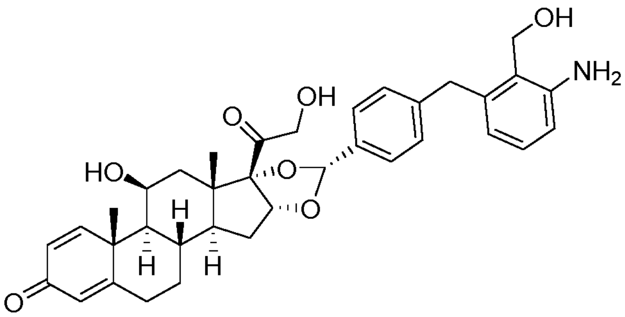 |
| I-102 | 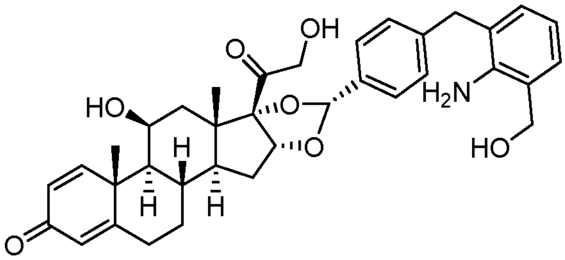 |

(continued)

| No. | Structure |
|---|---|
| I-103 | 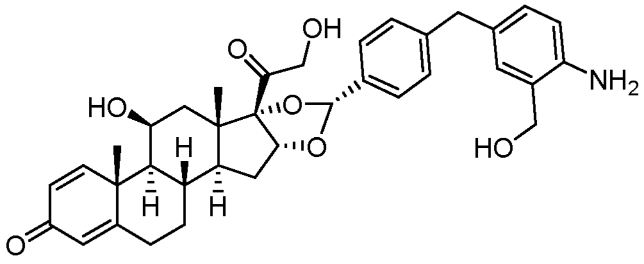 |
| I-104 | 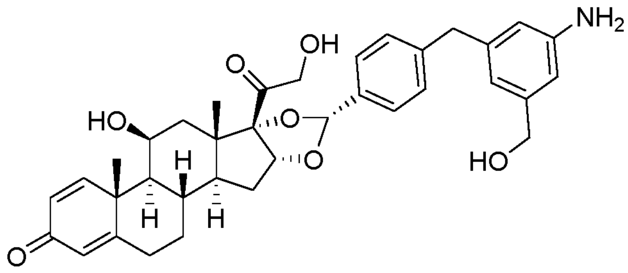 |
| I-105 | 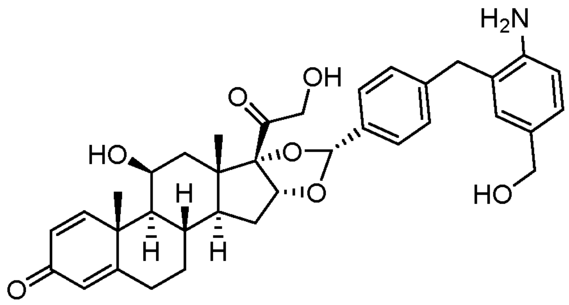 |
| I-106 | 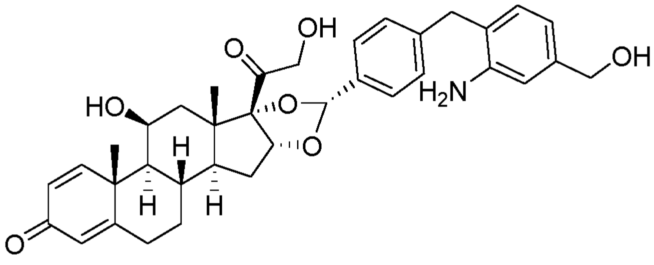 |
| I-107 | 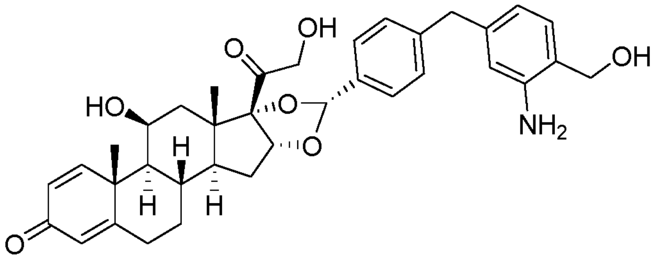 |

(continued)

| No. | Structure |
|---|---|
| I-108 | 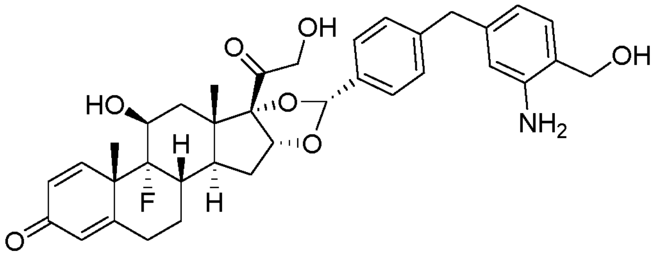 |
| I-109 | 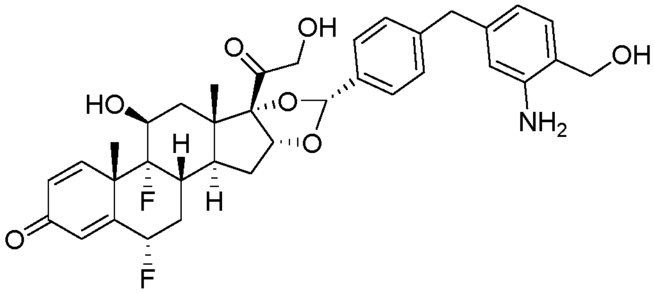 |
| I-110 | 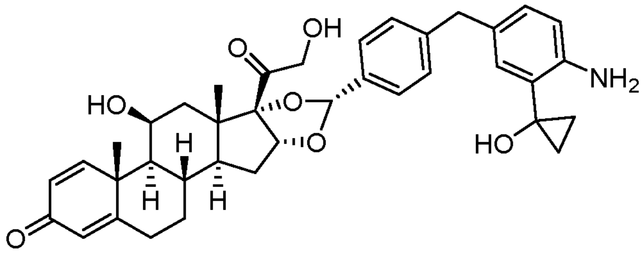 |
| I-111 | 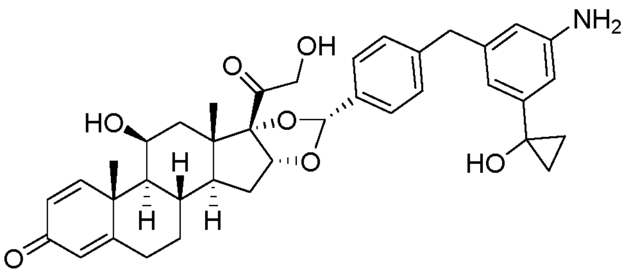 |
| I-112 | 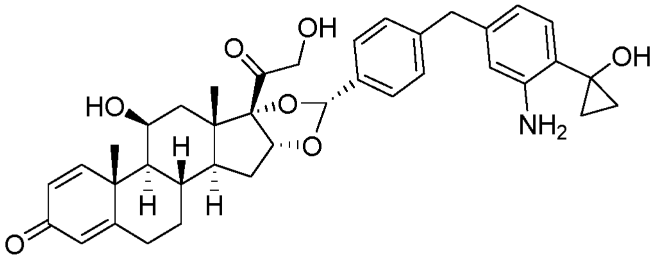 |

(continued)

| No. | Structure |
|---|---|
| I-113 | 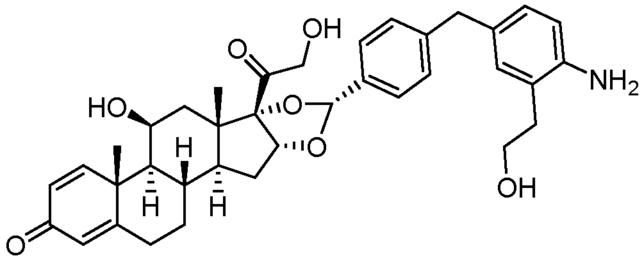 |
| I-114 | 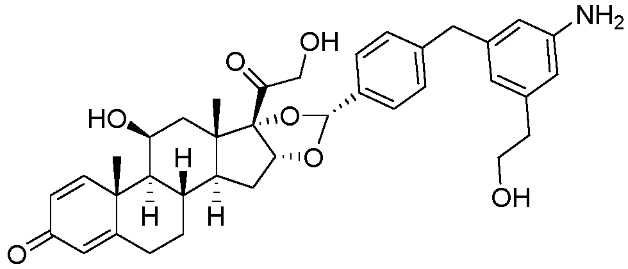 |
| I-115 | 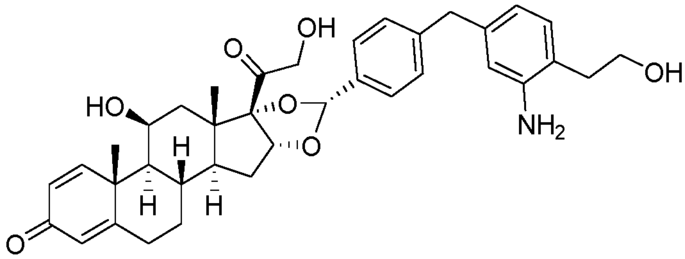 |
| I-116 | 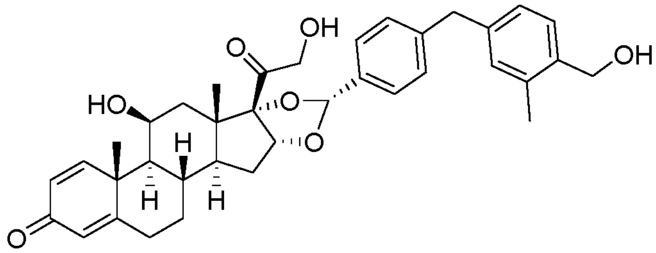 |
| I-117 | 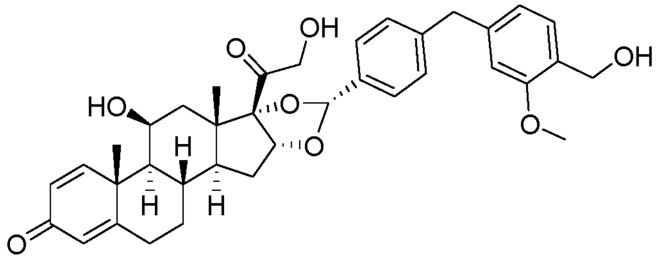 |
| I-118 | 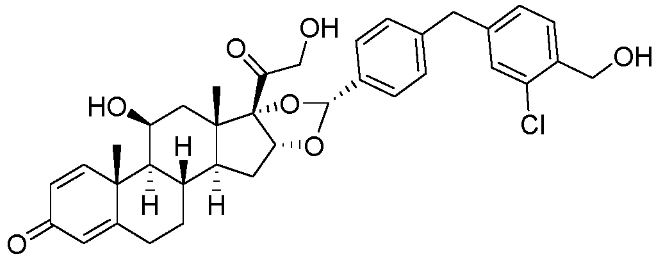 |

(continued)

| No. | Structure |
|---|---|
| I-119 | 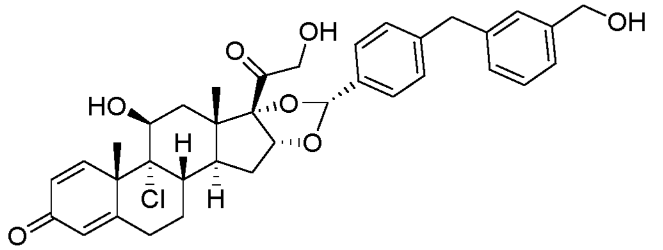 |
| I-120 | 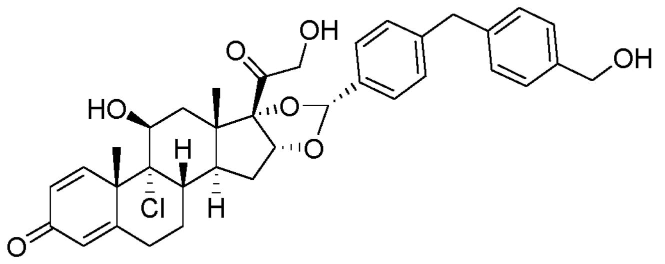 |
| I-121 | 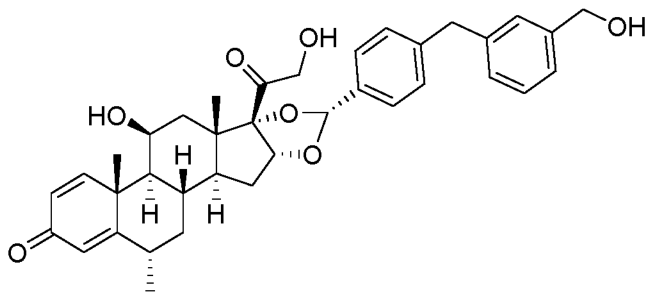 |
| I-122 | 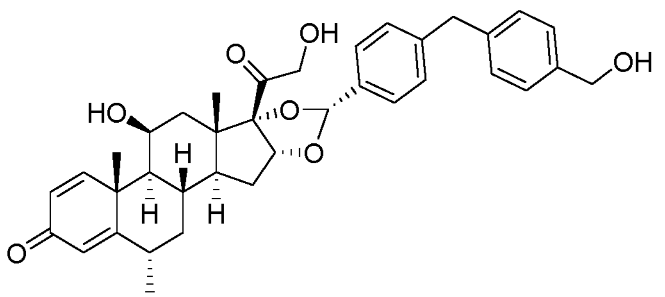 |
| I-123 | 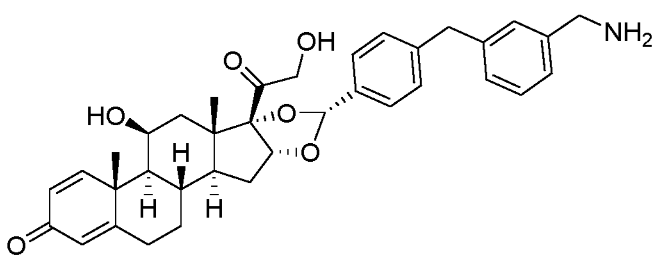 |

(continued)

| No. | Structure |
|---|---|
| I-124 | 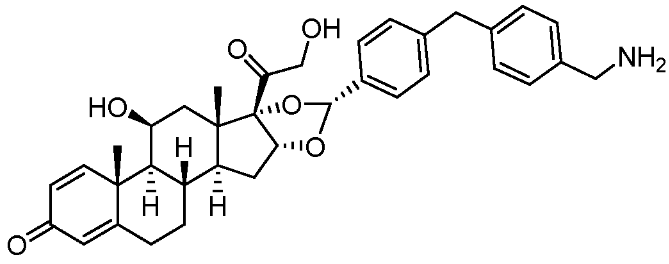 |
| I-125 | 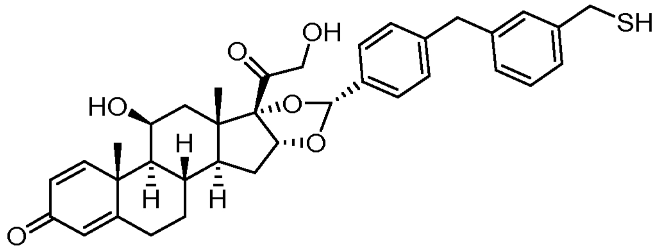 |
| I-126 | 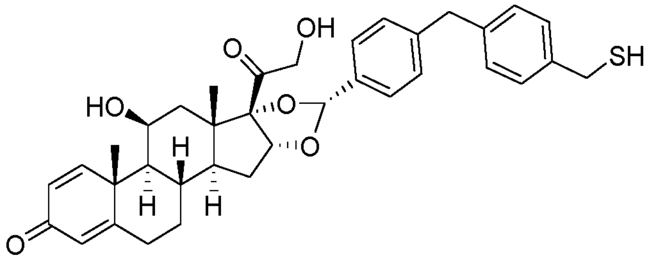 |
| I-127 | 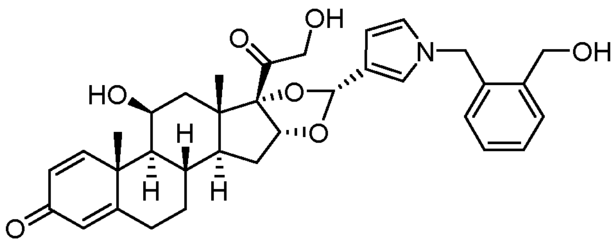 |
| I-128 | 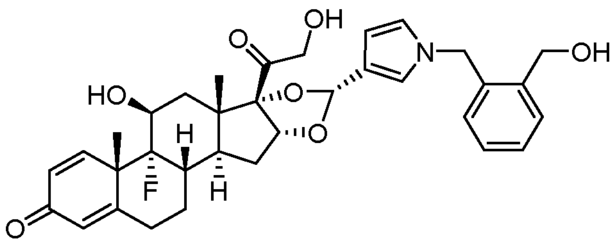 |
| I-129 | 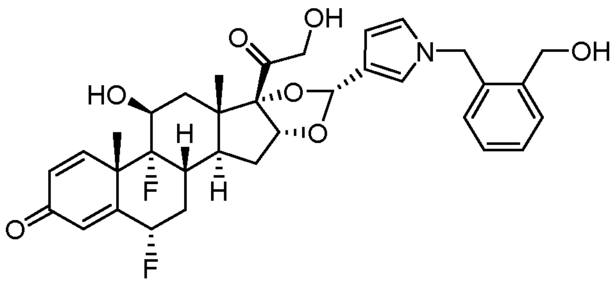 |

(continued)

| No. | Structure |
|---|---|
| I-130 | 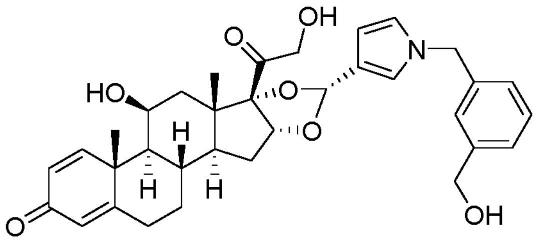 |
| I-131 | 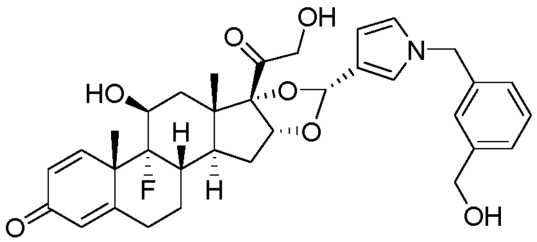 |
| I-132 | 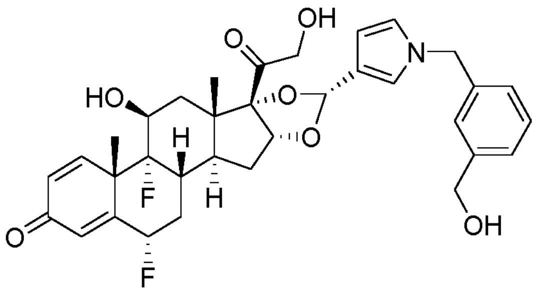 |
| I-133 | 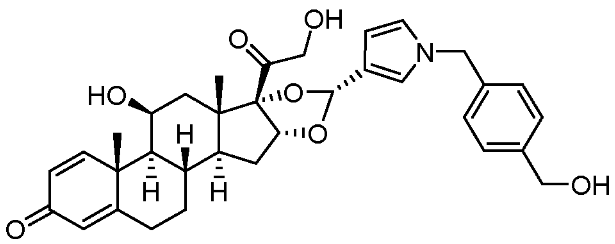 |
| I-134 | 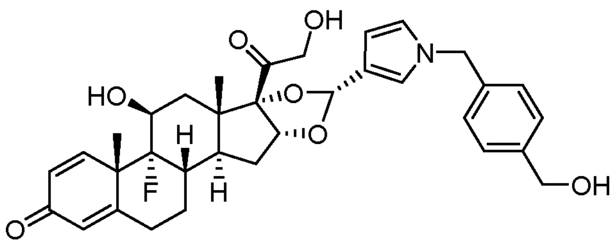 |
| I-135 | 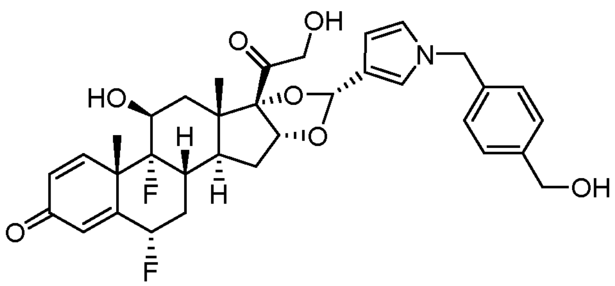 |

(continued)

| No. | Structure |
|---|---|
| I-136 | 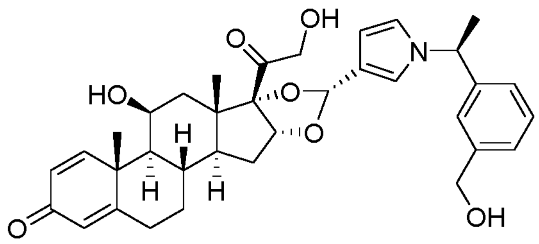 |
| I-137 | 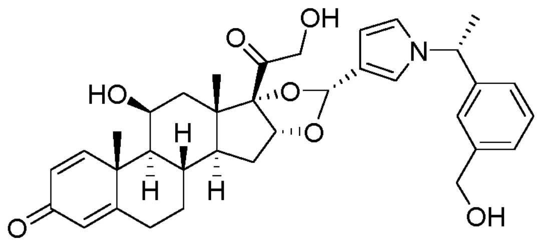 |
| I-138 | 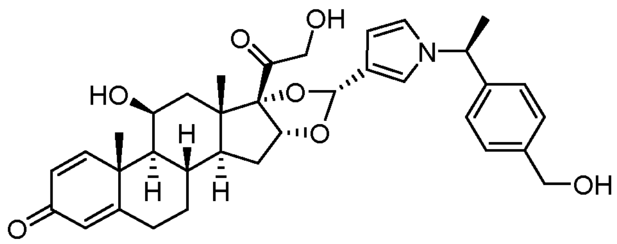 |
| I-139 | 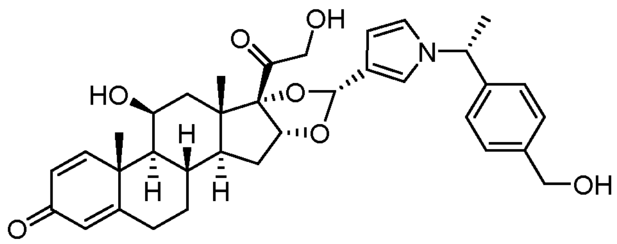 |
| I-140 | 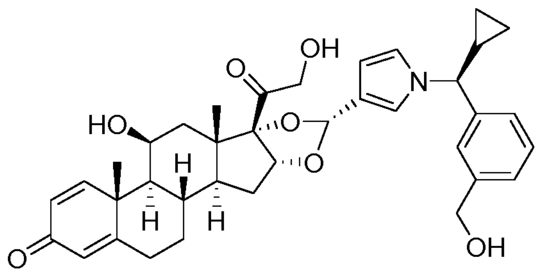 |
| I-141 | 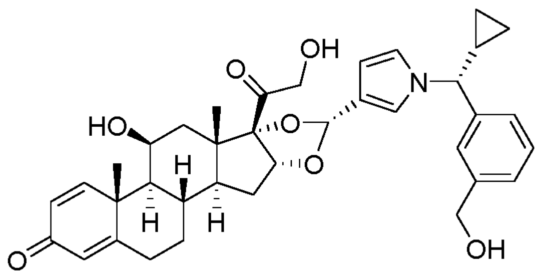 |

(continued)

| No. | Structure |
|---|---|
| I-142 | 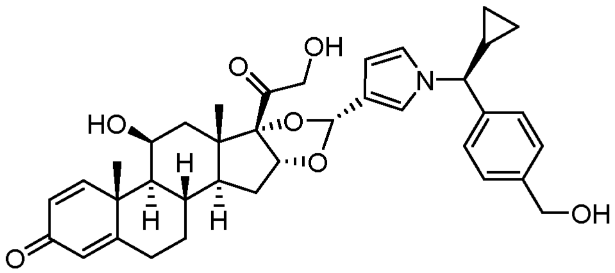 |
| I-143 | 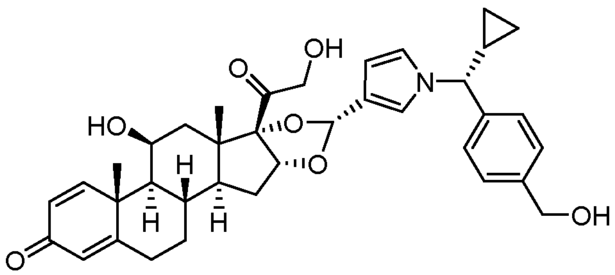 |
| I-144 | 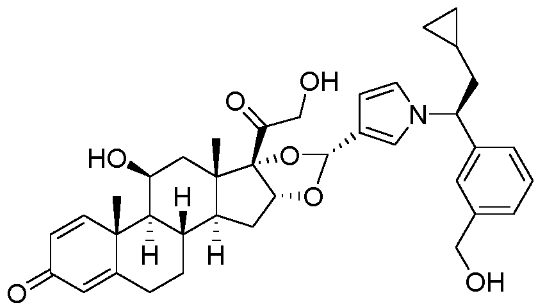 |
| I-145 | 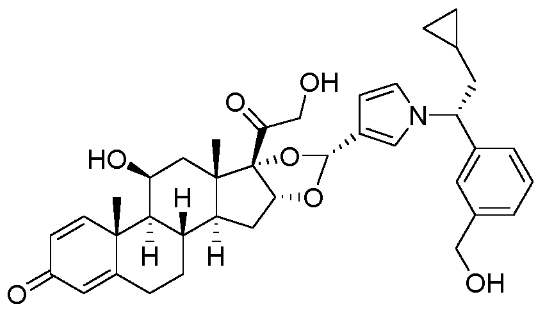 |
| I-146 | 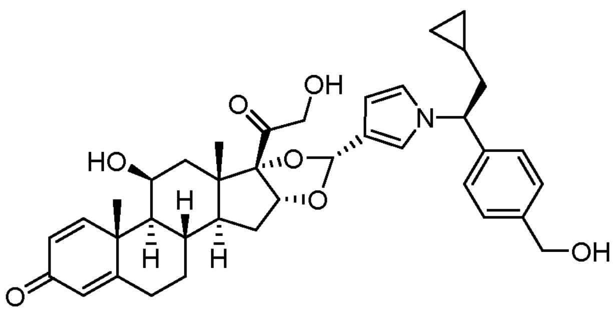 |

(continued)

| No. | Structure |
|---|---|
| I-147 | 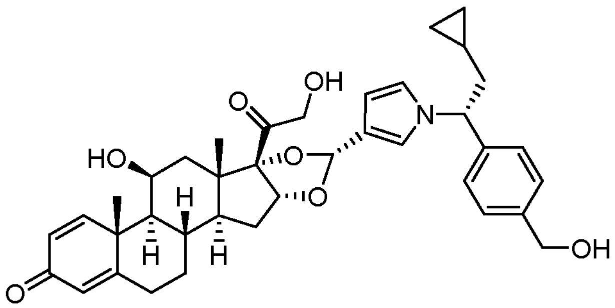 |
| I-148 | 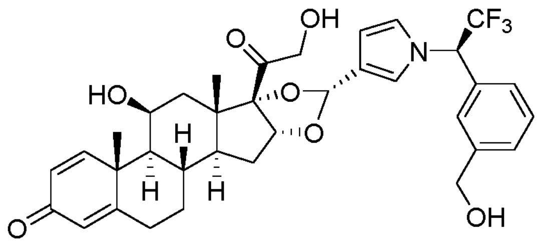 |
| I-149 | 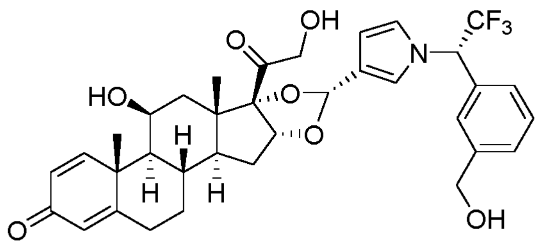 |
| I-150 | 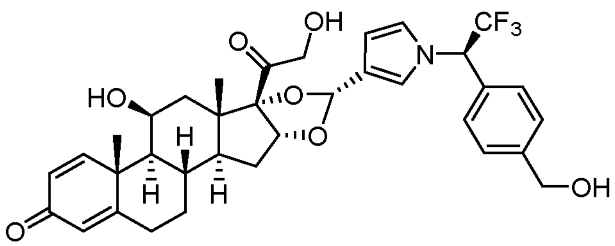 |
| I-151 | 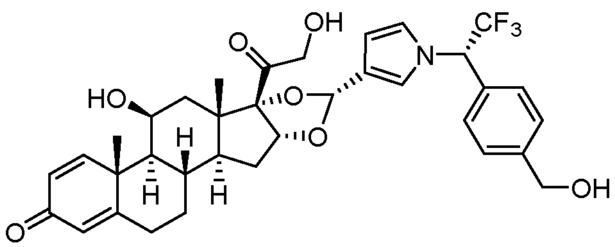 |
| I-152 | 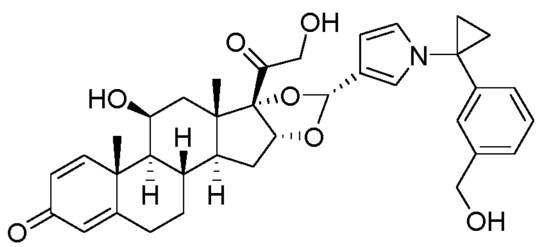 |

(continued)

| No. | Structure |
|---|---|
| I-153 | 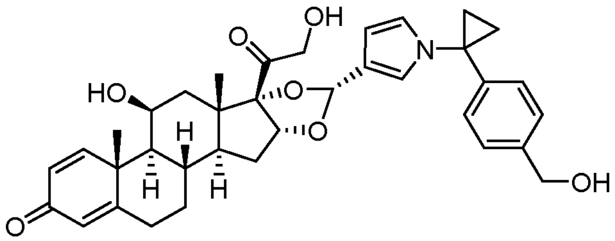 |
| I-154 | 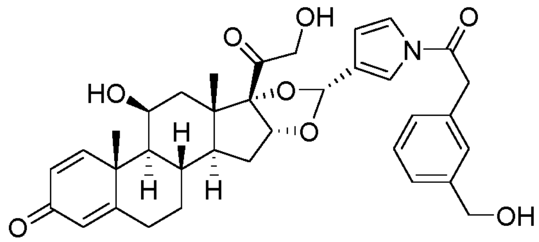 |
| I-155 | 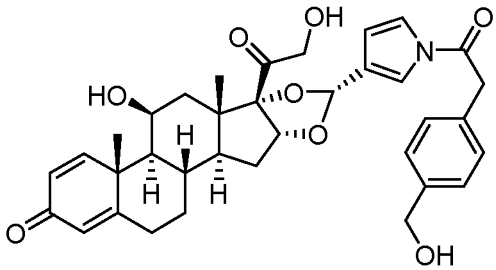 |
| I-156 | 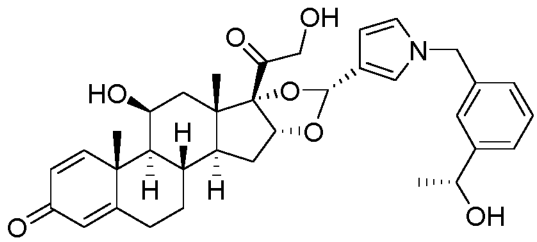 |
| I-157 | 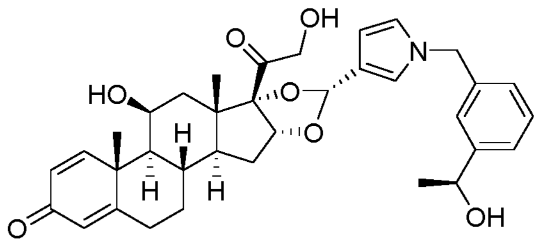 |
| I-158 | 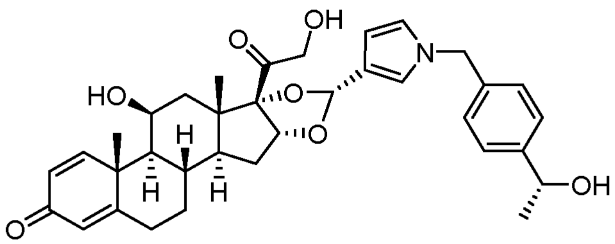 |

(continued)

| No. | Structure |
|---|---|
| I-159 | 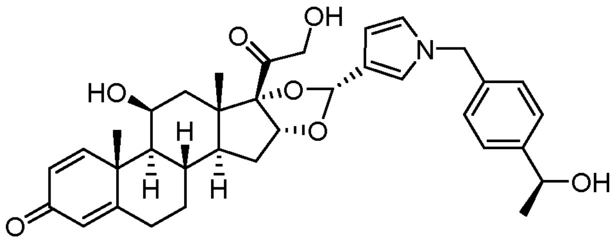 |
| I-160 | 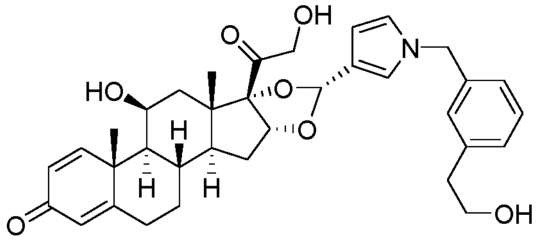 |
| I-161 | 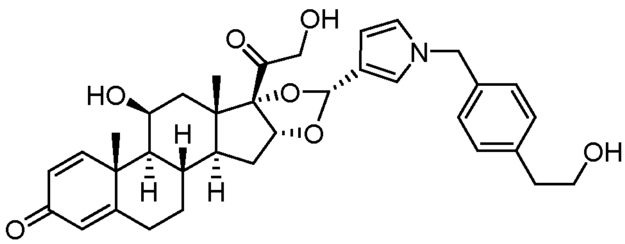 |
| I-162 | 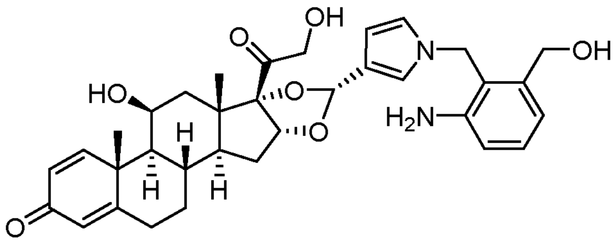 |
| I-163 | 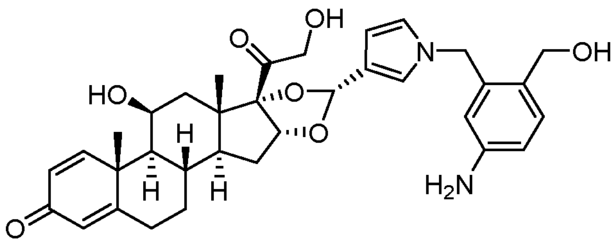 |
| I-164 | 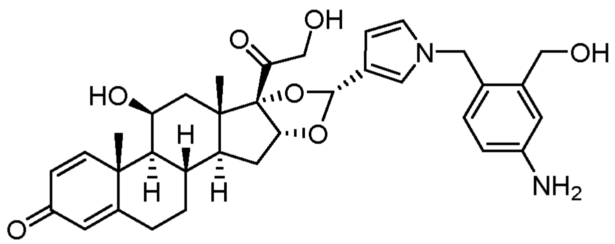 |

(continued)

| No. | Structure |
|---|---|
| I-165 | 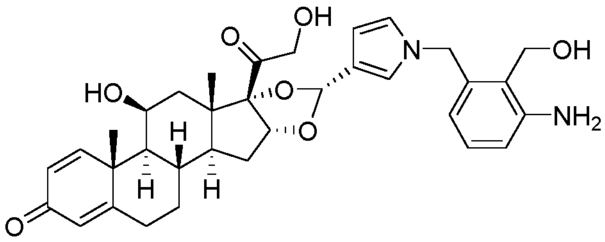 |
| I-166 | 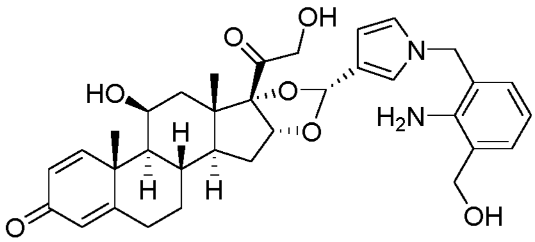 |
| I-167 | 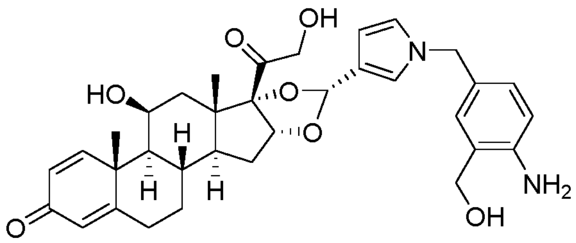 |
| I-168 | 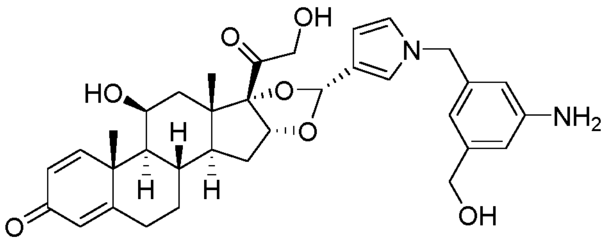 |
| I-169 | 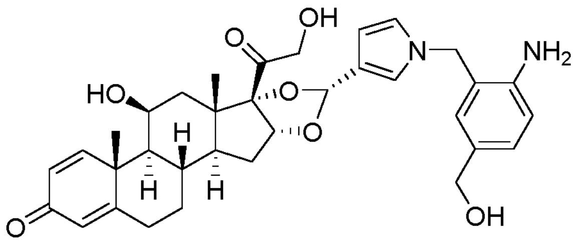 |
| I-170 | 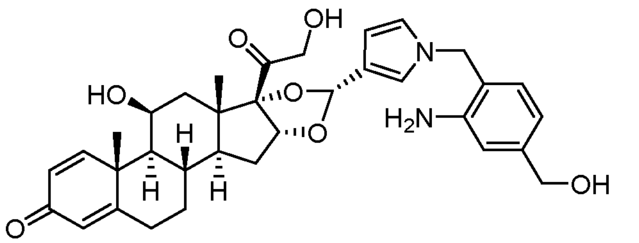 |

(continued)

| No. | Structure |
|---|---|
| I-171 | 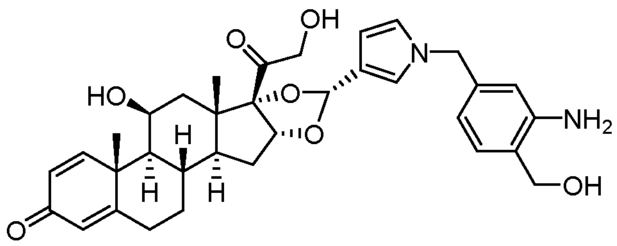 |
| I-172 | 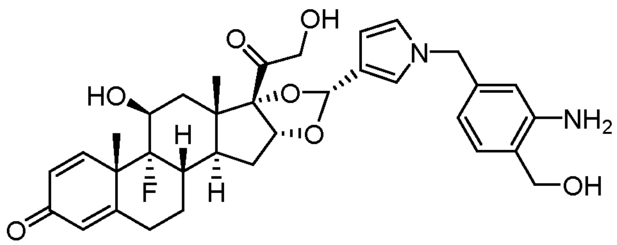 |
| I-173 | 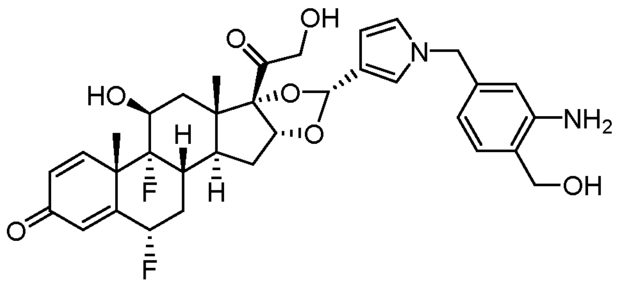 |
| I-174 | 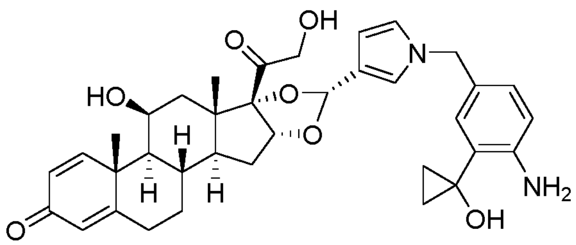 |
| I-175 | 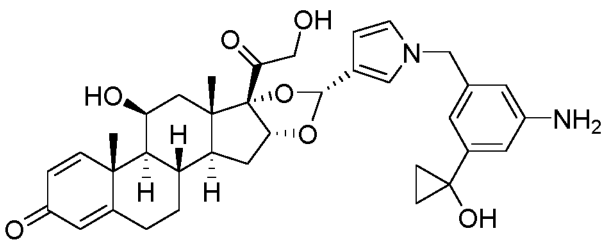 |
| I-176 | 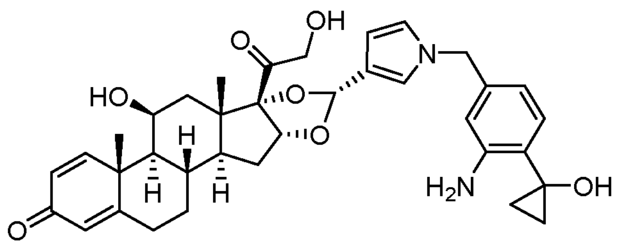 |

(continued)

| No. | Structure |
|---|---|
| I-177 | 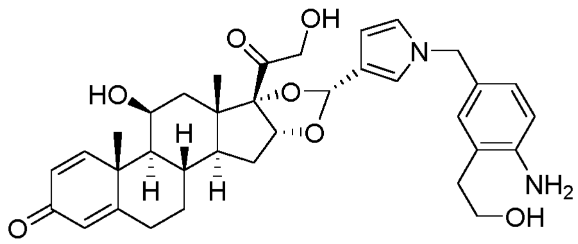 |
| I-178 | 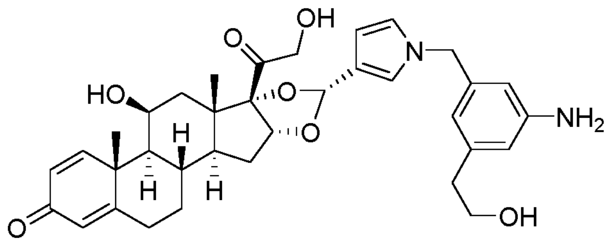 |
| I-179 | 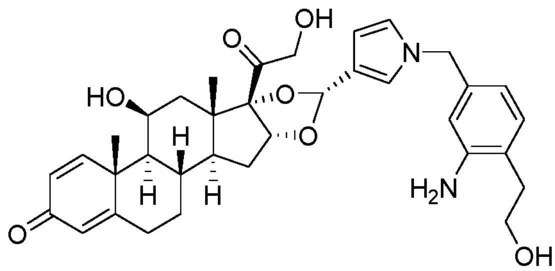 |
| I-180 | 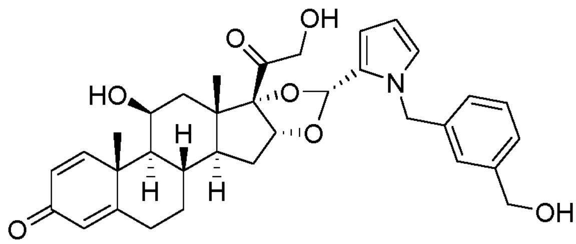 |
| I-181 | 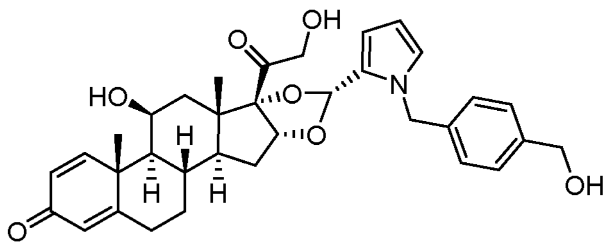 |
| I-182 | 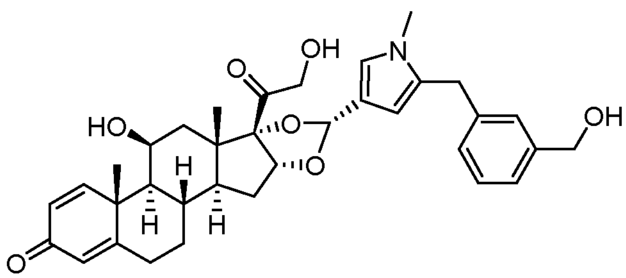 |

(continued)

| No. | Structure |
|---|---|
| I-183 | 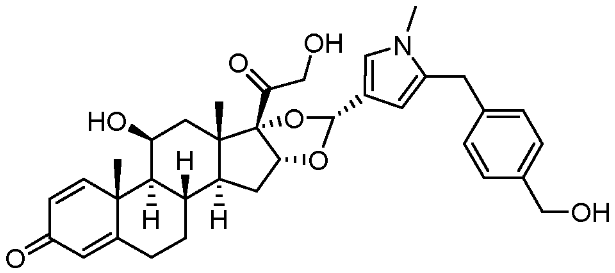 |
| I-184 | 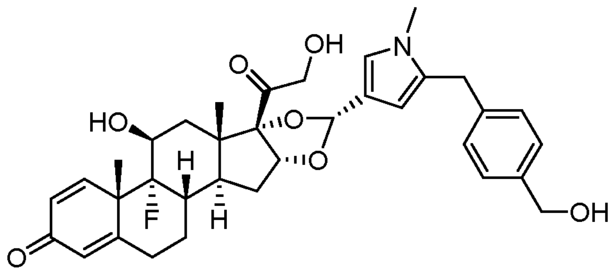 |
| I-185 | 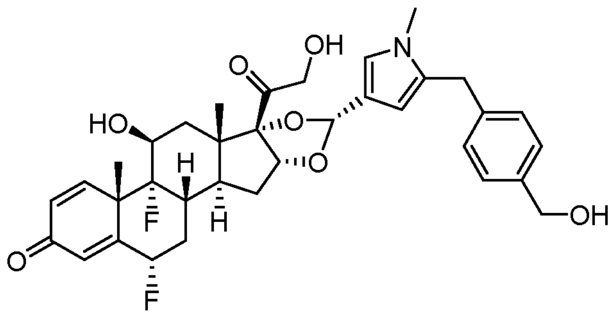 |
| I-186 | 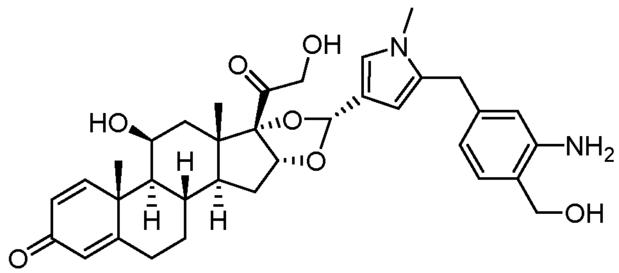 |
| I-187 | 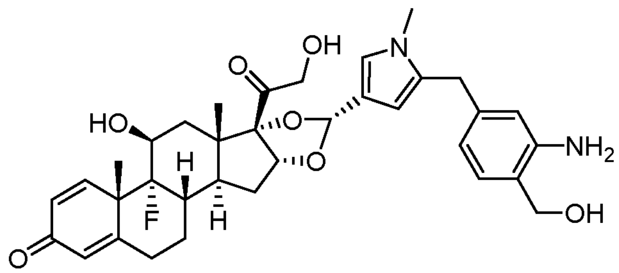 |

(continued)

| No. | Structure |
|---|---|
| I-188 | 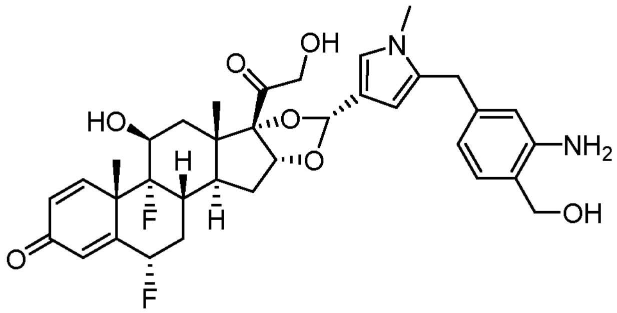 |
| I-189 | 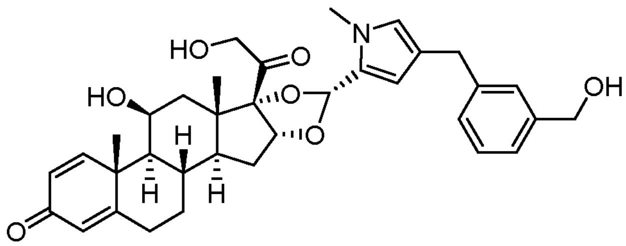 |
| I-190 | 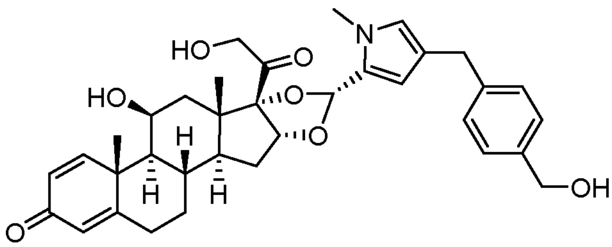 |
| I-191 | 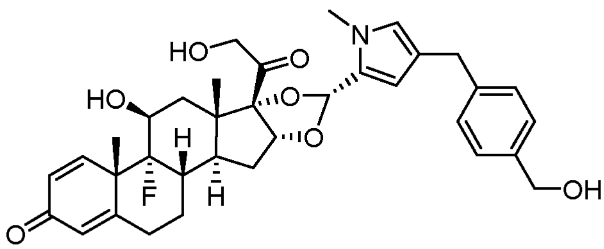 |
| I-192 | 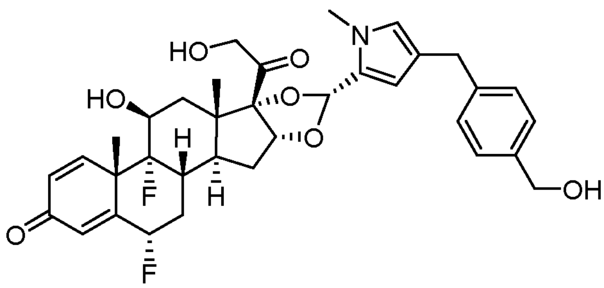 |
| I-193 | 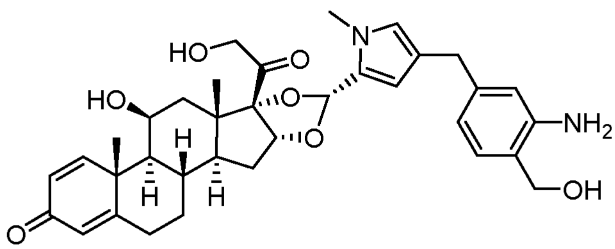 |

(continued)

| No. | Structure |
|---|---|
| I-194 | 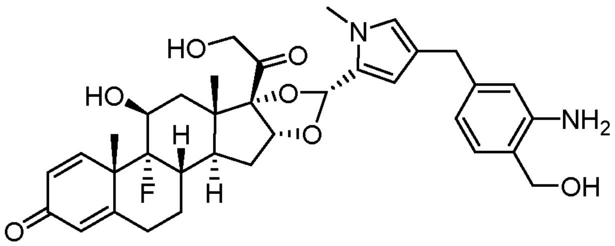 |
| I-195 | 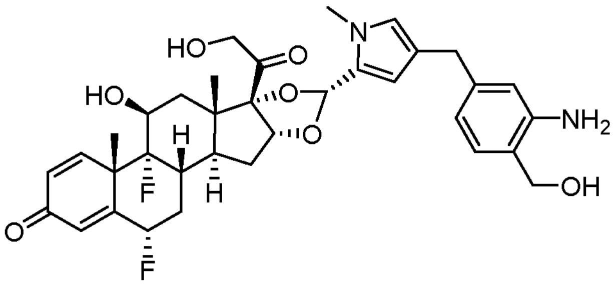 |
| I-196 | 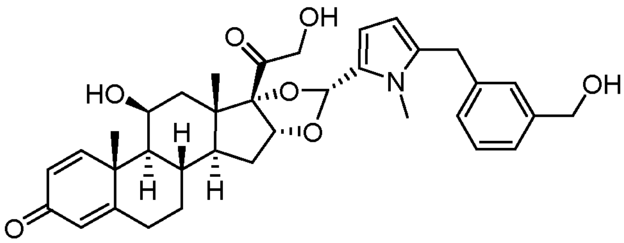 |
| I-197 | 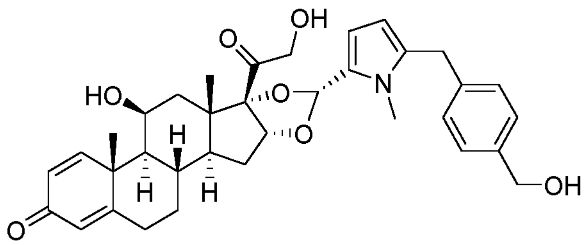 |
| I-198 | 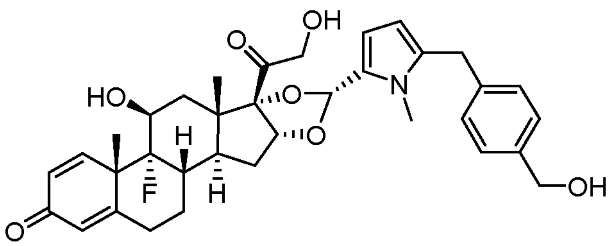 |
| I-199 | 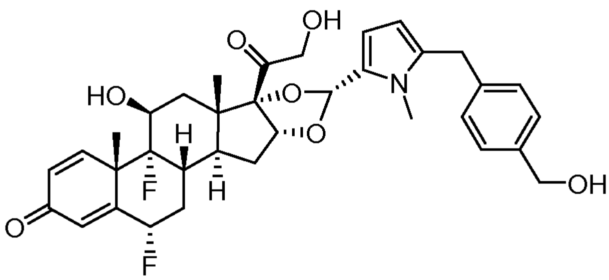 |

(continued)

| No. | Structure |
|---|---|
| I-200 | 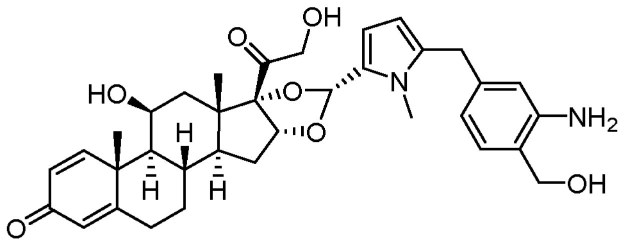 |
| I-201 | 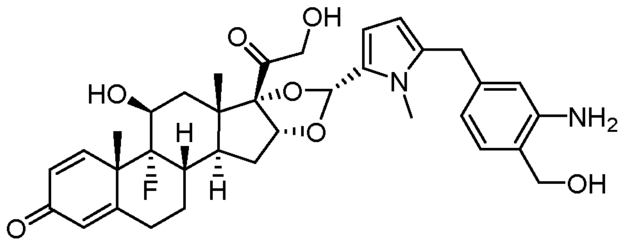 |
| I-202 | 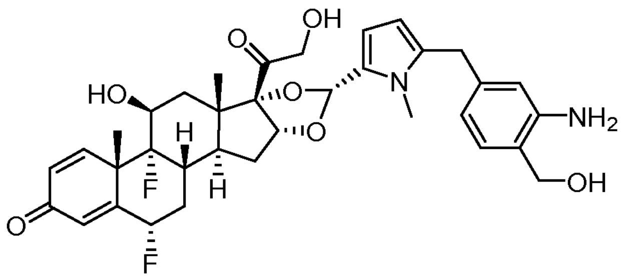 |
| I-203 | 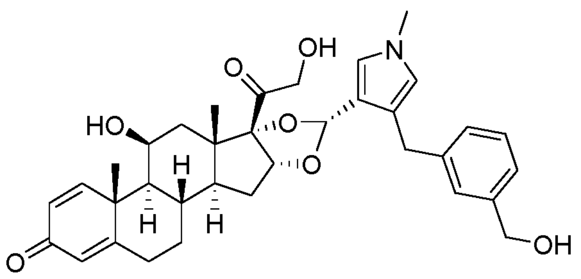 |
| I-204 | 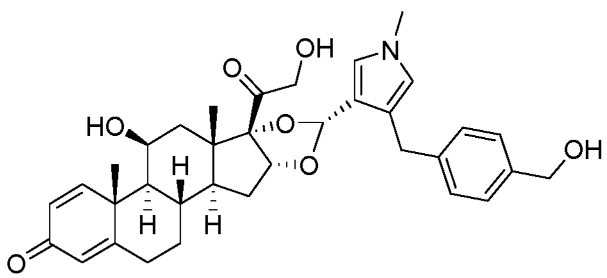 |
| I-205 | 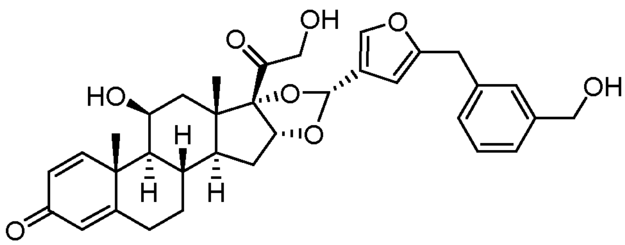 |

(continued)

| No. | Structure |
|---|---|
| I-206 | 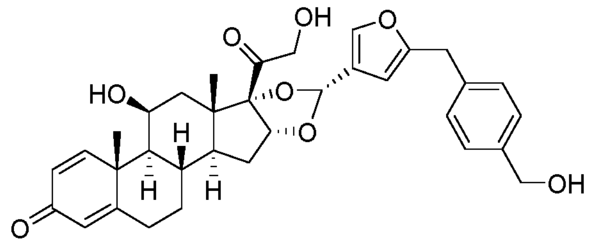 |
| I-207 | 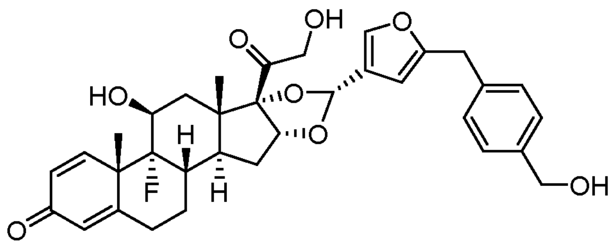 |
| I-208 | 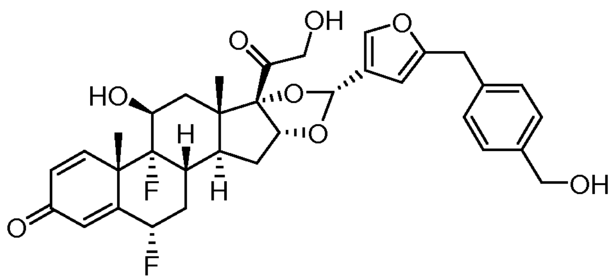 |
| I-209 | 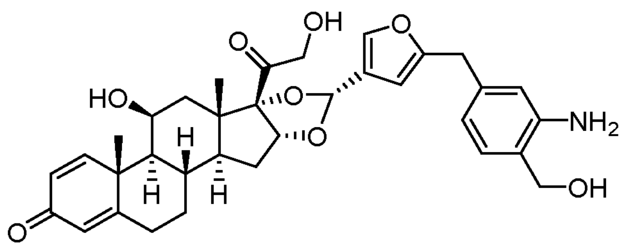 |
| I-210 | 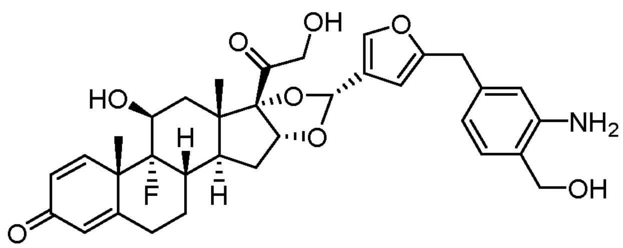 |
| I-211 | 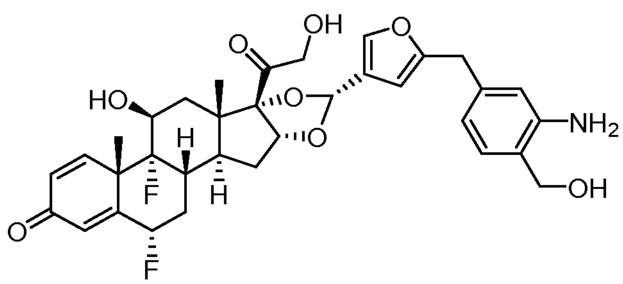 |

(continued)

| No. | Structure |
|---|---|
| I-212 | 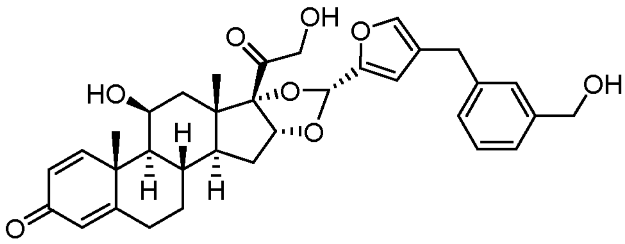 |
| I-213 | 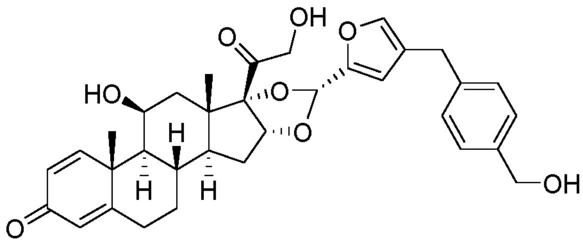 |
| I-214 | 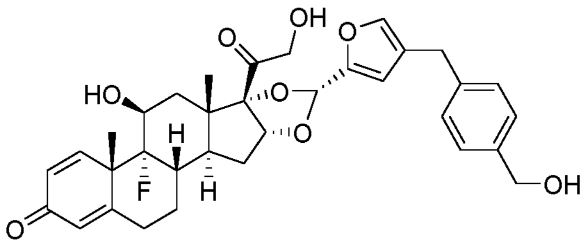 |
| I-215 | 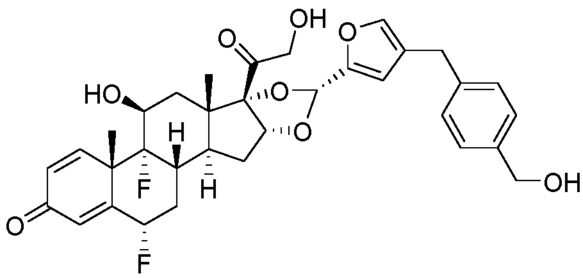 |
| I-216 | 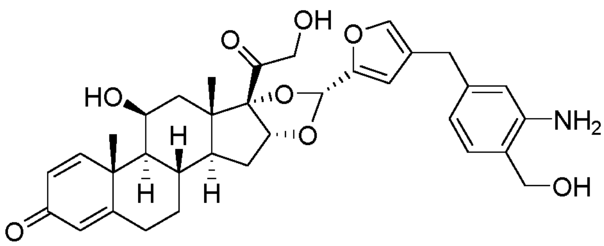 |
| I-217 | 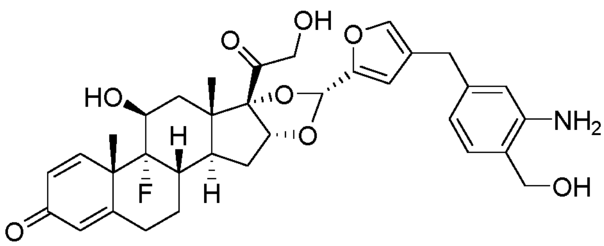 |

(continued)

| No. | Structure |
|---|---|
| I-218 | 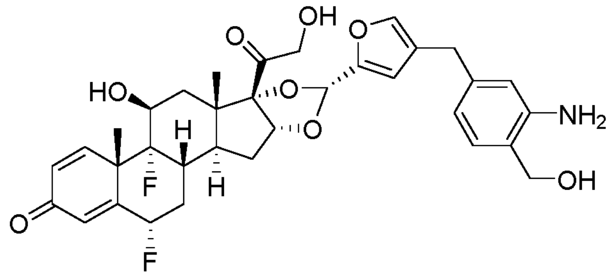 |
| I-219 | 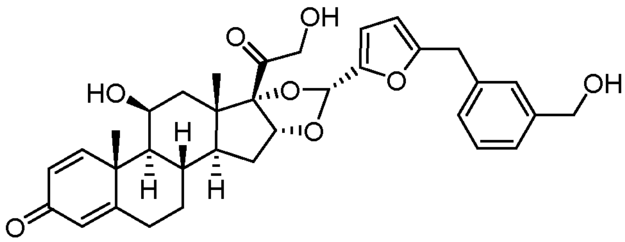 |
| I-220 | 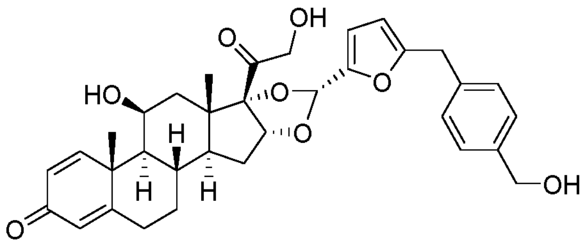 |
| I-221 | 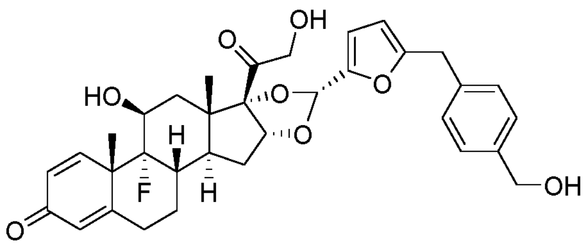 |
| I-222 | 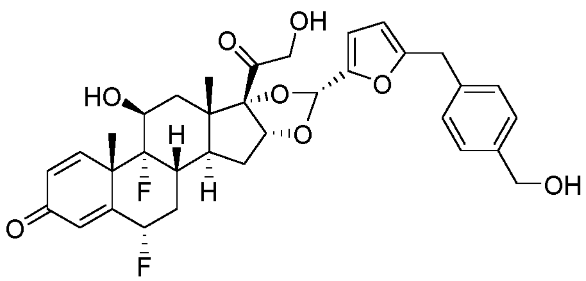 |
| I-223 | 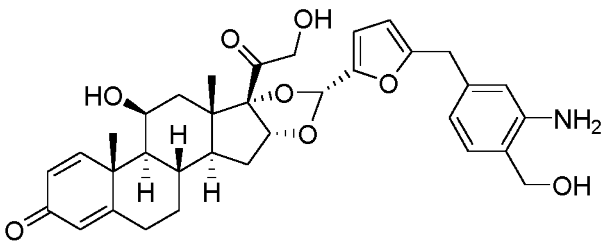 |

(continued)

| No. | Structure |
|---|---|
| I-224 | 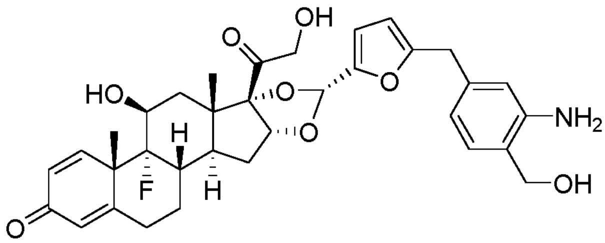 |
| I-225 | 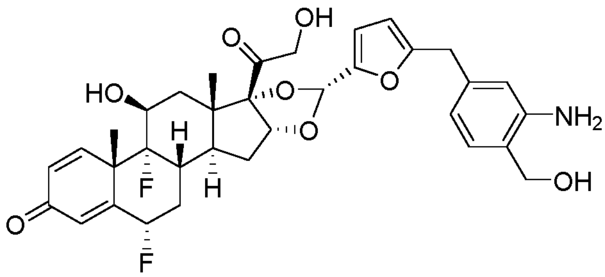 |
| I-226 | 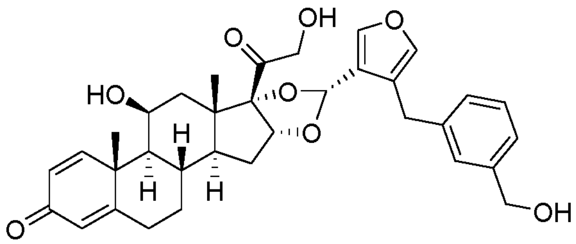 |
| I-227 | 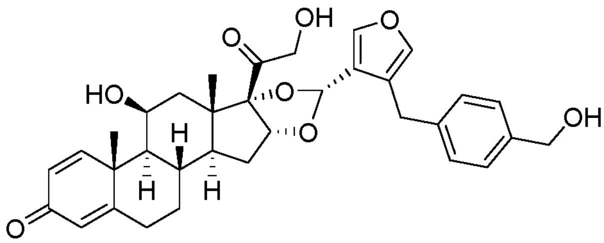 |
| I-228 | 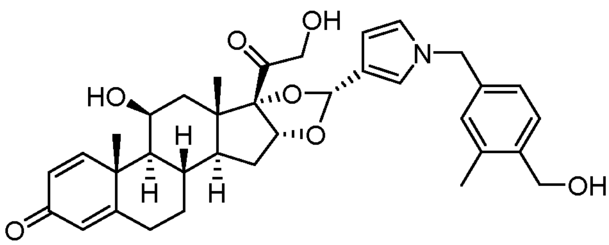 |
| I-229 | 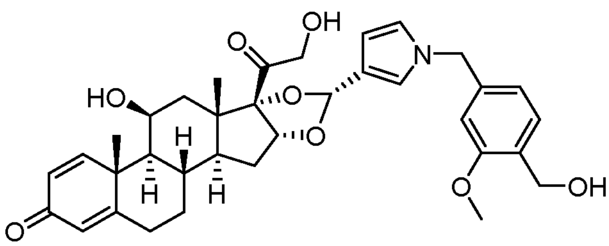 |

(continued)

| No. | Structure |
|---|---|
| I-230 | 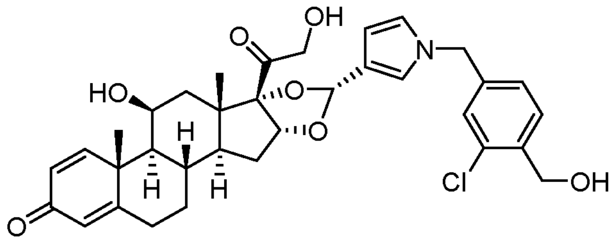 |
| I-231 | 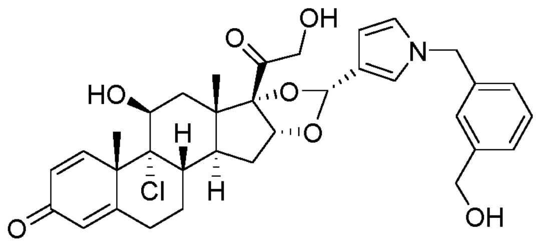 |
| I-232 | 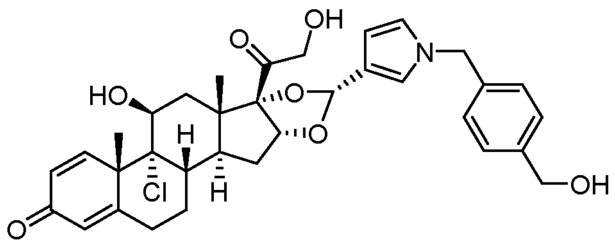 |
| I-233 | 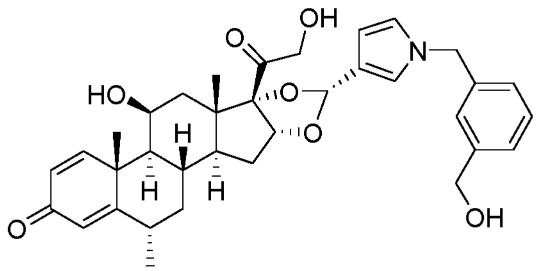 |
| I-234 | 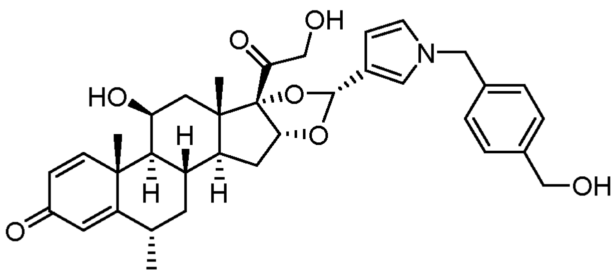 |
| I-235 | 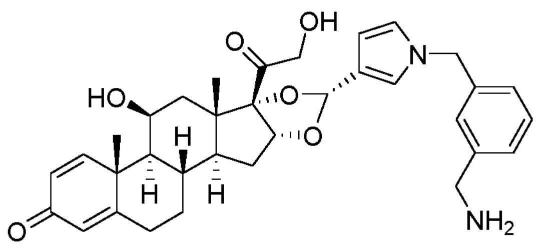 |

(continued)

| No. | Structure |
|---|---|
| I-236 | 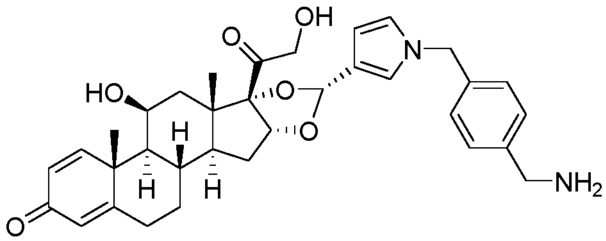 |
| I-237 | 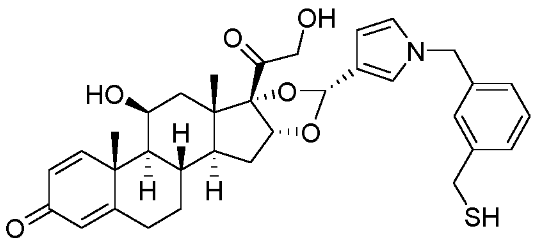 |
| I-238 | 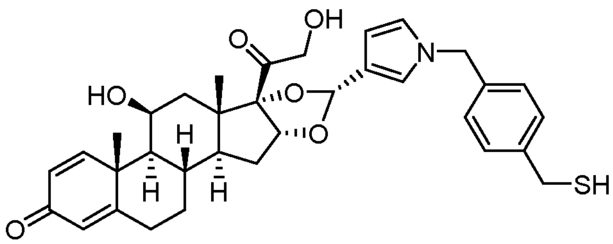 |
| I-239 | 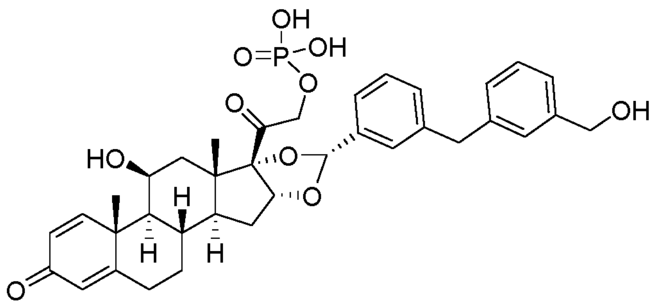 |
| I-240 | 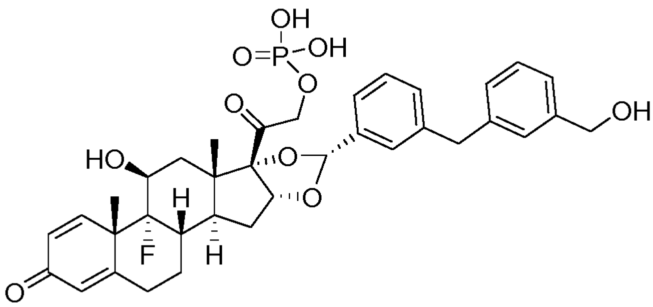 |

(continued)

| No. | Structure |
|---|---|
| I-241 | 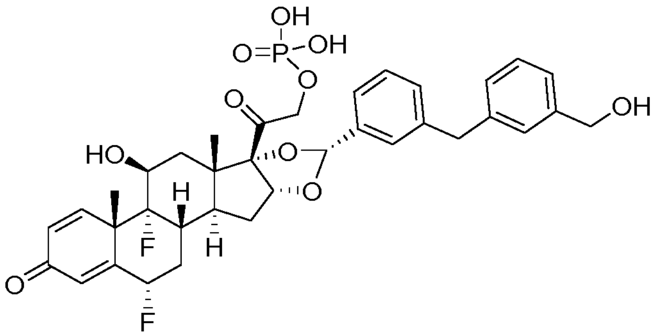 |
| I-242 | 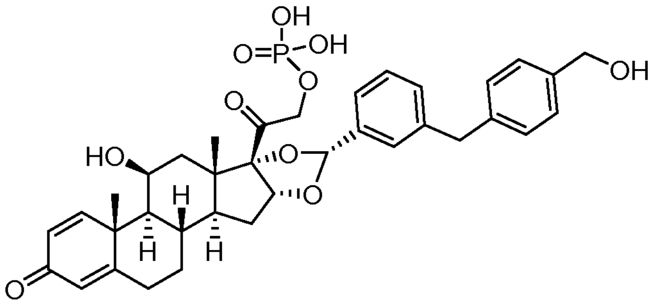 |
| I-243 | 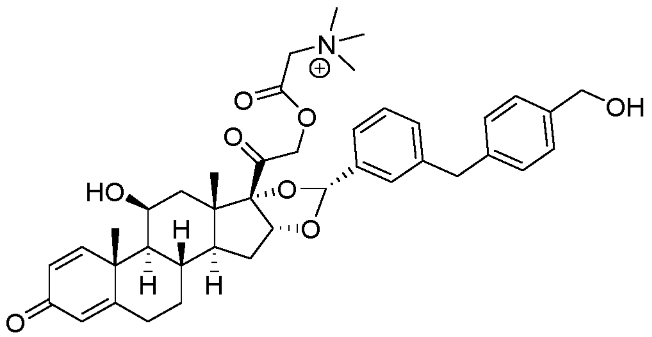 |
| I-244 | 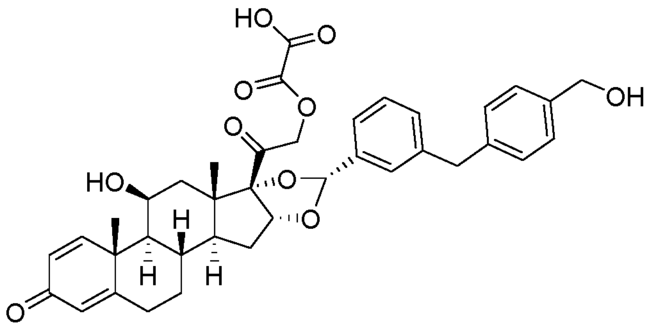 |

(continued)

| No. | Structure |
|---|---|
| I-245 | 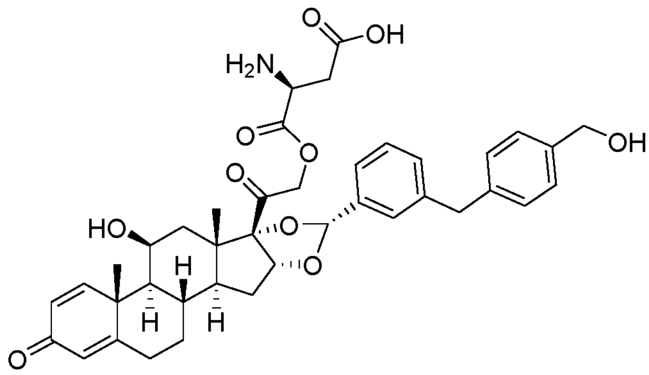 |
| I-246 | 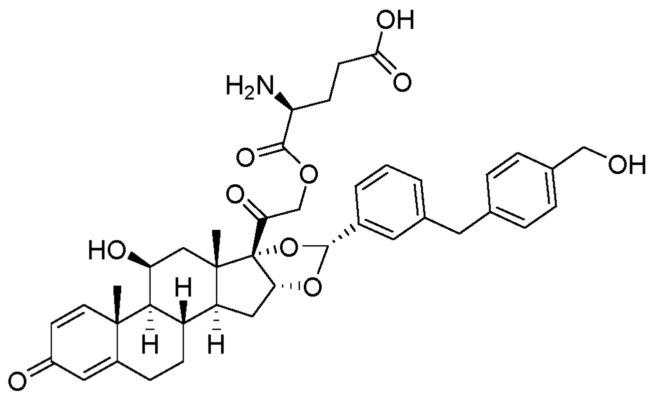 |
| I-247 | 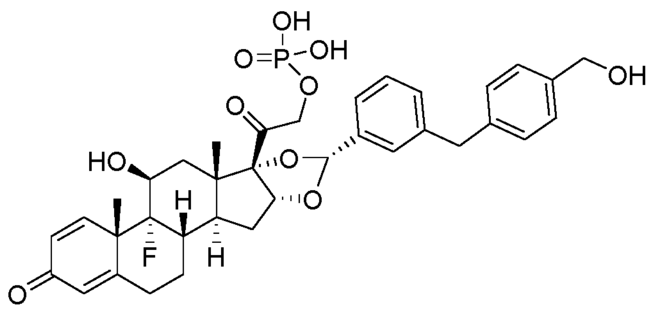 |
| I-248 | 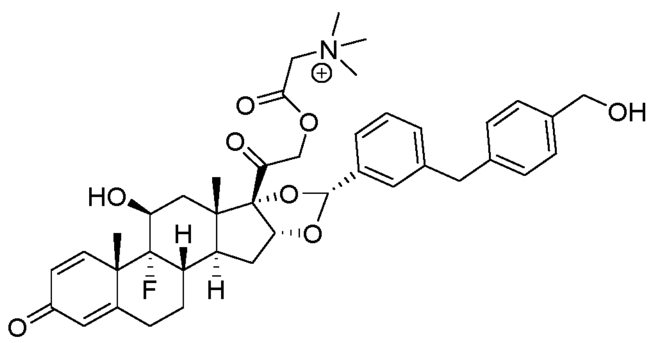 |

(continued)

| No. | Structure |
|---|---|
| I-249 | 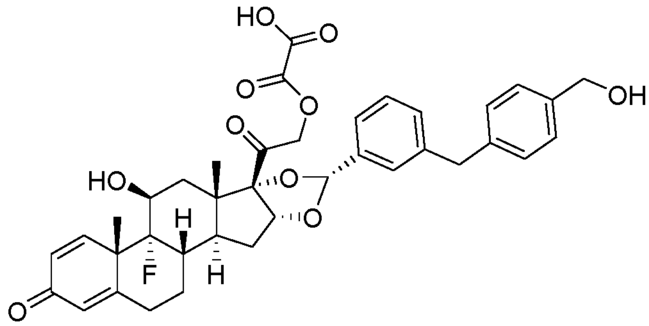 |
| I-250 | 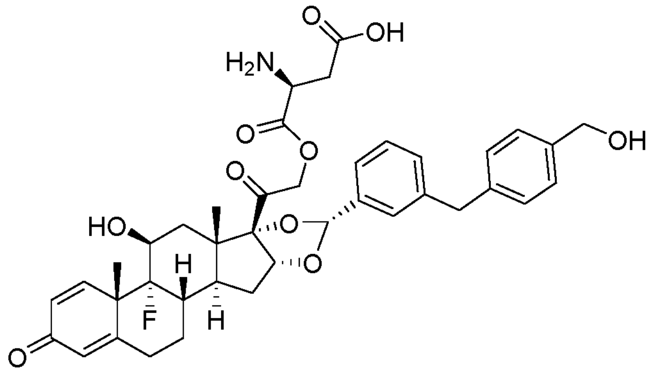 |
| I-251 | 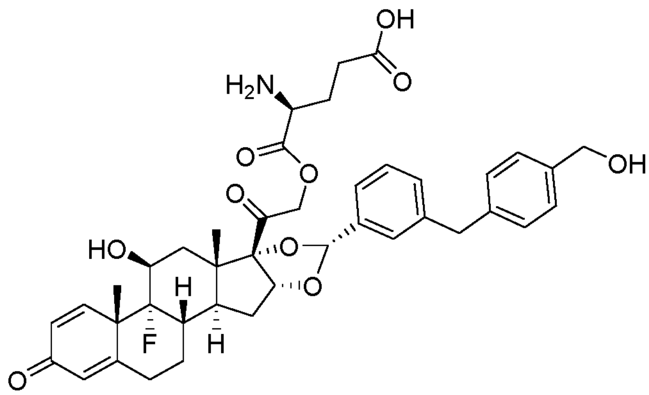 |
| I-252 | 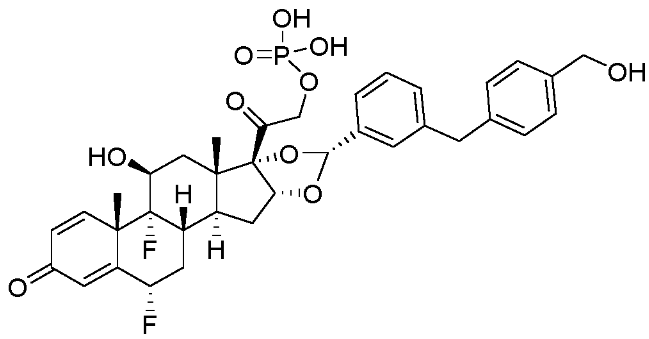 |

(continued)

| No. | Structure |
|---|---|
| I-253 | 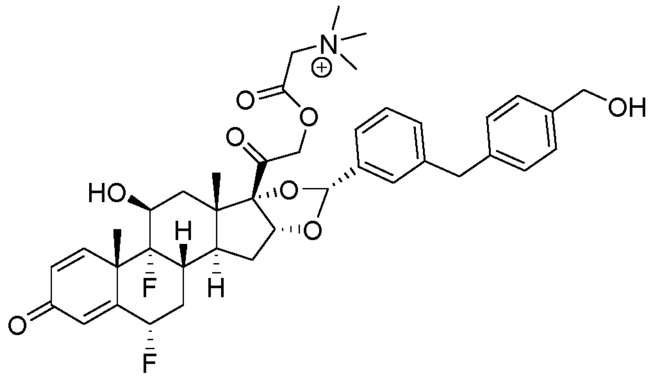 |
| I-254 | 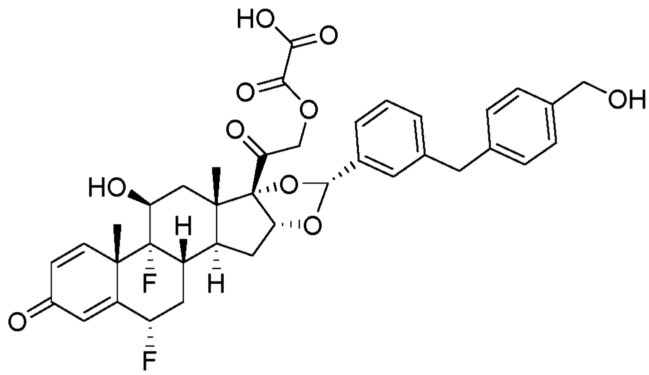 |
| I-255 | 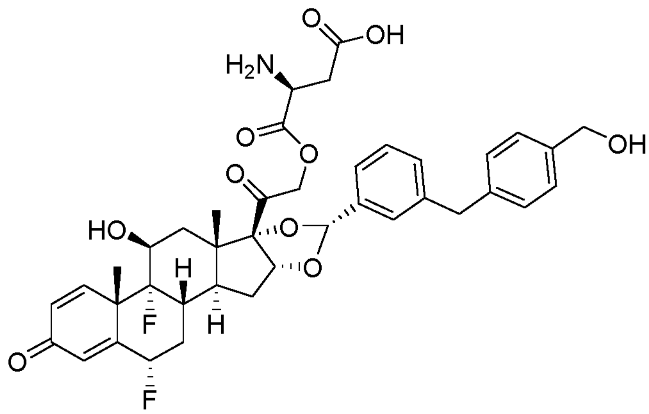 |
| I-256 | 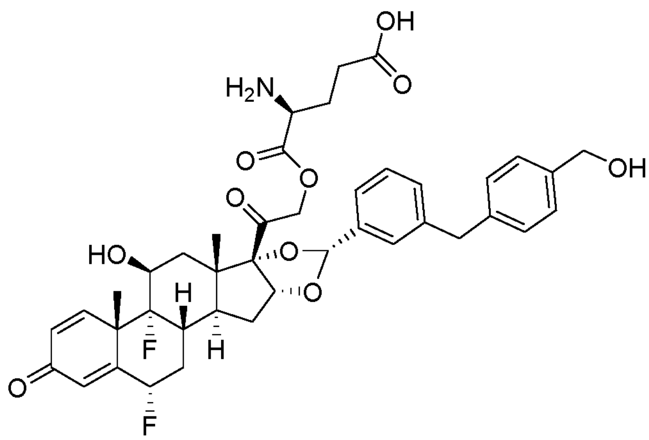 |

(continued)

| No. | Structure |
|---|---|
| I-257 | 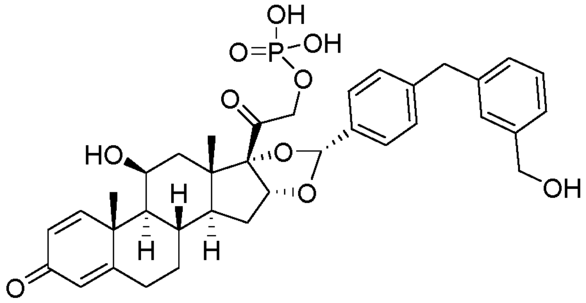 |
| I-258 | 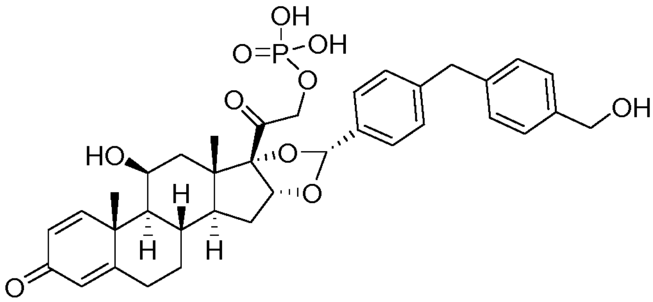 |
| I-259 | 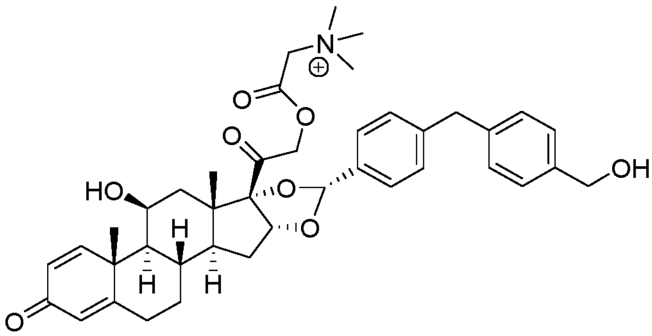 |
| I-260 | 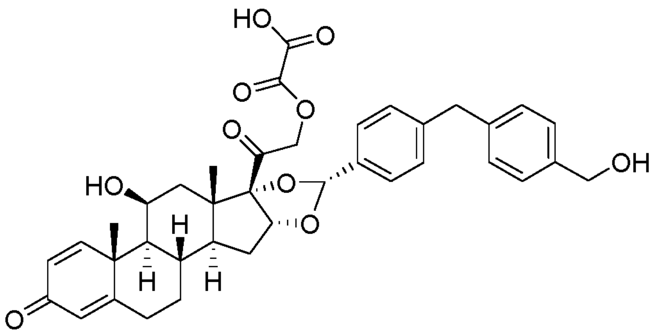 |

(continued)

| No. | Structure |
|---|---|
| I-261 | 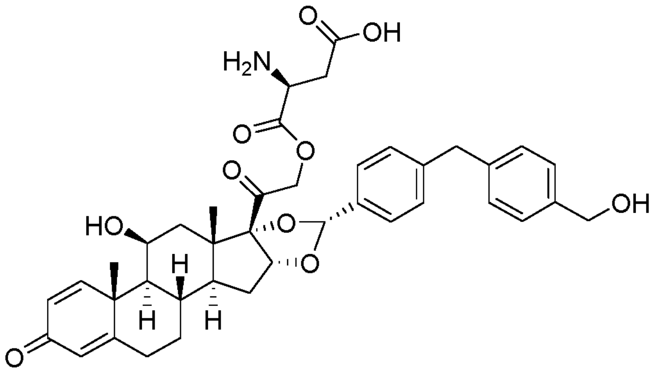 |
| I-262 | 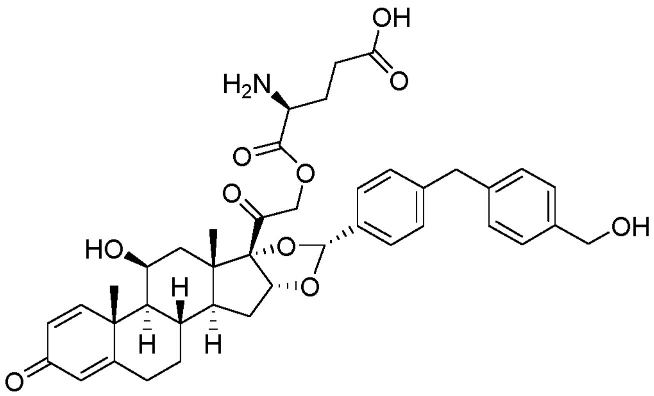 |
| I-263 | 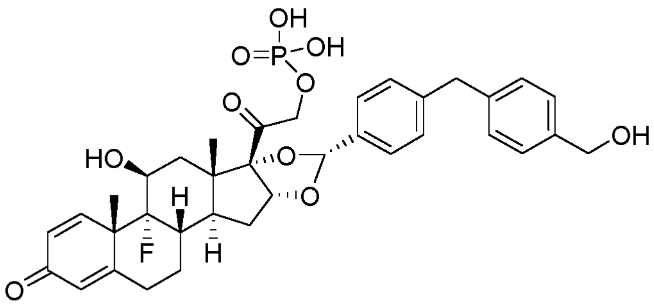 |
| I-264 | 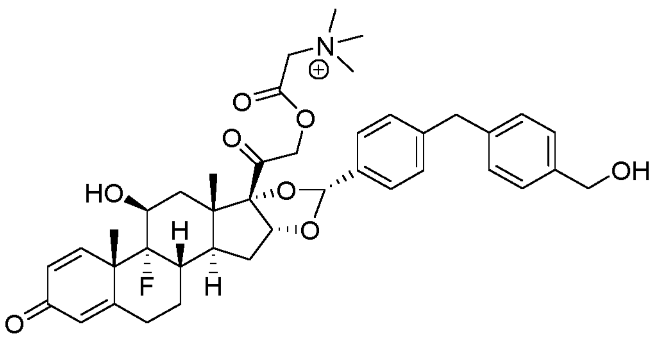 |

(continued)

| No. | Structure |
|---|---|
| I-265 | 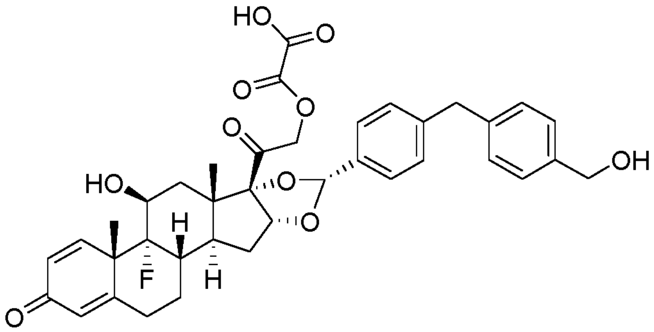 |
| I-266 | 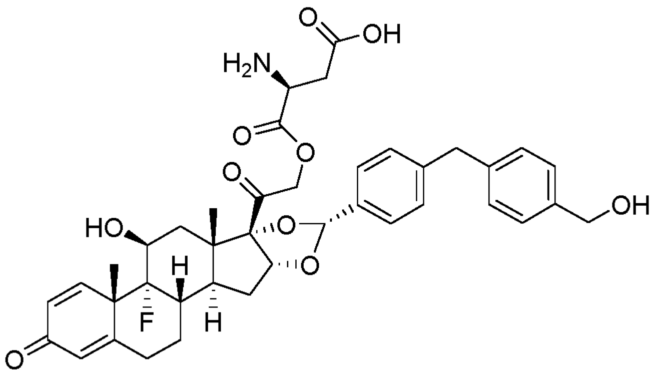 |
| I-267 | 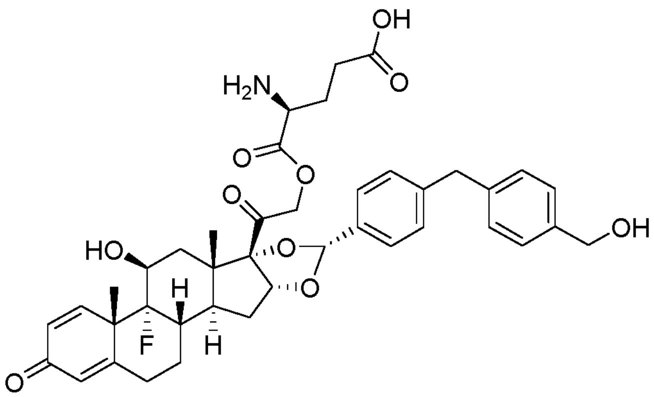 |
| I-268 | 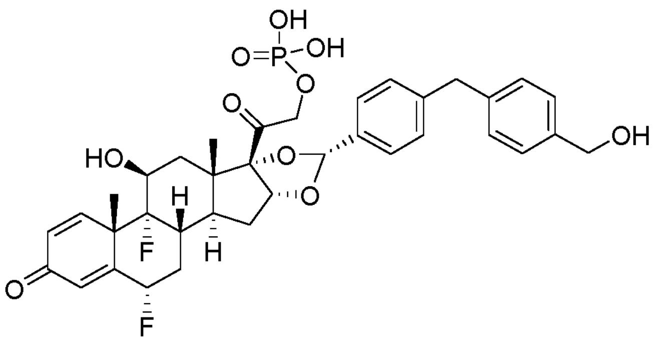 |

(continued)

| No. | Structure |
|---|---|
| I-269 | 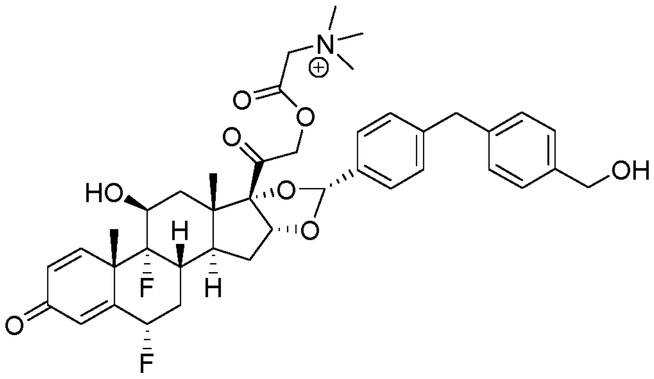 |
| I-270 | 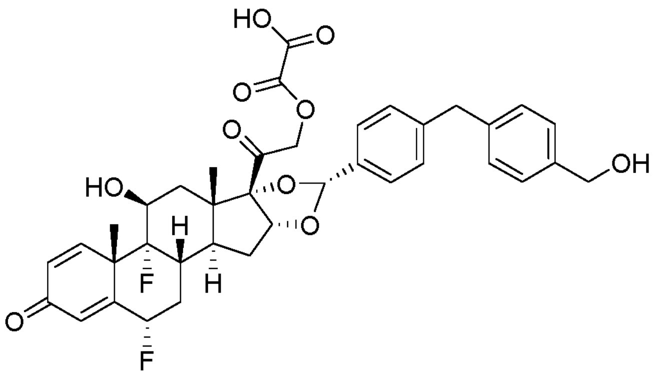 |
| I-271 | 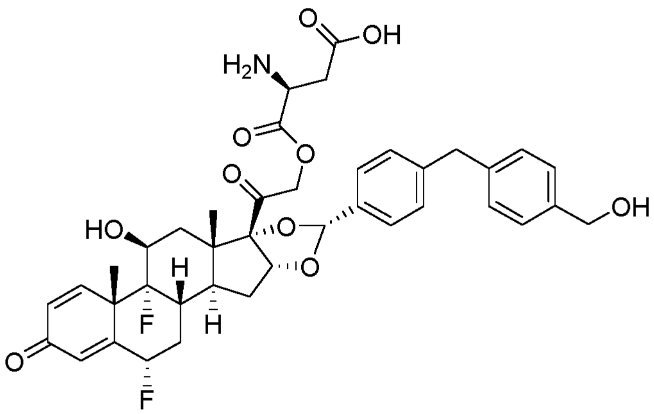 |
| I-272 | 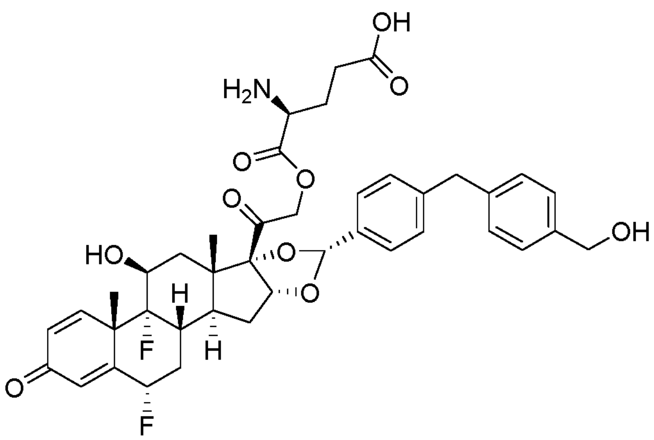 |

(continued)

| No. | Structure |
| --- | --- |
| I-273 | 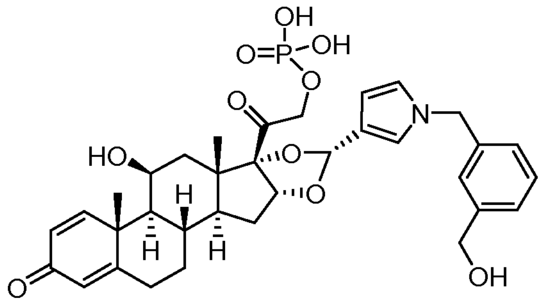 |
| I-274 | 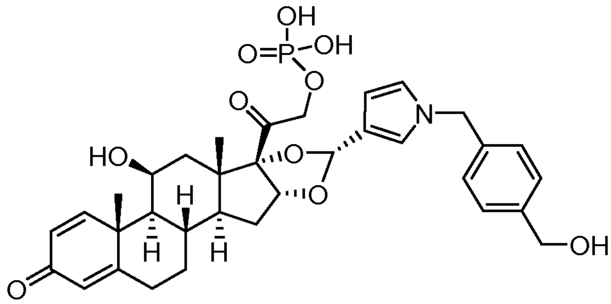 |
| I-275 | 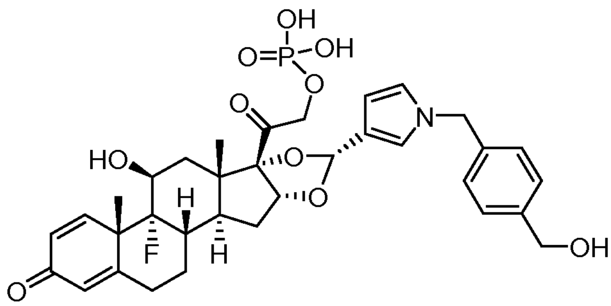 |
| I-276 | 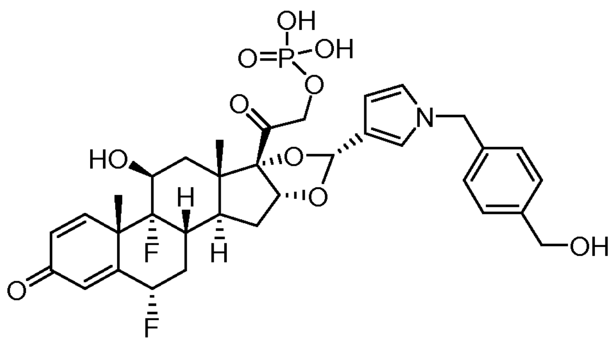 |
| I-277 | 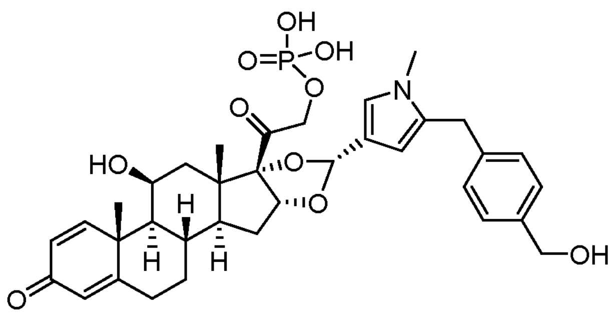 |

(continued)

| No. | Structure |
|---|---|
| I-278 | 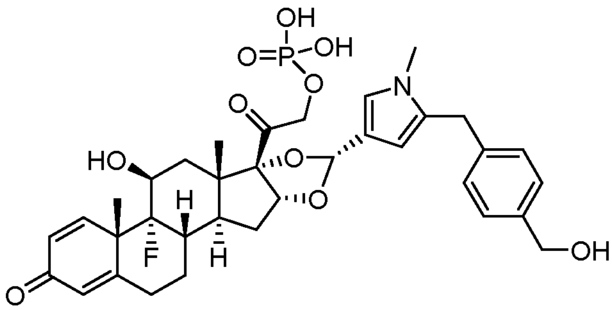 |
| I-279 | 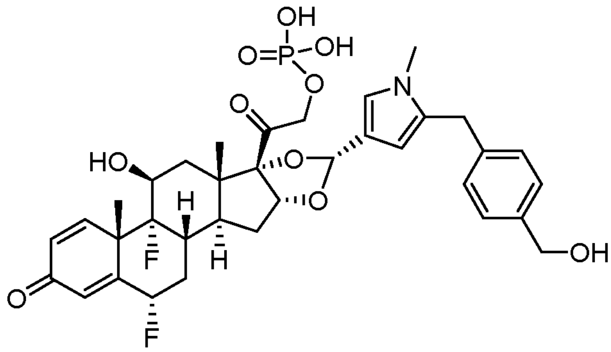 |
| I-280 | 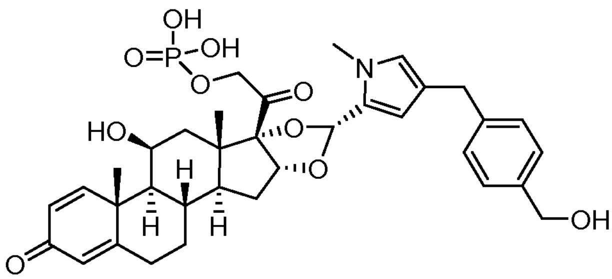 |
| I-281 | 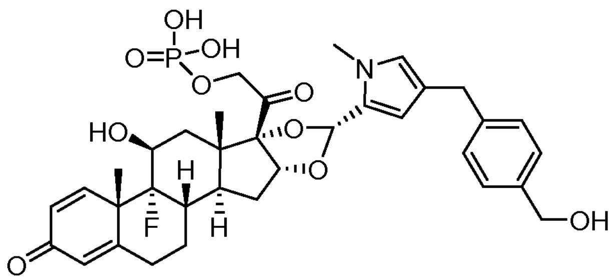 |
| I-282 | 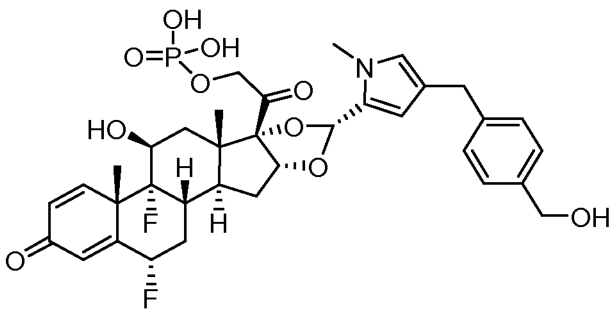 |

(continued)

| No. | Structure |
|---|---|
| I-283 | 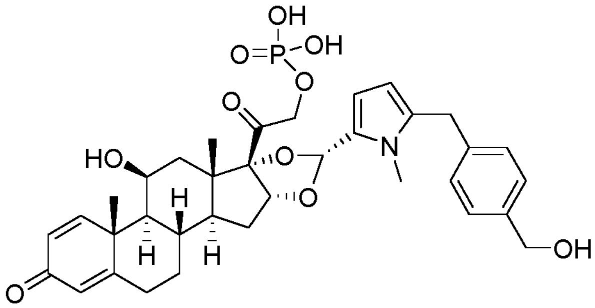 |
| I-284 | 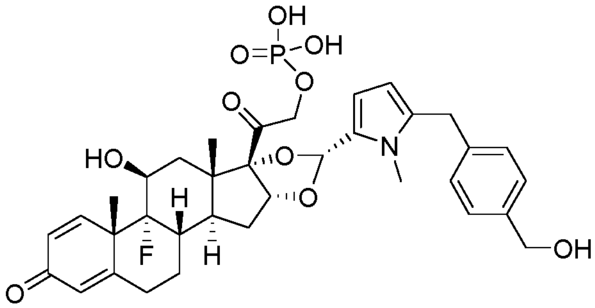 |
| I-285 | 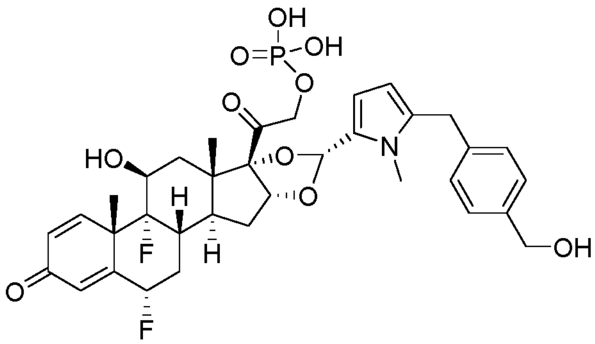 |
| I-286 | 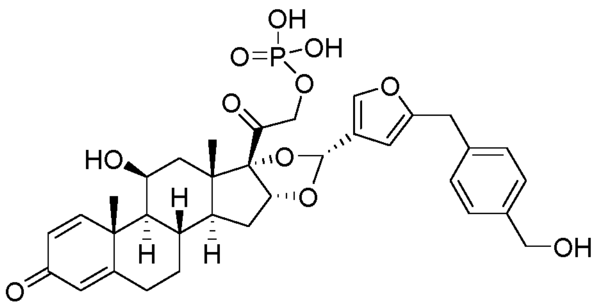 |
| I-287 | 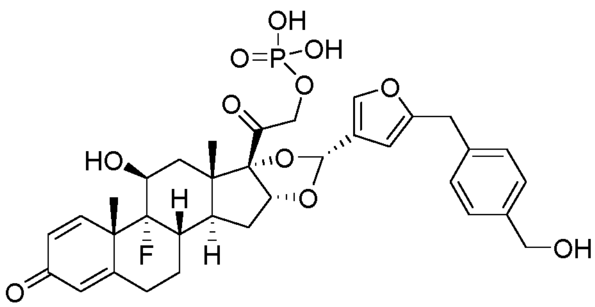 |

(continued)

| No. | Structure |
|---|---|
| I-288 | 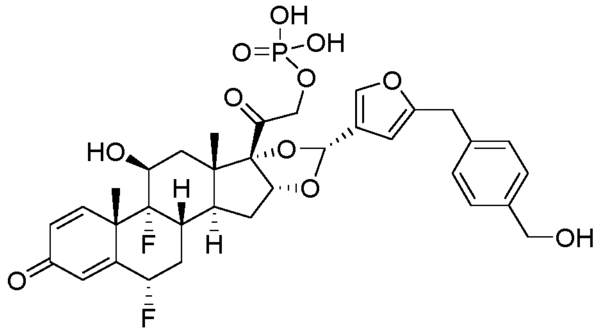 |
| I-289 | 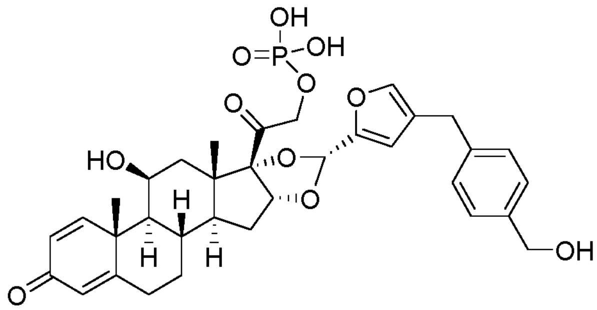 |
| I-290 | 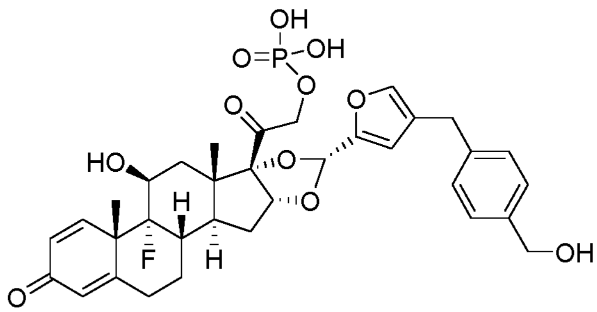 |
| I-291 | 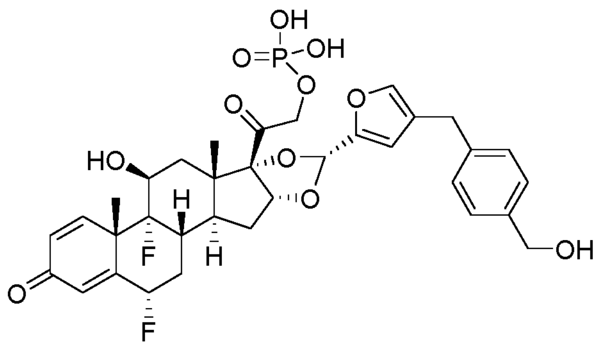 |
| I-292 | 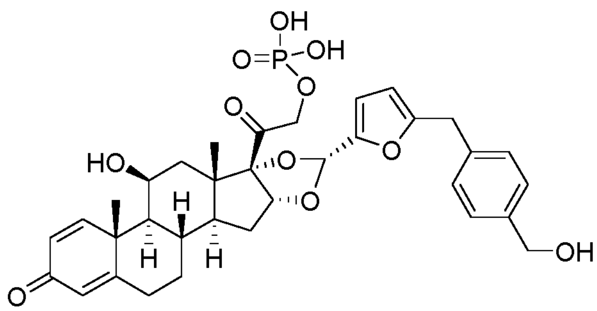 |

(continued)

| No. | Structure |
|---|---|
| I-293 | 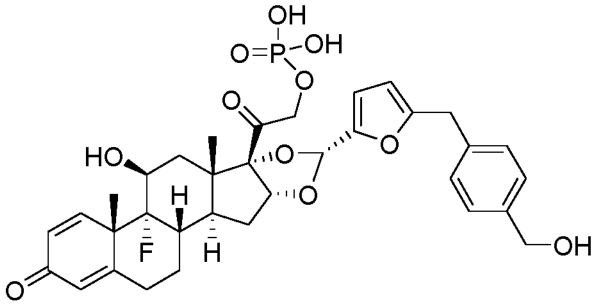 |
| I-294 | 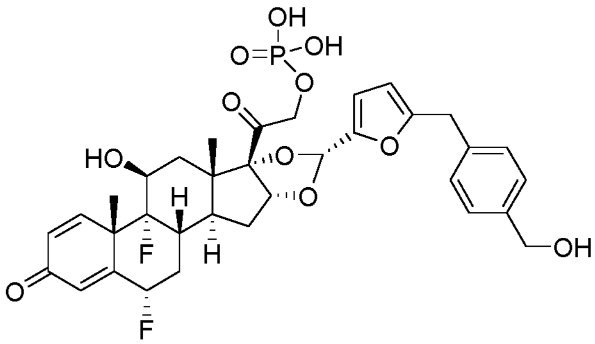 |
| I-295 | 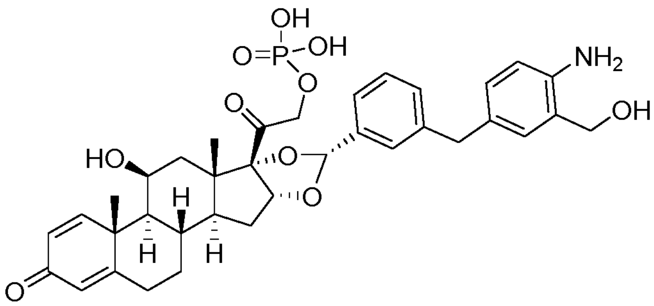 |
| I-296 | 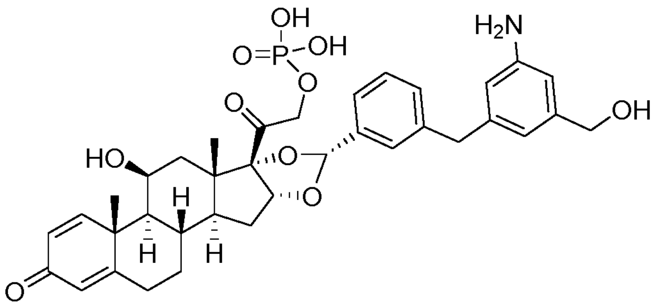 |
| I-297 | 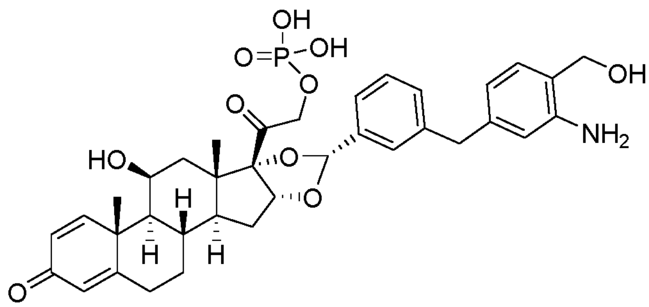 |

(continued)

| No. | Structure |
|---|---|
| I-298 | 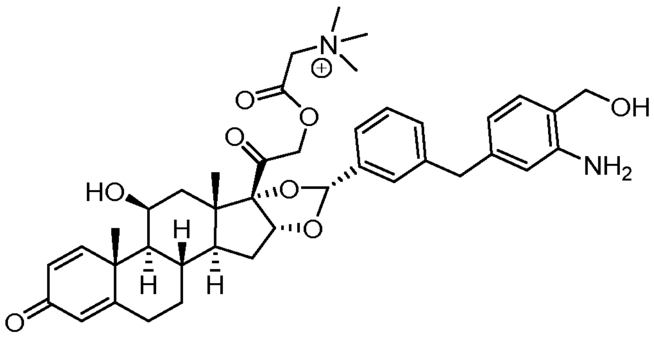 |
| I-299 | 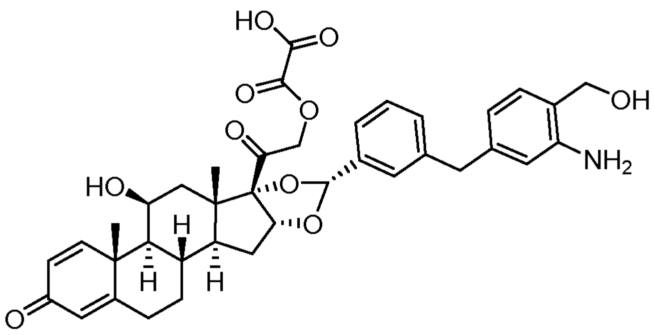 |
| I-300 | 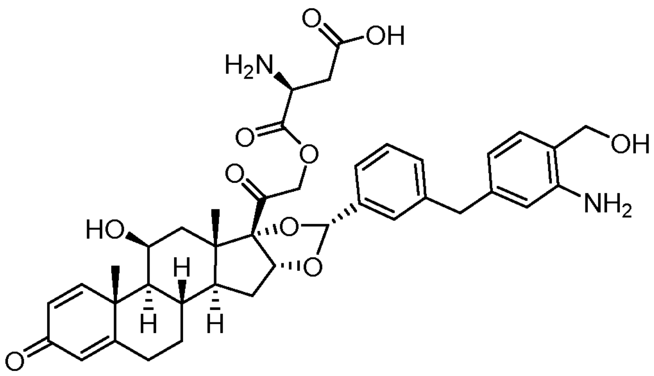 |
| I-301 | 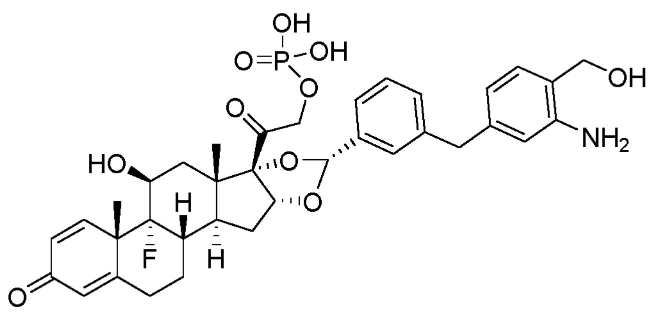 |

(continued)

| No. | Structure |
|---|---|
| I-302 | 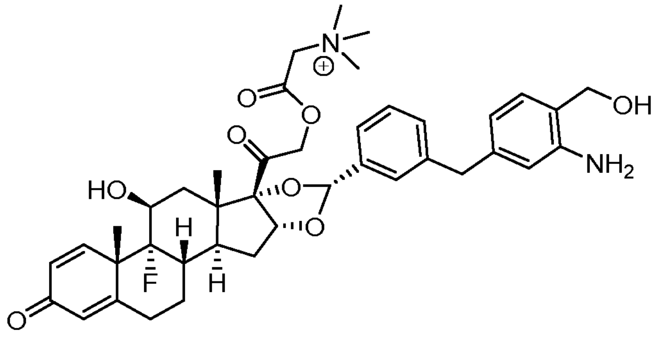 |
| I-303 | 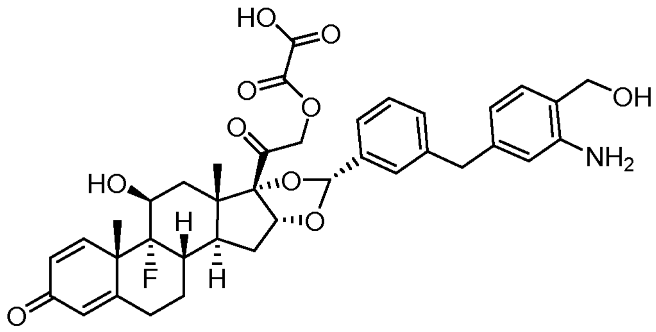 |
| I-304 | 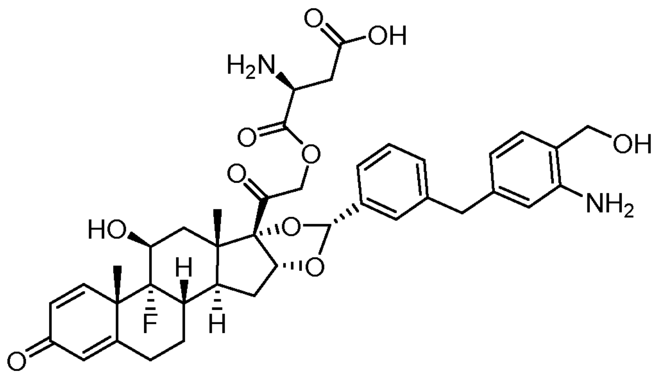 |
| I-305 | 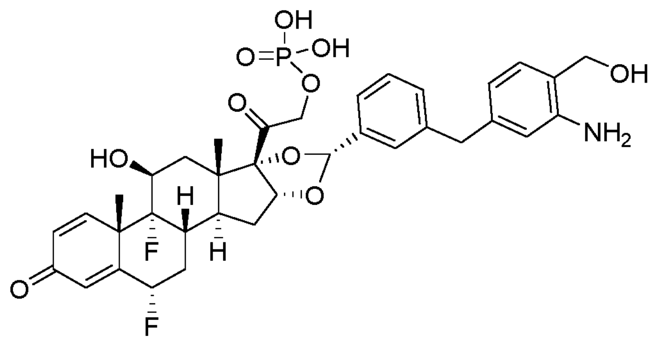 |

(continued)

| No. | Structure |
|---|---|
| I-306 | 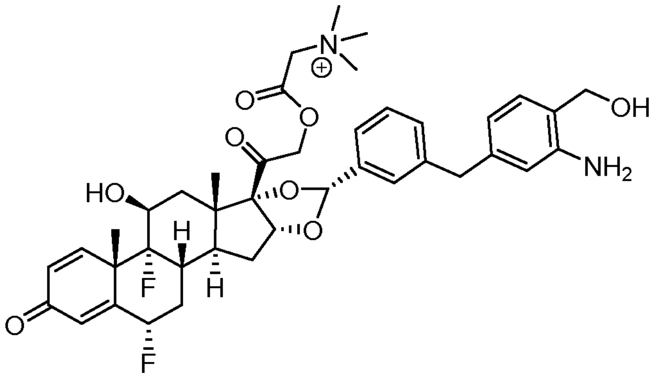 |
| I-307 | 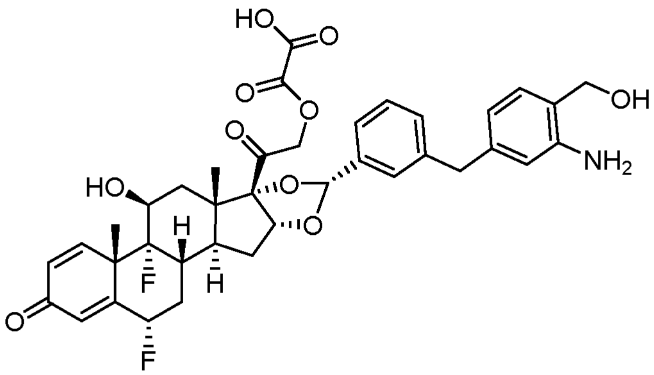 |
| I-308 | 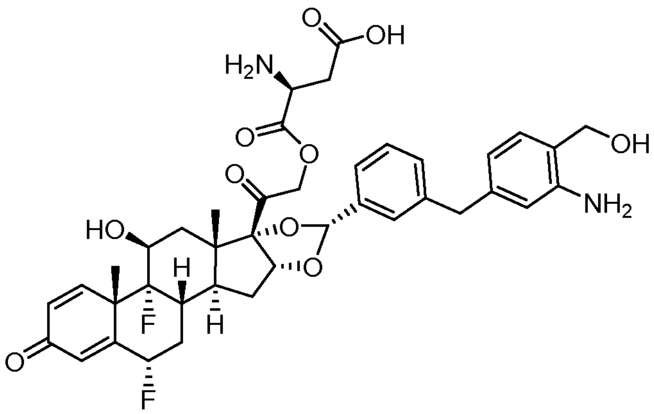 |
| I-309 | 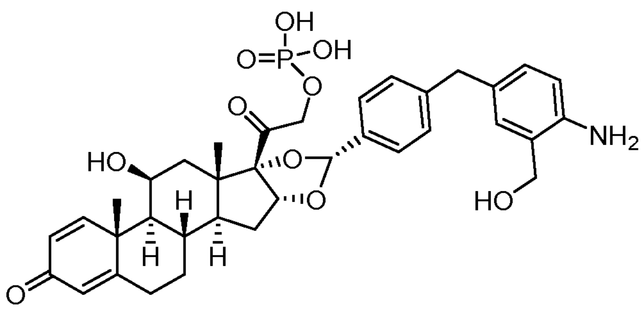 |

(continued)

| No. | Structure |
|---|---|
| I-310 | 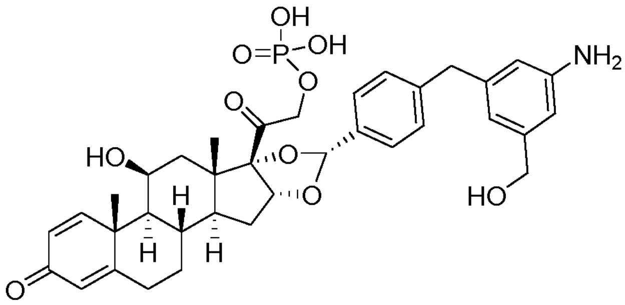 |
| I-311 | 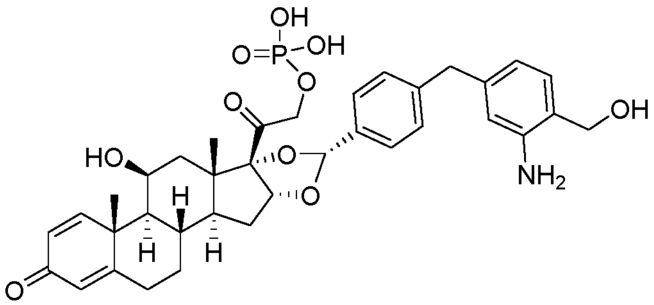 |
| I-312 | 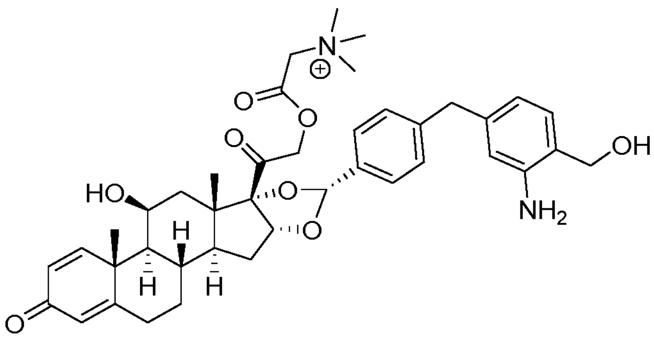 |
| I-313 | 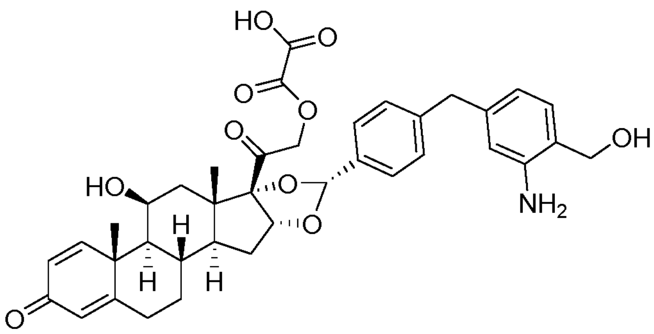 |

(continued)

| No. | Structure |
|---|---|
| I-314 | 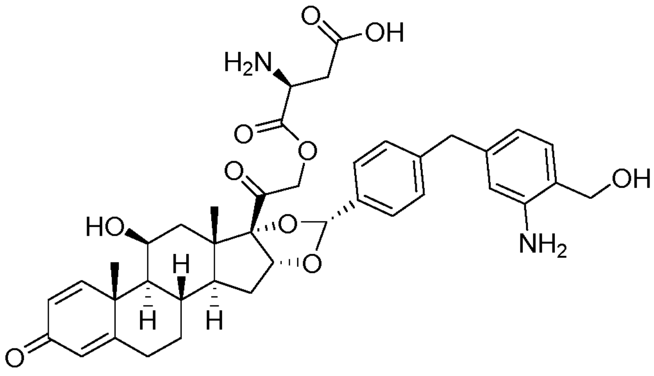 |
| I-315 | 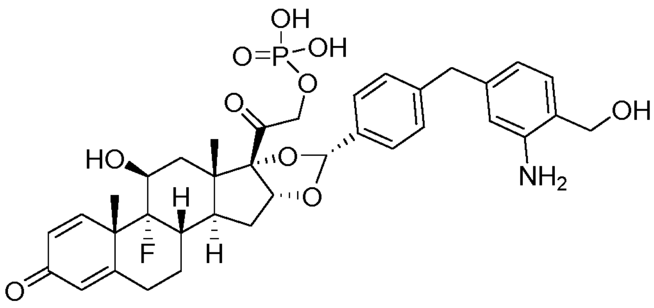 |
| I-316 | 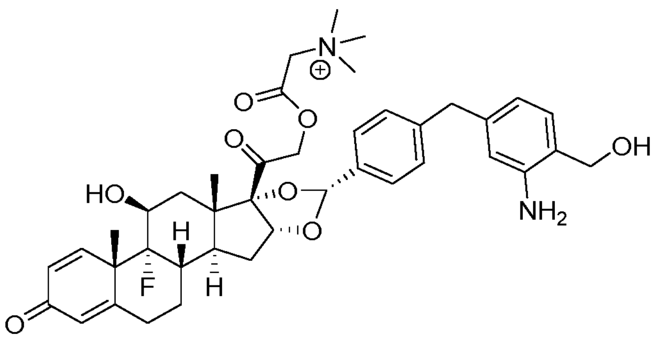 |
| I-317 | 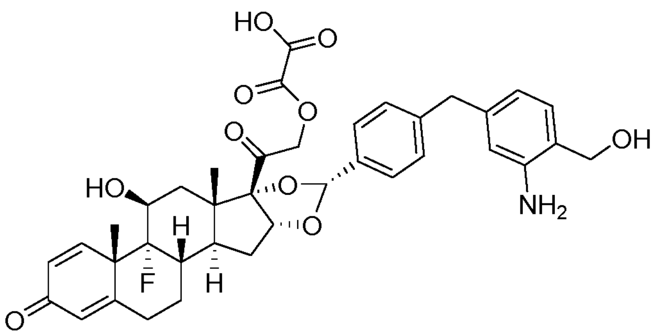 |

(continued)

| No. | Structure |
| --- | --- |
| I-318 | 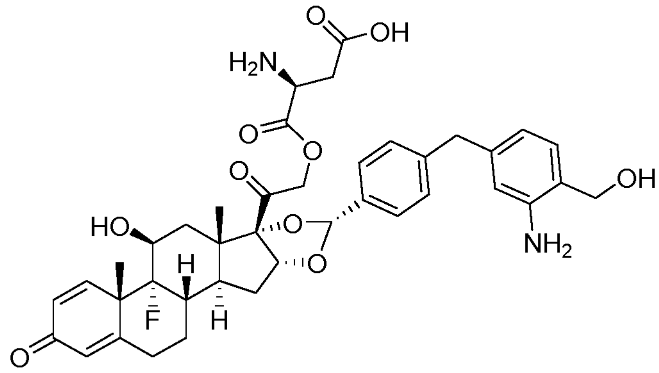 |
| I-319 | 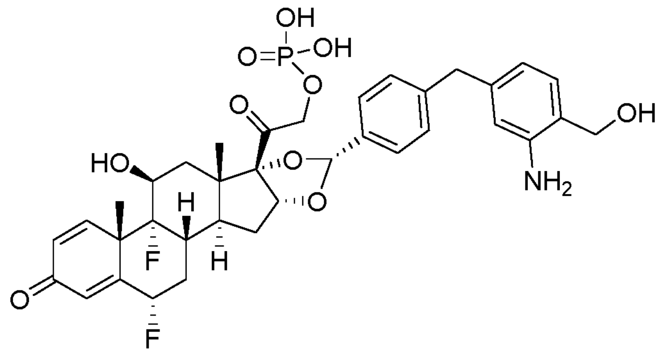 |
| I-320 | 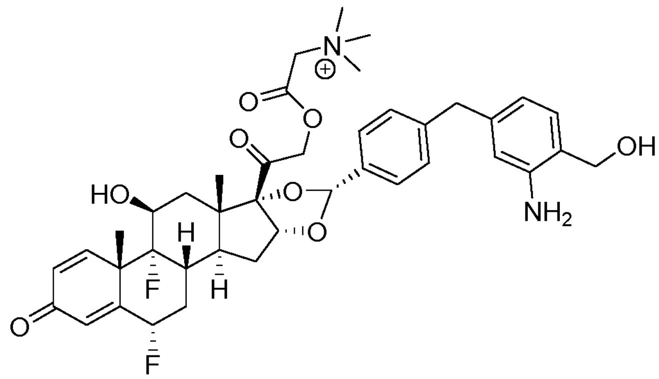 |
| I-321 | 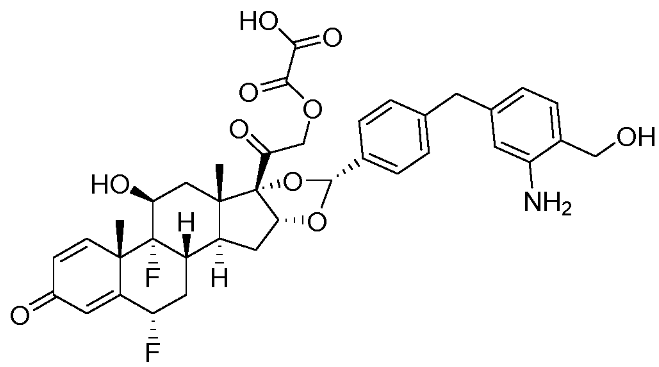 |

(continued)

| No. | Structure |
|---|---|
| I-322 | 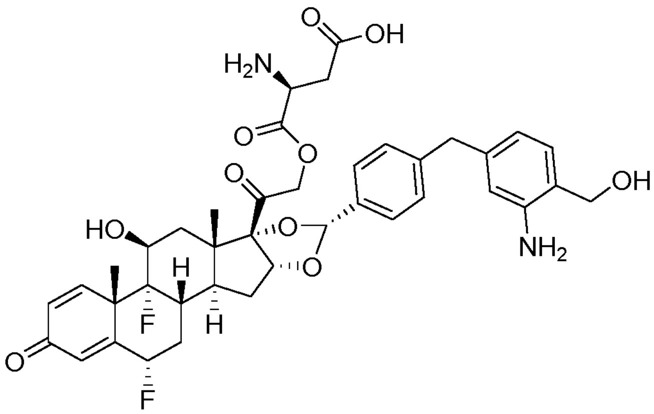 |
| I-323 | 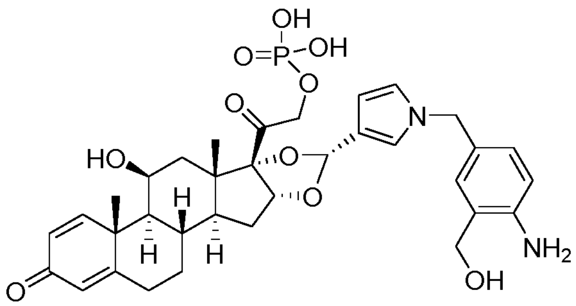 |
| I-324 | 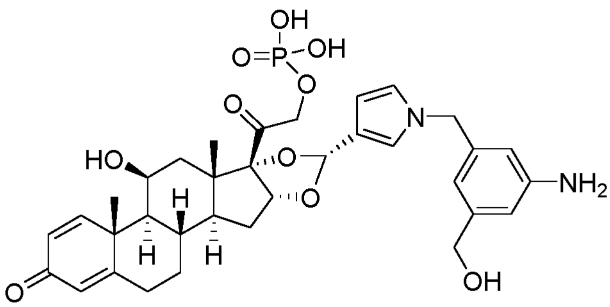 |
| I-325 | 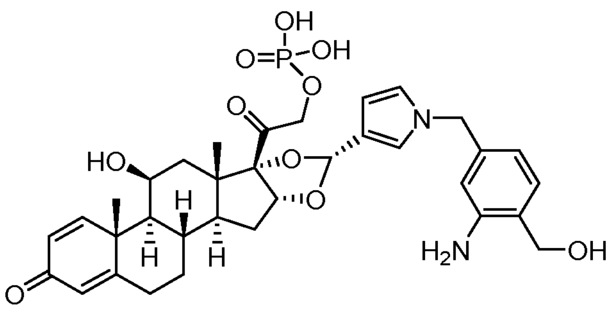 |

(continued)

| No. | Structure |
|---|---|
| I-326 | 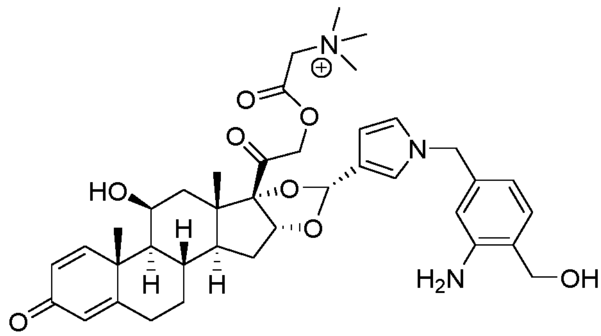 |
| I-327 | 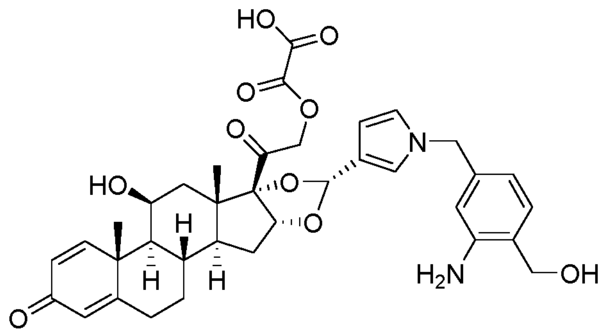 |
| I-328 | 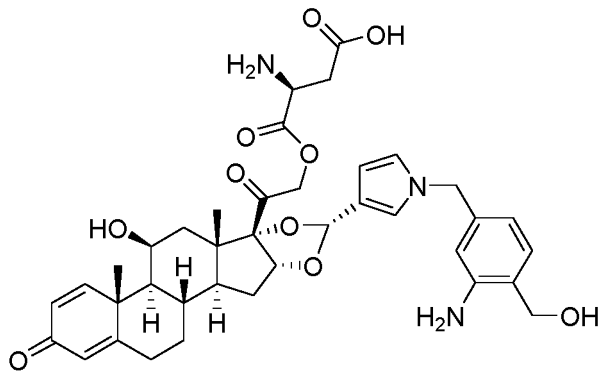 |
| I-329 | 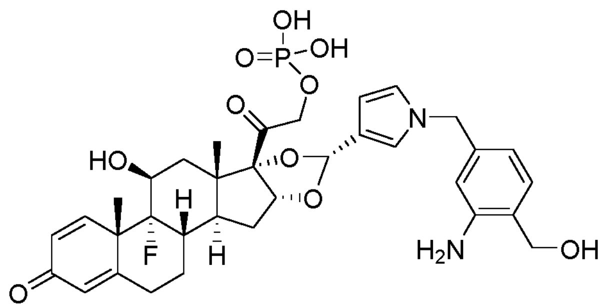 |

(continued)

| No. | Structure |
|---|---|
| I-330 | 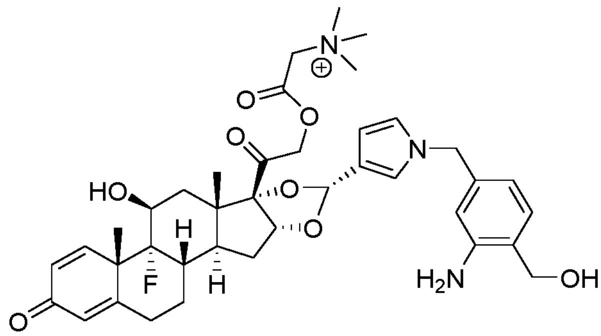 |
| I-331 | 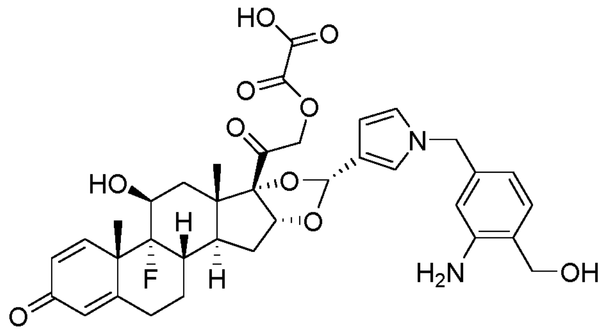 |
| I-332 | 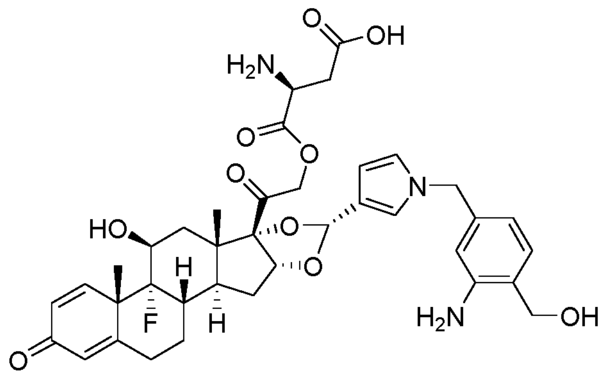 |
| I-333 | 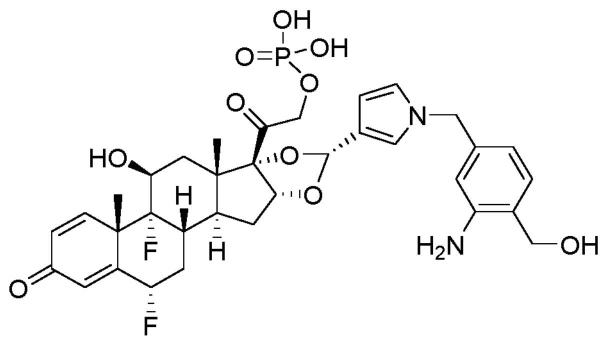 |

(continued)

| No. | Structure |
|---|---|
| I-334 | 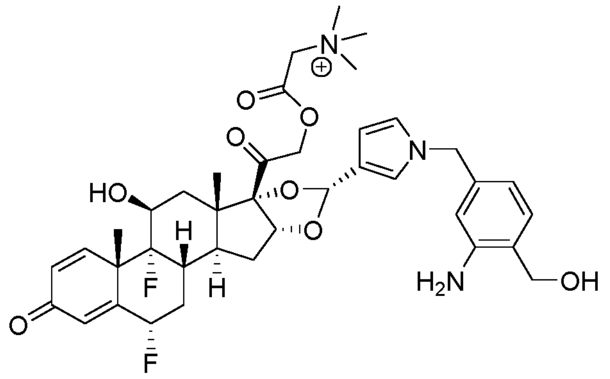 |
| I-335 | 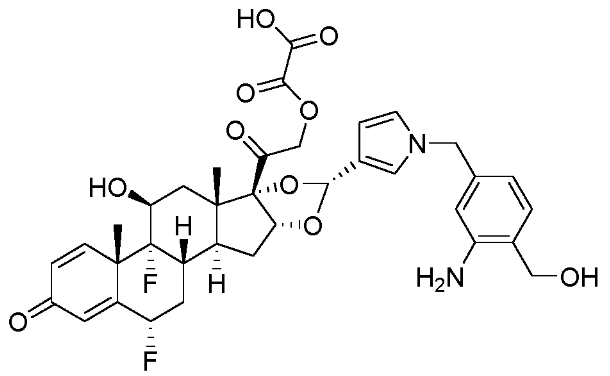 |
| I-336 | 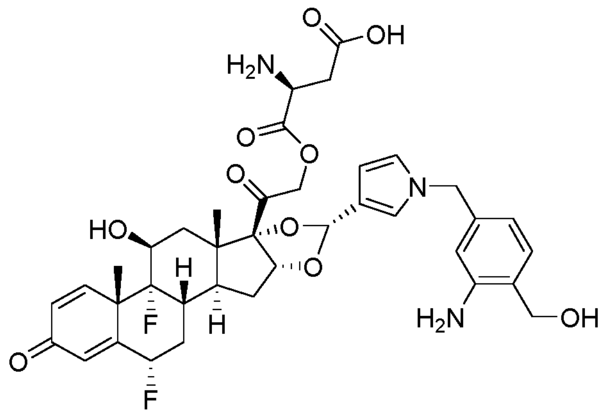 |
| I-337 | 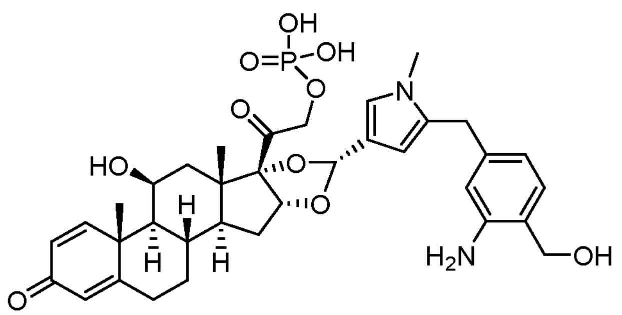 |

(continued)

| No. | Structure |
|---|---|
| I-338 | 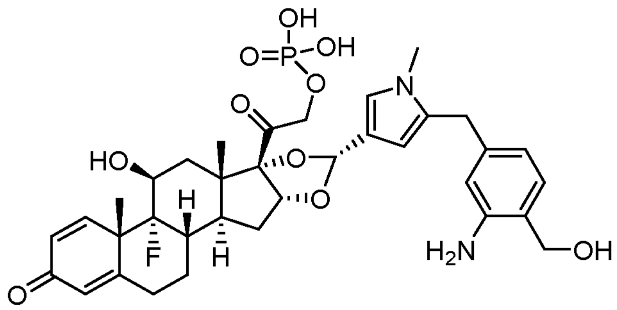 |
| I-339 | 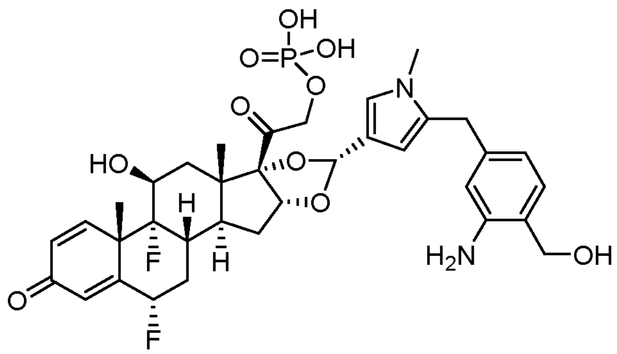 |
| I-340 | 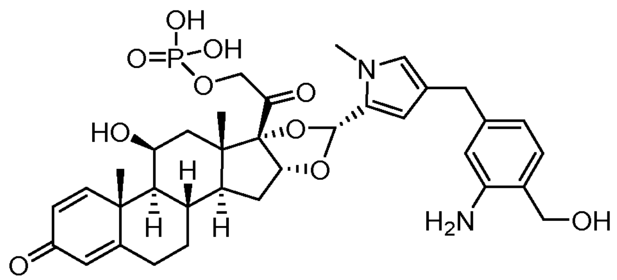 |
| I-341 | 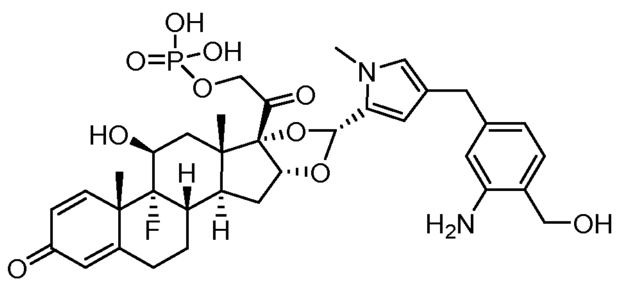 |
| I-342 | 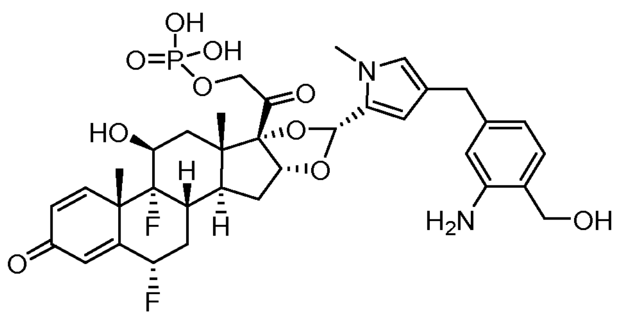 |

(continued)

| No. | Structure |
|---|---|
| I-343 | 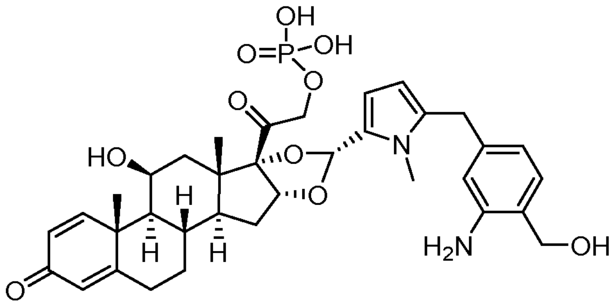 |
| I-344 | 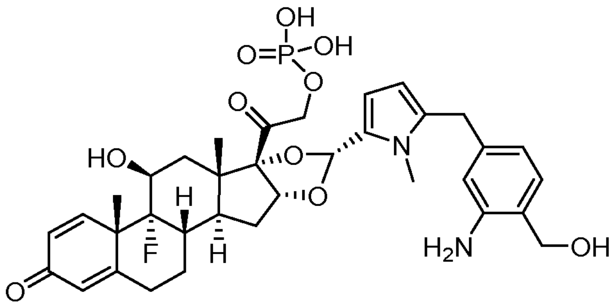 |
| I-345 | 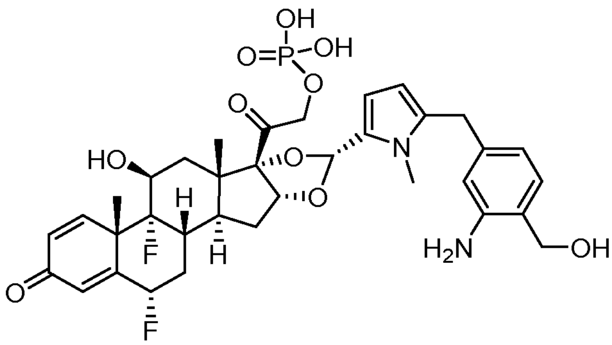 |
| I-346 | 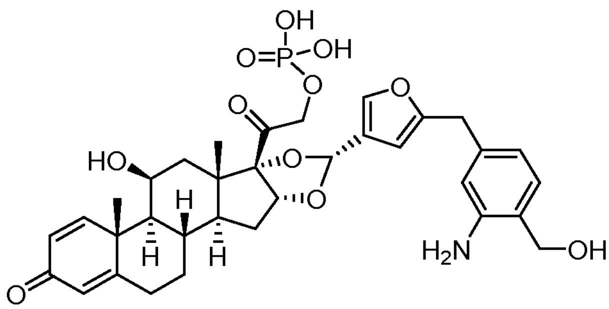 |
| I-347 | 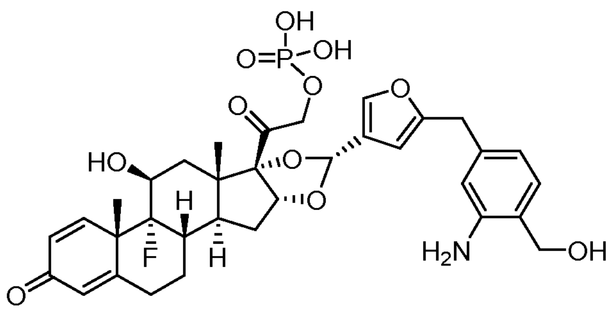 |

(continued)

| No. | Structure |
|---|---|
| I-348 | 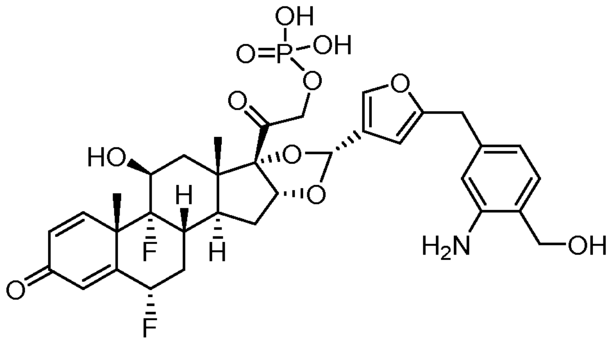 |
| I-349 | 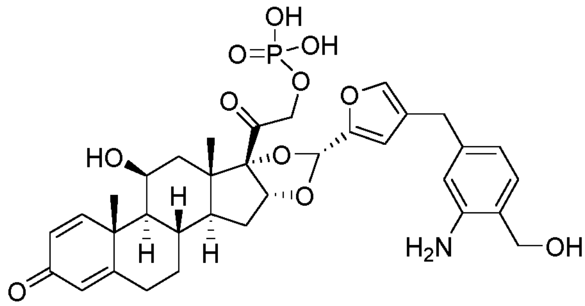 |
| I-350 | 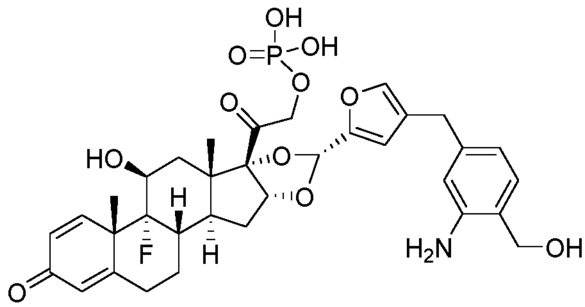 |
| I-351 | 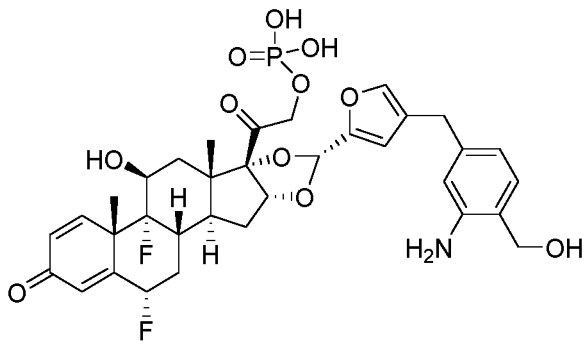 |
| I-352 | 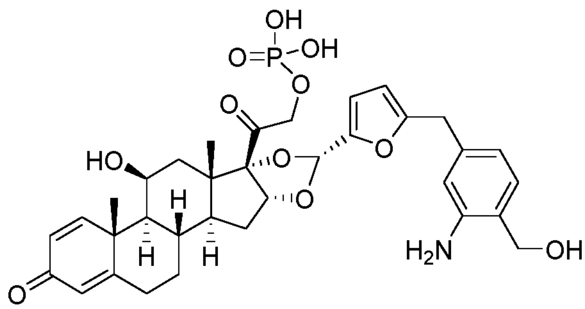 |

(continued)

| No. | Structure |
|---|---|
| I-353 | 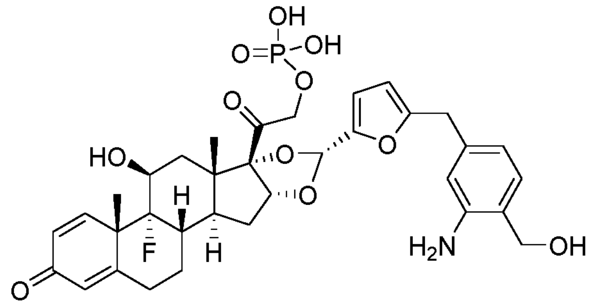 |
| I-354 | 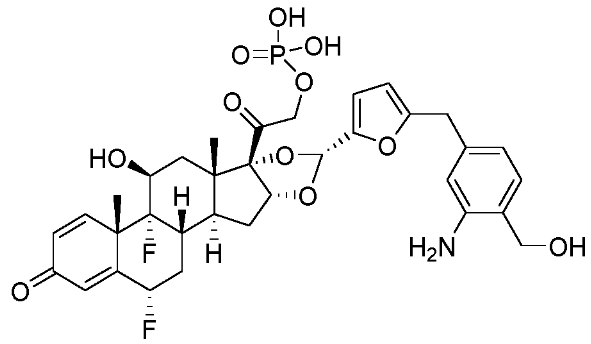 |
| I-355 | 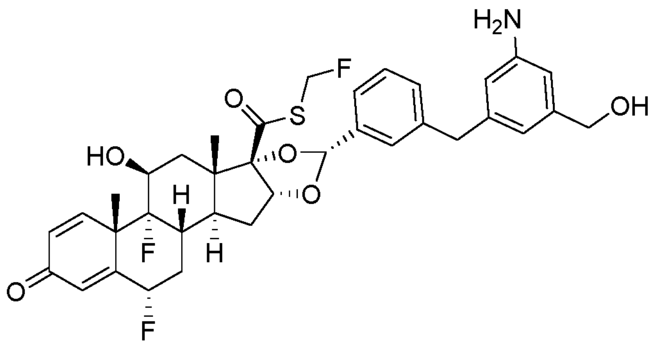 |
| I-356 | 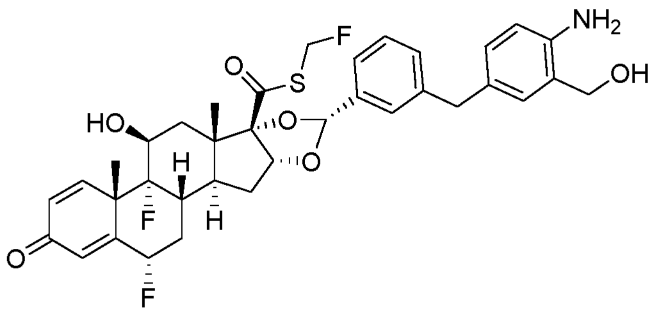 |
| I-357 | 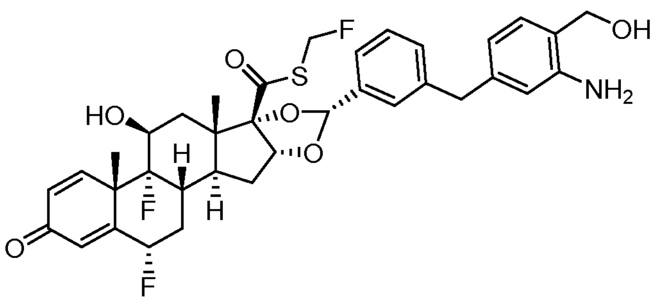 |

(continued)

| No. | Structure |
|---|---|
| I-358 | 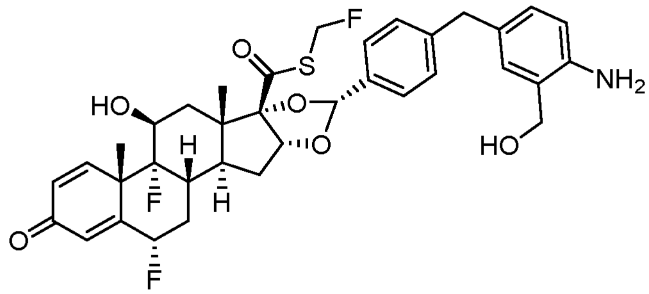 |
| I-359 | 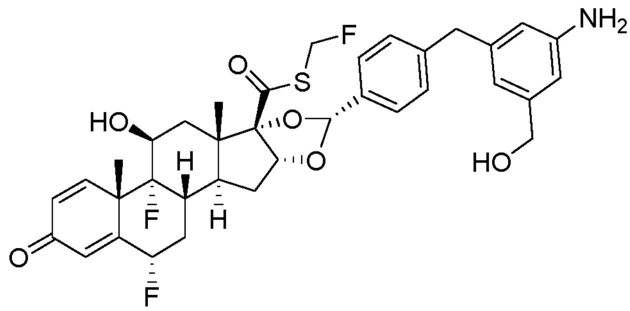 |
| I-360 | 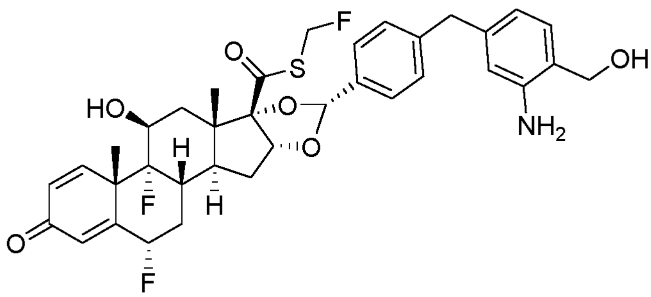 |
| I-361 | 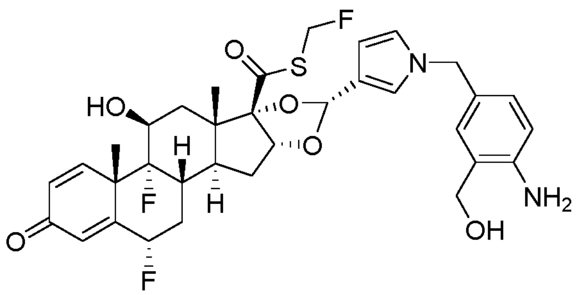 |
| I-362 | 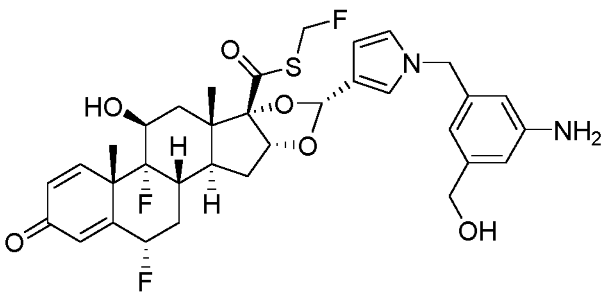 |

(continued)

| No. | Structure |
|---|---|
| I-363 | 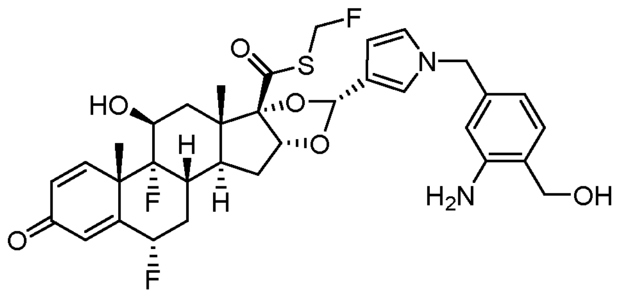 |

In another aspect, the present invention provides a compound of a formula below or a tautomer, a mesomer, a racemate, an enantiomer or a diastereoisomer thereof, or a mixture thereof, or a pharmaceutically acceptable salt thereof, wherein the compound is selected from the group consisting of:

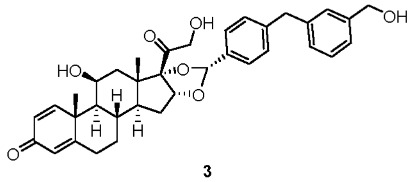
3

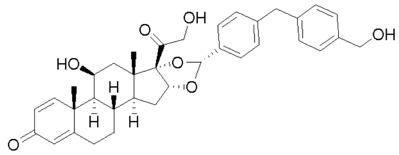
4

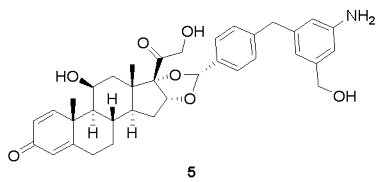
5

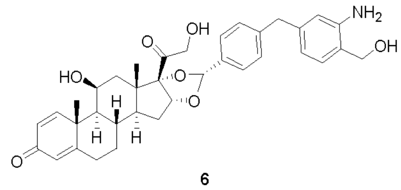
6

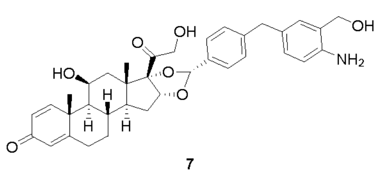
7

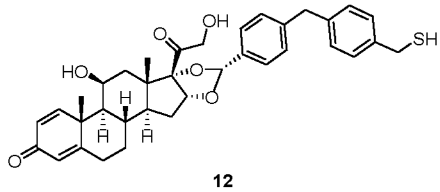
12

.

In another aspect, the present application provides a compound of formula IIa or IIb:

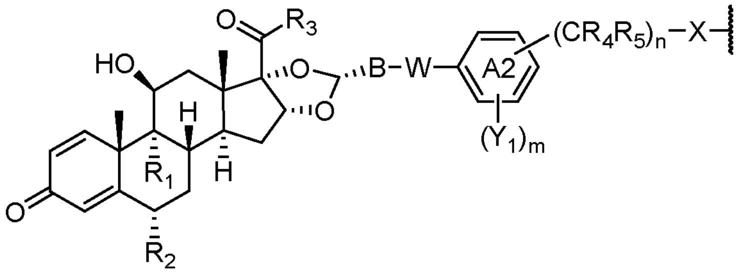

or

(IIa)

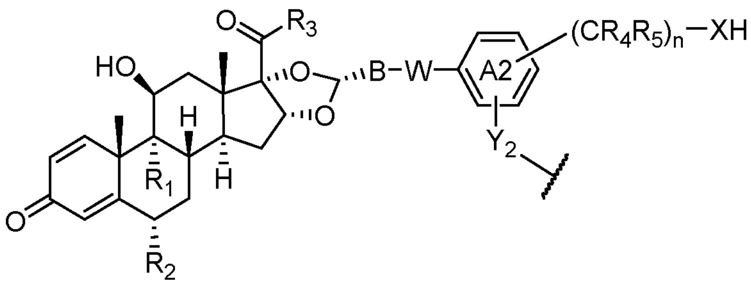

,

(IIb)

or a tautomer, a mesomer, a racemate, an enantiomer or a diastereoisomer thereof, or a mixture thereof, or a pharmaceutically acceptable salt thereof, wherein,

$R_1$, $R_2$, $R_3$, $R_4$ and $R_5$ are each independently any group;
B is absent or B is any group;
W is absent or W is any group;
A2 is a substituted benzene ring;
$CR_4R_5$ units are each independently unreplaced or replaced by a group selected from the group consisting of: -O-, -S-, -NR-, -S(O)-, $-S(O)_2-$, -C(=O)-, -C=C- and -C=C-, wherein $R_4$ and $R_5$ can together form optionally substituted cycloalkyl, optionally substituted heterocyclyl, optionally substituted bridged cyclyl, optionally substituted bridged heterocyclyl, optionally substituted spiro cyclyl and optionally substituted spiro heterocyclyl, and n is any integer from 1 to 20;
X is selected from the group consisting of: -O-, -S- and -NR-;
$Y_1$ is any group, and m is any integer from 0 to 4;
$Y_2$ is selected from the group consisting of -O-, -S- and -NR-;
wherein substituents are selected from the group consisting of: protium, deuterium, tritium, halogen, -CN, =O, =N-OH, =N-OR, =N-R, -OR, -C(O)R, -C(O)OR, -OC(O)R, -OC(O)OR, -C(O)NHR, - $C(O)NR_2$, -OC(O)NHR, $-OC(O)NR_2$, -SR, -S(O)R, $-S(O)_2R$, -NHR, $-N(R)_2$, -NHC(O)R, -NRC(O)R, -NHC(O)OR, -NRC(O)OR, $-S(O)_2NHR$, $-S(O)_2N(R)_2$, $-NHS(O)_2NR_2$, $-NRS(O)_2NR_2$, $-NHS(O)_2R$, - $NRS(O)_2R$, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy; wherein each R is independently selected from the group consisting of hydrogen, protium, deuterium, tritium, oxygen, halogen, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy.

In certain embodiments, the wavy line

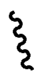

in the formula IIa or lib represents being directly linked to a ligand via the X or $Y_2$ group, or being linked to the ligand via a Linker fragment.

In certain preferred embodiments of the present invention, certain groups in the compounds of formulas (IIa) and (IIb) or pharmaceutically acceptable salts thereof are defined as follows, and groups not mentioned are as described in any one of the embodiments of the present application ("in certain embodiments" for short).

In certain embodiments, $CR_4R_5$ units are each independently unreplaced or replaced by a group selected from the group consisting of: -O-, -S-and -C(=O)-.

In certain embodiments, $R_4$ and $R_5$ together form optionally substituted cycloalkyl or optionally substituted heterocyclyl. For example, $R_4$ and $R_5$ may together form cyclopropyl or cyclobutyl.

In certain embodiments, $R_4$ and $R_5$ are each independently selected from the group consisting of: hydrogen, protium, deuterium, tritium, halogen, $-NO_2$, -CN, =O, =S, optionally substituted -S(=O)H, optionally substituted $-S(=O)_2H$, optionally substituted -C(=O)H, optionally substituted -OH, optionally substituted -SH, optionally substituted $-PH_2$, optionally substituted $-P(=O)H_2$, optionally substituted $-NH_2$, optionally substituted alkyl, optionally substituted alkenyl, optionally substituted alkynyl, optionally substituted cycloalkyl, optionally substituted heterocycloalkyl, optionally substituted aryl and optionally substituted heteroaryl; wherein, when $R_4$ and $R_5$ comprise a methylene unit, the methylene

units of $R_4$ and $R_5$ are each independently unreplaced, or the methylene units of $R_4$ and $R_5$ are each independently replaced by a group selected from the group consisting of: -S(=O)-, -S(=O)$_2$-, -O-, -S-, -C(=O)-, optionally substituted -PH-, optionally substituted -P(=O)H-, optionally substituted -NH-, optionally substituted alkylene, optionally substituted alkenylene, optionally substituted alkynylene, optionally substituted cycloalkylene, optionally substituted heterocycloalkylene, optionally substituted arylene and optionally substituted heteroarylene.

In certain embodiments, $R_4$ and $R_5$ are each independently selected from the group consisting of: hydrogen, protium, deuterium, tritium, halogen, optionally substituted -C(=O)H, optionally substituted -OH, optionally substituted -SH, optionally substituted -$NH_2$, optionally substituted alkyl, optionally substituted alkenyl, optionally substituted alkynyl, optionally substituted cycloalkyl, optionally substituted heterocycloalkyl, optionally substituted aryl and optionally substituted heteroaryl; wherein, when $R_4$ and $R_5$ comprise a methylene unit, the methylene units of $R_4$ and $R_5$ are each independently unreplaced, or the methylene units of $R_4$ and $R_5$ are each independently replaced by a group selected from the group consisting of: -S(=O)-, -S(=O)$_2$-, -O-, -S-, -C(=O)-, optionally substituted -PH-, optionally substituted -P(=O)H-, optionally substituted -NH-, optionally substituted alkylene, optionally substituted alkenylene, optionally substituted alkynylene, optionally substituted cycloalkylene, optionally substituted heterocycloalkylene, optionally substituted arylene and optionally substituted heteroarylene.

In certain embodiments, provided is the compound or the tautomer, the mesomer, the racemate, the enantiomer or the diastereoisomer thereof, or the mixture thereof, or the pharmaceutically acceptable salt thereof of the present invention, wherein $R_4$ and $R_5$ are each independently selected from the group consisting of: H, F, Cl, -OH, -$NH_2$ and $C_1$-$C_6$ alkyl; and n is selected from the group consisting of 1, 2 and 3.

In certain embodiments, $Y_1$ is selected from the group consisting of: protium, deuterium, tritium, halogen, -OR, -SR, -NHR, -N(R)$_2$, -PHR, -P(R)$_2$, -P(=O)HR, -P(=O)$R_2$, optionally substituted alkyl, optionally substituted alkenyl, optionally substituted alkynyl, optionally substituted cycloalkyl, optionally substituted heterocycloalkyl, optionally substituted aryl and optionally substituted heteroaryl; wherein, each R is independently selected from the group consisting of hydrogen, protium, deuterium, tritium, oxygen, halogen, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy.

In certain embodiments, $Y_1$ is selected from the group consisting of: protium, deuterium, tritium, halogen, -OR, -SR, -NHR, -N(R)$_2$, optionally substituted $C_1$-$C_6$ alkyl and optionally substituted $C_1$-$C_6$ alkoxy; wherein each R is independently selected from the group consisting of hydrogen, protium, deuterium, tritium, halogen, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy.

In certain embodiments, $Y_1$ is absent or selected from the group consisting of: -OR, -SR, -N(R)$_2$ and optionally substituted $C_1$-$C_6$ alkyl; wherein each R is independently selected from the group consisting of hydrogen, protium, deuterium, tritium, $C_1$-$C_6$ alkyl and $C_1$-$C_6$ alkoxy.

In certain embodiments, $Y_1$ is absent or selected from the group consisting of: hydroxy, sulfhydryl, amino and $C_1$-$C_6$ alkyl.

In certain embodiments, $Y_2$ comprises -NR-; wherein each R is independently selected from the group consisting of hydrogen, protium, deuterium, tritium, halogen, $C_1$-$C_6$ alkyl, $C_1$-$C_6$ alkoxy, aryl, heteroaryl, cycloalkyl, heterocycloalkyl, halogenated $C_1$-$C_6$ alkyl, and halogenated $C_1$-$C_6$ alkoxy.

For example, A2 (structural unit

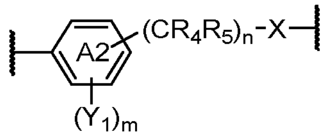

) may be selected from the group consisting of:

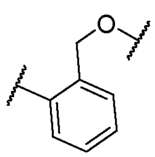,
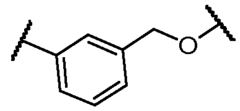,
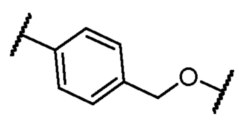,
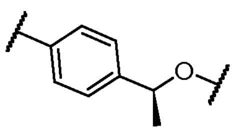,
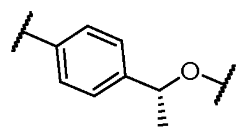,

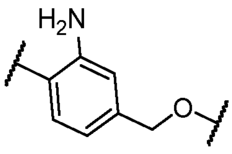, 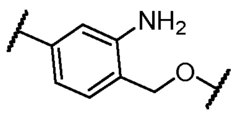, 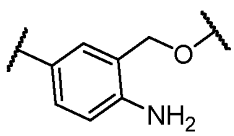, 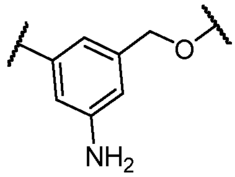, 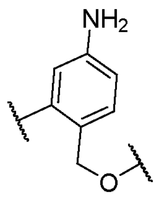,

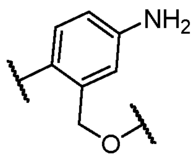, 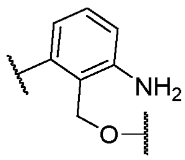, 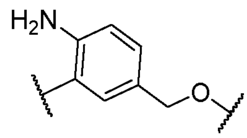, 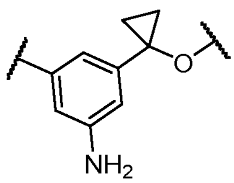, 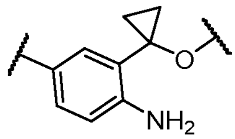,

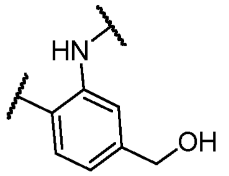, 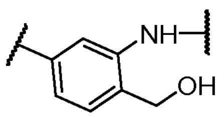, 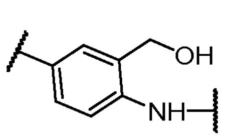, 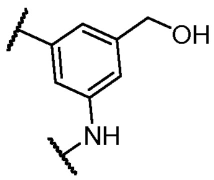, 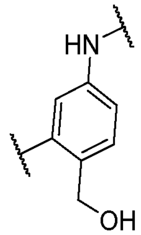,

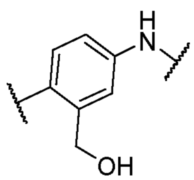, 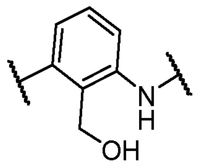, 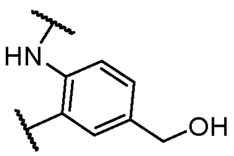, 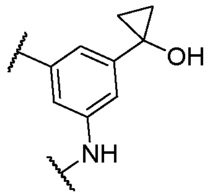, 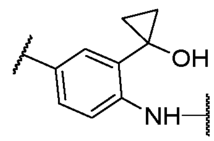,

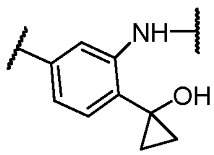, 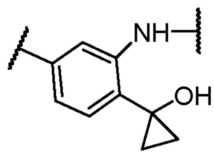, 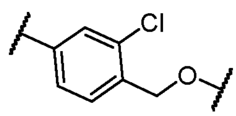, 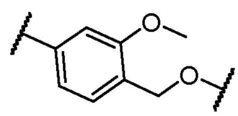, 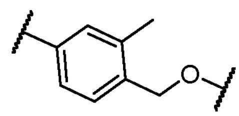,

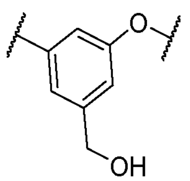, 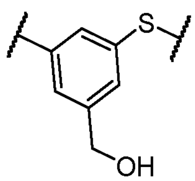, 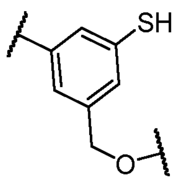, 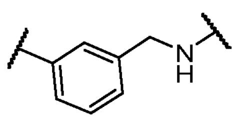, 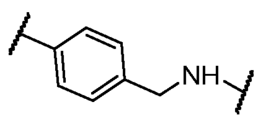,

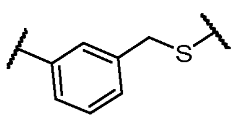, 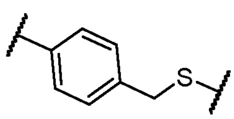, 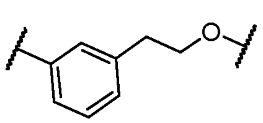, 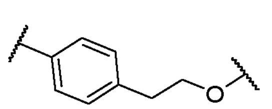,

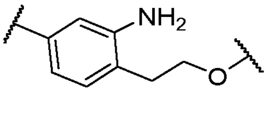, 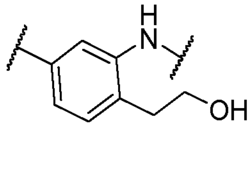, 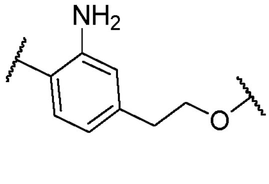 and 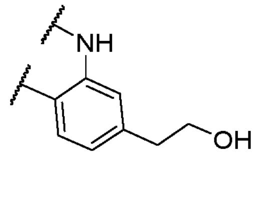.

In certain embodiments, $R_1$, $R_2$ and $R_3$ are each independently selected from the group consisting of: hydrogen, protium, deuterium, tritium, halogen, $-NO_2$, -CN, =O, =S, optionally substituted -S(=O)H, optionally substituted $-S(=O)_2H$, optionally substituted -C(=O)H, optionally substituted -OH, optionally substituted -SH, optionally substituted $-PH_2$, optionally substituted $-P(=O)H_2$, optionally substituted $-NH_2$, optionally substituted alkyl, optionally substituted alkenyl, optionally substituted alkynyl, optionally substituted cycloalkyl, optionally substituted heterocycloalkyl, optionally substituted aryl and optionally substituted heteroaryl; wherein, when $R_1$, $R_2$ and $R_3$ comprise a methylene unit, the methylene units of $R_1$, $R_2$ and $R_3$ are each independently unreplaced, or the methylene units of $R_1$, $R_2$ and $R_3$ are each independently replaced by a group selected from the group consisting of: -S(=O)-, $-S(=O)_2-$, -O-, -S-, -C(=O)-, optionally substituted -PH-, optionally substituted -P(=O)H-, optionally substituted -NH-, optionally substituted alkylene, optionally substituted alkenylene, optionally substituted alkynylene, optionally substituted cycloalkylene, optionally substituted heterocycloalkylene, optionally substituted arylene and optionally substituted heteroarylene.

In certain embodiments, $R_1$ and $R_2$ are each independently selected from the group consisting of: H, F, Cl, Br and optionally substituted $C_1$-$C_6$ alkyl.

In certain embodiments, $R_1$ and $R_2$ are each independently selected from the group consisting of: H, F, Cl, Br and optionally substituted methyl.

In certain embodiments, $R_3$ is selected from the group consisting of: hydrogen, optionally substituted -OH, optionally substituted -SH and optionally substituted $C_1$-$C_6$ alkyl.

In certain embodiments, when $R_3$ is optionally substituted methyl, $R_3$ is substituted with $R_{31}$, and $R_{31}$ is selected from the group consisting of: hydrogen, protium, deuterium, tritium, halogen, $-NO_2$, -CN, =O, =S, optionally substituted -S(=O)H, optionally substituted $-S(=O)_2H$, optionally substituted - C(=O)H, optionally substituted -OH, optionally substituted -SH, optionally substituted $-PH_2$, optionally substituted $-P(=O)H_2$, optionally substituted $-NH_2$, optionally substituted alkyl, optionally substituted alkenyl, optionally substituted alkynyl, optionally substituted cycloalkyl, optionally substituted heterocyclyl, optionally substituted aryl and optionally substituted heteroaryl; wherein, when $R_{31}$ comprises a methylene unit, the methylene unit of $R_{31}$ is each independently unreplaced, or the methylene unit of $R_{31}$ is each independently replaced by a group selected from the group consisting of: -S(=O)-, $-S(=O)_2-$, -O-, -S-, -C(=O)-, optionally substituted -PH-, optionally substituted -P(=O)H-, optionally substituted -NH-, optionally substituted alkylene, optionally substituted alkenylene, optionally substituted alkynylene, optionally substituted cycloalkylene, optionally substituted heterocyclylene, optionally substituted arylene and optionally substituted heteroarylene. In certain embodiments, $R_{31}$ is selected from the group consisting of: hydrogen, halogen, optionally substituted -OH and optionally substituted -SH.

In certain embodiments, when $R_{31}$ is optionally substituted -OH, $R_{31}$ is substituted with $R_{311}$, and $R_{311}$ is selected from the group consisting of: hydrogen, protium, deuterium, tritium, halogen, $-NO_2$, -CN, =O, =S, optionally substituted -S(=O)H, optionally substituted $-S(=O)_2H$, optionally substituted - C(=O)H, optionally substituted -OH, optionally substituted -SH, optionally substituted $-PH_2$, optionally substituted $-P(=O)H_2$, optionally substituted $-NH_2$, optionally substituted alkyl, optionally substituted alkenyl, optionally substituted alkynyl, optionally substituted cycloalkyl, optionally substituted heterocyclyl, optionally substituted aryl and optionally substituted heteroaryl; wherein, when $R_{311}$ comprises a methylene unit, the methylene unit of $R_{311}$ is each independently unreplaced, or the methylene unit of $R_{311}$ is each independently replaced by a group selected from the group consisting of: -S(=O)-, $-S(=O)_2-$, -O-, -S-, -C(=O)-, optionally substituted -PH-, optionally substituted -P(=O)H-, optionally substituted -NH-, optionally substituted alkylene, optionally substituted alkenylene, optionally substituted alkynylene, optionally substituted cycloalkylene, optionally substituted heterocyclylene, optionally substituted arylene and optionally substituted heteroarylene. In certain embodiments, $R_{311}$ is selected from the group consisting of: hydrogen, optionally substituted $-P(=O)H_2$, optionally substituted -S(=O)H, optionally substituted $-S(=O)_2H$, optionally substituted $C_1$-$C_6$ alkyl and optionally substituted $C_2$-$C_9$ heterocyclylene; wherein, when $R_{311}$ comprises a methylene unit, the methylene unit of $R_{311}$ is each independently unreplaced, or the methylene unit of $R_{311}$ is each independently replaced by a group selected from the group consisting of: -S(=O)-, $-S(=O)_2-$, -O-, -S-, -C(=O)-, optionally substituted -PH-, optionally substituted - $P(=O)H_2-$ and optionally substituted -NH-; wherein substituents are selected from the group consisting of: protium, deuterium, tritium, halogen, -CN, =O, =N-OH, =N-OR, =N-R, -OR, -C(O)R, -C(O)OR, -OC(O)R, -OC(O)OR, -C(O)NHR, $-C(O)NR_2$, -OC(O)NHR, $-OC(O)NR_2$, -SR, -S(O)R, - $S(O)_2R$, -NHR, $-N(R)_2$, $-N^+(R)_3$, -PHR, $-P(R)_2$, $-P(=O)R_2$, $-OP(=O)R_2$, -NHC(O)R, -NRC(O)R, - NHC(O)OR, -NRC(O)OR, $-S(O)_2NHR$, $-S(O)_2N(R)_2$, $-NHS(O)_2NR_2$, $-NRS(O)_2NR_2$, $-NHS(O)_2R$, - $NRS(O)_2R$, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy, wherein each R is independently selected from the group consisting of hydrogen, protium, deuterium, tritium, oxygen, hydroxy, halogen, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy.

In certain embodiments, $R_{311}$ is selected from the group consisting of: hydrogen, optionally substituted

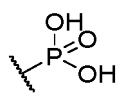

,

optionally substituted

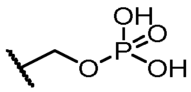,

optionally substituted

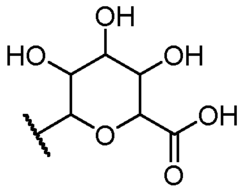,

optionally substituted

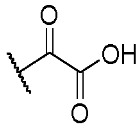,

optionally substituted

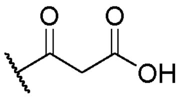,

optionally substituted

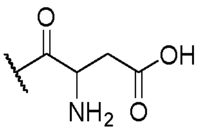,

optionally substituted

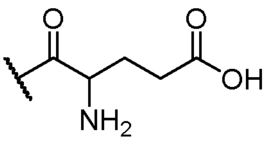,

optionally substituted

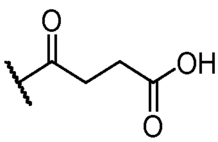,

optionally substituted

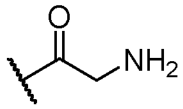

,

optionally substituted

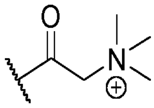

,

optionally substituted

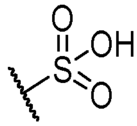

,

optionally substituted

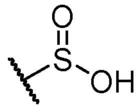

,

optionally substituted

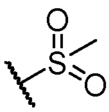

.

In certain embodiments, when $R_3$ is optionally substituted -OH, $R_3$ is substituted with $R_{32}$, and $R_{32}$ is selected from the group consisting of: hydrogen, protium, deuterium, tritium, halogen, $-NO_2$, -CN, =O, =S, optionally substituted -S(=O)H, optionally substituted $-S(=O)_2H$, optionally substituted - C(=O)H, optionally substituted -OH, optionally substituted -SH, optionally substituted $-PH_2$, optionally substituted $-P(=O)H_2$, optionally substituted $-NH_2$, optionally substituted alkyl, optionally substituted alkenyl, optionally substituted alkynyl, optionally substituted cycloalkyl, optionally substituted heterocyclyl, optionally substituted aryl and optionally substituted heteroaryl; wherein, when $R_{32}$ comprises a methylene unit, the methylene unit of $R_{32}$ is each independently unreplaced, or the methylene unit of $R_{32}$ is each independently replaced by a group selected from the group consisting of: -S(=O)-, $-S(=O)_2-$, -O-, -S-, -C(=O)-, optionally substituted -PH-, optionally substituted -P(=O)H-, optionally substituted -NH-, optionally substituted alkylene, optionally substituted alkenylene, optionally substituted alkynylene, optionally substituted cycloalkylene, optionally substituted heterocyclylene, optionally substituted arylene and optionally substituted heteroarylene. In certain embodiments, $R_{32}$ is selected from the group consisting of: hydrogen and optionally substituted $C_1$-$C_6$ alkyl.

In certain embodiments, when $R_{32}$ is optionally substituted methyl or optionally substituted ethyl, $R_{32}$ is substituted with $R_{321}$, and $R_{321}$ is selected from the group consisting of: hydrogen, protium, deuterium, tritium, halogen, $-NO_2$, -CN, =O, =S, optionally substituted -S(=O)H, optionally substituted $-S(=O)_2H$, optionally substituted -C(=O)H, optionally substituted -OH, optionally substituted -SH, optionally substituted $-PH_2$, optionally substituted $-P(=O)H_2$, optionally substituted $-NH_2$, optionally substituted alkyl, optionally substituted alkenyl, optionally substituted alkynyl, optionally substituted cycloalkyl, optionally substituted heterocyclyl, optionally substituted aryl and optionally substituted heteroaryl; wherein, when $R_{321}$ comprises a methylene unit, the methylene unit of $R_{321}$ is each independently unreplaced, or the methylene unit of $R_{321}$ is each independently replaced by a group selected from the group consisting of: -S(=O)-, $-S(=O)_2-$, -O-, -S-, -C(=O)-, optionally substituted -PH-, optionally substituted -P(=O)H-, optionally substituted -NH-, optionally substituted alkylene, optionally substituted alkenylene, optionally substituted alkynylene, optionally substituted cycloalkylene, optionally substituted heterocyclylene, optionally substituted arylene and optionally substituted heteroarylene.

In certain embodiments, $R_{321}$ is selected from the group consisting of: hydrogen, halogen, -CN and optionally substituted $C_1$-$C_6$ alkyl.

In certain embodiments, $R_{321}$ is selected from the group consisting of: hydrogen, F, Cl, -CN and optionally substituted methyl.

In certain embodiments, when $R_3$ is optionally substituted -SH, $R_3$ is substituted with $R_{33}$, and $R_{33}$ is selected from the group consisting of: hydrogen, protium, deuterium, tritium, halogen, $-NO_2$, -CN, =O, =S, optionally substituted -S(=O)H, optionally substituted $-S(=O)_2H$, optionally substituted - C(=O)H, optionally substituted -OH, optionally substituted -SH, optionally substituted $-PH_2$, optionally substituted $-P(=O)H_2$, optionally substituted $-NH_2$, optionally substituted alkyl, optionally substituted alkenyl, optionally substituted alkynyl, optionally substituted cycloalkyl, optionally substituted heterocyclyl, optionally substituted aryl and optionally substituted heteroaryl; wherein, when $R_{33}$ comprises a methylene unit, the methylene unit of $R_{33}$ is each independently unreplaced, or the methylene unit of $R_{33}$ is each independently replaced by a group selected from the group consisting of: -S(=O)-, $-S(=O)_2-$, -O-, -S-, -C(=O)-, optionally substituted -PH-, optionally substituted -P(=O)H-, optionally substituted -NH-, optionally substituted alkylene, optionally substituted alkenylene, optionally substituted alkynylene, optionally substituted cycloalkylene, optionally substituted heterocyclylene, optionally substituted arylene and optionally substituted heteroarylene. In certain embodiments, $R_{33}$ is selected from the group consisting of: hydrogen and optionally substituted $C_1$-$C_6$ alkyl.

In certain embodiments, when $R_{33}$ is optionally substituted methyl or optionally substituted ethyl, $R_{33}$ is substituted with $R_{331}$, and $R_{331}$ is selected from the group consisting of: hydrogen, protium, deuterium, tritium, halogen, $-NO_2$, -CN, =O, =S, optionally substituted -S(=O)H, optionally substituted $-S(=O)_2H$, optionally substituted -C(=O)H, optionally substituted -OH, optionally substituted -SH, optionally substituted $-PH_2$, optionally substituted $-P(=O)H_2$, optionally substituted $-NH_2$, optionally substituted alkyl, optionally substituted alkenyl, optionally substituted alkynyl, optionally substituted cycloalkyl, optionally substituted heterocyclyl, optionally substituted aryl and optionally substituted heteroaryl; wherein, when $R_{331}$ comprises a methylene unit, the methylene unit of $R_{331}$ is each independently unreplaced, or the methylene unit of $R_{331}$ is each independently replaced by a group selected from the group consisting of: -S(=O)-, $-S(=O)_2-$, -O-, -S-, -C(=O)-, optionally substituted -PH-, optionally substituted -P(=O)H-, optionally substituted -NH-, optionally substituted alkylene, optionally substituted alkenylene, optionally substituted alkynylene, optionally substituted cycloalkylene, optionally substituted heterocyclylene, optionally substituted arylene and optionally substituted heteroarylene.

In certain embodiments, $R_{331}$ is selected from the group consisting of: hydrogen, chlorine, fluorine and -CN.

For example, $R_3$ may be selected from the group consisting of: optionally substituted $-CH_2Cl$, optionally substituted $-CH_2SH$, optionally substituted $-CH_2OH$, optionally substituted

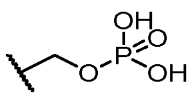

,

optionally substituted

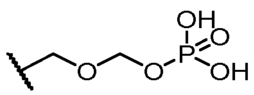

,

optionally substituted

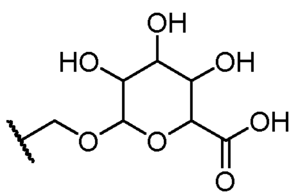

,

optionally substituted

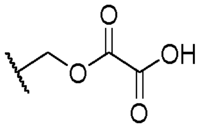

,

optionally substituted

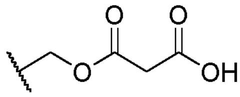

,

optionally substituted

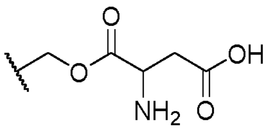

,

optionally substituted

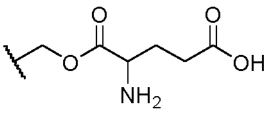

,

optionally substituted

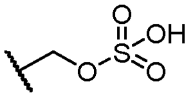

,

optionally substituted

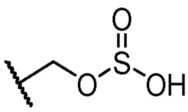

,

optionally substituted

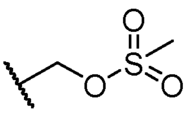

,

optionally substituted -OH, optionally substituted $-OCH_3$, optionally substituted $-OCH_2F$, optionally substituted $-OCH_2Cl$, optionally substituted $-OCH_2CN$, optionally substituted $-OCH_2CH_3$, optionally substituted sulfhydryl, optionally substituted $-SCH_2F$, optionally substituted $-SCH_2Cl$, optionally substituted $-SCH_2CF_3$ and optionally substituted - $SCH_2CN$.

In certain embodiments, provided is the compound or the tautomer, the mesomer, the racemate, the enantiomer or the diastereoisomer thereof, or the mixture thereof, or the pharmaceutically acceptable salt thereof described herein, wherein $R_3$ is selected from the group consisting of: $-CH_2Cl$, $-CH_2SH$, $-CH_2OH$,

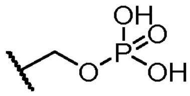,

$-OCH_3$, $-OCH_2F$, $-OCH_2Cl$, $-OCH_2CN$, $-OCH_2CH_3$, -SH, $-SCH_2F$, - $SCH_2Cl$, $-SCH_2CF_3$ and $-SCH_2CN$.

In certain embodiments, $R_1$ and $R_2$ are each independently selected from the group consisting of hydrogen, protium, deuterium, tritium and halogen, and $R_3$ is selected from the group consisting of $CH_2OH$, $-SCH_2F$,

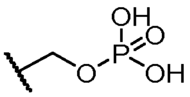, 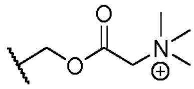, 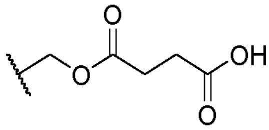, 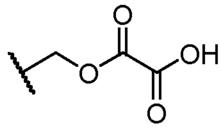,

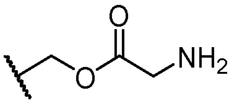, 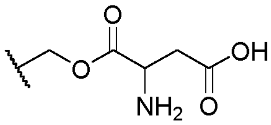, 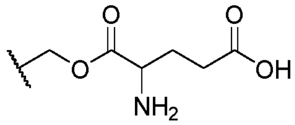, 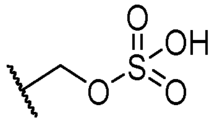,

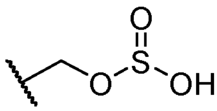 and 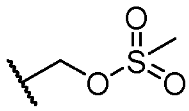.

In certain embodiments, B is absent or selected from the group consisting of: optionally substituted $C_1$-$C_6$ alkyl, optionally substituted $C_3$-$C_8$ cycloalkyl, optionally substituted $C_3$-$C_8$ heterocyclyl, optionally substituted $C_6$-$C_{10}$ aryl and optionally substituted $C_5$-$C_{10}$ heteroaryl, wherein substituents are selected from the group consisting of: protium, deuterium, tritium, halogen, -CN, =O, =N-OH, =N-OR, =N-R, -OR, -C(O)R, -C(O)OR, -OC(O)R, -OC(O)OR, -C(O)NHR, $-C(O)NR_2$, -OC(O)NHR, $-OC(O)NR_2$, -SR, -S(O)R, $-S(O)_2R$, -NHR, $-N(R)_2$, -NHC(O)R, -NRC(O)R, -NHC(O)OR, - NRC(O)OR, $-S(O)_2NHR$, $-S(O)_2N(R)_2$, $-NHS(O)_2NR_2$, $-NRS(O)_2NR_2$, $-NHS(O)_2R$, $-NRS(O)_2R$, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy; wherein each R is independently selected from the group consisting of hydrogen, protium, deuterium, tritium, halogen, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy.

In certain embodiments, B is selected from the group consisting of: optionally substituted

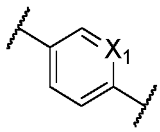,

optionally substituted

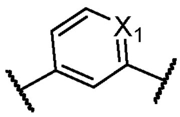

and optionally substituted

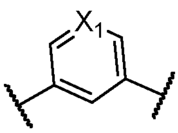;

and $X_1$ is selected from the group consisting of: N and optionally substituted CH.

In certain embodiments, when $X_1$ is optionally substituted CH, $X_1$ is substituted with $R_6$, and $R_6$ is selected from the group consisting of: hydrogen, protium, deuterium, tritium, halogen, $-NO_2$, -CN, =O, =S, optionally substituted -S(=O)H, optionally substituted $-S(=O)_2H$, optionally substituted - C(=O)H, optionally substituted -OH, optionally substituted -SH, optionally substituted $-PH_2$, optionally substituted $-P(=O)H_2$, optionally substituted $-NH_2$, optionally substituted alkyl, optionally substituted alkenyl, optionally substituted alkynyl, optionally substituted cycloalkyl, optionally substituted heterocyclyl, optionally substituted aryl and optionally substituted heteroaryl; wherein, when $R_6$ comprises a methylene unit, the methylene unit of $R_6$ is each independently unreplaced, or the methylene unit of $R_6$ is each independently replaced by a group selected from the group consisting of: -S(=O)-, $-S(=O)_2-$, -O-, -S-, -C(=O)-, optionally substituted -PH-, optionally substituted -P(=O)H-, optionally substituted -NH-, optionally substituted alkylene, optionally substituted alkenylene, optionally substituted alkynylene, optionally substituted cycloalkylene, optionally substituted heterocyclylene, optionally substituted arylene and optionally substituted heteroarylene.

In certain embodiments, $R_6$ is selected from the group consisting of: hydrogen and optionally substituted -OH.

In certain embodiments, when $R_6$ is optionally substituted -OH, $R_6$ is substituted with $R_{61}$, and $R_{61}$ is selected from the group consisting of: hydrogen, protium, deuterium, tritium, halogen, $-NO_2$, -CN, =O, =S, optionally substituted -S(=O)H, optionally substituted $-S(=O)_2H$, optionally substituted - C(=O)H, optionally substituted -OH, optionally substituted -SH, optionally substituted $-PH_2$, optionally substituted $-P(=O)H_2$, optionally substituted $-NH_2$, optionally substituted alkyl, optionally substituted alkenyl, optionally substituted alkynyl, optionally substituted cycloalkyl, optionally substituted heterocyclyl, optionally substituted aryl and optionally substituted heteroaryl; wherein, when $R_{61}$ comprises a methylene unit, the methylene unit of $R_{61}$ is each independently unreplaced, or the methylene unit of $R_{61}$ is each independently replaced by a group selected from the group consisting of: -S(=O)-, $-S(=O)_2-$, -O-, -S-, -C(=O)-, optionally substituted -PH-, optionally substituted -P(=O)H-, optionally substituted -NH-, optionally substituted alkylene, optionally substituted alkenylene, optionally substituted alkynylene, optionally substituted cycloalkylene, optionally substituted heterocyclylene, optionally substituted arylene and optionally substituted heteroarylene. In certain embodiments, $R_{61}$ is selected from the group consisting of: H and optionally substituted $C_1$-$C_6$ alkyl.

In certain embodiments, $X_1$ is selected from the group consisting of: CH. C(-O-$CH_3$) and N.

In certain embodiments, B may be selected from the group consisting of: optionally substituted

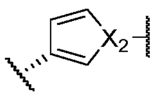

,

optionally substituted

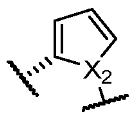

,

optionally substituted

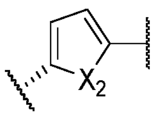

,

optionally substituted

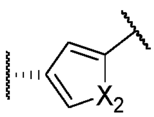

,

optionally substituted

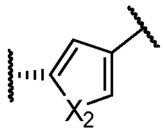,

optionally substituted

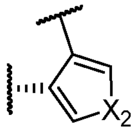,

optionally substituted

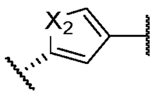,

optionally substituted

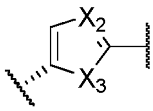,

optionally substituted

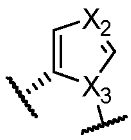,

optionally substituted

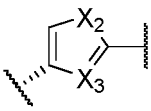,

optionally substituted

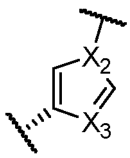,

optionally substituted

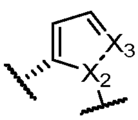

and optionally substituted

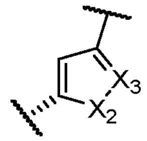
;

and $X_2$ and $X_3$ are each independently selected from the group consisting of: O, S and optionally substituted NH.

In certain embodiments, when $X_2$ and $X_3$ are optionally substituted NH, $X_2$ or $X_3$ is substituted with $R_7$, and $R_7$ is selected from the group consisting of: hydrogen, protium, deuterium, tritium, halogen, $-NO_2$, -CN, =O, =S, optionally substituted -S(=O)H, optionally substituted $-S(=O)_2H$, optionally substituted -C(=O)H, optionally substituted -OH, optionally substituted -SH, optionally substituted - $PH_2$, optionally substituted $-P(=O)H_2$, optionally substituted $-NH_2$, optionally substituted alkyl, optionally substituted alkenyl, optionally substituted alkynyl, optionally substituted cycloalkyl, optionally substituted heterocyclyl, optionally substituted aryl and optionally substituted heteroaryl; wherein, when $R_7$ comprises a methylene unit, the methylene unit of $R_7$ is each independently unreplaced, or the methylene unit of $R_7$ is each independently replaced by a group selected from the group consisting of: -S(=O)-, $-S(=O)_2-$, -O-, -S-, -C(=O)-, optionally substituted -PH-, optionally substituted -P(=O)H-, optionally substituted -NH-, optionally substituted alkylene, optionally substituted alkenylene, optionally substituted alkynylene, optionally substituted cycloalkylene, optionally substituted heterocyclylene, optionally substituted arylene and optionally substituted heteroarylene.

In certain embodiments, $R_7$ is selected from the group consisting of: hydrogen and optionally substituted $C_1$-$C_6$ alkyl.

In certain embodiments, when $R_7$ is optionally substituted $C_1$-$C_6$ alkyl, $R_7$ is substituted with $R_{71}$, and $R_{71}$ is selected from the group consisting of: hydrogen, protium, deuterium, tritium, halogen, $-NO_2$, - CN, =O, =S, optionally substituted -S(=O)H, optionally substituted $-S(=O)_2H$, optionally substituted -C(=O)H, optionally substituted -OH, optionally substituted -SH, optionally substituted $-PH_2$, optionally substituted $-P(=O)H_2$, optionally substituted $-NH_2$, optionally substituted alkyl, optionally substituted alkenyl, optionally substituted alkynyl, optionally substituted cycloalkyl, optionally substituted heterocyclyl, optionally substituted aryl and optionally substituted heteroaryl; wherein, when $R_{71}$ comprises a methylene unit, the methylene unit of $R_{71}$ is each independently unreplaced, or the methylene unit of $R_{71}$ is each independently replaced by a group selected from the group consisting of: -S(=O)-, $-S(=O)_2-$, -O-, -S-, -C(=O)-, optionally substituted -PH-, optionally substituted -P(=O)H-, optionally substituted -NH-, optionally substituted alkylene, optionally substituted alkenylene, optionally substituted alkynylene, optionally substituted cycloalkylene, optionally substituted heterocyclylene, optionally substituted arylene and optionally substituted heteroarylene. In certain embodiments, $R_{71}$ is selected from the group consisting of: H and optionally substituted $C_1$-$C_6$ alkyl.

In certain embodiments, $X_2$ or $X_3$ is selected from the group consisting of: NH, $N(CH_3)$, O and S.

In certain embodiments, B is selected from the group consisting of: optionally substituted

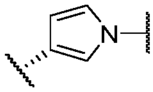
,

optionally substituted

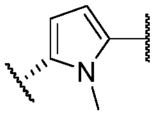
,

optionally substituted

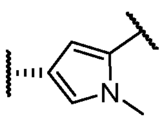
,

optionally substituted

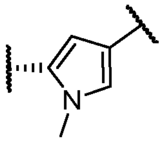,

optionally substituted

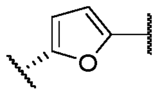,

optionally substituted

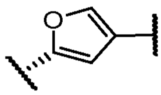,

optionally substituted

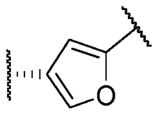,

optionally substituted

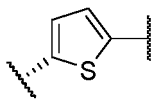,

optionally substituted

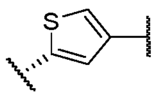,

optionally substituted

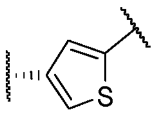,

optionally substituted

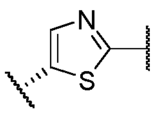,

optionally substituted

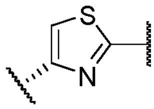
,

optionally substituted

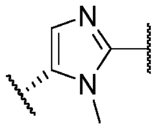
,

optionally substituted

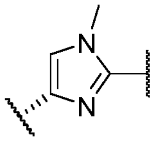
,

optionally substituted

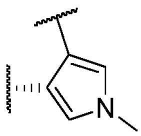
,

optionally substituted

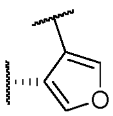
,

optionally substituted

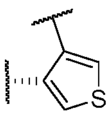
,

optionally substituted

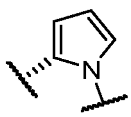
,

optionally substituted

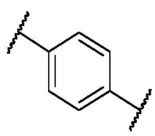

and optionally substituted

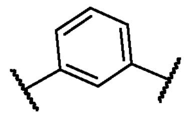,

wherein substituents are selected from the group consisting of: protium, deuterium, tritium, halogen, -CN, =O, =N-OH, =N-OR, =N-R, -OR, - C(O)R, -C(O)OR, -OC(O)R, -OC(O)OR, -C(O)NHR, -C(O)$NR_2$, -OC(O)NHR, -OC(O)$NR_2$, -SR, - S(O)R, -S$(O)_2$R, -NHR, -N$(R)_2$, -NHC(O)R, -NRC(O)R, -NHC(O)OR, -NRC(O)OR, -S$(O)_2$NHR, - S$(O)_2$N$(R)_2$, -NHS$(O)_2$$NR_2$, -NRS$(O)_2$$NR_2$, -NHS$(O)_2$R, -NRS$(O)_2$R, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy, wherein each R is independently selected from the group consisting of hydrogen, protium, deuterium, tritium, oxygen, halogen, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy.

In certain embodiments, provided is the compound or the tautomer, the mesomer, the racemate, the enantiomer or the diastereoisomer thereof, or the mixture thereof, or the pharmaceutically acceptable salt thereof of the present invention, wherein B is selected from the group consisting of:

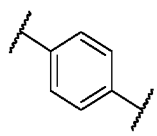,

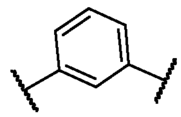 and 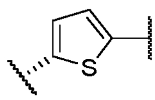.

In certain embodiments, W is absent or W is selected from the group consisting of: -S(=O)-, -S$(=O)_2$-, -O-, -S-, -NHC(=O)-, -C(=O)NH-, optionally substituted -PH-, optionally substituted -P(=O)H-, optionally substituted -NH-, optionally substituted alkylene, optionally substituted alkenylene, optionally substituted alkynylene, optionally substituted cycloalkylene, optionally substituted heterocycloalkylene, optionally substituted arylene and optionally substituted heteroarylene; wherein, when W comprises a methylene unit, the methylene unit of W is each independently unreplaced, or the methylene unit of W is each independently replaced by a group selected from the group consisting of: -S(=O)-, -S$(=O)_2$-, -O-, -S-, -C(=O)-, optionally substituted -PH-, optionally substituted -P(=O)H-, optionally substituted -NH-, optionally substituted alkylene, optionally substituted alkenylene, optionally substituted alkynylene, optionally substituted cycloalkylene, optionally substituted heterocyclylene, optionally substituted arylene and optionally substituted heteroarylene.

In certain embodiments, when W is optionally substituted $C_1$-$C_6$ alkylene, W is substituted with $R_8$, and $R_8$ is selected from the group consisting of: hydrogen, protium, deuterium, tritium, halogen, - $NO_2$, -CN, =O, =S, optionally substituted -S(=O)H, optionally substituted -S$(=O)_2$H, optionally substituted -C(=O)H, optionally substituted -OH, optionally substituted -SH, optionally substituted - $PH_2$, optionally substituted -P(=O)$H_2$, optionally substituted -$NH_2$, optionally substituted alkyl, optionally substituted alkenyl, optionally substituted alkynyl, optionally substituted cycloalkyl, optionally substituted heterocyclyl, optionally substituted aryl and optionally substituted heteroaryl; wherein, when $R_8$ comprises a methylene unit, the methylene unit of $R_8$ is each independently unreplaced, or the methylene unit of $R_8$ is each independently replaced by a group selected from the group consisting of: -S(=O)-, -S$(=O)_2$-, -O-, -S-, -C(=O)-, optionally substituted -PH-, optionally substituted -P(=O)H-, optionally substituted -NH-, optionally substituted alkylene, optionally substituted alkenylene, optionally substituted alkynylene, optionally substituted cycloalkylene, optionally substituted heterocyclylene, optionally substituted arylene and optionally substituted heteroarylene.

In certain embodiments, $R_8$ is selected from the group consisting of: hydrogen, =O, optionally substituted $C_1$-$C_6$ alkyl and optionally substituted cycloalkyl.

In certain embodiments, $R_8$ is optionally substituted methyl.

In certain embodiments, when $R_8$ is optionally substituted methyl, $R_8$ is substituted with $R_{81}$, and $R_{81}$ is selected from the group consisting of: hydrogen, protium, deuterium, tritium, halogen, -$NO_2$, -CN, =O, =S, optionally substituted -S(=O)H, optionally substituted -S$(=O)_2$H, optionally substituted - C(=O)H, optionally substituted -OH, optionally substituted -SH, optionally substituted -$PH_2$, optionally substituted -P(=O)$H_2$, optionally substituted -$NH_2$, optionally substituted

alkyl, optionally substituted alkenyl, optionally substituted alkynyl, optionally substituted cycloalkyl, optionally substituted heterocycloalkyl, optionally substituted aryl and optionally substituted heteroaryl.

In certain embodiments, $R_{81}$ is selected from the group consisting of: halogen and optionally substituted cycloalkyl.

In certain embodiments, $R_{81}$ is selected from the group consisting of: F and optionally substituted cyclopropyl.

In certain embodiments, W is absent or W is selected from the group consisting of: -S(=O)-, $-S(=O)_2-$, -O-, -S-, -C(=O)-, optionally substituted $-CH_2NHC(=O)-$, optionally substituted $-NHC(=O)CH_2-$, optionally substituted -C(=O)NH-, optionally substituted -NH-, optionally substituted -CH=CH-, - C=C-, optionally substituted $C_1$-$C_6$ alkylene, optionally substituted $-OCH_2-$, optionally substituted - $CH_2O-$, optionally substituted $-SCH_2-$, optionally substituted $-CH_2S-$, optionally substituted - NHC(=O)-, optionally substituted $-COCH_2-$, optionally substituted $-CH_2NH-$, optionally substituted $-NHCH_2-$, optionally substituted $-CH(CH_3)-$, optionally substituted

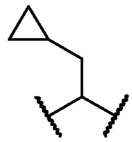,

optionally substituted

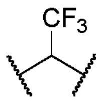,

optionally substituted

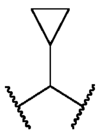,

optionally substituted

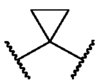

and optionally substituted

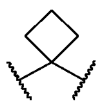.

For example, W may be absent or W is selected from the group consisting of:

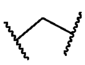, 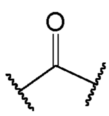, 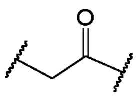, 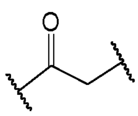, 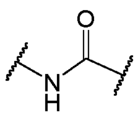, 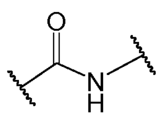, 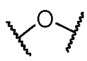, 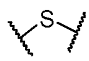, 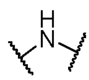,

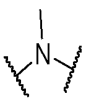, 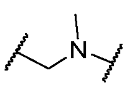, 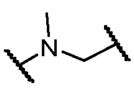, 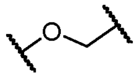, 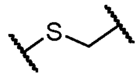, 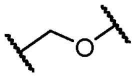, 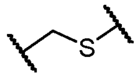, 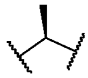,

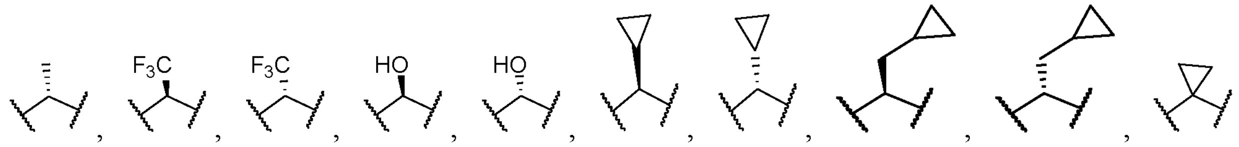 , , , , , , , , , , ,

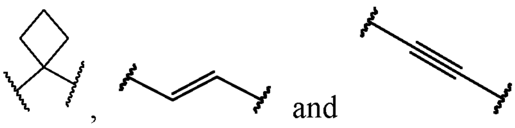 , , and .

In certain embodiments, provided is the compound or the tautomer, the mesomer, the racemate, the enantiomer or the diastereoisomer thereof, or the mixture thereof, or the pharmaceutically acceptable salt thereof of the present invention, wherein W is absent or W is

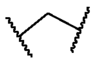 .

For example, B may be absent or selected from the group consisting of:

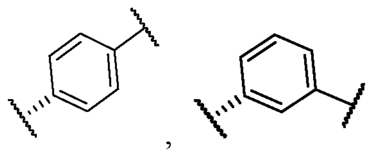 , ,

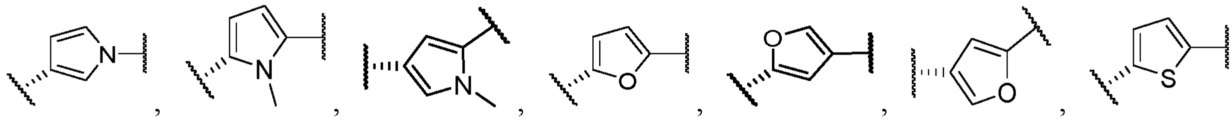 , , , , , , ,

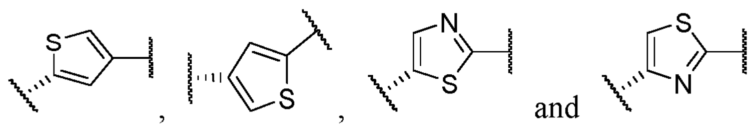 , , , and ;

W may be absent or W is selected from the group consisting of:

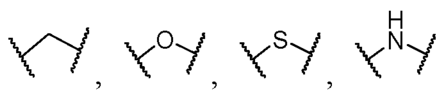 , , , ,

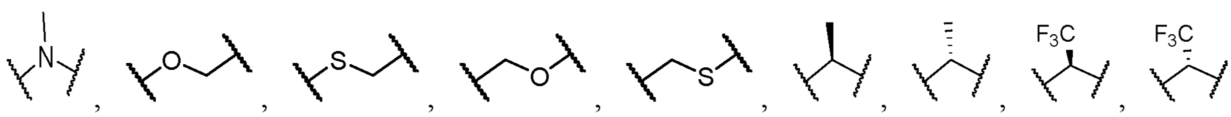 , , , , , , , , ,

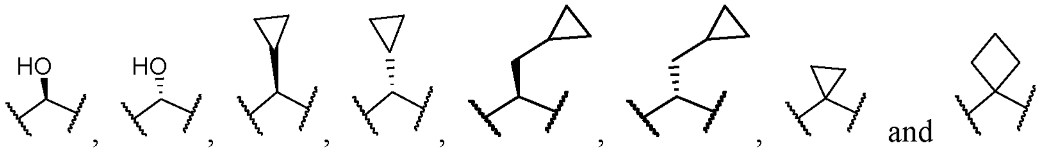 , , , , , , , and ;

A2 may be selected from the group consisting of:

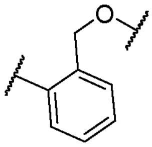, 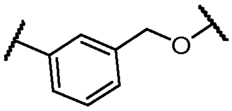, 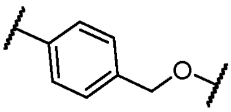, 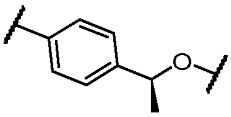, 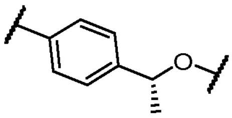,

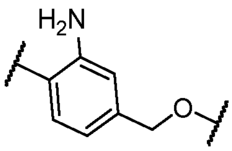, 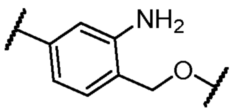, 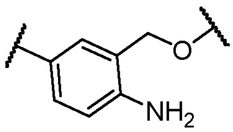, 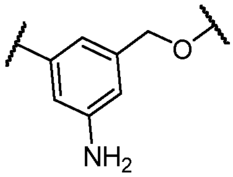, 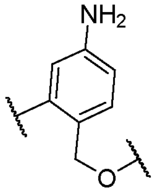,

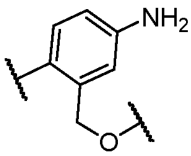, 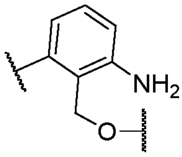, 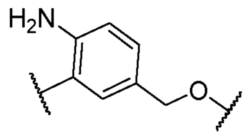, 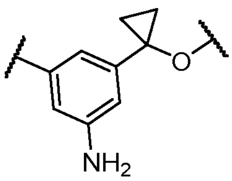, 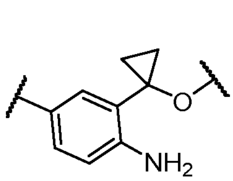,

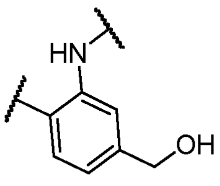, 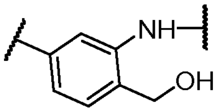, 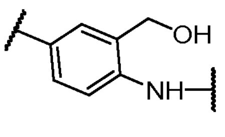, 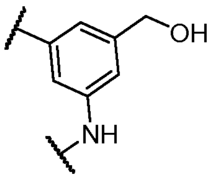, 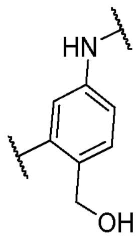,

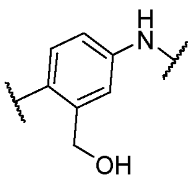, 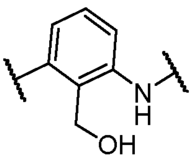, 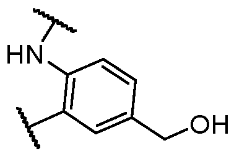, 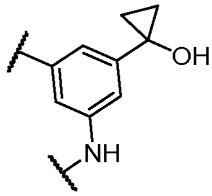, 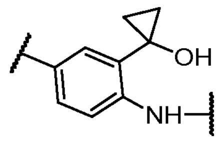,

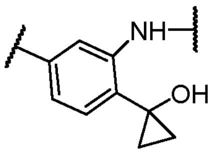, 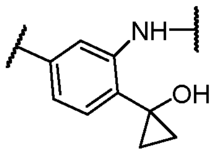, 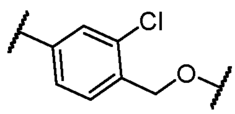, 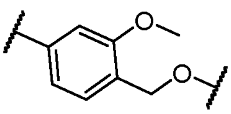, 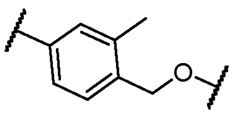,

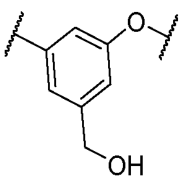, 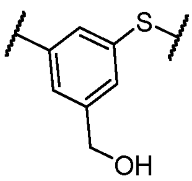, 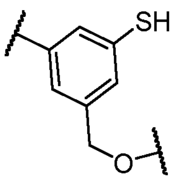, 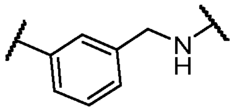, 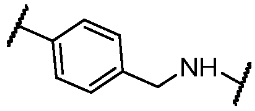,

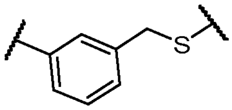, 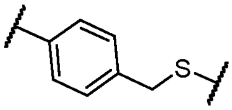, 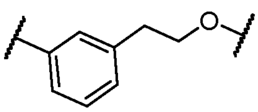, 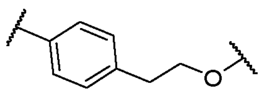,

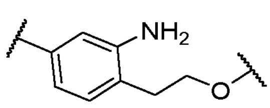, 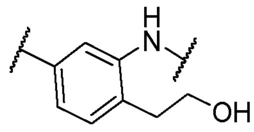, 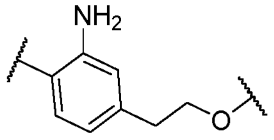 and 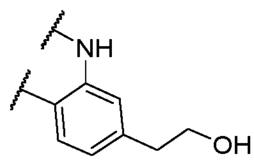.

In certain embodiments, provided is the compound or the tautomer, the mesomer, the racemate, the enantiomer or the diastereoisomer thereof, or the mixture thereof, or the pharmaceutically acceptable salt thereof of the present invention, which is selected from the group consisting of:

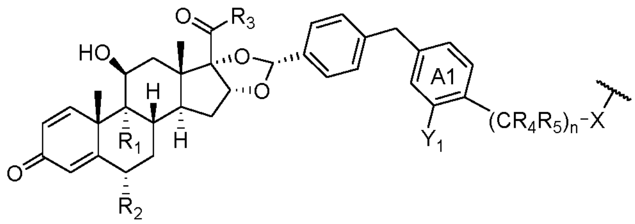

(IIa-1)

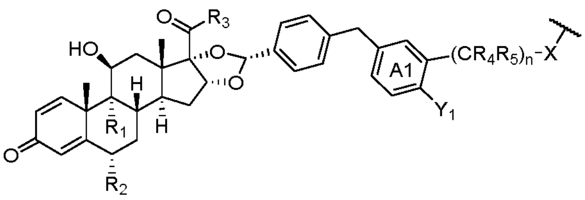

(IIb-1)

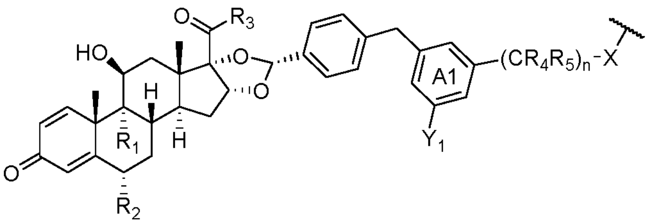

(IIc-1) ,

wherein,

X is selected from the group consisting of -O-, -S- and -NH-;
$R_4$ and $R_5$ are each independently selected from the group consisting of: H, F, Cl, -OH, $-NH_2$ and $C_1$-$C_6$ alkyl;
N is selected from the group consisting of 1, 2 and 3;
$Y_1$ is absent or selected from the group consisting of: hydroxy, sulfhydryl, amino and $C_1$-$C_6$ alkyl;
$R_1$ and $R_2$ are each independently selected from the group consisting of hydrogen, fluorine, chlorine and methyl;
$R_3$ is selected from the group consisting of: $-CH_2Cl$, $-CH_2SH$, $-CH_2OH$,

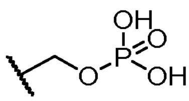,

$-OCH_3$, - $OCH_2F$, $-OCH_2Cl$, $-OCH_2CN$, $-OCH_2CH_3$, -SH, $-SCH_2F$, $-SCH_2Cl$, $-SCH_2CF_3$ and $-SCH_2CN$.

In certain embodiments, provided is the compound or the tautomer, the mesomer, the racemate, the enantiomer or the diastereoisomer thereof, or the mixture thereof, or the pharmaceutically acceptable salt thereof of the present invention, wherein the compound is selected from the group consisting of:

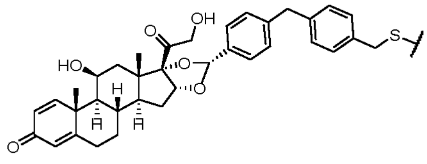
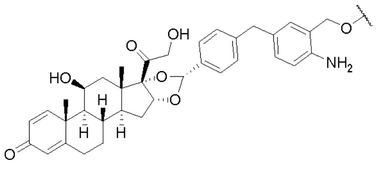
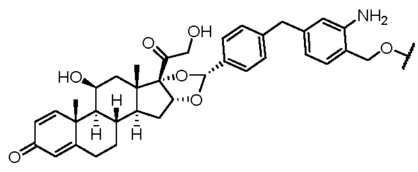
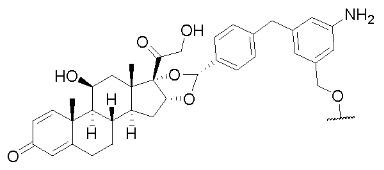
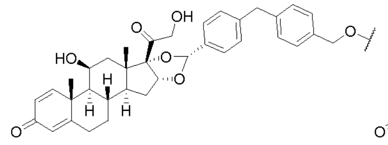
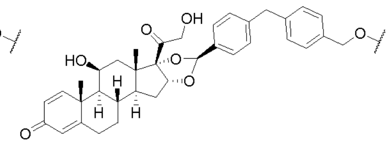
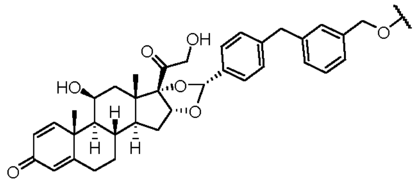
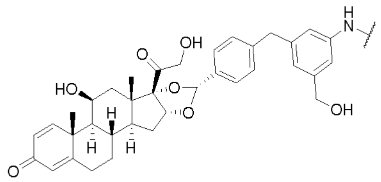
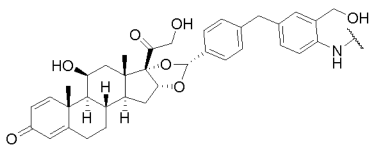
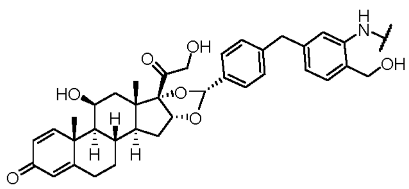

.

In certain embodiments, the compound of formula IIa or IIb or the tautomer, the mesomer, the racemate, the enantiomer or the diastereoisomer thereof, or the mixture thereof, or the pharmaceutically acceptable salt thereof further comprises a Trigger fragment (Tr), and the compound comprises the following structure:

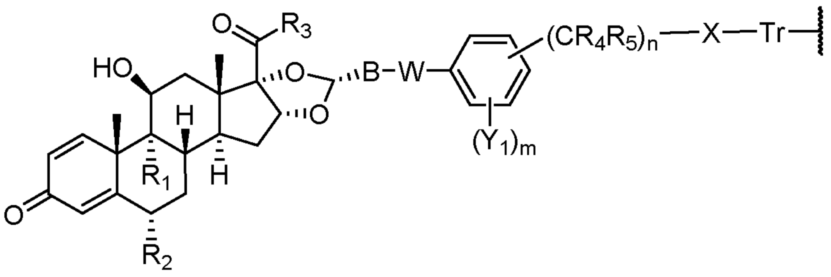

(IIIa)

or

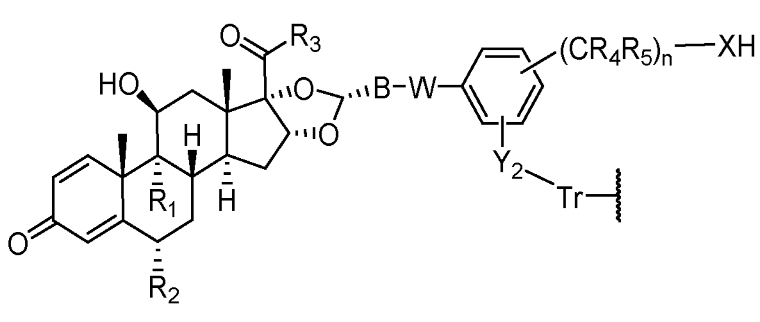

(IIIb)

wherein, Tr is selected from the group consisting of the following structures:

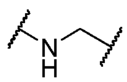 , 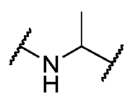 , 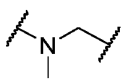 , 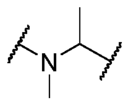 , 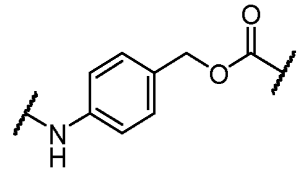 ,

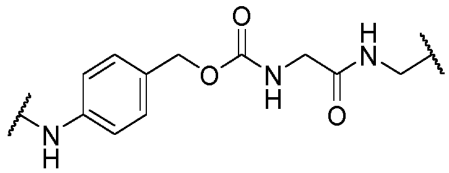 , 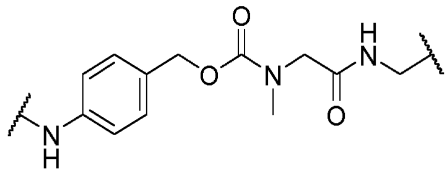 ,

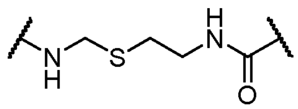 , 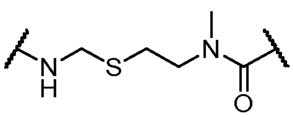 , 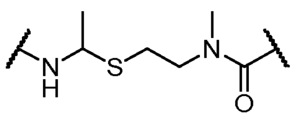 ,

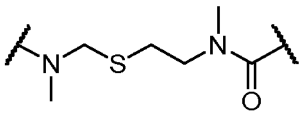 and 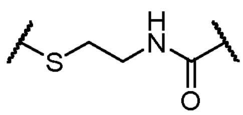 ;

$R_1$, $R_2$, $R_3$, $R_4$ and $R_5$ are each independently any group;
B is absent or B is any group;
W is absent or W is any group;
$CR_4R_5$ units are each independently unreplaced or replaced by a group selected from the group consisting of: -O-, -S-, -NR-, -S(O)-, $-S(O)_2-$, -C(=O)-, -C=C- and -C=C-, wherein $R_4$ and $R_5$ can together form optionally substituted cycloalkyl, optionally substituted heterocyclyl, optionally substituted bridged cyclyl, optionally substituted bridged heterocyclyl, optionally substituted spiro cyclyl and optionally substituted spiro heterocyclyl, and n is any integer from 1 to 20;
X is selected from the group consisting of: -O-, -S- and -NR-;
$Y_1$ is any group, and m is any integer from 0 to 4;
$Y_2$ is selected from the group consisting of -O-, -S- and -NR-;
wherein substituents are selected from the group consisting of: protium, deuterium, tritium, halogen, -CN, =O, =N-OH, =N-OR, =N-R, -OR, -C(O)R, -C(O)OR, -OC(O)R, -OC(O)OR, -C(O)NHR, - $C(O)NR_2$, -OC(O)NHR, $-OC(O)NR_2$, -SR, -S(O)R, $-S(O)_2R$, -NHR, $-N(R)_2$, -NHC(O)R, -NRC(O)R, -NHC(O)OR, -NRC(O)OR, $-S(O)_2NHR$, $-S(O)_2N(R)_2$, $-NHS(O)_2NR_2$, $-NRS(O)_2NR_2$, $-NHS(O)_2R$, - $NRS(O)_2R$, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy; wherein each R is independently selected from the group consisting of hydrogen, protium, deuterium, tritium, oxygen, halogen, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy.

For example, $R_1$ and $R_2$ may each independently be selected from the group consisting of: H, F, Cl, Br and optionally substituted methyl.

For example, $R_3$ may be selected from the group consisting of: optionally substituted $-CH_2Cl$, optionally substituted $-CH_2SH$, optionally substituted $-CH_2OH$, optionally substituted

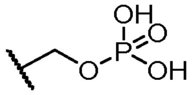 ,

optionally substituted

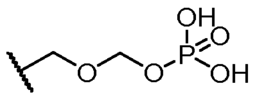,

optionally substituted

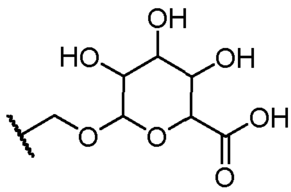,

optionally substituted

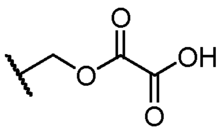,

optionally substituted

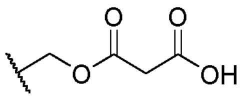,

optionally substituted

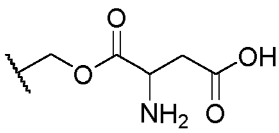,

optionally substituted

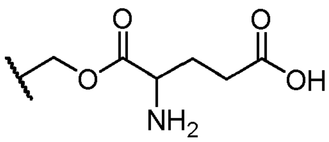,

optionally substituted

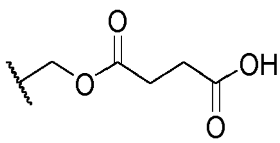,

optionally substituted

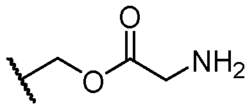

,

optionally substituted

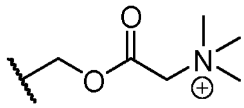

,

optionally substituted

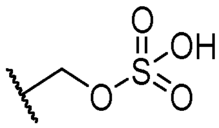

,

optionally substituted

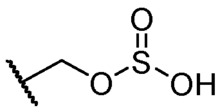

,

optionally substituted

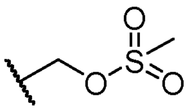

,

optionally substituted -OH, optionally substituted $-OCH_3$, optionally substituted $-OCH_2F$, optionally substituted $-OCH_2Cl$, optionally substituted $-OCH_2CN$, optionally substituted $-OCH_2CH_3$, optionally substituted sulfhydryl, optionally substituted $-SCH_2F$, optionally substituted $-SCH_2Cl$, optionally substituted $-SCH_2CF_3$ and optionally substituted $-SCH_2CN$.

For example, B may be selected from the group consisting of: optionally substituted

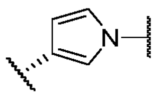

,

optionally substituted

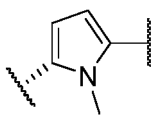

,

optionally substituted

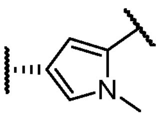

,

optionally substituted

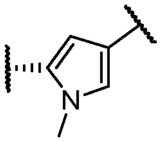,

optionally substituted

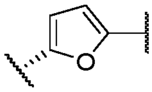,

optionally substituted

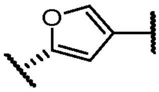,

optionally substituted

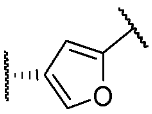,

optionally substituted

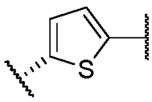,

optionally substituted

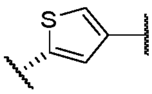,

optionally substituted

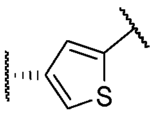,

optionally substituted

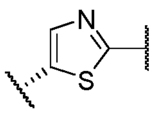,

optionally substituted

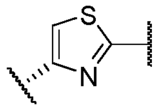
,

optionally substituted

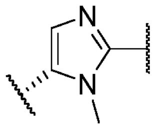
,

optionally substituted

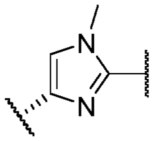
,

optionally substituted

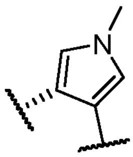
,

optionally substituted

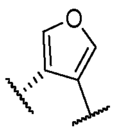
,

optionally substituted

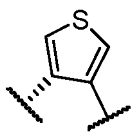
,

optionally substituted

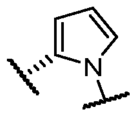
,

optionally substituted

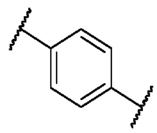

and optionally substituted

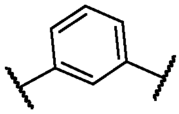,

wherein substituents are selected from the group consisting of: protium, deuterium, tritium, halogen, -CN, =O, =N-OH, =N-OR, =N-R, -OR, - C(O)R, -C(O)OR, -OC(O)R, -OC(O)OR, -C(O)NHR, $-C(O)NR_2$, -OC(O)NHR, $-OC(O)NR_2$, -SR, -S(O)R, $-S(O)_2R$, -NHR, $-N(R)_2$, -NHC(O)R, -NRC(O)R, -NHC(O)OR, -NRC(O)OR, $-S(O)_2NHR$, - $S(O)_2N(R)_2$, $-NHS(O)_2NR_2$, $-NRS(O)_2NR_2$, $-NHS(O)_2R$, $-NRS(O)_2R$, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy, wherein each R is independently selected from the group consisting of hydrogen, protium, deuterium, tritium, oxygen, halogen, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy.

For example, W may be absent or W is selected from the group consisting of:

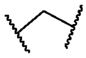, 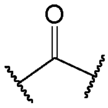, 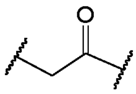, 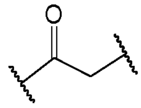, 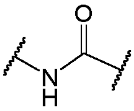, 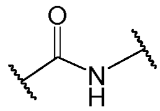, 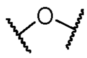, 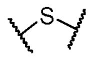, 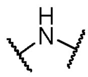,

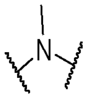, 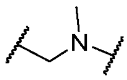, 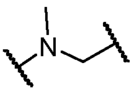, 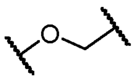, 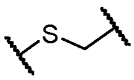, 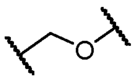, 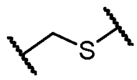, 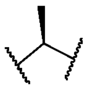,

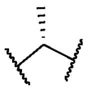, 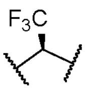, 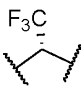, 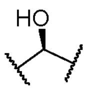, 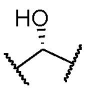, 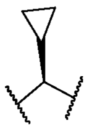, 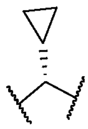, 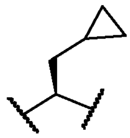, 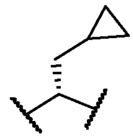, 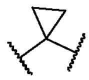,

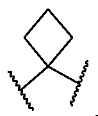, 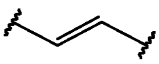 and 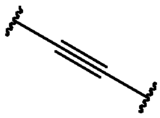.

In certain embodiments, the Linker fragment comprises an $L_1$ fragment, an $L_2$ fragment and/or an $L_3$ fragment, and the compound has the following structure:

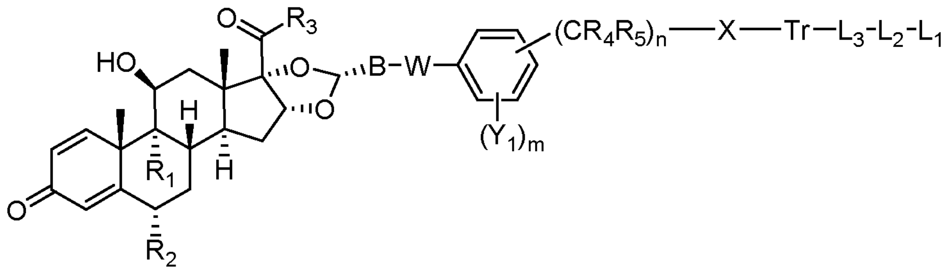

,

(IVa)

or

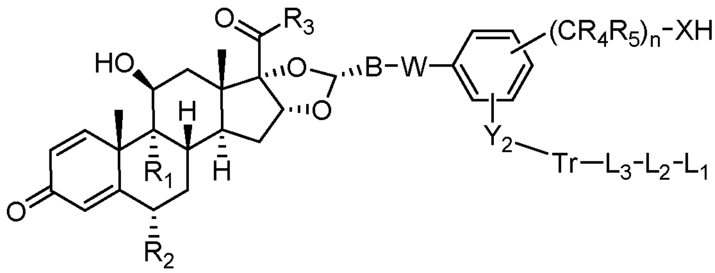

(IVb)

wherein,

Tr is absent or Tr is any group;
$L_3$ is selected from a polypeptide fragment;
$L_2$ is absent or selected from a linker fragment;
$L_1$ is selected from a coupling unit;
$R_1$, $R_2$, $R_3$, $R_4$ and $R_5$ are each independently any group;
B is absent or B is any group;
W is absent or W is any group;
$CR_4R_5$ units are each independently unreplaced or replaced by a group selected from the group consisting of: -O-, -S-, -NR-, -S(O)-, $-S(O)_2-$, -C(=O)-, -C=C- and -C=C-, wherein $R_4$ and $R_5$ can together form optionally substituted cycloalkyl, optionally substituted heterocyclyl, optionally substituted bridged cyclyl, optionally substituted bridged heterocyclyl, optionally substituted spiro cyclyl and optionally substituted spiro heterocyclyl, and n is any integer from 1 to 20;
X is selected from the group consisting of: -O-, -S- and -NR-;
$Y_1$ is any group, and m is any integer from 0 to 4;
$Y_2$ is selected from the group consisting of -O-, -S- and -NR-;
wherein substituents are selected from the group consisting of: protium, deuterium, tritium, halogen, -CN, =O, =N-OH, =N-OR, =N-R, -OR, -C(O)R, -C(O)OR, -OC(O)R, -OC(O)OR, -C(O)NHR, - $C(O)NR_2$, -OC(O)NHR, $-OC(O)NR_2$, -SR, -S(O)R, $-S(O)_2R$, -NHR, $-N(R)_2$, -NHC(O)R, -NRC(O)R, -NHC(O)OR, -NRC(O)OR, $-S(O)_2NHR$, $-S(O)_2N(R)_2$, $-NHS(O)_2NR_2$, $-NRS(O)_2NR_2$, $-NHS(O)_2R$, - $NRS(O)_2R$, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy; wherein each R is independently selected from the group consisting of hydrogen, protium, deuterium, tritium, oxygen, halogen, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy.

In certain preferred embodiments of the present invention, certain groups in the compounds of formulas (IVa) and (IVb) or pharmaceutically acceptable salts thereof are defined as follows, and groups not mentioned are as described in any one of the embodiments of the present application ("in certain embodiments" for short).
In certain embodiments, $L_3$ is selected from the group consisting of a dipeptide, a tripeptide and a tetrapeptide.
In certain embodiments, the dipeptide is selected from the group consisting of: GA, GG, AG, EG, EA, GE, DG, DA, GD, VC, VA, AA and VK.
In certain embodiments, the tripeptide is selected from the group consisting of: EAG, EGG, GEG, GEA, DAG,

DGG, GDG, GDA, GGA, GAG, GFG, AAG, AAA, VAG, VCG and VKG.

In certain embodiments, the tetrapeptide is selected from the group consisting of: GGFG, GGAG, GGGG, GEGG, GEAG, GDGG, GDAG, AAAG and EAGG.

In certain embodiments, $L_3$ is selected from the group consisting of: glycine-glycine-phenylalanine-glycine (GGFG), alanine-alanine-alanine-glycine (AAAG), glycine-glycine-glycine-glycine (GGGG), valine-alanine-glycine (VAG), valine-citrulline-glycine (VCG), alanine-alanine-glycine (AAG), alanine-alanine-alanine (AAA), valine-alanine (VA), valine-citrulline (VC), alanine-alanine (AA), glutamic acid-alanine-glycine-glycine (EAGG), glycine-glutamic acid-alanine-glycine (GEAG), glycine-glutamic acid-glycine-glycine (GEGG), glutamic acid-glycine-glycine (EGG), glutamic acid-alanine-glycine (EAG), valine-lysine-glycine (VKG), glycine-glutamic acid-glycine (GEG), glutamic acid-alanine (EA), glutamic acid-glycine (EG) and glycine-glutamic acid (GE).

In certain embodiments, $L_2$ is absent.

In certain embodiments, $L_2$ comprises or does not comprise a PEG branch chain or a PEG linear chain.

In certain embodiments, when $L_2$ does not comprise a PEG, $L_2$ is selected from the group consisting of:

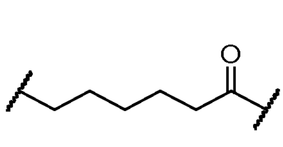,
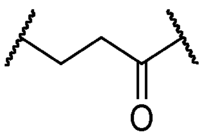 and 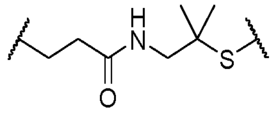.

In certain embodiments, when $L_2$ comprises the PEG linear chain, $L_2$ is selected from the group consisting of:

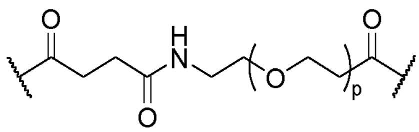, 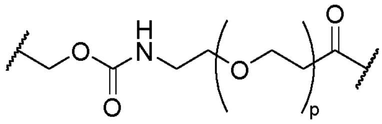, 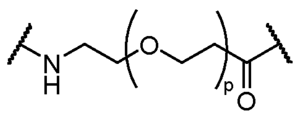,

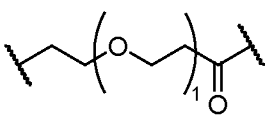, 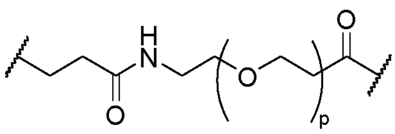

and

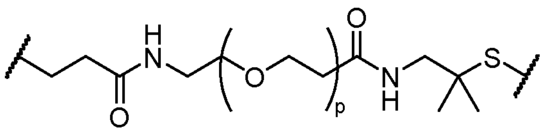,

wherein p is any integer from 1 to 20.

In certain embodiments, when $L_2$ comprises the PEG linear chain, $L_2$ is selected from the group consisting of:

| |
|---|
| 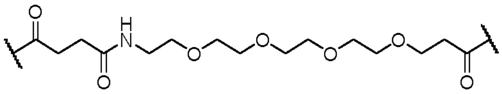 |
| 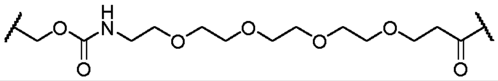 |
| 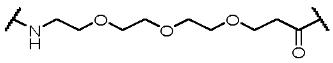 |
| 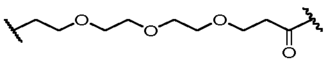 |
| 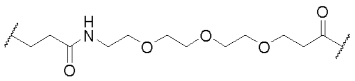 |

| |
|---|
| 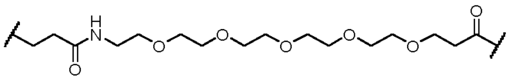 |
| 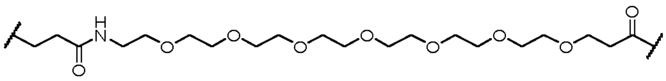 |
| 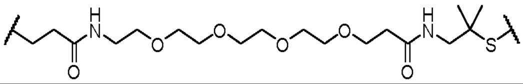 |

In certain embodiments, when $L_2$ comprises the PEG branch chain, $L_2$ is selected from the group consisting of:

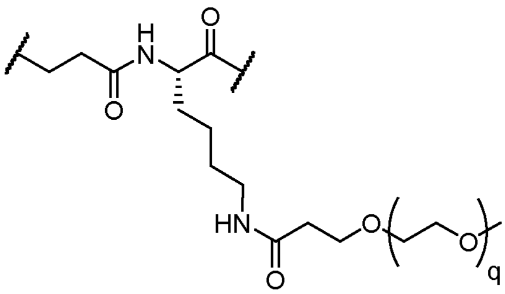

,

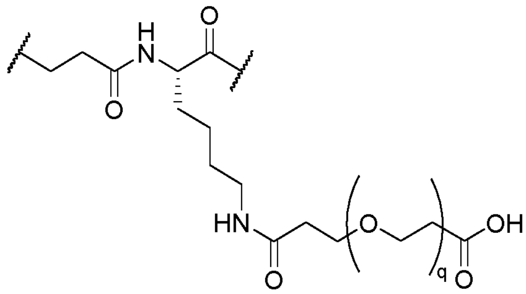

,

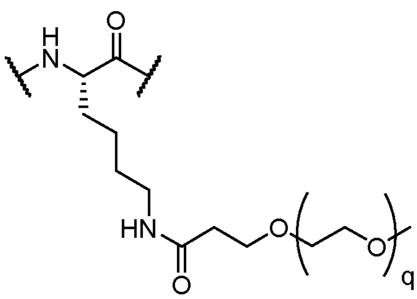

,

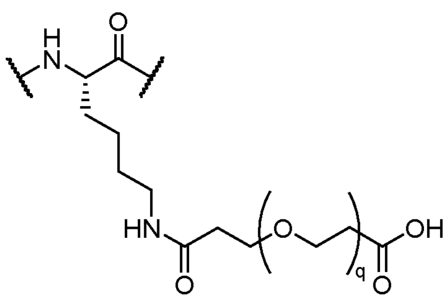

,

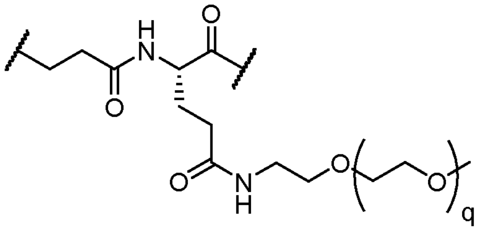
,
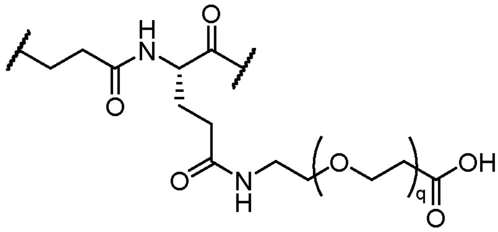
,

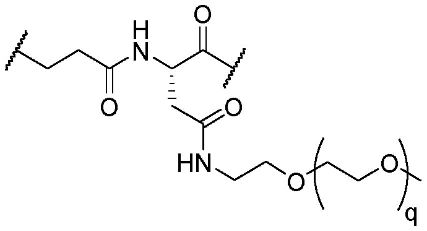
,
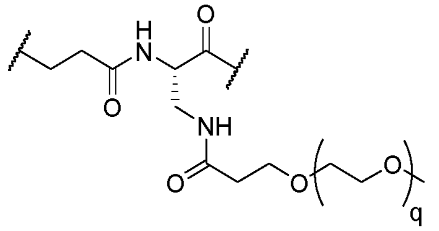
,

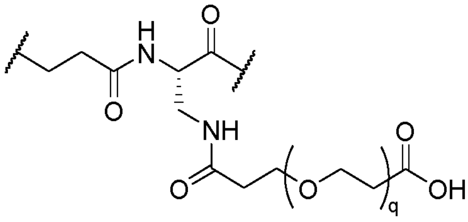
,
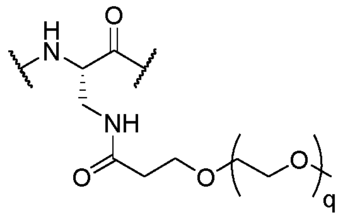
,
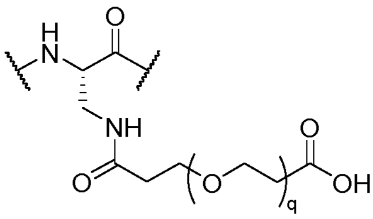
,

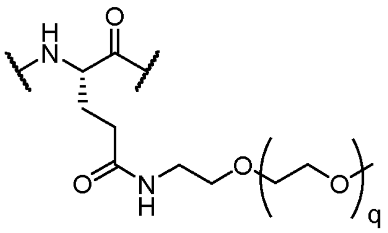
,
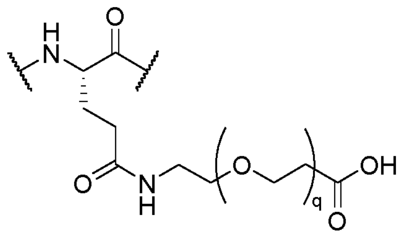
,

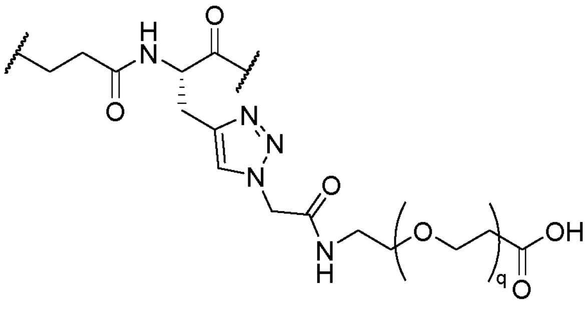
,
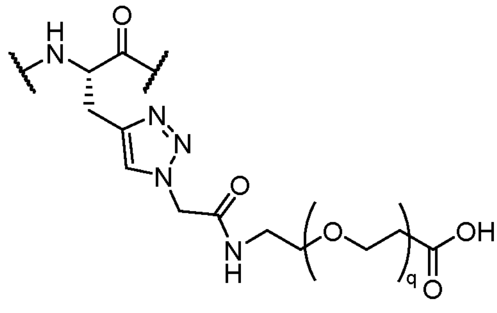
,

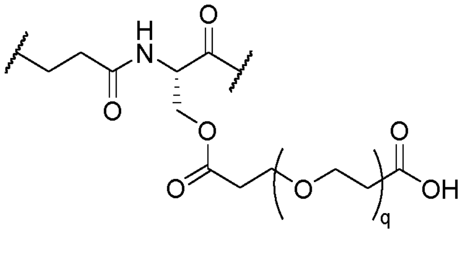
,
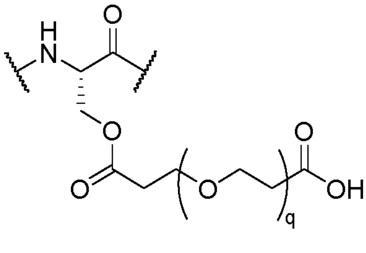
,

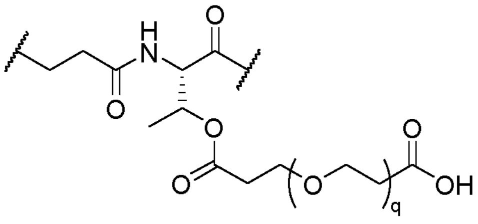 and 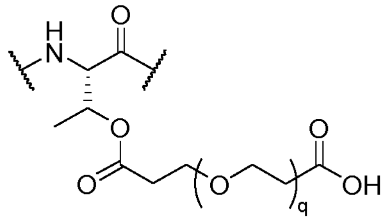 ,

wherein q is selected from any integer from 1 to 30.

In certain embodiments, when $L_2$ comprises the PEG branch chain, $L_2$ is selected from the group consisting of:

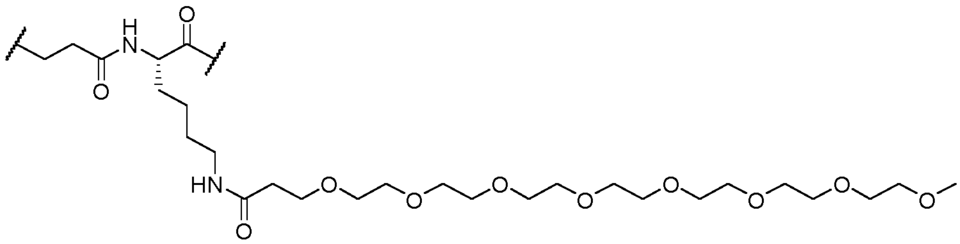

PEG8

| |
|---|
| 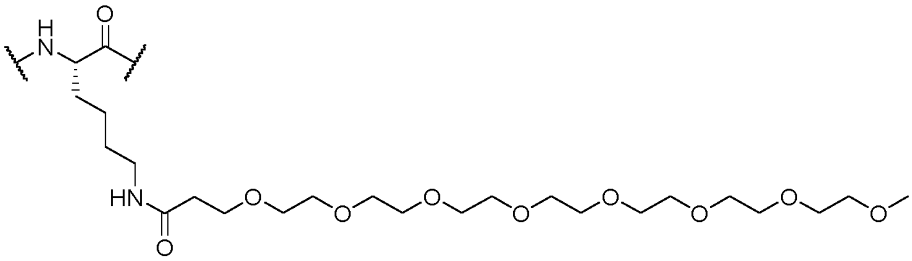 PEG8 |
| 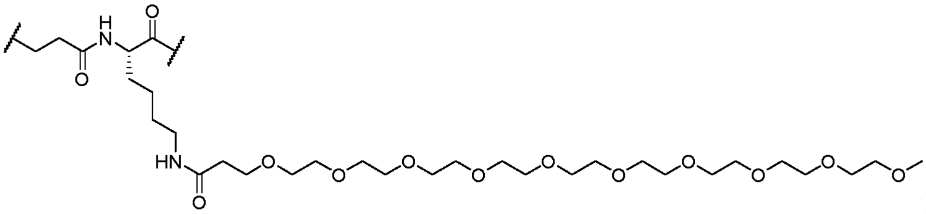 PEG10 |
| 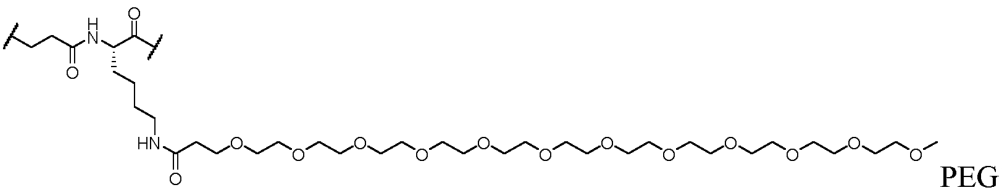 PEG12 |
| 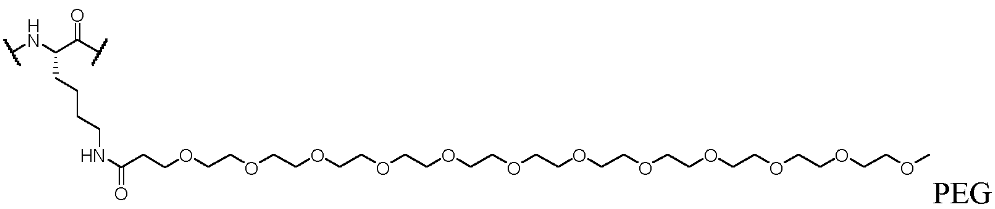 PEG12 |
| 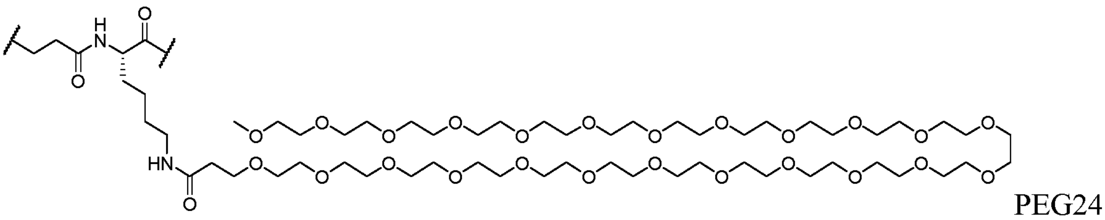 PEG24 |
| 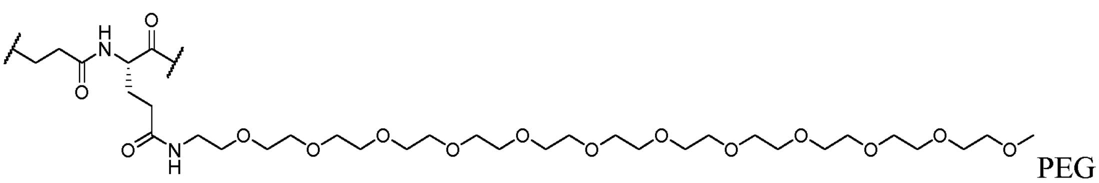 PEG12 |
| 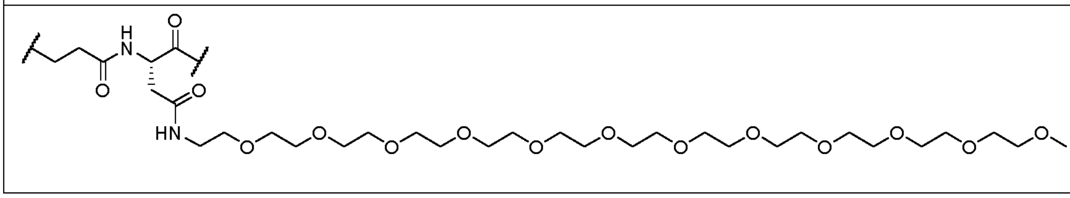 PEG12 |

| |
|---|
| 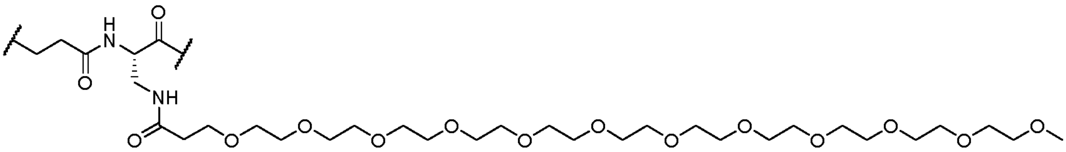 PEG12 |
| 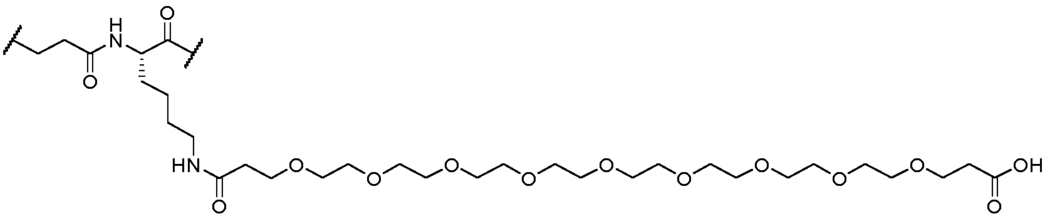 PEG10 |
| 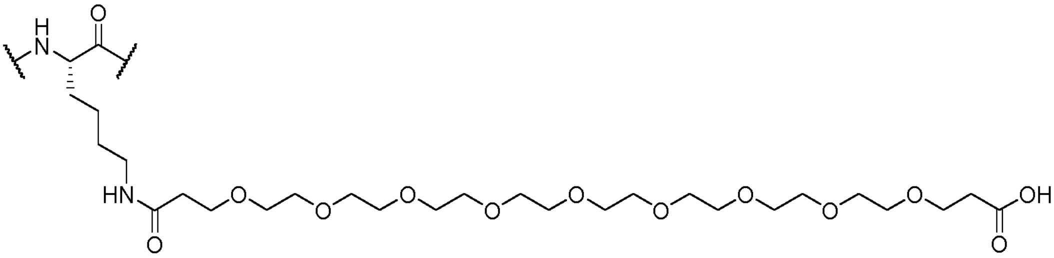 PEG10 |
| 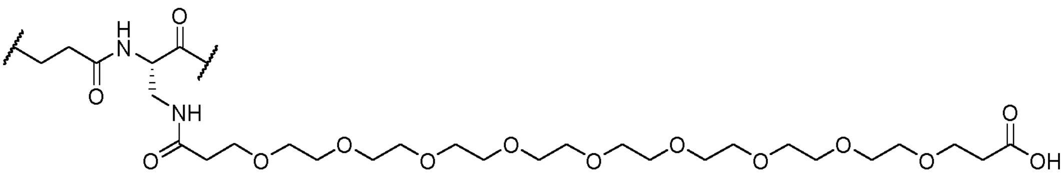 PEG10 |
| 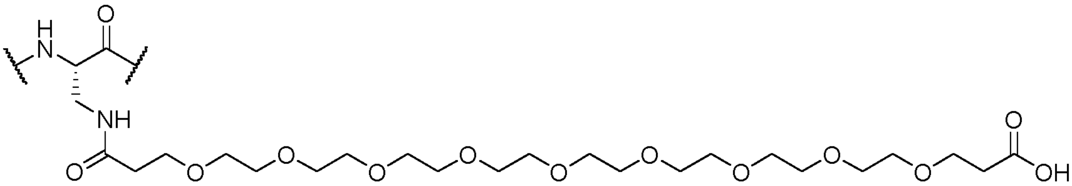 PEG10 |
| 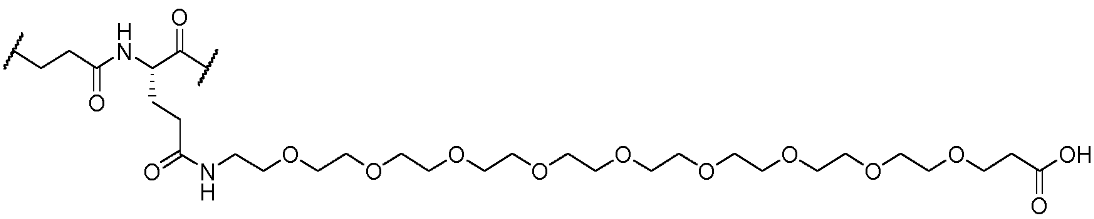 PEG10 |

| |
|---|
| 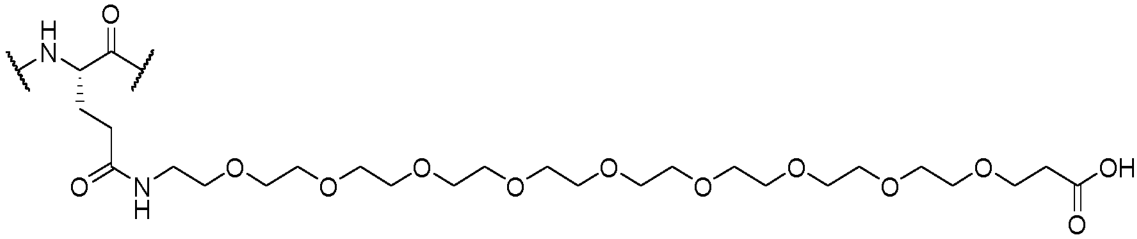 PEG10 |
| 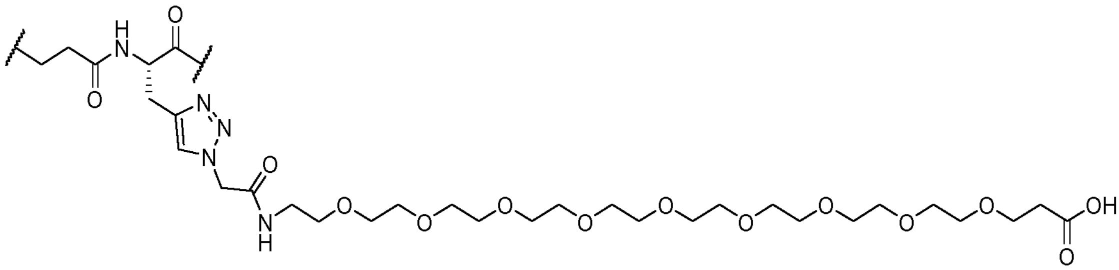 PEG10 |
| 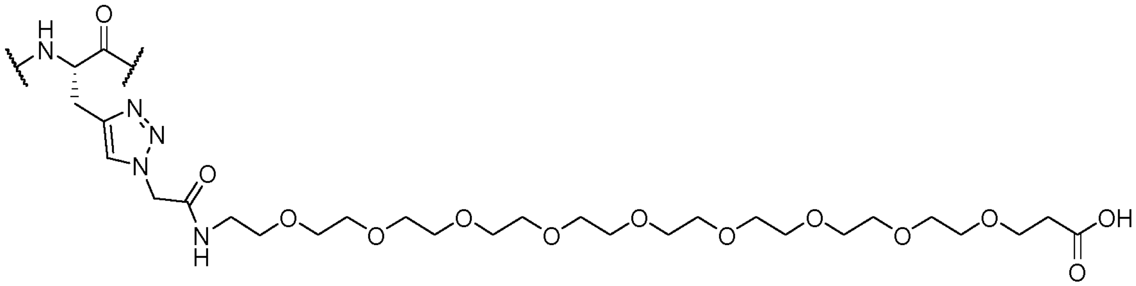 PEG10 |
| 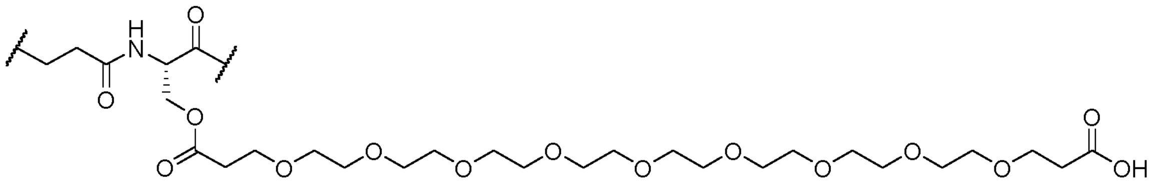 PEG10 |
| 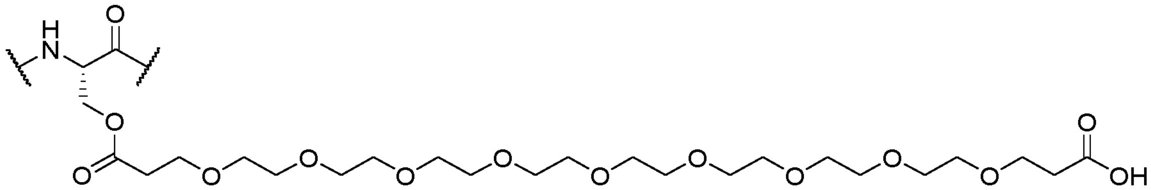 PEG10 |

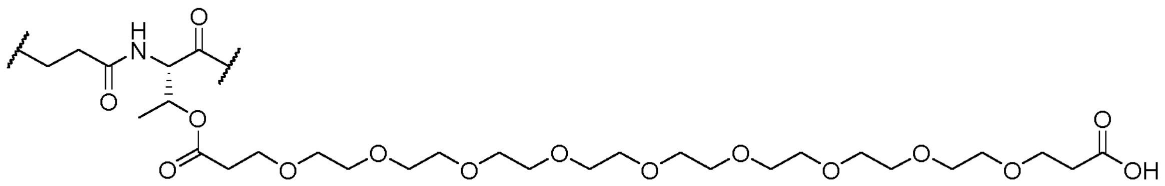

PEG10

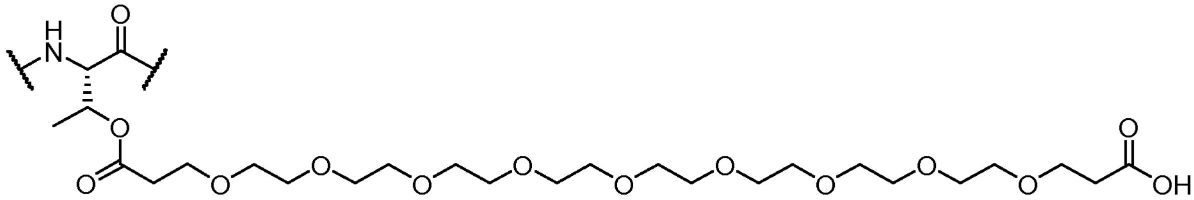

PEG10

In certain embodiments, when $L_1$ is coupled to sulfhydryl of the ligand, $L_1$ is selected from the group consisting of:

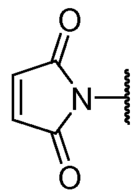, 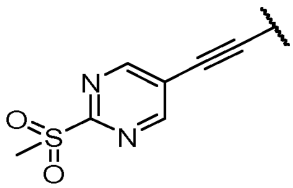, 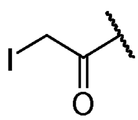, 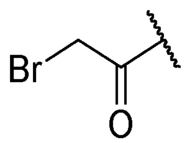, 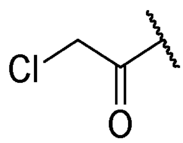, 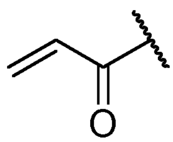,

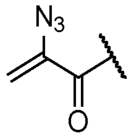, 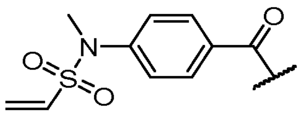, 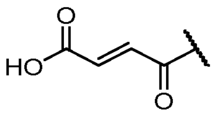, 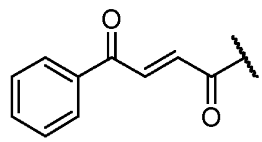,

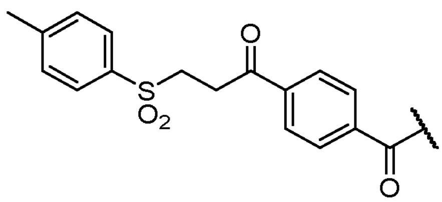, 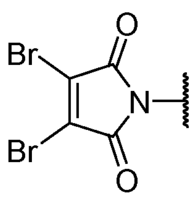, 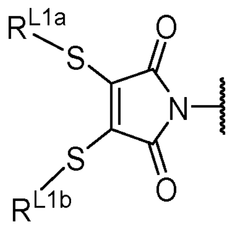, 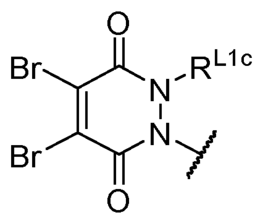,

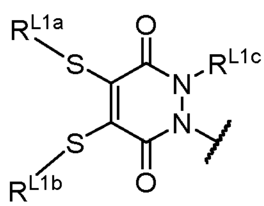, 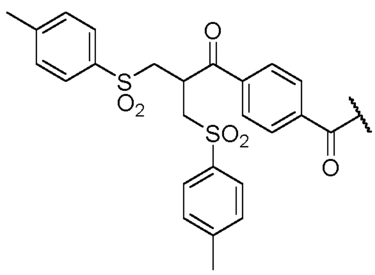, 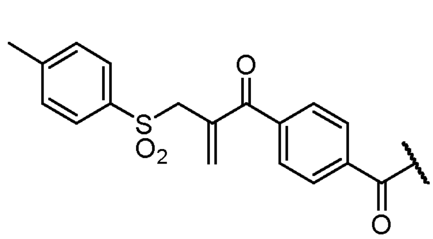

and

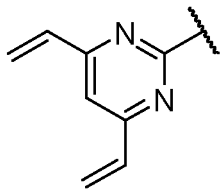

;

wherein, $R^{L1a}$, $R^{L1b}$ and $R^{L1c}$ are each independently selected from the group consisting of: hydrogen, protium, deuterium, tritium, halogen, $-NO_2$, -CN, -OH, -SH, $-NH_2$, -C(O)H, $-CO_2H$, -C(O)C(O)H, $-C(O)CH_2C(O)H$, -S(O)H, $-S(O)_2H$, $-C(O)NH_2$, $-SO_2NH_2$, -OC(O)H, - $N(H)SO_2H$, alkyl, alkenyl, alkynyl, alcyl, heterocyclyl, aryl and heteroaryl.

In certain embodiments, $R^{L1a}$, $R^{L1b}$ and $R^{L1c}$ are each independently selected from the group consisting of: hydrogen, optionally substituted methyl, optionally substituted ethyl, optionally substituted aryl and optionally substituted benzyl.

In certain embodiments, when $L_1$ is coupled to the ligand via amino, $L_1$ is selected from the group consisting of:

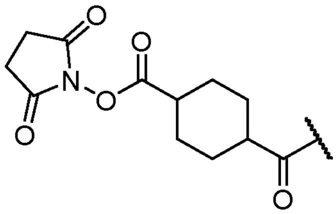

,

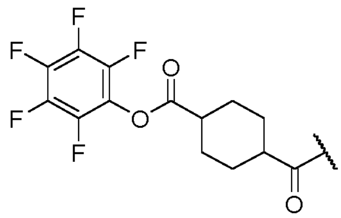

and

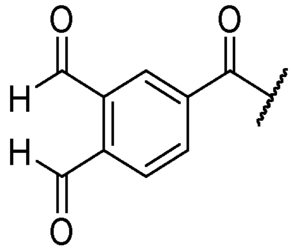

.

In certain embodiments, when $L_1$ is coupled via click chemistry, $L_1$ is selected from the group consisting of:

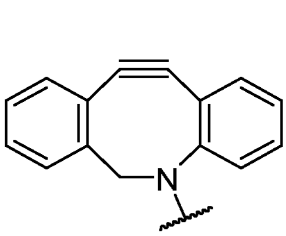

,

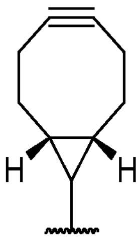

,

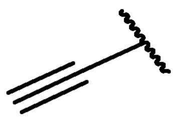

and

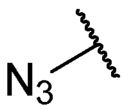

.

For example, the compound of formula IVa or IVb may be selected from the group consisting of the following structures:

| No. | Structure |
| --- | --- |
| IV-1 | 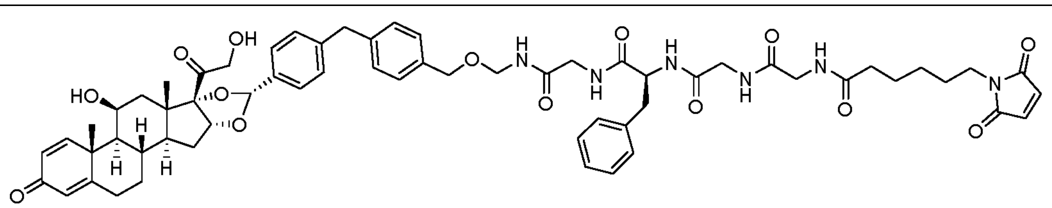 |
| IV-2 | 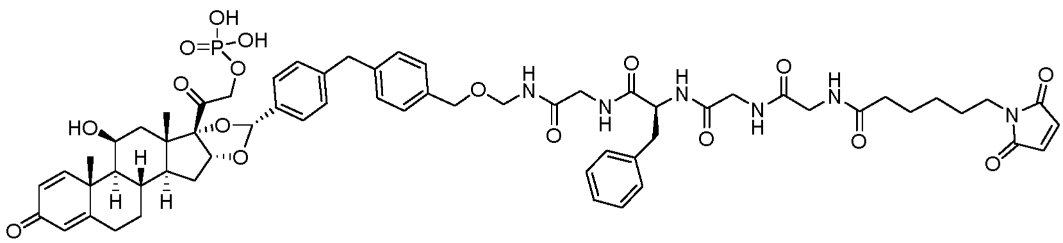 |

(continued)

| No. | Structure |
|---|---|
| IV-6 | 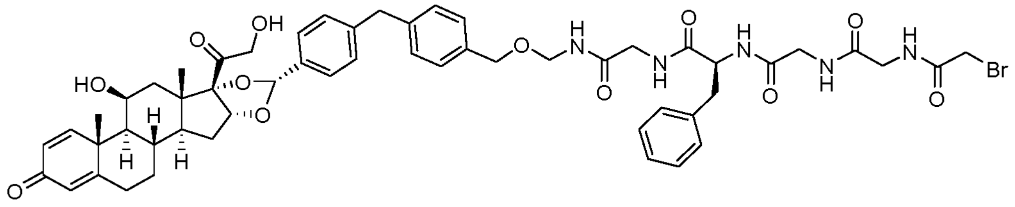 |
| IV-7 | 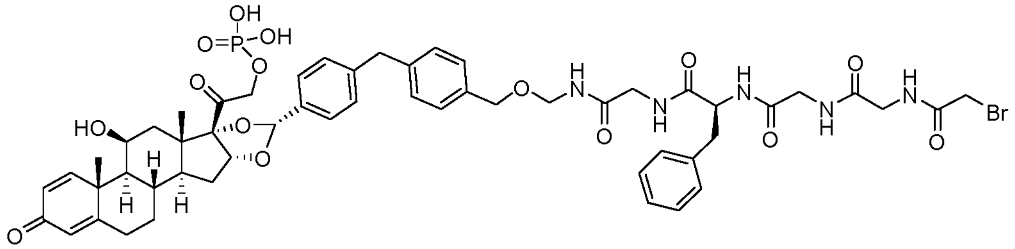 |
| IV-11 | 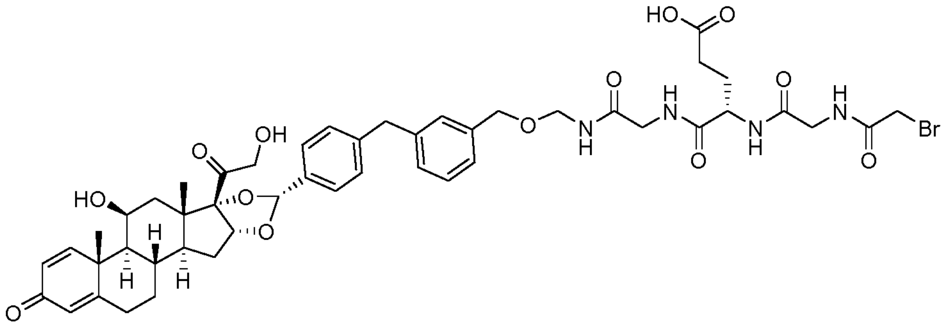 |
| IV-12 | 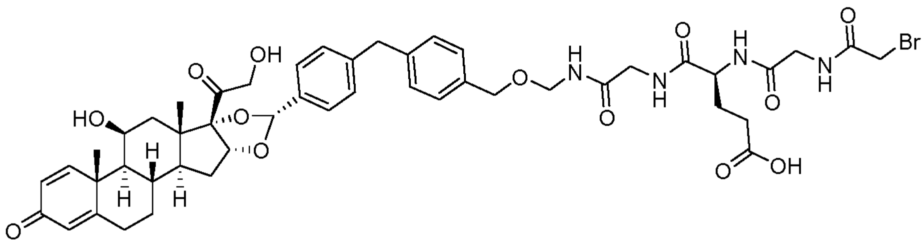 |
| IV-13 | 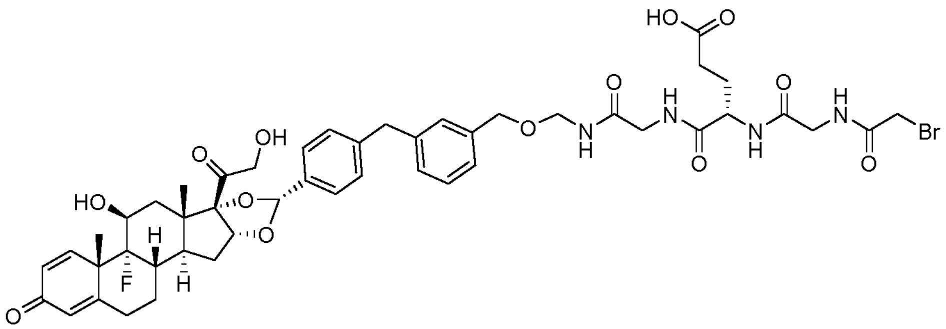 |

(continued)

| No. | Structure |
|---|---|
| IV-14 | 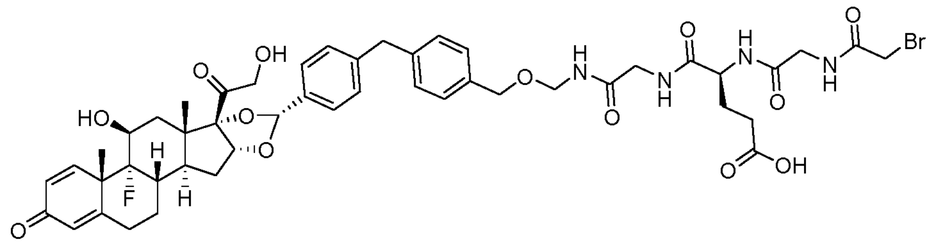 |
| IV-15 | 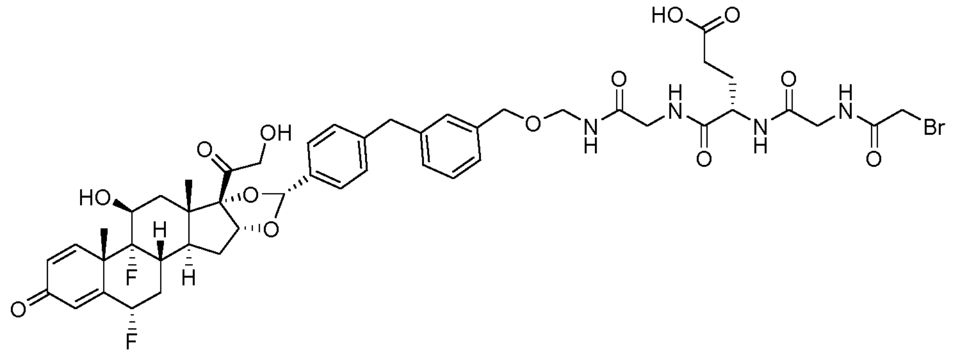 |
| IV-16 | 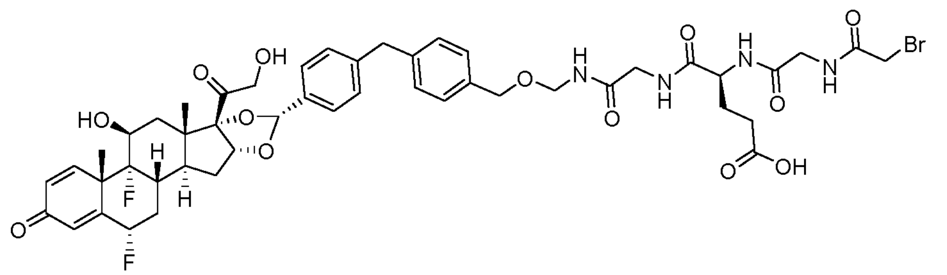 |
| IV-17 | 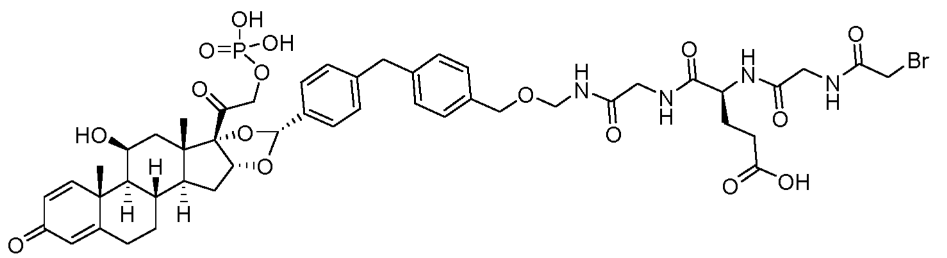 |
| IV-21 | 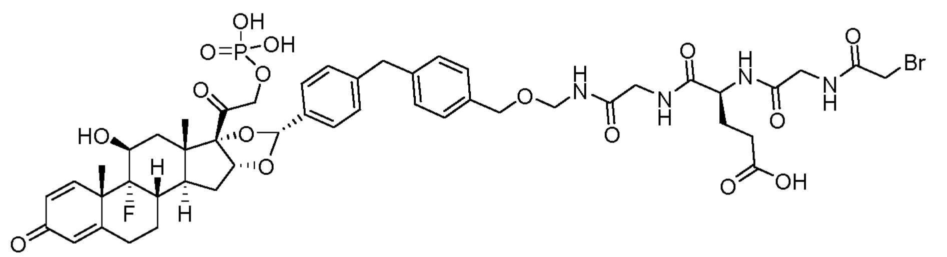 |

(continued)

| No. | Structure |
|---|---|
| IV-25 | 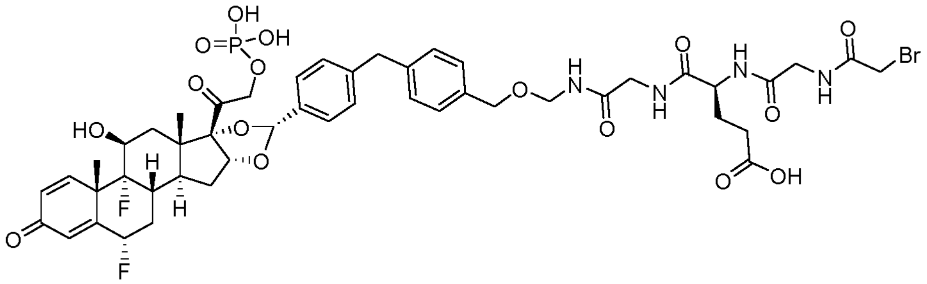 |
| IV-29 | 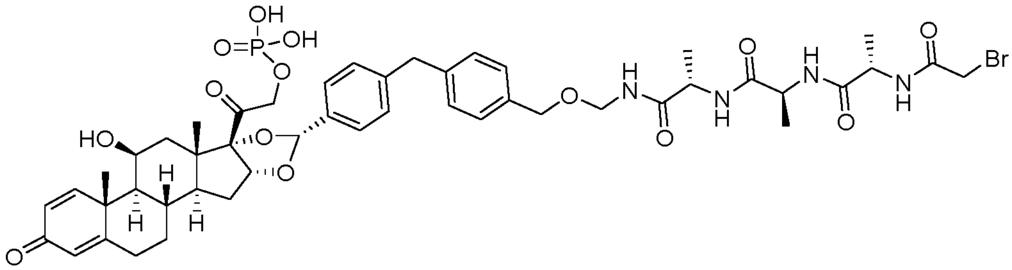 |
| IV-30 | 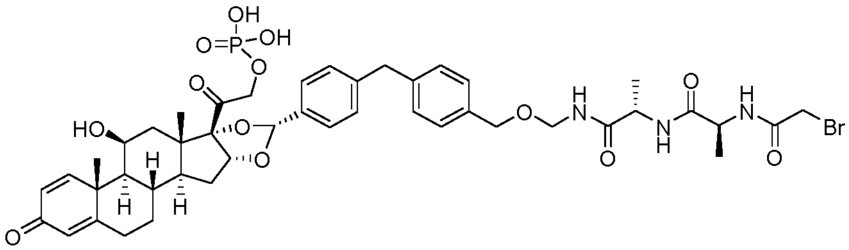 |
| IV-34 | 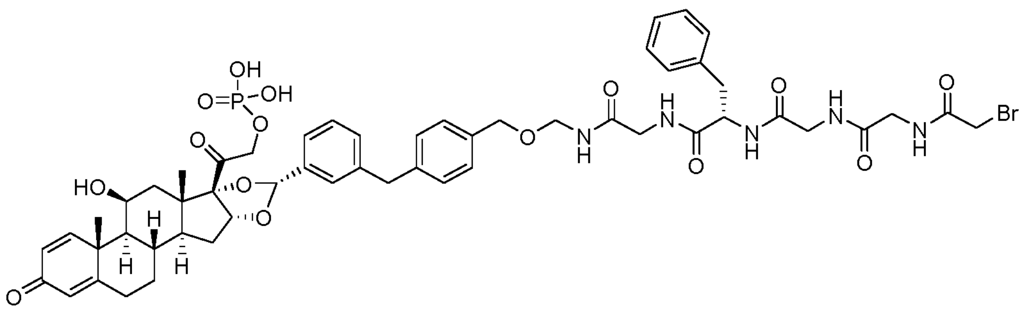 |
| IV-35 | 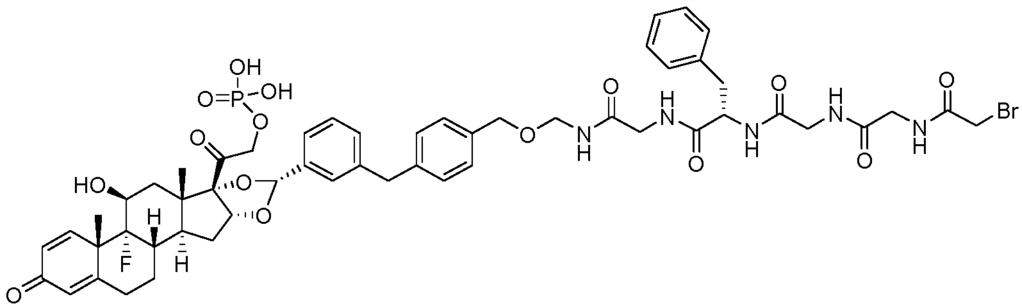 |

(continued)

| No. | Structure |
| --- | --- |
| IV-39 | 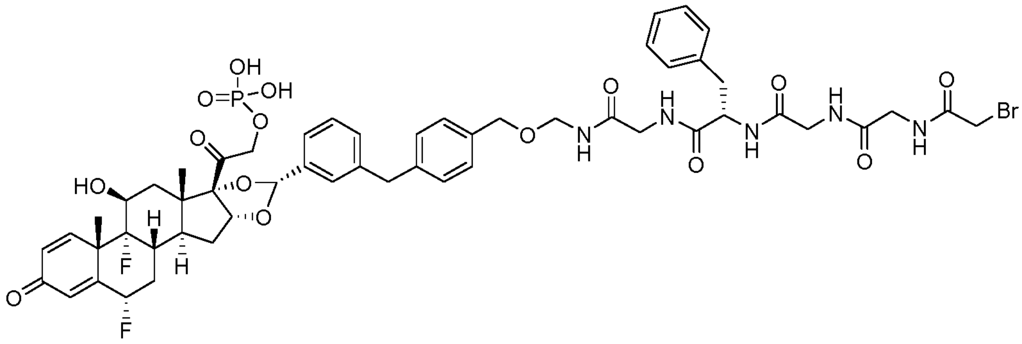 |
| IV-43 | 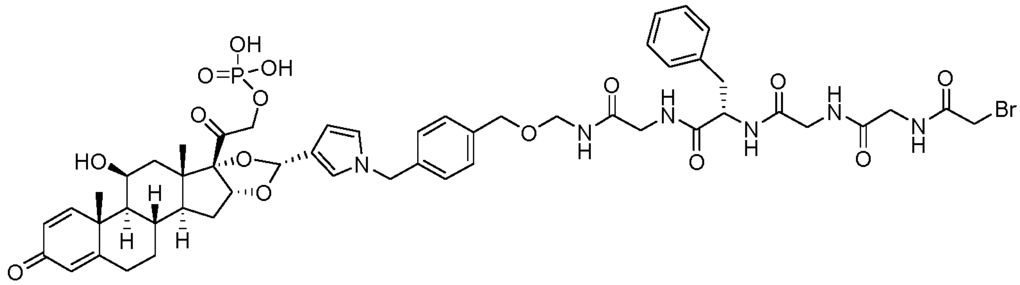 |
| IV-44 | 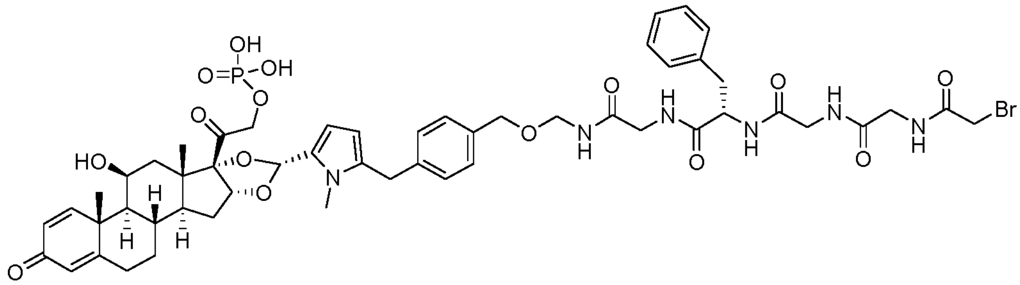 |
| IV-45 | 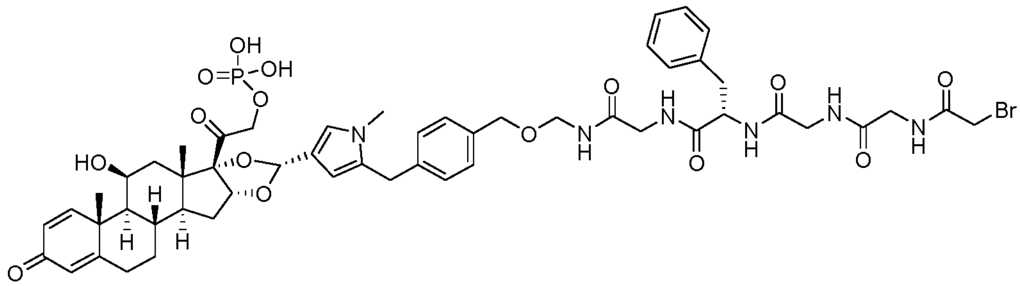 |
| IV-46 | 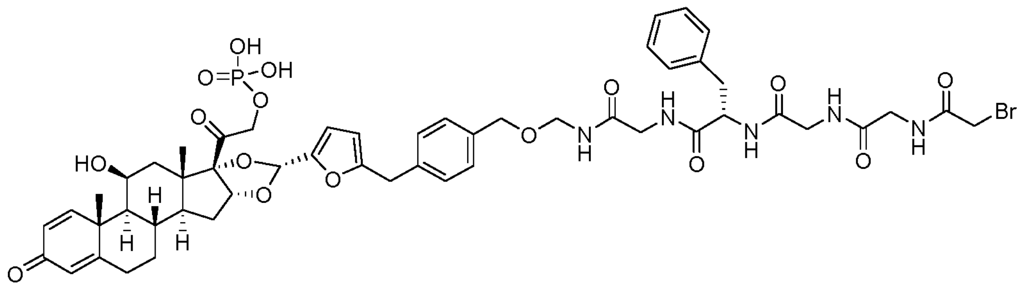 |

(continued)

| No. | Structure |
|---|---|
| IV-47 | 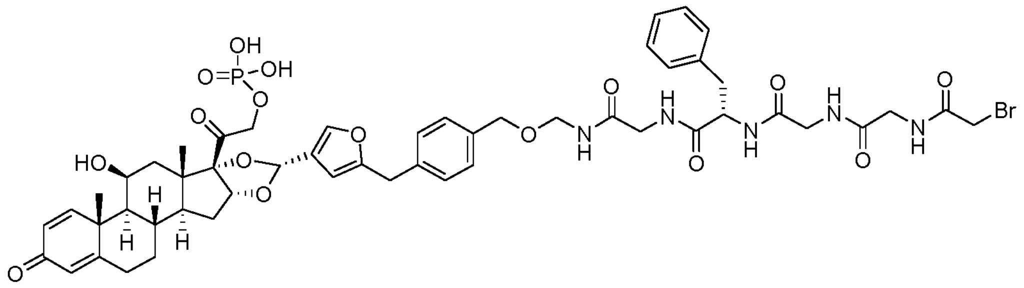 |
| IV-48 | 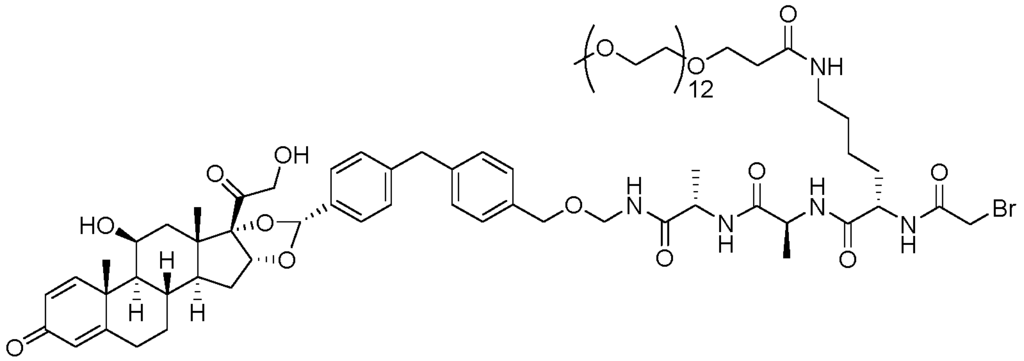 |
| IV-49 | 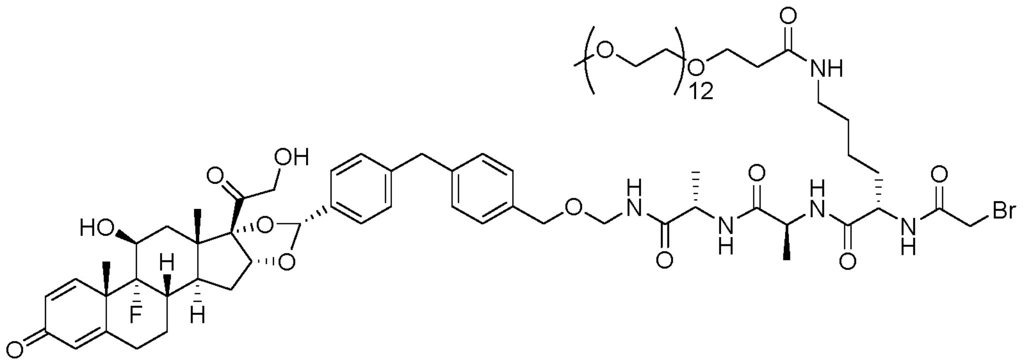 |
| IV-50 | 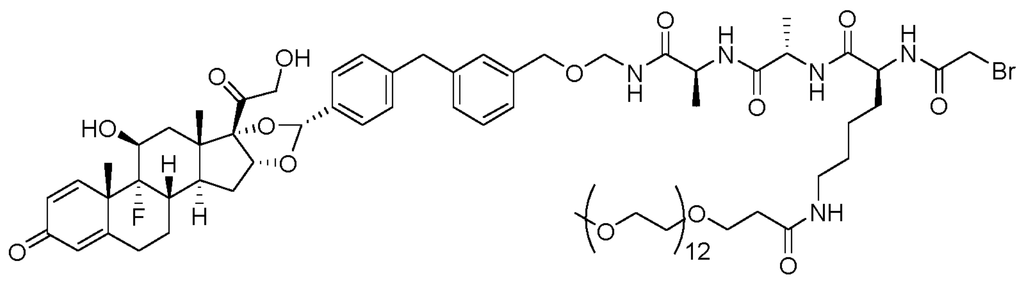 |

(continued)

| No. | Structure |
|---|---|
| IV-51 | 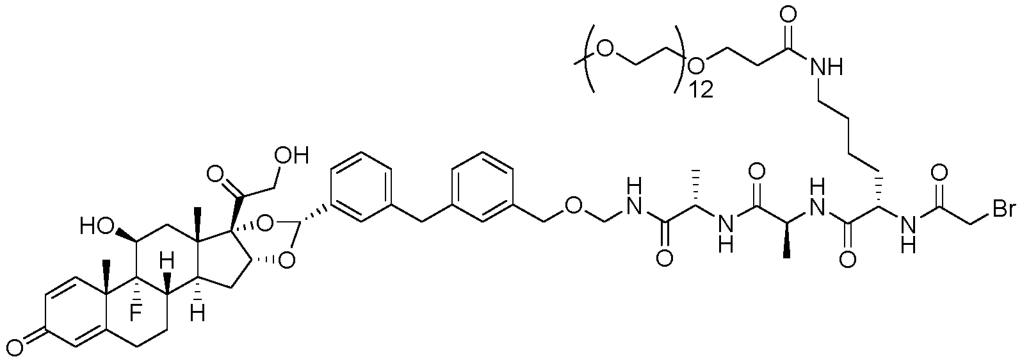 |
| IV-52 | 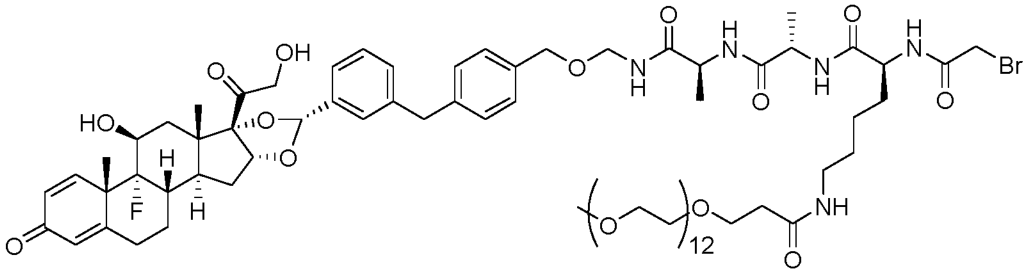 |
| IV-53 | 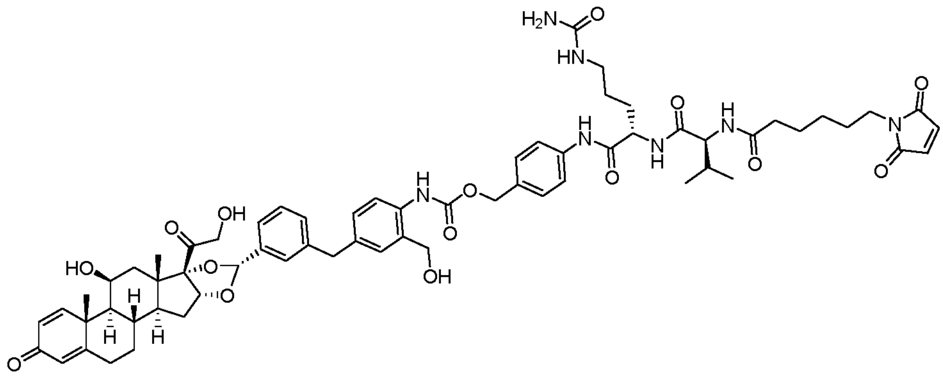 |
| IV-54 | 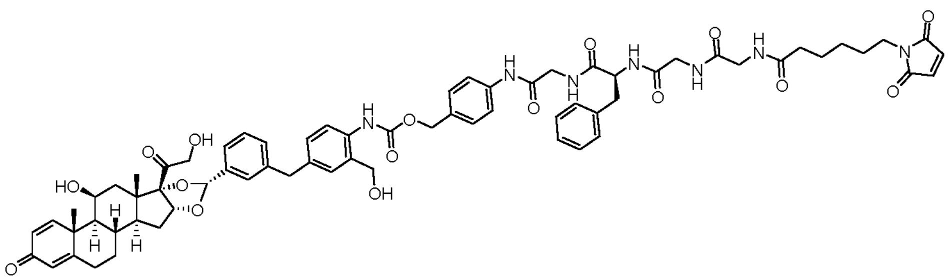 |
| IV-55 | 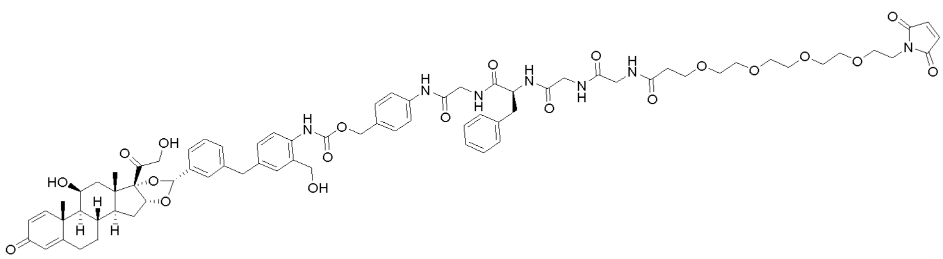 |

(continued)

| No. | Structure |
|---|---|
| IV-56 | 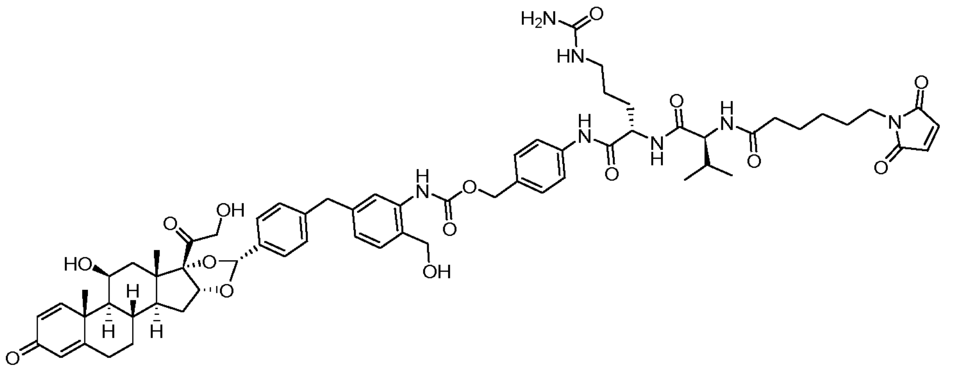 |
| IV-57 | 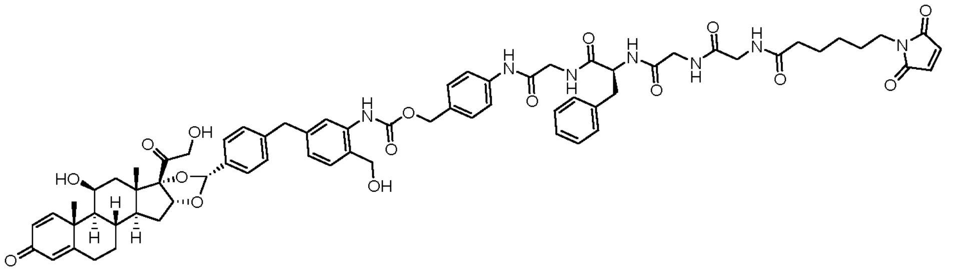 |
| IV-58 | 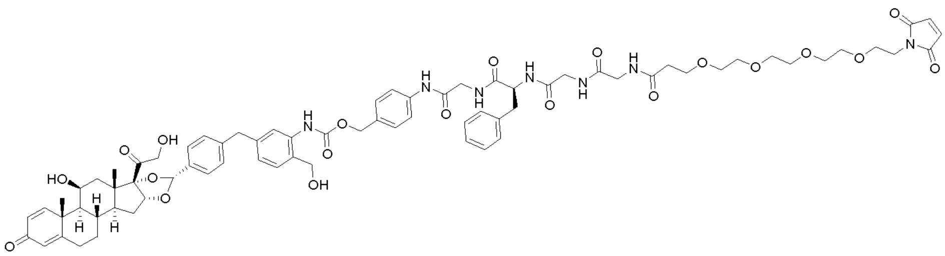 |
| IV-59 | 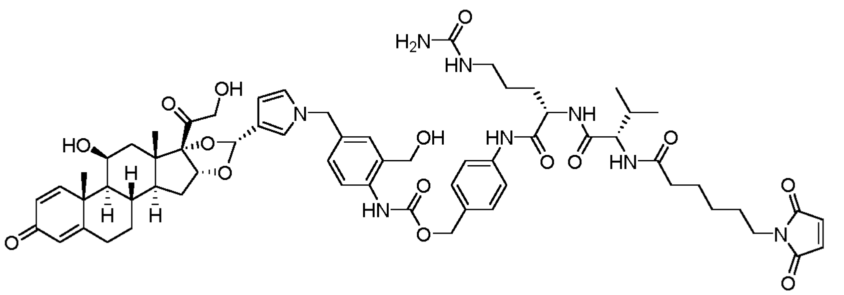 |
| IV-60 | 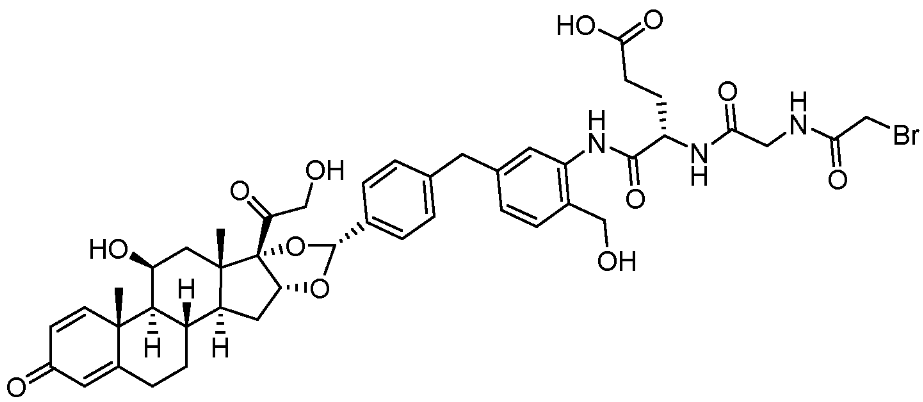 |

(continued)

| No. | Structure |
|---|---|
| IV-61 | 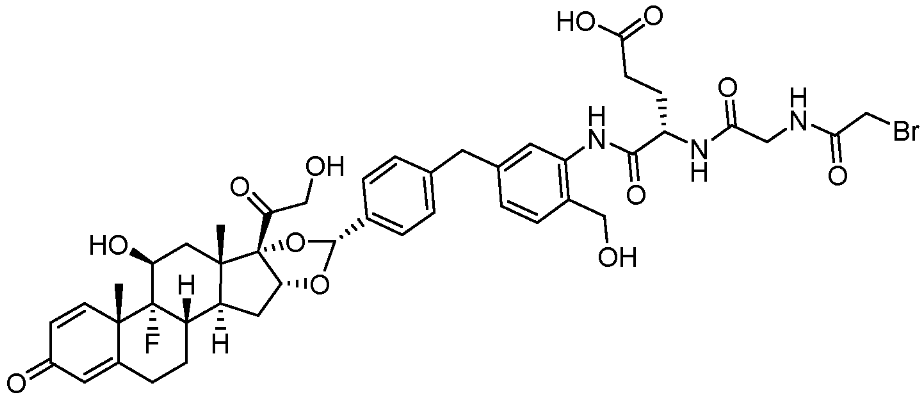 |
| IV-62 | 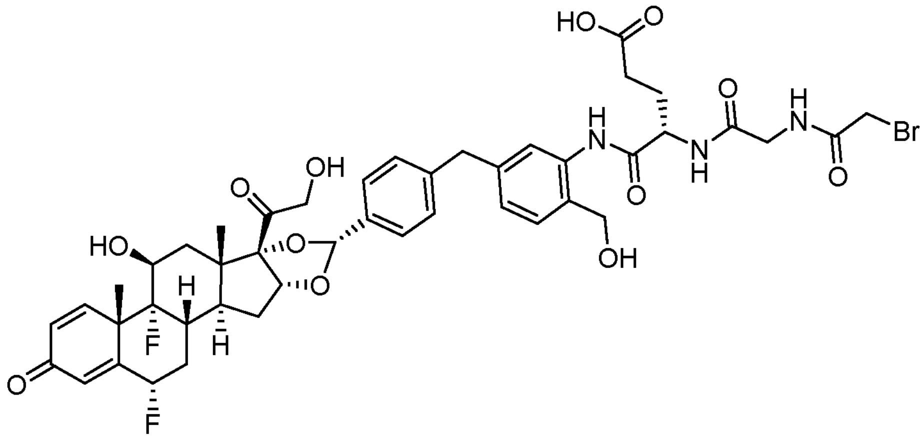 |
| IV-63 | 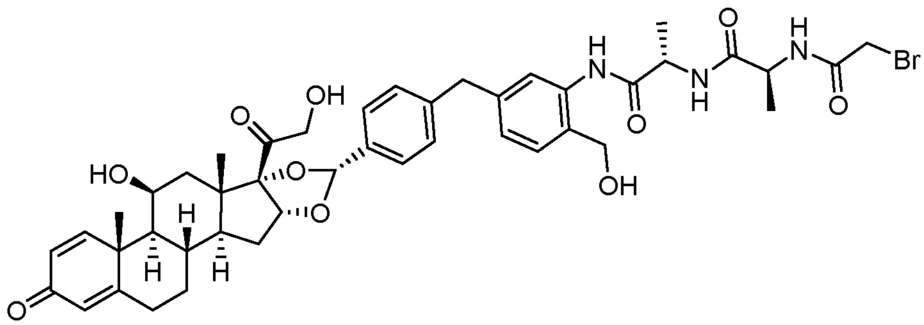 |
| IV-64 | 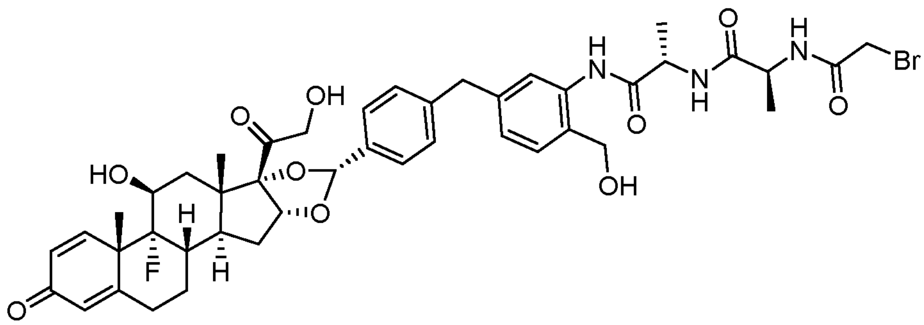 |

(continued)

| No. | Structure |
|---|---|
| IV-65 | 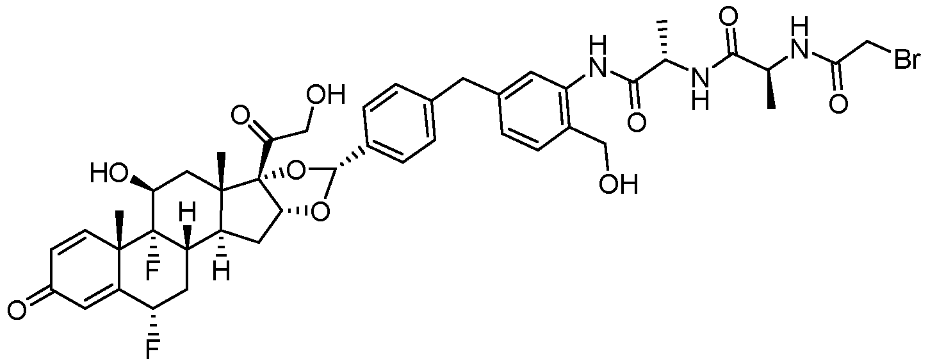 |
| IV-66 | 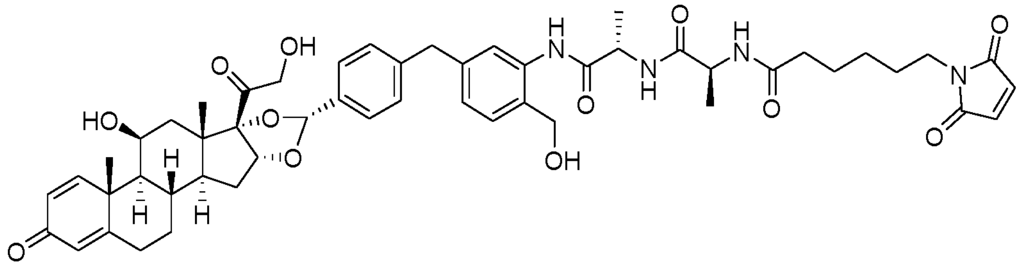 |
| IV-67 | 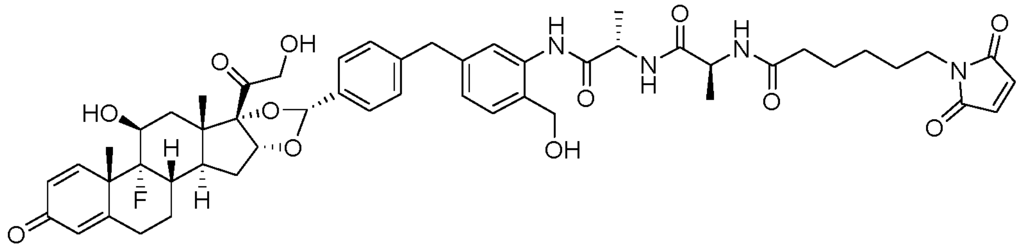 |
| IV-68 | 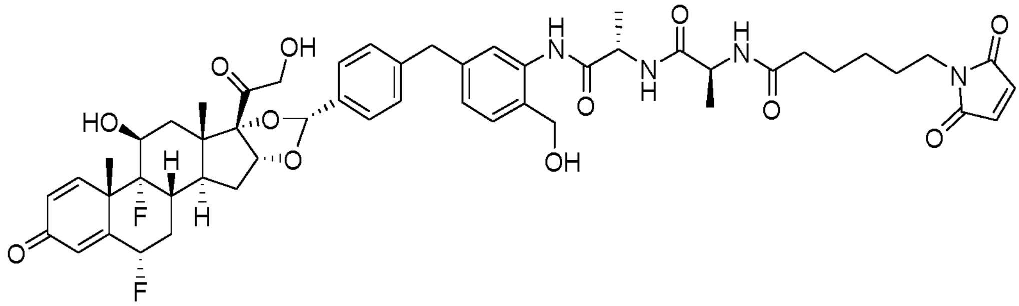 |
| IV-69 | 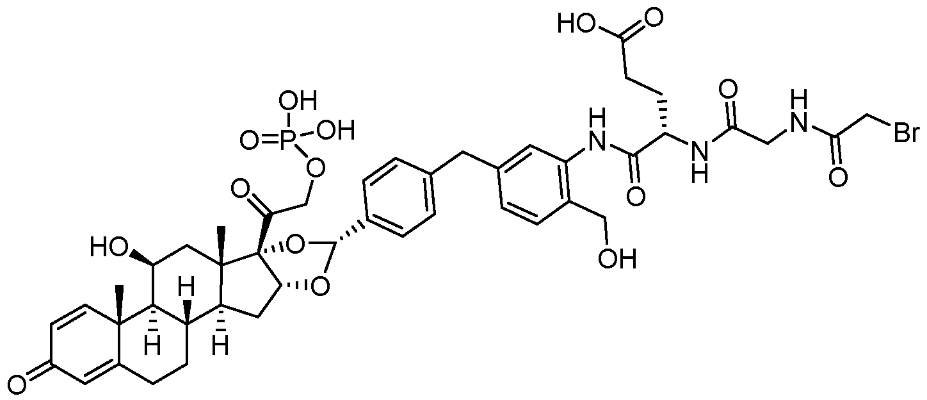 |

(continued)

| No. | Structure |
|---|---|
| IV-70 | 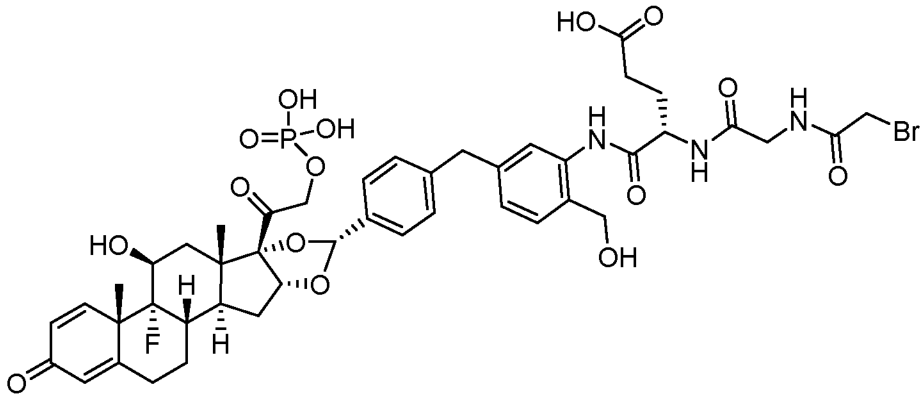 |
| IV-71 | 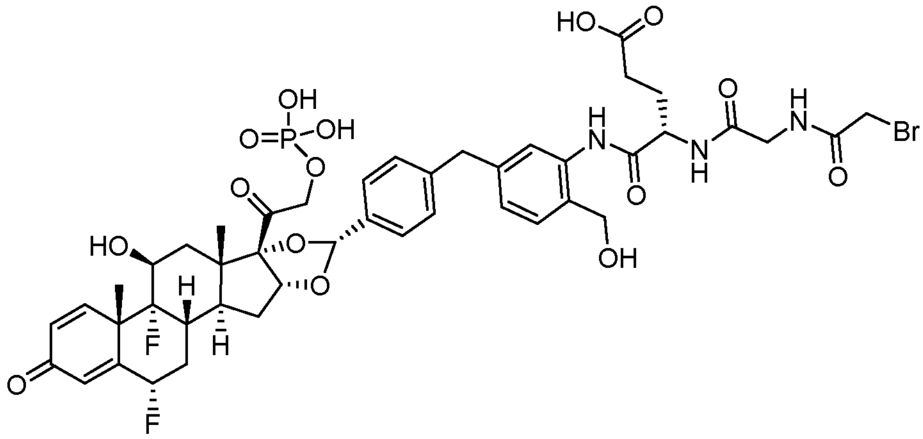 |
| IV-81 | 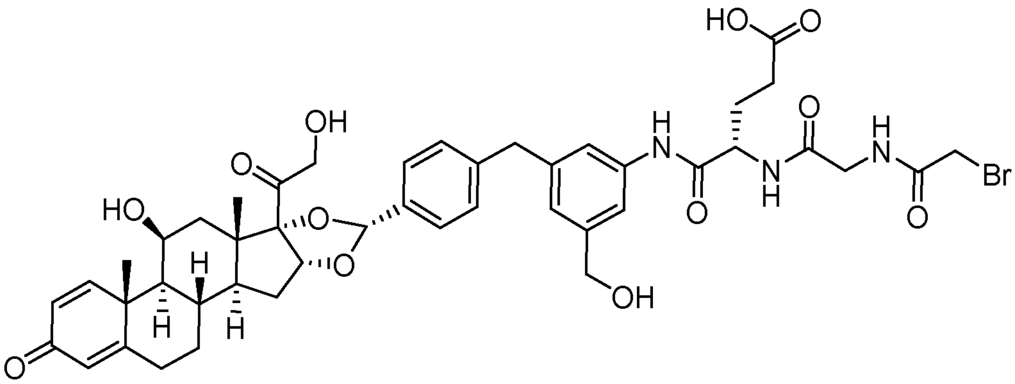 |
| IV-82 | 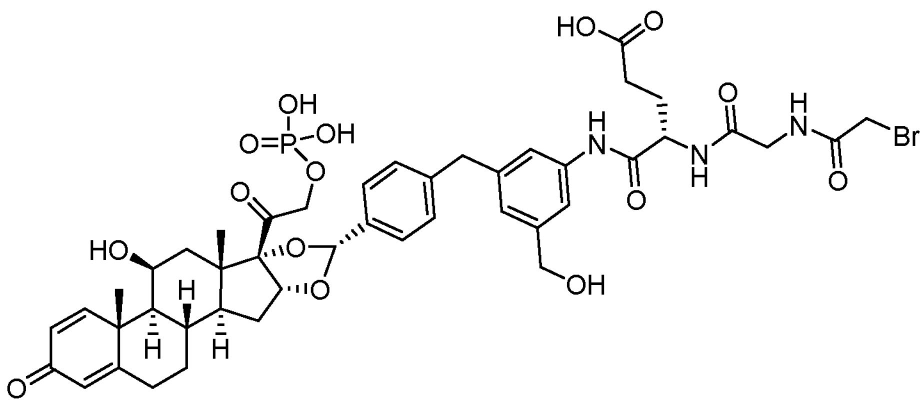 |

(continued)

| No. | Structure |
|---|---|
| IV-83 | 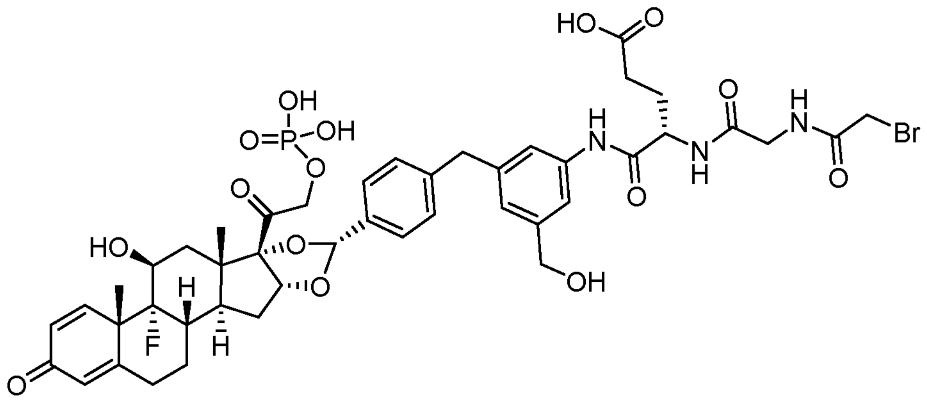 |
| IV-84 | 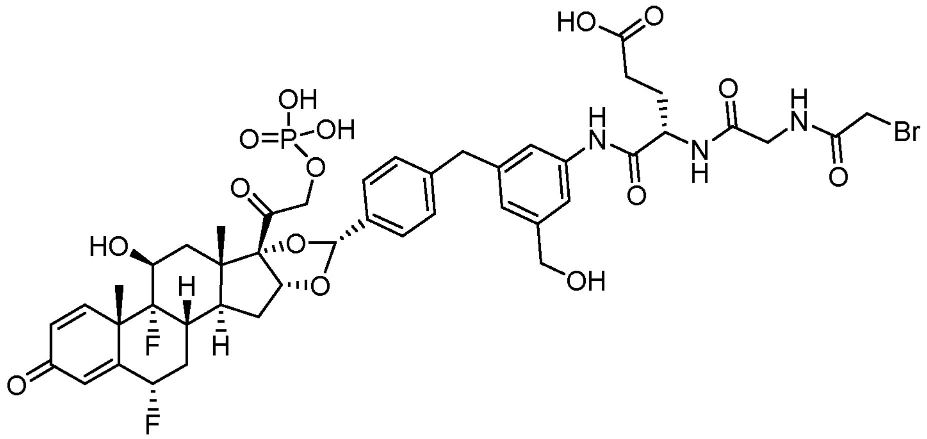 |
| IV-91 | 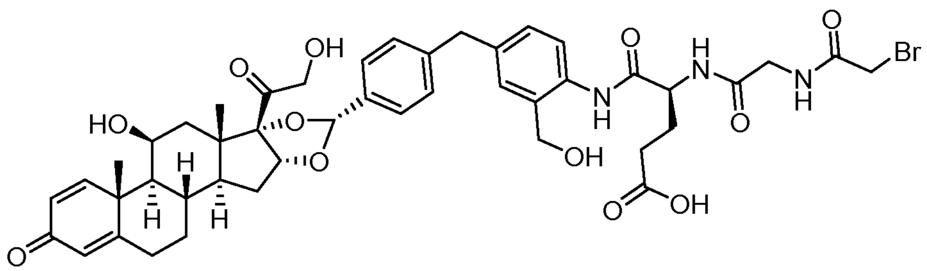 |
| IV-92 | 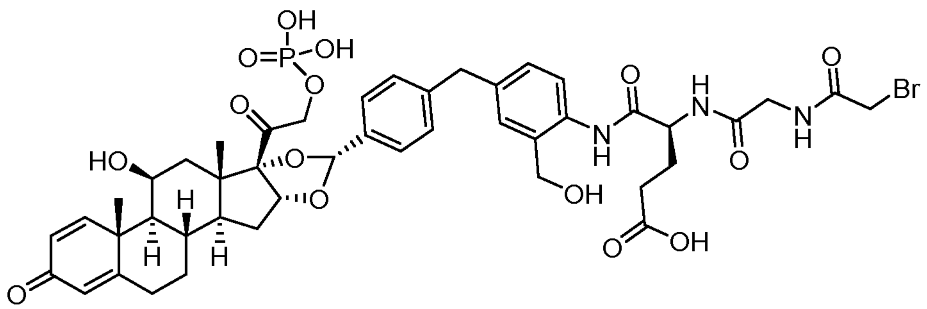 |

(continued)

| No. | Structure |
|---|---|
| IV-93 | 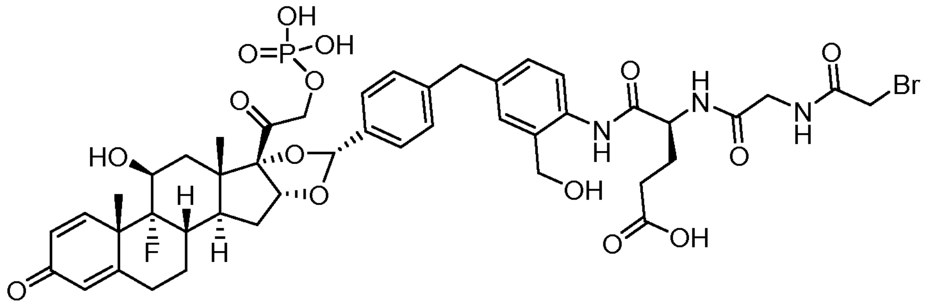 |
| IV-94 | 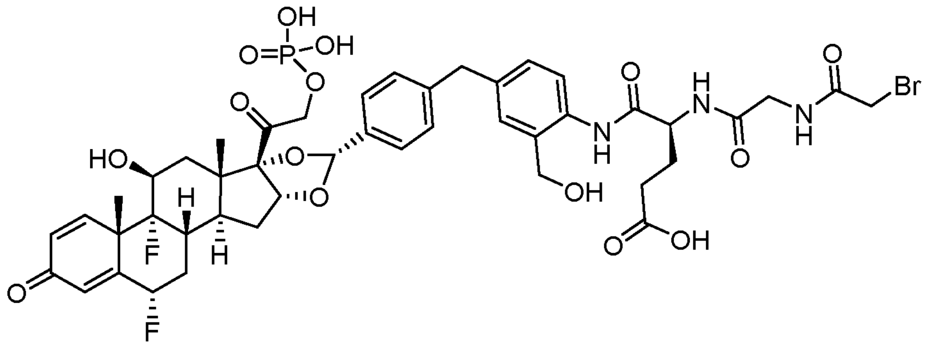 |
| IV-101 | 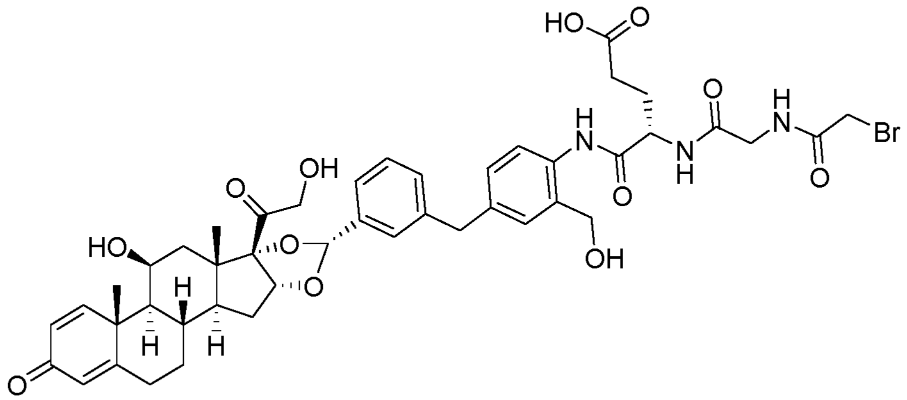 |
| IV-102 | 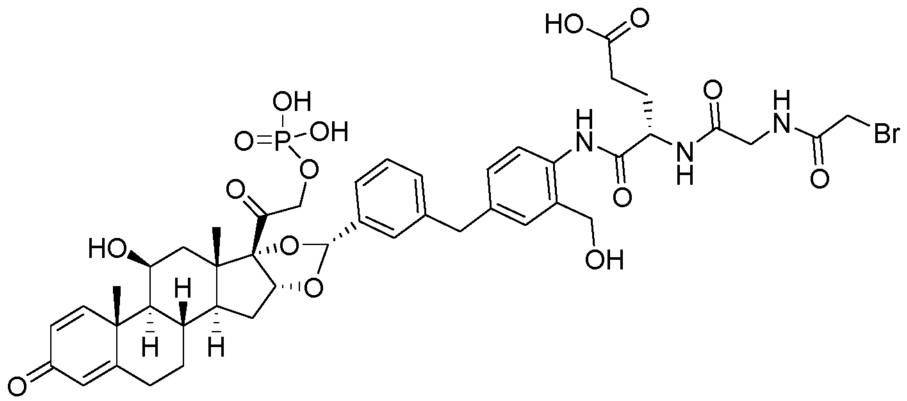 |

(continued)

| No. | Structure |
|---|---|
| IV-103 | 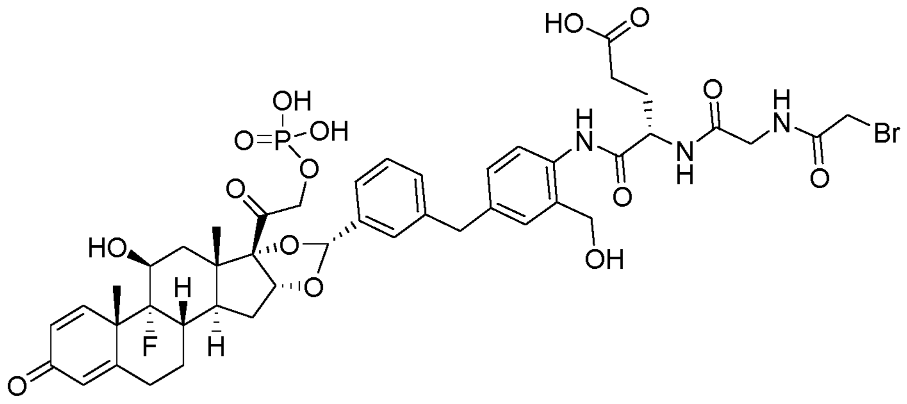 |
| IV-104 | 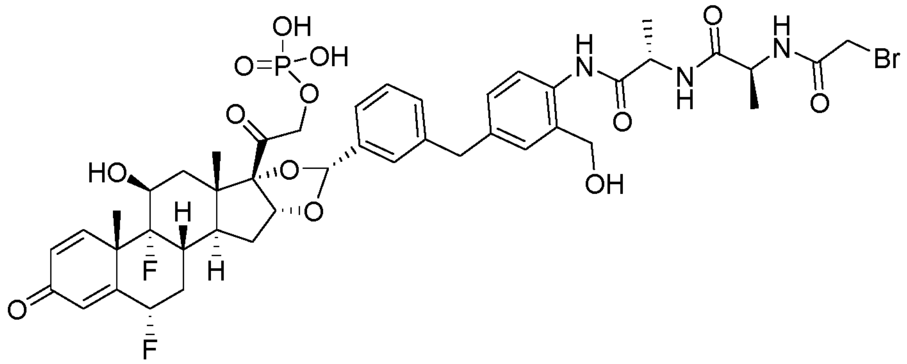 |
| IV-111 | 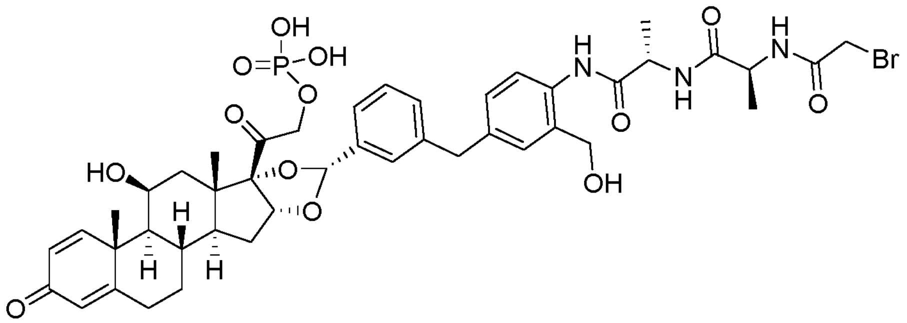 |
| IV-112 | 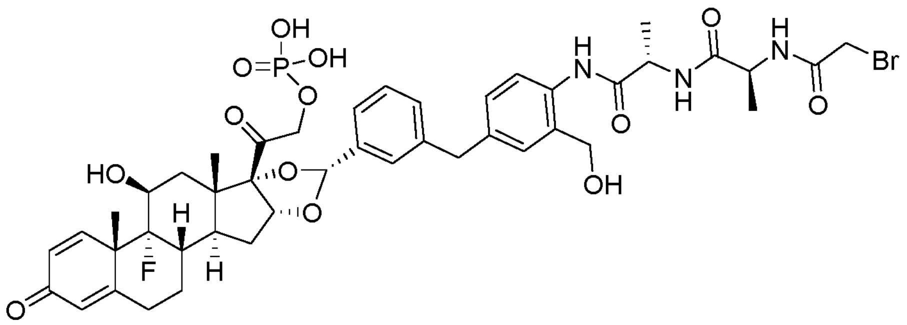 |

(continued)

| No. | Structure |
|---|---|
| IV-113 | 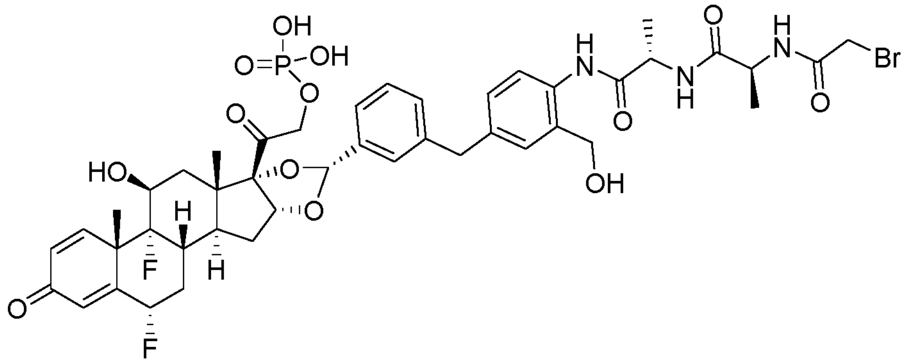 |
| IV-114 | 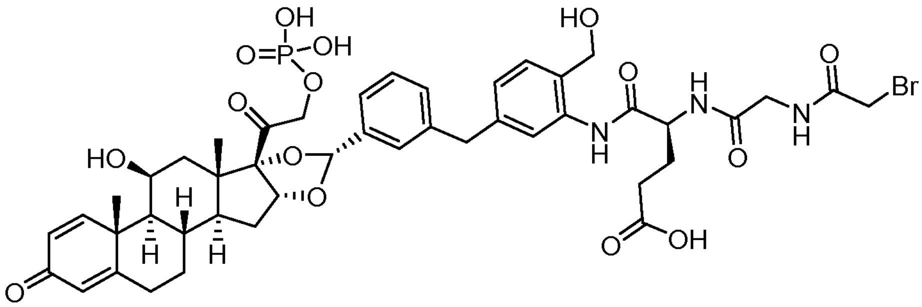 |
| IV-115 | 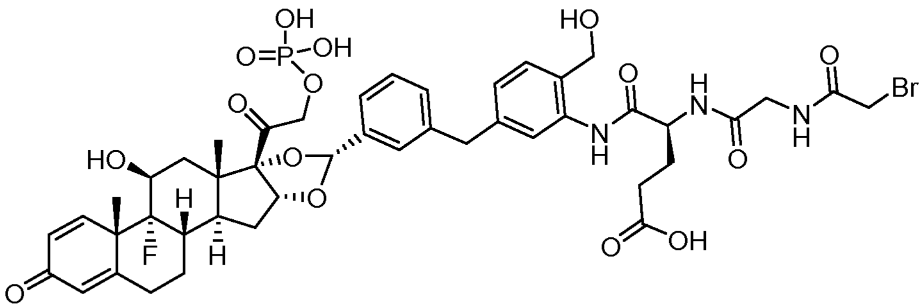 |
| IV-116 | 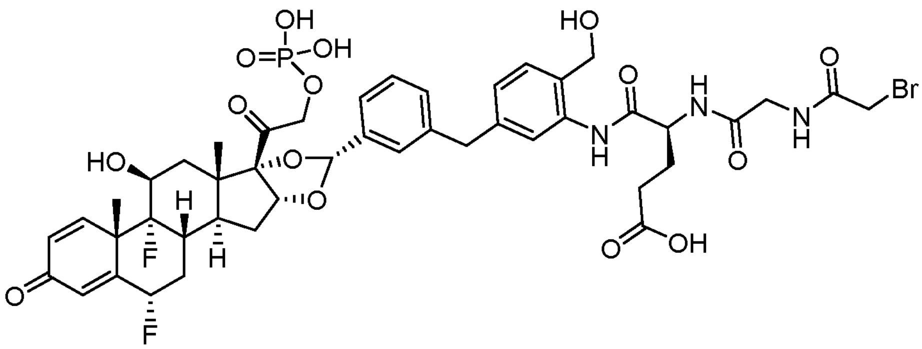 |

(continued)

| No. | Structure |
|---|---|
| IV-117 | 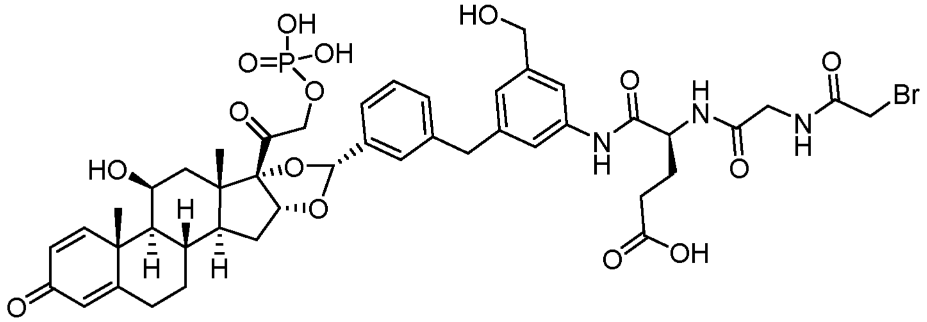 |
| IV-118 | 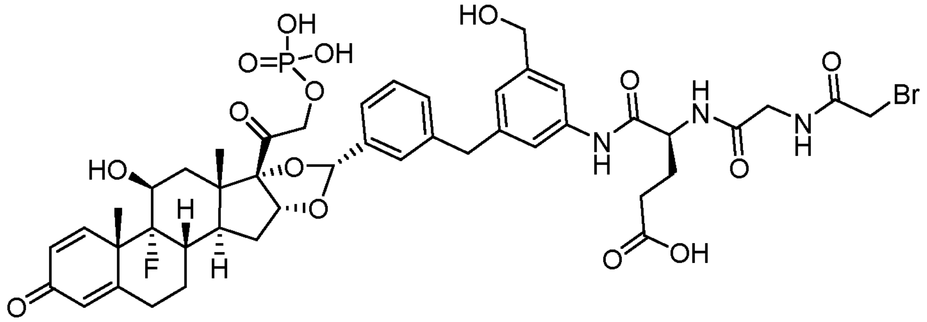 |
| IV-119 | 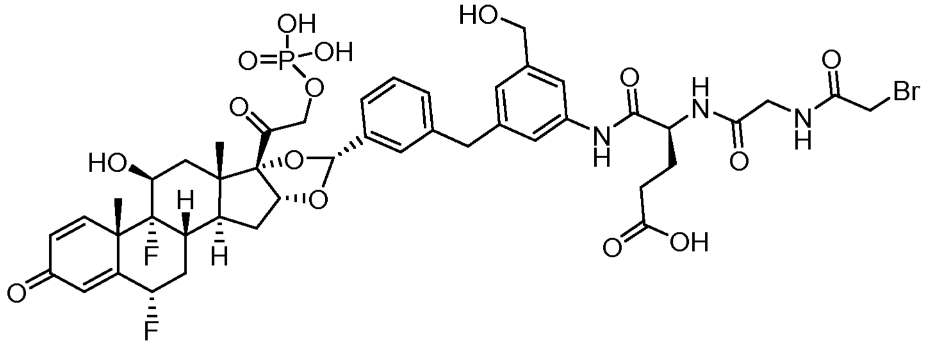 |
| IV-120 | 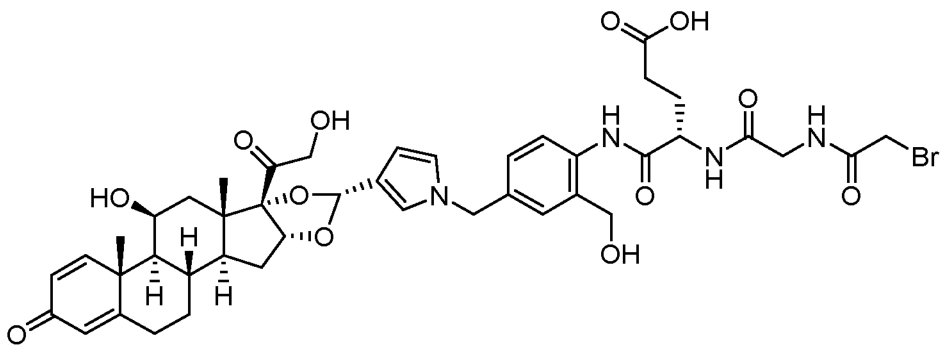 |

(continued)

| No. | Structure |
|---|---|
| IV-121 | 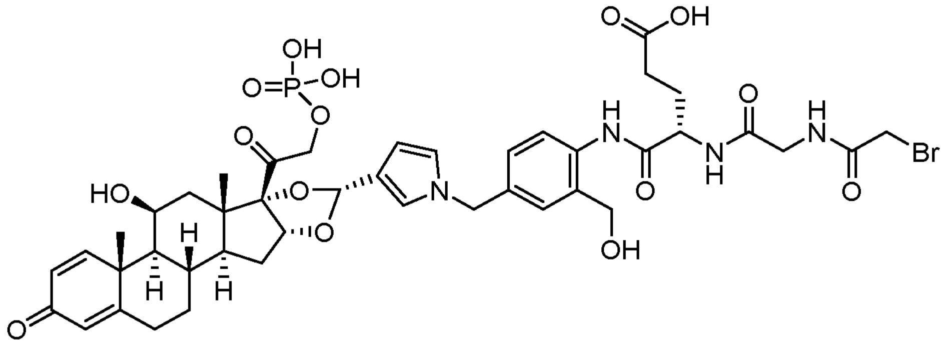 |
| IV-122 | 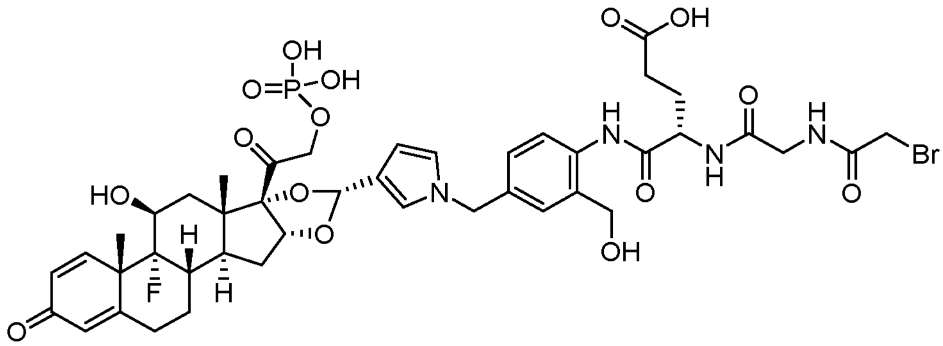 |
| IV-123 | 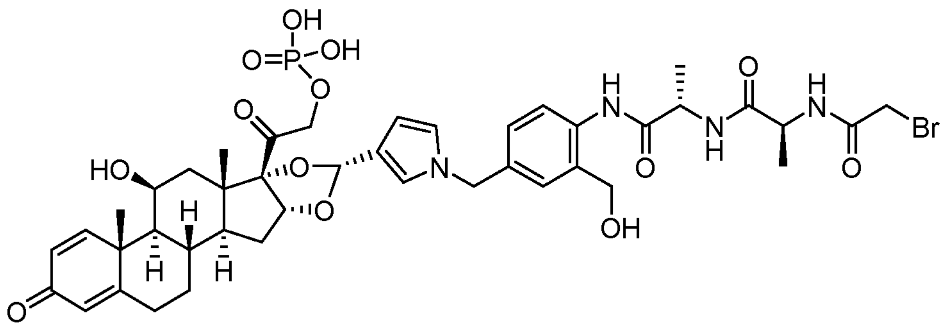 |
| IV-124 | 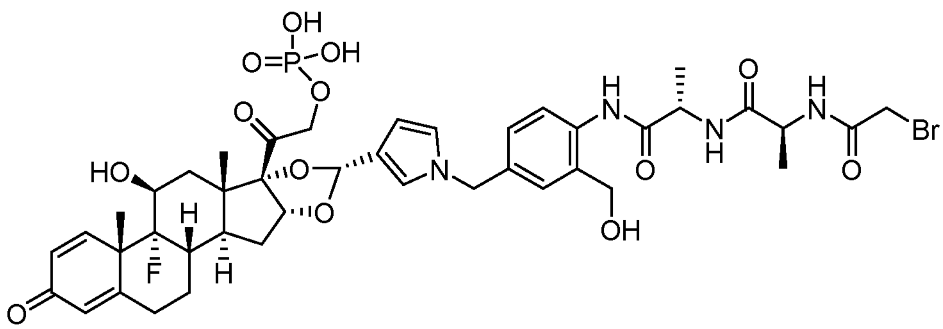 |

(continued)

| No. | Structure |
|---|---|
| IV-125 | 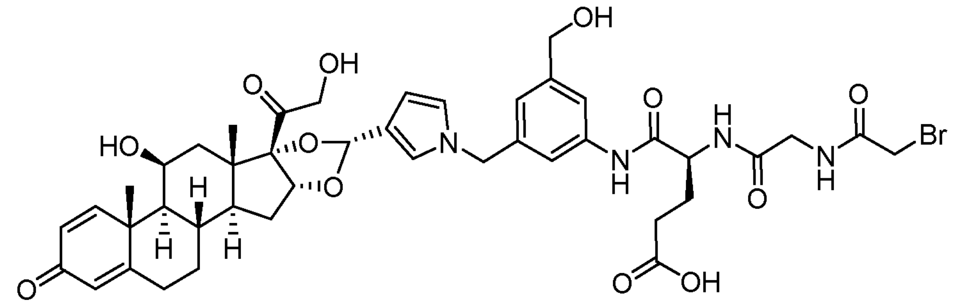 |
| IV-126 | 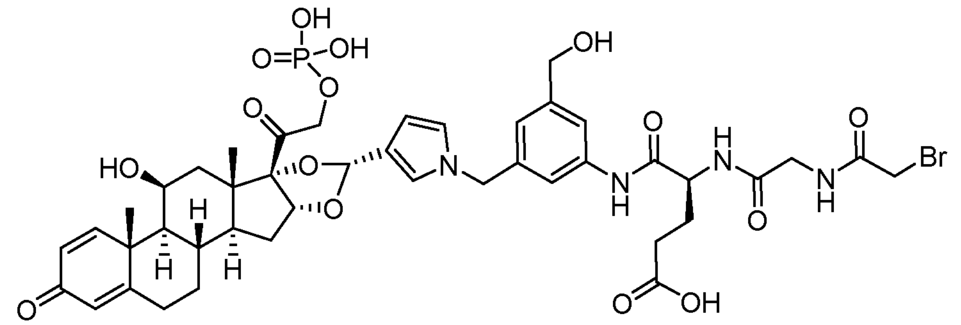 |
| IV-127 | 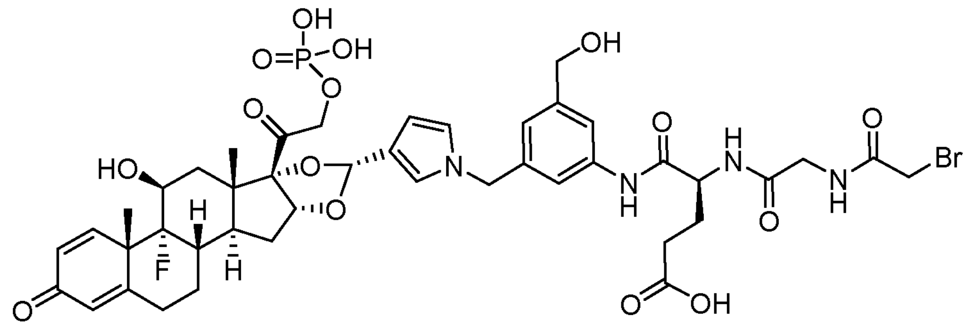 |
| IV-128 | 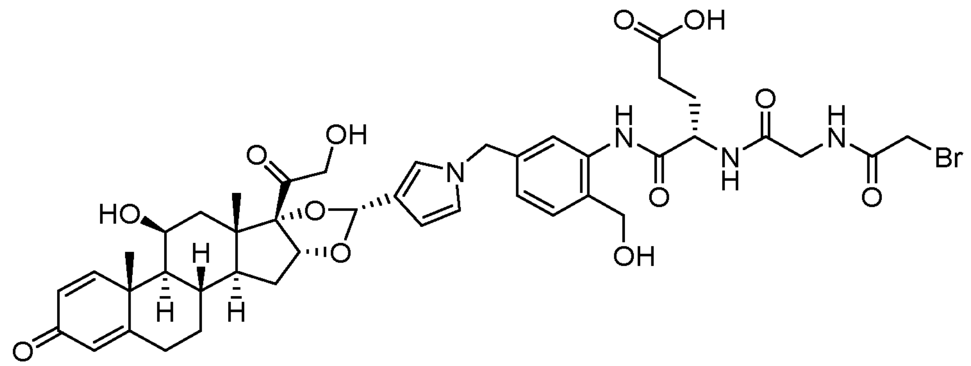 |

(continued)

| No. | Structure |
|---|---|
| IV-129 | 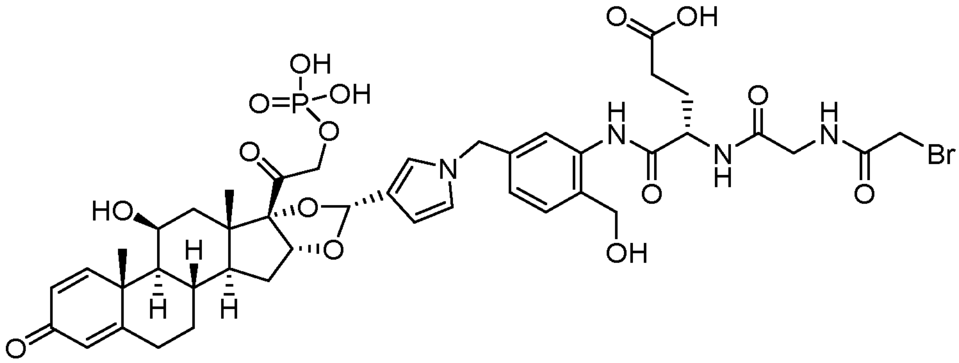 |
| IV-130 | 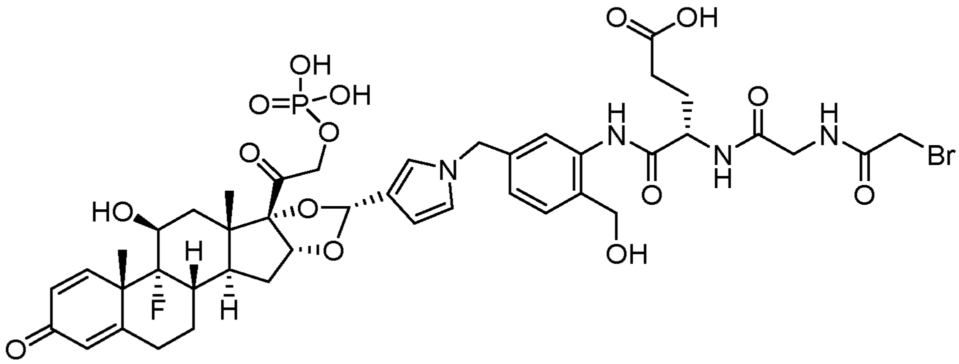 |
| IV-131 | 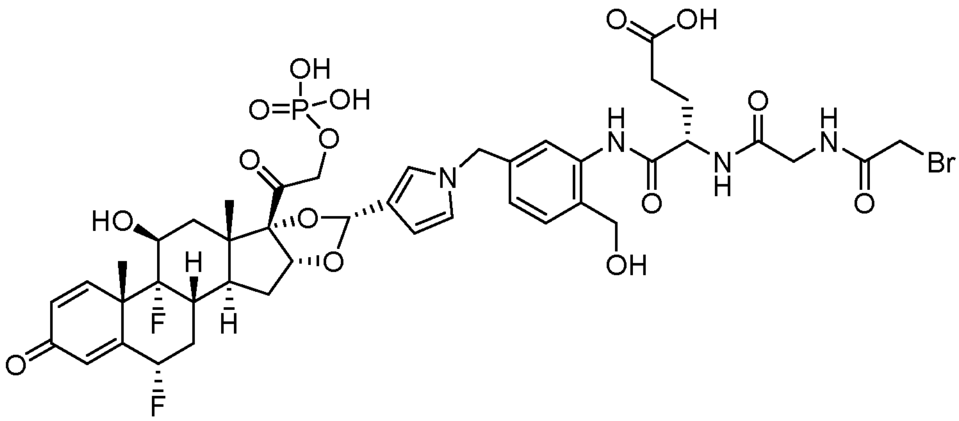 |
| IV-141 | 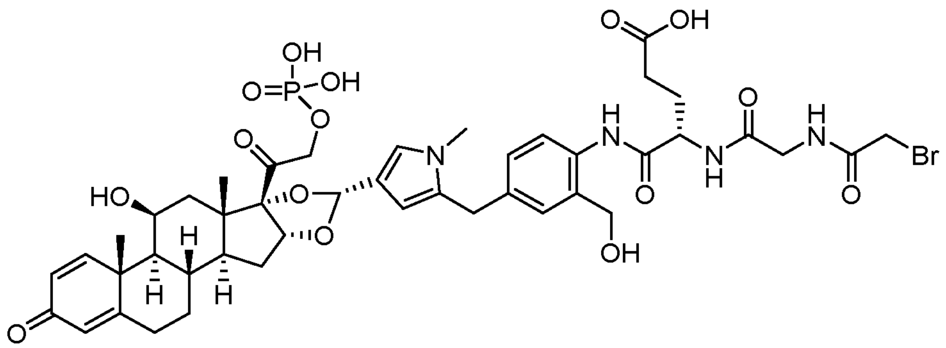 |

(continued)

| No. | Structure |
|---|---|
| IV-142 | 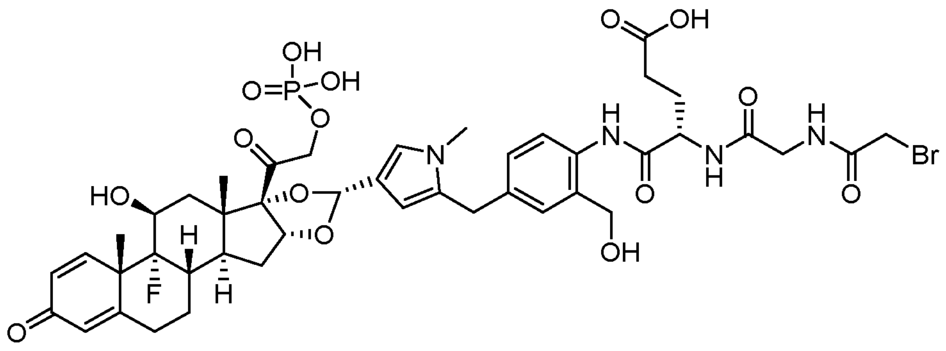 |
| IV-143 | 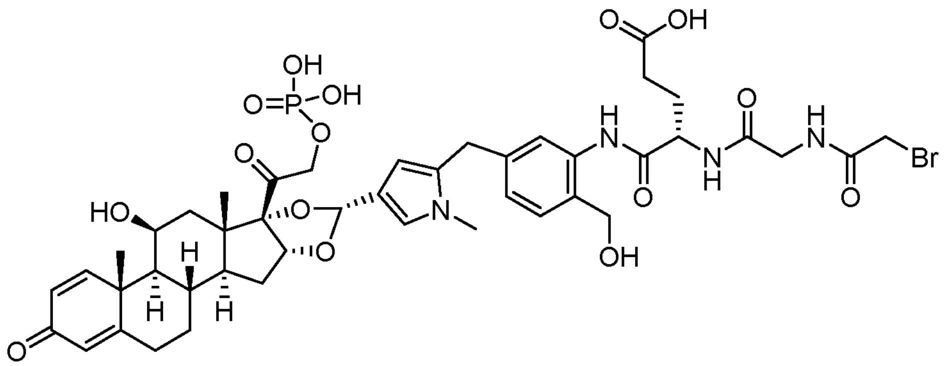 |
| IV-144 | 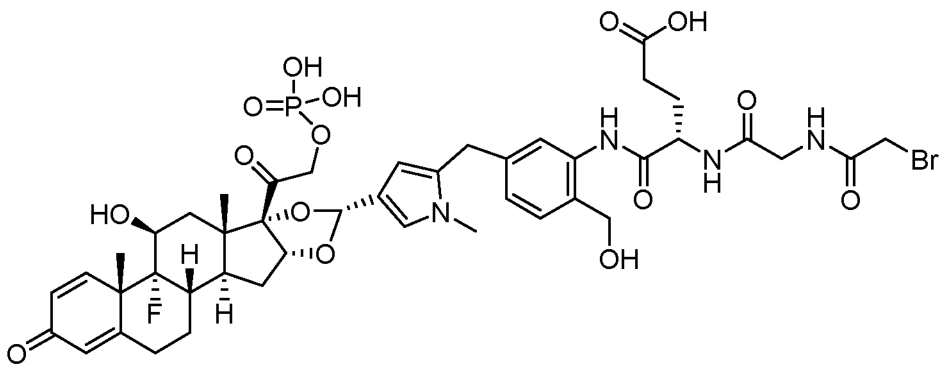 |
| IV-145 | 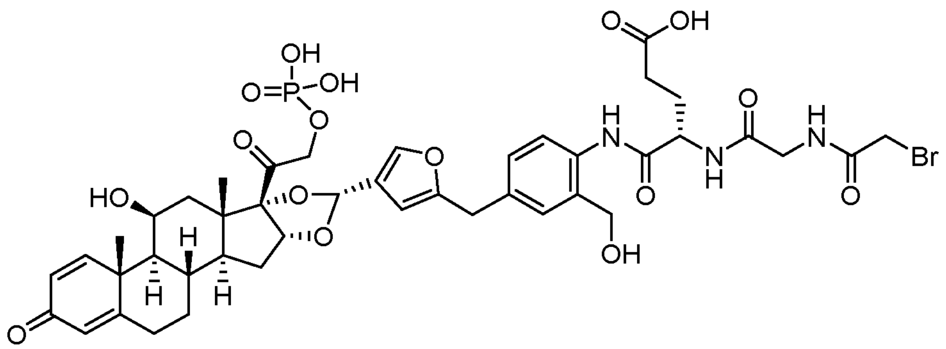 |

(continued)

| No. | Structure |
|---|---|
| IV-146 | 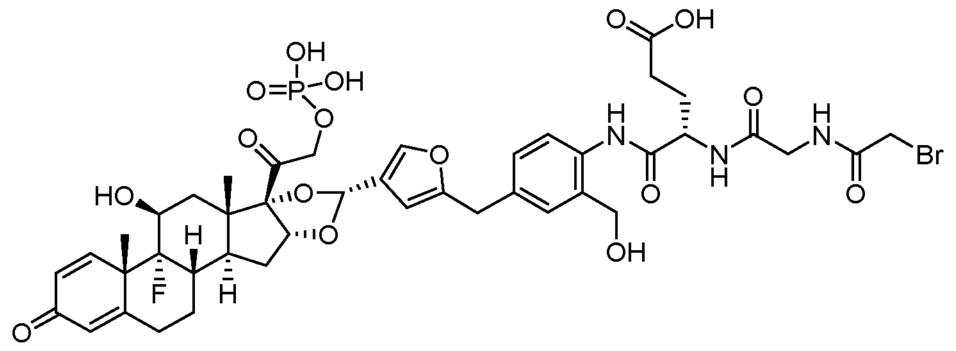 |
| IV-147 | 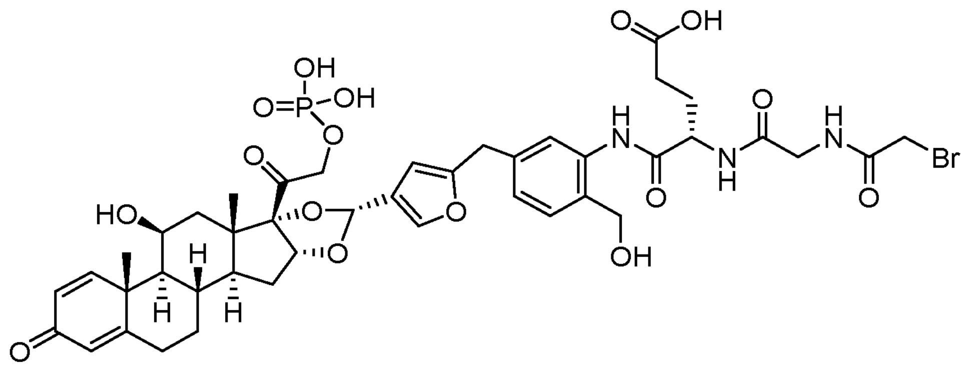 |
| IV-148 | 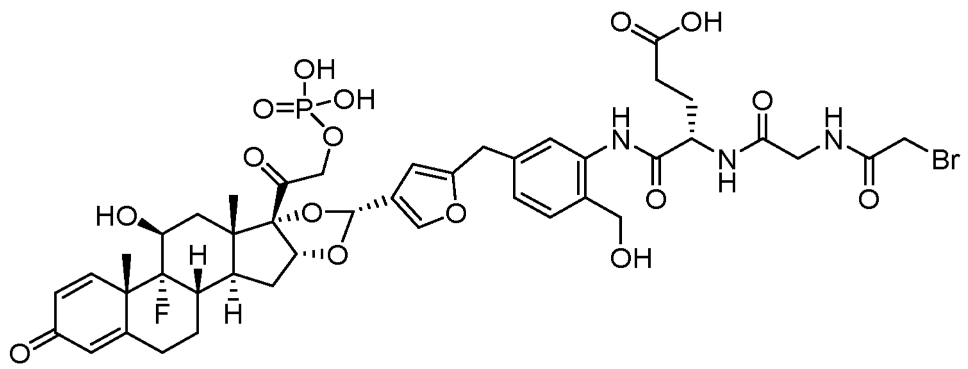 |
| IV-149 | 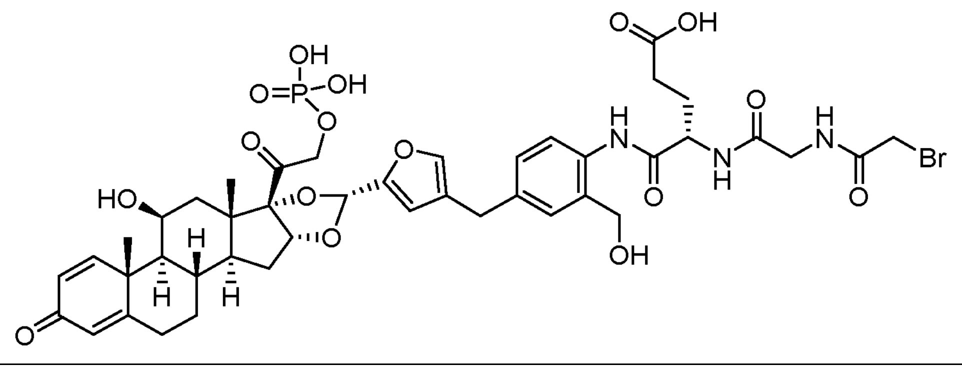 |

(continued)

| No. | Structure |
|---|---|
| IV-150 | 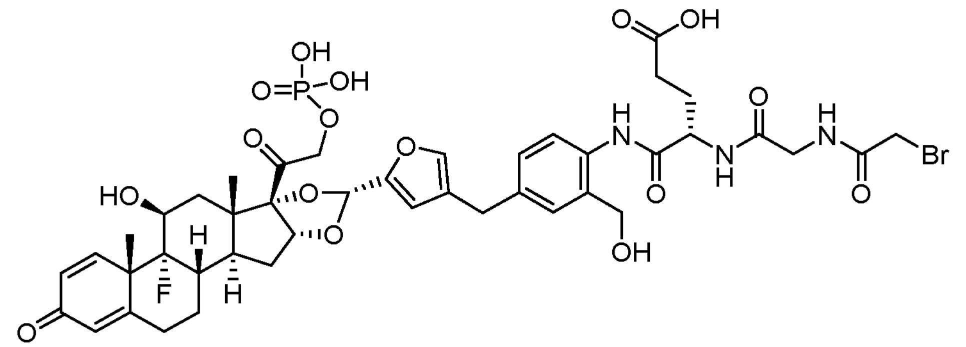 |
| IV-151 | 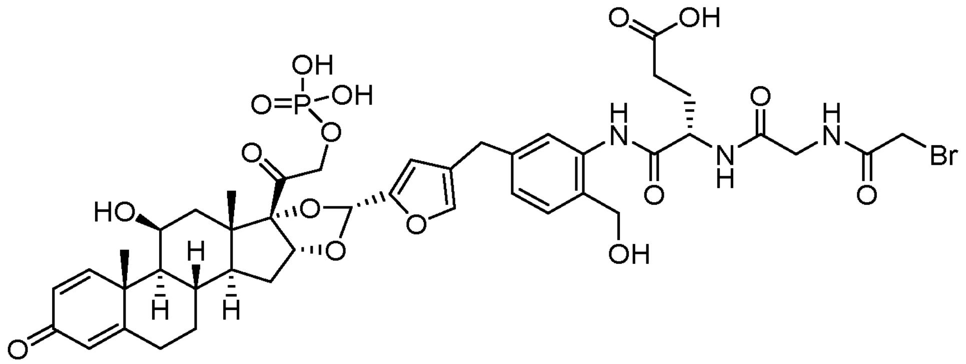 |
| IV-152 | 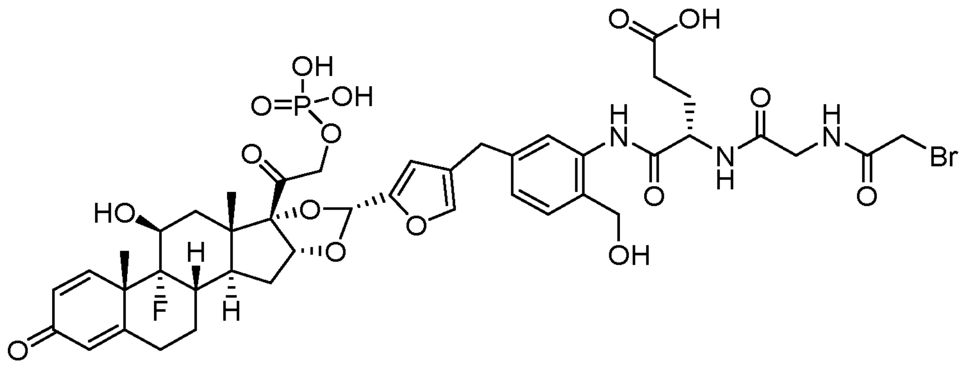 |
| IV-153 | 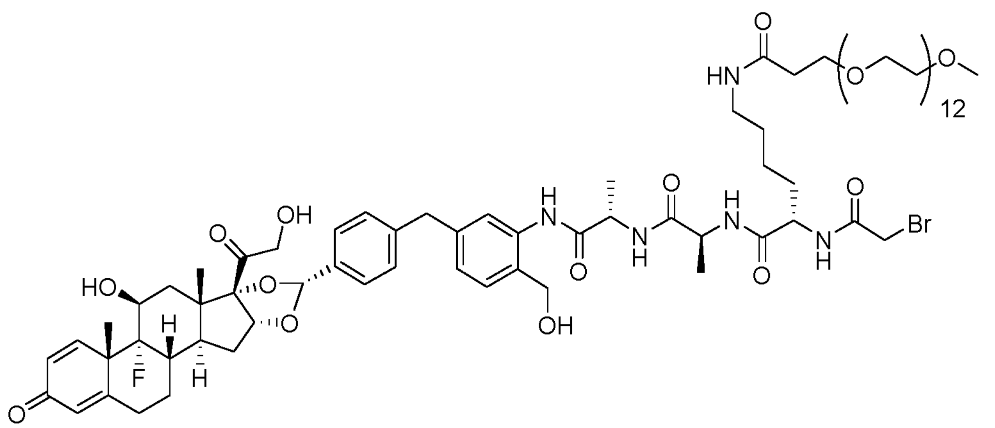 |

(continued)

| No. | Structure |
|---|---|
| IV-154 | 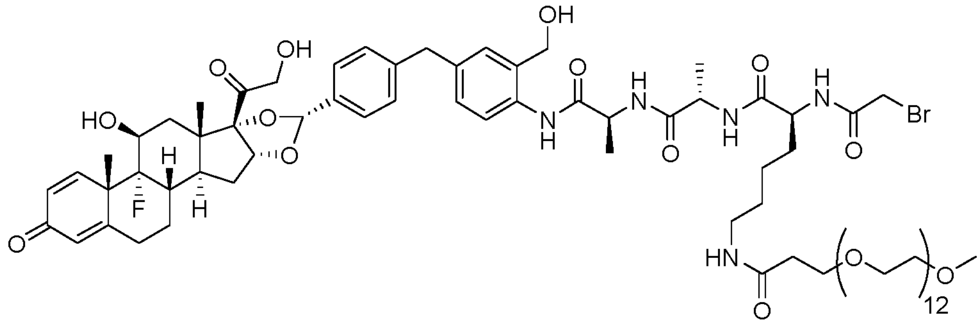 |
| IV-155 | 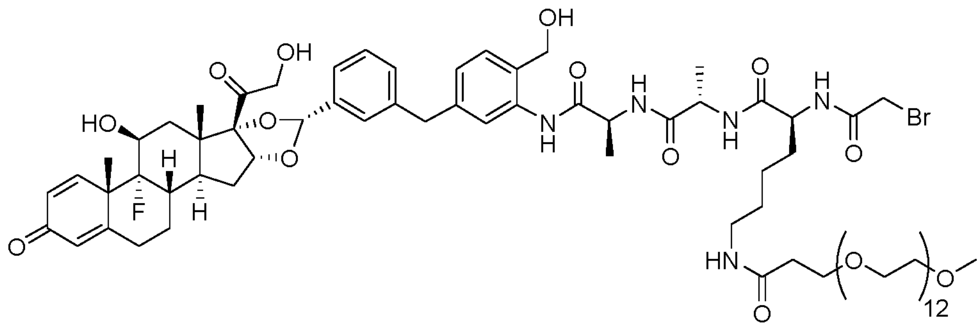 |
| IV-156 | 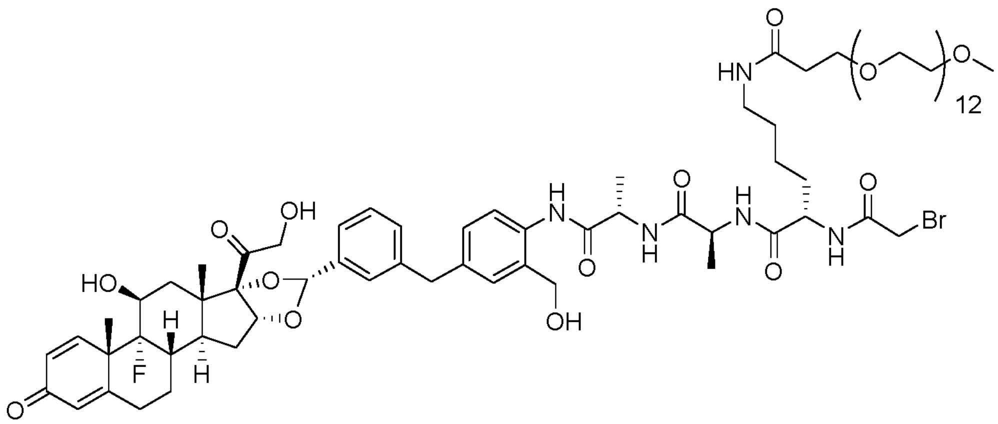 |
| IV-157 | 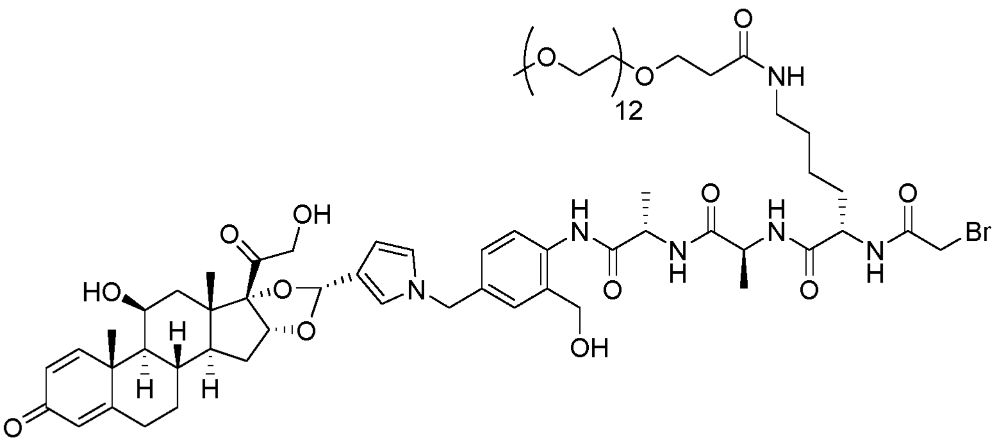 |

(continued)

| No. | Structure |
|---|---|
| IV-158 | 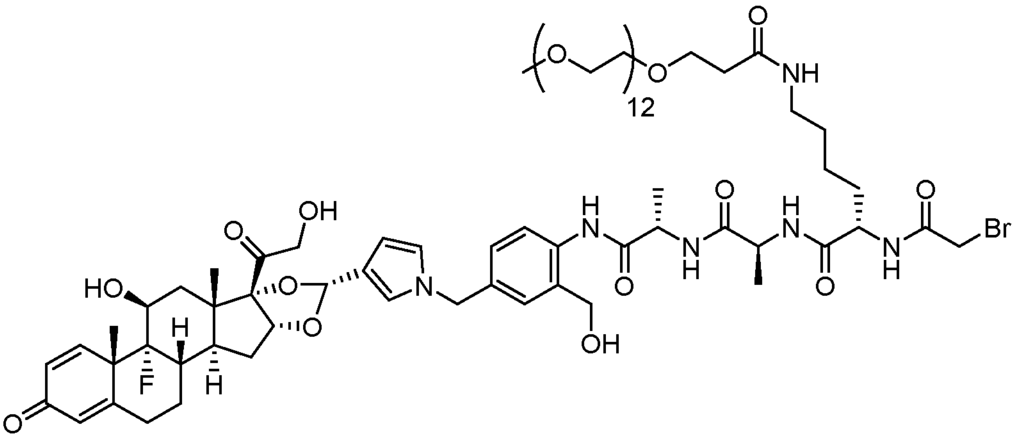 |
| IV-159 | 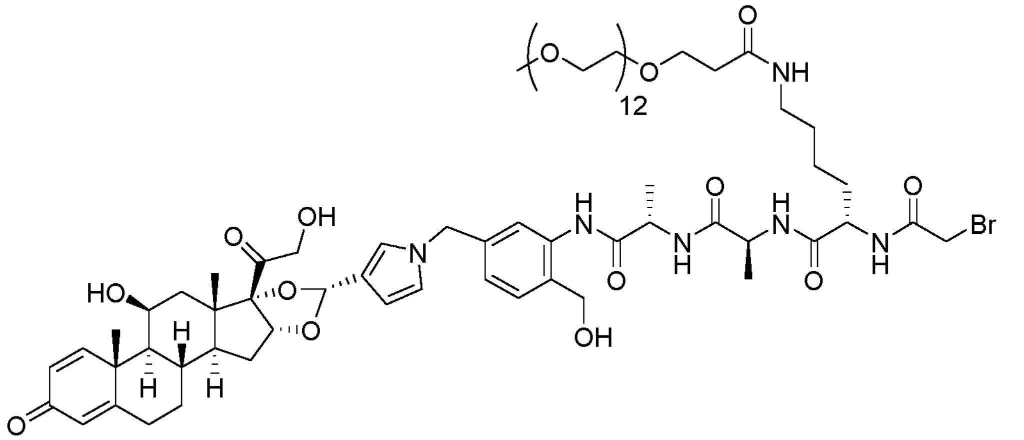 |
| IV-160 | 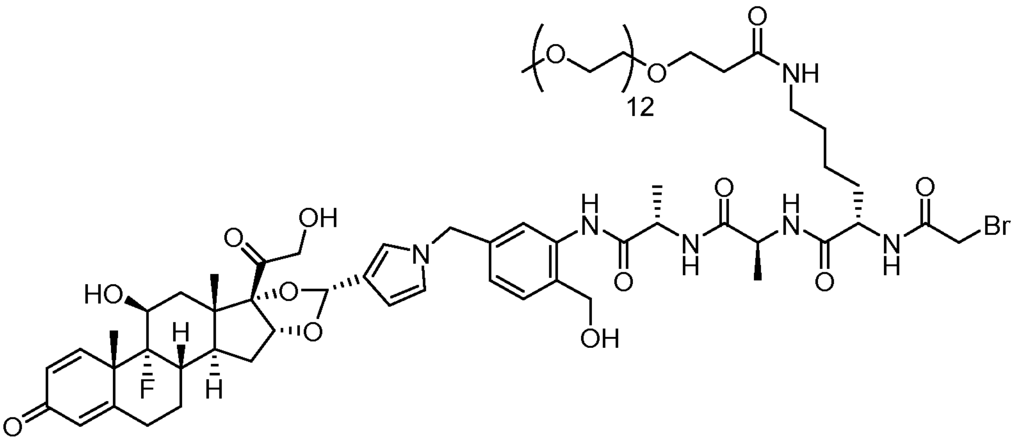 |

In certain embodiments, provided is the compound or the tautomer, the mesomer, the racemate, the enantiomer or the diastereoisomer thereof, or the mixture thereof, or the pharmaceutically acceptable salt thereof of the present invention, wherein the compound of formula IVa or IVb is selected from the group consisting of:

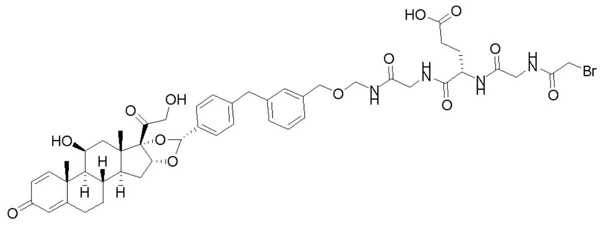

8

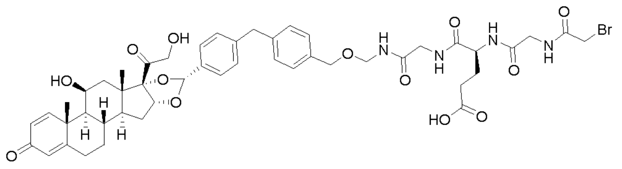

9

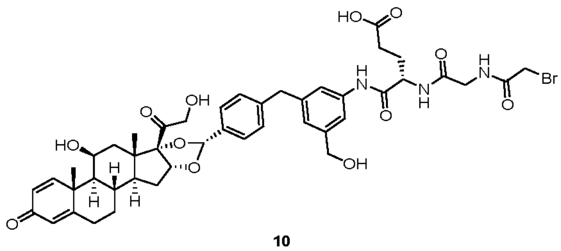

10

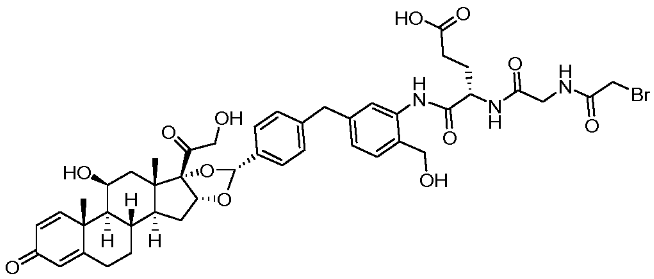

11

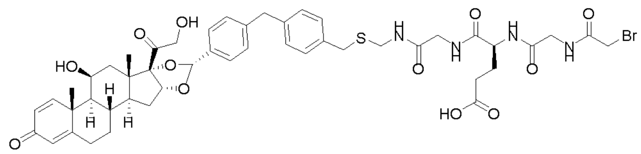

13

.

In certain embodiments, provided is the compound or the tautomer, the mesomer, the racemate, the enantiomer or the diastereoisomer thereof, or the mixture thereof, or the pharmaceutically acceptable salt thereof of the present invention, wherein the compound further comprises a Linker fragment, the compound of formula IIa or IIb is capable of being coupled to the ligand via the Linker fragment, and the compound has the following structure:

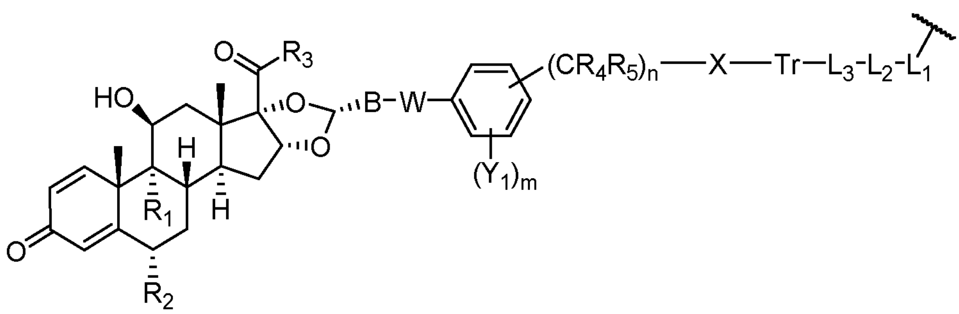

(IVa-1)

or

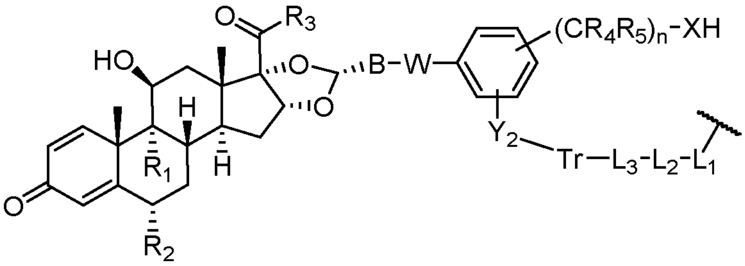

(IVb-2)

wherein,

Tr is absent or Tr is any group;
$L_3$ is selected from a polypeptide fragment;
$L_2$ is absent or selected from a linker fragment;
$L_1$ is selected from a coupling unit; and $L_1$ in the formulas IVa-1 and IVb-1 is in a linked form;
$R_1$, $R_2$, $R_3$, $R_4$, $R_5$, B, W, $CR_4R_5$, n, X, $Y_1$ and $Y_2$ are each as described in any embodiment of the present invention;
the wavy line

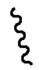

in the general formulas represents being linked to the ligand via the $L_1$ group.

In certain embodiments, provided is the compound or the tautomer, the mesomer, the racemate, the enantiomer or the diastereoisomer thereof, or the mixture thereof, or the pharmaceutically acceptable salt thereof of the present invention, wherein the structural unit -Tr-$L_3$-$L_2$-$L_1$- is selected from the group consisting of:

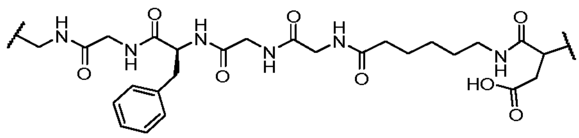

,

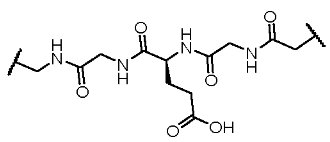

,

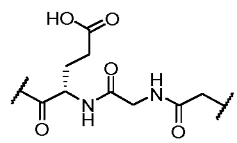

and

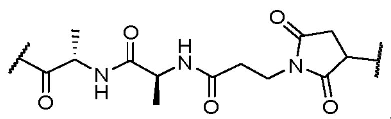

.

In another aspect, the present application provides a conjugate comprising the compound or the tautomer, the mesomer, the racemate, the enantiomer or the diastereoisomer thereof, or the mixture thereof, or the pharmaceutically acceptable salt thereof described above.

In certain embodiments, the conjugate comprises a ligand-drug conjugate. For example, the conjugate may comprise an antibody-drug conjugate.

In certain embodiments, the ligand comprises an antibody or an antigen-binding fragment thereof. In certain embodiments, the antibody is selected from the group consisting of: a human antibody, a humanized antibody, a chimeric antibody, a multispecific antibody, a monoclonal antibody and a polyclonal antibody.

In certain embodiments, the antigen-binding fragment is selected from the group consisting of: a Fab, a Fab', a F(ab')2, an Fv, an scFv, a diabody, an Fd, a dAb, a VHH, a maxibody and a complementarity determining region (CDR) fragment.

In certain embodiments, the ligand specifically binds to an antigen selected from the group consisting of: AXL, BAFFR, BCMA, BCR-list components, BDCA2, BDCA4, BTLA, BTNL2 BTNL3, BTNL8, BTNL9, C10orf54, CCR1, CCR3, CCR4, CCR5, CCR6, CCR7, CCR9, CCR10, CD11c, CD137, CD138, CD14, CD163, CD168, CD177, CD19, CD20, CD209, CD209L, CD22, CD226, CD248, CD25, CD27, CD274, CD276, CD28, CD30, CD300A, CD33, CD37, CD38, CD4, cluster of differentiation 40 (CD40), CD44, CD45, CD46, CD47, CD48, CD5, CD52, CD55, CD56, CD59, CD62E,

CD68, CD69, CD70, CD74, CD79a, CD79b, CD8, CD80, CD86, CD90.2, CD96, CLEC12A, CLEC12B, CLEC7A, CLEC9A, CR1, CR3, CRTAM, CSF1R, CTLA4, CXCR1/2, CXCR4, CXCR5, DDR1, DDR2, DEC-205, DLL4, DR6, FAP, FCamR, FCMR, FcR's, Fire, GITR, HHLA2, HLA class II, HVEM, ICOSLG, IFNAR, type I interferon receptor subunit (IFNAR1), IFNLR1, IL10R1, IL10R2, IL12R, IL13RA1, IL13RA2, IL15R, IL17RA, IL17RB, IL17RC, IL17RE, IL20R1, IL20R2, IL21R, IL22R1, IL22RA, IL23R, IL27R, IL29R, IL2Rg, IL31R, IL36R, IL3RA, IL4R, IL6R, IL5R, IL7R, IL9R, integrins, LAG3, LIFR, MAG/Siglec-4 (sialic acid-binding immunoglobulin-like lectin-4), MMR, MSR1, NCR3LG1, NKG2D, NKp30, NKp46, OX40 (CD134), PDCD1, PROKR1, PVR, PVRIG, PVRL2, PVRL3, RELT, SIGIRR, Siglec-1 (sialic acid-binding immunoglobulin-like lectin-1), Siglec-10, Siglec-5, Siglec-6, Siglec-7, Siglec-8, Siglec-9, SIRPA, SLAMF7, TACI, TCR-list components/assoc, PTCRA, TCRb, CD3z, CD3, TEK, TGFBR1, TGFBR2, TGFBR3, TIGIT, TLR2, TLR4, tumor necrosis factor α (TNFα), TROY, TSLPR, TYRO, VLDLR, VSIG4, IL2R-y and VTCN1.

In certain embodiments, the ligand specifically binds to: TNFα, CD40 and/or IFNAR1.

In certain embodiments, the ligand is selected from the group consisting of: an anti-TNFα antibody or an antigen-binding fragment thereof, an anti-CD40 antibody or an antigen-binding fragment thereof, and an anti-IFNAR1 antibody or an antigen-binding fragment thereof. In certain embodiments, the ligand is selected from the group consisting of: an anti-TNFα antibody or an antigen-binding fragment thereof, an anti-CD40 antibody or an antigen-binding fragment thereof, an anti-BDCA2 antibody or an antigen-binding fragment thereof, and an anti-IFNAR1 antibody or an antigen-binding fragment thereof.

For example, the ligand may be selected from the group consisting of: an anti-TNFα monoclonal antibody, an anti-CD40 monoclonal antibody and an anti-IFNAR1 monoclonal antibody.

The present disclosure also provides an immunoconjugate comprising a glucocorticoid receptor agonist linked to an anti-TNFα protein. In certain embodiments, the anti-TNFα protein is an antibody or an antigen-binding fragment thereof. In certain embodiments, the anti-TNFα protein is an antibody or an antigen-binding fragment thereof that binds to TNFα (e.g., a soluble TNFα and/or a membrane-bound TNFα). In certain embodiments, the anti-TNFα protein is a soluble TNF receptor protein, e.g., a soluble TNF receptor protein fused to a heavy chain constant domain or a fragment thereof (e.g., Fc). In some embodiments, the anti-TNFα protein (e.g., an anti-TNF antibody, an antigen-binding fragment thereof or a soluble TNF receptor) can bind to TNFα on the surface of a cell and become internalized. For example, US2014/0294813 (which is incorporated herein by reference in its entirety) discloses an anti-TNF protein that exhibits internalization upon binding to human TNF on the surface of a cell. In certain embodiments, the antibody or the antigen-binding fragment thereof binds to human and/or mouse TNF-α. Antibodies and antigen-binding fragments that bind to TNF-α are known in the art.

Anti-TNF-α antibodies and antigen-binding fragments thereof include, for example, adalimumab, infliximab, certolizumab pegol, afelimomab, nerelimomab, ozoralizumab, placulumab, golimumab and anti-mouse TNFα mIgG2a. Other anti-TNF-α antibodies and antigen-binding fragments are provided in, for example, WO 2013/087912, WO 2014/152247 and WO 2015/073884, each of which is incorporated herein by reference in its entirety.

Adalimumab is described in U.S. Patent No. 6,258,562, which is incorporated herein by reference in its entirety. Infliximab is described in U.S. Patent No. 5,656,272, which is incorporated herein by reference in its entirety. Certolizumab pegol is discussed in WO 01/94585, which is incorporated herein by reference in its entirety. Afelimomab (also known as MAK195) was discussed in Vincent, Int.J.Clin.Pract. 54: 190-193 (2000), which is incorporated herein by reference in its entirety. Ozoralizumab (also known as ATN-103) is a nanobody. It contains three heavy chain variable regions fused by GlySer linkers. Variable regions 1 and 3 are identical, and ozoralizumab does not contain a heavy chain. Ozoralizumab is discussed in WO 2012/131053, which is incorporated herein by reference in its entirety. Placulumab (also known as CEP-37247) is a domain antibody consisting of a dimer of VL-pCH1-CH2-CH3 or [V-κ]2-Fc, and is discussed in Gay et al., Mabs 2: 625-638 (2010), which is incorporated herein by reference in its entirety. Golimumab (also known as CNTO 148) is discussed in WO2013/087912, and the sequence is provided in GenBank: DI496971.1 and GenBank DI 496970.1, each of which is incorporated herein by reference in its entirety. Anti-mouse TNFα mIgG2a is described in McRae BL et al., J Crohns Colitis 10 (1): 69-76 (2016), each of which is incorporated herein by reference in its entirety.

The anti-TNF-α antibodies and the antigen-binding fragments thereof also include antibodies and antigen-binding fragments thereof that competitively inhibit the binding of adalimumab, infliximab, certolizumab pegol, afelimomab, nerelimomab, ozoralizumab, placulumab or golimumab to TNF-α. The anti-TNF-α antibodies and the antigen-binding fragments thereof also include antibodies and antigen-binding fragments that bind to the same TNF-α epitope as adalimumab, infliximab, certolizumab pegol, afelimomab, nerelimomab, ozoralizumab, placulumab or golimumab.

In certain embodiments, the anti-TNF-α antibody or the antigen-binding fragment thereof competitively inhibits the binding of adalimumab to TNF-α. In certain embodiments, the anti-TNF-α antibody or the antigen-binding fragment thereof binds to the same TNF-α epitope as adalimumab. In certain embodiments, the anti-TNF-α antibody or the antigen-binding fragment thereof is adalimumab or an antigen-binding fragment thereof. In certain embodiments, the anti-TNF-α antibody or the antigen-binding fragment thereof is adalimumab.

In certain embodiments, the anti-TNF-α antibody or the antigen-binding fragment thereof comprises a sequence

of adalimumab, infliximab, certolizumab pegol, afelimomab, nerelimomab, ozoralizumab, placulumab or golimumab, such as a complementarity determining region (CDR), a variable heavy chain domain (VH), and/or a variable light chain domain (VL).

In certain embodiments, the anti-TNFα antibody or the antigen-binding fragment thereof comprises a heavy chain variable region (VH) of the antibody, wherein the heavy chain variable region comprises an HCDR1, an HCDR2 and an HCDR3 that have at least about 80%, about 85%, about 90%, about 95%, about 96%, about 97%, about 98% or about 99% sequence identity to an HCDR1, an HCDR2 and an HCDR3, respectively, of the following molecules: adalimumab, infliximab, afelimomab or golimumab.

In certain embodiments, the anti-TNFα antibody or the antigen-binding fragment thereof comprises a heavy chain variable region of the antibody, wherein the heavy chain variable region has at least about 80%, about 85%, about 90%, about 95%, about 96%, about 97%, about 98% or about 99% sequence identity to a heavy chain variable region of the following molecules: adalimumab, infliximab, afelimomab or golimumab.

In certain embodiments, the anti-TNFα antibody or the antigen-binding fragment thereof comprises a heavy chain of the antibody, wherein the heavy chain has at least about 80%, about 85%, about 90%, about 95%, about 96%, about 97%, about 98% or about 99% sequence identity to a heavy chain of the following molecules: adalimumab, infliximab, afelimomab or golimumab.

In certain embodiments, the anti-TNFα antibody or the antigen-binding fragment thereof comprises a light chain variable region (VL) of the antibody, wherein the light chain variable region comprises an LCDR1, an LCDR2 and an LCDR3 that have at least about 80%, about 85%, about 90%, about 95%, about 96%, about 97%, about 98% or about 99% sequence identity to an LCDR1, an LCDR2 and an LCDR3, respectively, of the following molecules: adalimumab, infliximab, afelimomab or golimumab.

In certain embodiments, the anti-TNFα antibody or the antigen-binding fragment thereof comprises a light chain variable region of the antibody, wherein the light chain variable region has at least about 80%, about 85%, about 90%, about 95%, about 96%, about 97%, about 98% or about 99% sequence identity to a light chain variable region of the following molecules: adalimumab, infliximab, afelimomab or golimumab.

In certain embodiments, the anti-TNFα antibody or the antigen-binding fragment thereof comprises a light chain of the antibody, wherein the light chain has at least about 80%, about 85%, about 90%, about 95%, about 96%, about 97%, about 98% or about 99% sequence identity to a light chain of the following molecules: adalimumab, infliximab, afelimomab or golimumab.

In certain embodiments, the present application provides an antibody or an antigen-binding fragment thereof, which can specifically bind to TNF-α and comprises the Chothia VL CDRs of the VL of adalimumab, infliximab, certolizumab pegol, afelimomab, nerelimomab, ozoralizumab, placulumab or golimumab. In certain aspects, provided herein is an antibody or an antigen-binding fragment thereof, which specifically binds to TNF-α and comprises the Chothia VH CDRs of the VH of adalimumab, infliximab, certolizumab pegol, afelimomab, nerelimomab, ozoralizumab, placulumab or golimumab. In certain aspects, provided herein is an antibody or an antigen-binding fragment thereof, which specifically binds to TNF-α and comprises the Chothia VL CDRs of the VL of adalimumab, infliximab, certolizumab pegol, afelimomab, nerelimomab, ozoralizumab, placulumab or golimumab and the Chothia VH CDRs of the VH of adalimumab, infliximab, certolizumab pegol, afelimomab, nerelimomab, ozoralizumab, placulumab or golimumab.

In certain embodiments, the anti-TNFα antibody or the antigen-binding fragment thereof comprises a heavy chain variable region (VH) of the antibody, wherein the heavy chain variable region comprises an HCDR1, an HCDR2 and an HCDR3 that have at least about 80%, about 85%, about 90%, about 95%, about 96%, about 97%, about 98% or about 99% sequence identity to an HCDR1, an HCDR2 and an HCDR3, respectively, of the following molecules: adalimumab, infliximab, afelimomab, golimumab or anti-mouse TNFα mIgG2a 8c11.

In certain embodiments, the anti-TNFα antibody or the antigen-binding fragment thereof comprises a heavy chain variable region of the antibody, wherein the heavy chain variable region has at least about 80%, about 85%, about 90%, about 95%, about 96%, about 97%, about 98% or about 99% sequence identity to a heavy chain variable region of the following molecules: adalimumab, infliximab, afelimomab, golimumab or anti-mouse TNFα mIgG2a 8c11.

In certain embodiments, the anti-TNFα antibody or the antigen-binding fragment thereof comprises a heavy chain of the antibody, wherein the heavy chain has at least about 80%, about 85%, about 90%, about 95%, about 96%, about 97%, about 98% or about 99% sequence identity to a heavy chain of the following molecules: adalimumab, infliximab, afelimomab, golimumab or anti-mouse TNFα mIgG2a 8c11.

In certain embodiments, the anti-TNFα antibody or the antigen-binding fragment thereof comprises a light chain variable region (VL) of the antibody, wherein the light chain variable region comprises an LCDR1, an LCDR2 and an LCDR3 that have at least about 80%, about 85%, about 90%, about 95%, about 96%, about 97%, about 98% or about 99% sequence identity to an LCDR1, an LCDR2 and an LCDR3, respectively, of the following molecules: adalimumab, infliximab, afelimomab, golimumab or anti-mouse TNFα mIgG2a 8c11.

For example, the anti-TNFα antibody or the antigen-binding fragment thereof comprises a heavy chain variable region (VH) and a light chain variable region (VL) of the antibody, wherein the heavy chain variable region comprises

an HCDR1, an HCDR2 and an HCDR3 and the light chain variable region comprises an LCDR1, an LCDR2 and an LCDR3, and these CDRs have at least about 80%, about 85%, about 90%, about 95%, about 96%, about 97%, about 98% or about 99% sequence identity to an HCDR1, an HCDR2, an HCDR3, an LCDR1, an LCDR2 and an LCDR3, respectively, of the following molecules: adalimumab, infliximab, afelimomab, golimumab or anti-mouse TNFα mIgG2a 8c11.

In certain embodiments, the anti-TNFα antibody or the antigen-binding fragment thereof comprises a light chain variable region of the antibody, wherein the light chain variable region has at least about 80%, about 85%, about 90%, about 95%, about 96%, about 97%, about 98% or about 99% sequence identity to a light chain variable region of the following molecules: adalimumab, infliximab, afelimomab, golimumab or anti-mouse TNFα mIgG2a 8c11.

In certain embodiments, the anti-TNFα antibody or the antigen-binding fragment thereof comprises a heavy chain variable region and a light chain variable region of the antibody, wherein the heavy chain variable region and the light chain variable region respectively have at least about 80%, about 85%, about 90%, about 95%, about 96%, about 97%, about 98% or about 99% sequence identity to a light chain variable region of the following molecules: adalimumab, infliximab, afelimomab, golimumab or anti-mouse TNFα mIgG2a 8c11.

In certain embodiments, the anti-TNFα antibody or the antigen-binding fragment thereof comprises a light chain of the antibody, wherein the light chain has at least about 80%, about 85%, about 90%, about 95%, about 96%, about 97%, about 98% or about 99% sequence identity to a light chain of the following molecules: adalimumab, infliximab, afelimomab, golimumab or anti-mouse TNFα mIgG2a 8c 11.

For example, the anti-TNFα antibody or the antigen-binding fragment thereof comprises a heavy chain and a light chain of the antibody, wherein the heavy chain and the light chain respectively have at least about 80%, about 85%, about 90%, about 95%, about 96%, about 97%, about 98% or about 99% sequence identity to a light chain of the following molecules: adalimumab, infliximab, afelimomab, golimumab or anti-mouse TNFα mIgG2a 8c11.

The sequences of exemplary anti-TNF-α antibodies or antigen-binding fragments thereof are provided in Tables 1-2.

Table 1. Amino acid sequences for adalimumab

| | |
|---|---|
| LCDR1 | RASQGIRNYLA (SEQ ID NO: 1) |
| LCDR2 | AASTLQS (SEQ ID NO: 2) |
| LCDR3 | QRYNRAPYT (SEQ ID NO: 3) |
| HCDR1 | DYAMH (SEQ ID NO: 4) |
| HCDR2 | AITWNSGHIDYADSVEG (SEQ ID NO: 5) |
| HCDR3 | AKVSYLSTASSLDY (SEQ ID NO: 6) |
| VL | DIQMTQSPSSLSASVGDRVTITCRASQGIRNYLAWYQQKPGKAPKLL IYAASTLQSGVPSRFSGSGSGTDFTLTISSLQPEDVATYYCQRYNRAP YTFGQGTKVEIK (SEQ ID NO: 7) |
| VH | EVQLVESGGGLVQPGRSLRLSCAASGFTFDDYAMHWVRQAPGKGL EWVSAITWNSGHIDYADSVEGRFTISRDNAKNSLYLQMNSLRAEDT AVYYCAKVSYLSTASSLDYWGQGTLVTVSS (SEQ ID NO: 8) |
| Light chain | DIQMTQSPSSLSASVGDRVTITCRASQGIRNYLAWYQQKPGKAPKLL IYAASTLQSGVPSRFSGSGSGTDFTLTISSLQPEDVATYYCQRYNRAP YTFGQGTKVEIKRTVAAPSVFIFPPSDEQLKSGTASVVCLLNNFYPRE AKVQWKVDNALQSGNSQESVTEQDSKDSTYSLSSTLTLSKADYEKH KVYACEVTHQGLSSPVTKSFNRGEC (SEQ ID NO: 9) |

(continued)

| | |
|---|---|
| Heavy chain | EVQLVESGGGLVQPGRSLRLSCAASGFTFDDYAMHWVRQAPGKGL EWVSAITWNSGHIDYADSVEGRFTISRDNAKNSLYLQMNSLRAEDT AVYYCAKVSYLSTASSLDYWGQGTLVTVSSASTKGPSVFPLAPSSKS TSGGTAALGCLVKDYFPEPVTVSWNSGALTSGVHTFPAVLQSSGLYS LSSVVTVPSSSLGTQTYICNVNHKPSNTKVDKKVEPKSCDKTHTCPP CPAPELLGGPSVFLFPPKPKDTLMISRTPEVTCVVVDVSHEDPEVKFN WYVDGVEVHNAKTKPREEQYNSTYRVVSVLTVLHQDWLNGKEYK CKVSNKALPAPIEKTISKAKGQPREPQVYTLPPSRDELTKNQVSLTCL |
| | VKGFYPSDIAVEWESNGQPENNYKTTPPVLDSDGSFFLYSKLTVDKS RWQQGNVFSCSVMHEALHNHYTQKSLSLSPGK (SEQ ID NO: 10) |

Table 2. Amino acid sequences for anti-mouse TNFα mIgG2a 8c11

| | |
|---|---|
| Light chain | QIVLSQSPAILSASPGEKVTMTCRASSSVSYMHWFQQKPGSSPKPWIY ATSNLASGVPARFSGSGSGTSYSLTISRVEAEDAATYYCQQWSSSPLT FGAGTKLELKRADAAPTVSIFPPSSEQLTSGGASVVCFLNNFYPKDIN VKWKIDGSERQNGVLNSWTDQDSKDSTYSMSSTLTLTKDEYERHNS YTCEATHKTSTSPIVKSFNRNEC (SEQ ID NO: 31) |
| Heavy chain | EFQLQQSGPELVKPGASVRISCKASGYSFTDYNMNWVKQSNGKSLE WVGVINPNYGSSTYNQKFKGKATLTVDQSSSTAYMQLNSLTSEDSA VYYCARKWGQLGRGFFDVWGTGTTVTVSSAKTTAPSVYPLAPVCG DTTGSSVTLGCLVKGYFPEPVTLTWNSGSLSSGVHTFPAVLQSDLYT LSSSVTVTSSTWPSQSITCNVAHPASSTKVDKKIEPRGPTIKPCPPCKC PAPNLLGGPSVFIFPPKIKDVLMISLSPIVTCVVVDVSEDDPDVQISWF VNNVEVHTAQTQTHREDYNSTLRVVSALPIQHQDWMSGKEFKCKV NNKDLPAPIERTISKPKGSVRAPQVYVLPPPEEEMTKKQVTLTCMVT DFMPEDIYVEWTNNGKTELNYKNTEPVLDSDGSYFMYSKLRVEKKN WVERNSYSCSVVHEGLHNHHTTKSFSRTPGK(SEQ ID NO: 32) |

In an embodiment, the antibody or the antigen-binding fragment thereof is an antagonist antibody or an antigen-binding fragment thereof that causes a reduction in CD40 activity or function as compared to CD40 activity or function

in the absence of the antibody or the antigen-binding fragment thereof. In particular embodiments, the antibody or the antigen-binding fragment thereof is substantially free of agonist activity, i.e., the antibody or the antigen-binding fragment thereof does not cause an increase in the amount of CD40 activity or function as compared to CD40 activity or function in the absence of the antibody or the antigen-binding fragment thereof. In certain embodiments, the anti-CD40 antibody is a polyclonal antibody, a monoclonal antibody, a chimeric antibody, a humanized antibody, a human antibody, or an antigen-binding fragment thereof.

In certain embodiments, the anti-CD40 antibody is iscalimab (CFZ533) (Novartis; as described in U.S. Pat. Nos. 8828396 and 9221913); the anti-CD40 antibody is lucatumumab (Novartis; as described in U.S. Pat. No. 8277810); Antibodies 5D12, 3A8 and 3C6, or humanized versions thereof (Novartis; as described in U.S. Pat. No. 5874082); Antibody 15B8 (Novartis; as described in U.S. Pat. No. 7445780); Antibody 4D11 (Kyowa Hakko Kirin; as described in U.S. Pat. No. 7193064); teneliximab (Bristol Myers Squibb; as described in U.S. Pat. No. 6051228); Antibody PG102 (PanGenetics; as described in U.S. Pat. No. 8669352); Antibody 2C10 (Primatope; U.S. Patent Application No. 20140093497); anti-CD40 antibodies described in U.S. Pat. Nos. 8591900 and 8778345 (Boehringer Ingelheim); anti-CD40 antibodies (Amgen) described in U.S. Pat. No. 5801227; or APX005 (Boehringer Ingelheim; as described in U.S. Patent Application No. 20120301488.

In certain embodiments, the anti-CD40 antibody or the antigen-binding fragment thereof comprises a heavy chain variable region (VH) of the antibody, wherein the heavy chain variable region comprises HCDR1, HCDR2 and HCDR3, each of which respectively has at least about 80%, about 85%, about 90%, about 95%, about 96%, about 97%, about 98% or about 99% sequence identity to HCDR1, HCDR2 and HCDR3 of the following molecule: iscalimab (CFZ533).

In certain embodiments, the anti-CD40 antibody or the antigen-binding fragment thereof comprises a heavy chain variable region of the antibody, wherein the heavy chain variable region has at least about 80%, about 85%, about 90%, about 95%, about 96%, about 97%, about 98% or about 99% sequence identity to a heavy chain variable region of the following molecule: iscalimab (CFZ533). In certain embodiments, the anti-CD40 antibody or the antigen-binding fragment thereof comprises a heavy chain of the antibody, wherein the heavy chain has at least about 80%, about 85%, about 90%, about 95%, about 96%, about 97%, about 98% or about 99% sequence identity to a heavy chain of the following molecule: iscalimab (CFZ533).

In certain embodiments, the anti-CD40 antibody or the antigen-binding fragment thereof comprises a light chain variable region (VL) of the antibody, wherein the light chain variable region comprises LCDR1, LCDR2 and LCDR3, each of which respectively has at least about 80%, about 85%, about 90%, about 95%, about 96%, about 97%, about 98% or about 99% sequence identity to LCDR1, LCDR2 and LCDR3 of the following molecule: iscalimab (CFZ533).

In certain embodiments, the anti-CD40 antibody or the antigen-binding fragment thereof comprises a light chain variable region of the antibody, wherein the light chain variable region has at least about 80%, about 85%, about 90%, about 95%, about 96%, about 97%, about 98% or about 99% sequence identity to a light chain variable region of the following molecule: iscalimab (CFZ533).

In certain embodiments, the anti-CD40 antibody or the antigen-binding fragment thereof comprises a light chain of the antibody, wherein the light chain has at least about 80%, about 85%, about 90%, about 95%, about 96%, about 97%, about 98% or about 99% sequence identity to a light chain of the following molecule: iscalimab (CFZ533).

The sequences of exemplary anti-CD40 antibodies or antigen-binding fragments thereof are provided in Table 3.

Table 3. Amino acid sequences for CFZ533 (Kabat)

| | |
|---|---|
| LCDR1 | RSSQSLLYSNGYNYLD (SEQ ID NO: 11) |
| LCDR2 | LGSNRAS (SEQ ID NO: 12) |
| LCDR3 | MQARQTPFT (SEQ ID NO: 13) |
| HCDR1 | SYGMH (SEQ ID NO: 14) |
| HCDR2 | VISYEESNRYHADSVKG (SEQ ID NO: 15) |
| HCDR3 | DGGIAAPGPDY (SEQ ID NO: 16) |
| VH | QVQLVESGGGVVQPGRSLRLSCAASGFTFSSYGMHWVRQAPGKGLE WVAVISYEESNRYHADSVKGRFTISRDNSKITLYLQMNSLRTEDTAV YYCARDGGIAAPGPDYWGQGTLVTVSS (SEQ ID NO: 17) |

(continued)

| | |
|---|---|
| VL | DIVMTQSPLSLTVTPGEPASISCRSSQSLLYSNGYNYLDWYLQKPGQS PQVLISLGSNRASGVPDRFSGSGSGTDFTLKISRVEAEDVGVYYCMQ ARQTPFTFGPGTKVDIR(SEQ ID NO: 18) |
| Light chain | DIVMTQSPLSLTVTPGEPASISCRSSQSLLYSNGYNYLDWYLQKPGQS PQVLISLGSNRASGVPDRFSGSGSGTDFTLKISRVEAEDVGVYYCMQ ARQTPFTFGPGTKVDIRRTVAAPSVFIFPPSDEQLKSGTASVVCLLNN FYPREAKVQWKVDNALQSGNSQESVTEQDSKDSTYSLSSTLTLSKA DYEKHKVYACEVTHQGLSSPVTKSFNRGEC (SEQ ID NO: 19) |
| Heavy chain | QVQLVESGGGVVQPGRSLRLSCAASGFTFSSYGMHWVRQAPGKGLE WVAVISYEESNRYHADSVKGRFTISRDNSKITLYLQMNSLRTEDTAV YYCARDGGIAAPGPDYWGQGTLVTVSSASTKGPSVFPLAPSSKSTSG GTAALGCLVKDYFPEPVTVSWNSGALTSGVHTFPAVLQSSGLYSLSS VVTVPSSSLGTQTYICNVNHKPSNTKVDKRVEPKSCDKTHTCPPCPA PELLGGPSVFLFPPKPKDTLMISRTPEVTCVVVDVSHEDPEVKFNWY VDGVEVHNAKTKPREEQYASTYRVVSVLTVLHQDWLNGKEYKCKV SNKALPAPIEKTISKAKGQPREPQVYTLPPSREEMTKNQVSLTCLVKG FYPSDIAVEWESNGQPENNYKTTPPVLDSDGSFFLYSKLTVDKSRWQ QGNVFSCSVMHEALHNHYTQKSLSLSPGK (SEQ ID NO: 20) |

In an embodiment, the antibodies of the present invention are specific for (i.e., specifically binds to) IFNAR1. Such antibodies may also be referred to herein as "anti-IFNAR1 antibodies of the present invention". In another embodiment, the antibodies of the present invention are specific for human IFNAR1. In another embodiment, the anti-IFNAR1 antibodies of the present invention may cross-react with IFNAR1 of a species other than human or of other proteins structurally related to human IFNAR1 (e.g., human IFNAR1 homologs). In other embodiments, the anti-IFNAR1 antibodies of the present invention may be specific for human IFNAR1 only, and do not exhibit cross-reactivity with species or other types of cross-reactivity.

Selection sequences for anti-IFNAR1 antibodies may be found in U.S. Pat. No. 5,235,038 and U.S. Patent Application No. 5,919,453. The entire contents of U.S. Pat. Nos. 10831,459, 10/182,058, 11157,494 and 11521,102 are incorporated herein by reference in their entirety for all purposes.

In certain embodiments, the anti-IFNAR1 antibody or the antigen-binding fragment thereof comprises a heavy chain variable region (VH) of the antibody, wherein the heavy chain variable region comprises HCDR1, HCDR2 and HCDR3, each of which respectively has at least about 80% sequence identity to HCDR1, HCDR2 and HCDR3 of the following molecule: anifrolumab (MEDI-546).

In certain embodiments, the anti-IFNAR1 antibody or the antigen-binding fragment thereof comprises a heavy chain variable region of the antibody, wherein the heavy chain variable region has at least about 80%, about 85%, about 90%, about 95%, about 96%, about 97%, about 98% or about 99% sequence identity to a heavy chain variable region of the following molecule: anifrolumab (MEDI-546).

In certain embodiments, the anti-IFNAR1 antibody or the antigen-binding fragment thereof comprises a heavy chain of the antibody, wherein the heavy chain has at least about 80%, about 85%, about 90%, about 95%, about 96%,

about 97%, about 98% or about 99% sequence identity to a heavy chain of the following molecule: anifrolumab (MEDI-546).

In certain embodiments, the anti-IFNAR1 antibody or the antigen-binding fragment thereof comprises a light chain variable region (VL) of the antibody, wherein the light chain variable region comprises LCDR1, LCDR2 and LCDR3, each of which respectively has at least about 80%, about 85%, about 90%, about 95%, about 96%, about 97%, about 98% or about 99% sequence identity to LCDR1, LCDR2 and LCDR3 of the following molecule: anifrolumab (MEDI-546).

In certain embodiments, the anti-IFNAR1 antibody or the antigen-binding fragment thereof comprises a light chain variable region of the antibody, wherein the light chain variable region has at least about 80%, about 85%, about 90%, about 95%, about 96%, about 97%, about 98% or about 99% sequence identity to a light chain variable region of the following molecule: anifrolumab (MEDI-546).

In certain embodiments, the anti-IFNAR1 antibody or the antigen-binding fragment thereof comprises a light chain of the antibody, wherein the light chain has at least about 80%, about 85%, about 90%, about 95%, about 96%, about 97%, about 98% or about 99% sequence identity to a light chain of the following molecule: anifrolumab (MEDI-546).

The sequences of exemplary anti-IFNAR1 antibodies or antigen-binding fragments thereof are provided in Table 4.

Table 4. Amino acid sequences for anifrolumab (Kabat)

| | |
|---|---|
| LCDR1 | RASQSVSSSFFA (SEQ ID NO: 21) |
| LCDR2 | GASSRAT (SEQ ID NO: 22) |
| LCDR3 | QQYDSSAIT (SEQ ID NO: 23) |
| HCDR1 | NYWIA (SEQ ID NO: 24) |
| HCDR2 | IIYPGDSDIRYSPSFQG (SEQ ID NO: 25) |
| HCDR3 | HDIEGFDY (SEQ ID NO: 26) |
| VH | EVQLVQSGAEVKKPGESLKISCKGSGYIFTNYWIAWVRQMPGKGLESMGIIYPGDSDIRYSPSFQGQVTISADKSITTAYLQWSSLKASDTAMYYCARHDIEGFDYWGRGTLVTVSS (SEQ ID NO: 27) |
| VL | EIVLTQSPGTLSLSPGERATLSCRASQSVSSSFFAWYQQKPGQAPRLLIYGASSRATGIPDRLSGSGSGTDFTLTITRLEPEDFAVYYCQQYDSSAITFGQGTRLEIK(SEQ ID NO: 28) |
| Light chain | EIVLTQSPGTLSLSPGERATLSCRASQSVSSSFFAWYQQKPGQAPRLLIYGASSRATGIPDRLSGSGSGTDFTLTITRLEPEDFAVYYCQQYDSSAITFGQGTRLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC(SEQ ID NO: 29) |

(continued)

| | |
|---|---|
| Heavy chain | EVQLVQSGAEVKKPGESLKISCKGSGYIFTNYWIAWVRQMPGKGLESMGIIYPGDSDIRYSPSFQGQVTISADKSITTAYLQWSSLKASDTAMYYCARHDIEGFDYWGRGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPEFEGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPASIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK(SEQ ID NO: 30) |

In certain embodiments, the anti-BDCA2 antibody or the antigen-binding fragment thereof comprises a heavy chain variable region of the antibody, wherein the heavy chain variable region has at least about 80% sequence identity to a heavy chain variable region of the following molecule: litifilimab (BIIB059). In certain embodiments, the anti-BDCA2 antibody or the antigen-binding fragment thereof comprises a light chain variable region of the antibody, wherein the light chain variable region has at least about 80% sequence identity to a light chain variable region of the following molecule: litifilimab (BIIB059).

In certain embodiments, the anti-BDCA2 antibody or the antigen-binding fragment thereof comprises a heavy chain of the antibody, wherein the heavy chain has at least about 80% sequence identity to a heavy chain of the following molecule: litifilimab (BIIB059). In certain embodiments, the anti-BDCA2 antibody or the antigen-binding fragment thereof comprises a light chain of the antibody, wherein the light chain has at least about 80% sequence identity to a light chain of the following molecule: litifilimab (BIIB059).

In certain embodiments, the anti-BDCA2 antibody or the antigen-binding fragment thereof comprises a heavy chain variable region (VH) of the antibody, wherein the heavy chain variable region has at least about 80%, about 85%, about 90%, about 95%, about 96%, about 97%, about 98% or about 99% sequence identity to a heavy chain variable region of the following molecule: litifilimab (BIIB059). In certain embodiments, the anti-BDCA2 antibody or the antigen-binding fragment thereof comprises a light chain variable region (VL) of the antibody, wherein the light chain variable region has at least about 80%, about 85%, about 90%, about 95%, about 96%, about 97%, about 98% or about 99% sequence identity to a light chain variable region of the following molecule: litifilimab (BIIB059). In certain embodiments, the anti-BDCA2 antibody or the antigen-binding fragment thereof comprises a heavy chain of the antibody, wherein the heavy chain has at least about 80%, about 85%, about 90%, about 95%, about 96%, about 97%, about 98% or about 99% sequence identity to a heavy chain of the following molecule: litifilimab (BIIB059).

In certain embodiments, the anti-BDCA2 antibody or the antigen-binding fragment thereof comprises a light chain of the antibody, wherein the light chain has at least about 80%, about 85%, about 90%, about 95%, about 96%, about 97%, about 98% or about 99% sequence identity to a light chain of the following molecule: litifilimab (BIIB059).

The sequences of exemplary anti-BDCA2 antibodies or antigen-binding fragments thereof are provided in Table 5.

Table 5. Amino acid sequences for litifilimab (BIIB059)

| | |
|---|---|
| VL | DIQLTQSPSSLSASVGDRVTITCKASQSVDYDGDSYMNWYQQKPGKAPKLLIYAASTLESGVPSRFSGSGSGTDFTLTISSLQPEDFATYYCQQANEDPRTFGQGTKVEIK(SEQ ID NO: 41) |

(continued)

| | |
|---|---|
| VH | DVQLVESGGGLVKPGGSLRLSCAASGFTFSTYTMSWVRQAPGKGLE WVATISPGDSFGYYYPDSVQGRFTISRDNAKNSLYLQMNSLRAEDTA VYYCTRDIYYNYGAWFAYWGQGTLVTVSS(SEQ ID NO: 36) |
| Light chain | DIQLTQSPSSLSASVGDRVTITCKASQSVDYDGDSYMNWYQQKPGK APKLLIYAASTLESGVPSRFSGSGSGTDFTLTISSLQPEDFATYYCQQA NEDPRTFGQGTKVEIKRTVAAPSVFIFPPSDEQLKSGTASVVCLLNNF YPREAKVQWKVDNALQSGNSQESVTEQDSKDSTYSLSSTLTLSKAD YEKHKVYACEVTHQGLSSPVTKSFNRGEC(SEQ ID NO: 42) |
| Heavy chain | DVQLVESGGGLVKPGGSLRLSCAASGFTFSTYTMSWVRQAPGKGLE WVATISPGDSFGYYYPDSVQGRFTISRDNAKNSLYLQMNSLRAEDTA VYYCTRDIYYNYGAWFAYWGQGTLVTVSSASTKGPSVFPLAPSSKS TSGGTAALGCLVKDYFPEPVTVSWNSGALTSGVHTFPAVLQSSGLYS LSSVVTVPSSSLGTQTYICNVNHKPSNTKVDKKVEPKSCDKTHTCPP CPAPELLGGPSVFLFPPKPKDTLMISRTPEVTCVVVDVSHEDPEVKFN WYVDGVEVHNAKTKPREEQYNSTYRVVSVLTVLHQDWLNGKEYK CKVSNKALPAPIEKTISKAKGQPREPQVYTLPPSRDELTKNQVSLTCL VKGFYPSDIAVEWESNGQPENNYKTTPPVLDSDGSFFLYSKLTVDKS RWQQGNVFSCSVMHEALHNHYTQKSLSLSPG(SEQ ID NO: 37) |

The present application also encompasses variants and equivalents that are substantially homologous to the anti-TNF-α antibody, the anti-CD40 antibody or the anti-IFNAR1 antibody set forth herein. Such variants and equivalents may contain, for example, conservative substitution mutations, i.e., substitution of one or more amino acids with a similar amino acid. For example, the conservative substitution refers to substitution of an amino acid with another amino acid of the same class, such as substitution of an acidic amino acid with another acidic amino acid, substitution of a basic amino acid with another basic amino acid, or substitution of a neutral amino acid with another neutral amino acid. The purpose of conservative amino acid substitutions is well known in the art. The antibody may be a recombinant polypeptide, a natural polypeptide or a synthetic polypeptide of the antibody. It will be appreciated in the art that some amino acid sequences of the present application may be altered without significantly affecting the structure or function of the protein. Thus, the present application further comprises variants of the polypeptides, which exhibit significant activity or which comprise regions of the antibodies. Such mutants include deletions, insertions, inversions, repeats and type substitutions.

In certain embodiments, the ligand is selected from the group consisting of: adalimumab, iscalimab (CFZ533), anifrolumab (MEDI-546), infliximab, afelimomab, golimumab, anti-mouse TNFα mIgG2a 8c11, litifilimab (BIIB059), and derivatives and biosimilars thereof.

For example, the ligand is selected from the group consisting of: adalimumab, iscalimab (CFZ533) and anifrolumab (MEDI-546).

In certain embodiments, the ligand-drug conjugate has the following structure:

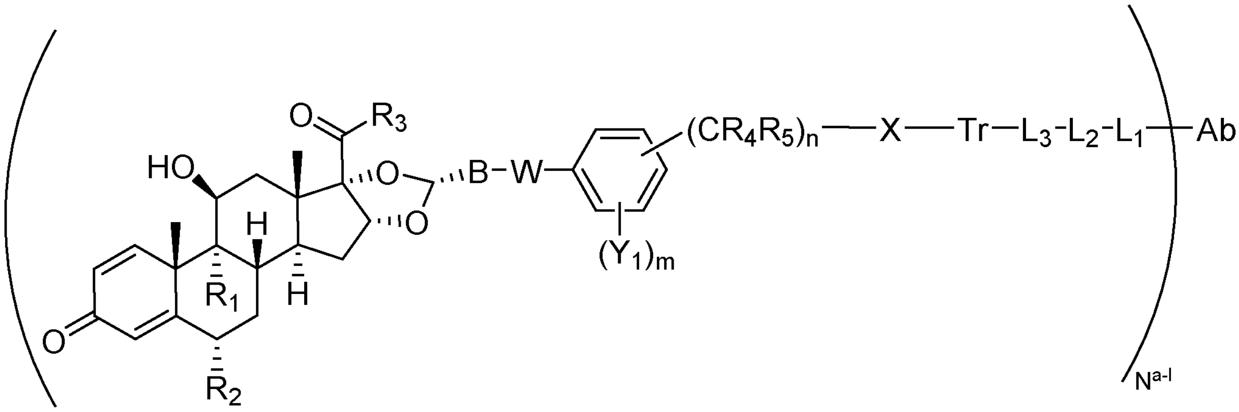

, or

(Va)

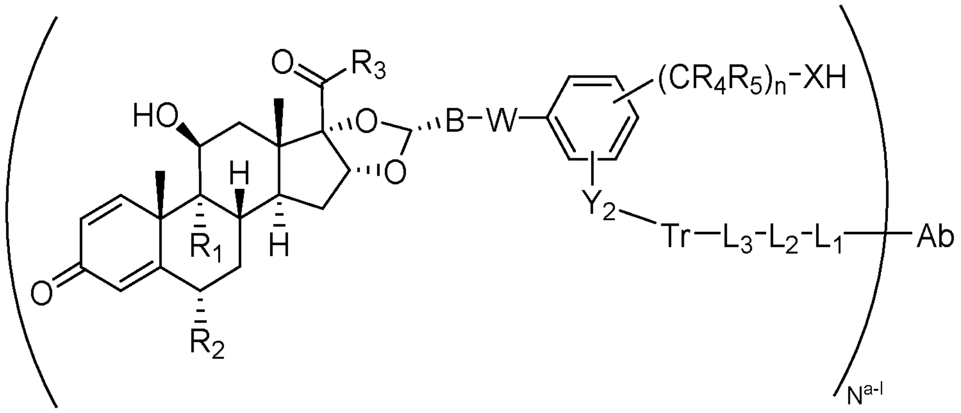

,

(Vb)

wherein,

Ab represents a ligand capable of binding to a target, including but not limited to, antibodies and antigen-binding fragments thereof;
$N^{a-l}$ is any number from 1 to 10;
Tr is absent or Tr is any group;
$L_3$ is selected from a polypeptide fragment;
$L_2$ is absent or selected from a linker fragment;
$L_1$ is selected from a coupling unit;
$R_1$, $R_2$, $R_3$, $R_4$ and $R_5$ are each independently any group;
B is absent or B is any group;
W is absent or W is any group;
$CR_4R_5$ units are each independently unreplaced or replaced by a group selected from the group consisting of: -O-, -S-, -NR-, -S(O)-, $-S(O)_2-$, -C(=O)-, -C=C- and -C=C-, wherein $R_4$ and $R_5$ can together form optionally substituted cycloalkyl, optionally substituted heterocyclyl, optionally substituted bridged cyclyl, optionally substituted bridged heterocyclyl, optionally substituted spiro cyclyl and optionally substituted spiro heterocyclyl, and n is any integer from 1 to 20;
X is selected from the group consisting of: -O-, -S- and -NR-;
$Y_1$ is any group, and m is any integer from 0 to 4;
$Y_2$ is selected from the group consisting of -O-, -S- and -NR-;
wherein substituents are selected from the group consisting of: protium, deuterium, tritium, halogen, -CN, =O, =N-OH, =N-OR, =N-R, -OR, -C(O)R, -C(O)OR, -OC(O)R, -OC(O)OR, -C(O)NHR, - $C(O)NR_2$, -OC(O)NHR, $-OC(O)NR_2$, -SR, -S(O)R, $-S(O)_2R$, -NHR, $-N(R)_2$, -NHC(O)R, -NRC(O)R, -NHC(O)OR, -NRC(O)OR, $-S(O)_2NHR$, $-S(O)_2N(R)_2$,

-NHS(O)$_2$NR$_2$, -NRS(O)$_2$NR$_2$, -NHS(O)$_2$R, - NRS(O)$_2$R, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy; wherein each R is independently selected from the group consisting of hydrogen, protium, deuterium, tritium, oxygen, halogen, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy.

The term "ligand" generally refers to any molecule capable of specifically binding to and/or reactively binding to or complexing a target molecule, such as a receptor, a substrate, an antigenic determinant or other binding site on a target cell or tissue. Examples of ligands include antibodies and fragments thereof (e.g., monoclonal antibodies or fragments thereof), enzymes (e.g., fibrinolytic enzymes), biological response modifiers (e.g., interleukins, interferons, erythropoietins or colony stimulating factors), peptide hormones and antigen-binding fragments thereof, polysaccharides, lipids, oligonucleotides, polynucleotides, synthetic molecules, inorganic molecules, organic molecules, and any combination thereof.

The term "target" or "target molecule" may include a wide variety of substances and molecules, ranging from simple molecules to complex targets. The target molecules may be proteins, nucleic acids, lipids, carbohydrates, or any other molecules that can be recognized by polypeptide domains. For example, the target molecules may include compounds (i.e., non-biological compounds, e.g., organic molecules, inorganic molecules, or molecules having organic and inorganic atoms, but excluding polynucleotides and proteins), mixtures of compounds, arrays of spatially localized compounds, biological macromolecules, phage peptide display libraries, polysome peptide display libraries, extracts made from biological materials (e.g., bacteria, plants, fungi, or animals (e.g., mammalian) cells or tissues), proteins, toxins, peptide hormones, cells, viruses, and the like. Other target molecules include, for example, whole cells, whole tissues, mixtures of related or unrelated proteins, mixtures of viral or bacterial strains, and the like.

The term "specifically binding" or "specific" generally refers to a measurable and reproducible interaction, such as binding between a target and an antibody, that may determine the presence of the target in the presence of a heterogeneous population of molecules (including biomolecules). For example, an antibody that specifically binds to a target (which may be an epitope) may be an antibody that binds to the target with greater affinity, avidity, more readily, and/or for greater duration than it binds to other targets. In certain embodiments, the antibody specifically binds to an epitope on the protein that is conserved in proteins of different species. In certain embodiments, specific binding may include, but is not required to be, exclusive binding.

The structure of the coupling unit $L_1$ may be changed before and after coupling to the ligand, i.e., the structure of $L_1$ may be changed in Linker-Payload structures (e.g., formula IVa or formula IVb) and drug-ligand conjugate structures (e.g., formula Va or formula Vb), and such changes may be easily determined by one of ordinary skill in the art.

For example, when $L_1$ is coupled to the ligand via sulfhydryl, the structural change of $L_1$ is as follows:

| Structure before coupling | Structure after coupling |
|---|---|
| 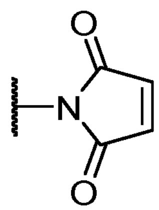 | 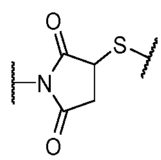 or 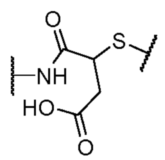 |
| 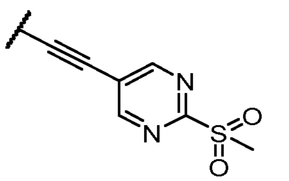 | 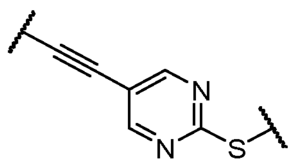 |
| 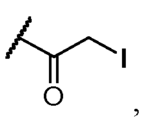, 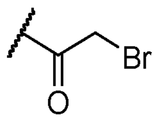, 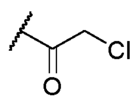 | 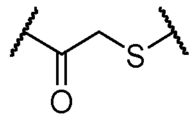 |
| 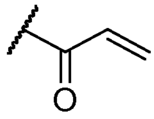 | 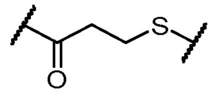 |

(continued)

| Structure before coupling | Structure after coupling |
|---|---|
| 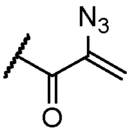 | 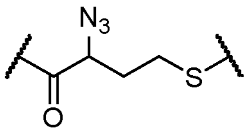 |
| 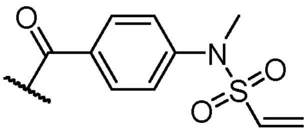 | 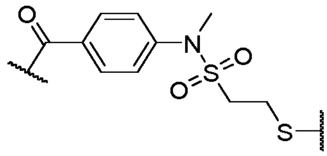 |
| 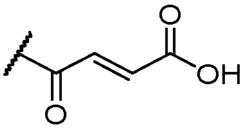 | 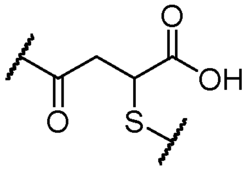 |
| 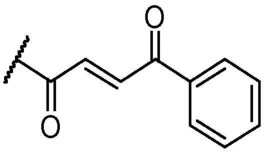 | 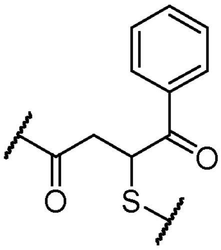 |
| 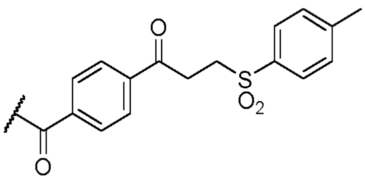 | 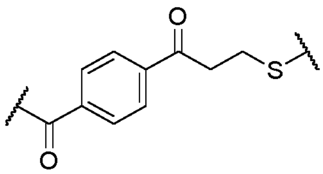 |
| 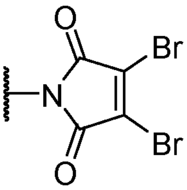 , 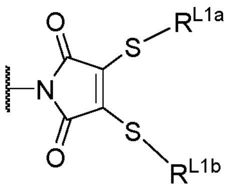 | 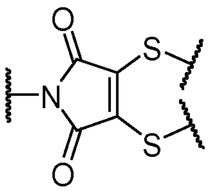 |
| 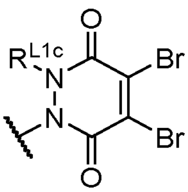 , 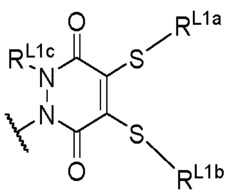 | 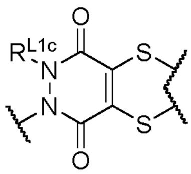 |

(continued)

| Structure before coupling | Structure after coupling |
|---|---|
| 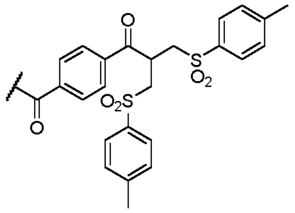 , 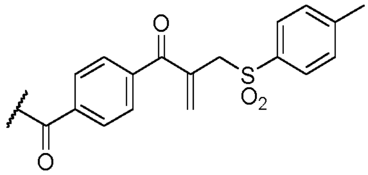 | 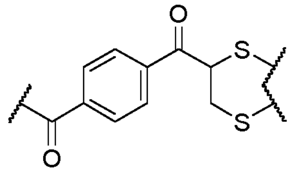 |
| 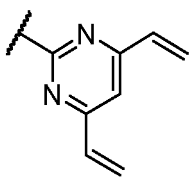 | 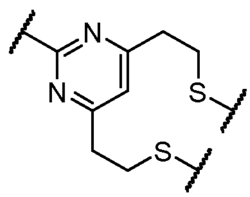 |

wherein sulfhydryl may be derived from a ligand, and the $R^{L1a}$, $R^{L1b}$ and $R^{L1c}$ are independently selected from the group consisting of: hydrogen, optionally substituted alkyl and optionally substituted aryl; for example, the $R^{L1a}$, $R^{L1b}$ and $R^{L1c}$ may each be independently selected from the group consisting of: hydrogen, optionally substituted methyl, optionally substituted ethyl, optionally substituted aryl and optionally substituted benzyl.

For example, when $L_1$ is coupled to the ligand via amino, the structural change of $L_1$ is as follows:

| Structure before coupling | Structure after coupling |
|---|---|
| 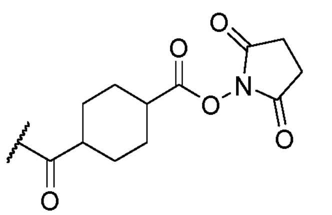 , 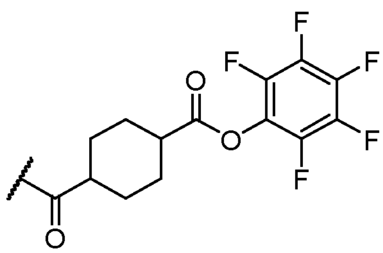 | 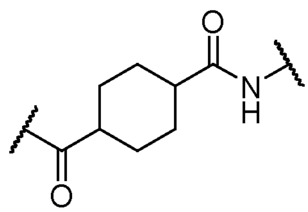 |
| 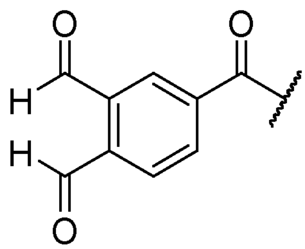 | 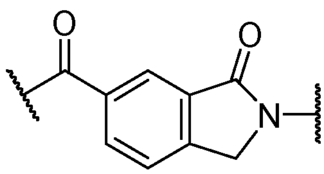 |

wherein amino may be derived from a ligand.

For example, when $L_1$ is coupled to a ligand via click chemistry, the structural change of $L_1$ is as follows:

| Structure before coupling | Structure after coupling |
|---|---|
| 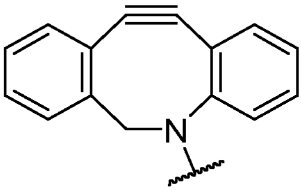 | 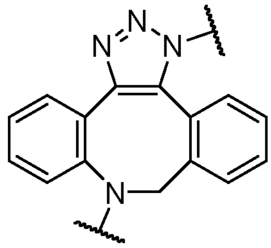 or 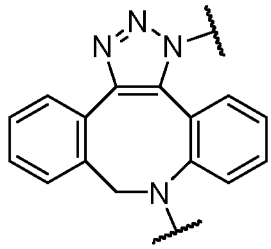 |
| 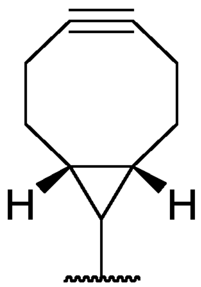 | 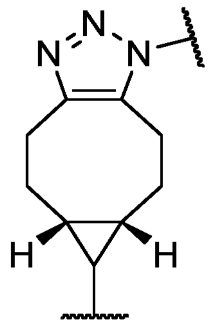 |
| 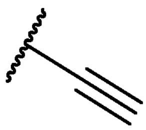 | 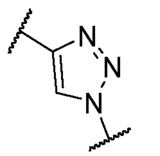 |
| 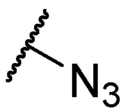 | 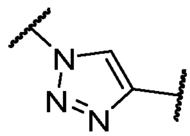 |

For example, $R_1$ and $R_2$ may each independently be selected from the group consisting of: H, F, Cl, Br and optionally substituted methyl.

For example, $R_3$ may be selected from the group consisting of: optionally substituted $-CH_2Cl$, optionally substituted $-CH_2SH$, optionally substituted $-CH_2OH$, optionally substituted

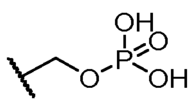 ,

optionally substituted

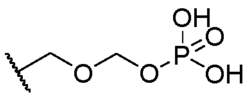
,

optionally substituted

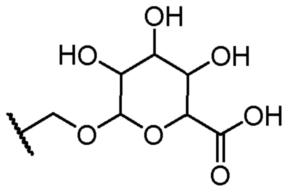
,

optionally substituted

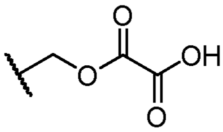
,

optionally substituted

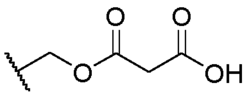
,

optionally substituted

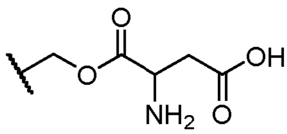
,

optionally substituted

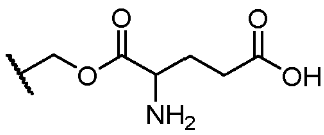
,

optionally substituted

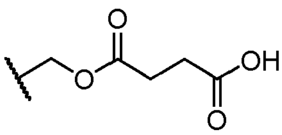
,

optionally substituted

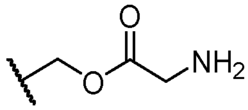,

optionally substituted

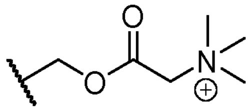,

optionally substituted

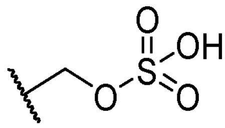,

optionally substituted

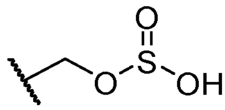,

optionally substituted

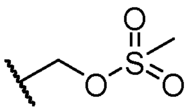,

optionally substituted -OH, optionally substituted $-OCH_3$, optionally substituted $-OCH_2F$, optionally substituted $-OCH_2Cl$, optionally substituted $-OCH_2CN$, optionally substituted $-OCH_2CH_3$, optionally substituted sulfhydryl, optionally substituted $-SCH_2F$, optionally substituted $-SCH_2Cl$, optionally substituted $-SCH_2CF_3$ and optionally substituted $-SCH_2CN$.

For example, B may be selected from the group consisting of: optionally substituted

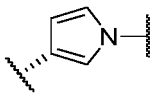,

optionally substituted

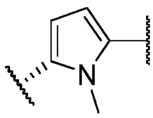,

optionally substituted

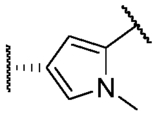,

optionally substituted

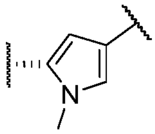,

optionally substituted

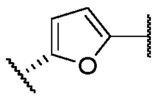,

optionally substituted

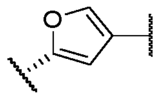,

optionally substituted

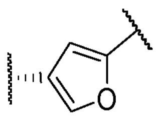,

optionally substituted

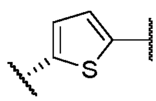,

optionally substituted

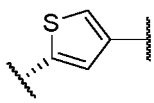,

optionally substituted

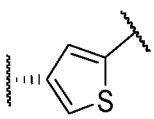,

optionally substituted

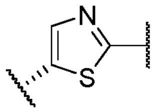
,

optionally substituted

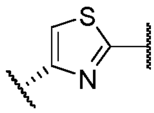
,

optionally substituted

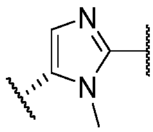
,

optionally substituted

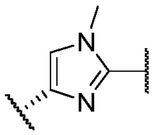
,

optionally substituted

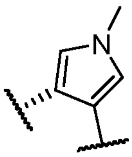
,

optionally substituted

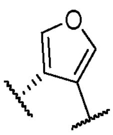
,

optionally substituted

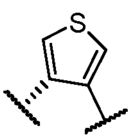
,

optionally substituted

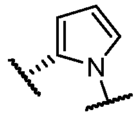,

optionally substituted

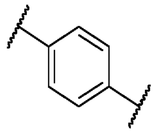

and optionally substituted

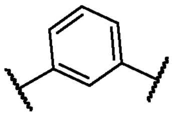,

wherein substituents are selected from the group consisting of: protium, deuterium, tritium, halogen, -CN, =O, =N-OH, =N-OR, =N-R, -OR, - C(O)R, -C(O)OR, -OC(O)R, -OC(O)OR, -C(O)NHR, -C(O)$NR_2$, -OC(O)NHR, -OC(O)$NR_2$, -SR, - S(O)R, -$S(O)_2R$, -NHR, -$N(R)_2$, -NHC(O)R, -NRC(O)R, -NHC(O)OR, -NRC(O)OR, -$S(O)_2NHR$, - $S(O)_2N(R)_2$, -$NHS(O)_2NR_2$, -$NRS(O)_2NR_2$, -$NHS(O)_2R$, -$NRS(O)_2R$, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy, wherein each R is independently selected from the group consisting of hydrogen, protium, deuterium, tritium, oxygen, halogen, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy.

For example, W may be absent or W is selected from the group consisting of:

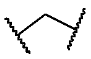, 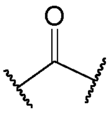, 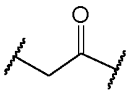, 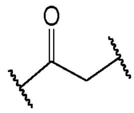, 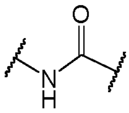, 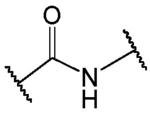, 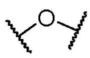, 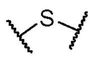, 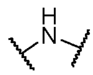,

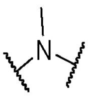, 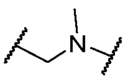, 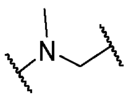, 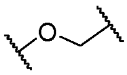, 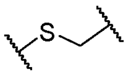, 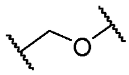, 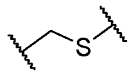, 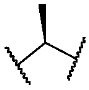,

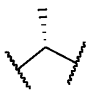, 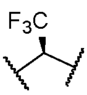, 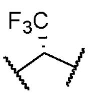, 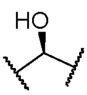, 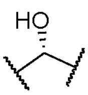, 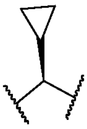, 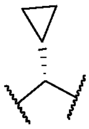, 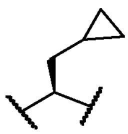, 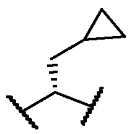, 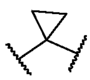,

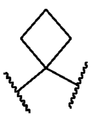, 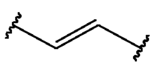 and 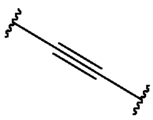.

For example, the conjugate may be selected from the group consisting of the following structures:

| No. | Structure |
|---|---|
| V-1 | 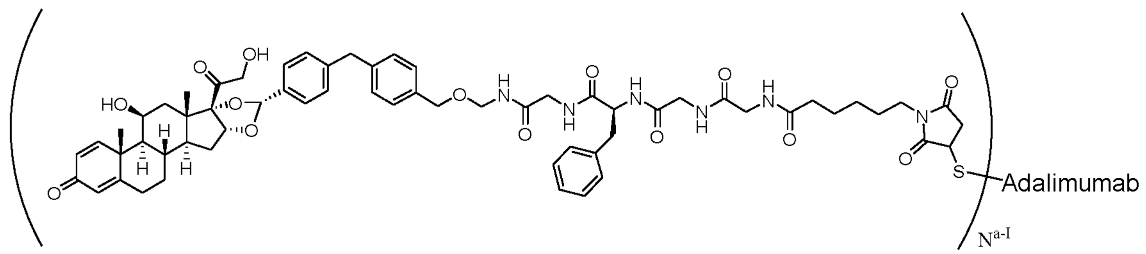 |
| V-2 | 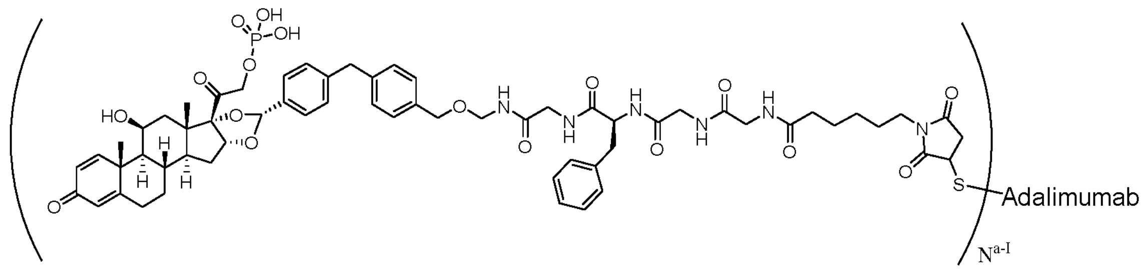 |
| V-6 | 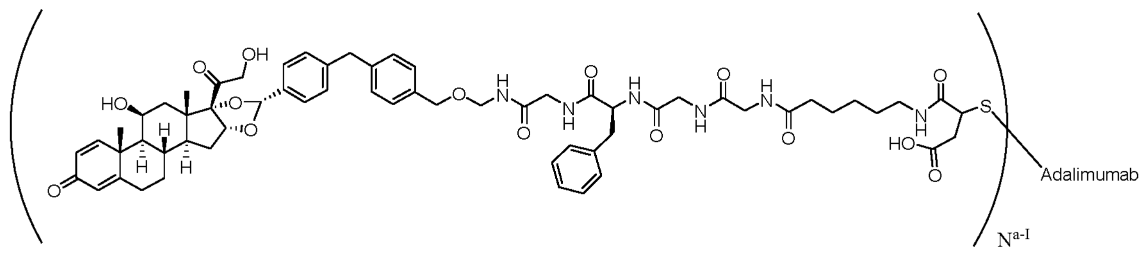 |
| V-7 | 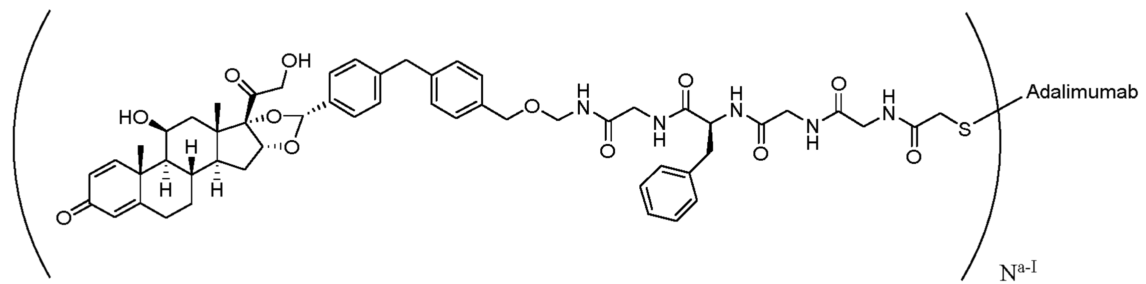 |

(continued)

| No. | Structure |
|---|---|
| V-8 | 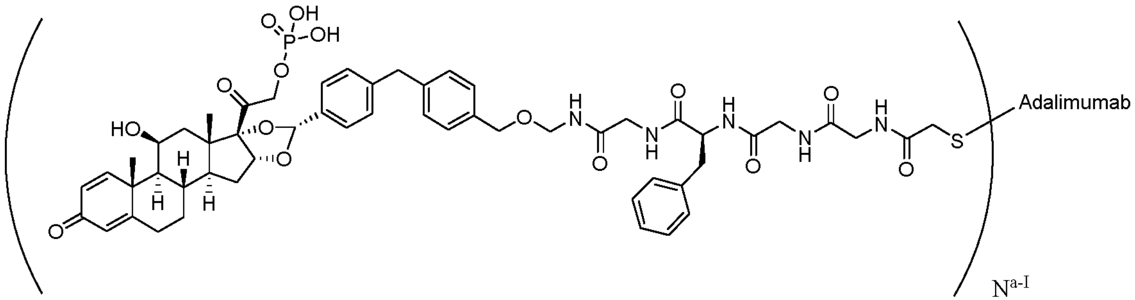 |
| V-12 | 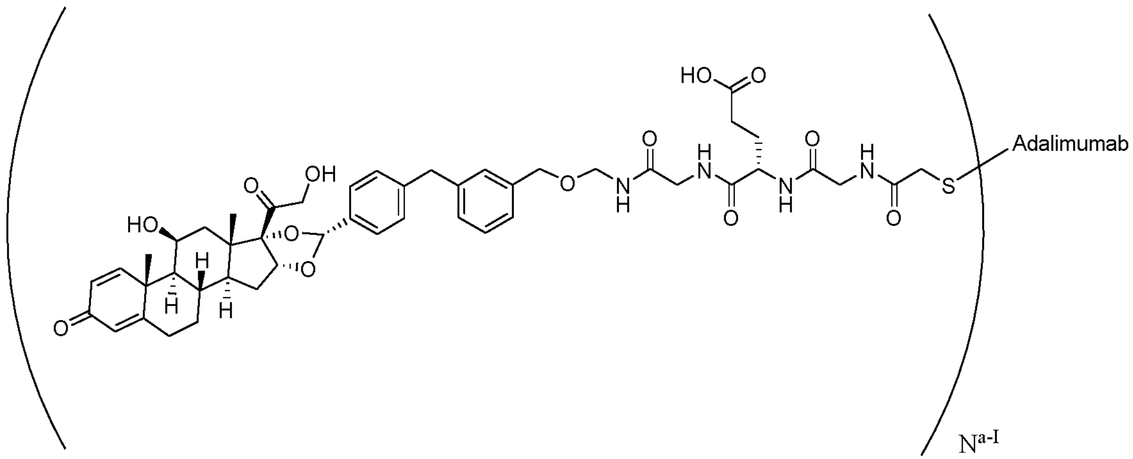 |

(continued)

| No. | Structure |
| --- | --- |
| V-13 | 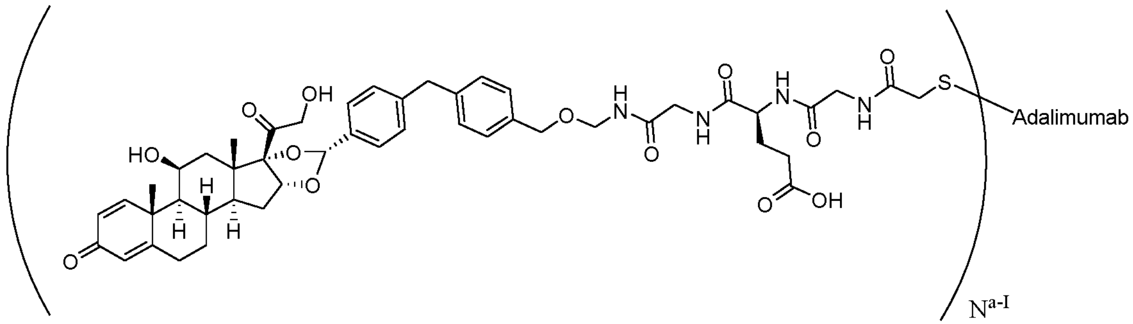 |
| V-14 | 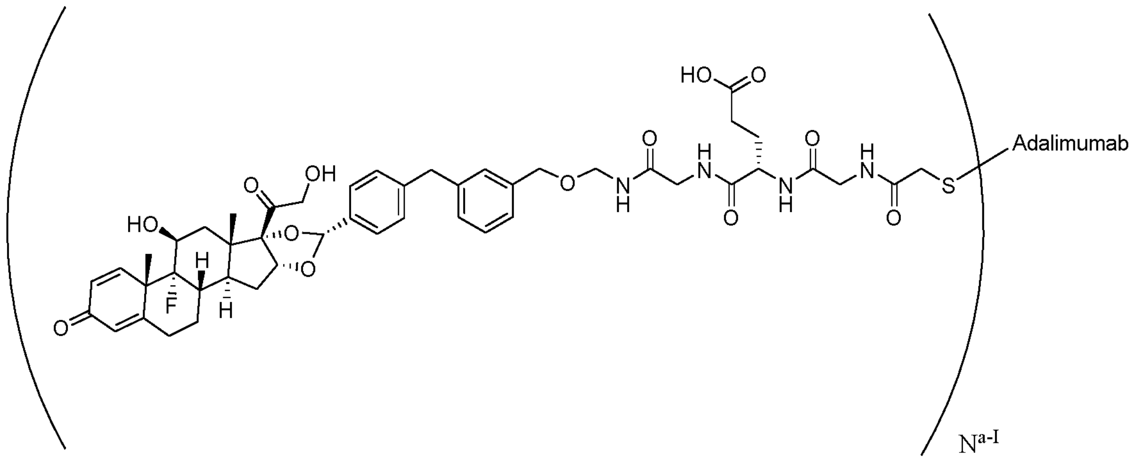 |

(continued)

| No. | Structure |
| --- | --- |
| V-15 | 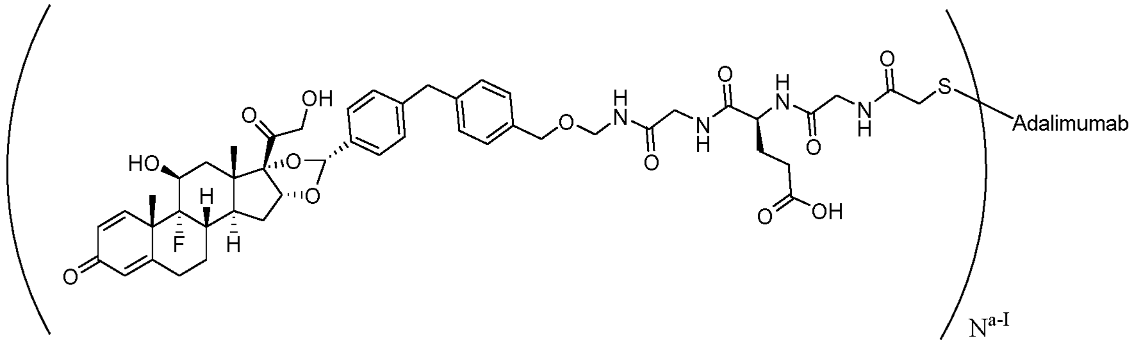 |
| V-16 | 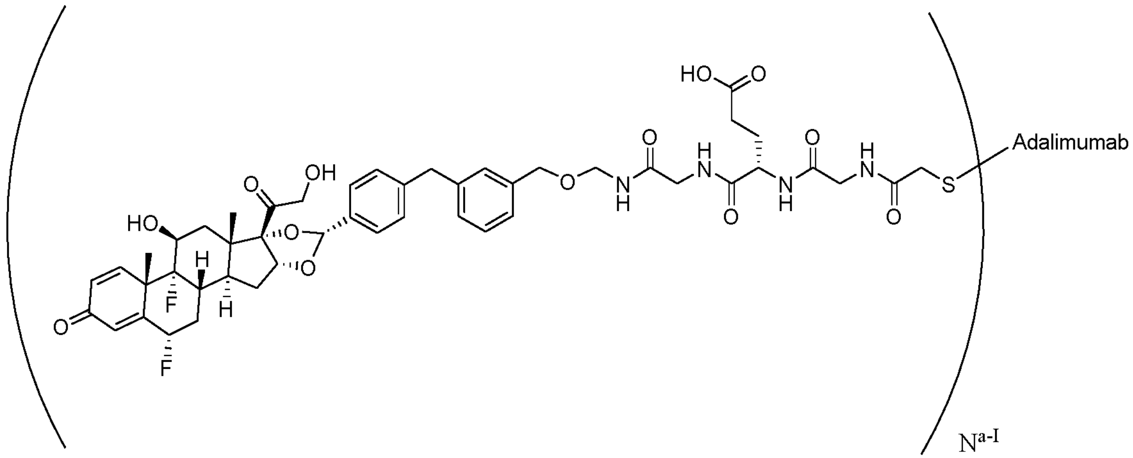 |

(continued)

| No. | Structure |
|---|---|
| V-17 | 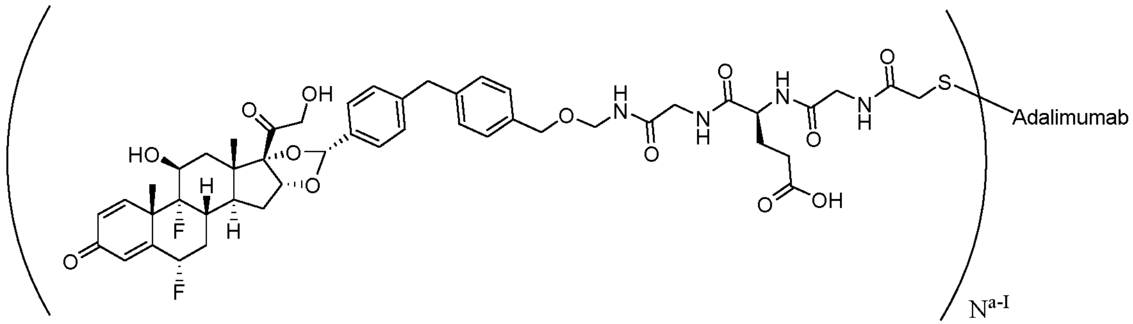 |
| V-18 | 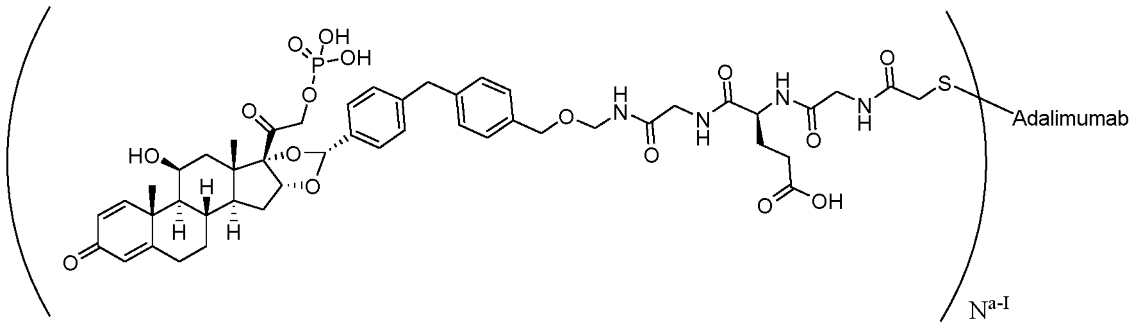 |
| V-22 | 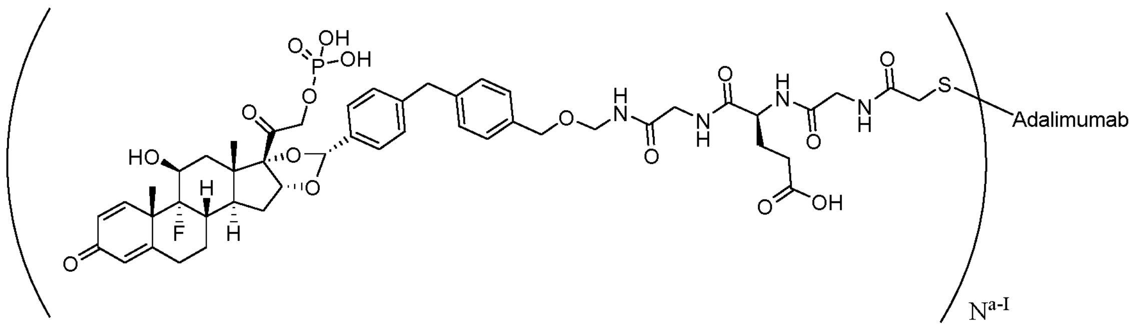 |

(continued)

| No. | Structure |
|---|---|
| V-25 | 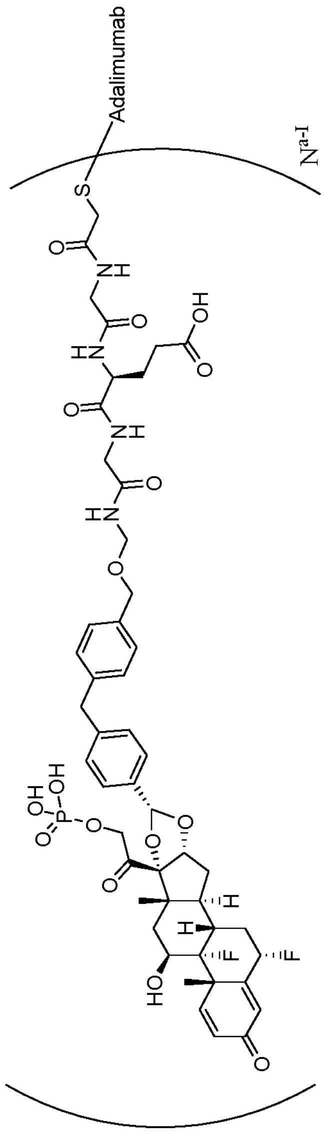 |
| V-29 | 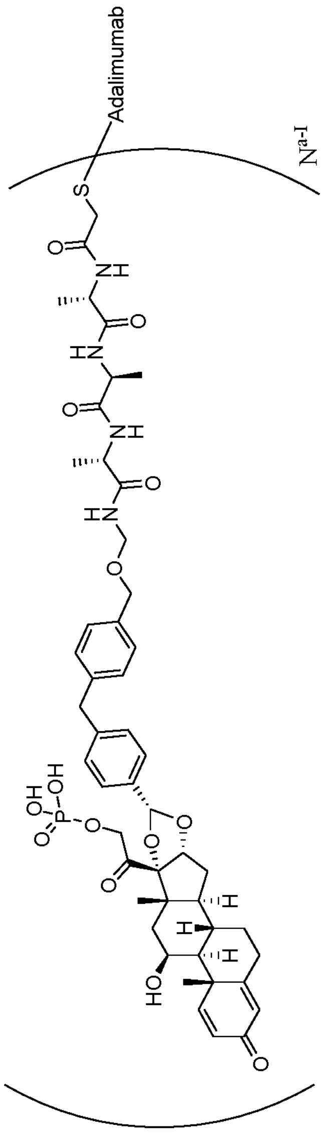 |
| V-30 | 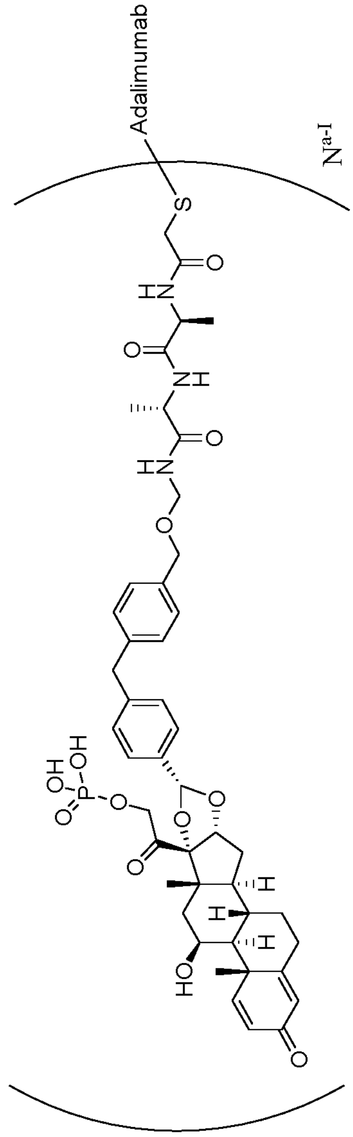 |

(continued)

| No. | Structure |
|---|---|
| V-34 | 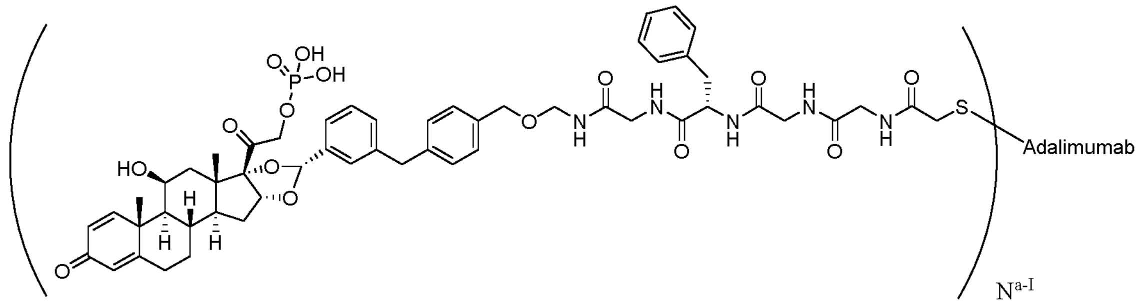 |
| V-35 | 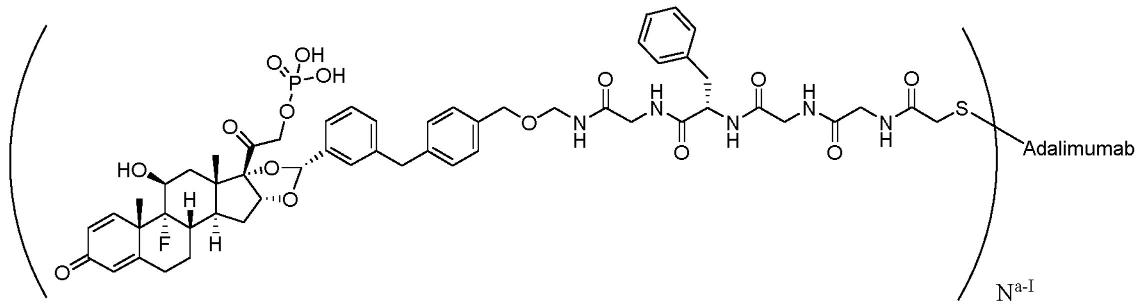 |
| V-39 | 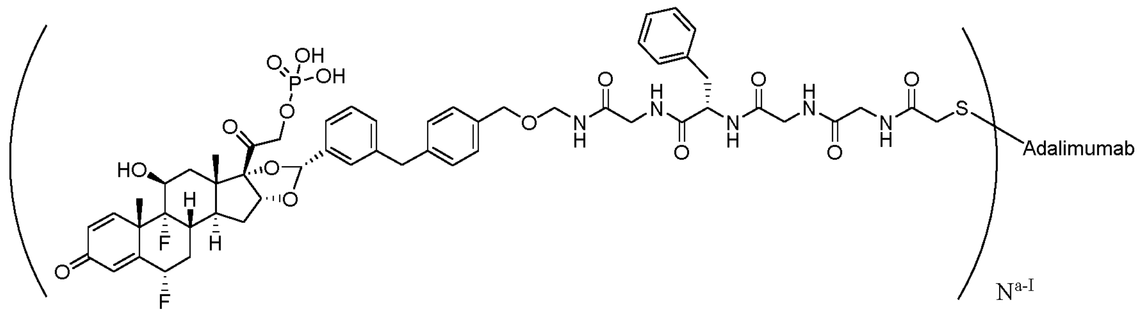 |

(continued)

| No. | Structure |
|---|---|
| V-43 | 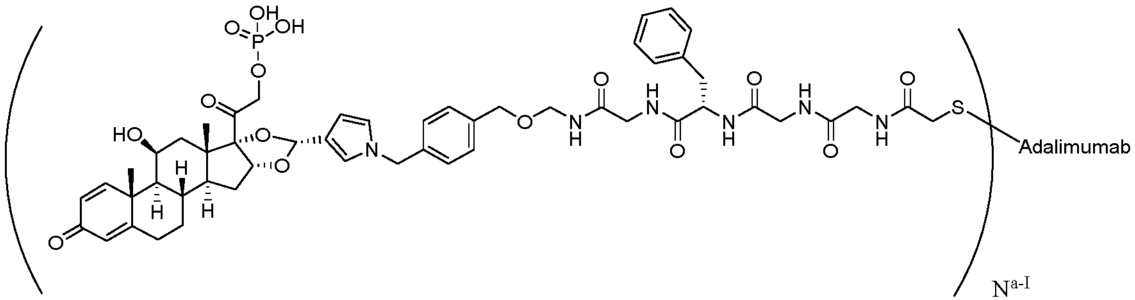 |
| V-44 | 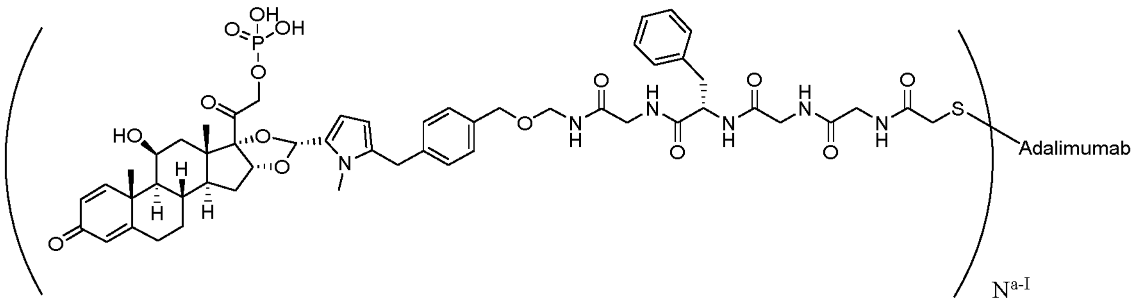 |
| V-45 | 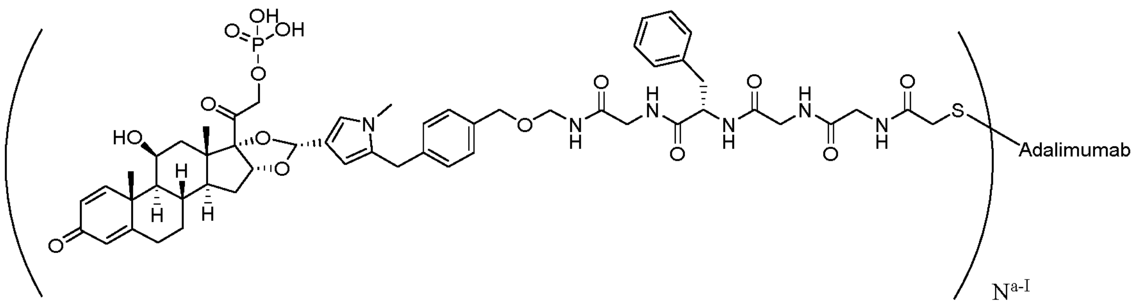 |

(continued)

| No. | Structure |
|---|---|
| V-46 | 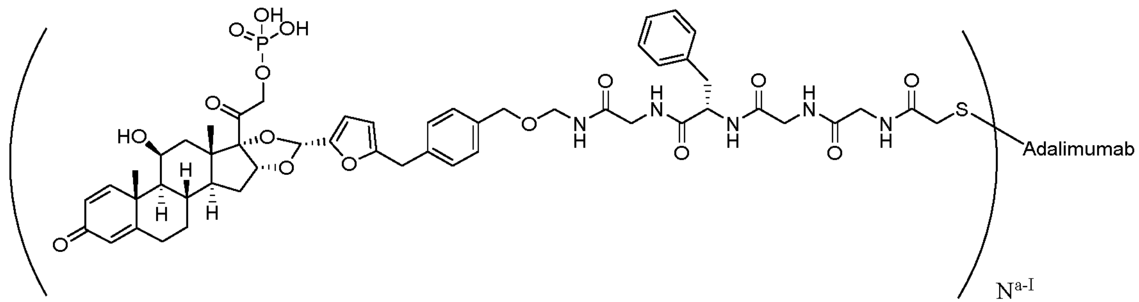 |
| V-47 | 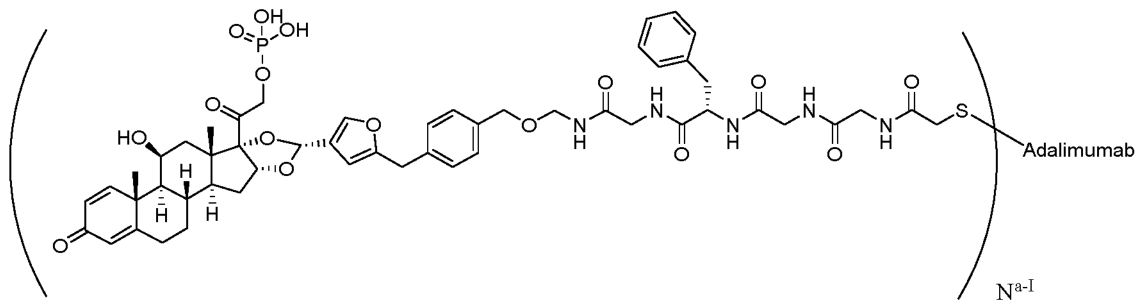 |
| V-48 | 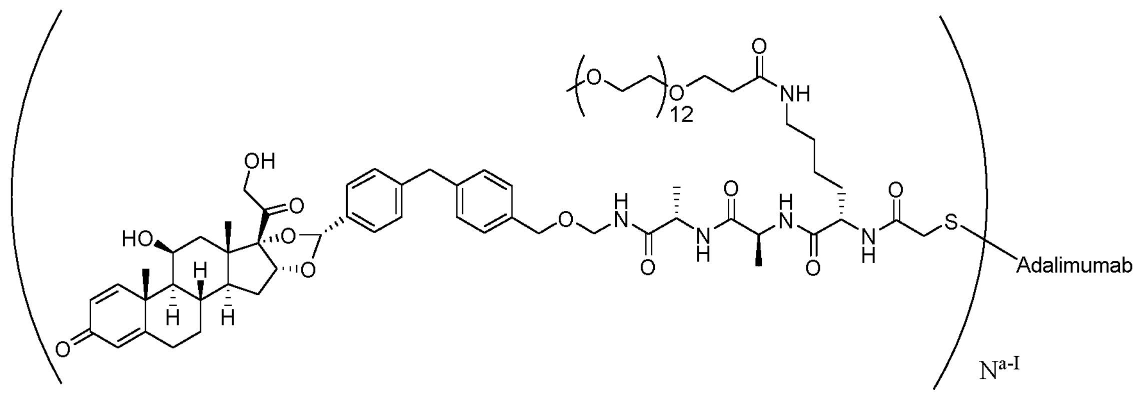 |

(continued)

| No. | Structure |
|---|---|
| V-49 | 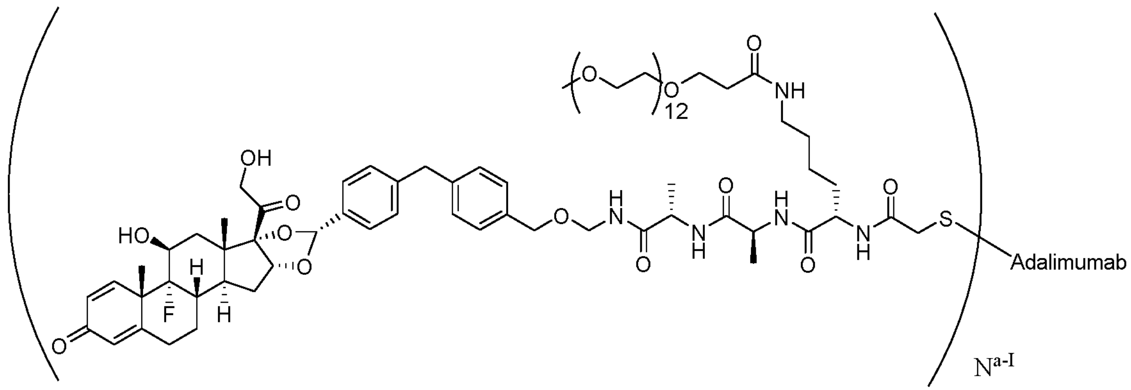 |
| V-50 | 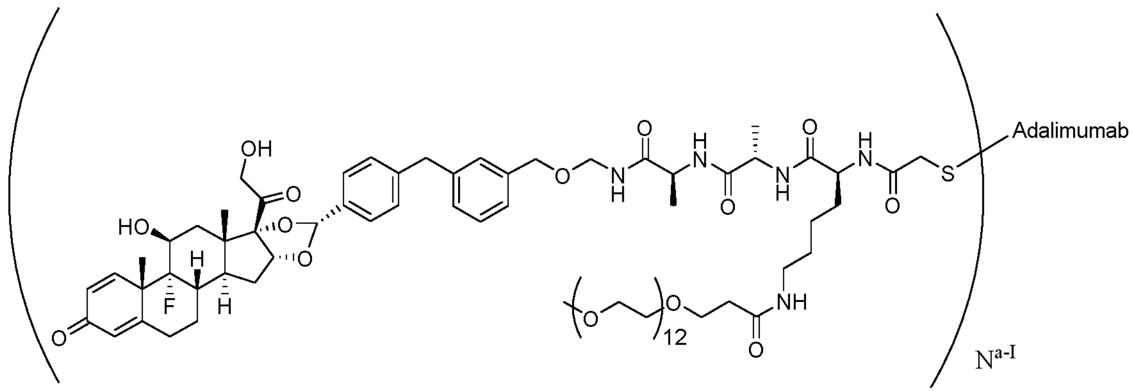 |

(continued)

| No. | Structure |
|---|---|
| V-51 | 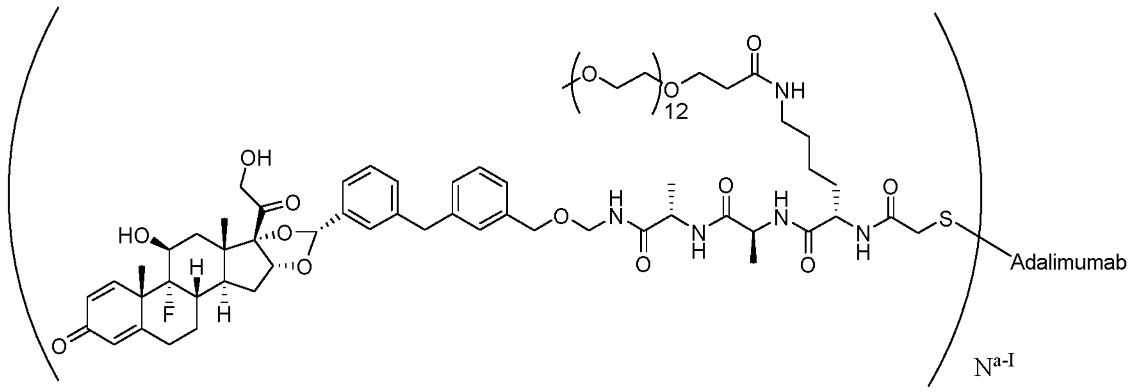 |
| V-52 | 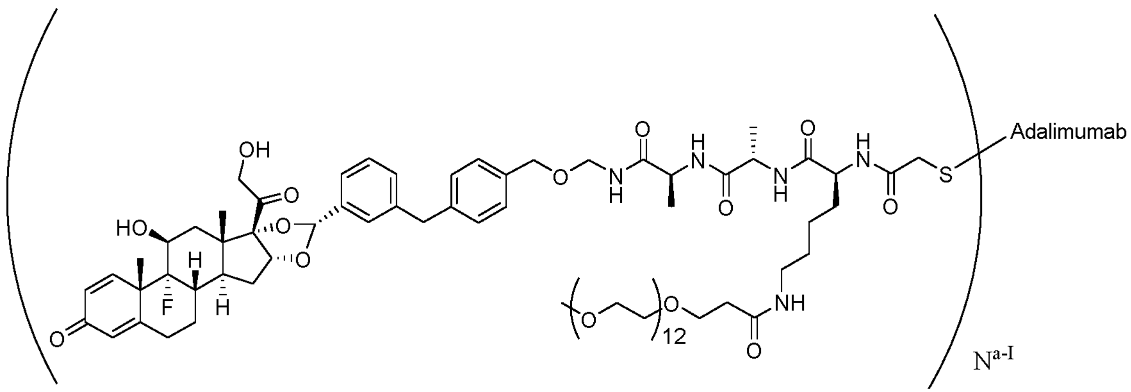 |

(continued)

| No. | Structure |
|---|---|
| V-53 | 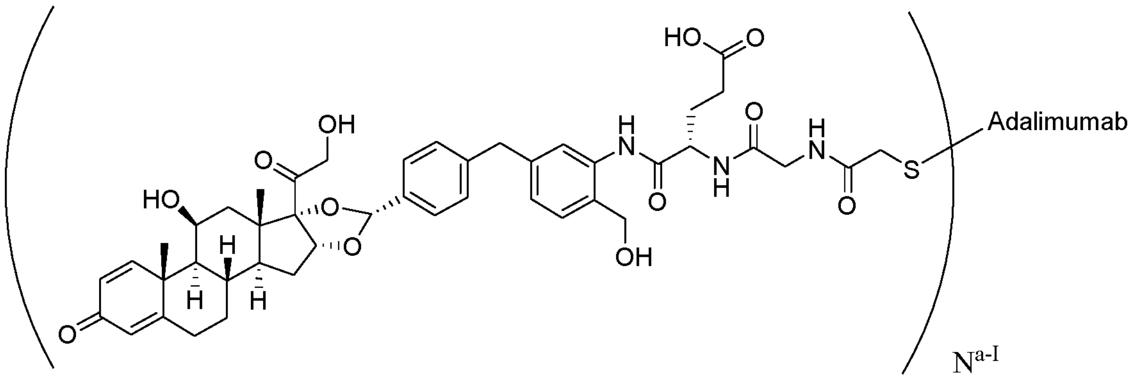 |
| V-54 | 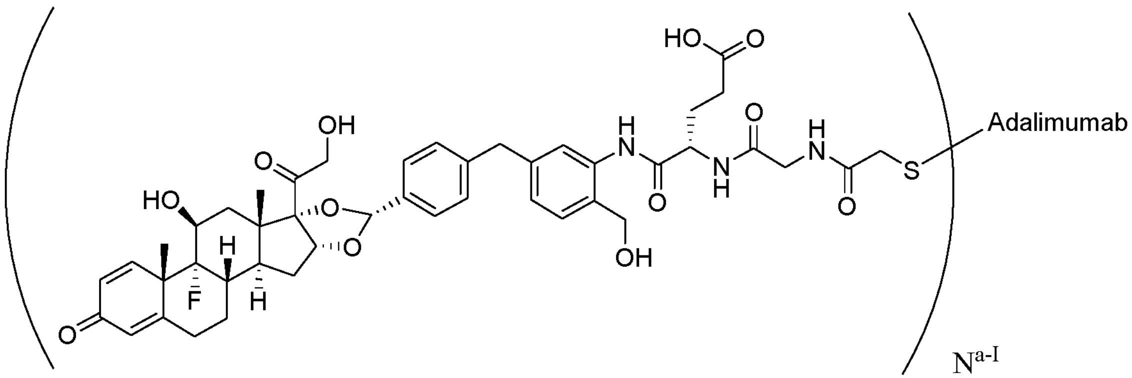 |

(continued)

| No. | Structure |
|---|---|
| V-55 | 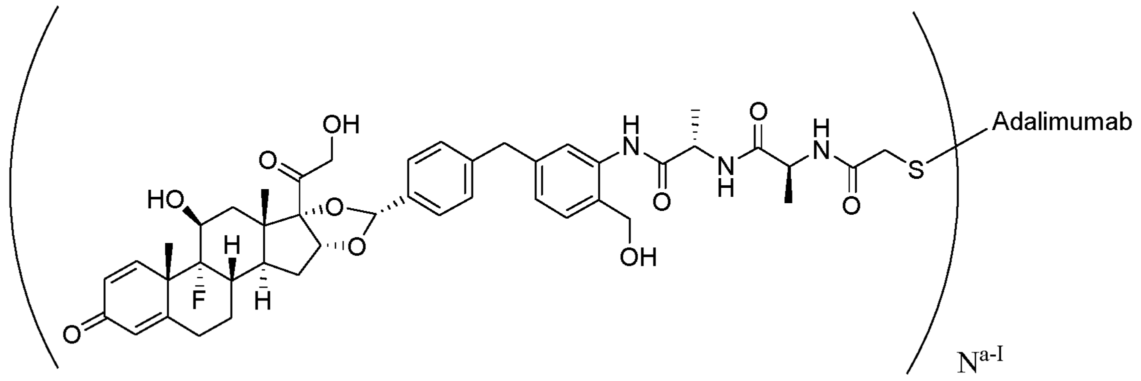 |
| V-56 | 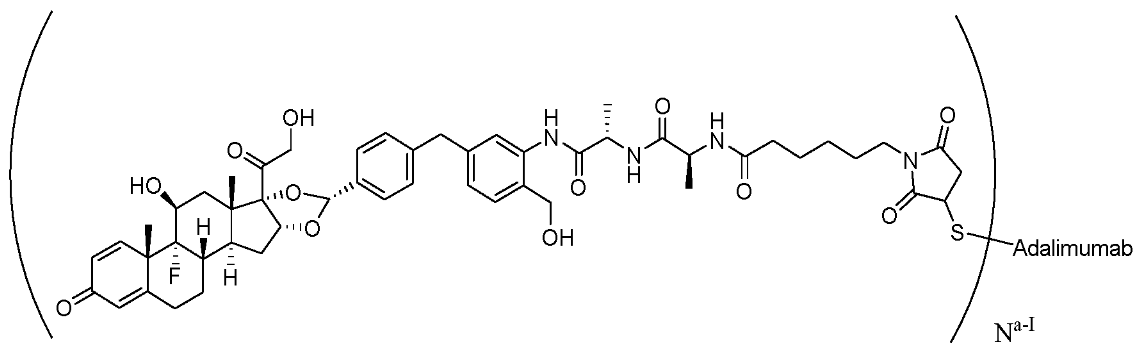 |

(continued)

| No. | Structure |
| --- | --- |
| V-57 | 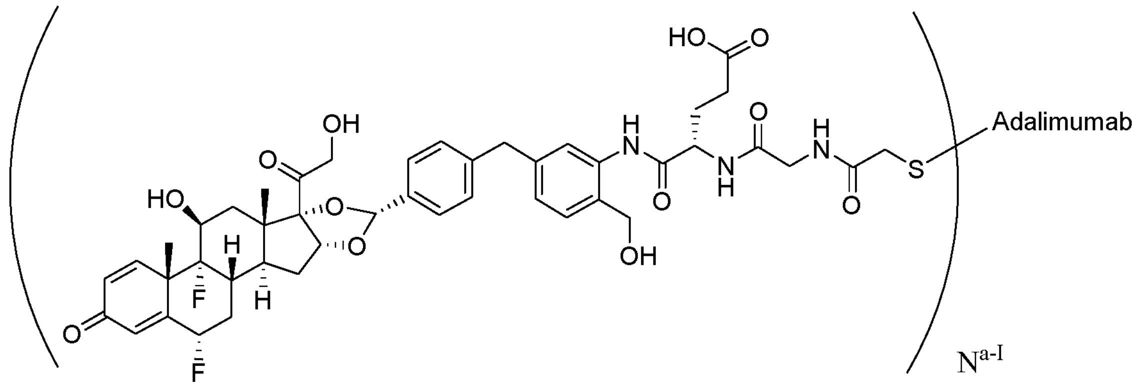 |
| V-58 | 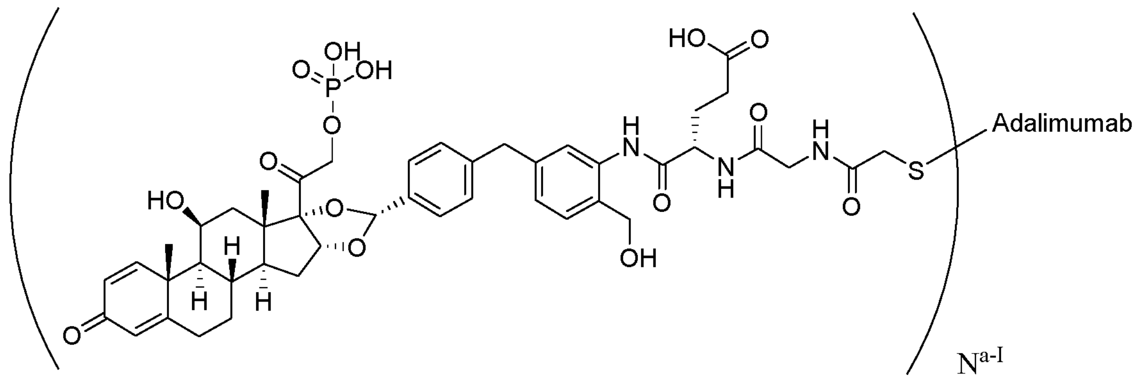 |

(continued)

| No. | Structure |
|---|---|
| V-59 | 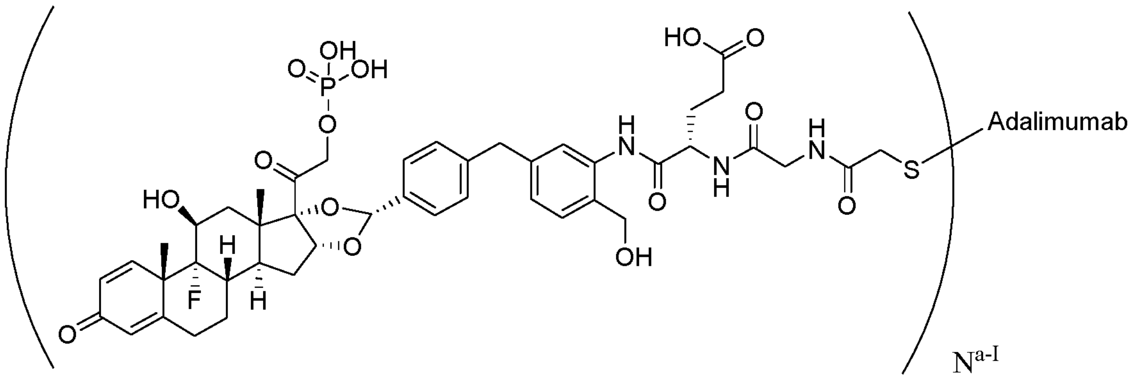 |
| V-60 | 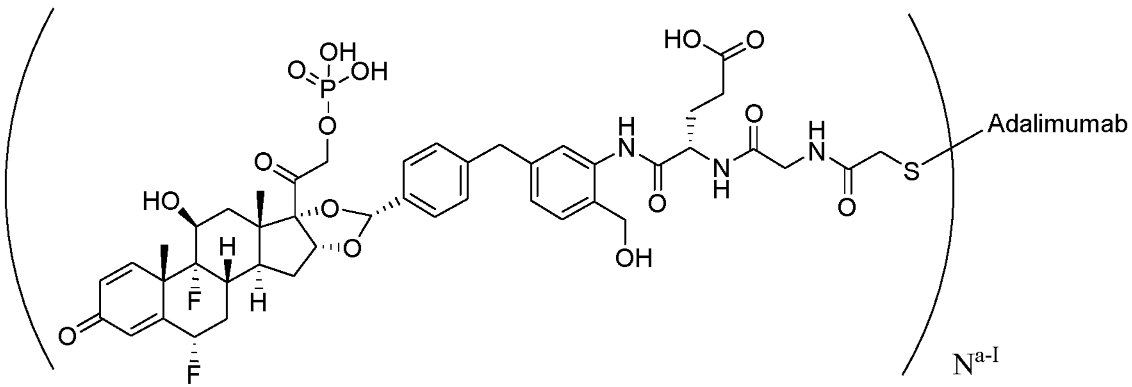 |

(continued)

| No. | Structure |
|---|---|
| V-70 | 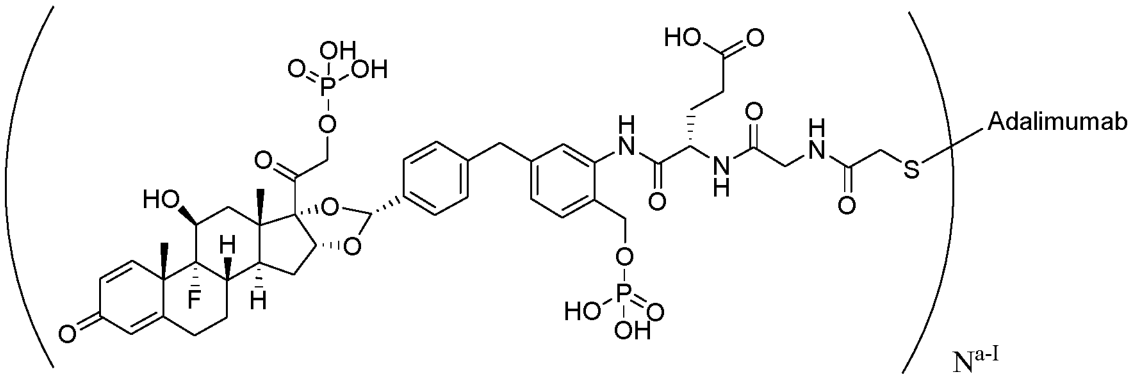 |
| V-74 | 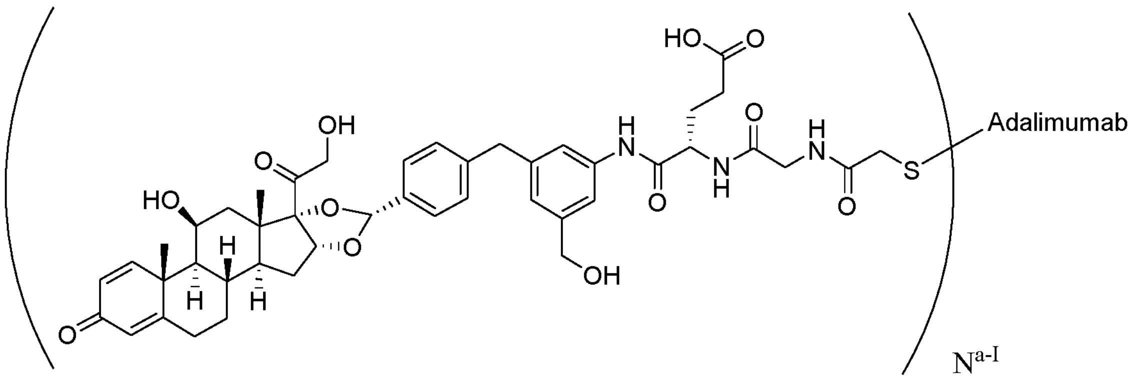 |

(continued)

| No. | Structure |
|---|---|
| V-75 | 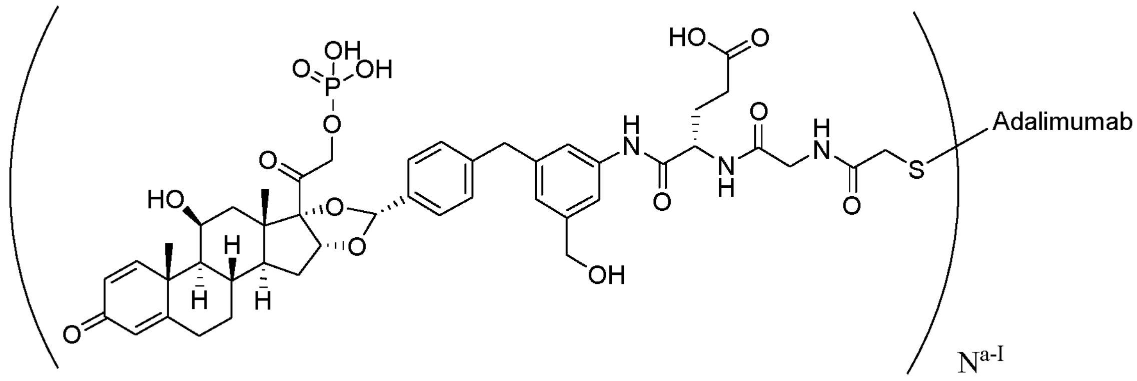 |
| V-78 | 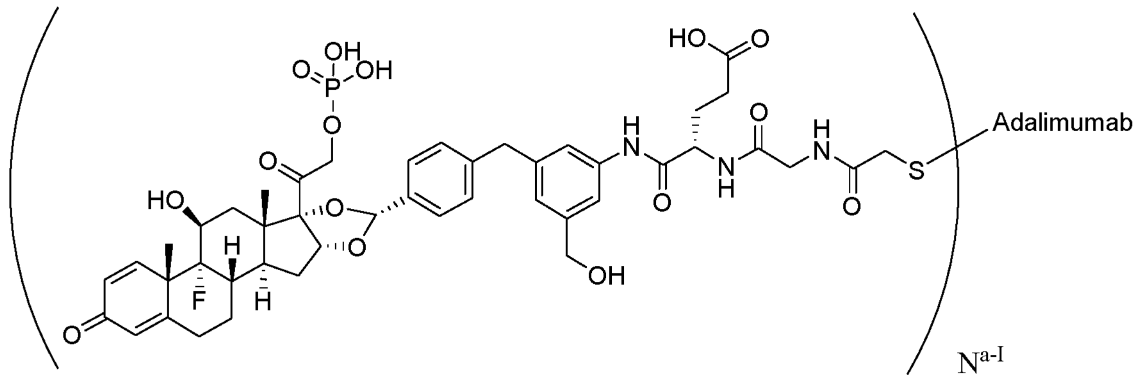 |

(continued)

| No. | Structure |
|---|---|
| V-81 | 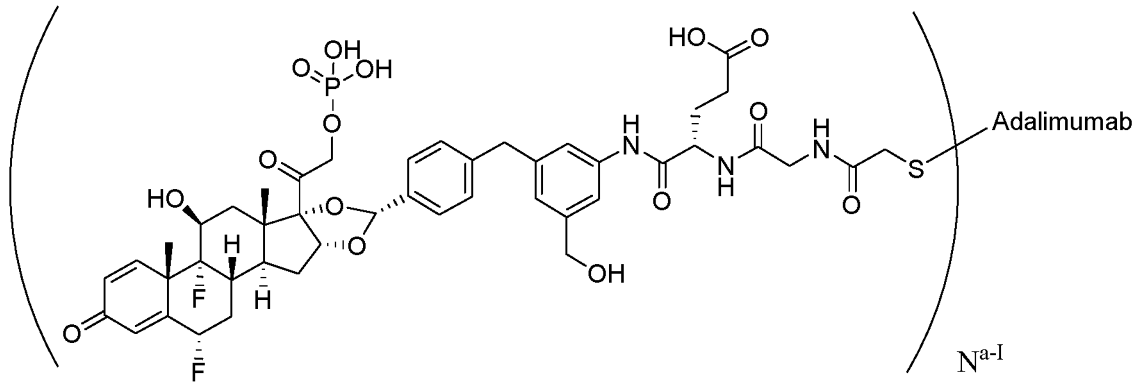 |
| V-84 | 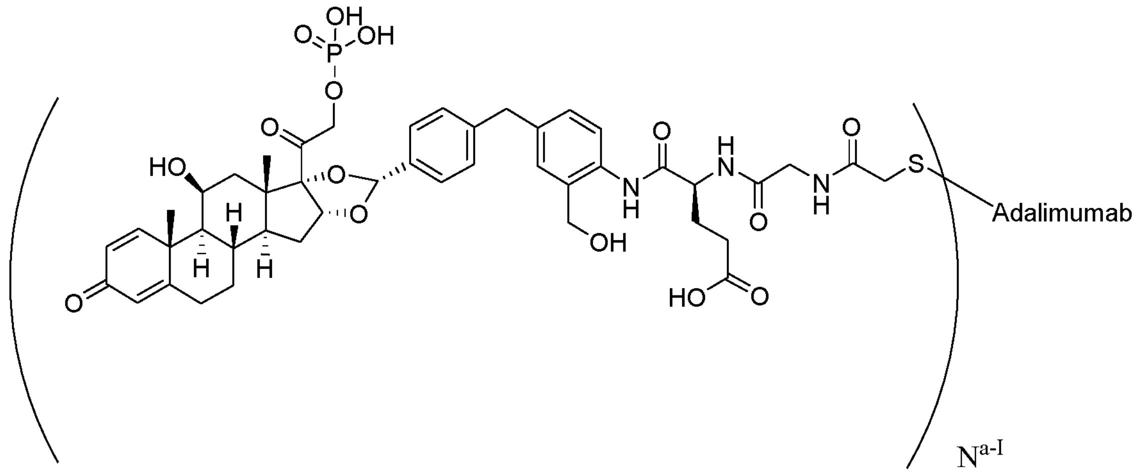 |

(continued)

| No. | Structure |
|---|---|
| V-87 | 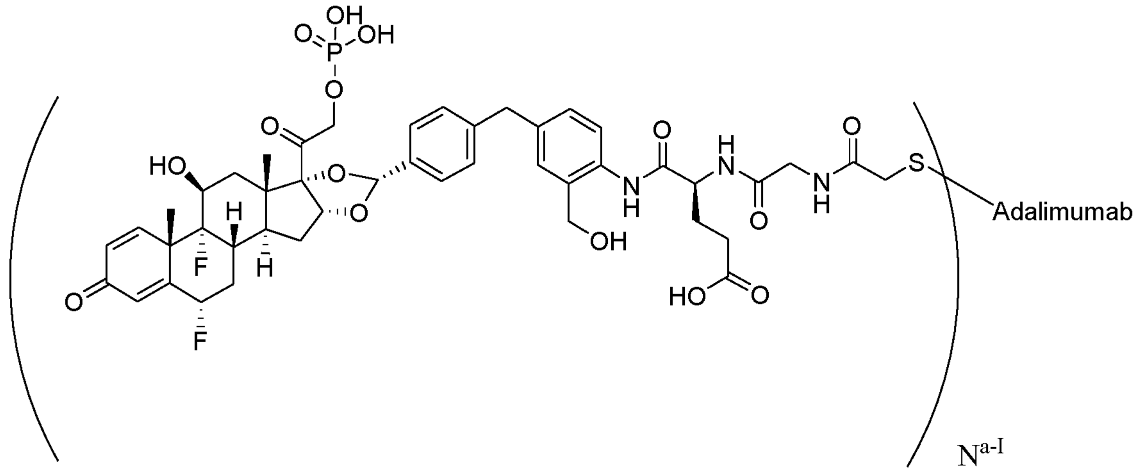 |
| V-90 | 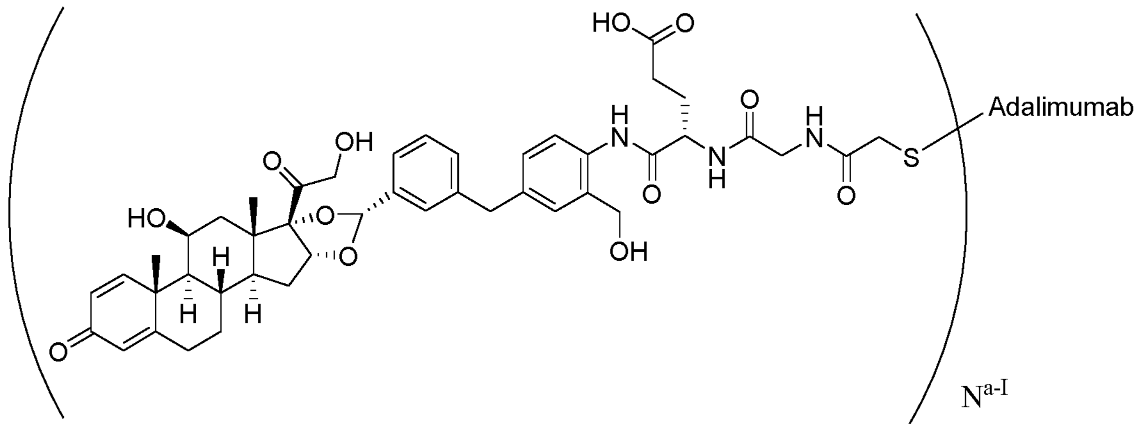 |

(continued)

| No. | Structure |
|---|---|
| V-91 | 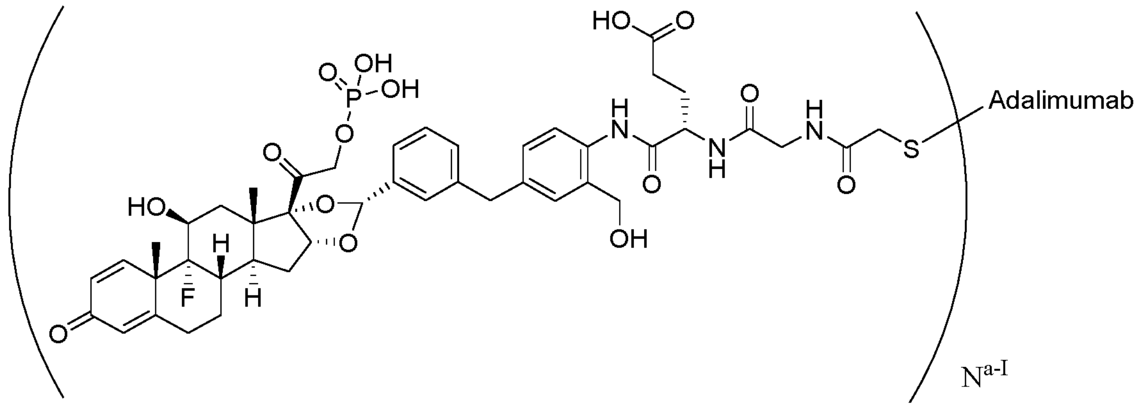 |
| V-92 | 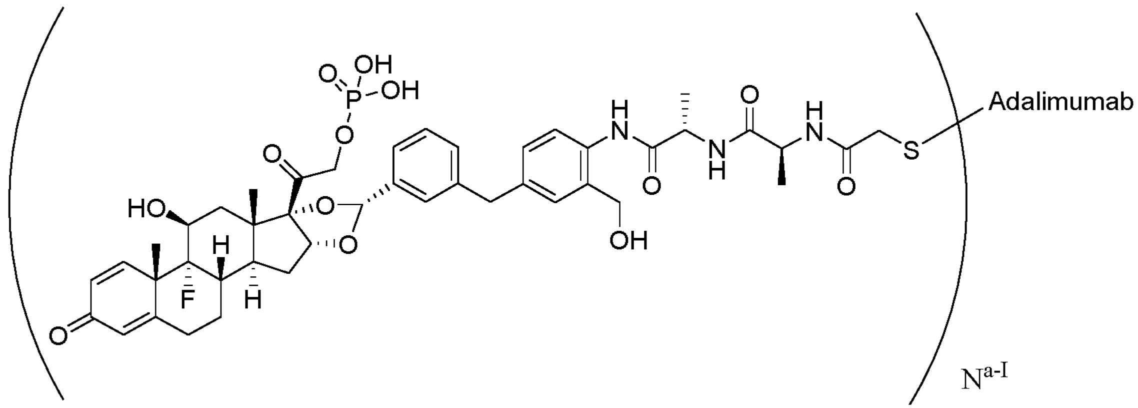 |

(continued)

| No. | Structure |
|---|---|
| V-95 | 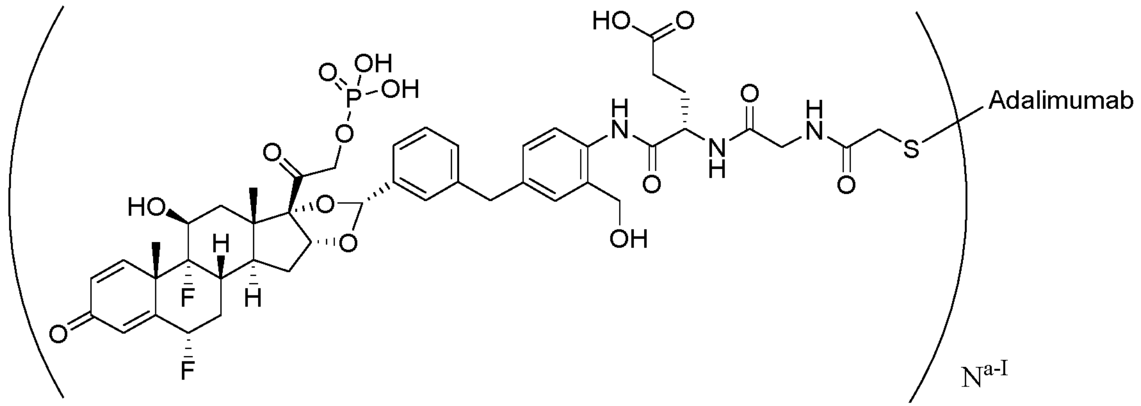 |
| V-98 | 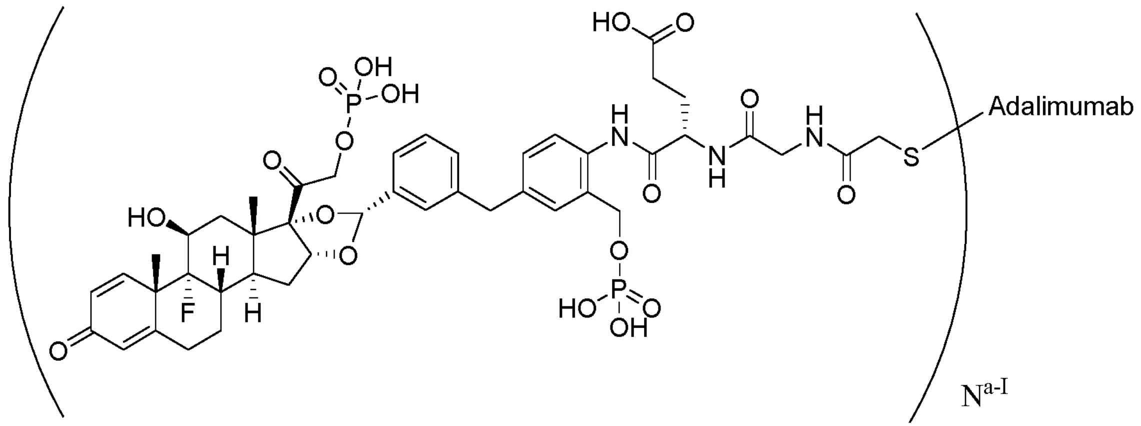 |

(continued)

| No. | Structure |
|---|---|
| V-102 | 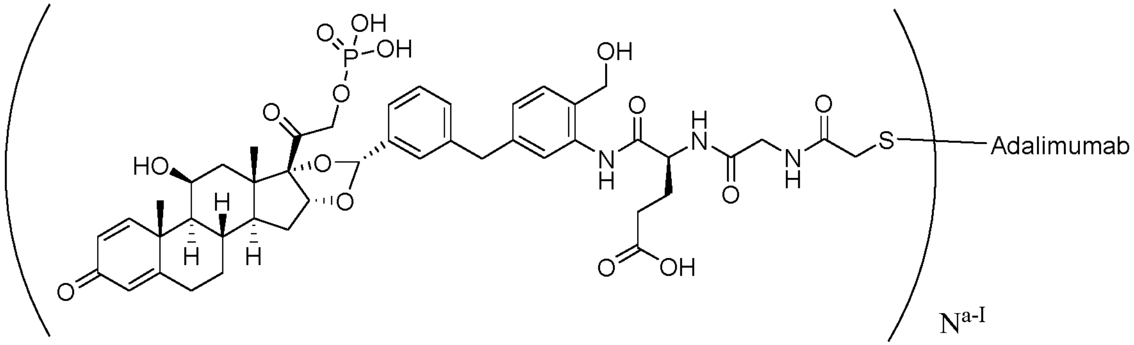 |
| V-103 | 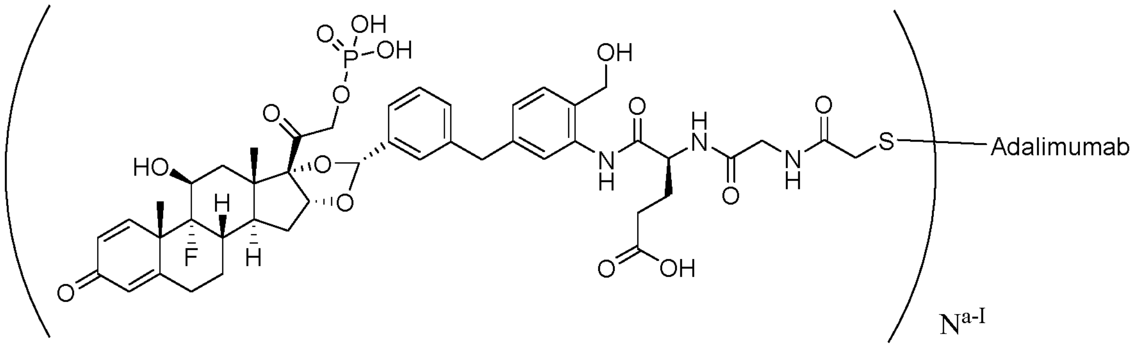 |
| V-104 | 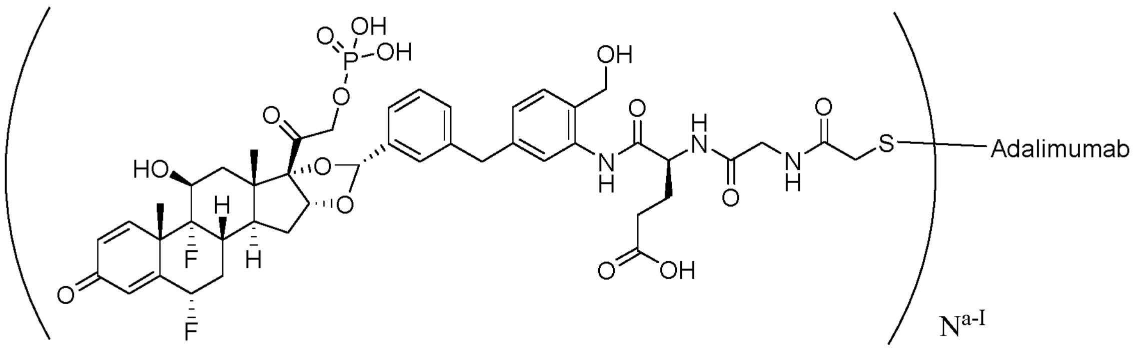 |

(continued)

| No. | Structure |
|---|---|
| V-105 | 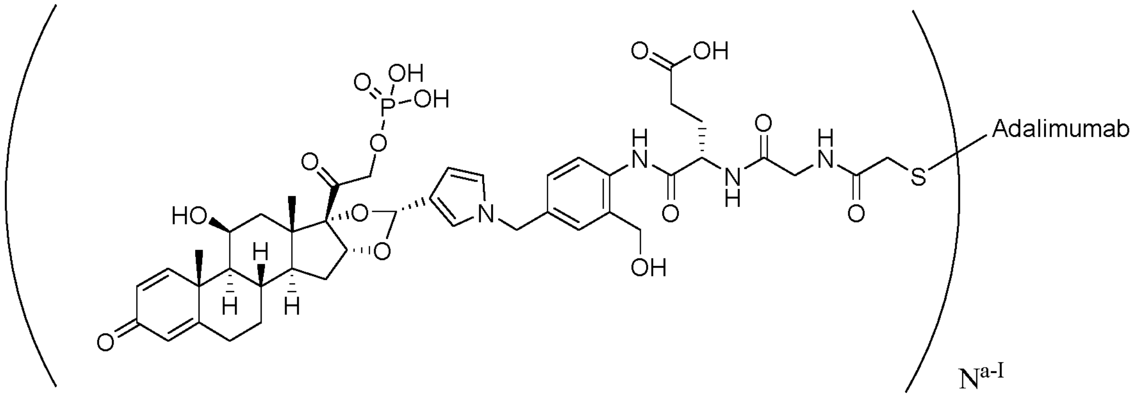 |
| V-106 | 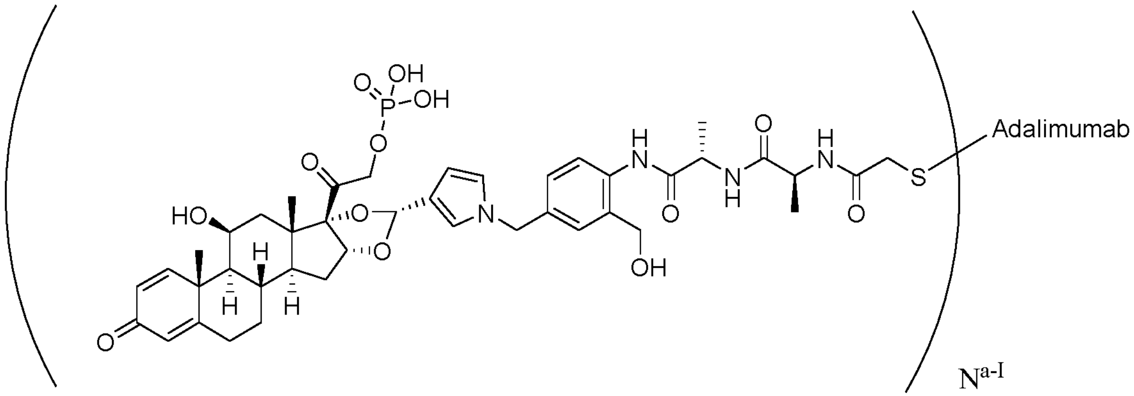 |

(continued)

| No. | Structure |
|---|---|
| V-109 | 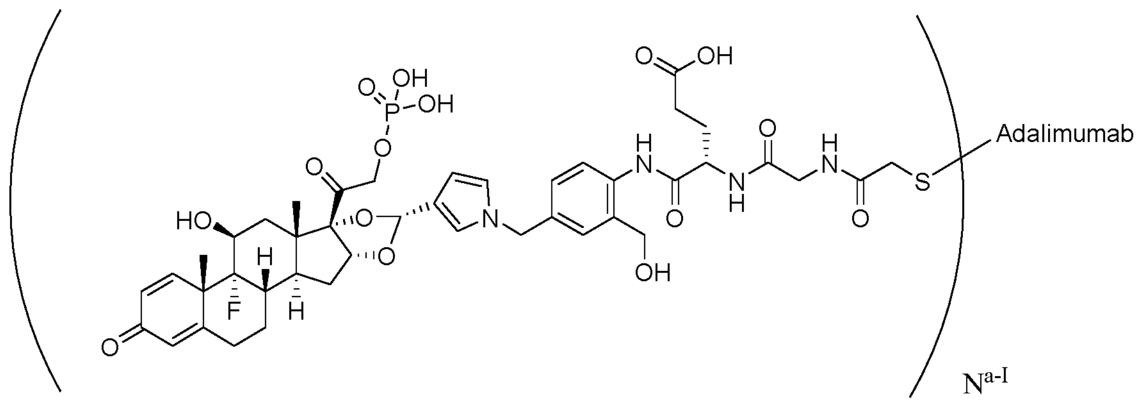 |
| V-110 | 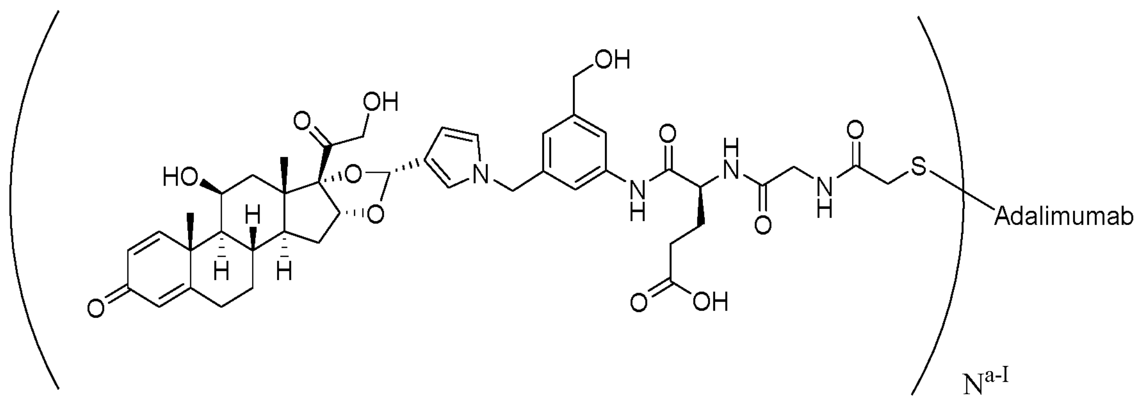 |

(continued)

| No. | Structure |
| --- | --- |
| V-111 | 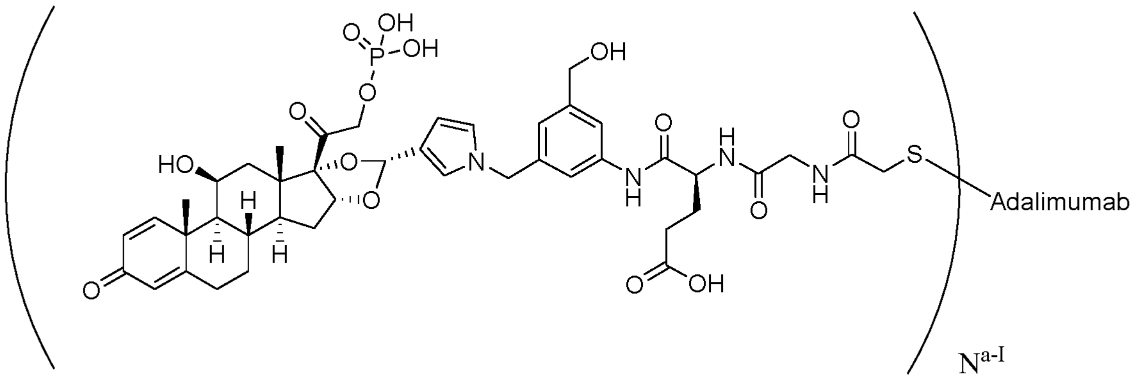 |
| V-112 | 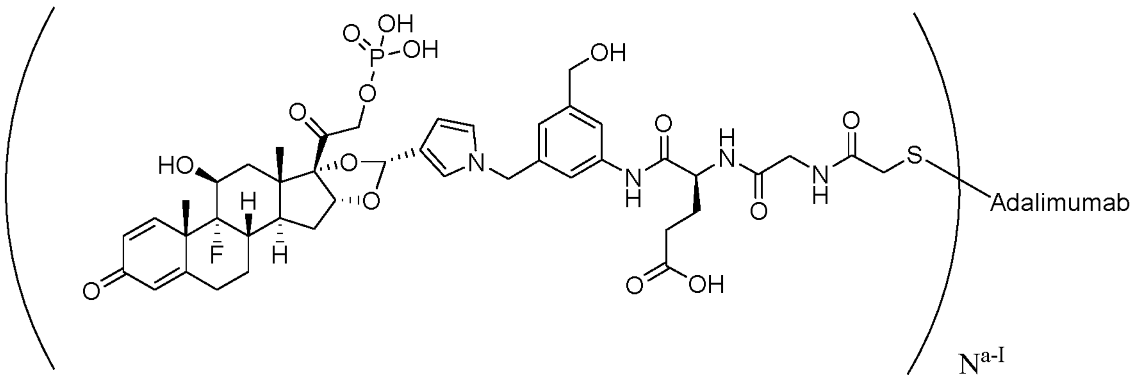 |

(continued)

| No. | Structure |
|---|---|
| V-113 | 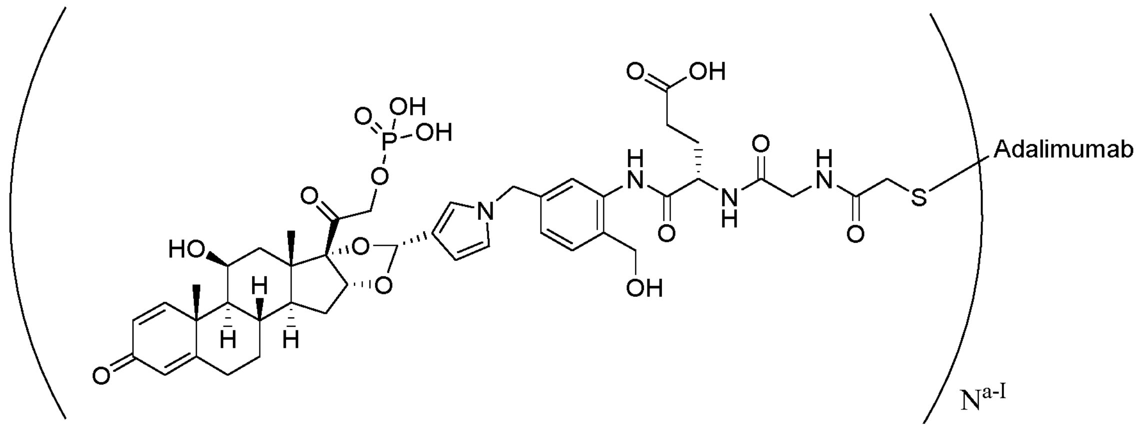 |
| V-114 | 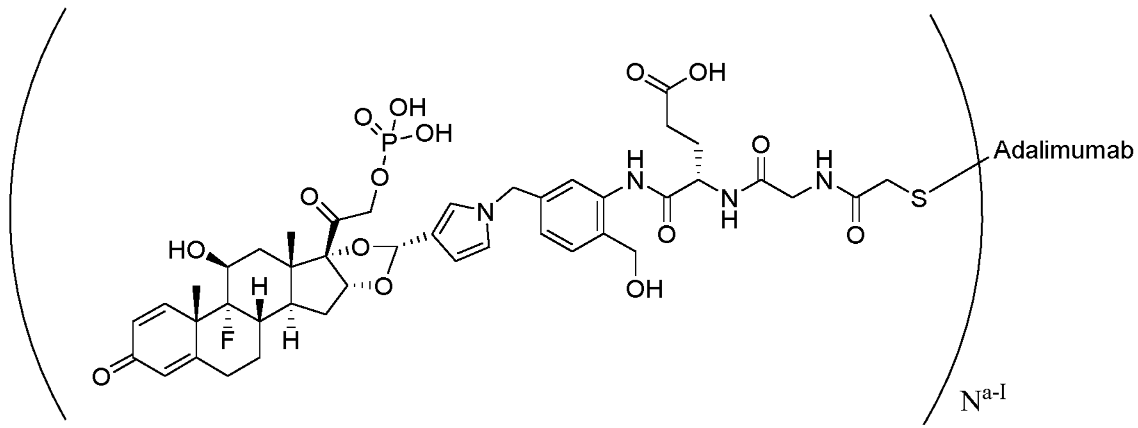 |

(continued)

| No. | Structure |
|---|---|
| V-120 | 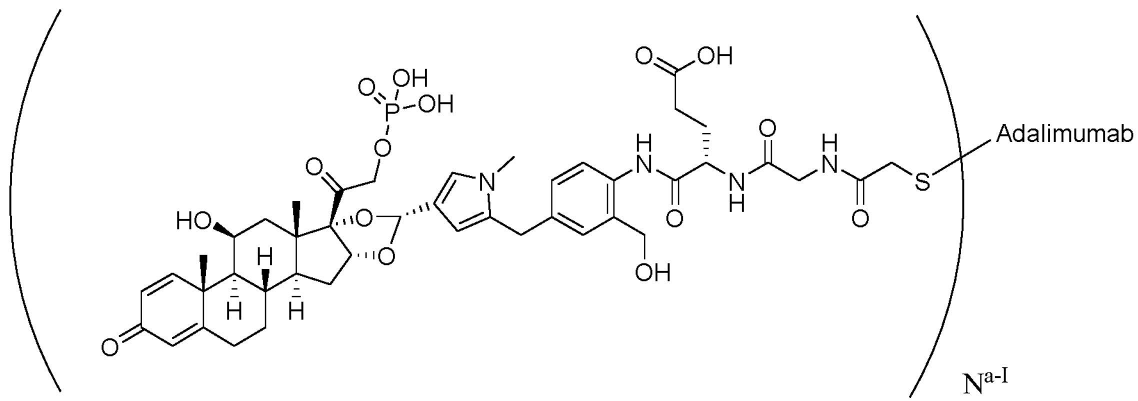 |
| V-121 | 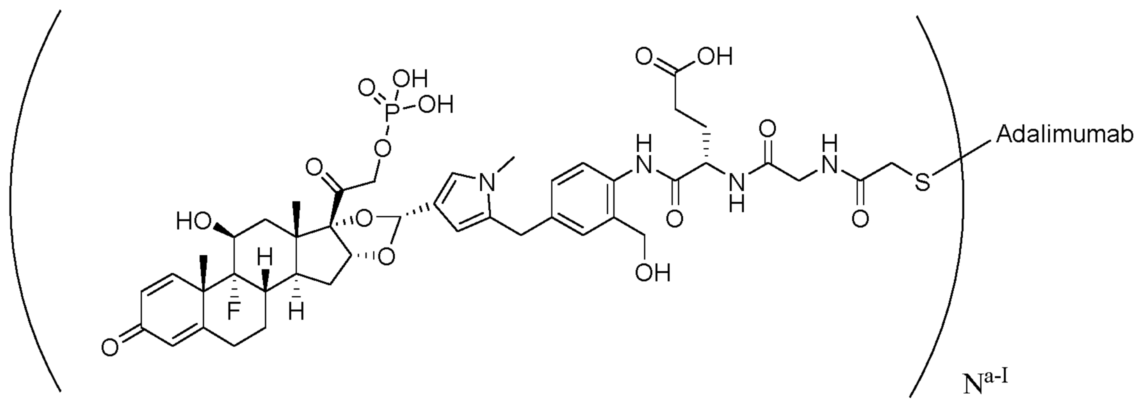 |

(continued)

| No. | Structure |
|---|---|
| V-122 | 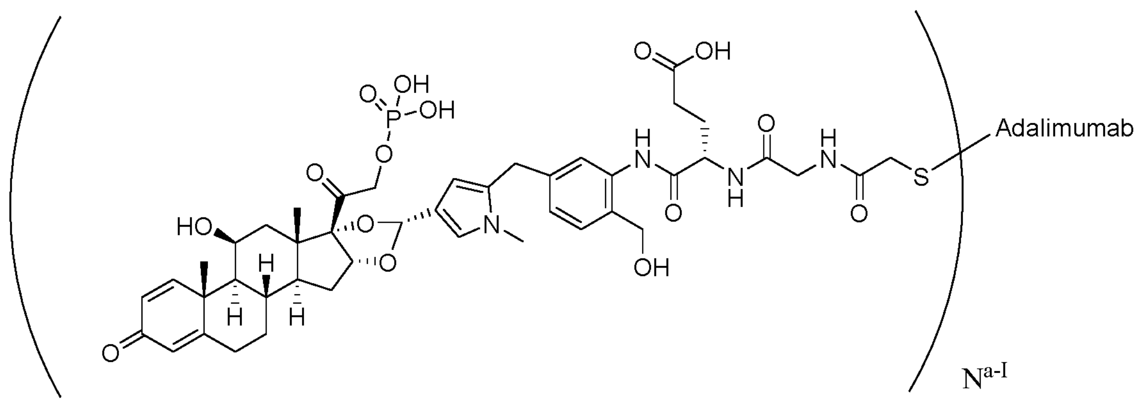 |
| V-123 | 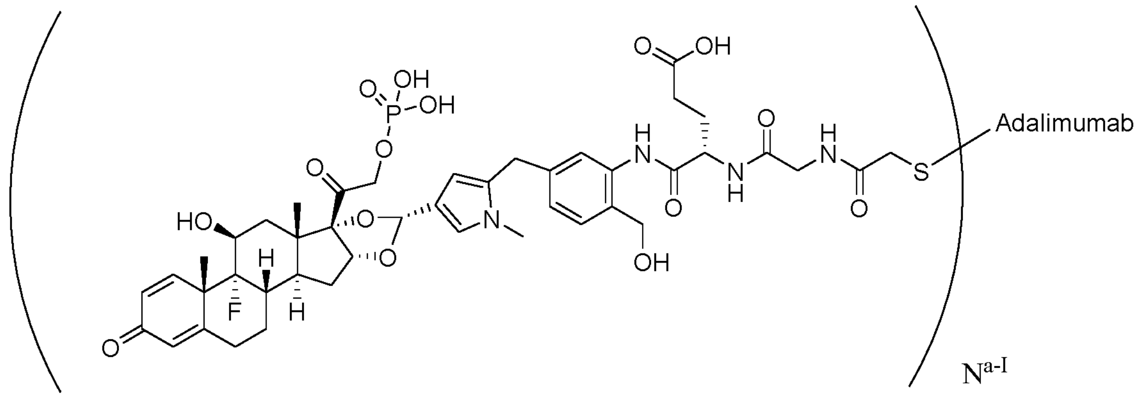 |

(continued)

| No. | Structure |
|---|---|
| V-124 | 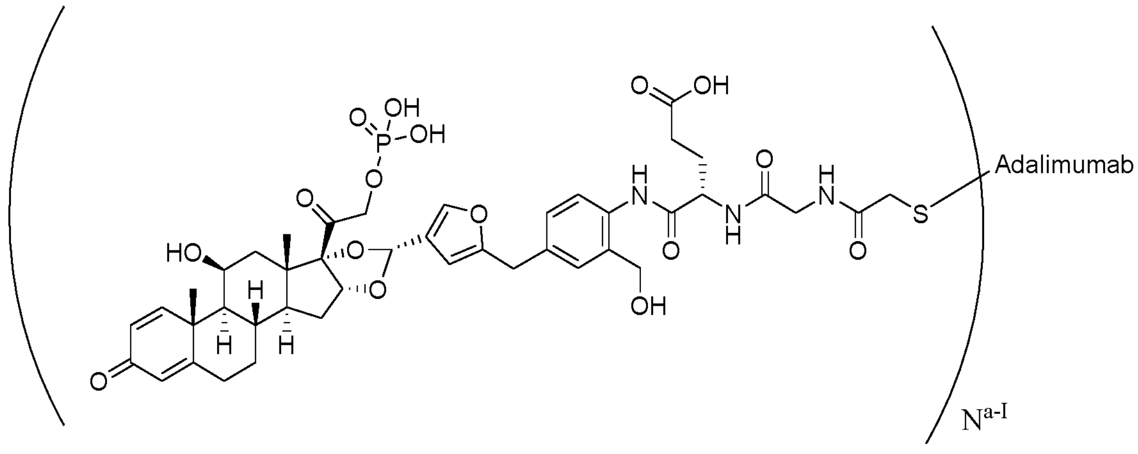 |
| V-125 | 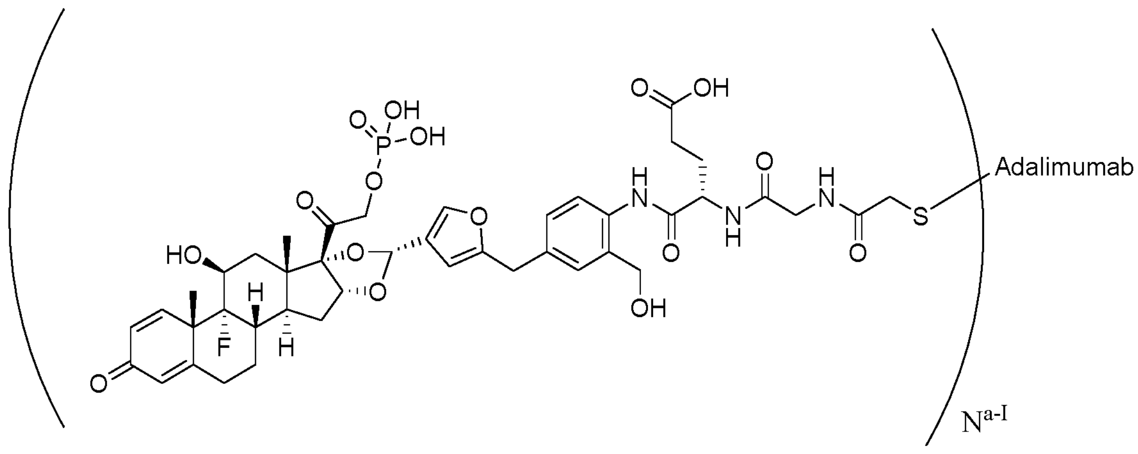 |

(continued)

| No. | Structure |
|---|---|
| V-126 | 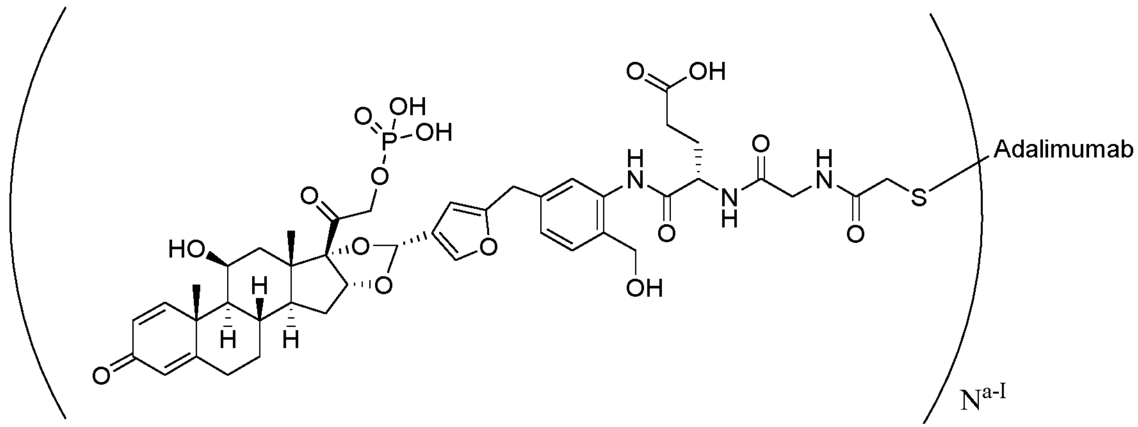 |
| V-127 | 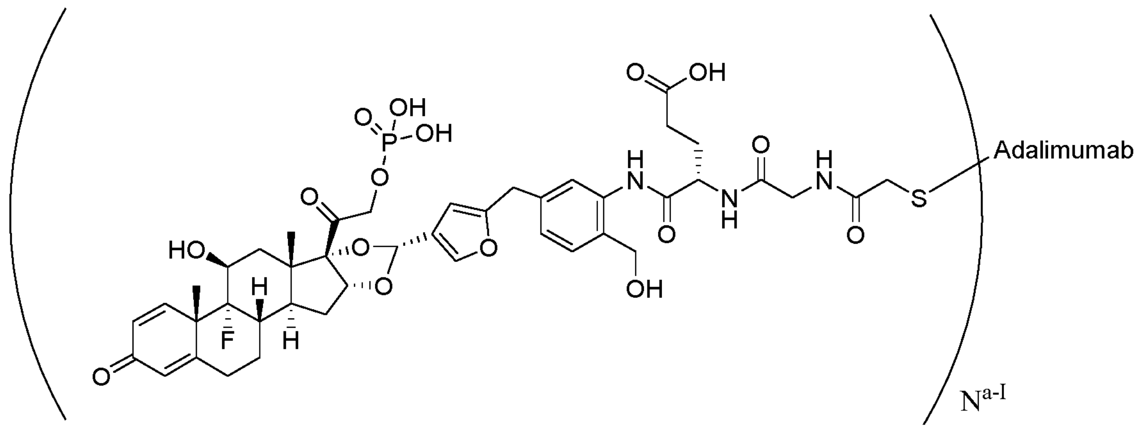 |

(continued)

| No. | Structure |
|---|---|
| V-128 | 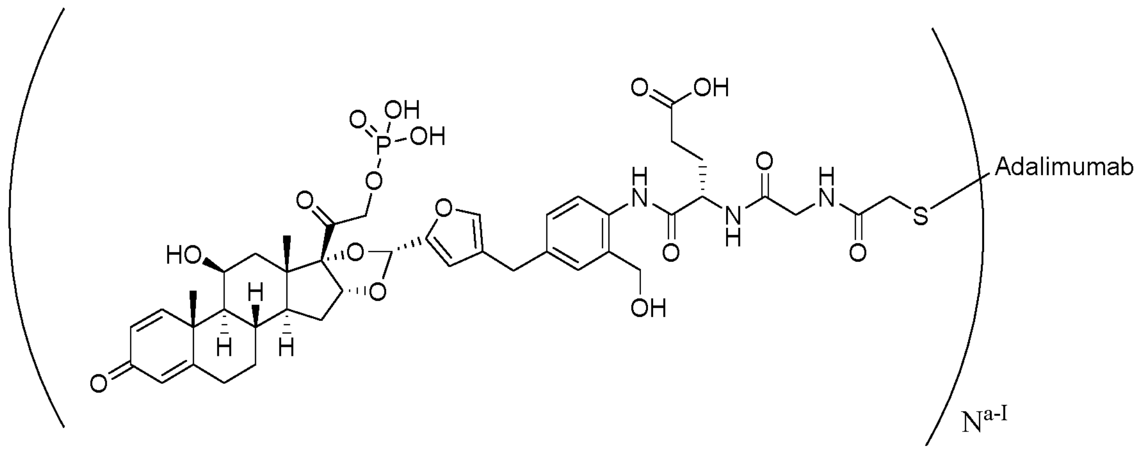 |
| V-129 | 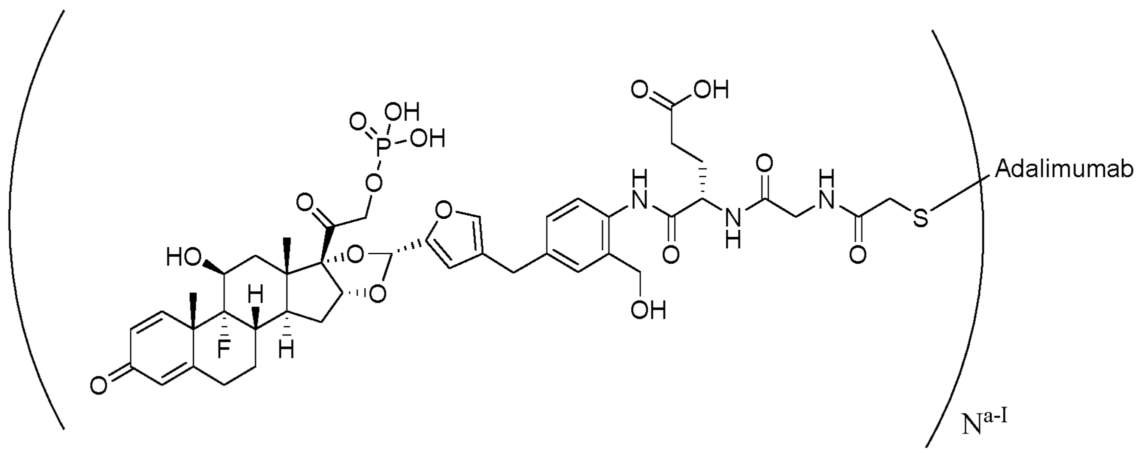 |

(continued)

| No. | Structure |
|---|---|
| V-130 | 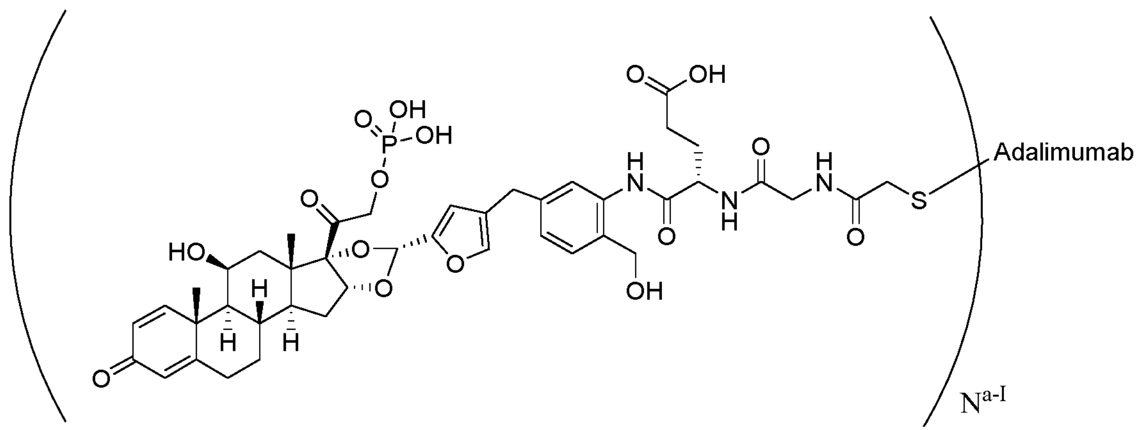 |
| V-131 | 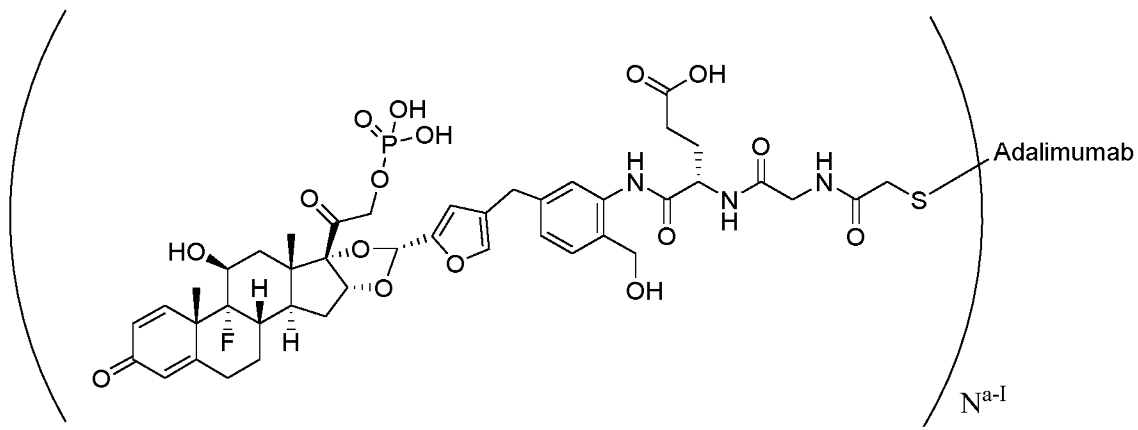 |

(continued)

| No. | Structure |
|---|---|
| V-132 | 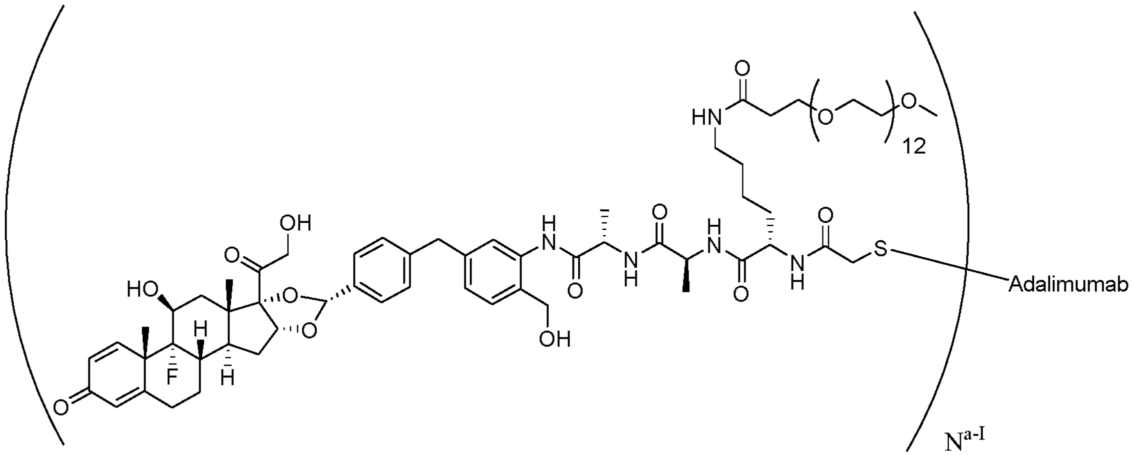 |
| V-133 | 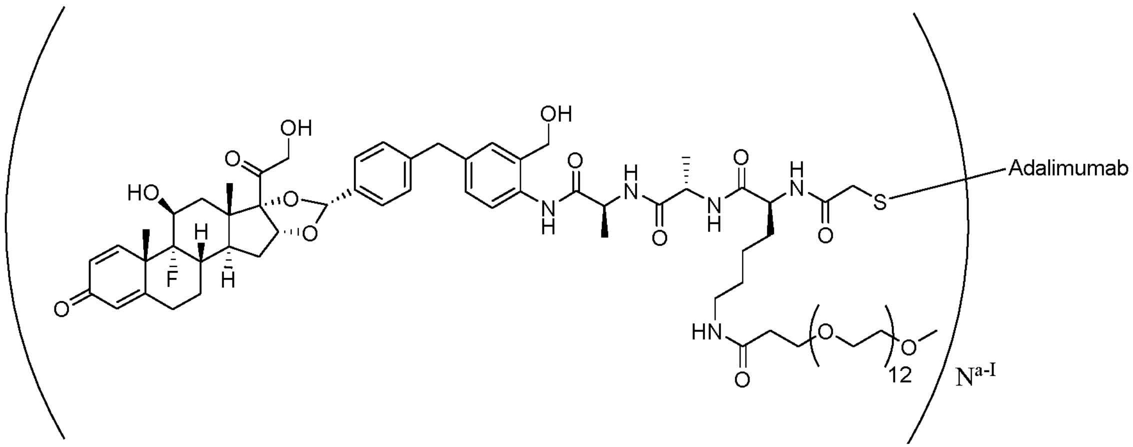 |

(continued)

| No. | Structure |
|---|---|
| V-134 | 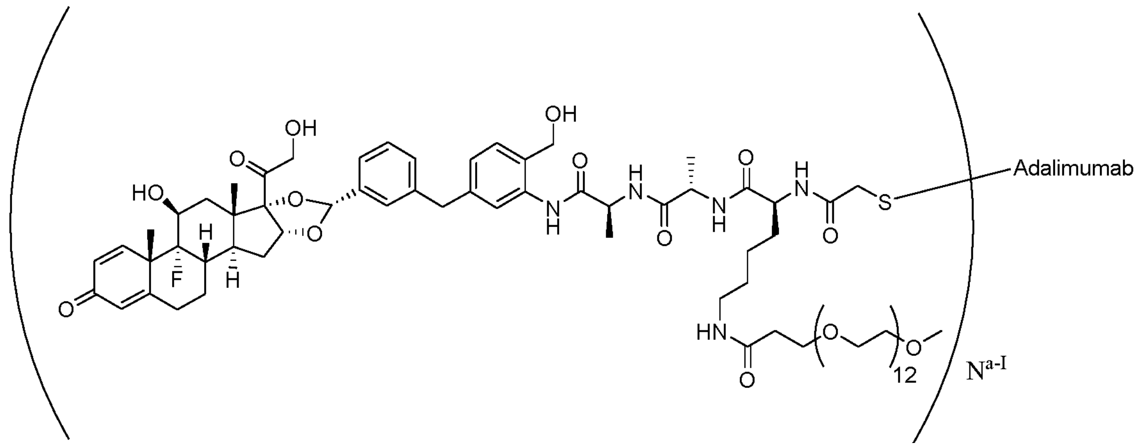 |
| V-135 | 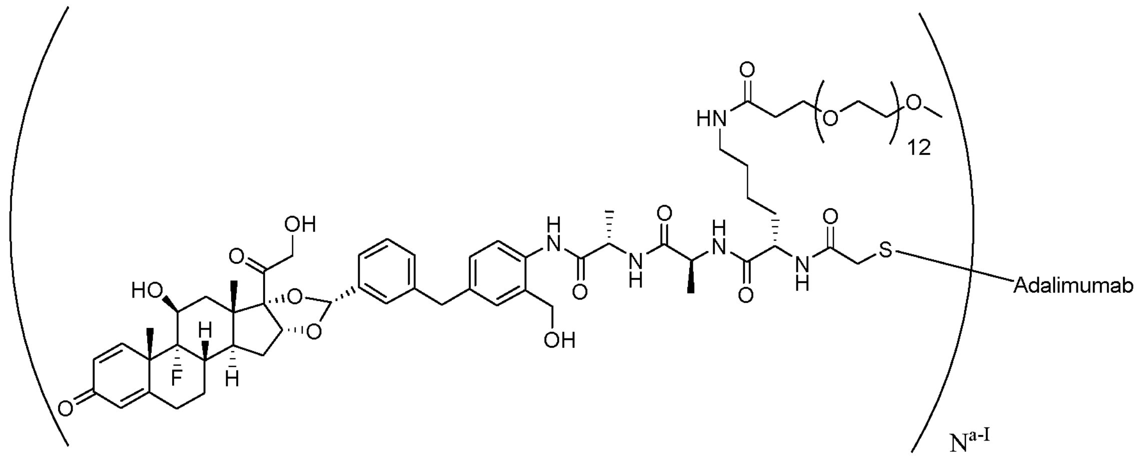 |

(continued)

| No. | Structure |
|---|---|
| V-136 | 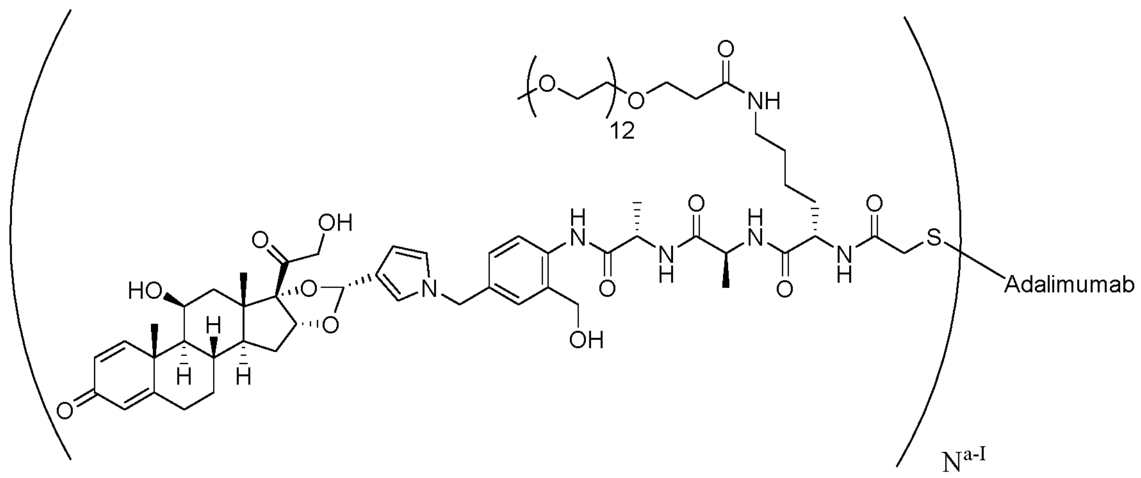 |
| V-137 | 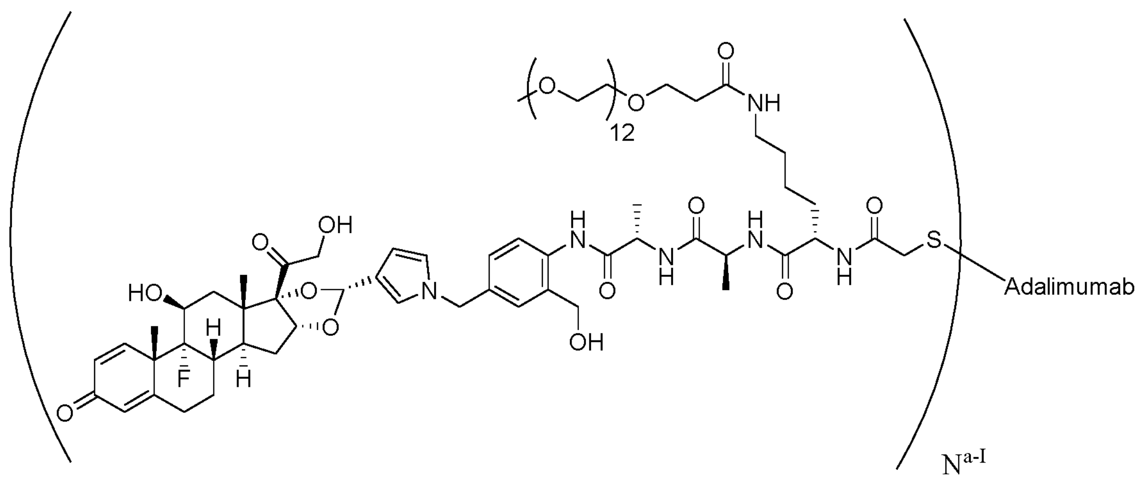 |

(continued)

| No. | Structure |
|---|---|
| V-138 | 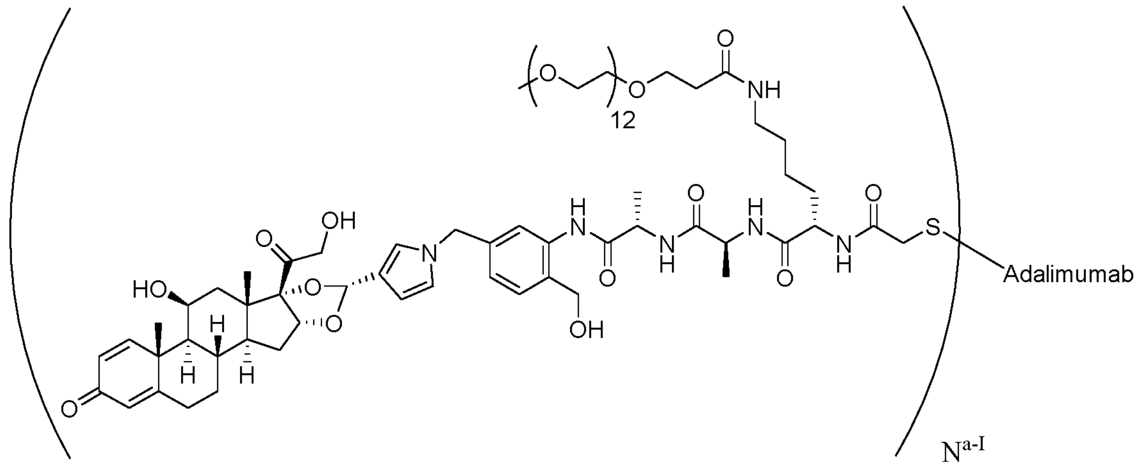 |
| V-139 | 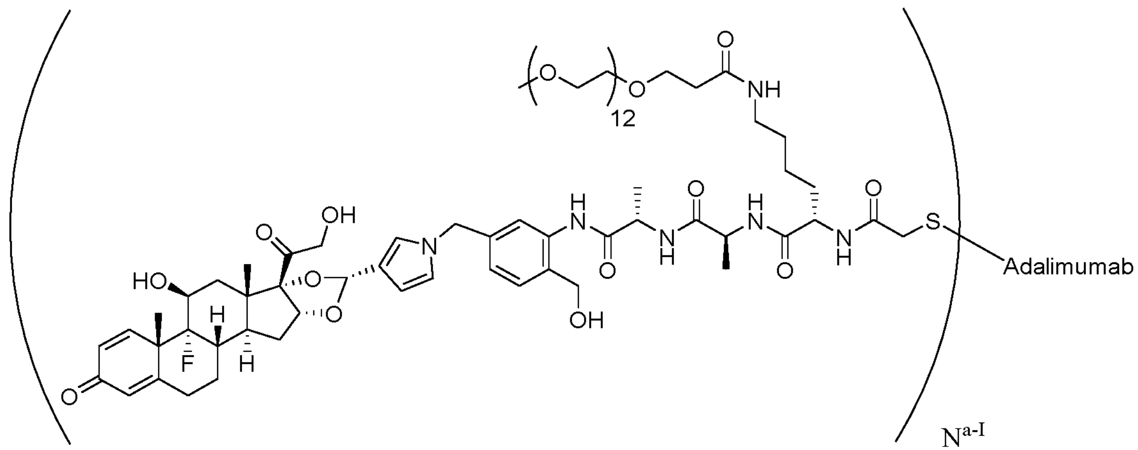 |

(continued)

| No. | Structure |
|---|---|
| V-140 | 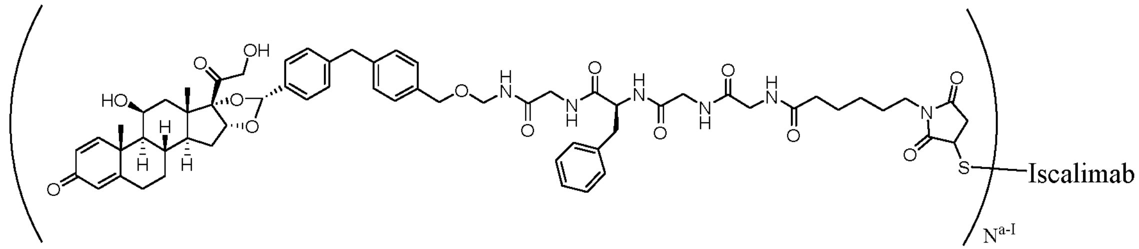 |
| V-141 | 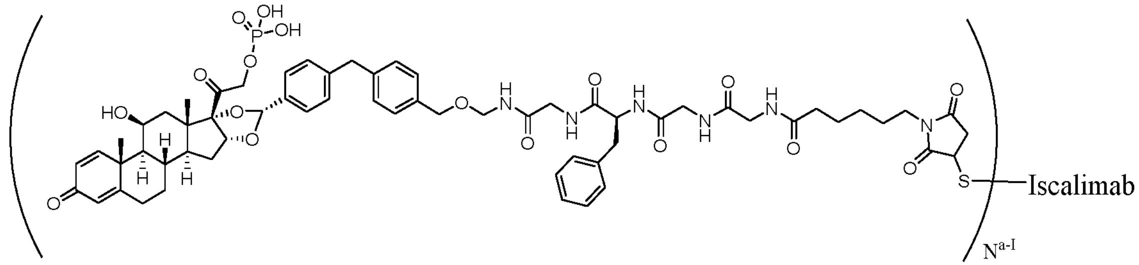 |
| V-145 | 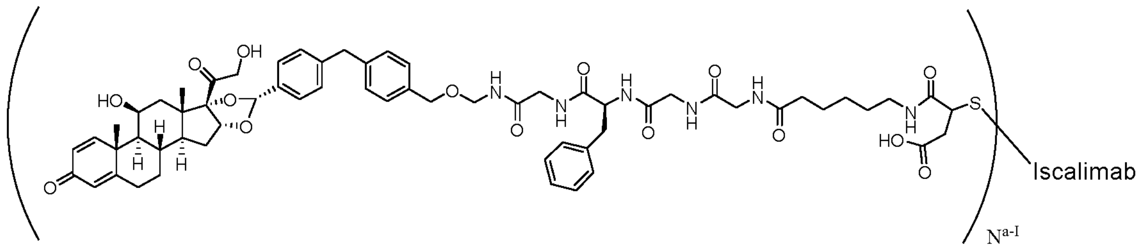 |
| V-146 | 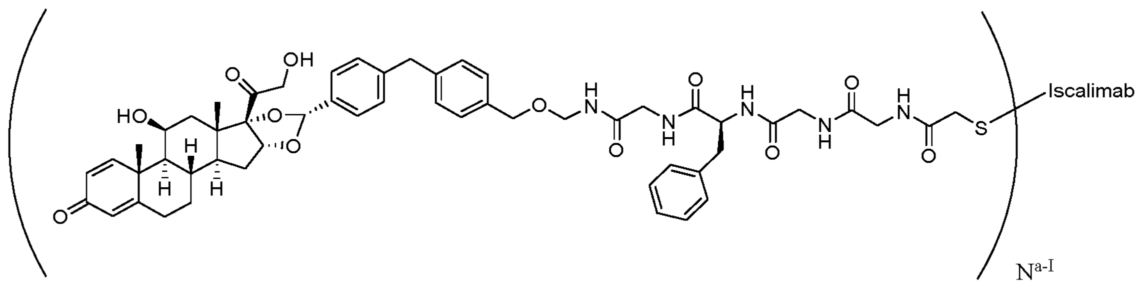 |

(continued)

| No. | Structure |
|---|---|
| V-147 | 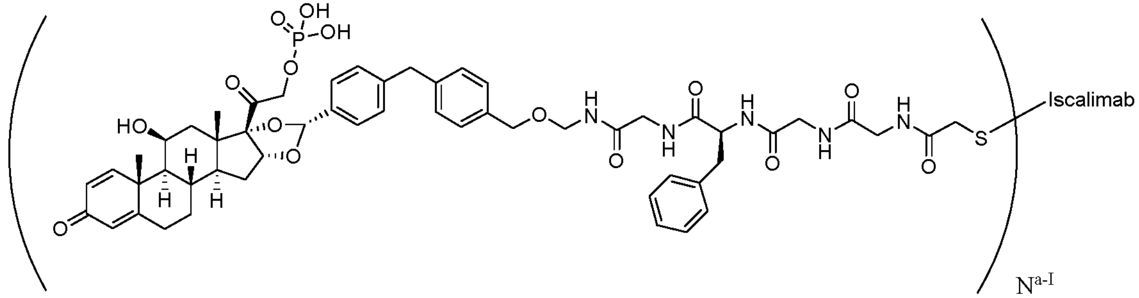 |
| V-151 | 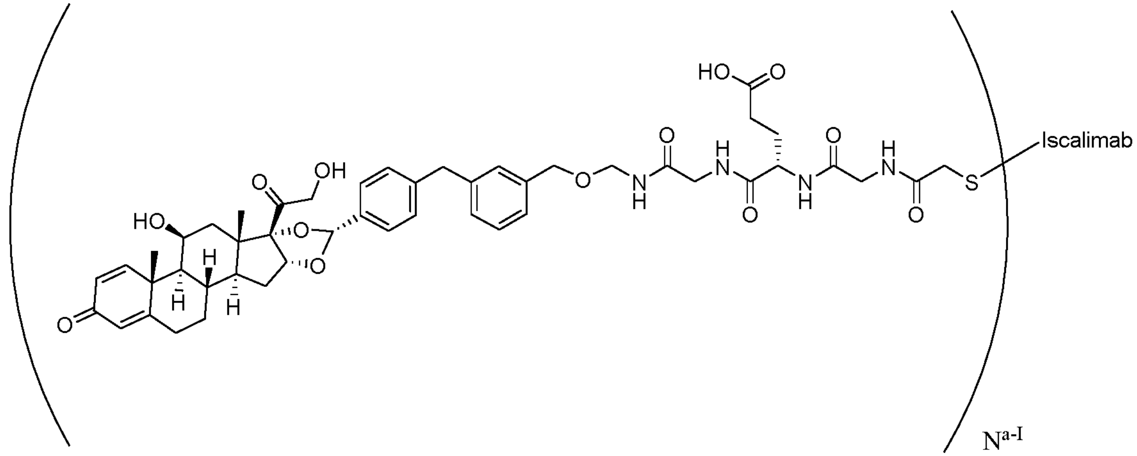 |

(continued)

| No. | Structure |
|---|---|
| V-152 | 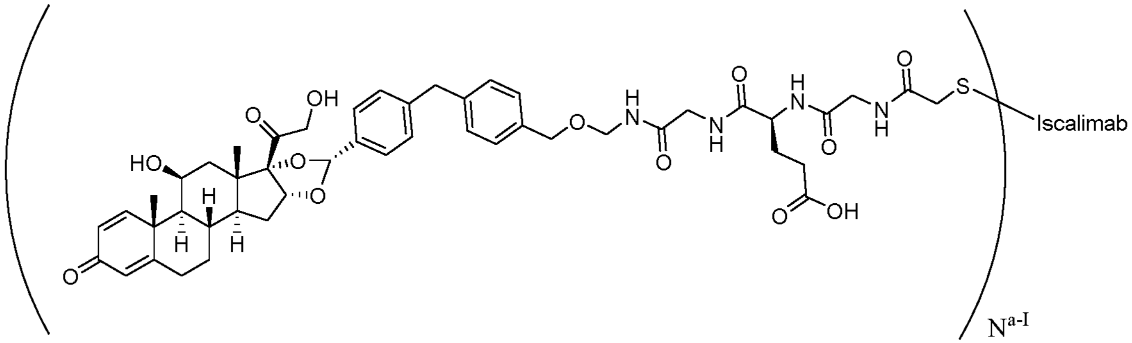 |
| V-153 | 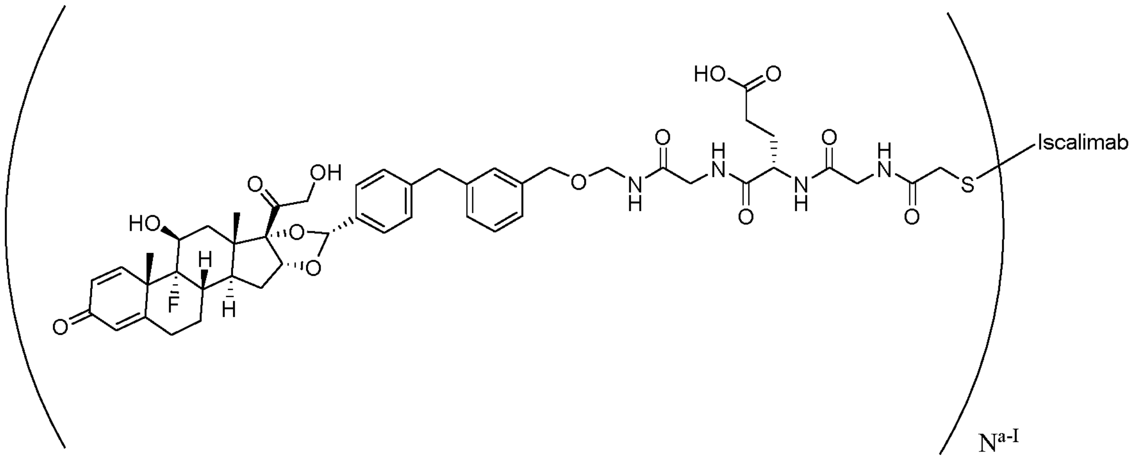 |

(continued)

| No. | Structure |
|---|---|
| V-154 | 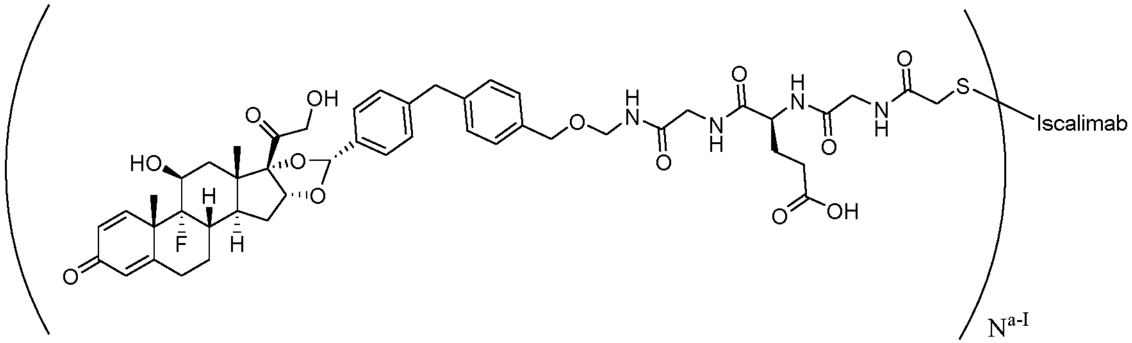 |
| V-155 | 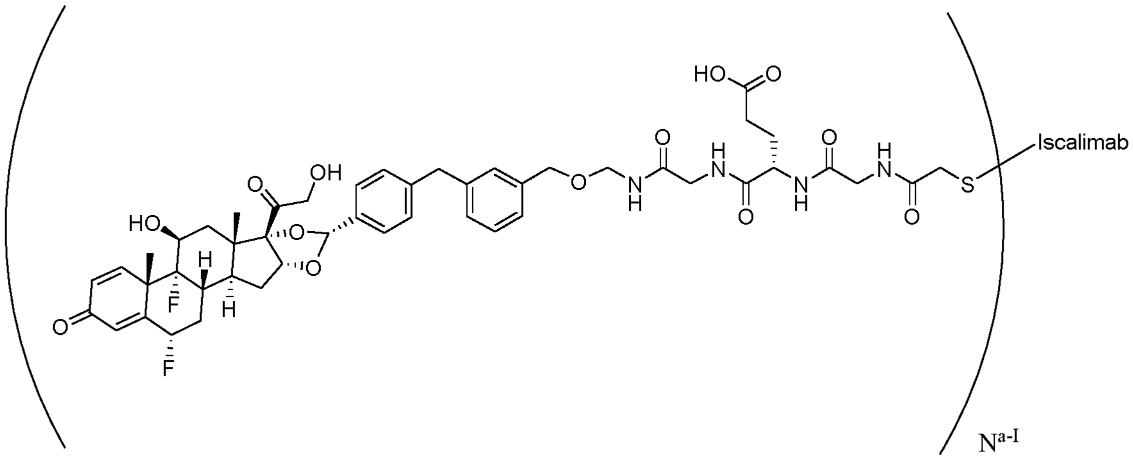 |

(continued)

| No. | Structure |
|---|---|
| V-156 | 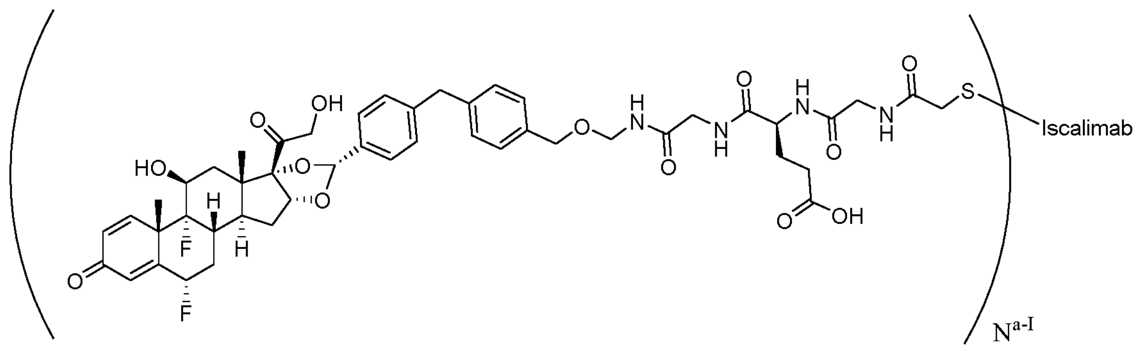 |
| V-157 | 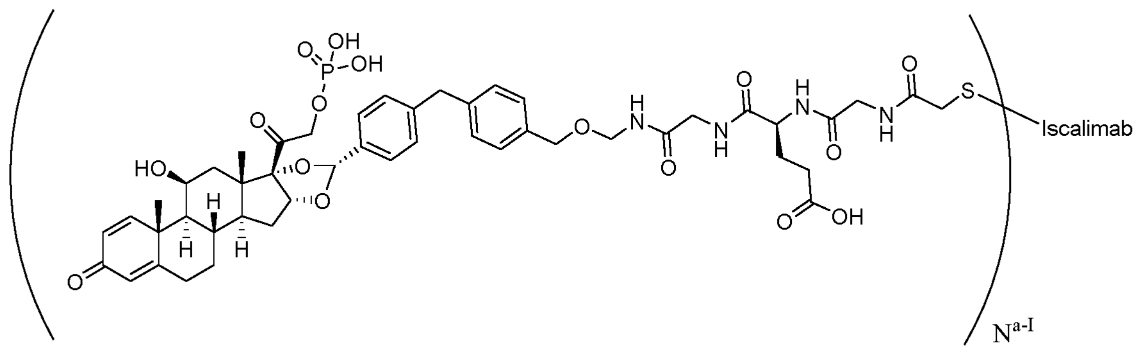 |
| V-161 | 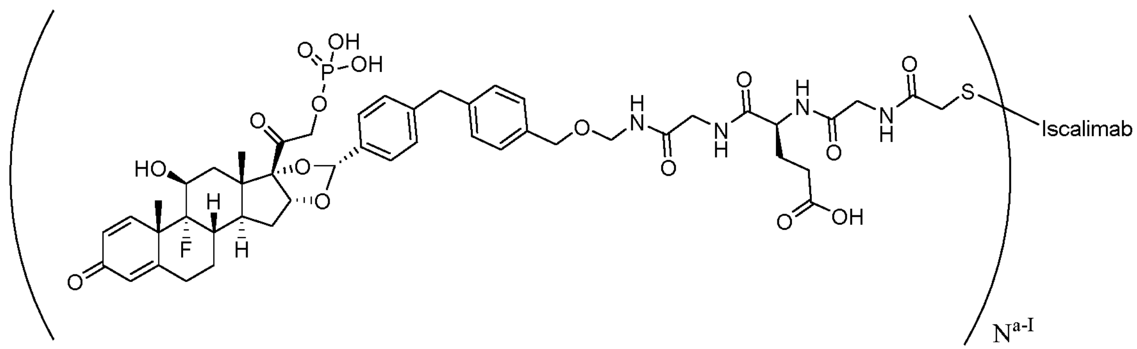 |

(continued)

| No. | Structure |
|---|---|
| V-164 | 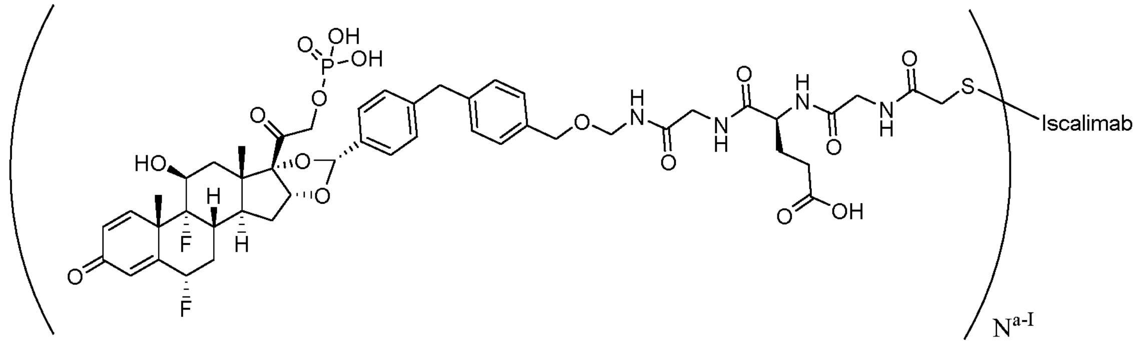 |
| V-168 | 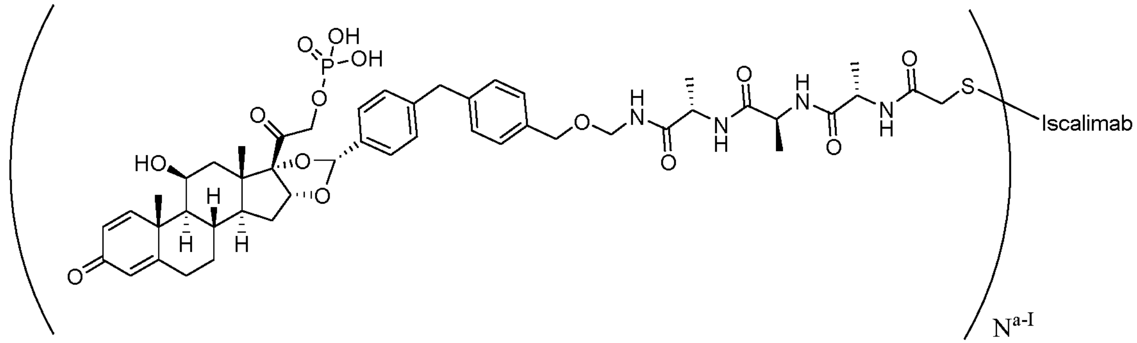 |
| V-169 | 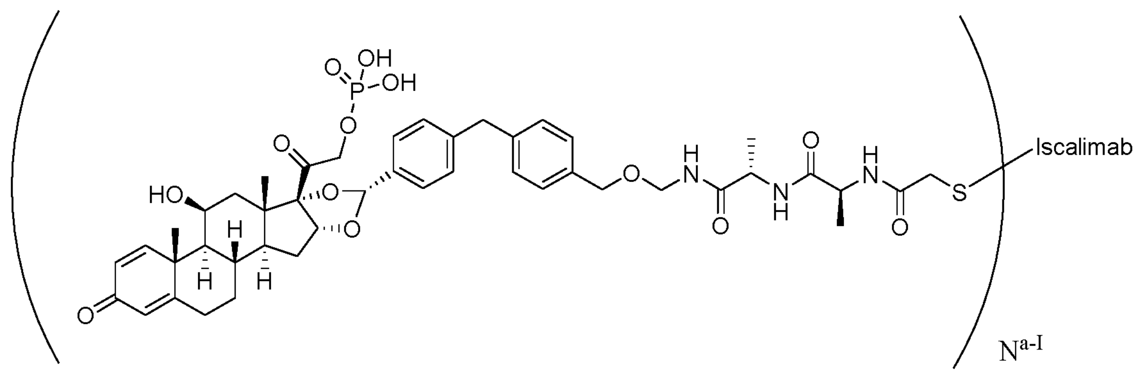 |

(continued)

| No. | Structure |
|---|---|
| V-173 | 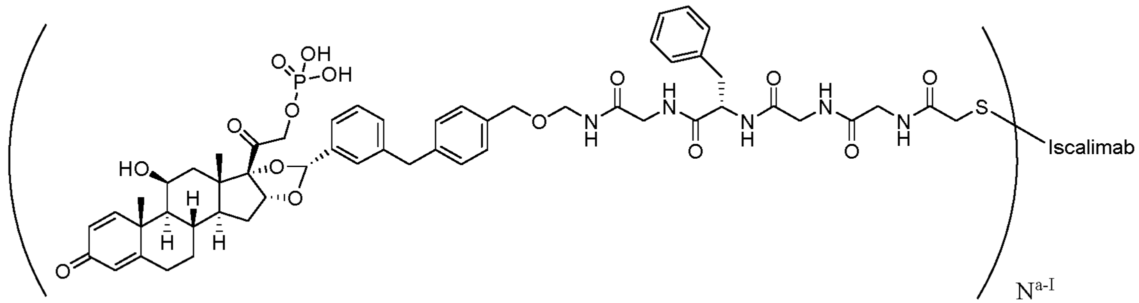 |
| V-174 | 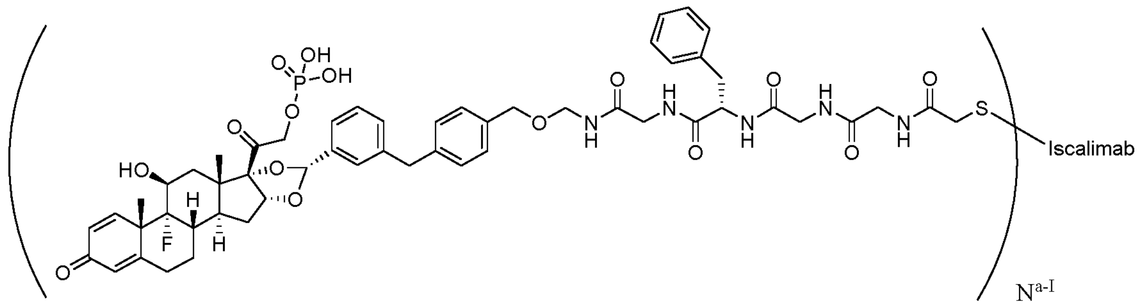 |
| V-178 | 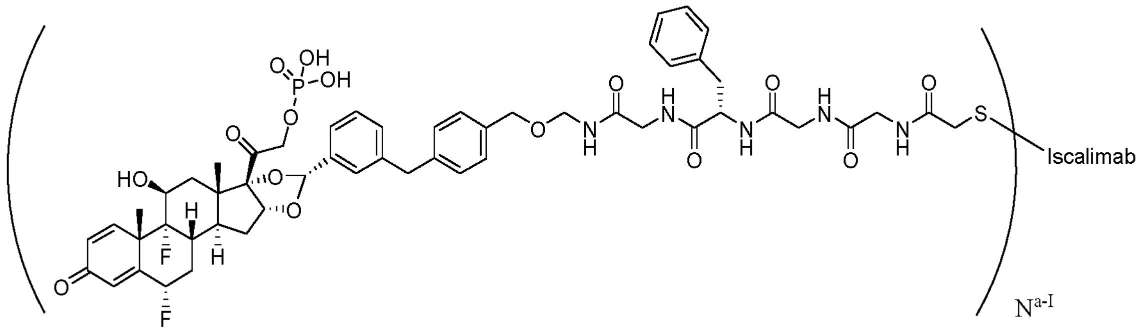 |

(continued)

| No. | Structure |
|---|---|
| V-182 | 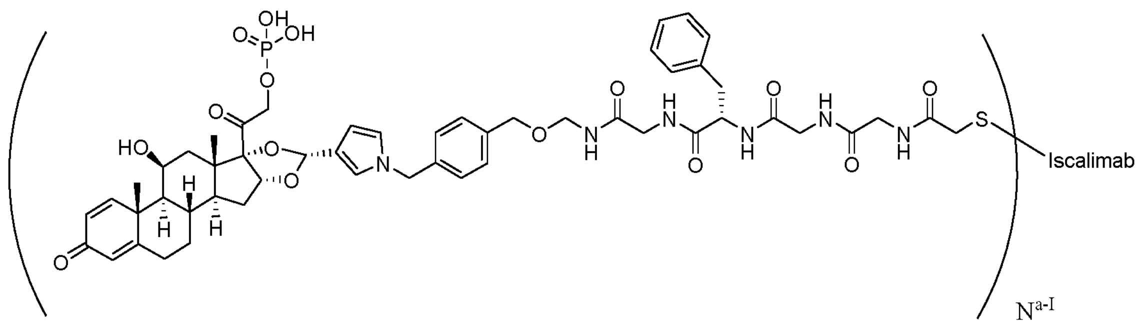 |
| V-183 | 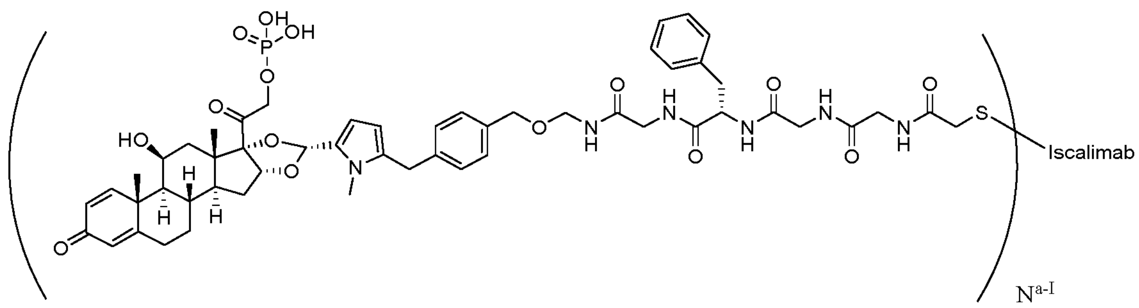 |
| V-184 | 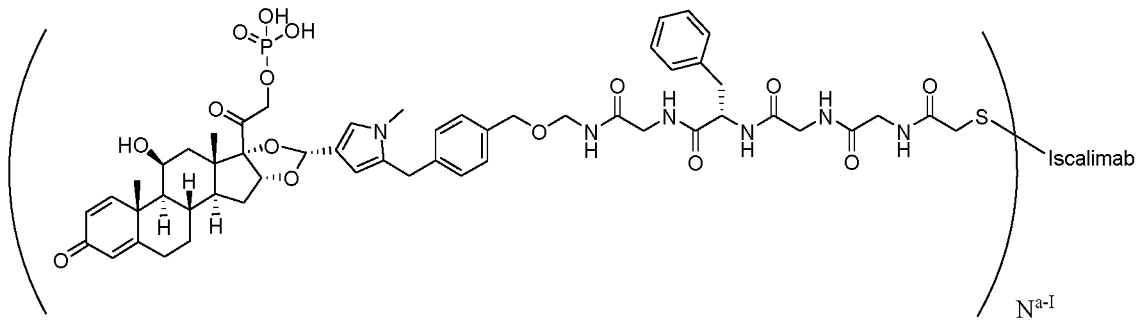 |

(continued)

| No. | Structure |
|---|---|
| V-185 | 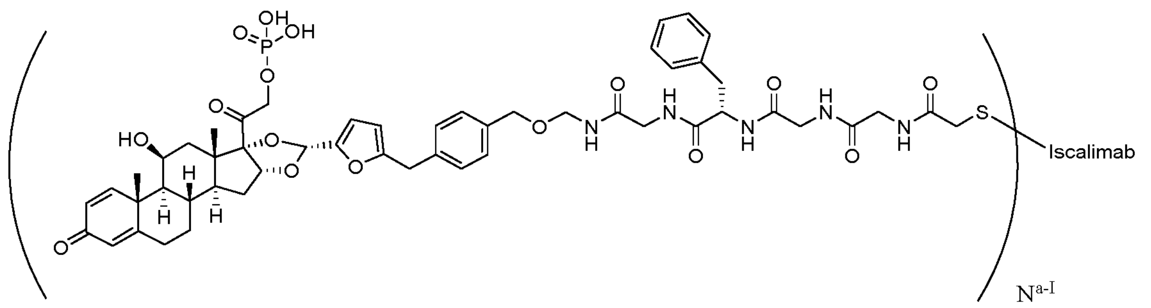 |
| V-186 | 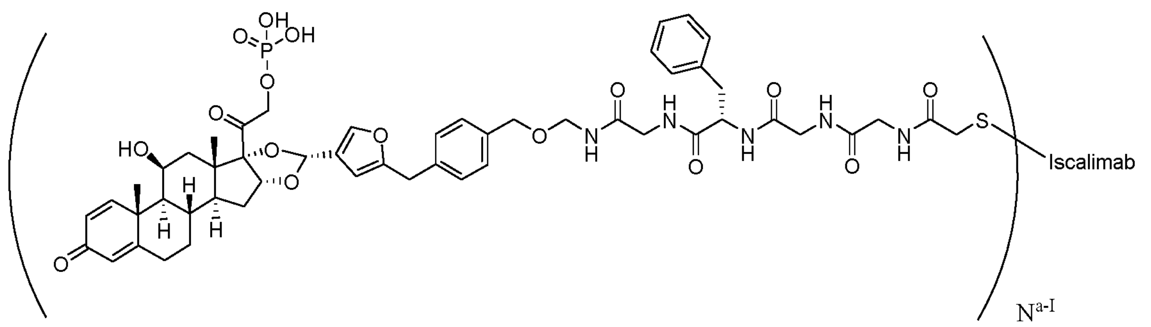 |
| V-187 | 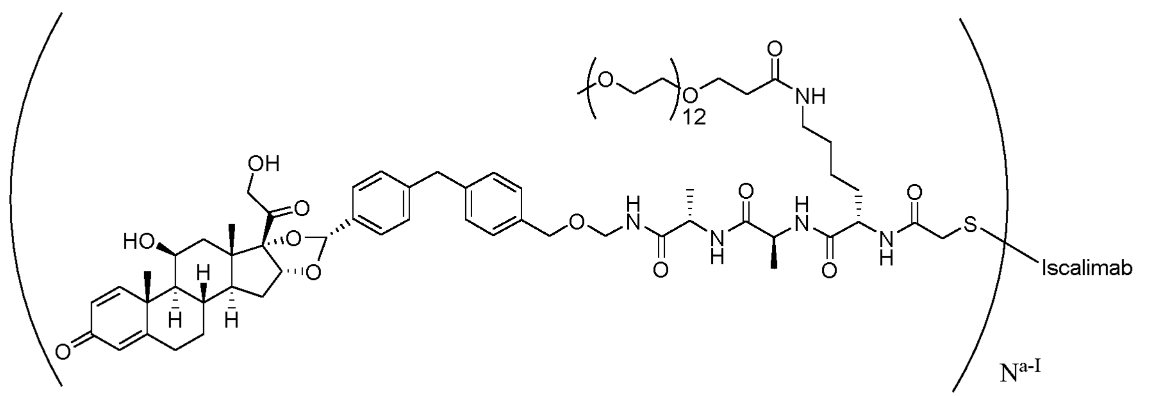 |

(continued)

| No. | Structure |
|---|---|
| V-188 | 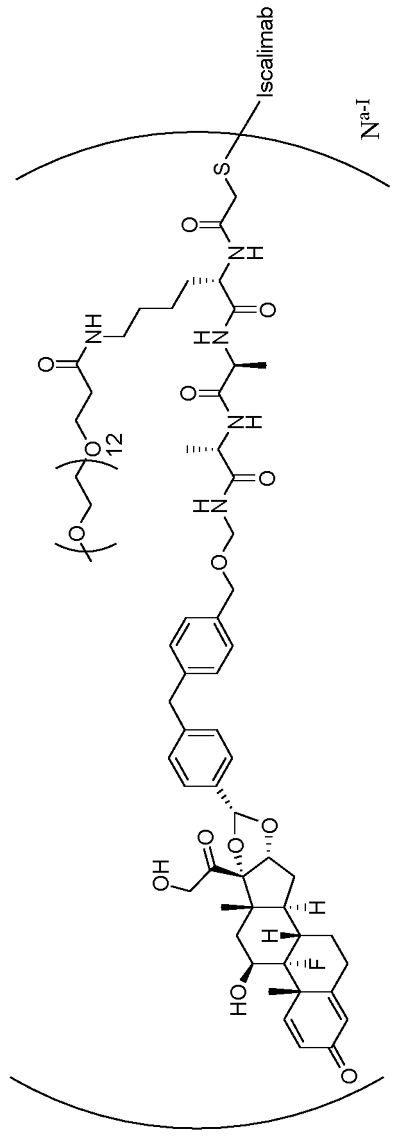 |
| V-189 | 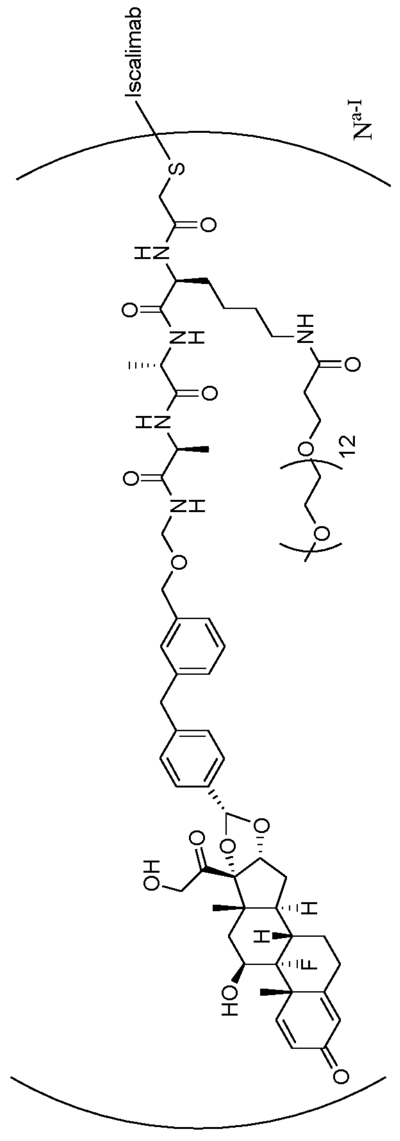 |

(continued)

| No. | Structure |
|---|---|
| V-190 | 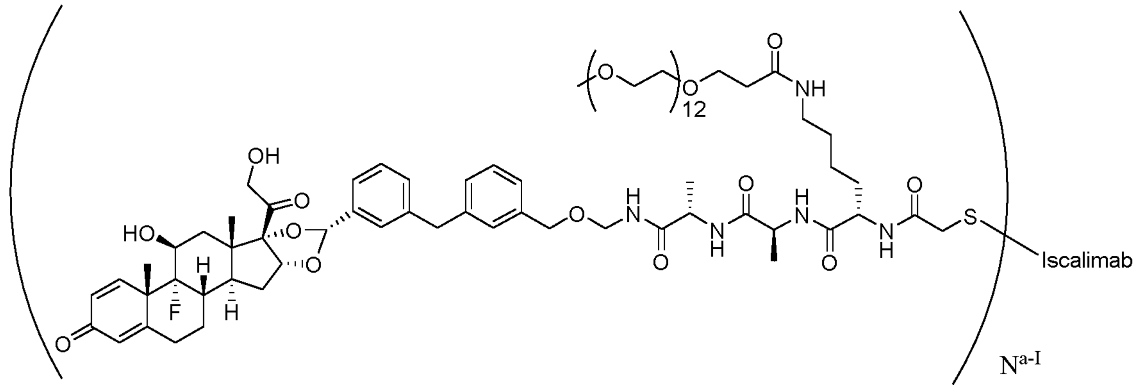 |
| V-191 | 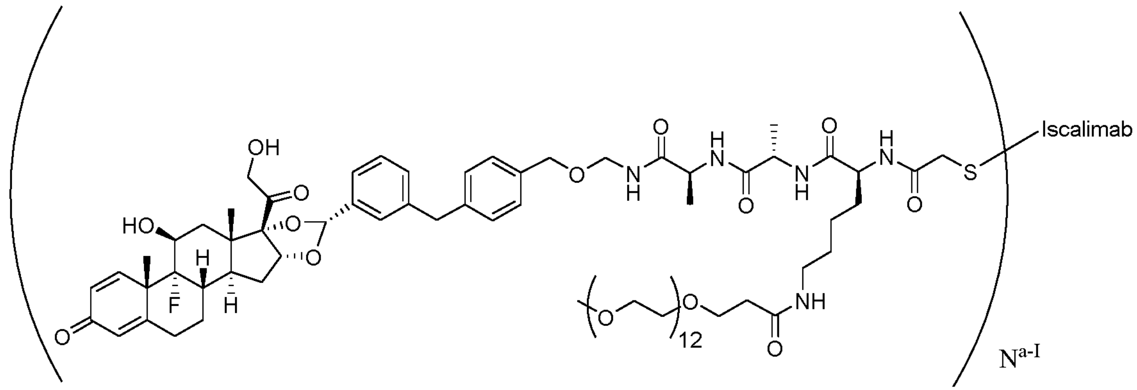 |

(continued)

| No. | Structure |
| --- | --- |
| V-192 | 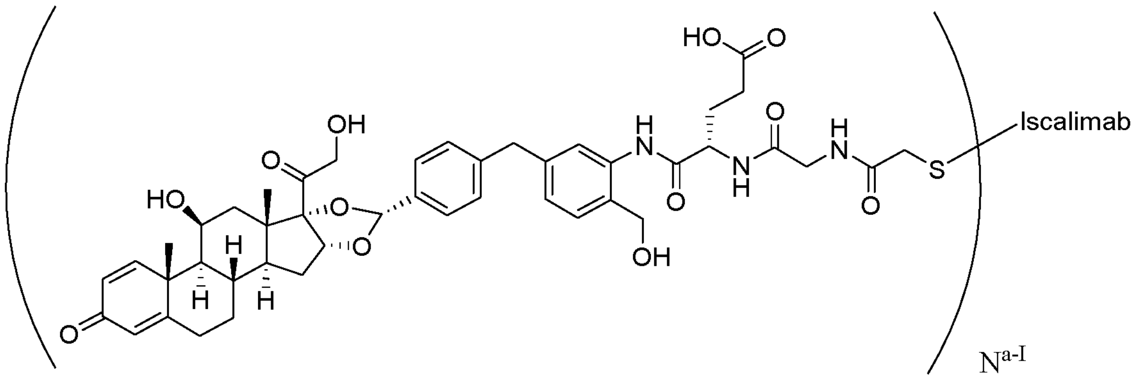 |
| V-193 | 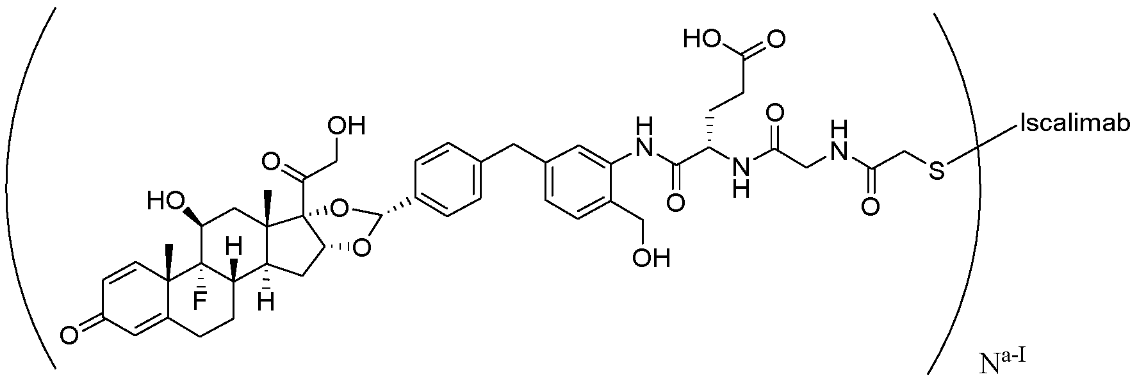 |

(continued)

| No. | Structure |
| --- | --- |
| V-194 | 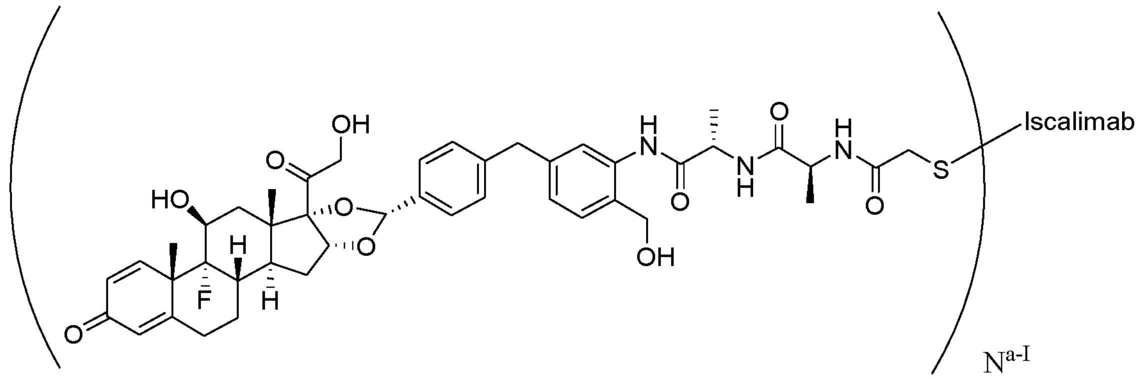 |
| V-195 | 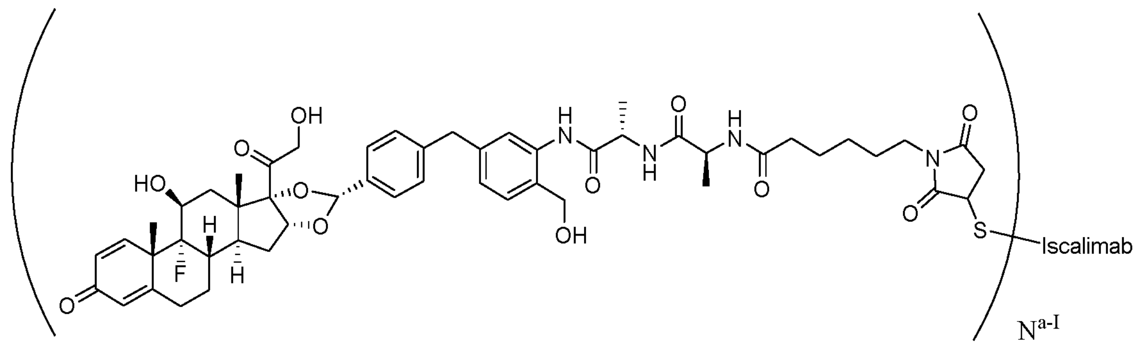 |

(continued)

| No. | Structure |
|---|---|
| V-196 | 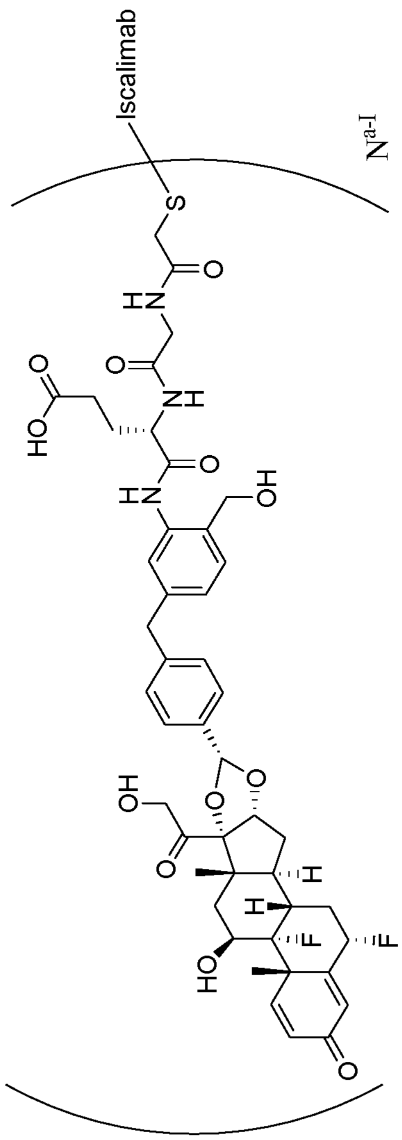 |
| V-197 | 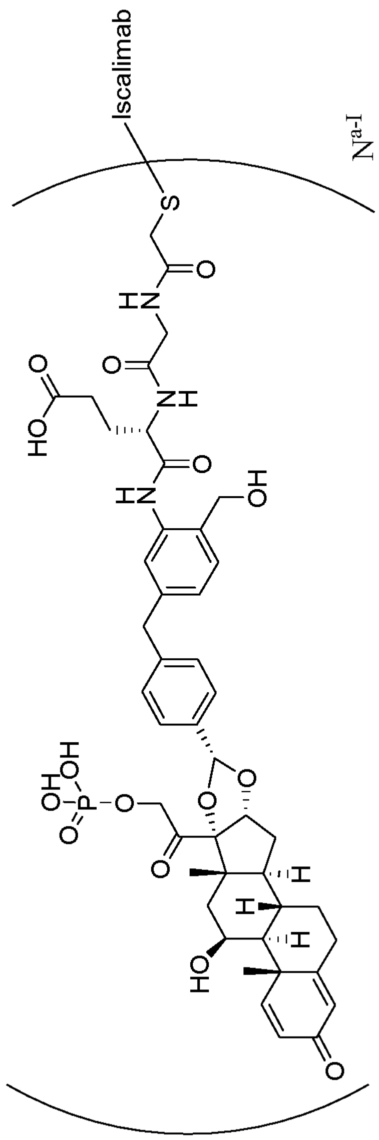 |

(continued)

| No. | Structure |
|---|---|
| V-198 | 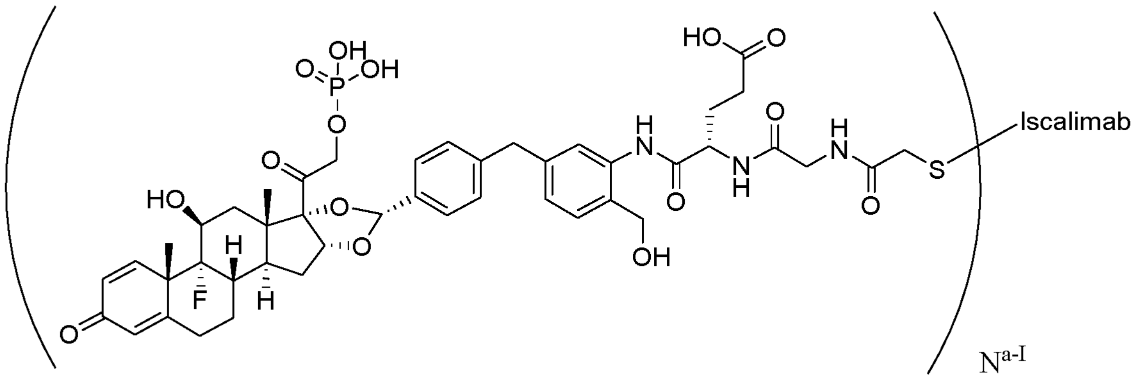 |
| V-199 | 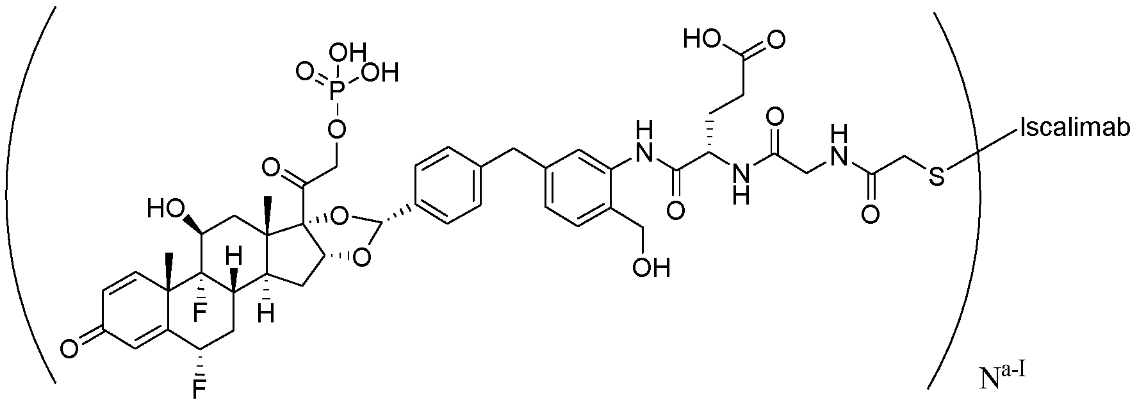 |

(continued)

| No. | Structure |
|---|---|
| V-209 | 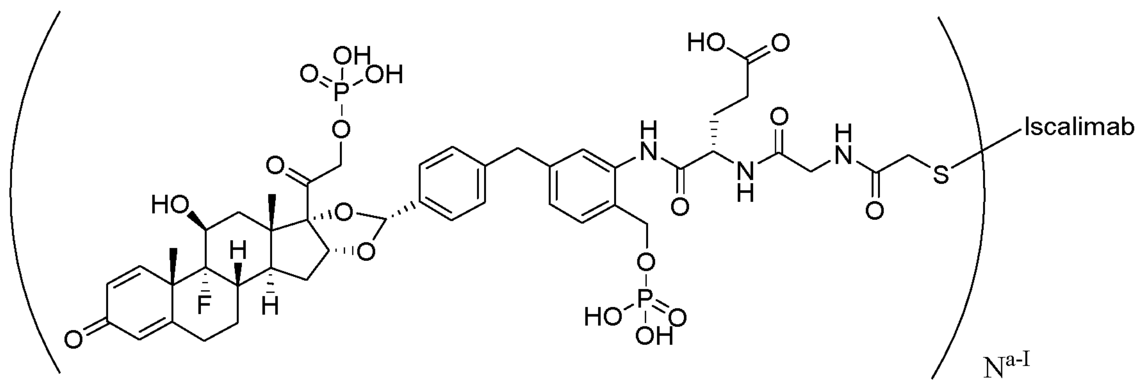 |
| V-213 | 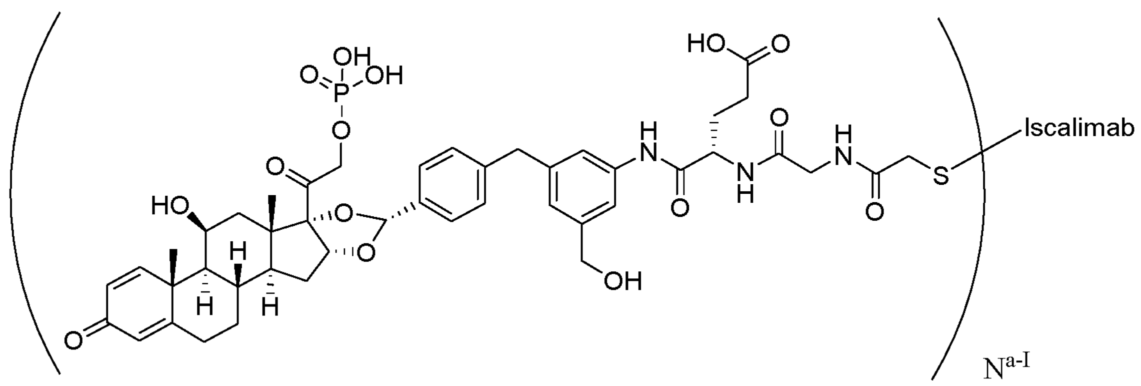 |

(continued)

| No. | Structure |
|---|---|
| V-216 | 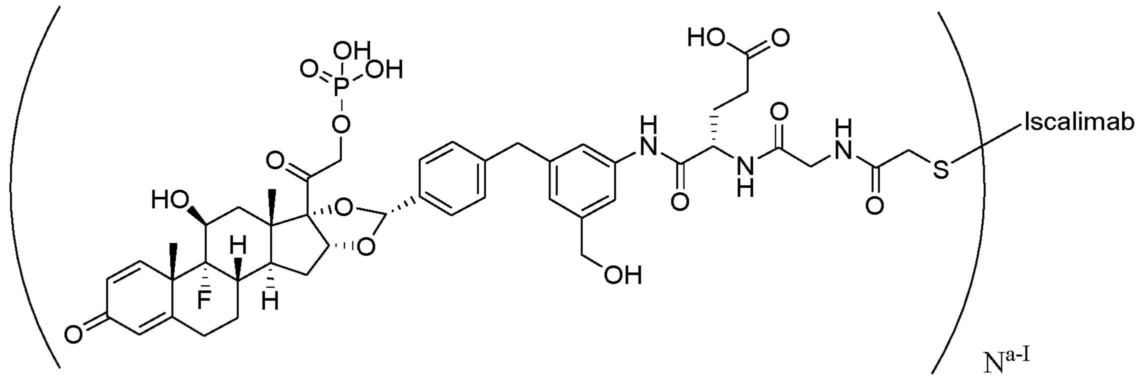 |
| V-219 | 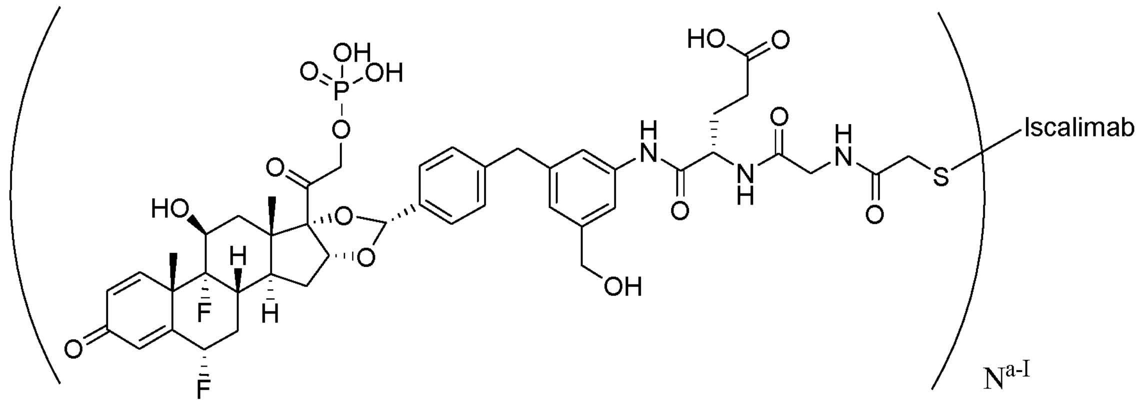 |

(continued)

| No. | Structure |
|---|---|
| V-222 | 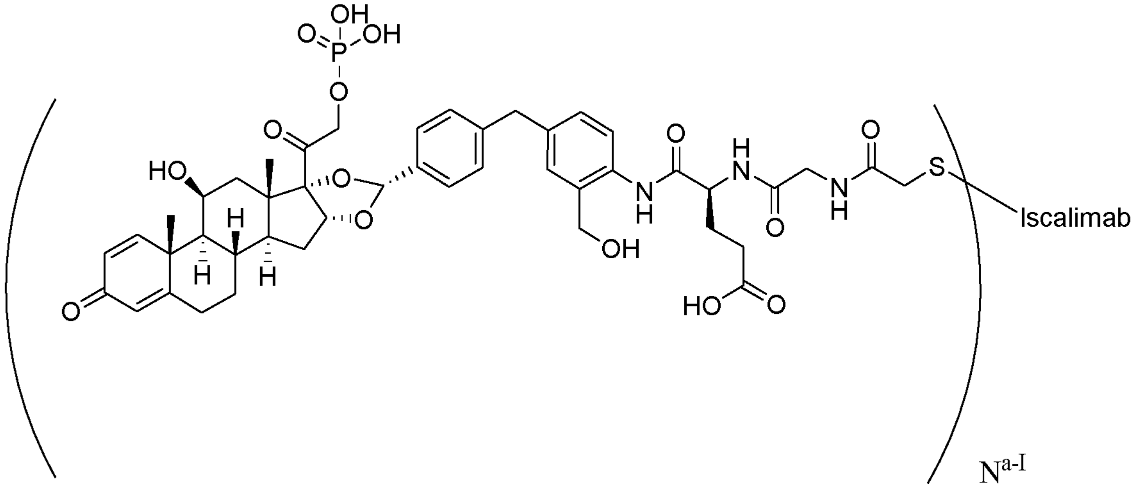 |
| V-225 | 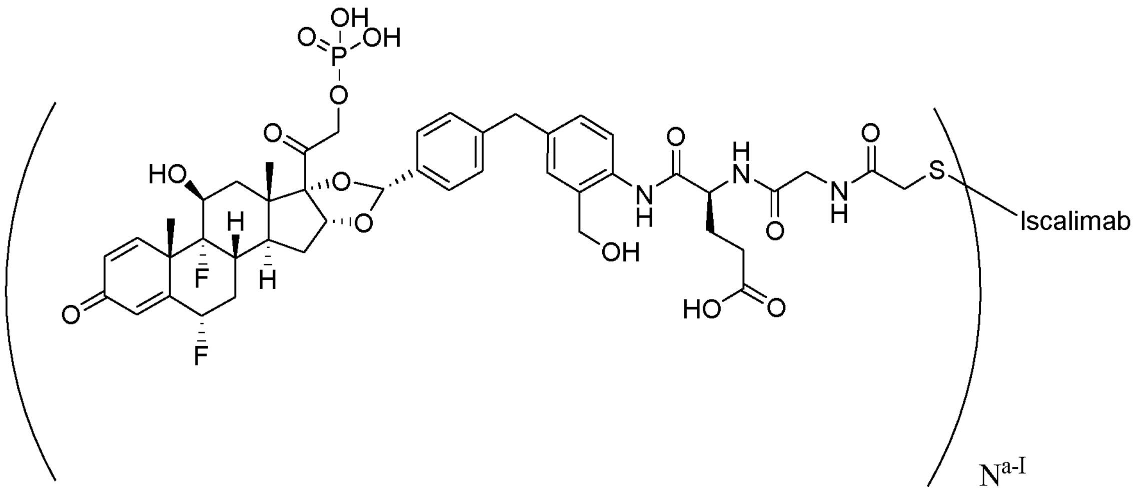 |

(continued)

| No. | Structure |
|---|---|
| V-228 | 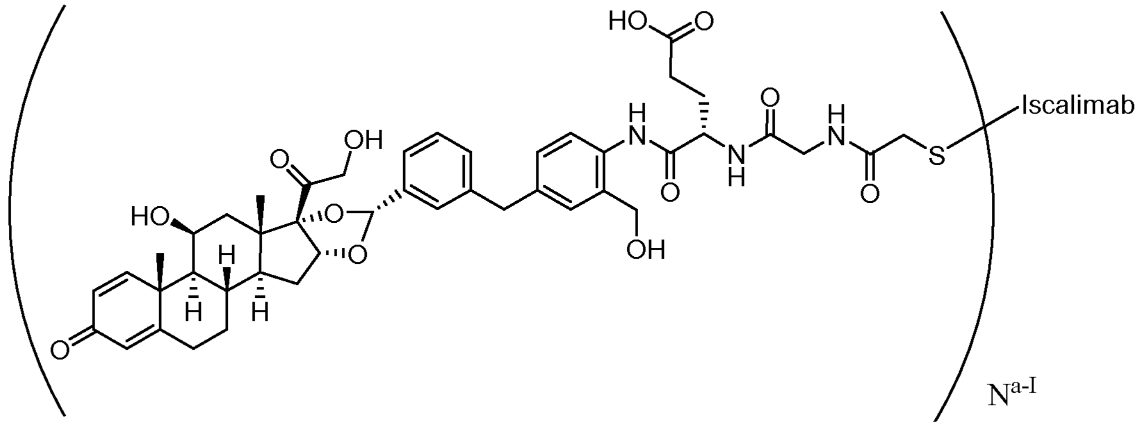 |
| V-229 | 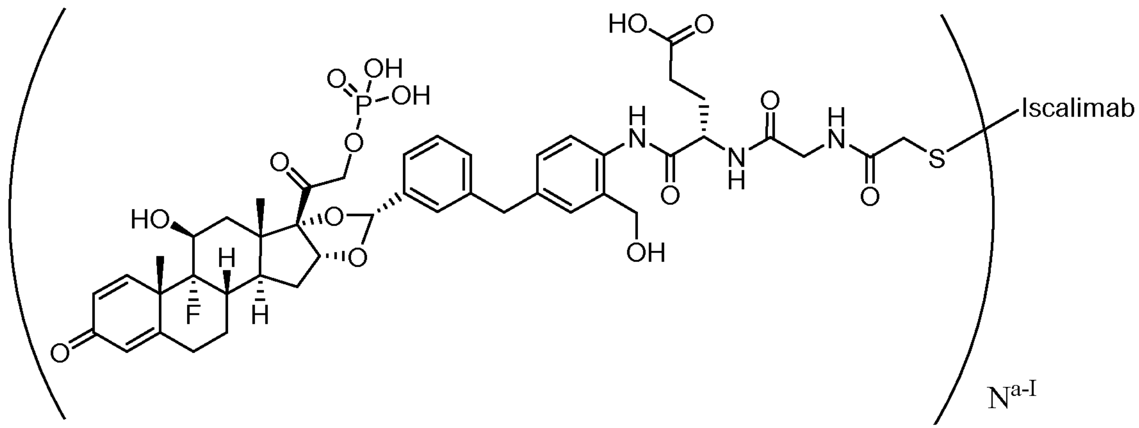 |

(continued)

| No. | Structure |
| --- | --- |
| V-230 | 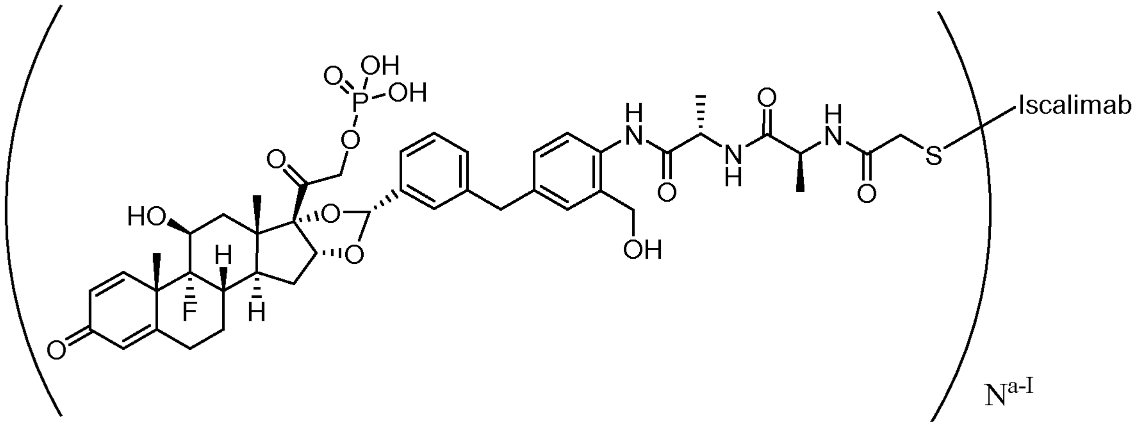 |
| V-233 | 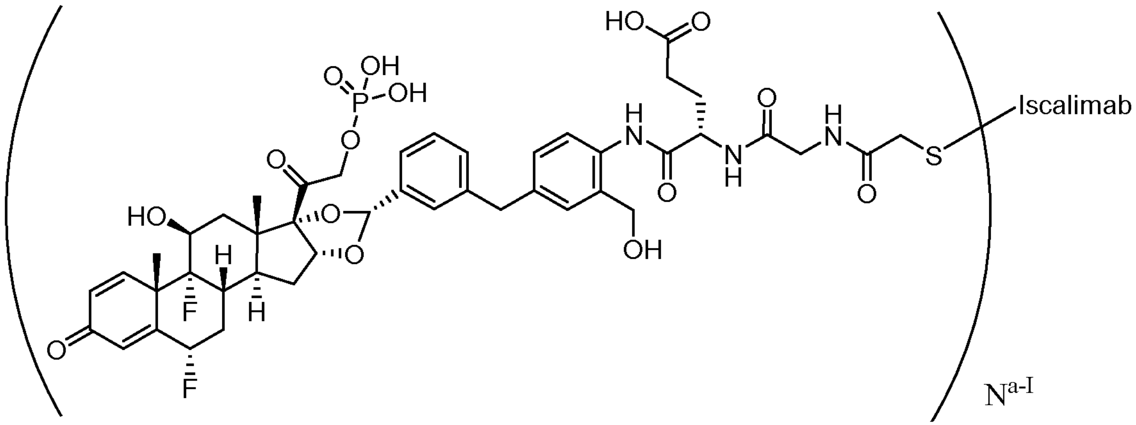 |

(continued)

| No. | Structure |
|---|---|
| V-236 | 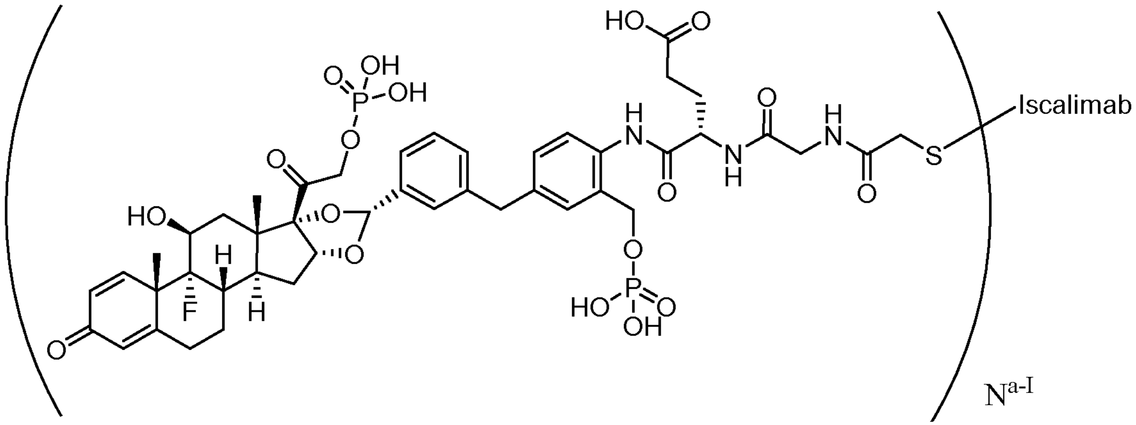 |
| V-240 | 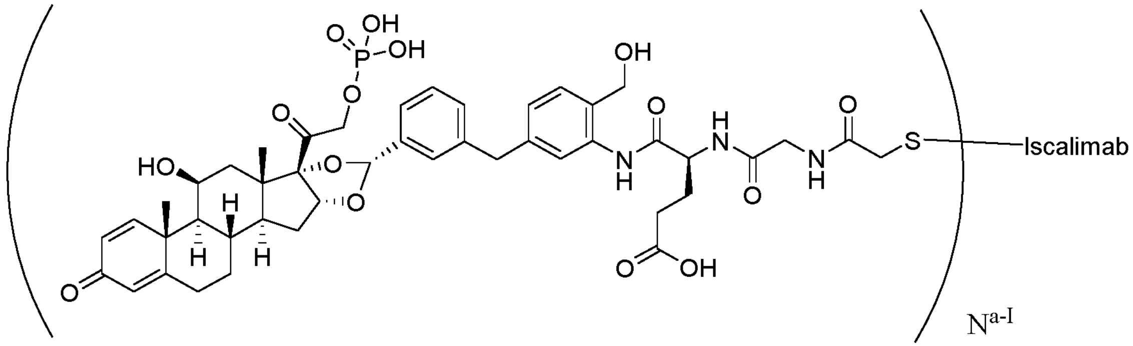 |

(continued)

| No. | Structure |
| --- | --- |
| V-241 | 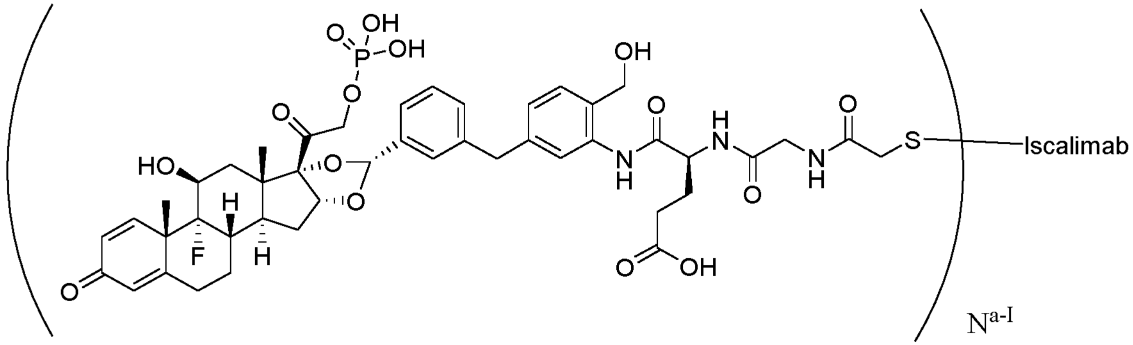 |
| V-242 | 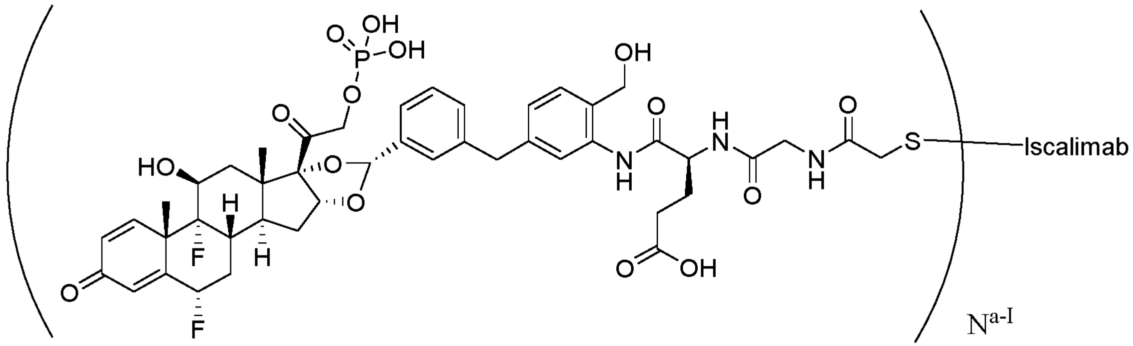 |

(continued)

| No. | Structure |
|---|---|
| V-243 | 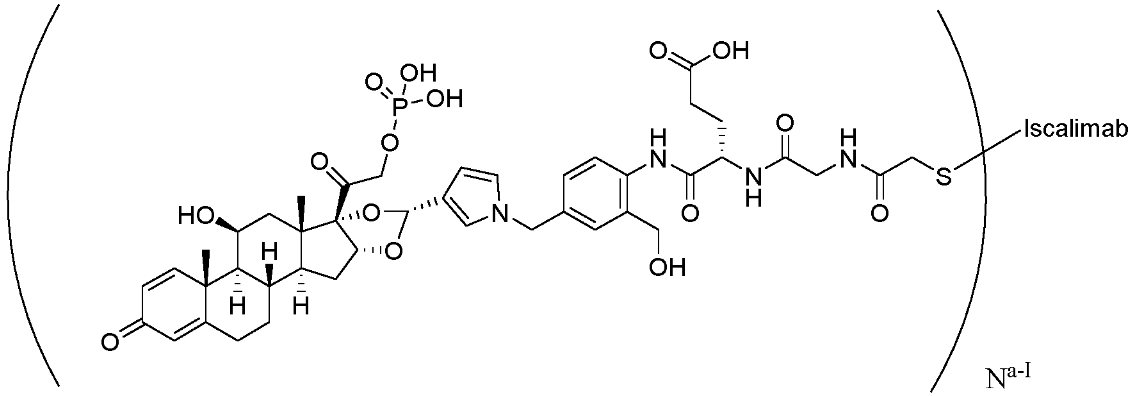 |
| V-244 | 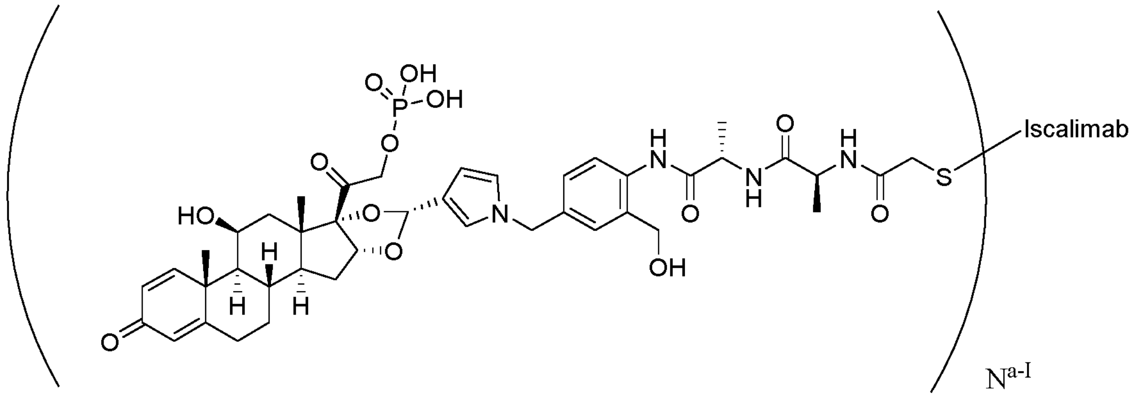 |

(continued)

| No. | Structure |
|---|---|
| V-247 | 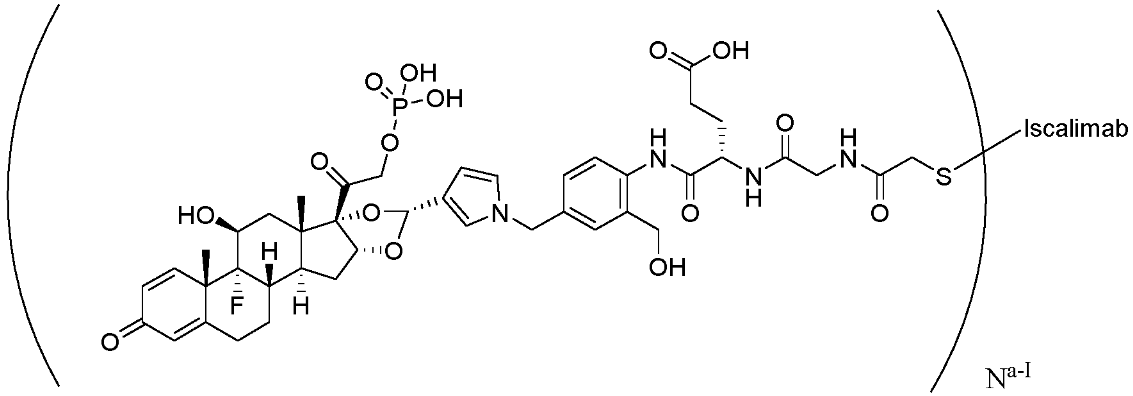 |
| V-248 | 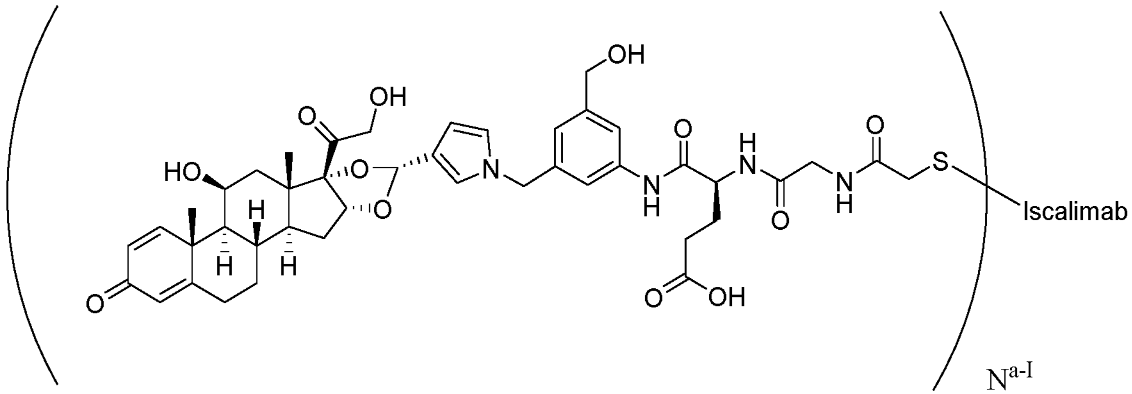 |

(continued)

| No. | Structure |
|---|---|
| V-249 | 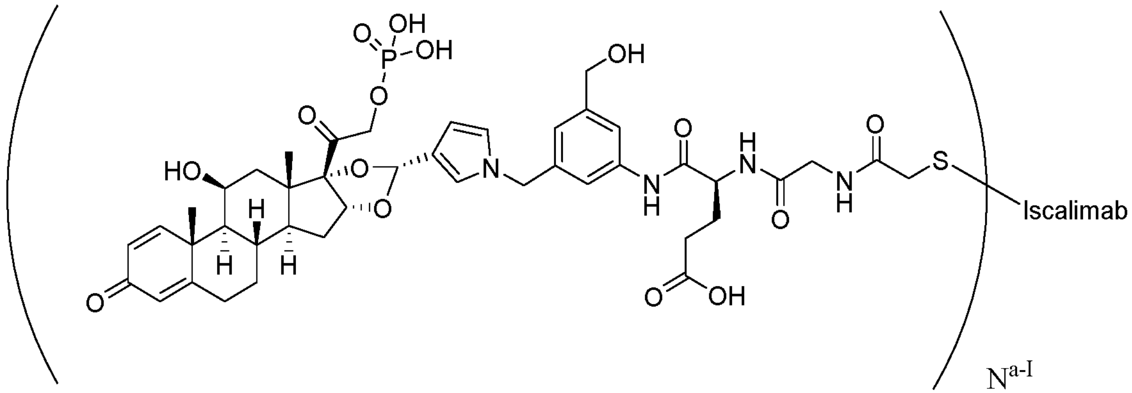 |
| V-250 | 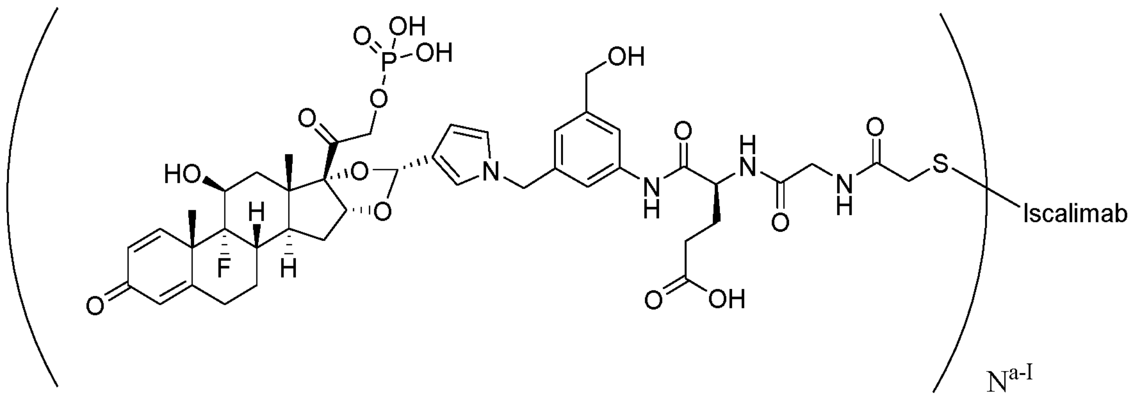 |

(continued)

| No. | Structure |
|---|---|
| V-251 | 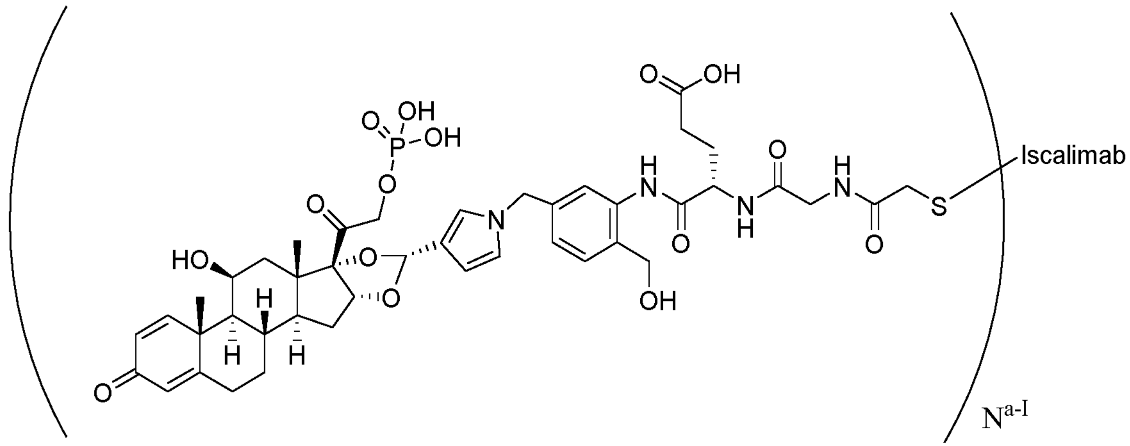 |
| V-252 | 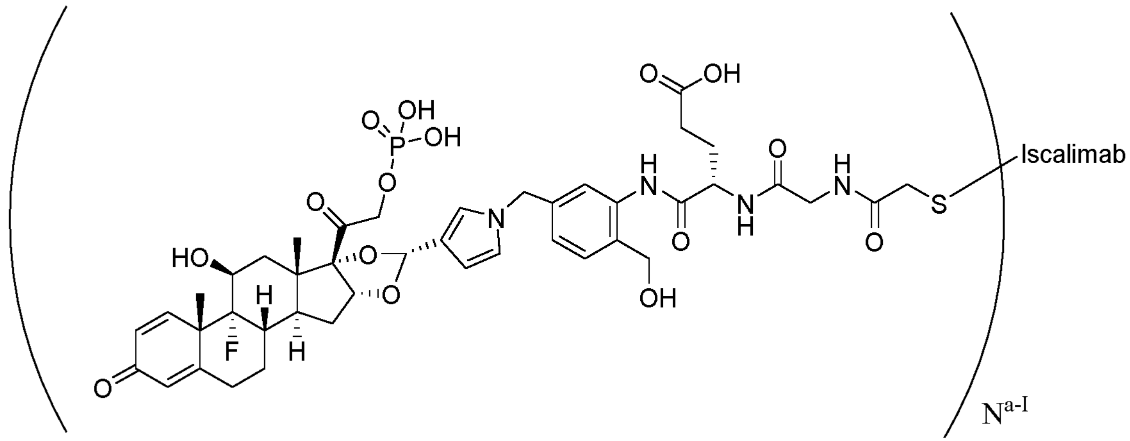 |

(continued)

| No. | Structure |
|---|---|
| V-258 | 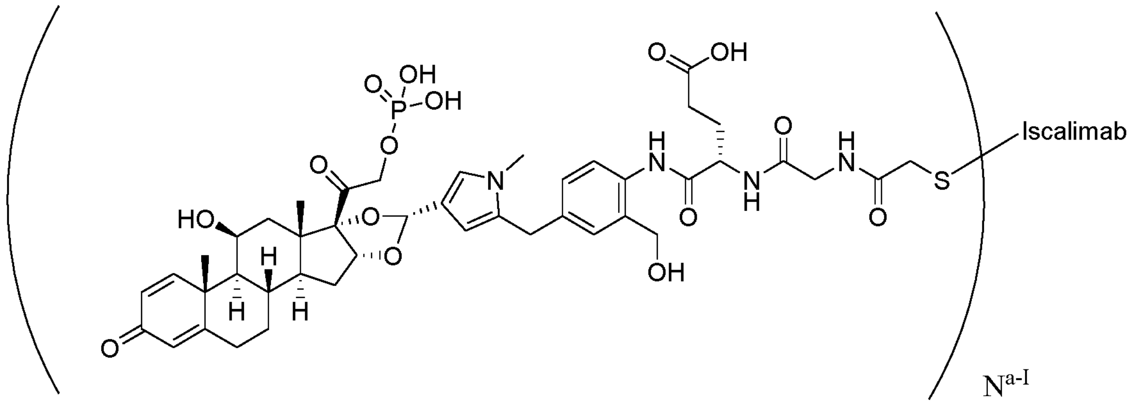 |
| V-259 | 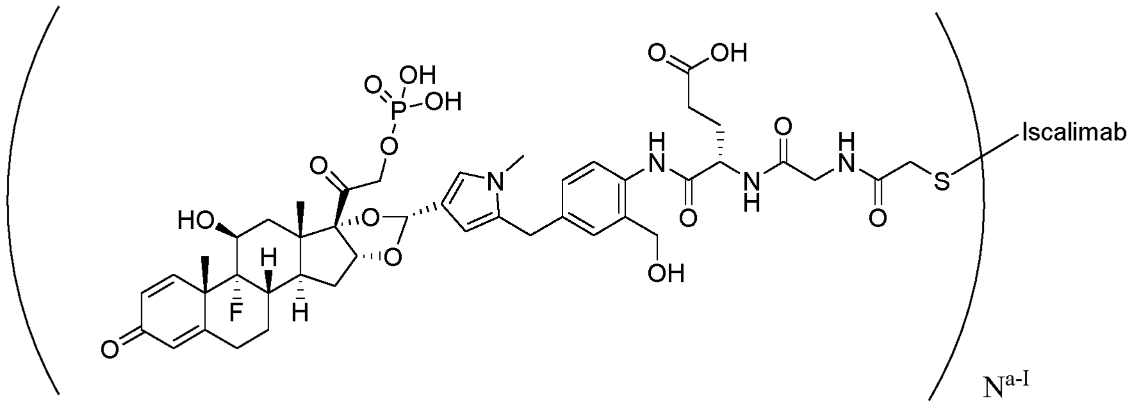 |

(continued)

| No. | Structure |
|---|---|
| V-260 | 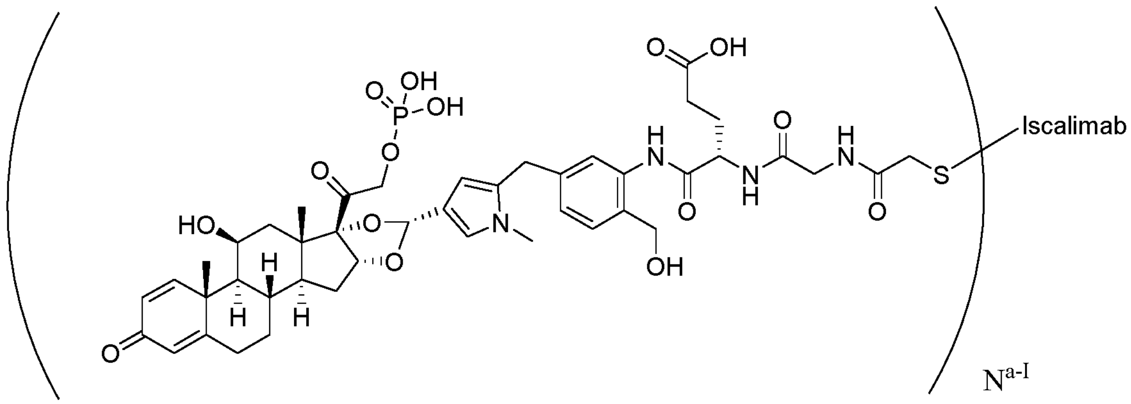 |
| V-261 | 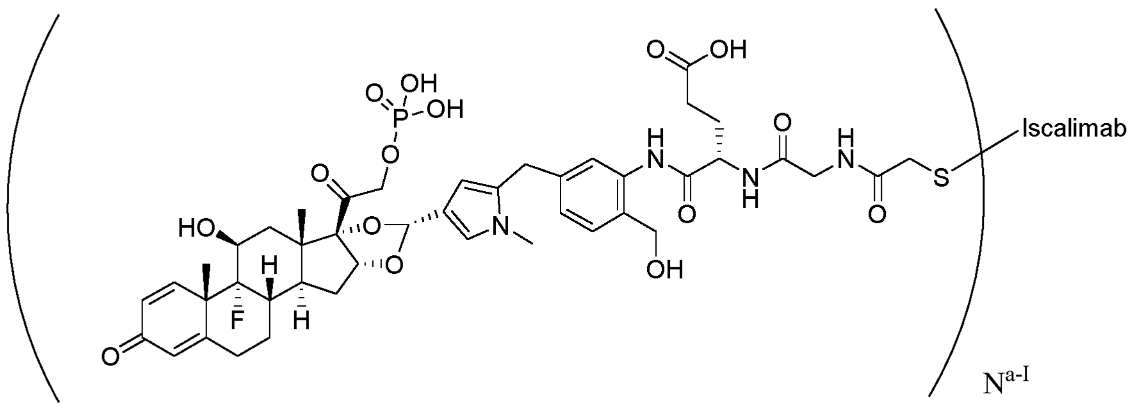 |

(continued)

| No. | Structure |
|---|---|
| V-262 | 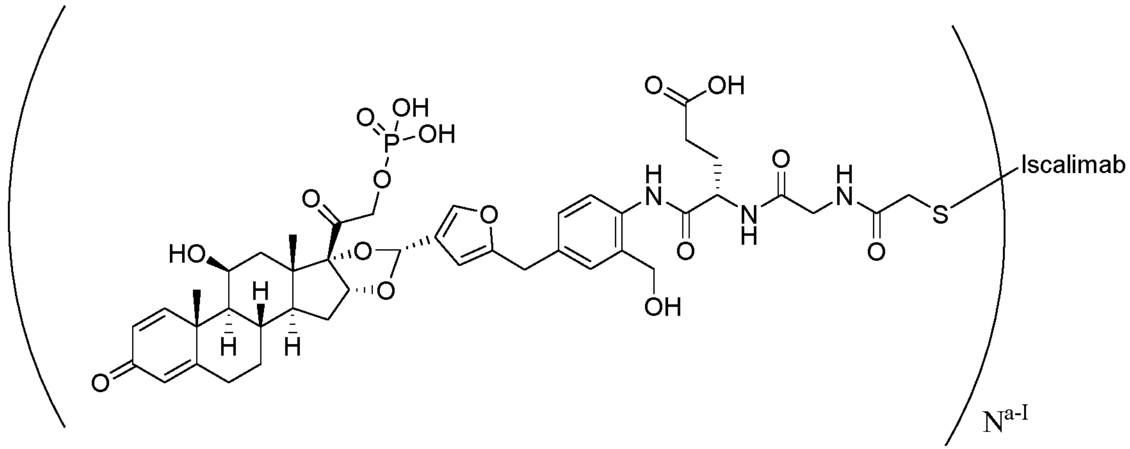 |
| V-263 | 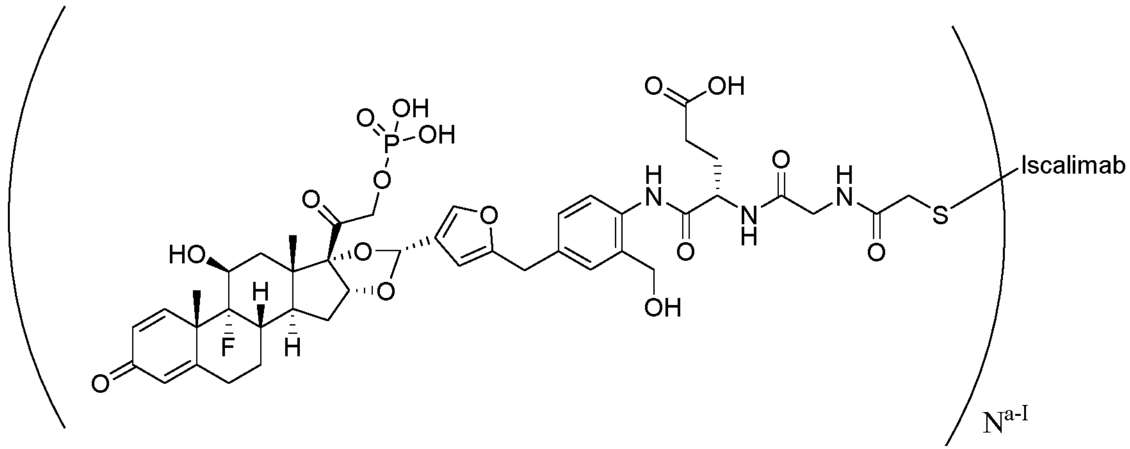 |

(continued)

| No. | Structure |
|---|---|
| V-264 | 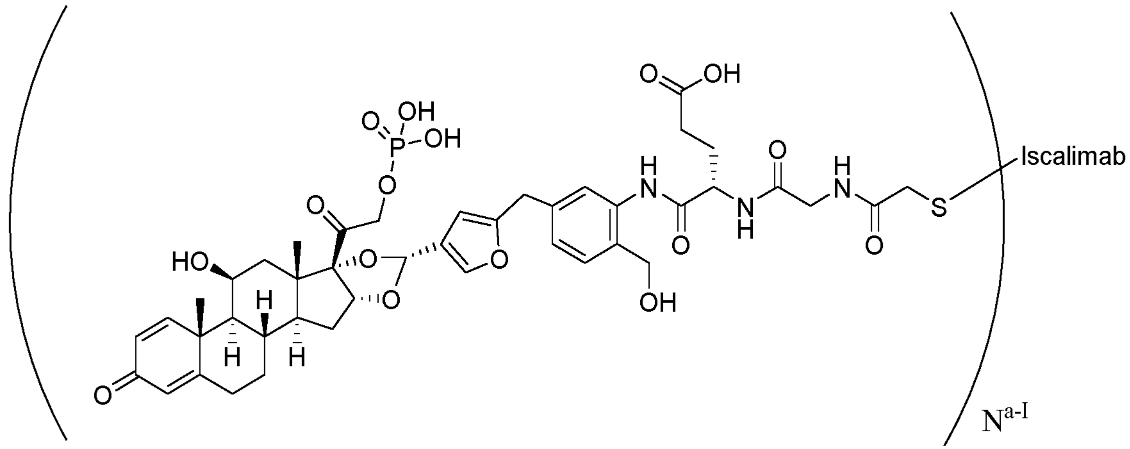 |
| V-265 | 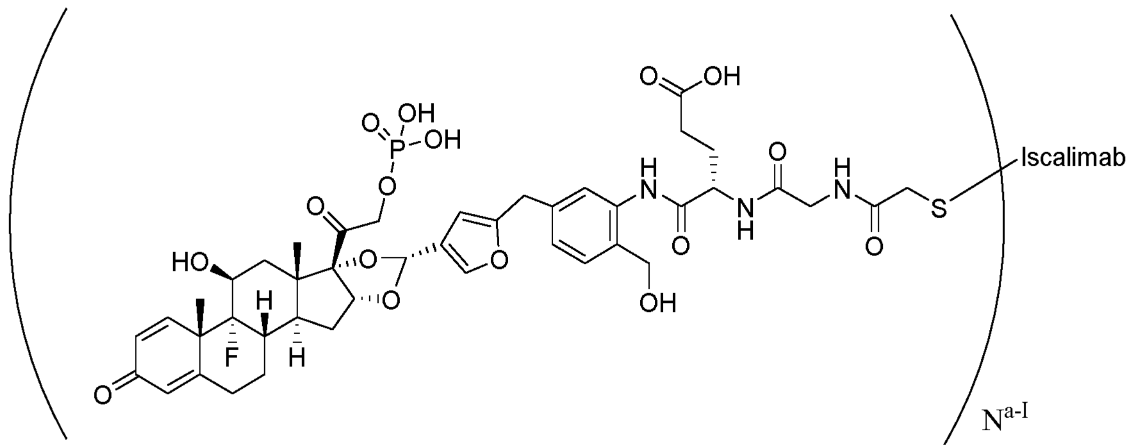 |

(continued)

| No. | Structure |
|---|---|
| V-266 | 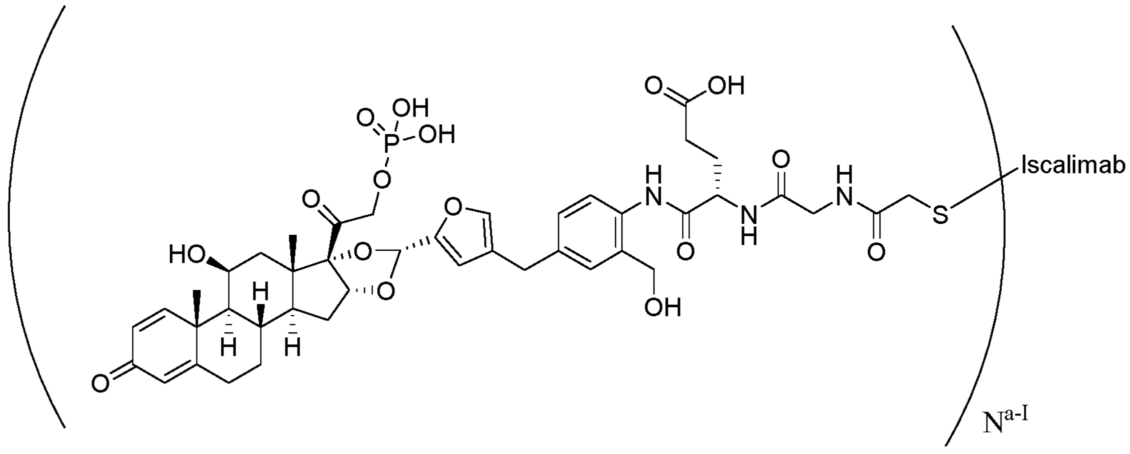 |
| V-267 | 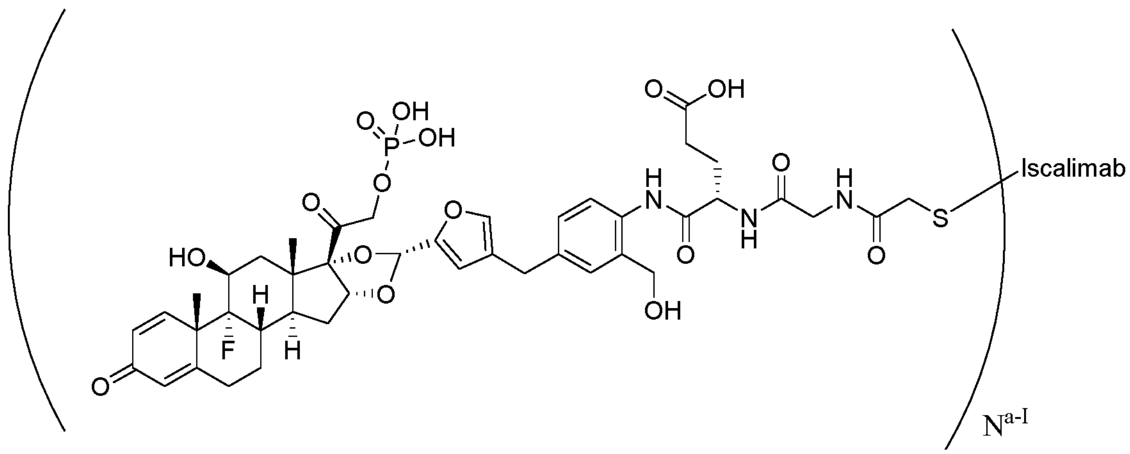 |

(continued)

| No. | Structure |
|---|---|
| V-268 | 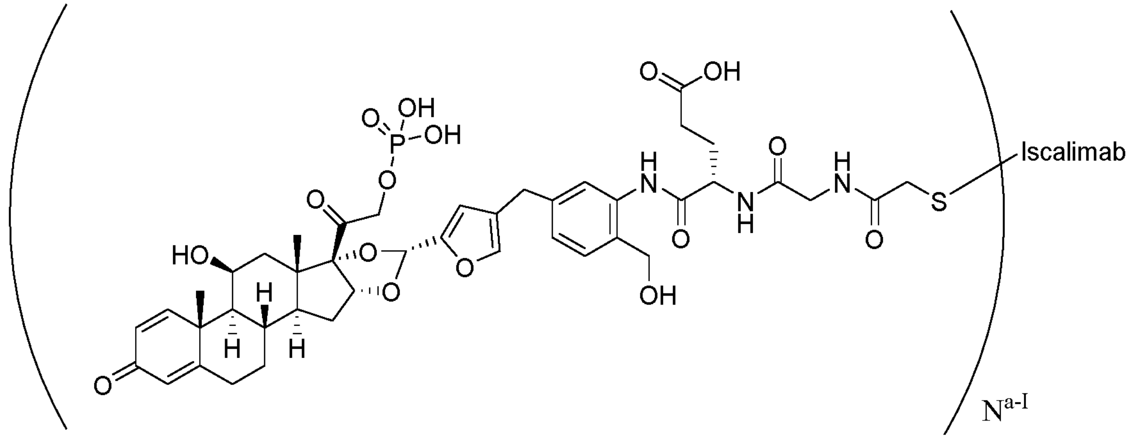 |
| V-269 | 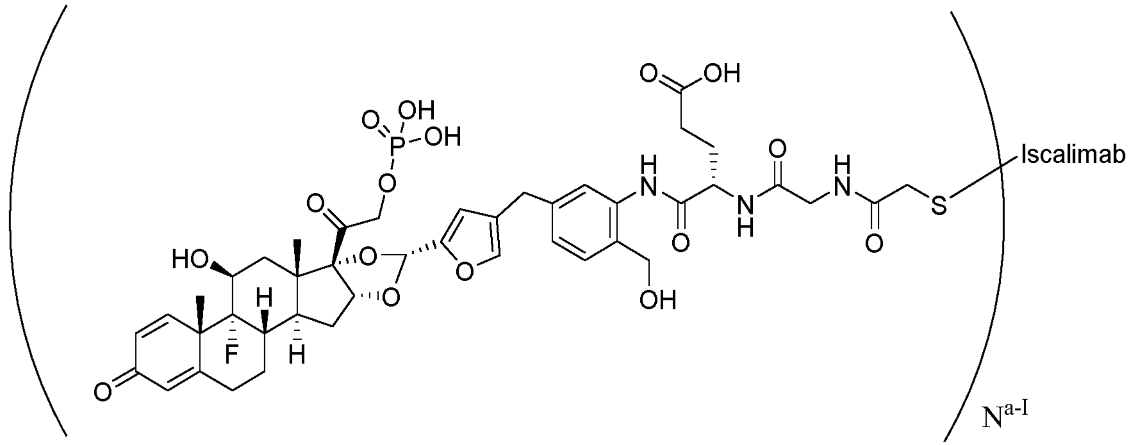 |

(continued)

| No. | Structure |
|---|---|
| V-270 | 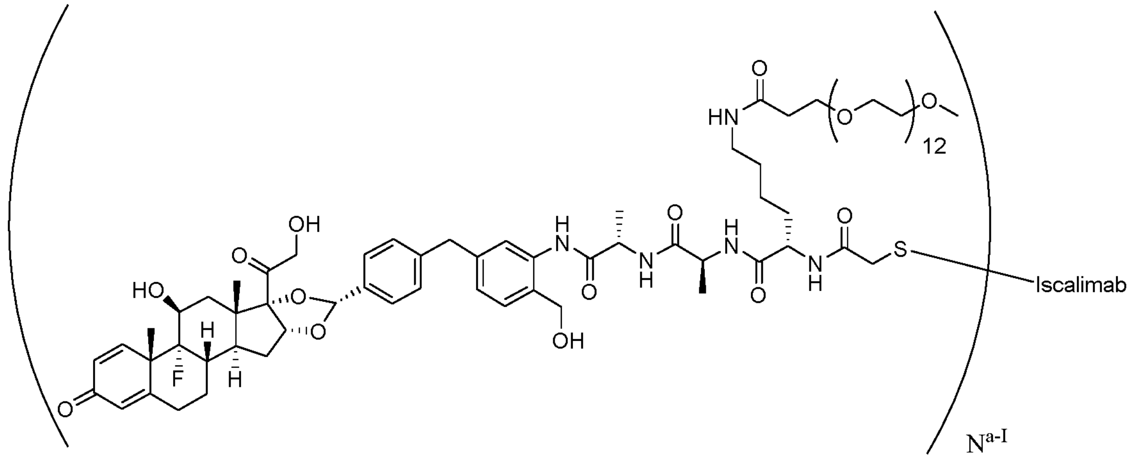 |
| V-271 | 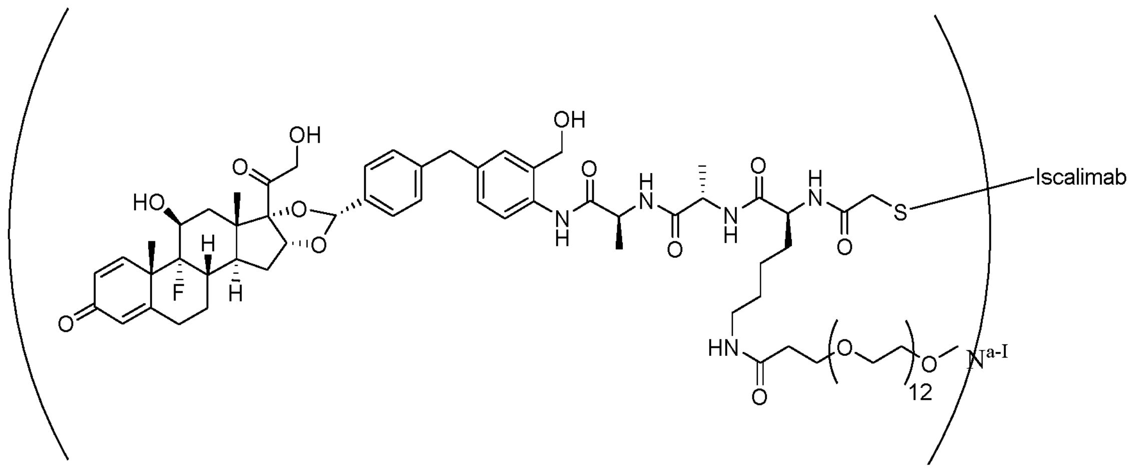 |

(continued)

| No. | Structure |
|---|---|
| V-272 | 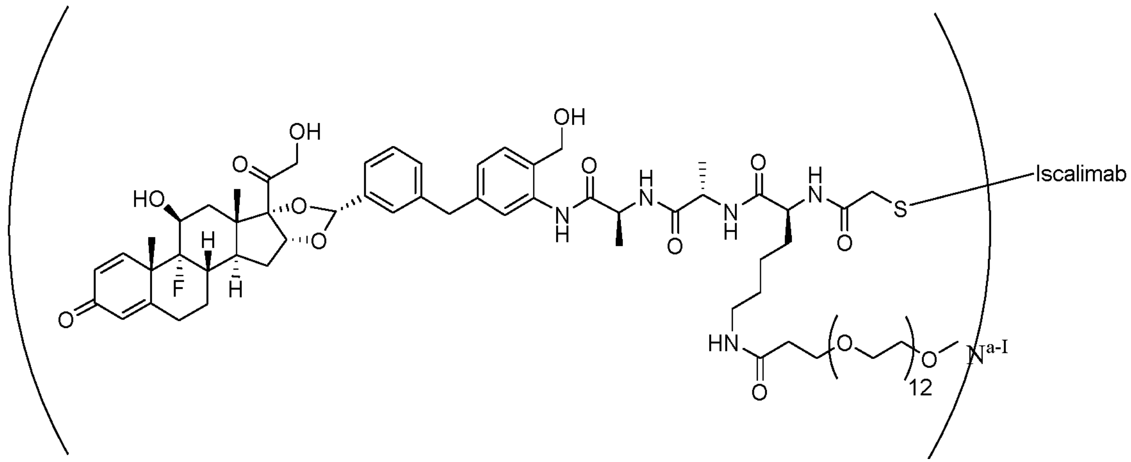 |
| V-273 | 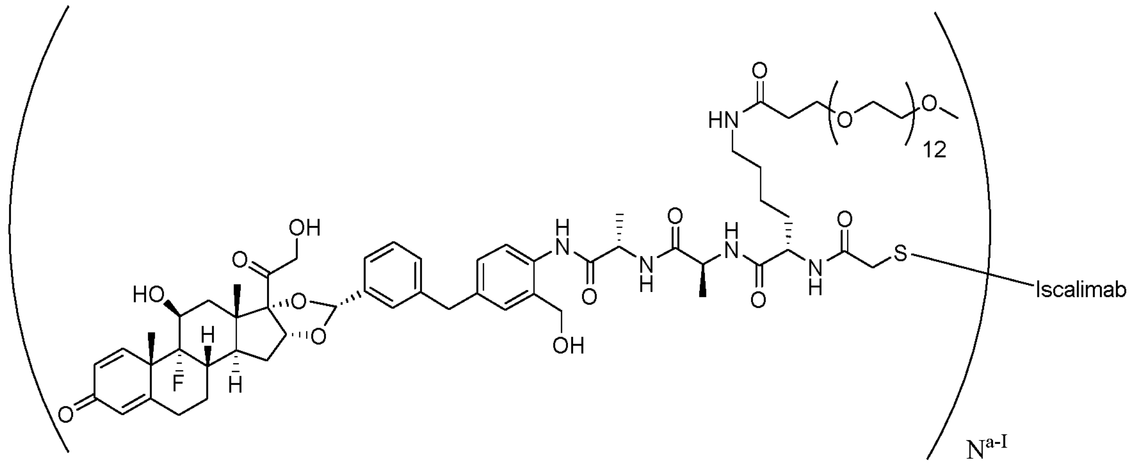 |

(continued)

| No. | Structure |
|---|---|
| V-274 | 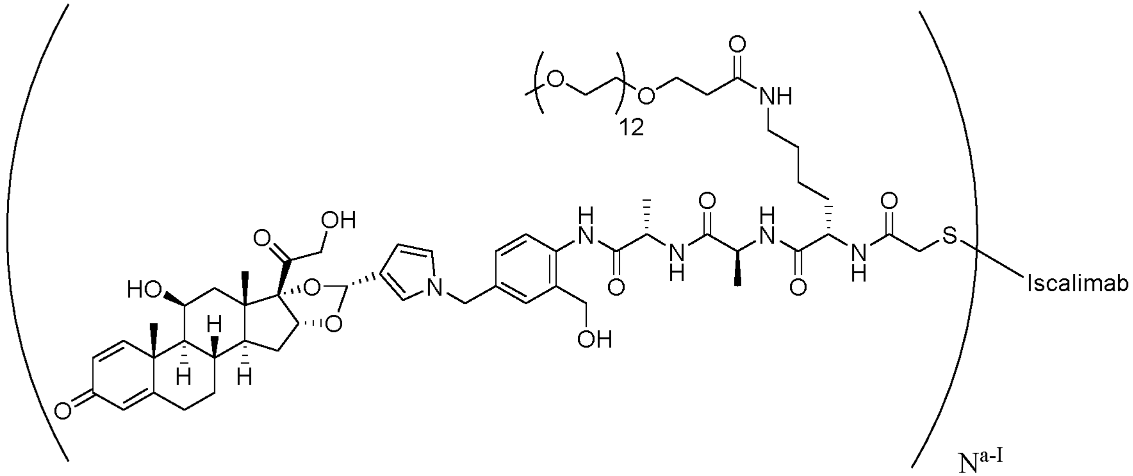 $N^{a-I}$ |
| V-275 | 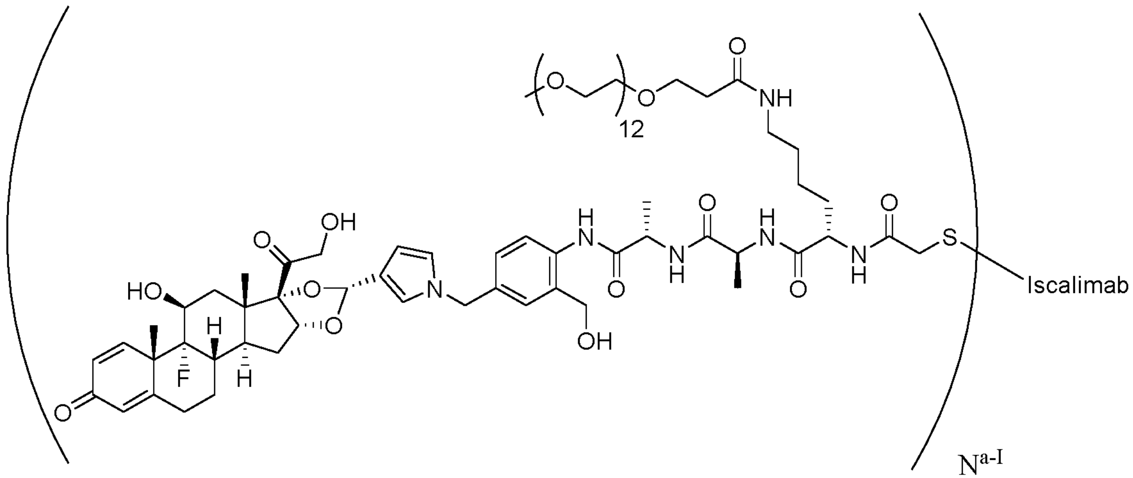 $N^{a-I}$ |

(continued)

| No. | Structure |
|---|---|
| V-276 | 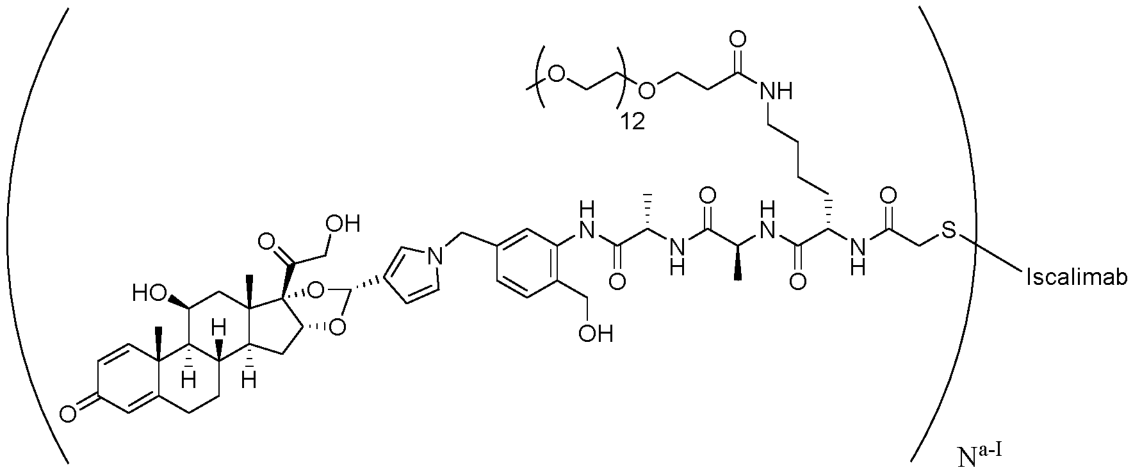 |
| V-277 | 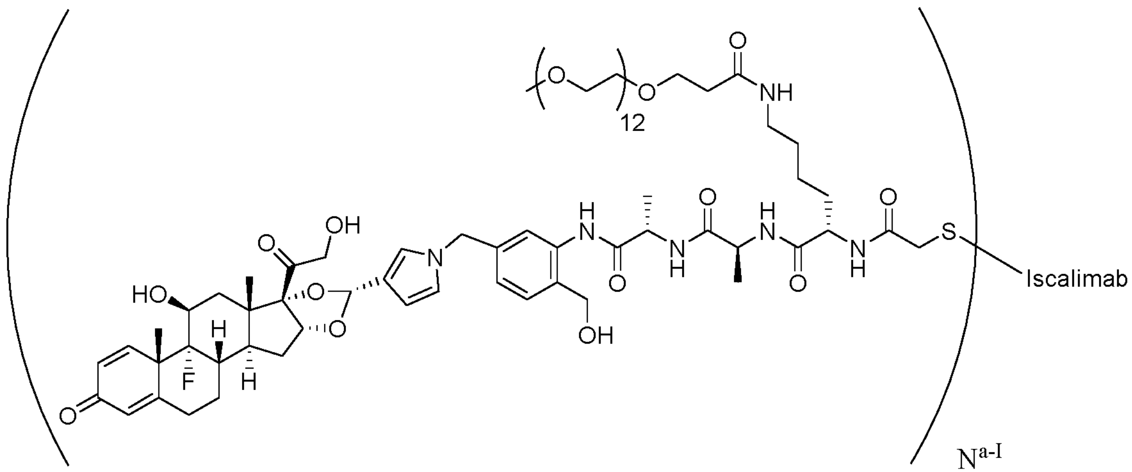 |

(continued)

| No. | Structure |
|---|---|
| V-278 | 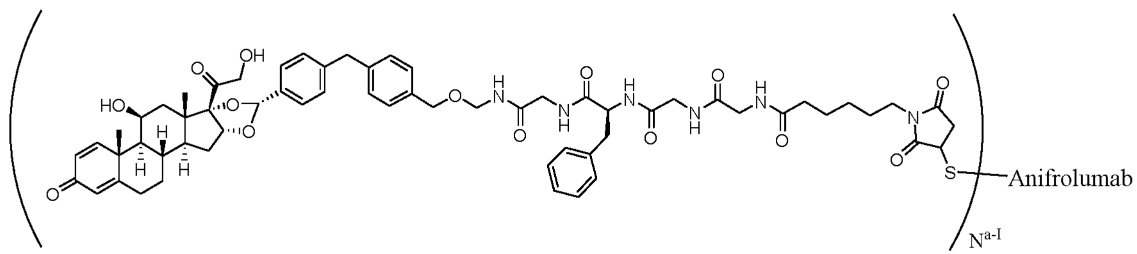 |
| V-279 | 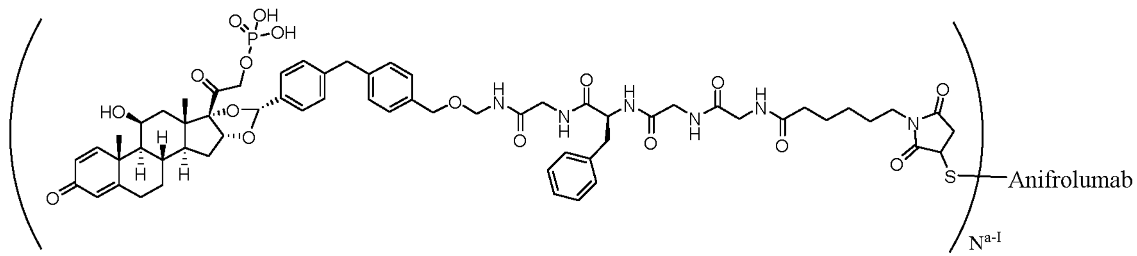 |
| V-283 | 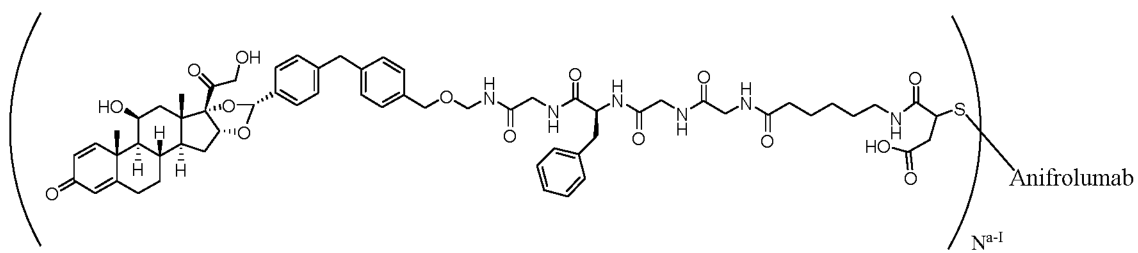 |
| V-284 | 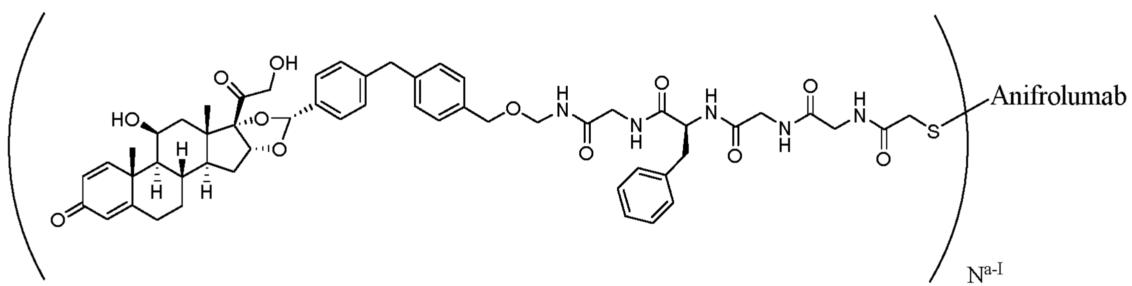 |

(continued)

| No. | Structure |
|---|---|
| V-285 | 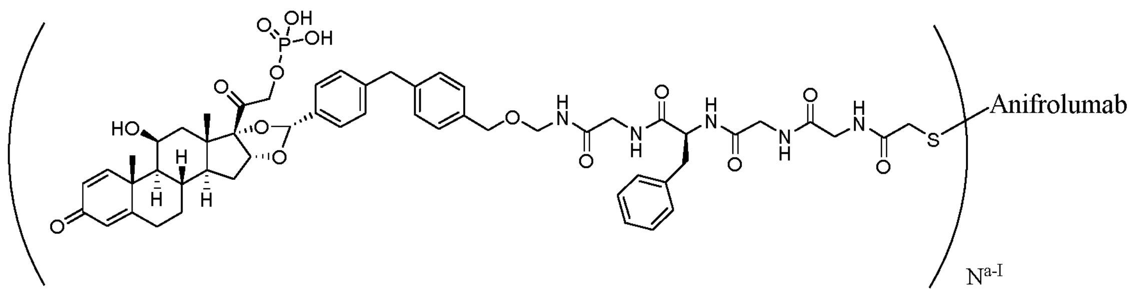 |
| V-289 | 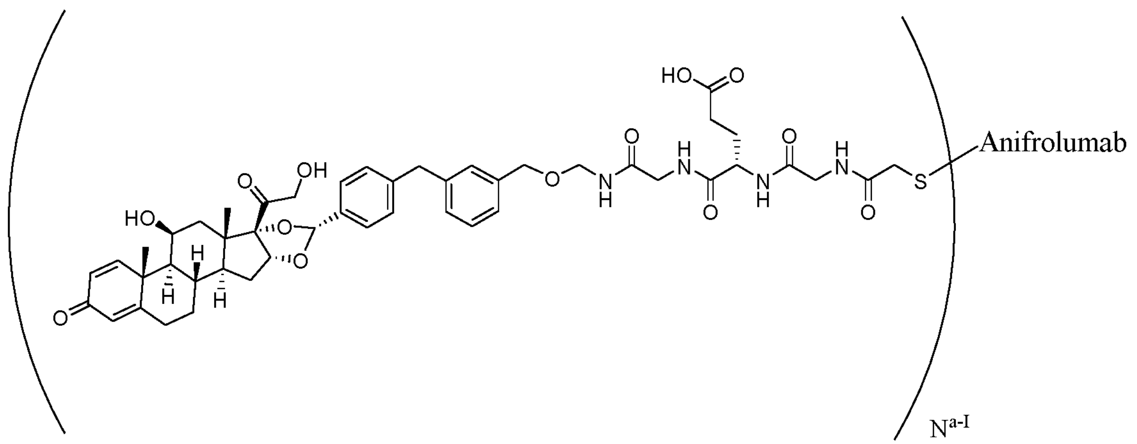 |
| V-290 | 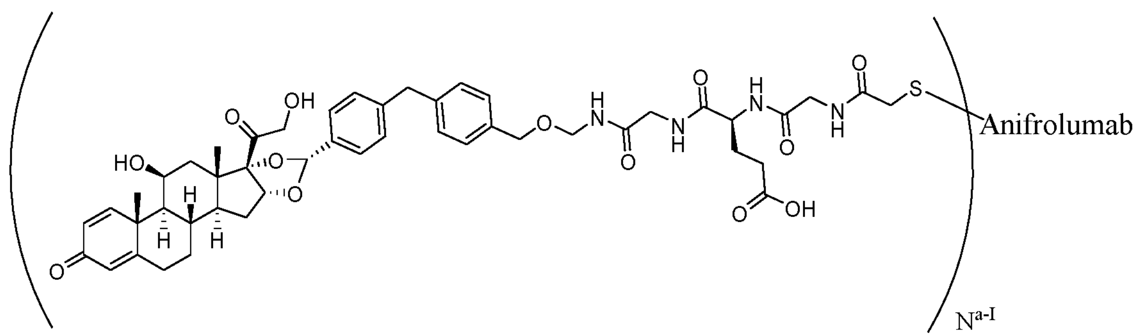 |

(continued)

| No. | Structure |
|---|---|
| V-291 | 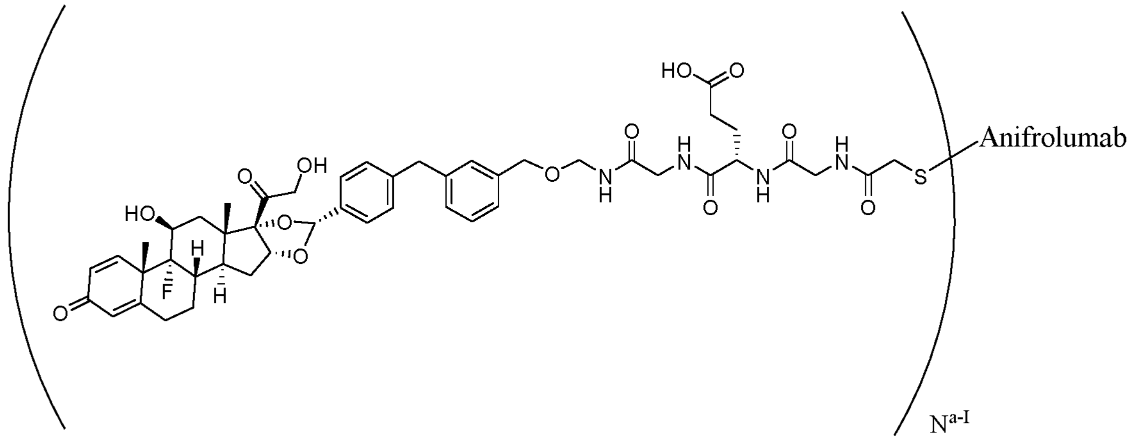 |
| V-292 | 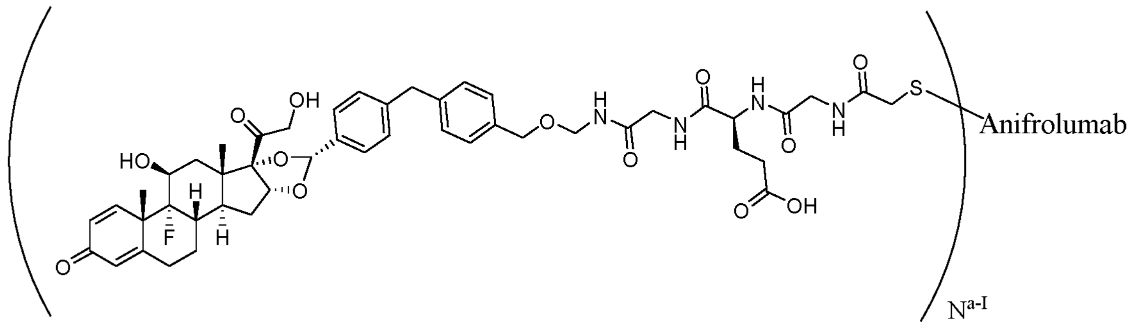 |

(continued)

| No. | Structure |
|---|---|
| V-293 | 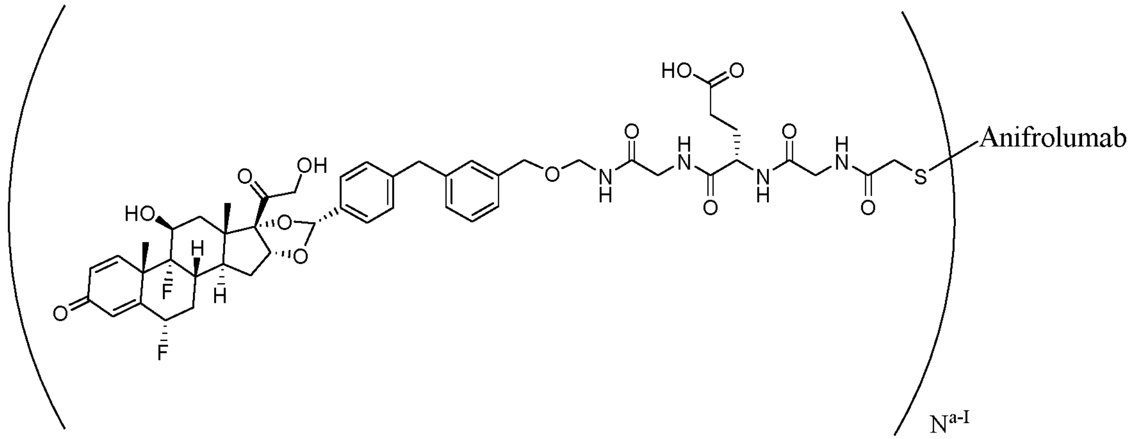 |
| V-294 | 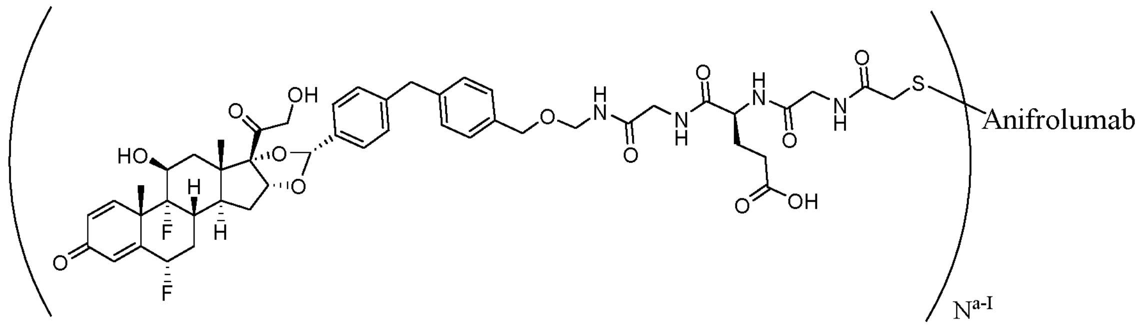 |

(continued)

| No. | Structure |
|---|---|
| V-295 | 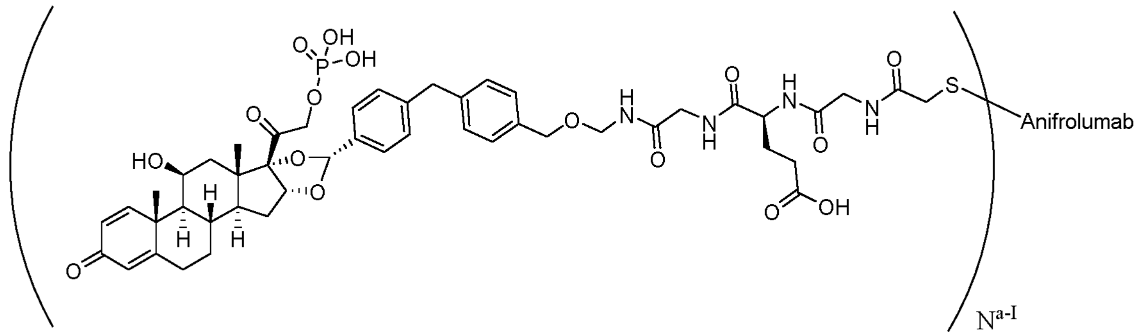 |
| V-299 | 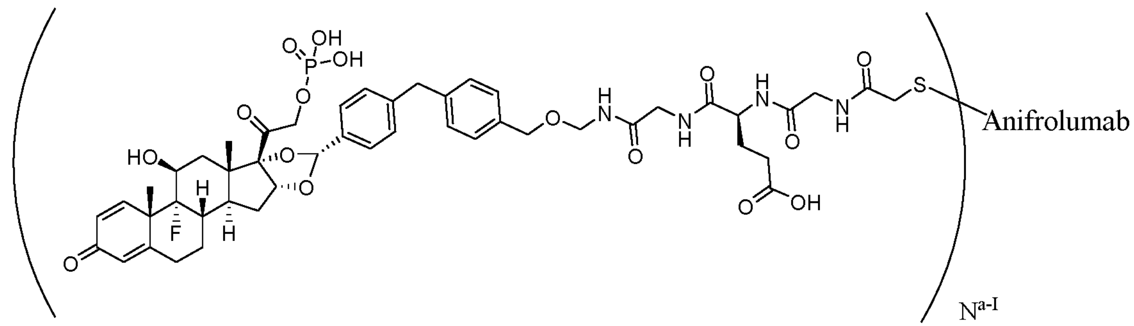 |
| V-302 | 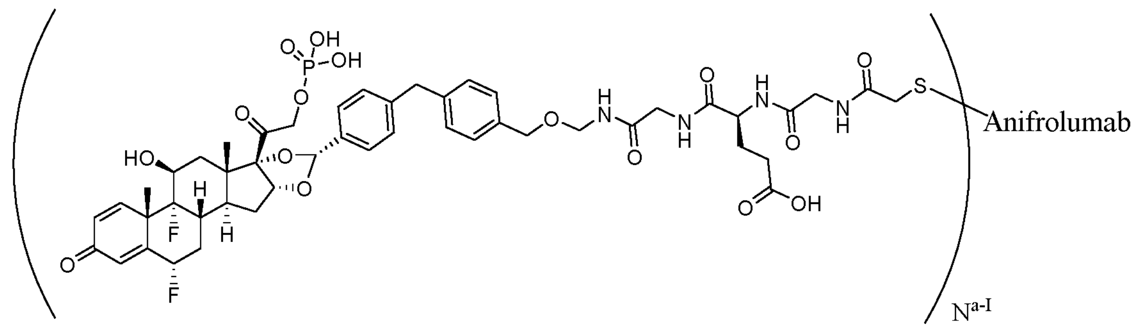 |

(continued)

| No. | Structure |
|---|---|
| V-306 | 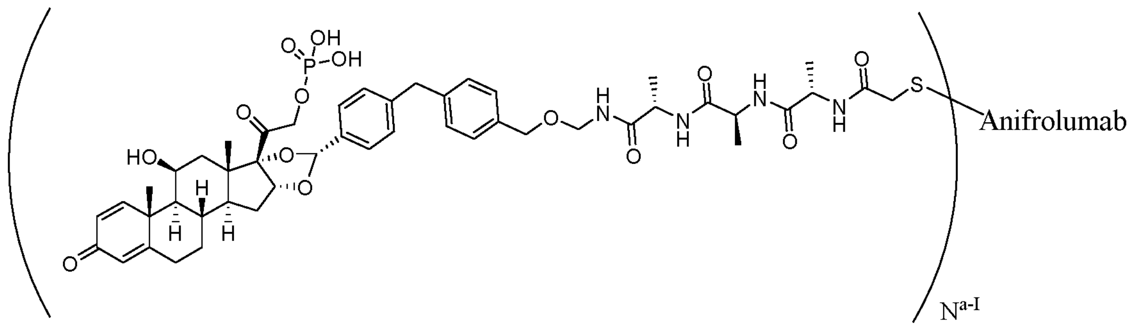 |
| V-307 | 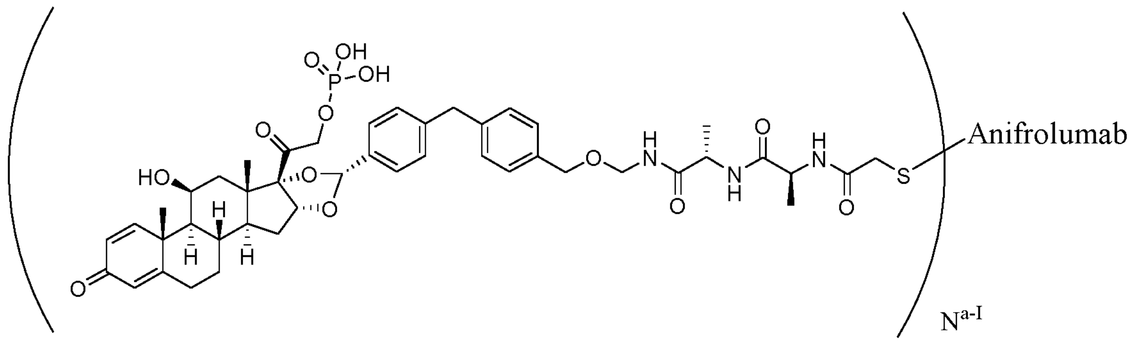 |
| V-311 | 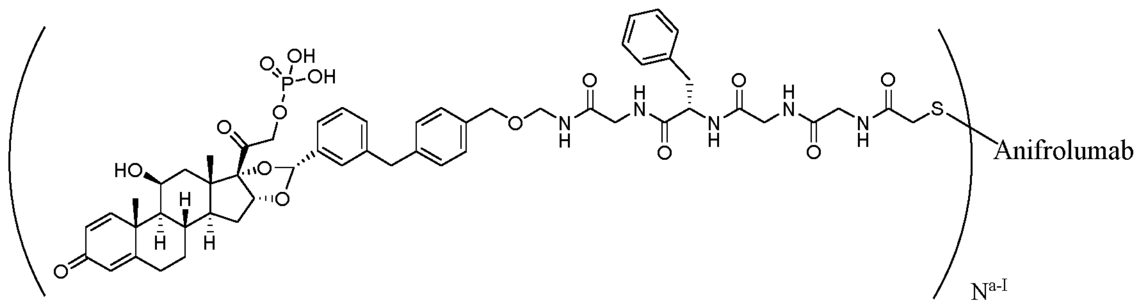 |

(continued)

| No. | Structure |
|---|---|
| V-312 | 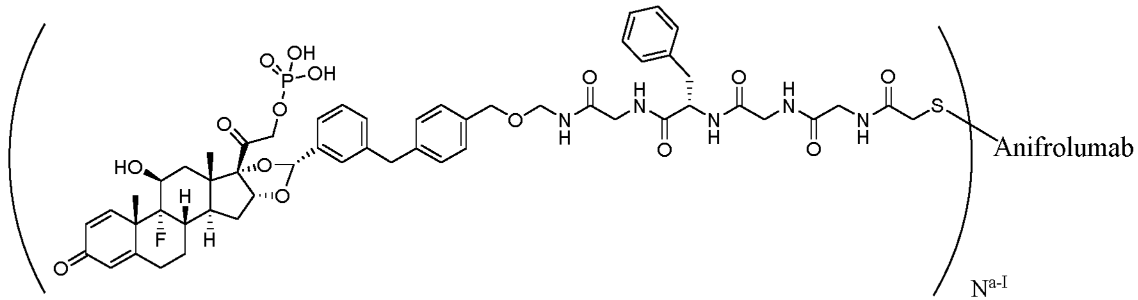 |
| V-316 | 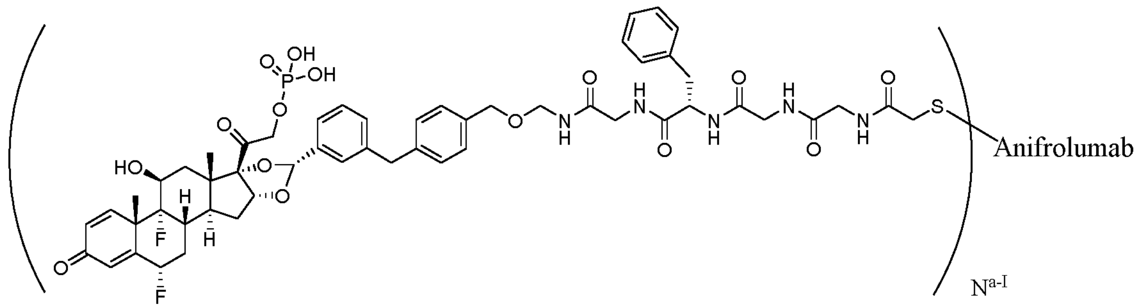 |
| V-320 | 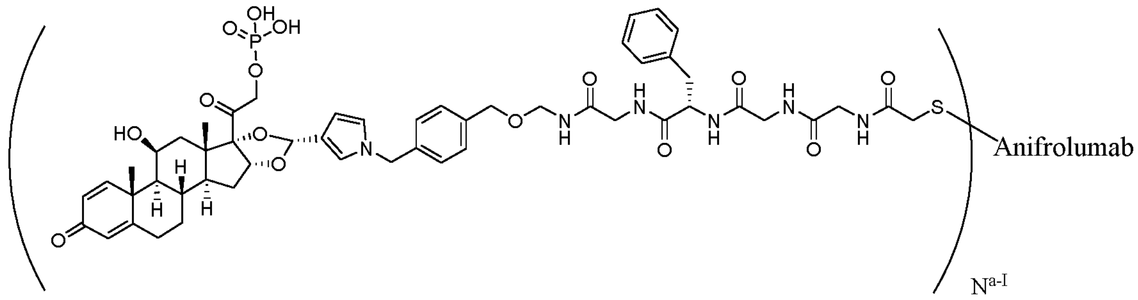 |

(continued)

| No. | Structure |
|---|---|
| V-321 | 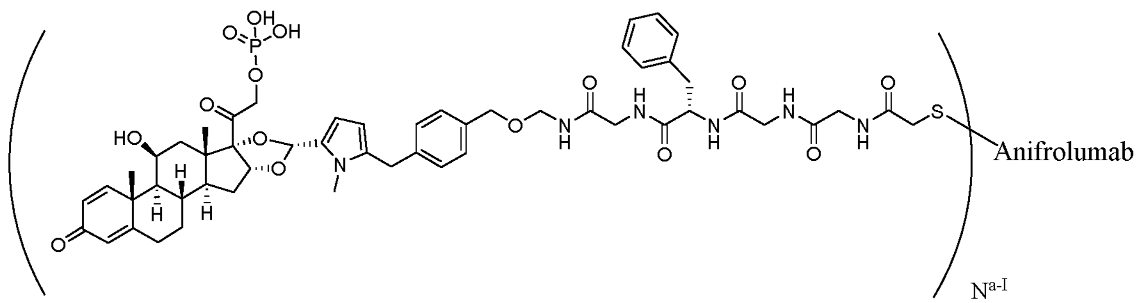 |
| V-322 | 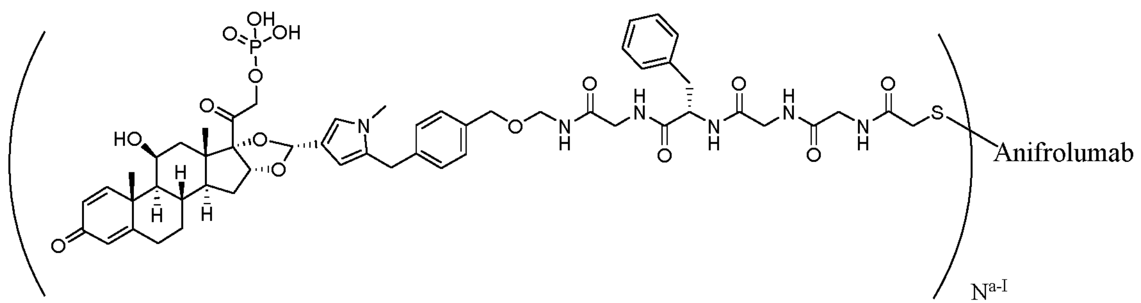 |
| V-323 | 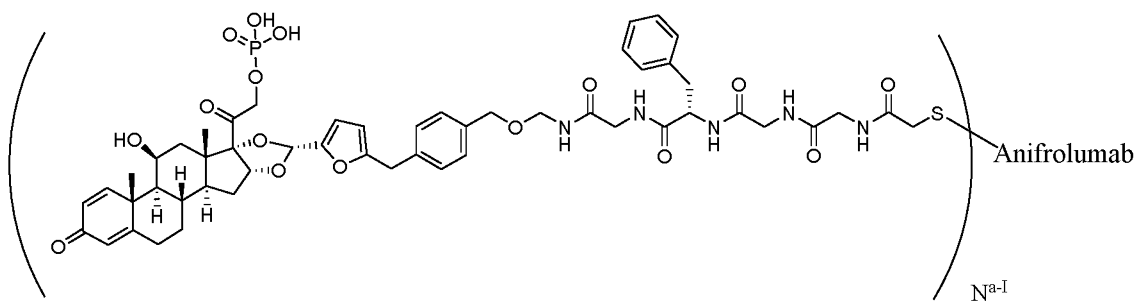 |

(continued)

| No. | Structure |
|---|---|
| V-324 | 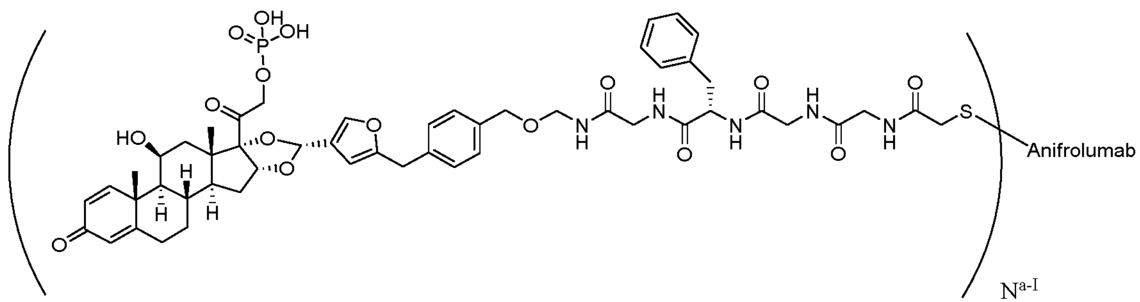 |
| V-325 | 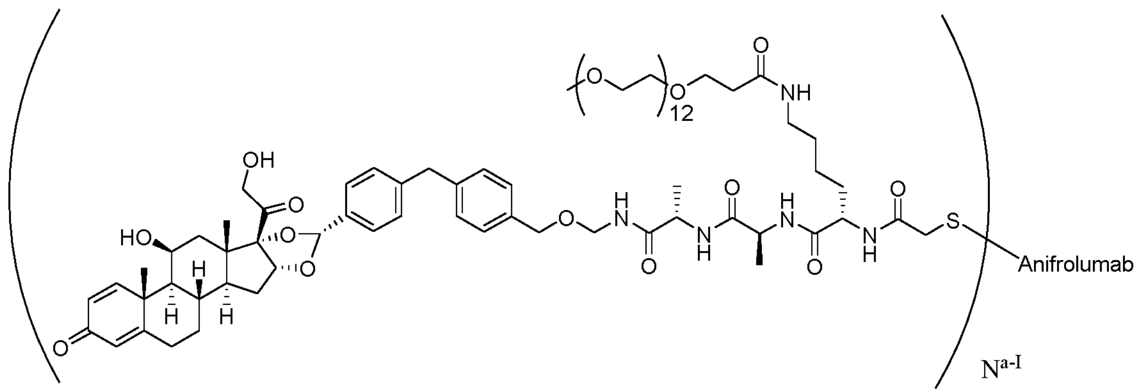 |

(continued)

| No. | Structure |
|---|---|
| V-326 | 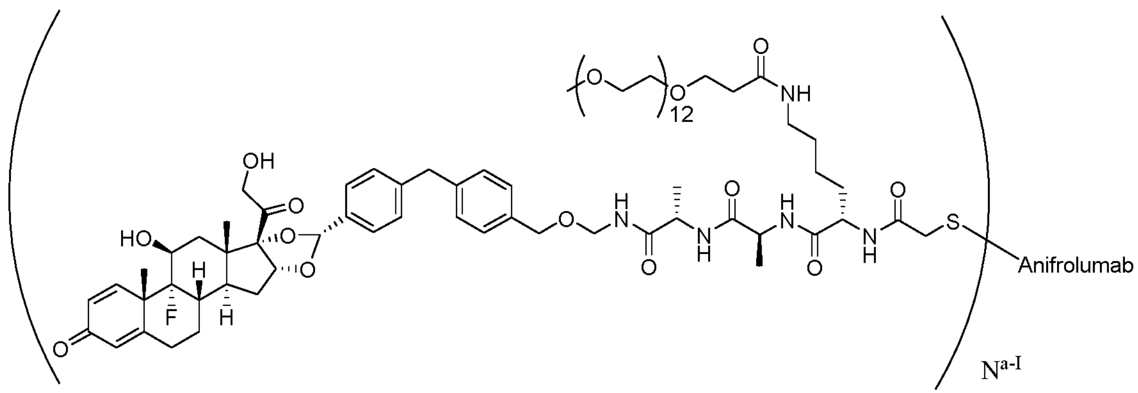 |
| V-327 | 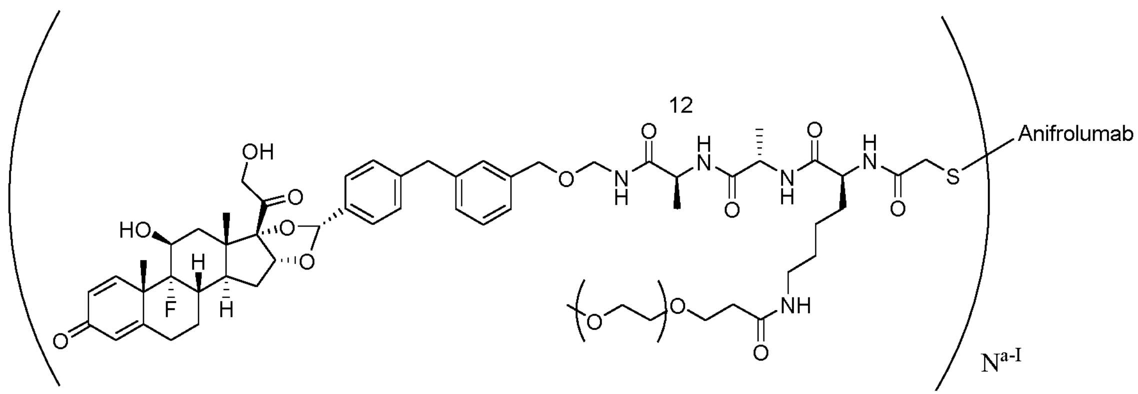 |

(continued)

| No. | Structure |
| --- | --- |
| V-328 | 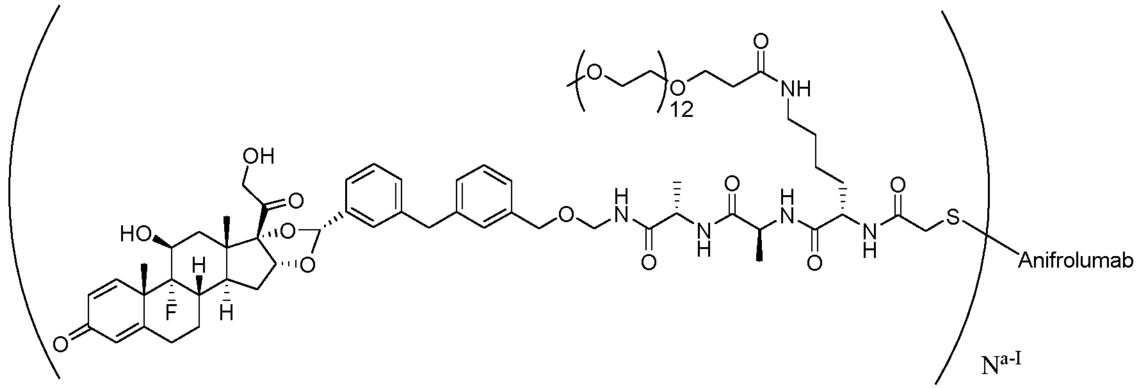 |
| V-329 | 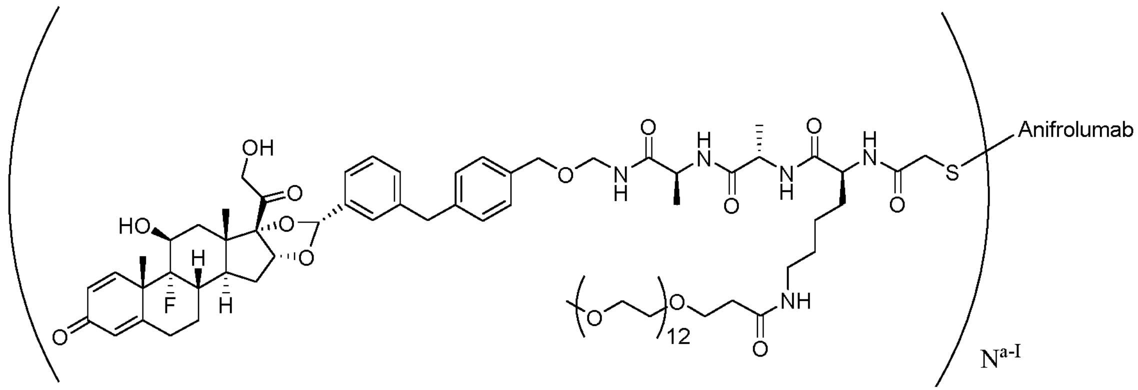 |

(continued)

| No. | Structure |
|---|---|
| V-330 | 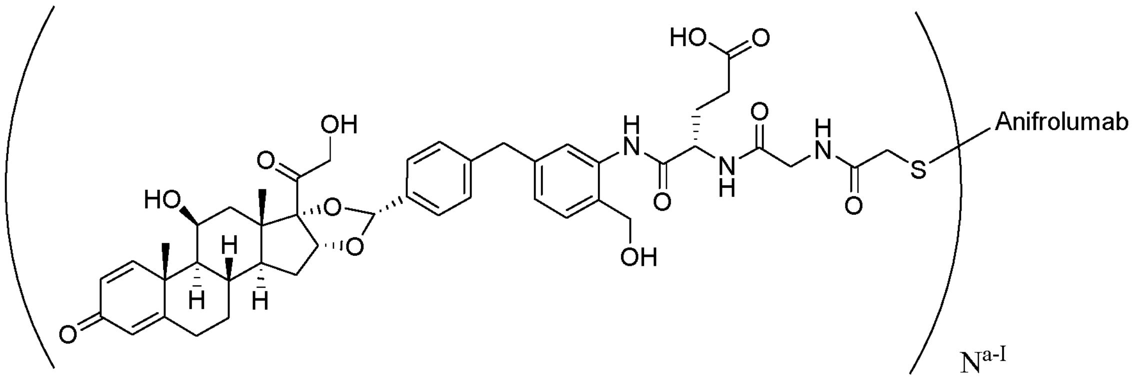 |
| V-331 | 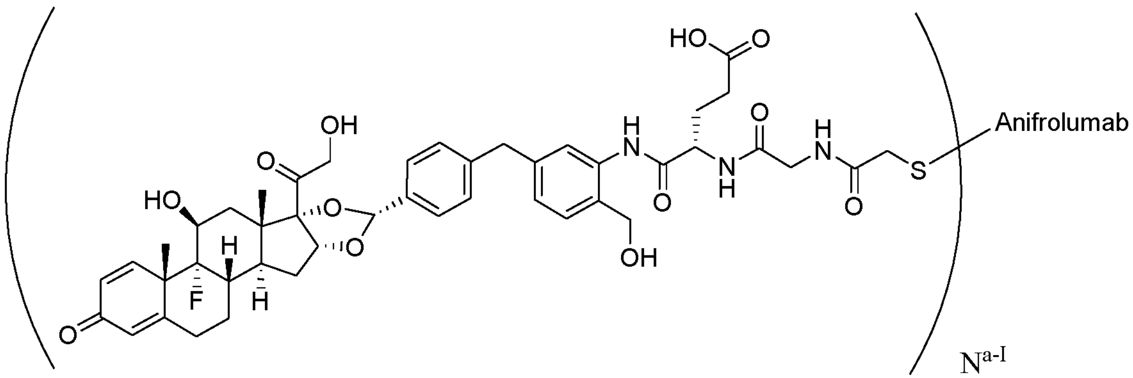 |

(continued)

| No. | Structure |
|---|---|
| V-332 | 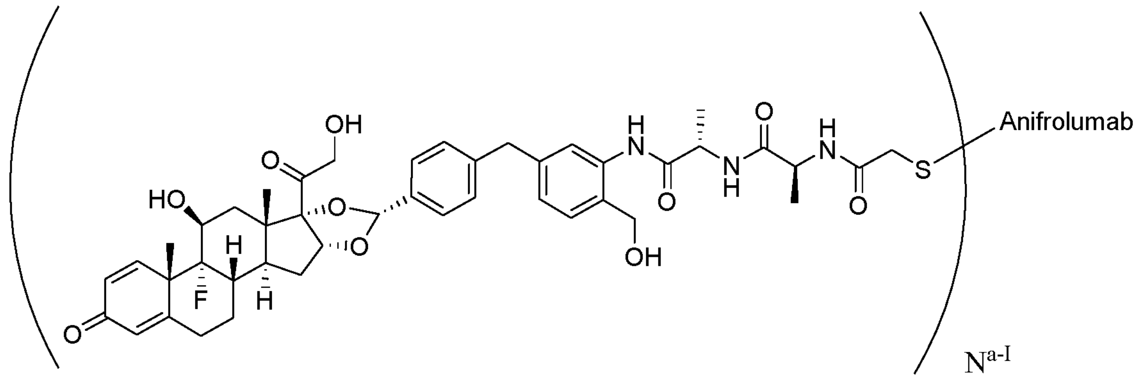 |
| V-333 | 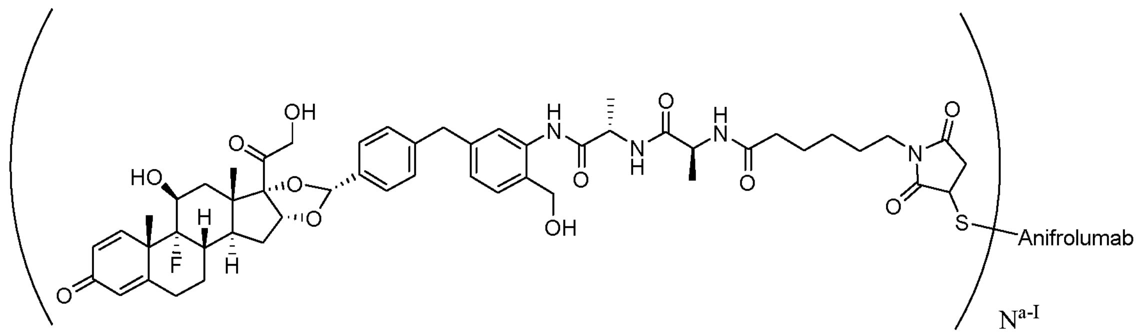 |

(continued)

| No. | Structure |
|---|---|
| V-334 | 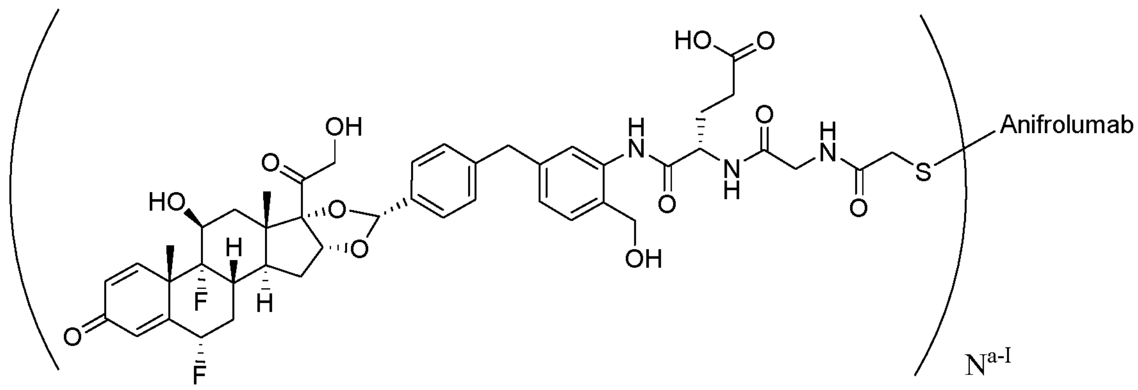 |
| V-335 | 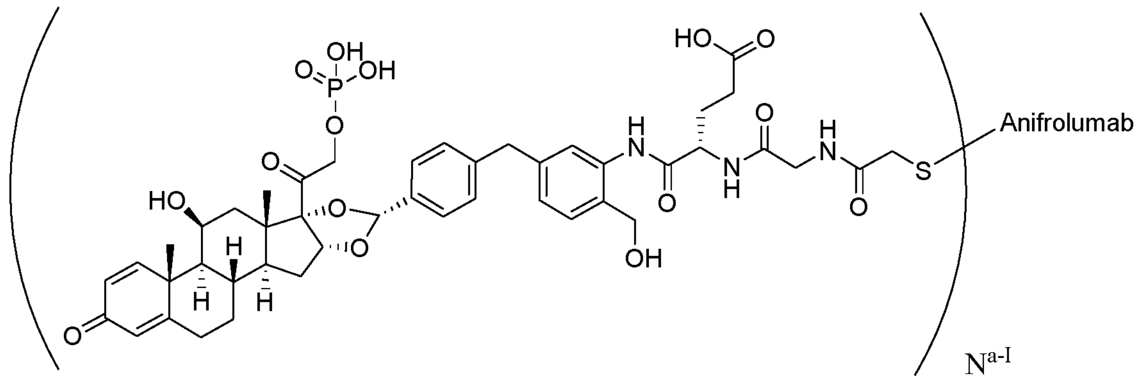 |

(continued)

| No. | Structure |
| --- | --- |
| V-336 | 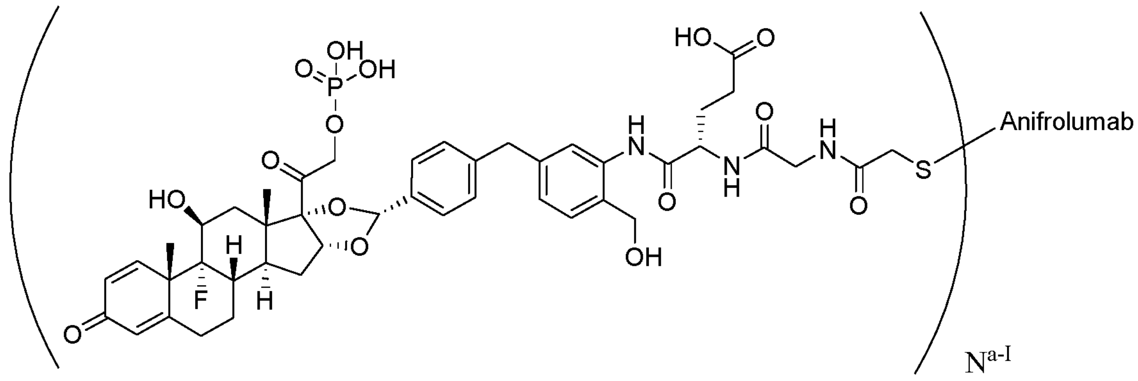 |
| V-337 | 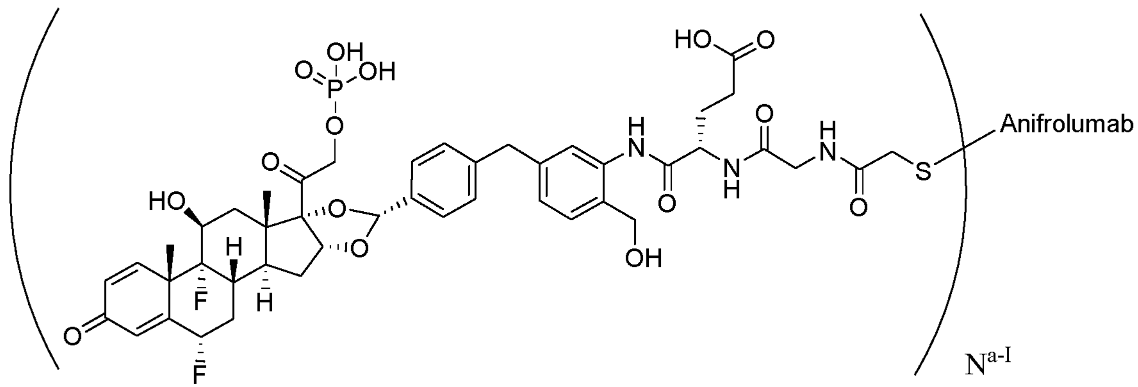 |

(continued)

| No. | Structure |
|---|---|
| V-347 | 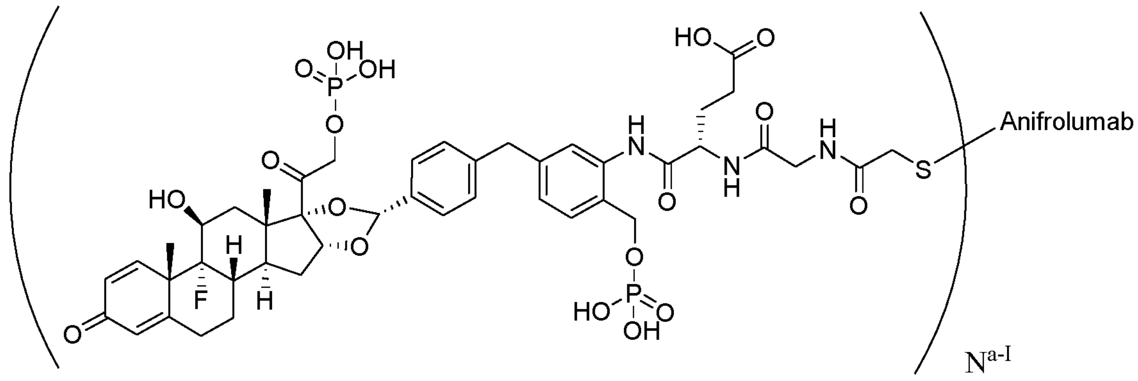 |
| V-351 | 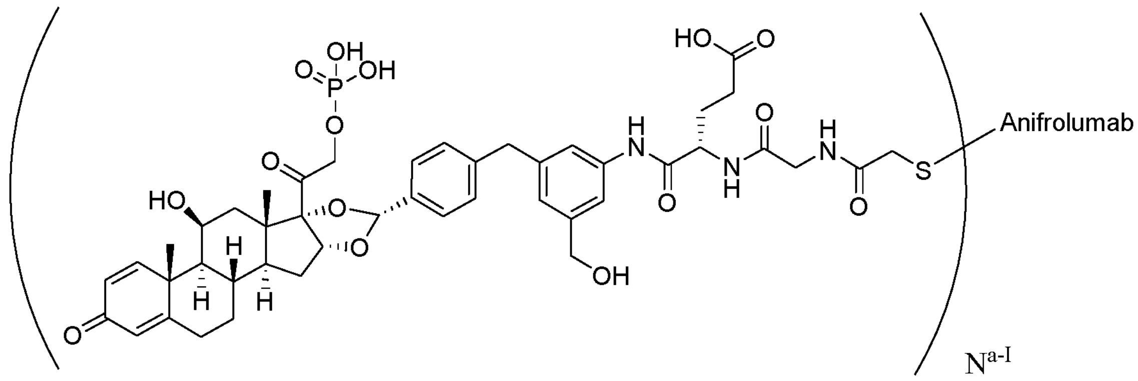 |

(continued)

| No. | Structure |
|---|---|
| V-354 | 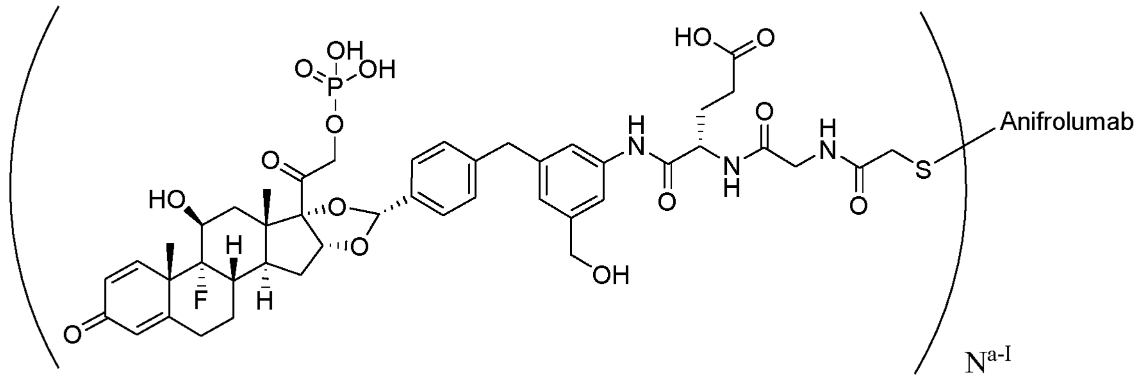 |
| V-357 | 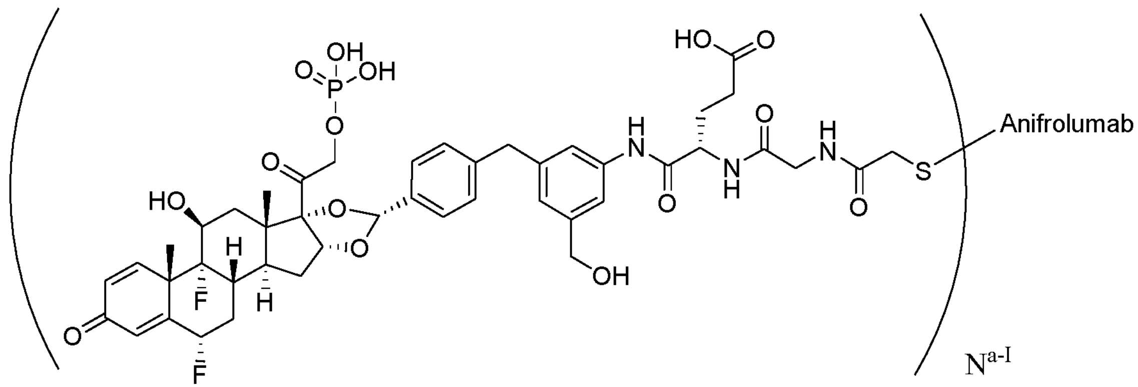 |

(continued)

| No. | Structure |
|---|---|
| V-360 | 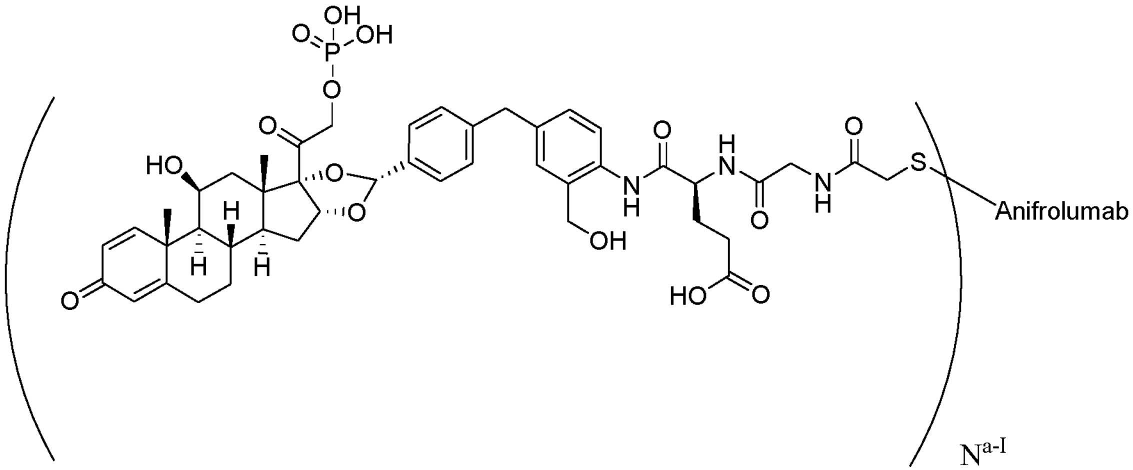 |
| V-363 | 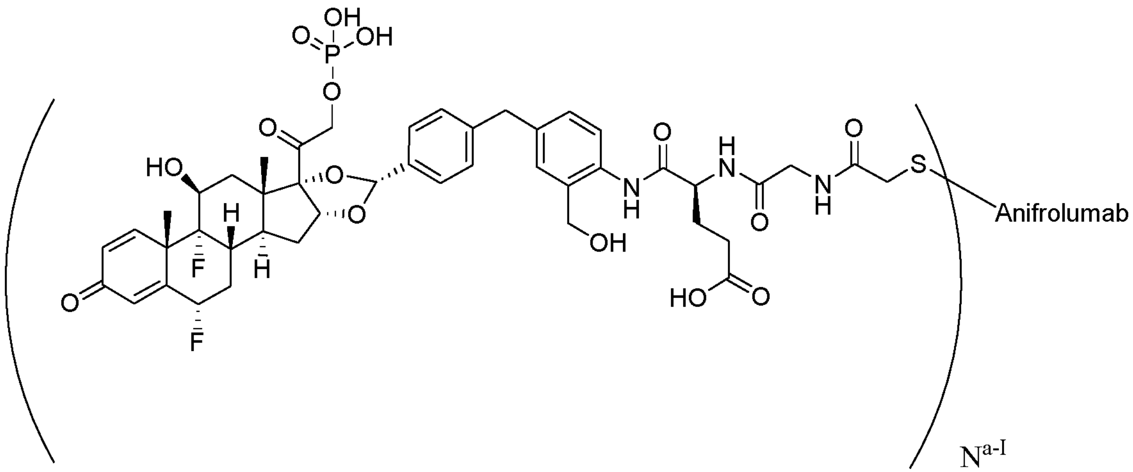 |

(continued)

| No. | Structure |
|---|---|
| V-366 | 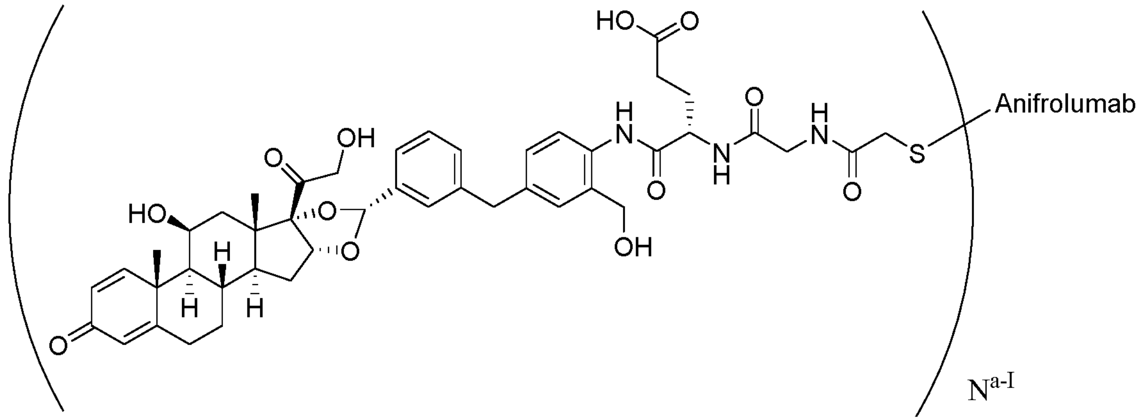 |
| V-367 | 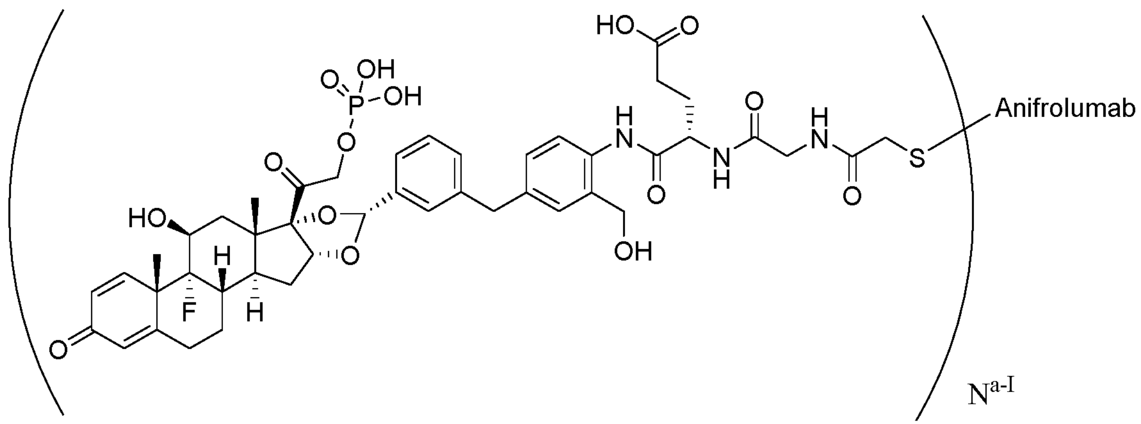 |

(continued)

| No. | Structure |
|---|---|
| V-368 | 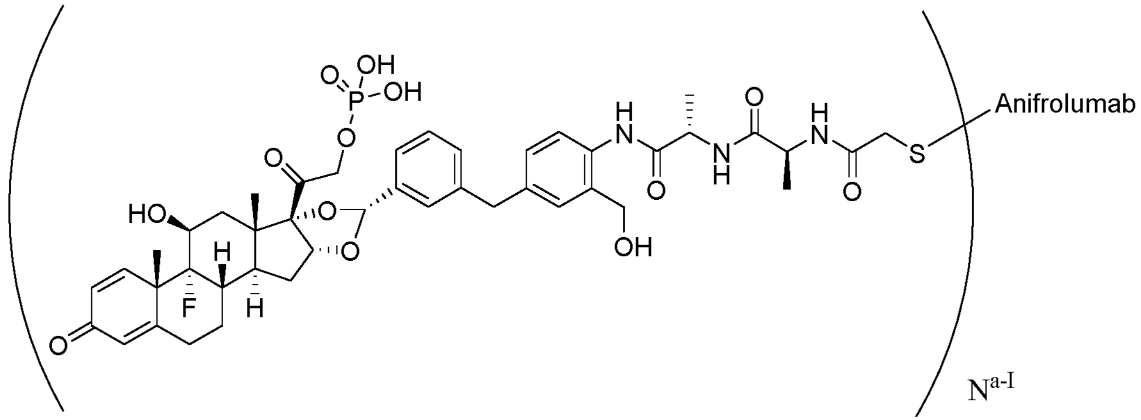 |
| V-371 | 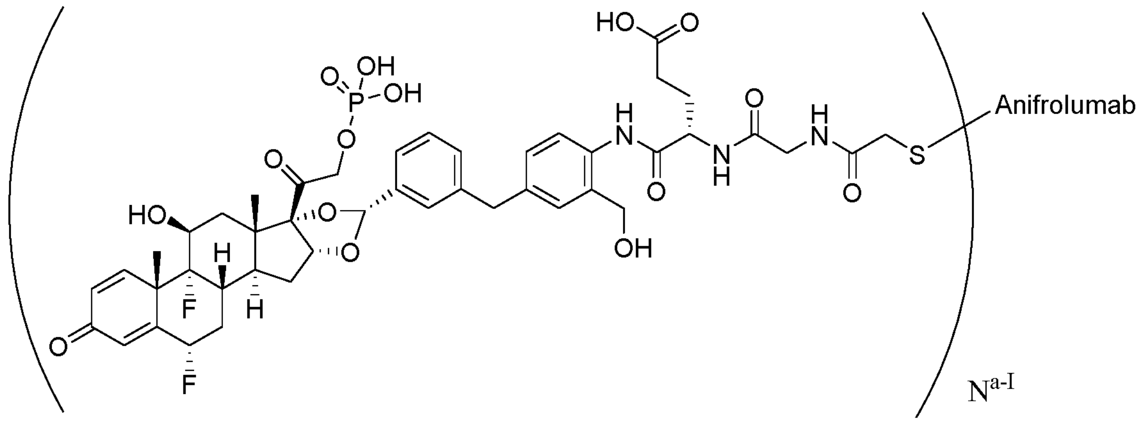 |

(continued)

| No. | Structure |
|---|---|
| V-374 | 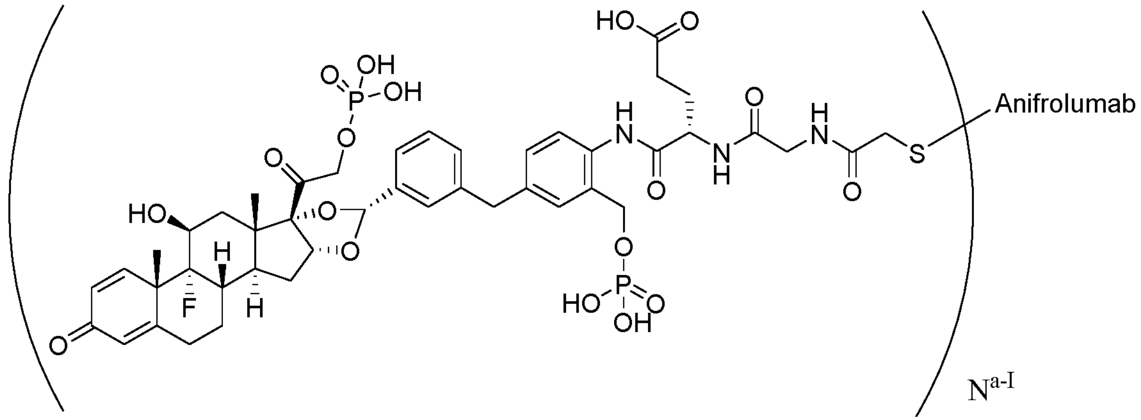 |
| V-378 | 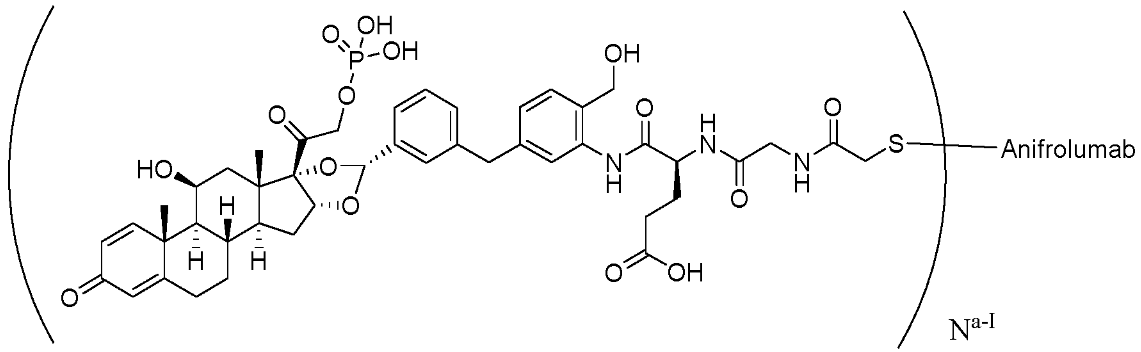 |

(continued)

| No. | Structure |
|---|---|
| V-379 | 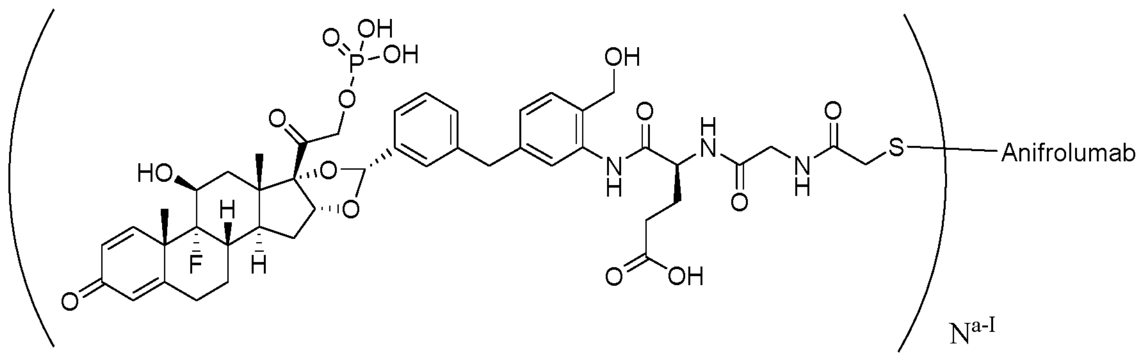 |
| V-380 | 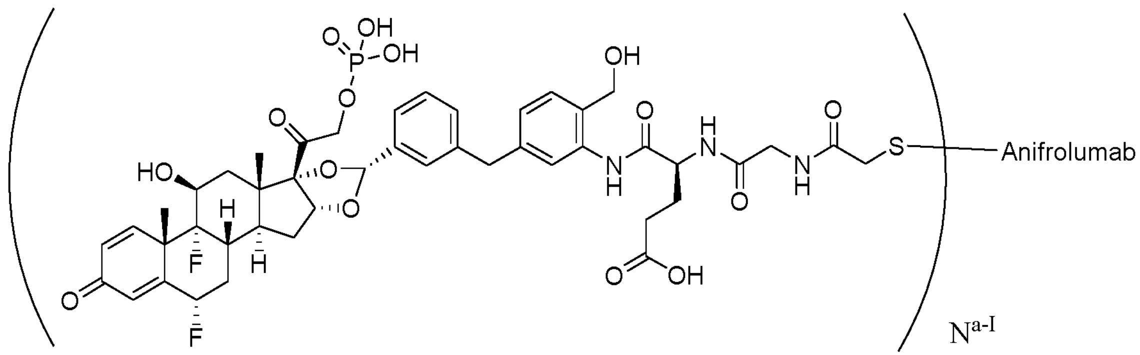 |

(continued)

| No. | Structure |
|---|---|
| V-381 | 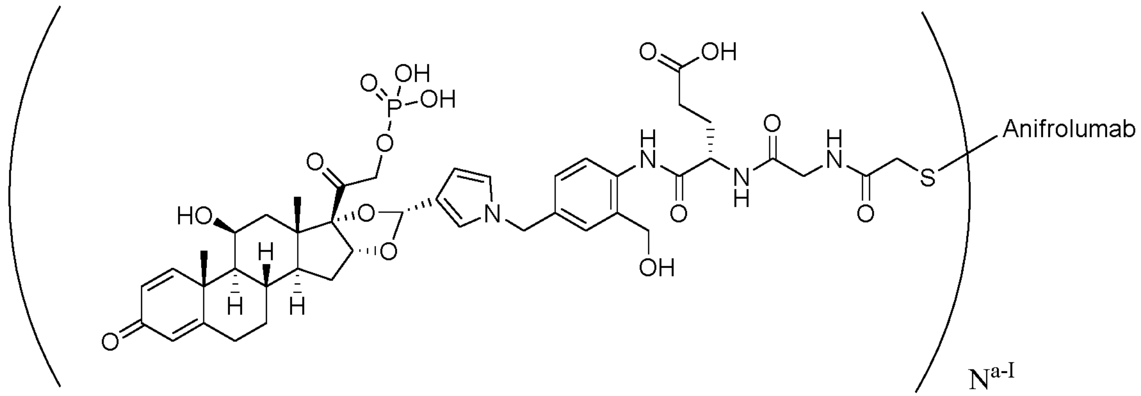 |
| V-382 | 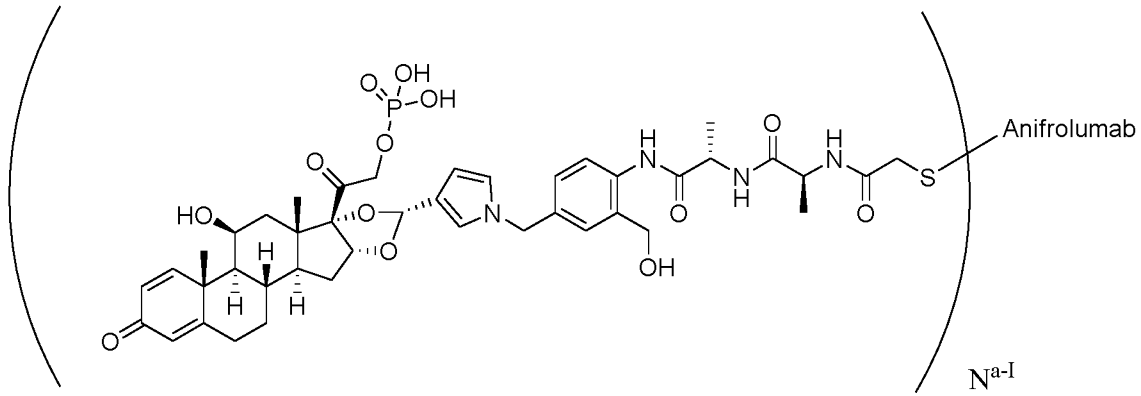 |

(continued)

| No. | Structure |
| --- | --- |
| V-385 | 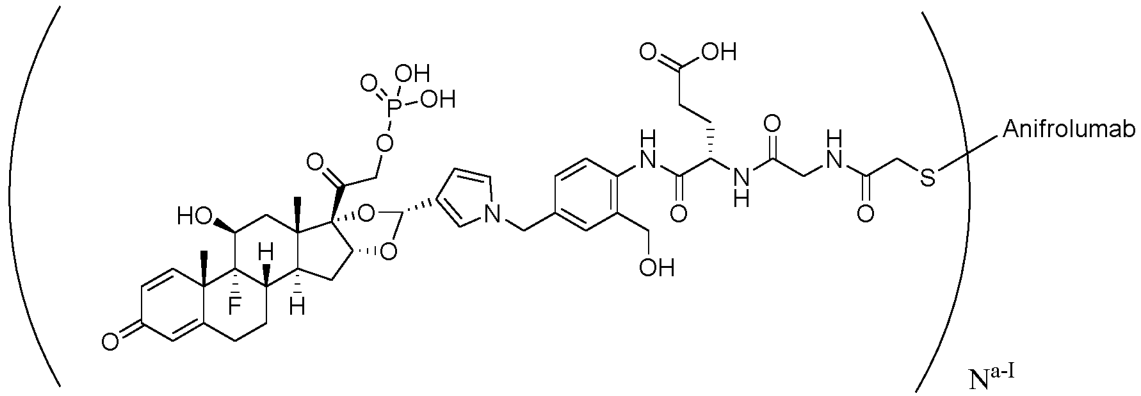 |
| V-386 | 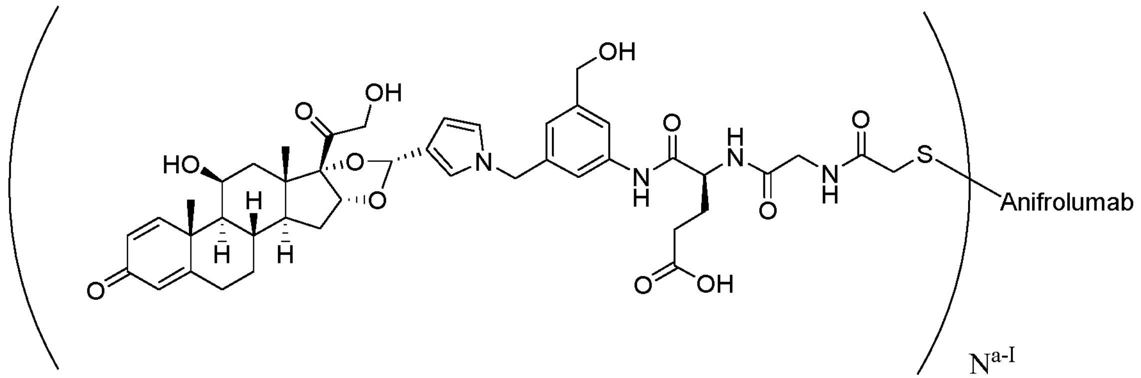 |

(continued)

| No. | Structure |
|---|---|
| V-387 | 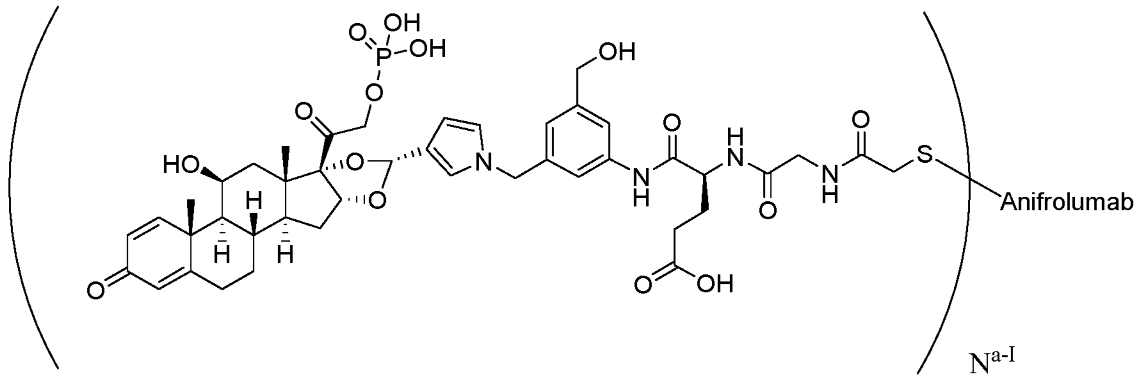 |
| V-388 | 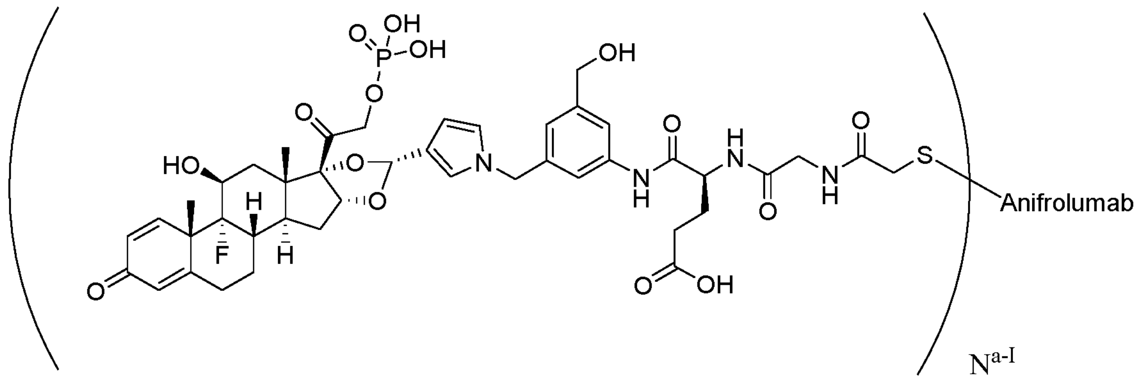 |

(continued)

| No. | Structure |
|---|---|
| V-389 | 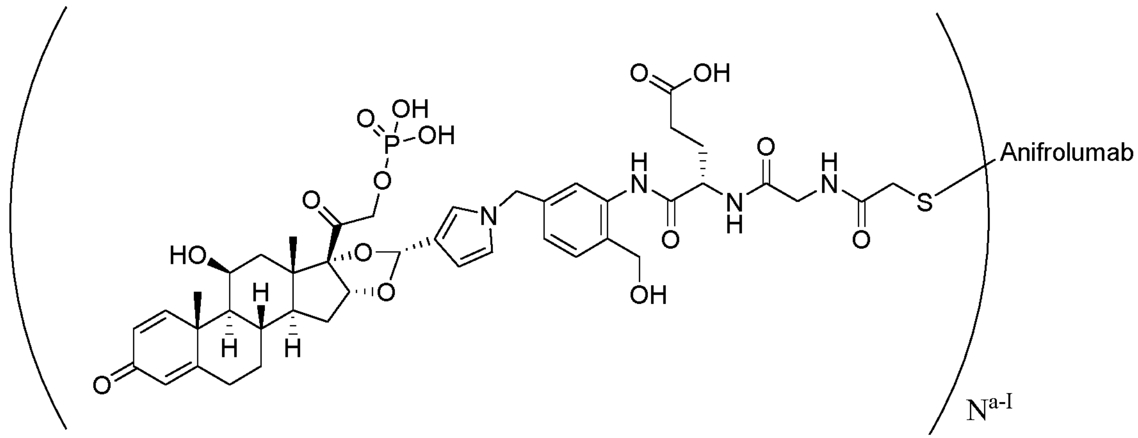 |
| V-390 | 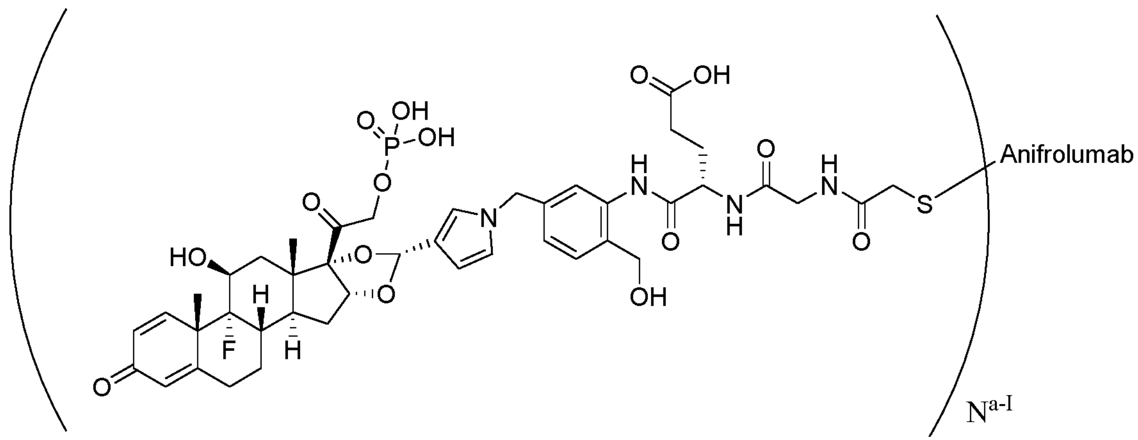 |

(continued)

| No. | Structure |
|---|---|
| V-396 | 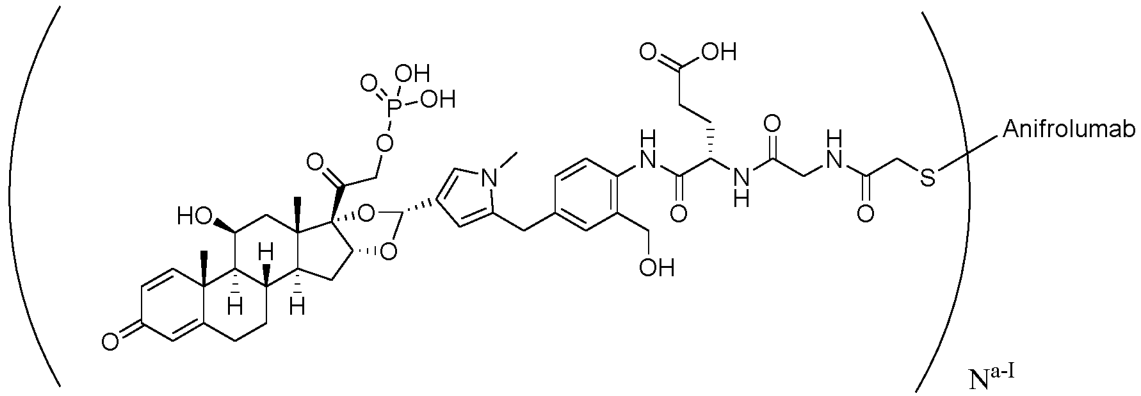 |
| V-397 | 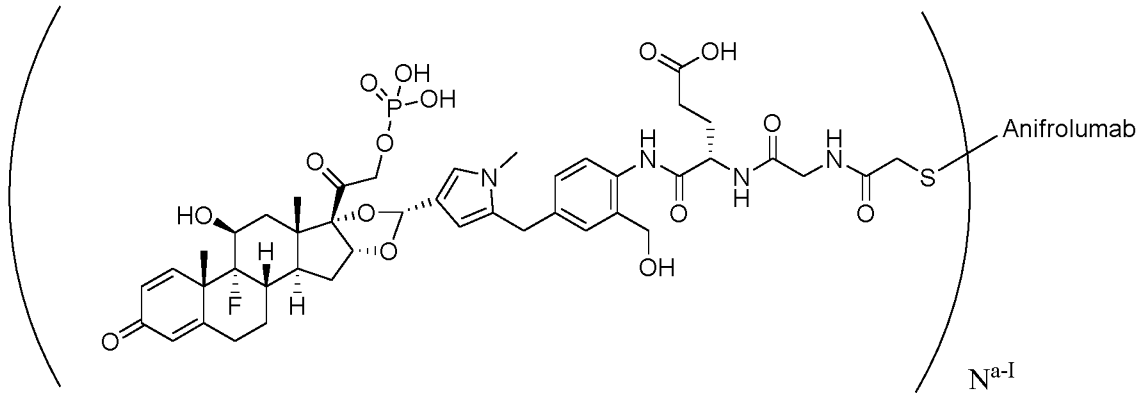 |

(continued)

| No. | Structure |
|---|---|
| V-398 | 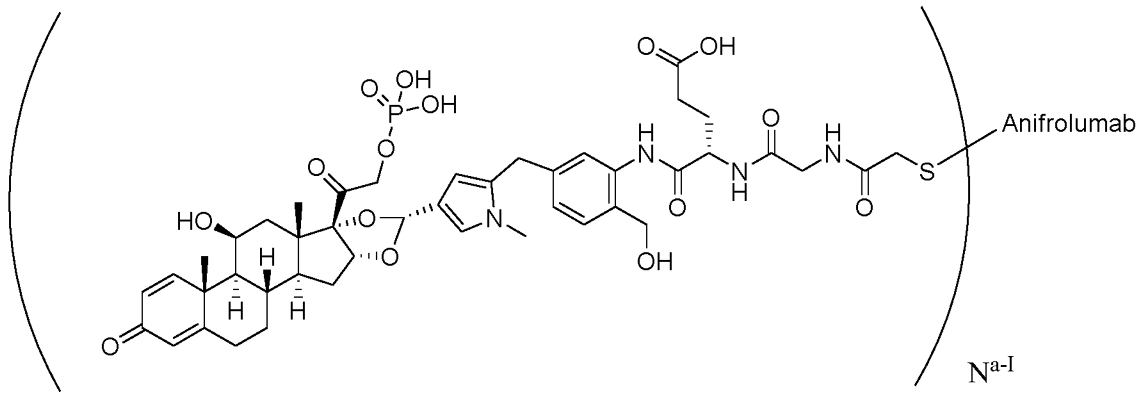 $N^{a-I}$ |
| V-399 | 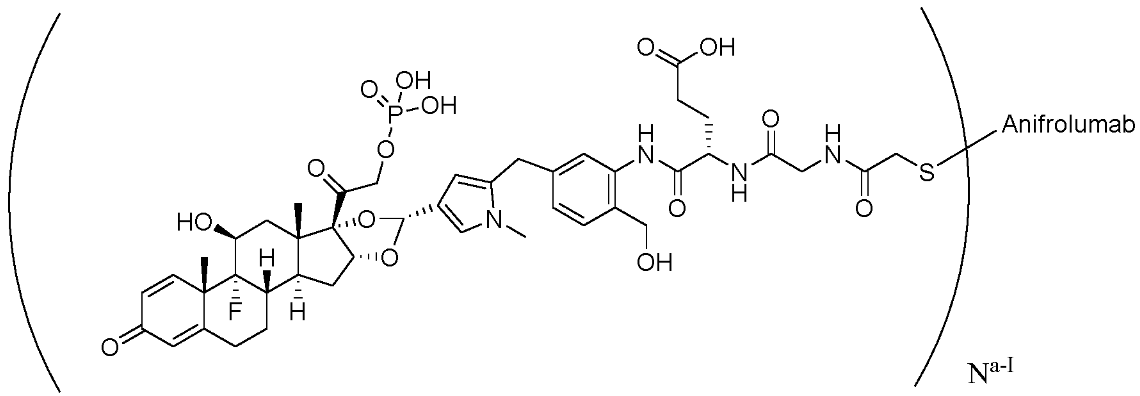 $N^{a-I}$ |

(continued)

| No. | Structure |
| --- | --- |
| V-400 | 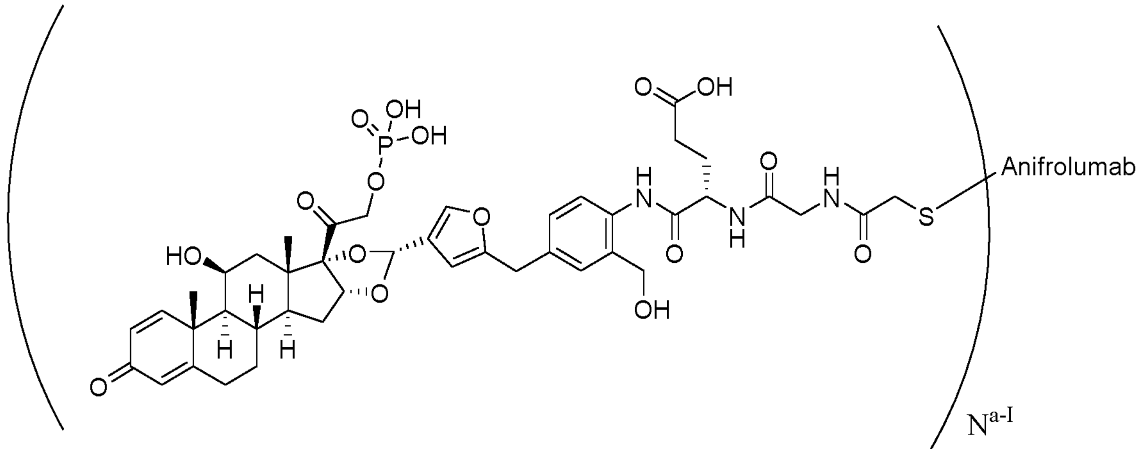 |
| V-401 | 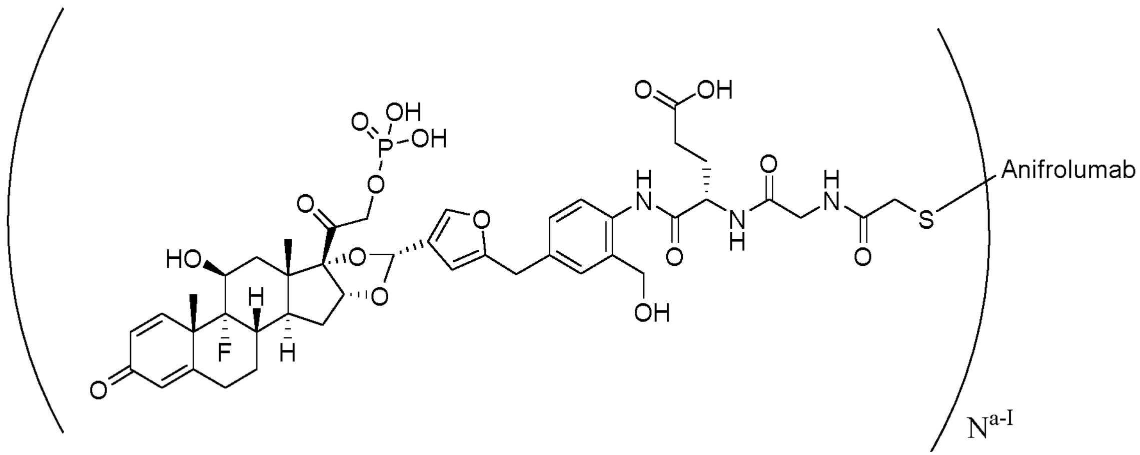 |

(continued)

| No. | Structure |
| --- | --- |
| V-402 | 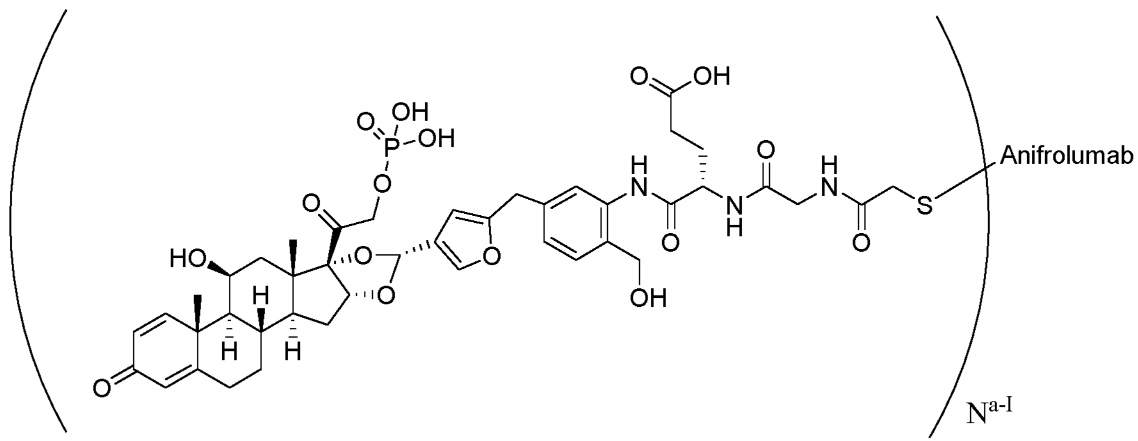 |
| V-403 | 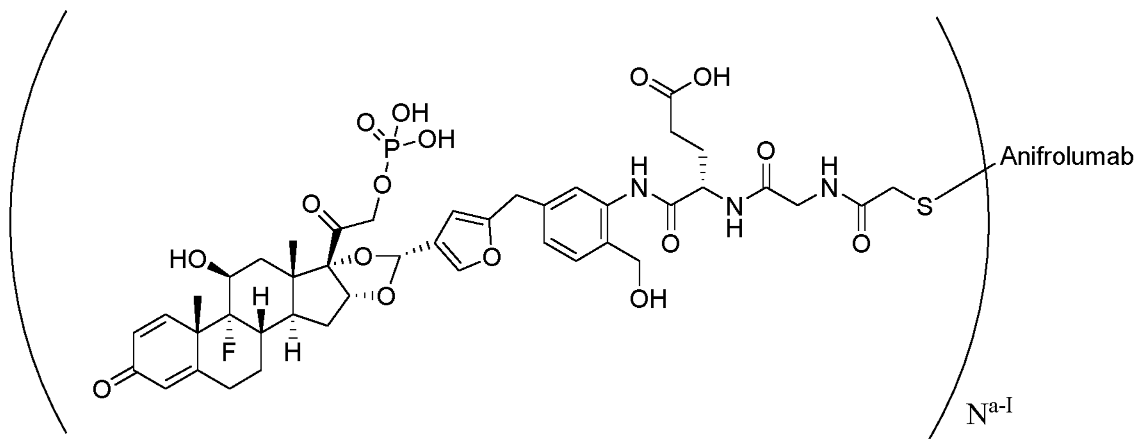 |

(continued)

| No. | Structure |
|---|---|
| V-404 | 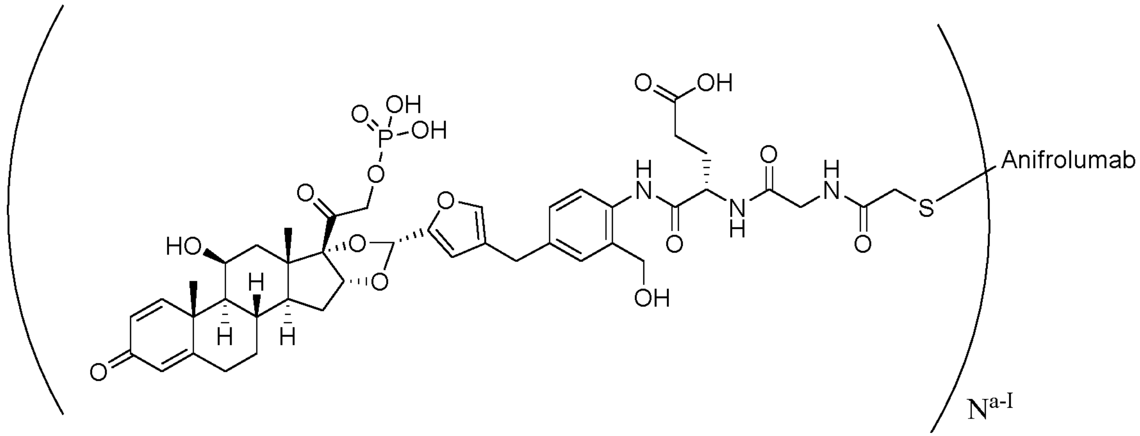 |
| V-405 | 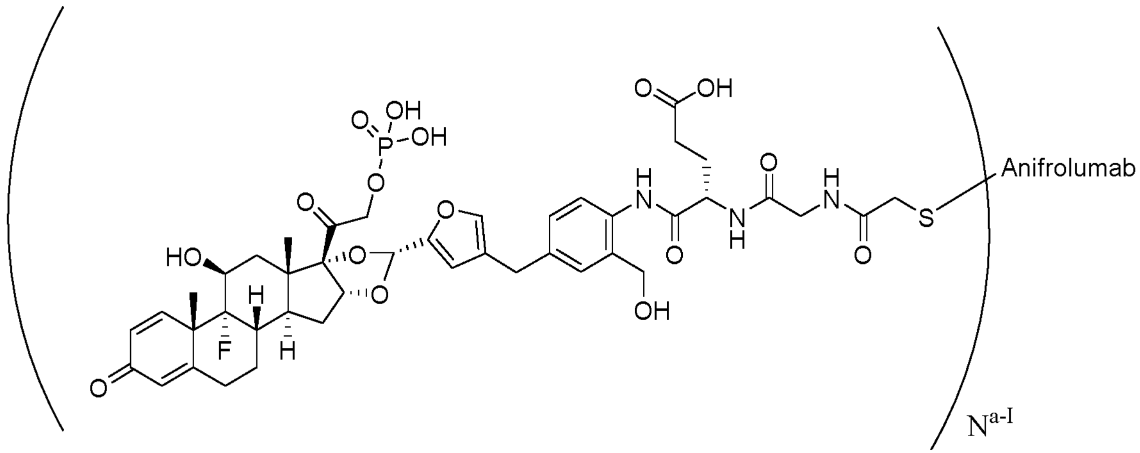 |

(continued)

| No. | Structure |
| --- | --- |
| V-406 | 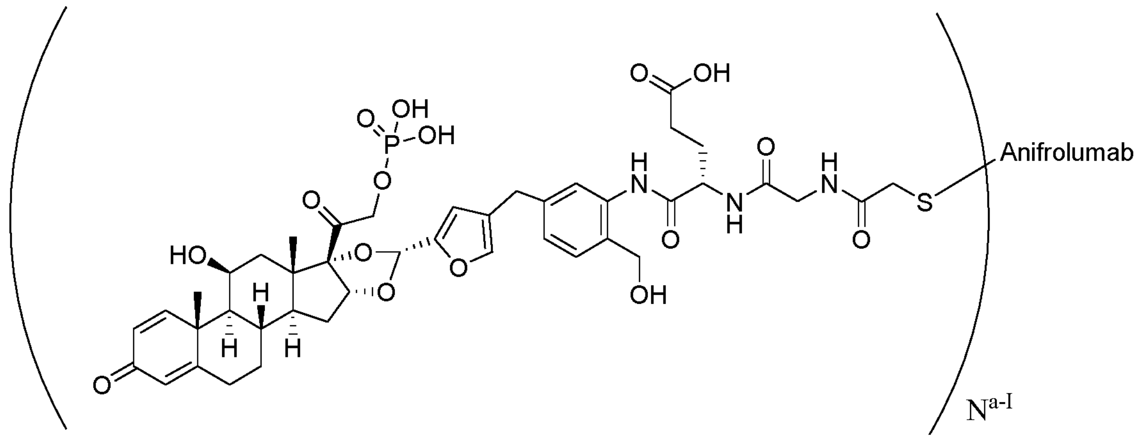 |
| V-407 | 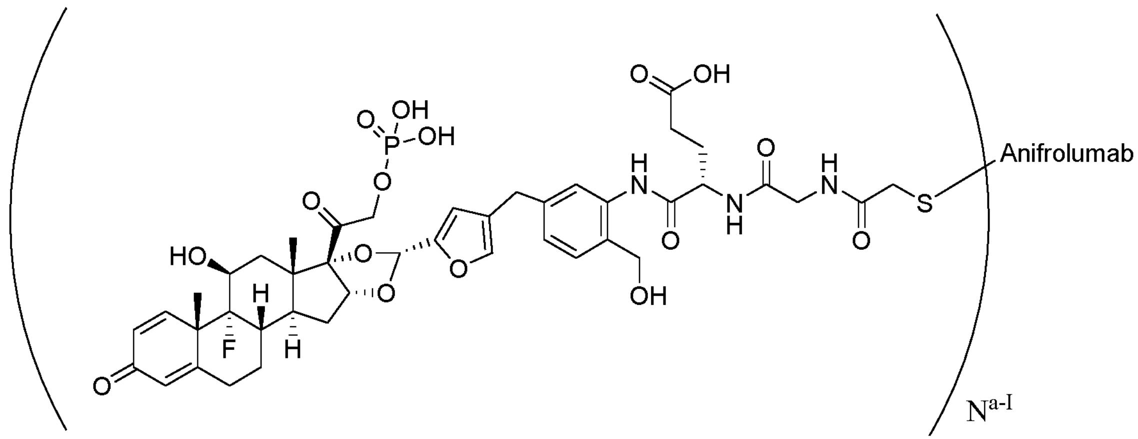 |

(continued)

| No. | Structure |
|---|---|
| V-408 | 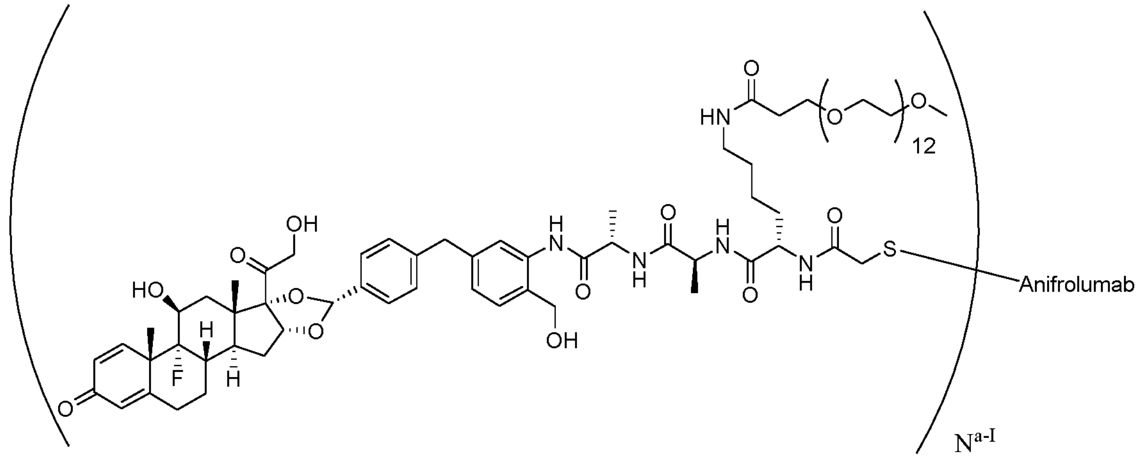 |
| V-409 | 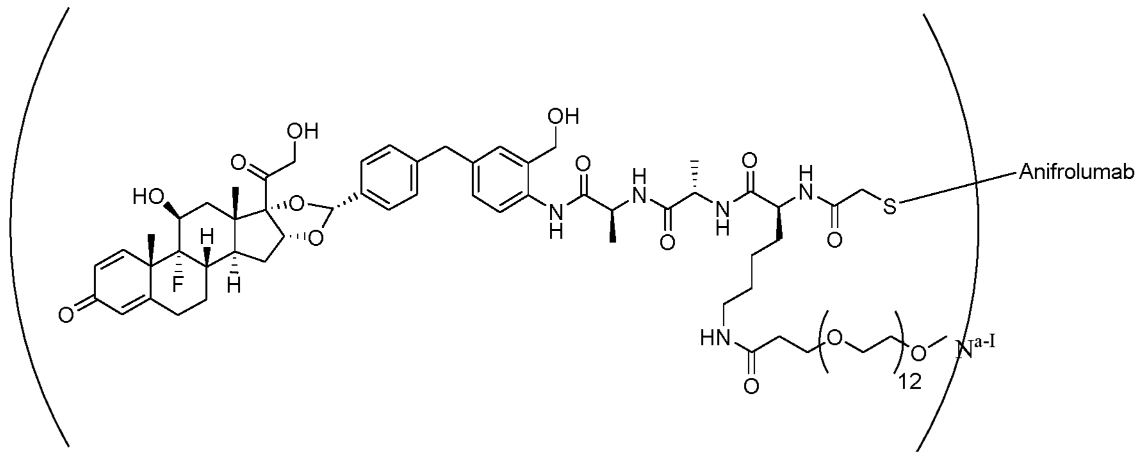 |

(continued)

| No. | Structure |
|---|---|
| V-410 | 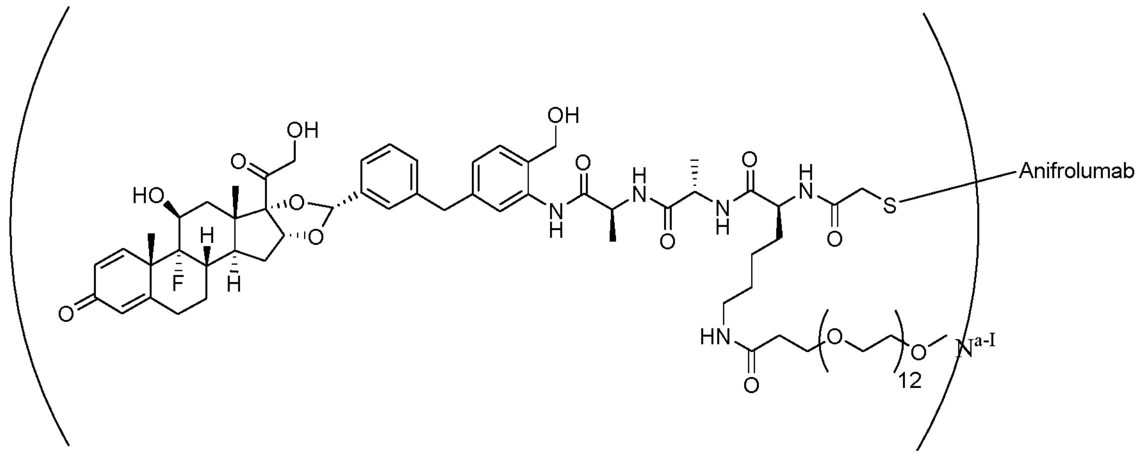 |
| V-411 | 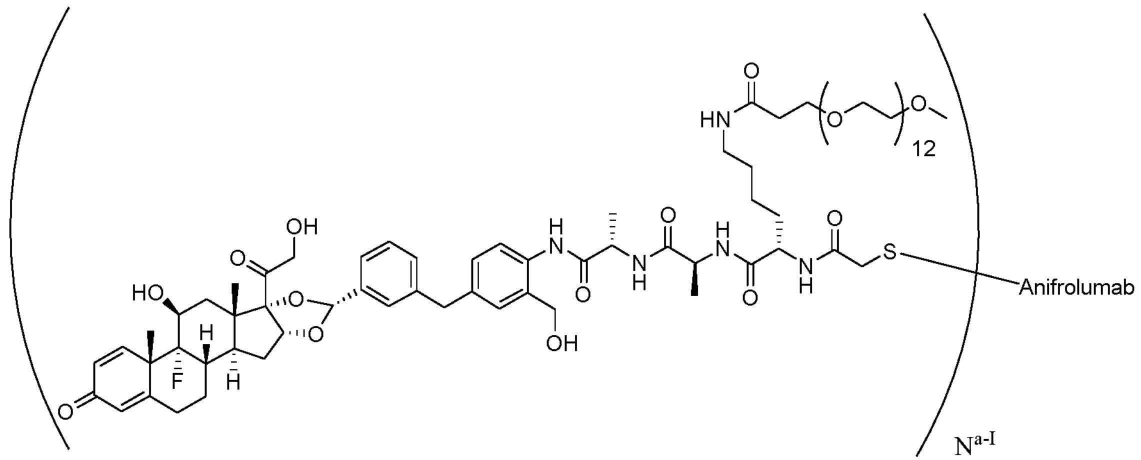 |

(continued)

| No. | Structure |
|---|---|
| V-412 | 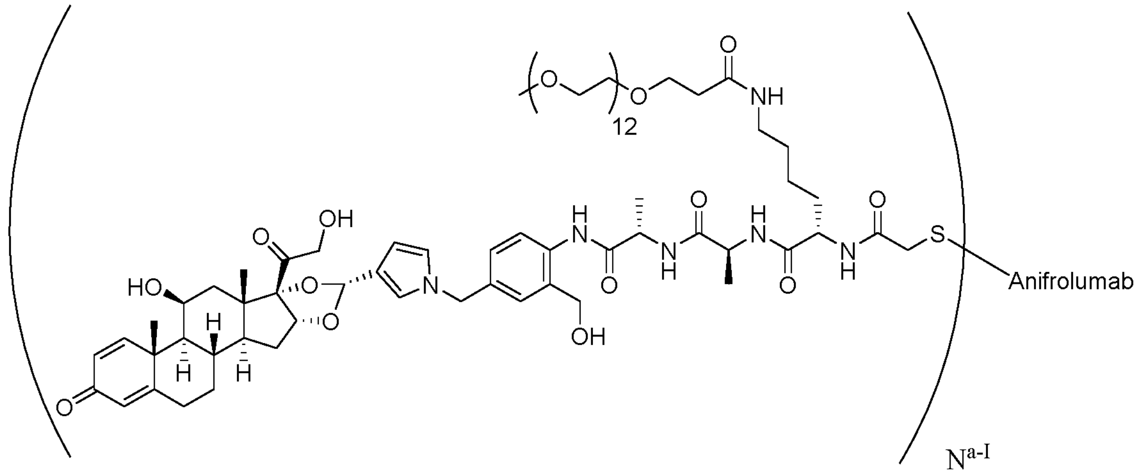 |
| V-413 | 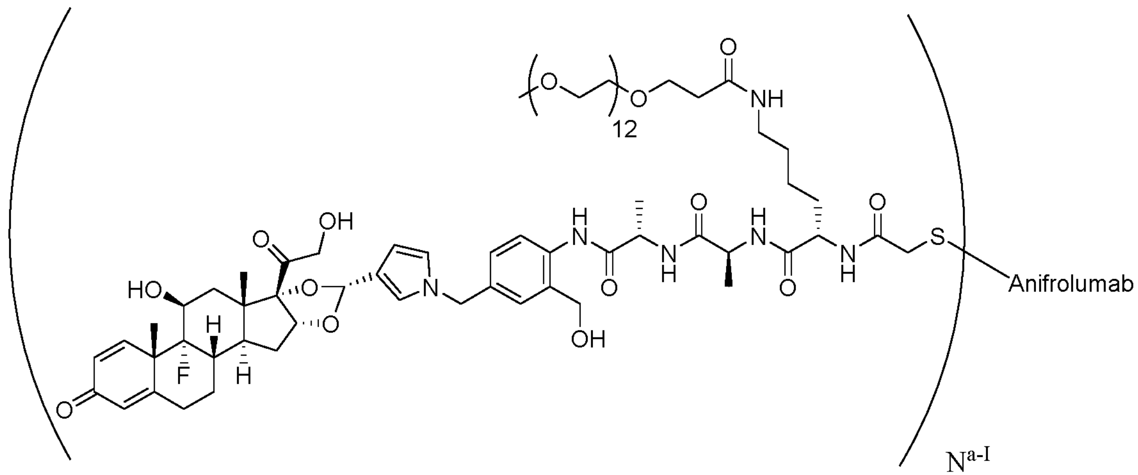 |

(continued)

| No. | Structure |
|---|---|
| V-414 | 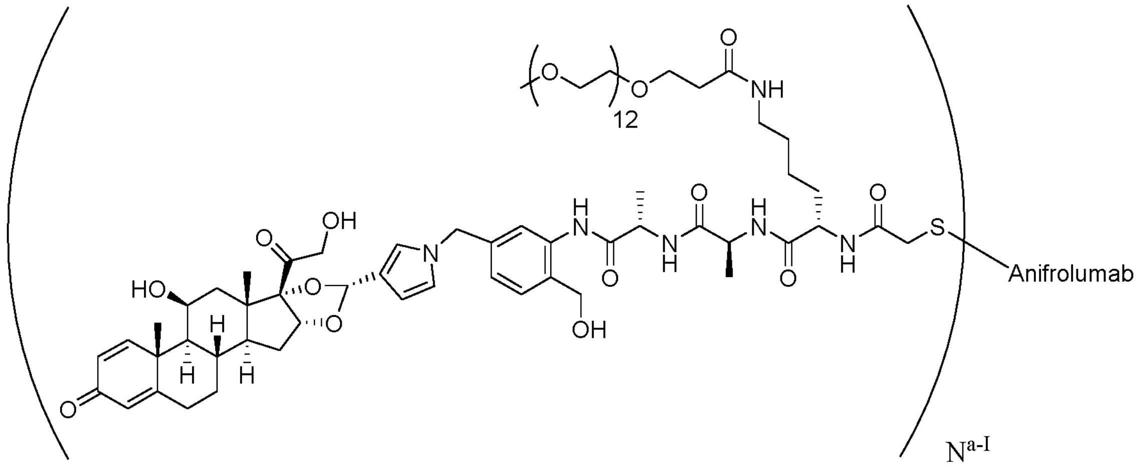 |
| V-415 | 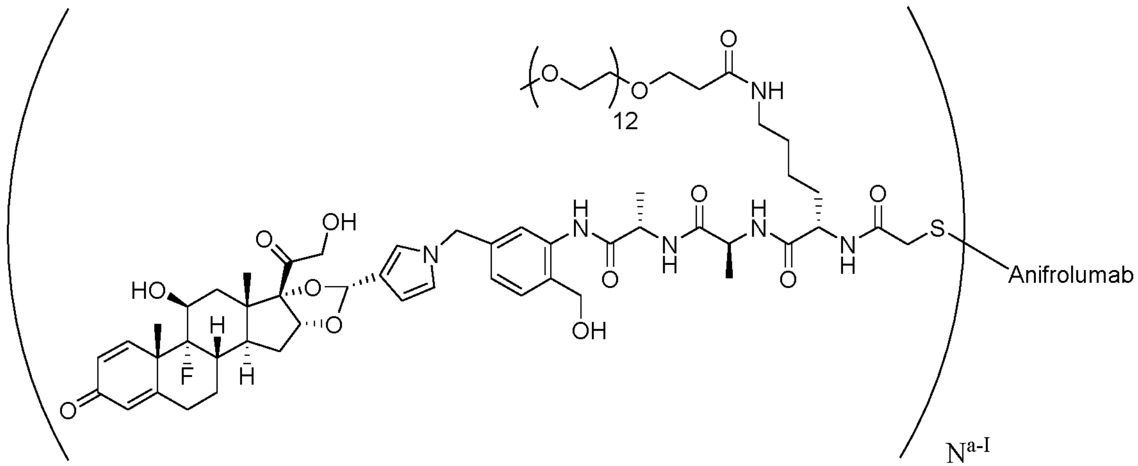 |

; wherein $N^{a-I}$ is any number from 1 to 10.

In certain embodiments, for the conjugates of the present invention, the ligand-drug conjugates have the following structure:

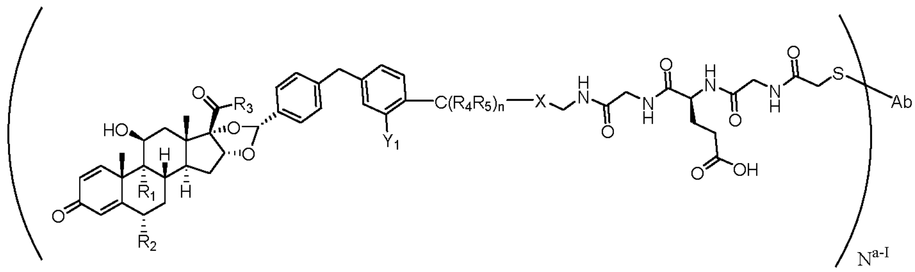

(Va-1)

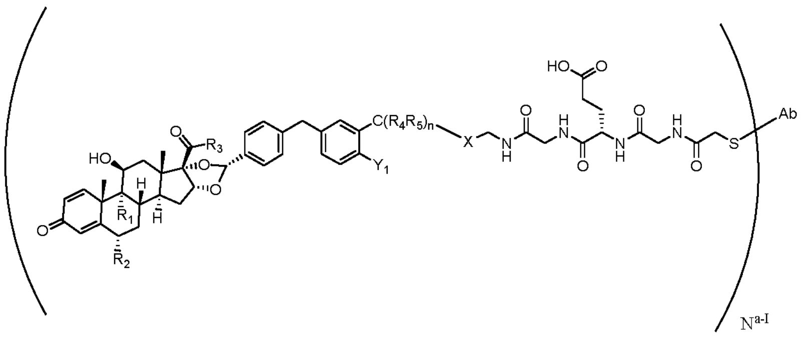

(Vb-1)

wherein,

Ab represents a ligand capable of binding to a target, preferably an antibody or an antigen-binding fragment thereof;
$N^{a-I}$ is any number from 1 to 10;
X is selected from the group consisting of -O-, -S- and -NH-;
$R_4$ and $R_5$ are each independently selected from the group consisting of: H, F, Cl, -OH, $-NH_2$ and $C_1$-$C_6$ alkyl;
N is selected from the group consisting of 1, 2 and 3;
$Y_1$ is absent or selected from the group consisting of: hydroxy, sulfhydryl, amino and $C_1$-$C_6$ alkyl;
$R_1$ and $R_2$ are each independently selected from the group consisting of hydrogen, fluorine, chlorine and methyl;
$R_3$ is selected from the group consisting of: $-CH_2Cl$, $-CH_2SH$, $-CH_2OH$,

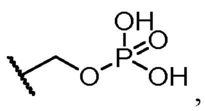

$-OCH_3$, - $OCH_2F$, $-OCH_2Cl$, $-OCH_2CN$, $-OCH_2CH_3$, -SH, $-SCH_2F$, $-SCH_2Cl$, $-SCH_2CF_3$ and $-SCH_2CN$.

In certain embodiments, the conjugates of the present invention are selected from the group consisting of the following structures:

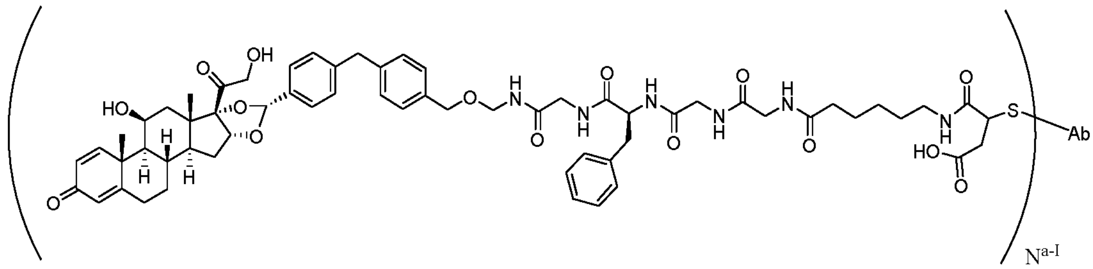

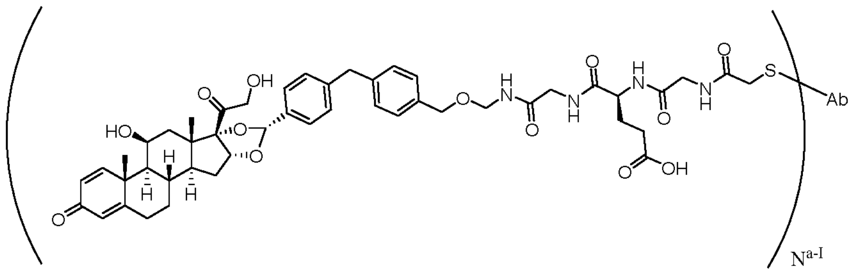

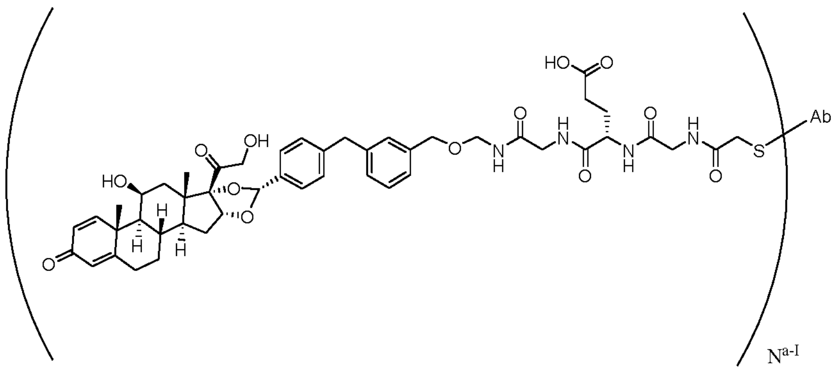

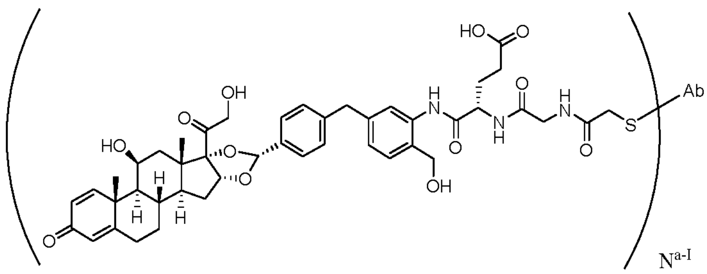

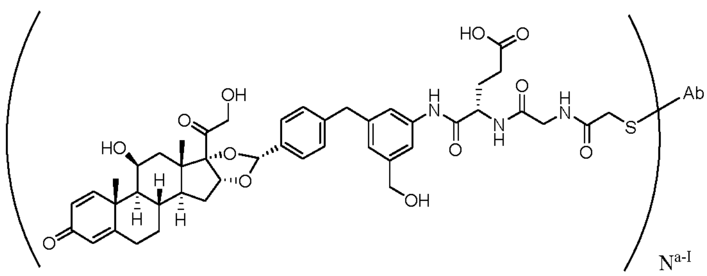

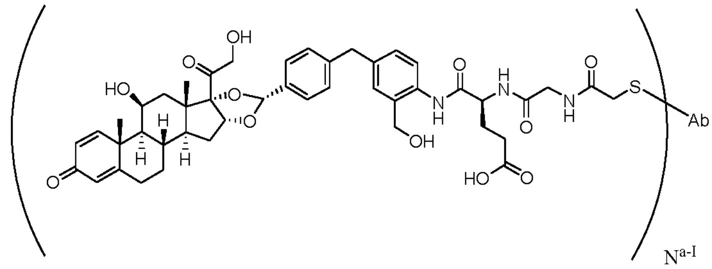

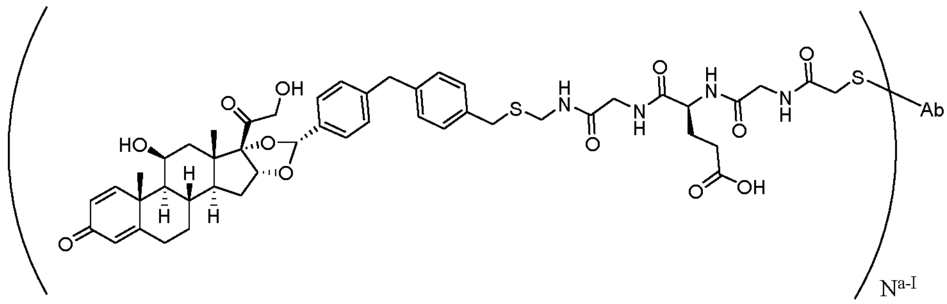

; wherein $N^{a-I}$ is any number from 1 to 10, and Ab is selected from an antibody or an antigen-binding fragment thereof.

In certain embodiments, the conjugates of the present invention are selected from the group consisting of the following structures:

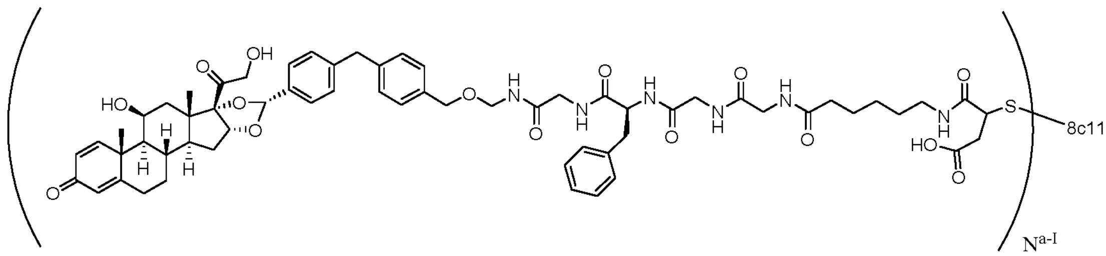

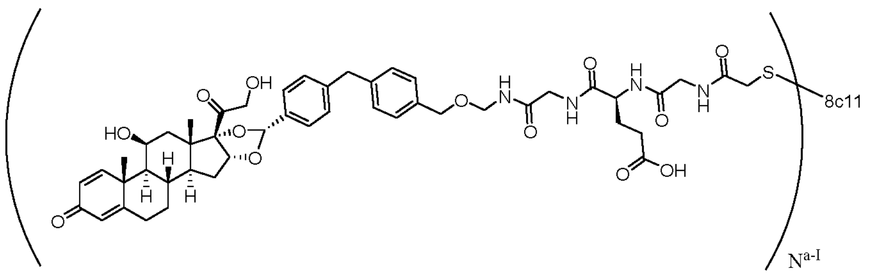

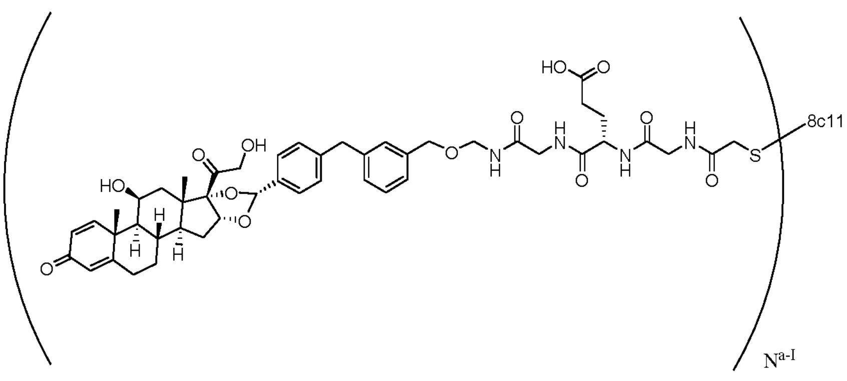

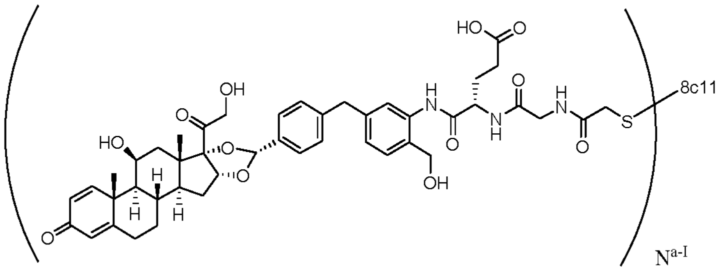

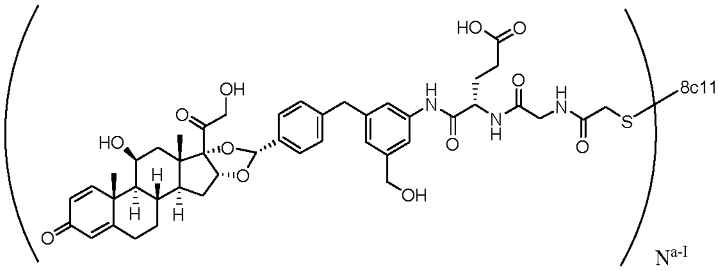

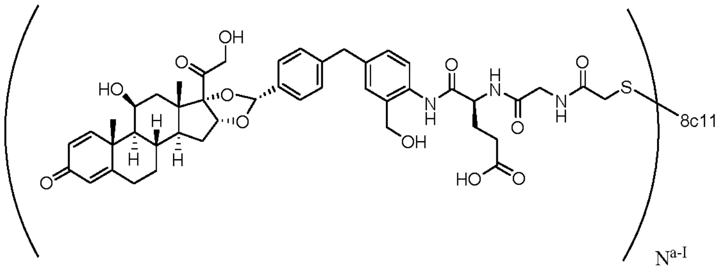

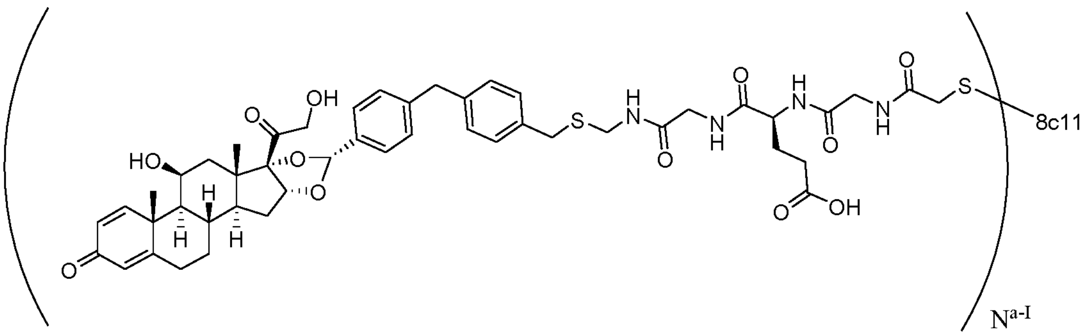

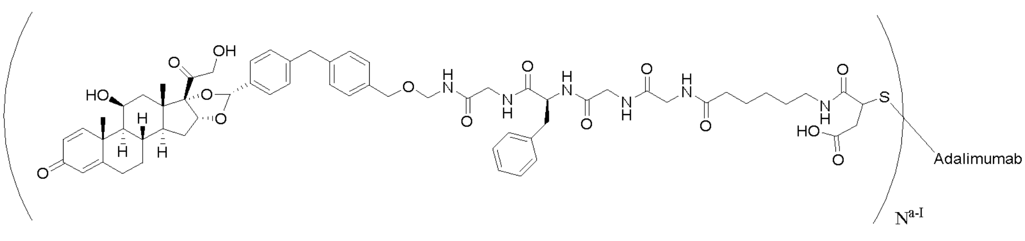

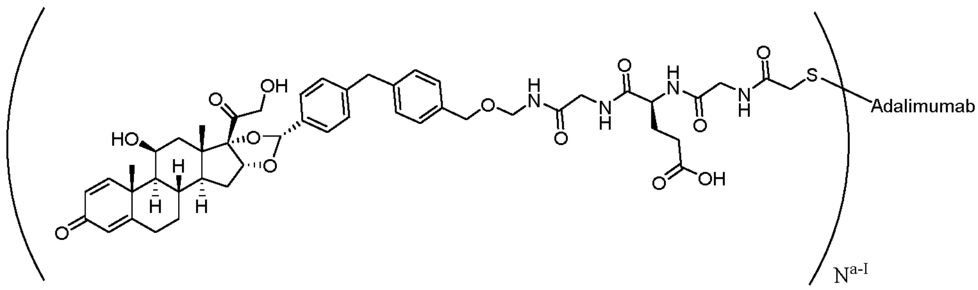

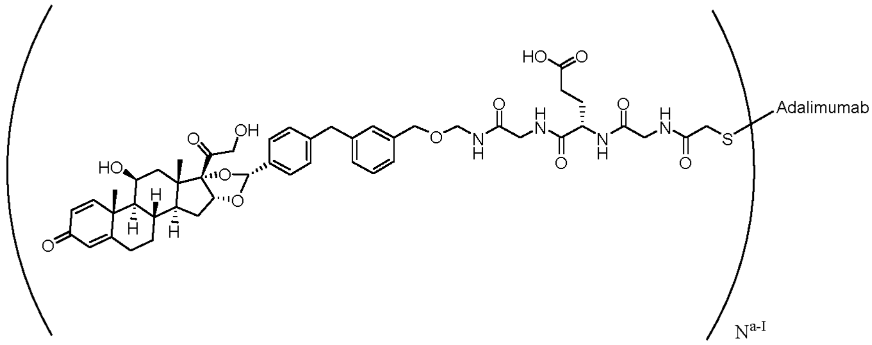

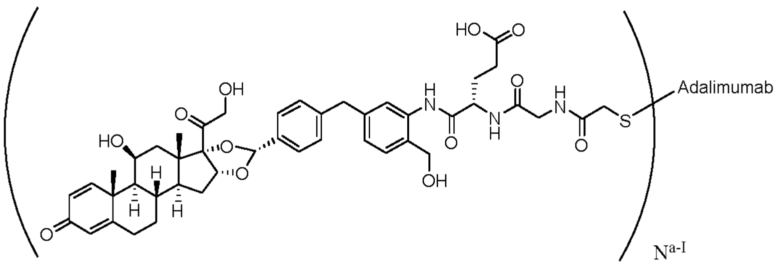

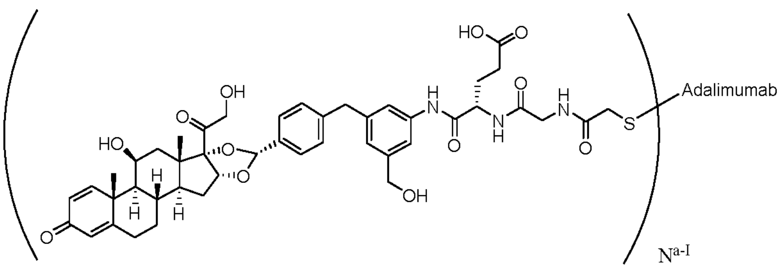

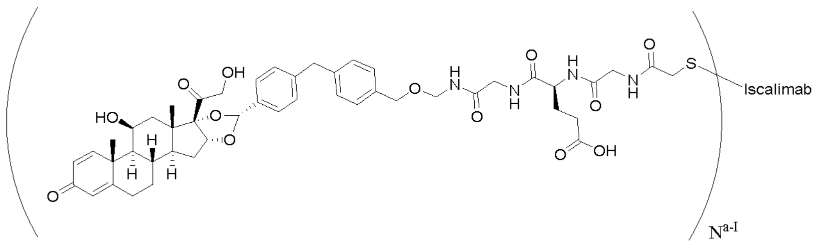

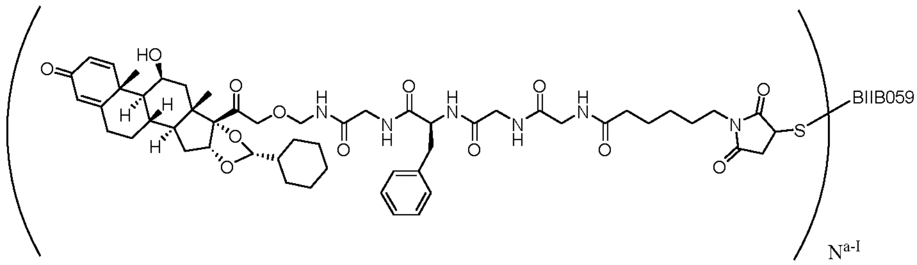

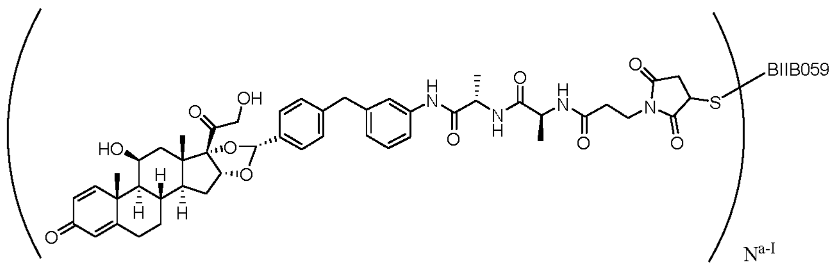

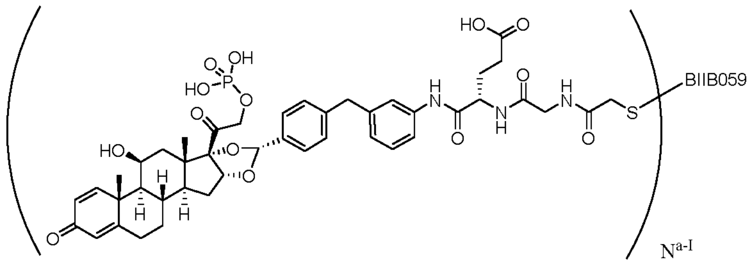

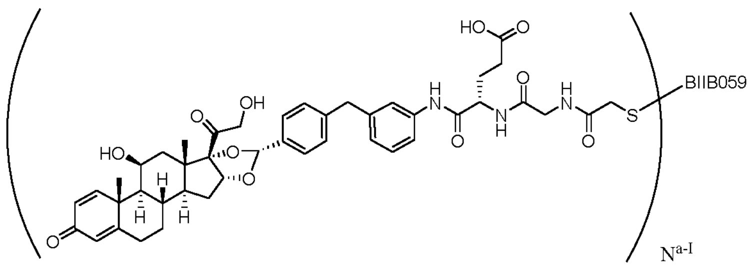

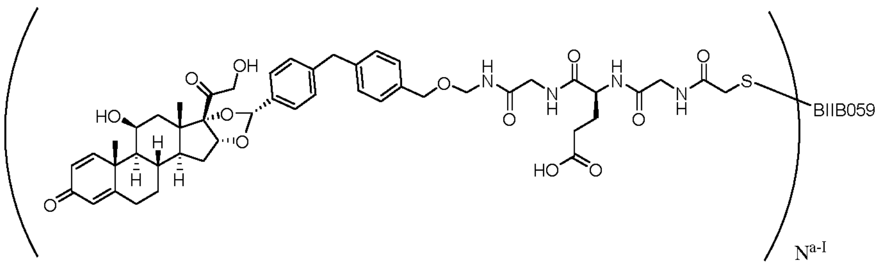

;

wherein $N^{a-I}$ is any number from 1 to 10.

In certain embodiments, the active metabolite of the conjugate comprises the compounds described above.

In another aspect, the present application provides a pharmaceutical composition comprising the compound or the tautomer, the mesomer, the racemate, the enantiomer or the diastereoisomer thereof, or the mixture thereof, or the pharmaceutically acceptable salt thereof described above and/or the conjugate described above, and optionally a pharmaceutically acceptable carrier.

In another aspect, the present application provides a method for influencing immune system functions, which comprises administering to a subject the compound or the tautomer, the mesomer, the racemate, the enantiomer or the

diastereoisomer thereof, or the mixture thereof, or the pharmaceutically acceptable salt thereof described above, the conjugate described above and/or the pharmaceutical composition described above.

In certain embodiments, the influencing immune system functions comprises influencing immune cell functions.

In certain embodiments, the immune cell is selected from the group consisting of: granulocytes and agranulocytes.

In certain embodiments, the immune cell is selected from the group consisting of: neutrophils, eosinophils, and basophils.

In certain embodiments, the immune cell is selected from the group consisting of: lymphocytes and phagocytes.

In certain embodiments, the immune cell is selected from the group consisting of: B cells, T cells, natural killer cells, monocytes, macrophages, mast cells and dendritic cells.

In another aspect, the present application provides use of the compound or the tautomer, the mesomer, the racemate, the enantiomer or the diastereoisomer thereof, or the mixture thereof, or the pharmaceutically acceptable salt thereof described above, the conjugate described above and/or the pharmaceutical composition described above for the manufacture of a medicament for the prevention and/or treatment of a disease and/or condition.

In certain embodiments, the disease and/or condition comprises a disease and/or condition associated with glucocorticoid receptor signaling.

In certain embodiments, the disease and/or condition is selected from the group consisting of: proliferative diseases and/or conditions, metabolic diseases and/or conditions, inflammatory diseases and/or conditions, and neurodegenerative diseases and/or conditions.

In certain embodiments, the disease and/or condition is selected from the group consisting of: systemic autoimmune diseases and/or conditions, blood system-related diseases and/or conditions, neuromuscular system-related diseases and/or conditions, digestive system-related diseases and/or conditions, urological system-related diseases and/or conditions, endocrine gland system-related diseases and/or conditions, cutaneous muscular system-related diseases and/or conditions, and respiratory system-related diseases and/or conditions.

In certain embodiments, the disease and/or condition is selected from the group consisting of: rheumatoid arthritis, systemic lupus erythematosus, scleroderma, Sjogren’s syndrome, ankylosing spondylitis, Wegener granulomatosis and systemic sclerosis.

In certain embodiments, the disease and/or condition is selected from the group consisting of: autoimmune hemolytic anemia, pernicious anemia, idiopathic thrombocytopenic purpura, idiopathic thrombocytopenia, and vasculitis.

In certain embodiments, the disease and/or condition is selected from the group consisting of: multiple sclerosis, myasthenia gravis, and Guillain-Barre syndrome.

In certain embodiments, the disease and/or condition is selected from the group consisting of: ulcerative colitis, Crohn’s disease, autoimmune liver disease, and atrophic gastritis.

In certain embodiments, the disease and/or condition is selected from the group consisting of: IgA nephropathy, primary nephrotic syndrome, autoimmune glomerulonephritis, Goodpasture’s syndrome, and lupus nephritis.

In certain embodiments, the disease and/or condition is selected from the group consisting of: type I diabetes, Grave’s disease, Hashimoto thyroiditis, primary adrenocortical atrophy and chronic thyroiditis.

In certain embodiments, the disease and/or condition is selected from the group consisting of: psoriasis, pemphigus vulgaris, cutaneous lupus erythematosis, dermatomyositis, and polymyalgia rheumatica.

In certain embodiments, the disease and/or condition is asthma.

In another aspect, the present application provides a method for preventing and/or treating a disease and/or condition, which comprises administering to a subject in need thereof the compound or the tautomer, the mesomer, the racemate, the enantiomer or the diastereoisomer thereof, or the mixture thereof, or the pharmaceutically acceptable salt thereof described above, the conjugate described above and/or the pharmaceutical composition described above.

In certain embodiments, the disease and/or condition comprises a disease and/or condition associated with glucocorticoid receptor signaling.

In certain embodiments, the disease and/or condition is selected from the group consisting of: proliferative diseases and/or conditions, metabolic diseases and/or conditions, inflammatory diseases and/or conditions, and neurodegenerative diseases and/or conditions.

In certain embodiments, the disease and/or condition is selected from the group consisting of: systemic autoimmune diseases and/or conditions, blood system-related diseases and/or conditions, neuromuscular system-related diseases and/or conditions, digestive system-related diseases and/or conditions, urological system-related diseases and/or conditions, endocrine gland system-related diseases and/or conditions, cutaneous muscular system-related diseases and/or conditions, and respiratory system-related diseases and/or conditions.

In certain embodiments, the disease and/or condition is selected from the group consisting of: rheumatoid arthritis, systemic lupus erythematosus, scleroderma, Sjogren’s syndrome, ankylosing spondylitis, Wegener granulomatosis and systemic sclerosis.

In certain embodiments, the disease and/or condition is selected from the group consisting of: autoimmune

hemolytic anemia, pernicious anemia, idiopathic thrombocytopenic purpura, idiopathic thrombocytopenia, and vasculitis.

In certain embodiments, the disease and/or condition is selected from the group consisting of: multiple sclerosis, myasthenia gravis, and Guillain-Barre syndrome.

In certain embodiments, the disease and/or condition is selected from the group consisting of: ulcerative colitis, Crohn's disease, autoimmune liver disease, and atrophic gastritis.

In certain embodiments, the disease and/or condition is selected from the group consisting of: IgA nephropathy, primary nephrotic syndrome, autoimmune glomerulonephritis, Goodpasture's syndrome, and lupus nephritis.

In certain embodiments, the disease and/or condition is selected from the group consisting of: type I diabetes, Grave's disease, Hashimoto thyroiditis, primary adrenocortical atrophy and chronic thyroiditis.

In certain embodiments, the disease and/or condition is selected from the group consisting of: psoriasis, pemphigus vulgaris, cutaneous lupus erythematosis, dermatomyositis, and polymyalgia rheumatica.

In certain embodiments, the disease and/or condition is asthma. Without being bound by any theory, the following examples are intended only to illustrate the compounds, preparation methods, use, etc., of the present application, and are not intended to limit the scope of the present application.

## Examples

In the present application,

reagents that provide bromination conditions include, but are not limited to, aqueous bromine, N-bromosuccinimide, dibromohydantoin, phosphorus tribromide, liquid bromine/triphenylphosphine, hydrobromic acid, and carbon tetrabromide;

titanium catalysts include, but are not limited to, tetraisopropyl titanate, triisopropoxytitanium chloride, titanium tetrachloride, and triisopropoxytitanium methyl;

palladium catalysts include, but are not limited to, tetrakis(triphenylphosphine)palladium, palladium acetate, palladium chloride, bis(triphenylphosphine)palladium(II) dichloride, tris(dibenzylideneacetone)dipalladium, bis(acetonitrile)palladium(II) dichloride, [1,1'-bis(diphenylphosphino)ferrocene]palladium(II) dichloride, [1,1'-bis(diphenylphosphino)ferrocene]palladium(II) dichloride dichloromethane complex, bis(benzonitrile)palladium chloride, 1,4-butylenebis(diphenylphosphine)-palladium dichloride, allylpalladium chloride dimer, and allyl(cyclopentadienyl)palladium(II);

Borate dimers include, but are not limited to, bis(pinacolato)diboron, bis(neopentyl glycolato)diboron, bis(hexylene glycolato)diboron, bis(catecholato)diboron, bis(diisopropyl-L-tartrate glycolato)diboron, bis[(-)pinanediol]diborate, bis[(1*S*,2*S*,3*R*,5*S*)-pinanediolato]diboron, tetramethyidiborane, bis(*N*,*N*,*N'*,*N'*-tetramethyl-D-tartramidate)diboron, tetrahydroxydiboron, bis(N,N,N,N-tetramethyl-L-tartramidate)diboron, bis(diisopropyl-D-tartrate glycolato)diboron, bis(diethyl-D-tartrate glycolato)diboron, bis(2,4-dimethyl-2,4-pentanediol)borate, bis(diethyl-L-tartrate glycolato)diboron, and 4,4,5,5-tetramethyl-1,3,2-dioxaborolan-2-ylboronic acid;

metallic copper salts include, but are not limited to, copper sulfate, copper sulfate pentahydrate, cuprous sulfate, cupric chloride, cuprous chloride, cupric carbonate, cupric phosphate, cupric acetate, and hydrates thereof, cupric oxalate, cupric fluoroborate, and hydrates thereof, cupric methoxide, cupric tartrate, copper formate, cuprous iodide, copper(II) trifluoroacetate, copper(II) trifluoromethanesulfonate, copper carbonate, cupric bromide, cuprous bromide, and cuprous oxide;

the ligand may be selected from any ligand commonly used in the Ullmann reaction, including but not limited to, L-proline, tyrosine, phenylalanine, 1,10-phenanthroline, *N*,*N*'-dimethylethylenediamine, ethylene glycol, 1,1'-binaphthyl-2,2'-diol, ethyl 2-cyclohexanonecarboxylate, and salicylaldehyde hydrazone;

the condensing agent may be selected from the group consisting of 4-(4,6-dimethoxy-1,3,5-triazin-2-yl)-4-methylmorpholinium chloride, 1-hydroxybenzotriazole and 1-(3-dimethylaminopropyl)-3-ethylcarbodiimide hydrochloride, *N*,*N'*-dicyclohexylcarbodiimide, *N*,*N*'-diisopropylcarbodiimide, O-benzotriazol-*N*,*N*,*N*',*N*'-tetramethyluronium tetrafluoroborate, 1-hydroxybenzotriazole, 1-hydroxy-7-azobenzotriazol, O-benzotriazol-*N*,*N*,*N*',*N*'-tetramethyluronium hexafluorophosphate, 2-(7-azobenzotriazol)-*N*,*N*,*N*',*N*'-tetramethyluronium hexafluorophosphate, benzotriazol-1-yloxytris(dimethylamino)phosphonium hexafluorophosphate, and benzotriazol-1-yl-oxytripyrrolidinophosphonium hexafluorophosphate, preferably 4-(4,6-dimethoxy-1,3,5-triazin-2-yl)-4-methylmorpholinium chloride, or 1-hydroxybenzotriazole and 1-(3-dimethylaminopropyl)-3-ethylcarbodiimide hydrochloride;

reagents that provide basic conditions include organic bases and inorganic bases, wherein the organic bases include, but are not limited to, triethylamine, diethylamine, *N*-methylmorpholine, pyridine, piperidine, *N*,*N*-diisopropylethylamine, *n*-butyllithium, lithium diisopropylamide, potassium acetate, sodium *tert*-butoxide, potassium *tert*-butoxide, and the like, and the inorganic bases include, but are not limited to, sodium hydride, potassium carbonate, sodium carbonate, cesium carbonate, sodium hydroxide, lithium hydroxide, sodium phosphate, and potassium phosphate;

reagents that provide acidic conditions include a protic acid and a Lewis acid, wherein the protic acid includes, but

is not limited to, hydrochloric acid, sulfuric acid, nitric acid, nitrous acid, sulfurous acid, phosphoric acid, phosphorous acid, formic acid, acetic acid, propionic acid, butyric acid, citric acid, benzoic acid, p-toluenesulfonic acid, p-nitrobenzoic acid, methanesulfonic acid, trifluoromethanesulfonic acid and trifluoroacetic acid, and the Lewis acid includes, but is not limited to, boron trifluoride, zinc chloride, magnesium chloride, aluminum chloride, stannic chloride and ferric chloride;

hydrogenation conditions include, but are not limited to: Pb/C/hydrogen, Pt/C/hydrogen, palladium chloride/hydrogen, Raney nickel/hydrogen, palladium hydroxide carbon/hydrogen, and palladium hydroxide/hydrogen;

reagents that provide oxidation conditions include, but are not limited to, Dess-Martin periodinane, hydrogen peroxide, sodium chlorite, sodium hypochlorite, and potassium perchlorate;

reagents that provide reducing conditions include, but are not limited to, sodium hydride, calcium hydride, lithium hydride, lithium aluminum hydride, sodium borohydride, lithium borohydride, sodium triethylborohydride, sodium triacetoxyborohydride and sodium cyanoborohydride;

reagents that provide oxidation conditions include, but are not limited to, Dess-Martin periodinane, hydrogen peroxide, sodium chlorite, sodium hypochlorite, and potassium perchlorate.

In addition, in the present application,
the structure of the compounds is determined by nuclear magnetic resonance (NMR) or mass spectrometry (MS). NMR is performed using a Quan tum-I NMR spectrometer with deuterated dimethyl sulfoxide (DMSO-D), deuterated chloroform (CDC13), and deuterated methanol (CD3OD) as solvents, and tetramethylsilane (TMS) as an internal standard, and chemical shifts are given in units of 10_6 (ppm).

MS is performed using an Angilent 6230 ESI-TOF mass spectrometer (manufacturer: Agilent, type c: 6230).

UPLC is performed using a Waters AcquityUPLCSQD liquid chromatograph-mass spectrometer (Poroshell 120 EC-C18, 2.1 mm × 50 mm, 1.9 μm column).

HPLC is performed using an Agilent 1260 high-performance liquid chromatograph (TOSOH G3000 SW SEC column).

UV is measured using a Thermo Nanodrop 2000 spectrophotometer.

Enzyme-linked immunoassays are performed using an EnVision microplate reader (PerkinElmer).

Yantai Yellow Sea HSGF254 or Qingdao GF254 silica gel plate is adopted as a thin layer chromatography (TLC) silica gel plate. The specification adopted by the TLC is 0.15-0.20 mm, and the specification adopted by the thin layer chromatography for the separation and purification of products is 0.4-0.5 mm.

Yantai Yellow Sea silica gel of 200-300 mesh is generally utilized as a carrier in column chromatography.

Known starting materials of the present application can be synthesized using or according to methods known in the art, or can be purchased from companies such as ABCR GMBH & Co. KG, Acros Organnics, Aldrich Chemical Company, Accela ChemBio Inc., and Darui Chemicals.

In the examples, all reactions are carried out under an argon atmosphere or a nitrogen atmosphere unless otherwise stated.

The argon atmosphere or nitrogen atmosphere means that the reaction flask is connected to a balloon containing about 1 L of argon or nitrogen.

The hydrogen atmosphere means that the reaction flask is connected to a balloon containing about 1 L of hydrogen.

In the examples, the solution in the reaction is an aqueous solution unless otherwise stated.

In the examples, the reaction temperature is room temperature unless otherwise stated. The room temperature is the optimum reaction temperature, ranging from 20 °C to 30 °C.

The system of eluents for column chromatography and the system of developing agents for thin layer chromatography used for purifying compounds include: A: dichloromethane and isopropanol system, B: dichloromethane and methanol system, and C: petroleum ether and ethyl acetate system. The volume ratio of solvents is regulated according to different polarities of the compound, and can also be regulated by adding a small amount of triethylamine and acidic or alkaline reagent.

Some of the compounds of the present disclosure are characterized by TOF-LC/MS. TOF-LC/MS analysis is performed using an Agilent 6230 time-of-flight mass spectrometer and an Agilent 1290-Infinity ultra-high performance liquid chromatograph.

An exemplary preparation route for the present application is as follows:

**I. Preparation of payload and linker-payload**

**Preparation route 1**

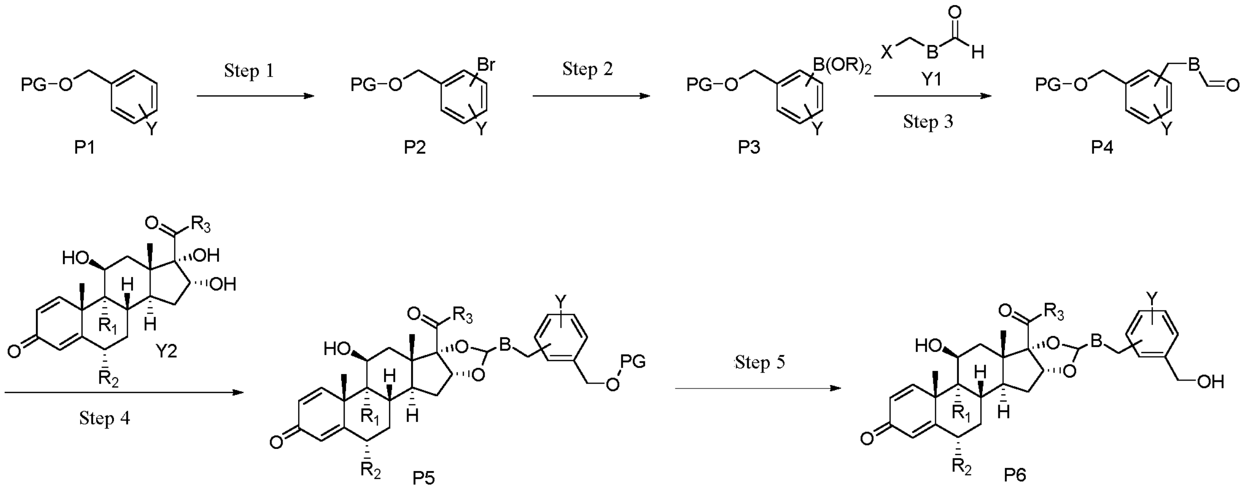


Step 1: introducing a bromine atom into a benzene ring of a compound of general formula (P 1) under optional bromination conditions;

Step 2: reacting a compound of general formula (P2) with a borate dimer under optional palladium reagent catalysis conditions to obtain a compound of general formula (P3);

Step 3: reacting the compound of general formula (P3) with a compound of general formula (Y1) under optional palladium reagent catalysis conditions to obtain a compound of general formula (P4);

Step 4: reacting the compound of general formula (P4) with a compound of general formula (Y2) under optional acidic or basic conditions to obtain a compound of general formula (P5); and

Step 5: removing the protecting group PG from the compound of general formula (P5) under optional acidic or basic conditions, followed by separation by preparative HPLC, to obtain a compound of general formula (P6).

In the above steps,

PG may be a common hydroxy protecting group;

Y is selected from the group consisting of: hydrogen, protium, deuterium, tritium, halogen, -OR, - SR, -NHR, $-N(R)_2$, -PHR, $-P(R)_2$, -P(=O)HR, $-P(=O)R_2$, optionally substituted alkyl, optionally substituted alkenyl, optionally substituted alkynyl, optionally substituted cycloalkyl, optionally substituted heterocycloalkyl, optionally substituted aryl, and optionally substituted heteroaryl;

ring B is optionally substituted aryl or heteroaryl; $-B(OR)_2$ is a boronate monomer in which two R may be linked to form a heterocycle, heterobridged ring or heterospiro ring which may be optionally substituted with $C_1$-$C_6$ alkyl, aryl, heteroaryl, carboxy or acyloxy $C_1$-$C_6$ alkyl;

X is chlorine, bromine or iodine;

wherein each R is independently selected from the group consisting of hydrogen, protium, deuterium, tritium, oxygen, hydroxy, halogen, $C_1$-$C_6$ alkyl, $C_1$-$C_6$ alkoxy, aryl, heteroaryl, cycloalkyl, heterocycloalkyl, halogenated $C_1$-$C_6$ alkyl, and halogenated $C_1$-$C_6$ alkoxy;

R, $R_1$, $R_2$ and $R_3$ may be defined as R, $R_1$, $R_2$ and $R_3$ in any one of formula (I), formula (IIa), formula (IIb), formula (IIIa), formula (IIIb), formula (IVa) or formula (IVb) of the present application.

**Preparation route 2**

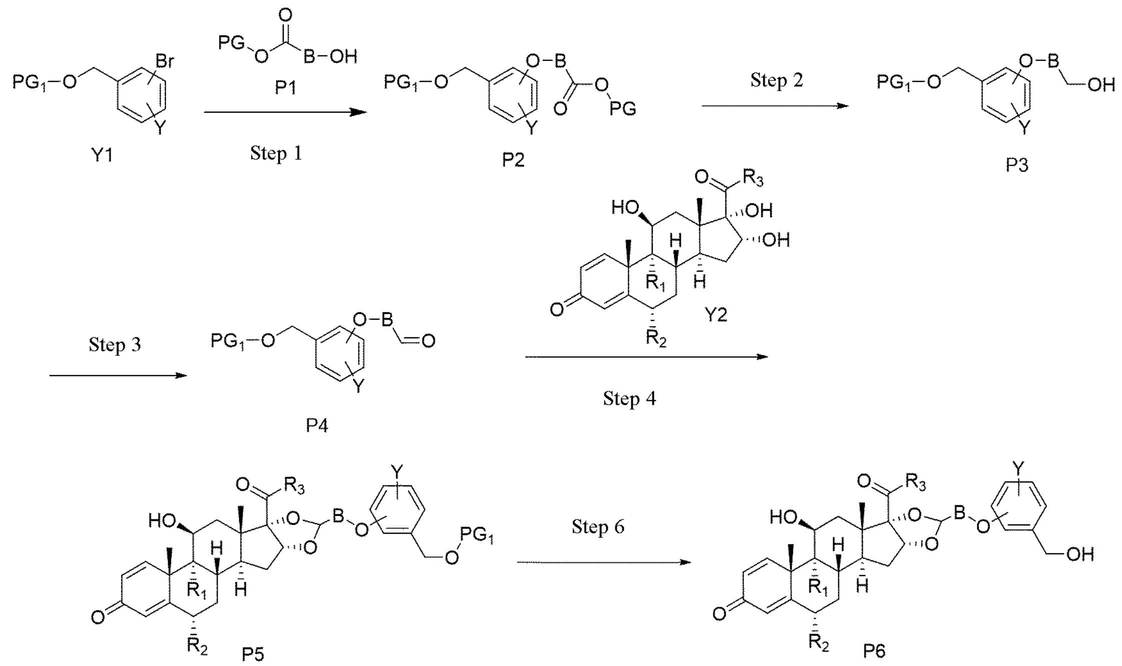


Step 1: reacting a compound of general formula (Y1) with a compound of general formula (P1) under optional metallic copper salt catalysis conditions and optional ligand and under optional basic conditions to obtain a compound of general formula (P2);
Step 2: reacting the compound of general formula (P2) under optional reducing conditions to obtain a compound of general formula (P3);
Step 3: reacting the compound of general formula (P3) under optional oxidation conditions to obtain a compound of general formula (P4);
Step 4: reacting the compound of general formula (P4) with a compound of general formula (Y2) under optional acidic or basic conditions to obtain a compound of general formula (P5); and Step 5: removing the protecting group PG from the compound of general formula (P5) under optional acidic or basic conditions, followed by separation by preparative HPLC, to obtain a compound of general formula (P6).

In the above steps,

PG may be a common hydroxy protecting group;
Y is selected from the group consisting of: hydrogen, protium, deuterium, tritium, halogen, -OR, - SR, -NHR, $-N(R)_2$, -PHR, $-P(R)_2$, -P(=O)HR, $-P(=O)R_2$, optionally substituted alkyl, optionally substituted alkenyl, optionally substituted alkynyl, optionally substituted cycloalkyl, optionally substituted heterocycloalkyl, optionally substituted aryl, and optionally substituted heteroaryl;
ring B is optionally substituted aryl or heteroaryl;
$R_1$, $R_2$ and $R_3$ may be defined as $R_1$, $R_2$ and $R_3$ in any one of formula (I), formula (IIa), formula (IIb), formula (IIIa), formula (IIIb), formula (IVa) or formula (IVb) of the present application.

Preparation route 3

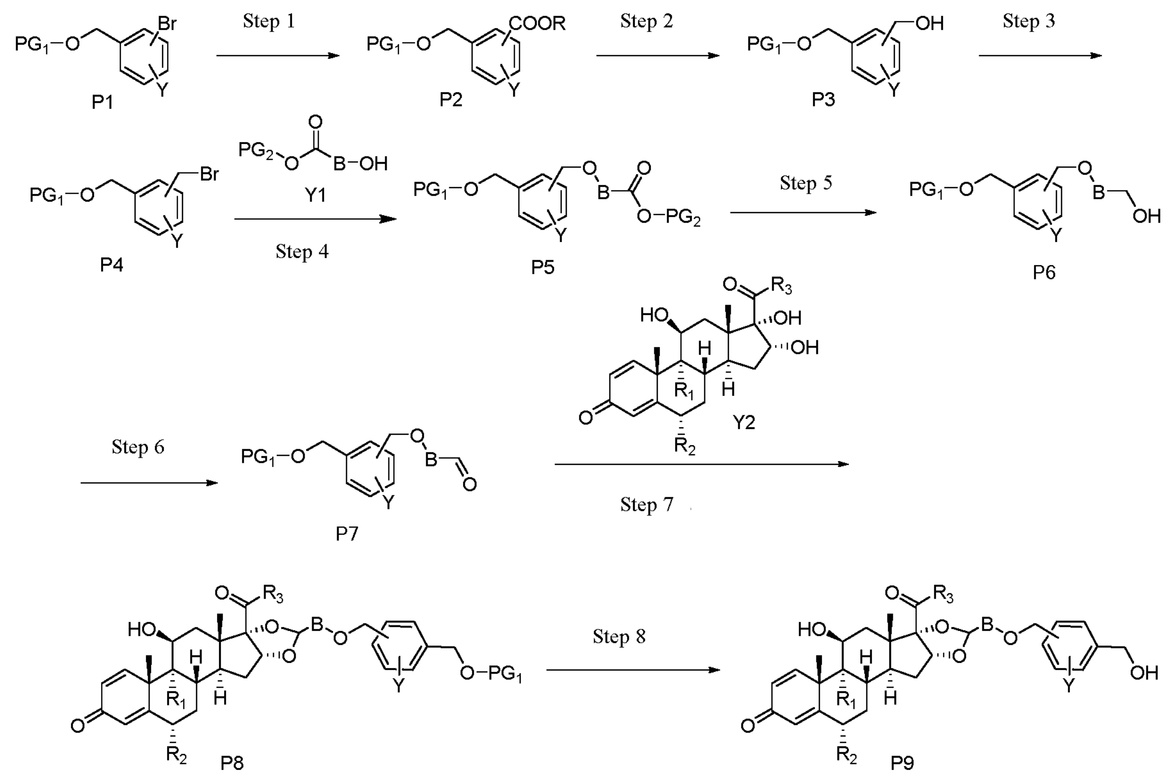


Step 1: subjecting a compound of general formula (P1) to a carbonyl insertion reaction under optional palladium reagent catalysis conditions to obtain a compound of general formula (P2);
Step 2: reacting the compound of general formula (P2) under optional reducing conditions to obtain a compound of general formula (P3);
Step 3: reacting the compound of general formula (P3) under optional bromination conditions to obtain a compound of general formula (P4);
Step 4: reacting the compound of general formula (P4) with a compound of general formula (Y1) under optional basic conditions to obtain a compound of general formula (P5);
Step 5: reacting the compound of general formula (P5) under optional reducing conditions to obtain a compound of general formula (P6);
Step 6: reacting the compound of general formula (P6) under optional oxidation conditions to obtain a compound of general formula (P7);
Step 7: reacting the compound of general formula (P7) with a compound of general formula (Y2) under optional acidic or basic conditions to obtain a compound of general formula (P8); and Step 8: removing the protecting group PG from the compound of general formula (P8) under optional acidic or basic conditions, followed by separation by preparative HPLC, to obtain a compound of general formula (P9).

In the above steps,

$PG_1$ and $PG_2$ may be common protecting groups for hydroxy or ester groups;
Y is selected from the group consisting of: hydrogen, protium, deuterium, tritium, halogen, -OR, - SR, -NHR, $-N(R)_2$, -PHR, $-P(R)_2$, -P(=O)HR, $-P(=O)R_2$, optionally substituted alkyl, optionally substituted alkenyl, optionally substituted alkynyl, optionally substituted cycloalkyl, optionally substituted heterocycloalkyl, optionally substituted aryl and optionally substituted heteroaryl; wherein, each R is independently selected from the group consisting of hydrogen, protium, deuterium, tritium, oxygen, hydroxy, halogen, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy;
R may be optionally substituted $C_1$-$C_6$ alkyl;
ring B is optionally substituted aryl or heteroaryl;
R, $R_1$, $R_2$ and $R_3$ may be defined as R, $R_1$, $R_2$ and $R_3$ in any one of formula (I), formula (IIa), formula (IIb), formula (IIIa), formula (IIIb), formula (IVa) or formula (IVb) of the present application.

**Preparation route 4**

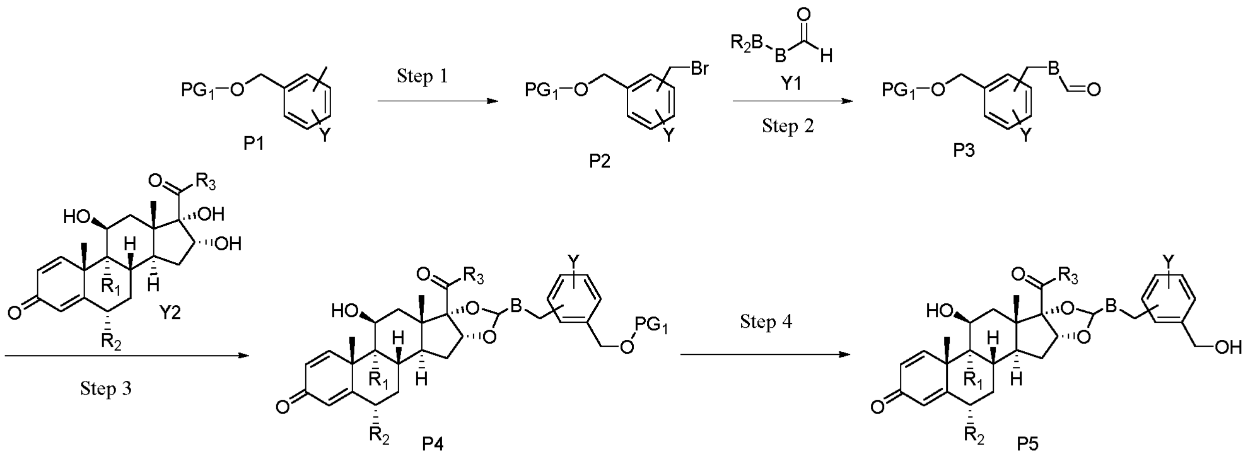


P1 P2 Y1 P3 Y2 P4 P5

Step 1: introducing a bromine atom into methyl of a compound of general formula (P1) under optional bromination conditions to obtain a compound of general formula (P2);
Step 2: reacting the compound of general formula (P2) with a compound of general formula (Y1) under optional palladium reagent catalysis conditions to obtain a compound of general formula (P3); Step 3: reacting the compound of general formula (P3) with a compound of general formula (Y2) under optional acidic or basic conditions to obtain a compound of general formula (P4); and Step 4: removing the protecting group PG from the compound of general formula (P4) under optional acidic or basic conditions, followed by separation by preparative HPLC, to obtain a compound of general formula (P5).

In the above steps,

$PG_1$ may be a common hydroxy protecting group;
Y is selected from the group consisting of: hydrogen, protium, deuterium, tritium, halogen, -OR, - SR, -NHR, $-N(R)_2$, -PHR, $-P(R)_2$, -P(=O)HR, $-P(=O)R_2$, optionally substituted alkyl, optionally substituted alkenyl, optionally substituted alkynyl, optionally substituted cycloalkyl, optionally substituted heterocycloalkyl, optionally substituted aryl and optionally substituted heteroaryl; wherein, each R is independently selected from the group consisting of hydrogen, protium, deuterium, tritium, oxygen, hydroxy, halogen, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy;
ring B is optionally substituted aryl or heteroaryl;
$R_1$, $R_2$ and $R_3$ may be defined as $R_1$, $R_2$ and $R_3$ in any one of formula (I), formula (IIa), formula (IIb), formula (IIIa), formula (IIIb), formula (IVa) or formula (IVb) of the present application.

**Preparation route 5**

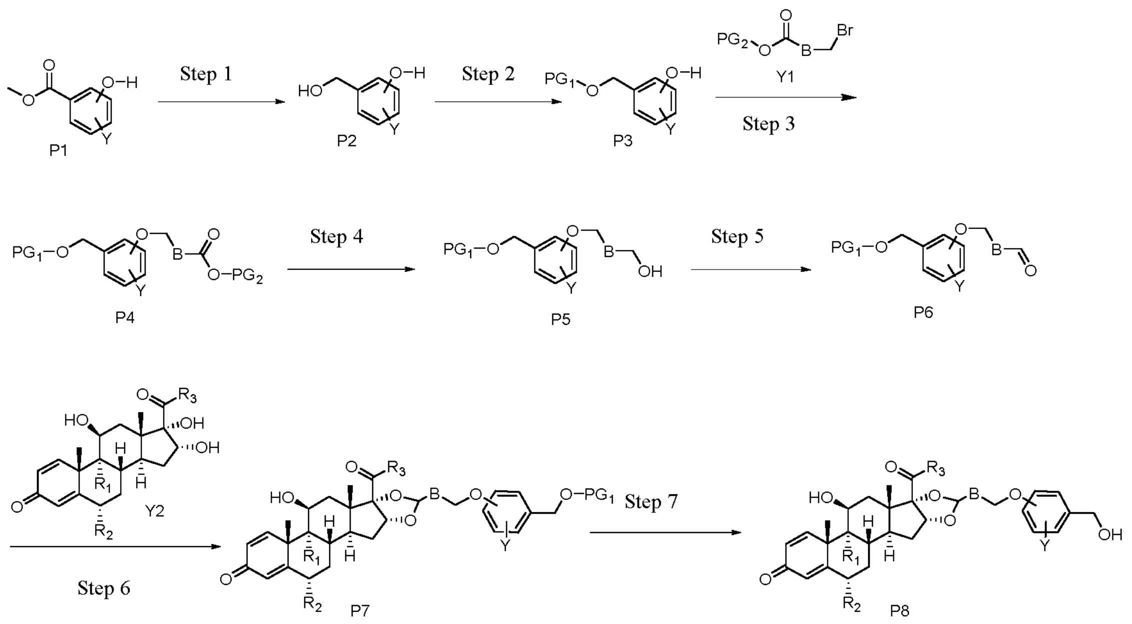


Step 1: reacting a compound of general formula (P1) under optional reducing conditions to obtain a compound of general formula (P2);
Step 2: adding a protecting group PG onto the compound of general formula (P2) under optional acidic or basic conditions to obtain a compound of general formula (P3);
Step 3: reacting the compound of general formula (P3) with a compound of general formula (Y1) under optional basic conditions to obtain a compound of general formula (P4);
Step 4: reacting a compound of general formula (P4) under optional reducing conditions to obtain a compound of general formula (P5);
Step 5: reacting the compound of general formula (P5) under optional oxidation conditions to obtain a compound of general formula (P6);
Step 6: reacting the compound of general formula (P6) with a compound of general formula (Y2) under optional acidic or basic conditions to obtain a compound of general formula (P7); and Step 7: removing the protecting group PG from the compound of general formula (P7) under optional acidic or basic conditions, followed by separation by preparative HPLC, to obtain a compound of general formula (P8).

In the above steps,

Y is selected from the group consisting of: hydrogen, protium, deuterium, tritium, halogen, -OR, - SR, -NHR, $-N(R)_2$, -PHR, $-P(R)_2$, -P(=O)HR, $-P(=O)R_2$, optionally substituted alkyl, optionally substituted alkenyl, optionally substituted alkynyl, optionally substituted cycloalkyl, optionally substituted heterocycloalkyl, optionally substituted aryl and optionally substituted heteroaryl; wherein, each R is independently selected from the group consisting of hydrogen, protium, deuterium, tritium, oxygen, hydroxy, halogen, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy;
ring B is optionally substituted aryl or heteroaryl;
$PG_1$ and $PG_2$ may be common hydroxy or protecting groups of hydroxy;
$R_1$, $R_2$ and $R_3$ may be defined as $R_1$, $R_2$ and $R_3$ in any one of formula (I), formula (IIa), formula (IIb), formula (IIIa), formula (IIIb), formula (IVa) or formula (IVb) of the present application.

**Preparation route 6**

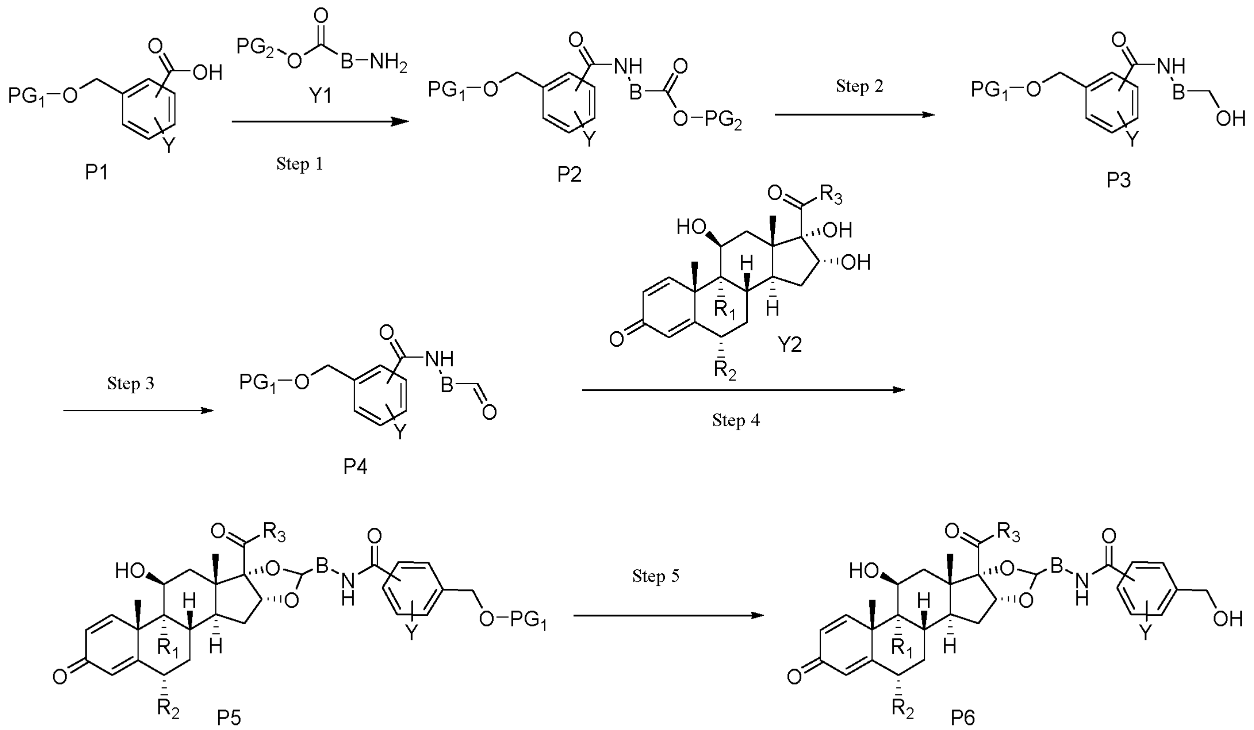


Step 1: reacting a compound of general formula (P1) with a compound of general formula (Y1) under optional condensation conditions to obtain a compound of general formula (P2);
Step 2: reacting the compound of general formula (P2) under optional reducing conditions to obtain a compound of general formula (P3);
Step 3: reacting the compound of general formula (P3) under optional oxidation conditions to obtain a compound of general formula (P4);
Step 4: reacting the compound of general formula (P4) with a compound of general formula (Y2) under optional acidic or basic conditions to obtain a compound of general formula (P5); and Step 5: reacting the compound of general formula (P5) under optional reducing conditions to obtain a compound of general formula (P6).

In the above steps,

$PG_1$ and $PG_2$ may be common protecting groups for hydroxy or ester groups;
Y is selected from the group consisting of: hydrogen, protium, deuterium, tritium, halogen, -OR, - SR, -NHR, $-N(R)_2$, -PHR, $-P(R)_2$, -P(=O)HR, $-P(=O)R_2$, optionally substituted alkyl, optionally substituted alkenyl, optionally substituted alkynyl, optionally substituted cycloalkyl, optionally substituted heterocycloalkyl, optionally substituted aryl and optionally substituted heteroaryl; wherein, each R is independently selected from the group consisting of hydrogen, protium, deuterium, tritium, oxygen, hydroxy, halogen, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy;
ring B is optionally substituted aryl or heteroaryl;
$R_1$, $R_2$ and $R_3$ may be defined as $R_1$, $R_2$ and $R_3$ in any one of formula (I), formula (IIa), formula (IIb), formula (IIIa), formula (IIIb), formula (IVa) or formula (IVb) of the present application.

**Preparation** route 7

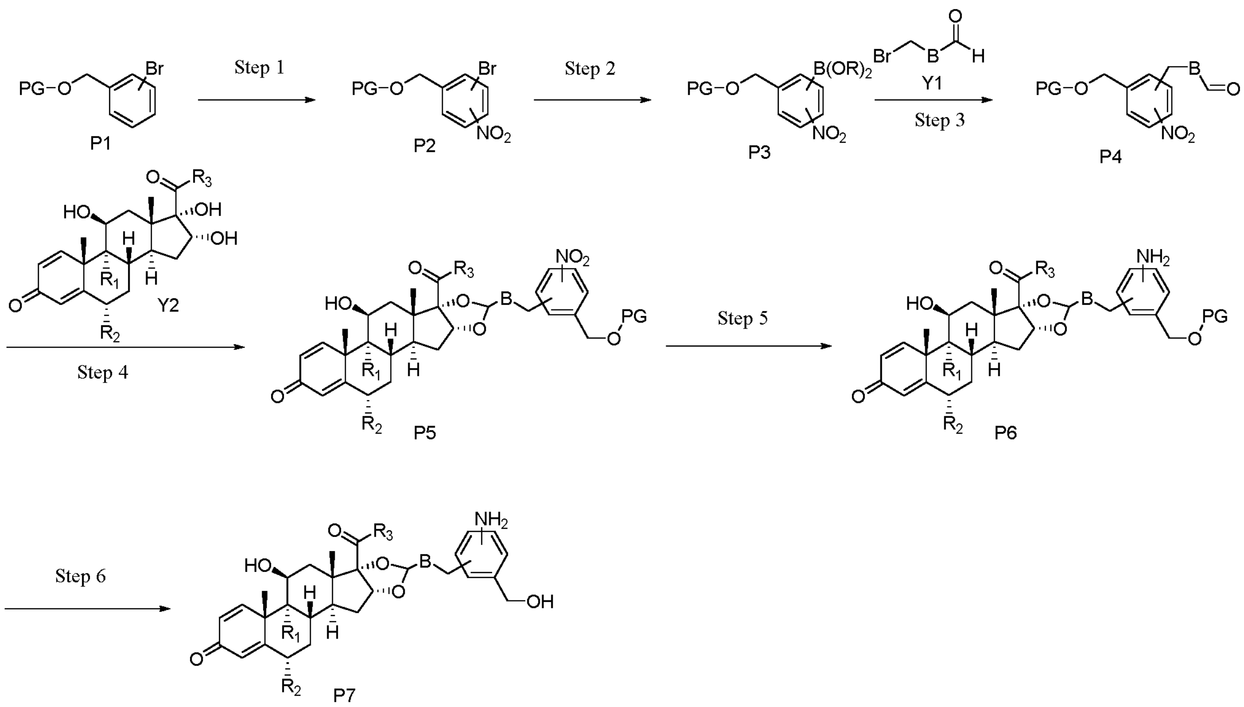


Step 1: introducing a nitro group into a benzene ring of a compound of general formula (P1) under nitration conditions to obtain a compound of general formula (P2);
Step 2: reacting a compound of general formula (P2) with a borate dimer under optional palladium reagent catalysis conditions to obtain a compound of general formula (P3);
Step 3: reacting the compound of general formula (P3) with a compound of general formula (Y1) under optional palladium reagent catalysis conditions to obtain a compound of general formula (P4);
Step 4: reacting the compound of general formula (P4) with a compound of general formula (Y2) under optional acidic or basic conditions to obtain a compound of general formula (P5); and
Step 5: reacting the compound of general formula (P5) under optional hydrogenation conditions to obtain a compound of general formula (P6); and
Step 6: removing the protecting group PG from the compound of general formula (P6) under optional acidic or basic conditions, followed by separation by preparative HPLC, to obtain a compound of general formula (P7).

In the above steps,

PG may be a common hydroxy protecting group;
$-B(OR)_2$ is a boronate monomer in which two R may be linked to form a heterocycle, heterobridged ring or heterospiro ring which may be optionally substituted with $C_1$-$C_6$ alkyl, aryl, heteroaryl, carboxy or acyloxy $C_1$-$C_6$ alkyl;
wherein each R is independently selected from the group consisting of hydrogen, protium, deuterium, tritium, oxygen, hydroxy, halogen, $C_1$-$C_6$ alkyl, $C_1$-$C_6$ alkoxy, aryl, heteroaryl, cycloalkyl, heterocycloalkyl, halogenated $C_1$-$C_6$ alkyl, and halogenated $C_1$-$C_6$ alkoxy;
ring B is optionally substituted aryl or heteroaryl;
$R_1$, $R_2$ and $R_3$ may be defined as $R_1$, $R_2$ and $R_3$ in any one of formula (I), formula (IIa), formula (IIb), formula (IIIa), formula (IIIb), formula (IVa) or formula (IVb) of the present application.

**Preparation line 8**

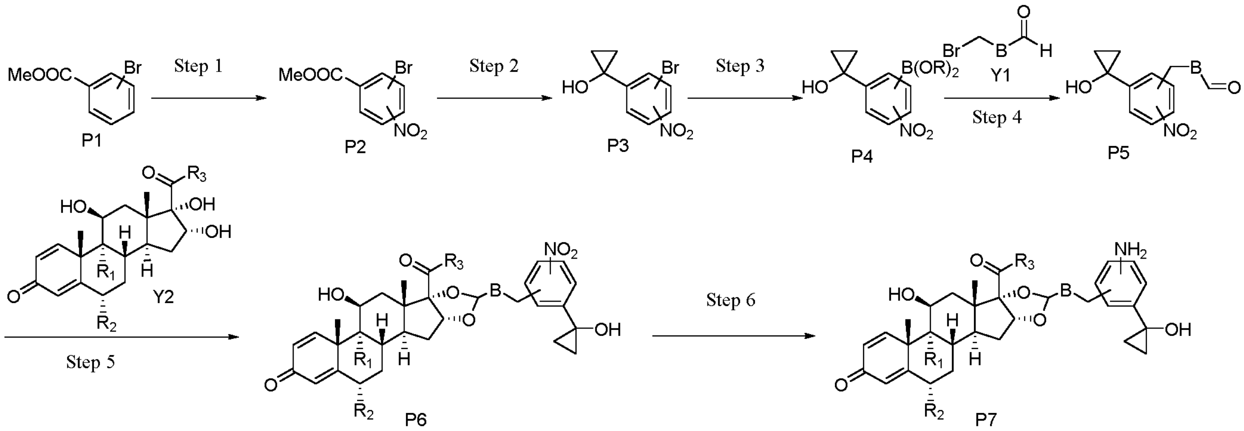


Step 1: introducing a nitro group into a benzene ring of a compound of general formula (P1) under nitration conditions to obtain a compound of general formula (P2);

Step 2: reacting the compound of general formula (P2) with an ethyl Grignard reagent under optional titanium catalysis conditions to obtain a compound of general formula (P3);

Step 3: reacting a compound of general formula (P2) with a borate dimer under optional palladium reagent catalysis conditions to obtain a compound of general formula (P3);

Step 4: reacting the compound of general formula (P3) with a compound of general formula (Y1) under optional palladium reagent catalysis conditions to obtain a compound of general formula (P4); Step 5: reacting the compound of general formula (P4) with a compound of general formula (Y2) under optional acidic or basic conditions to obtain a compound of general formula (P5); and

Step 6: reducing the nitro group of the compound of general formula (P5) under optional hydrogenation conditions, followed by separation by preparative HPLC, to obtain a compound of general formula (P6).

In the above steps,

ring B is optionally substituted aryl or heteroaryl; $-B(OR)_2$ is a boronate monomer in which two R may be linked to form a heterocycle, heterobridged ring or heterospiro ring which may be optionally substituted with $C_1$-$C_6$ alkyl, aryl, heteroaryl, carboxy or acyloxy $C_1$-$C_6$ alkyl;

wherein each R is independently selected from the group consisting of hydrogen, protium, deuterium, tritium, oxygen, hydroxy, halogen, $C_1$-$C_6$ alkyl, $C_1$-$C_6$ alkoxy, aryl, heteroaryl, cycloalkyl, heterocycloalkyl, halogenated $C_1$-$C_6$ alkyl, and halogenated $C_1$-$C_6$ alkoxy;

$R_1$, $R_2$ and $R_3$ may be defined as $R_1$, $R_2$ and $R_3$ in any one of formula (I), formula (IIa), formula (IIb), formula (IIIa), formula (IIIb), formula (IVa) or formula (IVb) of the present application.

**Preparation route 9**

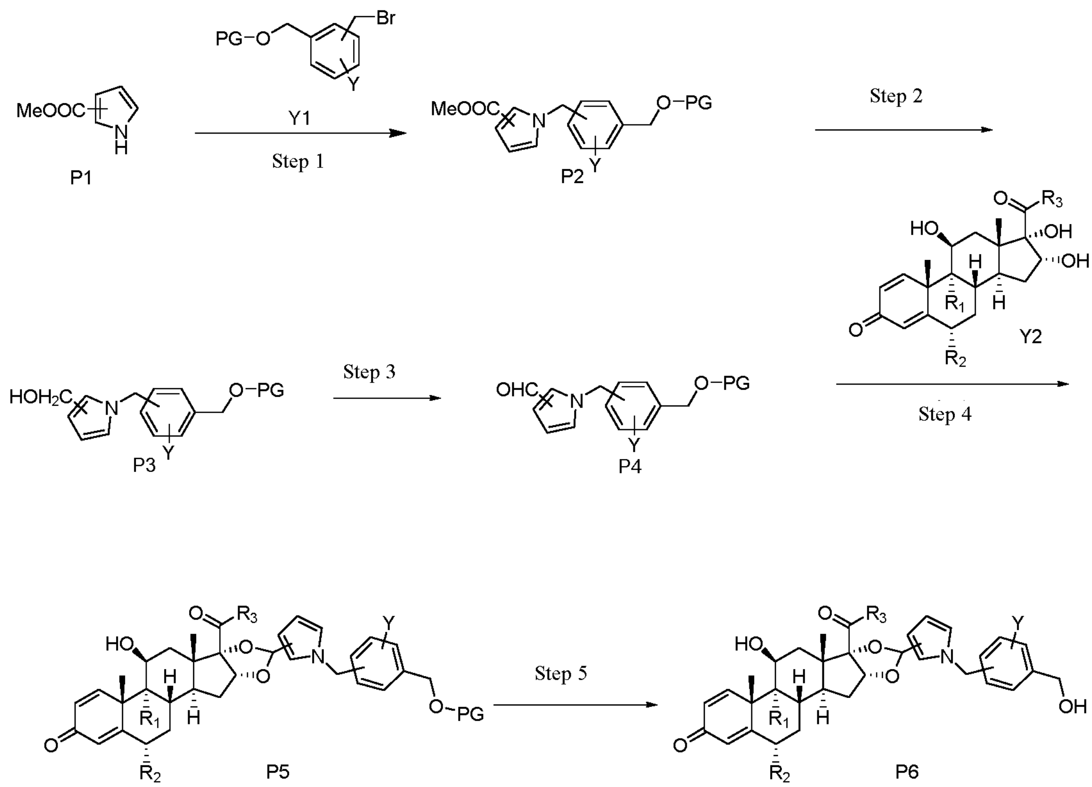


Step 1: reacting a compound of general formula (P1) with a compound of general formula (Y1) under optional basic conditions to obtain a compound of general formula (P2);
Step 2: reacting the compound of general formula (P2) under optional reducing conditions to obtain a compound of general formula (P3);
Step 3: reacting the compound of general formula (P3) under optional oxidation conditions to obtain a compound of general formula (P4);
Step 4: reacting the compound of general formula (P4) with a compound of general formula (Y2) under optional acidic or basic conditions to obtain a compound of general formula (P5); and
Step 5: removing the protecting group PG from the compound of general formula (P5) under optional acidic or basic conditions, followed by separation by preparative HPLC, to obtain a compound of general formula (P6).

In the above steps,

PG may be a common hydroxy protecting group;
Y is selected from the group consisting of: hydrogen, protium, deuterium, tritium, halogen, -OR, - SR, -NHR, $-N(R)_2$, -PHR, $-P(R)_2$, -P(=O)HR, $-P(=O)R_2$, optionally substituted alkyl, optionally substituted alkenyl, optionally substituted alkynyl, optionally substituted cycloalkyl, optionally substituted heterocycloalkyl, optionally substituted aryl and optionally substituted heteroaryl; wherein, each R is independently selected from the group consisting of hydrogen, protium, deuterium, tritium, oxygen, hydroxy, halogen, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy;
ring B is optionally substituted aryl or heteroaryl;
$R_1$, $R_2$ and $R_3$ may be defined as $R_1$, $R_2$ and $R_3$ in any one of formula (I), formula (IIa), formula (IIb), formula (IIIa), formula (IIIb), formula (IVa) or formula (IVb) of the present application.

**Preparation route 10**

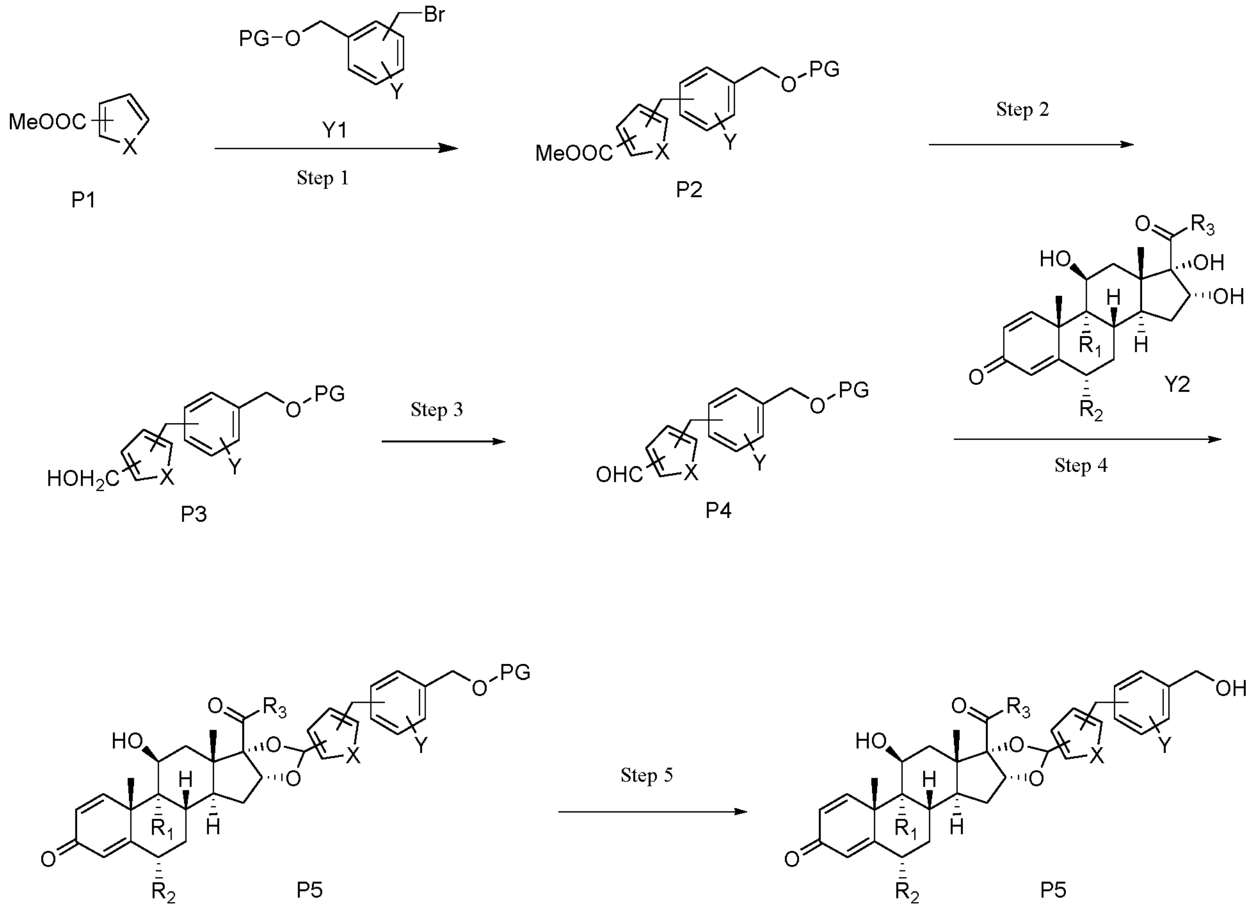


Step 1: reacting a compound of general formula (P1) with a compound of general formula (Y1) under optional palladium catalysis conditions to obtain a compound of general formula (P2);
Step 2: reacting the compound of general formula (P2) under optional reducing conditions to obtain a compound of general formula (P3);
Step 3: reacting the compound of general formula (P3) under optional oxidation conditions to obtain a compound of general formula (P4);
Step 4: reacting the compound of general formula (P4) with a compound of general formula (Y2) under optional acidic or basic conditions to obtain a compound of general formula (P5); and
Step 5: removing the protecting group PG from the compound of general formula (P5) under optional acidic or basic conditions, followed by separation by preparative HPLC, to obtain a compound of general formula (P6).

In the above steps,

X is N-R or -O-, and R is optionally substituted $C_1$-$C_6$ alkyl;
PG may be a common hydroxy protecting group;
Y is selected from the group consisting of: hydrogen, protium, deuterium, tritium, halogen, -OR, - SR, -NHR, -N(R)$_2$, -PHR, -P(R)$_2$, -P(=O)HR, -P(=O)R$_2$, optionally substituted alkyl, optionally substituted alkenyl, optionally substituted alkynyl, optionally substituted cycloalkyl, optionally substituted heterocycloalkyl, optionally substituted aryl and optionally substituted heteroaryl; wherein, each R is independently selected from the group consisting of hydrogen, protium, deuterium, tritium, oxygen, hydroxy, halogen, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy;
ring B is optionally substituted aryl or heteroaryl;
$R_1$, $R_2$ and $R_3$ may be defined as $R_1$, $R_2$ and $R_3$ in any one of formula (I), formula (IIa), formula (IIb), formula (IIIa), formula (IIIb), formula (IVa) or formula (IVb) of the present application.

**Preparation route 11**

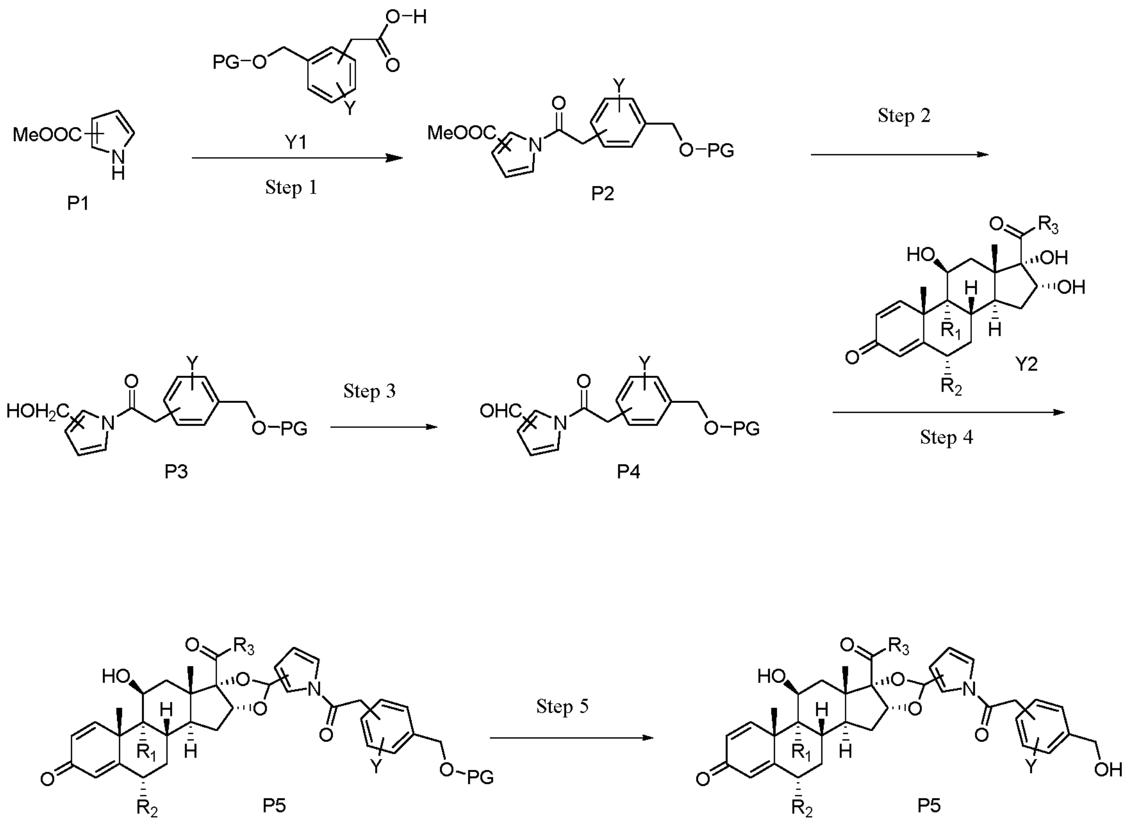


Step 1: reacting a compound of general formula (P1) with a compound of general formula (Y1) under optional condensation conditions to obtain a compound of general formula (P2);

Step 2: reacting the compound of general formula (P2) under optional reducing conditions to obtain a compound of general formula (P3);

Step 3: reacting the compound of general formula (P3) under optional oxidation conditions to obtain a compound of general formula (P4);

Step 4: reacting the compound of general formula (P4) with a compound of general formula (Y2) under optional acidic or basic conditions to obtain a compound of general formula (P5); and Step 5: reacting the compound of general formula (P5) under optional reducing conditions, followed by separation by preparative HPLC, to obtain a compound of general formula (P6).

In the above steps,

PG may be a common hydroxy protecting group;

Y is selected from the group consisting of: hydrogen, protium, deuterium, tritium, halogen, -OR, - SR, -NHR, $-N(R)_2$, -PHR, $-P(R)_2$, -P(=O)HR, $-P(=O)R_2$, optionally substituted alkyl, optionally substituted alkenyl, optionally substituted alkynyl, optionally substituted cycloalkyl, optionally substituted heterocycloalkyl, optionally substituted aryl and optionally substituted heteroaryl; wherein, each R is independently selected from the group consisting of hydrogen, protium, deuterium, tritium, oxygen, hydroxy, halogen, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy;

$R_1$, $R_2$ and $R_3$ may be defined as $R_1$, $R_2$ and $R_3$ in any one of formula (I), formula (IIa), formula (IIb), formula (IIIa), formula (IIIb), formula (IVa) or formula (IVb) of the present application.

Preparation route 12

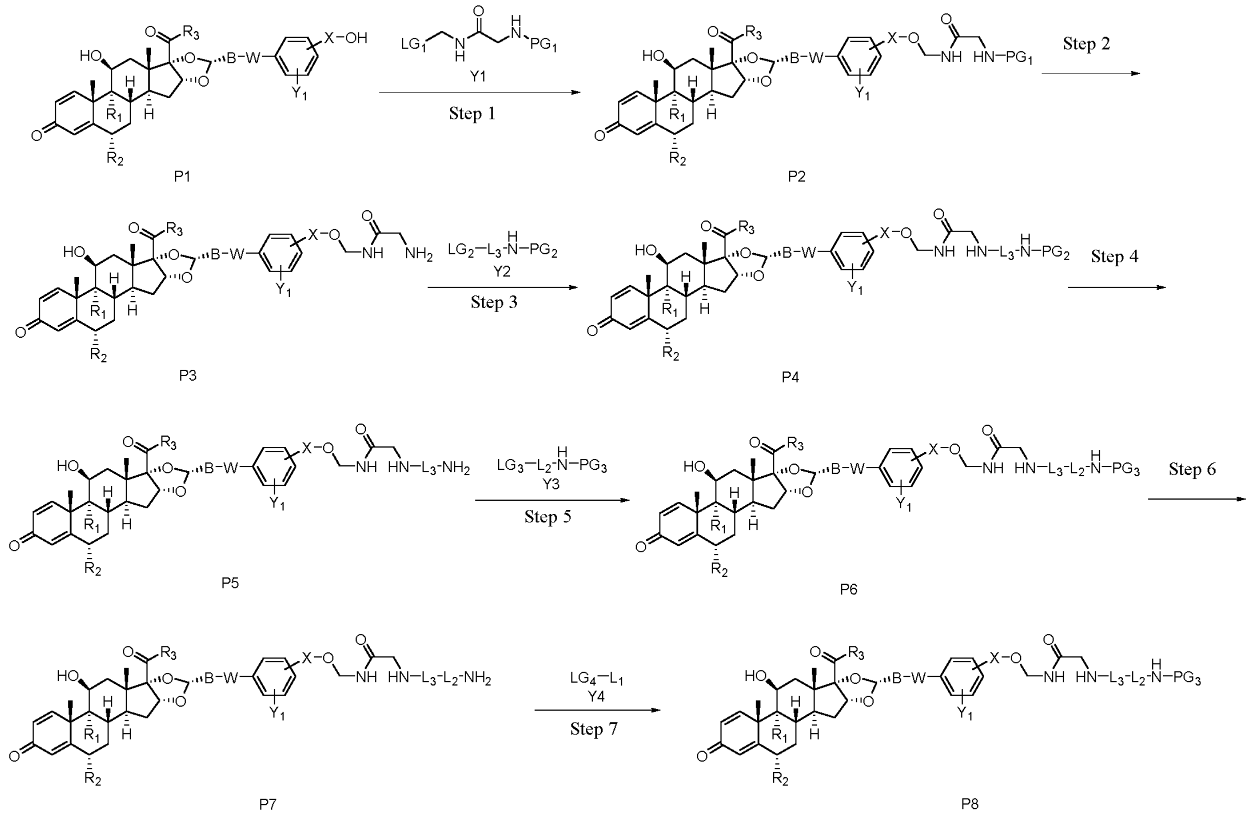


Step 1: reacting a compound of general formula (P1) with a compound of general formula (Y1) under optional condensation conditions to obtain a compound of general formula (P2);
Step 2: removing the protecting group $PG_1$ from the compound of general formula (P2) under optional acidic or basic conditions to obtain a compound of general formula (P3);
Step 3: reacting the compound of general formula (P3) with a compound of general formula (Y2) under optional condensation conditions to obtain a compound of general formula (P4);
Step 4: removing the protecting group $PG_1$ from the compound of general formula (P4) under optional acidic or basic conditions to obtain a compound of general formula (P5);
Step 5: reacting the compound of general formula (P5) with a compound of general formula (Y3) under optional condensation conditions to obtain a compound of general formula (P6);
Step 6: removing the protecting group $PG_1$ from the compound of general formula (P6) under optional acidic or basic conditions to obtain a compound of general formula (P7); and Step 7: reacting the compound of general formula (P7) with a compound of general formula (Y3) under optional condensation conditions, followed by separation by preparative HPLC, to obtain a compound of general formula (P8).

In the above steps,

ring B is optionally substituted aryl or heteroaryl;
W is absent or W is any group;
X is $-CH_2-$, $-CH(CH_3)-$, $-CH_2CH_2-$ or

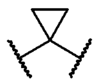
;

$Y_1$ is hydrogen, hydroxy, fluoro, chloro, methyl, hydroxy or methoxy;
$R_1$, $R_2$ and $R_3$ may be defined as $R_1$, $R_2$ and $R_3$ in any one of formula (I), formula (II), formula (III) or formula (IV) of the present application;
$LG_1$, $LG_2$, $LG_3$ and $LG_4$ may be common hydroxy of activated ester or carboxy;
$PG_1$, $PG_2$ and $PG_3$ may be common amino protecting groups;
$R_1$, $R_2$ and $R_3$ may be defined as $R_1$, $R_2$ and $R_3$ in any one of formula (I), formula (IIa), formula (IIb), formula (IIIa),

formula (IIIb), formula (IVa) or formula (IVb) of the present application.

Tr is absent or Tr is any group;

$L_3$ is selected from a polypeptide fragment;

$L_2$ is absent or selected from a linker fragment;

$L_1$ is selected from a coupling unit.

**Preparation route 13**

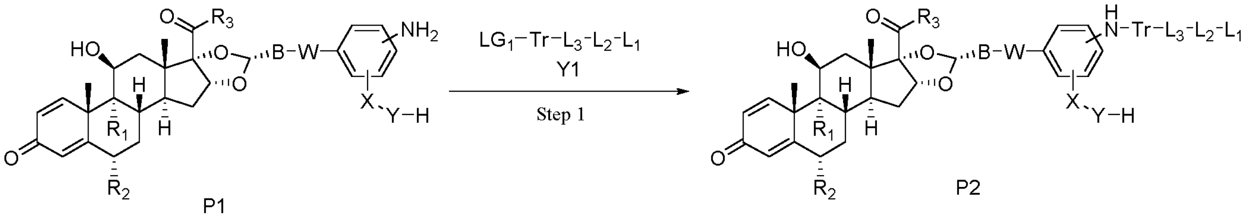


P1 P2

Step 1: reacting a compound of general formula (P1) with a compound of general formula (Y1) under optional condensation conditions, followed by separation by preparative HPLC, to obtain a compound of general formula (P2).

In the above step,

ring B is optionally substituted aryl or heteroaryl;

W is absent or W is any group;

X is $-CH_2-$, $-CH(CH_3)-$, $-CH_2CH_2-$ or

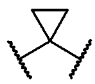;

Y is -O-, -S- or -NH-;

$R_1$, $R_2$ and $R_3$ may be defined as $R_1$, $R_2$ and $R_3$ in any one of formula (I), formula (IIa), formula (IIb), formula (IIIa), formula (IIIb), formula (IVa) or formula (IVb) of the present application.

$LG_1$ may be common hydroxy of activated ester or carboxy;

Tr is absent or Tr is any group;

$L_3$ is selected from a polypeptide fragment;

$L_2$ is absent or selected from a linker fragment;

$L_1$ is selected from a coupling unit.

**Preparation** route 14

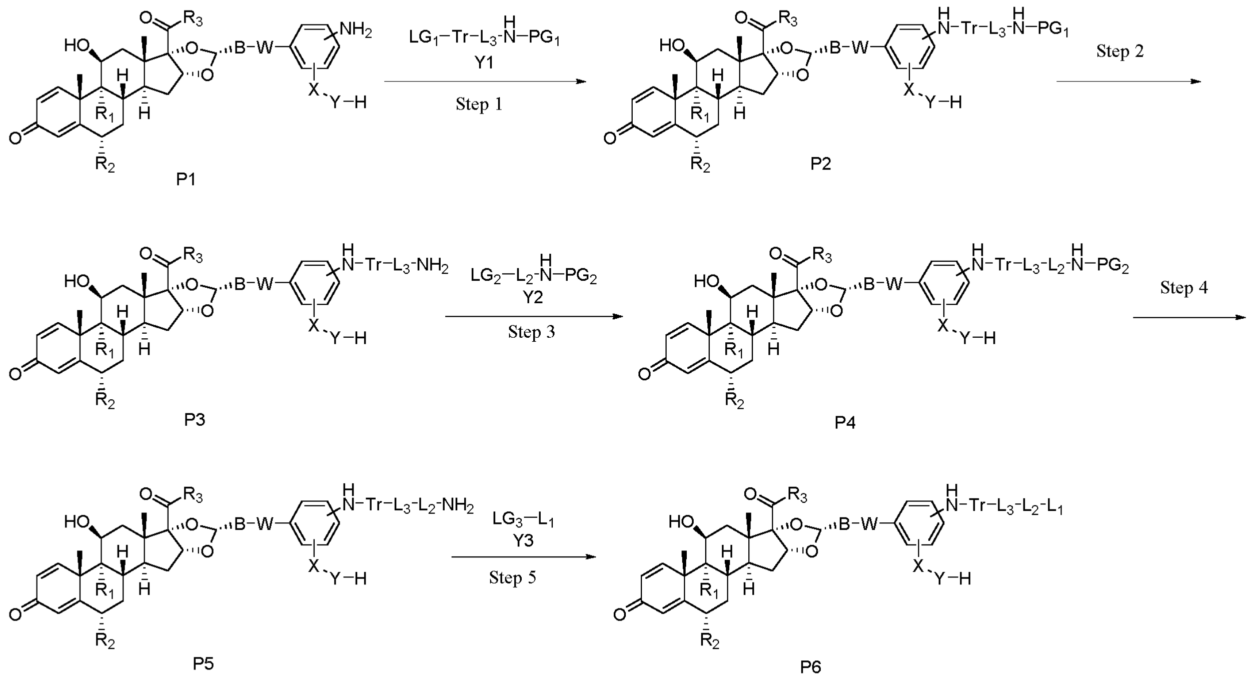


Step 1: reacting a compound of general formula (P1) with a compound of general formula (Y1) under optional condensation conditions to obtain a compound of general formula (P2);

Step 2: removing the protecting group $PG_1$ from the compound of general formula (P2) under optional acidic or basic conditions to obtain a compound of general formula (P3);

Step 3: reacting the compound of general formula (P3) with a compound of general formula (Y2) under optional condensation conditions to obtain a compound of general formula (P4);

Step 4: removing the protecting group $PG_1$ from the compound of general formula (P4) under optional acidic or basic conditions to obtain a compound of general formula (P5);

Step 5: reacting the compound of general formula (P5) with a compound of general formula (Y3) under optional condensation conditions, followed by separation by preparative HPLC, to obtain a compound of general formula (P6).

In the above steps,

ring B is optionally substituted aryl or heteroaryl;
W is absent or W is any group;
X is $-CH_2-$, $-CH(CH_3)-$, $-CH_2CH_2-$ or

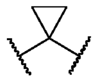;

Y is -O-, -S- or -NH-;
$LG_1$, $LG_2$ and $LG_3$ may be common hydroxy of activated ester or carboxy;
$PG_1$, $PG_2$ and $PG_3$ may be common amino protecting groups;
$R_1$, $R_2$ and $R_3$ may be defined as $R_1$, $R_2$ and $R_3$ in any one of formula (I), formula (IIa), formula (IIb), formula (IIIa), formula (IIIb), formula (IVa) or formula (IVb) of the present application.
Tr is absent or Tr is any group;
$L_3$ is selected from a polypeptide fragment;
$L_2$ is absent or selected from a linker fragment;
$L_1$ is selected from a coupling unit.

## II. Preparation of antibody-drug conjugates (ADCs)

**Preparation route 15**

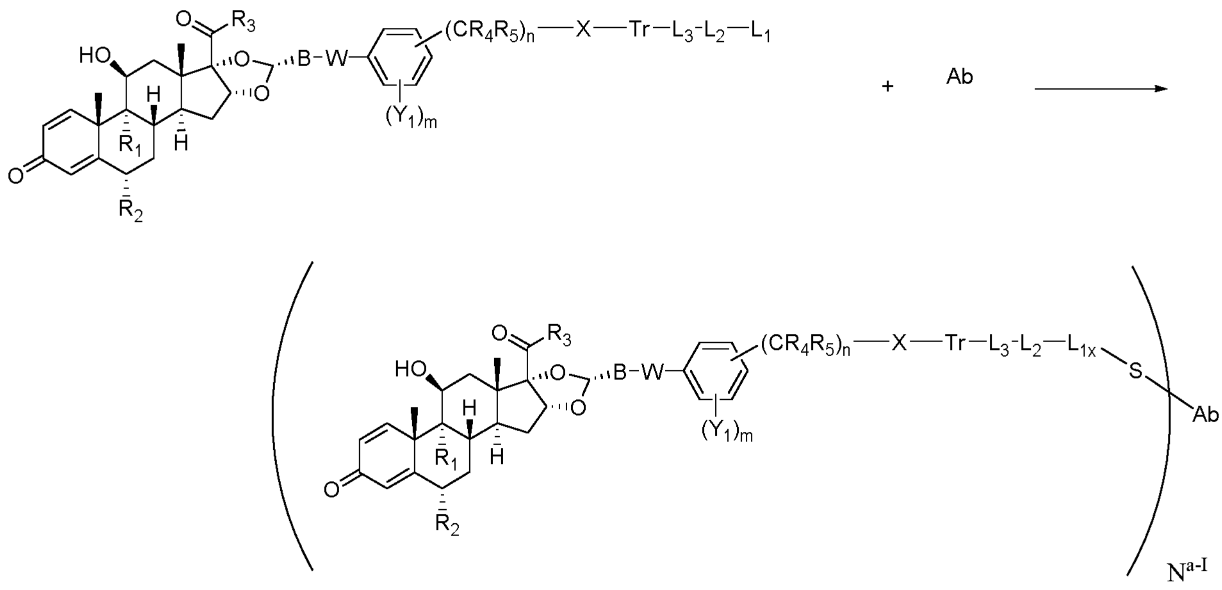

The ligand Ab was reacted with a compound of any one of formula (IVa) or formula (IVb) in an acidic, neutral or alkaline buffer to obtain a compound of formula (Va) or formula (Vb);

wherein, Ab is a ligand containing at least one free sulfhydryl (-SH), wherein the free sulfhydryl may be obtained by reducing the ligand by using a reducing agent; the reducing agent includes, but is not limited to, tris(2-carboxyethyl)phosphine, mercaptoethanol, dithiothreitol, cysteine, reduced glutathione, and the like; particularly, disulfide bonds (-S-S-) between ligand chains may be reduced to form free sulfhydryl; wherein the S atom in the compound of formula (I-C) or formula (II-C) may be sulfhydryl derived from Ab;

Tr, $L_1$, $L_2$ and $L_3$ may be defined as Tr, $L_1$, $L_2$ and $L_3$ in any one of the compounds of formula (IVa) or formula (IVb) of the present application; Ab and $N^{a-I}$ may be defined as Ab and $N^{a-I}$ in any one of the compounds of formula (Va) or formula (Vb) of the present application; $L_{1x}$ represents the structure after coupling to $L_1$ coupled to sulfhydryl;

the buffer is selected from the group consisting of the following buffers with a pH of 2 to 12: citric acid-sodium citrate buffer, phosphoric acid-sodium phosphate buffer, phosphoric acid-potassium phosphate buffer, sodium dihydrogen phosphate-disodium hydrogen phosphate buffer, potassium dihydrogen phosphate-dipotassium hydrogen phosphate buffer, succinic acid-sodium succinate buffer, acetic acid-sodium acetate buffer, boric acid-borax buffer, boric acid-potassium borate buffer, borax-sodium hydroxide buffer, histidine-hydrochloric acid buffer, glycine-sodium hydroxide buffer, arginine-hydrochloric acid buffer, sodium bicarbonate-sodium carbonate buffer, potassium bicarbonate-potassium carbonate buffer, Tris-hydrochloric acid buffer, aqueous ammonia-ammonium chloride buffer, barbiturate sodium-hydrochloric acid buffer, borax-sodium carbonate buffer, boric acid-potassium chloride buffer, and a combination of two or more of the above.

Preparation route 16

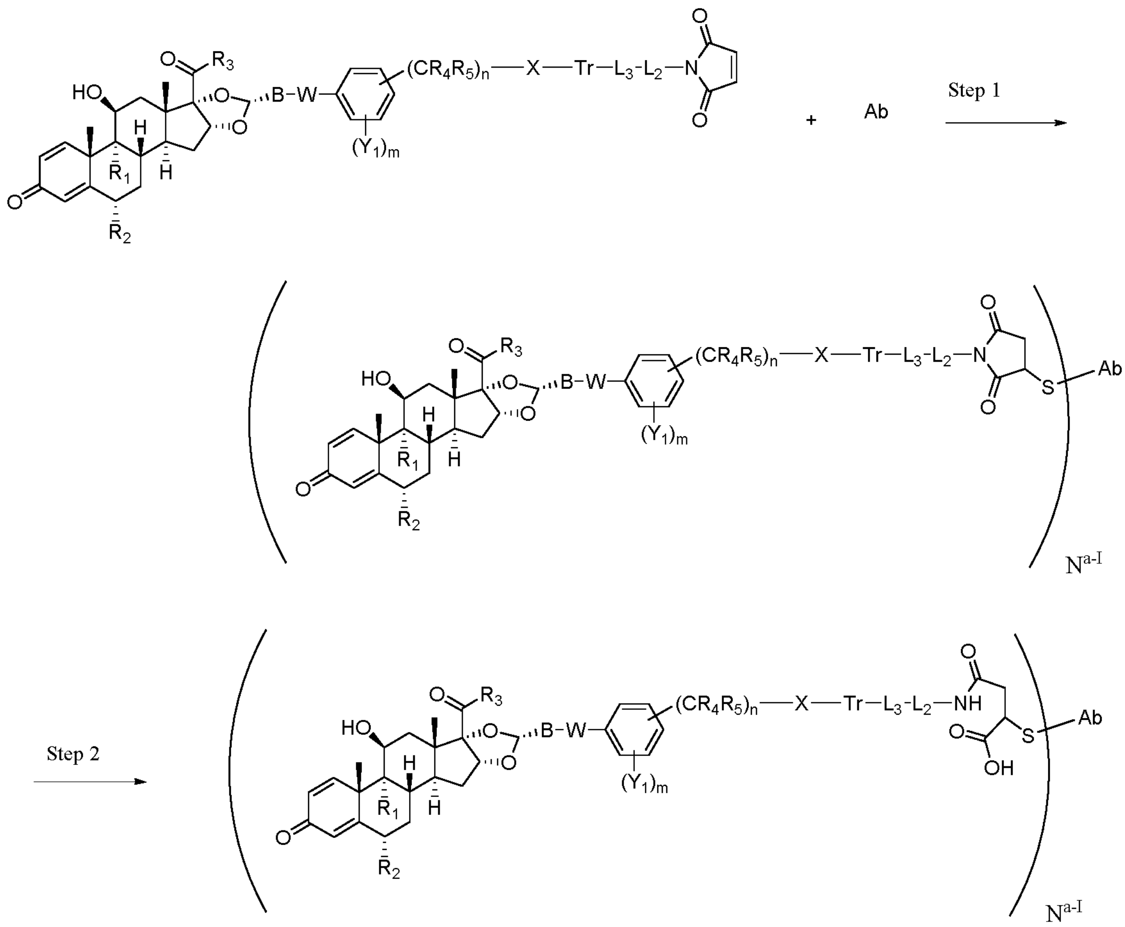


In Step 1, the ligand Ab was reacted with a compound of any one of formula (IVa) or formula (IVb) in an acidic, neutral or alkaline buffer to obtain a compound of formula (Va) or formula (Vb); wherein, Ab is a ligand containing at least one free sulfhydryl (-SH), wherein the free sulfhydryl may be obtained by reducing the ligand by using a reducing agent; the reducing agent includes, but is not limited to, tris(2-carboxyethyl)phosphine, mercaptoethanol, dithiothreitol, cysteine, reduced glutathione, and the like; particularly, disulfide bonds (-S-S-) between ligand chains may be reduced to form free sulfhydryl; wherein the S atom in the compound of formula (Va) or formula (Vb) may be sulfhydryl derived from Ab;

in Step 2, the compound of any one of formula (Va) or formula (Vb) was incubated in an alkaline buffer at a selected temperature for a selected time to obtain another compound of any one of formula (Va) or formula (Vb) of the present application;

Tr, $L_2$ and $L_3$ may be defined as Tr, $L_2$ and $L_3$ in any one of the compounds of formula (IVa) or formula (IVb) of the present application; Ab and $N^{a-I}$ may be defined as Ab and $N^{a-I}$ in any one of the compounds of formula (Va) or formula (Vb) of the present application;

the buffer is selected from the group consisting of the following buffers with a pH of 2 to 12: citric acid-sodium citrate buffer, phosphoric acid-sodium phosphate buffer, phosphoric acid-potassium phosphate buffer, sodium dihydrogen phosphate-disodium hydrogen phosphate buffer, potassium dihydrogen phosphate-dipotassium hydrogen phosphate buffer, succinic acid-sodium succinate buffer, acetic acid-sodium acetate buffer, boric acid-borax buffer, boric acid-potassium borate buffer, borax-sodium hydroxide buffer, histidine-hydrochloric acid buffer, glycine-sodium hydroxide buffer, arginine-hydrochloric acid buffer, sodium bicarbonate-sodium carbonate buffer, potassium bicarbonate-potassium carbonate buffer, Tris-hydrochloric acid buffer, aqueous ammonia-ammonium chloride buffer, barbiturate sodium-hydrochloric acid buffer, borax-sodium carbonate buffer, boric acid-potassium chloride buffer, and a combination of two or more of the above; the selected temperature may be 5 °C to 60 °C; the incubation time may be 0 h to 72 h.

**Preparation route 17**

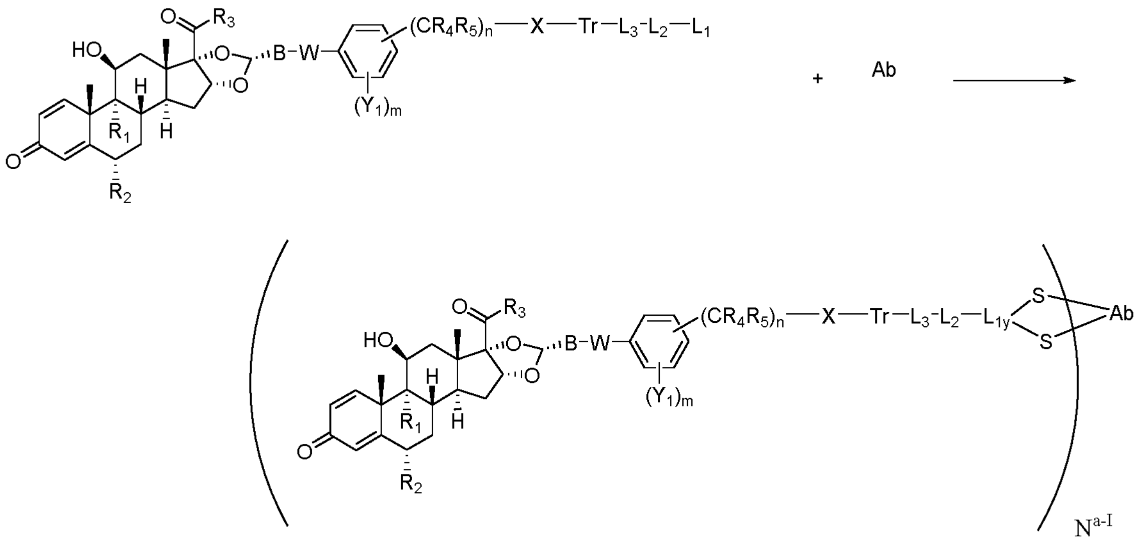


The ligand Ab was reacted with a compound of any one of formula (IVa) or formula (IVb) having a group capable of being coupled to two sulfydryl in an acidic, neutral or alkaline buffer to obtain a compound of formula (Va) or formula (Vb);

wherein, Ab is a ligand containing at least two free sulfhydryl (-SH), wherein the free sulfhydryl may be obtained by reducing the ligand by using a reducing agent; the reducing agent includes, but is not limited to, tris(2-carboxyethyl)phosphine, mercaptoethanol, dithiothreitol, cysteine, reduced glutathione, and the like; particularly, disulfide bonds (-S-S-) between ligand chains may be reduced to form free sulfhydryl; wherein the S atom in the compound of formula (Va) or formula (Vb) may be sulfhydryl derived from Ab;

Tr, $L_1$, $L_2$ and $L_3$ may be defined as Tr, $L_1$, $L_2$ and $L_3$ in any one of the compounds of formula (IVa) or formula (IVb) of the present application; Ab and $N^{a-I}$ may be defined as Ab and $N^{a-I}$ in any one of the compounds of formula (Va) or formula (Vb) of the present application; $L_{1y}$ represents the structure after coupling to $L_1$ coupled to two sulfhydryl; the compound of formula (Va) or formula (Vb) having a group capable of being coupled to two sulfhydryl may comprise an $L_{1y}$ group selected from the group consisting of the following:

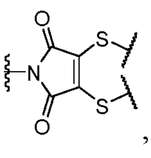 , 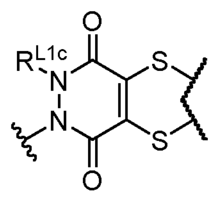 , 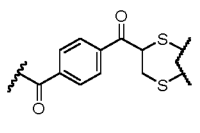 and 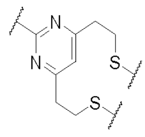 ,

wherein each of $R^{L1a}$, $R^{L1b}$ and $R^{L1c}$ is independently selected from the group consisting of the following: hydrogen, protium, deuterium, tritium, halogen, $-NO_2$, -CN, -OH, -SH, $-NH_2$, -C(O)H, $-CO_2H$, -C(O)C(O)H, $-C(O)CH_2C(O)H$, -S(O)H, $-S(O)_2H$, $-C(O)NH_2$, $-SO_2NH_2$, -OC(O)H, $-N(H)SO_2H$, alkyl, alkenyl, alkynyl, alcyl, heterocyclyl, aryl, and heteroaryl;

the buffer is selected from the group consisting of the following buffers with a pH of 2 to 12: citric acid-sodium citrate buffer, phosphoric acid-sodium phosphate buffer, phosphoric acid-potassium phosphate buffer, sodium dihydrogen phosphate-disodium hydrogen phosphate buffer, potassium dihydrogen phosphate-dipotassium hydrogen phosphate buffer, succinic acid-sodium succinate buffer, acetic acid-sodium acetate buffer, boric acid-borax buffer, boric acid-potassium borate buffer, borax-sodium hydroxide buffer, histidine-hydrochloric acid buffer, glycine-sodium hydroxide buffer, arginine-hydrochloric acid buffer, sodium bicarbonate-sodium carbonate buffer, potassium bicarbonate-potassium carbonate buffer, Tris-hydrochloric acid buffer, aqueous ammonia-ammonium chloride buffer, barbiturate sodium-hydrochloric acid buffer, borax-sodium carbonate buffer, boric acid-potassium chloride buffer, and a combination of two or more of the above.

**Preparation route 18**

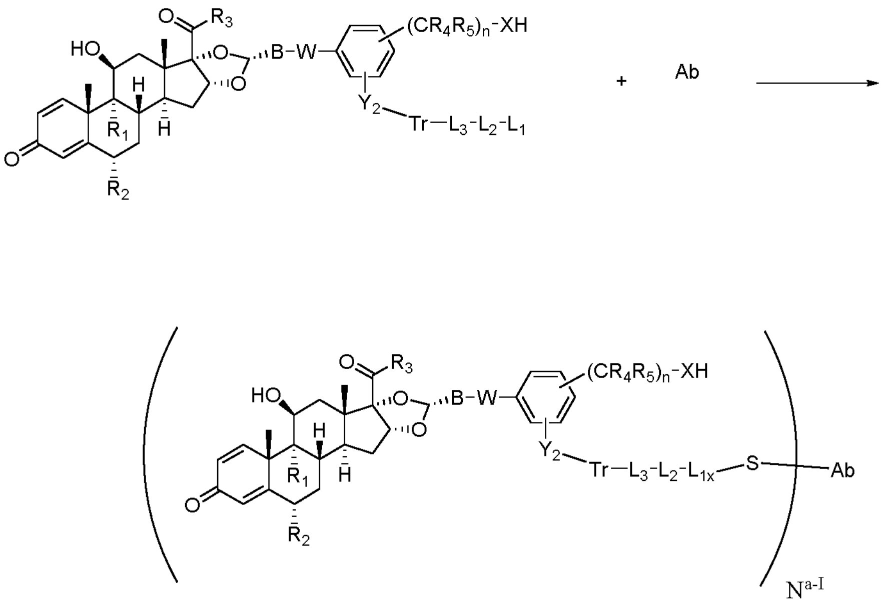

The ligand Ab was reacted with a compound of any one of formula (IVa) or formula (IVb) having a group capable of being coupled to two sulfydryl in an acidic, neutral or alkaline buffer to obtain a compound of formula (Va) or formula (Vb);

wherein, Ab is a ligand containing at least one free sulfhydryl (-SH), wherein the free sulfhydryl may be obtained by reducing the ligand by using a reducing agent; the reducing agent includes, but is not limited to, tris(2-carboxyethyl)phosphine, mercaptoethanol, dithiothreitol, cysteine, reduced glutathione, and the like; particularly, disulfide bonds (-S-S-) between ligand chains may be reduced to form free sulfhydryl; wherein the S atom in the compound of formula (Va) or formula (Vb) may be sulfhydryl derived from Ab;

Tr, $L_1$, $L_2$ and $L_3$ may be defined as Tr, $L_1$, $L_2$ and $L_3$ in any one of the compounds of formula (IVa) or formula (IVb) of the present application; Ab and $N^{a-I}$ may be defined as Ab and $N^{a-I}$ in any one of the compounds of formula (Va) or formula (Vb) of the present application; $L_{1x}$ represents the structure after coupling to $L_1$ coupled to sulfhydryl;

the buffer is selected from the group consisting of the following buffers with a pH of 2 to 12: citric acid-sodium citrate buffer, phosphoric acid-sodium phosphate buffer, phosphoric acid-potassium phosphate buffer, sodium dihydrogen phosphate-disodium hydrogen phosphate buffer, potassium dihydrogen phosphate-dipotassium hydrogen phosphate buffer, succinic acid-sodium succinate buffer, acetic acid-sodium acetate buffer, boric acid-borax buffer, boric acid-potassium borate buffer, borax-sodium hydroxide buffer, histidine-hydrochloric acid buffer, glycine-sodium hydroxide buffer, arginine-hydrochloric acid buffer, sodium bicarbonate-sodium carbonate buffer, potassium bicarbonate-potassium carbonate buffer, Tris-hydrochloric acid buffer, aqueous ammonia-ammonium chloride buffer, barbiturate sodium-hydrochloric acid buffer, borax-sodium carbonate buffer, boric acid-potassium chloride buffer, and a combination of two or more of the above.

**Preparation route 19**

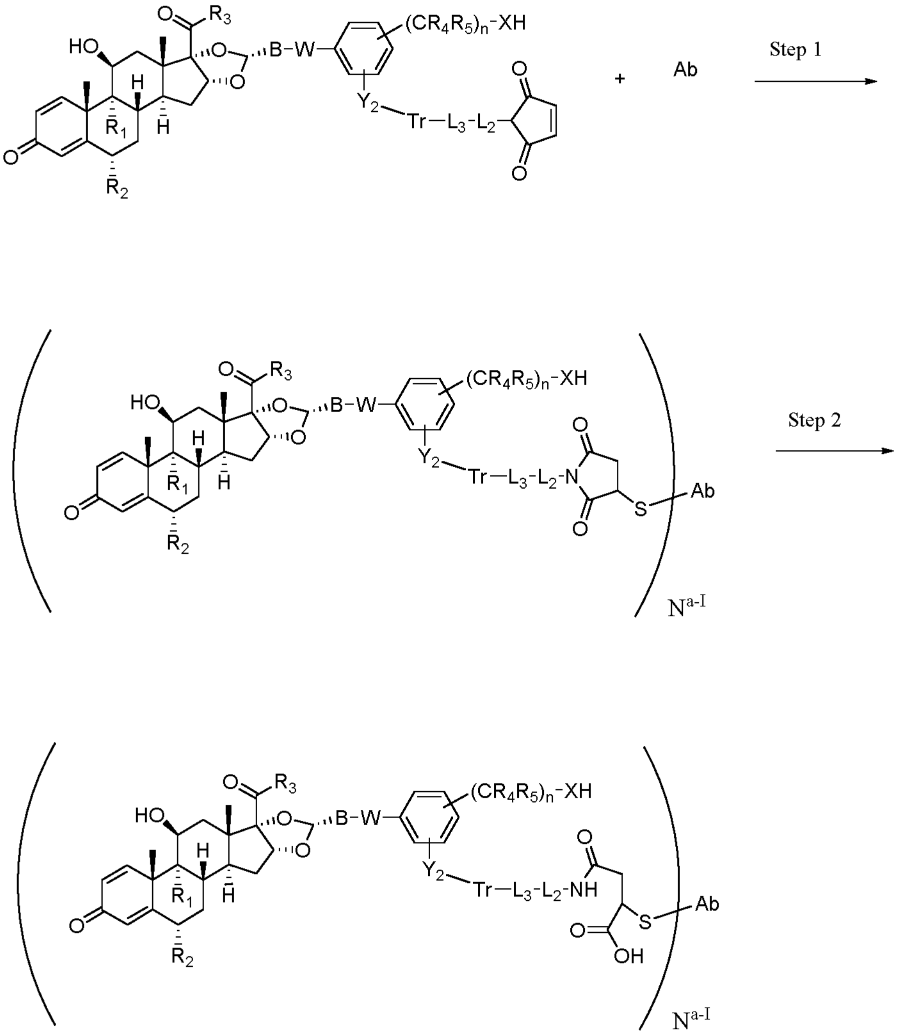


In Step 1, the ligand Ab was reacted with a compound of any one of formula (IVa) or formula (IVb) in an acidic, neutral or alkaline buffer to obtain a compound of formula (Va) or formula (Vb); wherein, Ab is a ligand containing at least one free sulfhydryl (-SH), wherein the free sulfhydryl may be obtained by reducing the ligand by using a reducing agent; the reducing agent includes, but is not limited to, tris(2-carboxyethyl)phosphine, mercaptoethanol, dithiothreitol, cysteine, reduced glutathione, and the like; particularly, disulfide bonds (-S-S-) between ligand chains may be reduced to form free sulfhydryl; wherein the S atom in the compound of formula (Va) or formula (Vb) may be sulfhydryl derived from Ab;

in Step 2, the compound of any one of formula (Va) or formula (Vb) was incubated in an alkaline buffer at a selected temperature for a selected time to obtain another compound of any one of formula (Va) or formula (Vb) of the present application;

Tr, $L_2$ and $L_3$ may be defined as Tr, $L_2$ and $L_3$ in any one of the compounds of formula (IVa) or formula (IVb) of the present application; Ab and $N^{a-I}$ may be defined as Ab and $N^{a-I}$ in any one of the compounds of formula (Va) or formula (Vb) of the present application;

the buffer is selected from the group consisting of the following buffers with a pH of 2 to 12: citric acid-sodium citrate buffer, phosphoric acid-sodium phosphate buffer, phosphoric acid-potassium phosphate buffer, sodium dihydrogen phosphate-disodium hydrogen phosphate buffer, potassium dihydrogen phosphate-dipotassium hydrogen phosphate buffer, succinic acid-sodium succinate buffer, acetic acid-sodium acetate buffer, boric acid-borax buffer, boric acid-potassium borate buffer, borax-sodium hydroxide buffer, histidine-hydrochloric acid buffer, glycine-sodium hydroxide buffer, arginine-hydrochloric acid buffer, sodium bicarbonate-sodium carbonate buffer, potassium bicarbonate-potassium carbonate buffer, Tris-hydrochloric acid buffer, aqueous ammonia-ammonium chloride buffer, barbiturate sodium-hydrochloric acid buffer, borax-sodium carbonate buffer, boric acid-potassium chloride buffer, and a combination of two or more of the above; the selected temperature may be 5 °C to 60 °C; the incubation time may be 0 h to 72 h.

**Preparation route 20**

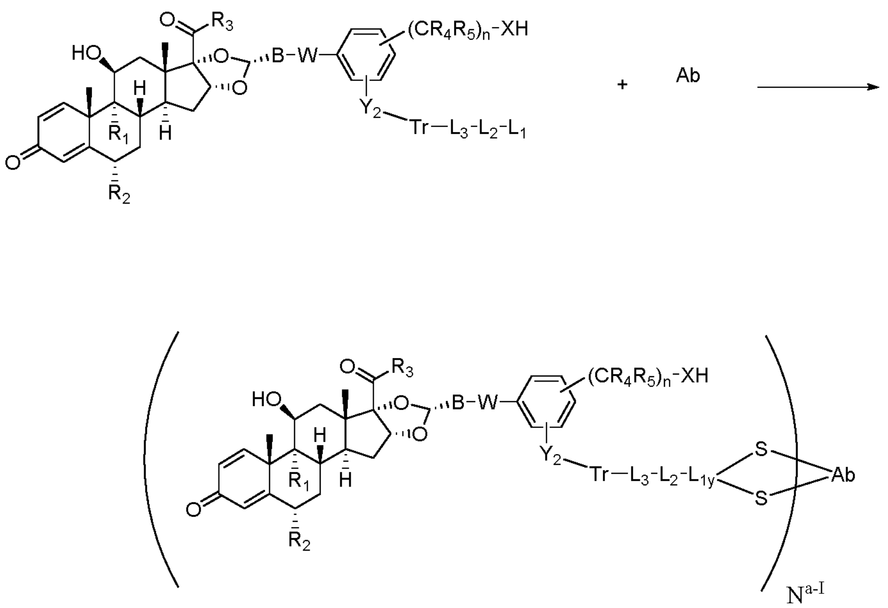

The ligand Ab was reacted with a compound of any one of formula (IVa) or formula (IVb) having a group capable of being coupled to two sulfydryl in an acidic, neutral or alkaline buffer to obtain a compound of formula (Va) or formula (Vb);

wherein, Ab is a ligand containing at least two free sulfhydryl (-SH), wherein the free sulfhydryl may be obtained by reducing the ligand by using a reducing agent; the reducing agent includes, but is not limited to, tris(2-carboxyethyl)phosphine, mercaptoethanol, dithiothreitol, cysteine, reduced glutathione, and the like; particularly, disulfide bonds (-S-S-) between ligand chains may be reduced to form free sulfhydryl; wherein the S atom in the compound of formula (Va) or formula (Vb) may be sulfhydryl derived from Ab;

Tr, $L_1$, $L_2$ and $L_3$ may be defined as Tr, $L_1$, $L_2$ and $L_3$ in any one of the compounds of formula (IVa) or formula (IVb) of the present application; Ab and $N^{a-I}$ may be defined as Ab and $N^{a-I}$ in any one of the compounds of formula (Va) or formula (Vb) of the present application; $L_{1y}$ represents the structure after coupling to $L_1$ coupled to two sulfhydryl; the compound of formula (Va) or formula (Vb) having a group capable of being coupled to two sulfhydryl may comprise an $L_{1y}$ group selected from the group consisting of the following:

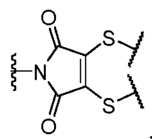, 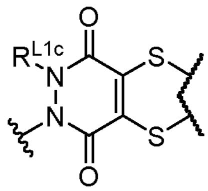, 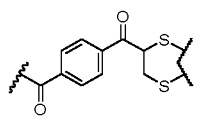 and 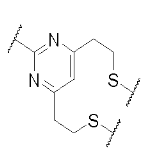,

wherein each of $R^{L1a}$, $R^{L1b}$ and $R^{L1c}$ is independently selected from the group consisting of the following: hydrogen, protium, deuterium, tritium, halogen, $-NO_2$, -CN, -OH, -SH, $-NH_2$, -C(O)H, $-CO_2H$, -C(O)C(O)H, $-C(O)CH_2C(O)H$, -S(O)H, $-S(O)_2H$, $-C(O)NH_2$, $-SO_2NH_2$, -OC(O)H, $-N(H)SO_2H$, alkyl, alkenyl, alkynyl, alcyl, heterocyclyl, aryl, and heteroaryl;

the buffer is selected from the group consisting of the following buffers with a pH of 2 to 12: citric acid-sodium citrate buffer, phosphoric acid-sodium phosphate buffer, phosphoric acid-potassium phosphate buffer, sodium dihydrogen phosphate-disodium hydrogen phosphate buffer, potassium dihydrogen phosphate-dipotassium hydrogen phosphate buffer, succinic acid-sodium succinate buffer, acetic acid-sodium acetate buffer, boric acid-borax buffer, boric acid-potassium borate buffer, borax-sodium hydroxide buffer, histidine-hydrochloric acid buffer, glycine-sodium hydroxide buffer, arginine-hydrochloric acid buffer, sodium bicarbonate-sodium carbonate buffer, potassium bicarbonate-potassium carbonate buffer, Tris-hydrochloric acid buffer, aqueous ammonia-ammonium chloride buffer, barbiturate sodium-hydrochloric acid buffer, borax-sodium carbonate buffer, boric acid-potassium chloride buffer, and a combination of two or more of the above.

Example 1

## Compound 1

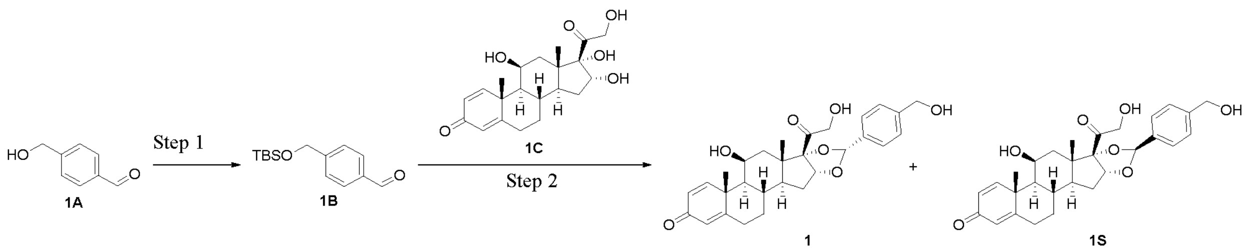


**Step** 1 Compound 1A (500 mg, 3.67 mmol) was dissolved in dichloromethane (10 mL), then DIEA (1.42 g, 10.99 mmol) was added, and TBSCl (830 mg, 5.51 mmol) in dichloromethane (5 mL) was added to the above reaction solution which was then stirred overnight for 17 h. After the reaction was completed as detected by TLC (PE/EA = 20/1), the reaction solution was directly dried by rotary evaporation under reduced pressure and subjected to column chromatography (PE:EA= 100:0-100:1) to give a milky-white oily liquid 1B (800 mg, yield: 87%).

**Step 2** Compound **1B** (125 mg, 0.50 mmol) and Compound **1C** (188 mg, 0.50 mmol) were added to and dissolved in ultra-dry acetonitrile (8 mL), the reaction solution was purged with nitrogen, cooled to 0 °C, added with mixture of methanesulfonic acid (48 mg, 0.50 mmol) and MeCN (2 mL)by a syringe, and then stirred overnight for 16 h. After the reaction was completed as detected by TLC (PE/EA = 3/1), the reaction solution was added with sodium bicarbonate, filtered, and dried by rotary evaporation to give a crude product which was then purified by preparative chromatography to give Compound 1 (62 mg, yield: 25%) as a white powder and **1S** (7 mg, yield: 3%) as a white powder, respectively.

Compound **1:**

MS-ESI: m/z 495.3 $[M+H]^+$.

$^1H$ NMR (400 MHz, DMSO-$d_6$) δ 7.41 (d, *J*= 8.1 Hz, 2H), 7.34-7.28 (m, 3H), 6.16 (dd, *J* = 10.1, 1.9 Hz, 1H), 5.93 (s, 1H), 5.43 (s, 1H), 4.93 (d, *J*= 4.9 Hz, 1H), 4.79 (brs, 1H), 4.52 (d, *J*= 19.5 Hz, 1H), 4.48 (s, 2H), 4.34-4.25 (m, 1H), 4.19 (d, *J*= 19.5 Hz, 1H), 2.61-2.52 (m, 1H), 2.35-2.27 (m, 1H), 2.19-1.98 (m, 2H), 1.84-1.58 (m, 5H), 1.39 (s, 3H), 1.13-0.95 (m, 2H), 0.87 (s, 3H).

Compound **1S**:

MS-ESI: m/z 495.3 $[M+H]^+$.

$^1H$ NMR (400 MHz, DMSO-$d_6$) δ 7.35-7.27 (m, 3H), 7.22 (d, *J* = 8.1 Hz, 2H), 6.17 (dd, *J* = 10.1, 1.9 Hz, 1H), 6.09 (s, 1H), 5.95 (s, 1H), 5.30 (d, *J*= 6.8 Hz, 1H), 4.81-4.76 (m, 1H), 4.48 (s, 2H), 4.30 (s, 1H), 4.25 (d, *J* = 19.2 Hz, 1H), 4.01 (d, *J* = 19.2 Hz, 1H), 2.59-2.53 (m, 1H), 2.36-2.28 (m, 1H), 2.12-1.97 (m, 2H), 1.90-1.69 (m, 5H), 1.39 (s, 3H), 1.26-1.13 (m, 1H), 1.06 (dd, *J*= 11.0, 3.4 Hz, 1H), 0.89 (s, 3H).

## Compound 2

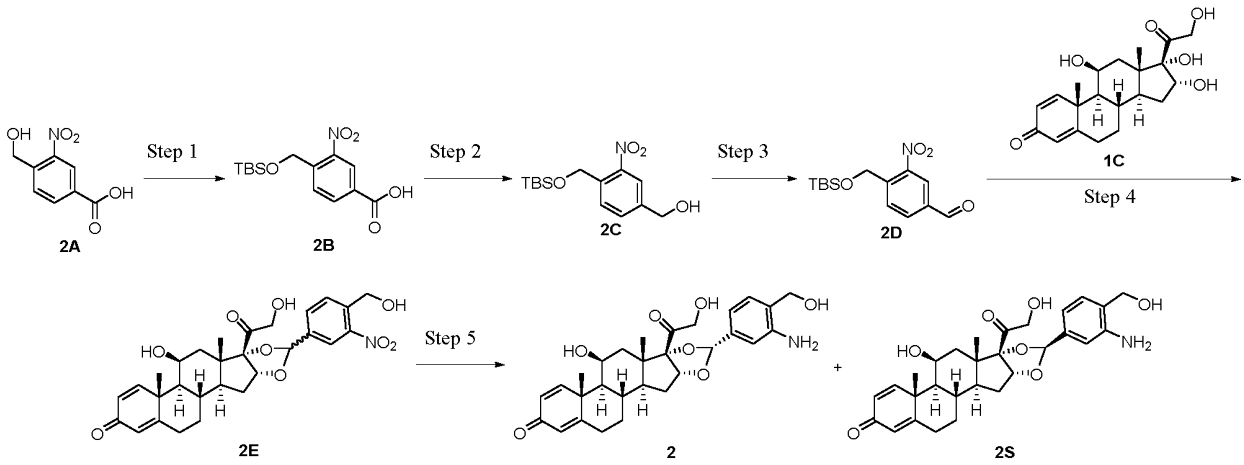


**Step 1** Compound **2A** (5.0 g, 25.4 mmol) and imidazole (2.59 g, 38.0 mmol) were dissolved in dichloromethane (50 mL), then TBSCl (4.9 g, 32.5 mmol) was added, and the reaction solution was reacted at 25 °C for 2 h. After the reaction was completed as detected by TLC, the reaction solution was added with 100 mL of water and extracted twice with 100 mL of dichloromethane (×2), the dichloromethane was combined, dried over anhydrous sodium sulfate, and dried by rotary evaporation, and the residue was directly stirred with silica gel and subjected to column chromatography (PE:EA = 20:1) to give a yellow solid **2B** (3 g, yield: 38%). MS-ESI: m/z 312.2 $[M+H]^+$.

**Step 2** Compound **2B** (1.0 g, 3.2 mmol) was dissolved in THF (10 mL), the reaction solution was purged with $N_2$, cooled to 0 °C, and then added with $BH_3$/THF (6.4 mL, 6.4 mmol), and the reaction was completed as detected by LCMS after 17 h. The reaction solution was added to methanol (40 mL) (maintained in an ice bath at 0 °C) to quench $BH_3$, and then dried by rotary evaporation to give a colorless oil **2C** (500 mg, yield: 52%). MS-ESI: m/z 298.1 $[M+H]^+$.

**Step 3** Compound **2C** (500 mg, 1.68 mmol) was dissolved in dichloromethane (8 mL), and the reaction solution was cooled to 0 °C, added with Dess-Martin reagent (1.43 g, 3.36 mmol), and reacted for 1.5 h. After the reaction was completed as detected by LCMS, the reaction solution was diluted with water (100 mL) and extracted twice with ethyl acetate (100 mL + 50 mL), and the organic phases were combined and washed with saturated brine, dried over anhydrous sodium sulfate, and dried by rotary evaporation to give a white solid **2D** (250 mg, yield: 50%). MS-ESI: m/z 296.0 $[M+H]^+$.

**Step 4** Compound **2D** (100 mg, 0.34 mmol), Compound **1C** (127 mg, 0.34 mmol) and $MgSO_4$ (250 mg, 2.08 mmol) were dissolved in acetonitrile (10 mL), the reaction solution was cooled to 0 °C and purged with nitrogen, added with trifluoromethanesulfonic acid (150 mg, 1.0 mmol), and maintained in an ice bath for 2 h after the addition was completed. After the reaction was completed as detected by LCMS, the reaction solution was directly filtered, and the filter cake was rinsed three times with acetonitrile. The mother solution was concentrated by rotary evaporation and subjected to column chromatography (DCM:MeOH = 30:1) to give a white solid **2E** (100 mg, yield: 55%). MS-ESI: m/z 540.0 $[M+H]^+$.

**Step 5** Compound **2E** (100 mg, 0.18 mmol) and iron powder (100 mg, 1.8 mmol) were added to ethanol (4 mL), $NH_4Cl$ (96 mg, 1.8 mmol) was dissolved in water and added to ethanol, and the reaction solution was purged with $N_2$, heated to 60 °C and reacted for 2 h. After the reaction was completed as detected by LCMS, the reaction solution was filtered through celite, and the filter cake was rinsed three times with ethanol. The mother solution was concentrated by rotary evaporation and subjected to preparative chromatography and lyophilized to give a white solid powder 2 (40 mg, yield: 42%) and a white solid powder **2S**(5 mg, yield: 5%), respectively.

Compound **2:**

MS-ESI: m/z 532.2 $[M+Na]^+$.

$^1$H NMR (400 MHz, DMSO-$d_6$) δ 7.32 (d, *J*= 10.1 Hz, 1H), 7.20 (d, *J* = 7.7 Hz, 1H), 6.94 (s, 1H), 6.89 (d, *J* = 7.7 Hz, 1H), 6.16 (dd, *J* = 10.1, 1.9 Hz, 1H), 5.93 (s, 1H), 5.34 (s, 1H), 4.95-4.89 (m, 1H), 4.80 (brs, 1H), 4.49 (d, *J*= 19.4 Hz, 1H), 4.44 (s, 2H), 4.33-4.27 (m, 1H), 4.18 (d, *J*= 19.4 Hz, 1H), 2.62-2.52 (m, 1H), 2.35-2.28 (m, 1H), 2.19-2.07 (m, 1H), 2.07-1.99 (m, 1H), 1.80-1.60 (m, 5H), 1.40 (s, 3H), 1.15-1.02 (m, 1H), 0.99 (dd, *J*= 11.2, 3.6 Hz, 1H), 0.86 (s, 3H).

Compound **2S:**

MS-ESI: m/z 532.3 $[M+Na]^+$.

$^1H$ NMR (400 MHz, DMSO-$d_6$) δ 7.32 (d, *J* = 10.1 Hz, 1H), 7.15-7.09 (m, 1H), 6.70 (s, 1H), 6.63 (d, *J*= 7.6 Hz, 1H), 6.17 (dd, *J*= 10.0, 1.9 Hz, 1H), 5.99 (s, 1H), 5.94 (s, 1H), 5.25 (d, *J*= 6.8 Hz, 1H), 4.78 (brs, 1H), 4.40 (s, 2H), 4.33-4.22 (m, 2H), 4.02 (d, *J*= 19.2 Hz, 1H), 2.60-2.53 (m, 1H), 2.36-2.28 (m, 1H), 2.11-1.97 (m, 2H), 1.90-1.69 (m, 5H), 1.39 (s, 3H), 1.26-1.13 (m, 1H), 1.09-1.02 (m, 1H), 0.88 (s, 3H).

## Compound 3

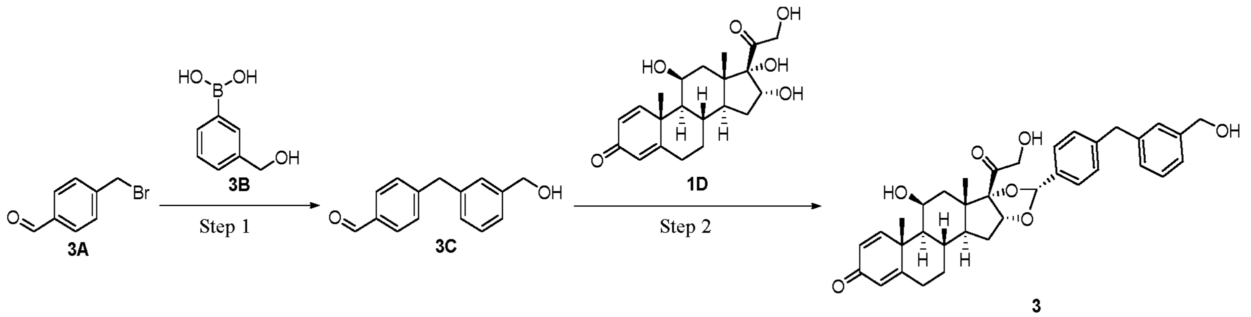


3

**Step 1** Compound **3A** (6.58 g, 33.08 mmol, 1.0 eq) and Compound **3B** (5.03 g, 33.08 mmol, 1.0 eq) were added into a 250-mL three-necked flask, THF (50 mL), water (5 mL) and $K_2CO_3$ (13.71 g, 99.24 mmol, 3.0 eq) were added, under nitrogen atmosphere, the reaction solution was added with Pd(dppf)$Cl_2$ (1.21 g, 1.65 mmol, 0.05 eq), purged three times with nitrogen, heated to 80 °C to reflux, and then stirred for 3-4 h. After the reaction was completed as detected by TLC (PE/EA = 5/1), the reaction solution was cooled to room temperature and filtered, and the filtrate was poured into 300 mL of water, and extracted with EA (200 mL). The organic phase was washed with water (100 mL × 3), washed once with saturated NaCl, dried over anhydrous $Na_2SO_4$, and concentrated by rotary evaporation, and the residue was stirred with silica gel and subjected to column chromatography (PE/EA = 7/1) to give a product which was concentrated by rotary evaporation to give a white solid **3C** (2.5 g, yield: 33%).

**Step 2** Compound **1D** (3 g, 7.97 mmol, 1.0 eq), Compound **3C** (1.8 g, 7.97 mmol, 1.0 eq) and $MgSO_4$ (2.88 g, 23.91 mmol, 3.0 eq) were added into a 100-mL three-necked flask, acetonitrile (30 mL) was added, under nitrogen atmosphere, trifluoromethanesulfonic acid (1.2 g, 7.97 mmol, 1.0 eq) was added in an ice-water bath, heated to room temperature and reacted for 2 h after the addition was completed. After the reaction was completed as detected by TLC (PE/EA = 3/1), the reaction solution was filtered, the filtrate was concentrated by rotary evaporation, and the residue was stirred with silica gel and subjected to column chromatography (PE/EA = 2/1) to give a product which was concentrated by rotary evaporation to give a white solid **3** (3.7 g, yield: 79%).

MS-ESI: m/z 585.3 $[M+H]^+$.

$^1H$ NMR (400 MHz, DMSO-$d_6$) δ 7.36 (d, *J*= 7.8 Hz, 2H), 7.30 (d, *J*= 10.1 Hz, 1H), 7.24 (d, *J*= 7.7 Hz, 2H), 7.20 (d, *J* = 7.5 Hz, 1H), 7.15 (s, 1H), 7.11 (d, *J* = 7.6 Hz, 1H), 7.07 (d, *J* = 7.6 Hz, 1H), 6.16 (dd, *J* = 10.1, 1.9 Hz, 1H), 5.92 (s, 1H), 5.39 (s, 1H), 4.91 (d, *J* = 4.8 Hz, 1H), 4.78 (brs, 1H), 4.49 (d, *J* = 19.4 Hz, 1H), 4.43 (s, 2H), 4.32-4.25 (m, 1H), 4.17 (d, *J* = 19.5 Hz, 1H), 3.90 (s, 2H), 2.59-2.51 (m, 1H), 2.35-2.26 (m, 1H), 2.18-1.95 (m, 2H), 1.82-1.56 (m, 5H), 1.39 (s, 3H), 1.11-0.96 (m, 4H), 0.85 (s, 3H).

## Compound 4

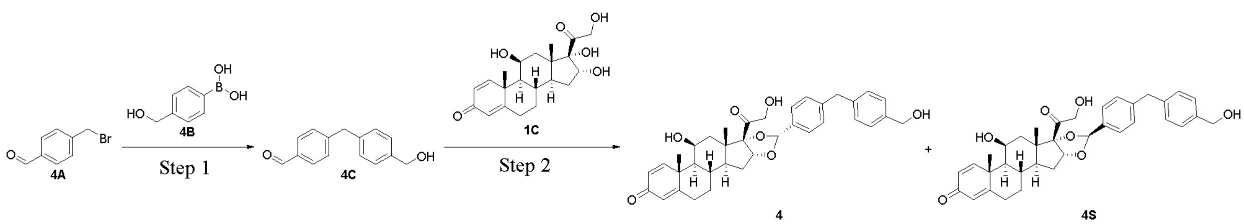


4 4S

**Step 1** Compound **4A** (500 mg, 2.51 mmol) was dissolved in THF/$H_2O$ (10/2 mL), and then Compound **4B** (573 mg, 3.77 mmol) and $K_2CO_3$ (1.04 g, 7.53 mmol) were added. The reaction solution was purged with nitrogen, then added with Pd(dppf)$Cl_2$ (367 mg, 0.50 mmol), and heated to 80 °C under nitrogen atmosphere and reacted for 2 h. The reaction solution was cooled to room temperature, diluted with water (20 mL), and extracted with ethyl acetate (20 mL × 3), the organic phases were combined and washed with saturated brine (10 mL), dried over anhydrous sodium sulfate, and concentrated, and the residue was subjected to column chromatography (PE/EA = 1:0 to 3:1) to give a yellow solid **4C** (270 mg, yield: 48%).

**Step 2** Compound **4C** (150 mg, 0.66 mmol) was dissolved in MeCN (10 mL), then Compound **1C** (250 mg, 0.66 mmol) and $MgSO_4$ (160 mg, 1.32 mmol) were added, and trifluoromethanesulfonic acid (299 mg, 1.99 mmol) was added at 0 °C under nitrogen atmosphere. The reaction solution was reacted at 0 °C for 1 hour, diluted with water (20 mL), and extracted with dichloromethane (10 mL × 3), and the organic phases were combined and washed with brine (10 mL), dried, and then concentrated by rotary evaporation to give a crude product which was then subjected to preparative chromatography to give a white powdery solid **4** (120 mg, yield: 31%) and a white powdery solid **4S** (13 mg, yield: 3%), respectively.

Compound **4:**

MS-ESI: m/z 585.4 $[M+H]^+$.

$^1H$ NMR (400 MHz, DMSO-*d*$_6$) δ 7.35 (d, *J*= 8.0 Hz, 2H), 7.30 (d, *J*= 10.1 Hz, 1H), 7.24-7.16 (m, 4H), 7.14 (d, *J*= 8.1 Hz, 2H), 6.16 (dd, *J* = 10.1, 1.9 Hz, 1H), 5.92 (s, 1H), 5.38 (s, 1H), 4.90 (d, *J* = 4.8 Hz, 1H), 4.48 (d, *J* = 19.4 Hz, 1H), 4.42 (s, 2H), 4.31-4.25 (m, 1H), 4.16 (d, *J* = 19.4 Hz, 1H), 3.89 (s, 2H), 2.59-2.52 (m, 1H), 2.36-2.25 (m, 1H), 2.18-1.96 (m, 2H), 1.82-1.55 (m, 5H), 1.38 (s, 3H), 1.10-0.94 (m, 2H), 0.85 (s, 3H).

Compound **4S:**

MS-ESI: m/z 585.3 $[M+H]^+$.

$^1H$ NMR (400 MHz, Chloroform-*d*) δ 7.31-7.26 (m, 3H), 7.21-7.11 (m, 6H), 6.31 (dd, *J* = 10.1, 1.9 Hz, 1H), 6.08 (s, 2H), 5.43-5.38 (m, 1H), 4.65 (s, 2H), 4.52-4.45 (m, 1H), 4.27 (d, *J*= 19.9 Hz, 1H), 4.05 (d, *J* = 19.9 Hz, 1H), 3.95 (s, 2H), 2.66-2.54 (m, 1H), 2.23-2.07 (m, 3H), 1.95-1.83 (m, 2H), 1.80-1.68 (m, 1H), 1.63 (dd, *J* = 14.1, 2.6 Hz, 1H), 1.46 (s, 3H), 1.31-1.13 (m, 2H), 0.99 (s, 3H).

## Compound 5

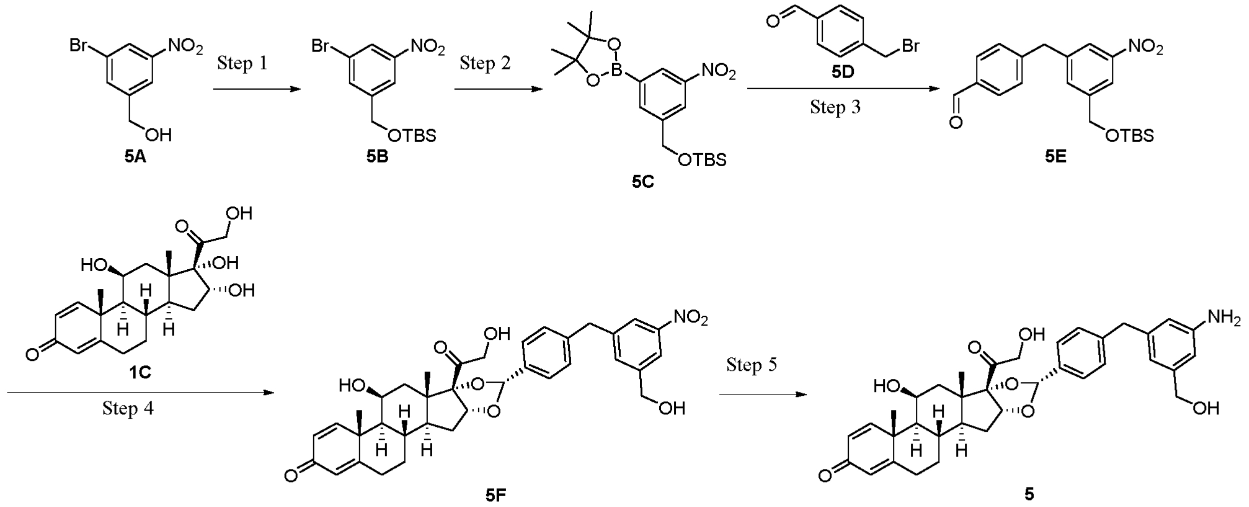


5A 5B 5C 5E 1C 5F 5

**Step 1** Compound **5A** (50 g, 216 mmol, 1.0 eq) and imidazole (22 g, 323 mmol, 1.5 eq) were added into a 2-L three-necked flask, the reaction mixture was dissolved in dichloromethane (700 mL), and then added slowly with TBSCl (44 g, 292 mmol, 1.35 eq). After the starting material was consumed completely as detected by TLC (PE/EA= 9/1), the reaction solution was cooled to room temperature, filtered, and extracted twice with dichloromethane (100 mL) and $H_2O$ (90 mL). The organic phase was dried over anhydrous sodium sulfate, concentrated by rotary evaporation, stirred with silica gel and purified by normal phase column chromatography (PE:EA = 10:1) to give a white solid **5B** (65 g, yield: 87%).

**Step 2** Compound **5B** (60 g, 173 mmol, 1.0 eq), bis(pinacolato)diboron (44 g, 173 mmol, 1.0 eq), $Pd(dppf)Cl_2$ (6.3 g, 8.6 mmol, 0.05 eq) and potassium acetate (51 g, 520 mmol, 3.0 eq) were added into a 2-L three-necked flask, the reaction mixture was dissolved in dioxane (1 L), under $N_2$ atmosphere, the reaction solution was stirred at 110 °C for 12 h. After the reaction was completed as detected by TLC (PE:EA = 9:1), the reaction solution was cooled to room temperature, filtered, and extracted twice with ethyl acetate (1 L) and $H_2O$ (1 L), and the organic phase was dried over anhydrous sodium sulfate, concentrated by rotary evaporation, stirred with silica gel and purified by normal phase column chromatography (PE:EA = 10:1) to give a white solid **5C** (60 g, yield: 88%). **Step 3** Compound **5C** (30 g, 76 mmol, 1.0 eq), Compound **5D** (15.2 g, 76 mmol, 1 eq) and $Pd(dppf)Cl_2$ (3 g, 4 mmol, 0.05 eq) were added into a 1-L three-necked flask, then dioxane (600 mL) and $Cs_2CO_3$ (74 g, 228 mmol, 3.0 eq) dissolved in water (60 mL) were added under nitrogen

atmosphere, the reaction solution was reacted at 110 °C for 2 h. After the reaction was completed as detected by TLC (PE:EA = 9:1), the reaction solution was cooled to room temperature, filtered, and extracted twice with ethyl acetate (1 L) and $H_2O$ (1 L). The organic phase was dried over anhydrous sodium sulfate, concentrated by rotary evaporation, stirred with silica gel and purified by normal phase column chromatography (PE:EA = 10:1) to give a white solid **5E** (20 g, yield: 68%).

**Step 4** Compound **5E** (2.0 g, 5.2 mmol, 1.0 eq), Compound **1C** (1.96 g, 5.2 mmol, 1.0 eq) and $MgSO_4$ (1.88 g, 15.6 mmol, 3.0 eq) were added into a 250-mL three necked flask, the reaction mixture was added with acetonitrile (50 mL), cooled to -20 °C with dry ice and ethanol, added slowly with trifluoromethanesulfonic acid (2.34 g, 15.6 mmol, 3.0 eq), and then stirred at 0 °C for 2 h. After the reaction was completed as detected by TLC (PE:EA = 1:1), sodium bicarbonate solution was added to adjust the pH to 7-8, acetonitrile was removed by rotary evaporation, and the reaction solution was extracted and slurried by PE:EA = 3:1 to give a crude product of Compound **5F** (3.2 g, yield: 98%) which was directly used in the next step. MS-ESI: m/z 630.1 $[M+H]^+$.

**Step 5** Compound **5F** (3.2 g, 5.08 mmol, 1 eq) and Fe (2.84 g, 50.8 mmol, 10 eq) were added into a 250-mL three-necked flask, ethanol (80 mL) was added, $NH_4Cl$ (2.72 g, 50.8 mmol, 10 eq) was dissolved in water (80 mL) and then added into the three-necked flask, and the reaction solution was reacted at 60 °C for 2 h. After the reaction was completed as detected by TLC (DCM:MOH = 10:1), ethanol was removed by evaporation under reduced pressure, and aqueous sodium bicarbonate was added to adjust the pH to 8. The reaction solution was extracted twice with 80 mL of dichloromethane and water, and the organic phase was dried over magnesium sulfate, filtered, and concentrated by rotary evaporation to give a yellow solid (2.6 g) as a crude product, 400 mg of which was subjected to reversed-phase column chromatography to give Compound 5 (43.9 mg, purity: ≥95%).

MS-ESI: m/z 600.1 $[M+H]^+$.

$^1H$ NMR (400 MHz, DMSO-$d_6$) δ 7.38 (d, *J* = 8.0 Hz, 2H), 7.31 (d, *J* = 10.1 Hz, 1H), 7.24 (d, *J* = 7.9 Hz, 2H), 6.96 (s, 1H), 6.90 (s, 1H), 6.76 (s, 1H), 6.16 (dd, *J* = 10.1, 1.8 Hz, 1H), 5.93 (s, 1H), 5.40 (s, 1H), 4.92 (d, *J* = 4.8 Hz, 1H), 4.79 (brs, 1H), 4.49 (d, *J* = 19.4 Hz, 1H), 4.43 (s, 2H), 4.32-4.26 (m, 1H), 4.17 (d, *J* = 19.5 Hz, 1H), 3.90 (s, 2H), 2.62-2.52 (m, 1H), 2.36-2.26 (m, 1H), 2.18-1.97 (m, 2H), 1.81-1.55 (m, 5H), 1.39 (s, 3H), 1.10-0.94 (m, 2H), 0.86 (s, 3H).

## Compound 6

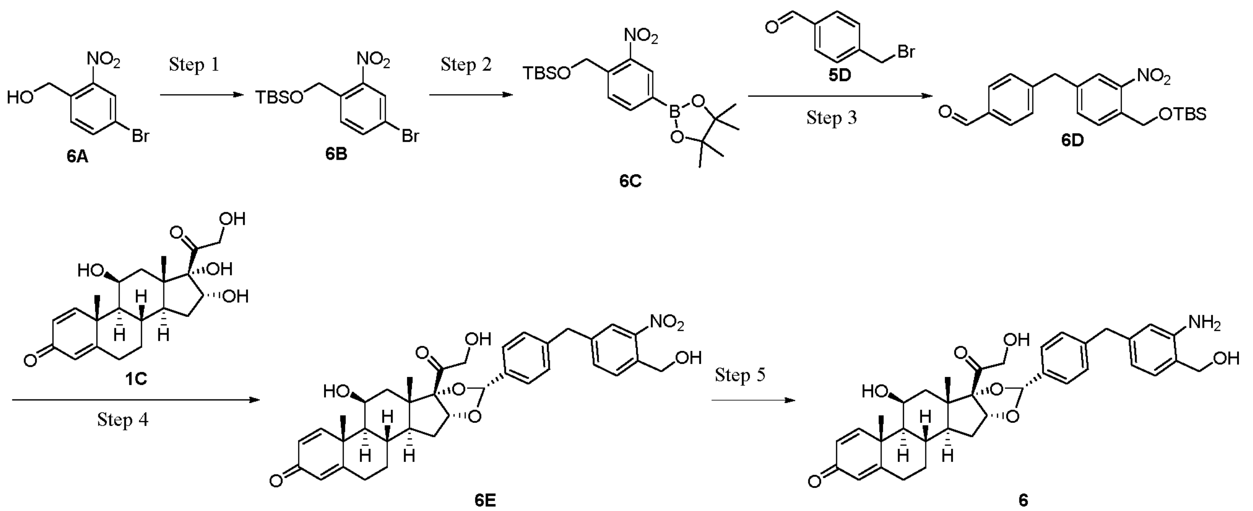

6A 6B 6C 5D 6D 1C 6E 6

**Step 1** Compound **6A** (10 g, 43.1 mmol) was dissolved in dichloromethane (100 mL), then DIEA (16.7 g, 129.3 mmol) was added, and TBSCl (9.7 g, 64.6 mmol) in dichloromethane (100 mL) was added to the above reaction solution which was then stirred overnight for 3 d. After the reaction was completed as detected by TLC (PE/EA = 40/1), the reaction solution was directly dried by rotary evaporation under reduced pressure and subjected to column chromatography with pure petroleum ether to give a light yellow oily liquid **6B** (12.0 g, yield: 80%).

**Step 2** Compound **6B** (1 g, 2.89 mmol), bis(pinacolato)diboron (883 mg, 3.48 mmol), KOAc (853 mg, 8.69 mmol) and Pd(dppf)$Cl_2$ (212 mg, 0.29 mmol) were added to 1,4-dioxane (10 mL), and the reaction was purged with nitrogen, heated (110 °C) and stirred overnight for 17 h. After the reaction was completed as detected by TLC (PE:EA= 40:1), the reaction solution was directly dried by rotary evaporation, and the residue was directly stirred with silica gel and subjected to column chromatography (petroleum ether:ethyl acetate = 50:1) to give a light yellow oily liquid 6C (183 mg, yield: 16%). MS-ESI: m/z 394.3 $[M+H]^+$.

**Step 3** Compound **6C** (180 mg, 0.46 mmol), Pd(dppf)$Cl_2$ (33.5 mg, 0.046 mmol), Compound 5D (90.6 mg, 0.46 mg), $Cs_2CO_3$ (298 mg, 0.92 mmol), 1,4-dioxane (8 mL) and water (2 mL) were added, and the reaction solution was

purged with $N_2$, heated (110 °C) and stirred overnight (20 h). After the reaction was completed as detected by TLC (PE/EA= 10/1), the reaction solution was directly dried by rotary evaporation, and the residue was directly stirred with silica gel and subjected to column chromatography (petroleum ether:ethyl acetate = 100:1) to give a light yellow oily liquid **6D** (95.8 mg, yield: 54%). MS-ESI: m/z 386.1 $[M+H]^+$.

**Step 4** Compound **6D** (500 mg, 1.30 mmol), Compound **1C** (488 mg, 1.30 mmol), MeCN (10 mL) and $MgSO_4$ (930 mg, 7.73 mmol) were added into a three-necked flask, the reaction solution was purged with $N_2$, cooled to 0 °C, added with trifluoromethanesulfonic acid (585 mg, 3.90 mmol) by a constant pressure dropping funnel and stirred (3 h). After the reaction was completed as detected by TLC (DCM/MeOH = 10/1), the reaction solution was directly dried by rotary evaporation, and the residue was directly stirred with silica gel and subjected to column chromatography (dichloromethane:MeOH = 50:1) to give a white solid **6E** (300 mg, yield: 37%); MS-ESI: m/z 630.1 $[M+H]^+$.

**Step 5** Compound **6E** (4 g, 6.35 mmol) and ethanol (100 mL) were added into a single-necked flask, iron powder (2.36 g, 42.1 mmol) and $NH_4Cl$ (3.4 g, 63.6 mmol) were dissolved in water (40 mL) and then added into the above single-necked flask, and the reaction solution was purged with $N_2$, heated to 60 °C and stirred (2 h). After the reaction was completed as detected by TLC (dichloromethane/$CH_3OH$ = 10/1), $NaHCO_3$ aqueous solution was added to adjust the pH to 7-8, the reaction solution was stirred for 10 min and filtered, the filtrate was concentrated by rotary evaporation, and the residue was added with dichloromethane to dissolve, stirred and subjected to column chromatography (dichloromethane:methanol = 30:1) to give a white powdery solid **6** (3.5 g, yield: 92%), 50 mg of which was taken to be subjected to preparative thin layer chromatography and filtered, the filtrate was concentrated by rotary evaporation, and the residue was added with acetonitrile and water (purified water) and lyophilized to give the product **6** (38 mg, purity: 90.08%).

MS-ESI: m/z 622.3 $[M+Na]^+$.

$^1H$ NMR (400 MHz, DMSO-$d_6$) δ 7.35 (d, *J*= 8.0 Hz, 2H), 7.30 (d, *J*= 10.2 Hz, 1H), 7.22-7.16 (m, 2H), 6.93 (d, *J* = 7.6 Hz, 1H), 6.45-6.40 (m, 1H), 6.40-6.34 (m, 1H), 6.16 (dd, *J* = 10.1, 1.9 Hz, 1H), 5.93 (s, 1H), 5.39 (s, 1H), 5.14-5.04 (m, 1H), 4.98-4.83 (m, 3H), 4.78 (d, *J*= 3.4 Hz, 1H), 4.54-4.45 (m, 1H), 4.34-4.26 (m, 3H), 4.22-4.12 (m, 1H), 3.75 (s, 2H), 2.60-2.53 (m, 1H), 2.36-2.26 (m, 1H), 2.17-1.96 (m, 2H), 1.81-1.60 (m, 5H), 1.39 (s, 3H), 1.09-0.97 (m, 2H), 0.85 (s, 3H).

## Compound 7

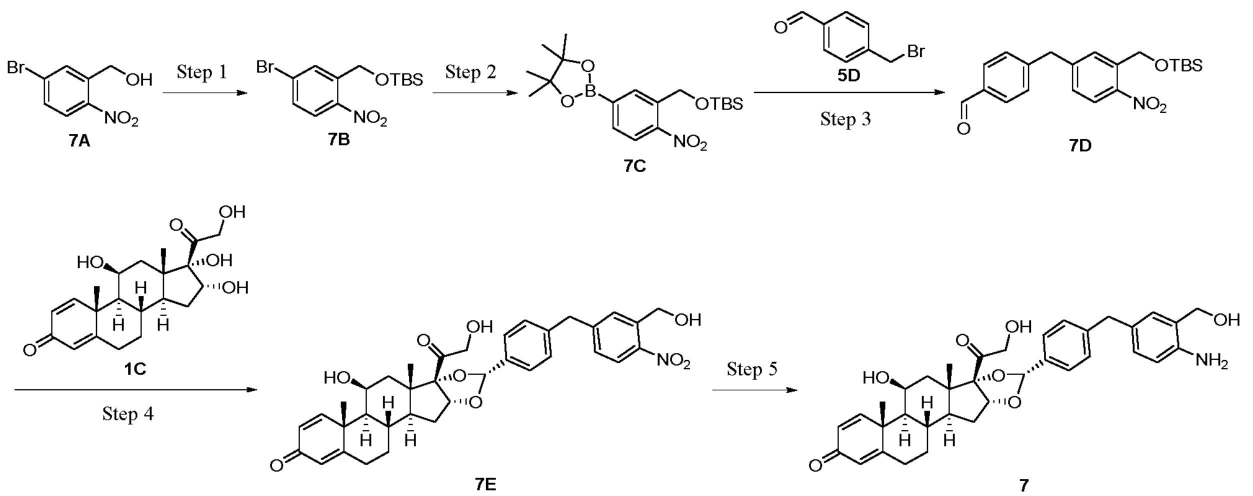


**Step** 1 Compound **7A** (65 g, 280 mmol, 1.0 eq) and imidazole (29 g, 420 mmol, 1.5 eq) were added into a 2-L three-necked flask, the reaction mixture was dissolved in dichloromethane (700 mL), and then added slowly with TBSCl (59 g, 392 mmol, 1.35 eq). After the starting material was consumed completely as detected by TLC (PE/EA= 9/1), the reaction solution was cooled to room temperature, filtered, and extracted twice with dichloromethane (100 mL) and $H_2O$ (90 mL). The organic phase was dried over anhydrous sodium sulfate, concentrated by rotary evaporation, stirred with silica gel and purified by normal phase column chromatography (PE:EA = 10:1) to give a white solid **7B** (80 g, yield: 82%).

**Step 2** Compound **7B** (70 g, 203 mmol, 1.0 eq), bis(pinacolato)diboron (51 g, 203 mmol, 1.0 eq), Pd(dppf)$Cl_2$ (7.4 g, 10.1 mmol, 0.05 eq) and potassium acetate (60 g, 609 mmol, 3.0 eq) were added into a 2-L three-necked flask, the reaction mixture was dissolved in dioxane (1 L), under $N_2$ atmosphere, the reaction solution was stirred at 110 °C for 12 h. After the reaction was completed as detected by TLC (PE:EA = 10:1), the reaction solution was cooled to room temperature, filtered, and extracted twice with ethyl acetate (1 L) and $H_2O$ (1 L), and the organic phase was dried over anhydrous sodium sulfate, concentrated by rotary evaporation, stirred with silica gel and purified by normal phase column chromatography (PE:EA = 10:1) to give a white solid **7C** (60 g, yield: 75%).

**Step 3** Compound **7C** (15 g, 38 mmol, 1.0 eq), Compound **5D** (7.6 g, 38 mmol, 1 eq) and Pd(dppf)$Cl_2$ (1.4 g, 1.9 mmol, 0.05 eq) were added into a 1-L three-necked flask, then dioxane (600 mL) was added, $Cs_2CO_3$ (37 g, 114 mmol, 3.0 eq) was dissolved in water (50 mL), and under nitrogen atmosphere, the reaction solution was reacted at 110 °C for 2 h. After the reaction was completed as detected by TLC (PE:EA = 10:1), the reaction solution was cooled to room temperature, filtered, and extracted twice with ethyl acetate (1 L) and $H_2O$ (1 L). The organic phase was dried over anhydrous sodium sulfate, concentrated by rotary evaporation, stirred with silica gel and purified by normal phase column chromatography (PE:EA = 10:1) to give a white solid **7D** (10 g, yield: 68%).

**Step 4** Compound **7D** (1 g, 2.6 mmol, 1.0 eq), Compound **1C** (0.98 g, 2.6 mmol, 1.0 eq) and $MgSO_4$ (0.94 g, 7.8 mmol, 3.0 eq) were added into a 250-mL three necked flask, the reaction mixture was added with acetonitrile (50 mL), cooled to -20 °C with dry ice and ethanol, added slowly with trifluoromethanesulfonic acid (1.17 g, 7.8 mmol, 3.0 eq), and then stirred at 0 °C for 2 h. After the reaction was completed as detected by TLC (dichloromethane:MeOH = 10:1), sodium bicarbonate solution was added to adjust the pH to 7-8, acetonitrile was removed by rotary evaporation, and the reaction solution was extracted and slurried by PE:EA = 3:1 to give Compound **7E** (1.4 g, yield: 86%); MS-ESI: m/z 630.1 $[M+H]^+$.

**Step 5** Compound **7E** (200 mg, 0.317 mmol, 1 eq) and iron powder (177 mg, 3.17 mmol, 10 eq) were added into a 25-mL three-necked flask, ethanol (6 mL) was added, $NH_4Cl$ (170 mg, 3.17 mmol, 10 eq) was dissolved in water (6 mL) and then added into the three-necked flask, and the reaction solution was reacted at 60 °C for 2 h. After the reaction was completed as detected by TLC (DCM:MeOH = 10:1), ethanol was removed by evaporation under reduced pressure, and aqueous sodium bicarbonate was added to adjust the pH to 8. The reaction solution was extracted twice with 80 mL of dichloromethane and water, and the organic phase was dried over magnesium sulfate, filtered, and concentrated by rotary evaporation to give a yellow solid (200 mg) which was subjected to reversed-phase column chromatography to give Compound 7 (42.6 mg, yield: 22%).

MS-ESI: m/z 600.8 $[M+H]^+$.

$^1$H NMR (400 MHz, DMSO-$d_6$) δ 7.36 (d, *J*= 8.0 Hz, 2H), 7.31 (d, *J* = 10.1 Hz, 1H), 7.22 (d, *J* = 7.9 Hz, 2H), 7.20-7.14 (m, 1H), 7.07 (d, *J* = 8.0 Hz, 1H), 6.98 (d, *J* = 8.0 Hz, 1H), 6.16 (dd, *J* = 10.1, 1.9 Hz, 1H), 5.93 (s, 1H), 5.39 (s, 1H), 4.91 (d, *J*= 4.7 Hz, 1H), 4.79 (s, 1H), 4.54-4.45 (m, 3H), 4.31-4.26 (m, 1H), 4.17 (d, *J* = 19.5 Hz, 1H), 3.87 (s, 2H), 2.60-2.53 (m, 1H), 2.36-2.26 (m, 1H), 2.18-1.96 (m, 2H), 1.81-1.54 (m, 5H), 1.39 (s, 3H), 1.12-0.94 (m, 2H), 0.86 (s, 3H).

## Compound 8

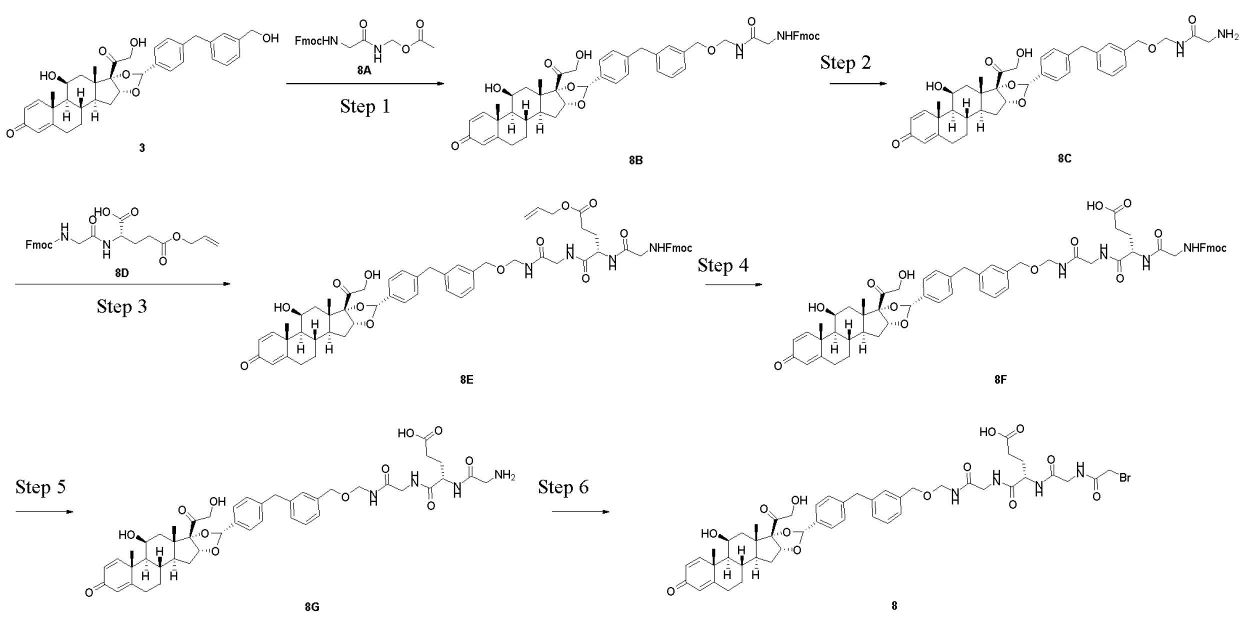


**Step 1** Compound **3** (2 g, 3.42 mmol, 1.0 eq) and Compound **8A** (2.52 g, 6.84 mmol, 2.0 eq) were added into a reaction flask, and the reaction mixture was dissolved in THF (30 mL), under nitrogen atmosphere, added with TsOH (0.63 g, 3.66 mmol, 1.07 eq) in an ice-water bath, and reacted at 5-10 °C for 30-40 min. After the reaction was completed as detected by TLC (PE/EA= 1/2), the reaction solution was added to water, the product was extracted with EA (100 mL), the organic phase was washed with water (50 mL × 3), dried over by anhydrous sodium sulfate and concentrated, and the residue was stirred with silica gel and subjected to column chromatography (PE/EA = 2/1) to give the product

which was concentrated by rotary evaporation to give a white solid **8B** (2.3 g, yield: 75%). MS-ESI: m/z 893.4 $[M+H]^+$.

**Step 2** Compound **8B** (2 g, 2.24 mmol) was dissolved in DCM (20 mL), and the reaction solution was added dropwise with DEA (6 mL) in an ice-water bath under nitrogen atmosphere, and then reacted at room temperature for 2 h after the addition was completed. After the reaction was completed as detected by TLC (PE/EA = 1/2), the reaction solution was concentrated by rotary evaporation, extracted three times with DCM, subjected to reversed-phase column chromatography and lyophilized to give an off-white solid **8C** (600 mg, yield: 40%). MS-ESI: m/z 671.3 $[M+H]^+$.

**Step 3** Compound **8C** (300 mg, 0.447 mmol, 1.0 eq) and Compound **8D** (230 mg, 0.492 mmol, 1.1 eq) were added into a reaction flask, and the reaction solution was dissolved in DMF (3.5 mL), added with DIEA (173 mg, 1.341 mmol, 3.0 eq) in an ice water-bath under nitrogen atmosphere, then added dropwise with HATU (221 mg, 0.581 mmol, 1.3 eq) in 1 mL DMF, and then stirred at 0-5 °C for 30 min after the addition was completed. After the reaction was completed as detected by LC-MS, the reaction solution was poured into ice water, EA (100 mL) was added, and the product was extracted. The organic phase was washed with water (50 mL × 3), dried over anhydrous sodium sulfate, and concentrated by rotary evaporation to give a yellow solid **8E** (430 mg, yield: 86%). MS-ESI: m/z 1119.4 $[M+H]^+$.

**Step 4** Under nitrogen atmosphere, Compound **8E** (300 mg, 0.268 mmol, 1 eq), $Pd(PPh_3)_4$ (62 mg, 0.0536 mmol, 0.2 eq) and N-methylmorpholine (452 mg, 4.47 mmol, 16.7 eq) were added into a reaction flask, and the reaction mixture was reacted at room temperature for 1 h under nitrogen atmosphere. After the reaction was completed as detected by TLC, the reaction solution was directly purified by reversed-phase column chromatography to give a white solid **8F** (210 mg, yield: 72%). MS-ESI: m/z 1079.3 $[M+H]^+$.

**Step 5** Under nitrogen atmosphere, Compound **8F** (200 mg, 0.185 mmol) was dissolved in DCM (2.4 mL), and the reaction solution was added dropwise with DEA (0.8 mL) in an ice-water bath, and heated to room temperature for 2 h after the addition was completed. After the reaction was completed as detected by LCMS, the reaction solution was directly extracted and washed three times with PE (60 mL × 3), the product precipitated and stuck to the wall of the bottle, and was dissolved with acetonitrile (3 mL), purified by reversed-phase column chromatography to give a white solid **8G** (62 mg, yield: 39%). MS-ESI: m/z 857.6 $[M+H]^+$.

**Step 6** Compound **8G** (60 mg, 0.07 mmol, 1.0 eq) was dissolved in THF (3 mL), the reaction solution was dissolved in water (0.6 mL), added with TEA (414 mg, 4.1 mmol, 58 eq) in an ice-water bath, stirred for 1 min, then added with bromoacetyl bromide (113 mg, 0.56 mmol, 8.0 eq), and stirred at 0-5 °C for 5 min. After the reaction was completed as detected by LC-MS, the reaction solution was stored with dry ice and ethanol, subjected to preparative chromatography and lyophilized to give a white solid **8** (11 mg, yield: 16%).

MS-ESI: m/z 977.3 $[M+H]^+$.

$^1$H NMR (400 MHz, DMSO-$d_6$) δ 8.62 (t, *J* = 6.6 Hz, 1H), 8.47 (t, *J* = 5.6 Hz, 1H), 8.24 (t, *J* = 5.9 Hz, 1H), 8.18 (d, *J* = 7.7 Hz, 1H), 7.40-7.34 (m, 2H), 7.31 (d, *J* = 10.1 Hz, 1H), 7.27-7.20 (m, 3H), 7.16 (brs, 1H), 7.14-7.07 (m, 2H), 6.16 (dd, *J*= 10.1, 1.9 Hz, 1H), 5.93 (s, 1H), 5.39 (s, 1H), 4.91 (d, *J* = 5.1 Hz, 1H), 4.77 (brs, 1H), 4.65-4.55 (m, 2H), 4.49 (d, *J* = 19.4 Hz, 1H), 4.39 (s, 2H), 4.33-4.25 (m, 2H), 4.17 (d, *J*= 19.5 Hz, 1H), 3.94-3.89 (m, 4H), 3.80 (d, *J*= 5.6 Hz, 2H), 3.72 (d, *J*= 5.8 Hz, 2H), 2.57-2.52 (m, 2H), 2.30-2.24 (m, 2H), 2.18-2.06 (m, 1H), 2.06-1.89 (m, 2H), 1.83-1.59 (m, 6H), 1.39 (s, 3H), 1.12-0.97 (m, 2H), 0.85 (s, 3H).

## Compound 9

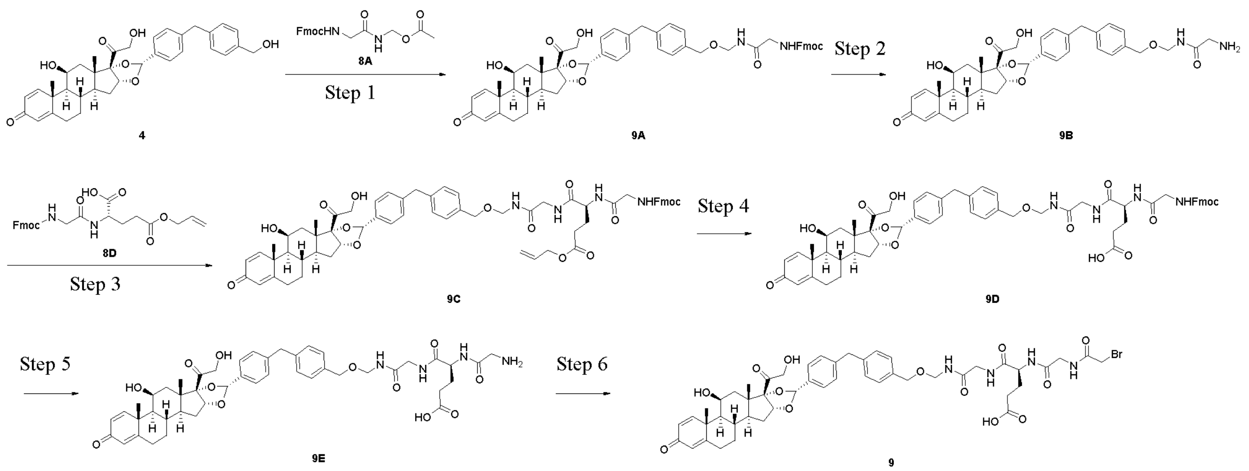


**Step 1** Compound **4** (2.4 g, 4.1 mmol, 1.0 eq) and Compound **8A** (4.5 g, 12.3 mmol, 3.0 eq) were added into a reaction flask, and the reaction mixture was dissolved in THF (30 mL), under nitrogen atmosphere, added with TsOH (1.1 g, 6.6 mmol, 1.6 eq) in an ice-water bath, and reacted at 5-10 °C for 30-40 min. After the reaction was completed

as detected by TLC (PE/EA = 1/2), the reaction solution was added to water, the product was extracted with EA (100 mL), the organic phase was washed with water (50 mL × 3), dried over anhydrous sodium sulfate and concentrated, and the residue was stirred with silica gel and subjected to column chromatography (PE/EA = 2/1) to give the product which was concentrated by rotary evaporation to give a white solid **9A** (1.5 g, yield: 41%); MS-ESI: m/z 893.4 $[M+H]^+$.

**Step 2** Compound **9A** (1.3 g, 1.46 mmol) was dissolved in DCM (13 mL), and the reaction solution was added dropwise with DEA (2.6 mL) in an ice-water bath under nitrogen atmosphere, and then reacted at room temperature for 2 h after the addition was completed. After the reaction was completed as detected by TLC (PE/EA = 1/2), the reaction solution was concentrated by rotary evaporation, extracted three times with DCM, subjected to reversed-phase column chromatography and lyophilized to give an off-white solid **9B** (350 mg, yield: 36%). MS-ESI: m/z 670.8 $[M+H]^+$.

**Step 3** Compound **9B** (350 mg, 0.52 mmol, 1.0 eq) and Compound **8D** (243 mg, 0.52 mmol, 1.0 eq) were added into a reaction flask, and the reaction solution was dissolved in DMF (3.5 mL), added with DIEA (168 mg, 1.3 mmol, 2.5 eq) in an ice water-bath under nitrogen atmosphere, then added dropwise with HATU (240 mg, 0.63 mmol, 1.21 eq) in 1 mL DMF, and then stirred at 0-5 °C for 30 min after the addition was completed. After the reaction was completed as detected by LC-MS, the reaction solution was poured into ice water, EA (100 mL) was added, and the product was extracted. The organic phase was washed with water (50 mL × 3), dried over anhydrous sodium sulfate, and concentrated by rotary evaporation to give a foamy yellow solid **9C** (600 mg), the yield was not calculated as crude product exceeds theoretic amount; MS-ESI: m/z 1119.4 $[M+H]^+$.

**Step 4** Under nitrogen atmosphere, Compound **9C** (400 mg, 0.35 mmol, 1.0 eq), $Pd(PPh_3)_4$ (82 mg, 0.071 mmol, 0.2 eq) and *N*-methylmorpholine (353 mg, 3.5 mmol, 10.0 eq) were added into a reaction flask, and the reaction mixture was reacted at room temperature for 1 h under nitrogen atmosphere. After the reaction was completed as detected by LC-MS, the reaction solution was directly purified by reversed-phase column chromatography to give a white solid **9D** (150 mg, yield: 39%). MS-ESI: m/z 1079.4 $[M+H]^+$.

**Step 5** Under nitrogen atmosphere, Compound **9D** (150 mg, 0.14 mmol) was dissolved in DCM (1.5 mL), and the reaction solution was added dropwise with DEA (0.3 mL) in an ice-water bath, and heated to room temperature for 2 h after the addition was completed. After the reaction was completed as detected by LCMS, the reaction solution was directly concentrated by rotary evaporation, extracted three times with DCM, and purified by reversed-phase column chromatography to give a white solid **9E** (60 mg, yield: 50%). MS-ESI: m/z 857.4 $[M+H]^+$.

**Step 6** Compound **9E** (50 mg, 0.058 mmol, 1.0 eq) was dissolved in THF (3 mL), the reaction solution was added with 3 drops of water for dissolving, added with TEA (117 mg, 1.16 mmol, 20.0 eq) in an ice-water bath, stirred for 1 min, then added with bromoacetyl bromide (93 mg, 0.46 mmol, 8.0 eq), and stirred at 0-5 °C for 5 min. After the reaction was completed as detected by LC-MS, the reaction solution was stored with dry ice, subjected to preparative chromatography and lyophilized to give a white solid **9** (11 mg, yield: 19%);

MS-ESI: m/z 977.3 $[M+H]^+$.

$^1$H NMR (400 MHz, DMSO-$d_6$) δ 8.62 (t, *J* = 6.6 Hz, 1H), 8.49 (t, *J* = 5.6 Hz, 1H), 8.26 (t, *J* = 5.8 Hz, 1H), 8.19 (d, *J* = 7.5 Hz, 1H), 7.36 (d, *J* = 7.9 Hz, 2H), 7.30 (d, *J* = 10.1 Hz, 1H), 7.26-7.14 (m, 6H), 6.16 (dd, *J*= 10.1, 1.9 Hz, 1H), 5.93 (s, 1H), 5.39 (s, 1H), 4.91 (d, *J* = 4.9 Hz, 1H), 4.79 (brs, 1H), 4.61-4.54 (m, 2H), 4.49 (d, *J*= 19.4 Hz, 1H), 4.37 (s, 2H), 4.32-4.24 (m, 2H), 4.16 (d, *J*= 19.4 Hz, 1H), 3.95-3.88 (m, 4H), 3.79 (d, *J* = 5.5 Hz, 2H), 3.72 (d, *J* = 5.8 Hz, 2H), 2.58-2.52 (m, 2H), 2.30-2.24 (m, 2H), 2.17-2.05 (m, 1H), 2.05-1.89 (m, 2H), 1.82-1.58 (m, 6H), 1.39 (s, 3H), 1.11-0.96 (m, 2H), 0.85 (s, 3H).

## Compound 10

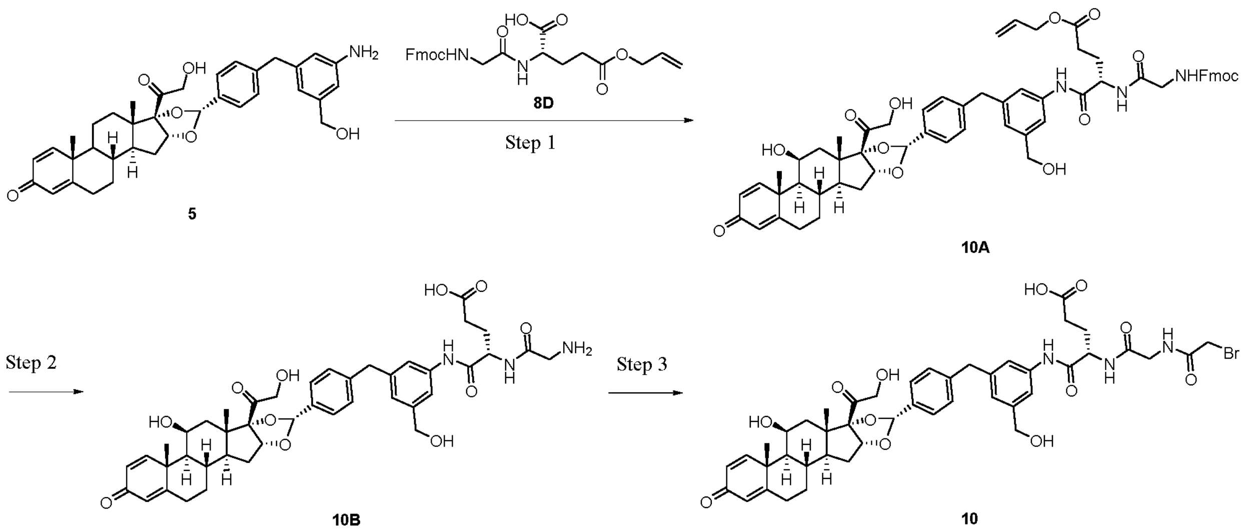


**Step 1** Compound **5** (1.9 g, 3.21 mmol, 1.0 eq), Compound **8D** (1.5 g, 3.21 mmol, 1.0 eq) and HATU (1.6 g, 4.24 mmol, 1.3 eq) were added into a 100-mL three-necked flask, and the reaction mixture was dissolved in DMF (45 mL), added slowly with 2,6-lutidine (1.4 g, 12.72 mmol, 4.0 eq). After the starting material was consumed completely as detected by LCMS, the reaction solution was added slowly and dropwise to water (300 mL) and filtered to give the product which was dried by oil pump under vacuum to give a yellow solid **10A** (3.3 g, yield: 94%). MS-ESI: m/z 1048.4 $[M+H]^+$.

**Step 2** Compound **10A** (1.8 g, 1.72 mmol, 1.0 eq) and $Pd(PPh_3)_4$ (392 mg, 0.34 mmol, 0.2 eq) were added into a 50-mL three-necked flask, the reaction mixture was dissolved in dichloromethane (30 mL), under nitrogen atmosphere, added slowly with *N*-methylmorpholine (868 mg, 8.6 mmol, 5.0 eq) and reacted for 0.5 h. After the reaction was completed as detected by LCMS, diethylamine (6 mL) was added slowly into the three-necked flask and stirred at room temperature for 1 h. After the reaction was completed as detected by LCMS, the reaction solution was added with DMF (20 mL), subjected to rotary evaporation under vacuum at room temperature until 20 mL of the solvent remained, then the rotary evaporation was stopped, and the residue was subjected to reversed-phase column chromatography (ACN in water from 30%-70%) and lyophilized to give a light yellow solid **10B** (455 mg, yield: 34%).

**Step 3** Compound **10B** (150 mg, 0.19 mmol, 1.0 eq) was dissolved in THF (5 mL) and water (0.2 mL), triethylamine (190 mg, 1.9 mmol, 10 eq) was added, and the reaction solution was cooled to 0 °C in an ice-water bath. Bromoacetyl bromide (153 mg, 0.76 mmol, 4.0 eq) was dissolved in 1 mL THF, and then the resulting solution was added slowly to the above reaction solution and reacted at 0 °C for 15 min. After the reaction was completed as detected by LCMS, the reaction solution was subjected to preparative chromatography to give a white solid **10** (20 mg, yield: 12%).

MS-ESI: m/z 906.3 $[M+H]^+$.

$^1H$ NMR (400 MHz, DMSO-$d_6$) $\delta$ 9.87 (s, 1H), 8.52 (s, 1H), 8.22 (d, *J* = 7.6 Hz, 1H), 7.43 (s, 1H), 7.38 (d, *J*= 7.9 Hz, 2H), 7.31 (d, *J*= 9.7 Hz, 2H), 7.23 (d, *J*= 7.9 Hz, 2H), 6.87 (s, 1H), 6.16 (d, *J*= 10.3 Hz, 1H), 5.93 (s, 1H), 5.39 (s, 1H), 4.91 (d, *J*= 5.1 Hz, 1H), 4.78 (s, 1H), 4.49 (d, *J*= 19.2 Hz, 1H), 4.41 (s, 2H), 4.39-4.32 (m, 1H), 4.29 (s, 1H), 4.17 (d, *J* = 19.5 Hz, 1H), 3.93 (s, 2H), 3.88 (s, 2H), 3.82-3.77 (m, 2H), 2.59-2.53 (m, 1H), 2.35-2.21 (m, 4H), 2.04-1.92 (m, 2H), 1.84-1.63 (m, 6H), 1.39 (s, 3H), 1.09-0.99 (m, 2H), 0.86 (s, 3H).

## Compound 11

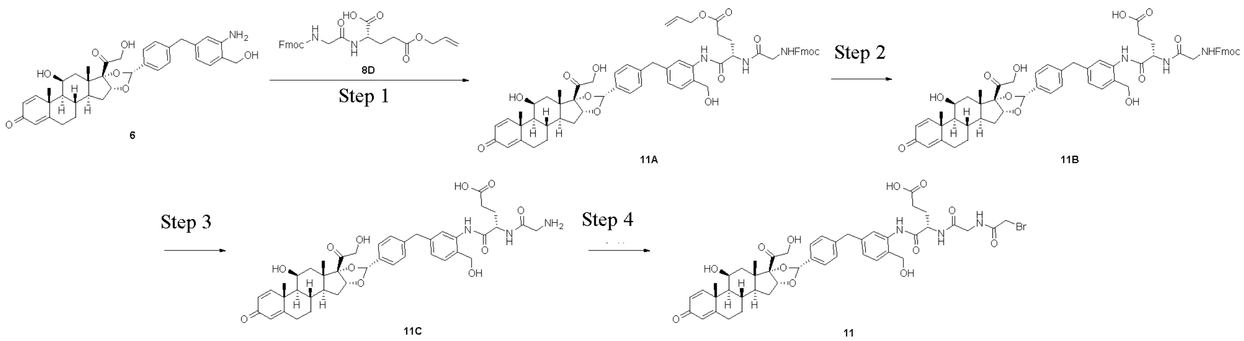


**Step 1** Compound **6** (300 mg, 0.5 mmol, 1.0 eq), Compound **8D** (233 mg, 0.5 mmol, 1.0 eq) and HATU (228 mg, 0.6 mmol, 1.2 eq) were added into a 25-mL three-necked flask, and the reaction mixture was dissolved in DMF (5 mL), and added slowly with 2,6-lutidine (150 mg, 1.4 mmol, 2.8 eq). After the starting material was consumed completely as detected by LCMS, the reaction solution was added slowly and dropwise to water (100 mL) and filtered to give the product which was dried by oil pump under vacuum to give a yellow solid **11A** (450 mg, yield: 86%). MS-ESI: m/z 1048.4 $[M+H]^+$.

**Step 2** Compound **11A** (450 mg, 0.43 mmol, 1.0 eq) and $Pd(PPh_3)_4$ (92 mg, 0.08 mmol, 0.2 eq) were added into a 50-mL three-necked flask, the reaction mixture was dissolved in THF (10 mL), and under nitrogen atmosphere, the reaction solution was added slowly with *N*-methylmorpholine (200 mg, 1.98 mmol, 4.6 eq) and reacted for 0.5 h. After the reaction was completed as detected by LCMS, the reaction solution was subjected to reversed-phase column chromatography (ACN in water from 30%-70%) and lyophilized to give a light yellow solid **11B** (250 mg, yield: 58%). MS-ESI: m/z 1008.3 $[M+H]^+$.

**Step 3** Compound **11B** (250 mg, 0.25 mmol) was added into a 25-mL three-necked flask, the reaction mixture was dissolved in DCM (10 mL), and under nitrogen atmosphere, the reaction solution was added with diethylamine (2 mL) and reacted for 0.5 h. After the reaction was completed as detected by LCMS, the reaction solution was subjected to reversed-phase column chromatography (ACN in water from 20%-50%) and lyophilized to give a light yellow solid **11C** (130 mg, yield: 67%). MS-ESI: m/z 786.4 $[M+H]^+$.

**Step 4** Compound **11C** (120 mg, 0.153 mmol, 1.0 eq) was dissolved in THF (5 mL) and water (0.2 mL), triethylamine (155 mg, 1.53 mmol, 10.0 eq) was added, and the reaction solution was cooled to 0 °C in an ice-water bath. Bromoacetyl bromide (123 mg, 0.61 mmol, 4.0 eq) was dissolved in 1 mL THF, and then the resulting solution was added slowly to the above reaction solution and reacted at 0 °C for 15 min. After the reaction was completed as detected by LCMS, the reaction solution was subjected to prep-HPLC to give a white solid **11** (12.2 mg, yield: 9%).

MS-ESI: m/z 906.3 $[M+H]^+$.

$^1H$ NMR (400 MHz, DMSO-$d_6$) $\delta$ 9.51 (s, 1H), 8.58-8.45 (m, 1H), 8.37 (d, *J* = 7.6 Hz, 1H), 7.51-7.46 (m, 1H), 7.40-7.34 (m, 2H), 7.31 (d, *J* = 10.0 Hz, 1H), 7.28-7.19 (m, 3H), 7.01-6.94 (m, 1H), 6.16 (dd, *J* = 10.0, 1.9 Hz, 1H), 5.93 (s, 1H), 5.39 (s, 1H), 4.91 (d, *J* = 5.0 Hz, 1H), 4.77 (brs, 1H), 4.49 (d, *J* = 19.5 Hz, 1H), 4.42 (d, *J* = 3.7 Hz, 1H), 4.39-4.32 (m, 1H), 4.29 (brs, 1H), 4.17 (d, *J* = 19.5 Hz, 1H), 3.98-3.77 (m, 6H), 2.60-2.52 (m, 2H), 2.34-2.28 (m, 2H), 2.18-1.96 (m, 3H), 1.88-1.55 (m, 6H), 1.39 (s, 3H), 1.13-0.98 (m, 2H), 0.85 (s, 3H).

## Compound 12

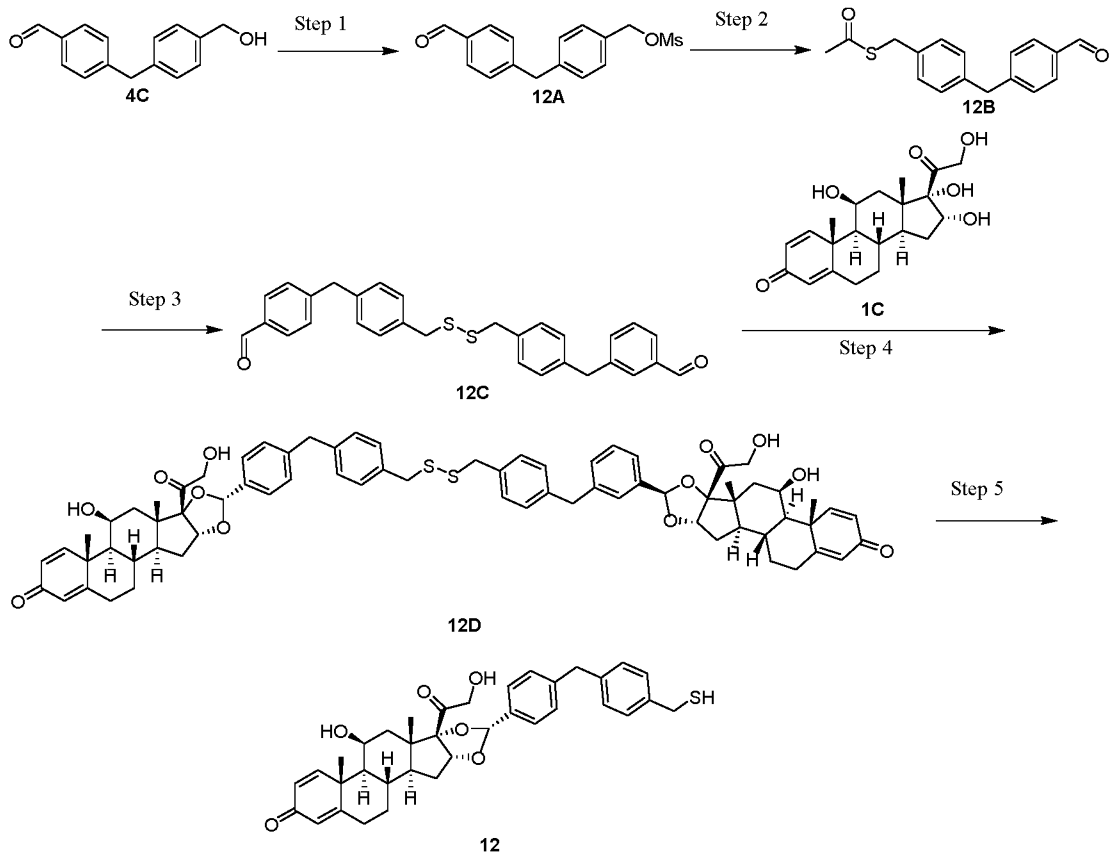


4C 12A 12B 1C 12C 12D 12

**Step 1** Compound **4C** (10.0 g, 44.2 mmol) in DCM (70 mL) was added with TEA (8.94 g, 88.39 mmol, 12.30 mL), and the reaction solution was cooled to 0 °C, added with MsCl (6.08 g, 53.0 mmol, 4.10 mL), and stirred at 25 °C for 12 h. After the starting material was consumed completely as detected by LCMS, the reaction solution was diluted with DCM (50 mL), quenched with water (200 mL), and extracted with DCM (100 mL × 3). The organic phases were combined, washed with saturated brine (100 mL), dried over anhydrous $Na_2SO_4$, and filtered, and the filtrate was concentrated by rotary evaporation to give Compound **12A** (8.60 g, crude) as a yellow oil.

**Step 2** Compound **12A** (8.60 g, 31.58 mmol) in DMF (51.6 mL) was added with thioglycolic acid (7.21 g, 63.15 mmol), and stirred at 25 °C for 5 h. After the starting material was consumed completely as detected by TLC (PE/EA= 5:1), the reaction solution was added with water (100 mL), and extracted with ethyl acetate (100 mL × 3). The organic phases were combined, washed with saturated saline solution and then with 5% aqueous LiCl solution, dried over anhydrous $Na_2SO_4$, and filtered, and the filtrate was concentrated by rotary evaporation to give Compound **12B** (5.75 g, yield: 57.82%, purity: 90.2%) as a brown oil. MS-ESI: m/z 285.2 $[M+H]^+$.

**Step 3** Compound **12B** (12.0 g, 42.2 mmol) was dissolved in methanol (240 mL), and the reaction solution was added with $K_2CO_3$ (8.75 g, 63.3 mmol) and stirred at 20 °C for 3 h. After the starting material was consumed completely as detected by LCMS, the reaction solution was poured into DCM (240 mL), and 0.5 M HCl (100 mL) was added, followed by liquid separation. The organic phase was dried over anhydrous sodium sulfate, and concentrated to give an off-white solid **12C** (10.0 g, yield: 97.7%). MS-ESI: m/z 483.2 $[M+H]^+$.

**Step 4** Compound **12C** (9.00 g, 37.1 mmol), Compound **1C** (13.9 g, 37.1 mmol) and anhydrous magnesium sulfate (13.4 g, 111 mmol) were added to acetonitrile (270 mL), TfOH (16.7 g, 111 mmol, 9.84 mL) was added dropwise at 0 °C, and the reaction solution was gradually heated to 20 °C after the addition was completed, and stirred for 3 h. After the starting material was consumed completely as detected by LCMS, the reaction solution was quenched with saturated aqueous sodium bicarbonate (60 mL), and extracted with ethyl acetate (100 mL × 2). The organic phase was washed with saturated brine (80 mL), dried over anhydrous sodium sulfate, and concentrated to give an off-white solid 12D (15.0 g, yield: 33.6%). MS-ESI: m/z 1199.4 $[M+H]^+$.

**Step 5** Compound **12D** (290 mg, crude) was added to DMF (4 mL), then DL-dithiothreitol (200 mg, 1.30 mmol) was added, and the reaction solution was reacted at room temperature for 16 h under nitrogen atmosphere. After the reaction was completed as detected by LCMS, the reaction solution was directly subjected to prep-HPLC to give a white powdery solid **12** (20 mg, yield: 13.8%). MS-ESI: m/z 601.2 $[M+H]^+$.

$^1H$ NMR (400 MHz, DMSO) δ 7.37 (d, *J*= 8.1 Hz, 2H), 7.31 (d, *J*= 10.1 Hz, 1H), 7.23 (dd, *J* = 8.1, 3.3 Hz, 4H),

7.14 (d, $J$ = 8.0 Hz, 2H), 6.16 (dd, $J$ = 10.1, 1.7 Hz, 1H), 5.93 (s, 1H), 5.39 (s, 1H), 4.91 (d, $J$ = 5.0 Hz, 1H), 4.78 (s, 1H), 4.49 (d, $J$ = 19.4 Hz, 1H), 4.29 (s, 1H), 4.17 (d, $J$ = 19.5 Hz, 1H), 3.88 (d, $J$ = 11.3 Hz, 2H), 3.67 (d, $J$ = 7.6 Hz, 2H), 2.77 (t, $J$ = 7.6 Hz, 1H), 2.50-2.55 (m, 1H), 2.45-2.25 (m, 1H), 2.25-2.05 (m, 2H), 1.80 - 1.53 (m, 5H), 1.38 (s, 3H), 1.14 - 0.94 (m, 2H), 0.86 (s, 3H).

Compound **13**

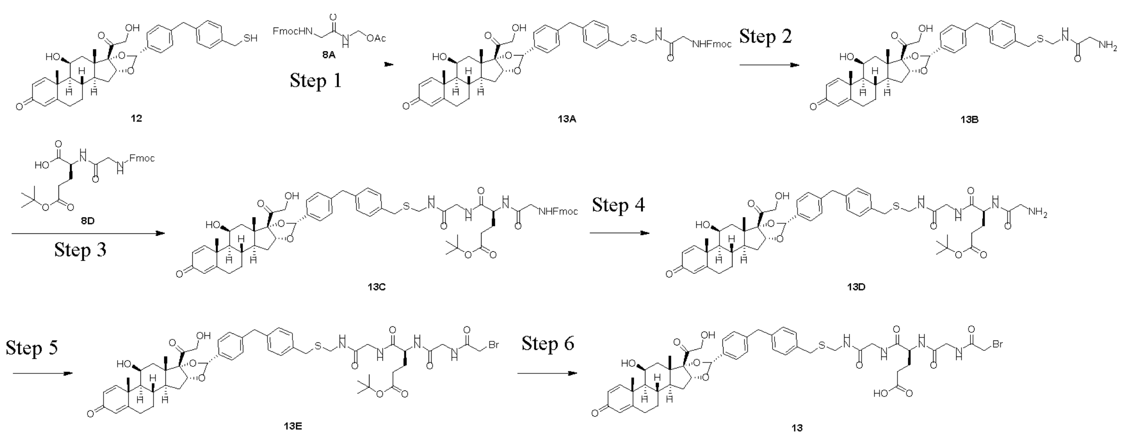


**Step 1** Compound **12** (7.00 g, 11.6 mmol) and Compound **8A** (4.29 g, 11.6 mmol) were added to DMF (49.0 mL), and the reaction solution was added with TfOH (3.50 g, 23.3 mmol, 2.06 mL) and stirred at 25 °C for 16 h. After the starting material was consumed completely as detected by LCMS, the reaction solution was quenched with saturated aqueous sodium bicarbonate (20 mL), and extracted with ethyl acetate (100 mL × 3). The organic phase was washed with saturated brine (100 mL), dried over anhydrous sodium sulfate, and concentrated to give Compound **13A** as a yellow oil (15.0 g, crude product did not calculate yield). MS-ESI: m/z 909.3 $[M+H]^+$.

**Step 2** Compound **13A** (15.0 g, 16.5 mmol) was added to acetonitrile (105 mL), and the reaction solution was added with diethylamine (6.03 g, 82.5 mmol, 8.50 mL) and stirred at 25 °C for 16 h. After the starting material was consumed completely as detected by LCMS, the reaction solution was slurried with acetonitrile (20.0 mL) and filtered, the resulting filter cake was suctioned to dry, and the crude product was subjected to preparative reversed-phase chromatography (water:acetonitrile = 25%-55%, 20 min), and lyophilized to give Compound **13B** as a white solid (0.900 g, yield: 7.94%). MS-ESI: m/z 687.3 $[M+H]^+$.

**Step 3** Compound **13B** (500 mg, 727 μmol) and Compound **8D** (526 mg, 1.09 mmol) were added to DMF (5 mL), and the reaction solution was added with HATU (830 mg, 2.18 mmol) and 2,6-lutidine (56.0 mg, 1.46 mmol, 169 μL), and stirred at 20 °C for 3 h. After the starting material was consumed completely as detected by LCMS, the reaction solution was dried under vacuum to give a crude product (550 mg), 250 mg of which was subjected to preparative reversed-phase chromatography to give Compound **13C** (100 mg, yield: 26.2%) as a white solid. MS-ESI: m/z 1151.4 $[M+H]^+$.

**Step 4** Compound **13C** (50.0 mg, 43.4 μmol) was added to acetonitrile (1 mL), and the reaction solution was added with diethylamine (15.8 mg, 217 μmol, 22.3 μL) and stirred at 25 °C for 6 h. After the starting material was consumed completely as detected by LCMS, the reaction solution was concentrated by rotary evaporation and slurried with methyl tert-butyl ether (3.00 mL) to give Compound **13D** as a yellow solid (40.0 mg, yield: 99.1%). MS-ESI: m/z 929.2 $[M+H]^+$.

**Step 5** EEDQ (31.9 mg, 129 μmol) and bromoacetic acid (11.3 mg, 81.8 μmol) were dissolved in DMF (0.8 mL), and the reaction solution was stirred at room temperature for 1 h, added with Compound **13D** (40.0 mg, 43.0 μmol), and stirred at 25 °C for 2 h. After the starting material was consumed completely as detected by LCMS, the reaction solution was concentrated by rotary evaporation to give Compound **13E** as a yellow solid (the crude product was directly used in the next step). MS-ESI: m/z 1049.2 $[M+H]^+$.

**Step 6** Compound **13E** (45.0 mg, 42.8 μmol) was dissolved in DCM (1.50 mL), and the reaction solution was added with trifluoroacetic acid (770 mg, 6.75 mmol, 500 μL) and stirred at 25 °C for 1 h. After the starting material was consumed completely as detected by LCMS, the reaction solution was concentrated by rotary evaporation, then the crude product was purified by preparative reversed-phase chromatography to give Compound **13** (3.06 mg, yield: 7.18%) as a yellow solid. MS-ESI: m/z 993.2 $[M+H]^+$.

$^1$HNMR (400 MHz, DMSO-$d_6$) $\delta$ 7.83 (br d, 1H, $J$= 7.6 Hz), 7.63 (br d, 1H, $J$= 7.2 Hz), 7.4 (m, 2H), 7.26 (br d, 2H, $J$ = 8.0 Hz), 7.16 (br d, 1H, $J$ = 7.6 Hz), 4.92 (br s, 1H), 4.48 (br s, 1H), 4.30 (br s, 2H), 4.1-4.3 (m, 2H), 4.02 (br d, 1H, $J$ = 13.6 Hz), 3.92 (br d, 2H, $J$ = 8.4 Hz), 3.81 (br d, 2H, $J$ = 5.2 Hz), 3.74 (br s, 3H), 3.63 (br s, 2H), 2.91 (s, 2H),

2.75 (br s, 1H), 2.3-2.4 (m, 2H), 2.27 (br s, 2H), 2.1-2.1 (m, 1H), 2.02 (br s, 1H), 1.92 (br s, 1H), 1.77 (br s, 3H), 1.3-1.5 (m, 8H), 1.25 (s, 2H), 1.18 (br d, 1H, *J* = 7.2 Hz), 1.02 (br s, 1H), 0.8-1.0 (m, 2H).

Compound **14 (Reference Compound P1)**:

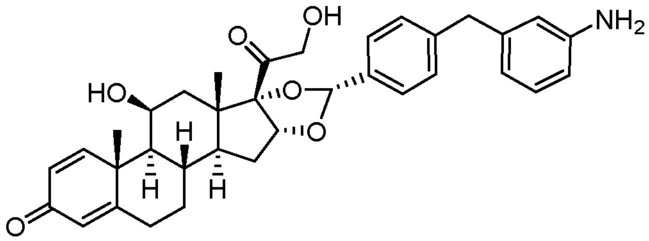

It was synthesized according to the reference patent CN109476699A.

Compound **15 (Reference Compound LP1)**:

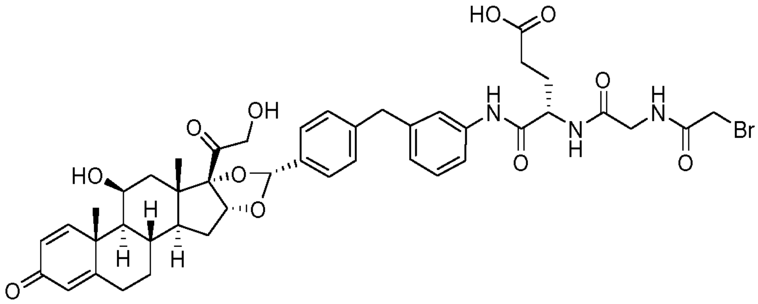

It was synthesized according to the reference patent CN111465399A.

Compound **16 (Reference Compound LP2)**:

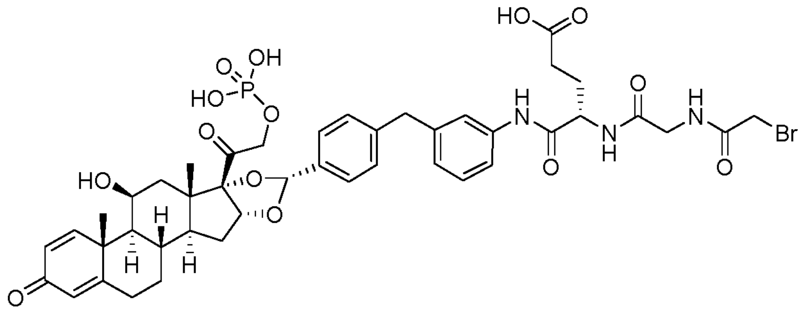

It was synthesized according to the reference patent CN111417410A.

**Example 2**

In the present application, an aqueous buffer of 20 mM histidine (pH = 5.5) was obtained by adjusting the pH of an aqueous 20 mM Histidine solution to 5.5 with HOAc. Stock solution **1:** 10 mM EDTA buffer, pH = 4.7; stock solution **2:** 800 mM sodium citrate aqueous buffer, pH = 8.4; stock solution **3:** 500 mM aqueous urea solution; stock solution **4:** 10 mM aqueous tris(2-carboxyethyl)phosphine solution.

The protein purity of the ADC in the present application was determined by SEC method, and the parameters are shown below.

| | |
|---|---|
| Instrument | Agilent Technologies 1260 |

(continued)

| | | |
|---|---|---|
| Chromatography column | TOSON - G3000SWXL, 300*7.8mm, 5μm | |
| Column temperature | 22 °C | |
| Wavelength | 280 nm | |
| Injection volume | 30 ~ 50 μg | |
| Mobile phase | 0.2 M $K_2HPO_4/KH_2PO_4$, 0.25 M KCl, 15%(v/v) 2-propanol, pH 7.0 | |
| Gradient | Time (min) | Flow rate (mL/min) |
| | 0.0 | 0.75 |
| | 18.0 | 0.75 |

The DAR value of the ADC in the present application can be detected by LC-MS method. The experimental procedure was as follows: to a solution containing 40.0 μg ADC sample was added 75.0 μL 8.0 mol/L guanidine hydrochloride (Gdn-HCl), 5.0 μL 1.0 mol/L Tris-HCl, and 2.0 μL 1 mol/L DTT. The solution was diluted to 100.0 μL with ultrapure water, mixed well and incubated at 22 °C for 30 min. Drug/antibody ratio (DAR) was then analyzed using LC-MS. The LC parameters are shown below.

| | | | | |
|---|---|---|---|---|
| Instrument | Agilent Technologies 1260 | | | |
| Reversed-phase chromatography column | Agilent, PLRP-S 2.1*50 mm, 8 μm | | | |
| Column temperature | 80°C | | | |
| Sample temperature | 2~8°C | | | |
| Detection wavelength | 280nm | | | |
| Injection volume | 10.0 μL | | | |
| Mobile phase | Mobile phase A: 0.025% TFA and 0.1% FA in ultrapure water | | | |
| | Mobile phase B: 0.025% TFA and 0.1% FA in ACN | | | |
| Gradient | Time (min) | %A | %B | Flow rate (mL/min) |
| | 0.0 | 75 | 25 | 0.5 |
| | 0.0 | 75 | 25 | |
| | 0.7 | 66 | 34 | |
| | 5.0 | 55 | 45 | |
| | 6.0 | 10 | 90 | |
| | 7.0 | 10 | 90 | |
| | 7.1 | 75 | 25 | |
| | 10.0 | 75 | 25 | |

The MS parameters are shown below.

| | |
|---|---|
| Instrument | Agilent Technologies 6224 TOF LC/MS |
| Ionization mode | Positive |
| Gas temperature | 350 °C |
| Air flow | 13 L/min |
| Atomizer pressure | 45 psig |
| Capillary voltage | 5000 V |

(continued)

| | |
|---|---|
| Crushing voltage | 250 V |
| Voltage of taper hole | 65 V |
| Peak-to-peak value of radio frequency voltage on octupole | 750 V |
| Acquisition mode | MS (seg) |

| | |
|---|---|
| Mass range | 500-8000 m/z |
| Acquisition rate | 1 spectra/s |
| Acquisition time | 1.4-7.0 min |

The DAR value of the ADC in the present application can also be detected by HIC method, and the parameters are shown below.

<table>
<tr><td>Chromatography column</td><td colspan="3">TSKgel Butyl-NPR, 4.6mm *3.5cm, 2.5μm</td></tr>
<tr><td>Column temperature</td><td colspan="3">25 °C</td></tr>
<tr><td>Detection wavelength</td><td colspan="3">280 nm</td></tr>
<tr><td>Sample load</td><td colspan="3">8 μL</td></tr>
<tr><td rowspan="2">Mobile phase</td><td colspan="3">Mobile phase A: 1.5 M $(NH_4)_2SO_4$, 0.05 M $K_2HPO_4.3H_2O$ (pH = 7.0)</td></tr>
<tr><td colspan="3">Mobile phase B: 21.3 mM $KH_2PO_4$, 28.6 mM $K_2HPO_4.3H_2O$, 25% (V/V) IPA (pH = 7.0)</td></tr>
<tr><td rowspan="2">Gradient</td><td>Time (min)</td><td>% B</td><td>Flow rate (mL/min)</td></tr>
<tr><td>0.0<br>2.0<br>15.0<br>16.0<br>17.0<br>20.0</td><td>0.0<br>0.0<br>100.0<br>100.0<br>0.0<br>0.0</td><td>0.6</td></tr>
</table>

Murine anti-TNFα mIgG2a 8c11: the light chain amino acid sequence is set forth as SEQ ID NO: 31 and the heavy chain amino acid sequence is set forth as SEQ ID NO: 32, prepared with reference to WO2015191783A2.

Antibody Adalimumab (Humira): the light chain amino acid sequence is set forth as SEQ ID NO: 9 and the heavy chain amino acid sequence is set forth as SEQ ID NO: 10, prepared with reference to US6090382A.

Antibody Litifilimab (BIIB059): the light chain amino acid sequence is set forth as SEQ ID NO: 42 and the heavy chain amino acid sequence is set forth as SEQ ID NO: 37, prepared with reference to CN105452295B.

Antibody Iscalimab: the light chain amino acid sequence is set forth as SEQ ID NO: 19 and the heavy chain amino acid sequence is set forth as SEQ ID NO: 20, prepared with reference to CN105949314B.

2.1 Conjugate 1 (Sell-conjugate 1)

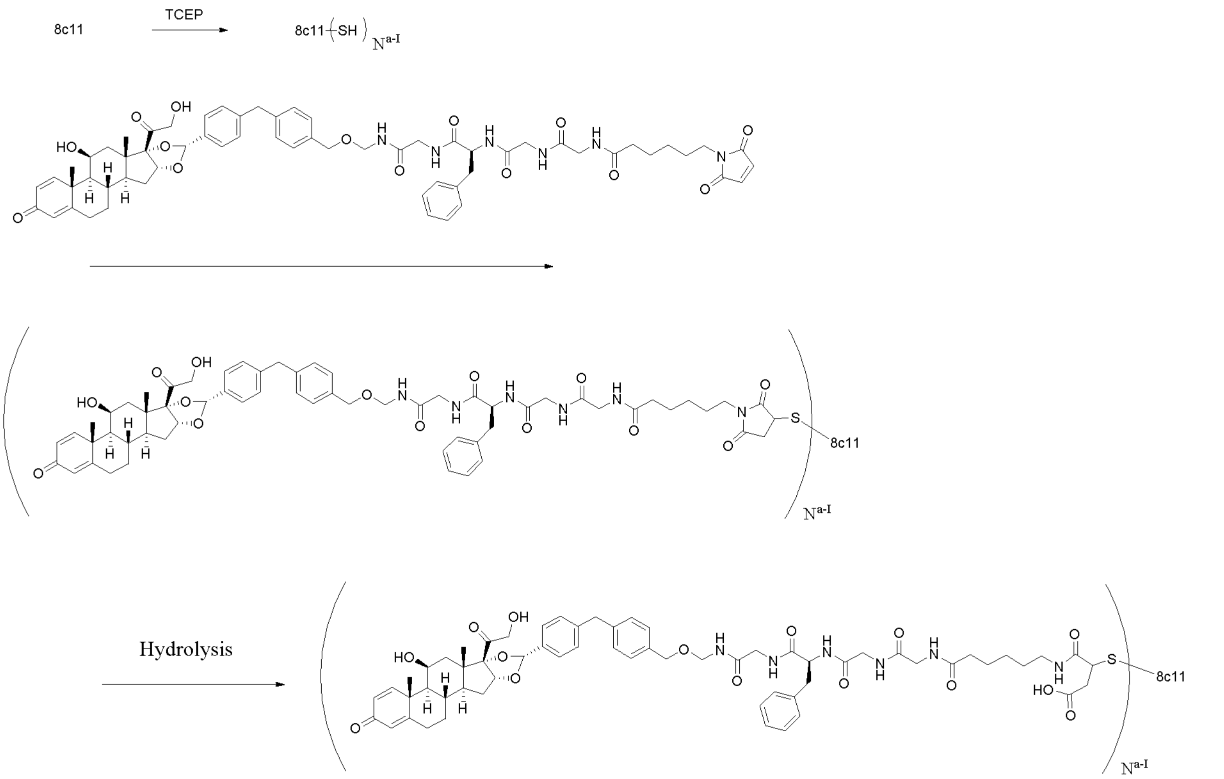


To an aqueous PB buffer (0.05 M PBS buffer at pH = 6.5; 2.5 mL, 9.96 mg/mL, 0.168 nmol) of antimouse $TNF_{\alpha}$ mIgG2a 8c11 was added prepared aqueous solution of tris(2-carboxyethyl)phosphine (10 mM, 0.054mL, 0.54 nmol) at 37 °C, and the reaction solution was placed in a water bath shaker, and shaken and reacted at 37 °C for 3 h before the reaction was stopped; the reaction solution was cooled to 25 °C in a water bath, diluted to 5.0 mg/mL, and 2.0 mL of the solution was taken out and used in the next step.

Compound IV-1 (1.67 mg, 2.02 nmol) was dissolved in 0.10 mL of DMA, and the resulting solution was added to the above solution (2.0 mL), placed in a water bath shaker, and shaken and reacted at 25 °C for 3 h before the reaction was stopped. The reaction solution was desalted and purified through SephadexG25 gel column (eluent: 0.05 M PBS buffer at pH 6.5, containing 0.001 M EDTA) to give PBS buffer (5.0 mg/mL, 1.1 mL) of the exemplary product of formula (Va).

The pH value of the PBS buffer of ADC was adjusted to 9 with 1 M Tris buffer, the resulting solution was incubated at 37 °C for 24 h, and the pH was adjusted to 6.5 with 1 mol/L citrate buffer to give Conjugate 1 (3.3 mg/mL, 5.5 mL). $N^{a-I}$ can be detected by LC-MS.

### 2.2 Conjugate 2 (Sell-conjugate 2)

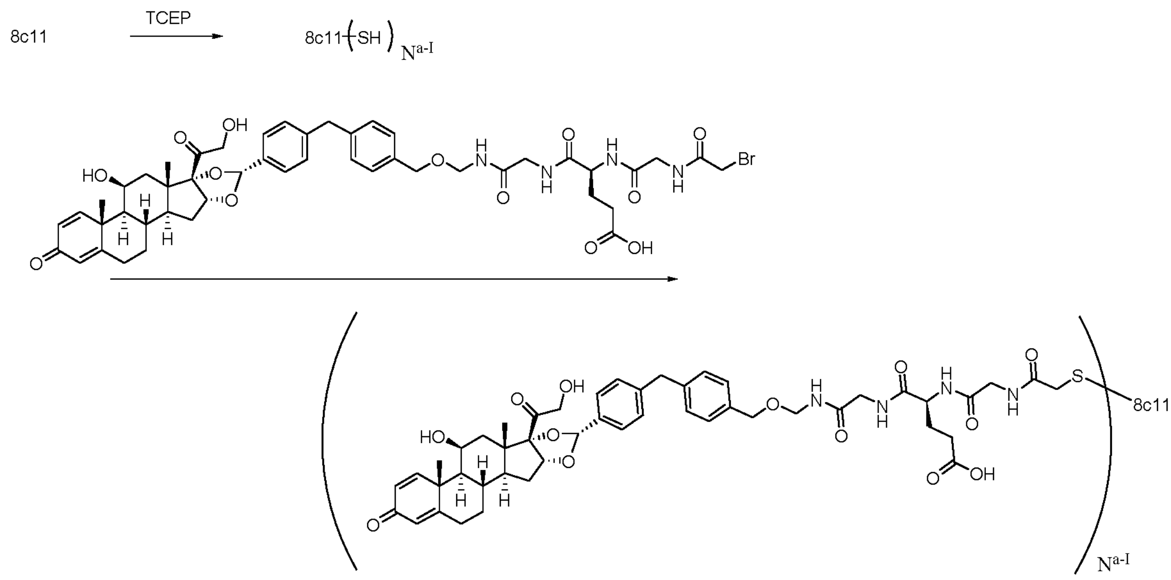


To an aqueous PB buffer (0.05 M PBS buffer at pH = 6.5; 2.5 mL, 9.96 mg/mL, 0.168 nmol) of antibody 8c11 was added prepared aqueous solution of tris(2-carboxyethyl)phosphine (10 mM, 0.054mL, 0.54 nmol) at 37 °C, and the reaction solution was placed in a water bath shaker, and shaken and reacted at 37 °C for 3 h before the reaction was stopped; the reaction solution was cooled to 25 °C in a water bath, diluted to 5.0 mg/mL, and 2.0 mL of the solution was taken out and used in the next step.

Compound IV-12 (1.67 mg, 2.02 nmol) was dissolved in 0.10 mL of DMA, and the resulting solution was added to the above solution (2.0 mL), placed in a water bath shaker, and shaken and reacted at 25 °C for 3 h before the reaction was stopped. The reaction solution was desalted and purified through SephadexG25 gel column (eluent: 0.05 M aqueous histidine buffer at pH 5.5, containing 0.001 M EDTA) to give Conjugate 2 (5.0 mg/mL, 1.1 mL).

$N^{a-I}$ can be detected by LC-MS.

### 2.3 Conjugate 3 (Sell-conjugate 3)

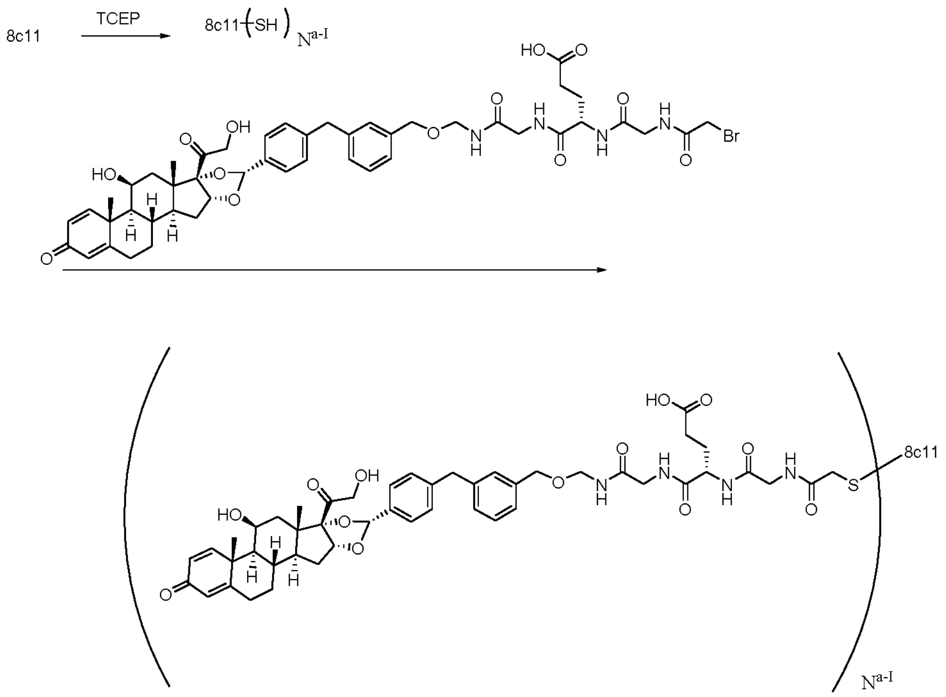

To an aqueous PB buffer (0.05 M PBS buffer at pH = 6.5; 2.5 mL, 9.96 mg/mL, 0.168 nmol) of antibody 8c11 was added prepared aqueous solution of tris(2-carboxyethyl)phosphine (10 mM, 0.054mL, 0.54 nmol) at 37 °C, and the reaction solution was placed in a water bath shaker, and shaken and reacted at 37 °C for 3 h before the reaction was stopped; the reaction solution was cooled to 25 °C in a water bath, diluted to 5.0 mg/mL, and 2.0 mL of the solution was taken out and used in the next step.

Compound IV-11 (1.67 mg, 2.02 nmol) was dissolved in 0.10 mL of DMA, and the resulting solution was added to the above solution (2.0 mL), placed in a water bath shaker, and shaken and reacted at 25 °C for 3 h before the reaction was stopped. The reaction solution was desalted and purified through SephadexG25 gel column (eluent: 0.05 M aqueous histidine buffer at pH 5.5, containing 0.001 M EDTA) to give Conjugate 3 (5.3 mg/mL, 1.5 mL).

N$^{a-I}$ can be detected by LC-MS.

2.4 Conjugate 4 (Sell-conjugate 4)

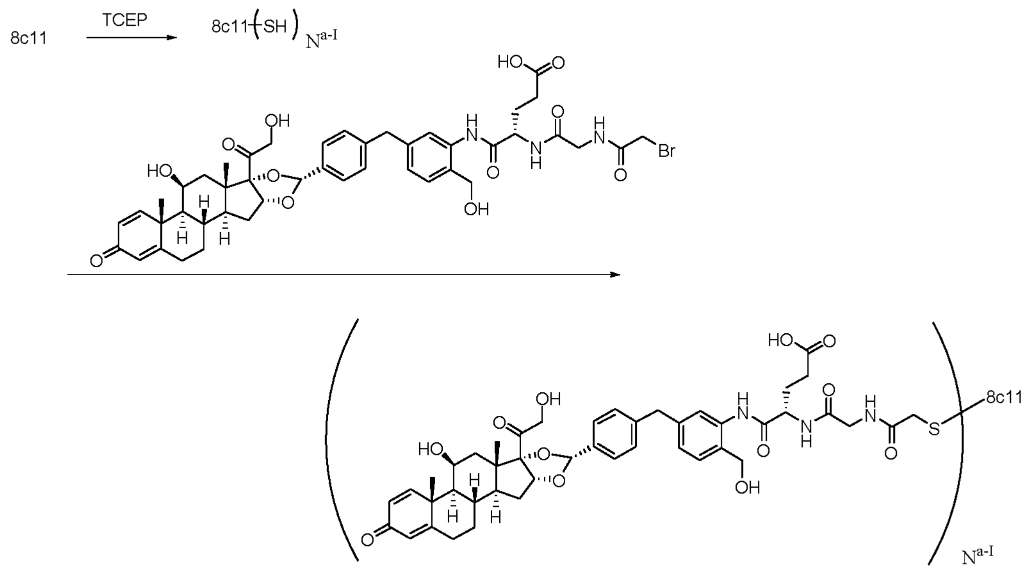


To an aqueous PB buffer (0.05 M PBS buffer at pH = 6.5; 2.5 mL, 9.96 mg/mL, 0.168 nmol) of antibody 8c11 was added prepared aqueous solution of tris(2-carboxyethyl)phosphine (10 mM, 0.054mL, 0.54 nmol) at 37 °C, and the reaction solution was placed in a water bath shaker, and shaken and reacted at 37 °C for 3 h before the reaction was stopped; the reaction solution was cooled to 25 °C in a water bath, diluted to 5.0 mg/mL, and 2.0 mL of the solution was taken out and used in the next step.

Compound IV-60 (1.67 mg, 2.02 nmol) was dissolved in 0.10 mL of DMA, and the resulting solution was added to the above solution (2.0 mL), placed in a water bath shaker, and shaken and reacted at 25 °C for 3 h before the reaction was stopped. The reaction solution was desalted and purified through SephadexG25 gel column (eluent: 0.05 M aqueous histidine buffer at pH 5.5, containing 0.001 M EDTA) to give Conjugate 4 (4.7 mg/mL, 2.2 mL).

N$^{a-I}$ can be detected by LC-MS.

2.5 Conjugate 5 (Sell-conjugate 5)

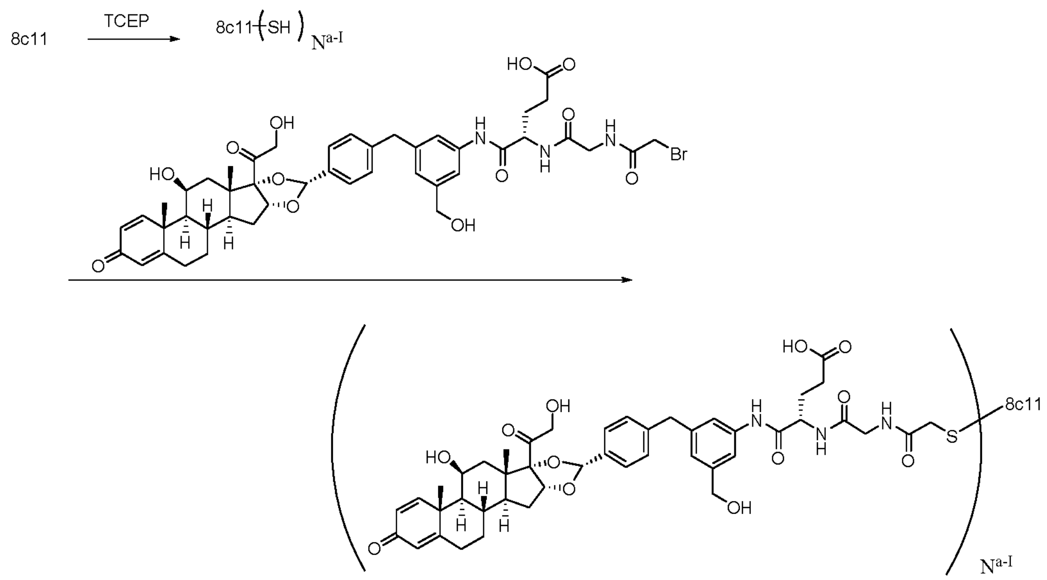


To an aqueous PB buffer (0.05 M PBS buffer at pH = 6.5; 2.5 mL, 9.96 mg/mL, 0.168 nmol) of antibody 8c11 was added prepared aqueous solution of tris(2-carboxyethyl)phosphine (10 mM, 0.054mL, 0.54 nmol) at 37 °C, and the reaction solution was placed in a water bath shaker, and shaken and reacted at 37 °C for 3 h before the reaction was stopped; the reaction solution was cooled to 25 °C in a water bath, diluted to 5.0 mg/mL, and 2.0 mL of the solution was taken out and used in the next step.

Compound IV-81 (1.67 mg, 2.02 nmol) was dissolved in 0.10 mL of DMA, and the resulting solution was added to the above solution (2.0 mL), placed in a water bath shaker, and shaken and reacted at 25 °C for 3 h before the reaction was stopped. The reaction solution was desalted and purified through SephadexG25 gel column (eluent: 0.05 M aqueous histidine buffer at pH 5.5, containing 0.001 M EDTA) to give Conjugate 5 (2.9 mg/mL, 4.3 mL).

$N^{a-I}$ can be detected by LC-MS.

2.6 Conjugate 6 (Sell-conjugate 6)

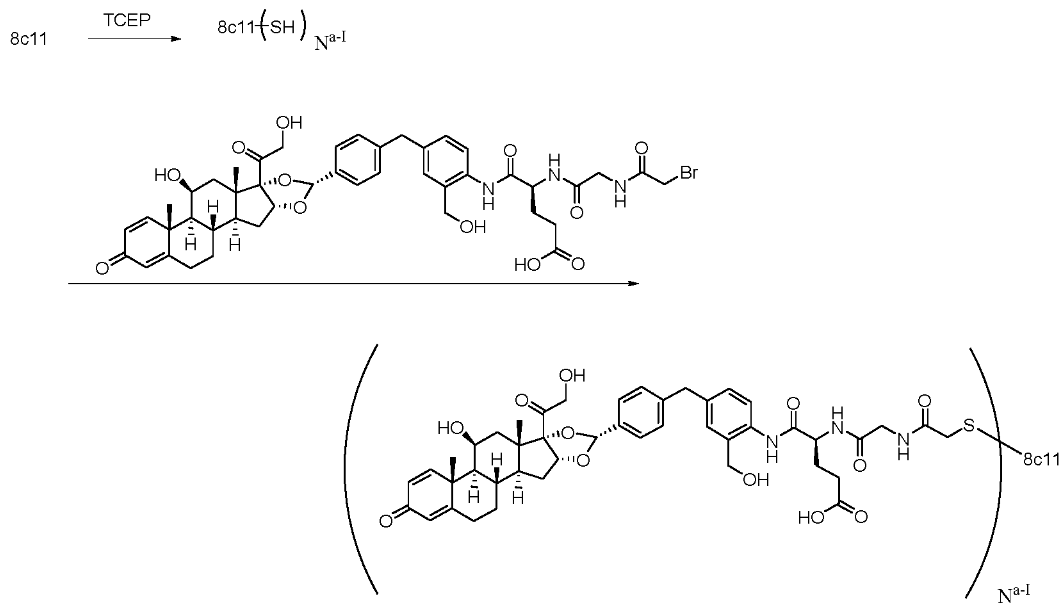


To an aqueous PB buffer (0.05 M PBS buffer at pH = 6.5; 2.5 mL, 9.96 mg/mL, 0.168 nmol) of antibody 8c11 was added prepared aqueous solution of tris(2-carboxyethyl)phosphine (10 mM, 0.054mL, 0.54 nmol) at 37 °C, and the

reaction solution was placed in a water bath shaker, and shaken and reacted at 37 °C for 3 h before the reaction was stopped; the reaction solution was cooled to 25 °C in a water bath, diluted to 5.0 mg/mL, and 2.0 mL of the solution was taken out and used in the next step.

Compound IV-91 (1.67 mg, 2.02 nmol) was dissolved in 0.10 mL of DMA, and the resulting solution was added to the above solution (2.0 mL), placed in a water bath shaker, and shaken and reacted at 25 °C for 3 h before the reaction was stopped. The reaction solution was desalted and purified through SephadexG25 gel column (eluent: 0.05 M aqueous histidine buffer at pH 5.5, containing 0.001 M EDTA) to give Conjugate 6 (3.6 mg/mL, 5.5 mL).

$N^{a-I}$ can be detected by LC-MS.

2.7 Conjugate V-6

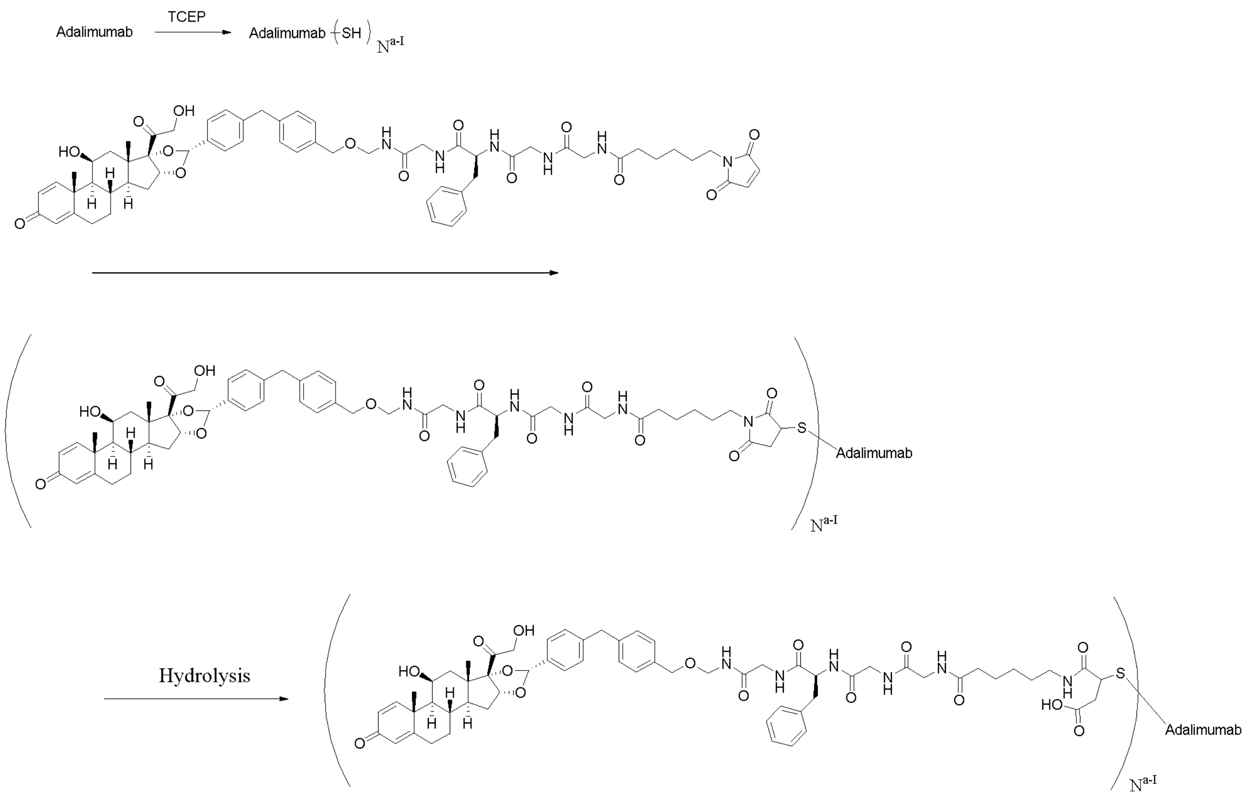


To an aqueous PB buffer (0.05 M PBS buffer at pH = 6.5; 2.5 mL, 9.96 mg/mL, 0.168 nmol) of antibody Adalimumab (Humira) was added prepared aqueous solution of tris(2-carboxyethyl)phosphine (10 mM, 0.054mL, 0.54 nmol) at 37 °C, and the reaction solution was placed in a water bath shaker, and shaken and reacted at 37 °C for 3 h before the reaction was stopped; the reaction solution was cooled to 25 °C in a water bath, diluted to 5.0 mg/mL, and 2.0 mL of the solution was taken out and used in the next step.

Compound IV-1 (1.67 mg, 2.02 nmol) was dissolved in 0.10 mL of DMA, and the resulting solution was added to the above solution (2.0 mL), placed in a water bath shaker, and shaken and reacted at 25 °C for 3 h before the reaction was stopped. The reaction solution was desalted and purified through SephadexG25 gel column (eluent: 0.05 M PBS buffer at pH 6.5, containing 0.001 M EDTA) to give PBS buffer (2.8 mg/mL, 4.7 mL) of the exemplary product of formula (Va).

The pH value of the PBS buffer of ADC was adjusted to 9 with 1 M Tris buffer, the resulting solution was incubated at 37 °C for 24 h, and the pH was adjusted to 6.5 with 1 mol/L citrate buffer to give Conjugate V-6 (3.3 mg/mL, 5.5 mL). $N^{a-I}$ can be detected by LC-MS.

2.8 Conjugate V-13

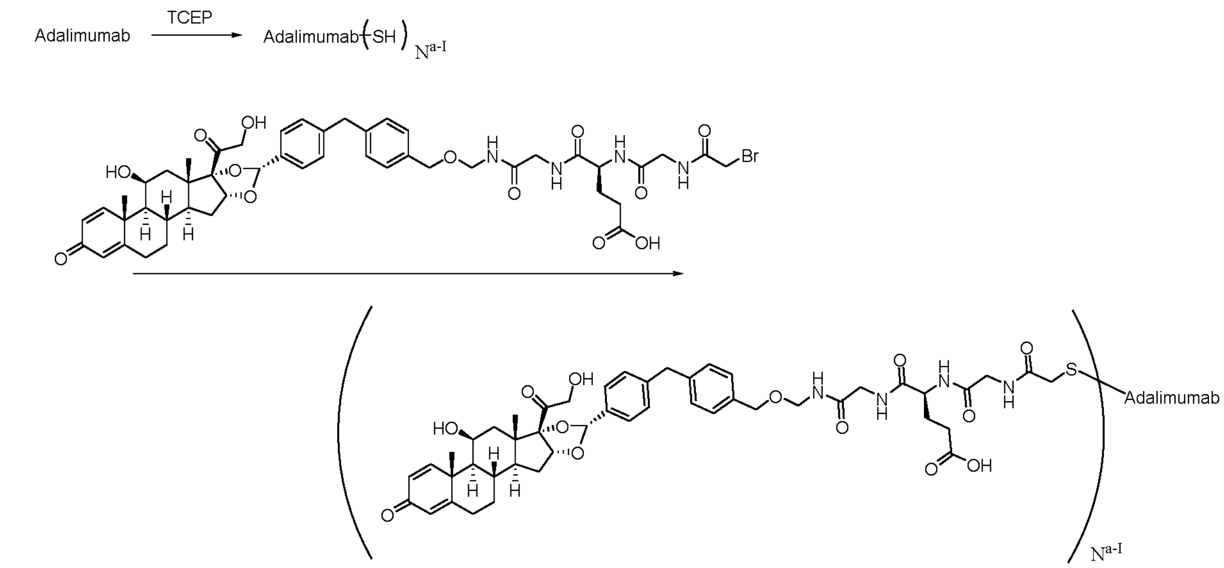


To an aqueous PB buffer (0.05 M PBS buffer at pH = 6.5; 2.5 mL, 9.96 mg/mL, 0.168 nmol) of antibody Humira was added prepared aqueous solution of tris(2-carboxyethyl)phosphine (10 mM, 0.054mL, 0.54 nmol) at 37 °C, the reaction solution was placed in a water bath shaker, and shaken and reacted at 37 °C for 3 h before the reaction was stopped; the reaction solution was cooled to 25 °C in a water bath, diluted to 5.0 mg/mL, and 2.0 mL of the solution was taken out and used in the next step.

Compound IV-12 (1.67 mg, 2.02 nmol) was dissolved in 0.10 mL of DMA, and the resulting solution was added to the above solution (2.0 mL), placed in a water bath shaker, and shaken and reacted at 25 °C for 3 h before the reaction was stopped. The reaction solution was desalted and purified through SephadexG25 gel column (eluent: 0.05 M aqueous histidine buffer at pH 5.5, containing 0.001 M EDTA) to give Conjugate V-13 (3.5 mg/mL, 5.6 mL).

$N^{a-I}$ can be detected by LC-MS.

**2.9 Conjugate V-12**

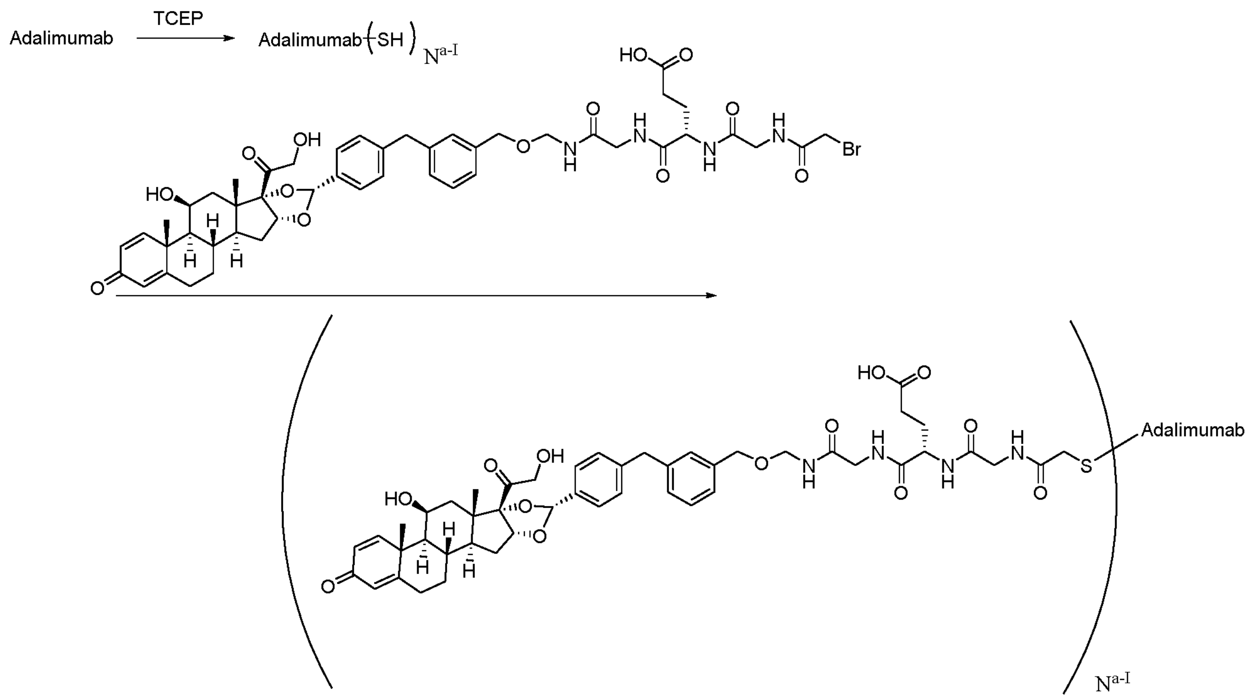

To an aqueous PB buffer (0.05 M PBS buffer at pH = 6.5; 2.5 mL, 9.96 mg/mL, 0.168 nmol) of antibody Humira was added prepared aqueous solution of tris(2-carboxyethyl)phosphine (10 mM, 0.054mL, 0.54 nmol) at 37 °C, the reaction solution was placed in a water bath shaker, and shaken and reacted at 37 °C for 3 h before the reaction was stopped; the reaction solution was cooled to 25 °C in a water bath, diluted to 5.0 mg/mL, and 2.0 mL of the solution was taken out and used in the next step.

Compound IV-11 (1.67 mg, 2.02 nmol) was dissolved in 0.10 mL of DMA, and the resulting solution was added to the above solution (2.0 mL), placed in a water bath shaker, and shaken and reacted at 25 °C for 3 h before the reaction was stopped. The reaction solution was desalted and purified through SephadexG25 gel column (eluent: 0.05 M aqueous histidine buffer at pH 5.5, containing 0.001 M EDTA) to give Conjugate V-12 (4.4 mg/mL, 4.3 mL).

$N^{a-I}$ can be detected by LC-MS.

**2.10 Conjugate V-49**

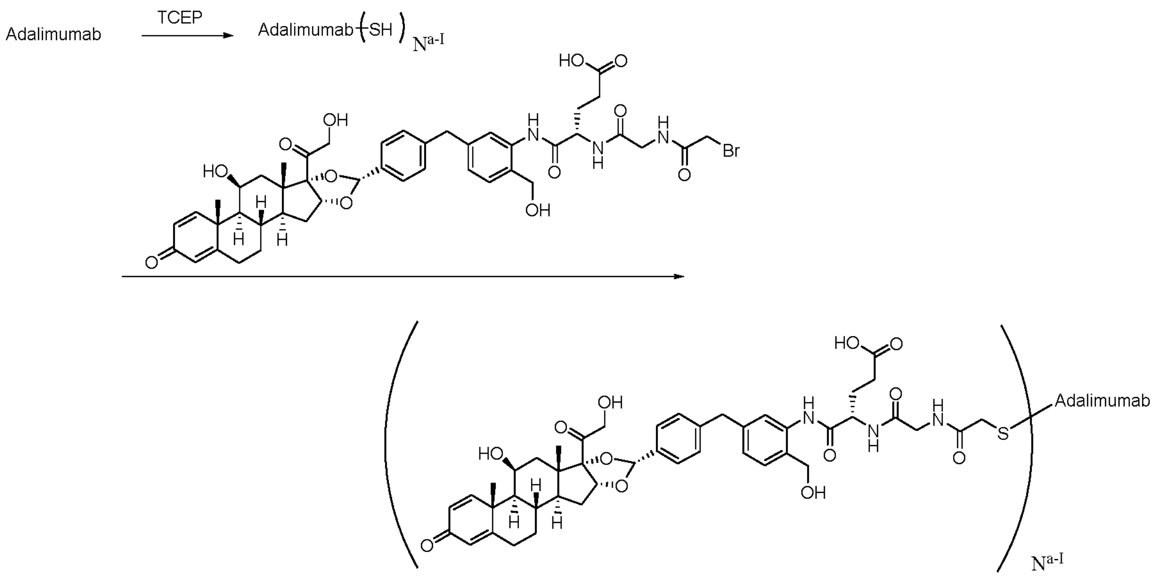


To an aqueous PB buffer (0.05 M PBS buffer at pH = 6.5; 2.5 mL, 9.96 mg/mL, 0.168 nmol) of antibody Humira was added prepared aqueous solution of tris(2-carboxyethyl)phosphine (10 mM, 0.054mL, 0.54 nmol) at 37 °C, the reaction solution was placed in a water bath shaker, and shaken and reacted at 37 °C for 3 h before the reaction was stopped; the reaction solution was cooled to 25 °C in a water bath, diluted to 5.0 mg/mL, and 2.0 mL of the solution was taken out and used in the next step.

Compound IV-60 (1.67 mg, 2.02 nmol) was dissolved in 0.10 mL of DMA, and the resulting solution was added to the above solution (2.0 mL), placed in a water bath shaker, and shaken and reacted at 25 °C for 3 h before the reaction was stopped. The reaction solution was desalted and purified through SephadexG25 gel column (eluent: 0.05 M aqueous histidine buffer at pH 5.5, containing 0.001 M EDTA) to give Conjugate V-53 (2.7 mg/mL, 4.7 mL).

$N^{a-I}$ can be detected by LC-MS.

**2.11 Conjugate V-70 (Humira-coujugate 5)**

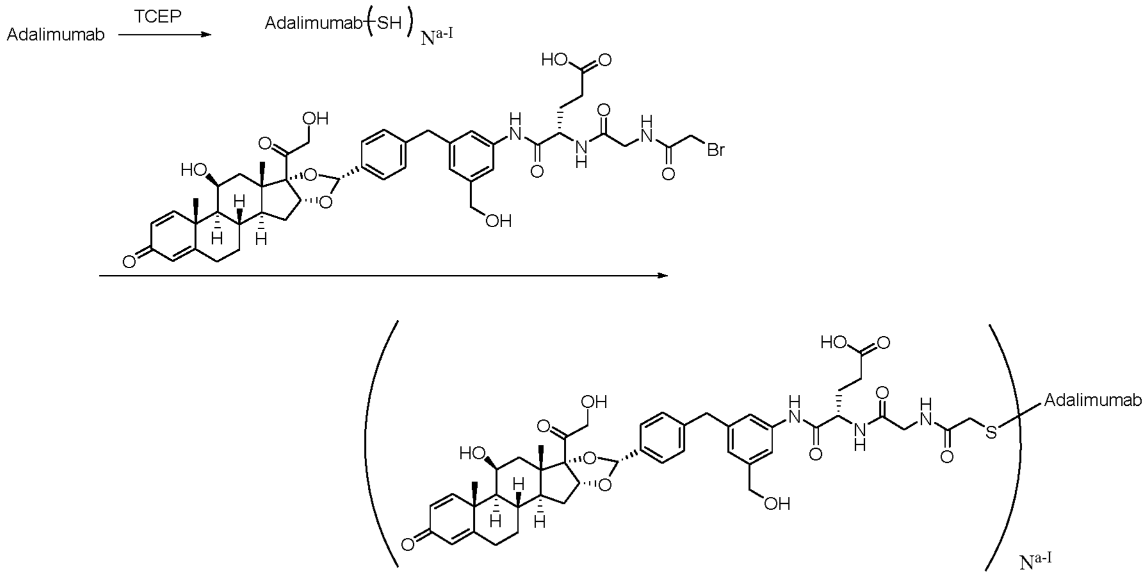


To an aqueous PB buffer (0.05 M PBS buffer at pH = 6.5; 2.5 mL, 9.96 mg/mL, 0.168 nmol) of antibody 8c11 was added prepared aqueous solution of tris(2-carboxyethyl)phosphine (10 mM, 0.054mL, 0.54 nmol) at 37 °C, and the reaction solution was placed in a water bath shaker, and shaken and reacted at 37 °C for 3 h before the reaction was stopped; the reaction solution was cooled to 25 °C in a water bath, diluted to 5.0 mg/mL, and 2.0 mL of the solution was taken out and used in the next step.

Compound IV-81 (1.67 mg, 2.02 nmol) was dissolved in 0.10 mL of DMA, and the resulting solution was added to the above solution (2.0 mL), placed in a water bath shaker, and shaken and reacted at 25 °C for 3 h before the reaction was stopped. The reaction solution was desalted and purified through SephadexG25 gel column (eluent: 0.05 M aqueous histidine buffer at pH 5.5, containing 0.001 M EDTA) to give Conjugate V-74 (2.8 mg/mL, 5.1 mL).

$N^{a-I}$ can be detected by LC-MS.

**2.12 Conjugate 9 (Sell-conjugate 9)**

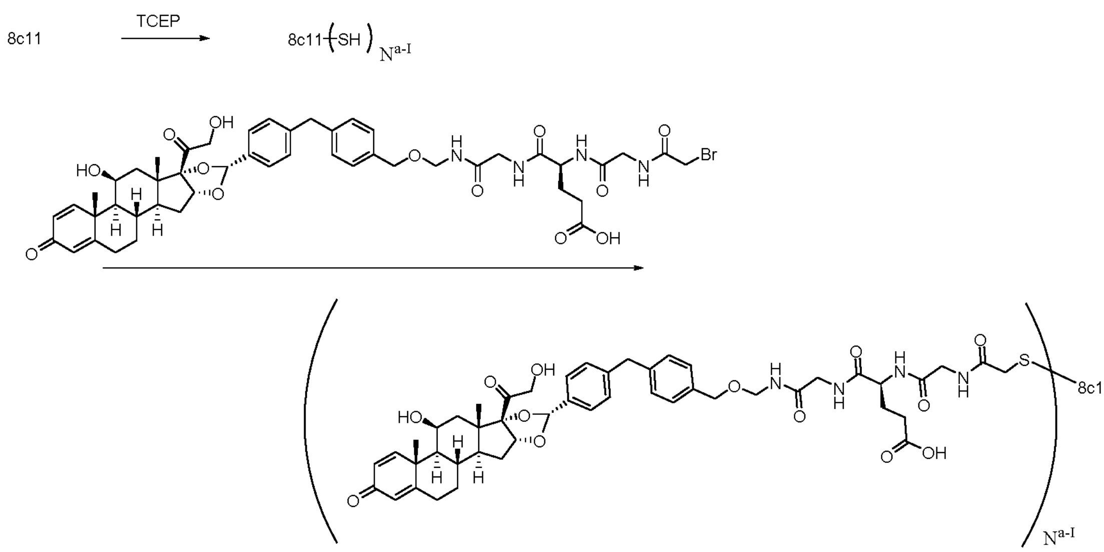


To an aqueous NaOAc buffer (0.05 M aqueous NaOAc buffer at pH = 7.4, containing 0.29 mM EDTA and 11.53 mM sodium citrate; 10.6 mL, 3.4 mg/mL, 0.243 μmol) of antibody 8c11 were added prepared aqueous solution of tris(2-carboxyethyl)phosphine (10 mM, 0.073 mL, 0.729 μmol) and Compound 9 (3.56 mg, 3.645 μmol) in DMSO (0.36 mL) at 22 °C. The resulting solution was placed in a water bath shaker, and shaken and reacted at 22 °C for 3 h before the

reaction was stopped. The reaction solution was added with 1/3 volumes of 100 mM aqueous histidine buffer (pH = 5.5), and desalted and purified through Zeba desalting spin column (eluent: 0.02 M aqueous histidine buffer at pH 5.5) (40K MWCO) to give Conjugate **9-1** (36.94 mg, 4.22 mg/mL, yield: 102%). $N^{a\text{-}I}$ was determined as 3.69 by RP.

Conjugate **9-1** (36.94 mg, 4.22 mg/mL) was mixed with 1.55 mL of Conjugate **10** (3.38 mg/mL, 5.24 mg, RP-DAR 6.13) to give Conjugate **9** (39 mg, 4.22 mg/mL). $N^{a\text{-}I}$ was determined as 4.05 by RP.

**2.13 Conjugate 10 (Sell-conjugate 10)**

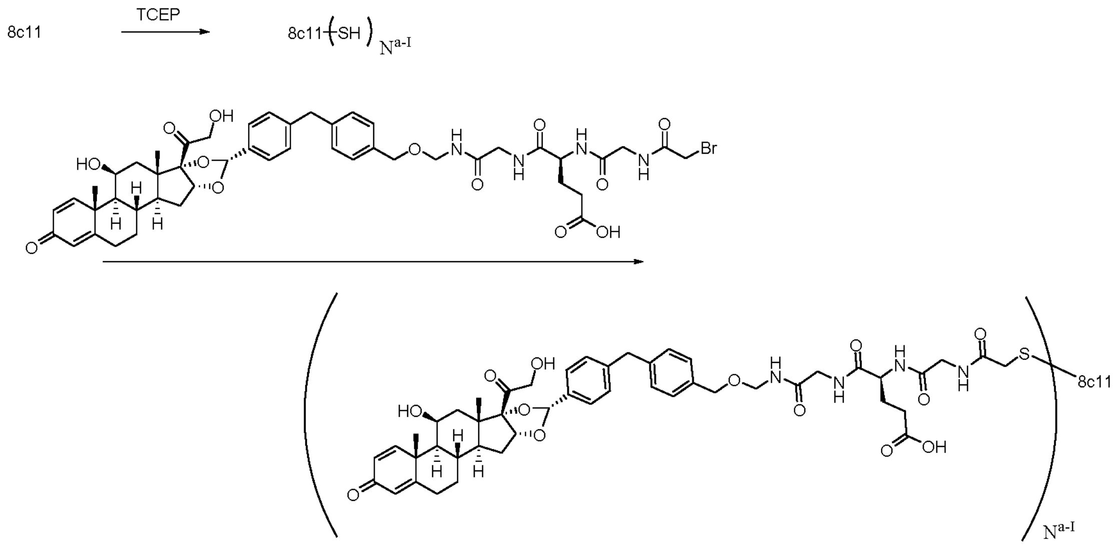


To an aqueous NaOAc buffer (0.05 M aqueous NaOAc buffer at pH = 7.4, containing 0.29 mM EDTA and 11.53 mM sodium citrate; 13.2 mL, 3.4 mg/mL, 0.303 μmol) of antibody 8c11 were added prepared aqueous solution of tris(2-carboxyethyl)phosphine (10 mM, 0.164 mL, 1.64 μmol) and Compound 9 (4.44 mg, 4.545 μmol) in DMSO (0.44 mL) at 22 °C. The resulting solution was placed in a water bath shaker, and shaken and reacted at 22 °C for 3 h before the reaction was stopped. The reaction solution was added with 1/3 volumes of 100 mM aqueous histidine buffer (pH = 5.5), and desalted and purified (eluent: 0.02M aqueous histidine buffer at pH 5.5) using a Zeba desalting spin column (40K MWCO) to give Conjugate **10** (39 mg, 3.38 mg/mL, yield: 87%).

$N^{a\text{-}I}$ was determined as 6.13 by RP.

**2.14 Conjugate 11 (Sell-conjugate 11)**

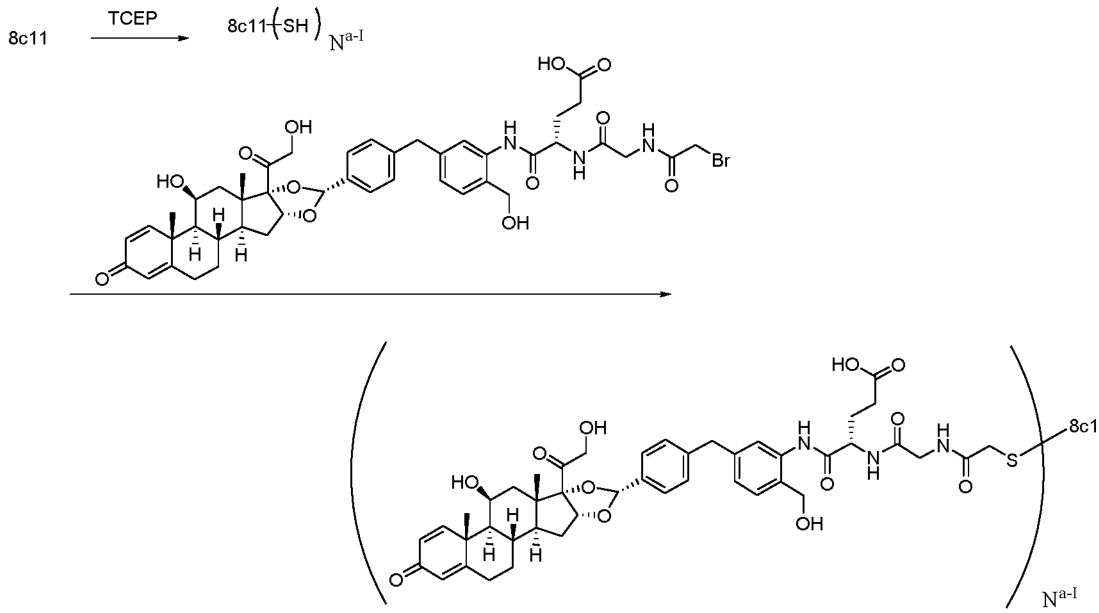


To an aqueous NaOAc buffer (0.05 M aqueous NaOAc buffer at pH = 5.5, containing 0.29 mM EDTA and 11.53 mM sodium citrate; 11.9 mL, 3.4 mg/mL, 0.273 μmol) of antibody 8c11 were added prepared aqueous solution of tris(2-carboxyethyl)phosphine (10 mM, 0.123 mL, 1.23 μmol) and Compound 11 (3.72 mg, 4.10 μmol) in DMSO (0.37 mL) at 22 °C. The resulting solution was placed in a water bath shaker, and shaken and reacted at 22 °C for 3 h before the reaction was stopped. The reaction solution was added with 1/3 volumes of 100 mM aqueous histidine buffer (pH = 5.5), and desalted and purified (eluent: 0.02M aqueous histidine buffer at pH 5.5) using a Zeba desalting spin column (40K MWCO) to give Conjugate **11** (20 mg, 2.48 mg/mL, yield: 49%).

$N^{a-I}$ was determined as 4.58 by RP.

**2.15 Conjugate 12 (Sell-conjugate 12)**

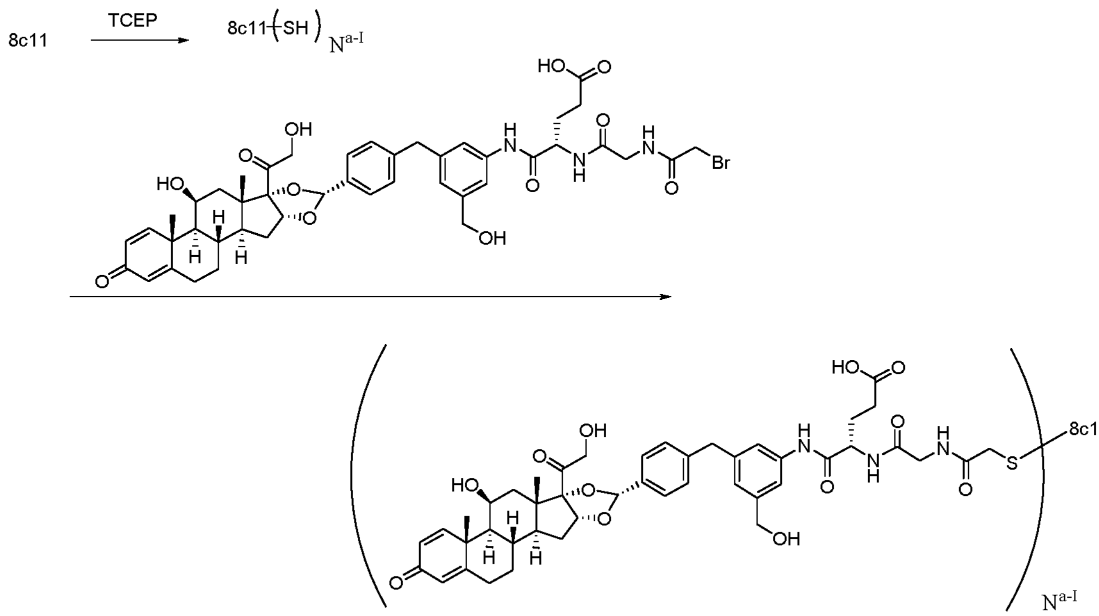


To an aqueous NaOAc buffer (0.05 M aqueous NaOAc buffer at pH = 5.5; 3.8 mL, 11.3 mg/mL, 0.290 μmol) of antibody 8c11 was added prepared aqueous solution of tris(2-carboxyethyl)phosphine (10 mM, 0.103 mL, 1.03 μmol) at 22 °C. The resulting solution was placed in a water bath shaker, and shaken and reacted at 22 °C for 3 h before the

reaction was stopped. Stock solution **1** and stock solution **2** were added such that the buffer system could contain 0.29 mM EDTA and 11.53 mM sodium citrate.

Compound 10 (4.73 mg, 5.22 μmol) was dissolved in 0.47 mL of DMSO, the resulting solution was added to the above solution, placed in a water bath shaker, and shaken and reacted at 22 °C for 9 h before the reaction was stopped. The reaction solution was added with 1/3 volumes of 800 mM aqueous sodium citrate buffer (pH = 5.5), and desalted and purified (eluent: 0.02 M aqueous histidine buffer at pH 5.5) using a Zeba desalting spin column (40K MWCO) to give Conjugate **12** (35.39 mg, 2.17 mg/mL, yield: 82%).

$N^{a-l}$ was determined as 4.21 by RP.

## 2.16 Conjugate 13 (Sell-conjugate 13)

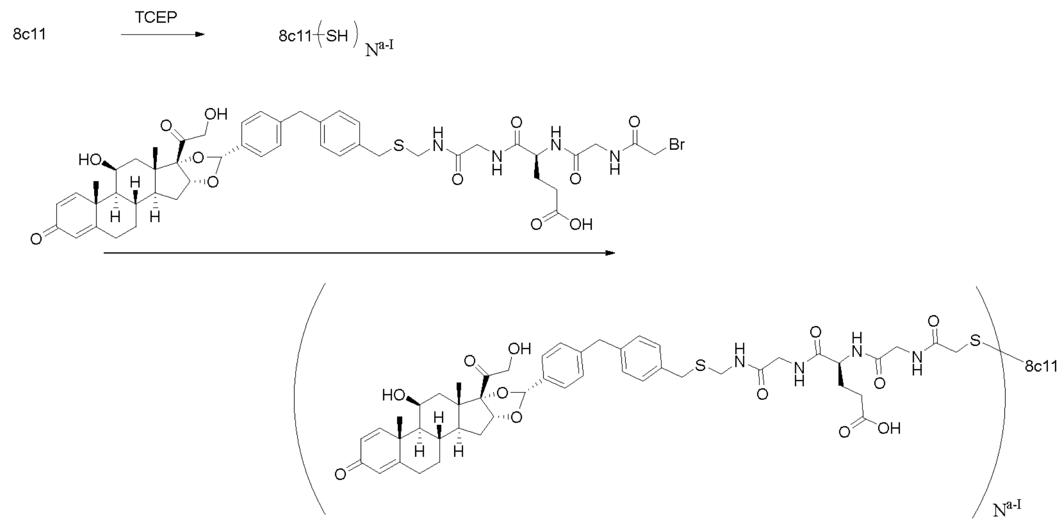


To an aqueous NaOAc buffer (0.05 M aqueous NaOAc buffer at pH = 5.5, containing 0.29 mM EDTA and 11.53 mM sodium citrate; 2.5 mL, 3.4 mg/mL, 0.057 μmol) of antibody 8c11 was added prepared aqueous solution of tris(2-carboxyethyl)phosphine (10 mM, 0.026 mL, 0.26 μmol) at 22 °C. The resulting solution was placed in a water bath shaker, and shaken and reacted at 22 °C for 3 h before the reaction was stopped.

Compound **13** (1.02 mg, 1.026 μmol) was dissolved in 0.10 mL of DMSO, and the resulting solution was added to the above solution, placed in a water bath shaker, and shaken and reacted at 22 °C for 6 h before the reaction was stopped. The reaction solution was desalted and purified (eluent phase: 0.02 M aqueous histidine buffer at pH 5.5) using a Zeba desalting spin column (40K MWCO) to give Conjugate **13** (5.8 mg, 1.72 mg/mL, yield: 68%).

$N^{a-l}$ was determined as 3.76 by LC-MS.

## 2.17 Conjugate 14 (Sell-conjugate 14, reference ADC1)

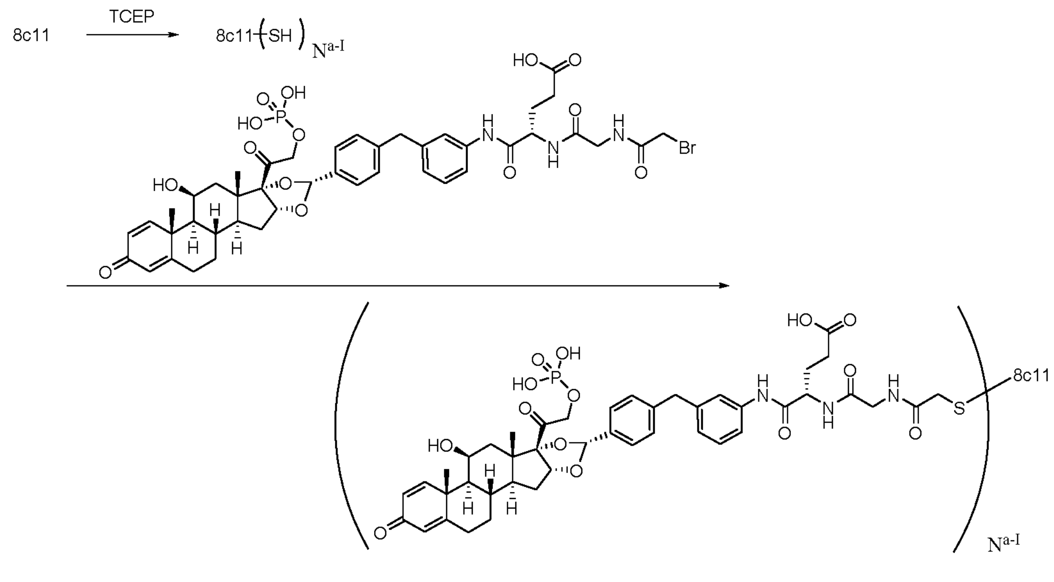


To an aqueous NaOAc buffer (0.05 M aqueous NaOAc buffer at pH = 5.5, containing 0.51 mM EDTA and 20.35 mM sodium citrate; 11.0 mL, 6.0 mg/mL, 0.446 μmol) of antibody 8c11 were added prepared aqueous solution of tris(2-carboxyethyl)phosphine (10 mM, 0.174 mL, 1.74 μmol) and Compound 16 (6.40 mg, 6.69 μmol) in DMSO (0.64 mL) at 22 °C. The resulting solution was placed in a water bath shaker, and shaken and reacted at 22 °C for 4.5 h before the reaction was stopped. The reaction solution was added with 1/3 volumes of 800 mM aqueous sodium citrate buffer (pH = 5.5) and 10% volumes of aqueous sucrose solution (60%), and desalted and purified using a Zeba desalted centrifugal column (40K MWCO) (eluent: 0.05 M aqueous sodium citrate buffer at pH 6.0, containing 6% sucrose) to give Conjugate **14** (54 mg, 3.18 mg/mL, yield: 82%).

$N^{a-I}$ was determined as 4.69 by RP.

### 2.20 Conjugate 17 (Adalimumab-conjugate 17)

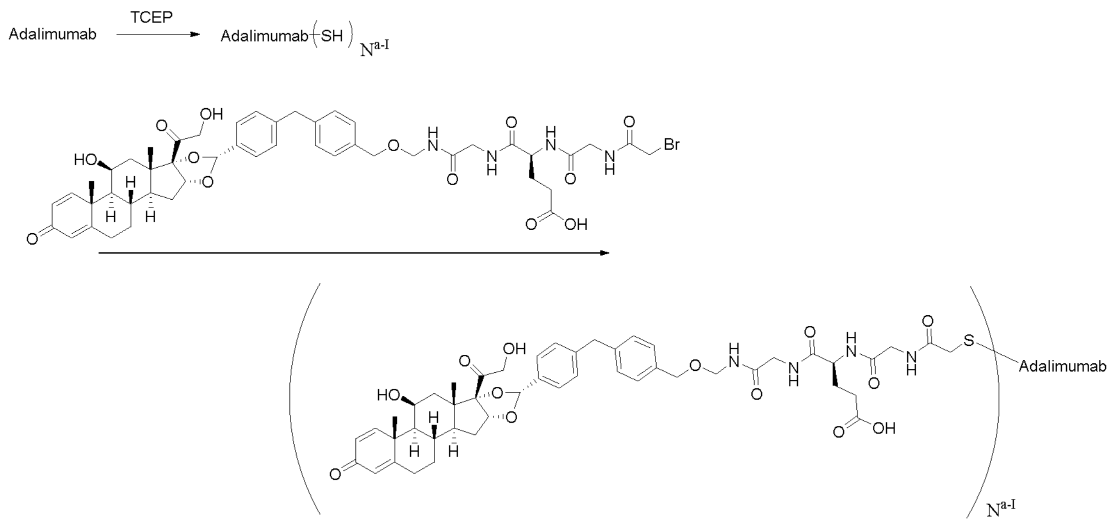


To an aqueous PB buffer (0.04 M aqueous PB buffer at pH = 6.97, containing 100 mM NaCl; 10.0 mL, 20 mg/mL, 1.351 μmol) of antibody Adalimumab were added prepared aqueous solution of tris(2-carboxyethyl)phosphine (10 mM, 0.338 mL, 3.378 μmol) and Compound 9 (18.49 mg, 18.91 μmol) in DMSO (1.85 mL) at 22 °C. The resulting solution was placed in a water bath shaker, and shaken and reacted at 22 °C for 3 h before the reaction was stopped.

To an aqueous PB buffer (0.04 M aqueous PB buffer at pH = 6.97, containing 100 mM NaCl; 60.0 mL, 20 mg/mL, 8.108 μmol) of antibody Adalimumab were added prepared aqueous solution of tris(2-carboxyethyl)phosphine (10 mM, 2.189 mL, 21.892 μmol) and Compound 9 (111.00 mg, 113.51 μmol) in DMSO (11.10 mL) at 22 °C. The

resulting solution was placed in a water bath shaker, and shaken and reacted at 22 °C for 5 h before the reaction was stopped.

The reaction solutions were combined, purified with UFDF (24 DV, 3 CV buffer + 2 CV buffer (containing 5% DMA) + 3 CV buffer + 2 CV buffer (5% DMA) + 3 CV buffer + 6 CV buffer + 5 CV buffer, the buffer being 0.02 M aqueous histidine buffer at pH 5.5), and filtered through a 0.22 μM membrane to give Conjugate **17** (1390 mg, 18.47 mg/mL, yield: 99%).

$N^{a-I}$ was determined as 3.66 by LC-MS. $N^{a-I}$ was determined as 3.83 by RP. $N^{a-I}$ was determined as 3.77 by HIC.

**2.21 Conjugate 18 (Iscalimab-conjugate 18)**

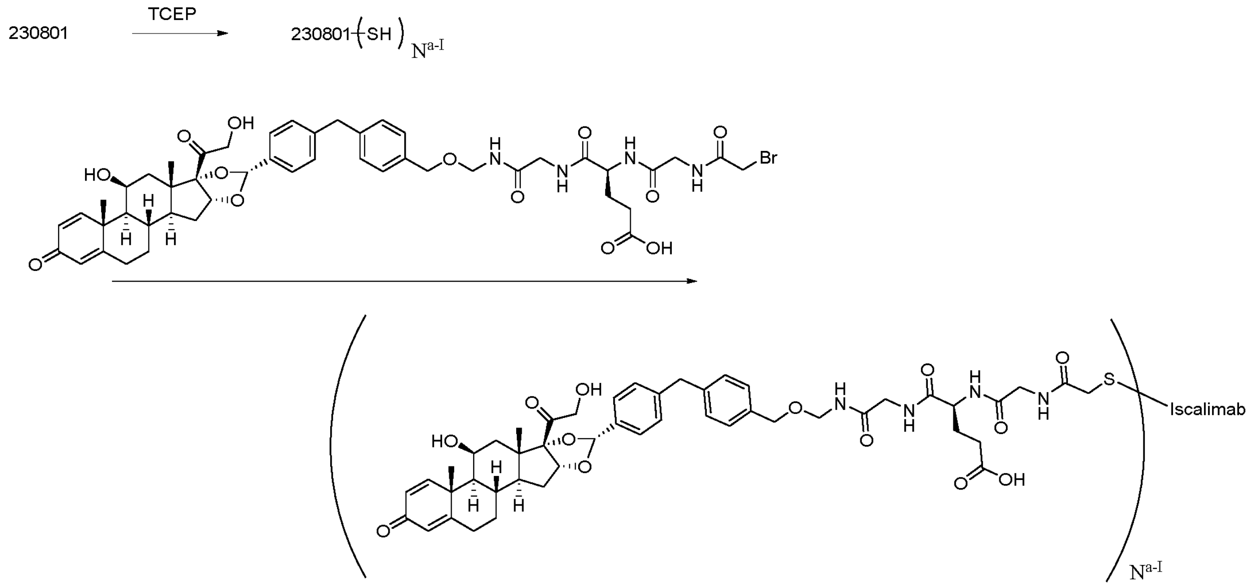


To an aqueous NaOAc buffer (0.05 M aqueous NaOAc buffer at pH = 5.5; 7.5 mL, 4.0 mg/mL, 0.203 μmol) of antibody Iscalimab was added prepared aqueous solution of tris(2-carboxyethyl)phosphine (10 mM, 0.173 mL, 1.726 μmol), Compound 9 (2.98 mg, 3.045 μmol) was dissolved in DMSO (0.30 mL), and the resulting solution was placed in a water bath shaker, and shaken and reacted at 22 °C for 4.5 h before the reaction was stopped.

The reaction solution was desalted and purified (eluent: 0.02 M aqueous histidine buffer at pH 5.5) using a Zeba desalting spin column (40K MWCO) to give Conjugate **18** (28 mg, 3.72 mg/mL, yield: 93%).

$N^{a-I}$ was determined as 4.31 as detected by LC-MS.

**2.22 Conjugate 19 (BIIB059-conjugate 19)**

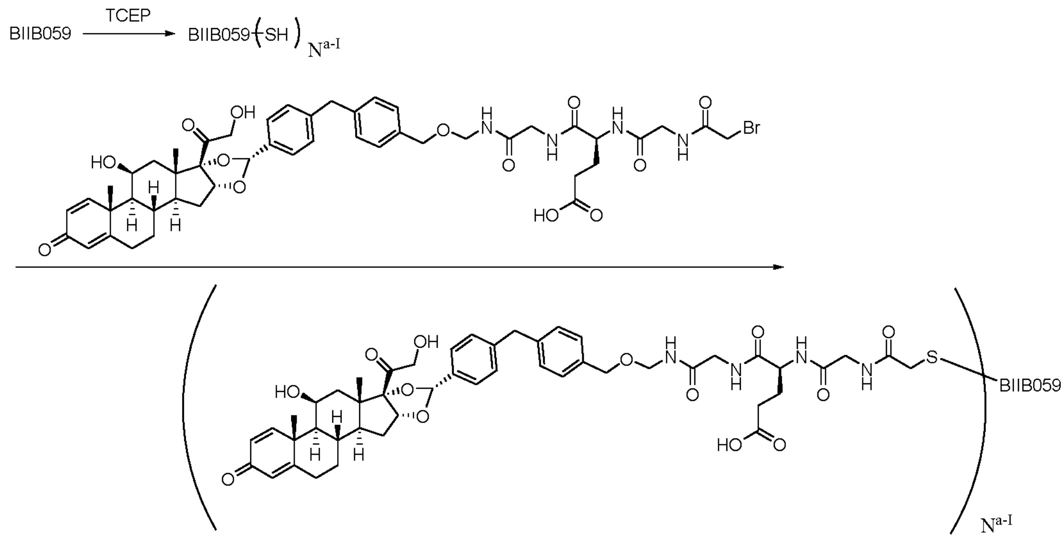


To an aqueous PB buffer (0.05 M PBS buffer at pH = 6.5; 2.5 mL, 9.96 mg/mL, 0.168 nmol) of antibody BIIB059 was added prepared aqueous solution of tris(2-carboxyethyl)phosphine (10 mM, 0.054mL, 0.54 nmol) at 37 °C, and the reaction solution was placed in a water bath shaker, and shaken and reacted at 37 °C for 3 h before the reaction was stopped; the reaction solution was cooled to 25 °C in a water bath, diluted to 5.0 mg/mL, and 2.0 mL of the solution was taken out and used in the next step.

Compound **9** (1.98 mg, 2.02 nmol) was dissolved in 0.10 mL of DMA, the resulting solution was added to the above solution (2.0 mL), placed in a water bath shaker, and shaken and reacted at 25 °C for 3 h before the reaction was stopped. The reaction solution was desalted and purified through Sephadex G25 gel column (eluent: 0.05 M aqueous histidine buffer at pH = 5.5, containing 0.001 M EDTA) to give Conjugate **19** (2.42 mg/mL, 3.31 mL, yield: 77%).

$N^{a-I}$ was determined as 4.75 by LC-MS.

### 2.23 Conjugate 20 (BIIB059-conjugate 20)

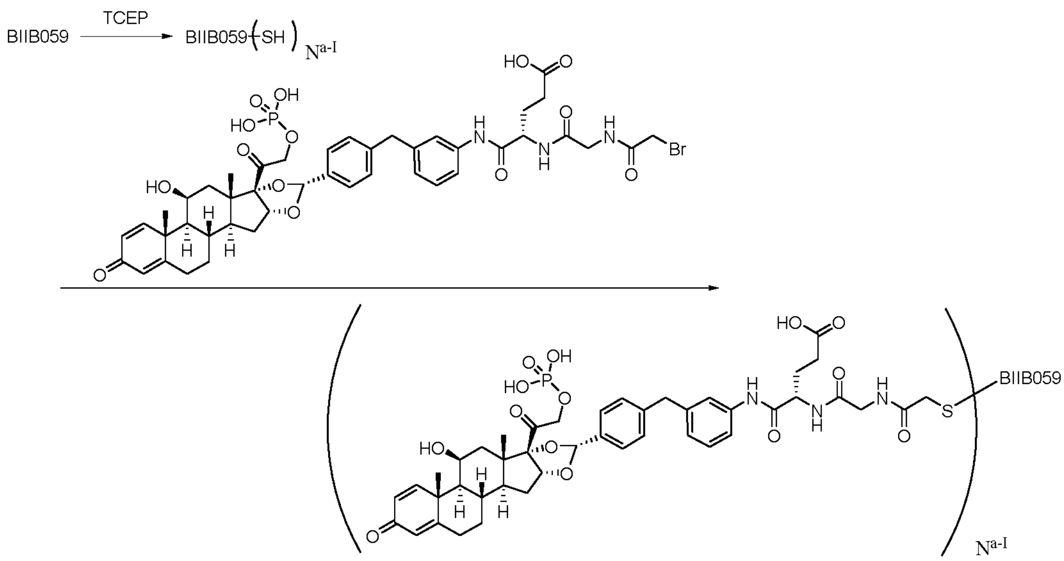


To an aqueous PB buffer (0.05 M PBS buffer at pH = 6.5; 2.5 mL, 9.96 mg/mL, 0.168 nmol) of antibody BIIB059 was added prepared aqueous solution of tris(2-carboxyethyl)phosphine (10 mM, 0.054mL, 0.54 nmol) at 37 °C, and the reaction solution was placed in a water bath shaker, and shaken and reacted at 37 °C for 3 h before the reaction was stopped; the reaction solution was cooled to 25 °C in a water bath, diluted to 5.0 mg/mL, and 2.0 mL of the solution was taken out and used in the next step.

Compound **16** (1.93 mg, 2.02 nmol) was dissolved in 0.10 mL of DMA, the resulting solution was added to the above solution (2.0 mL), placed in a water bath shaker, and shaken and reacted at 25 °C for 3 h before the reaction was stopped. The reaction solution was desalted and purified through Sephadex G25 gel column (eluent: 0.05 M aqueous histidine buffer at pH = 5.5, containing 0.001 M EDTA) to give Conjugate **20** (2.56 mg/mL, 3.52 mL, yield: 83%).

$N^{a-I}$ was determined as 4.27 by LC-MS.

**2.24 Conjugate 21 (BIIB059-conjugate 21)**

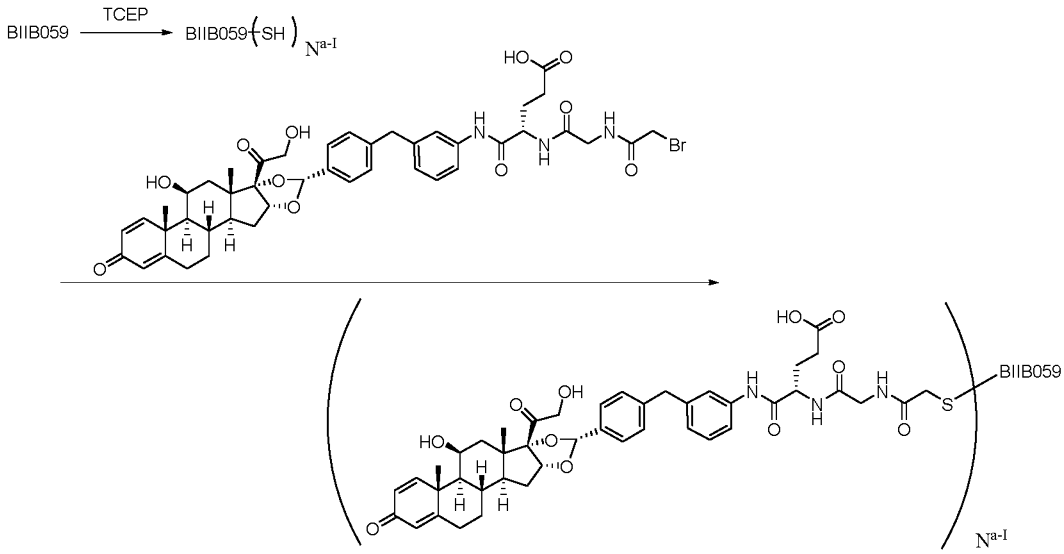


To an aqueous PB buffer (0.05 M PBS buffer at pH = 6.5; 2.5 mL, 9.96 mg/mL, 0.168 nmol) of antibody BIIB059 was added prepared aqueous solution of tris(2-carboxyethyl)phosphine (10 mM, 0.054mL, 0.54 nmol) at 37 °C, and the reaction solution was placed in a water bath shaker, and shaken and reacted at 37 °C for 3 h before the reaction was stopped; the reaction solution was cooled to 25 °C in a water bath, diluted to 5.0 mg/mL, and 2.0 mL of the solution was taken out and used in the next step.

Compound **15** (1.77 mg, 2.02 nmol) was dissolved in 0.10 mL of DMA, the resulting solution was added to the above solution (2.0 mL), placed in a water bath shaker, and shaken and reacted at 25 °C for 3 h before the reaction was stopped. The reaction solution was desalted and purified through Sephadex G25 gel column (eluent: 0.05 M aqueous histidine buffer at pH = 5.5, containing 0.001 M EDTA) to give Conjugate **21** (2.49 mg/mL, 3.53 mL, yield: 79%).

$N^{a-I}$ was determined as 4.30 by LC-MS.

**Example 3: Measurement of Glucocorticoid Receptor Reporter Gene GRE Activity**

**3.1 Measurement 1 of glucocorticoid receptor reporter gene GRE activity**

1. Cell culturing

HEK293 cells were cultured in DMEM +10% FBS medium in a carbon dioxide incubator humidified at 37 °C at 5% carbon dioxide.

2. Plasmid transfection and cell plating

HEK293 cells were trypsinized, and the HEK293 cell resuspension was adjusted to 200,000 cell/mL in medium (96 well plate: 100 μL/well).

Transfection reagent mixtures were prepared according to Tables A and B below, mixed well and left at room temperature for 20 minutes.

| Experimental transfection material | Volume (μL) |
|---|---|
| pGL5 plasmid (100 ng/μL) | 25 |
| pBIND-GR(100ng/uL) | 25 |
| Opti-MEM medium | 250.0 |
| P3000 | 10.00 |
| Opti-MEM medium | 250.0 |
| Lipofectamine | 7.5 |

| Experimental transfection material | Volume (μL) |
|---|---|
| PLvx-EF1a-hBDCA2-CMV-hFcεRIγ-IRES-puro(100ng/uL) | 50 |
| pGL5 plasmid (100 ng/μL) | 25 |
| pBIND-GR(100ng/uL) | 25 |
| Opti-MEM medium | 250.0 |
| P3000 | 10.00 |
| Opti-MEM medium | 250.0 |
| Lipofectamine | 7.5 |

The prepared transfection mixtures were added to 10 mL of the cell density-adjusted resuspension, mixed well by turning the mixtures upside down, and seeded into a 96-well plate at a volume of 100 μL/well.
The 96-well plate was incubated in a humidified incubator at 37 °C and 5% carbon dioxide for 24 hours.

3. Previous treatment

A 210 μM dexamethasone (purchased from the Sphsine) in DMSO was prepared and serially diluted at 3.3× to obtain 10 concentrations. 5 μL of the above dexamethasone in DMSO with each concentration was added into 45 μL of DMEM + 10% FBS respectively and mixed evenly to obtain dexamethasone dilutions with 10 concentrations.
2.1 μM of solutions of the compounds of the present application or the compounds of the reference examples (diluted with culture medium) were prepared and serially diluted at 3.3* to obtain test compound dilutions with 10 concentrations.
5 μL of dexamethasone dilution or test compound dilution was added into each well (100 μL of cells had been added). 5 μL of 10% DMSO medium was added into Min wells, and 5 μL 21 μM dexamethasone (dissolved in medium, 10% DMSO) was added into Max wells.
The test plate was placed back in the incubator and incubated for 24 hours.

4. Luciferase assay

The test plate was detected using the Dual-Glo Luciferase Assay System kit from Promega, and the fluorescence signal of Firefly Luciferase and the fluorescence signal of Renilla Luciferase were read using Enspire, respectively.

5. Data processing

The final measurement value was a normalized value "F/R" obtained by dividing the Firefly Luciferase fluorescent signal by the Renilla Luciferase fluorescent signal.
The data were copied and pasted into Excel, and the activation rate was obtained through an equation.

$$\text{Rate of activation}(\%) = \frac{\text{Final measurement value} - \text{MinMean value}}{\text{MaxMean value} - \text{MinMean value}} \times 100\%$$

The data were imported into MS Excel and subjected to curve fitting by using XLFit excel add-in version 5.4.0.8 to obtain an $EC_{50}$ value.

$$\text{Formula: } Y = \text{Bottom} + (\text{Top} - \text{Bottom})/(1 + (EC_{50}/X)\text{^HillSlope}$$

Y is the agonism and X is the concentration of the compound.

Table 6 shows that the compounds of the present application exhibited stronger glucocorticoid receptor agonistic activity.

Table 6. Measurement 1 of glucocorticoid receptor reporter gene GRE activity

| Compound No. | EC50 (nM) |
|---|---|
| Dexamethasone | 2.11 |
| Compound 14 (Reference Compound P1) | 0.19 |
| Compound 4 | 0.04 |
| Compound 4S | 1.42 |

### 3.2 Measurement 2 of glucocorticoid receptor reporter gene GRE activity

Example HEK293 GRE cells were plated at 50,000 cells/well in 50 μL assay medium (RPMI, 1% CSFBS, 1% L-glutamine, 1% sodium pyruvate, and 1% MEAA) on a 96-well tissue culture treated white plate (Costar: 3917). The compounds of the present application were serially three-fold diluted in 100% DMSO at an initial concentration of 1 μM for 9 times. 2 μL of serially-diluted compounds were transferred into 248 μL of assay medium in a secondary dilution plate (1:125 dilution) such that the small molecule compounds were further diluted in the assay medium. The cells were then treated with 25 μL of 1:125 diluted GR agonist compound or medium alone at a final initial concentration of 266.7 nM (1:3) and incubated at 37 °C with 5% $CO_2$ for 24 hours. After 24 hours of incubation, the cells were treated with 75 μL of Dual-Glo luciferase assay system (Promega-E2920) for 10 minutes and analyzed for luminescence using TopCount or MicroBeta2 (PerkinElmer).

Table 7 shows that the compounds of the present application exhibited significant glucocorticoid receptor agonistic activity.

Table 7. Measurement 2 of glucocorticoid receptor reporter gene GRE activity

| Compound No. | EC50 (nM) |
|---|---|
| Compound 14 (Reference Compound P1) | 0.43 |
| Compound 6 | 0.12 |

### Example 4: GR Luciferase Reporter Gene Elution Experiment

Objective

The compounds of the present application were evaluated for their ability to continue activation upon binding to GR.

Procedures

1. Cell culture

HEK293 cells were cultured in DMEM +10% FBS medium in a carbon dioxide incubator humidified at 37 °C at 5% carbon dioxide.

2. Plasmid transfection and cell plating

1) HEK293 cells were trypsinized, and the HEK293 cell resuspension was adjusted to 200,000 cell/mL in medium (96 well plate: 100 μL/well).
2) Transfection reagent mixtures were prepared according to the table below, mixed well and left at room temperature for 20 minutes.

| Experimental transfection material | Volume (μL) |
|---|---|
| pGL5 plasmid (100 ng/μL) | 25 |
| pBIND-GR(100ng/uL) | 25 |
| Opti-MEM medium | 250.0 |
| P3000 | 10.00 |
| Opti-MEM medium | 250.0 |
| Lipofectamine | 7.5 |

3) The prepared transfection mixtures were added to 10 mL of the cell density-adjusted resuspension, mixed well by turning the mixtures upside down, and seeded into a 96-well plate at a volume of 100 μL/well.
4) The 96-well plate was incubated in a humidified incubator at 37 °C and 5% carbon dioxide for 24 hours.

3. Drug treatment

1) 210 μM dexamethasone in DMSO was prepared and serially diluted at 3× to obtain 10 concentrations. 5 μL of the above dexamethasone in DMSO with each concentration was added into 45 μL of DMEM + 10% FBS respectively and mixed evenly to obtain dexamethasone dilutions with 10 concentrations.
2) A DMSO solution of the test compound at 210-fold final concentration was prepared and serially diluted at 3× to obtain dilutions of the test compound with 10 concentrations. 5 μL of the above DMSO solution with each concentration was added into 45 μL of DMEM + 10% FBS respectively and mixed evenly to obtain dilutions with 10 concentrations.
3) 5 μL of dexamethasone dilution or test compound dilution was added into each well (100 μL of cells had been added). 5 μL of 10% DMSO medium was added into Min wells, and 5 μL 21 μM dexamethasone (dissolved in medium, 10% DMSO) was added into Max wells.
4) After the test plate was placed back into the incubator for incubation for a certain period of time (15 min/30 min/1 h/2 h/4 h), the supernatant was carefully and slowly removed with a pipette and added into the medium at 100 μL/well, then the supernatant was carefully and slowly removed again and finally added into the medium at 100 μL/well, followed by placement into the incubator for incubation for 24 hours.

4. Luciferase assay

The test plate was detected using the Dual-Glo Luciferase Assay System kit from Promega, and the fluorescence signal of Firefly Luciferase and the fluorescence signal of Renilla Luciferase were read using Enspire, respectively.

5. Data processing

1) The final measurement value was a normalized value "F/R" obtained by dividing the Firefly Luciferase fluorescent signal by the Renilla Luciferase fluorescent signal.
2) The data were copied and pasted into Excel, and the activation rate was obtained through an equation.

$$\text{Rate of activation}(\%) = \frac{\text{Final measurement value} - \text{MinMean value}}{\text{MaxMean value} - \text{MinMean value}} \times 100\%$$

3) The data were imported into MS Excel and subjected to curve fitting by using XLFit excel add-in version 5.4.0.8

to obtain an $EC_{50}$ value.

$$\text{Formula: } Y = \text{Bottom} + (\text{Top} - \text{Bottom})/(1 + (EC_{50}/X)^\wedge \text{HillSlope}$$

Y is the agonism and X is the concentration of the compound.

Experimental results

The result in Table 8 shows that the compounds of the present application bound more tightly to the glucocorticoid receptor, were less likely to be eluted, and had a longer lasting activation of the glucocorticoid receptor.

Table 8. GR luciferase reporter gene elution experiment result

| Wash off time (Rate of activation%) | | | | | |
|---|---|---|---|---|---|
| Compound No. | 15 min | 30 min | 1h | 2h | 4h |
| Compound 4 | 127.6 | 127.0 | 164.6 | 131.3 | 139.9 |
| | 129.6 | 128.5 | 106.6 | 124.8 | 125.3 |
| Compound 14 (Reference Compound P1) | 15.2 | 14.6 | -0.6 | 15.3 | 0.1 |

**Example 5: MR Luciferase Reporter Gene Experiment**

1. Cell culture

HEK293 cells were cultured in DMEM +10% FBS medium in a carbon dioxide incubator humidified at 37 °C at 5% carbon dioxide.

2. Plasmid transfection and cell plating

1) HEK293 cells were trypsinized, and the HEK293 cell resuspension was adjusted to 200,000 cell/mL in medium (96 well plate: 100 μL/well).

2) Transfection reagent mixtures were prepared according to the table below, mixed well and left at room temperature for 20 minutes.

| Experimental transfection material | Volume (μL) |
|---|---|
| pGL5 plasmid (100 ng/μL) | 25 |
| pBIND-MR(100ng/uL) | 25 |
| Opti-MEM medium | 250.0 |
| P3000 | 10.00 |
| Opti-MEM medium | 250.0 |
| Lipofectamine | 7.5 |

3) The prepared transfection mixtures were added to 10 mL of the cell density-adjusted resuspension, mixed well by turning the mixtures upside down, and seeded into a 96-well plate at a volume of 100 μL/well.

4) The 96-well plate was incubated in a humidified incubator at 37 °C and 5% carbon dioxide for 24 hours.

3. Drug treatment

5) 21 μM aldosterone in DMSO was prepared and serially diluted at 3.3× to obtain 10 concentrations. 5 μL of the

above aldosterone in DMSO with each concentration was added into 45 μL of DMEM + 10% FBS respectively and mixed evenly to obtain aldosterone dilutions with 10 concentrations.

6) A DMSO solution of the test compound at 210-fold final concentration was prepared and serially diluted at 3.3× to obtain dilutions of the test compound with 10 concentrations. 5 μL of the above DMSO solution with each concentration was added into 45 μL of DMEM + 10% FBS respectively and mixed evenly to obtain dilutions with 10 concentrations.

7) 5 μL of aldosterone dilution or test compound dilution was added into each well (100 μL of cells had been added). 5 μL of 10% DMSO medium was added into Min wells, and 5 μL 2.1 μM aldosterone (dissolved in medium, 10% DMSO) was added into Max wells.

8) The test plate was placed back in the incubator and incubated for 24 hours.

4. Luciferase assay

The test plate was detected using the Dual-Glo Luciferase Assay System kit from Promega, and the fluorescence signal of Firefly Luciferase and the fluorescence signal of Renilla Luciferase were read using Enspire, respectively.

5. Data processing

4) The final measurement value was a normalized value "F/R" obtained by dividing the Firefly Luciferase fluorescent signal by the Renilla Luciferase fluorescent signal.

5) The data were copied and pasted into Excel, and the activation rate was obtained through an equation.

$$\text{Rate of activation}(\%) = \frac{\text{Final measurement value} - \text{MinMean value}}{\text{MaxMean value} - \text{MinMean value}} \times 100\%$$

6) The data were imported into MS Excel and subjected to curve fitting by using XLFit excel add-in version 5.4.0.8 to obtain an $EC_{50}$ value.

$$\text{Formula: } Y = \text{Bottom} + (\text{Top} - \text{Bottom})/(1 + (EC_{50}/X)^\wedge\text{HillSlope}$$

Y is the agonism and X is the concentration of the compound.

Table 9. MR luciferase reporter gene experiment result

| Compound ID | MREC50 (nM) |
|---|---|
| Dexamethasone | >1000.00 |
| Compound 4 | >1000.00 |
| Compound 5 | >1000.00 |
| Compound 3 | >1000.00 |

According to the result in Table 9, the compounds of the present invention had a low affinity for mineralocorticoid receptors.

**Example 6: ERa Luciferase Reporter Gene Experiment**

1. Cell culture

HEK293 cells were cultured in DMEM +10% FBS medium in a carbon dioxide incubator humidified at 37 °C at 5% carbon dioxide.

2. Plasmid transfection and cell plating

1) HEK293 cells were trypsinized, and the HEK293 cell resuspension was adjusted to 200,000 cell/mL in medium (96 well plate: 100 μL/well).
2) Transfection reagent mixtures were prepared according to the table below, mixed well and left at room temperature for 20 minutes.

| Experimental transfection material | Volume (μL) |
|---|---|
| pGL5 plasmid (100 ng/μL) | 25 |
| pBIND-ERα(100ng/uL) | 25 |
| Opti-MEM medium | 250.0 |
| P3000 | 10.00 |
| Opti-MEM medium | 250.0 |
| Lipofectamine | 7.5 |

3) The prepared transfection mixtures were added to 10 mL of the cell density-adjusted resuspension, mixed well by turning the mixtures upside down, and seeded into a 96-well plate at a volume of 100 μL/well.
4) The 96-well plate was incubated in a humidified incubator at 37 °C and 5% carbon dioxide for 24 hours.

3. Drug treatment

5) 21 μM estradiol in DMSO was prepared and serially diluted at 3× to obtain 10 concentrations.
5 μL of the above solution of estradiol in DMSO with each concentration was added into 45 μL of DMEM + 10% FBS respectively and mixed evenly to obtain estradiol dilutions with 10 concentrations.
6) A DMSO solution of the test compound at 210-fold final concentration was prepared and serially diluted at 3× to obtain dilutions of the test compound with 10 concentrations. 5 μL of the above DMSO solution with each concentration was added into 45 μL of DMEM + 10% FBS respectively and mixed evenly to obtain dilutions with 10 concentrations.
7) 5 μL of estradiol dilution or test compound dilution was added into each well (100 μL of cells had been added). 5 μL of 10% DMSO medium was added into Min wells, and 5 μL 2.1 μM estradiol (dissolved in medium, 10% DMSO) was added into Max wells.
8) The test plate was placed back in the incubator and incubated for 24 hours.

4. Luciferase assay

The test plate was detected using the Dual-Glo Luciferase Assay System kit from Promega, and the fluorescence signal of Firefly Luciferase and the fluorescence signal of Renilla Luciferase were read using Enspire, respectively.

5. Data processing

9) The final measurement value was a normalized value "F/R" obtained by dividing the Firefly Luciferase fluorescent signal by the Renilla Luciferase fluorescent signal.
10) The data were copied and pasted into Excel, and the activation rate was obtained through an equation.

$$\text{Rate of activation}(\%) = \frac{\text{Final measurement value} - \text{MinMean value}}{\text{MaxMean value} - \text{MinMean value}} \times 100\%$$

11) The data were imported into MS Excel and subjected to curve fitting by using XLFit excel add-in version 5.4.0.8

to obtain an $EC_{50}$ value.

$$\text{Formula: } Y = \text{Bottom} + (\text{Top} - \text{Bottom})/(1 + (EC_{50}/X)^{\wedge}\text{HillSlope}$$

Y is the agonism and X is the concentration of the compound.

Table 10. ERa luciferase reporter gene experiment result

| Compound ID | ERα EC50 (nM) |
|---|---|
| Dexamethasone | >1000.00 |
| Compound 4 | >1000.00 |
| Compound 5 | >1000.00 |
| Compound 3 | >1000.00 |

According to the result in Table 10, the compounds of the present invention had a low affinity for estrogen receptors.

**Example 7: Inhibitory Effect of Compounds of the Present Invention on R848-Induced Release of IFNα and TNFα from Human Peripheral Blood Monocytes**

Objective

The compounds of the present application were tested for their inhibitory effect on the production of peripheral blood monocytes. The peripheral blood monocytes were treated *in vitro* with the ligand-drug conjugate at different concentrations, and IFNα and TNFα generated by the peripheral blood monocytes were quantitatively detected after a certain time. The compounds of the present invention were evaluated for *in-vitro* activity.

Test method

The cryopreserved human PBMC cells were resuspended in a complete medium and counted, and the cell suspension was adjusted to 2.5 × 10*6 cells/mL with the culture medium. After the suspension was centrifuged to collect the cells the next day, the cells were resuspended using fresh culture medium. Test compounds were diluted to 4-fold final concentration in cell culture medium, 50 μL of the compounds were added into a 96-well cell plate, and 100 μL of diluted cells (800,000 cells/well) were added into each well of the 96-well plate containing the compounds, followed by addition of 50 μL R848 to a final concentration of 5 μM. The cell plate was incubated at 37 °C with 5% $CO_2$ for 24 hours. The supernatant was collected, and the TNF-α and IFN-α concentration levels in the supernatant were detected using an ELISA kit.

TNF-α concentration assay was performed using Dakewe TNF-α ELISA kit (Cat# 1117202), 100 μL of the serially-diluted cytokine standard was added into each well, 100 μL of the above supernatant diluted 10-fold was added into each well, and 100 μL of 1× dilution buffer R was added into each blank well. 50 μL of biotinylated antibody working solution was added into each well. After mixing well, the plate was covered with the plate-sealing membrane and incubated at room temperature for 3 hours. The plate was washed for 3 times: the liquid in each well was discarded, and 300 μL of 1× washing buffer working solution was added into each well; after 1 minute of residence, the liquid in the wells was discarded. The procedure was repeated for 3 times. 100 μL of Streptavidin-HRP working solution was added into each well. The plate was covered with the plate-sealing membrane and incubated at room temperature for 20 minutes. The plate was washed for 3 times, 100 μL of TMB liquid was added into each well, and the plate was incubated for 25 minutes at room temperature (18-25 °C) in the dark. The reaction was stopped by adding 100 μL stop solution rapidly to each well. The reading at a wavelength of 450 nm was detected by an M2e instrument within 10 minutes after termination.

IFN-α concentration assay was performed using Dakewe TNF-α ELISA kit (Cat# 1110012), 100 μL of the serially-diluted cytokine standard was added into each well, 100 μL of the above supernatant diluted 2-fold was added into each well, and 100 μL of 1× dilution buffer R was added into each blank well. 50 μL of biotinylated antibody working solution was added into each well. After mixing well, the plate was covered with the plate-sealing membrane and incubated at room temperature for 3 hours. The plate was washed for 3 times: the liquid in each well was discarded, and 300 μL of 1× washing buffer working solution was added into each well; after 1 minute of residence, the liquid in the wells was

discarded. The procedure was repeated for 3 times. 100 μL of Streptavidin-HRP working solution was added into each well. The plate was covered with the plate-sealing membrane and incubated at room temperature for 20 minutes. The plate was washed for 3 times, 100 μL of TMB liquid was added into each well, and the plate was incubated for 25 minutes at room temperature (18-25 °C) in the dark. The reaction was stopped by adding 100 μL stop solution rapidly to each well. The reading at a wavelength of 450 nm was detected by an M2e instrument within 10 minutes after termination.

The contents of TNF-α and IFN-α were calculated from the OD450 values. The results are shown in Table 11-1 and Table 11-2.

Table 11-1. Inhibition of compounds on TNF-α secretion (pg/mL) from PBMC under R848 stimulation

| Concentration of the compound | Compound 4 | Compound 14 (Reference Compound P1) | Dexamethasone | Blank |
|---|---|---|---|---|
| 100nM | 356.1±0.2 | 471.9±138.4 | 396.4±88 | 2507.3±25 |
| 20nM | 320±18.8 | 465.6±37.9 | 761.9±112.6 | |
| 4nM | 443.6±41.2 | 868±69.3 | 1446.5±10.8 | |
| TNF-α content was expressed as mean ± standard deviation. | | | | |

Table 11-2. Inhibition of compounds on IFN-α secretion (pg/mL) from PBMC under R848 stimulation

| Concentration of the compound | Compound 4 | Compound 6 | Compound 14 (Reference Compound P1) | Dexamethasone | Blank |
|---|---|---|---|---|---|
| 100nM | 0±0 | 6.9±6.9 | 13±13 | 0±0 | 995.1±69.3 |
| 20nM | 27.9±27.9 | 45.9±45.9 | 10.2±10.2 | 169.6±94.4 | |
| 4nM | 13.2±13.2 | 99.8±5.7 | 146.4±16.6 | 620.7±70.4 | |
| IFN-α content was expressed as mean ± standard deviation. | | | | | |

The results show that the compounds of the present application could affect the ability of peripheral blood monocytes to produce TNF-α and IFN-α. The ligand-drug conjugates of the present application can inhibit inflammation, and can be used for preventing and/or treating diseases and/or conditions such as inflammation.

**Example 8: Inhibitory Effect of Drug Conjugates of the Present Invention on R848-Induced Release of IFNα, TNFα, IL-6 and IL-8 from Human Peripheral Blood Monocytes**

Objective

The inhibitory effect of the ligand-drug conjugate (ADC) of the present application on the production of IFNα by peripheral blood monocytes was examined. The peripheral blood monocytes were treated *in vitro* with the ligand-drug conjugate at different concentrations, and IFNα and TNFα generated by the peripheral blood monocytes were quantitatively detected after a certain time. The ligand-drug conjugates were evaluated for *in-vitro* activity.

Test method

The cryopreserved human PBMC cells were resuspended in a complete medium and counted, and the cell suspension was adjusted to 2.5 × 10*6 cells/mL with the culture medium. After the suspension was centrifuged to collect the cells the next day, the cells were resuspended using fresh culture medium, and 100 μL of diluted cells (800,000 cells/well) were added into a 96-well plate. The test samples were diluted to 3-fold final stimulation concentration using fresh culture medium, 50 μL of the diluted test samples were added into the 96-well cell plate, and the cell plate was incubated at 37 °C with 5% $CO_2$ for 12 hours, followed by addition of 50 μL 20μM R848 each well. The cell plate was incubated at 37 °C with 5% $CO_2$ for 20 hours. The supernatant was collected, and the cytokine concentration in the supernatant was detected using a kit.

TNF-α concentration assay was performed using Dakewe TNF-α ELISA kit (Cat# 1117202), 100 μL of the serially-diluted cytokine standard was added into each well, 100 μL of the above supernatant diluted 10-fold was added

into each well, and 100 μL of 1× dilution buffer R was added into each blank well. 50 μL of biotinylated antibody working solution was added into each well. After mixing well, the plate was covered with the plate-sealing membrane and incubated at room temperature for 3 hours. The plate was washed for 3 times: the liquid in each well was discarded, and 300 μL of 1× washing buffer working solution was added into each well; after 1 minute of residence, the liquid in the wells was discarded. The procedure was repeated for 3 times. 100 μL of Streptavidin-HRP working solution was added into each well. The plate was covered with the plate-sealing membrane and incubated at room temperature for 20 minutes. The plate was washed for 3 times, 100 μL of TMB liquid was added into each well, and the plate was incubated for 25 minutes at room temperature (18-25 °C) in the dark. The reaction was stopped by adding 100 μL stop solution rapidly to each well. The reading at a wavelength of 450 nm was detected by an M2e instrument within 10 minutes after termination.

IFN-α concentration assay was performed using Dakewe TNF-α ELISA kit (Cat# 1110012), 100 μL of the serially-diluted cytokine standard was added into each well, 100 μL of the above supernatant diluted 2-fold was added into each well, and 100 μL of 1× dilution buffer R was added into each blank well. 50 μL of biotinylated antibody working solution was added into each well. After mixing well, the plate was covered with the plate-sealing membrane and incubated at room temperature for 3 hours. The plate was washed for 3 times: the liquid in each well was discarded, and 300 μL of 1× washing buffer working solution was added into each well; after 1 minute of residence, the liquid in the wells was discarded. The procedure was repeated for 3 times. 100 μL of Streptavidin-HRP working solution was added into each well. The plate was covered with the plate-sealing membrane and incubated at room temperature for 20 minutes. The plate was washed for 3 times, 100 μL of TMB liquid was added into each well, and the plate was incubated for 25 minutes at room temperature (18-25 °C) in the dark. The reaction was stopped by adding 100 μL stop solution rapidly to each well. The reading at a wavelength of 450 nm was detected by an M2e instrument within 10 minutes after termination.

IL-6 concentration assay was performed using Dakewe IL-6 ELISA kit (Cat# DKW12-1060-096), 100 μL of serially-diluted cytokine standard was added into each well, 100 μL of the above supernatant diluted 2-fold was added into each well, and 100 μL of 1× dilution buffer R was added into each blank well. 50 μL of biotinylated antibody working solution was added into each well. After mixing well, the plate was covered with the plate-sealing membrane and incubated at room temperature for 3 hours. The plate was washed for 3 times: the liquid in each well was discarded, and 300 μL of 1 × washing buffer working solution was added into each well; after 1 minute of residence, the liquid in the wells was discarded. The procedure was repeated for 3 times. 100 μL of Streptavidin-HRP working solution was added into each well. The plate was covered with the plate-sealing membrane and incubated at room temperature for 20 minutes. The plate was washed for 3 times, 100 μL of TMB liquid was added into each well, and the plate was incubated for 25 minutes at room temperature (18-25 °C) in the dark. The reaction was stopped by adding 100 μL stop solution rapidly to each well. The reading at a wavelength of 450 nm was detected by an M2e instrument within 10 minutes after termination.

IL-8 concentration assay was performed using Dakewe IL-8 ELISA kit (Cat# 1110802), 100 μL of the serially-diluted cytokine standard was added into each well, 100 μL of the above supernatant diluted 2-fold was added into each well, and 100 μL of 1× dilution buffer R was added into each blank well. 50 μL of biotinylated antibody working solution was added into each well. After mixing well, the plate was covered with the plate-sealing membrane and incubated at room temperature for 3 hours. The plate was washed for 3 times: the liquid in each well was discarded, and 300 μL of 1× washing buffer working solution was added into each well; after 1 minute of residence, the liquid in the wells was discarded. The procedure was repeated for 3 times. 100 μL of Streptavidin-HRP working solution was added into each well. The plate was covered with the plate-sealing membrane and incubated at room temperature for 20 minutes. The plate was washed for 3 times, 100 μL of TMB liquid was added into each well, and the plate was incubated for 25 minutes at room temperature (18-25 °C) in the dark. The reaction was stopped by adding 100 μL stop solution rapidly to each well. The reading at a wavelength of 450 nm was detected by an M2e instrument within 10 minutes after termination.

The contents of TNF-α, IFN-α, IL-6 and IL-8 were calculated from the OD450 values, and the results are shown in FIGs. 1a, 1b, 1c and 1d.

According to FIG. 1a, when the incubation concentration of antibodies BIIB059, Conjugate 19, Conjugate 20 and Conjugate 21 was 300 nm, the IFN-α contents in the detection system were 1639.9 pg/mL, 113.2 pg/mL, 1422.5 pg/mL, 911.9 pg/mL, respectively; the inhibitory effect of Conjugate 19, Conjugate 20 and Conjugate 21 on IFNα was better than that of antibody BIIB059, and the inhibitory effect of Conjugate 19 was significantly better than that of Conjugate 20 and Conjugate 21.

According to FIG. 1b, when the incubation concentration of antibodies BIIB059, Conjugate 19, Conjugate 20 and Conjugate 21 was 300 nm, the IFN-α contents in the detection system were 7040.1 pg/mL, 761.8 pg/mL, 3280.0 pg/mL, 2644.1 pg/mL, respectively; the inhibitory effect of Conjugate 19, Conjugate 20 and Conjugate 21 on IFN-α was better than that of antibody BIIB059, and the inhibitory effect of Conjugate 19 was significantly better than that of Conjugate 20 and Conjugate 21.

According to FIG. 1c, when the incubation concentration of antibodies BIIB059, Conjugate 19, Conjugate 20 and Conjugate 21 was 300 nm, the IL-6 contents in the detection system were 65932.1 pg/mL, 8861.7 pg/mL, 34744.1 pg/mL, 29867.1 pg/mL, respectively; the inhibitory effect of Conjugate 19, Conjugate 20 and Conjugate 21 on IL-6 was better than that of antibody BIIB059, and the inhibitory effect of Conjugate 19 was significantly better than that of Conjugate

20 and Conjugate 21.

According to FIG. 1d, when the incubation concentration of antibodies BIIB059, Conjugate 19, Conjugate 20 and Conjugate 21 was 300 nm, the IL-8 contents in the detection system were 86905.7 pg/mL, 26565.5 pg/mL, 56315.8 pg/mL, 43544.9 pg/mL, respectively; the inhibitory effect of Conjugate 19, Conjugate 20 and Conjugate 21 on IL-8 was better than that of antibody BIIB059, and the inhibitory effect of Conjugate 19 was significantly better than that of Conjugate 20 and Conjugate 21.

The results show that the ligand-drug conjugates of the present application could affect the ability of peripheral blood monocytes to produce IIFNα, TNFα, IL-6 and IL-8. The ligand-drug conjugates of the present application can inhibit inflammation, and can be used for preventing and/or treating diseases and/or conditions such as inflammation.

## Example 9: Determination of Biological Activity in Fluorescein Isothiocyanate (FITC)-Induced Delayed-Type IV Hypersensitivity Mouse Model

ADC was evaluated in an acute contact hypersensitivity model in which a delayed type hypersensitivity (DTH) response (T cell drive) was used to trigger acute skin inflammation by the application of sensitizers (fluorescein isothiocyanate, FITC). The potency of the ligand-drug conjugates (ADCs) of the present application was measured by their ability to reduce ear swelling. Before the experiment began, a specified number of 7-9 week-old female Balb/c mice were selected, random grouping was performed according to the weight of mice, with 10 mice in each group, and the average weight values among different groups were ensured to be similar so as to reduce the difference among the groups. The day of grouping the mice was defined as day 0 of the experiment. After random grouping, all mice were shaved off abdominal hair under mild anesthesia with 5% isoflurane, and mice in the disease-induced group were sensitized firstly by applying 100 μL of 2% fluorescein isothiocyanate (FITC) solution to their abdomen, while mice in the disease-controlled group were applied with 100 μL of vehicle control solution (acetone:DBP, volume ratio 1:1) to their abdomen. After the solvent solution and the 2% FITC solution were absorbed and the abdominal skin was dry, the mice were placed back into the cage box after they were awake. On day 1 of the experiment, the procedure described above was repeated for a second 2% FITC sensitization.

All mice were weighed on day 5 of the experiment. According to the experimental design, the mice in the solvent control group or each treatment group were respectively injected with the sterile PBS solution or the specified administration solution in the abdominal cavity. 1 hour after dosing, all mice were tested for left and right ear thickness using Mitutoya Caliper under mild anesthesia with 5% isoflurane. After ear thickness measurement, all mice in the disease-induced group were coated with 10 μL of 1% FTIC solution on the right ear and 10 μL of vehicle control solution (acetone:DBP, volume ratio 1: 1) on the left ear.

On day 6 of the experiment, 24 hours after 1% FTIC excitation, all mice were tested for left and right ear thickness using Mitutoya Caliper under mild anesthesia with 5% isoflurane and mouse ear thickness change was calculated according to the following formula: (R-L)-(R0-L0), wherein R0 and L0 are right and left ear thickness before 1% FITC excitation, respectively, and R and L are right and left ear thickness after 24 hours of 1% FITC excitation, respectively. The results are shown in FIG. 2. As shown in FIG. 2, when the administration concentration of Conjugate 9, antibody 8c11 and Conjugate 14 was 20 mg/kg, the mean increase in ear thickness of mice was 0.066 mm, 0.249 mm and 0.128 mm, respectively (see ADC1). The inhibitory effect of Conjugate 9 on hypersensitivity was significantly better than that of antibody 8c11 and Conjugate 14.

The results show that the ligand-drug conjugate (ADC) of the present application had the ability to inhibit hypersensitivity, and that the ligand drug conjugate (ADC) of the present application could be used for preventing and/or treating diseases and or conditions of inflammation.

## Example 10: Determination of Bioactivity of Bovine Type II Collagen Mixed Adjuvant-Induced DBA/1 Mouse Arthritis Model

The potency of the ligand-drug conjugates (ADCs) of the present application was evaluated in a collagen-induced arthritis (CIA) model.

Male DBA/1J mice were obtained from Jackson Labs. The mice were used at 6-8 weeks of age. All animals were fed free diet water under 12 hours light/dark cycle at constant temperature and humidity. Body weight and condition were monitored and if > 20% weight loss was exhibited, the animals were euthanized.

Bovine collagen (Chondrex, Inc. Cat: 20021) was dissolved in 0.1 M acetic acid overnight in a 2-8 °C refrigerator at a collagen concentration of 8 mg/mL, and an equal volume of CFA (complete Freund's adjuvant, Difco, Laurence, KS) was added into the collagen solution and emulsified in a high-speed homogenizer to make an emulsion. At Day 0, animals in need of immunization were anesthetized with 2%-5% isoflurane and male DBA/J mice were immunized once with a single subcutaneous injection of 50 μL collagen/CFA emulsion at the tail root of 2-3 cm from the body. Three weeks later, namely at Day 21, the tail root was immunized twice with the same volume of collagen emulsion by the

same injection method. After the second immunization, 120 animals with an average score of about 2 for the limb arthritis were selected and randomly grouped. The animals were treated by a single intraperitoneal injection of test reagent according to the experimental design after grouping. For the dexamethasone (Sphsine) treatment group, the drug was administered the day after grouping until the end of the trial. The extremities of each group were observed twice weekly for the onset of arthritis from the time of the second immunization, and the observation was continued until the end of the trial.

Scoring according to the degree of lesion (redness and swelling) was performed on a scale of 0-4 points, with a maximum score of 4 for each limb and a total maximum score of 16 for each animal's limbs. The scoring criteria are as follows: 0 points, no redness and swelling; 1 point, 1-2 red and swollen interphalangeal joints; 2 points, 3-4 red and swollen interphalangeal joints; 3 points, more than 4 red and swollen interphalangeal joints; 4 points, severe redness and swelling in toes or fingers to ankle joints or wrist joints. For joint swelling, ankle thickness of hind paw of each animal was measured with a screw micrometer before one immunization. Since the second immunization, measurements were performed 2 times per week, and changes in ankle thickness before and after the immunization were compared. After the trial was completed, all animals were anesthetized with 2-5% isoflurane and then euthanized by exsanguination.

Score AUC: area under the curve for arthritis score.

The results are shown in FIGs. 3a and 3b.

The results show that the ligand-drug conjugates (ADCs) of the present application had the ability to reduce swelling of the hind paw of the mice, and could exhibit an extended duration of action of about 28 days, as compared to the blank agent, and had the ability to reduce the severity of arthritis and biological safety (e.g., blood safety).

**Example 11**

**11.1 GRE Activity assay**

Generation of human and mouse transmembrane TNF-α GRE reporter cell lines

To generate the parental cell line, K562 cells were seeded at 500,000 cells per well onto a 6-well dish (Costar: 3516) with 2 mL of complete growth medium (RPMI, 10% FBS, 1% L-glutamine, 1% sodium pyruvate, and 1% MEMNEAA (an optional amino acid solution)) at 37 °C with 5% $CO_2$ within 24 hours. The next day, 1.5 μg of pGL4.36 [Luc2P/MMTV/Hygro] (Promega) and 3 μL of PLUS reagent (Invitrogen) were diluted into 244 μL Opti-MEM (Gibco: 31985-070) and incubated at room temperature for 15 minutes. pGL4.36 [luc2P/MMTV/Hygro] vector contains the murine mammary tumor virus long terminal repeated sequence, which drives transcription of the luciferase reporter gene luc2P in response to activation of several nuclear receptors (e.g. glucocorticoid receptor and androgen receptor). After incubation, the diluted DNA solution was pre-incubated with 1:1 lipofectamine LTX solution (13.2 μL + 256.8 μL Opti-MEM) and incubated at room temperature for 25 minutes to form a DNA-lipofectamine LTX complex. After incubation, 500 μL of DNA-lipofectamine complex was added directly into the wells containing cells. K562 cells were transfected at 37 °C with 5% $CO_2$ for 24 hours. After incubation, the cells were washed with 3 mL of PBS and selected for two weeks with complete growth medium containing 125 μg/mL hygromycin B. "K562pGL4.36 [Luc2P/MMTV/Hygro]_pGL4.75 [hRLuc/CMV]" cells were generated.

To generate murine transmembrane TNF-α GRE reporter cells, the parent cells, K562pGL4.36 [Luc2P/MMTV/Hygro]_pGL4.75[hRLuc/CMV], were seeded at 500,000 cells per well onto a 6-well dish containing 2 mL of complete growth medium (RPMI, 10% FBS, 1% L-glutamine, 1% sodium pyruvate and 1% MEMNEAA) at 37 °C with 5% $CO_2$ within 24 hours. The next day, 3 μg of mFL_TNFα DNA encoding unlabeled mouse TNF and 3 μL of PLUS reagent (Invitrogen: 10964-021) were diluted into 244 μL of Opti-MEM (Gibco: 31985-070) and incubated at room temperature for 15 minutes. After incubation, the diluted DNA solution was pre-incubated with 1:1 lipofectamine LTX solution (13.2 μL + 256.8 μL Opti-MEM) and incubated at room temperature for 25 minutes to form a DNA-lipofectamine LTX complex. After incubation, 500 μL of DNA-lipofectamine complex was added directly into the wells containing cells. The parent K562pGL4.36\[Luc2P/MMTV/Hygro]_pGL4.75[hRLuc/CMV] cells were transfected at 37 °C with 5% $CO_2$ for 24 hours. After incubation, the cells were washed with 3 mL of PBS and selected for two weeks with complete growth medium containing 125 μg/mL hygromycin B (Invitrogen: 10687-010) and 250 μg/mL G418 (Gibco: 10131-027). "K562 mouse FL-TNFaGRE (pGL4.36[luc2P/MMTV/Hygro])" cells were generated.

To generate the human transmembrane TNF-α GRE reporter cell line, the parent cell K562pGL4.36[Luc2P/MMTV/Hygro]_pGL4.75[hRLuc/CMV] was transfected with the hTNFδ1-12C-MycpcDNA3.1(-) plasmid construct. The plasmid was pcDNA3.1 (Thermo Fisher, Cat. V79020), which encodes tace-resistant transmembrane TNF. (See PerezC et al., Cell 63(2): 251-8 (1990) which discusses tace-resistant transmembrane TNF). "K562 human TNFδ1-12GRE (pGL4.36[luc2P/MMTV/Hygro])" cells were generated. These cell lines were then used in the TNF-α reporter assay described in the examples below.

K562 parent GRE cells ("K562pGL4.36 [Luc2P/MMTV/Hygro]_pGL4.75[hRLuc/CMV]" cells), and GRE-mouse

TNF-α cells ("K562 mouse FL-TNF-α GRE(pGL4.36[luc2P/MMTV/Hygro])" cells) or GRE-human TNF-α cells ("K562 human TNFδ1-12GRE (pGL4.36[luc2P/MMTV/Hygro])" cells) were seeded onto a 96-well plate at 50,000 cells per well. The ligand-drug conjugates (ADCs) of the present application were serially diluted at 3× in media and added into the above 96-well plate and incubated at 37 °C with 5% $CO_2$ for 48 hours. Luminescence was analyzed after treatment with the luciferase assay system. The data were analyzed using a four-parameter curve fit to generate $EC_{50}$ values. The maximum% activation was normalized to 100 nM dexamethasone. The ligand-drug conjugates (ADCs) of the present application measured *in-vitro* activity in a mouse transmembrane TNFα GRE reporter assay; and the ligand drug conjugates (ADCs) of the present application measured *in-vitro* activity in the human transmembrane TNFα GRE reporter assay.

The results show that the ligand-drug conjugates (ADCs) of the present application had the ability to affect the level of cellular GRE activation, and that the ligand drug conjugates (ADCs) of the present application could affect the level of glucocorticoid-mediated signaling pathway activation.

### 11.2 Lipopolysaccharide (LPS) induced cytokine release and downstream signal detection

Primary human peripheral blood monocytes (PBMCs) were washed in 50 mL PBS, resuspended in FBS (fetal bovine serum) with 5% DMSO, divided in equal and cryopreserved in liquid nitrogen until use. The PBMCs were thawed, resuspended in cell culture medium (e.g., RPMI cell culture medium) with 2% FBS and 1% penicillin streptomycin, and seeded onto a 96-well plate. The cells were then incubated with different concentrations of ADC for 4 hours at 37 °C with 5% $CO_2$. The cells were treated with a stimulant such as lipopolysaccharides (LPS) at a certain concentration for a certain time. The plate was then spun at 1000 rpm for 5 minutes and 100 μL of the supernatant culture medium was transferred directly into another 96-well plate, and the concentrations of cytokines such as IL-6 and IL-1β and phosphorylated STAT1 levels were analyzed.

The results show that the ligand-drug conjugates (ADC) of the present application had the ability to affect the release of cytokines, such as IL-6 and IL-1 β, from PBMC cells, and the ability to phosphorylate STAT1.

## Example 12

### 12.1 Type I Interferon-induced response element signal detection

HEK293 cells were constructed and transferred into pHTS-ISRE fluorescein reporter gene plasmid, and a reporter system of type I interferon response signals were constructed. The cells were cultured in a DMEM medium containing FBS and Geneticin with certain concentrations, and the serially-diluted ligand-drug conjugate and type I interferon IFN or recombinant type I interferon with certain concentrations induced by plasmacytoid dendritic cells were added for incubation. The cells were lysed, fluorescein intensity was determined, dose-response data were fitted to an S-shaped curve using non-linear regression, and $IC_{50}$ values were calculated.

The results show that the ligand-drug conjugates of the present application could influence type I interferon-induced response element signals. The ligand-drug conjugates of the present application can inhibit inflammation, and can be used for preventing and/or treating diseases and/or conditions such as inflammation.

### 12.2 Cytokine release induced by CpG-A and the like and downstream signal molecule detection

Primary human peripheral blood monocytes (PBMCs) were washed in 50 mL PBS, resuspended in FBS (fetal bovine serum) with 5% DMSO, divided in equal and cryopreserved in liquid nitrogen until use. The PBMCs were thawed, resuspended in cell culture medium (e.g., RPMI cell culture medium) with 2% FBS and 1% penicillin streptomycin, and seeded onto a 96-well plate. The cells were then incubated with different concentrations of the ligand-drug conjugate at 37 °C with 5% $CO_2$. The cells were treated with CpG-A or other stimulants at a certain concentration for a certain time. The plate was then spun at 1000 rpm for 5 minutes and 100 μL of the supernatant culture medium was transferred directly into another 96-well plate, and the levels of cytokines such as IL-6 and IL-1β were analyzed.

The ligand-drug conjugate with a certain concentration was added into human peripheral blood monocytes for culturing. Then type I interferon IFN or recombinant type I interferon with certain concentrations induced by plasmacytoid dendritic cells was added for incubation. The lysed cells were subjected to electrophoresis and western blot detection, and STAT1 phosphorylation levels were determined using anti-human STAT1 pTY701 antibody.

The results show that the ligand-drug conjugates (ADC) of the present application had the ability to affect the release of cytokines from PBMC cells, and the ability to phosphorylate STAT1.

### 12.3 Evaluation of drug efficacy in plasmacytoid dendritic cell xenograft mouse model

Objective

The ligand-drug conjugates (ADCs) of the present application act on a human plasmacytoid dendritic cell xenograft mouse model, and the *in-vivo* drug effect of the ligand-drug conjugates is evaluated by means of immunohistochemistry, gene transcription analysis and the like.

Test method

1. Imiquimod induction model

The backs of 4-8 week-old severely-immunodeficient mice (CB17/Icr-Prkdcscid/IcrIcoCrl, Charles River) were shaved. The 5% imiquimod cream was applied to the backs of the mice, and 12 hours later, a second application was made. The mice were then injected intraperitoneally with a certain concentration of the ligand-drug conjugates of the present application or the control ligand-drug conjugate. 12 hours later, 1-10 $\times$ $10^5$ human plasmacytoid dendritic cells were injected into the tail vein of the mice. After 12 hours of incubation, the mice were euthanized and dorsal skin samples were collected for testing.

2. Bleomycin induction model

The backs of 4-8 week-old severely-immunodeficient mice (CB17/Icr-Prkdcscid/IcrIcoCrl, Charles River) were shaved. Bleomycin with a certain concentration was injected subcutaneously into a single site on the backs of the mice for three weeks every two days. 1-10 $\times$ $10^5$ human plasmacytoid dendritic cells were injected into the tail vein of the mice on Days 0, 7 and 14 of the first injection of bleomycin. The mice were injected intraperitoneally with the ligand-drug conjugates of the present application or the control ligand-drug conjugate with a certain concentration 24 hours before the first injection of bleomycin, and the injection was performed once every 5 days.

3. Detection method and index

(1) In the kit, mouse skin cell RNA was extracted and subjected to reverse transcription to obtain cDNA, and the influence of the ligand-drug conjugate on the transcription of the type IIFN signaling pathway response gene was determined by utilizing Real-time PCR and taking a mouse sample treated with a control ligand-drug conjugate as a control.
(2) Mouse skin samples were fixed with formalin and embedded in paraffin, which was then sectioned at 5 $\mu$M for hematoxylin-eosin staining. The degree of fibrosis of the skin samples was identified by Masson staining. Immunohistochemical analysis was performed with the pSTAT1 Tyr701 antibody.
(3) After being processed, the mouse skin samples were analyzed by FACS, and the proportion of plasmacytoid dendritic cells in the mouse skin samples was determined according to the cell surface markers.
(4) The collagen content of the mouse skin samples was measured by a collagen analysis method after being processed.

The results show that the ligand-drug conjugates of the present application could affect (1) the ability of the type I IFN signaling pathway to respond to gene transcription, (2) the degree of fibrosis in a skin sample, (3) the ability of plasmacytoid dendritic cells to proliferate and/or (4) the collagen content of skin samples. The ligand-drug conjugate of the present application can inhibit inflammation *in vivo,* and can be used for preventing and/or treating diseases and/or conditions such as inflammation.

## Example 13

### 13.1 Cytokine release experiment for CD40L and LPS-induced dendritic cells derived from human peripheral blood monocytes

Fresh human peripheral blood monocytes (PBMCs) were prepared. Human monocytes were isolated using a human monocyte (CD14+) isolation kit. After washing, the cells were resuspended, added with human IL-4 (80 ng/mL) and human GM-CSF (100 ng/mL) and placed in an incubator and cultured for 5 days.

The cells were harvested, washed, added into a 96-well flat-bottom plate at 105 cell/well, and stimulated with 0.1 ng/mL LPS for 2 hours, and then the supernatant was washed off. A positive compound with a certain concentration was added and incubated for 2 hours. 1 μg/mL Mega CD40L and 0.2 ng/mL LPS were added and incubated in an incubator at 37 °C with 5% $CO_2$ for 20 hours. The cell supernatant was collected by centrifugation, and the TNFα and IL-6 levels in the supernatant were detected using a kit. Dose-response data were fitted to an S-shaped curve using non-linear regression, and IC50 values were calculated.

The results show that the ligand-drug conjugates of the present application could inhibit cytokine release from dendritic cells derived from human peripheral blood monocyte. The ligand-drug conjugates of the present application can inhibit inflammation, and can be used for preventing and/or treating diseases and/or conditions such as inflammation.

**13.2 Evaluation of drug efficacy in LPS-induced acute inflammation model**

8-10 week-old CD40 humanized female mice were randomly grouped and injected intraperitoneally with a concentration of the ligand-drug conjugates of the present application or the control ligand-drug conjugate. After 2-16 hours, the mice were injected intraperitoneally with LPS with a certain concentration. After 6 hours, 100 μL of blood sample was collected, and the TNFα, IL-6 and IL-1β contents were measured using a kit. After another 18 hours, the mice were euthanized, spleens were collected for single cell treatment, and the cells were stained with antibodies to determine the proportion of activated dendritic cells.

The results show that the ligand-drug conjugates of the present application could inhibit the release of cytokines in an LPS-induced acute inflammation mouse model and reduce the proportion of activated dendritic cells in the spleen. The ligand-drug conjugates of the present application can inhibit inflammation, and can be used for preventing and/or treating diseases and/or conditions such as inflammation.

**Example 14: Pharmacokinetic and Toxicity Studies of ADCs at a Single Administration**

Objective

To investigate the pharmacokinetic properties of the drug in monkeys and observe the toxic manifestation of the animals after a single intravenous drip of the ligand-drug conjugates (ADCs) of the present application in monkeys.

Test method

Pharmacokinetics: after the single intravenous drip of the ligand-drug conjugates (ADCs) of the present application at difference doses in monkeys, blood samples were collected at a plurality of continuous time points, and the concentration of the drugs in the blood was detected by a specific detection method.

Toxicity study: after the single intravenous drip of the ligand-drug conjugates (ADCs) of the present application at difference doses in monkeys, the tolerance of animals and drug-related toxicity toxic manifestation were investigated in multiple aspects such as clinical observation, body weight and food intake, hematology, blood biochemistry, urine and gross anatomy.

The results show that: (1) after the ligand-drug conjugates (ADCs) of the present application were subjected to single intravenous drip on a monkey, the concentration of free drugs was very low, and the pharmacokinetic properties of a total antibody and the ligand-drug conjugates (ADCs) of the present application were similar, so that the ligand-drug conjugates (ADCs) of the present application were slowly released in the monkey, the coupling mode was stable, and the clinically planned administration frequency could be supported; and (2) after single intravenous drip of the ligand-drug conjugates (ADCs) of the present application in monkeys, animals had good tolerance and did not show serious or intolerable drug-related toxicity, which indicated that the ligand-drug conjugates (ADCs) of the present application were controllable in safety and could support further clinical application.

The foregoing detailed description is provided by way of illustration and example, and is not intended to limit the scope of the appended claims. Various modifications of the embodiments currently enumerated in the present application will be apparent to those of ordinary skill in the art and are intended to be within the scope of the appended claims and their equivalents.

**Claims**

**1.** A compound of formula I:

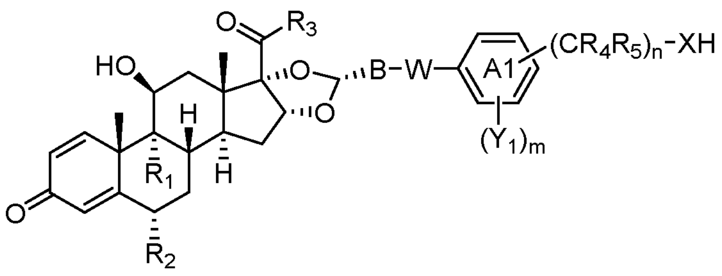

(I)

or a tautomer, a mesomer, a racemate, an enantiomer or a diastereoisomer thereof, or a mixture thereof, or a pharmaceutically acceptable salt thereof, wherein,

$R_1$, $R_2$, $R_3$, $R_4$ and $R_5$ are each independently any group;
B is absent or B is any group;
W is absent or W is any group;
A1 is a substituted benzene ring;
$CR_4R_5$ units are each independently unreplaced or replaced by a group selected from the group consisting of: -O-, -S-, -NR-, -S(O)-, $-S(O)_2-$, -C(=O)-, -C=C- and -C=C-, wherein $R_4$ and $R_5$ can together form optionally substituted cycloalkyl, optionally substituted heterocyclyl, optionally substituted bridged cyclyl, optionally substituted bridged heterocyclyl, optionally substituted spiro cyclyl and optionally substituted spiro heterocyclyl, and n is any integer from 1 to 20;
X is selected from the group consisting of: -O-, -S- and -NR-;
$Y_1$ is any group, and m is any integer from 0 to 4;
wherein substituents are selected from the group consisting of: protium, deuterium, tritium, halogen, -CN, =O, =N-OH, =N-OR, =N-R, -OR, -C(O)R, -C(O)OR, -OC(O)R, -OC(O)OR, -C(O)NHR, - $C(O)NR_2$, -OC(O)NHR, $-OC(O)NR_2$, -SR, -S(O)R, $-S(O)_2R$, -NHR, $-N(R)_2$, -NHC(O)R, -NRC(O)R, -NHC(O)OR, -NRC(O)OR, $-S(O)_2NHR$, $-S(O)_2N(R)_2$, $-NHS(O)_2NR_2$, $-NRS(O)_2NR_2$, $-NHS(O)_2R$, - $NRS(O)_2R$, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy, wherein each R is independently selected from the group consisting of hydrogen, protium, deuterium, tritium, oxygen, hydroxy, halogen, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy.

**2.** The compound of formula I or the tautomer, the mesomer, the racemate, the enantiomer or the diastereoisomer thereof, or the mixture thereof, or the pharmaceutically acceptable salt thereof according to claim 1, selected from the group consisting of:

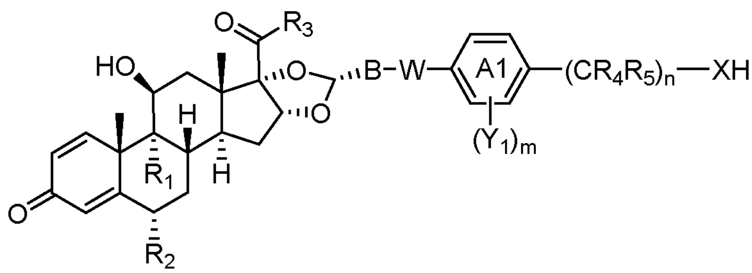

,

(Ia)

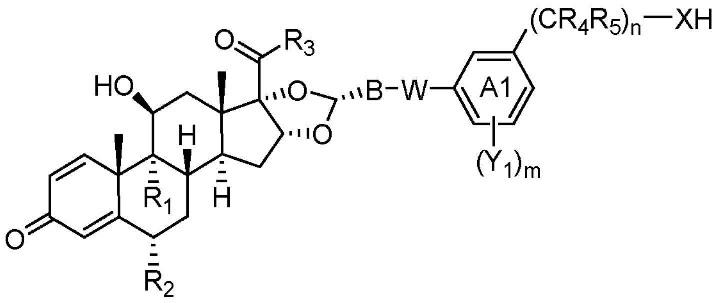

(Ib)

and

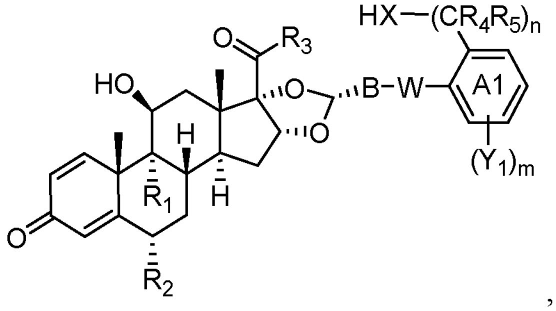

,

(Ic)

wherein,

$R_1$, $R_2$, $R_3$, $R_4$ and $R_5$ are each independently any group;
B is absent or B is any group;
W is absent or W is any group;
A1 is a substituted benzene ring;
$CR_4R_5$ units are each independently unreplaced or replaced by a group selected from the group consisting of: -O-, -S-, -NR-, -S(O)-, $-S(O)_2-$, -C(=O)-, -C=C- and -C=C-, wherein $R_4$ and $R_5$ can together form optionally substituted cycloalkyl, optionally substituted heterocyclyl, optionally substituted bridged cyclyl, optionally substituted bridged heterocyclyl, optionally substituted spiro cyclyl and optionally substituted spiro heterocyclyl, and n is any integer from 1 to 20;
X is selected from the group consisting of: -O-, -S- and -NR-;
$Y_1$ is any group, and m is 0 or 1;
wherein substituents are selected from the group consisting of: protium, deuterium, tritium, halogen, -CN, =O, =N-OH, =N-OR, =N-R, -OR, -C(O)R, -C(O)OR, -OC(O)R, -OC(O)OR, -C(O)NHR, - $C(O)NR_2$, -OC(O)NHR, $-OC(O)NR_2$, -SR, -S(O)R, $-S(O)_2R$, -NHR, $-N(R)_2$, -NHC(O)R, -NRC(O)R, -NHC(O)OR, -NRC(O)OR, $-S(O)_2NHR$, $-S(O)_2N(R)_2$, $-NHS(O)_2NR_2$, $-NRS(O)_2NR_2$, $-NHS(O)_2R$, - $NRS(O)_2R$, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy, wherein each R is independently selected from the group consisting of hydrogen, protium, deuterium, tritium, oxygen, hydroxy, halogen, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy.

**3.** The compound or the tautomer, the mesomer, the racemate, the enantiomer or the diastereoisomer thereof, or the mixture thereof, or the pharmaceutically acceptable salt thereof according to any one of claims 1-2, wherein X is -O-, -S- or -NH-.

**4.** The compound or the tautomer, the mesomer, the racemate, the enantiomer or the diastereoisomer thereof, or the mixture thereof, or the pharmaceutically acceptable salt thereof according to any one of claims 1-3, wherein $CR_4R_5$

units are each independently unreplaced or replaced by a group selected from the group consisting of: -O-, -S- and -C(=O)-.

5. The compound or the tautomer, the mesomer, the racemate, the enantiomer or the diastereoisomer thereof, or the mixture thereof, or the pharmaceutically acceptable salt thereof according to any one of claims 1-4, wherein $R_4$ and $R_5$ are each independently selected from the group consisting of: hydrogen, protium, deuterium, tritium, halogen, optionally substituted -C(=O)H, optionally substituted -OH, optionally substituted -SH, optionally substituted $-NH_2$, optionally substituted alkyl, optionally substituted alkenyl, optionally substituted alkynyl, optionally substituted cycloalkyl, optionally substituted heterocycloalkyl, optionally substituted aryl and optionally substituted heteroaryl; wherein, when $R_4$ and $R_5$ comprise a methylene unit, the methylene units of $R_4$ and $R_5$ are each independently unreplaced, or the methylene units of $R_4$ and $R_5$ are each independently replaced by a group selected from the group consisting of: -S(=O)-, $-S(=O)_2-$, -O-, -S-, -C(=O)-, optionally substituted -PH-, optionally substituted -P(=O)H-, optionally substituted -NH-, optionally substituted alkylene, optionally substituted alkenylene, optionally substituted alkynylene, optionally substituted cycloalkylene, optionally substituted heterocycloalkylene, optionally substituted arylene and optionally substituted heteroarylene.

6. The compound or the tautomer, the mesomer, the racemate, the enantiomer or the diastereoisomer thereof, or the mixture thereof, or the pharmaceutically acceptable salt thereof according to claim 5, wherein $R_4$ and $R_5$ are each independently selected from the group consisting of: H, F, Cl, -OH, $-NH_2$ and $C_1$-$C_6$ alkyl, or $R_4$ and $R_5$ together form $C_3$-$C_6$ cycloalkyl or 3-6 membered heterocyclyl; and n is selected from the group consisting of 1, 2 and 3.

7. The compound or the tautomer, the mesomer, the racemate, the enantiomer or the diastereoisomer thereof, or the mixture thereof, or the pharmaceutically acceptable salt thereof according to claim 6, wherein the $(CR_4R_5)_n$- are each independently selected from the group consisting of $-CH_2-$,

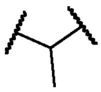,

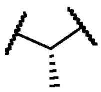, 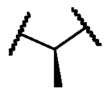,

$-CH_2CH_2-$ and

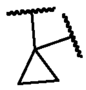.

8. The compound or the tautomer, the mesomer, the racemate, the enantiomer or the diastereoisomer thereof, or the mixture thereof, or the pharmaceutically acceptable salt thereof according to any one of claims 1-7, wherein $Y_1$ is absent or selected from the group consisting of: protium, deuterium, tritium, halogen, -OR, -SR, -NHR, $-N(R)_2$, optionally substituted $C_1$-$C_6$ alkyl and optionally substituted $C_1$-$C_6$ alkoxy; wherein each R is independently selected from the group consisting of hydrogen, protium, deuterium, tritium, hydroxy, halogen, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy.

9. The compound or the tautomer, the mesomer, the racemate, the enantiomer or the diastereoisomer thereof, or the mixture thereof, or the pharmaceutically acceptable salt thereof according to any one of claims 1-8, wherein $Y_1$ is absent or selected from the group consisting of: hydroxy, sulfhydryl, amino and optionally substituted $C_1$-$C_6$ alkyl.

10. The compound or the tautomer, the mesomer, the racemate, the enantiomer or the diastereoisomer thereof, or the mixture thereof, or the pharmaceutically acceptable salt thereof according to any one of claims 1-8, wherein A1 is selected from the group consisting of:

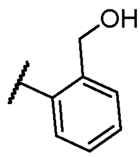, 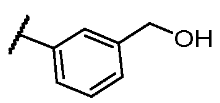, 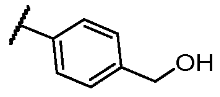, 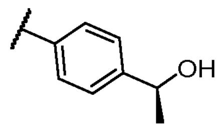, 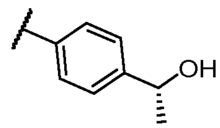,

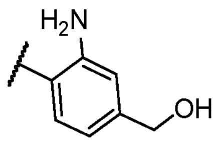, 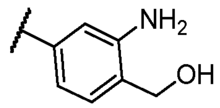, 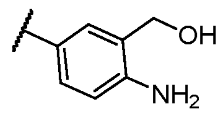, 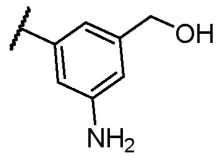, 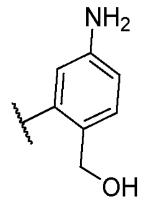,

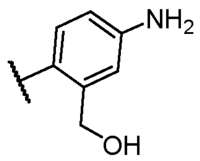, 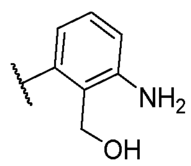, 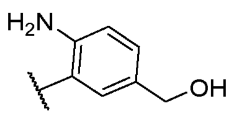, 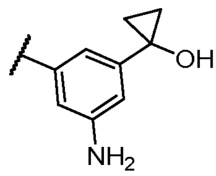, 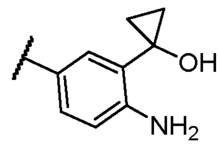,

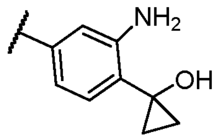, 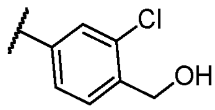, 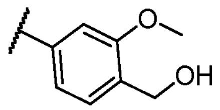, 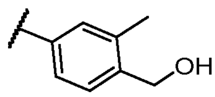, 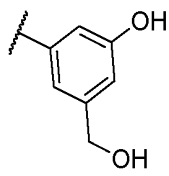,

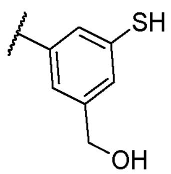, 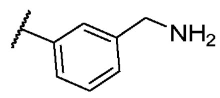, 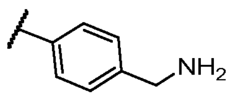, 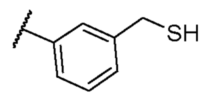, 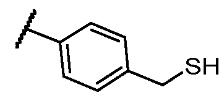,

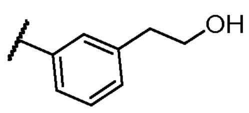, 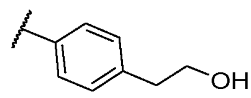, 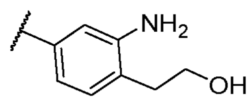, 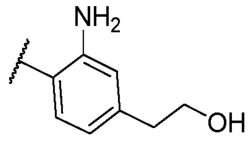,

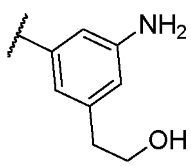 and 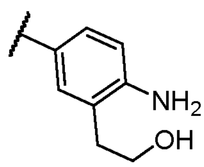.

11. The compound or the tautomer, the mesomer, the racemate, the enantiomer or the diastereoisomer thereof, or the mixture thereof, or the pharmaceutically acceptable salt thereof according to any one of claims 1-10, wherein $R_1$ and $R_2$ are each independently selected from the group consisting of: H, F, Cl, Br and optionally substituted $C_1$-$C_6$ alkyl.

12. The compound or the tautomer, the mesomer, the racemate, the enantiomer or the diastereoisomer thereof, or the mixture thereof, or the pharmaceutically acceptable salt thereof according to any one of claims 1-11, wherein $R_1$ and $R_2$ are each independently selected from the group consisting of: H, F, Cl, Br and optionally substituted methyl.

13. The compound or the tautomer, the mesomer, the racemate, the enantiomer or the diastereoisomer thereof, or the mixture thereof, or the pharmaceutically acceptable salt thereof according to any one of claims 1-12, wherein $R_3$ is selected from the group consisting of: hydrogen, optionally substituted -OH, optionally substituted -SH and optionally substituted $C_1$-$C_6$ alkyl.

14. The compound or the tautomer, the mesomer, the racemate, the enantiomer or the diastereoisomer thereof, or the mixture thereof, or the pharmaceutically acceptable salt thereof according to any one of claims 1-13, wherein $R_3$ is selected from the group consisting of: optionally substituted $-CH_2Cl$, optionally substituted $-CH_2SH$, optionally substituted $-CH_2OH$, optionally substituted

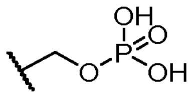

,

optionally substituted

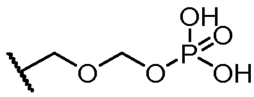

,

optionally substituted

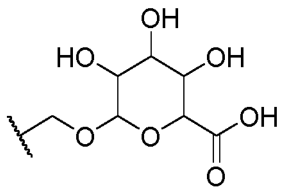

,

optionally substituted

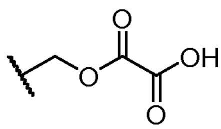

,

optionally substituted

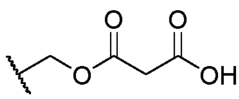

,

optionally substituted

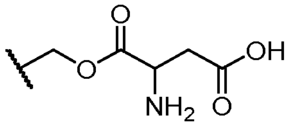

,

optionally substituted

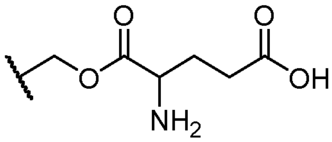,

optionally substituted

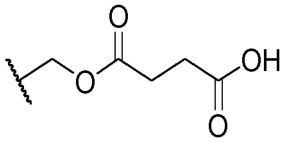,

optionally substituted

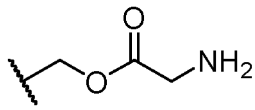,

optionally substituted

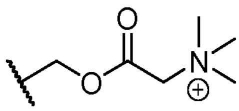,

optionally substituted

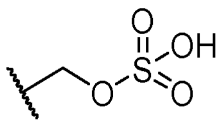,

optionally substituted

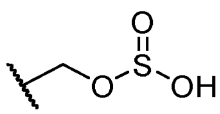,

optionally substituted

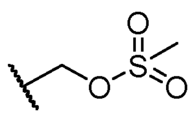,

optionally substituted -OH, optionally substituted $-OCH_3$, optionally substituted-$OCH_2F$, optionally substituted-$OCH_2Cl$, optionally substituted $-OCH_2CN$, optionally substituted $-OCH_2CH_3$, optionally substituted sulfhydryl, optionally substituted $-SCH_2F$, optionally substituted $-SCH_2Cl$, optionally substituted $-SCH_2CF_3$ and optionally substituted $-SCH_2CN$.

**15.** The compound or the tautomer, the mesomer, the racemate, the enantiomer or the diastereoisomer thereof, or the mixture thereof, or the pharmaceutically acceptable salt thereof according to any one of claims 1-14, wherein $R_3$ is selected from the group consisting of: $-CH_2Cl$, $-CH_2SH$, $-CH_2OH$,

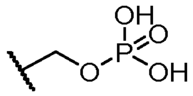,

$-OCH_3$, $-OCH_2F$, $-OCH_2Cl$, $-OCH_2CN$, $-OCH_2CH_3$, -SH, $-SCH_2F$, $-SCH_2Cl$, - $SCH_2CF_3$ and $-SCH_2CN$.

**16.** The compound or the tautomer, the mesomer, the racemate, the enantiomer or the diastereoisomer thereof, or the mixture thereof, or the pharmaceutically acceptable salt thereof according to any one of claims 1-15, wherein B is selected from the group consisting of: optionally substituted

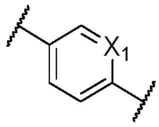,

optionally substituted

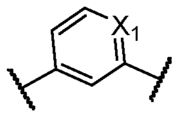

and optionally substituted

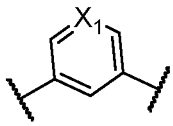;

and $X_1$ is selected from the group consisting of: CH, C(-O-$CH_3$) and N.

**17.** The compound or the tautomer, the mesomer, the racemate, the enantiomer or the diastereoisomer thereof, or the mixture thereof, or the pharmaceutically acceptable salt thereof according to any one of claims 1-15, wherein B is selected from the group consisting of: optionally substituted

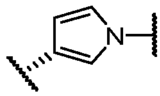,

optionally substituted

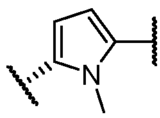,

optionally substituted

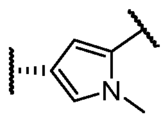,

optionally substituted

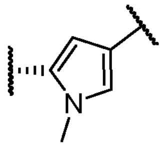

,

optionally substituted

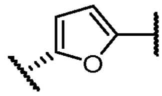

,

optionally substituted

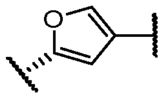

,

optionally substituted

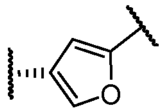

,

optionally substituted

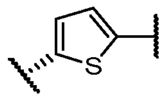

,

optionally substituted

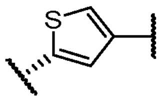

,

optionally substituted

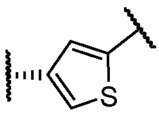

,

optionally substituted

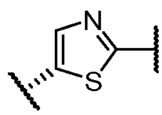

,

optionally substituted

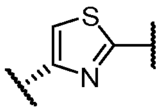,

optionally substituted

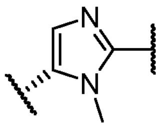,

optionally substituted

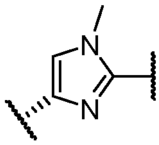,

optionally substituted

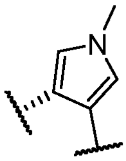,

optionally substituted

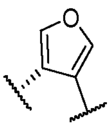,

optionally substituted

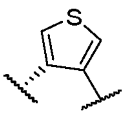,

optionally substituted

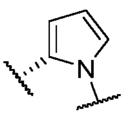,

optionally substituted

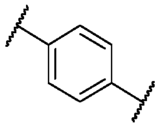

and optionally substituted

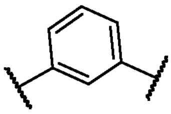,

wherein substituents are selected from the group consisting of: protium, deuterium, tritium, halogen, -CN, =O, =N-OH, =N-OR, =N-R, -OR, - C(O)R, -C(O)OR, -OC(O)R, -OC(O)OR, -C(O)NHR, $-C(O)NR_2$, -OC(O)NHR, $-OC(O)NR_2$, -SR, - S(O)R, $-S(O)_2R$, -NHR, $-N(R)_2$, -NHC(O)R, -NRC(O)R, -NHC(O)OR, -NRC(O)OR, $-S(O)_2NHR$, - $S(O)_2N(R)_2$, $-NHS(O)_2NR_2$, $-NRS(O)_2NR_2$, $-NHS(O)_2R$, $-NRS(O)_2R$, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy, wherein each R is independently selected from the group consisting of hydrogen, protium, deuterium, tritium, oxygen, halogen, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy.

**18.** The compound or the tautomer, the mesomer, the racemate, the enantiomer or the diastereoisomer thereof, or the mixture thereof, or the pharmaceutically acceptable salt thereof according to any one of claims 1-17, wherein B is selected from the group consisting of:

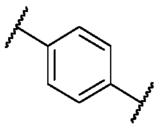, 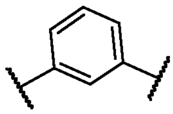 and 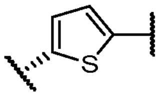.

**19.** The compound or the tautomer, the mesomer, the racemate, the enantiomer or the diastereoisomer thereof, or the mixture thereof, or the pharmaceutically acceptable salt thereof according to any one of claims 1-18, wherein W is absent or W is selected from the group consisting of: -S(=O)-, $-S(=O)_2-$, -O-, -S-, -C(=O)-, optionally substituted $-CH_2NHC(=O)-$, optionally substituted $-NHC(=O)CH_2-$, optionally substituted -C(=O)NH-, optionally substituted -NH-, optionally substituted -CH=CH-, - C=C-, optionally substituted $C_1$-$C_6$ alkylene, optionally substituted $-OCH_2-$, optionally substituted - $CH_2O-$, optionally substituted $-SCH_2-$, optionally substituted $-CH_2S-$, optionally substituted - NHC(=O)-, optionally substituted $-C(=O)CH_2-$, optionally substituted $-CH_2NH-$, optionally substituted $-NHCH_2-$, optionally substituted

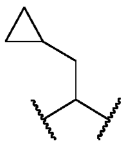,

optionally substituted

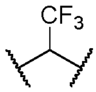,

optionally substituted

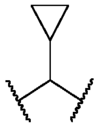,

optionally substituted

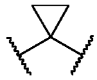

and optionally substituted

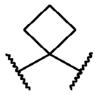.

**20.** The compound or the tautomer, the mesomer, the racemate, the enantiomer or the diastereoisomer thereof, or the mixture thereof, or the pharmaceutically acceptable salt thereof according to claim 19, wherein W is absent or W is selected from the group consisting of:

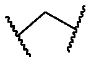, 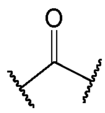, 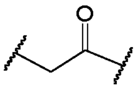, 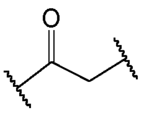, 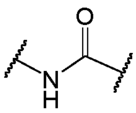, 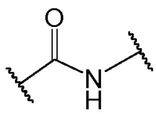, 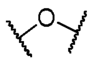, 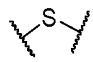, 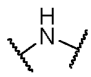,

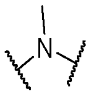, 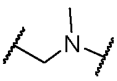, 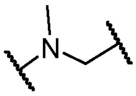, 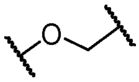, 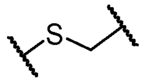, 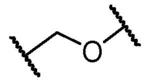, 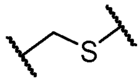, 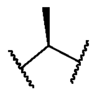,

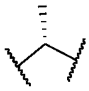, 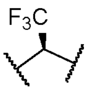, 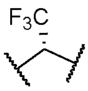, 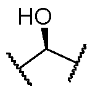, 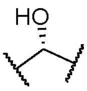, 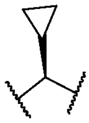, 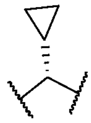, 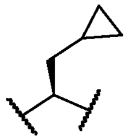, 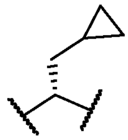, 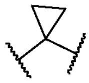,

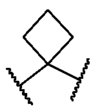, 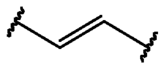 and 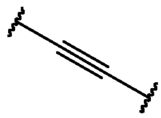.

**21.** The compound or the tautomer, the mesomer, the racemate, the enantiomer or the diastereoisomer thereof, or the mixture thereof, or the pharmaceutically acceptable salt thereof according to claim 20, wherein W is absent or W is

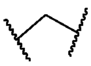.

**22.** The compound or the tautomer, the mesomer, the racemate, the enantiomer or the diastereoisomer thereof, or the mixture thereof, or the pharmaceutically acceptable salt thereof according to any one of claims 1-21, wherein B is absent or selected from the group consisting of:

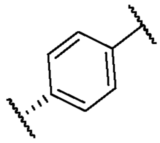,

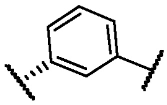, 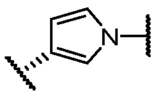, 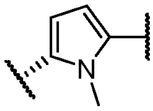, 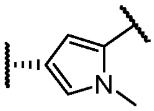, 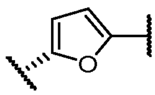, 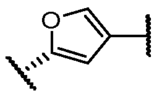,

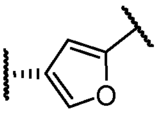, 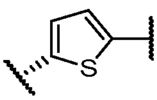, 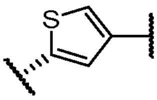, 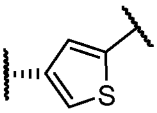, 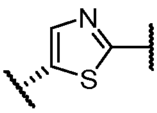 and 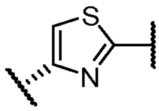;

W is absent or W is selected from the group consisting of:

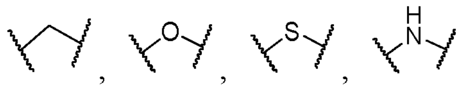 , , , ,

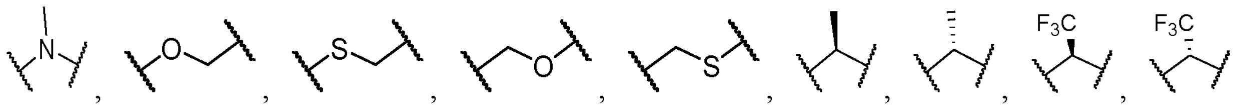 , , , , , , , , ,

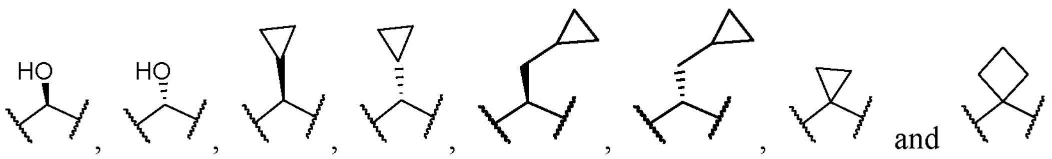 , , , , , , and ;

A1 is selected from the group consisting of:

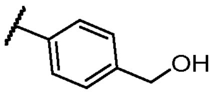, 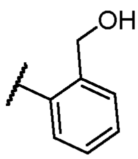, 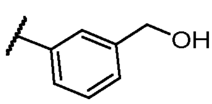, 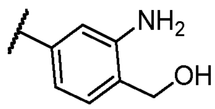, 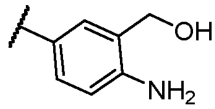,

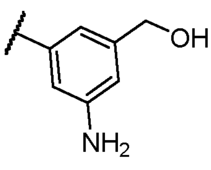, 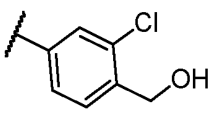, 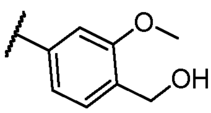, 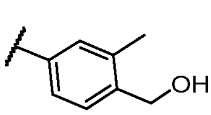, 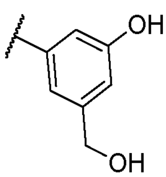,

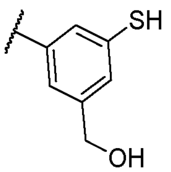, 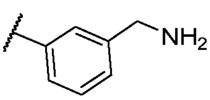, 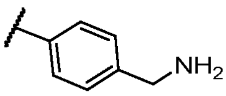, 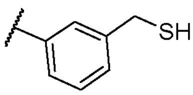, 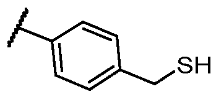,

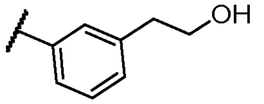 and 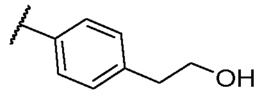.

**23.** The compound or the tautomer, the mesomer, the racemate, the enantiomer or the diastereoisomer thereof, or the mixture thereof, or the pharmaceutically acceptable salt thereof according to any one of claims 1-22, selected from the group consisting of

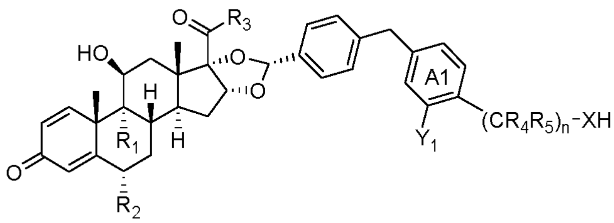

(Ia-1)

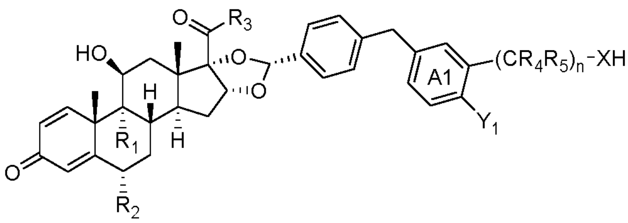

(Ib-1)

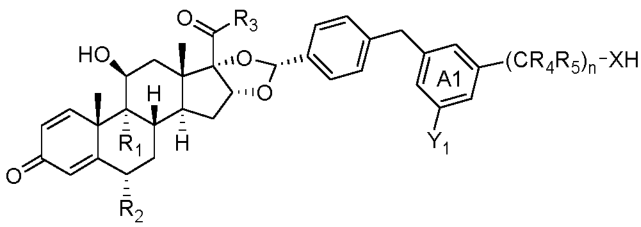

(Ic-1)

,

wherein,

X is selected from the group consisting of -O-, -S- and -NH-;
$R_4$ and $R_5$ are each independently selected from the group consisting of: H, F, Cl, -OH, $-NH_2$ and $C_1$-$C_6$ alkyl;
n is selected from the group consisting of 1, 2 and 3;
$Y_1$ is absent or selected from the group consisting of: hydroxy, sulfhydryl, amino and $C_1$-$C_6$ alkyl;
$R_1$ and $R_2$ are each independently selected from the group consisting of hydrogen, fluorine, chlorine and methyl;
$R_3$ is selected from the group consisting of: $-CH_2Cl$, $-CH_2SH$, $-CH_2OH$,

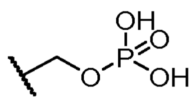,

$-OCH_3$, - $OCH_2F$, $-OCH_2Cl$, $-OCH_2CN$, $-OCH_2CH_3$, -SH, $-SCH_2F$, $-SCH_2Cl$, $-SCH_2CF_3$ and $-SCH_2CN$.

**24.** The compound or the tautomer, the mesomer, the racemate, the enantiomer or the diastereoisomer thereof, or the mixture thereof, or the pharmaceutically acceptable salt thereof according to any one of claims 1-23, wherein the compound is selected from the group consisting of the following structures:

| No. | Structure |
|---|---|
| I-1 | 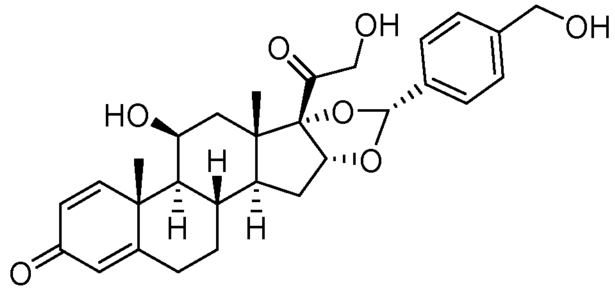 |
| I-2 | 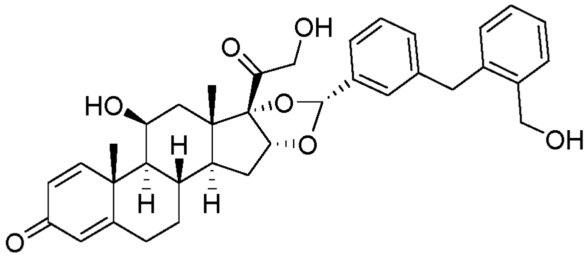 |
| I-3 | 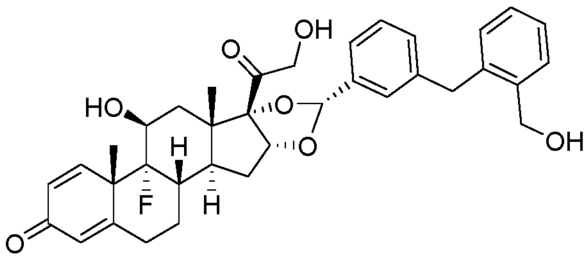 |
| I-4 | 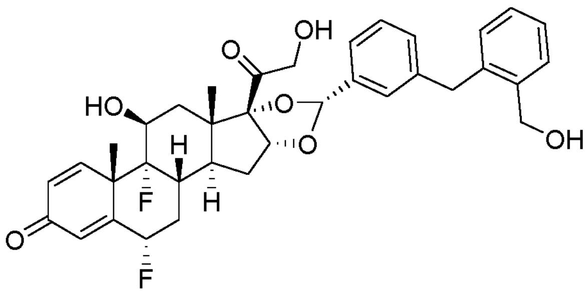 |
| I-5 | 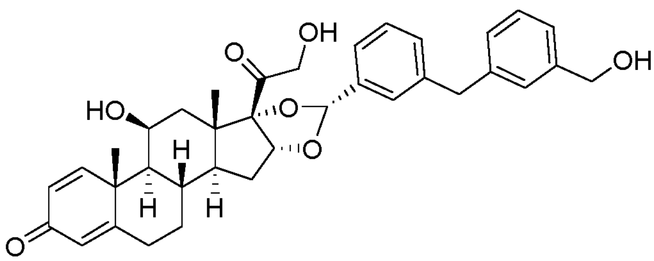 |
| I-6 | 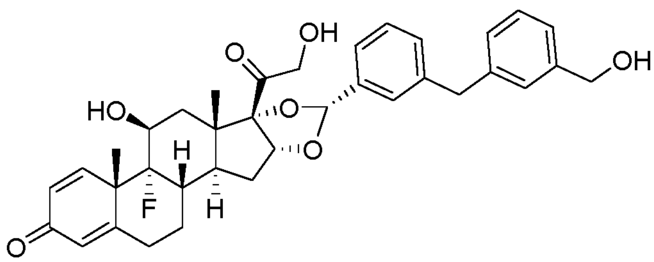 |

(continued)

| No. | Structure |
|---|---|
| I-7 | 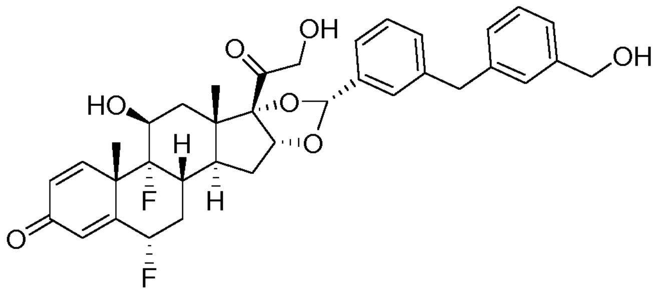 |
| I-8 | 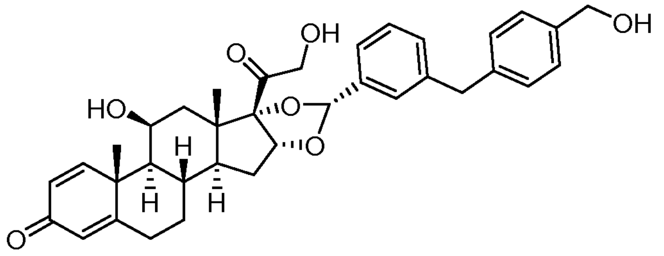 |
| I-9 | 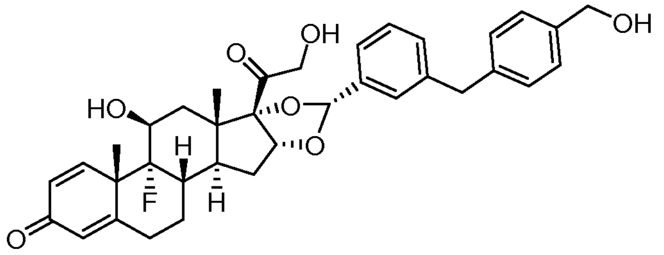 |
| I-10 | 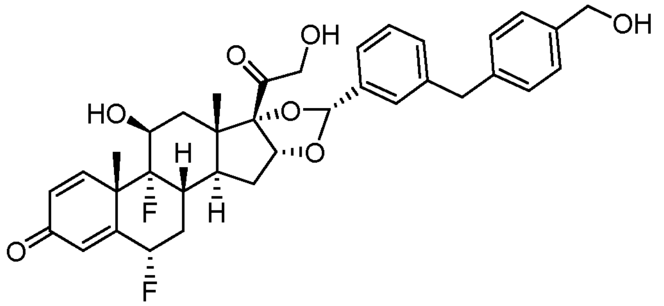 |
| I-11 | 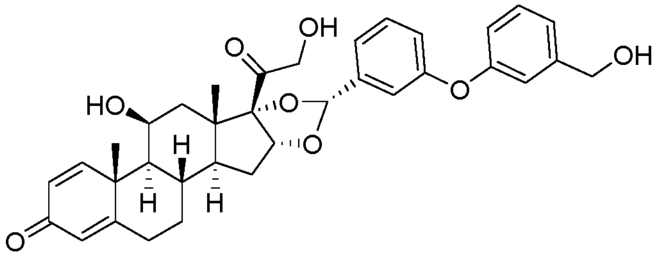 |

(continued)

| No. | Structure |
|---|---|
| I-12 | 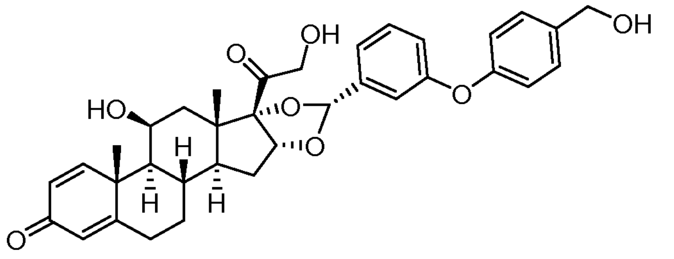 |
| I-13 | 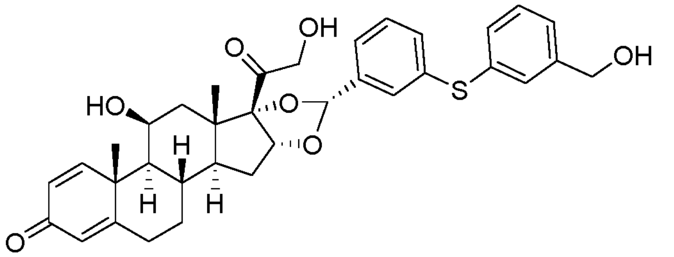 |
| I-14 | 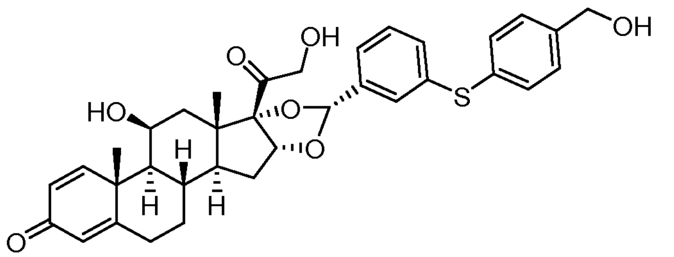 |
| I-15 | 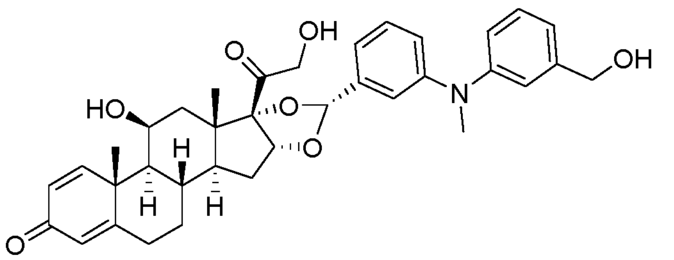 |
| I-16 | 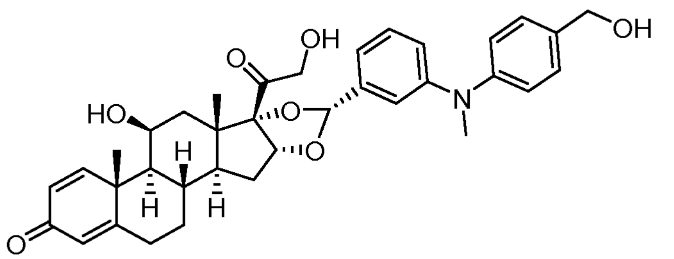 |
| I-17 | 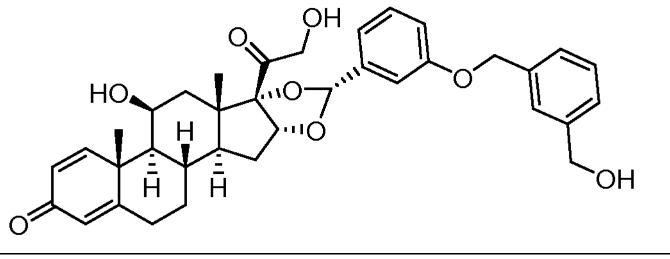 |

(continued)

| No. | Structure |
| --- | --- |
| I-18 | 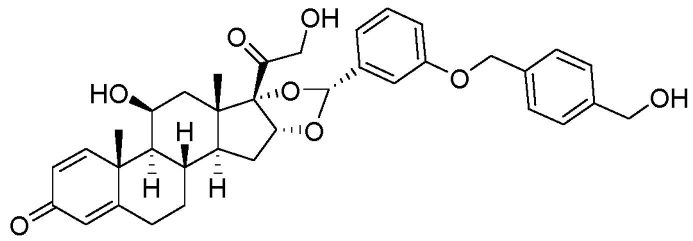 |
| I-19 | 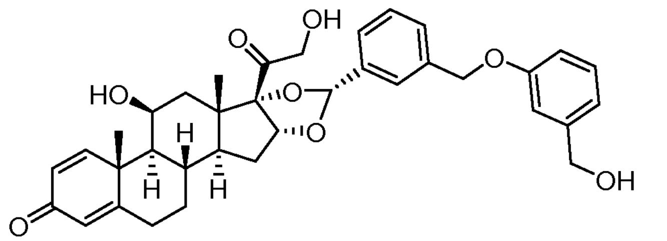 |
| I-20 | 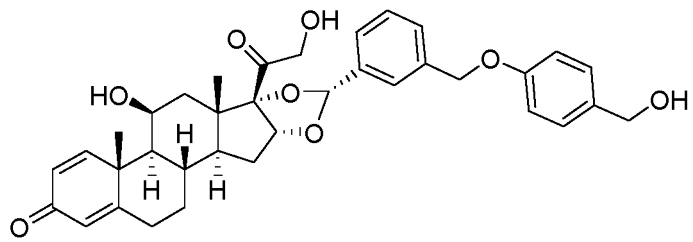 |
| I-21 | 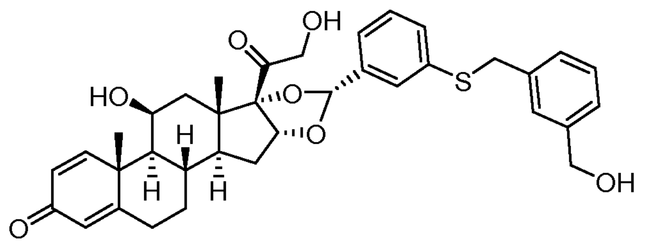 |
| I-22 | 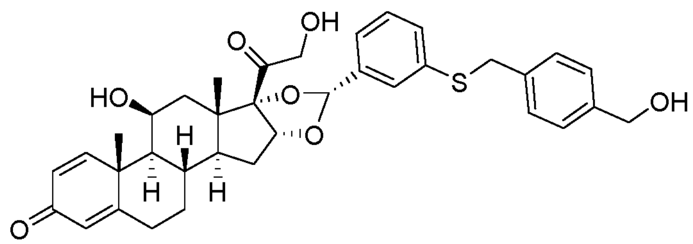 |
| I-23 | 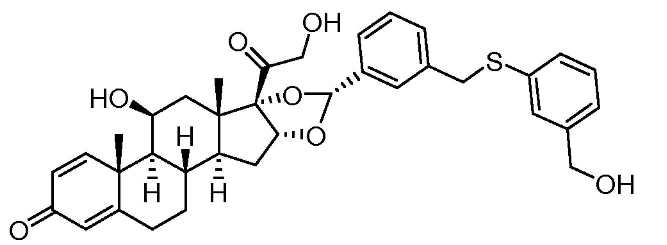 |

(continued)

| No. | Structure |
|---|---|
| I-24 | 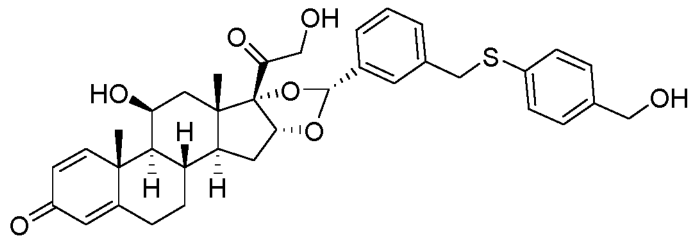 |
| I-25 | 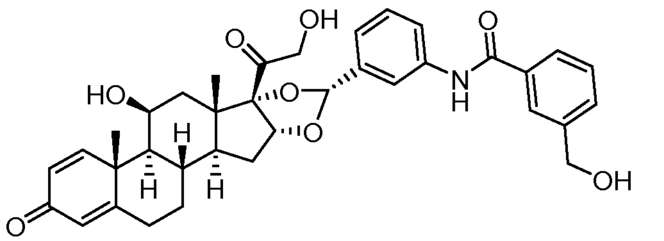 |
| I-26 | 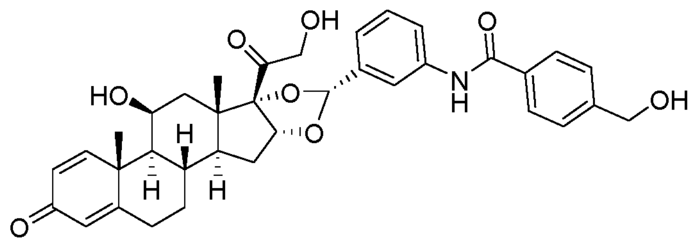 |
| I-27 | 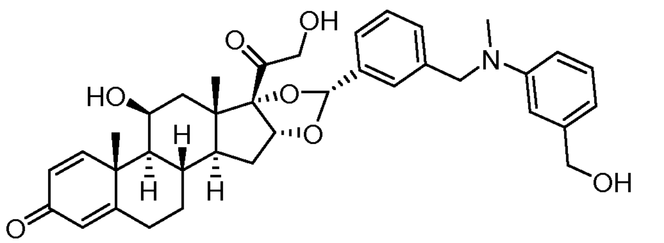 |
| I-28 | 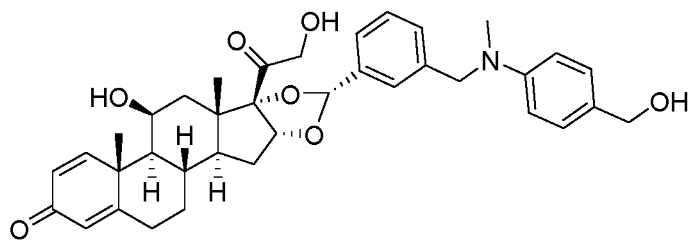 |
| I-29 | 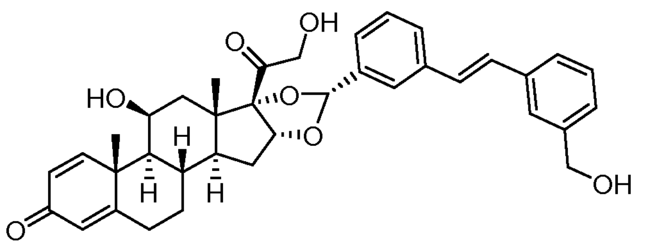 |

(continued)

| No. | Structure |
|---|---|
| 1-30 | 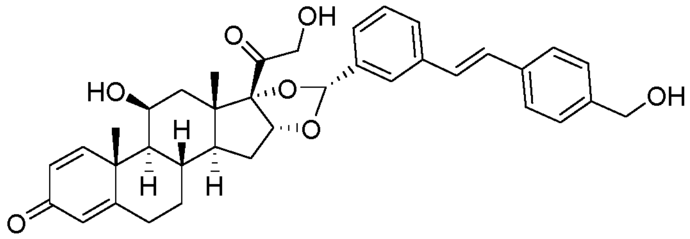 |
| I-31 | 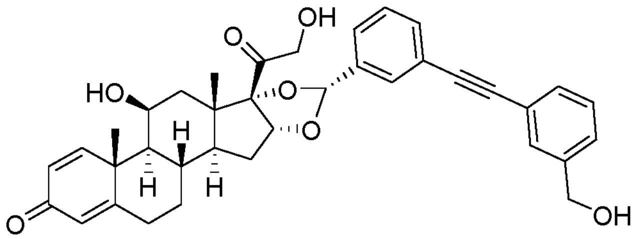 |

| | |
|---|---|
| I-32 | 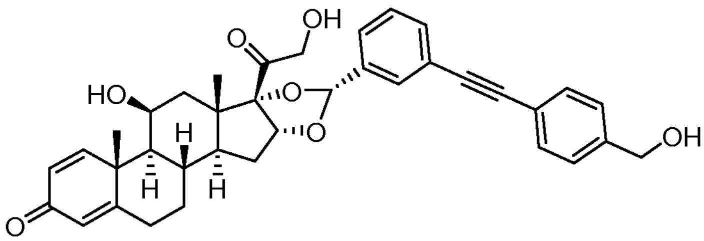 |
| I-33 | 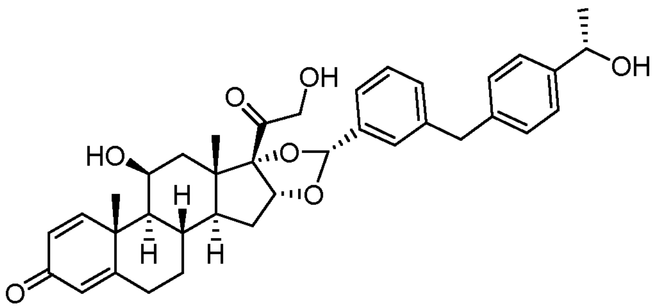 |
| I-34 | 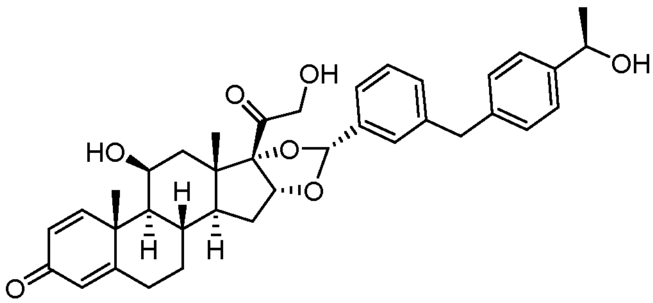 |

(continued)

| | |
|---|---|
| I-35 | 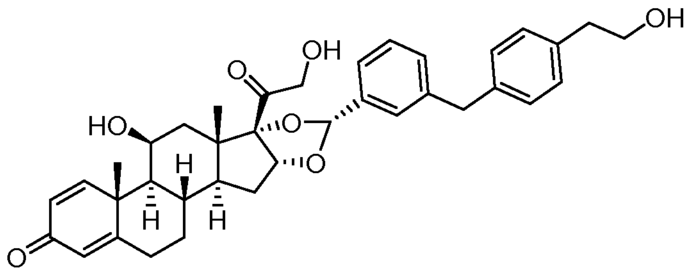 |
| I-36 | 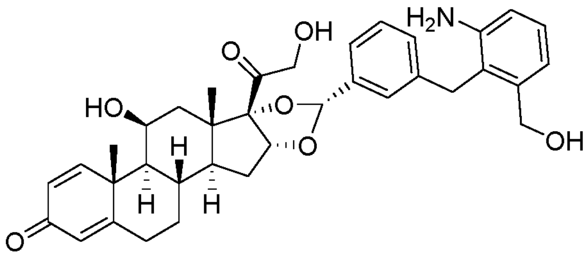 |
| I-37 | 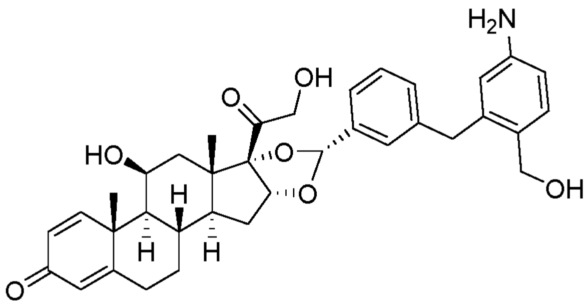 |
| I-38 | 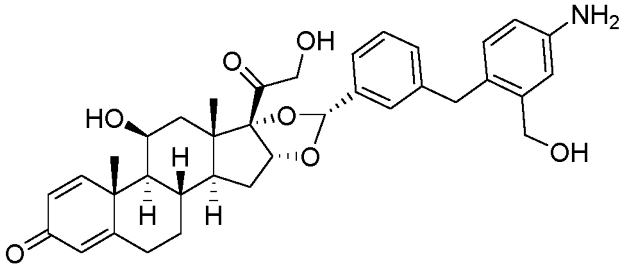 |
| I-39 | 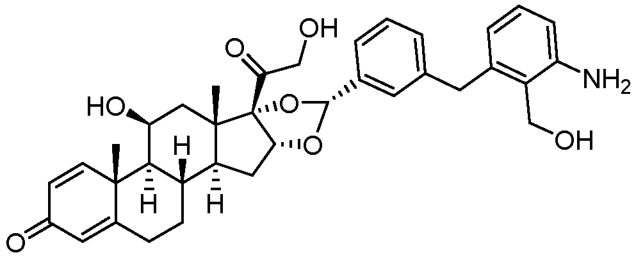 |
| I-40 | 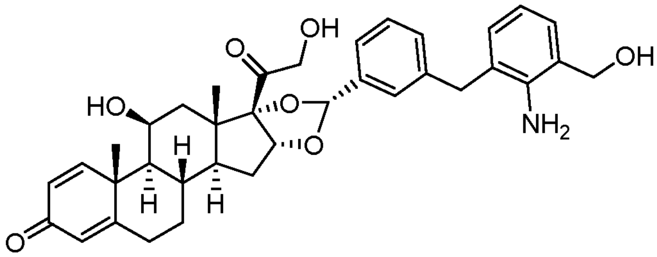 |

(continued)

| | |
|---|---|
| I-41 | 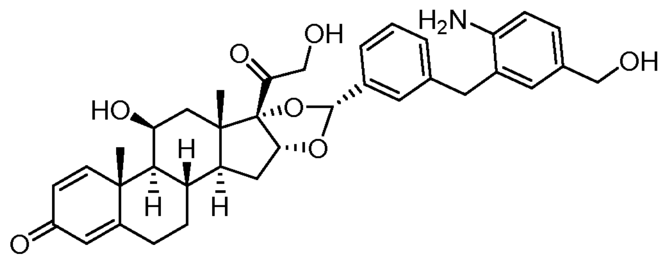 |
| I-42 | 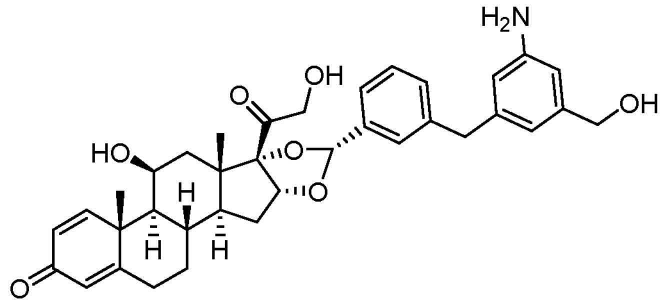 |
| I-43 | 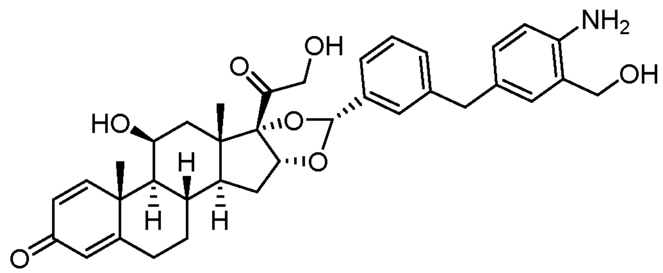 |
| I-44 | 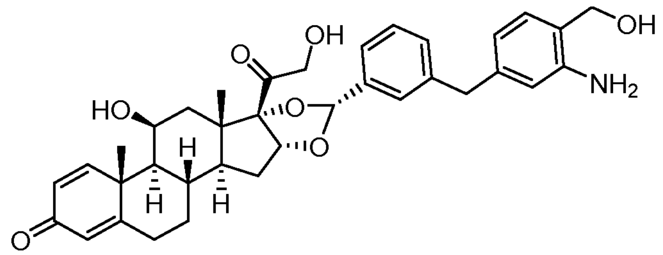 |
| I-45 | 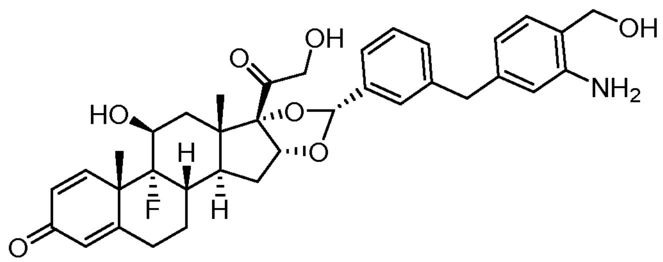 |

(continued)

| | |
|---|---|
| I-46 | 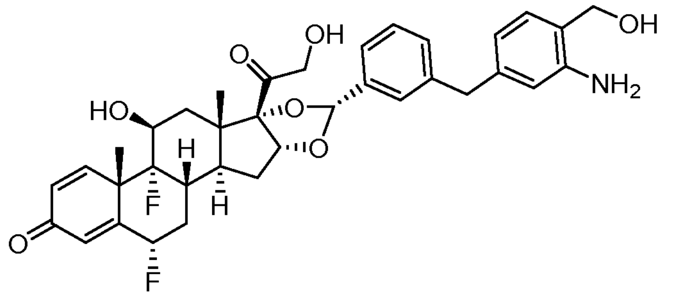 |
| I-47 | 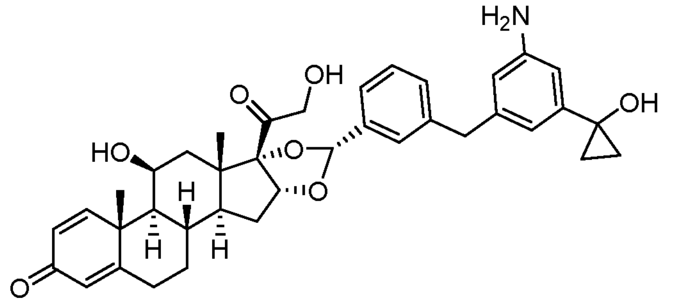 |
| I-48 | 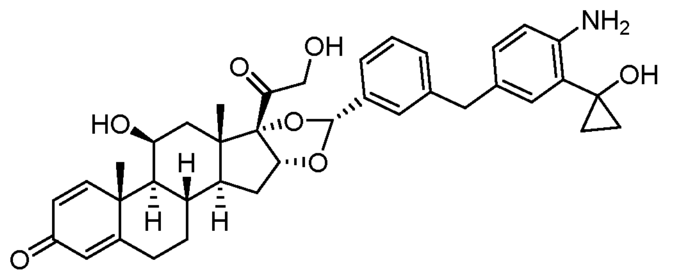 |
| I-49 | 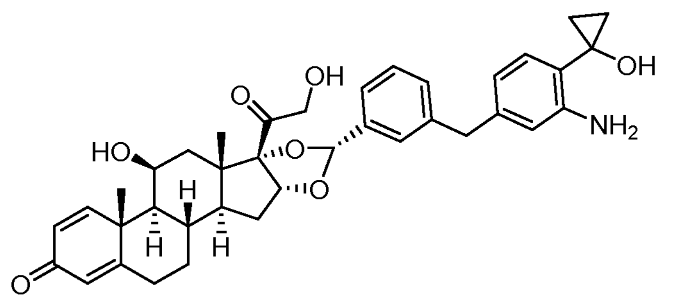 |
| I-50 | 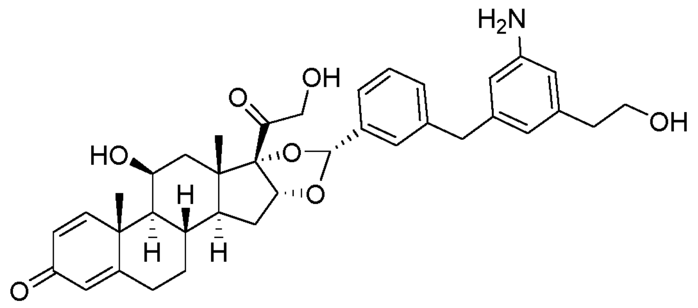 |

(continued)

| | |
|---|---|
| I-51 | 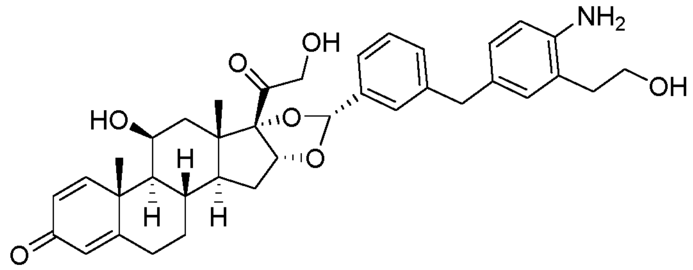 |
| I-52 | 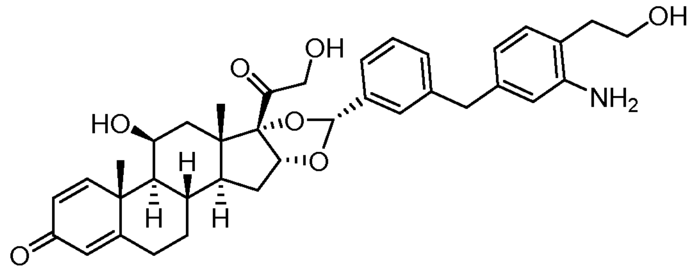 |
| I-53 | 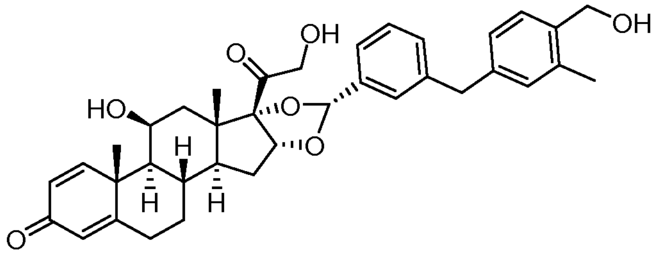 |
| I-54 | 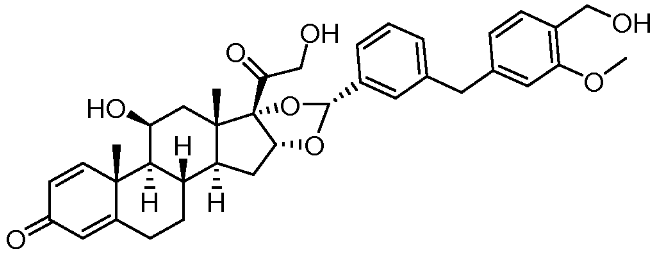 |
| 1-55 | 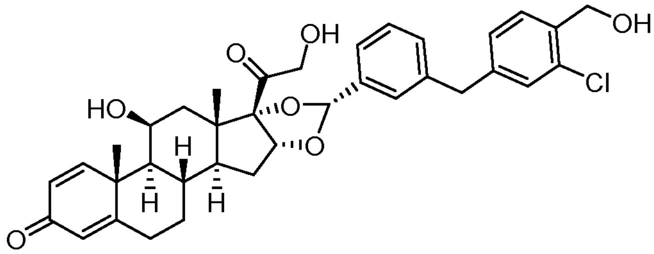 |
| I-56 | 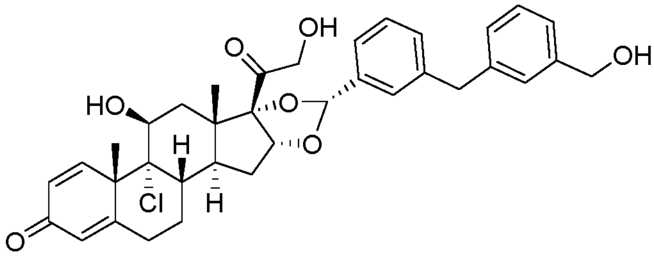 |

(continued)

| | |
|---|---|
| I-57 | 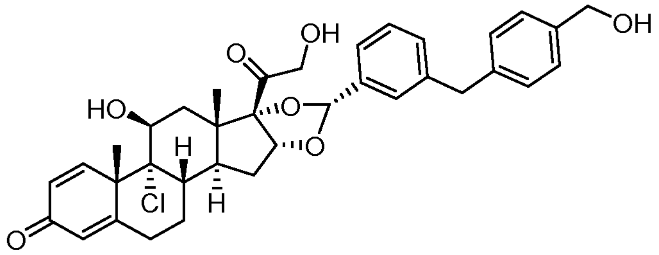 |
| I-58 | 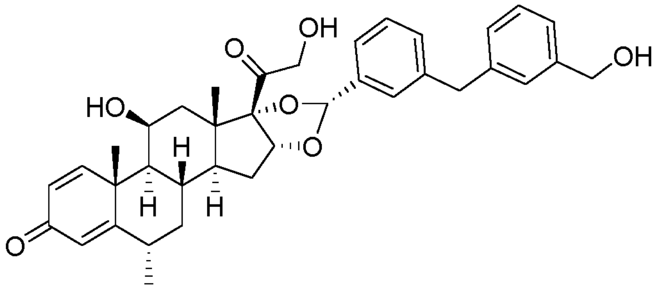 |
| I-59 | 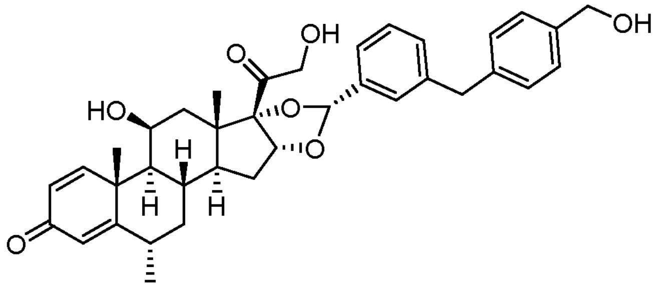 |
| I-60 | 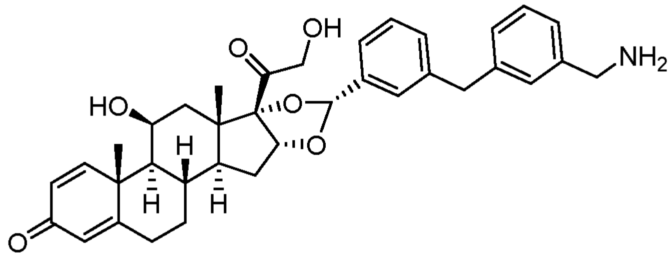 |
| I-61 | 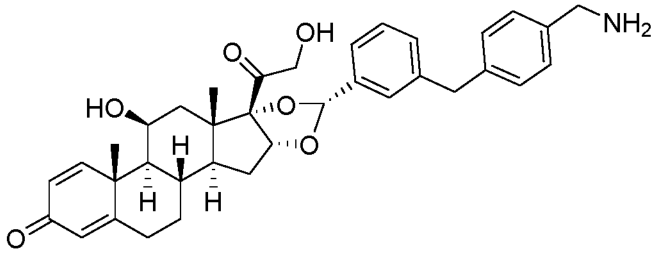 |
| I-62 | 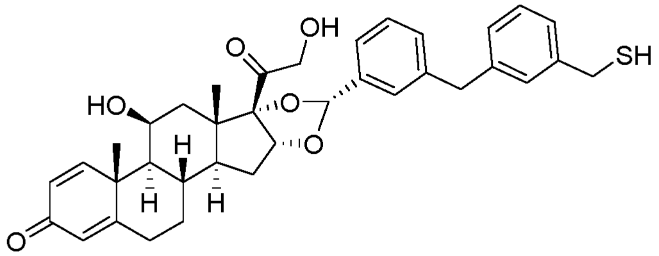 |

(continued)

| | |
|---|---|
| I-63 | 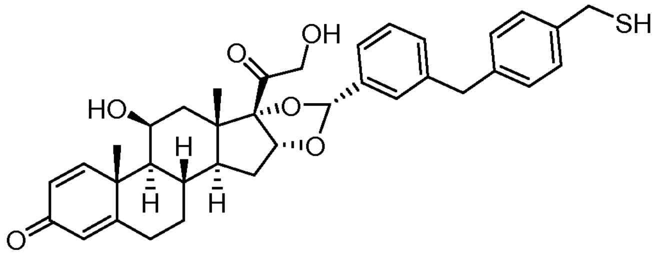 |
| I-64 | 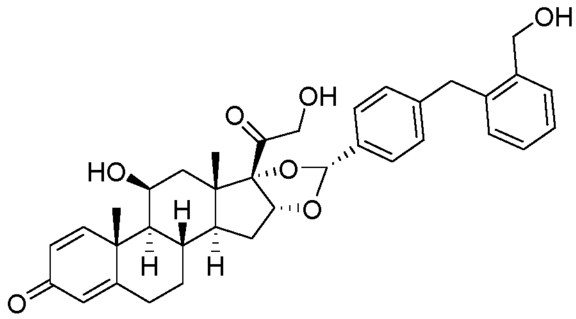 |
| I-65 | 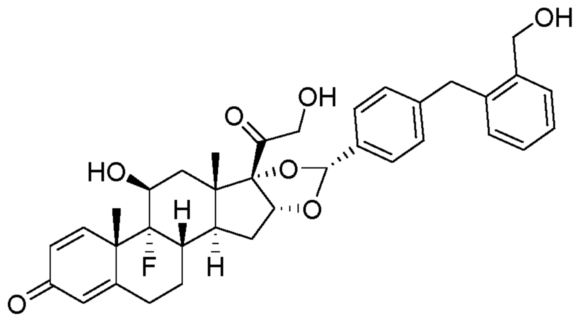 |
| I-66 | 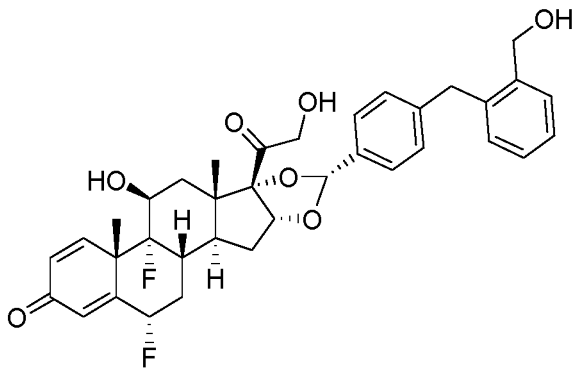 |
| I-67 | 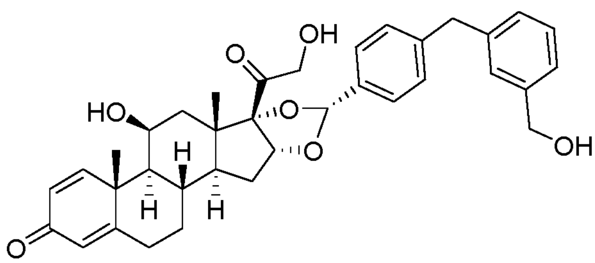 |

(continued)

| | |
|---|---|
| I-68 | 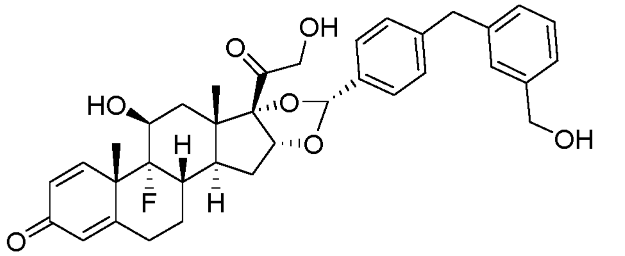 |
| I-69 | 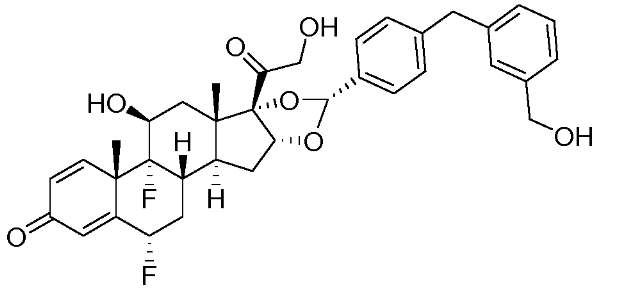 |
| I-70 | 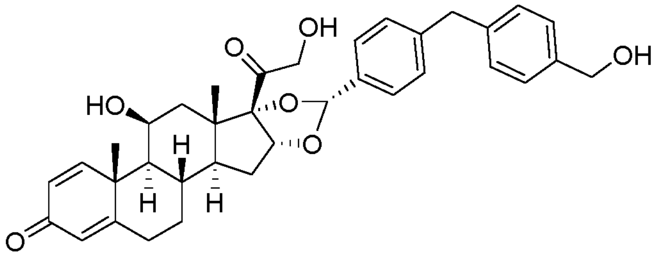 |
| I-71 | 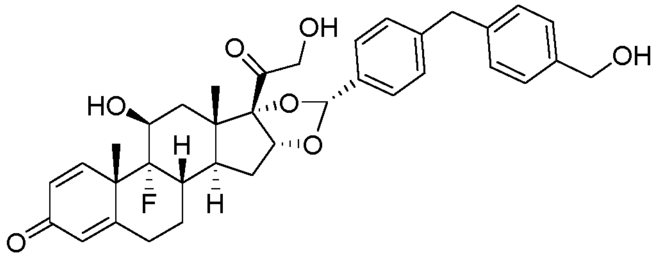 |
| I-72 | 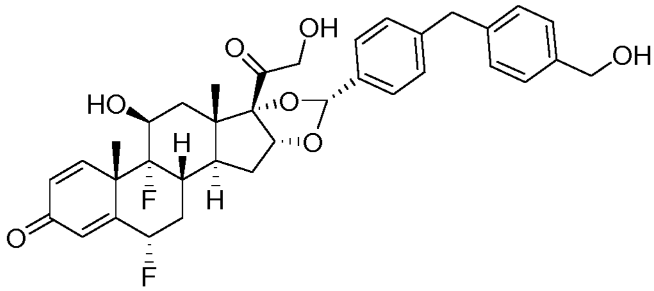 |
| I-73 | 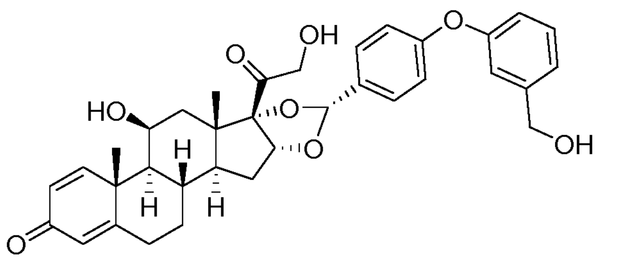 |

(continued)

| | |
|---|---|
| I-74 | 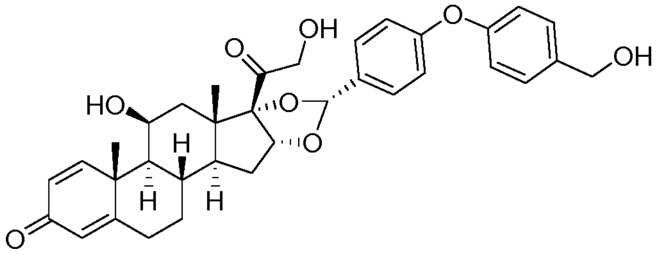 |
| I-75 | 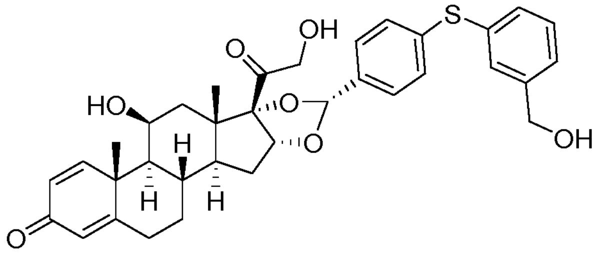 |
| I-76 | 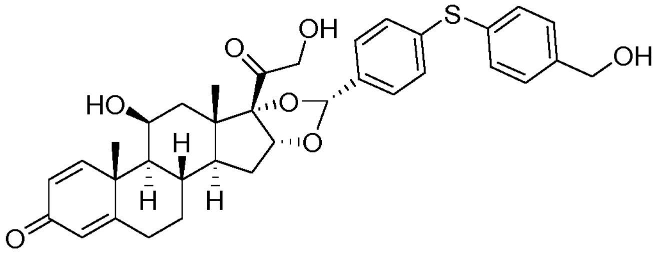 |
| I-77 | 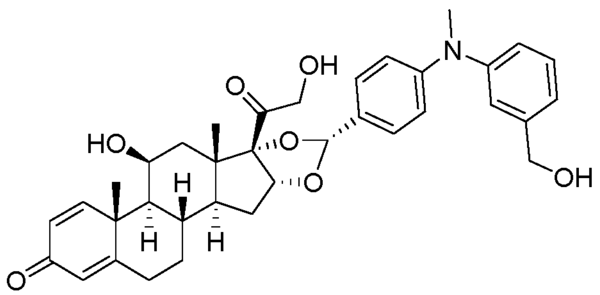 |
| I-78 | 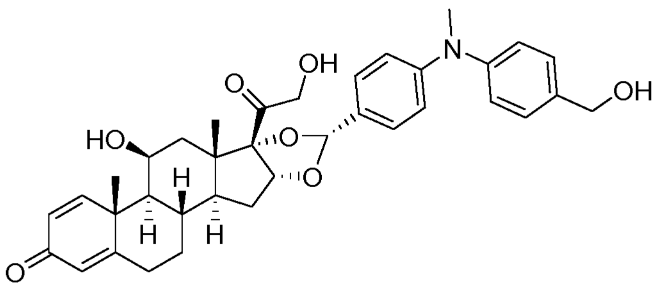 |

(continued)

| | |
|---|---|
| I-79 | 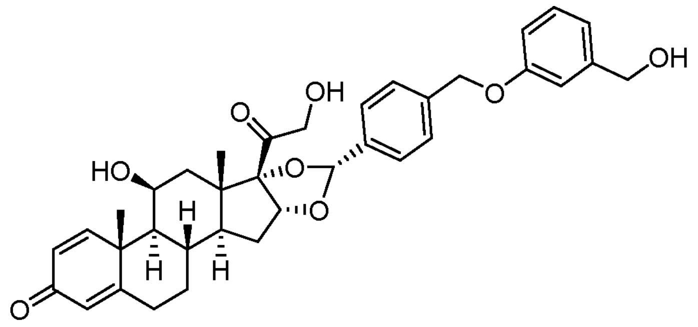 |
| I-80 | 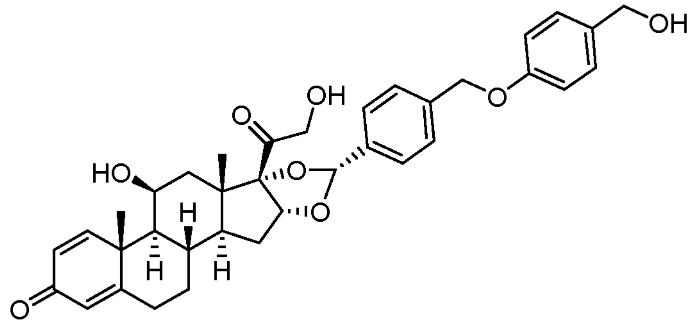 |
| I-81 | 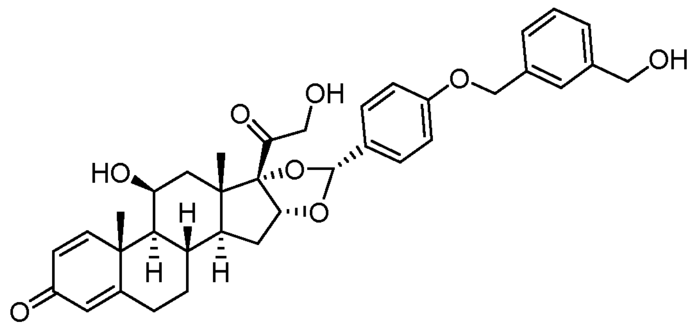 |
| I-82 | 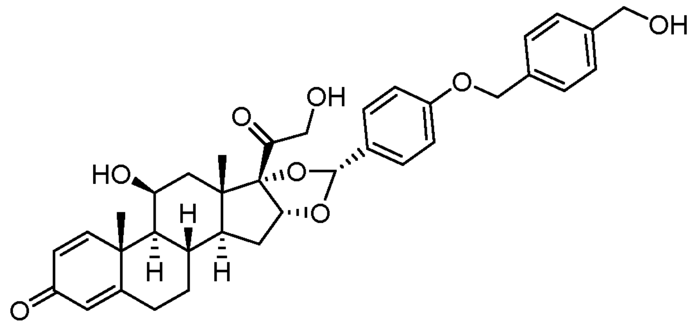 |
| I-83 | 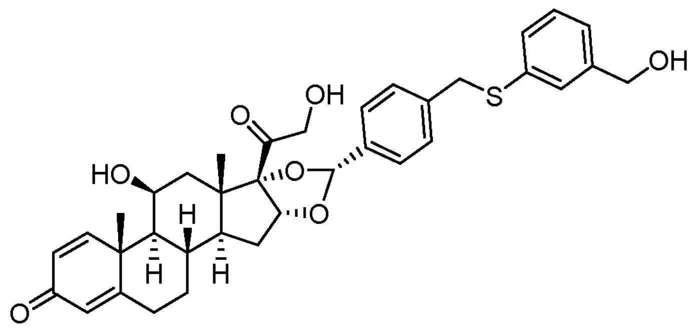 |

(continued)

| | |
|---|---|
| I-84 | 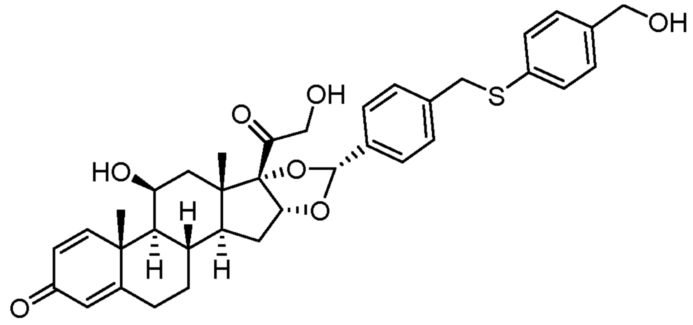 |
| I-85 | 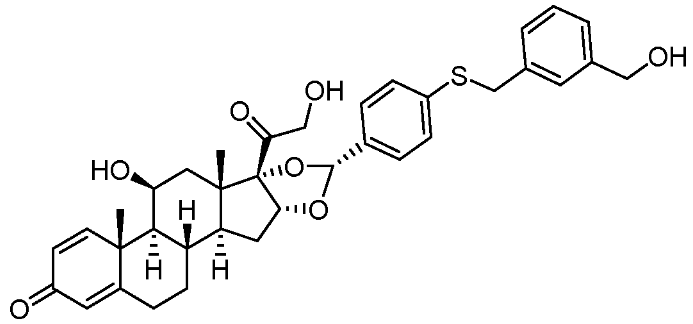 |
| I-86 | 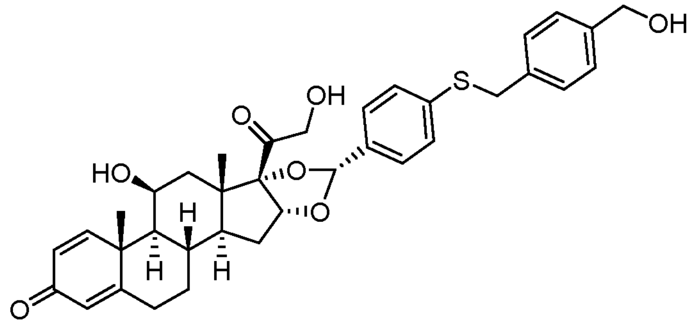 |
| I-87 | 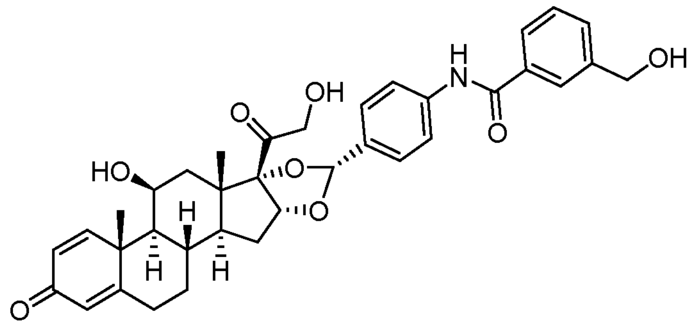 |
| I-88 | 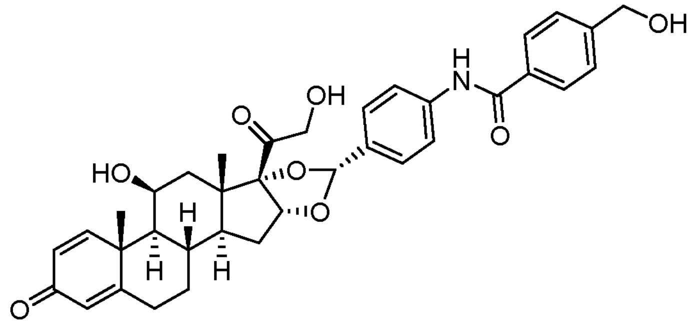 |

(continued)

| | |
|---|---|
| I-89 | 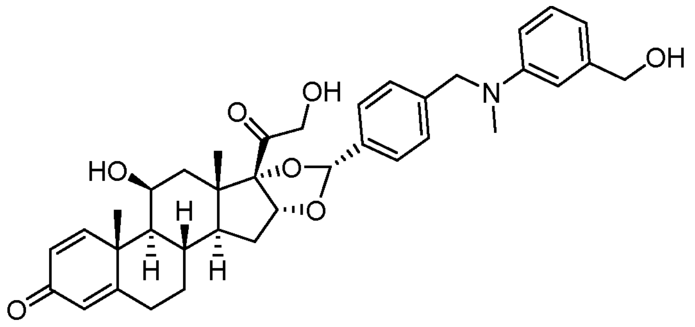 |
| I-90 | 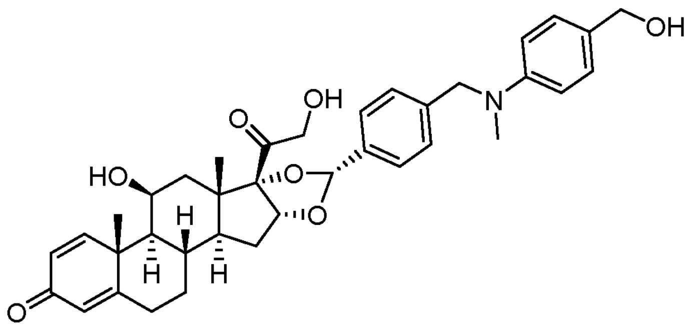 |
| I-91 | 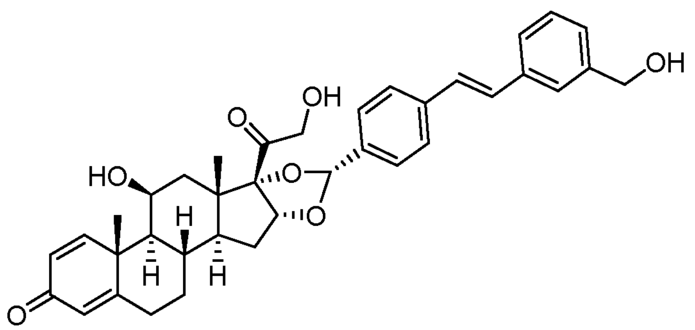 |
| I-92 | 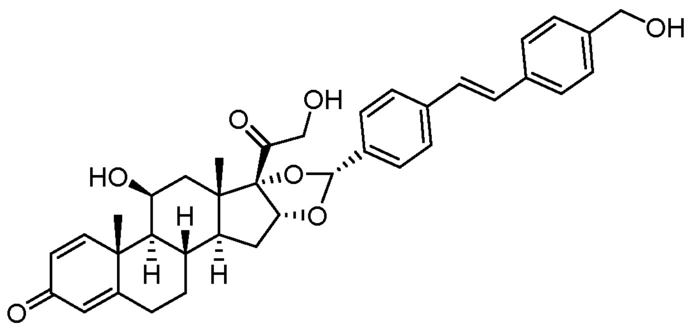 |
| I-93 | 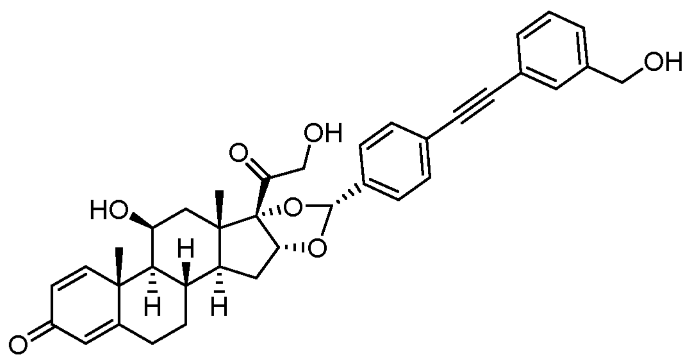 |

(continued)

| | |
|---|---|
| I-94 | 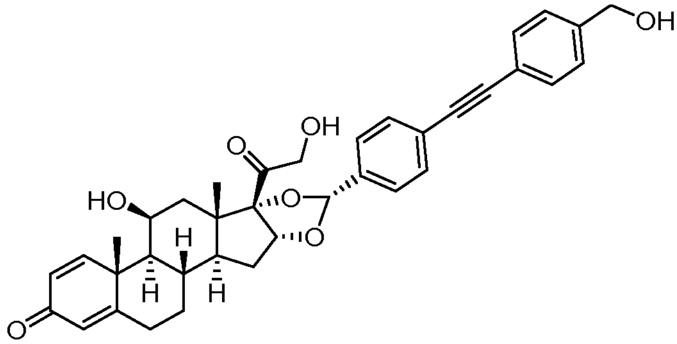 |
| I-95 | 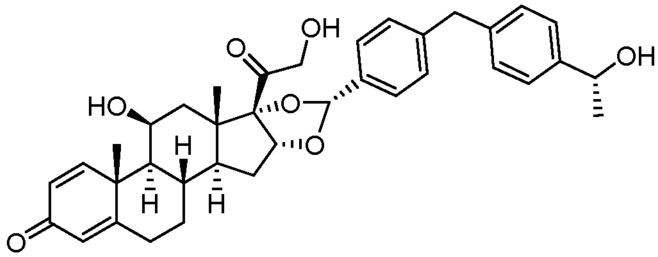 |
| I-96 | 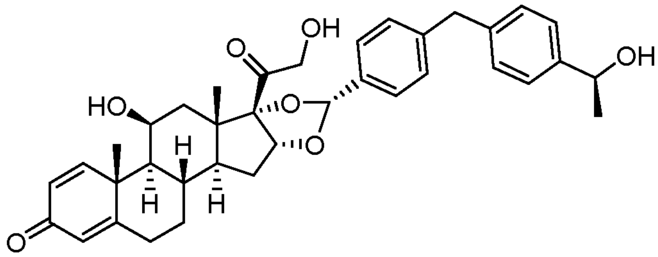 |
| I-97 | 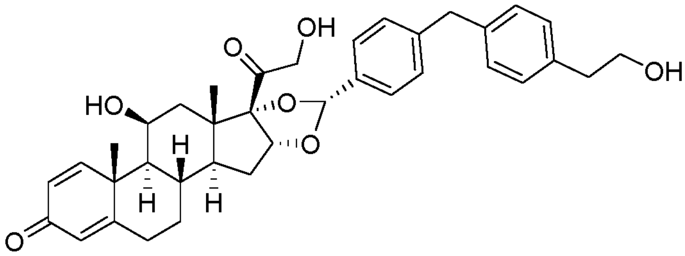 |
| I-98 | 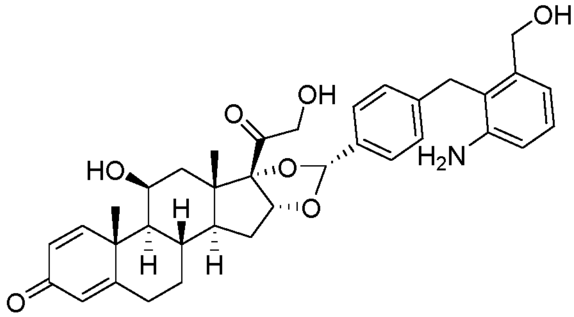 |

(continued)

| | |
|---|---|
| I-99 | 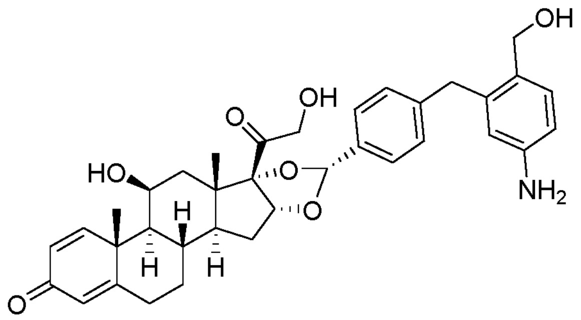 |
| I-100 | 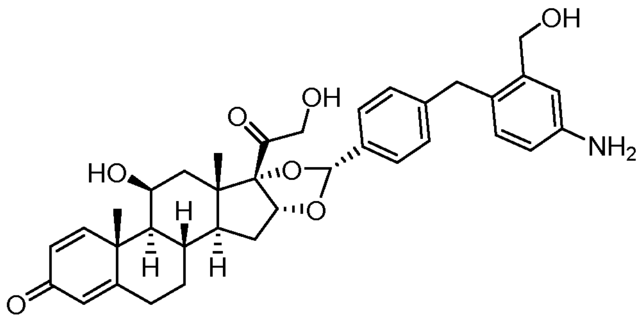 |
| I-101 | 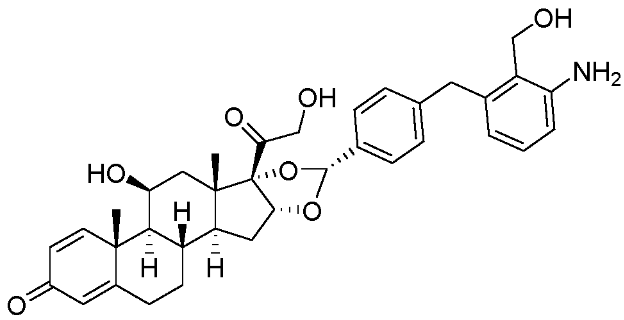 |
| I-102 | 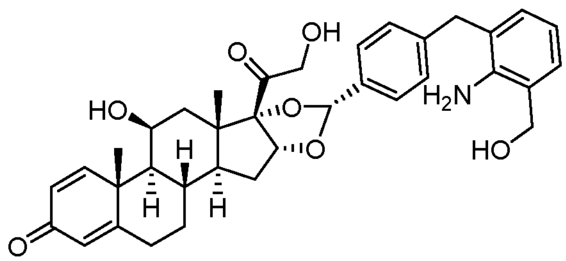 |
| I-103 | 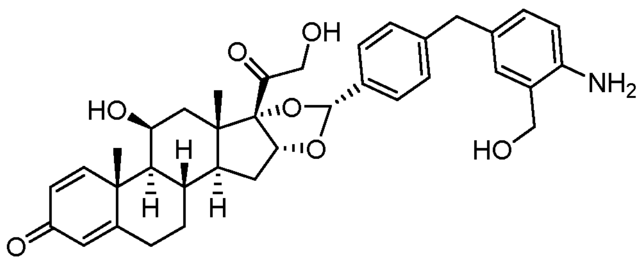 |

(continued)

| | |
|---|---|
| I-104 | 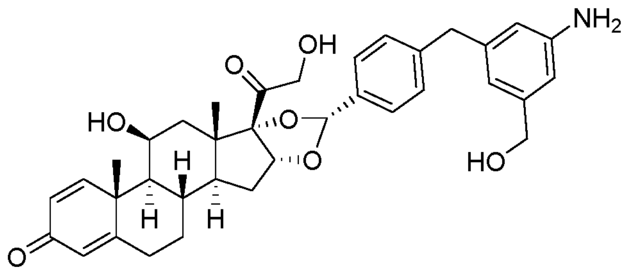 |
| I-105 | 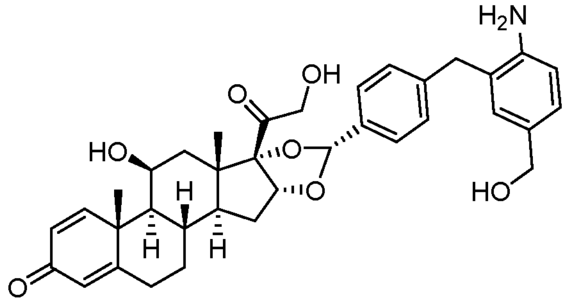 |
| I-106 | 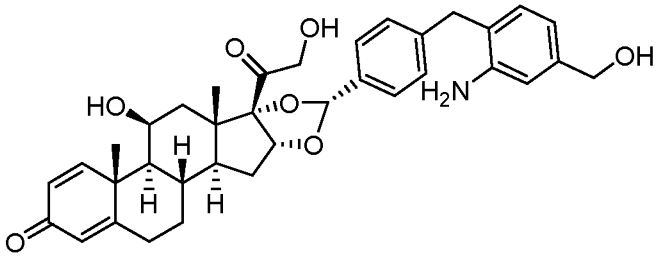 |
| I-107 | 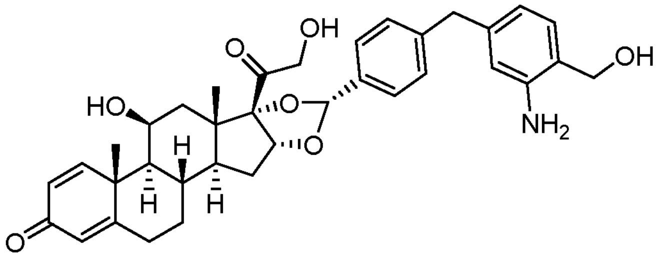 |
| I-108 | 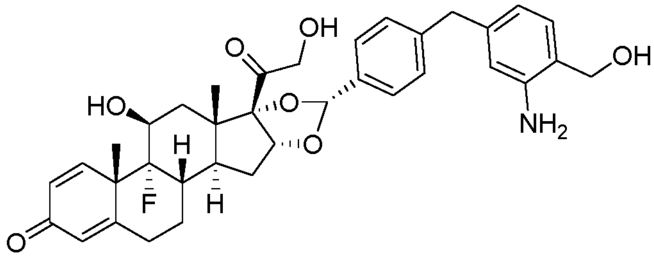 |

(continued)

| | |
|---|---|
| I-109 | 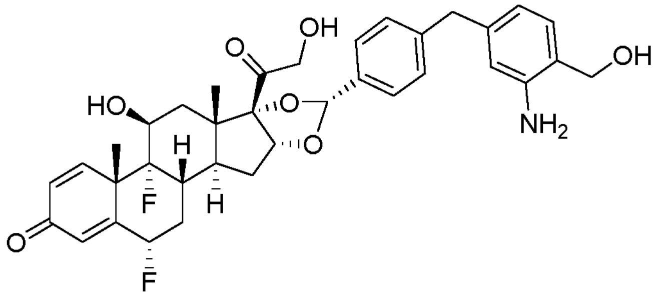 |
| I-110 | 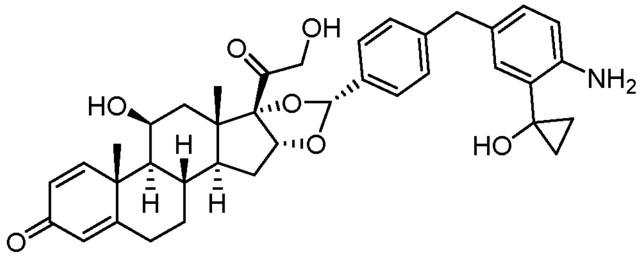 |
| I-111 | 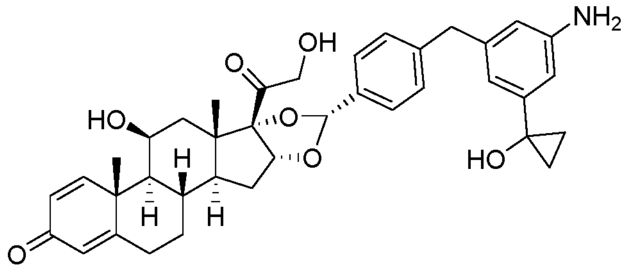 |
| I-112 | 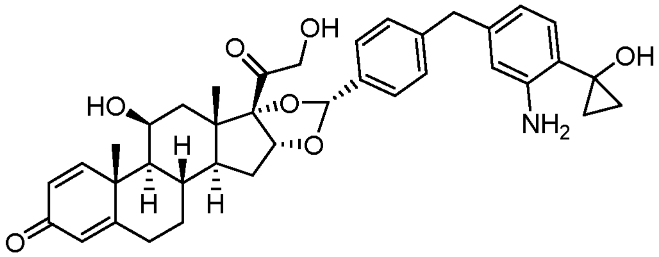 |
| I-113 | 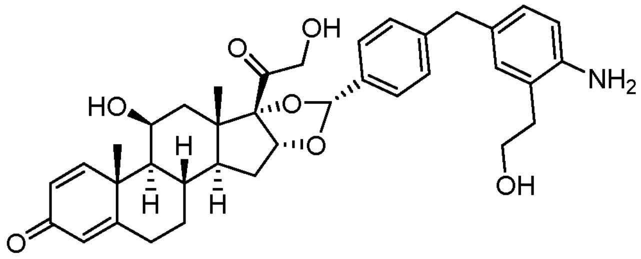 |
| I-114 | 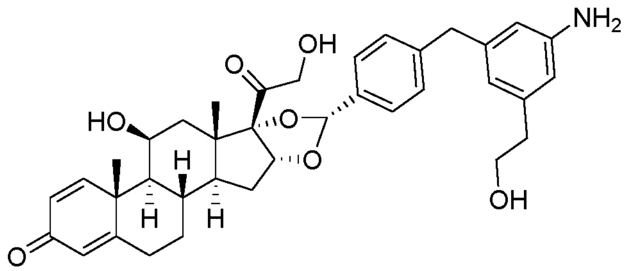 |

(continued)

| | |
|---|---|
| I-115 | 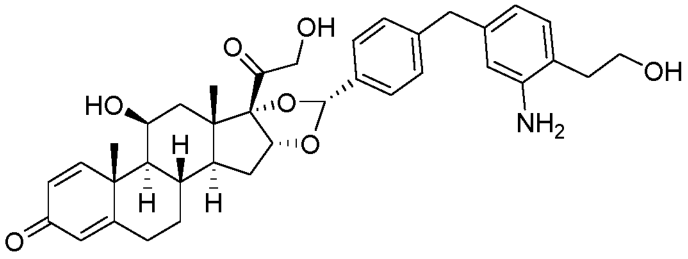 |
| I-116 | 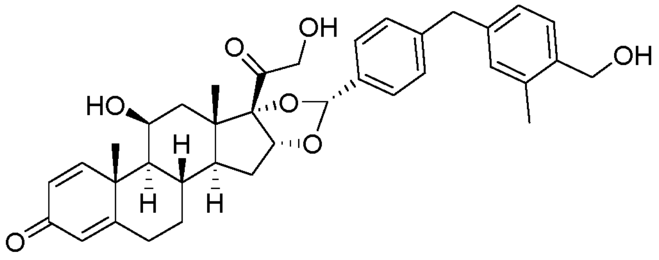 |
| I-117 | 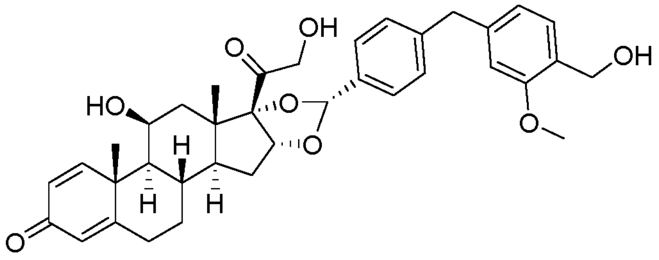 |
| I-118 | 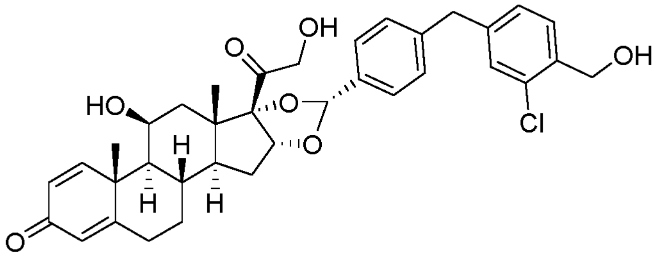 |
| I-119 | 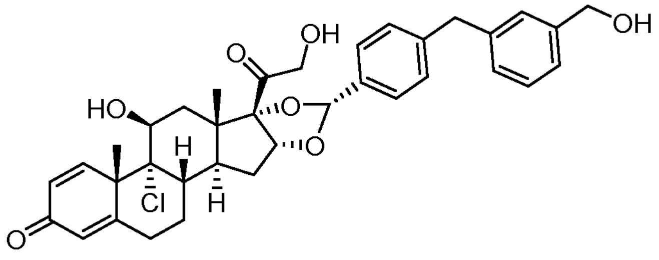 |
| I-120 | 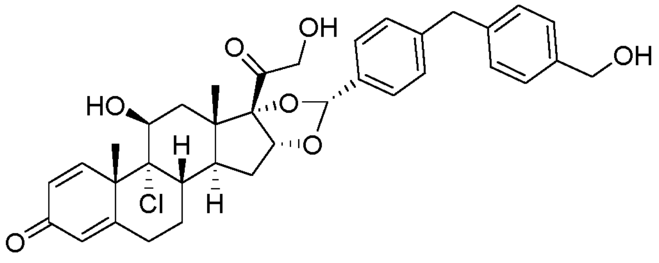 |

(continued)

| | |
|---|---|
| I-121 | 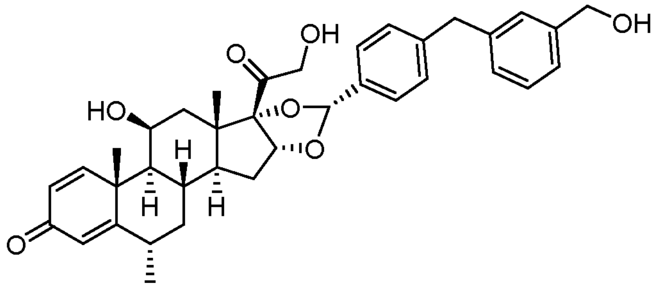 |
| I-122 | 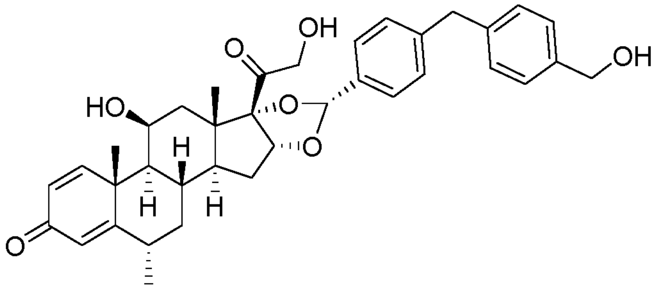 |
| I-123 | 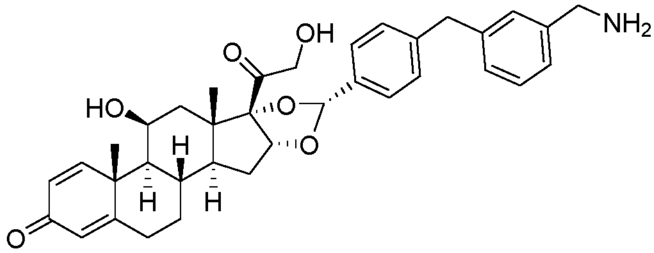 |
| I-124 | 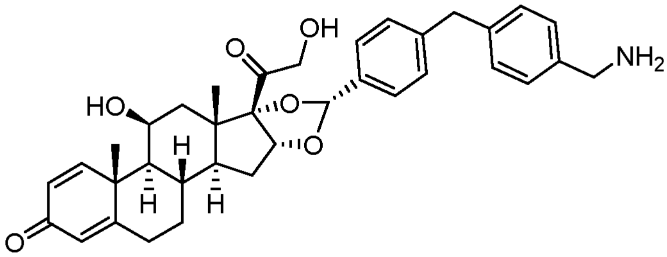 |
| I-125 | 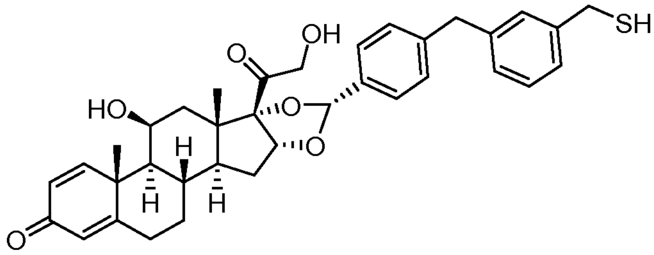 |
| I-126 | 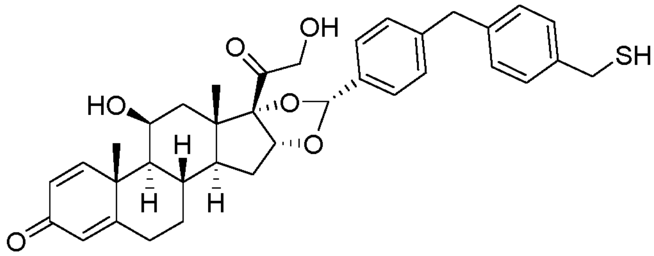 |

(continued)

| | |
|---|---|
| I-127 | 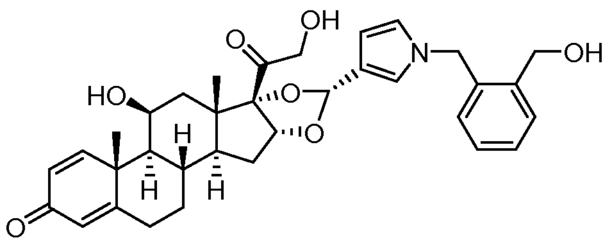 |
| I-128 | 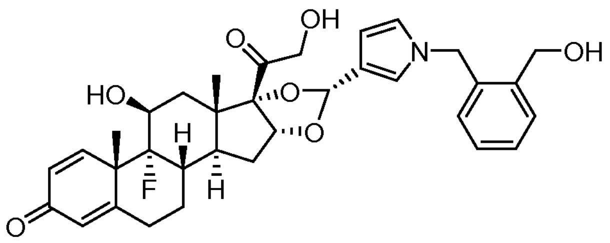 |
| I-129 | 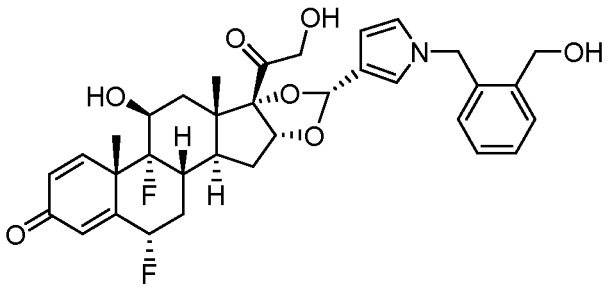 |
| I-130 | 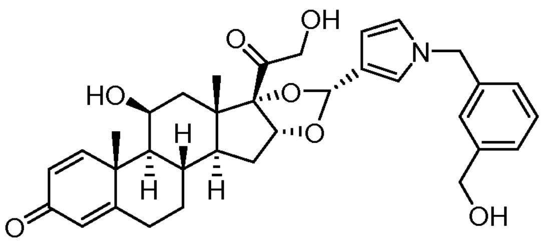 |
| I-131 | 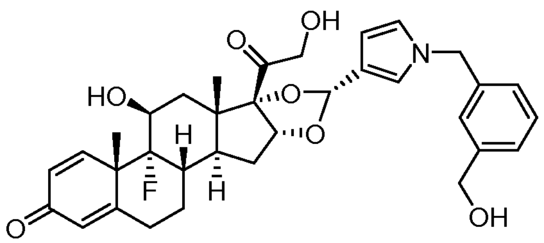 |
| I-132 | 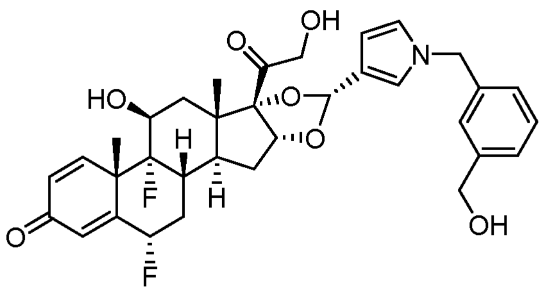 |

(continued)

| | |
|---|---|
| I-133 | 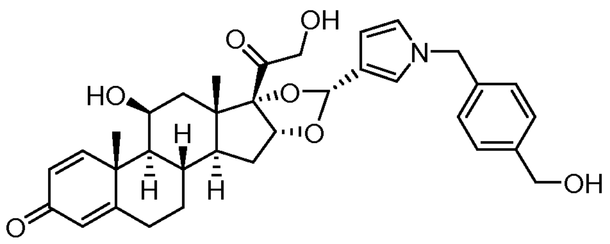 |
| I-134 | 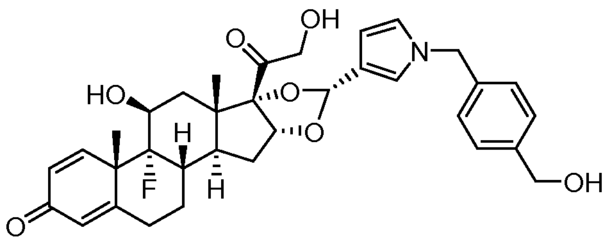 |
| I-135 | 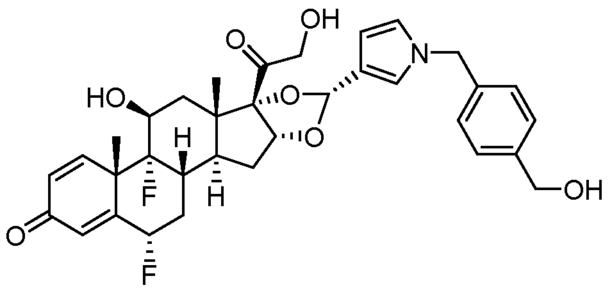 |
| I-136 | 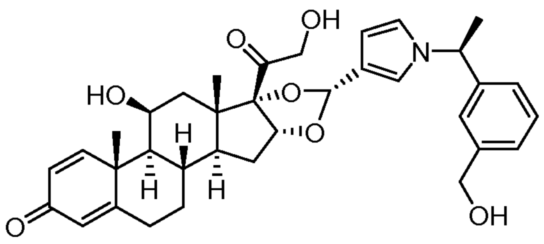 |
| I-137 | 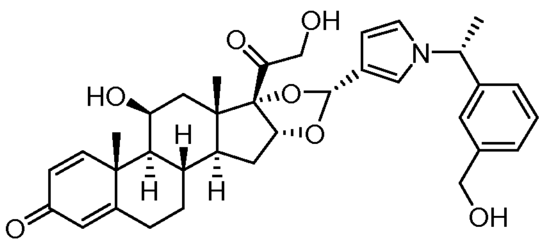 |
| I-138 | 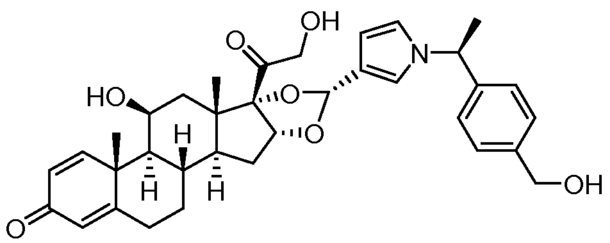 |

(continued)

| | |
|---|---|
| I-139 | 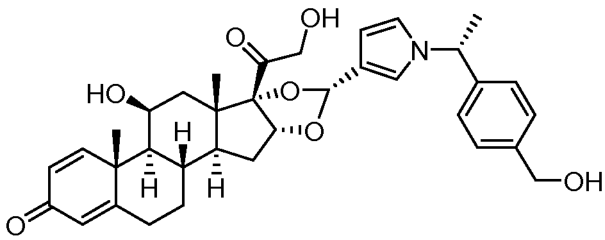 |
| I-140 | 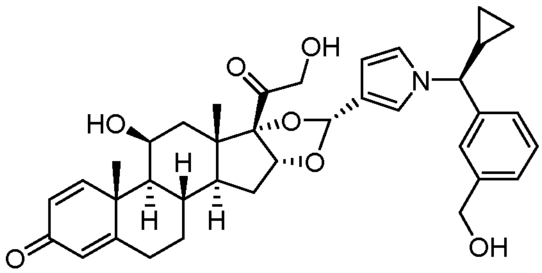 |
| I-141 | 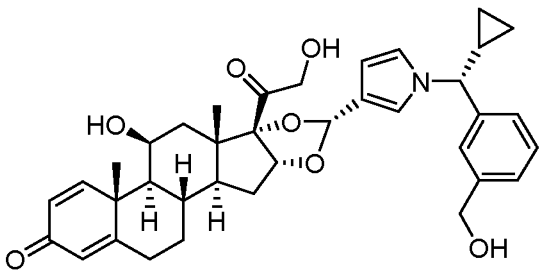 |
| I-142 | 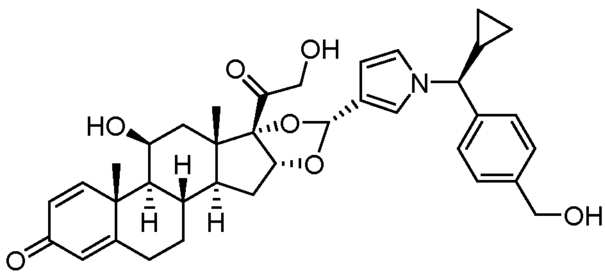 |
| I-143 | 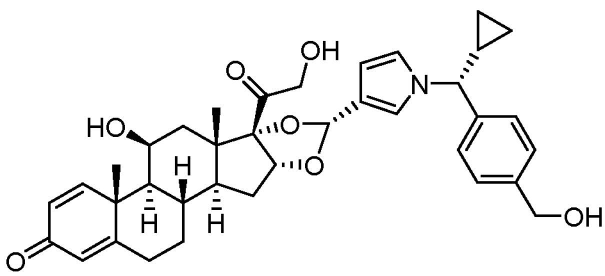 |

(continued)

| | |
|---|---|
| I-144 | 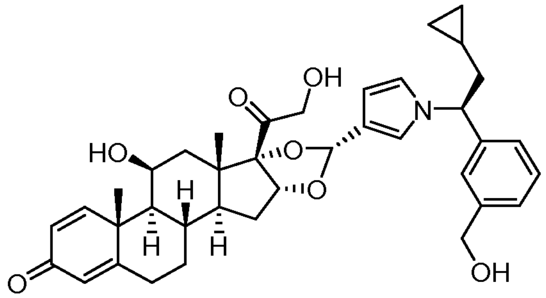 |
| I-145 | 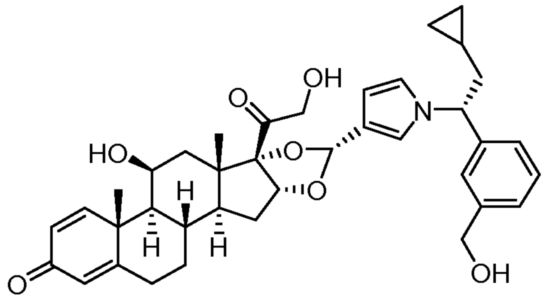 |
| I-146 | 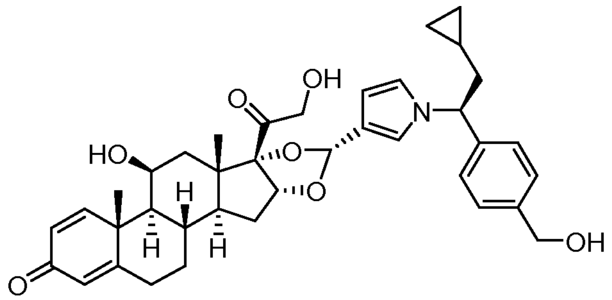 |
| I-147 | 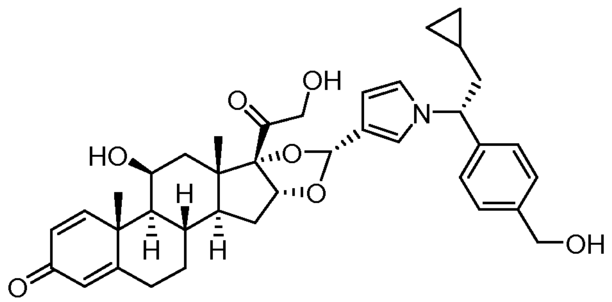 |
| I-148 | 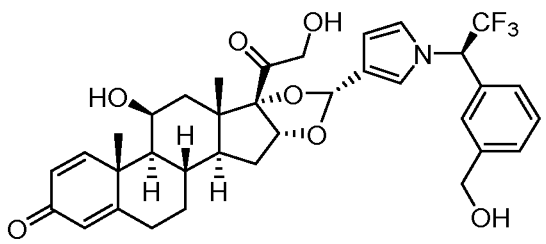 |

(continued)

| | |
|---|---|
| I-149 | 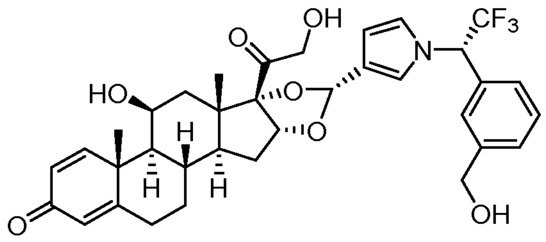 |
| I-150 | 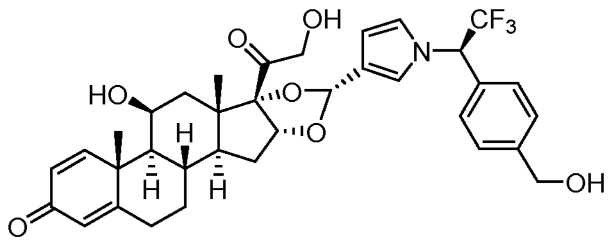 |
| I-151 | 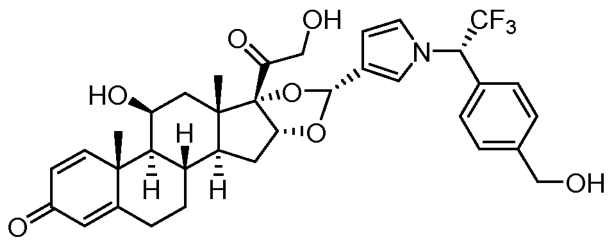 |
| I-152 | 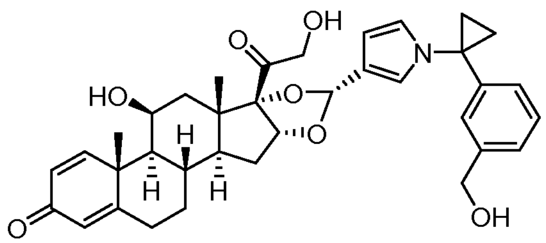 |
| I-153 | 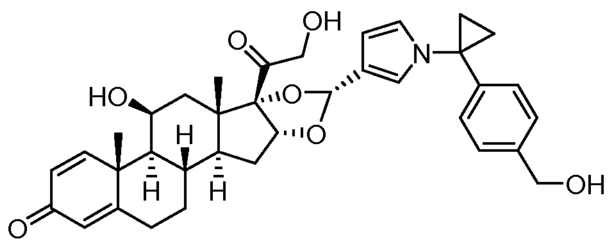 |
| I-154 | 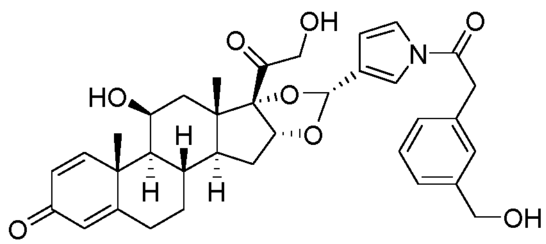 |

(continued)

| | |
|---|---|
| I-155 | 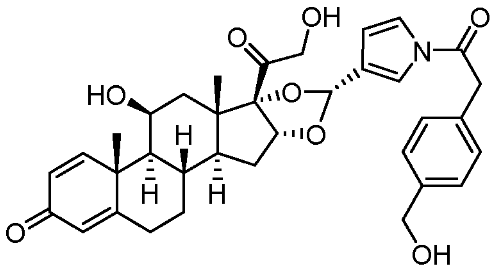 |
| I-156 | 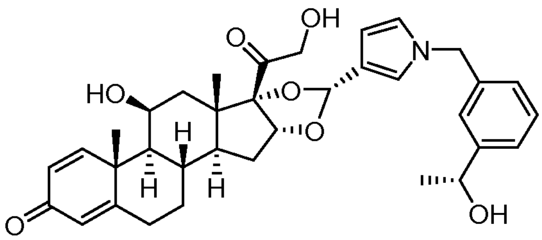 |
| I-157 | 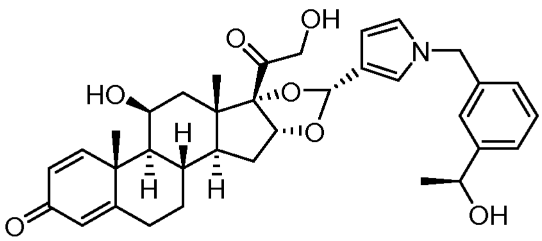 |
| I-158 | 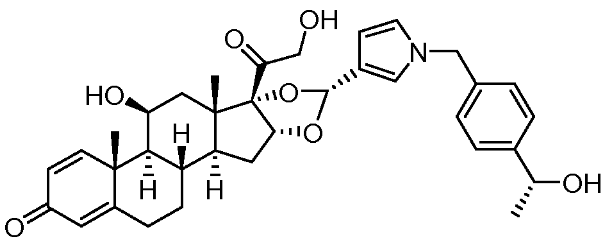 |
| I-159 | 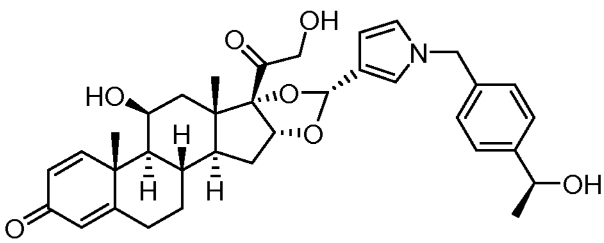 |
| I-160 | 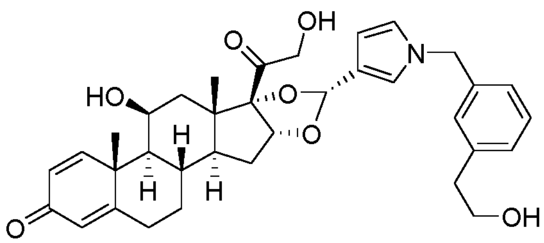 |

(continued)

| | |
|---|---|
| I-161 | 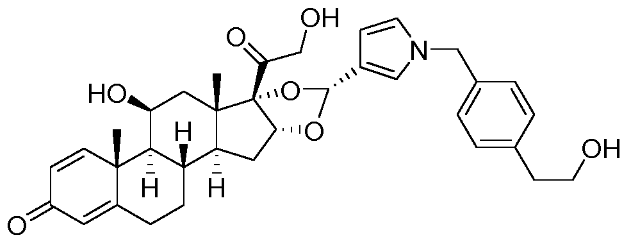 |
| I-162 | 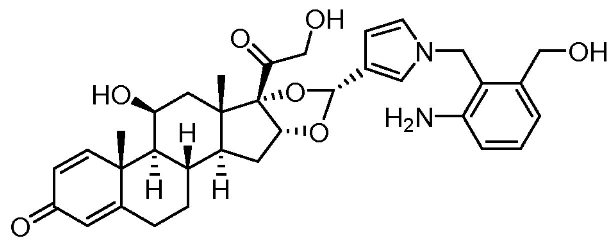 |
| I-163 | 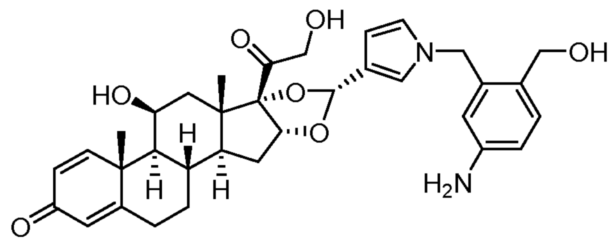 |
| I-164 | 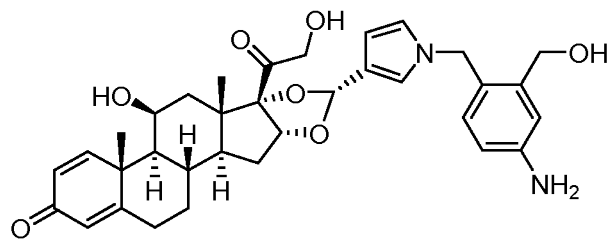 |
| I-165 | 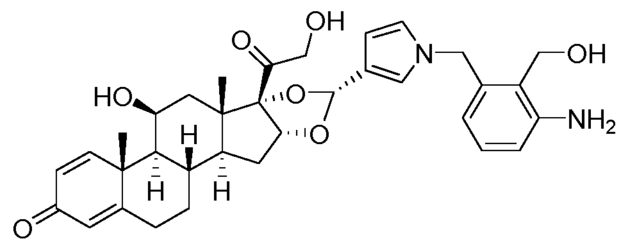 |
| I-166 | 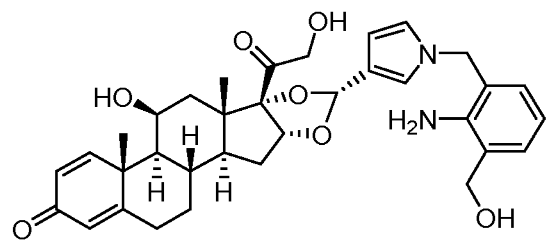 |

(continued)

| | |
|---|---|
| I-167 | 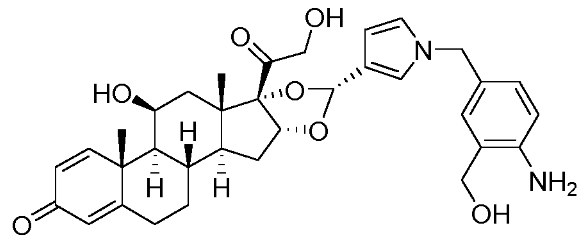 |
| I-168 | 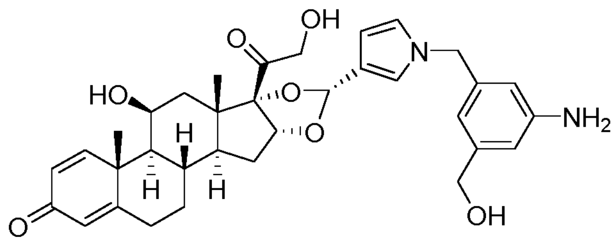 |
| I-169 | 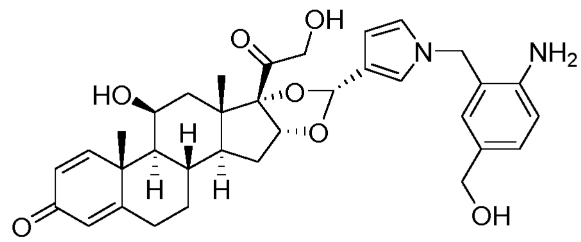 |
| I-170 | 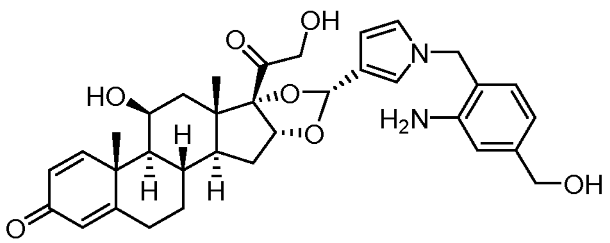 |
| I-171 | 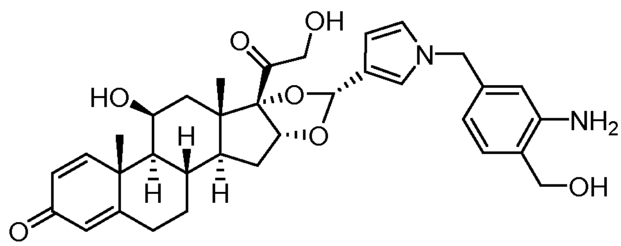 |
| I-172 | 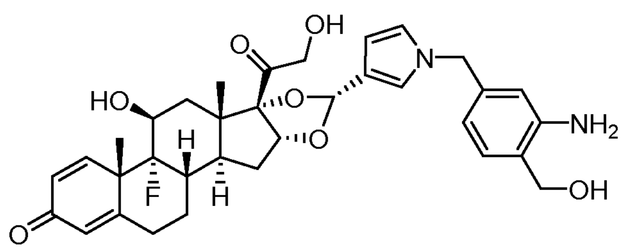 |

(continued)

| | |
|---|---|
| I-173 | 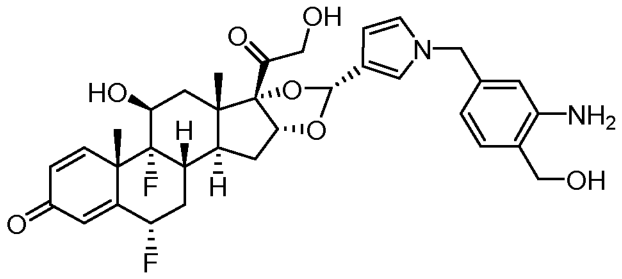 |
| I-174 | 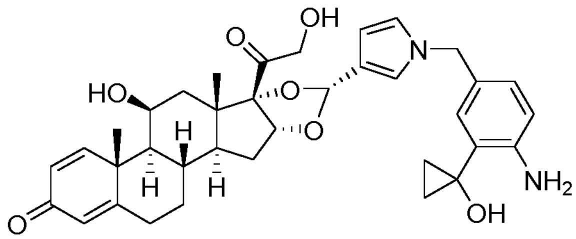 |
| I-175 | 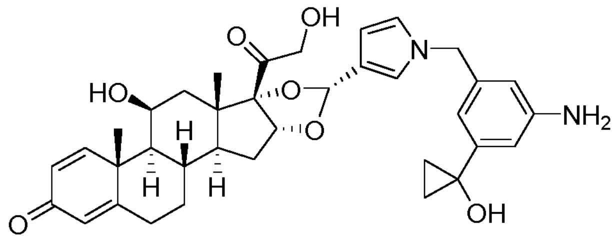 |
| I-176 | 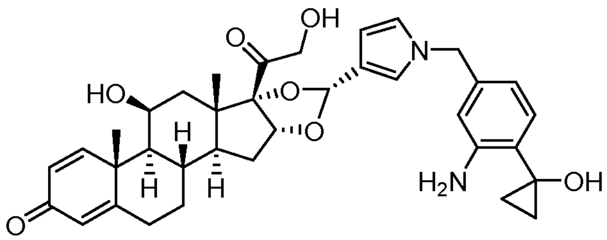 |
| I-177 | 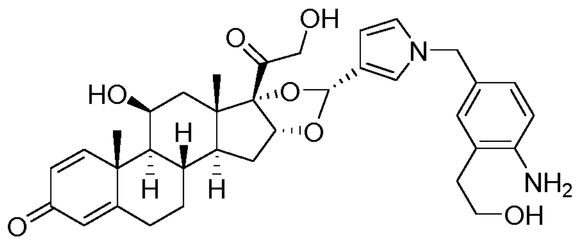 |
| I-178 | 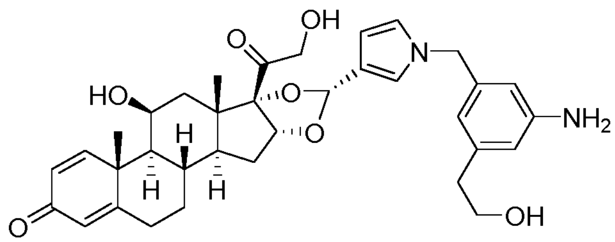 |

(continued)

| | |
|---|---|
| I-179 | 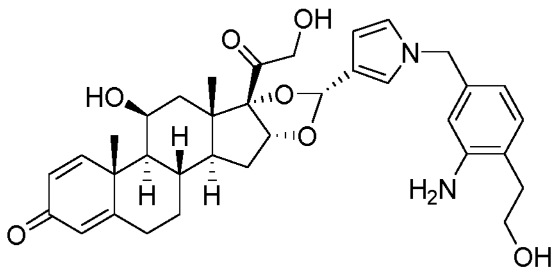 |
| I-180 | 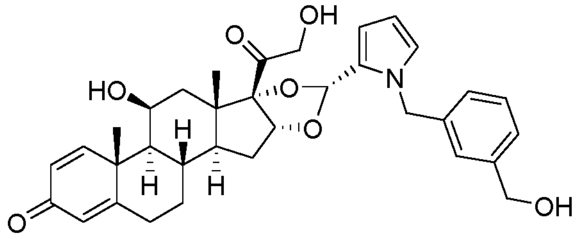 |
| I-181 | 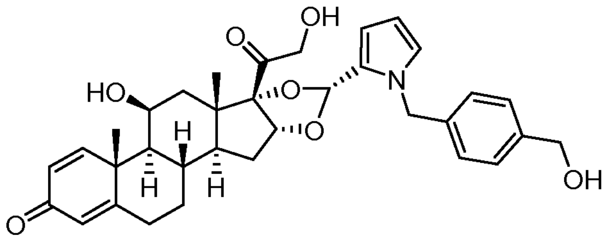 |
| I-182 | 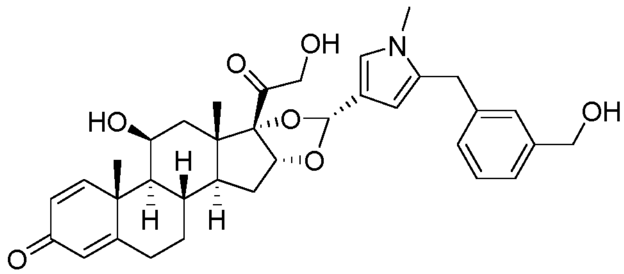 |
| I-183 | 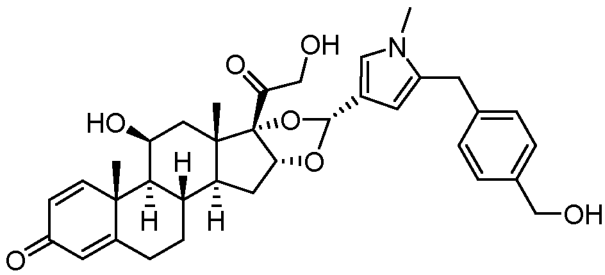 |
| I-184 | 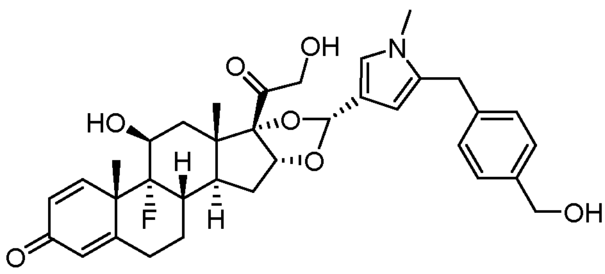 |

(continued)

| | |
|---|---|
| I-185 | 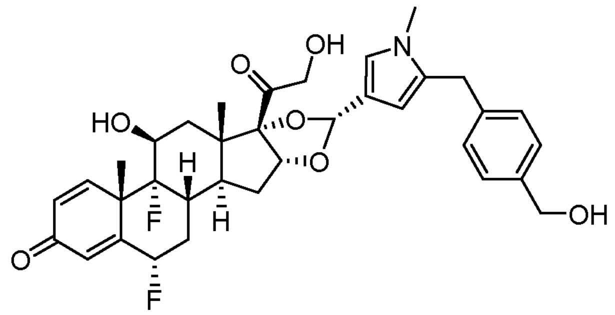 |
| I-186 | 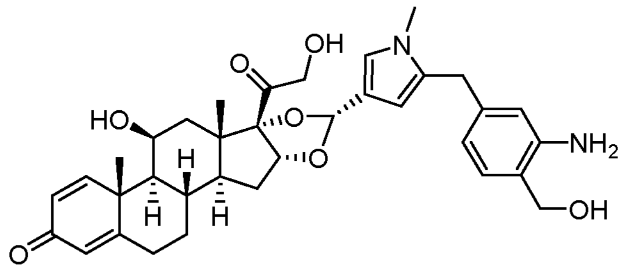 |
| I-187 | 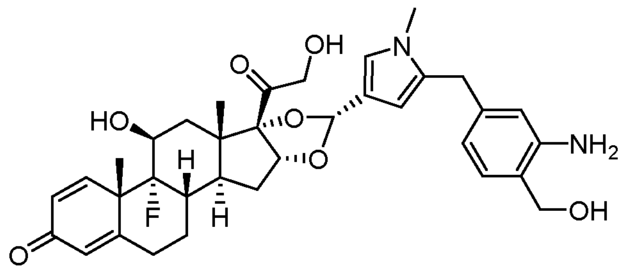 |
| I-188 | 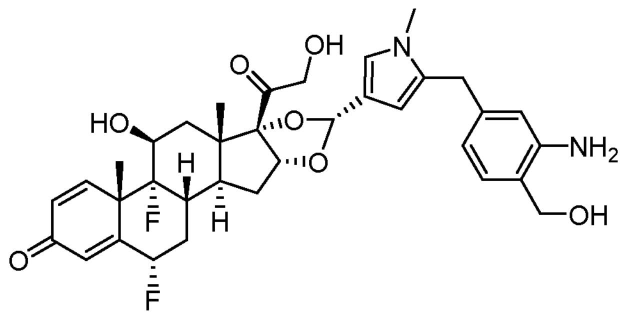 |
| I-189 | 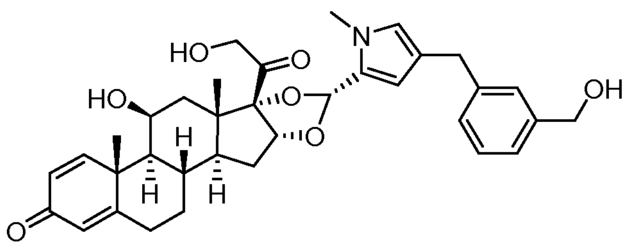 |

(continued)

| | |
|---|---|
| I-190 | 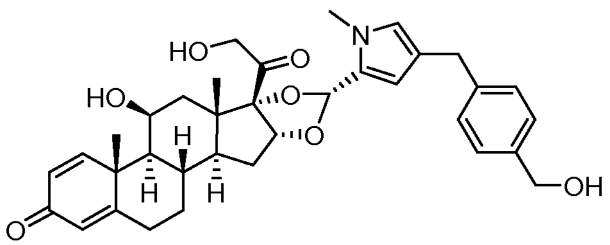 |
| I-191 | 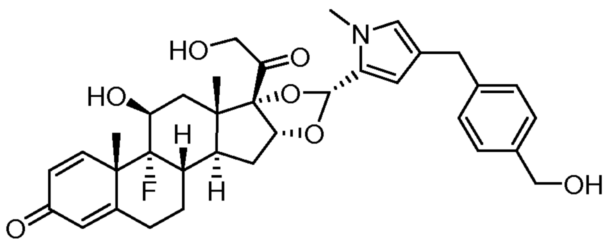 |
| I-192 | 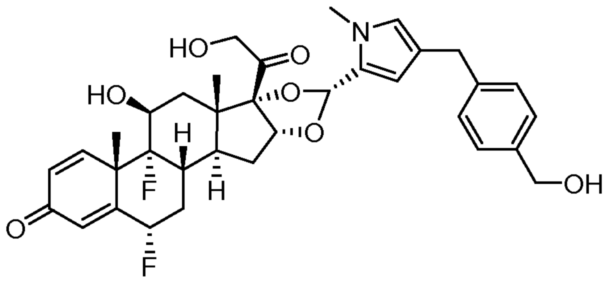 |
| I-193 | 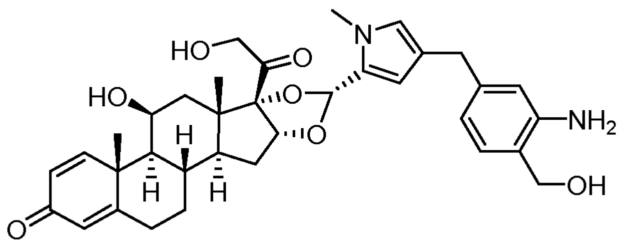 |
| I-194 | 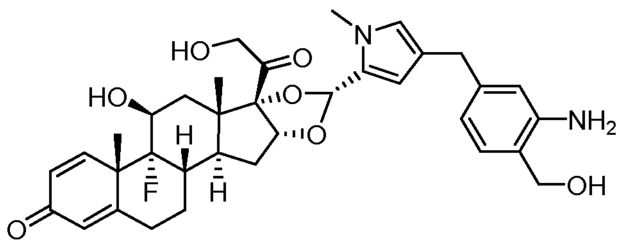 |
| I-195 | 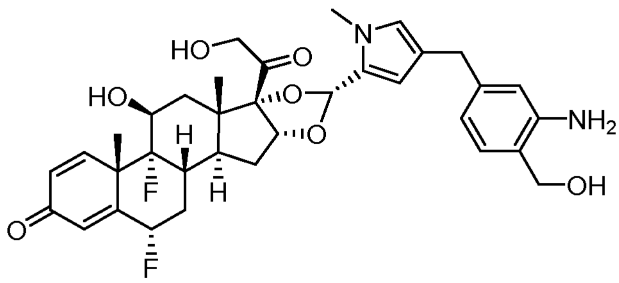 |

(continued)

| | |
|---|---|
| I-196 | 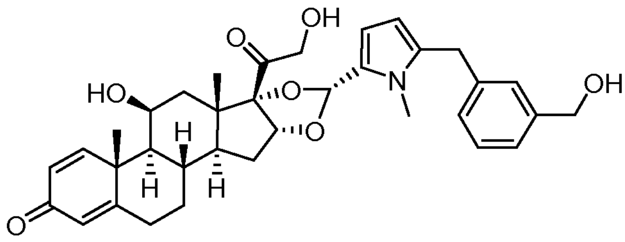 |
| I-197 | 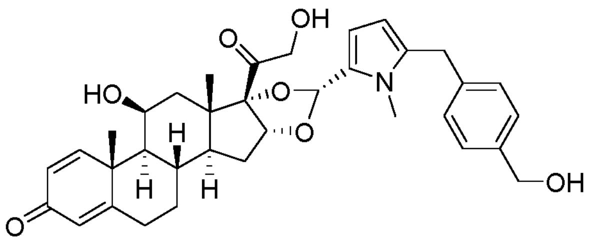 |
| I-198 | 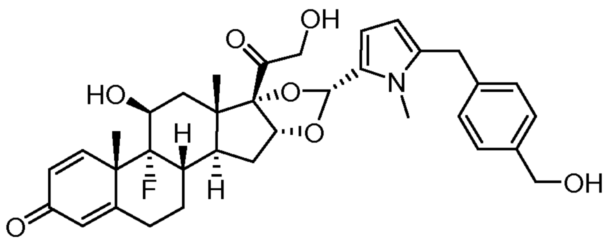 |
| I-199 | 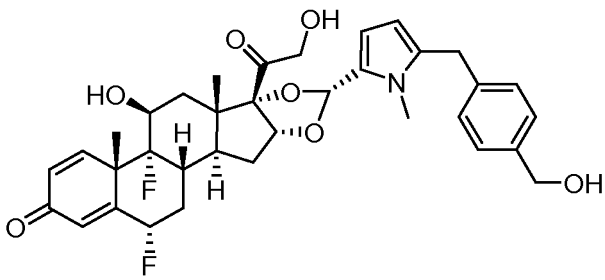 |
| I-200 | 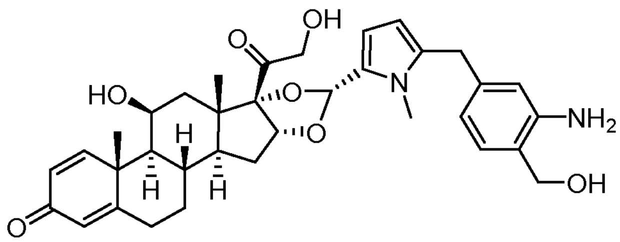 |
| I-201 | 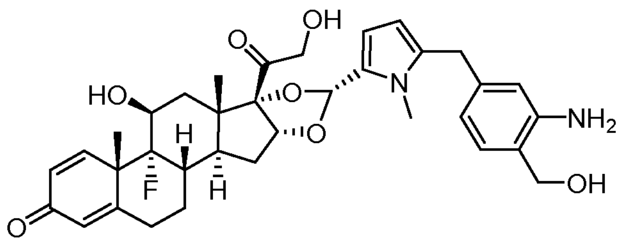 |

(continued)

| | |
|---|---|
| I-202 | 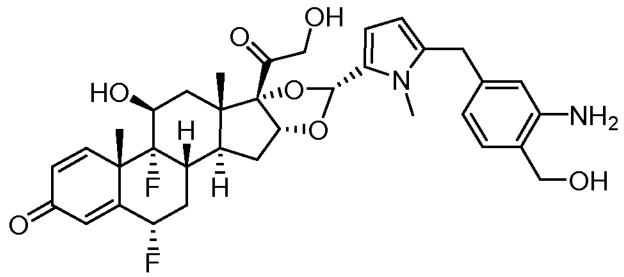 |
| I-203 | 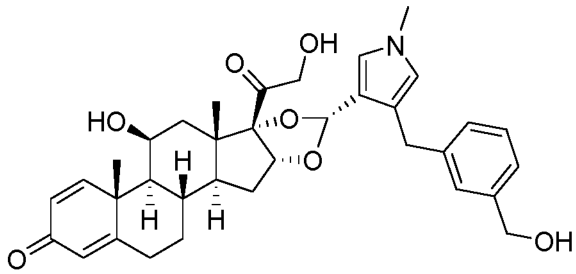 |
| I-204 | 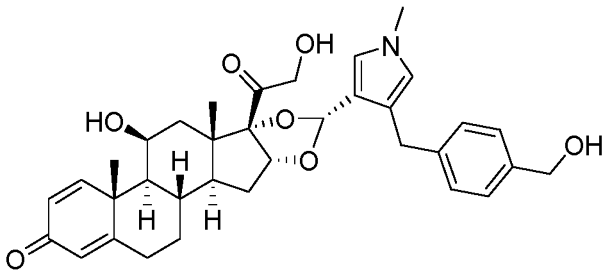 |
| I-205 | 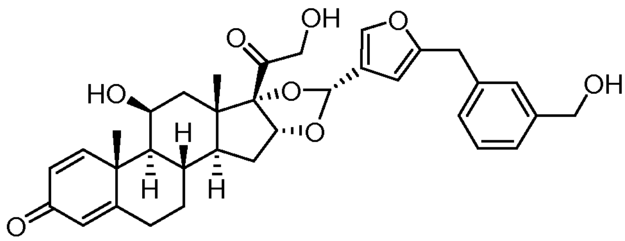 |
| I-206 | 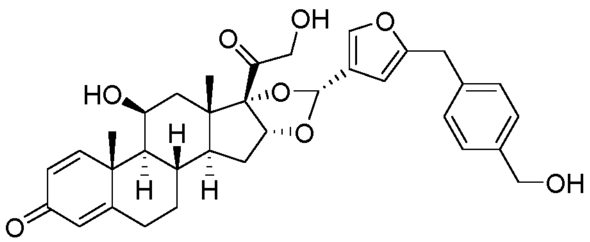 |
| I-207 | 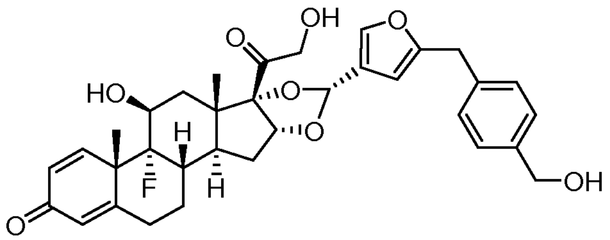 |

(continued)

| | |
|---|---|
| I-208 | 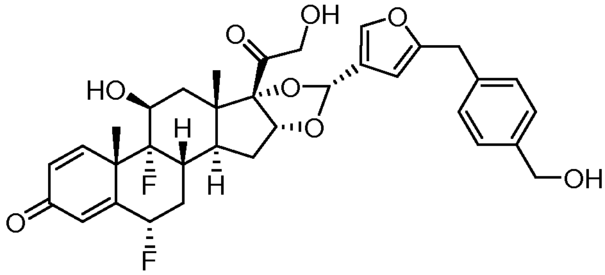 |
| I-209 | 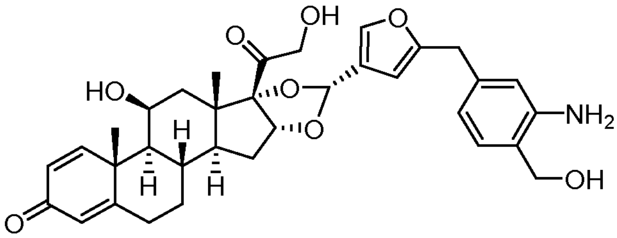 |
| I-210 | 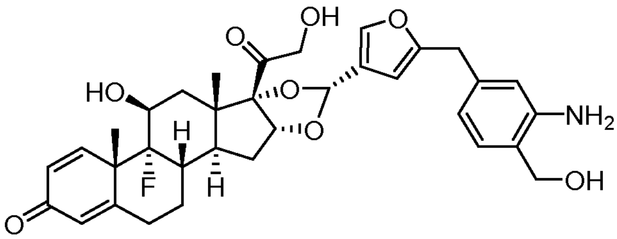 |
| I-211 | 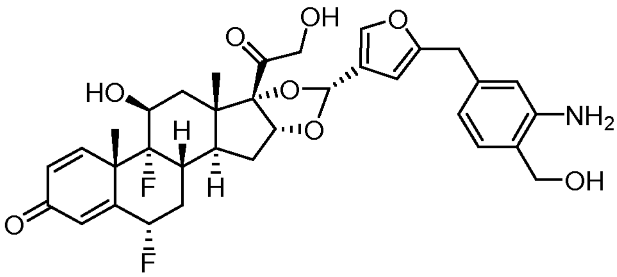 |
| I-212 | 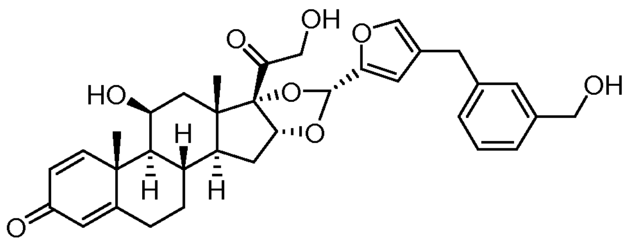 |
| I-213 | 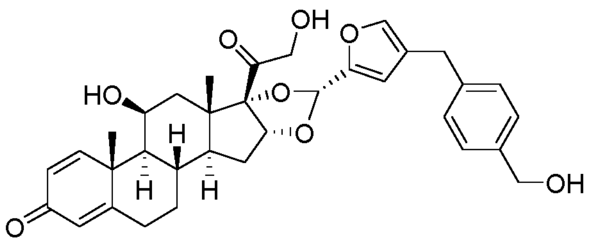 |

(continued)

| | |
|---|---|
| I-214 | 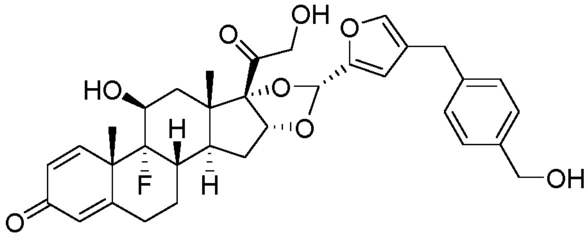 |
| I-215 | 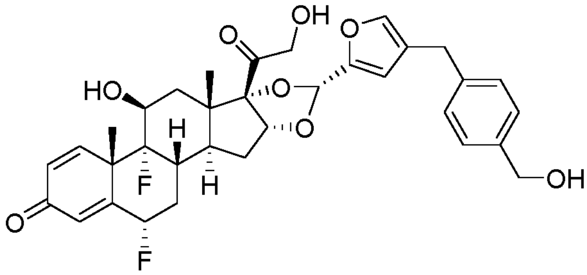 |
| I-216 | 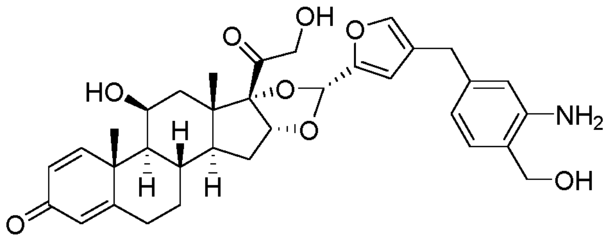 |
| I-217 | 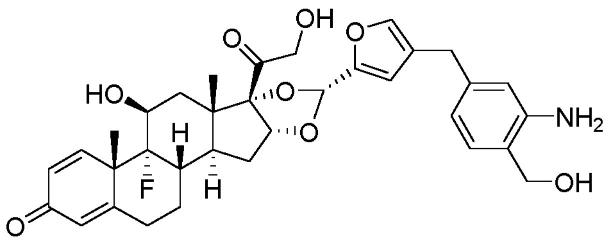 |
| I-218 | 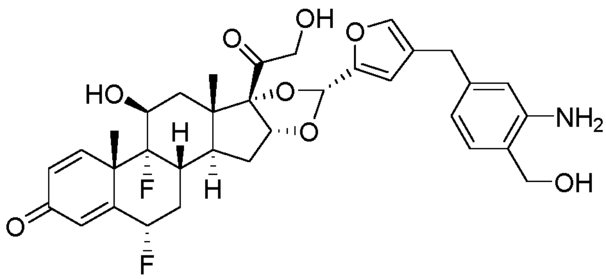 |
| I-219 | 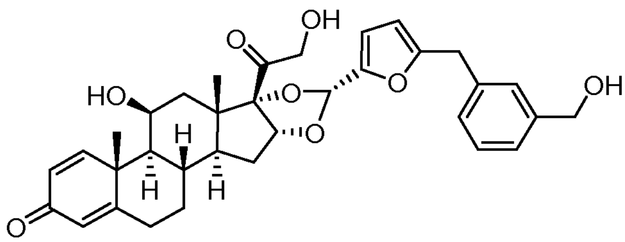 |

(continued)

| | |
|---|---|
| I-220 | 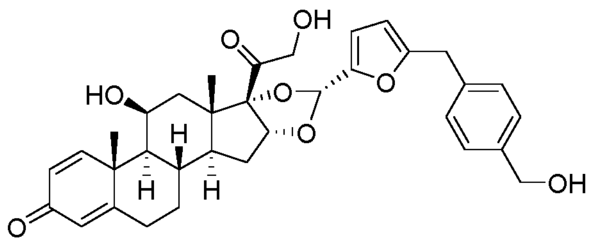 |
| I-221 | 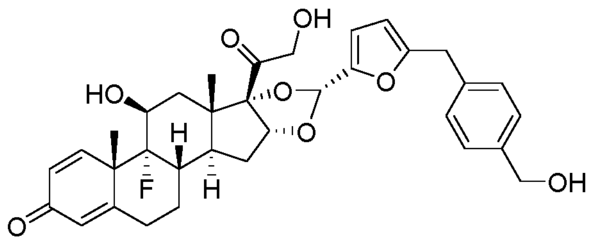 |
| I-222 | 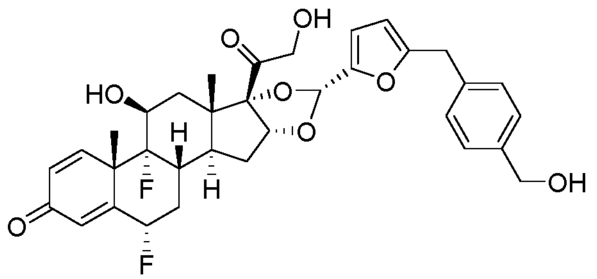 |
| I-223 | 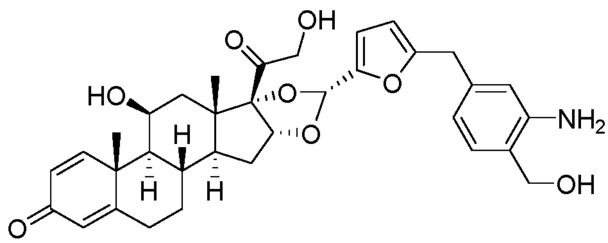 |
| I-224 | 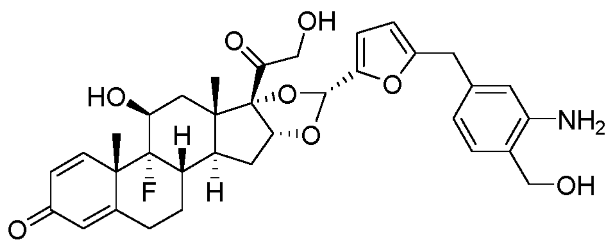 |
| I-225 | 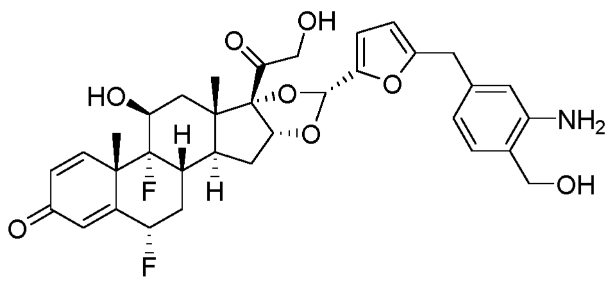 |

(continued)

| | |
|---|---|
| I-226 | 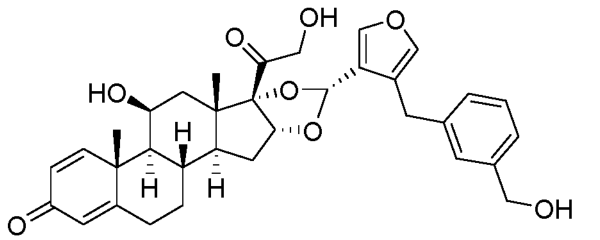 |
| I-227 | 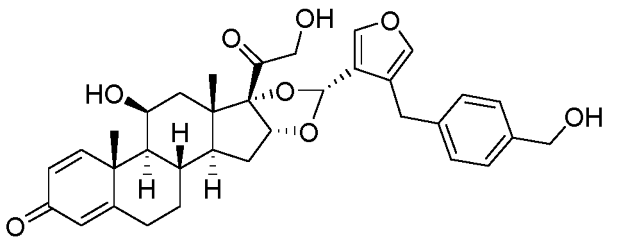 |
| I-228 | 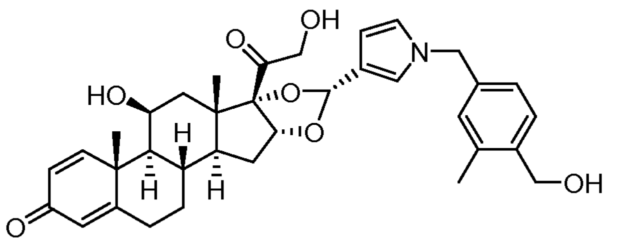 |
| I-229 | 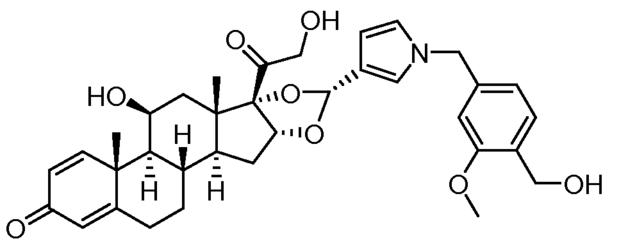 |
| I-230 | 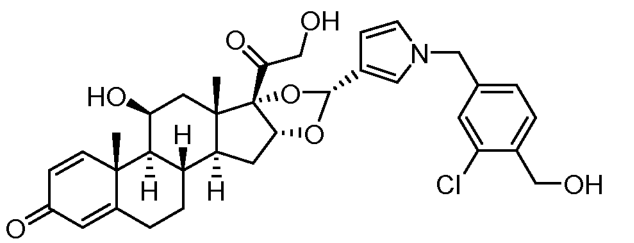 |
| I-231 | 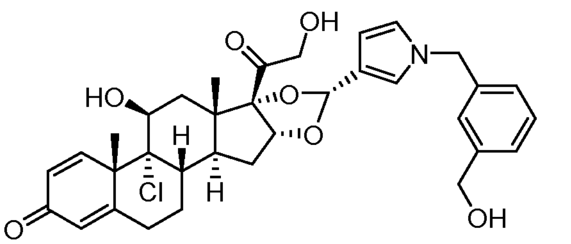 |

(continued)

| | |
|---|---|
| I-232 | 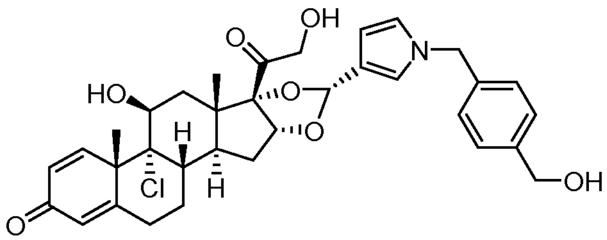 |
| I-233 | 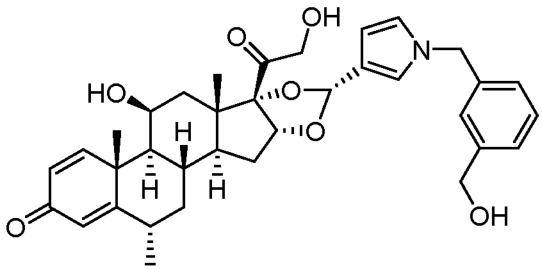 |
| I-234 | 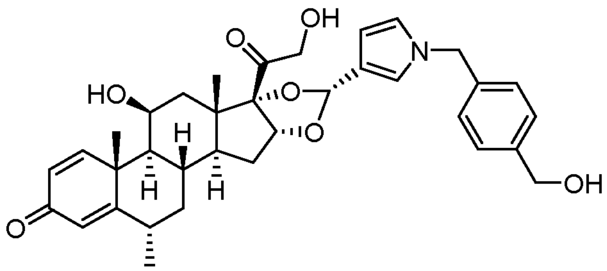 |
| I-235 | 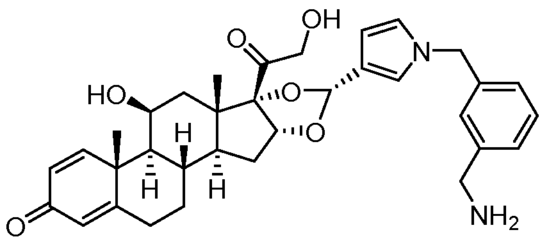 |
| I-236 | 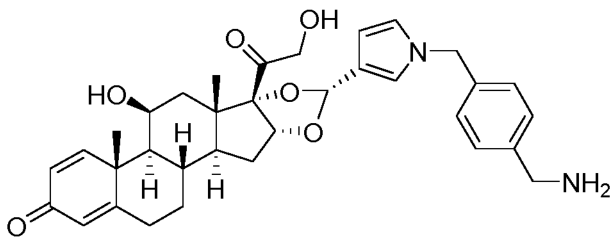 |
| I-237 | 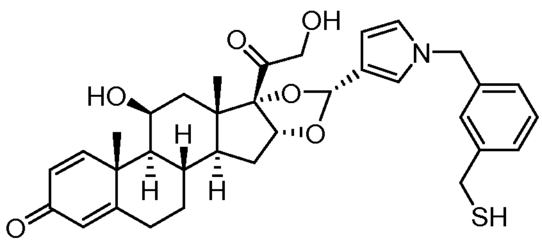 |

(continued)

| | |
|---|---|
| I-238 | 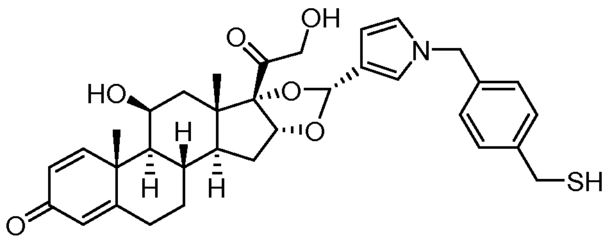 |
| I-239 | 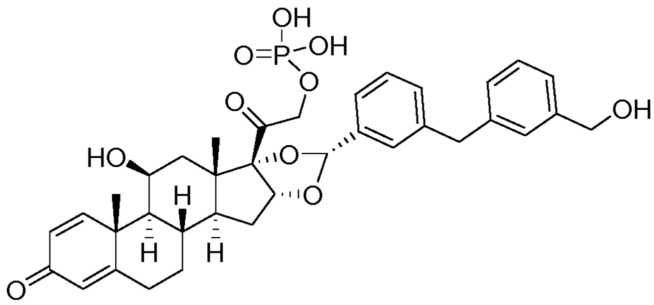 |
| I-240 | 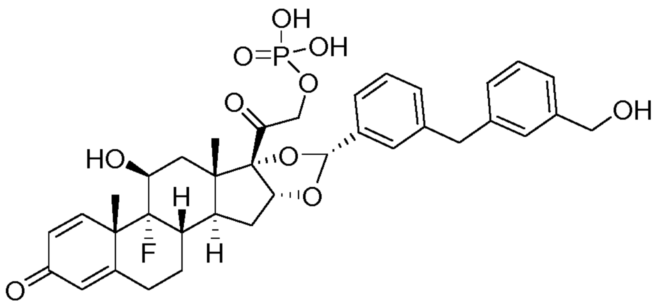 |
| I-241 | 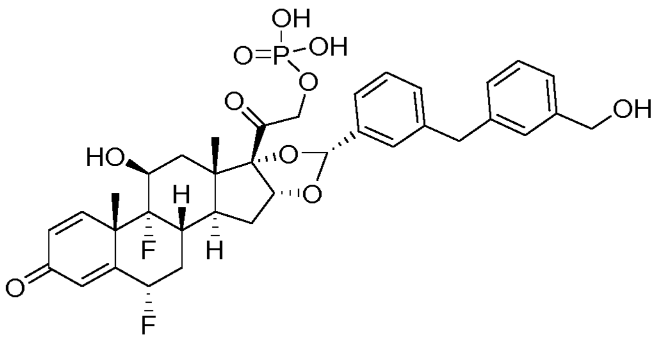 |
| I-242 | 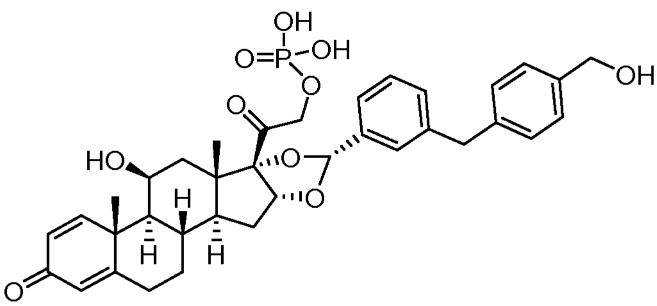 |

(continued)

| | |
|---|---|
| I-243 | 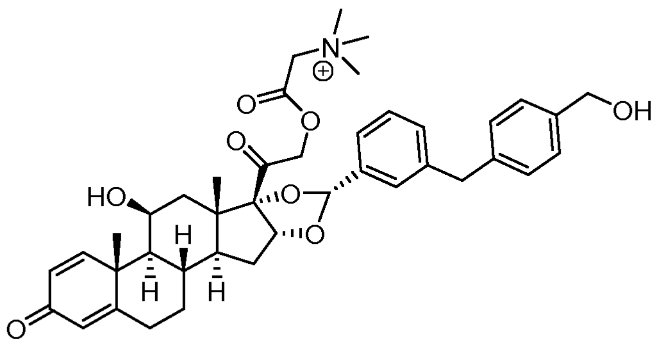 |
| I-244 | 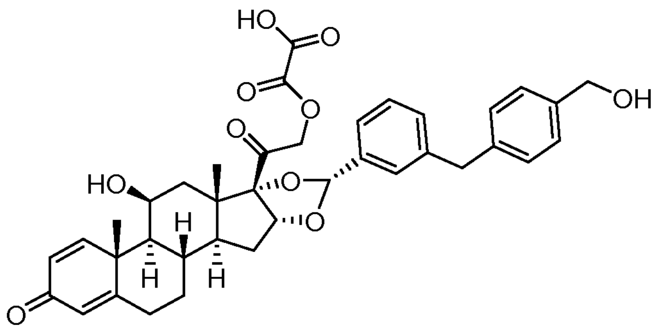 |
| I-245 | 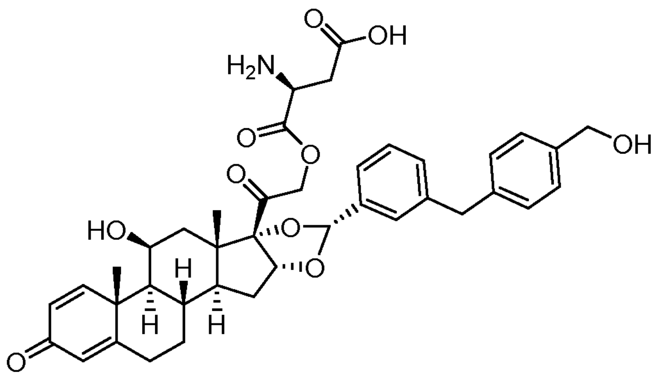 |
| I-246 | 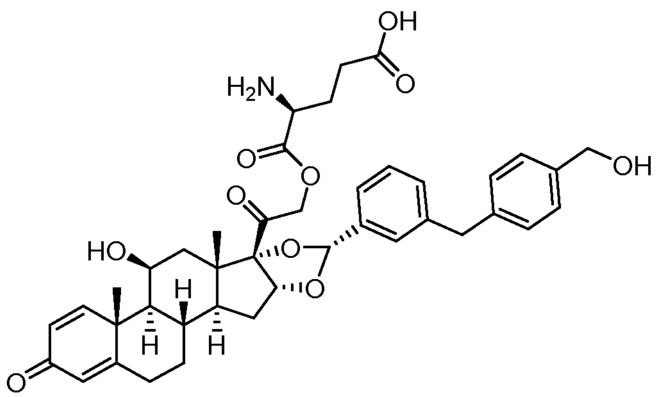 |

(continued)

| | |
|---|---|
| I-247 | 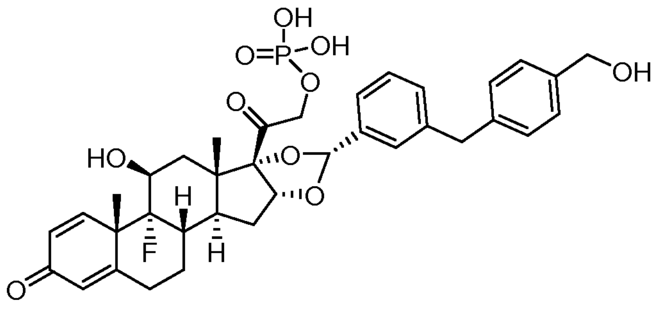 |
| I-248 | 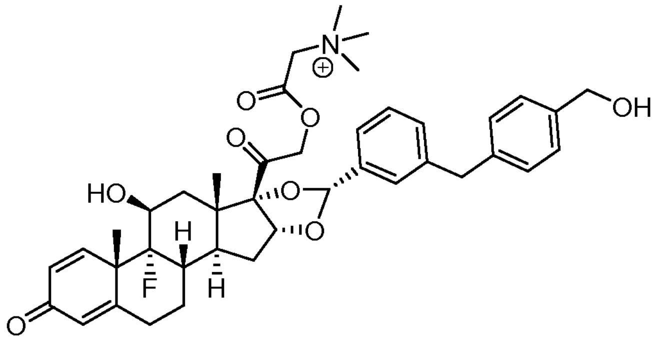 |
| I-249 | 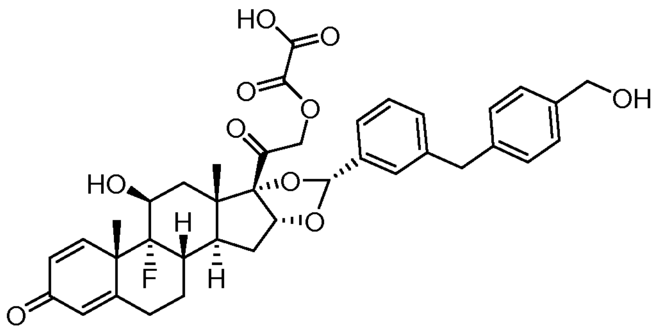 |
| I-250 | 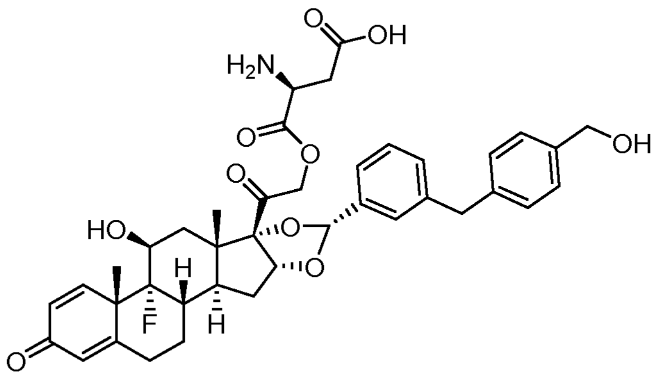 |

(continued)

| | |
|---|---|
| I-251 | 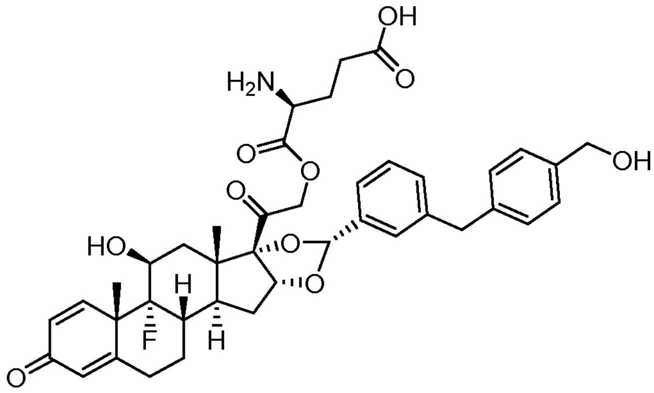 |
| I-252 | 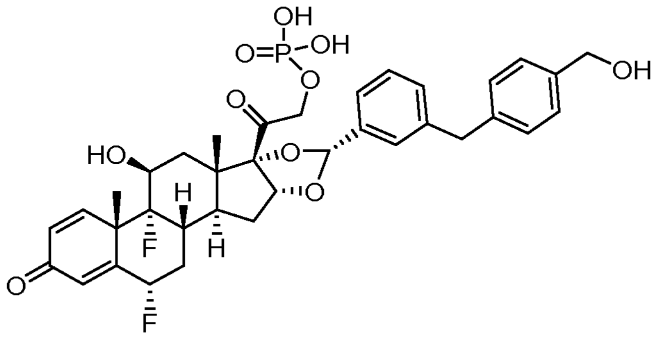 |
| I-253 | 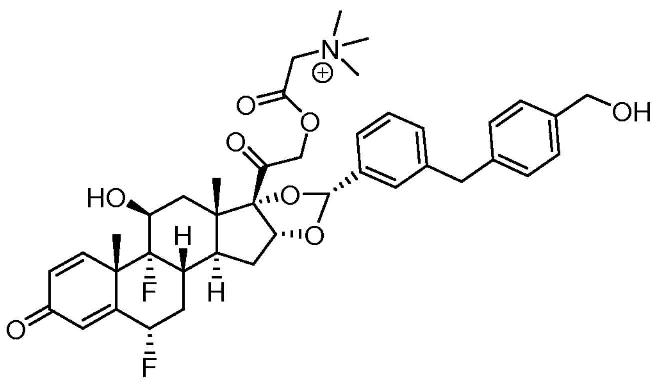 |
| I-254 | 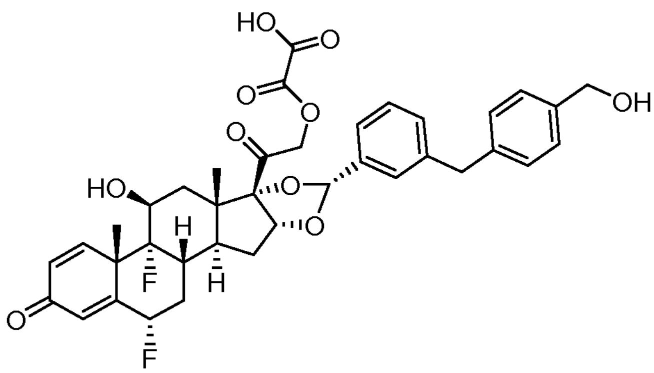 |

(continued)

| | |
|---|---|
| I-255 | 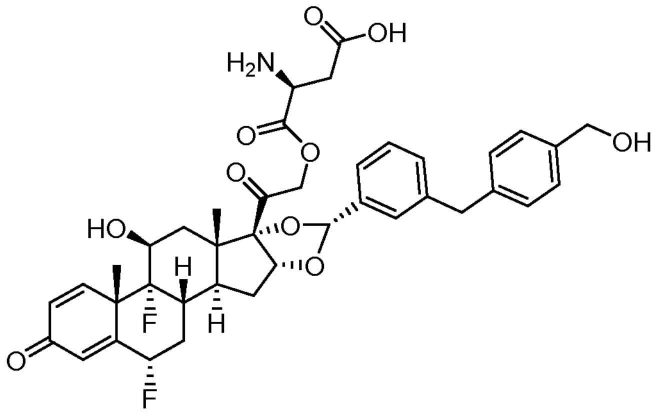 |
| I-256 | 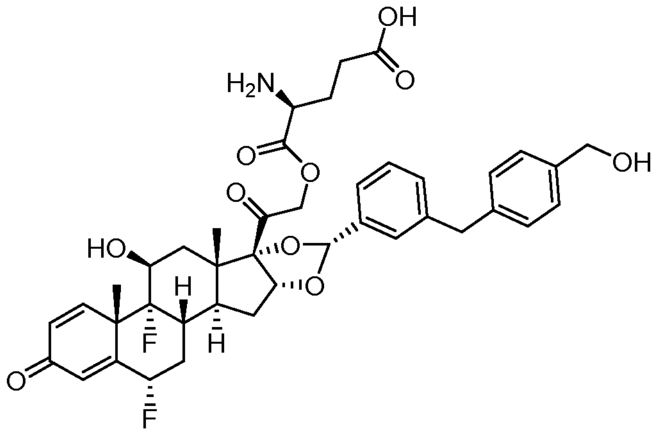 |
| I-257 | 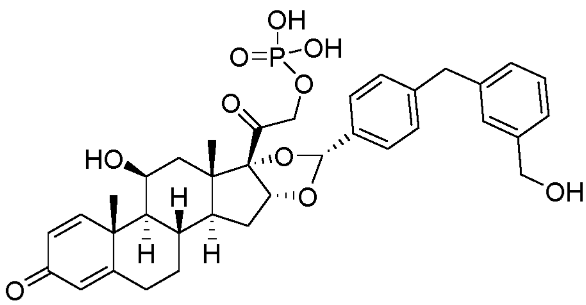 |
| I-258 | 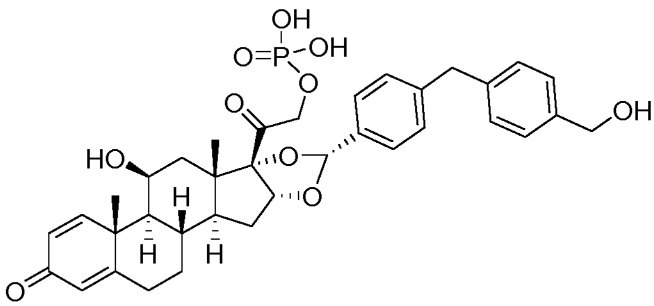 |

(continued)

| | |
|---|---|
| I-259 | 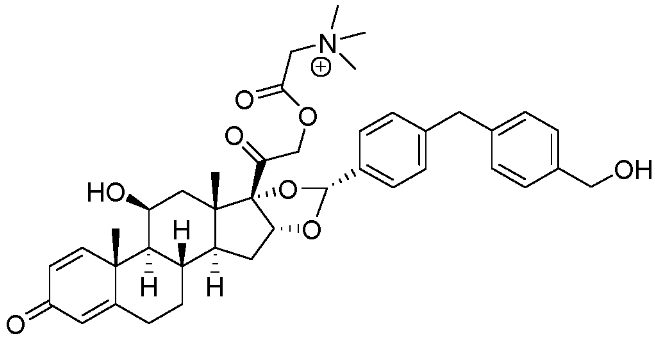 |
| I-260 | 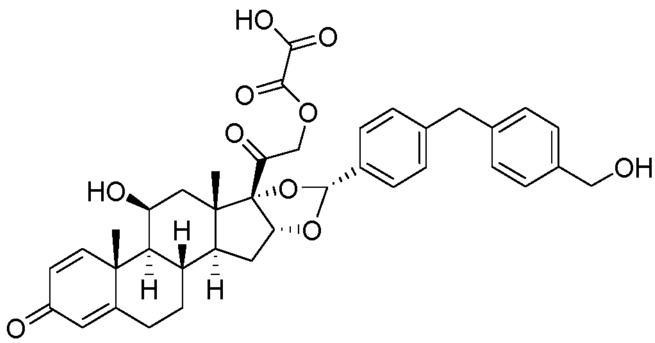 |
| I-261 | 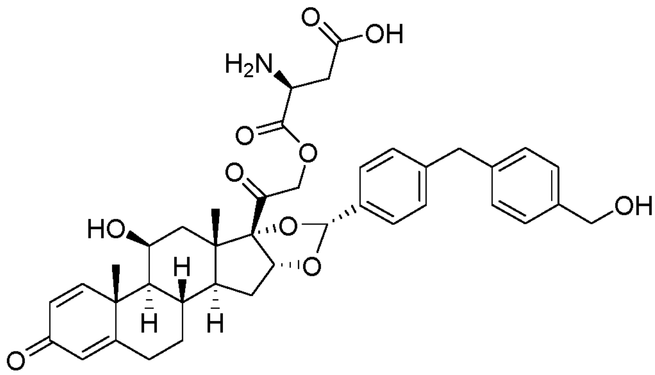 |
| I-262 | 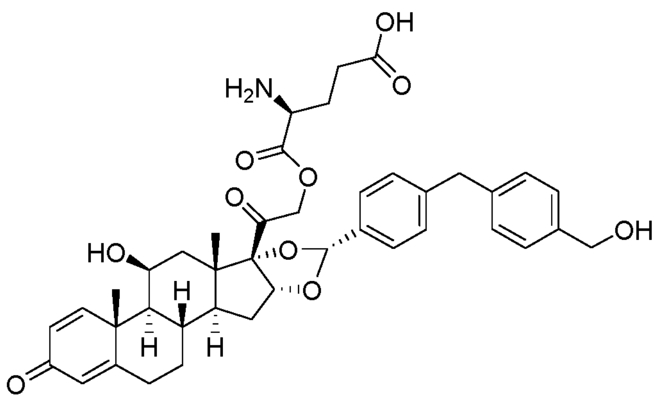 |

(continued)

| | |
|---|---|
| I-263 | 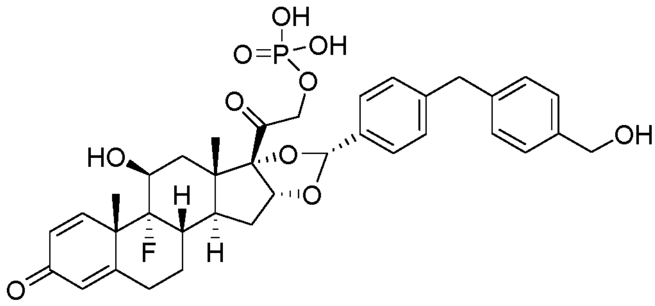 |
| I-264 | 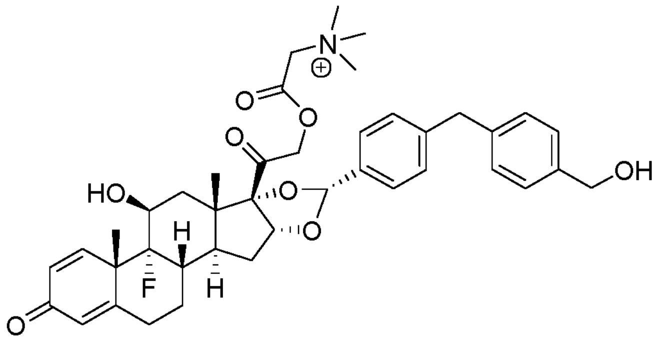 |
| I-265 | 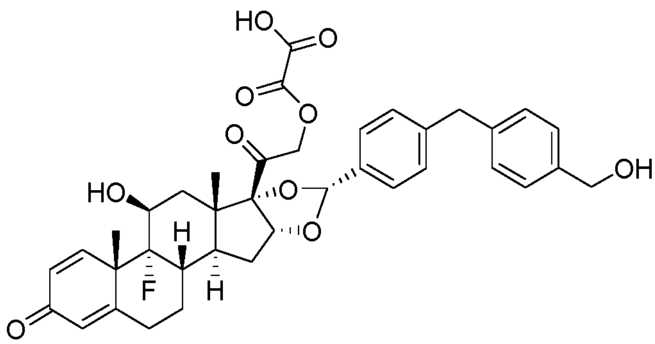 |
| I-266 | 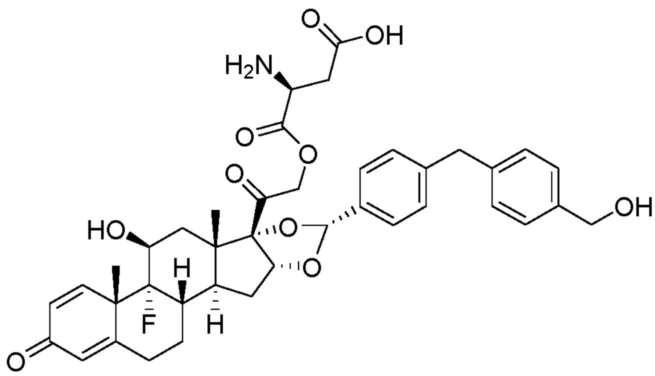 |

(continued)

| | |
|---|---|
| I-267 | 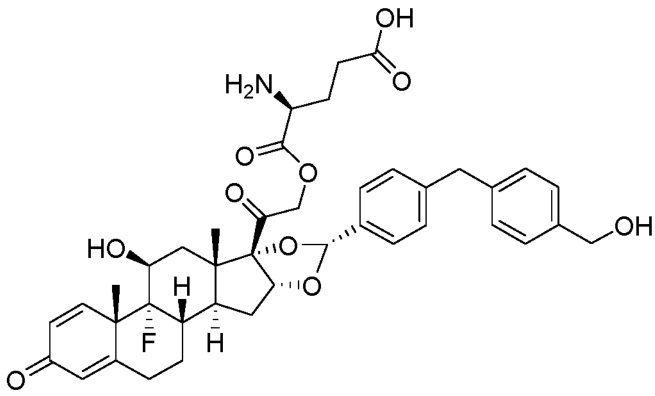 |
| I-268 | 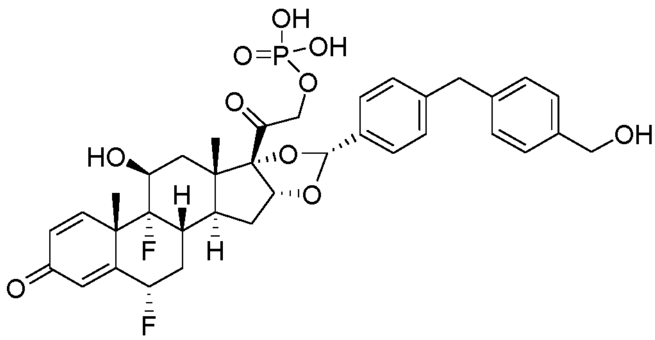 |
| I-269 | 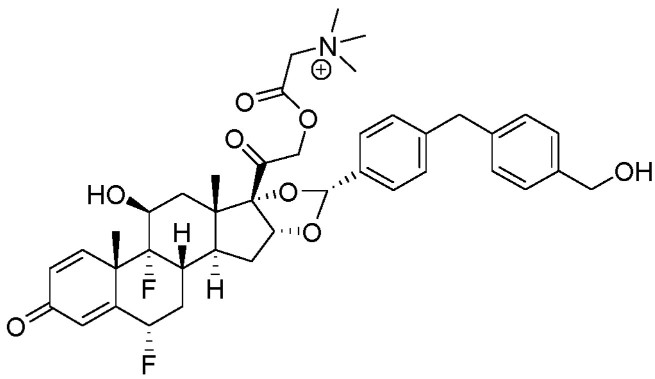 |
| I-270 | 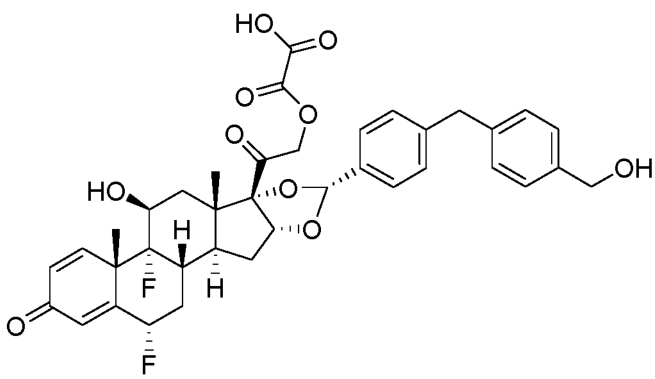 |

(continued)

| | |
|---|---|
| I-271 | 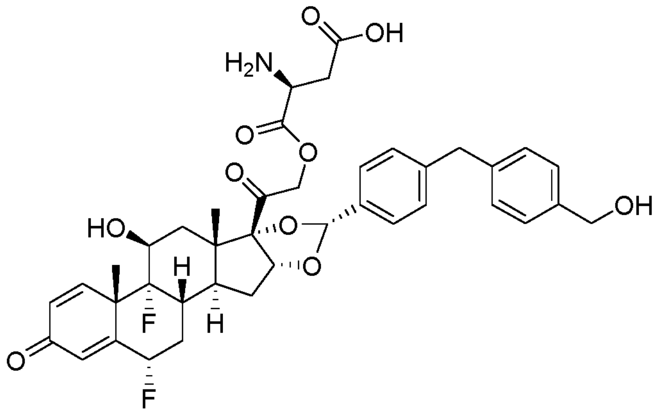 |
| I-272 | 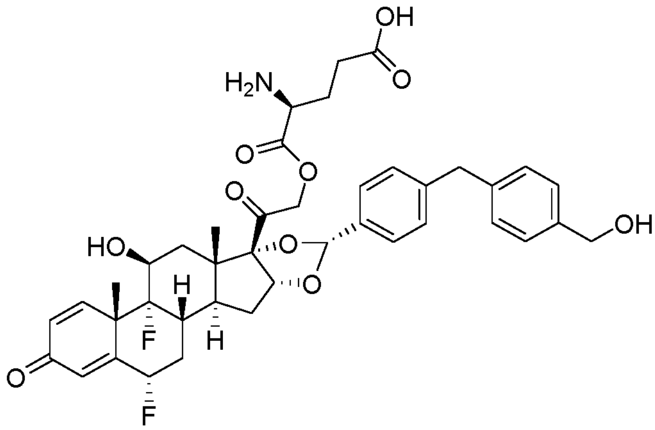 |
| I-273 | 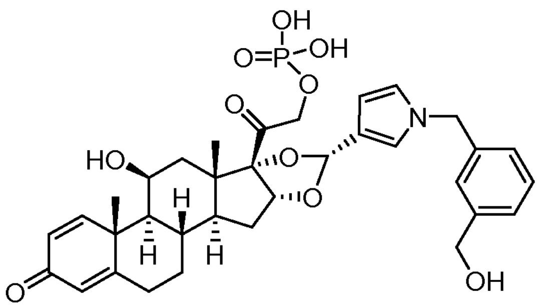 |
| I-274 | 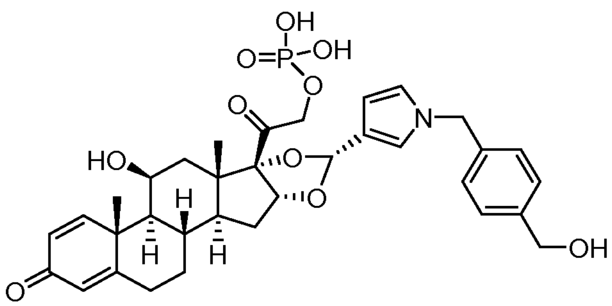 |

(continued)

| | |
|---|---|
| I-275 | 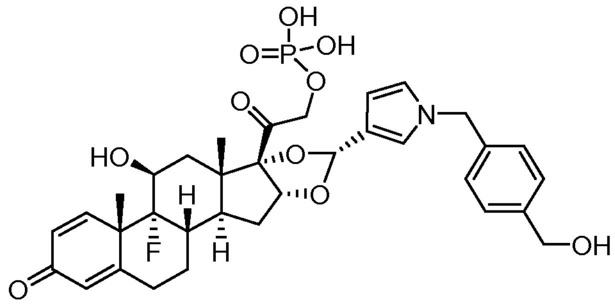 |
| I-276 | 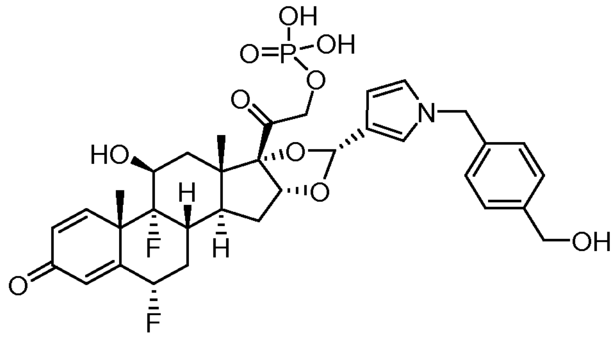 |
| I-277 | 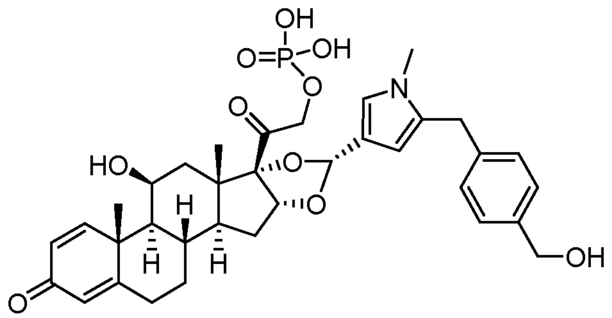 |
| I-278 | 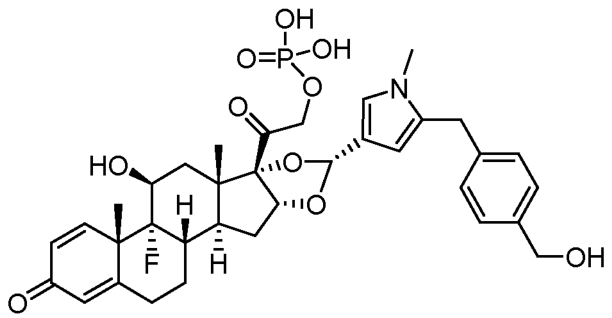 |
| I-279 | 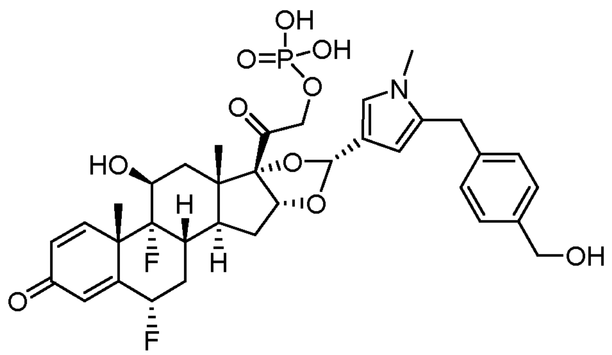 |

(continued)

| | |
|---|---|
| I-280 | 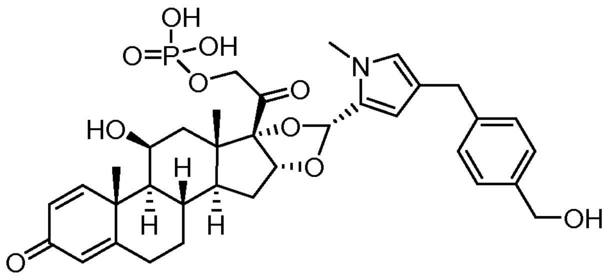 |
| I-281 | 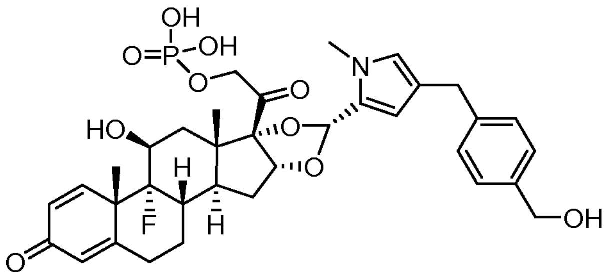 |
| I-282 | 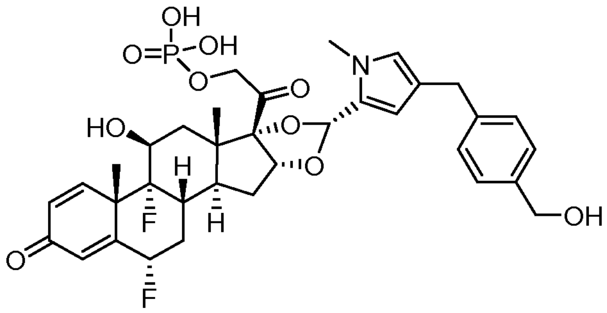 |
| I-283 | 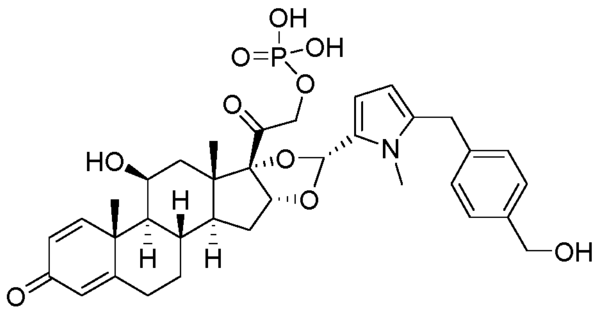 |
| I-284 | 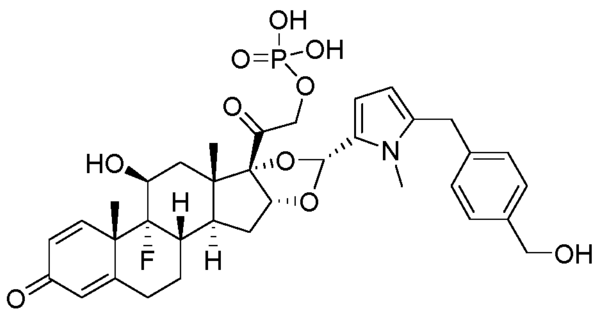 |

(continued)

| | |
|---|---|
| I-285 | 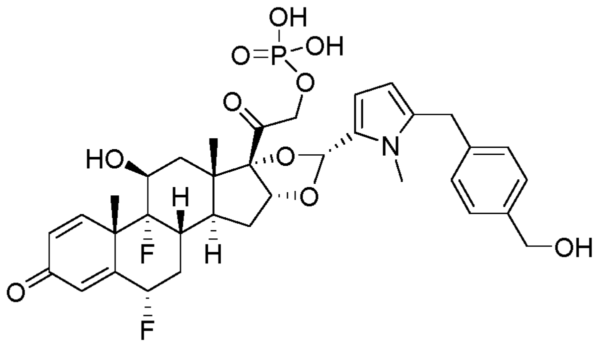 |
| I-286 | 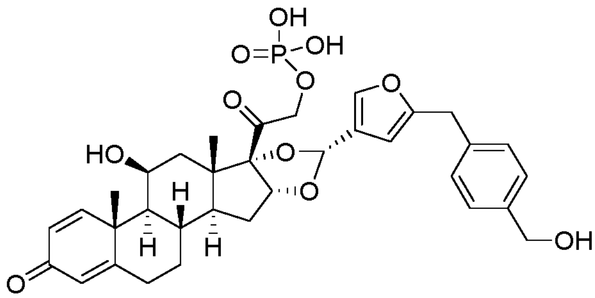 |
| I-287 | 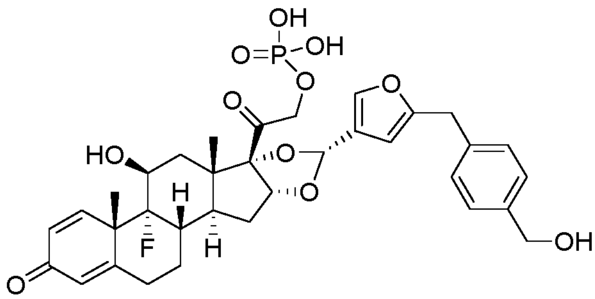 |
| I-288 | 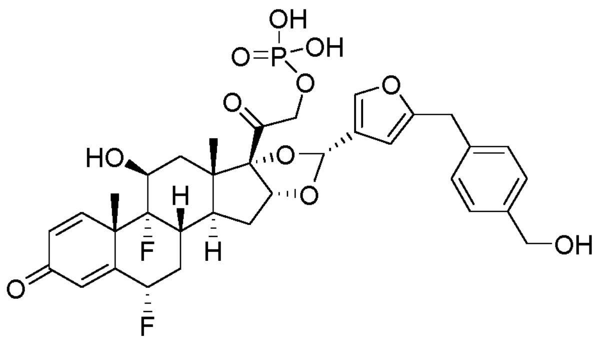 |
| I-289 | 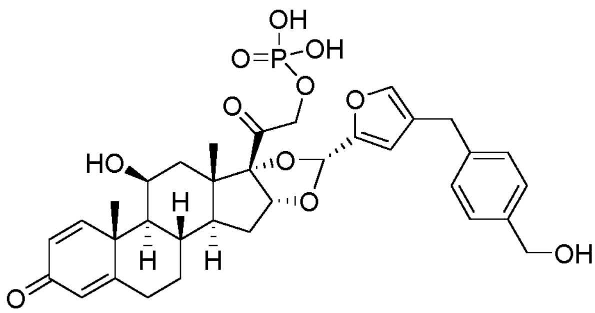 |

(continued)

| | |
|---|---|
| I-290 | 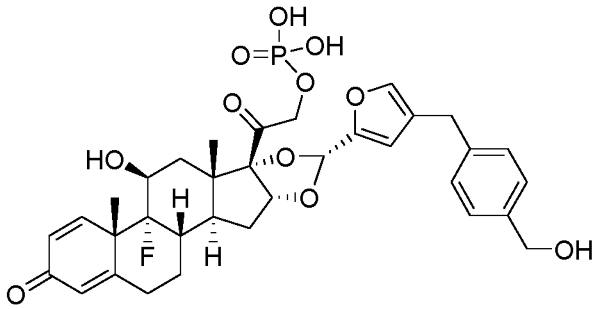 |
| I-291 | 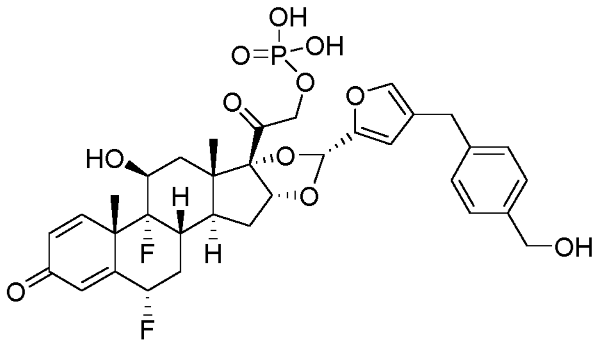 |
| I-292 | 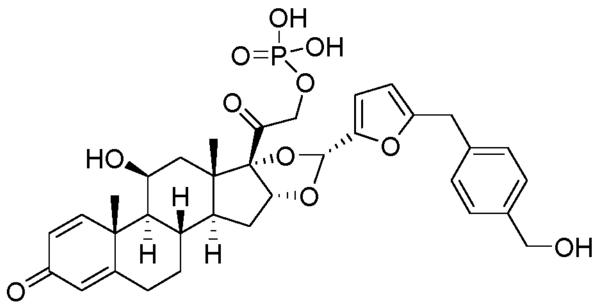 |
| I-293 | 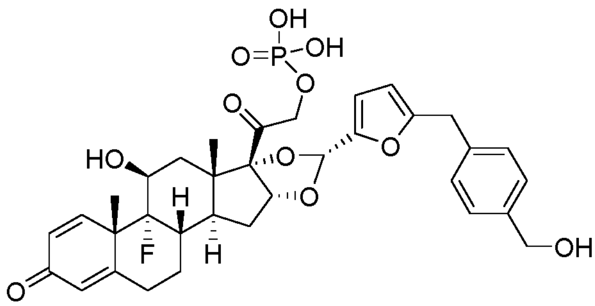 |
| I-294 | 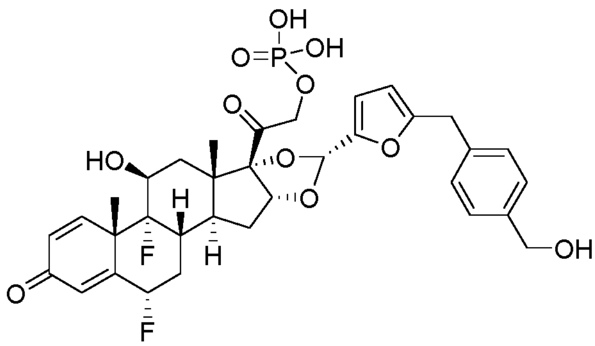 |

(continued)

| | |
|---|---|
| I-295 | 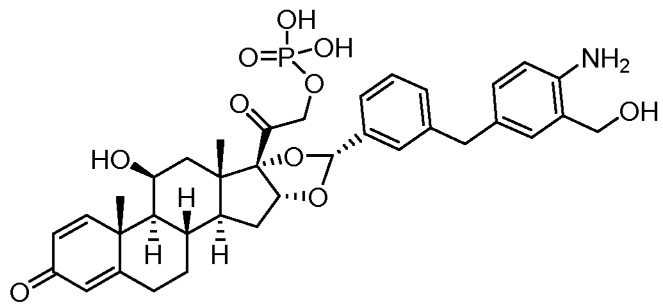 |
| I-296 | 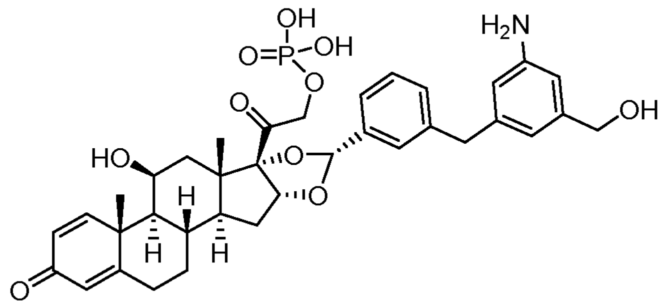 |
| I-297 | 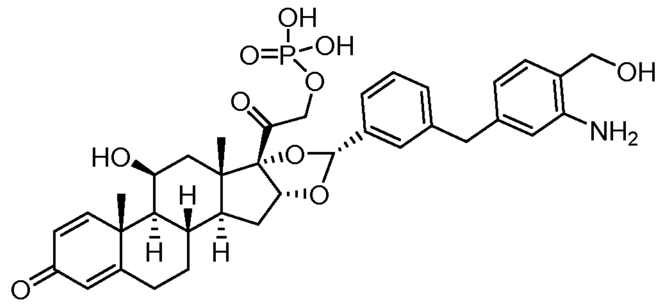 |
| I-298 | 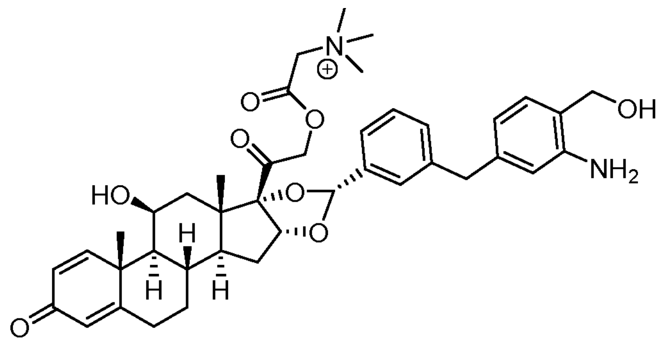 |
| I-299 | 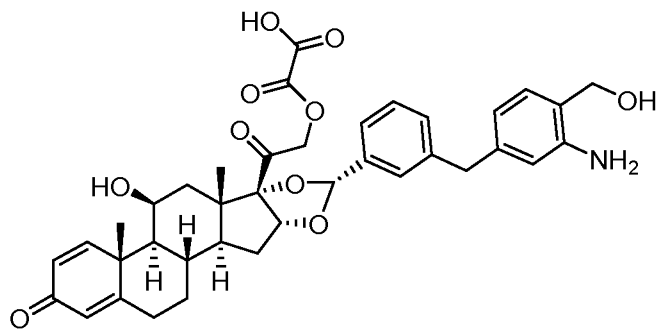 |

(continued)

| | |
|---|---|
| I-300 | 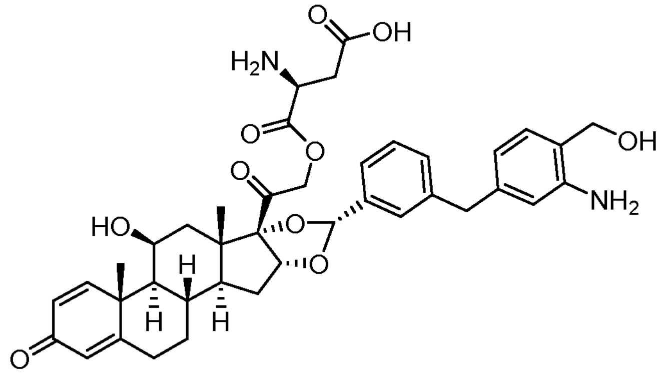 |
| I-301 | 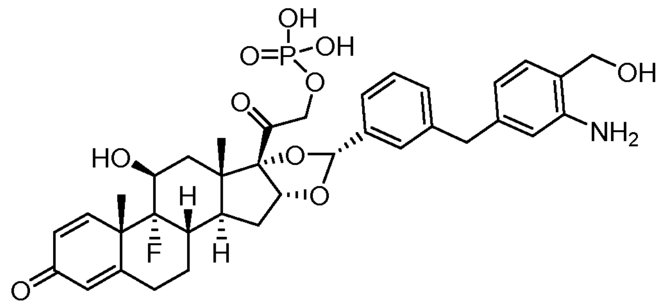 |
| I-302 | 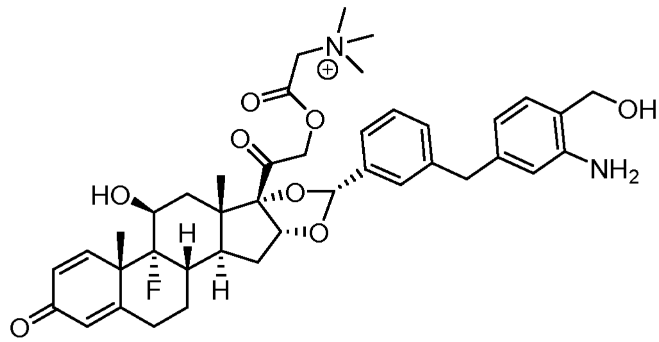 |
| I-303 | 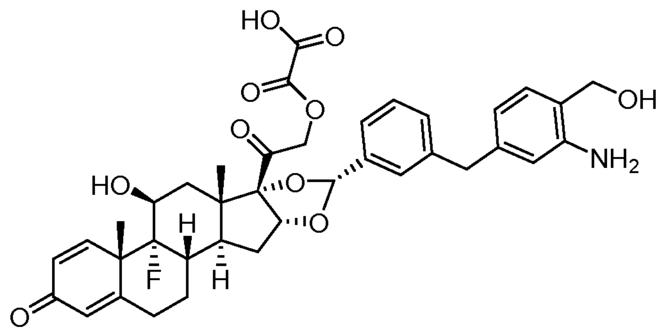 |

(continued)

| | |
|---|---|
| I-304 | 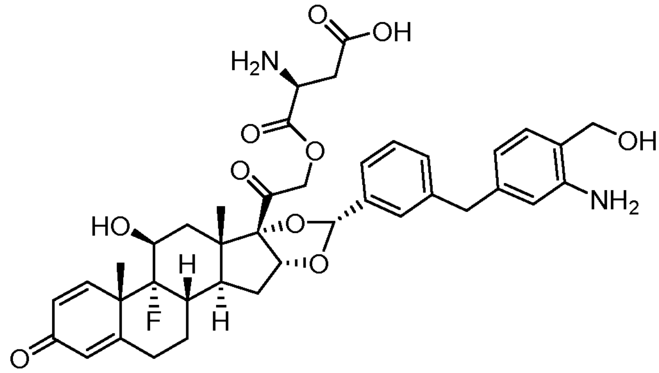 |
| I-305 | 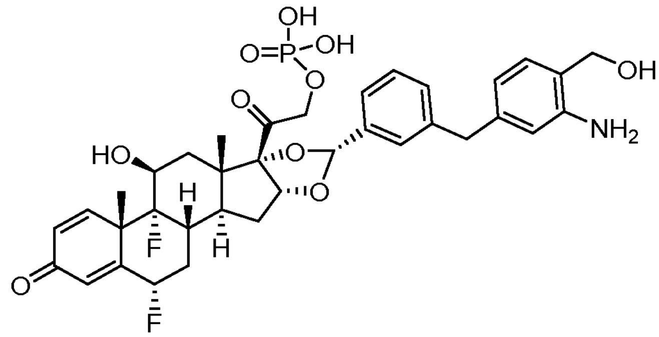 |
| I-306 | 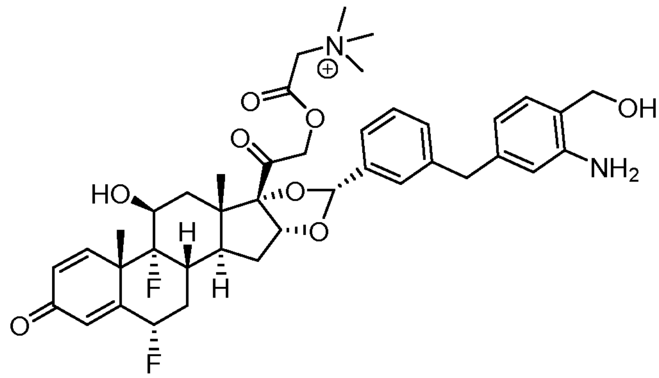 |
| I-307 | 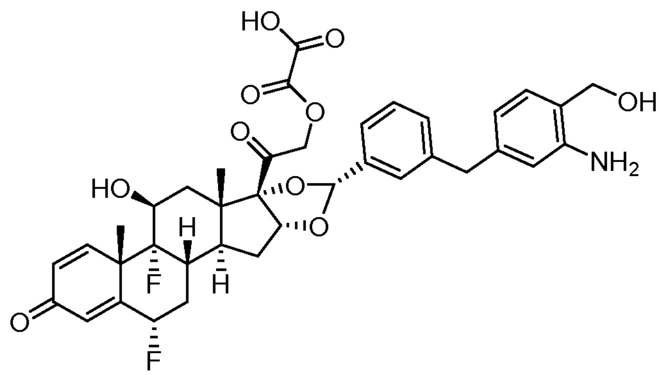 |

(continued)

| | |
|---|---|
| I-308 | 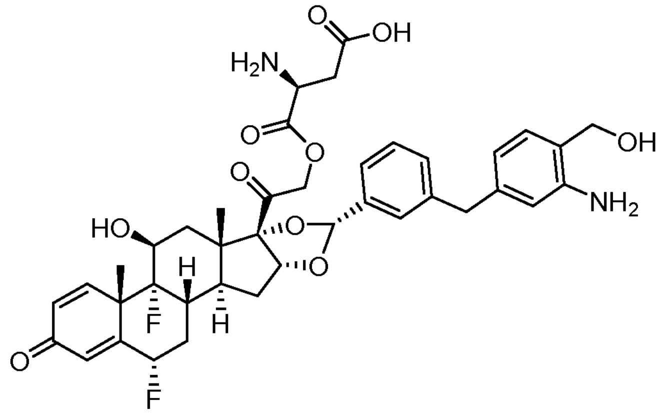 |
| I-309 | 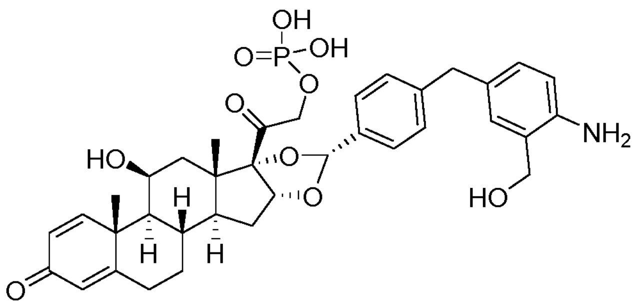 |
| I-310 | 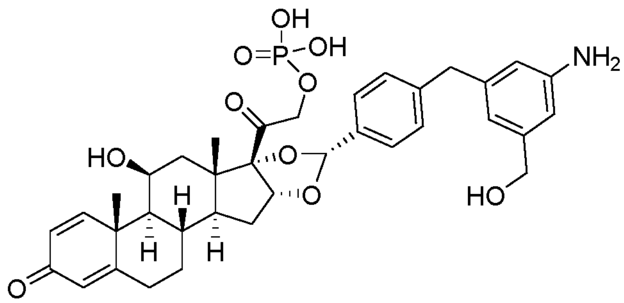 |
| I-311 | 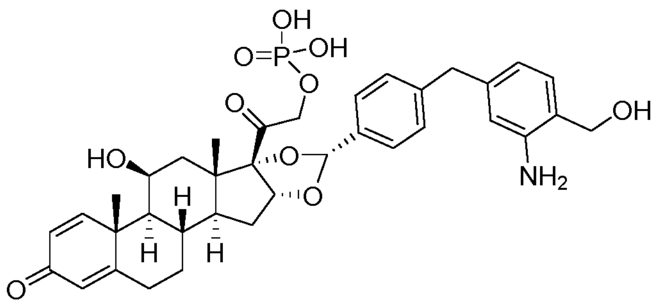 |

(continued)

| | |
|---|---|
| I-312 | 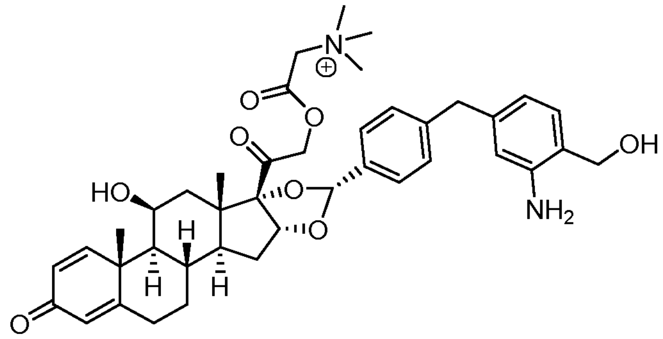 |
| I-313 | 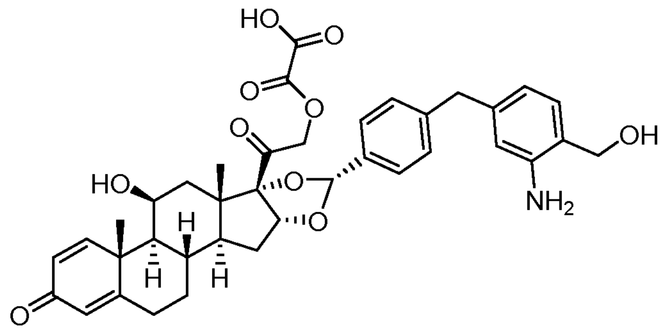 |
| I-314 | 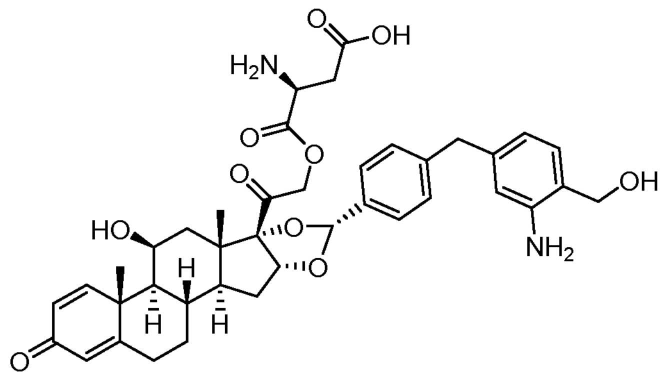 |
| I-315 | 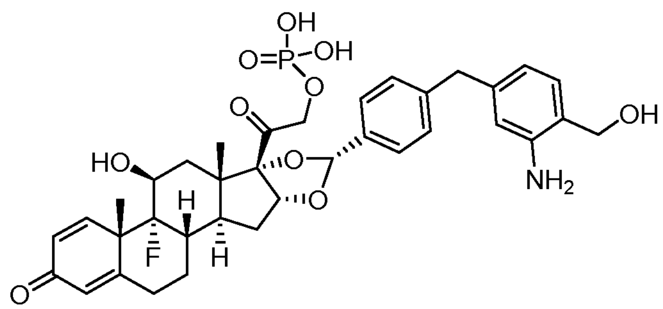 |

(continued)

| | |
|---|---|
| I-316 | 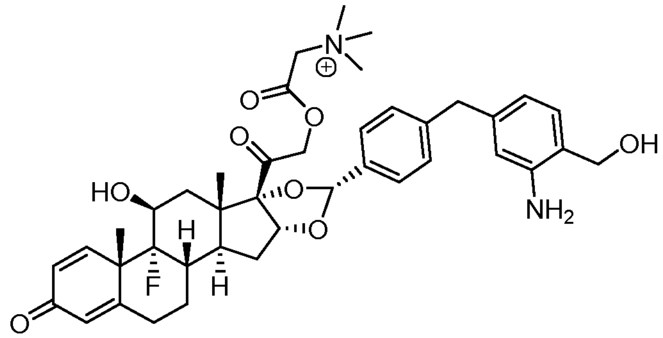 |
| I-317 | 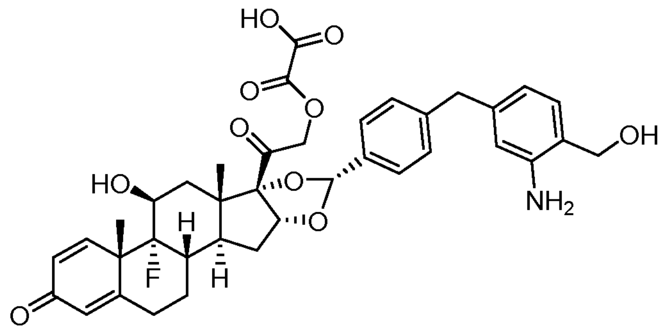 |
| I-318 | 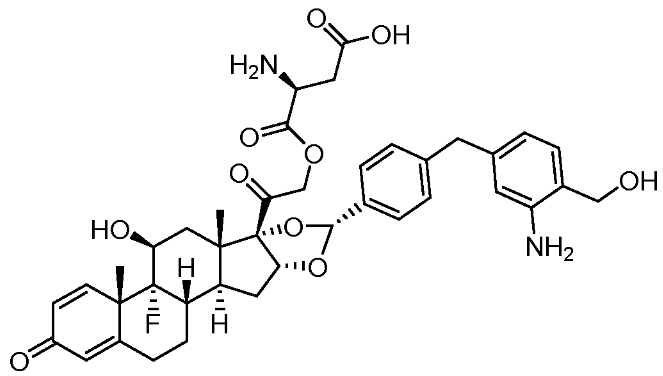 |
| I-319 | 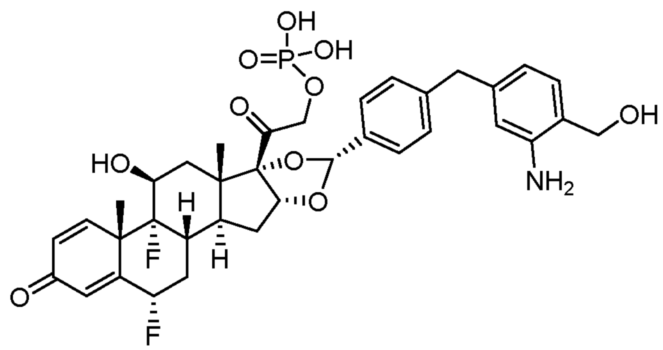 |

(continued)

| | |
|---|---|
| I-320 | 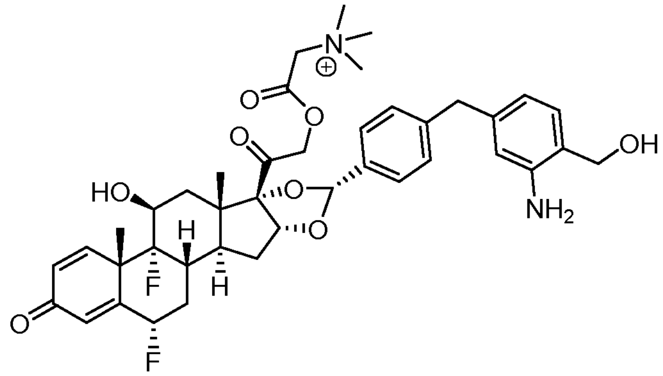 |
| I-321 | 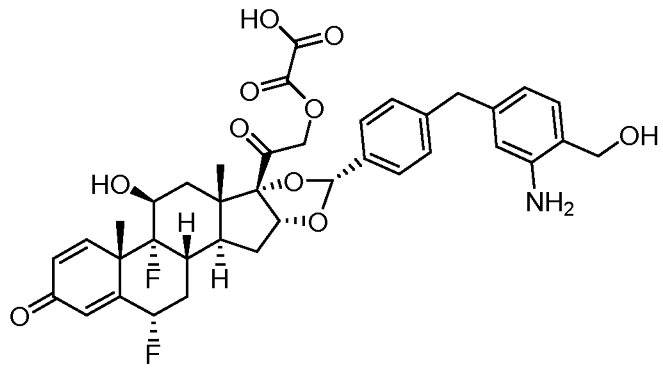 |
| I-322 | 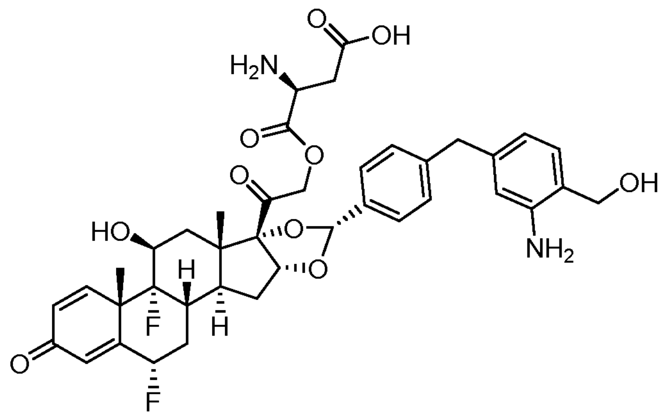 |
| I-323 | 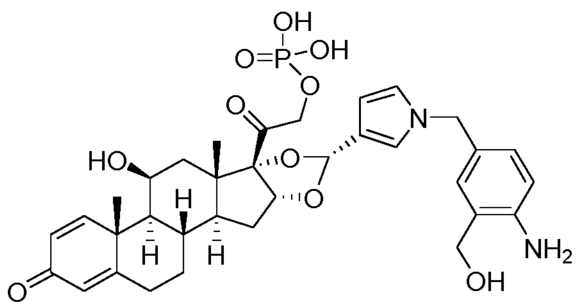 |

(continued)

| | |
|---|---|
| I-324 | 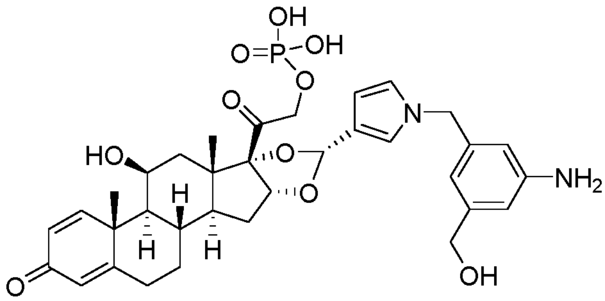 |
| I-325 | 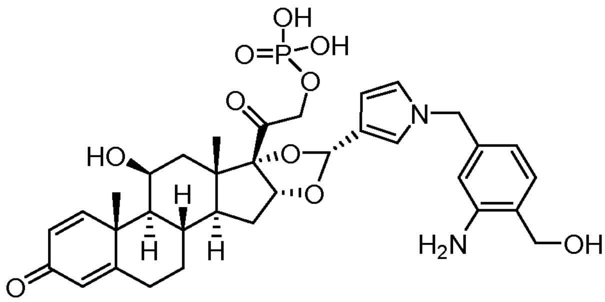 |
| I-326 | 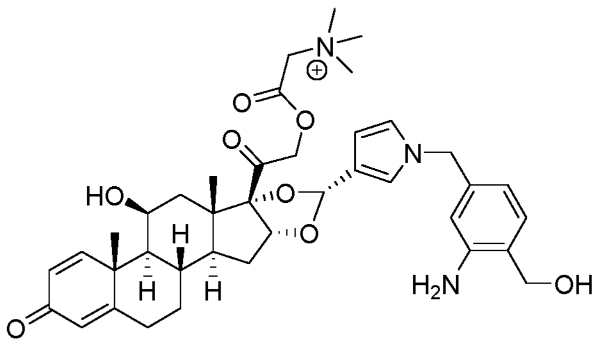 |
| I-327 | 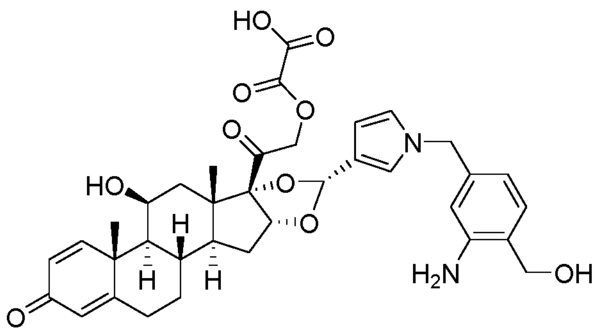 |
| I-328 | 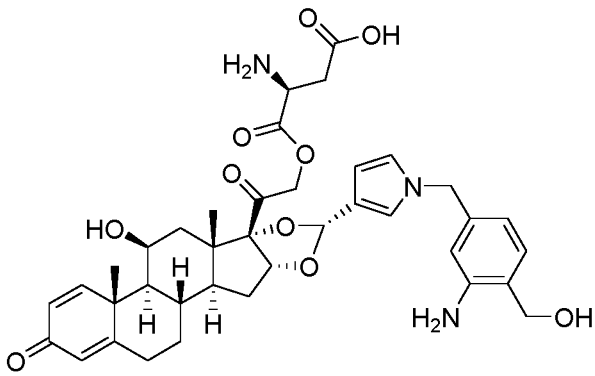 |

(continued)

| | |
|---|---|
| I-329 | 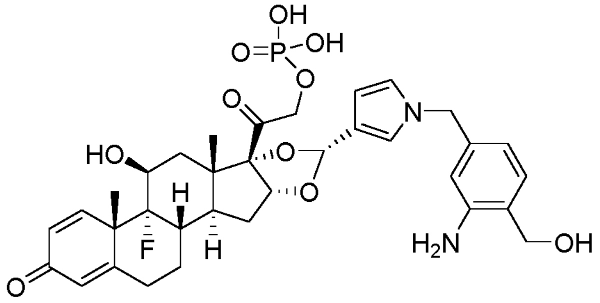 |
| I-330 | 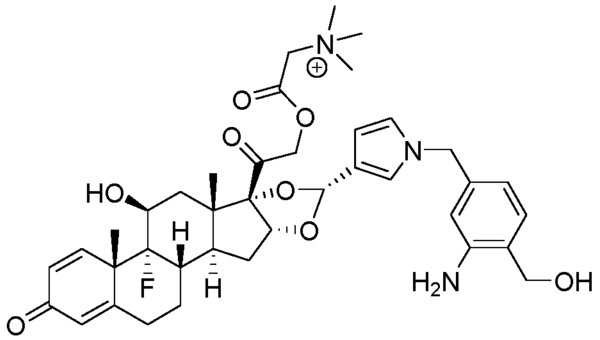 |
| I-331 | 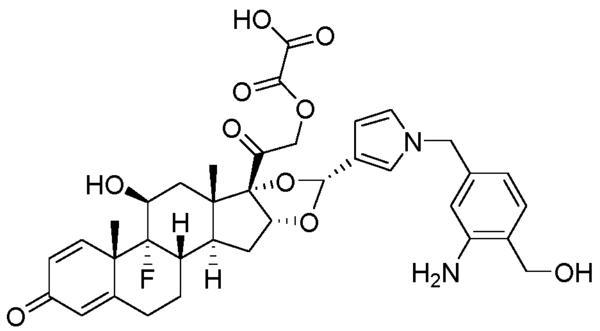 |
| I-332 | 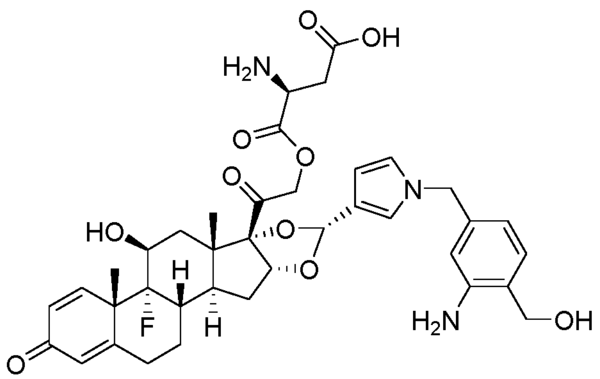 |

(continued)

| | |
|---|---|
| I-333 | 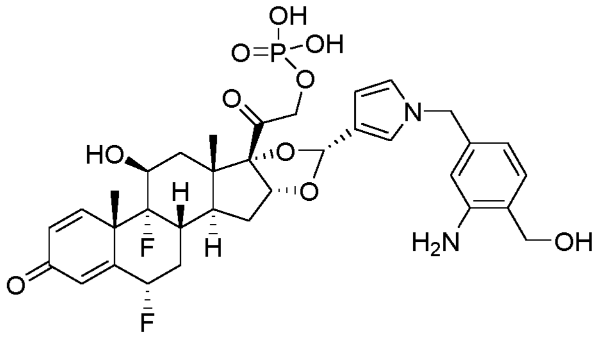 |
| I-334 | 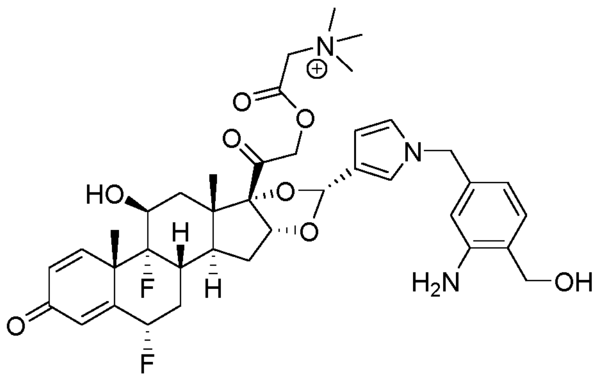 |
| I-335 | 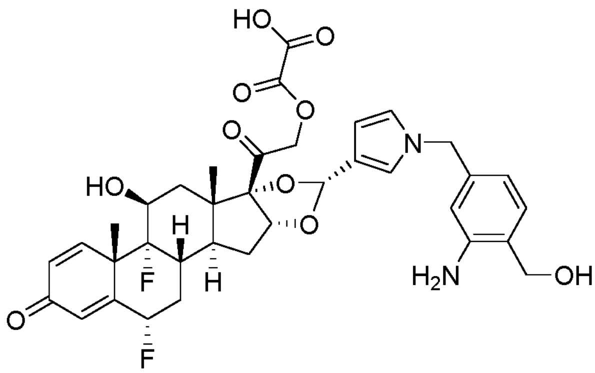 |
| I-336 | 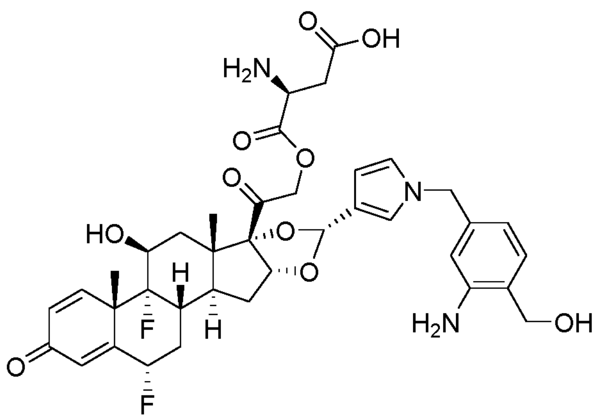 |

(continued)

| | |
|---|---|
| I-337 | 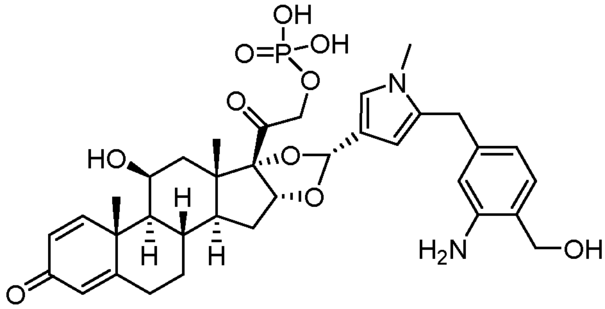 |
| I-338 | 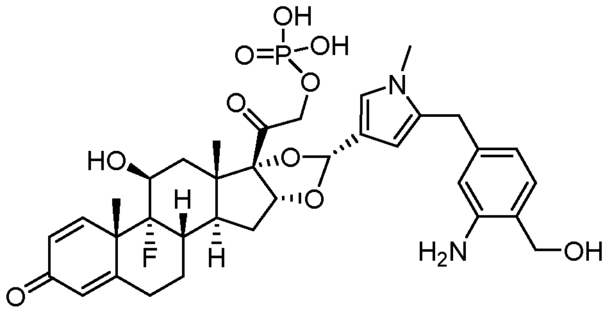 |
| I-339 | 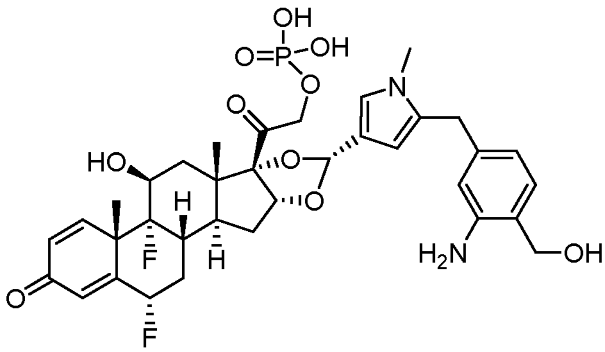 |
| I-340 | 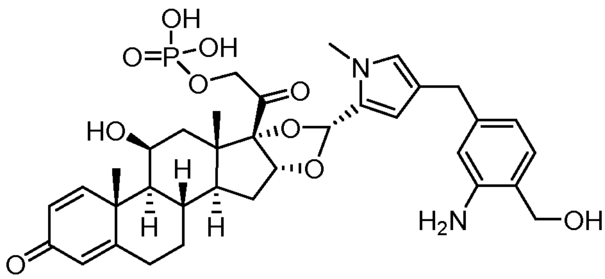 |
| I-341 | 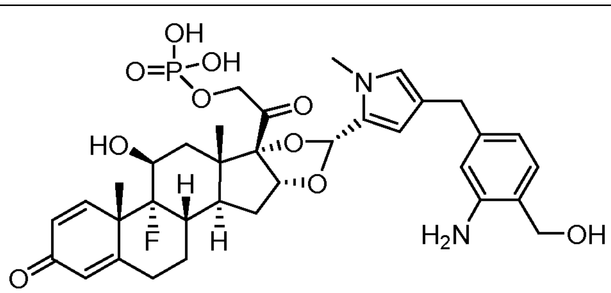 |

(continued)

| | |
|---|---|
| I-342 | 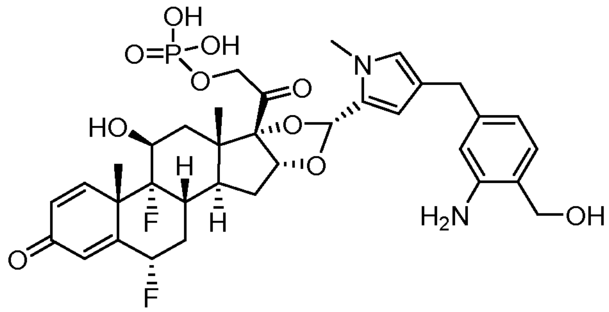 |
| I-343 | 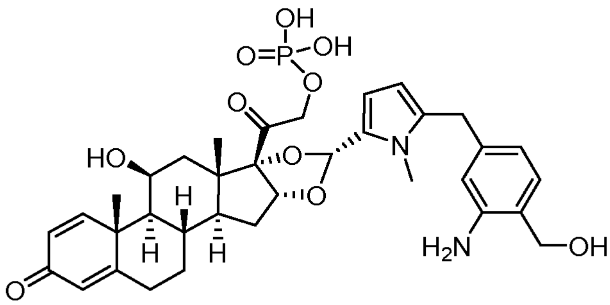 |
| I-344 | 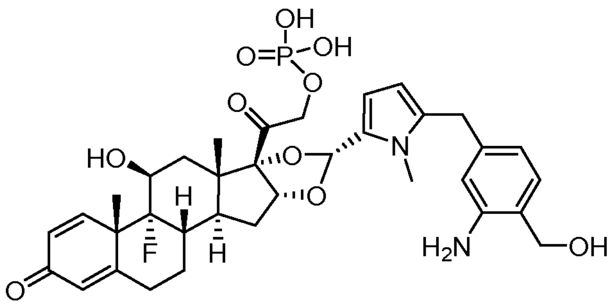 |
| I-345 | 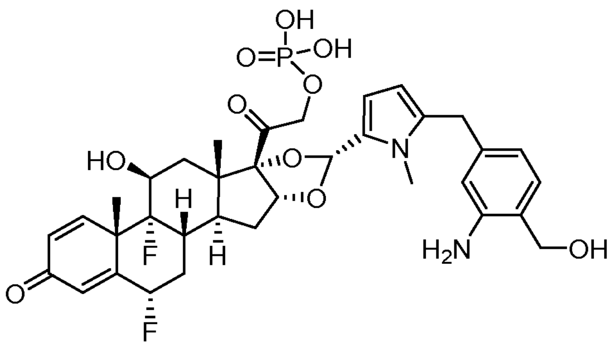 |
| I-346 | 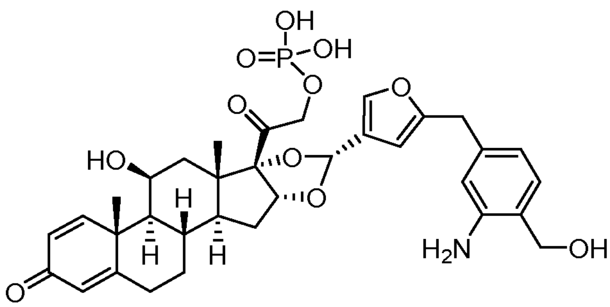 |

(continued)

| | |
|---|---|
| I-347 | 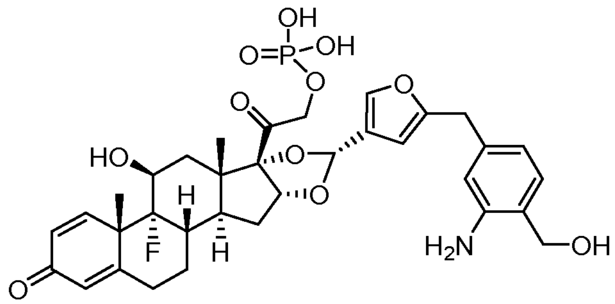 |
| I-348 | 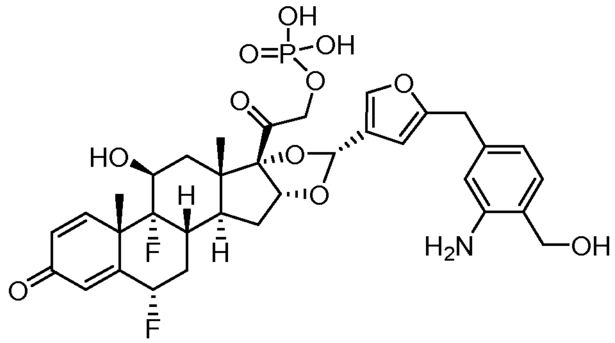 |
| I-349 | 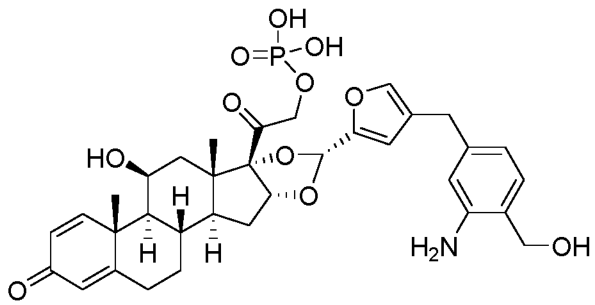 |
| I-350 | 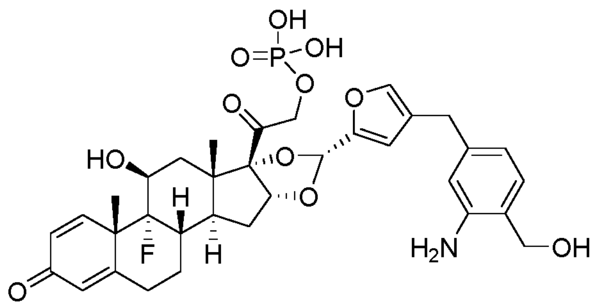 |
| I-351 | 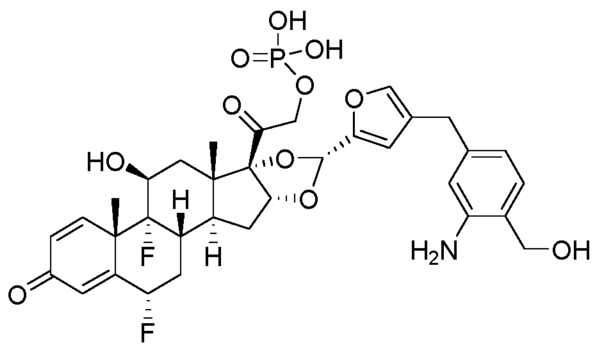 |

(continued)

| | |
|---|---|
| I-352 | 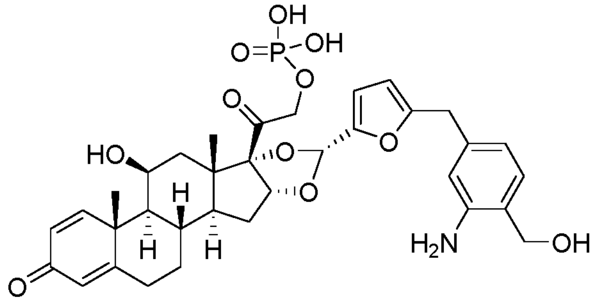 |
| I-353 | 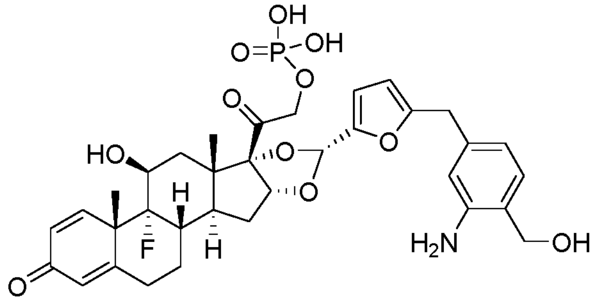 |
| I-354 | 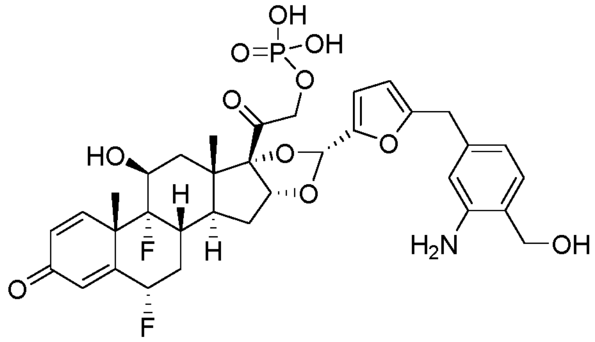 |
| I-355 | 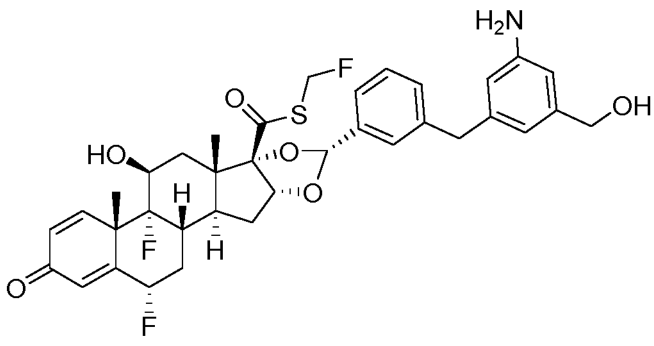 |
| I-356 | 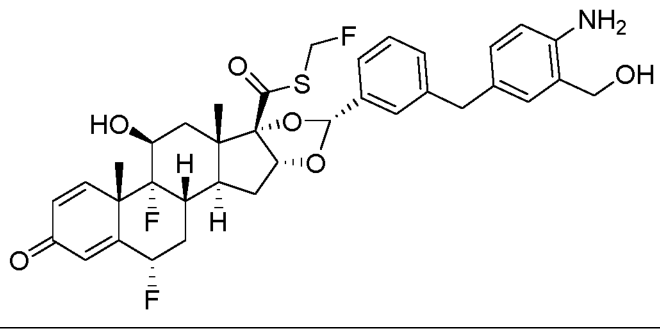 |

(continued)

| | |
|---|---|
| I-357 | 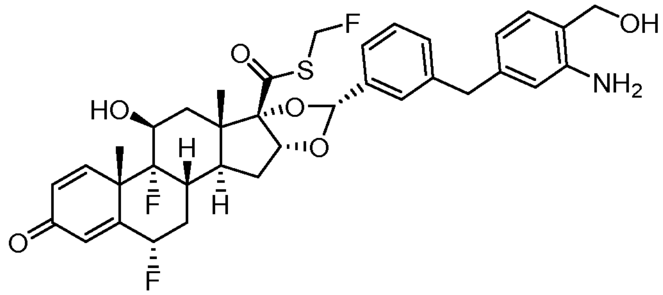 |
| I-358 | 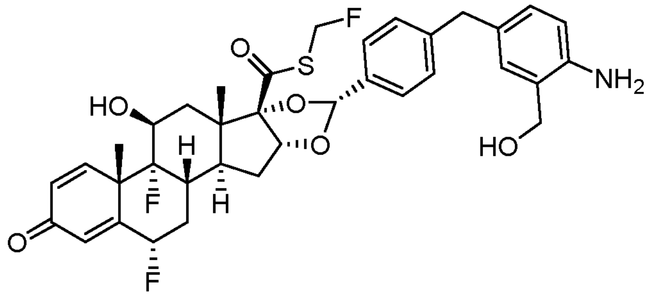 |
| I-359 | 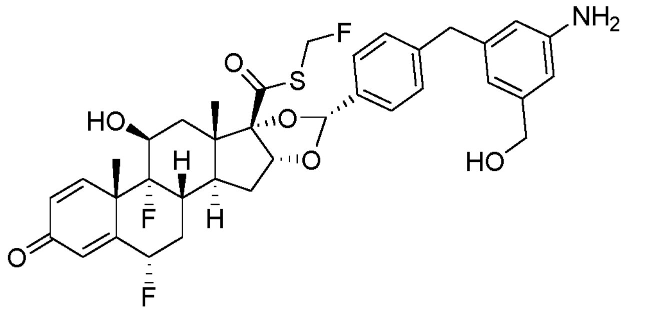 |
| I-360 | 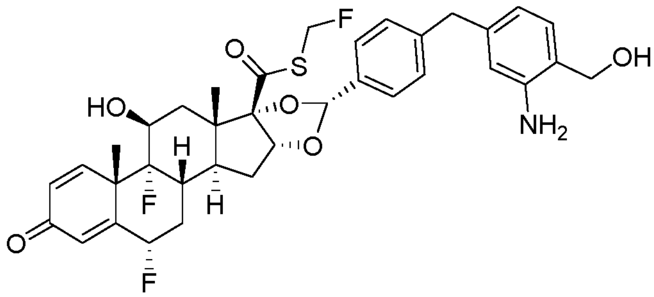 |
| I-361 | 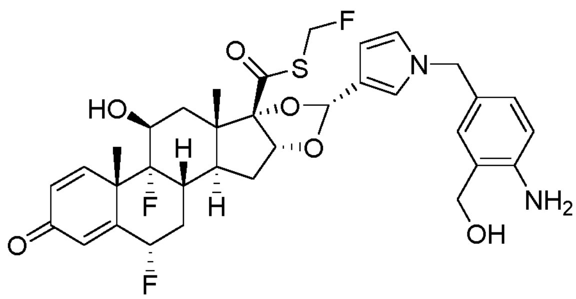 |

(continued)

| | |
|---|---|
| I-362 | 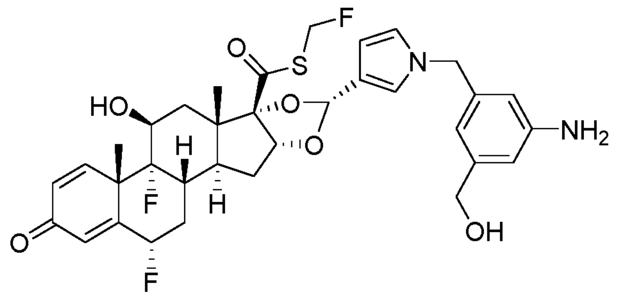 |
| I-363 | 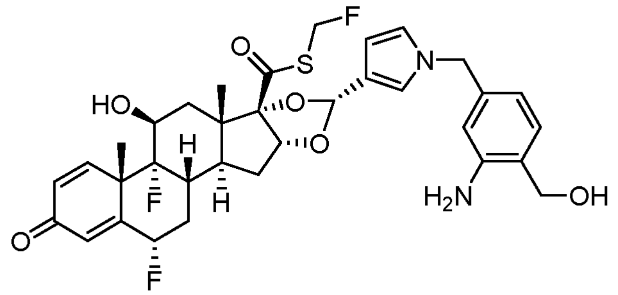 |

**25.** A compound of a formula below or a tautomer, a mesomer, a racemate, an enantiomer or a diastereoisomer thereof, or a mixture thereof, or a pharmaceutically acceptable salt thereof, wherein the compound is selected from the group consisting of:

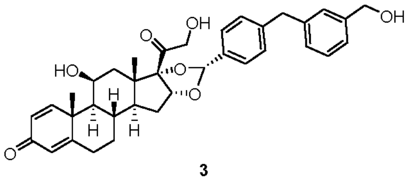

3

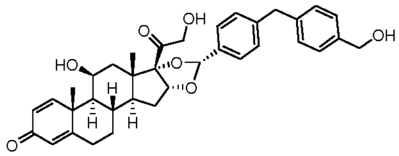

4

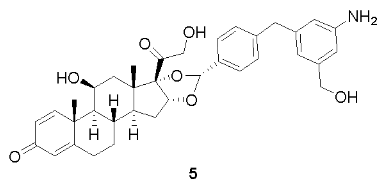

5

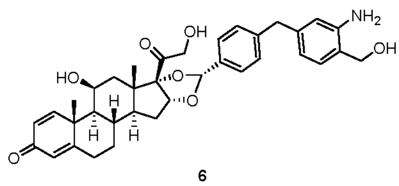

6

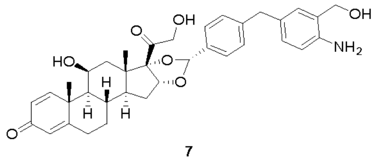

7

and

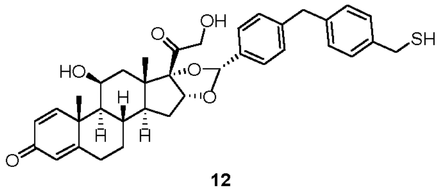

12

.

**26.** A compound of formula IIa or IIb:

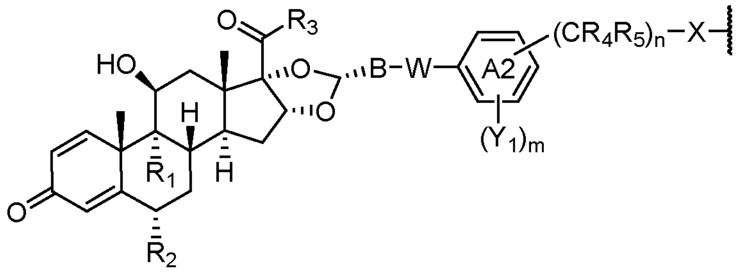

(IIa)

or

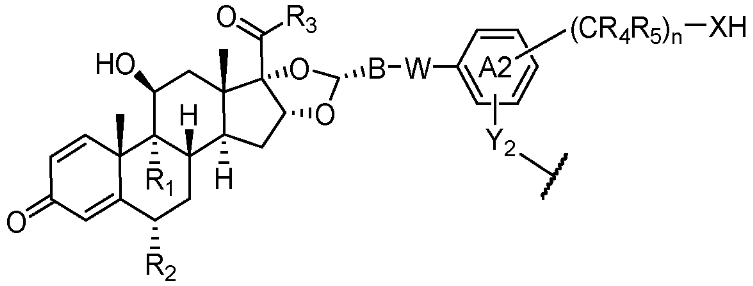

(IIb)

or a tautomer, a mesomer, a racemate, an enantiomer or a diastereoisomer thereof, or a mixture thereof, or a pharmaceutically acceptable salt thereof, wherein,
$R_1$, $R_2$, $R_3$, $R_4$ and $R_5$ are each independently any group;
B is absent or B is any group;
W is absent or W is any group;
A2 is a substituted benzene ring;
$CR_4R_5$ units are each independently unreplaced or replaced by a group selected from the group consisting of: -O-, -S-, -NR-, -S(O)-, $-S(O)_2-$, -C(=O)-, -C=C- and -C=C-, wherein $R_4$ and $R_5$ can together form optionally substituted cycloalkyl, optionally substituted heterocyclyl, optionally substituted bridged cyclyl, optionally substituted bridged heterocyclyl, optionally substituted spiro cyclyl and optionally substituted spiro heterocyclyl, and n is any integer from 1 to 20;
X is selected from the group consisting of: -O-, -S- and -NR-;
$Y_1$ is any group, and m is any integer from 0 to 4;
$Y_2$ is selected from the group consisting of -O-, -S- and -NR-;
wherein substituents are selected from the group consisting of: protium, deuterium, tritium, halogen, -CN, =O, =N-OH, =N-OR, =N-R, -OR, -C(O)R, -C(O)OR, -OC(O)R, -OC(O)OR, -C(O)NHR, - $C(O)NR_2$, -OC(O)NHR, $-OC(O)NR_2$, -SR, -S(O)R, $-S(O)_2R$, -NHR, $-N(R)_2$, -NHC(O)R, -NRC(O)R, -NHC(O)OR, -NRC(O)OR, $-S(O)_2NHR$, $-S(O)_2N(R)_2$, $-NHS(O)_2NR_2$, $-NRS(O)_2NR_2$, $-NHS(O)_2R$, - $NRS(O)_2R$, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy; wherein each R is independently selected from the group consisting of hydrogen, protium, deuterium, tritium, oxygen, halogen, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy; the wavy line

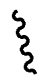

in the general formula represents being directly linked to a ligand via the X or $Y_2$ group, or being linked to the ligand via a linker fragment.

**27.** The compound of formula IIa or IIb or the tautomer, the mesomer, the racemate, the enantiomer or the diastereoisomer

thereof, or the mixture thereof, or the pharmaceutically acceptable salt thereof according to claims 26, wherein $R_4$ and $R_5$ are each independently selected from the group consisting of: hydrogen, protium, deuterium, tritium, halogen, optionally substituted -C(=O)H, optionally substituted -OH, optionally substituted -SH, optionally substituted $-NH_2$, optionally substituted alkyl, optionally substituted alkenyl, optionally substituted alkynyl, optionally substituted cycloalkyl, optionally substituted heterocycloalkyl, optionally substituted aryl and optionally substituted heteroaryl; wherein, when $R_4$ and $R_5$ comprise a methylene unit, the methylene units of $R_4$ and $R_5$ are each independently unreplaced, or the methylene units of $R_4$ and $R_5$ are each independently replaced by a group selected from the group consisting of: -S(=O)-, $-S(=O)_2-$, -O-, -S-, -C(=O)-, optionally substituted -PH-, optionally substituted -P(=O)H-, optionally substituted -NH-, optionally substituted alkylene, optionally substituted alkenylene, optionally substituted alkynylene, optionally substituted cycloalkylene, optionally substituted heterocycloalkylene, optionally substituted arylene and optionally substituted heteroarylene.

**28.** The compound of formula IIa or IIb or the tautomer, the mesomer, the racemate, the enantiomer or the diastereoisomer thereof, or the mixture thereof, or the pharmaceutically acceptable salt thereof according to claim 27, wherein $R_4$ and $R_5$ are each independently selected from the group consisting of: H, F, Cl, -OH, $-NH_2$ and $C_1$-$C_6$ alkyl; and n is selected from the group consisting of 1, 2 and 3.

**29.** The compound of formula IIa or IIb or the tautomer, the mesomer, the racemate, the enantiomer or the diastereoisomer thereof, or the mixture thereof, or the pharmaceutically acceptable salt thereof according to any one of claims 26-28, wherein $Y_1$ is absent or selected from the group consisting of: -OR, -SR, $-N(R)_2$ and optionally substituted $C_1$-$C_6$ alkyl; wherein each R is independently selected from the group consisting of hydrogen, protium, deuterium, tritium, $C_1$-$C_6$ alkyl and $C_1$-$C_6$ alkoxy.

**30.** The compound of formula IIa or IIb or the tautomer, the mesomer, the racemate, the enantiomer or the diastereoisomer thereof, or the mixture thereof, or the pharmaceutically acceptable salt thereof according to claim 29, wherein $Y_1$ is absent or selected from the group consisting of: hydroxy, sulfhydryl, amino and $C_1$-$C_6$ alkyl.

**31.** The compound of formula IIa or IIb or the tautomer, the mesomer, the racemate, the enantiomer or the diastereoisomer thereof, or the mixture thereof, or the pharmaceutically acceptable salt thereof according to any one of claims 26-30, wherein A2 is selected from the group consisting of:

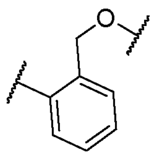 , 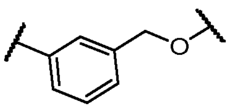 , 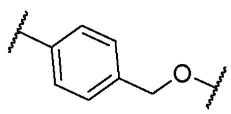 , 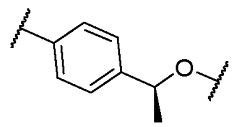 , 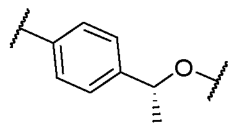 ,

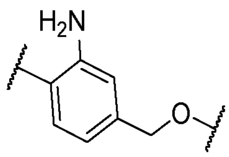 , 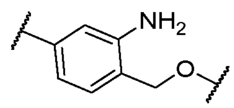 , 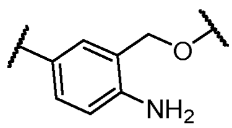 , 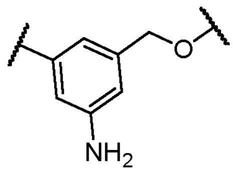 , 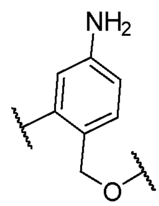 ,

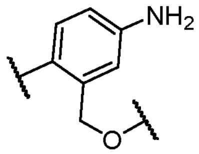 , 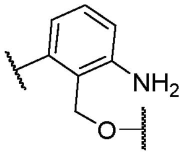 , 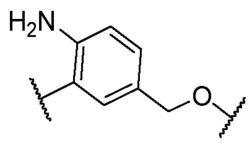 , 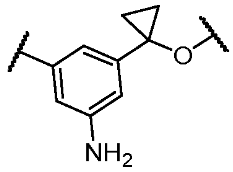 , 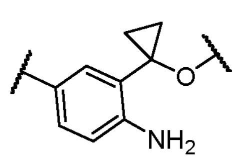 ,

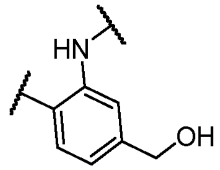, 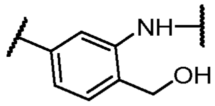, 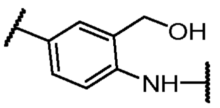, 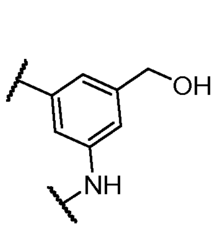, 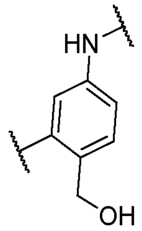,

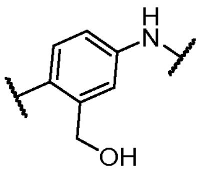, 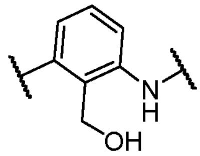, 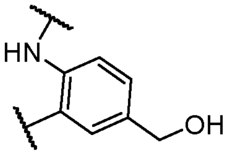, 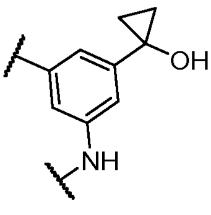, 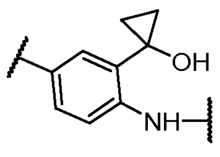,

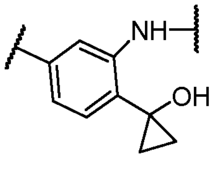, 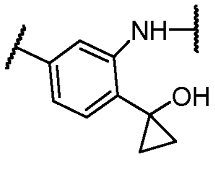, 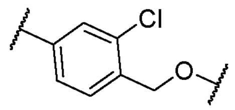, 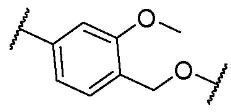, 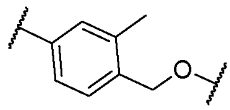,

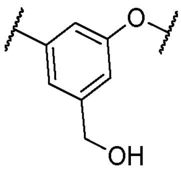, 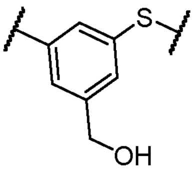, 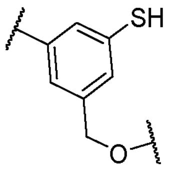, 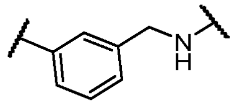, 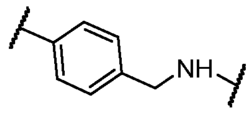,

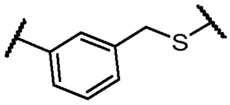, 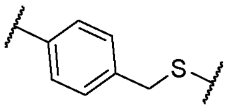, 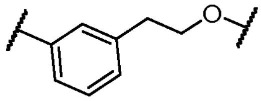, 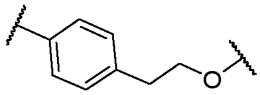,

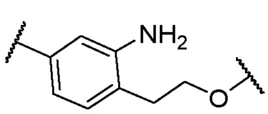, 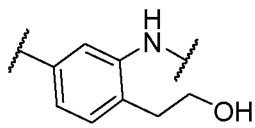, 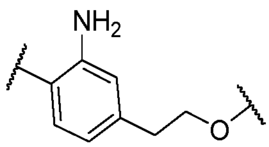 and 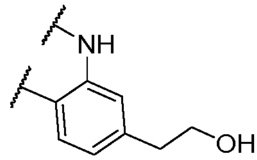.

**32.** The compound of formula IIa or IIb or the tautomer, the mesomer, the racemate, the enantiomer or the diastereoisomer thereof, or the mixture thereof, or the pharmaceutically acceptable salt thereof according to any one of claims 26-31, wherein $R_1$ and $R_2$ are each independently selected from the group consisting of: H, F, Cl, Br and optionally substituted $C_1$-$C_6$ alkyl.

**33.** The compound of formula IIa or IIb or the tautomer, the mesomer, the racemate, the enantiomer or the diastereoisomer thereof, or the mixture thereof, or the pharmaceutically acceptable salt thereof according to any one of claims 26-33, wherein $R_1$ and $R_2$ are each independently selected from the group consisting of: H, F, Cl, Br and optionally substituted methyl.

**34.** The compound of formula IIa or IIb or the tautomer, the mesomer, the racemate, the enantiomer or the diastereoisomer thereof, or the mixture thereof, or the pharmaceutically acceptable salt thereof according to any one of claims 58-71, wherein $R_3$ is selected from the group consisting of: hydrogen, optionally substituted -OH, optionally substituted -SH and optionally substituted $C_1$-$C_6$ alkyl.

**35.** The compound of formula IIa or IIb or the tautomer, the mesomer, the racemate, the enantiomer or the diastereoisomer thereof, or the mixture thereof, or the pharmaceutically acceptable salt thereof according to any one of claims 26-34, wherein $R_3$ is selected from the group consisting of: optionally substituted $-CH_2Cl$, optionally substituted $-CH_2SH$, optionally substituted $-CH_2OH$, optionally substituted

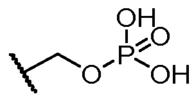,

optionally substituted

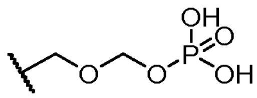,

optionally substituted

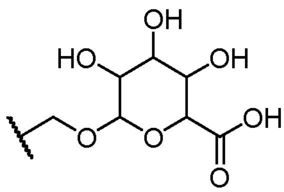,

optionally substituted

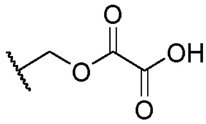,

optionally substituted

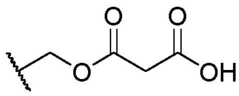,

optionally substituted

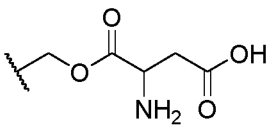,

optionally substituted

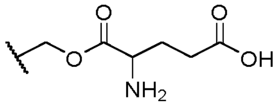,

optionally substituted

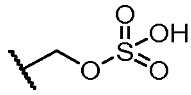

optionally substituted

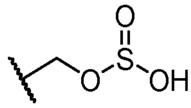

optionally substituted

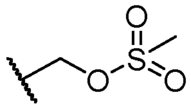

optionally substituted -OH, optionally substituted $-OCH_3$, optionally substituted $-OCH_2F$, optionally substituted $-OCH_2Cl$, optionally substituted $-OCH_2CN$, optionally substituted $-OCH_2CH_3$, optionally substituted sulfhydryl, optionally substituted $-SCH_2F$, optionally substituted $-SCH_2Cl$, optionally substituted $-SCH_2CF_3$ and optionally substituted - $SCH_2CN$.

**36.** The compound of formula IIa or IIb or the tautomer, the mesomer, the racemate, the enantiomer or the diastereoisomer thereof, or the mixture thereof, or the pharmaceutically acceptable salt thereof according to any one of claims 26-35, wherein $R_3$ is selected from the group consisting of: $-CH_2Cl$, $-CH_2SH$, $-CH_2OH$,

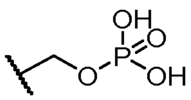

$-OCH_3$, $-OCH_2F$, $-OCH_2Cl$, $-OCH_2CN$, $-OCH_2CH_3$, -SH, - $SCH_2F$, $-SCH_2Cl$, $-SCH_2CF_3$ and $-SCH_2CN$.

**37.** The compound of formula IIa or IIb or the tautomer, the mesomer, the racemate, the enantiomer or the diastereoisomer thereof, or the mixture thereof, or the pharmaceutically acceptable salt thereof according to any one of claims 26-36, wherein B is selected from the group consisting of: optionally substituted

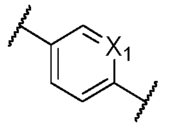

optionally substituted

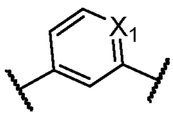

and optionally substituted

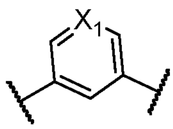

;

and $X_1$ is selected from the group consisting of: CH, C(-O-$CH_3$) and N.

**38.** The compound of formula IIa or IIb or the tautomer, the mesomer, the racemate, the enantiomer or the diastereoisomer thereof, or the mixture thereof, or the pharmaceutically acceptable salt thereof according to any one of claims 26-36, wherein B is selected from the group consisting of: optionally substituted

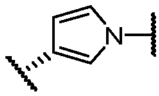

,

optionally substituted

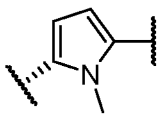

,

optionally substituted

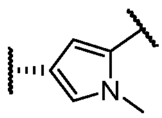

,

optionally substituted

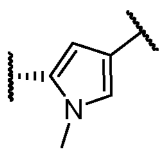

,

optionally substituted

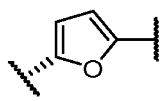

,

optionally substituted

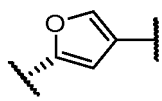

,

optionally substituted

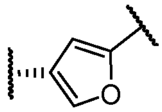,

optionally substituted

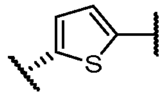,

optionally substituted

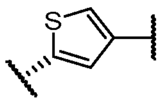,

optionally substituted

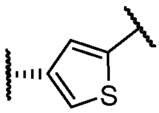,

optionally substituted

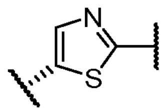,

optionally substituted

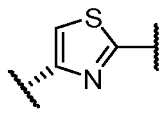,

optionally substituted

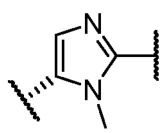,

optionally substituted

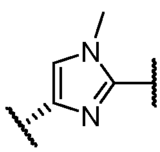,

optionally substituted

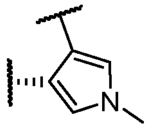,

optionally substituted

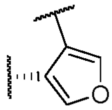,

optionally substituted

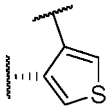,

optionally substituted

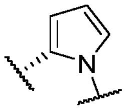,

optionally substituted

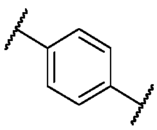

and optionally substituted

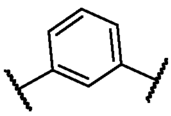,

wherein substituents are selected from the group consisting of: protium, deuterium, tritium, halogen, -CN, =O, =N-OH, =N-OR, =N-R, -OR, - C(O)R, -C(O)OR, -OC(O)R, -OC(O)OR, -C(O)NHR, -C(O)$NR_2$, -OC(O)NHR, -OC(O)$NR_2$, -SR, - S(O)R, -S$(O)_2$R, -NHR, -N$(R)_2$, -NHC(O)R, -NRC(O)R, -NHC(O)OR, -NRC(O)OR, -S$(O)_2$NHR, - S$(O)_2$N$(R)_2$, -NHS$(O)_2$$NR_2$, -NRS$(O)_2$$NR_2$, -NHS$(O)_2$R, -NRS$(O)_2$R, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy, wherein each R is independently selected from the group consisting of hydrogen, protium, deuterium, tritium, oxygen, halogen, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy.

**39.** The compound or the tautomer, the mesomer, the racemate, the enantiomer or the diastereoisomer thereof, or the mixture thereof, or the pharmaceutically acceptable salt thereof according to any one of claims 26-38, wherein B is selected from the group consisting of:

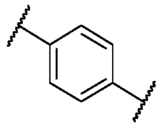, 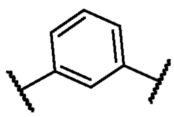

and

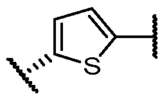.

**40.** The compound of formula IIa or IIb or the tautomer, the mesomer, the racemate, the enantiomer or the diastereoisomer thereof, or the mixture thereof, or the pharmaceutically acceptable salt thereof according to any one of claims 26-39, wherein W is absent or W is selected from the group consisting of: -S(=O)-, $-S(=O)_2-$, -O-, -S-, -C(=O)-, optionally substituted $-CH_2NHC(=O)-$, optionally substituted $-NHC(=O)CH_2-$, optionally substituted -C(=O)NH-, optionally substituted -NH-, optionally substituted -CH=CH-, -C=C-, optionally substituted $C_1$-$C_6$ alkylene, optionally substituted $-OCH_2-$, optionally substituted $-CH_2O-$, optionally substituted $-SCH_2-$, optionally substituted $-CH_2S-$, optionally substituted -NHC(=O)-, optionally substituted $-COCH_2-$, optionally substituted $-CH_2NH-$, optionally substituted $-NHCH_2-$, optionally substituted $-CH(CH_3)-$, optionally substituted

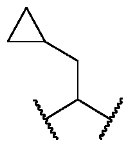,

optionally substituted

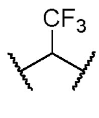,

optionally substituted

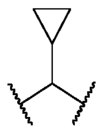,

optionally substituted

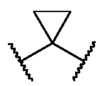

and optionally substituted

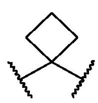.

**41.** The compound of formula IIa or IIb or the tautomer, the mesomer, the racemate, the enantiomer or the diastereoisomer thereof, or the mixture thereof, or the pharmaceutically acceptable salt thereof according to claim 40, wherein W is

absent or W is selected from the group consisting of:

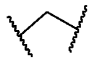, 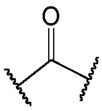, 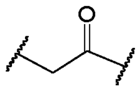, 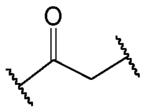, 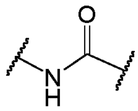, 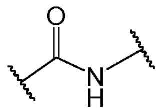, 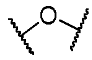, 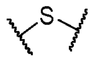, 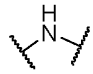,

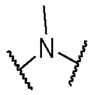, 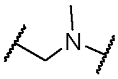, 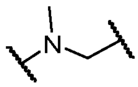, 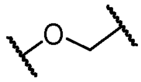, 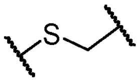, 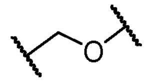, 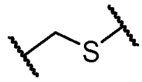, 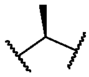,

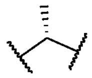, 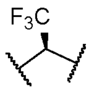, 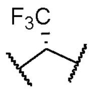, 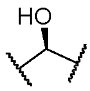, 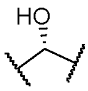, 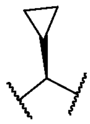, 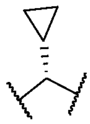, 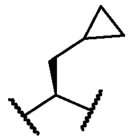, 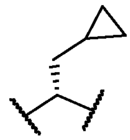, 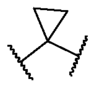,

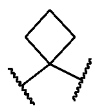, 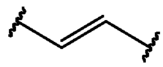 and 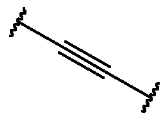.

**42.** The compound of formula IIa or IIb or the tautomer, the mesomer, the racemate, the enantiomer or the diastereoisomer thereof, or the mixture thereof, or the pharmaceutically acceptable salt thereof according to claim 41, wherein W is absent or W is

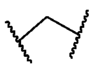.

**43.** The compound of formula IIa or IIb or the tautomer, the mesomer, the racemate, the enantiomer or the diastereoisomer thereof, or the mixture thereof, or the pharmaceutically acceptable salt thereof according to any one of claims 26-42, wherein B is absent or selected from the group consisting of:

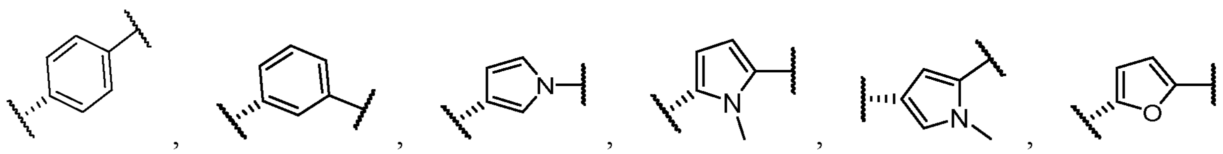, , , , , ,

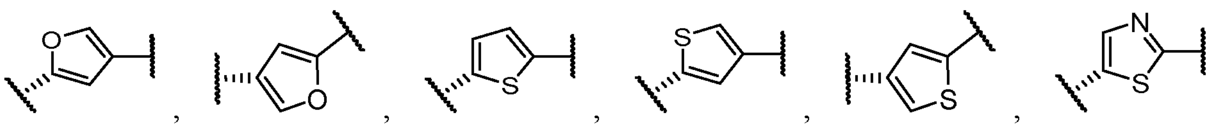, , , , ,

and

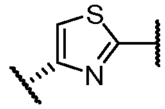;

W is absent or W is selected from the group consisting of:

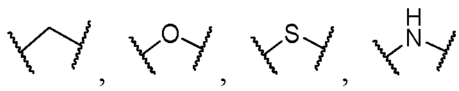, , , ,

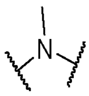, 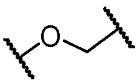, 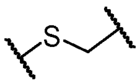, 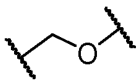, 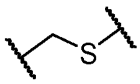, 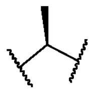, 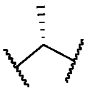, 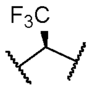, 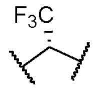,

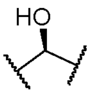, 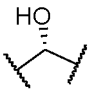, 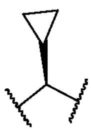, 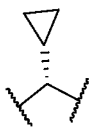, 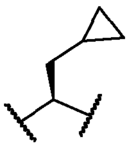, 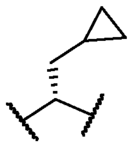, 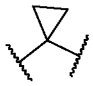 and 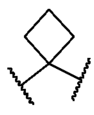;

A2 is selected from the group consisting of:

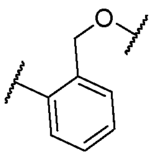, 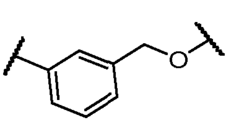, 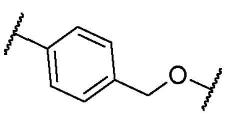, 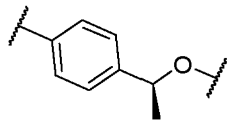, 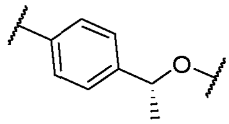,

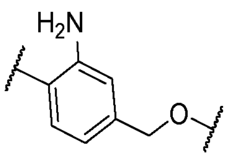, 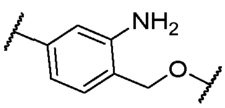, 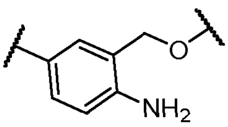, 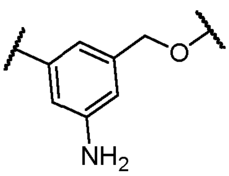, 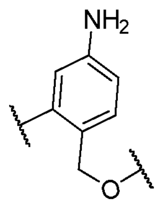,

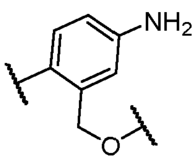, 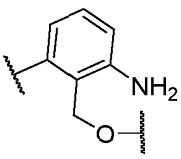, 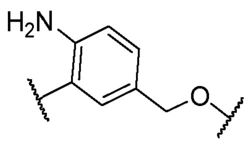, 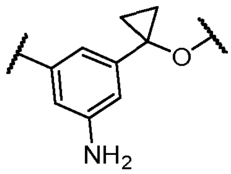, 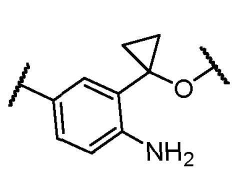,

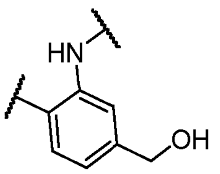, 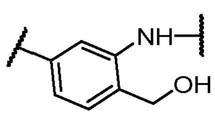, 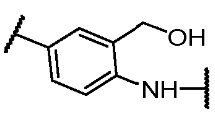, 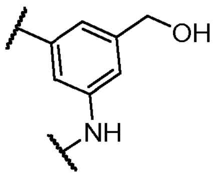, 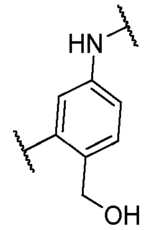,

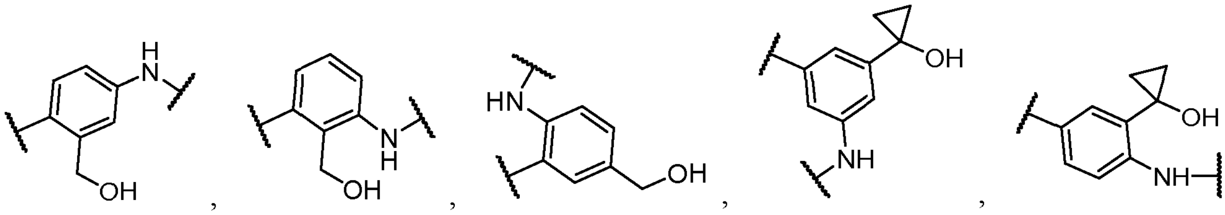, , , , ,

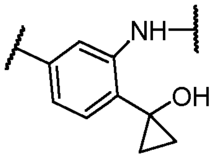, 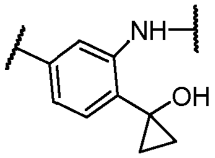, 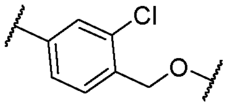, 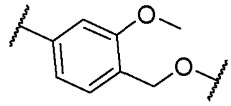, 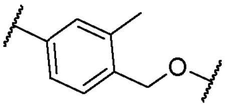,

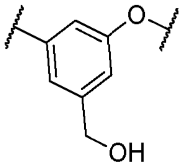, 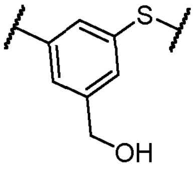, 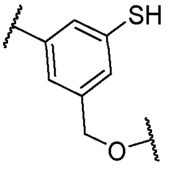, 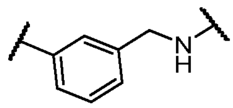, 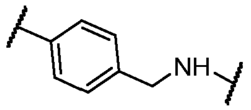,

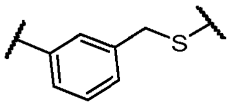, 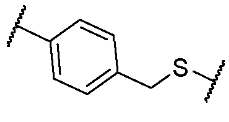, 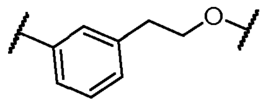, 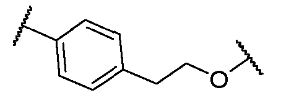,

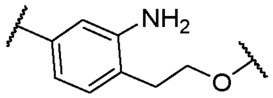, 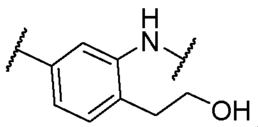, 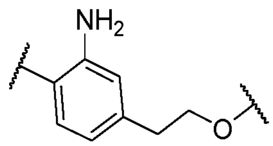 and 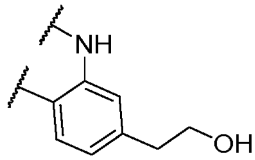.

**44.** The compound of formula IIa or IIb or the tautomer, the mesomer, the racemate, the enantiomer or the diastereoisomer thereof, or the mixture thereof, or the pharmaceutically acceptable salt thereof according to any one of claims 26-43, selected from the group consisting of:

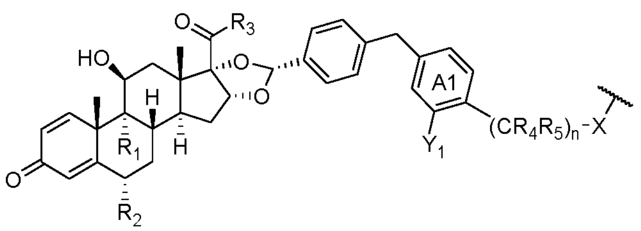

(IIa-1)

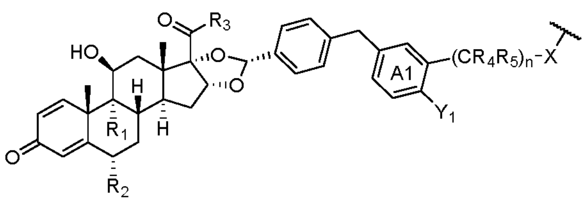

(IIb-1)

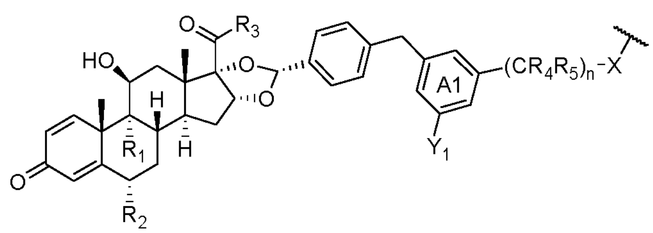

(IIc-1) ,

wherein,

X is selected from the group consisting of -O-, -S- and -NH-;
$R_4$ and $R_5$ are each independently selected from the group consisting of: H, F, Cl, -OH, $-NH_2$ and $C_1$-$C_6$ alkyl;
n is selected from the group consisting of 1, 2 and 3;
$Y_1$ is absent or selected from the group consisting of: hydroxy, sulfhydryl, amino and $C_1$-$C_6$ alkyl;
$R_1$ and $R_2$ are each independently selected from the group consisting of hydrogen, fluorine, chlorine and methyl;
$R_3$ is selected from the group consisting of: $-CH_2Cl$, $-CH_2SH$, $-CH_2OH$,

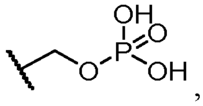

$-OCH_3$, $-OCH_2F$, $-OCH_2Cl$, $-OCH_2CN$, $-OCH_2CH_3$, $-SH$, $-SCH_2F$, $-SCH_2Cl$, $-SCH_2CF_3$ and $-SCH_2CN$.

**45.** The compound of formula IIa or IIb or the tautomer, the mesomer, the racemate, the enantiomer or the diastereoisomer thereof, or the mixture thereof, or the pharmaceutically acceptable salt thereof according to any one of claims 26-44, wherein the compound is selected from the group consisting of:

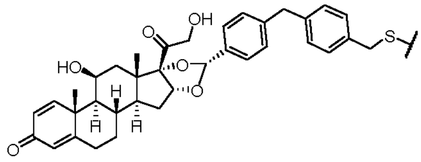
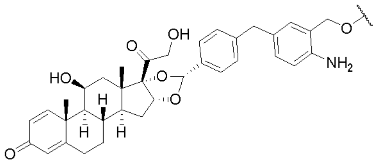
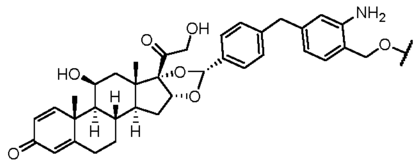
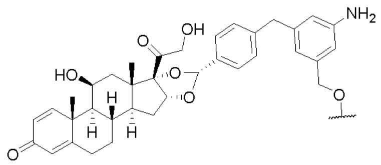
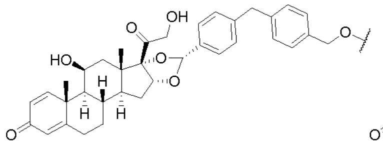
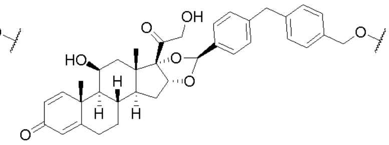
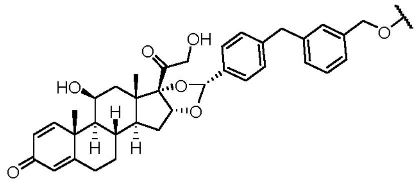
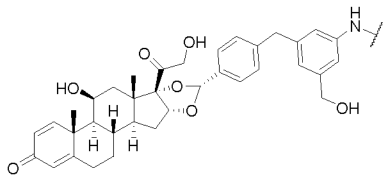
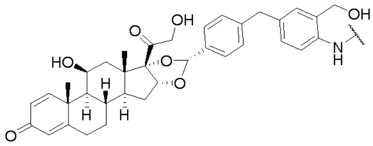
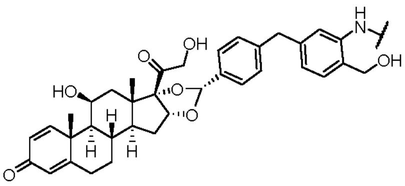

.

**46.** The compound of formula IIa or IIb or the tautomer, the mesomer, the racemate, the enantiomer or the diastereoisomer thereof, or the mixture thereof, or the pharmaceutically acceptable salt thereof according to any one of claims 26-45, wherein the compound further comprises a linker fragment, and the compound of formula IIa or lib is capable of being coupled to a ligand via the linker fragment, wherein the linker fragment comprises a $L_1$ fragment, a $L_2$ fragment and/or an $L_3$ fragment, and the compound has the following structure:

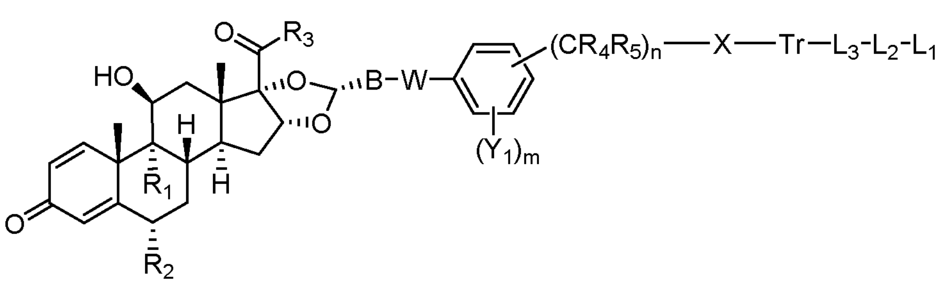

(IVa)

or

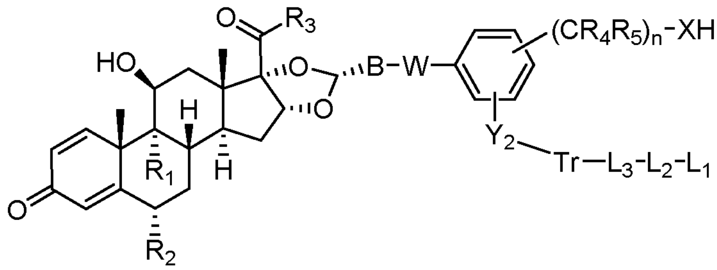

,

(IVb)

wherein,

Tr is absent or Tr is any group;
$L_3$ is selected from a polypeptide fragment;
$L_2$ is absent or selected from a linker fragment;
$L_1$ is selected from a coupling unit;
$R_1$, $R_2$, $R_3$, $R_4$ and $R_5$ are each independently any group;
B is absent or B is any group;
W is absent or W is any group;
$CR_4R_5$ units are each independently unreplaced or replaced by a group selected from the group consisting of: -O-, -S-, -NR-, -S(O)-, $-S(O)_2-$, -C(=O)-, -C=C- and -C=C-, wherein $R_4$ and $R_5$ can together form optionally substituted cycloalkyl, optionally substituted heterocyclyl, optionally substituted bridged cyclyl, optionally substituted bridged heterocyclyl, optionally substituted spiro cyclyl and optionally substituted spiro heterocyclyl, and n is any integer from 1 to 20;
X is selected from the group consisting of: -O-, -S- and -NR-;
$Y_1$ is any group, and m is any integer from 0 to 4;
$Y_2$ is selected from the group consisting of -O-, -S- and -NR-;
wherein substituents are selected from the group consisting of: protium, deuterium, tritium, halogen, -CN, =O, =N-OH, =N-OR, =N-R, -OR, -C(O)R, -C(O)OR, -OC(O)R, -OC(O)OR, -C(O)NHR, - $C(O)NR_2$, -OC(O)NHR, $-OC(O)NR_2$, -SR, -S(O)R, $-S(O)_2R$, -NHR, $-N(R)_2$, -NHC(O)R, -NRC(O)R, -NHC(O)OR, -NRC(O)OR, $-S(O)_2NHR$, $-S(O)_2N(R)_2$, $-NHS(O)_2NR_2$, $-NRS(O)_2NR_2$, $-NHS(O)_2R$, - $NRS(O)_2R$, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy; wherein each R is independently selected from the group consisting of hydrogen, protium, deuterium, tritium, oxygen, halogen, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy.

**47.** The compound of formula IIa or IIb or the tautomer, the mesomer, the racemate, the enantiomer or the diastereoisomer thereof, or the mixture thereof, or the pharmaceutically acceptable salt thereof according to claim 46, wherein Tr is absent or selected from the group consisting of the following structures:

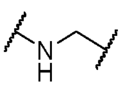 , 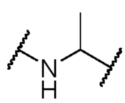 , 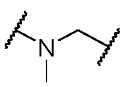 , 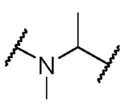 , 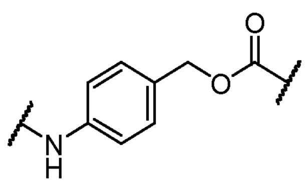 ,

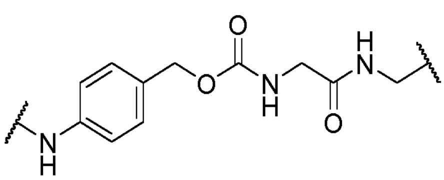 , 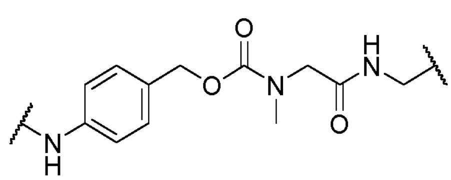 ,

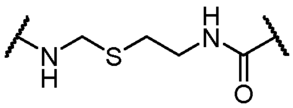,
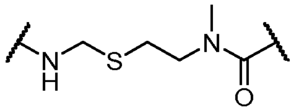,
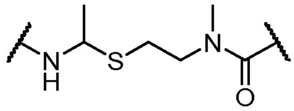,

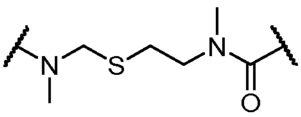 and 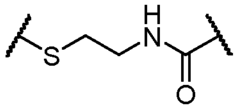.

**48.** The compound of formula IIa or IIb or the tautomer, the mesomer, the racemate, the enantiomer or the diastereoisomer thereof, or the mixture thereof, or the pharmaceutically acceptable salt thereof according to claim 47, wherein $L_3$ is selected from the group consisting of a dipeptide, a tripeptide, and a tetrapeptide, wherein

the dipeptide is selected from the group consisting of: GA, GG, AG, EG, EA, GE, DG, DA, GD, VC, VA, AA and VK,
the tripeptide is selected from the group consisting of: EAG, EGG, GEG, GEA, DAG, DGG, GDG, GDA, GGA, GAG, GFG, AAG, AAA, VAG, VCG and VKG, and
the tetrapeptide is selected from the group consisting of: GGFG, GGAG, GGGG, GEGG, GEAG, GDGG, GDAG, AAAG and EAGG.

**49.** The compound of formula IIa or IIb or the tautomer, the mesomer, the racemate, the enantiomer or the diastereoisomer thereof, or the mixture thereof, or the pharmaceutically acceptable salt thereof according to claim 48, wherein $L_3$ is selected from the group consisting of: glycine-glycine-phenylalanine-glycine (GGFG), alanine-alanine-alanine-glycine (AAAG), glycine-glycine-glycine-glycine (GGGG), valine-alanine-glycine (VAG), valine-citrulline-glycine (VCG), alanine-alanine-glycine (AAG), alanine-alanine-alanine (AAA), valine-alanine (VA), valine-citrulline (VC), alanine-alanine (AA), glutamic acid-alanine-glycine-glycine (EAGG), glycine-glutamic acid-alanine-glycine (GEAG), glycine-glutamic acid-glycine-glycine (GEGG), glutamic acid-glycine-glycine (EGG), glutamic acid-alanine-glycine (EAG), valine-lysine-glycine (VKG), glycine-glutamic acid-glycine (GEG), glutamic acid-alanine (EA), glutamic acid-glycine (EG) and glycine-glutamic acid (GE).

**50.** The compound of formula IIa or IIb or the tautomer, the mesomer, the racemate, the enantiomer or the diastereoisomer thereof, or the mixture thereof, or the pharmaceutically acceptable salt thereof according to any one of claims 46-49, wherein $L_2$ is absent, or $L_2$ comprises or does not comprise a PEG branch chain or a PEG linear chain.

**51.** The compound of formula IIa or IIb or the tautomer, the mesomer, the racemate, the enantiomer or the diastereoisomer thereof, or the mixture thereof, or the pharmaceutically acceptable salt thereof according to claim 50, wherein

(1) when $L_2$ does not comprise PEG, $L_2$ is selected from the group consisting of:

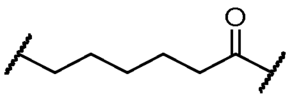, 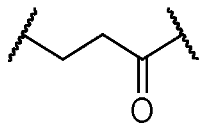 and 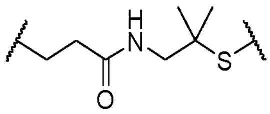;

(2) when $L_2$ comprises the PEG linear chain, $L_2$ is selected from the group consisting of:

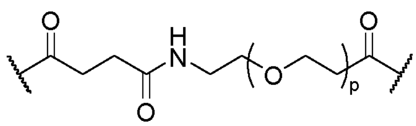,
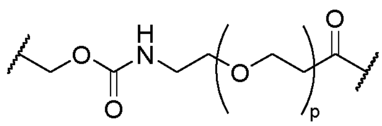,
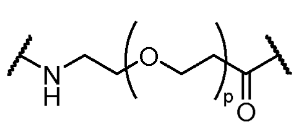,

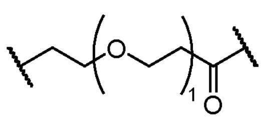,
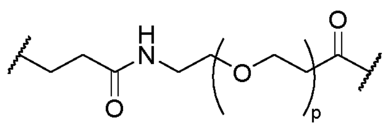 and

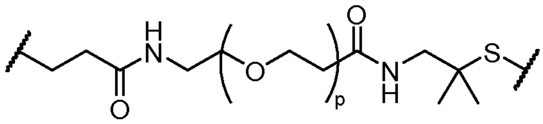,

wherein p is any integer from 1 to 20;

(3) when $L_2$ comprises the PEG branch chain, $L_2$ is selected from the group consisting of:

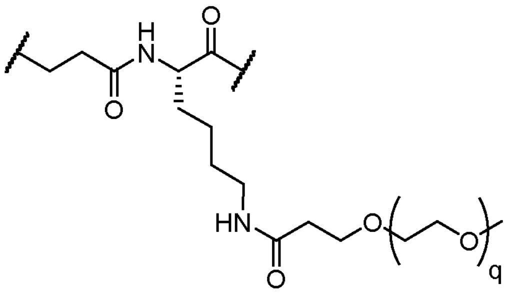,

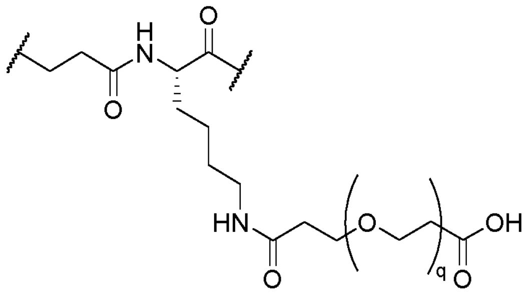,

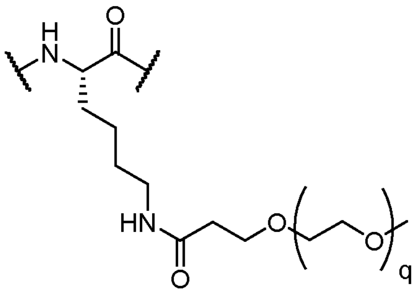,

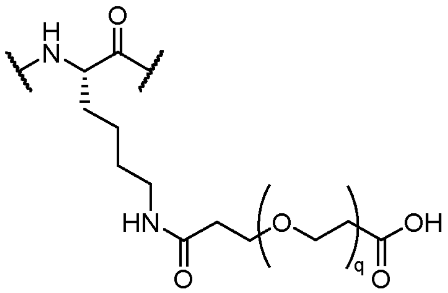,

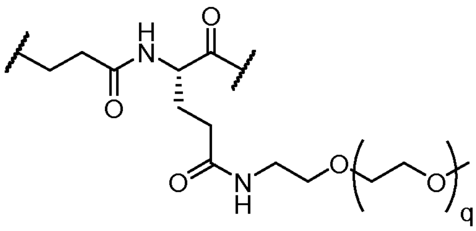,

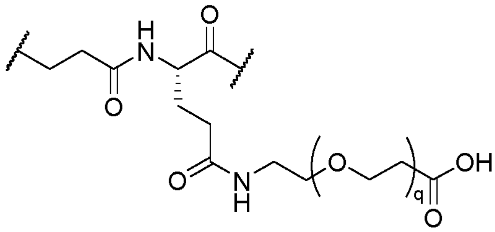,

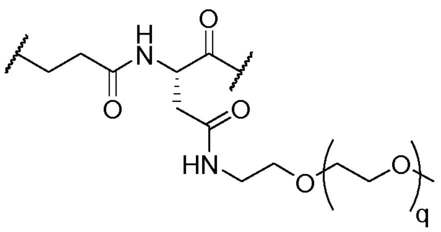,

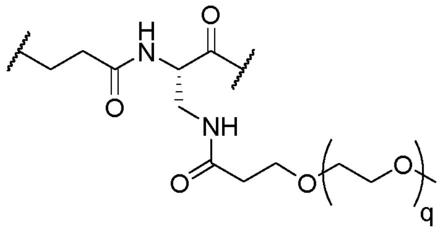,

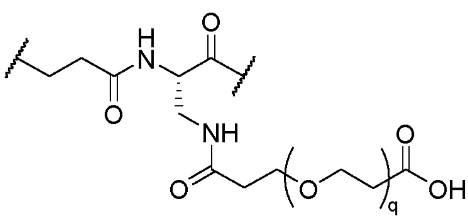,

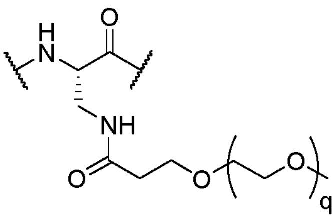,

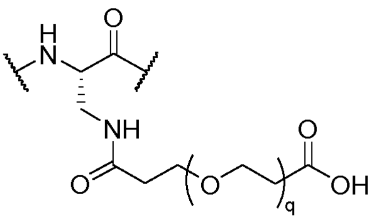,

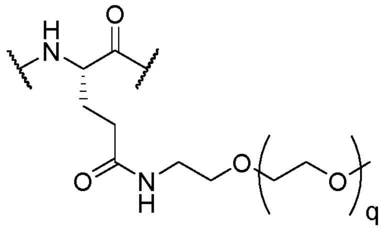 , 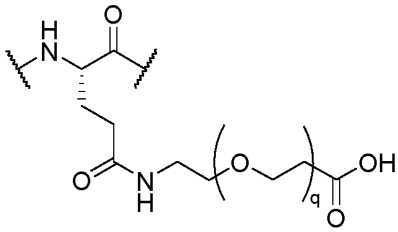 ,

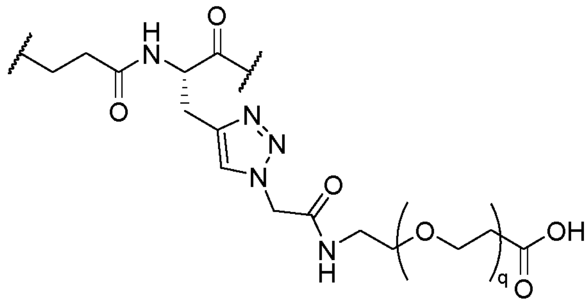 , 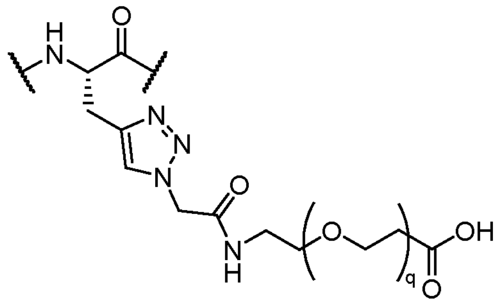 ,

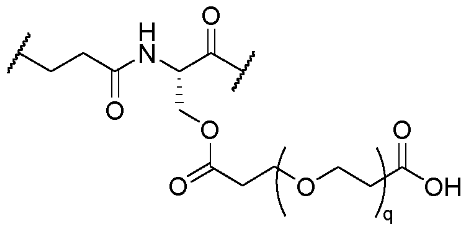 , 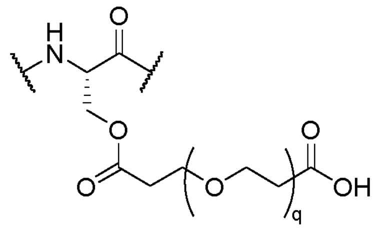 ,

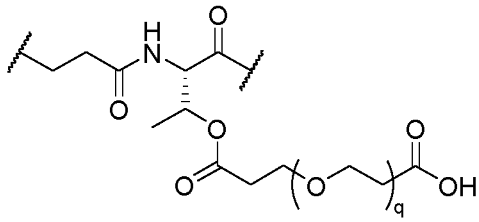 and 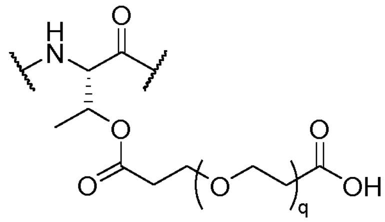 ,

wherein q is selected from any integer from 1 to 30.

**52.** The compound of formula IIa or IIb or the tautomer, the mesomer, the racemate, the enantiomer or the diastereoisomer thereof, or the mixture thereof, or the pharmaceutically acceptable salt thereof according to claim 51, wherein

(1) when $L_2$ comprises the PEG linear chain, $L_2$ is selected from the group consisting of:

| |
|---|
| 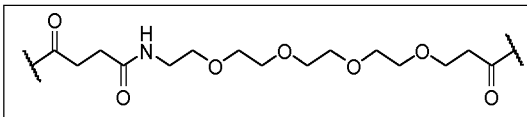 |
| 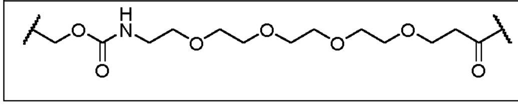 |

| |
|---|
| 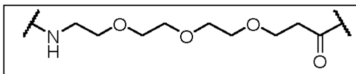 |
| 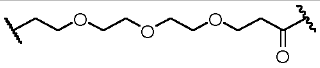 |
| 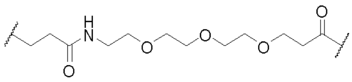 |
| 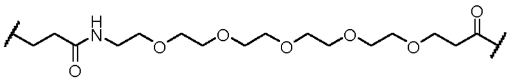 |
| 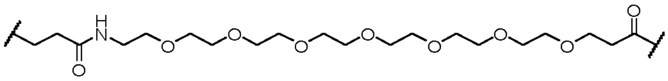 |
| 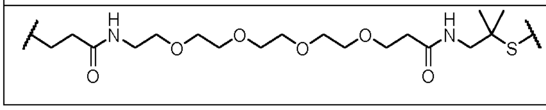 |

(2) when $L_2$ comprises the PEG branch chain, $L_2$ is selected from the group consisting of:

| |
|---|
| 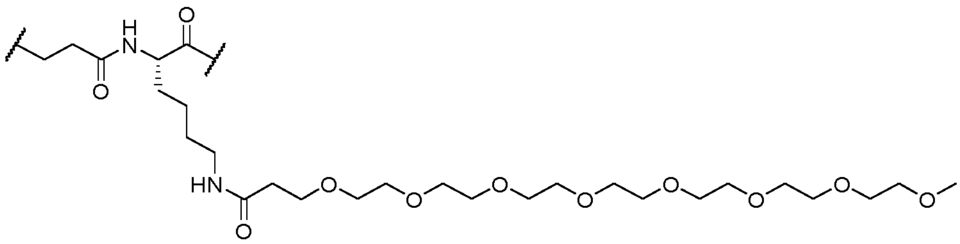 PEG8 |
| 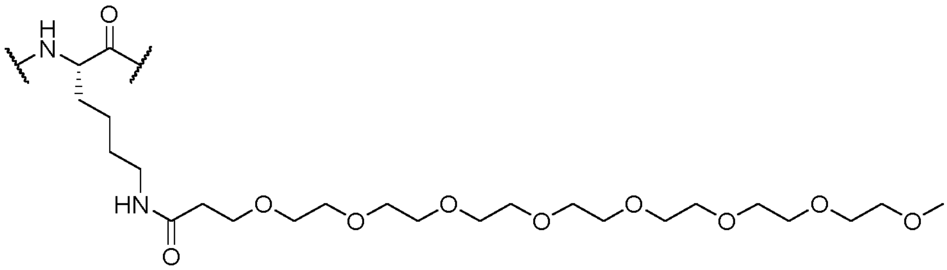 PEG8 |
| 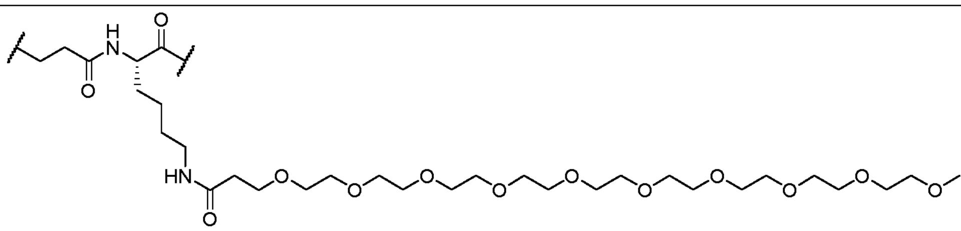 PEG10 |
| 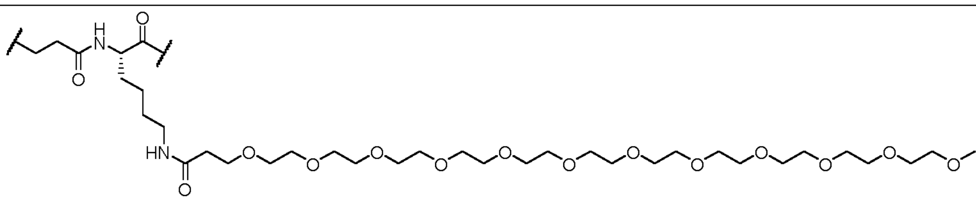 PEG12 |

| |
|---|
| 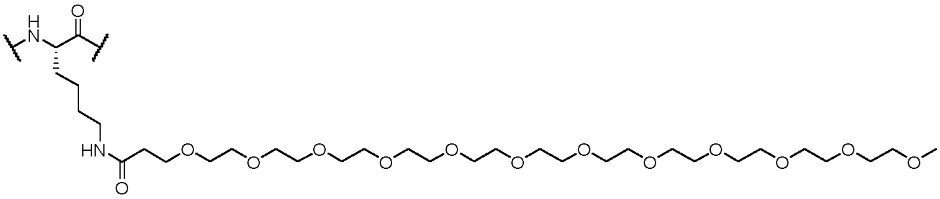 PEG12 |
| 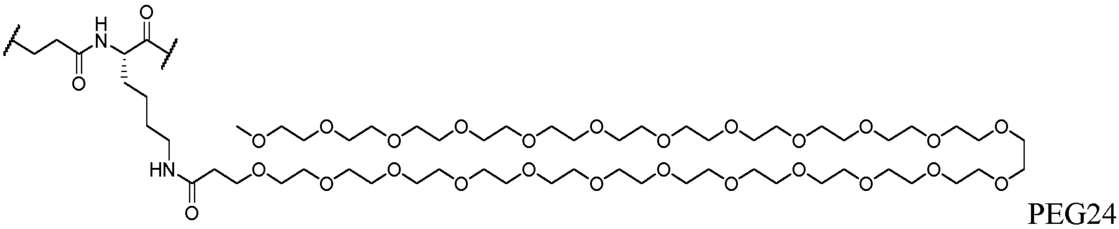 PEG24 |
| 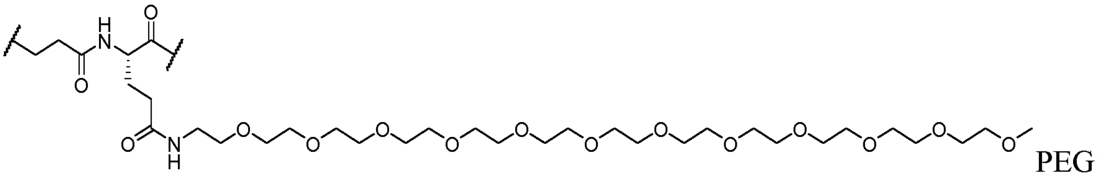 PEG12 |
| 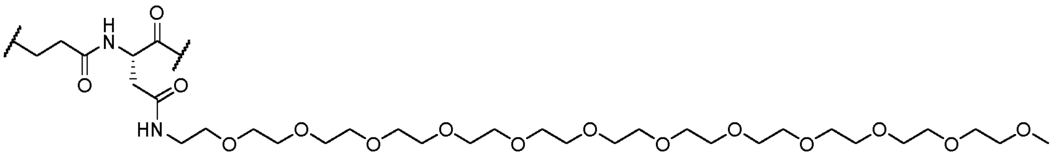 PEG12 |
| 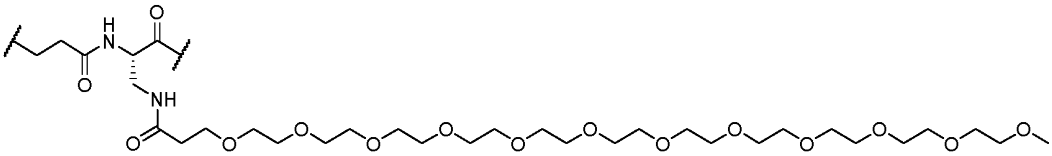 PEG12 |
| 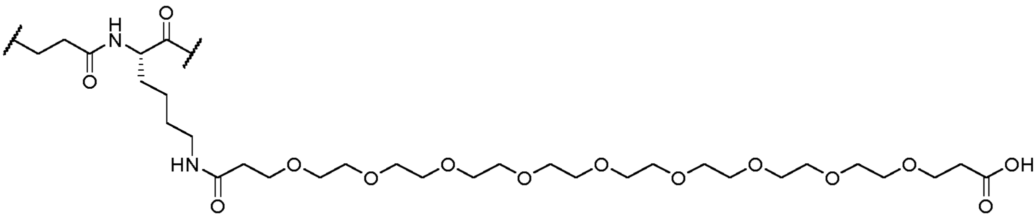 PEG10 |
| 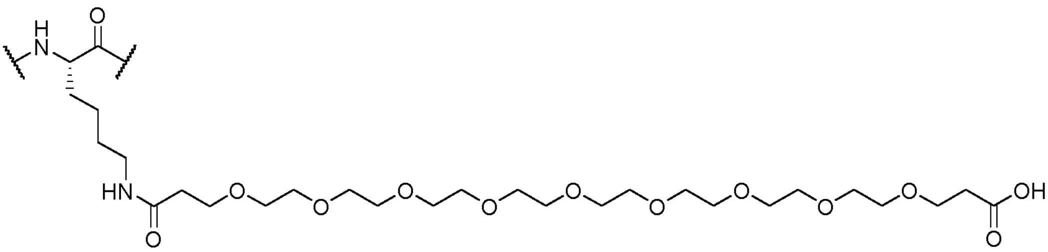 PEG10 |
| 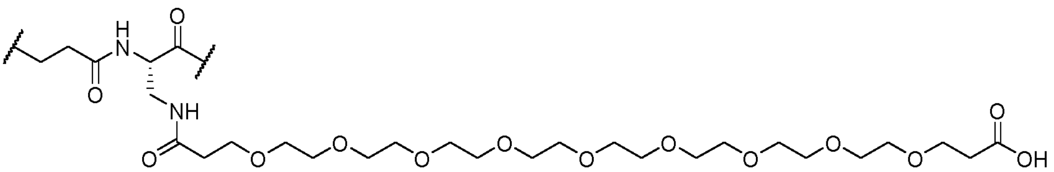 PEG10 |

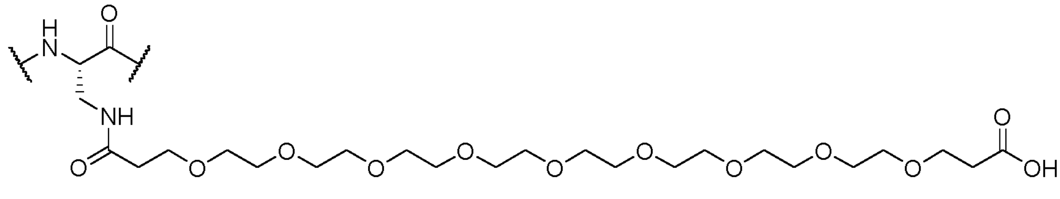

PEG10

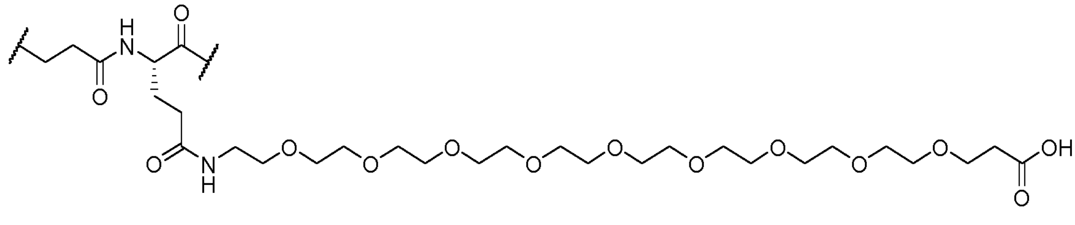

PEG10

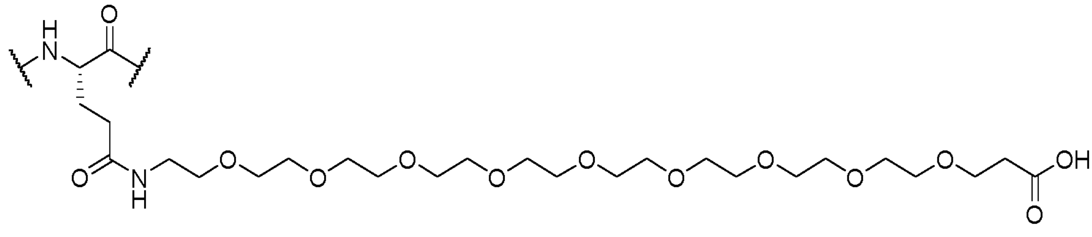

PEG10

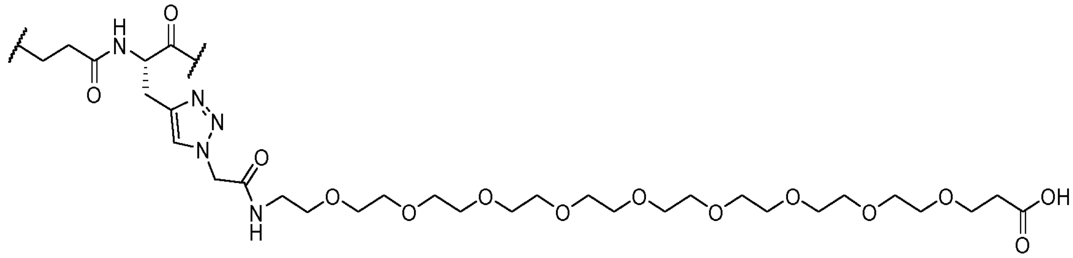

PEG10

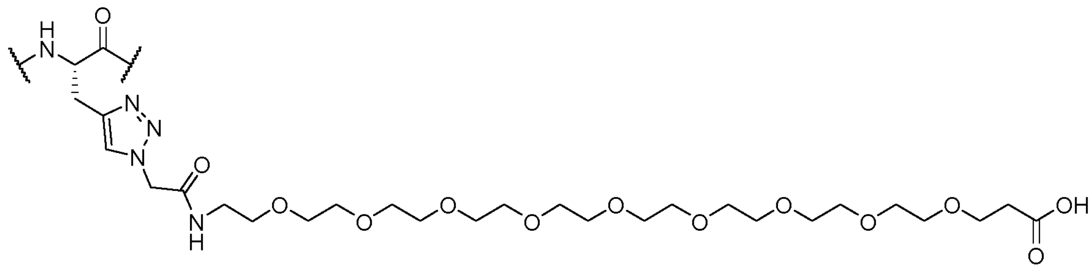

PEG10

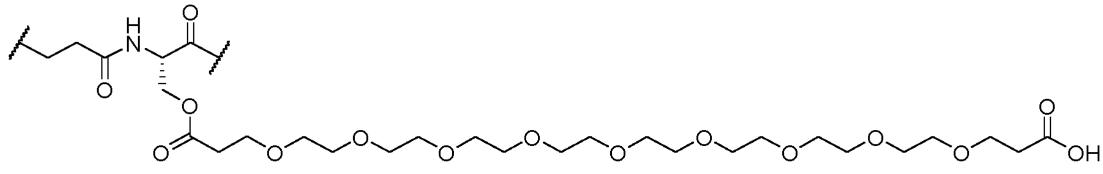

PEG10

| |
|---|
| 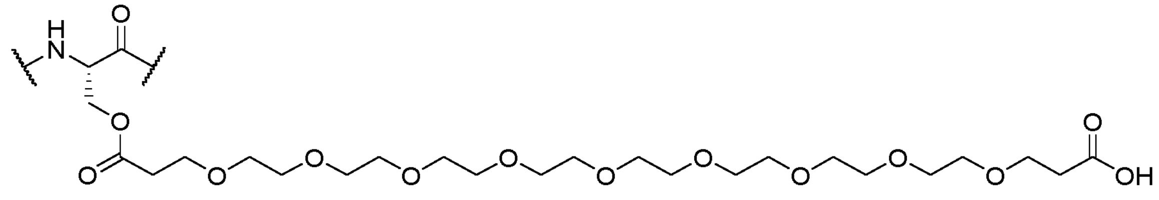<br>PEG10 |
| 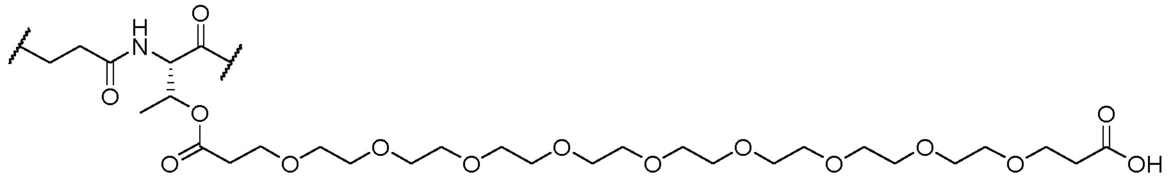<br>PEG10 |
| 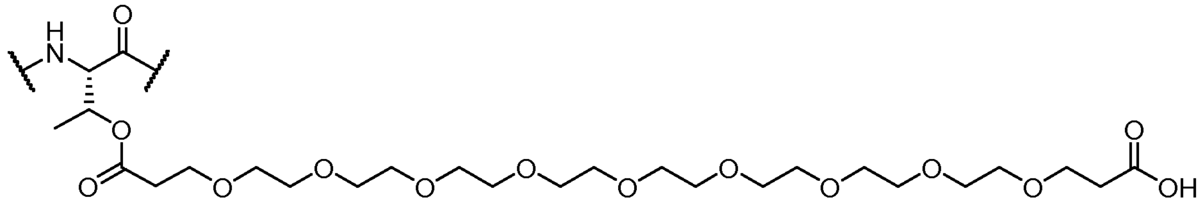<br>PEG10 |

**53.** The compound of formula IIa or IIb or the tautomer, the mesomer, the racemate, the enantiomer or the diastereoisomer thereof, or the mixture thereof, or the pharmaceutically acceptable salt thereof according to any one of claims 46-52, wherein,

(1) when $L_1$ is coupled to the ligand via sulfhydryl, $L_1$ is selected from the group consisting of:

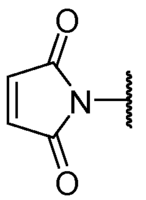 , 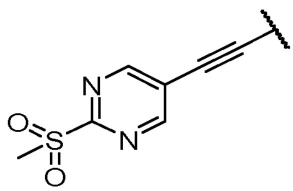 , 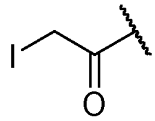 , 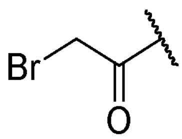 , 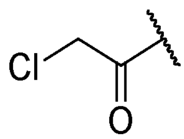 ,

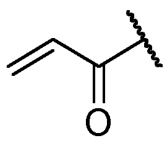 , 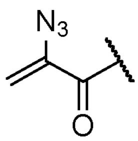 , 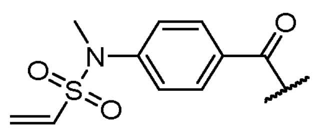 , 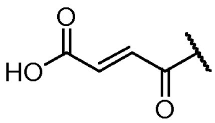 , 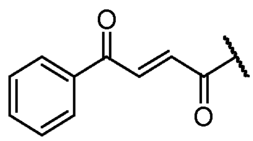 ,

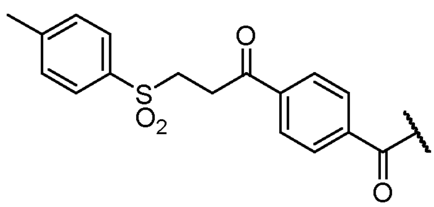 , 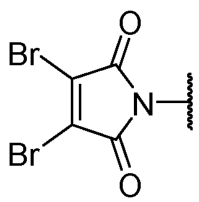 , 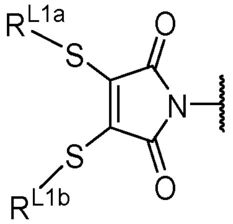 , 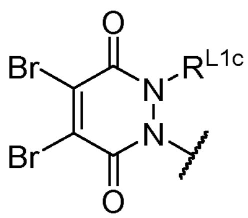 ,

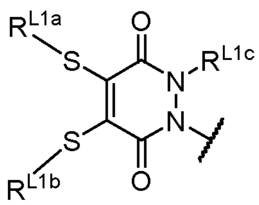
,
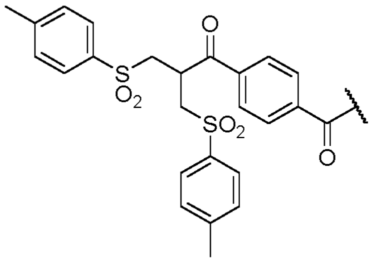
,
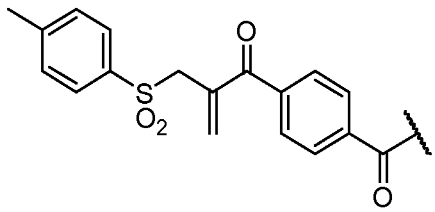

and

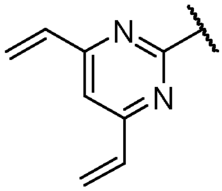
;

wherein, $R^{L1a}$, $R^{L1b}$ and $R^{L1c}$ are each independently selected from the group consisting of: hydrogen, optionally substituted methyl, optionally substituted ethyl, optionally substituted aryl and optionally substituted benzyl;

(2) when $L_1$ is coupled to the ligand via amino, $L_1$ is selected from the group consisting of:

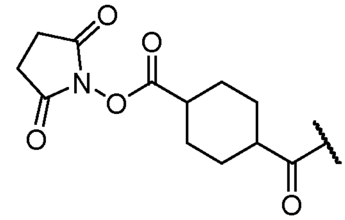
,
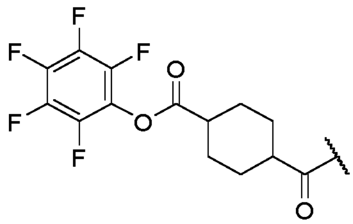
and
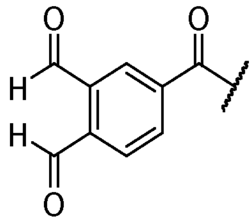
;

(3) when $L_1$ is coupled via click chemistry, $L_1$ is selected from the group consisting of:

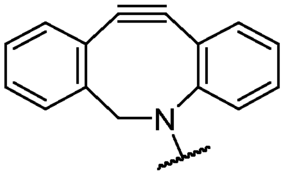
,
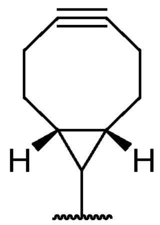
,
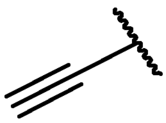
and
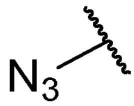
.

**54.** The compound of formula IIa or IIb or the tautomer, the mesomer, the racemate, the enantiomer or the diastereoisomer thereof, or the mixture thereof, or the pharmaceutically acceptable salt thereof according to any one of claims 46-53, wherein the compound of formula IVa or IVb is selected from the group consisting of the following structures:

| No. | Structure |
| --- | --- |
| IV-1 | 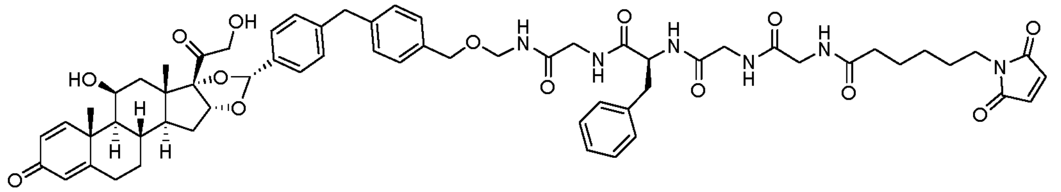 |

(continued)

| No. | Structure |
|---|---|
| IV-2 | 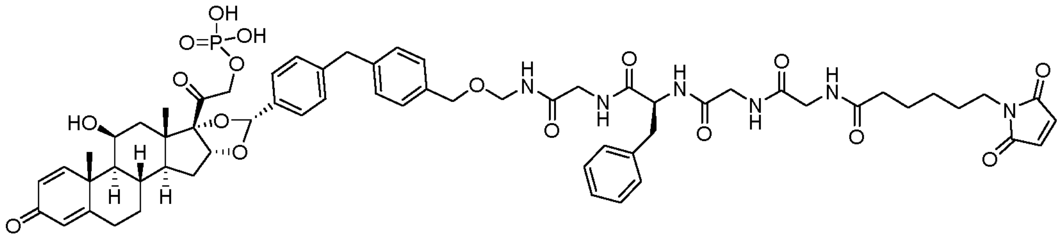 |
| IV-6 | 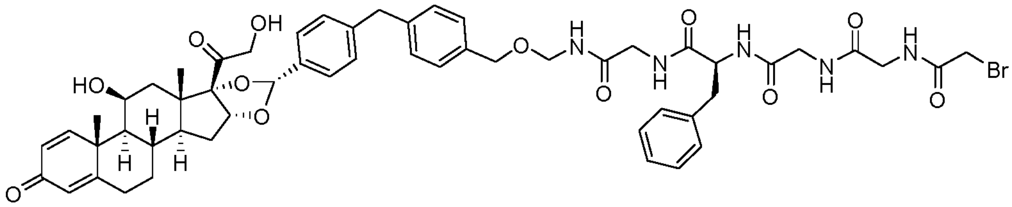 |
| IV-7 | 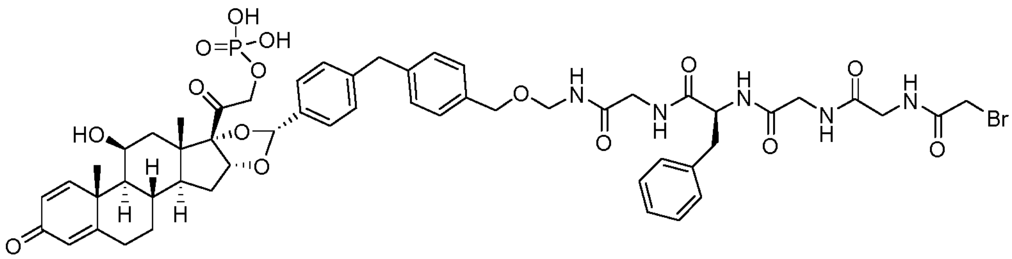 |
| IV-11 | 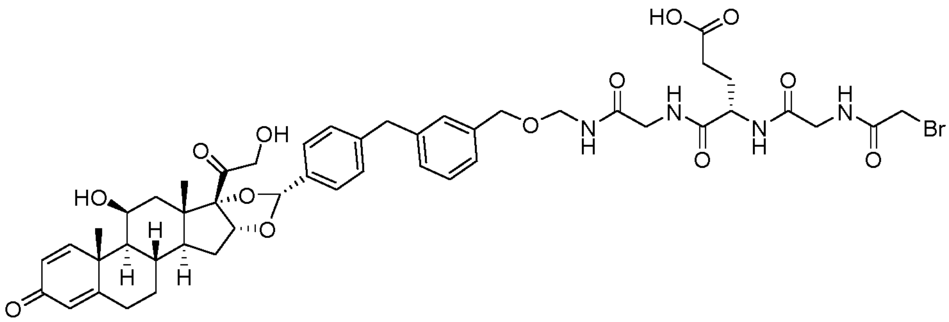 |
| IV-12 | 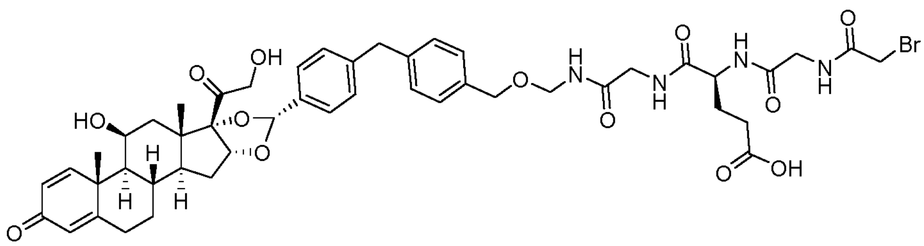 |

(continued)

| No. | Structure |
|---|---|
| IV-13 | 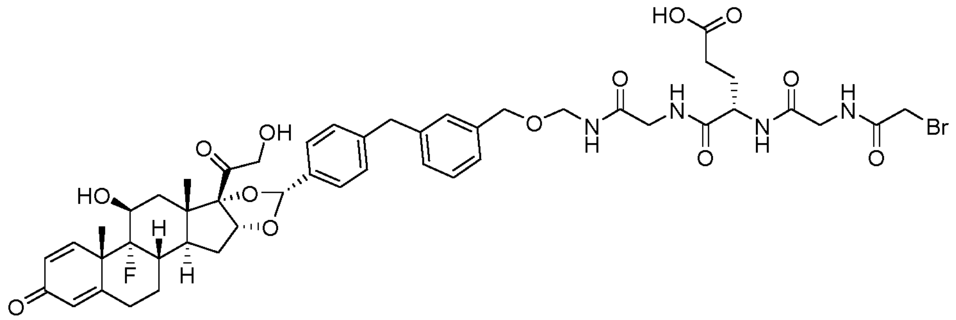 |
| IV-14 | 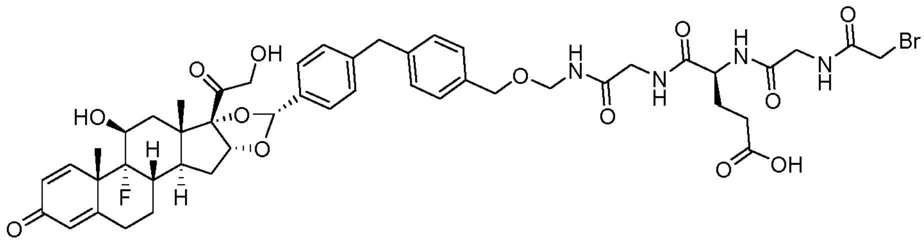 |
| IV-15 | 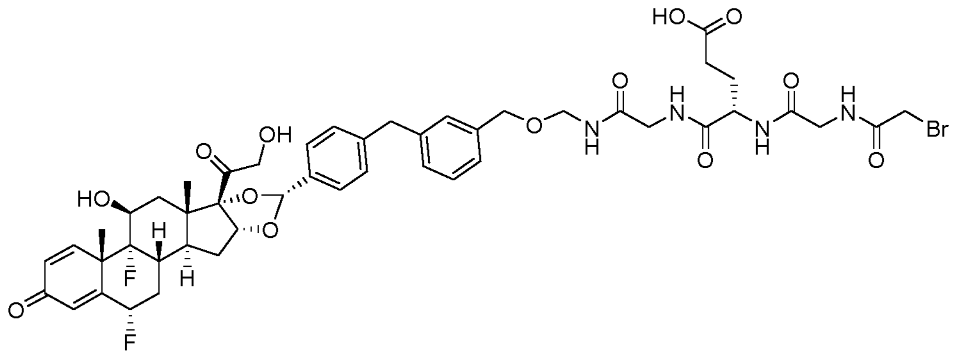 |
| IV-16 | 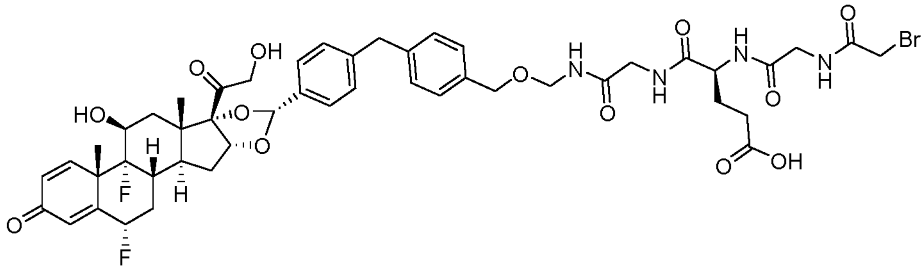 |
| IV-17 | 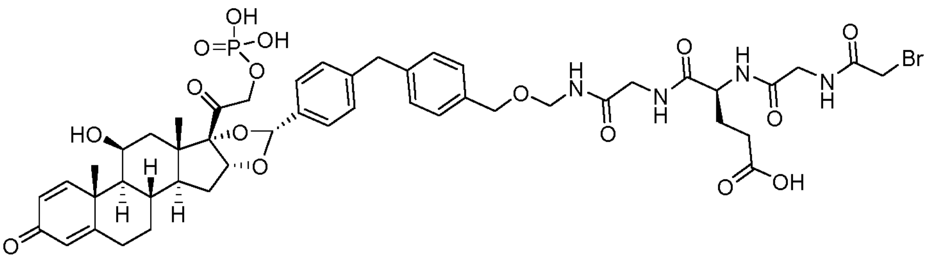 |

(continued)

| No. | Structure |
|---|---|
| IV-21 | 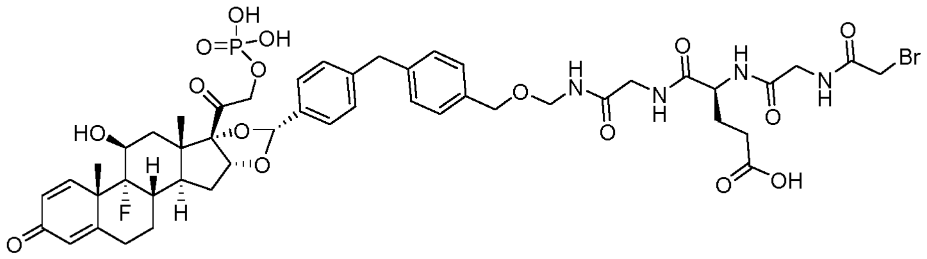 |
| IV-25 | 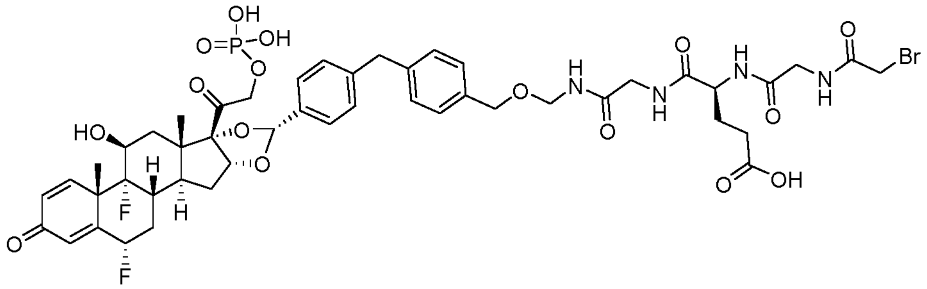 |
| IV-29 | 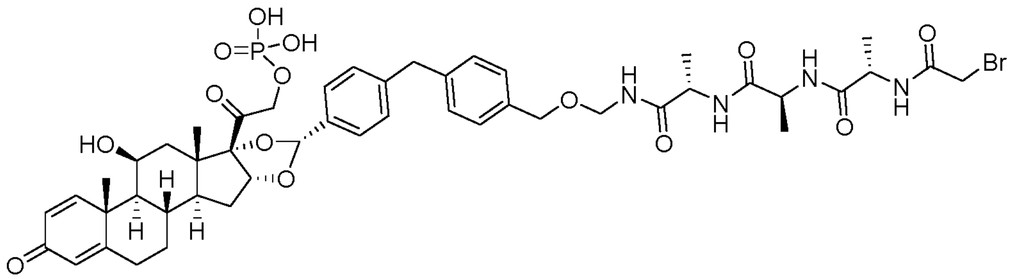 |
| IV-30 | 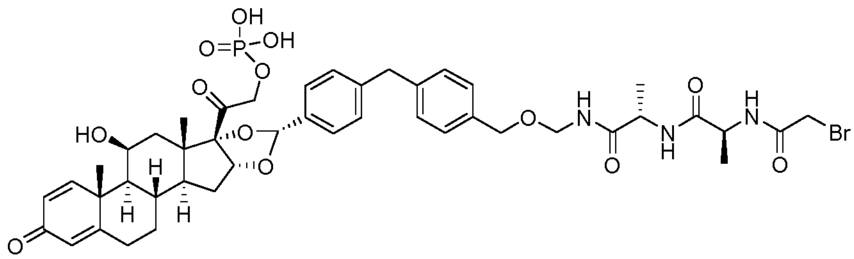 |
| IV-34 | 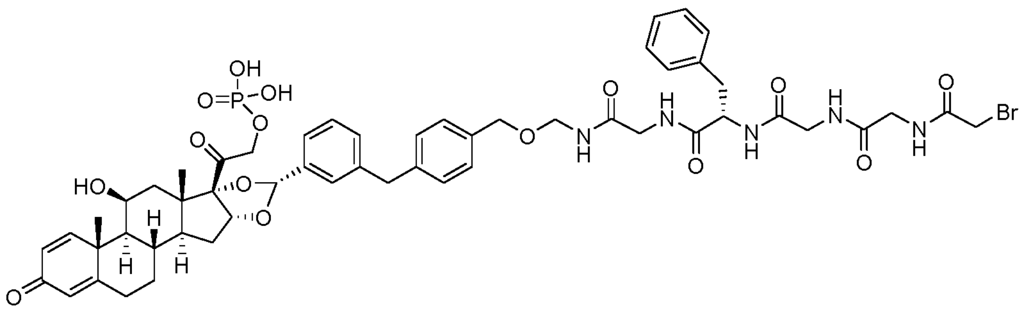 |

(continued)

| No. | Structure |
|---|---|
| IV-35 | 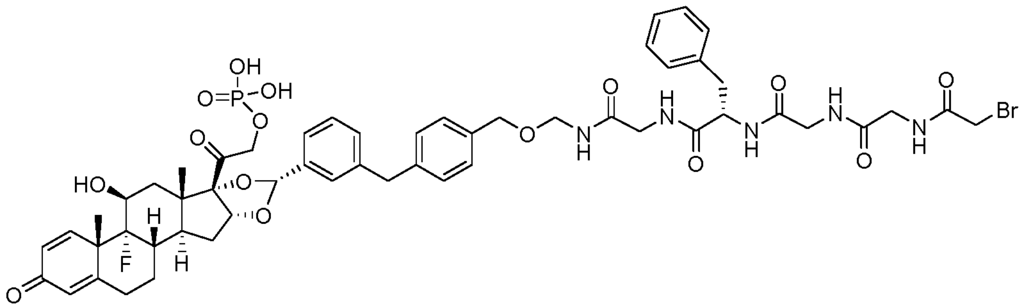 |
| IV-39 | 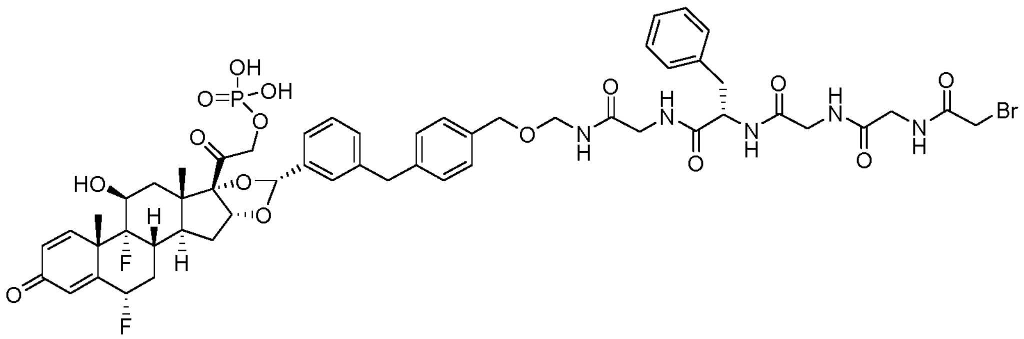 |
| IV-43 | 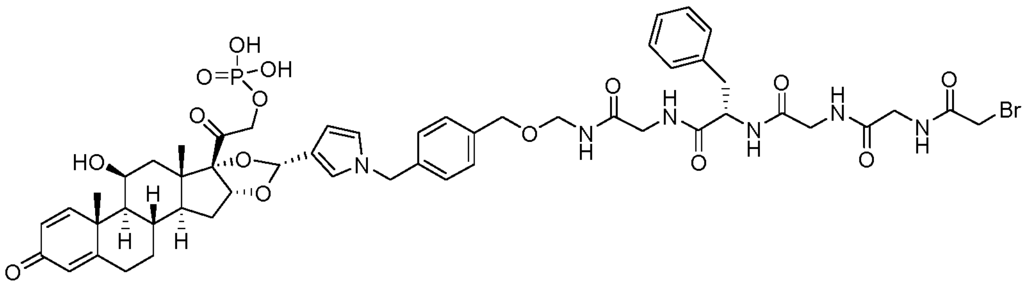 |
| IV-44 | 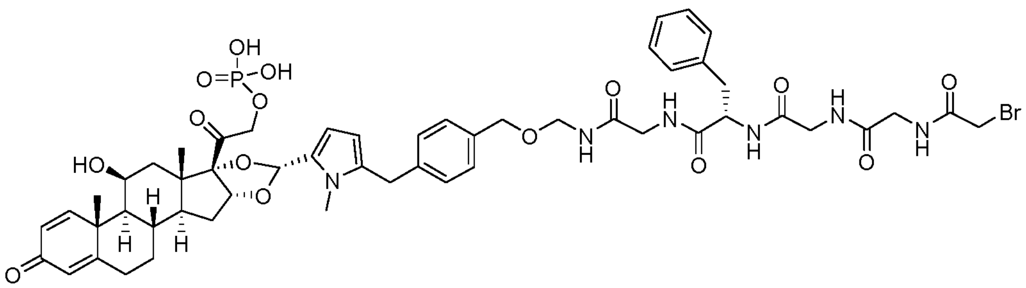 |
| IV-45 | 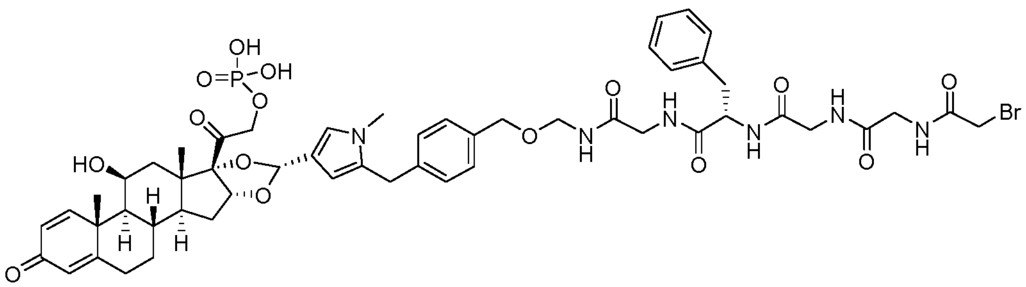 |

(continued)

| No. | Structure |
|---|---|
| IV-46 | 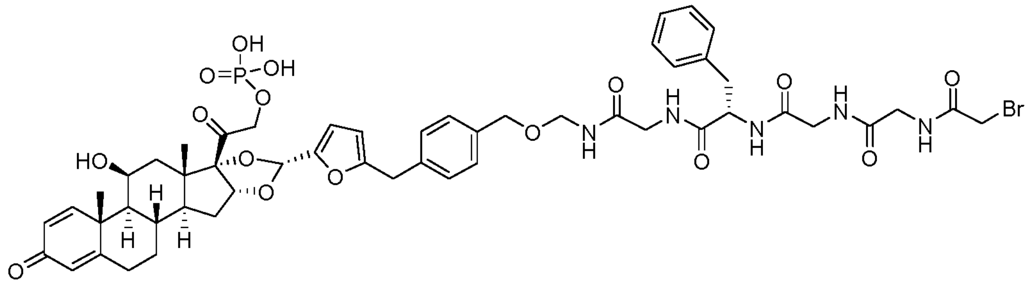 |
| IV-47 | 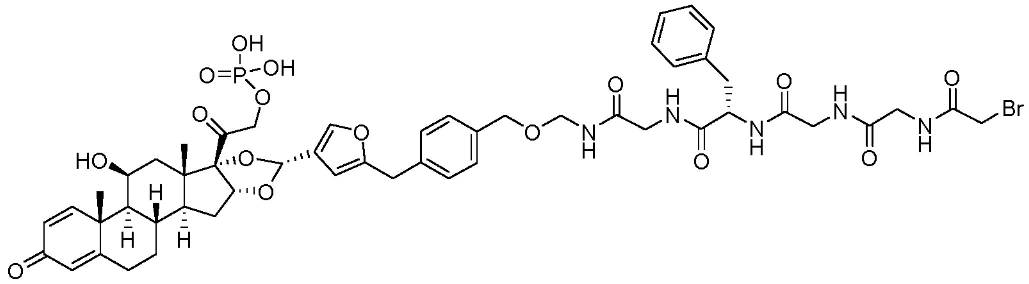 |
| IV-48 | 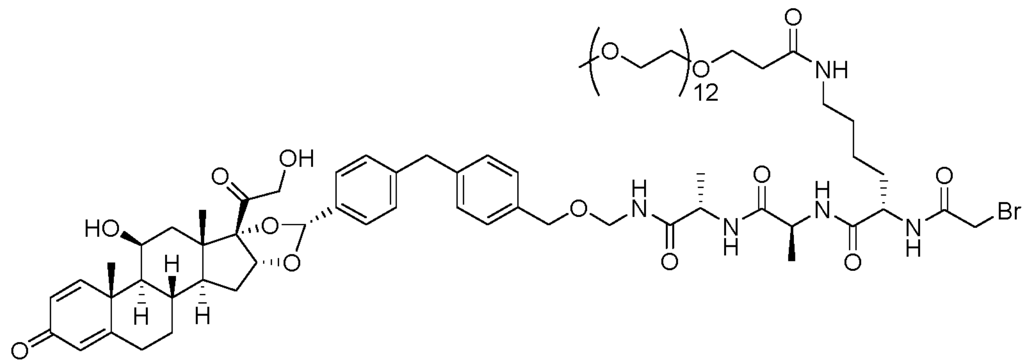 |
| IV-49 | 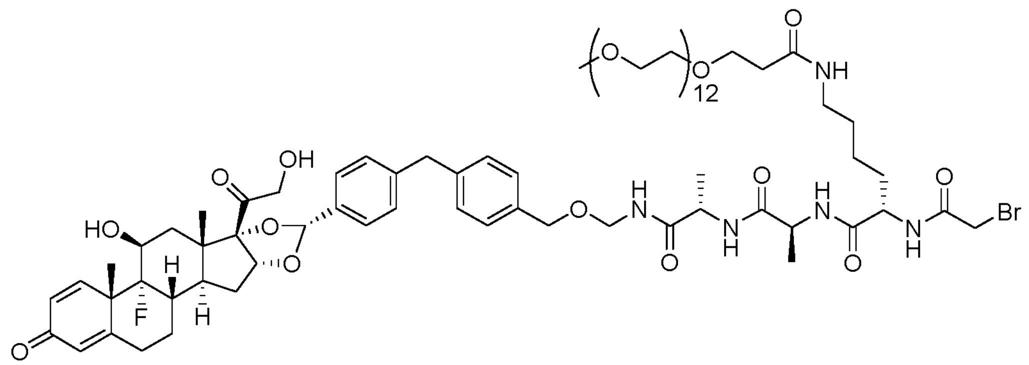 |
| IV-50 | 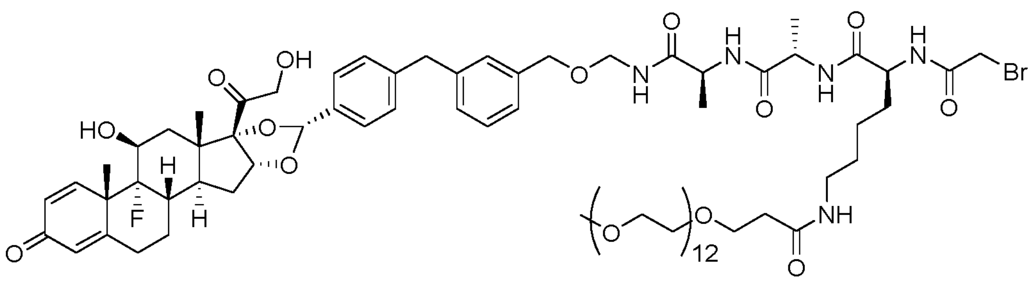 |

(continued)

| No. | Structure |
|---|---|
| IV-51 | 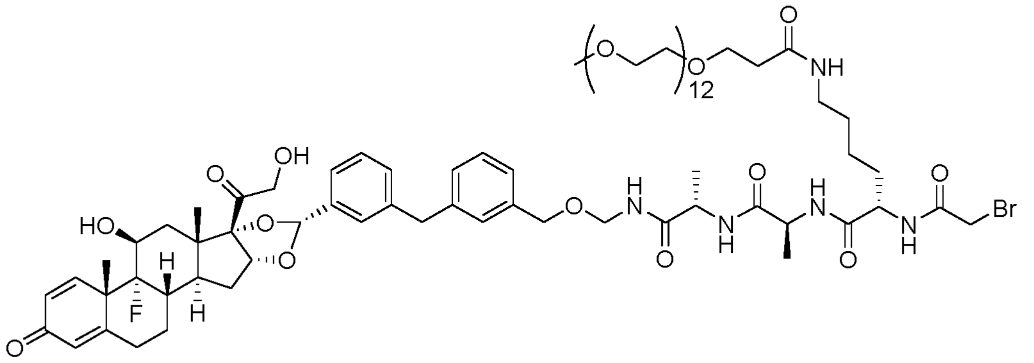 |
| IV-52 | 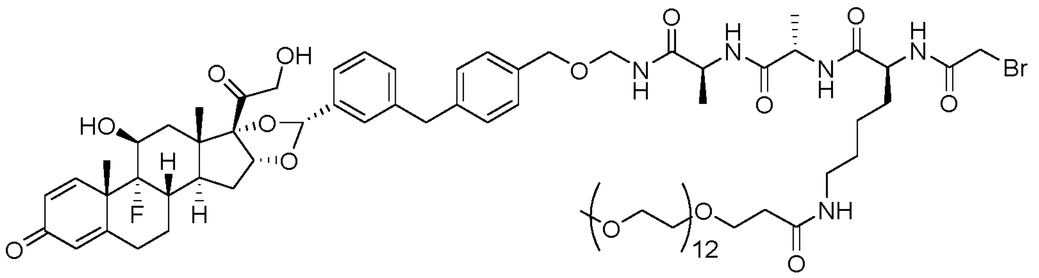 |
| IV-53 | 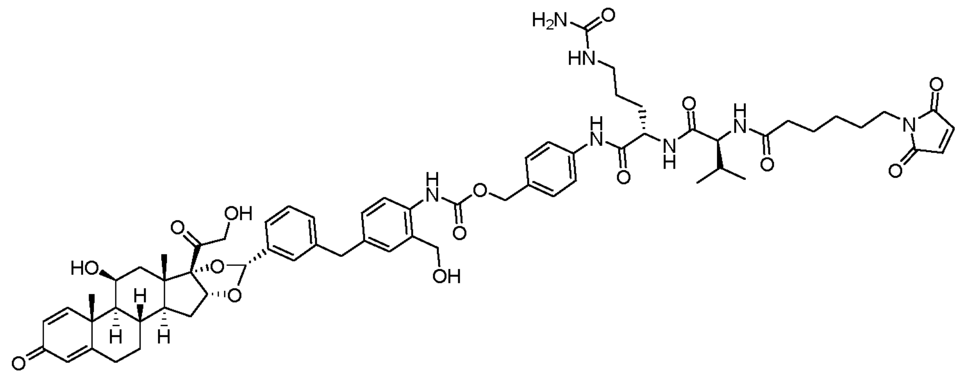 |
| IV-54 | 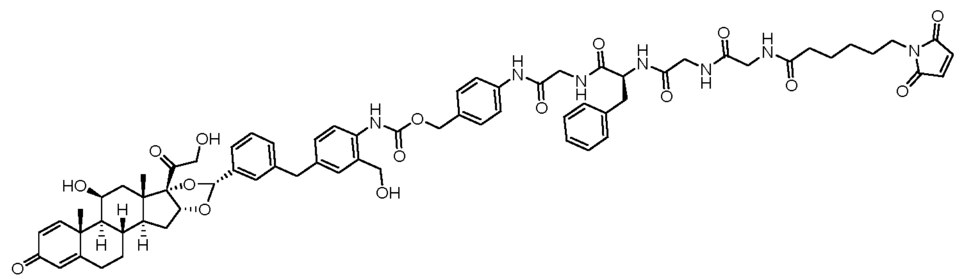 |
| IV-55 | 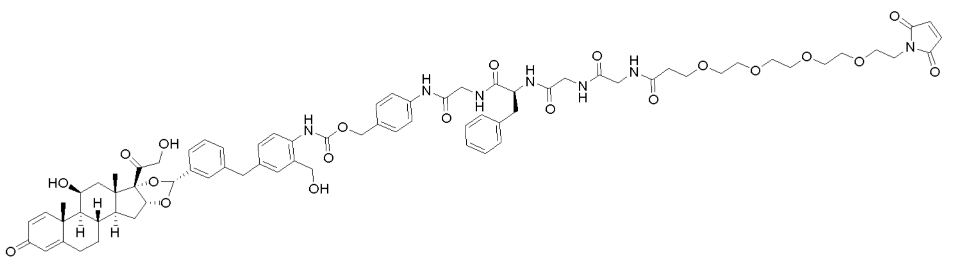 |

(continued)

| No. | Structure |
|---|---|
| IV-56 | 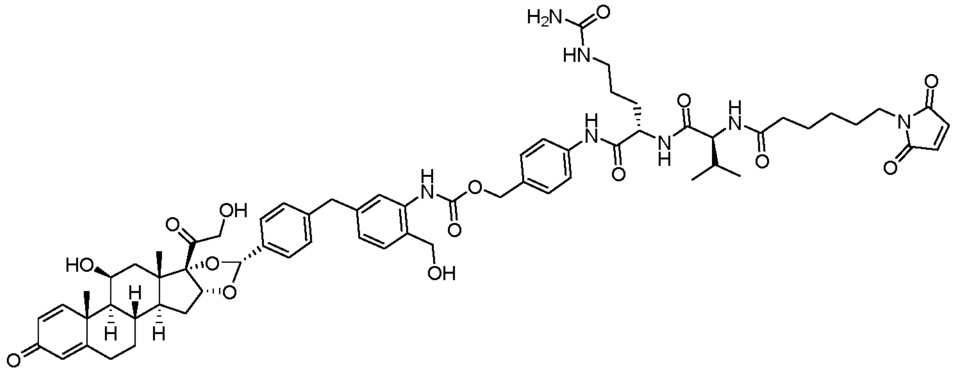 |
| IV-57 | 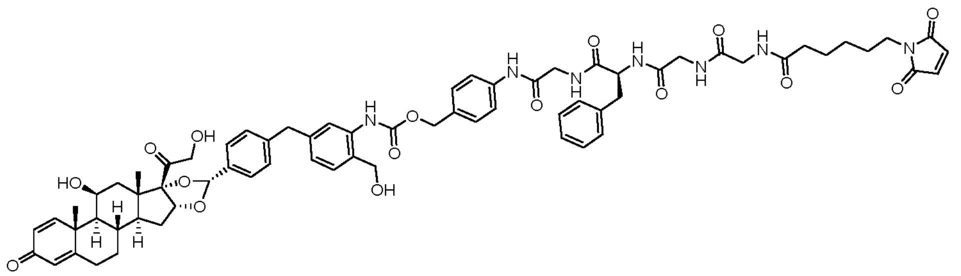 |
| IV-58 | 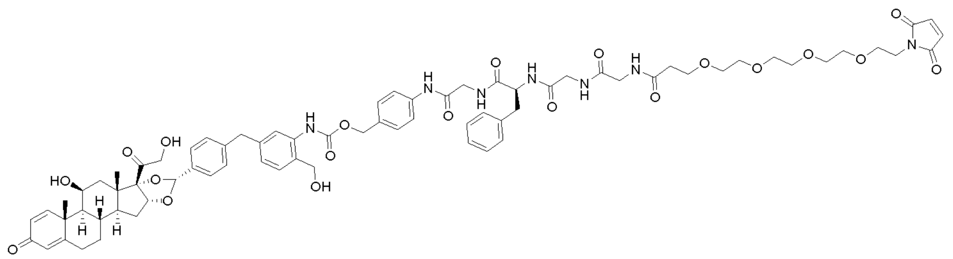 |
| IV-59 | 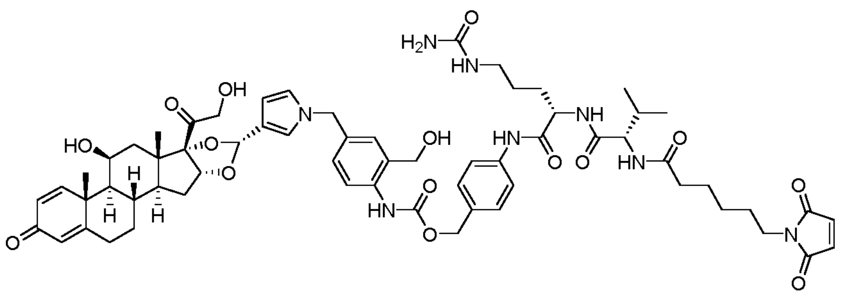 |
| IV-60 | 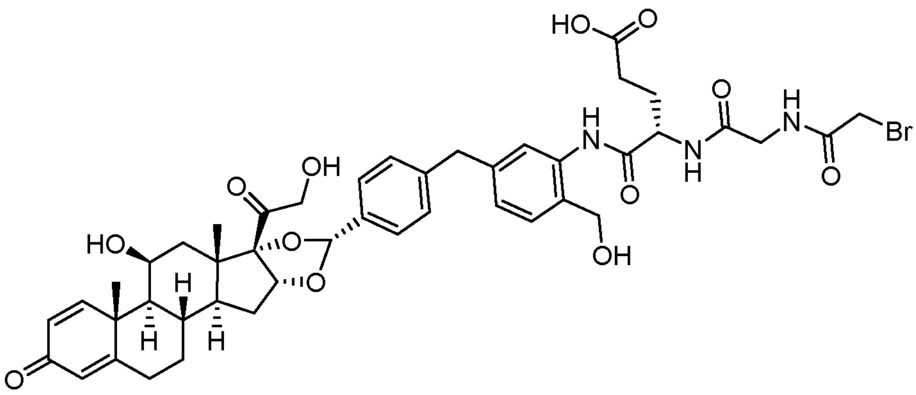 |

(continued)

| No. | Structure |
|---|---|
| IV-61 | 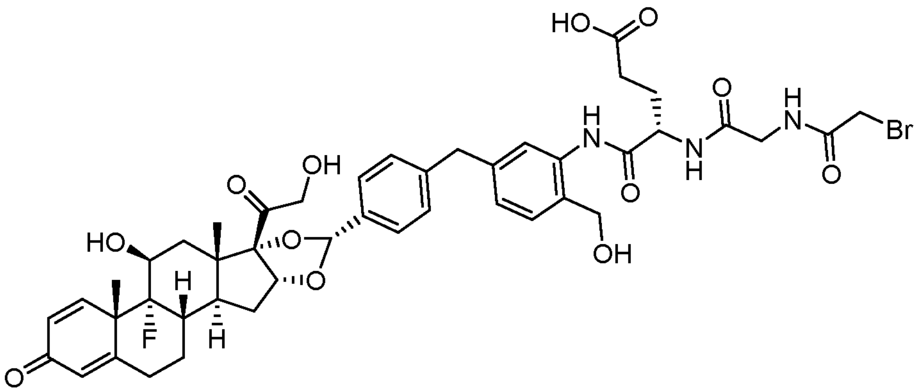 |
| IV-62 | 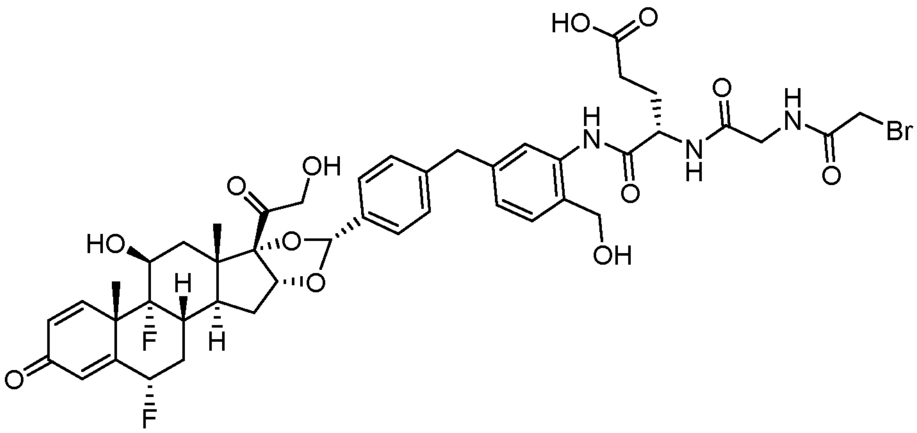 |
| IV-63 | 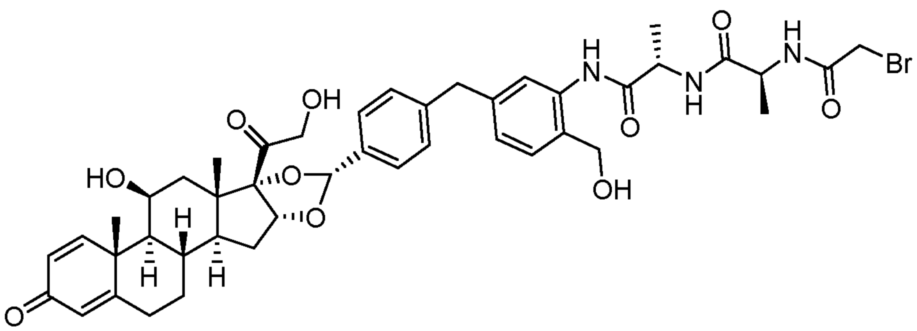 |
| IV-64 | 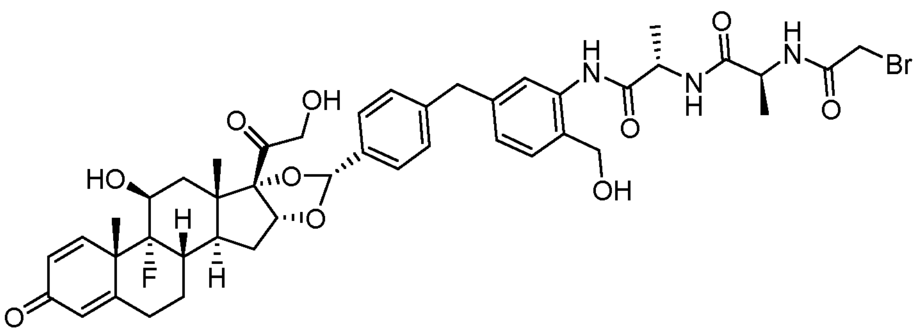 |

(continued)

| No. | Structure |
|---|---|
| IV-65 | 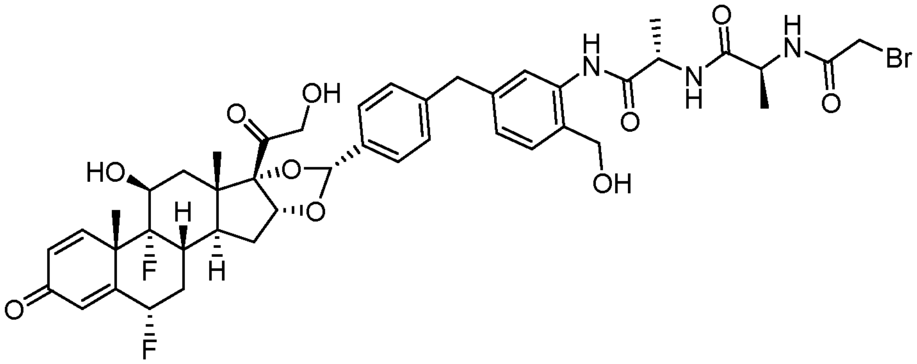 |
| IV-66 | 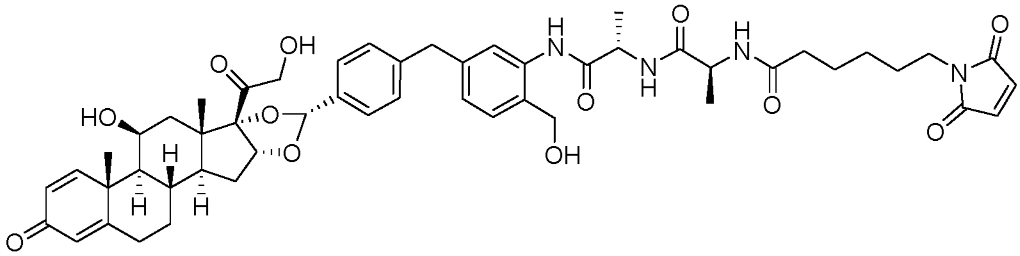 |
| IV-67 | 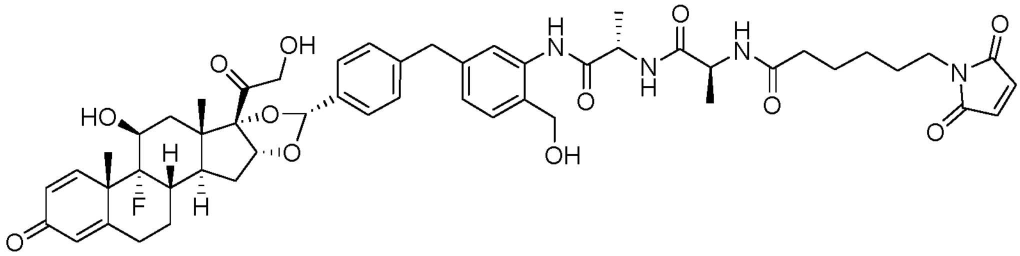 |
| IV-68 | 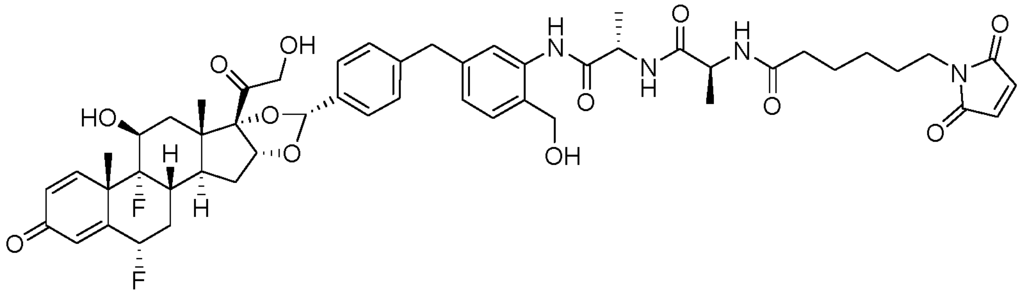 |
| IV-69 | 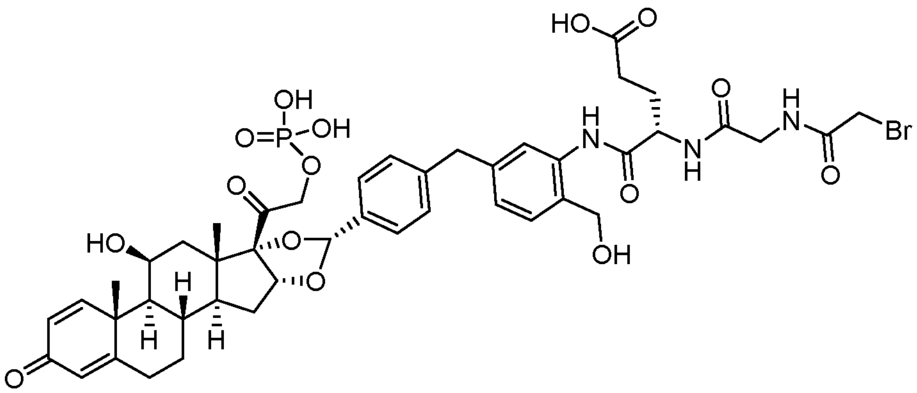 |

(continued)

| No. | Structure |
|---|---|
| IV-70 | 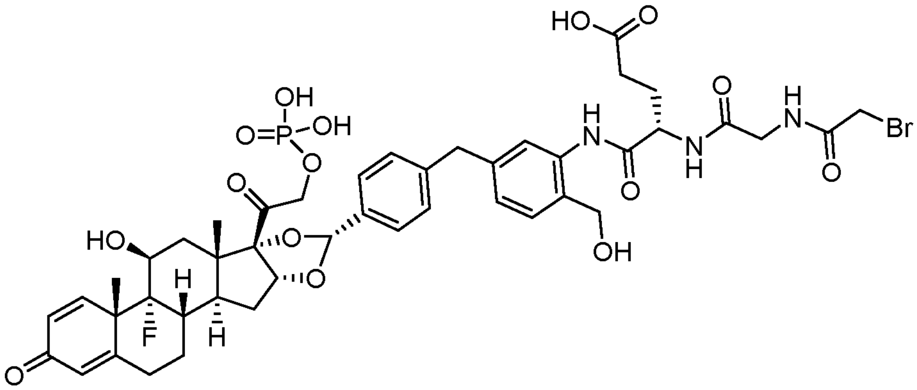 |
| IV-71 | 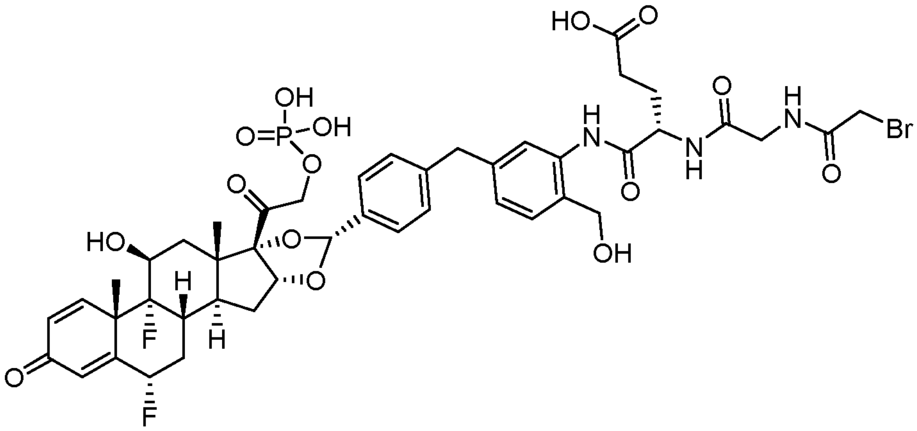 |
| IV-81 | 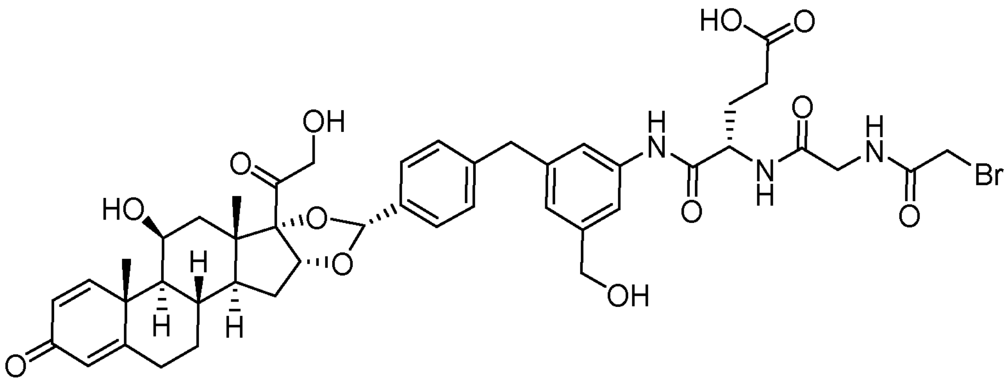 |
| IV-82 | 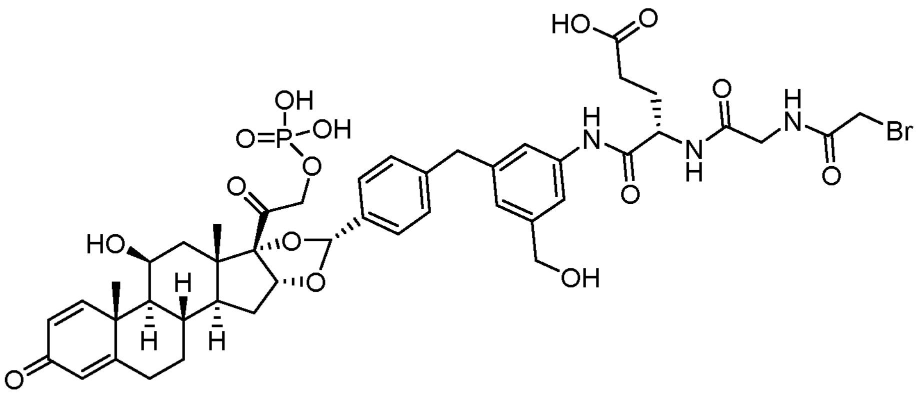 |

(continued)

| No. | Structure |
|---|---|
| IV-83 | 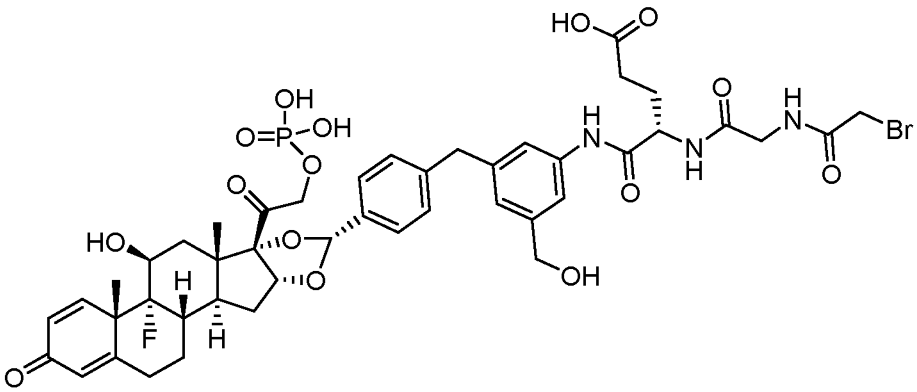 |
| IV-84 | 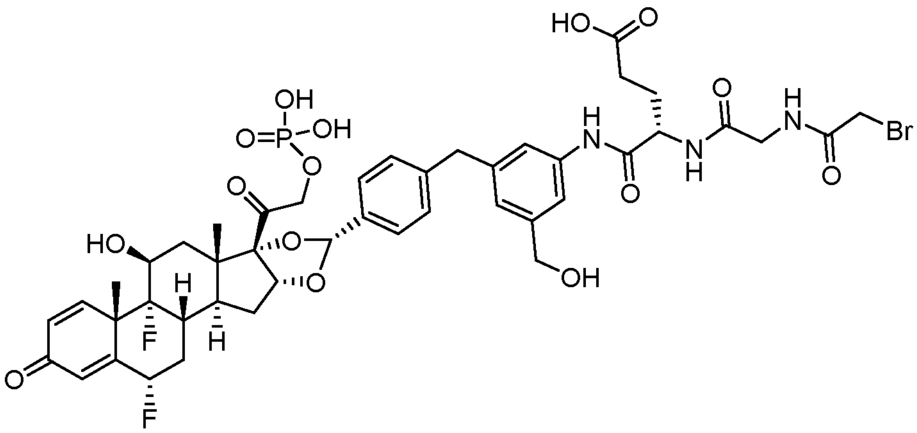 |
| IV-91 | 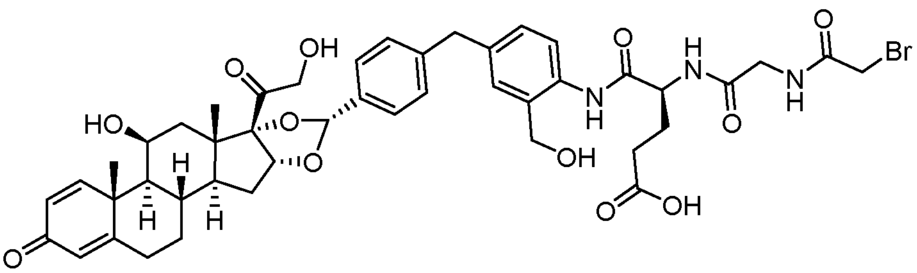 |
| IV-92 | 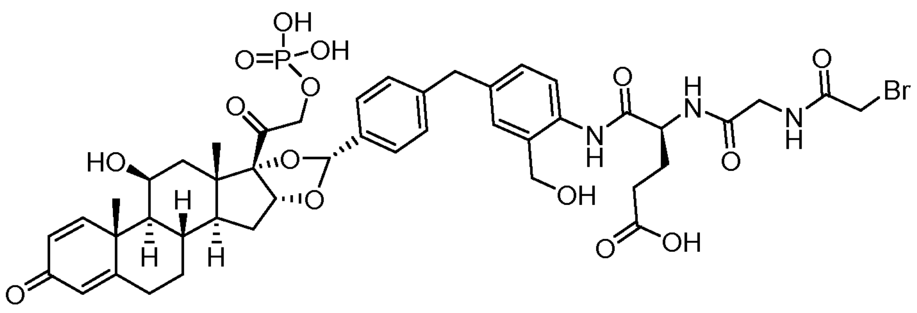 |

(continued)

| No. | Structure |
|---|---|
| IV-93 | 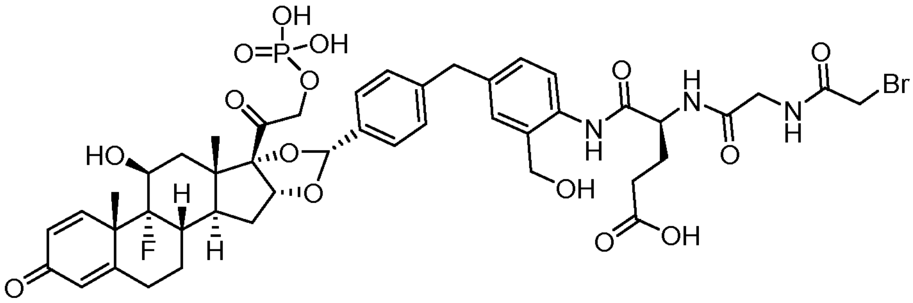 |
| IV-94 | 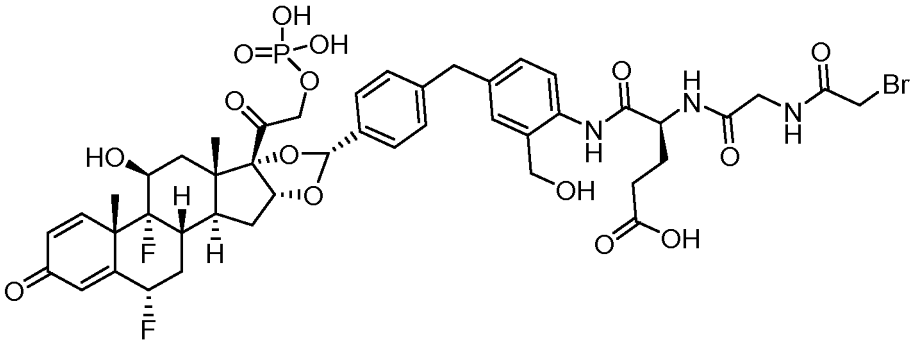 |
| IV-101 | 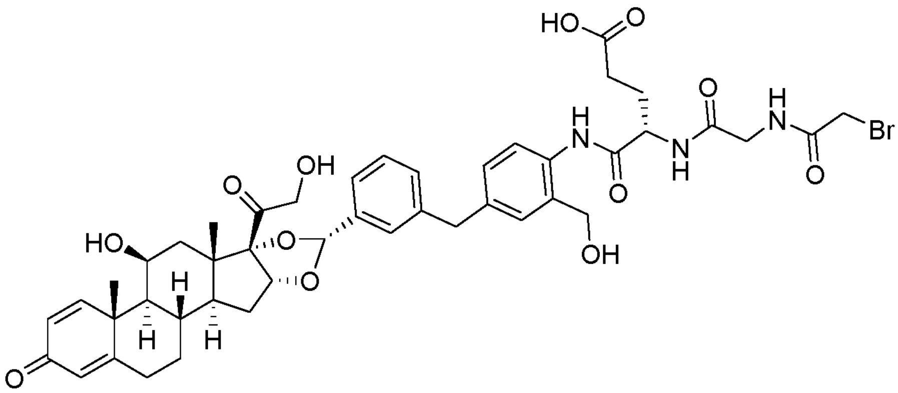 |
| IV-102 | 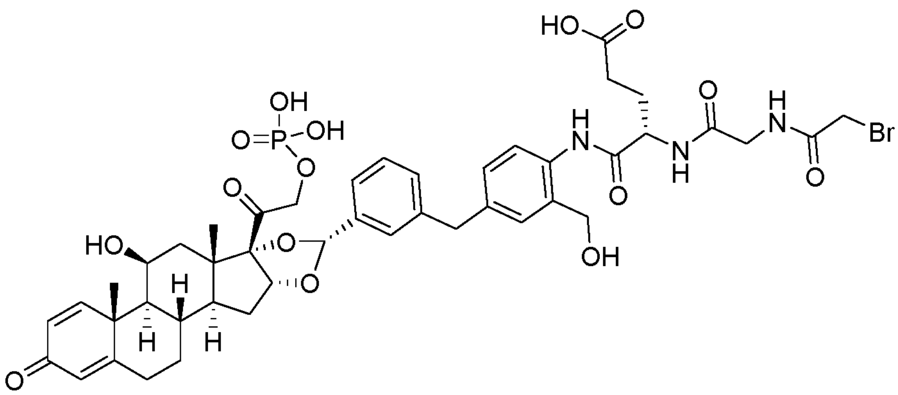 |

(continued)

| No. | Structure |
|---|---|
| IV-103 | 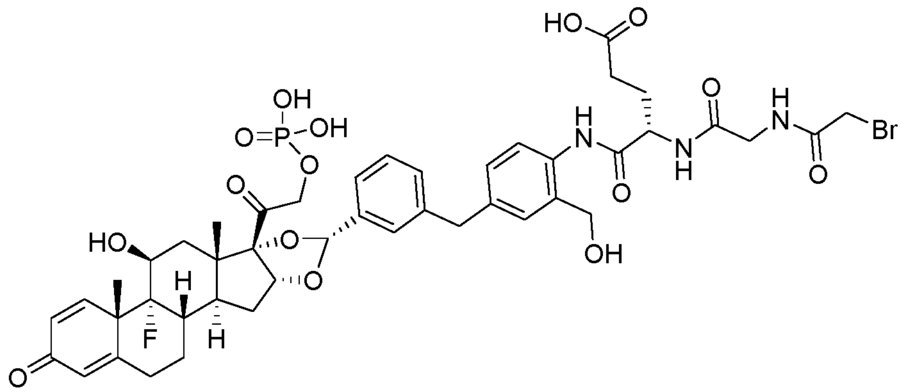 |
| IV-104 | 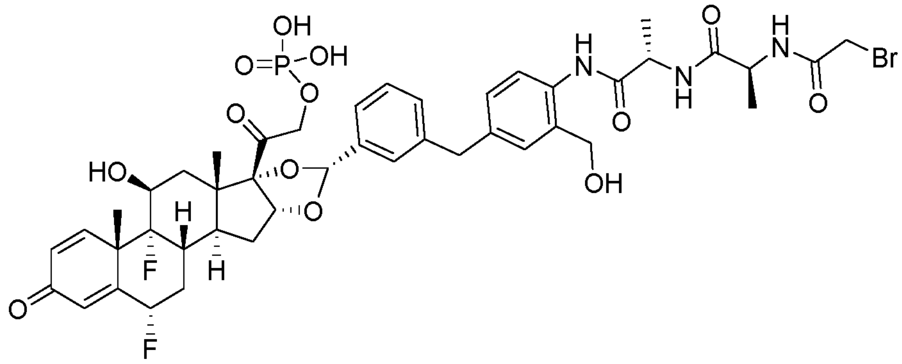 |
| IV-111 | 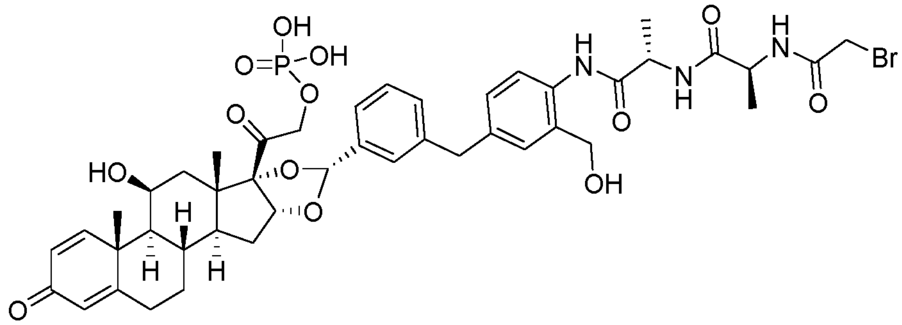 |
| IV-112 | 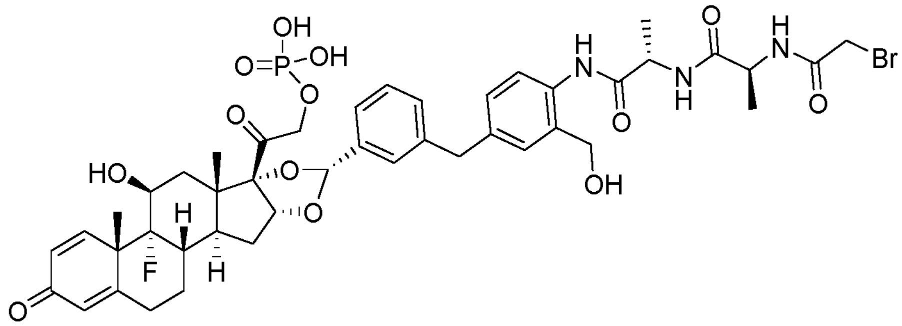 |

(continued)

| No. | Structure |
|---|---|
| IV-113 | 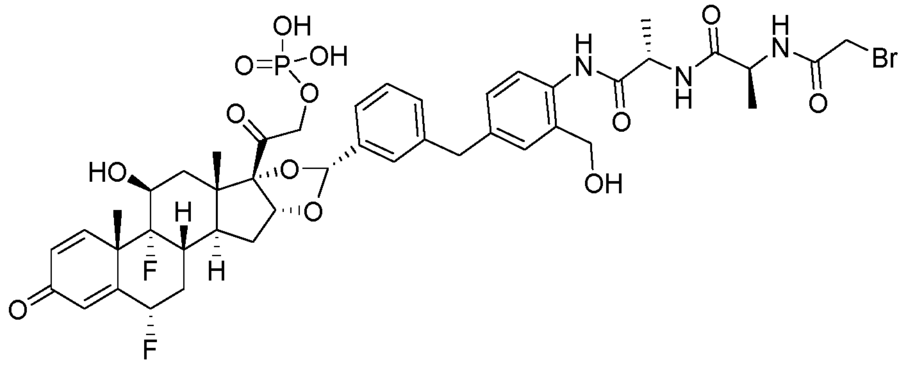 |
| IV-114 | 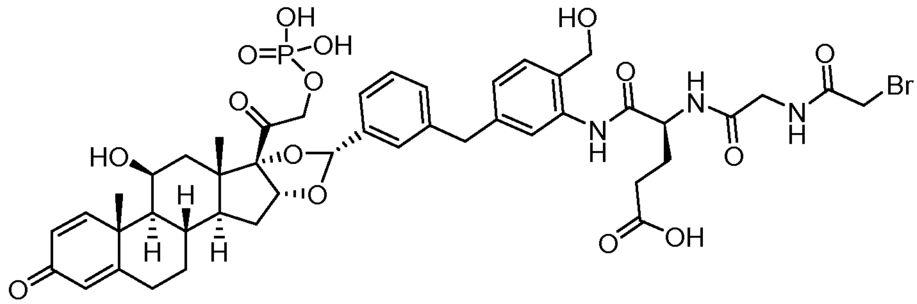 |
| IV-115 | 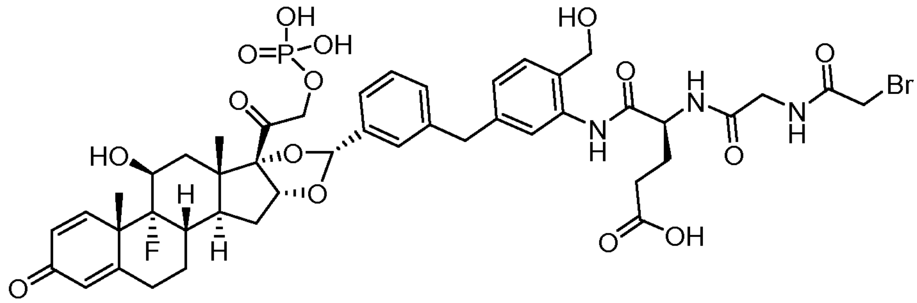 |
| IV-116 | 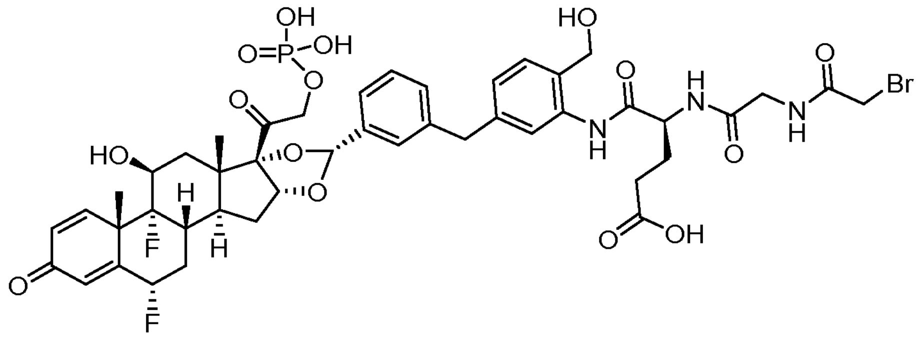 |

(continued)

| No. | Structure |
|---|---|
| IV-117 | 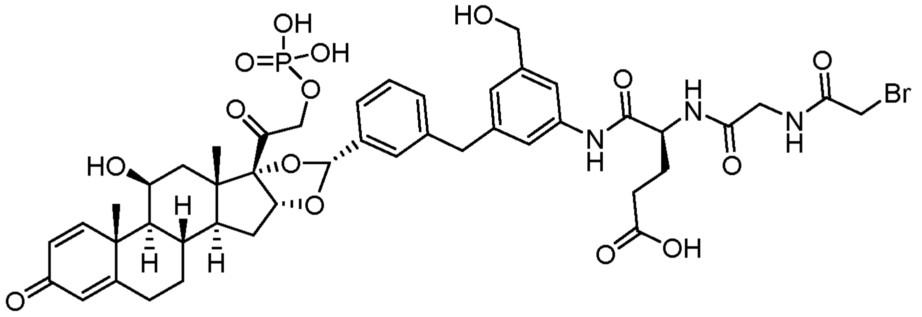 |
| IV-118 | 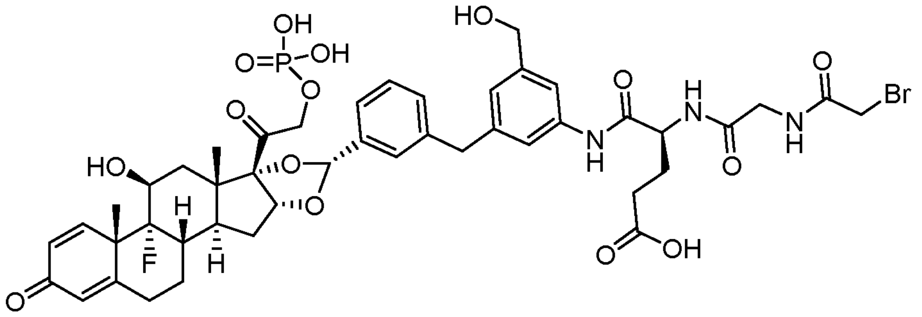 |
| IV-119 | 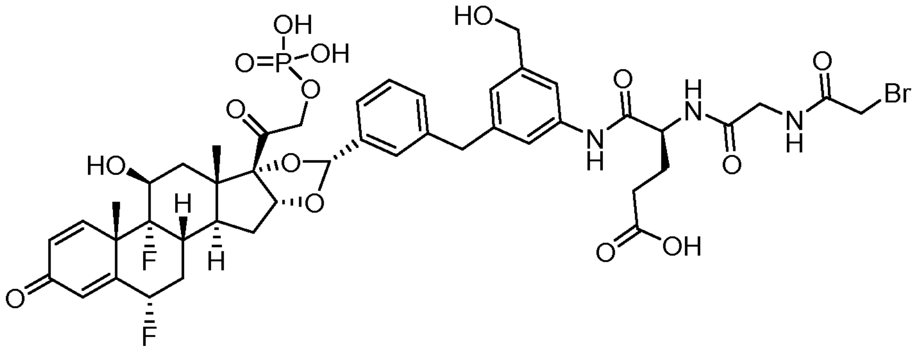 |
| IV-120 | 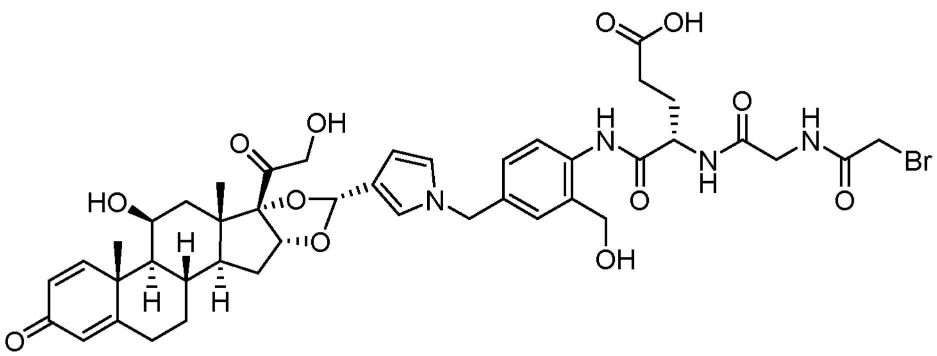 |

(continued)

| No. | Structure |
|---|---|
| IV-121 | 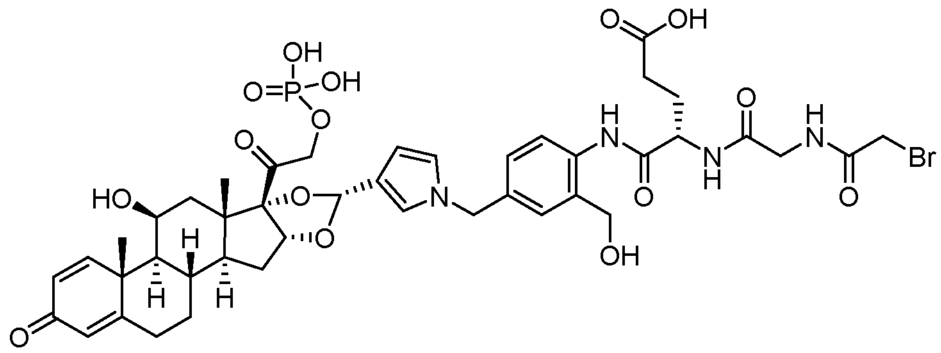 |
| IV-122 | 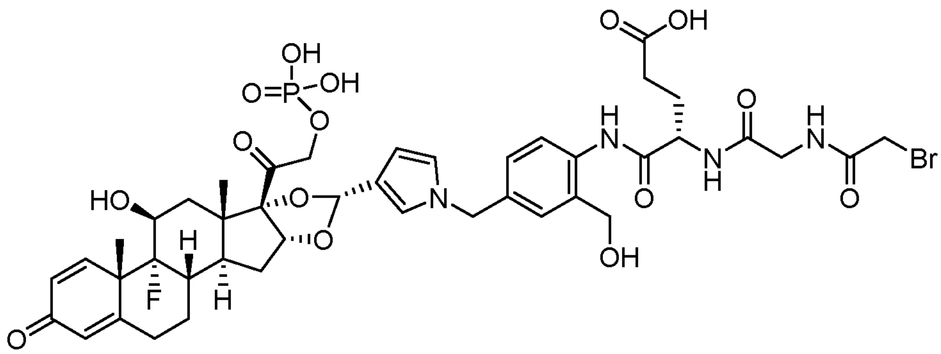 |
| IV-123 | 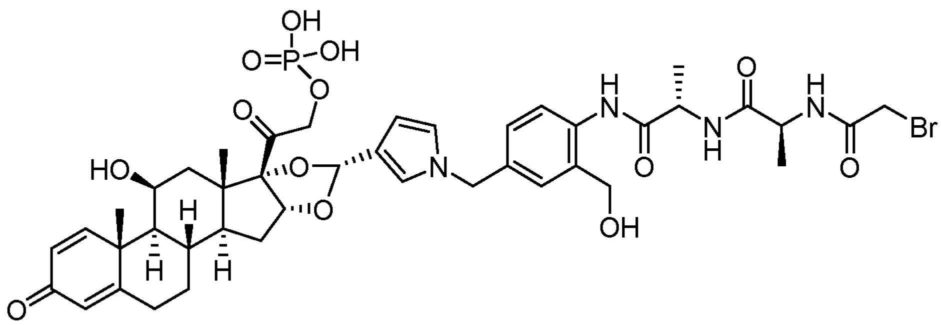 |
| IV-124 | 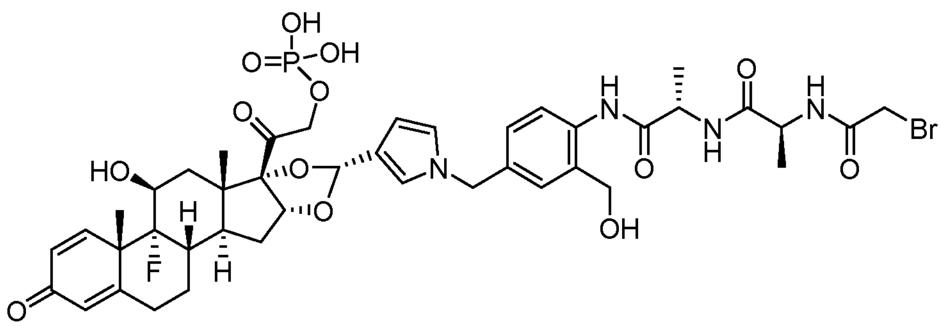 |

(continued)

| No. | Structure |
| --- | --- |
| IV-125 | 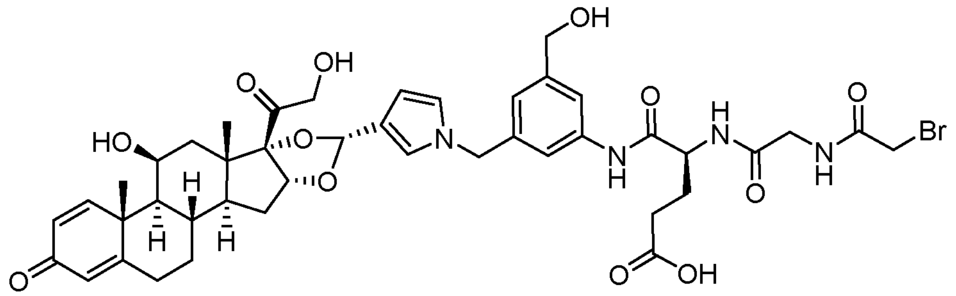 |
| IV-126 | 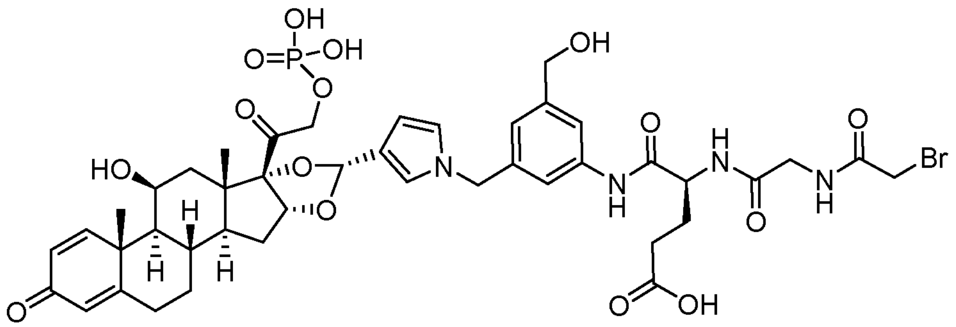 |
| IV-127 | 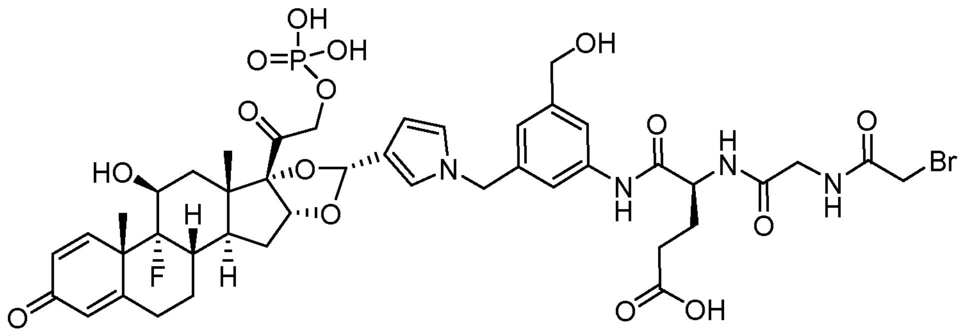 |
| IV-128 | 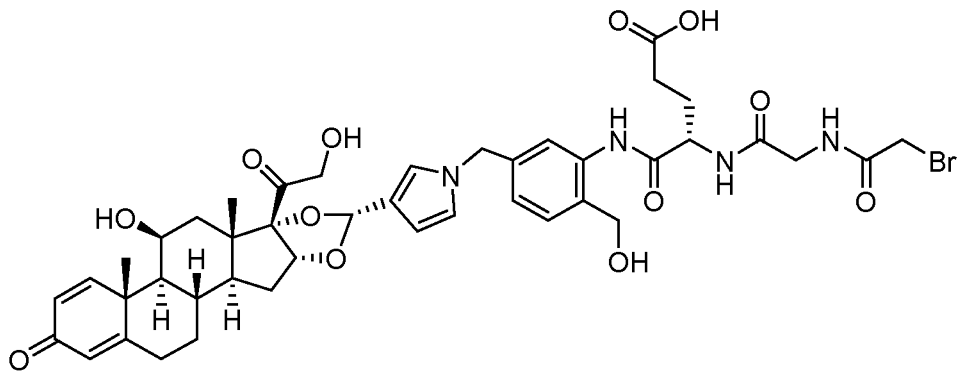 |

(continued)

| No. | Structure |
|---|---|
| IV-129 | 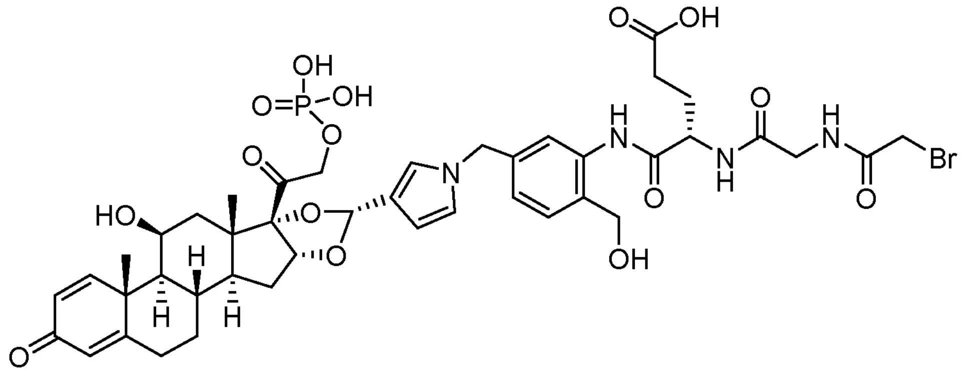 |
| IV-130 | 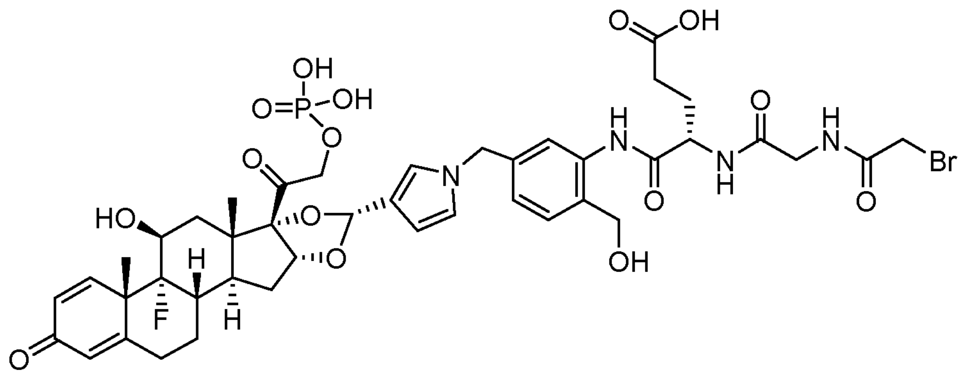 |
| IV-131 | 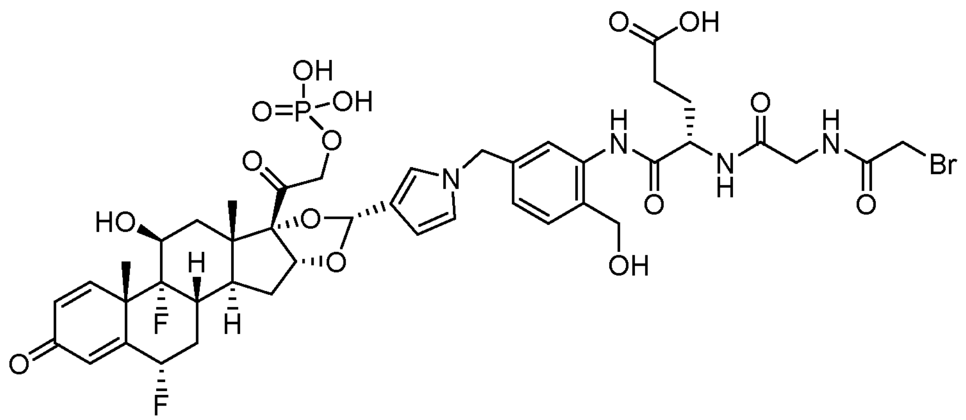 |
| IV-141 | 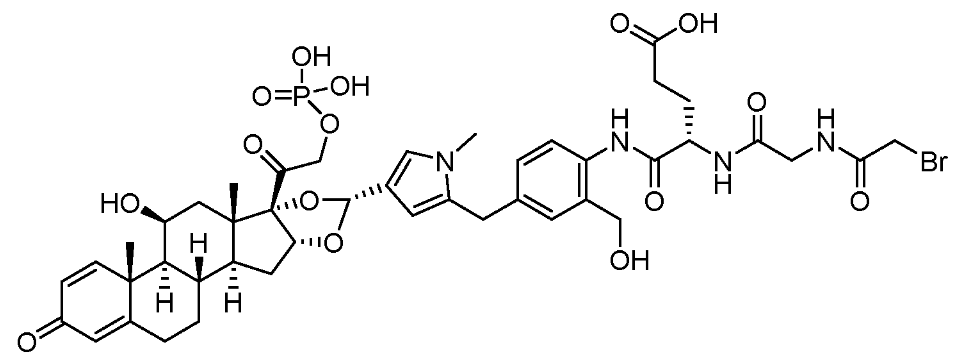 |

(continued)

| No. | Structure |
|---|---|
| IV-142 | 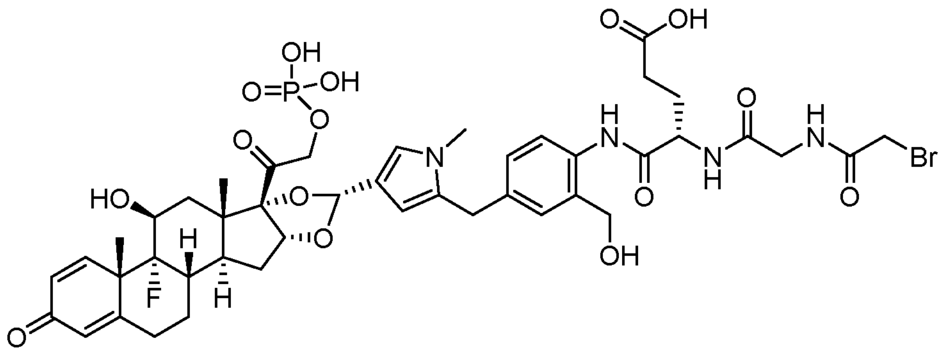 |
| IV-143 | 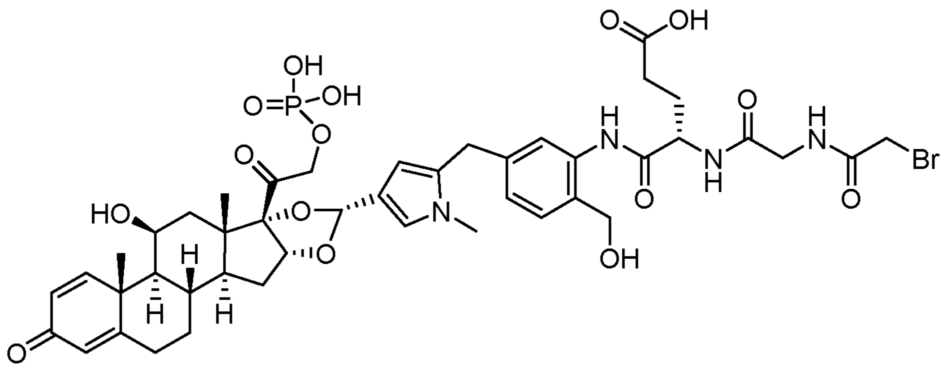 |
| IV-144 | 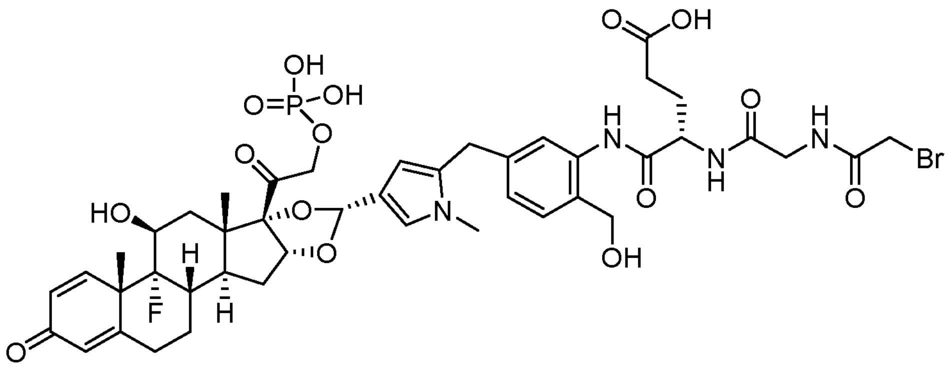 |
| IV-145 | 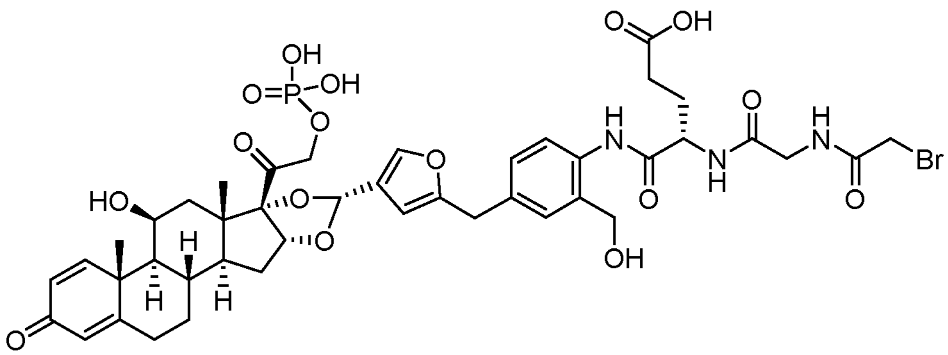 |

(continued)

| No. | Structure |
|---|---|
| IV-146 | 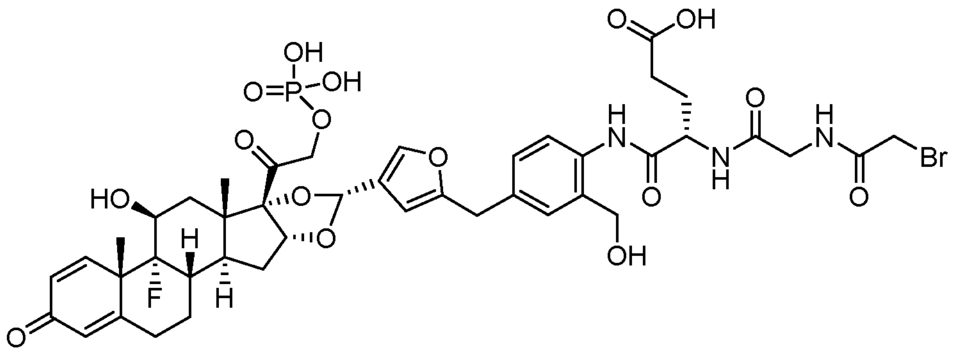 |
| IV-147 | 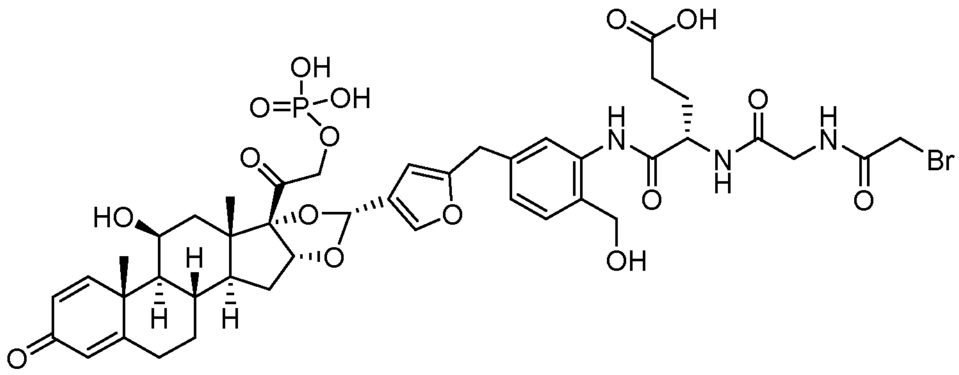 |
| IV-148 | 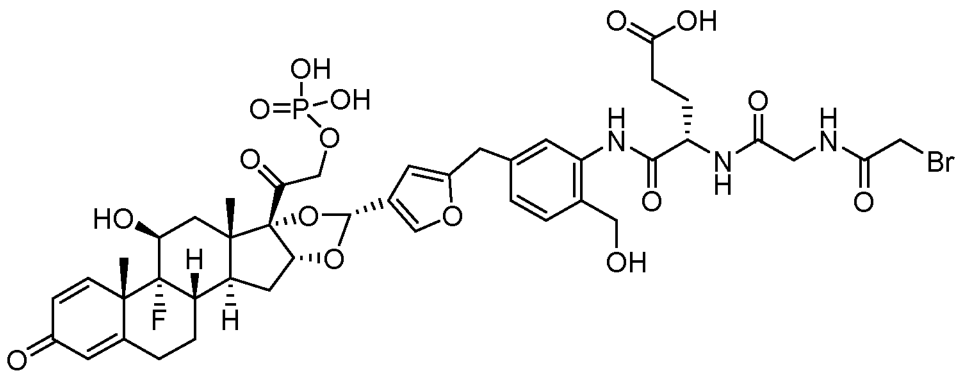 |
| IV-149 | 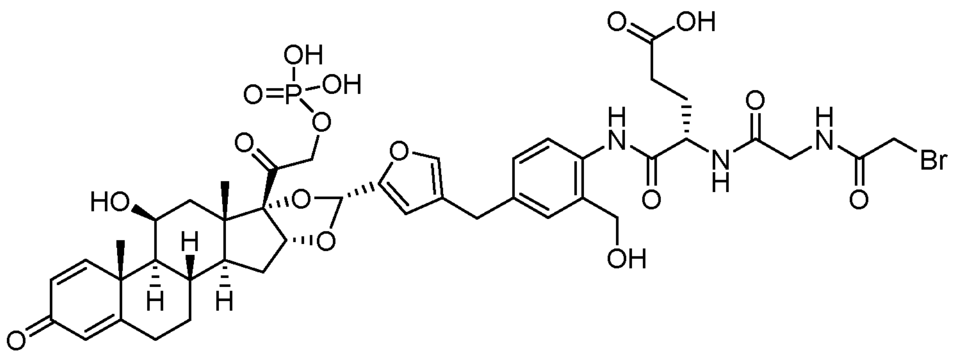 |

(continued)

| No. | Structure |
|---|---|
| IV-150 | 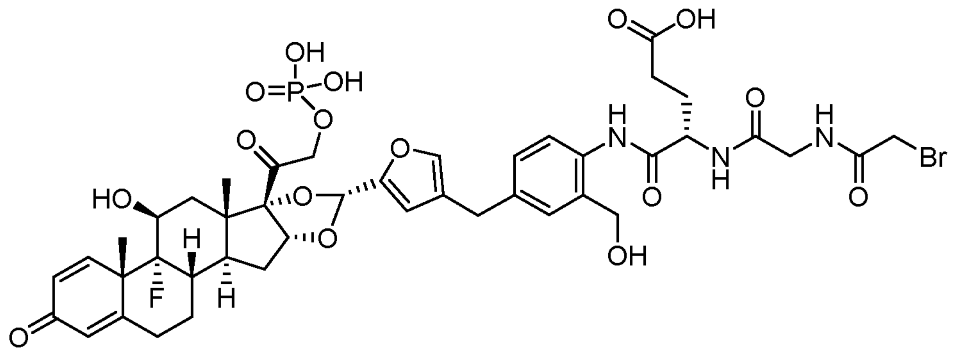 |
| IV-151 | 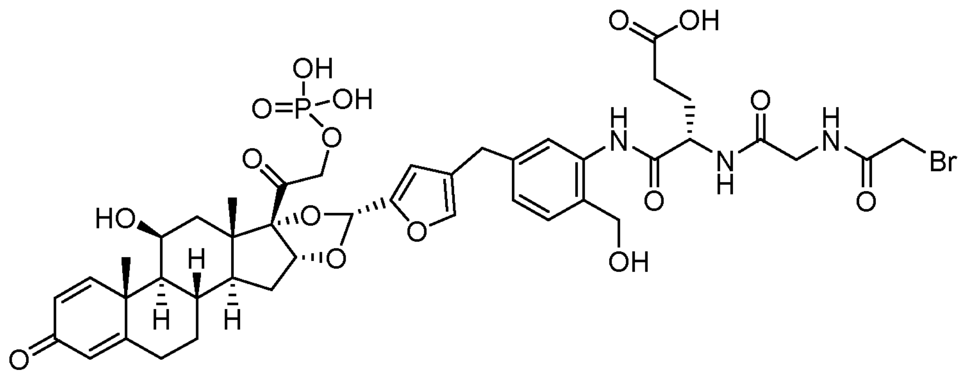 |
| IV-152 | 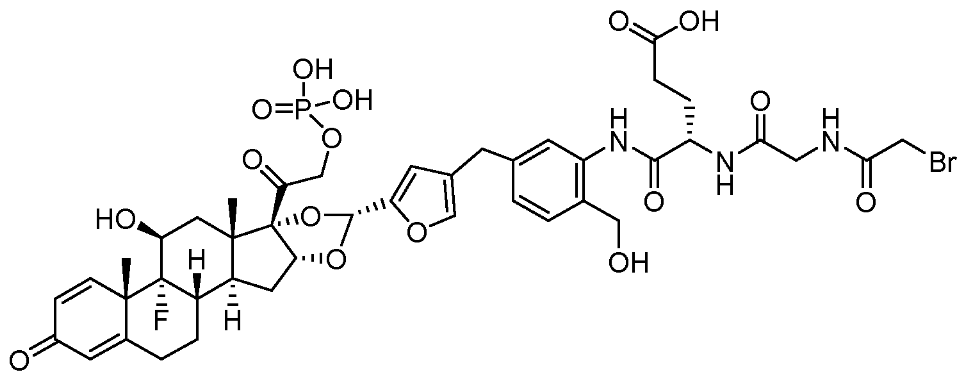 |
| IV-153 | 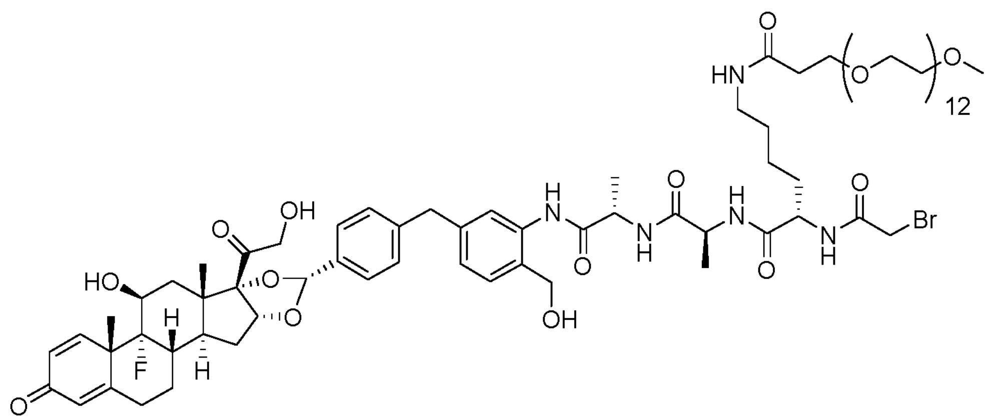 |

(continued)

| No. | Structure |
|---|---|
| IV-154 | 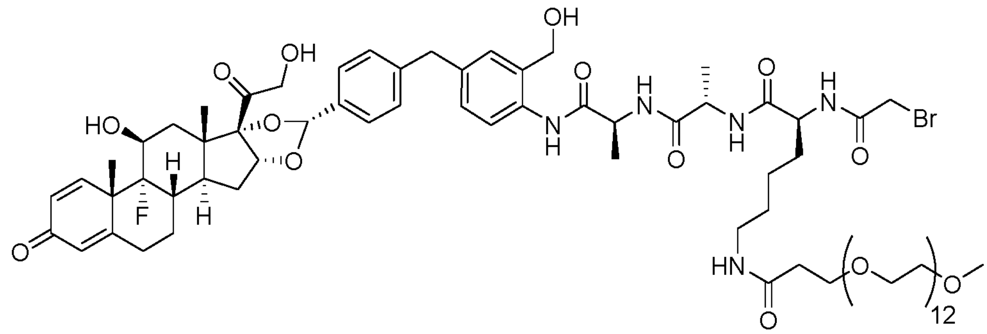 |
| IV-155 | 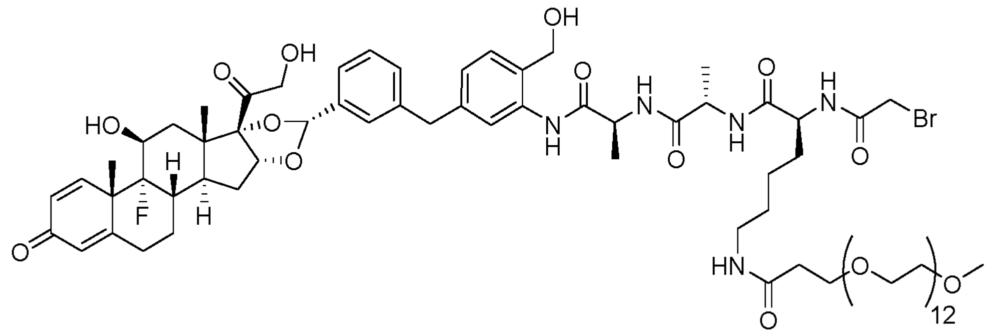 |
| IV-156 | 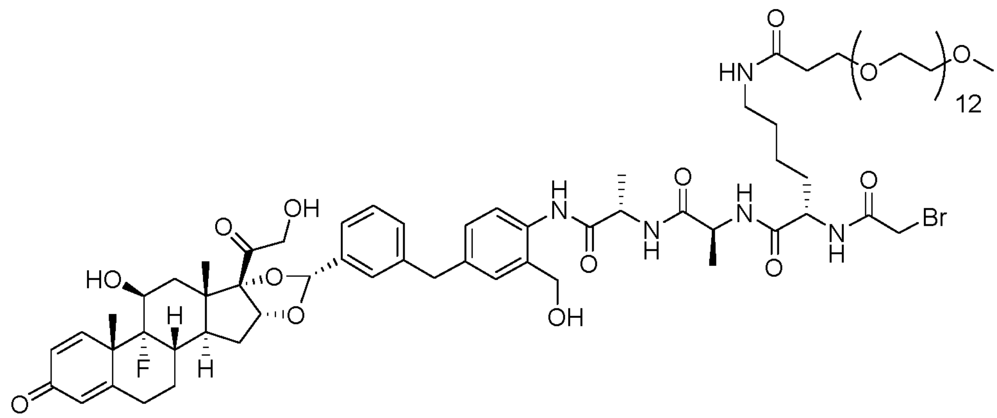 |
| IV-157 | 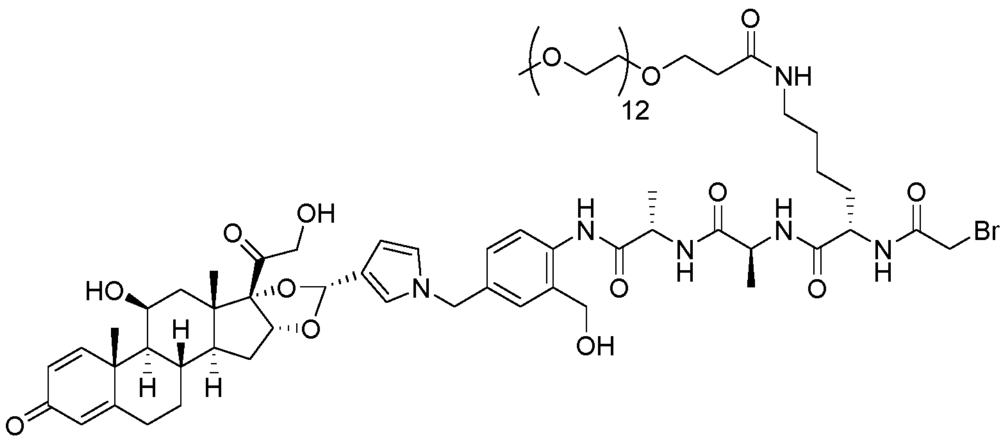 |

(continued)

| No. | Structure |
|---|---|
| IV-158 | 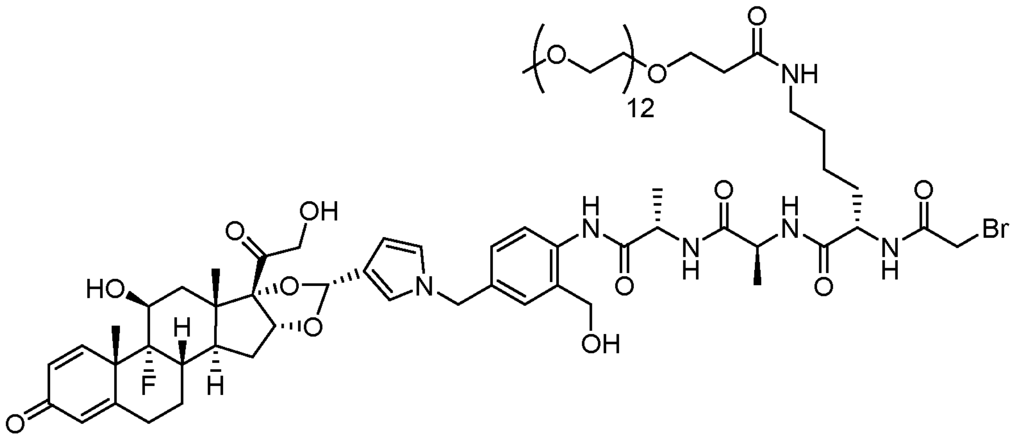 |
| IV-159 | 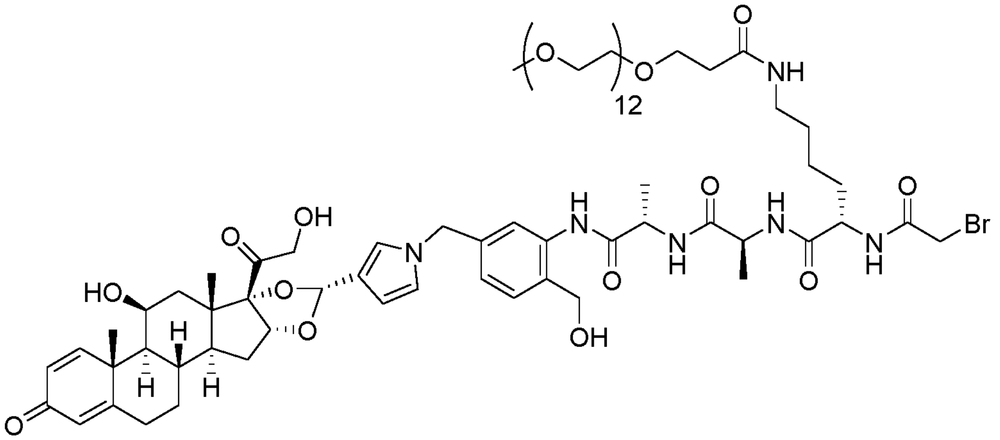 |
| IV-160 | 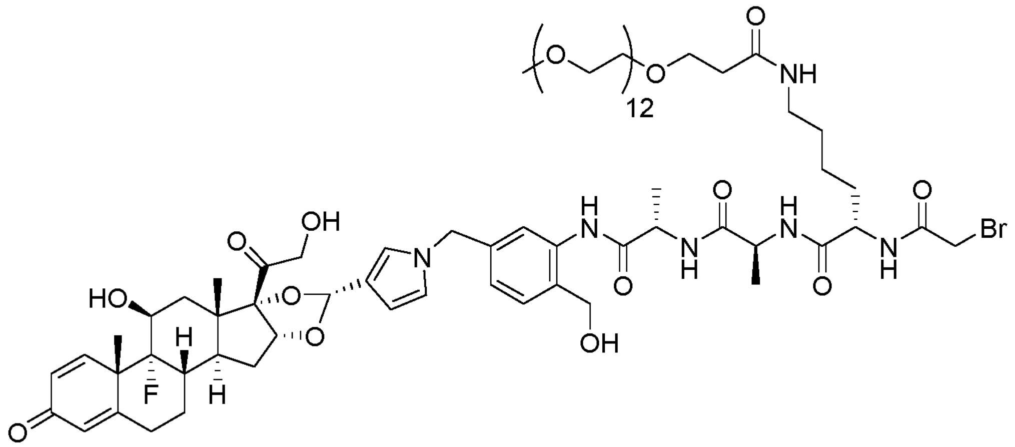 |

**55.** The compound of formula IIa or IIb or the tautomer, the mesomer, the racemate, the enantiomer or the diastereoisomer thereof, or the mixture thereof, or the pharmaceutically acceptable salt thereof according to claim 54, wherein the compound of formula IVa or IVb is selected from the group consisting of the following structures:

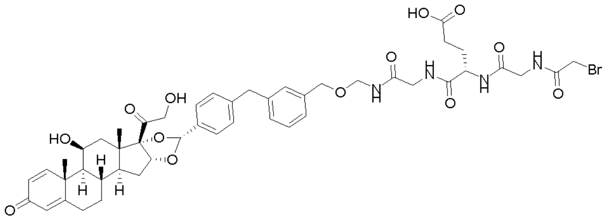

8

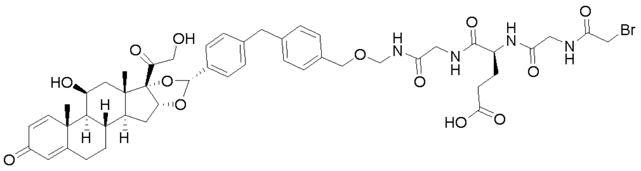

9

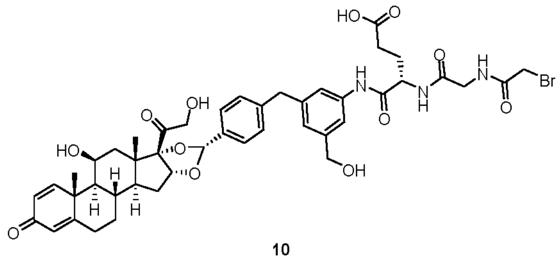

10

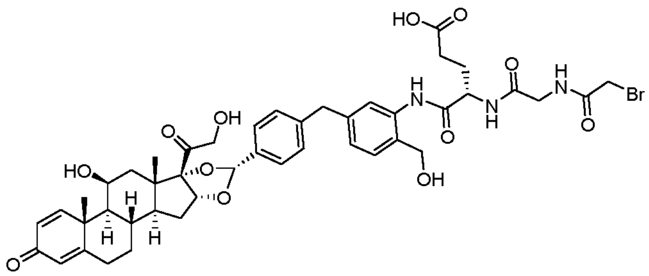

11

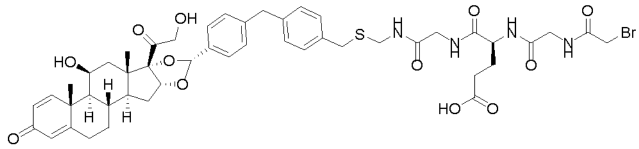

13

56. The compound of formula IIa or IIb or the tautomer, the mesomer, the racemate, the enantiomer or the diastereoisomer thereof, or the mixture thereof, or the pharmaceutically acceptable salt thereof according to any one of claims 26-45, wherein the compound further comprises a linker fragment, the compound of formula IIa or IIb is capable of being coupled to the ligand via the linker fragment, and the compound has the following structure:

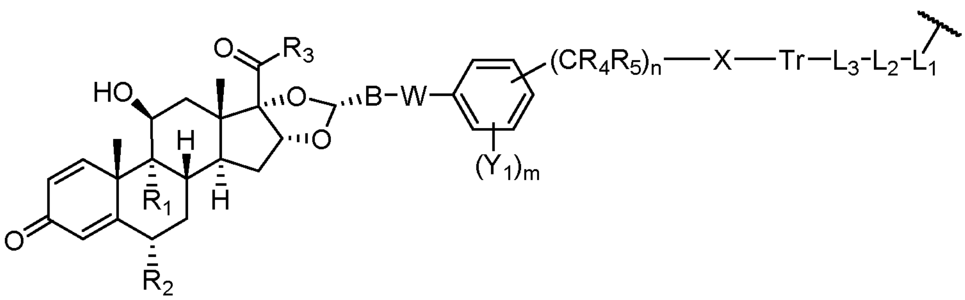

(IVa-1)

or

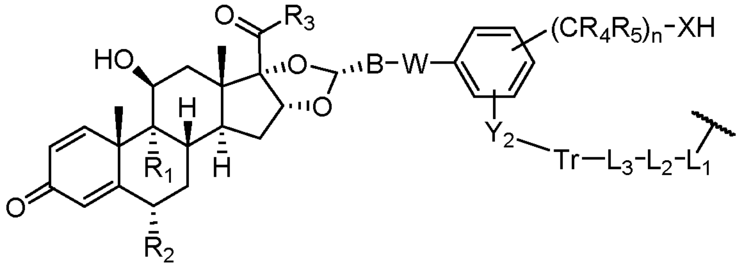

(IVb-2)

wherein,

Tr is absent or Tr is any group;
$L_3$ is selected from a polypeptide fragment;
$L_2$ is absent or selected from a linker fragment;
$L_1$ is selected from a coupling unit; and $L_1$ in the formulas IVa-1 and IVb-1 is in a linked form;
$R_1$, $R_2$, $R_3$, $R_4$, $R_5$, B, W, $CR_4R_5$, n, X, $Y_1$ and $Y_2$ are each as described in any one of claims 26-45; the wavy line

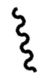

in the general formulas represents being linked to the ligand via the $L_1$ group.

**57.** The compound of formula IIa or IIb or the tautomer, the mesomer, the racemate, the enantiomer or the diastereoisomer thereof, or the mixture thereof, or the pharmaceutically acceptable salt thereof according to claim 56, wherein the structural unit $-Tr-L_3-L_2-L_1-$ is selected from the group consisting of:

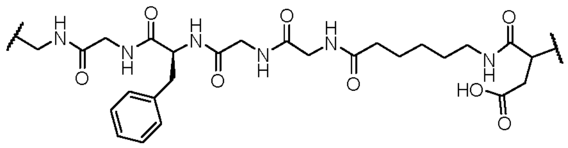,
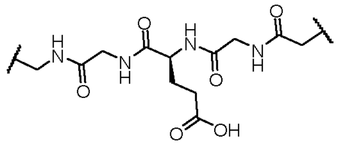,
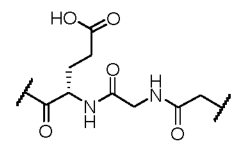

and

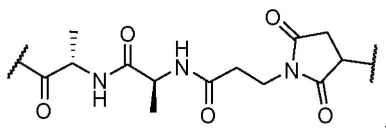.

**58.** A conjugate, comprising the compound or the tautomer, the mesomer, the racemate, the enantiomer or the diastereoisomer thereof, or the mixture thereof, or the pharmaceutically acceptable salt thereof according to any one of claims 1-57, wherein the conjugate comprises a ligand-drug conjugate.

**59.** The conjugate according to claim 58, wherein the ligand comprises an antibody or an antigen-binding fragment thereof.

**60.** The conjugate according to claim 59, wherein the antibody is selected from the group consisting of: a human antibody, a humanized antibody, a chimeric antibody, a multispecific antibody, a monoclonal antibody and a polyclonal antibody; the antigen-binding fragment is selected from the group consisting of: a Fab, a Fab', a F(ab')2, a Fv, a scFv, a diabody, a Fd, a dAb, a VHH, a maxibody and a complementarity determining region (CDR) fragment.

**61.** The conjugate according to claims 58-60, wherein the ligand specifically binds to an antigen selected from the group consisting of: AXL, BAFFR, BCMA, BCR-list components, BDCA2, BDCA4, BTLA, BTNL2 BTNL3, BTNL8, BTNL9, C10orf54, CCR1, CCR3, CCR4, CCR5, CCR6, CCR7, CCR9, CCR10, CD11c, CD137, CD138, CD14, CD163, CD168, CD177, CD19, CD20, CD209, CD209L, CD22, CD226, CD248, CD25, CD27, CD274, CD276, CD28, CD30,

CD300A, CD33, CD37, CD38, CD4, cluster of differentiation 40 (CD40), CD44, CD45, CD46, CD47, CD48, CD5, CD52, CD55, CD56, CD59, CD62E, CD68, CD69, CD70, CD74, CD79a, CD79b, CD8, CD80, CD86, CD90.2, CD96, CLEC12A, CLEC12B, CLEC7A, CLEC9A, CR1, CR3, CRTAM, CSF1R, CTLA4, CXCR1/2, CXCR4, CXCR5, DDR1, DDR2, DEC-205, DLL4, DR6, FAP, FCamR, FCMR, FcR's, Fire, GITR, HHLA2, HLA class II, HVEM, ICOSLG, IFNAR, type I interferon receptor subunit (IFNAR1), IFNLR1, IL10R1, IL10R2, IL12R, IL13RA1, IL13RA2, IL15R, IL17RA, IL17RB, IL17RC, IL17RE, IL20R1, IL20R2, IL21R, IL22R1, IL22RA, IL23R, IL27R, IL29R, IL2Rg, IL31R, IL36R, IL3RA, IL4R, IL6R, IL5R, IL7R, IL9R, integrins, LAG3, LIFR, MAG/Siglec-4 (sialic acid-binding immunoglobulin-like lectin-4), MMR, MSR1, NCR3LG1, NKG2D, NKp30, NKp46, OX40 (CD134), PDCD1, PROKR1, PVR, PVRIG, PVRL2, PVRL3, RELT, SIGIRR, Siglec-1 (sialic acid-binding immunoglobulin-like lectin-1), Siglec-10, Siglec-5, Siglec-6, Siglec-7, Siglec-8, Siglec-9, SIRPA, SLAMF7, TACI, TCR-list components/assoc, PTCRA, TCRb, CD3z, CD3, TEK, TGFBR1, TGFBR2, TGFBR3, TIGIT, TLR2, TLR4, tumor necrosis factor $\alpha$(TNF$\alpha$), TROY, TSLPR, TYRO, VLDLR, VSIG4, IL2R-y and VTCN1.

**62.** The conjugate according to claim 61, wherein the ligand is selected from the group consisting of: an anti-TNF$\alpha$ antibody or an antigen-binding fragment thereof, an anti-CD40 antibody or an antigen-binding fragment thereof, and an anti-IFNAR1 antibody or an antigen-binding fragment thereof.

**63.** The conjugate according to claims 58-62, wherein the ligand is selected from the group consisting of: adalimumab, iscalimab (CFZ533), anifrolumab (MEDI-546), infliximab, afelimomab, golimumab, BIIB059, 8c11, and derivatives and biosimilars thereof.

**64.** The conjugate according to claims 58-63, wherein the ligand-drug conjugate has the following structure:

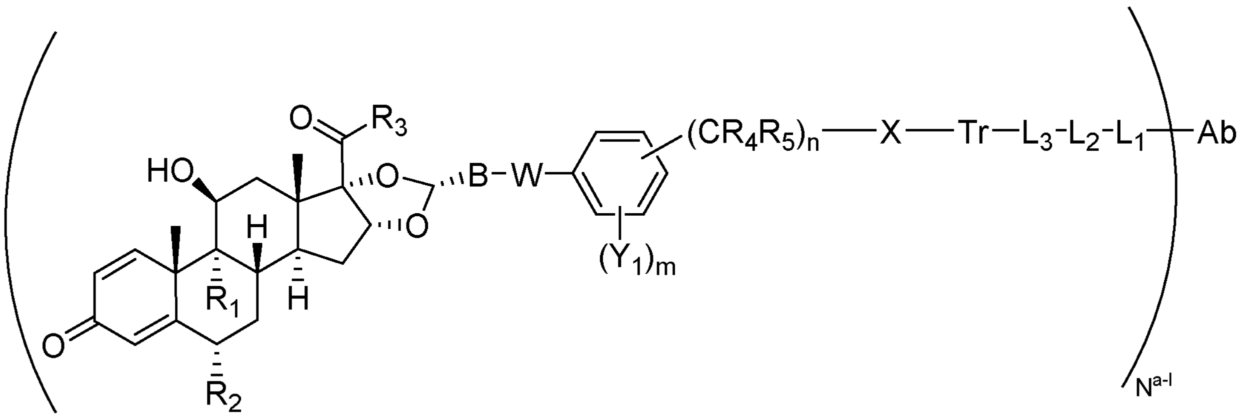

or

(Va)

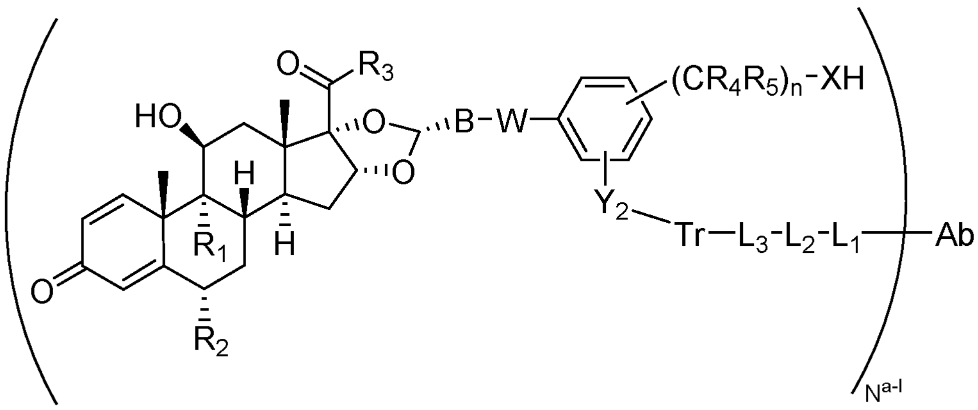

,

(Vb)

wherein,

Ab represents a ligand capable of binding to a target, and $N^{a-l}$ is any number from 1 to 10;
Tr is absent or Tr is any group;
$L_3$ is selected from a polypeptide fragment;
$L_2$ is absent or selected from a linker fragment;
$L_1$ is selected from a coupling unit;
$R_1$, $R_2$, $R_3$, $R_4$ and $R_5$ are each independently any group;
B is absent or B is any group;
W is absent or W is any group;
$CR_4R_5$ units are each independently unreplaced or replaced by a group selected from the group consisting of: -O-, -S-, -NR-, -S(O)-, $-S(O)_2-$, -C(=O)-, -C=C- and -C=C-, wherein $R_4$ and $R_5$ can together form optionally substituted cycloalkyl, optionally substituted heterocyclyl, optionally substituted bridged cyclyl, optionally substituted bridged heterocyclyl, optionally substituted spiro cyclyl and optionally substituted spiro heterocyclyl, and n is any integer from 1 to 20;
X is selected from the group consisting of: -O-, -S- and -NR-;
$Y_1$ is any group, and m is any integer from 0 to 4;
$Y_2$ is selected from the group consisting of -O-, -S- and -NR-;
wherein substituents are selected from the group consisting of: protium, deuterium, tritium, halogen, -CN, =O, =N-OH, =N-OR, =N-R, -OR, -C(O)R, -C(O)OR, -OC(O)R, -OC(O)OR, -C(O)NHR, - $C(O)NR_2$, -OC(O)NHR, $-OC(O)NR_2$, -SR, -S(O)R, $-S(O)_2R$, -NHR, $-N(R)_2$, -NHC(O)R, -NRC(O)R, -NHC(O)OR, -NRC(O)OR, $-S(O)_2NHR$, $-S(O)_2N(R)_2$, $-NHS(O)_2NR_2$, $-NRS(O)_2NR_2$, $-NHS(O)_2R$, - $NRS(O)_2R$, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy; wherein each R is independently selected from the group consisting of hydrogen, protium, deuterium, tritium, oxygen, halogen, $C_1$-$C_6$ alkyl, $C_1$-$C_6$ alkoxy, aryl, heteroaryl, cycloalkyl, heterocycloalkyl, halogenated $C_1$-$C_6$ alkyl and halogenated $C_1$-$C_6$ alkoxy.

**65.** The conjugate according to claim 64, wherein when $L_1$ is coupled to Ab via sulfhydryl, $L_1$ is selected from the group consisting of the following structures:

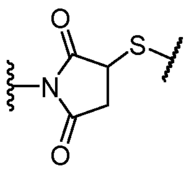,
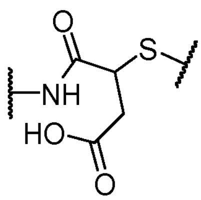,
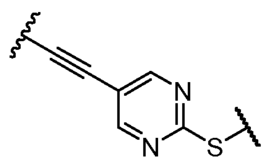,
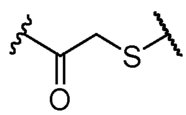,
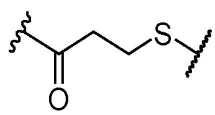,

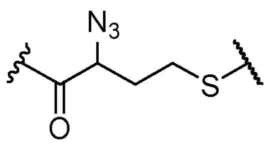,
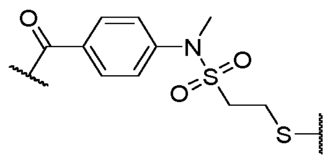,
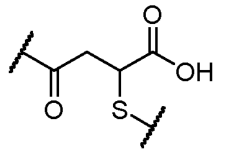,
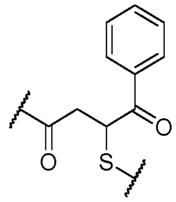,

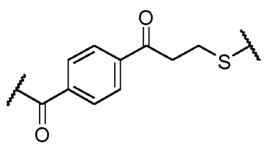,
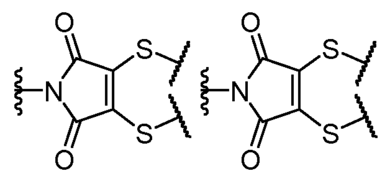,
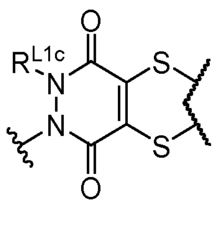,
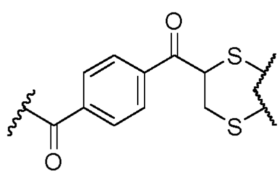

and

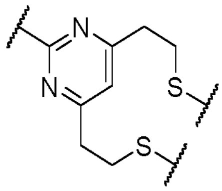

,

wherein $R^{L1c}$ is selected from the group consisting of: hydrogen, optionally substituted alkyl and optionally substituted aryl;

when $L_1$ is coupled to Ab via amino, $L_1$ is selected from the group consisting of the following structures:

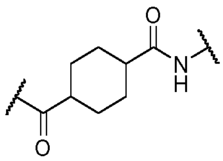

and

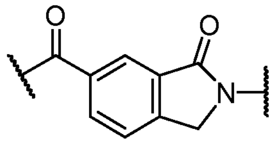

;

when $L_1$ is coupled to Ab via click chemistry, $L_1$ is selected from the group consisting of the following structures:

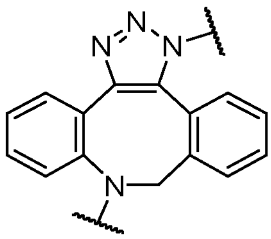

,

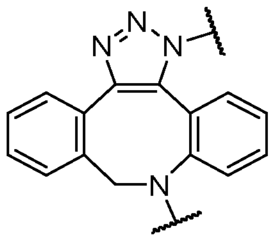

,

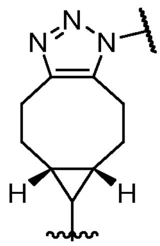

and

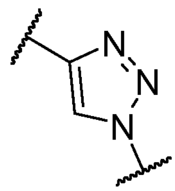

.

**66.** The conjugate according to any one of claims 59-65, wherein the conjugate is selected from the group consisting of the following structures:

| No. | Structure |
|---|---|
| V-1 | 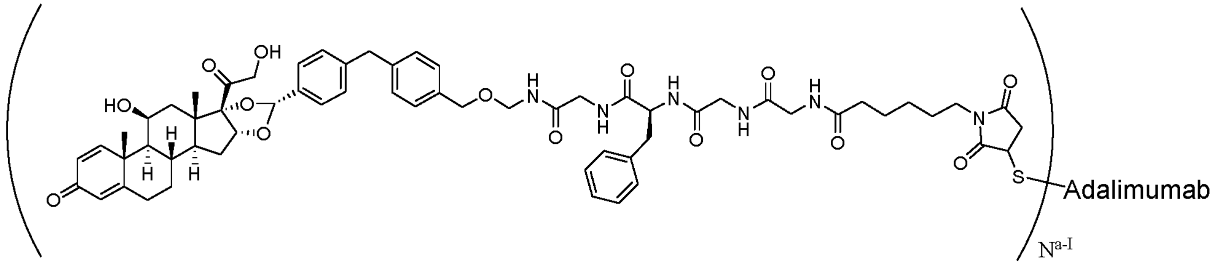 |
| V-2 | 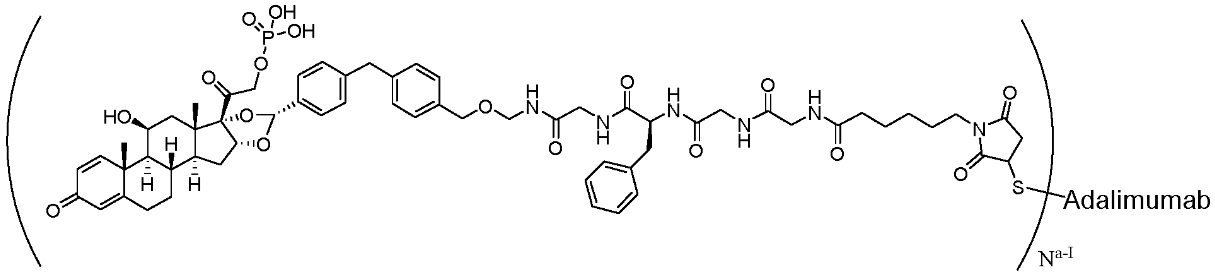 |
| V-6 | 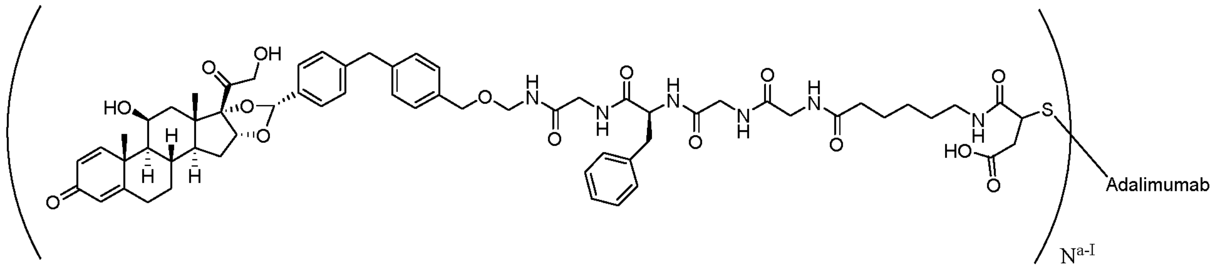 |

(continued)

| No. | Structure |
|---|---|
| V-7 | 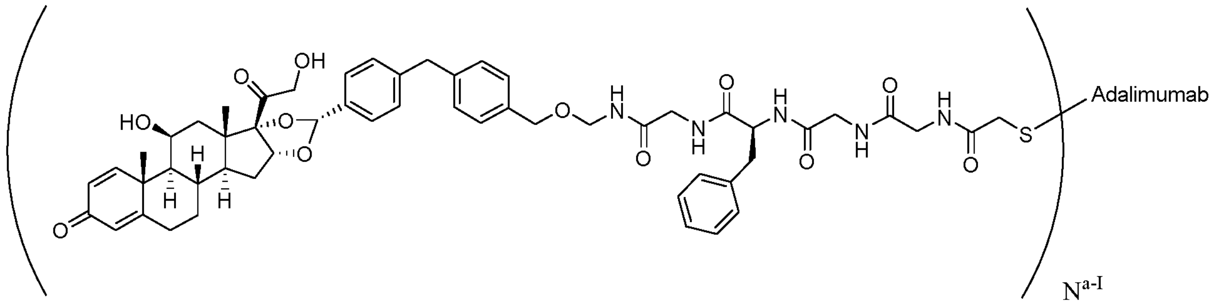 |
| V-8 | 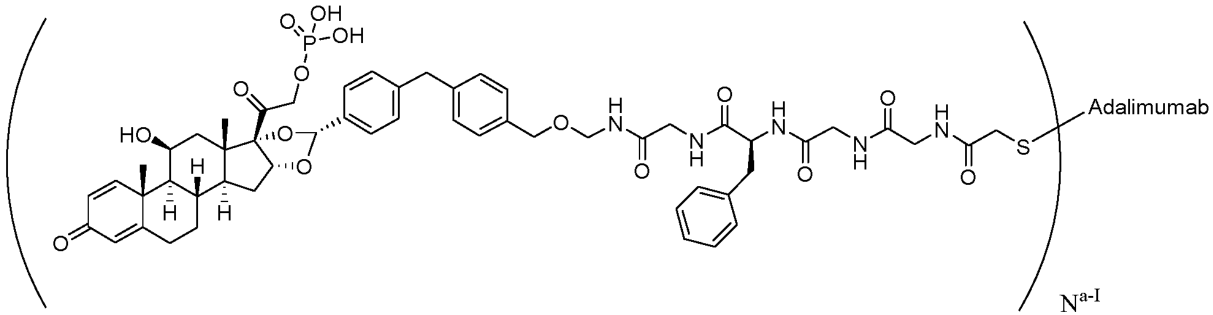 |

(continued)

| No. | Structure |
| --- | --- |
| V-12 | 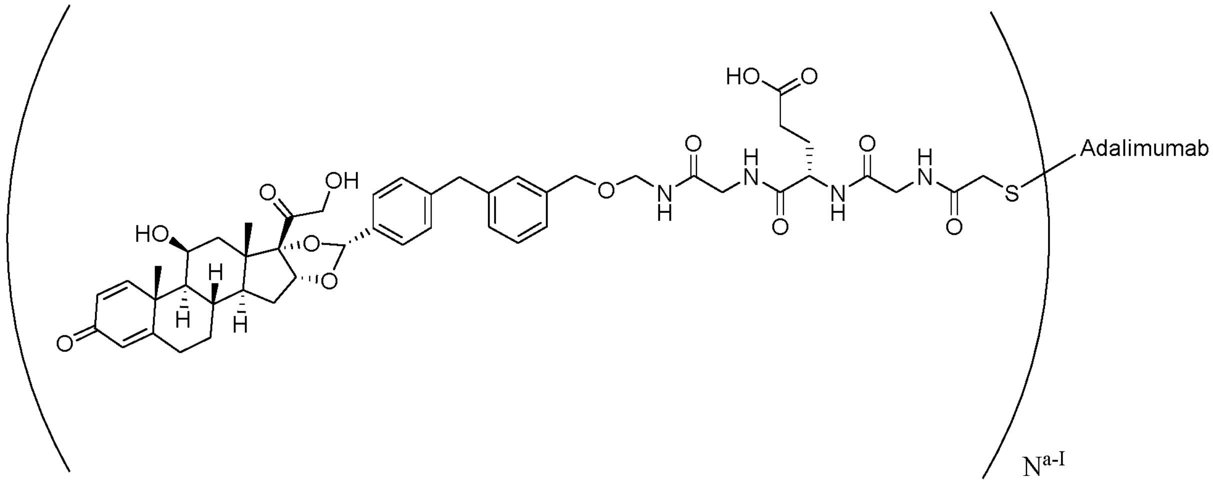 |
| V-13 | 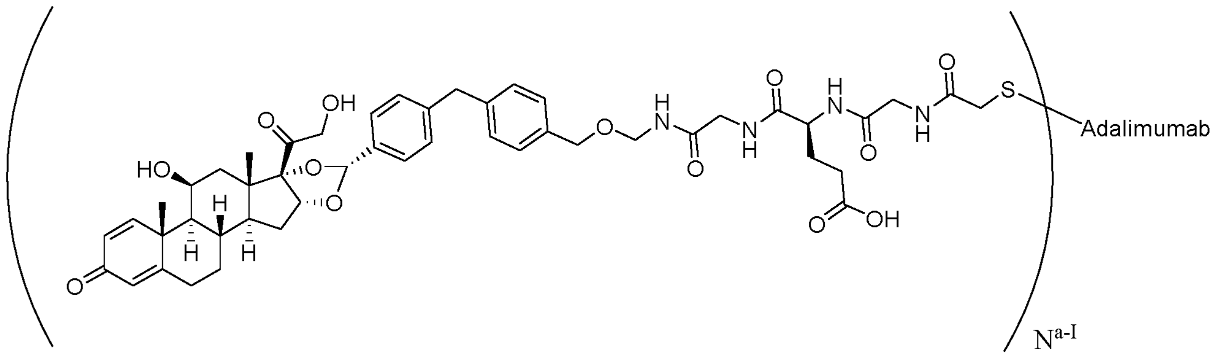 |

(continued)

| No. | Structure |
|---|---|
| V-14 | 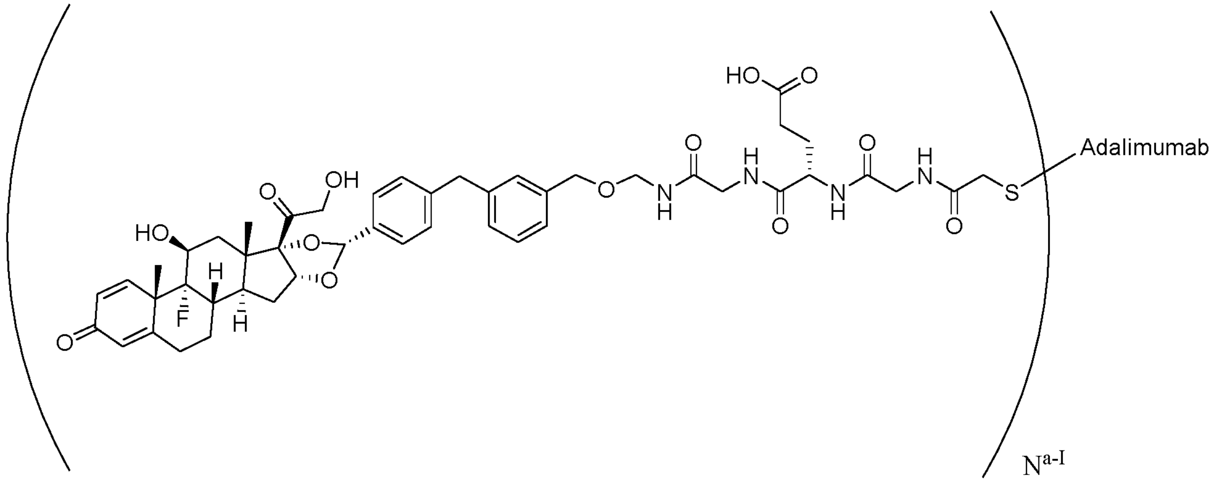 |
| V-15 | 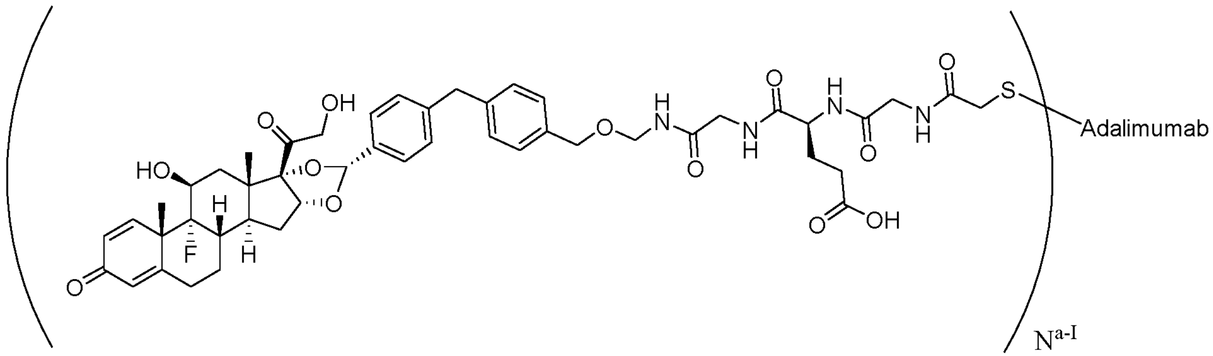 |

(continued)

| No. | Structure |
|---|---|
| V-16 | 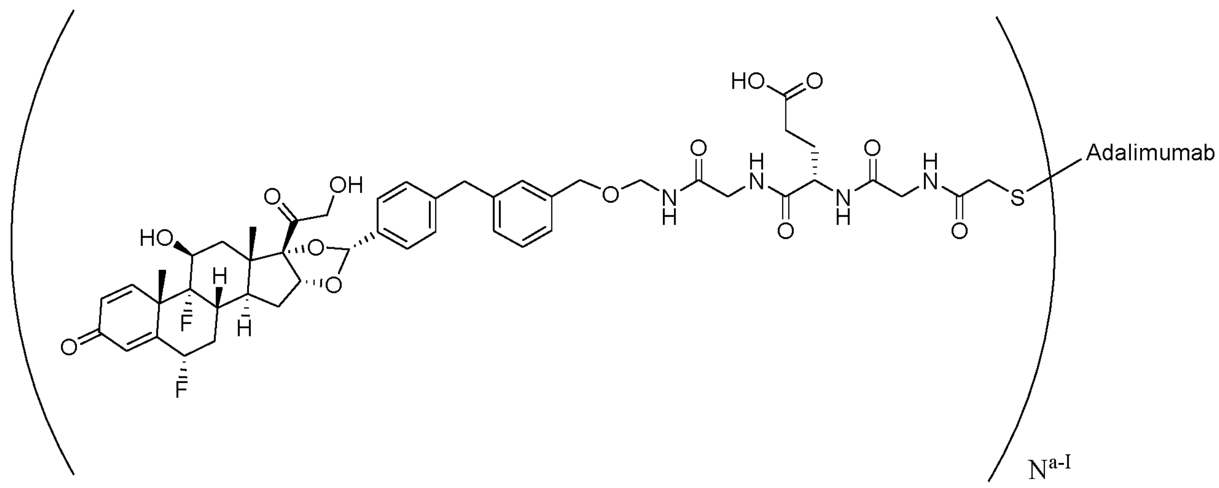 |
| V-17 | 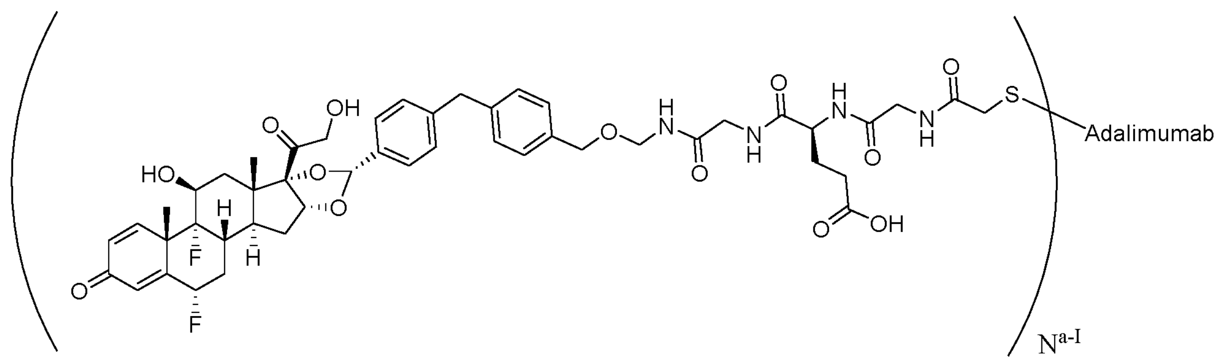 |

(continued)

| No. | Structure |
|---|---|
| V-18 | 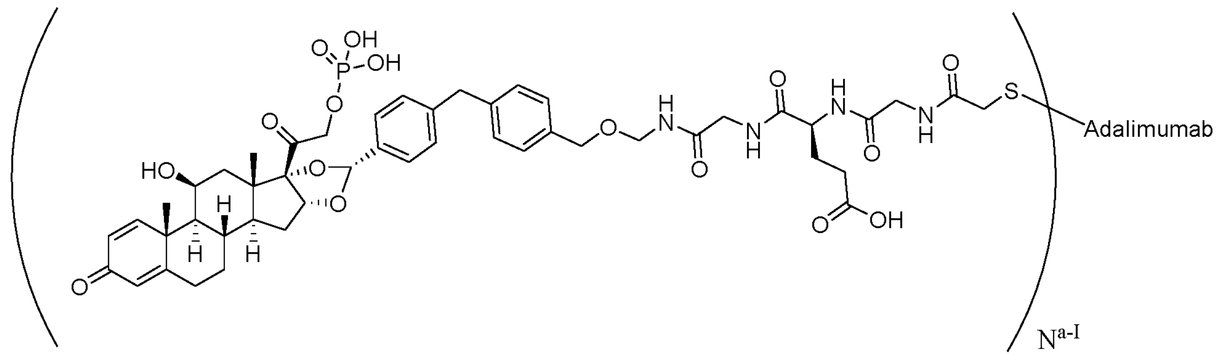 |
| V-22 | 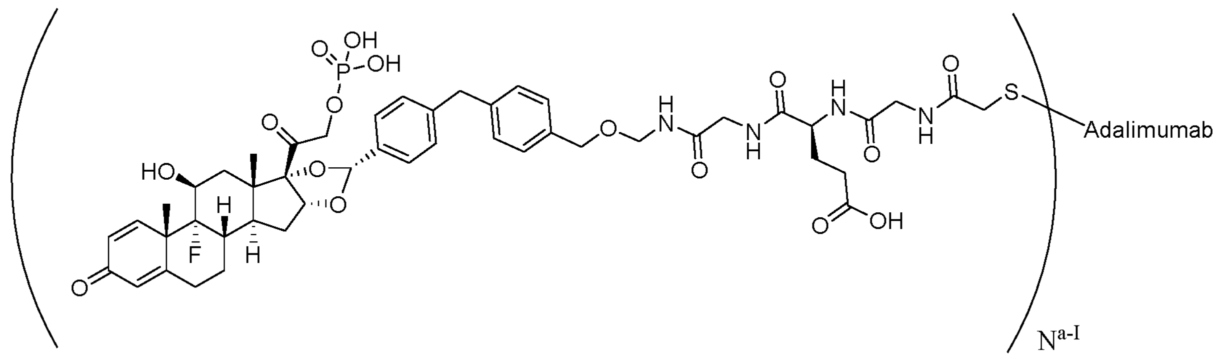 |
| V-25 | 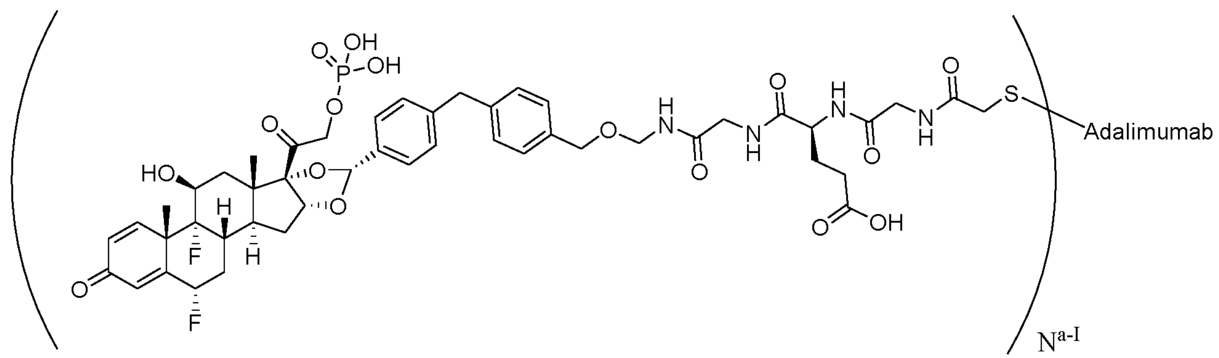 |

(continued)

| No. | Structure |
|---|---|
| V-29 | 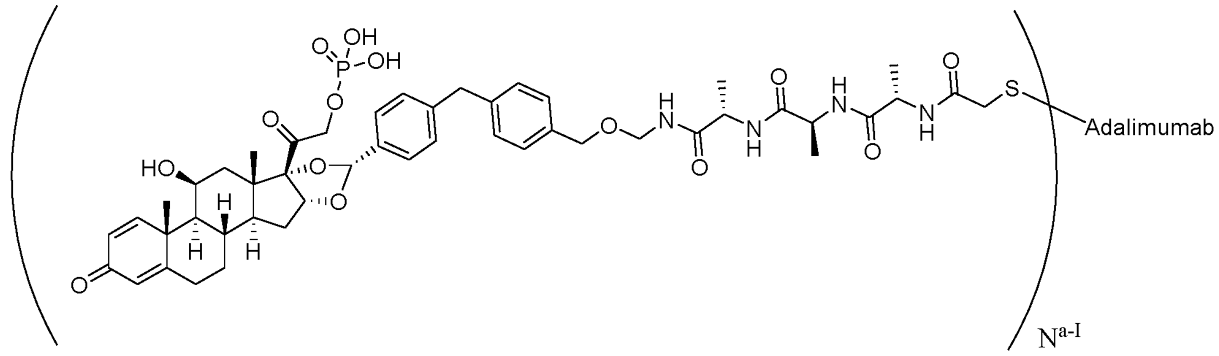 |
| V-30 | 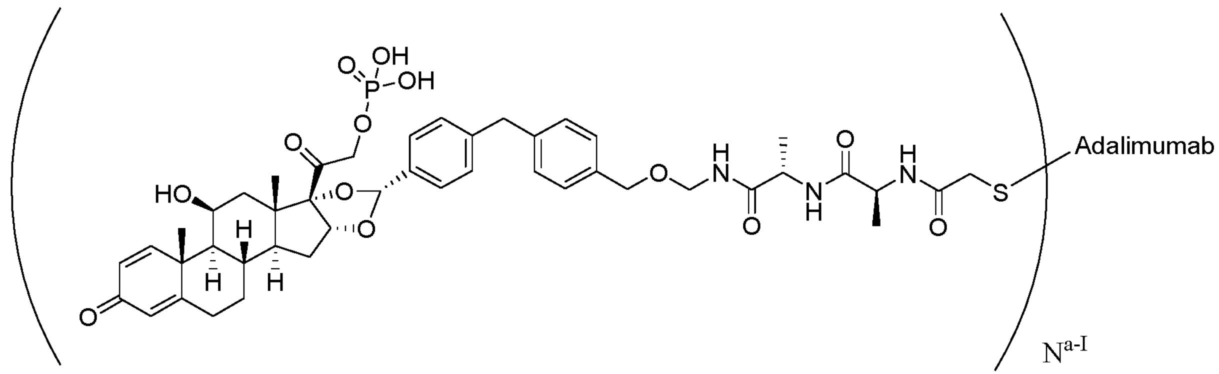 |
| V-34 | 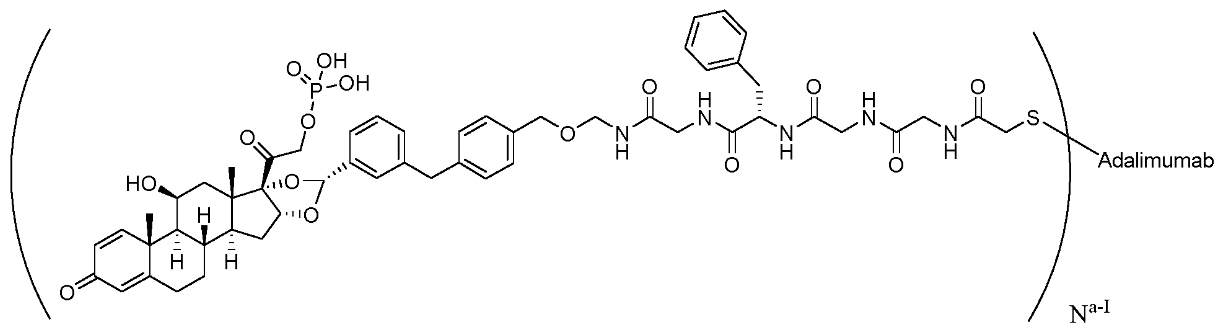 |

(continued)

| No. | Structure |
|---|---|
| V-35 | 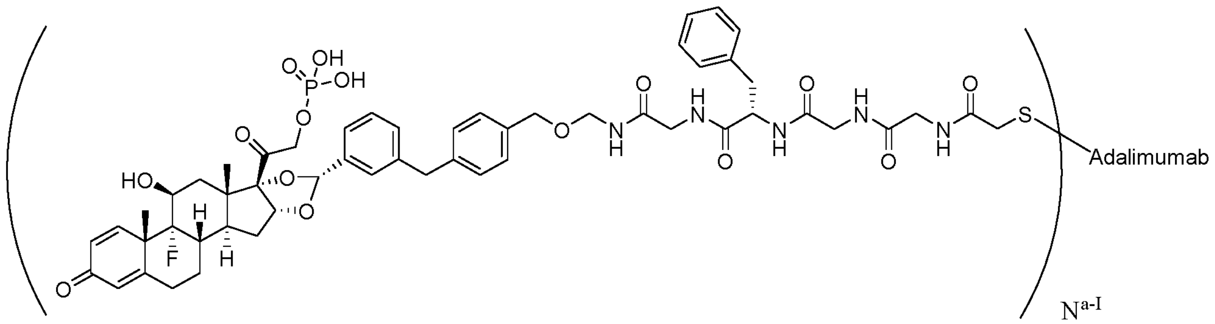 |
| V-39 | 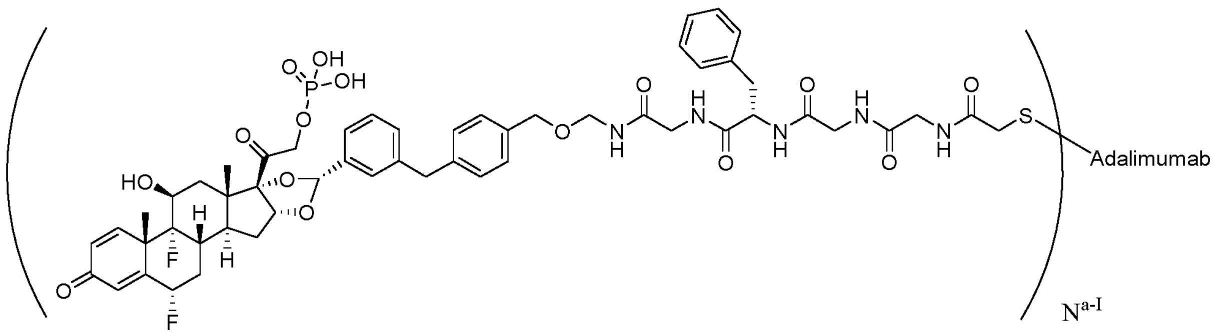 |
| V-43 | 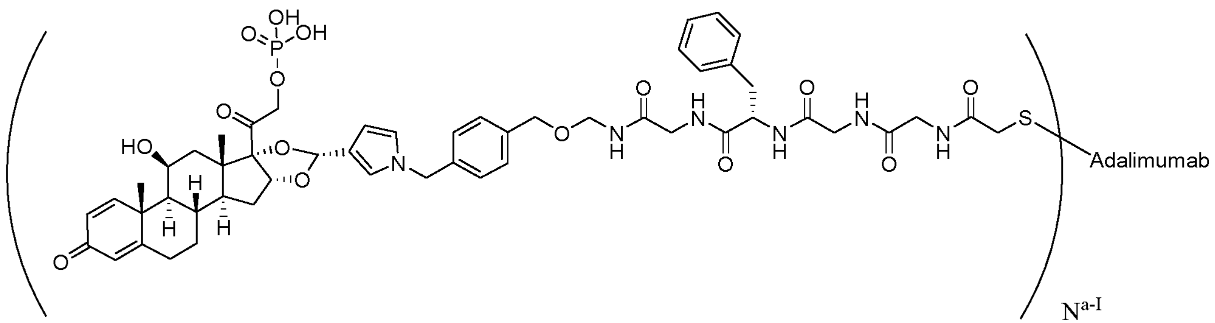 |

(continued)

| No. | Structure |
|---|---|
| V-44 | 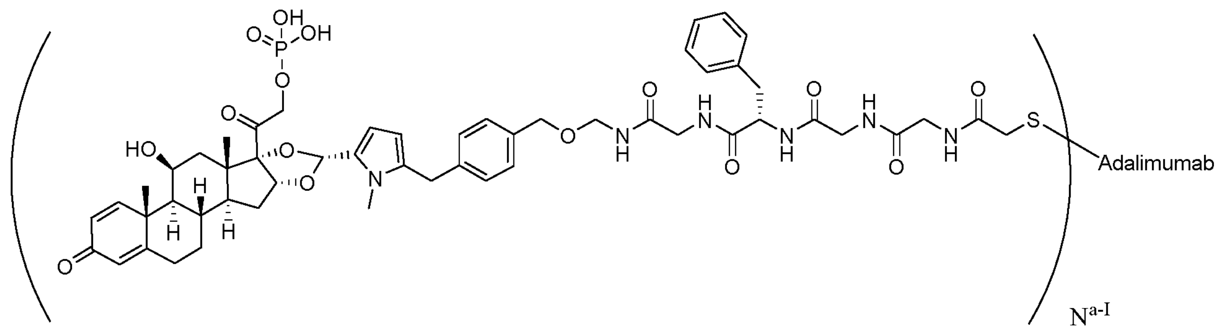 |
| V-45 | 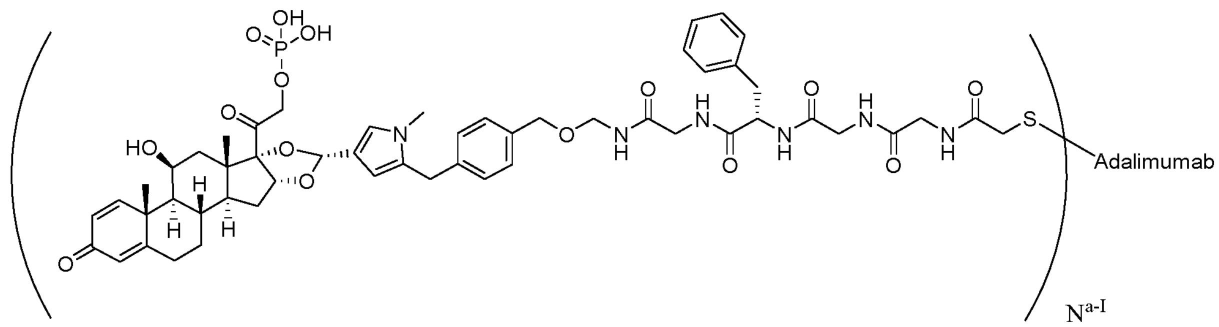 |
| V-46 | 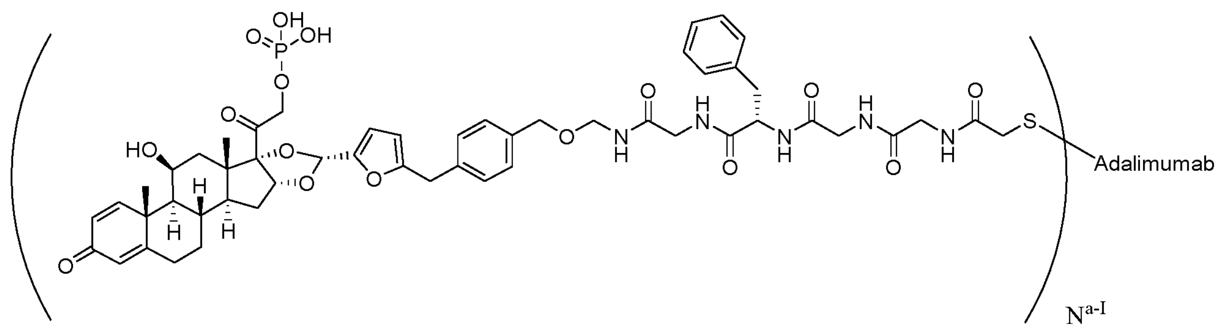 |

(continued)

| No. | Structure |
|---|---|
| V-47 | 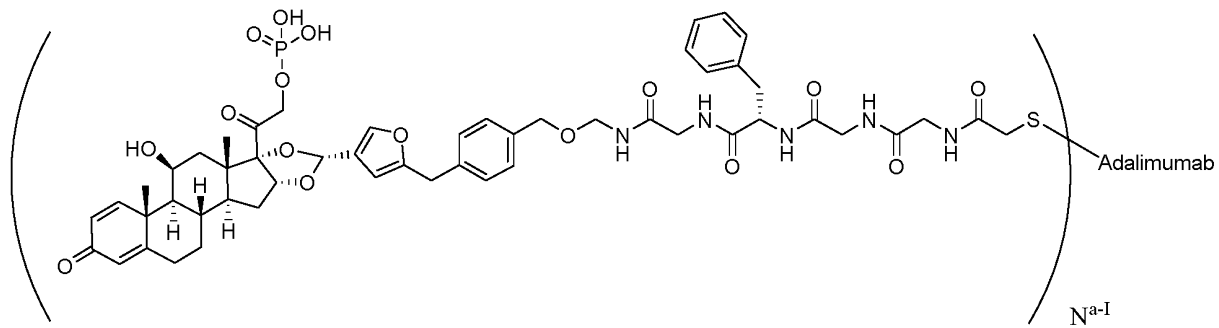 |
| V-48 | 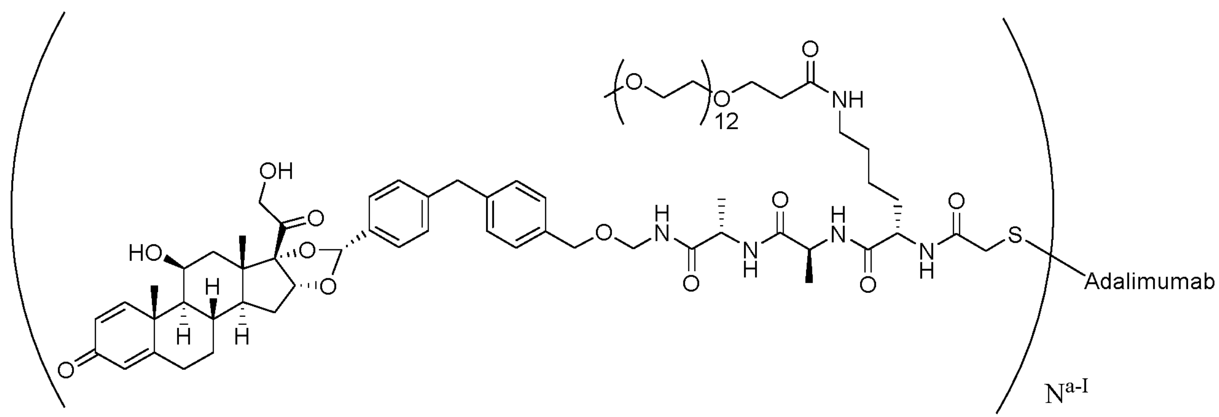 |

(continued)

| No. | Structure |
|---|---|
| V-49 | 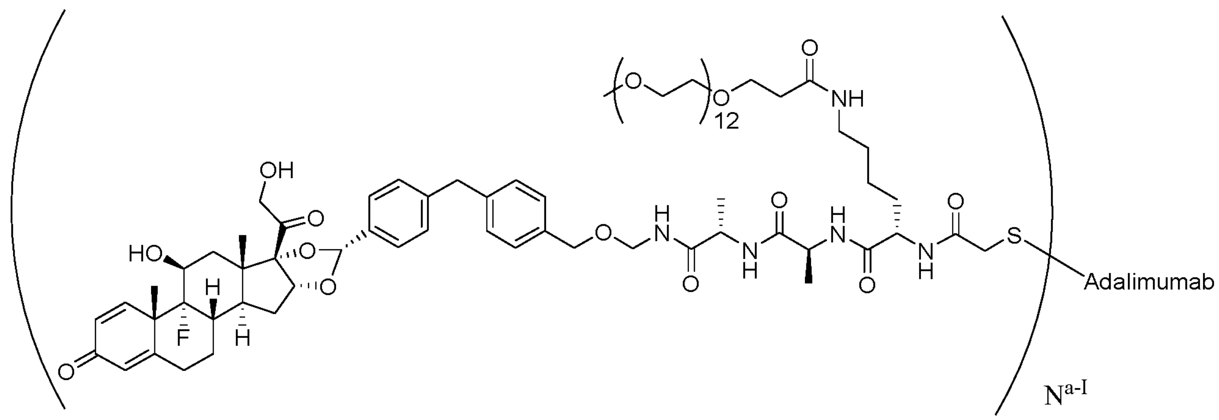 |
| V-50 | 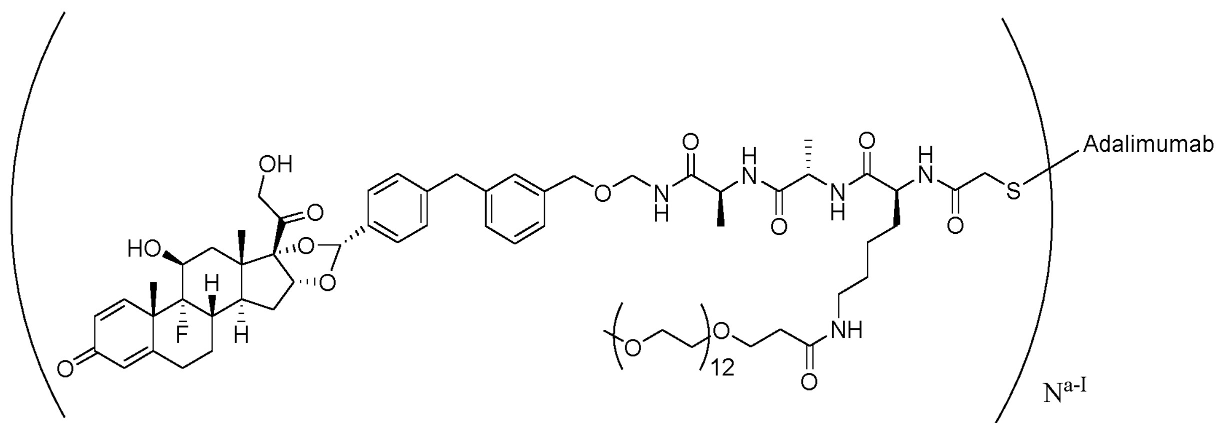 |

(continued)

| No. | Structure |
|---|---|
| V-51 | 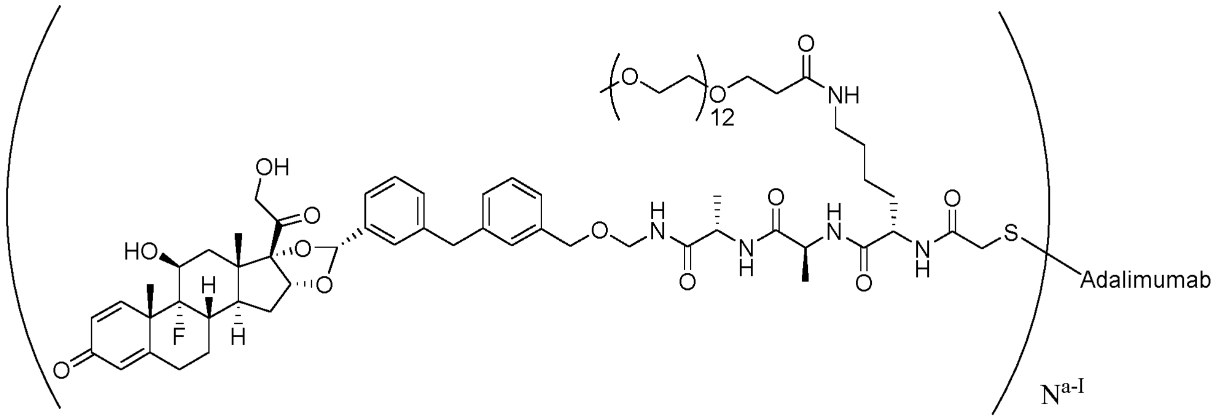 |
| V-52 | 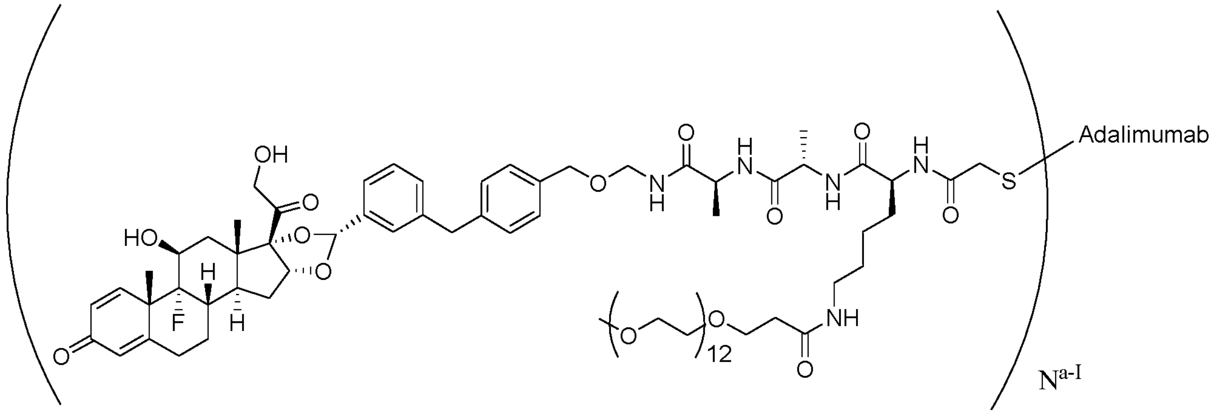 |

(continued)

| No. | Structure |
|---|---|
| V-53 | 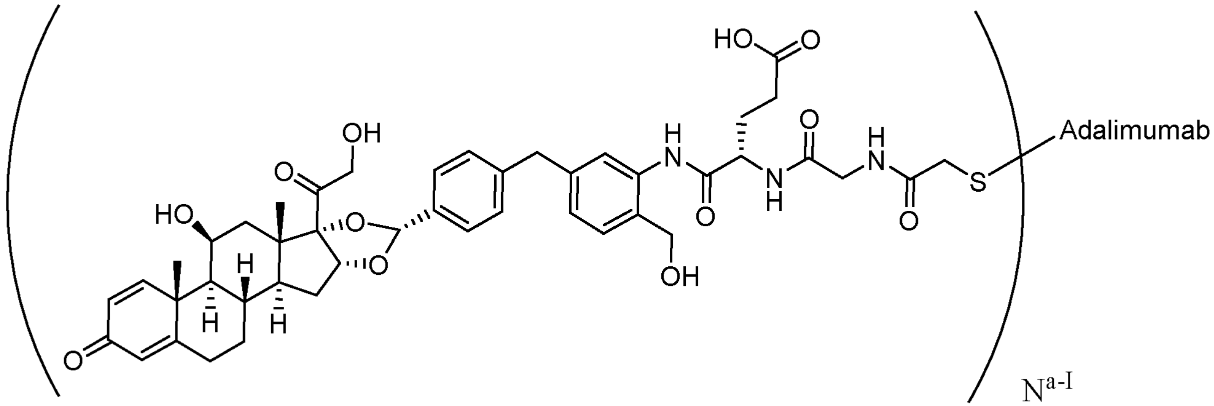 $N^{a-I}$ |
| V-54 | 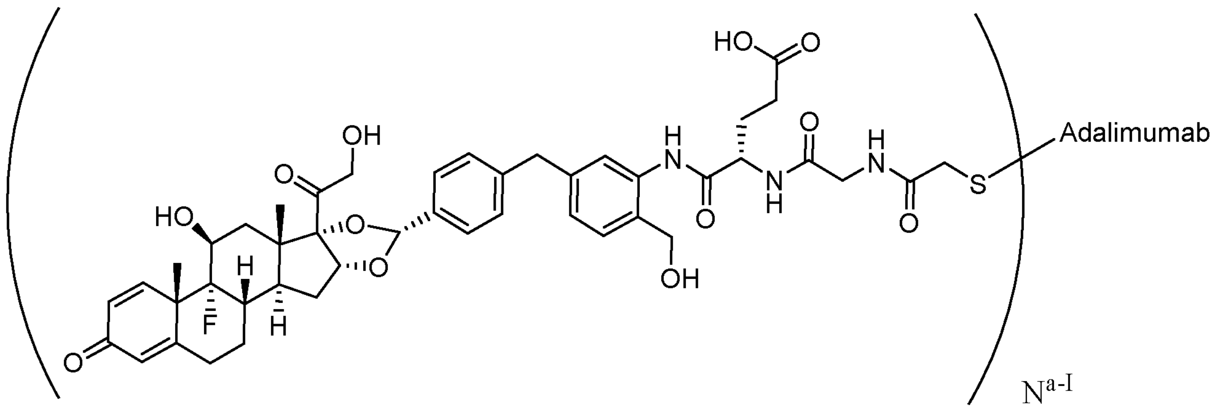 $N^{a-I}$ |

(continued)

| No. | Structure |
|---|---|
| V-55 | 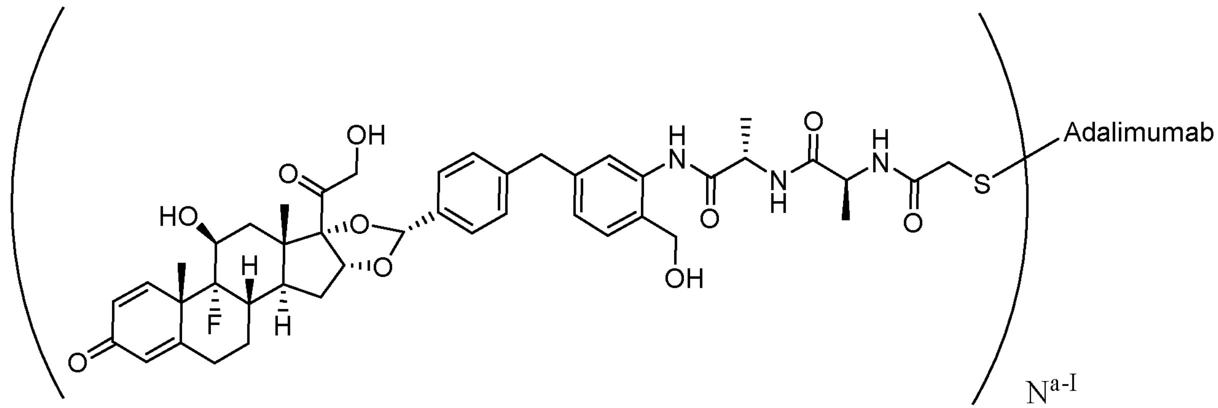 |
| V-56 | 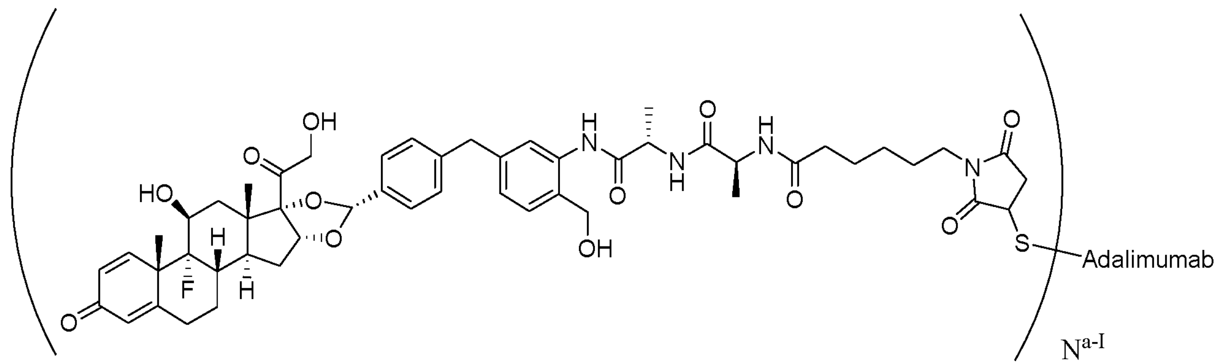 |

(continued)

| No. | Structure |
|---|---|
| V-57 | 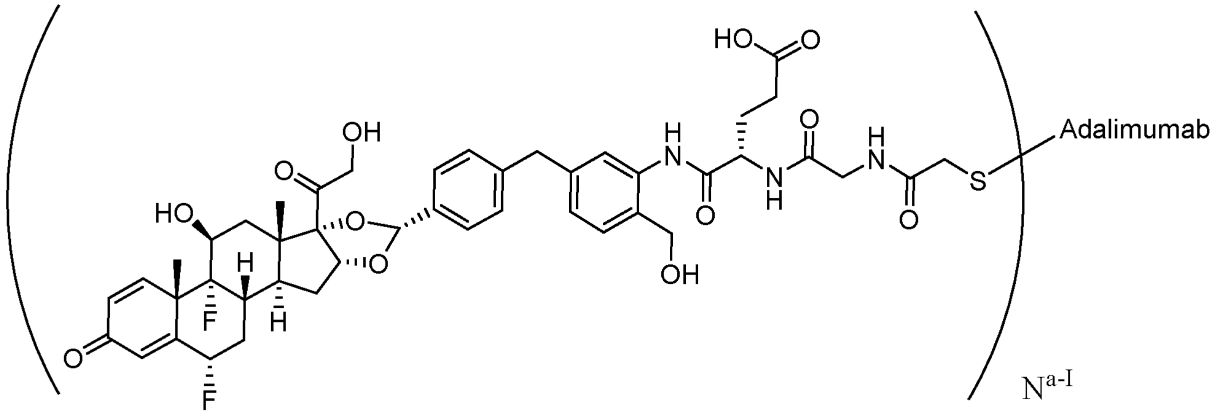 |
| V-58 | 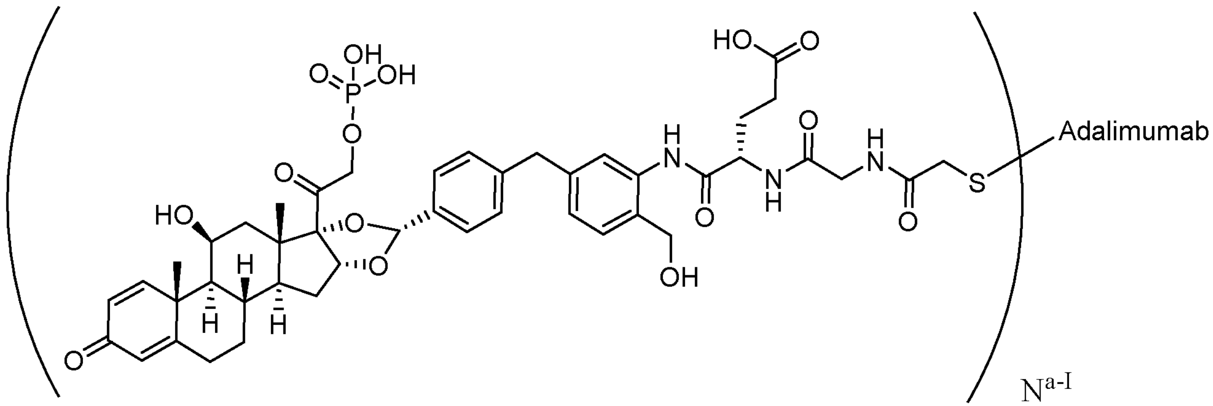 |

(continued)

| No. | Structure |
|---|---|
| V-59 | 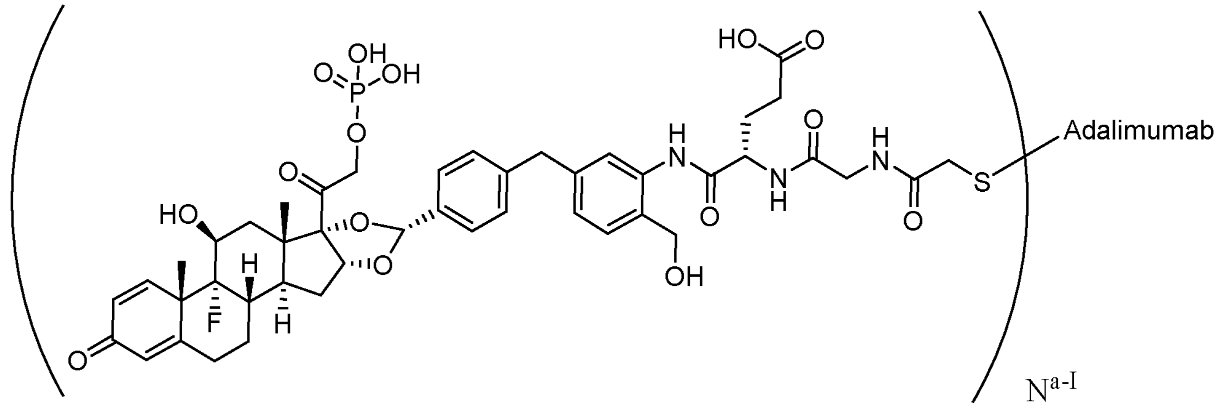 |
| V-60 | 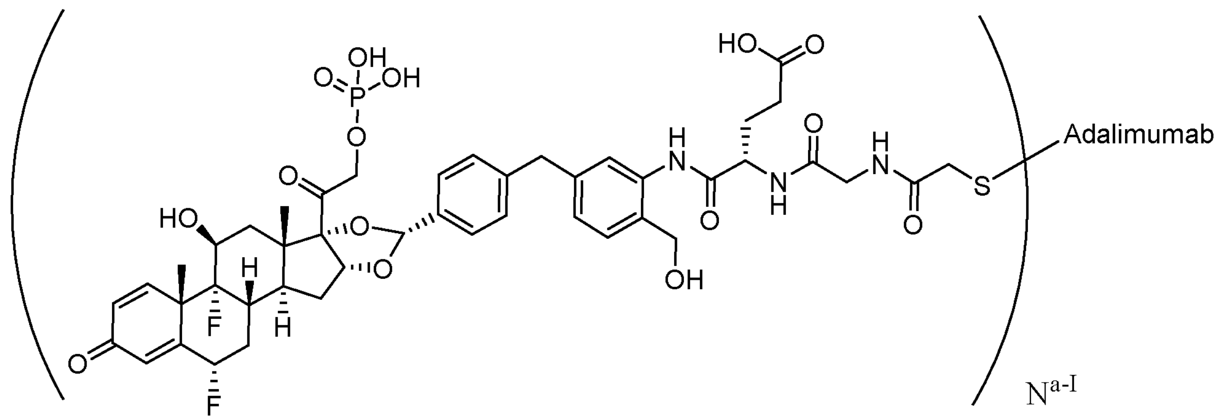 |

(continued)

| No. | Structure |
|---|---|
| V-70 | 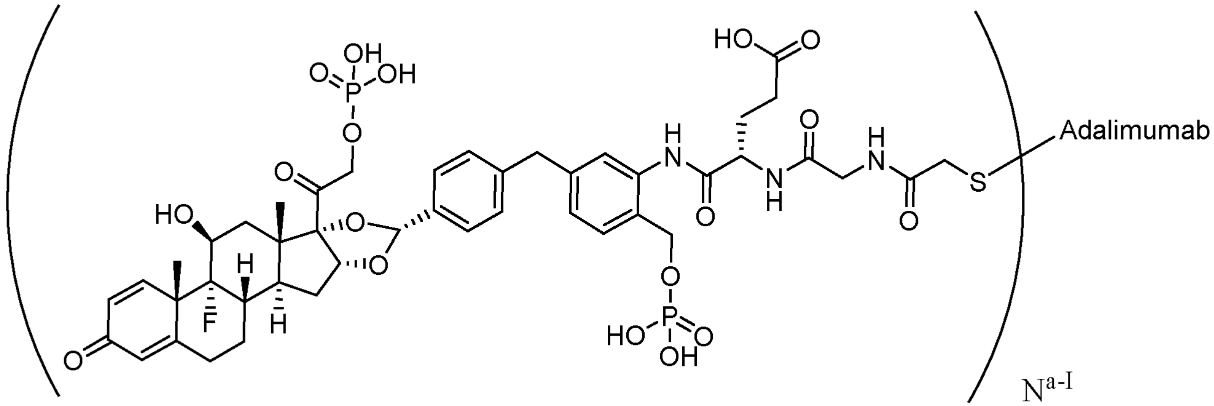 |
| V-74 | 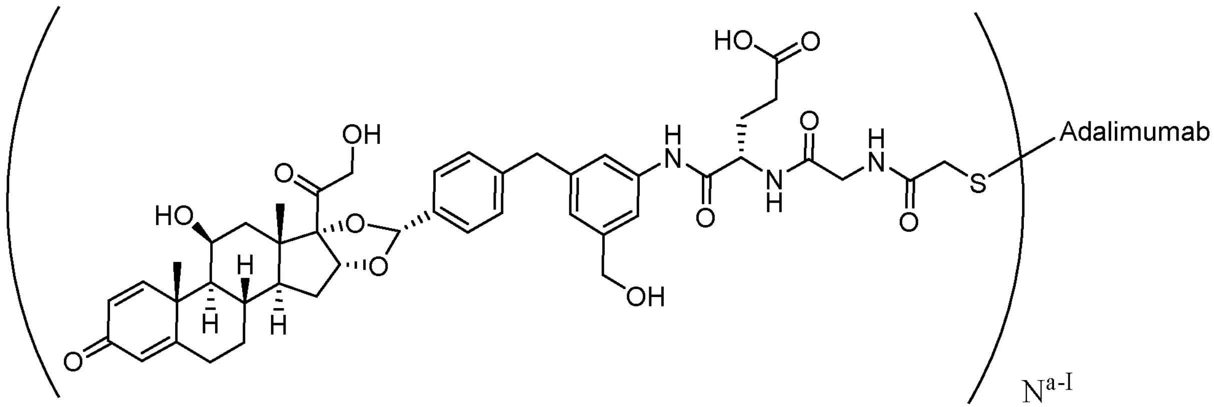 |

(continued)

| No. | Structure |
|---|---|
| V-75 | 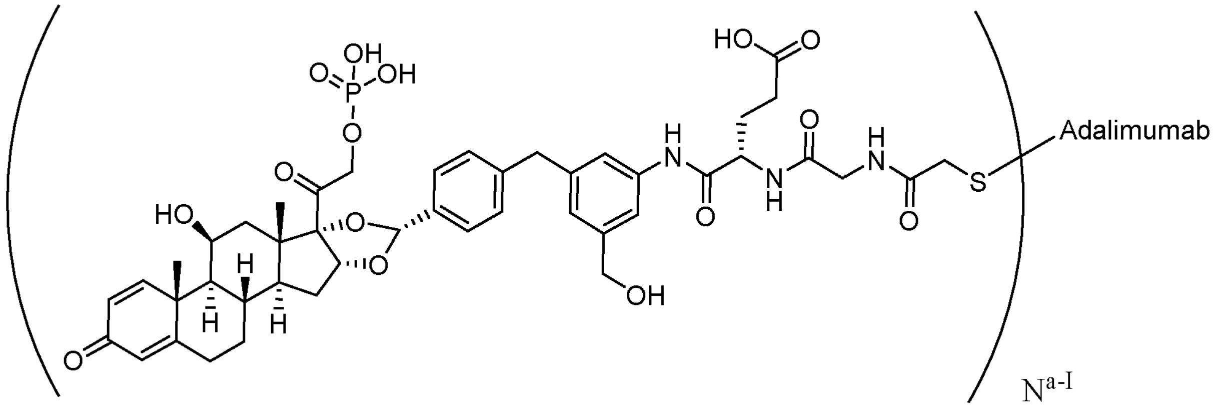 |
| V-78 | 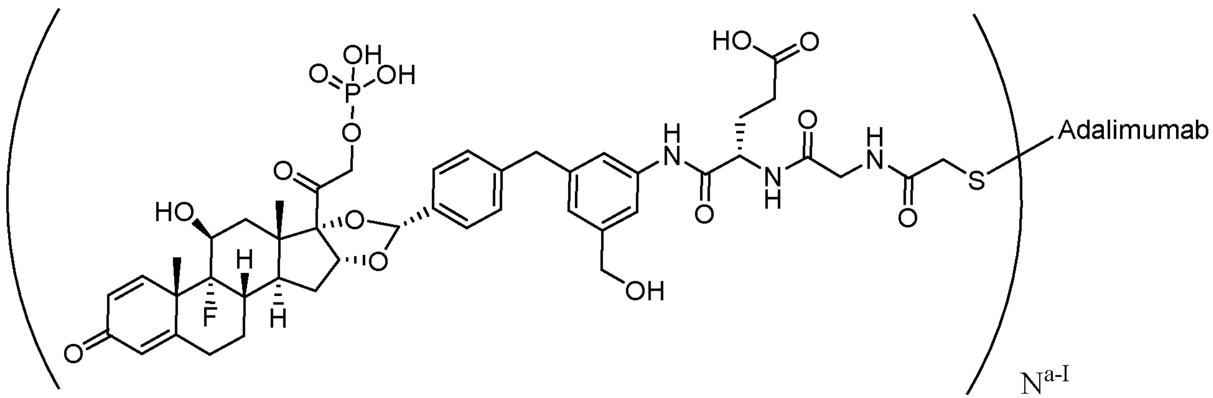 |

(continued)

| No. | Structure |
|---|---|
| V-81 | 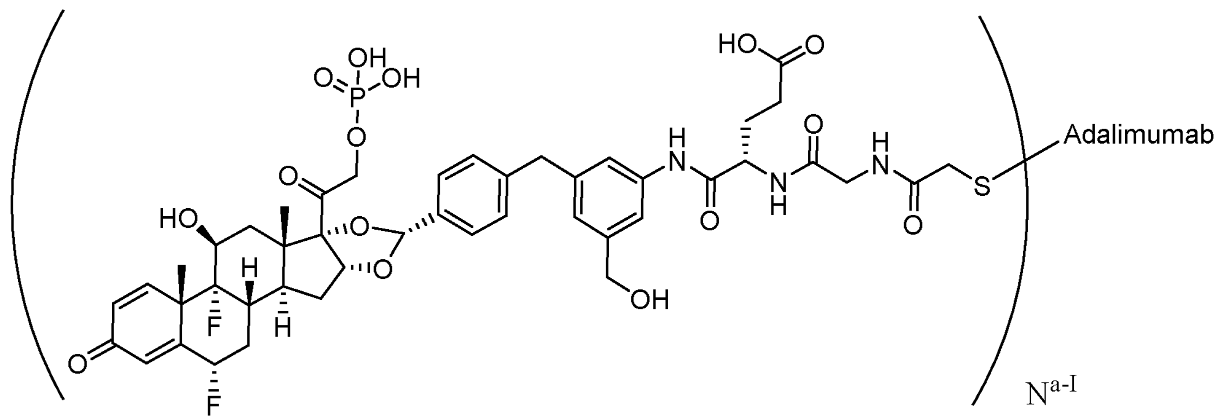 |
| V-84 | 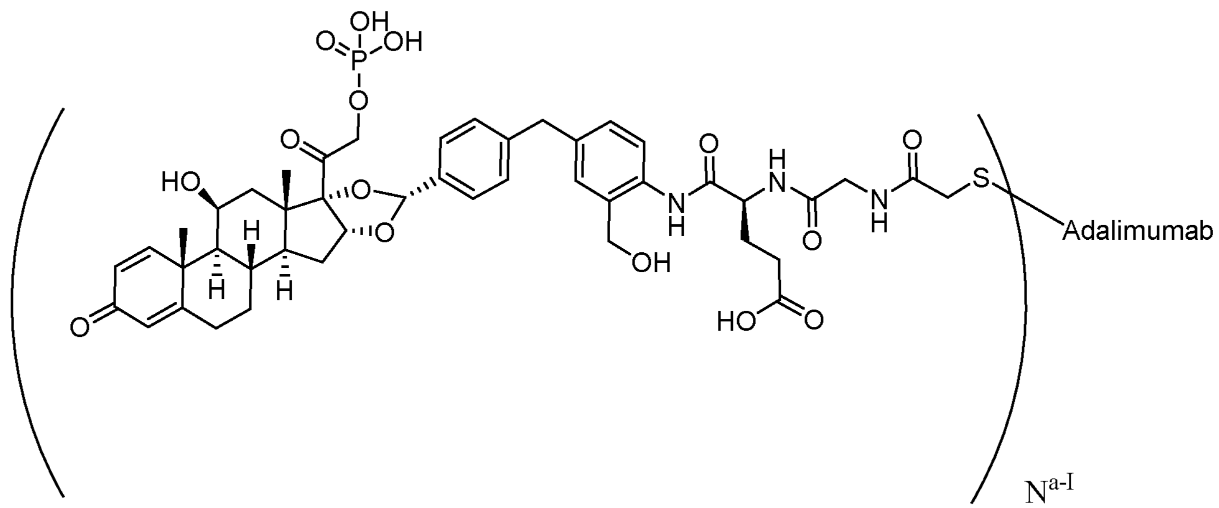 |

(continued)

| No. | Structure |
|---|---|
| V-87 | 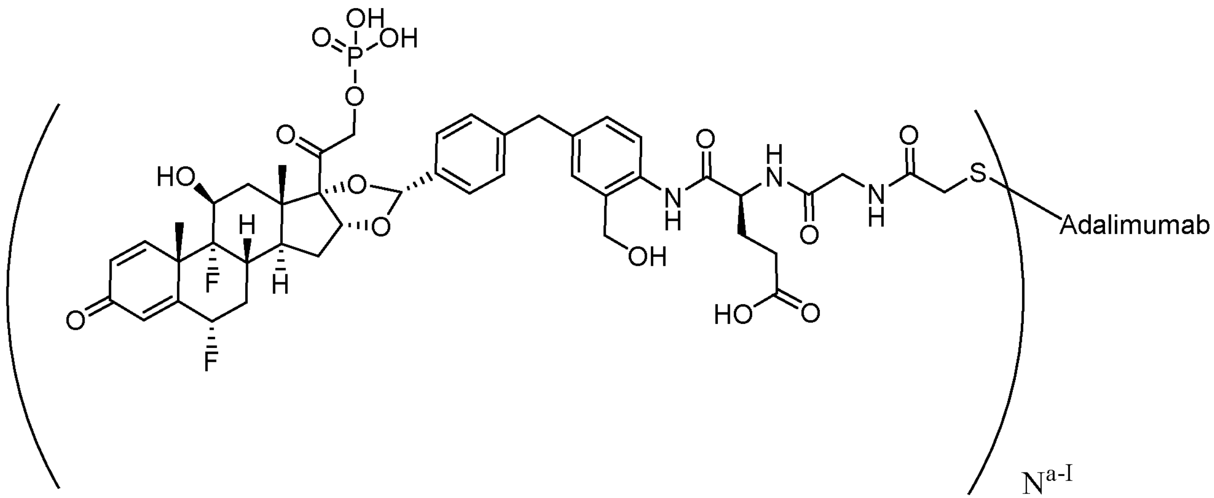 |
| V-90 | 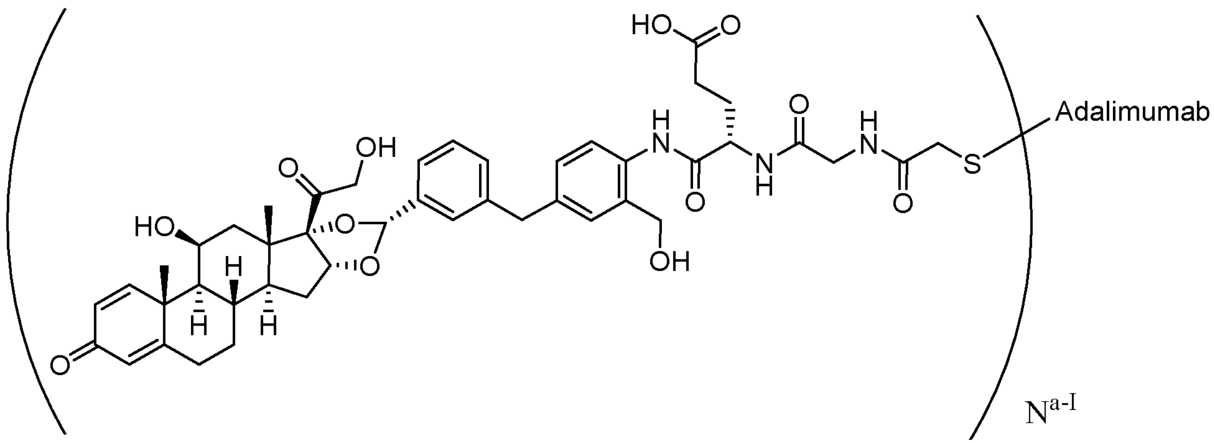 |

(continued)

| No. | Structure |
|---|---|
| V-91 | 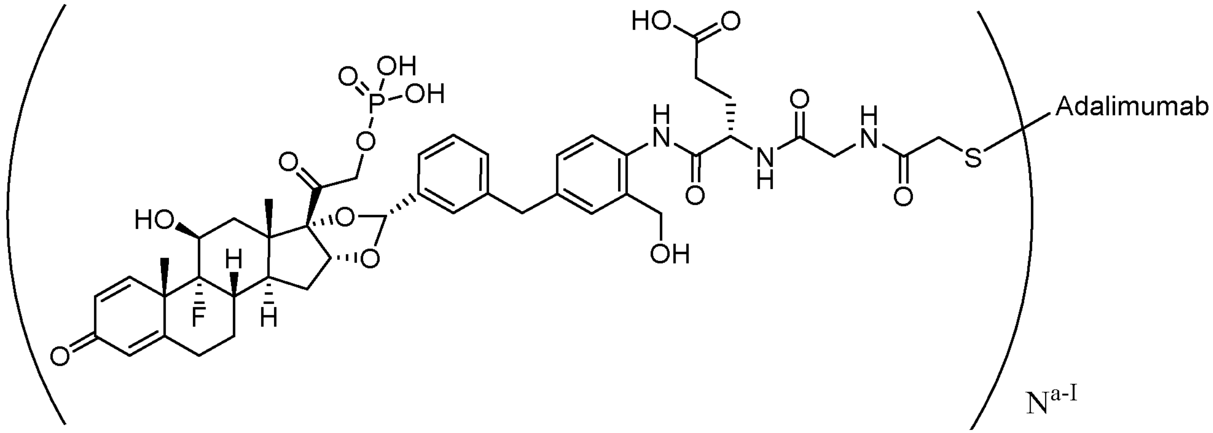 |
| V-92 | 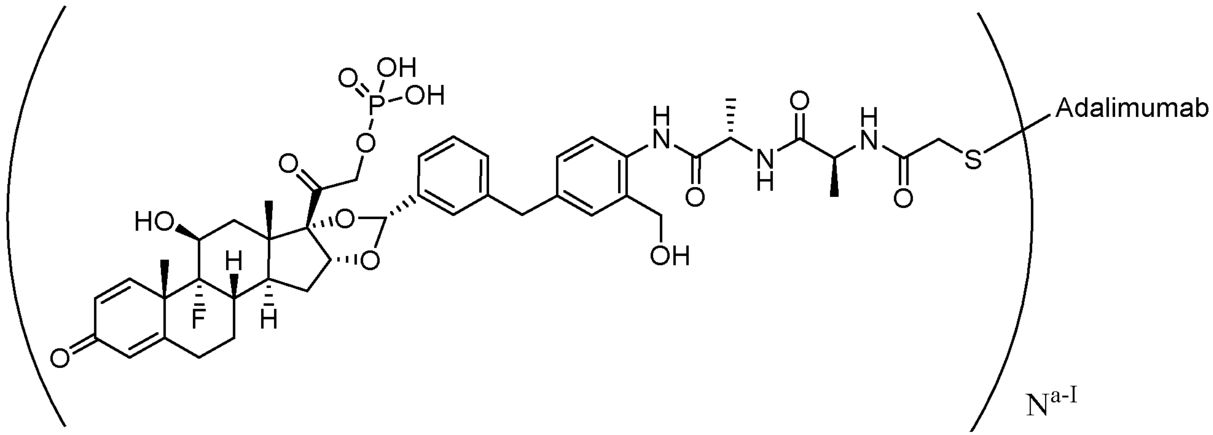 |

(continued)

| No. | Structure |
|---|---|
| V-95 | 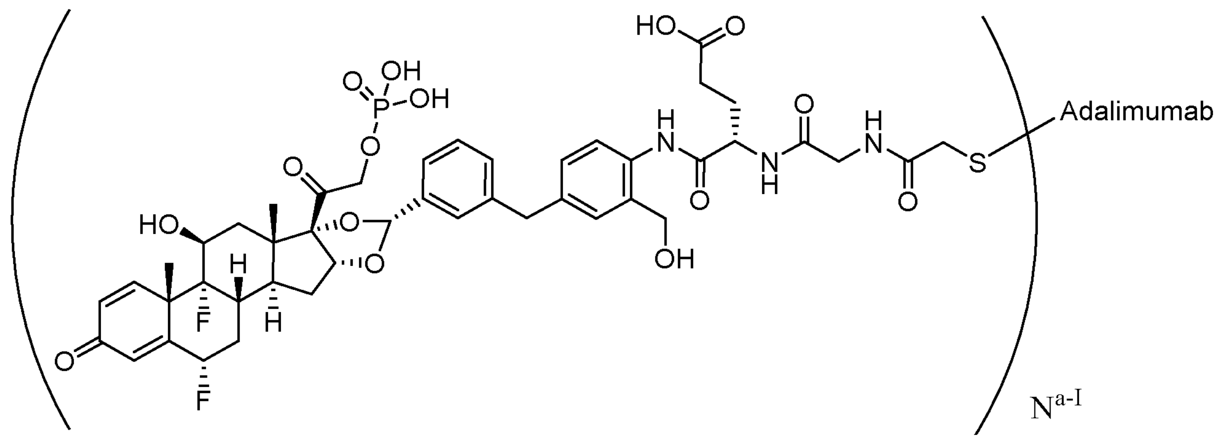 |
| V-98 | 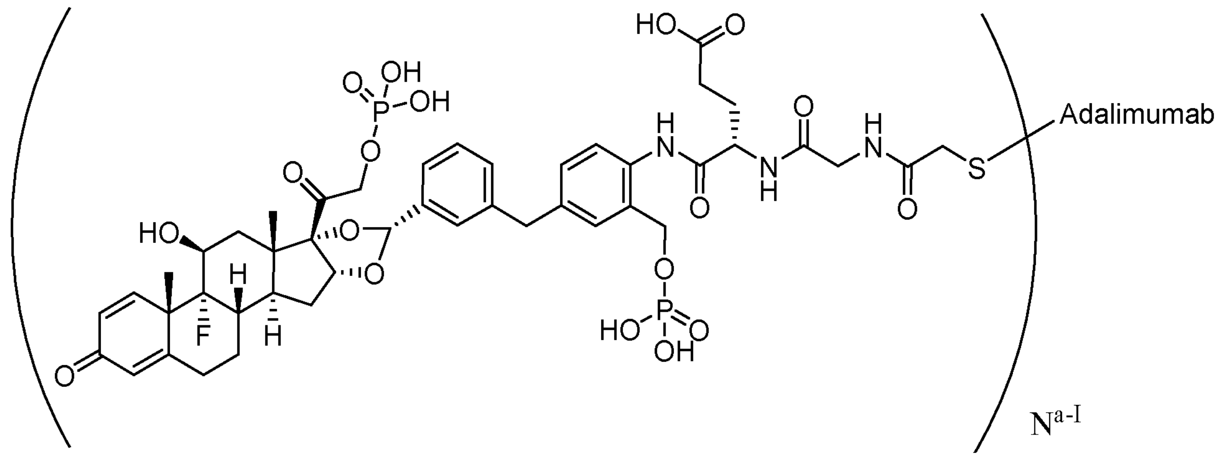 |

(continued)

| No. | Structure |
|---|---|
| V-102 | 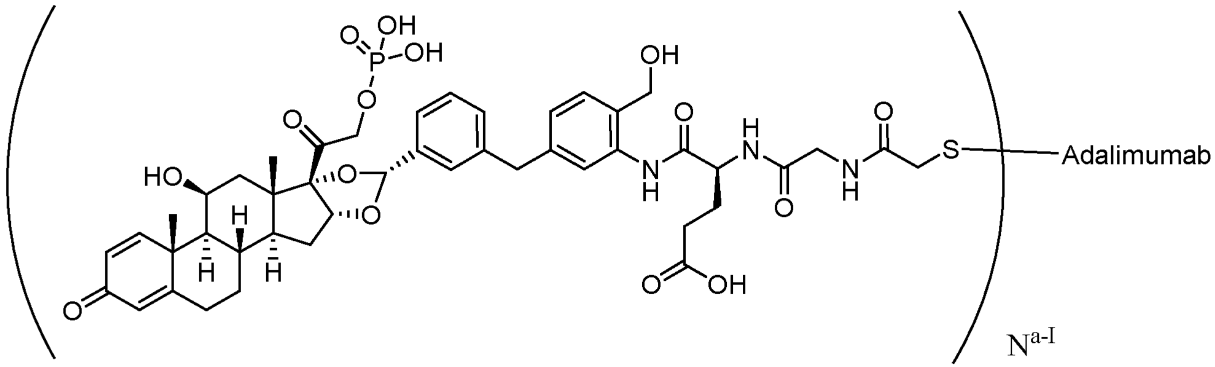 |
| V-103 | 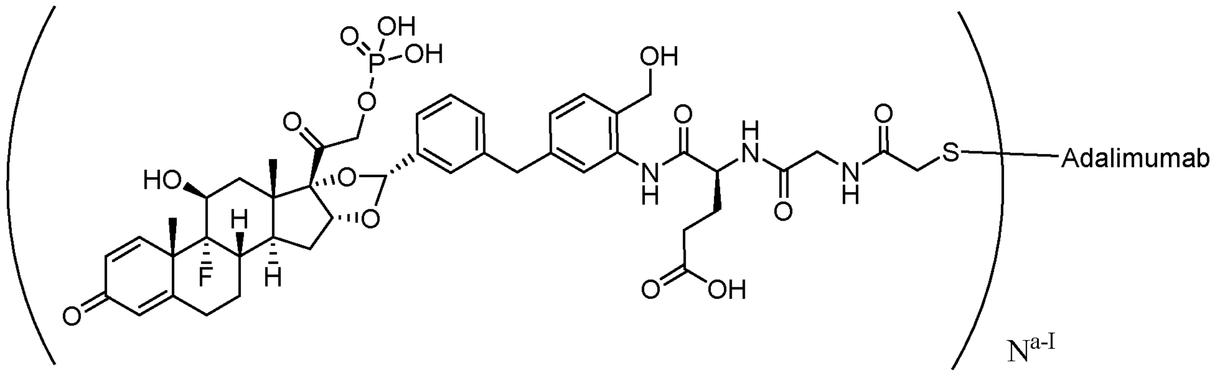 |

(continued)

| No. | Structure |
|---|---|
| V-104 | 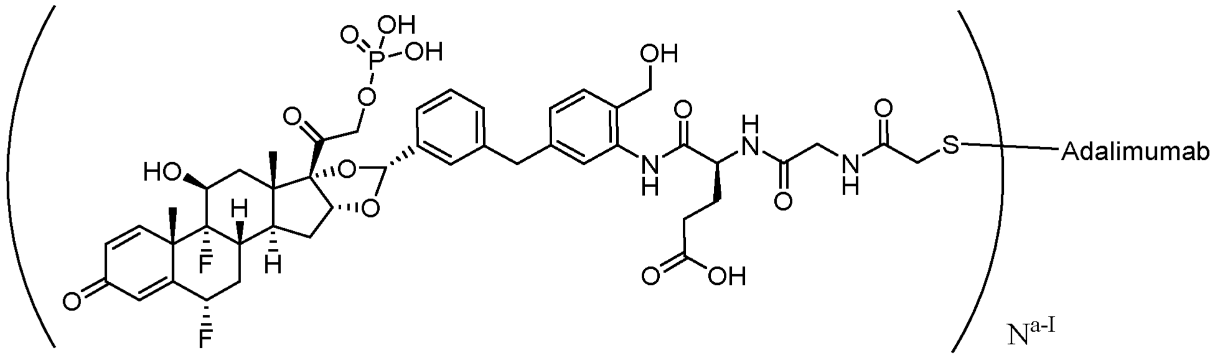 |
| V-105 | 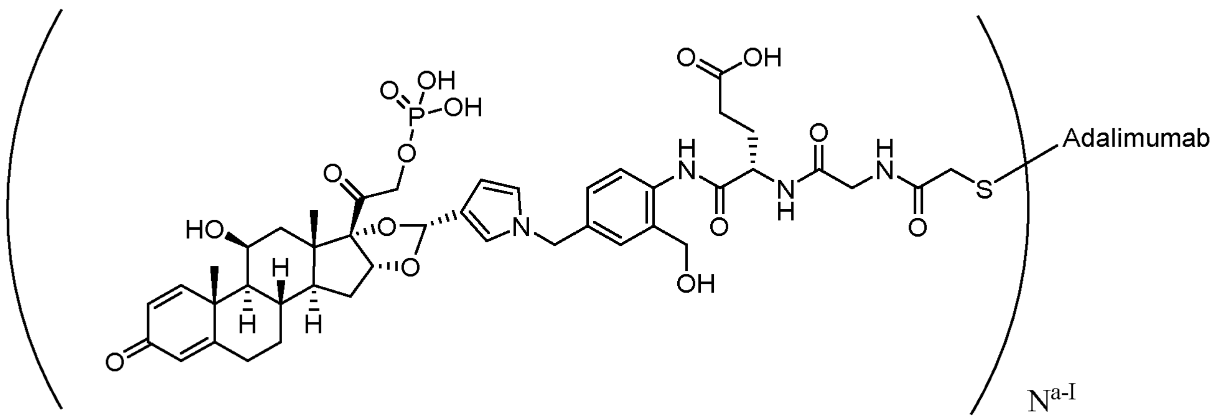 |

(continued)

| No. | Structure |
|---|---|
| V-106 | 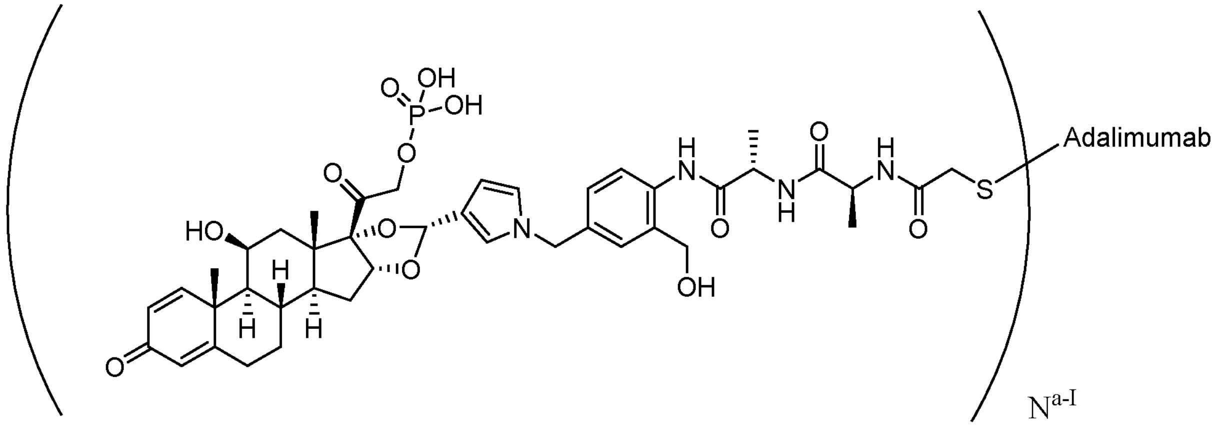 |
| V-109 | 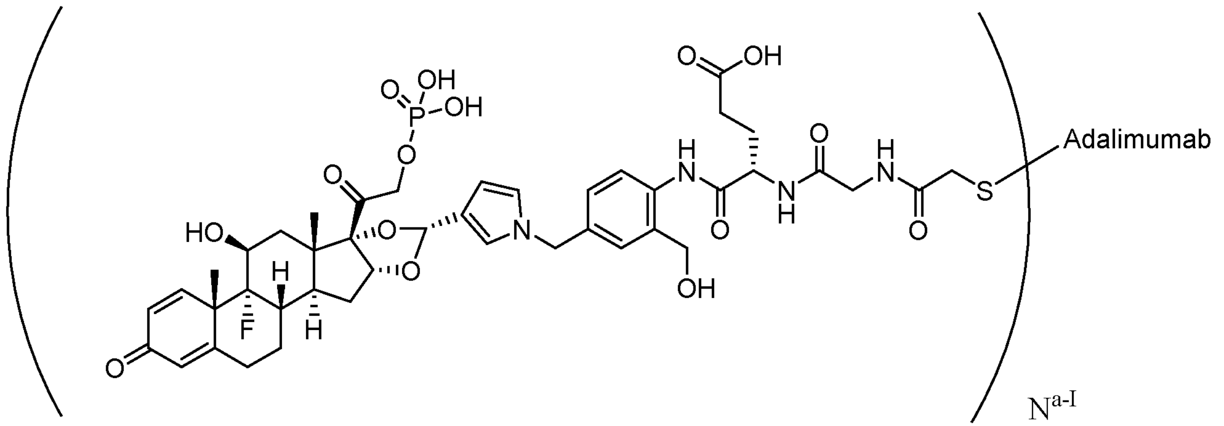 |

(continued)

| No. | Structure |
|---|---|
| V-110 | 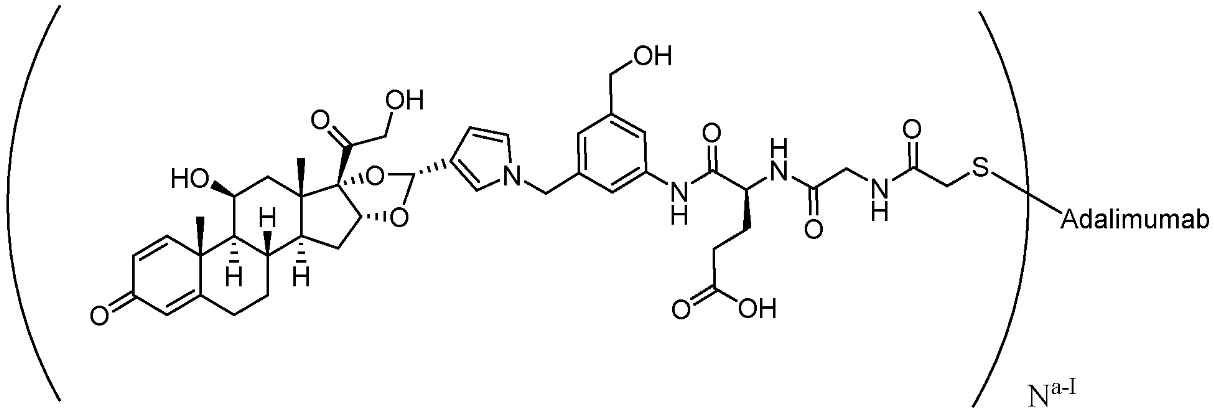 |
| V-111 | 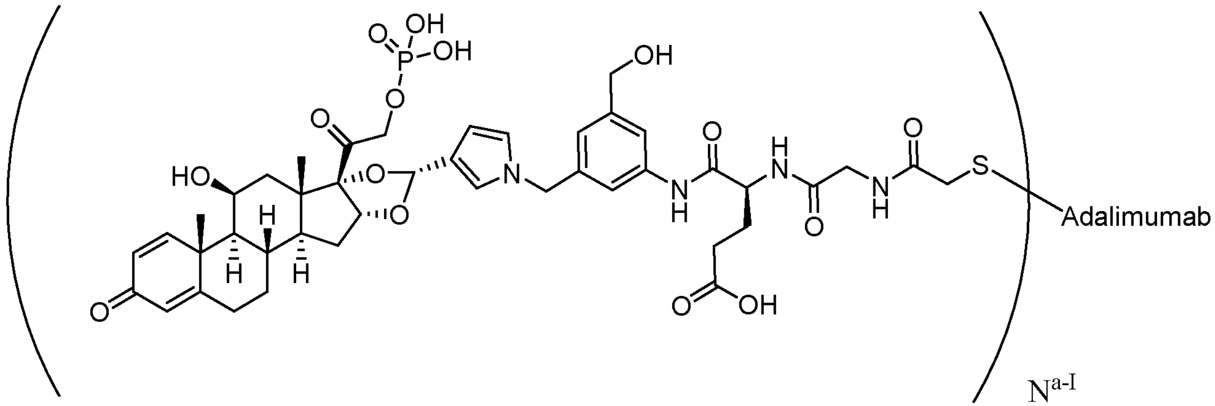 |

(continued)

| No. | Structure |
|---|---|
| V-112 | 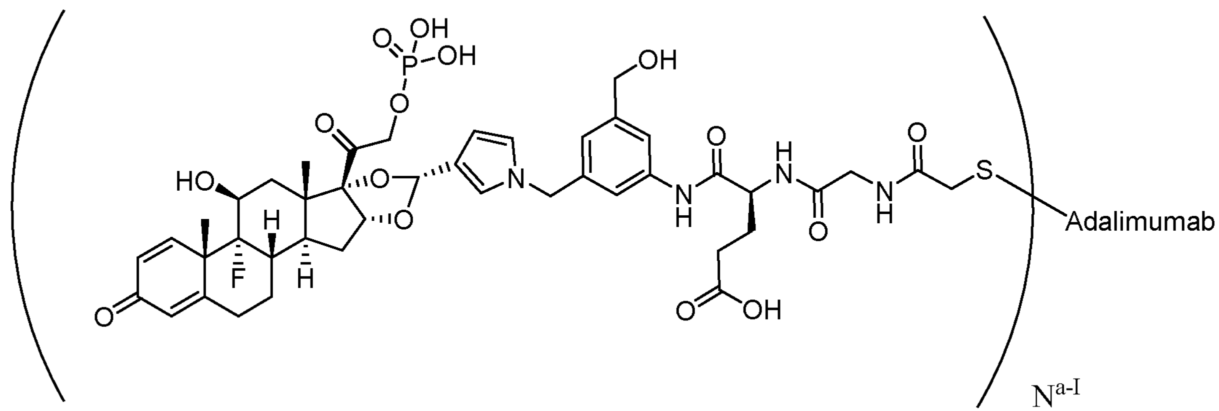 |
| V-113 | 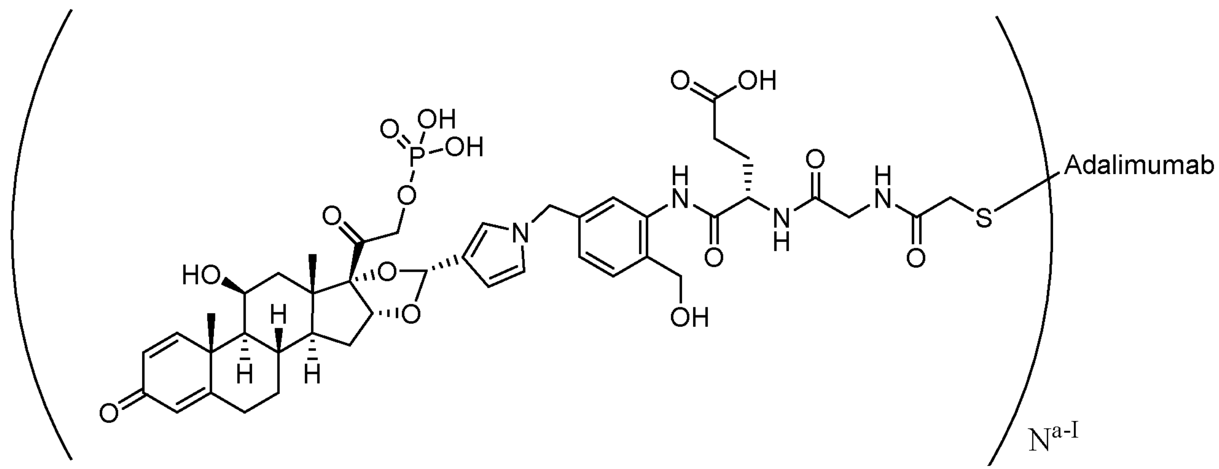 |

(continued)

| No. | Structure |
|---|---|
| V-114 | 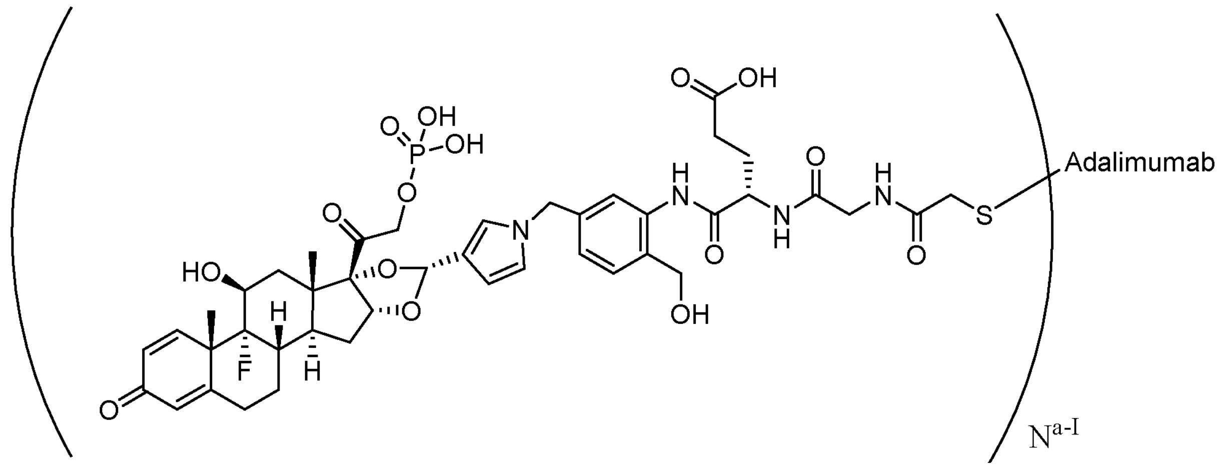 |
| V-120 | 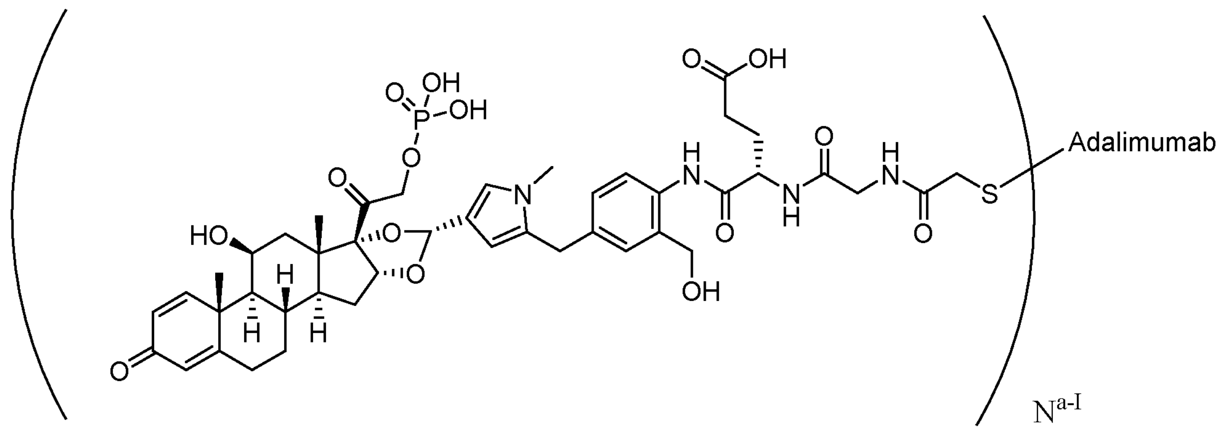 |

(continued)

| No. | Structure |
|---|---|
| V-121 | 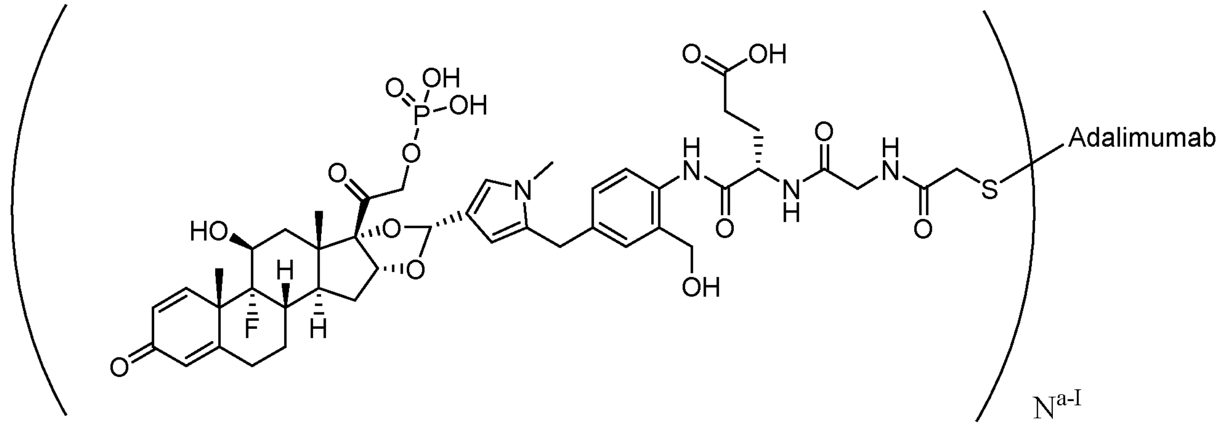 |
| V-122 | 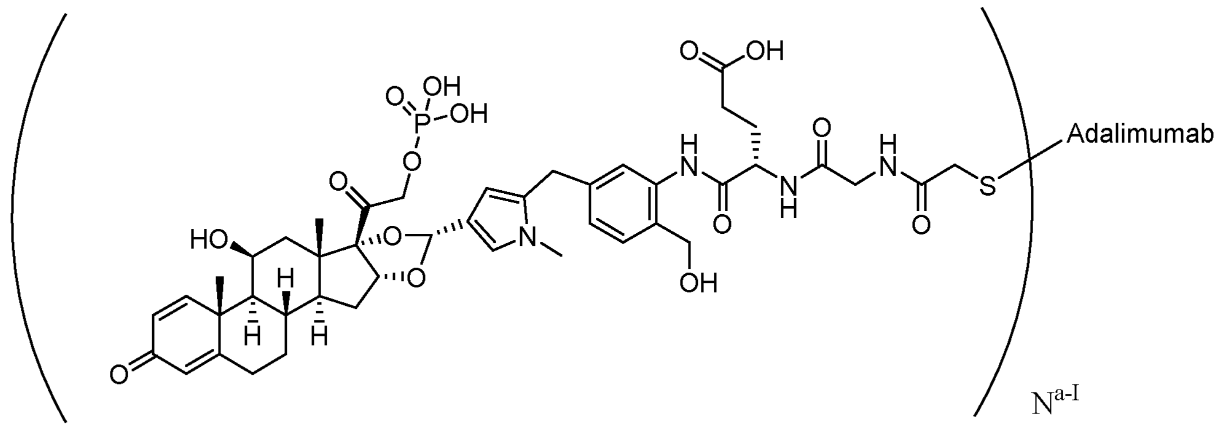 |

(continued)

| No. | Structure |
|---|---|
| V-123 | 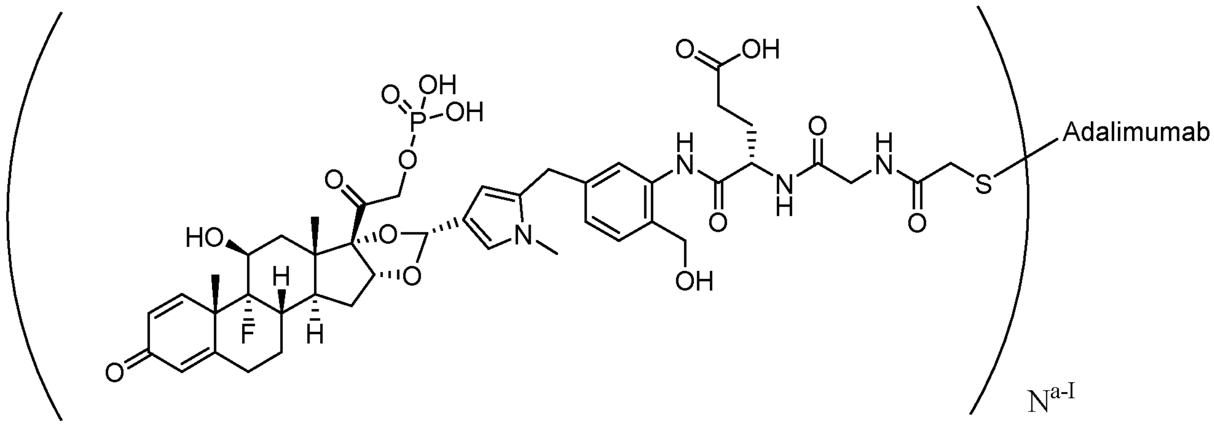 |
| V-124 | 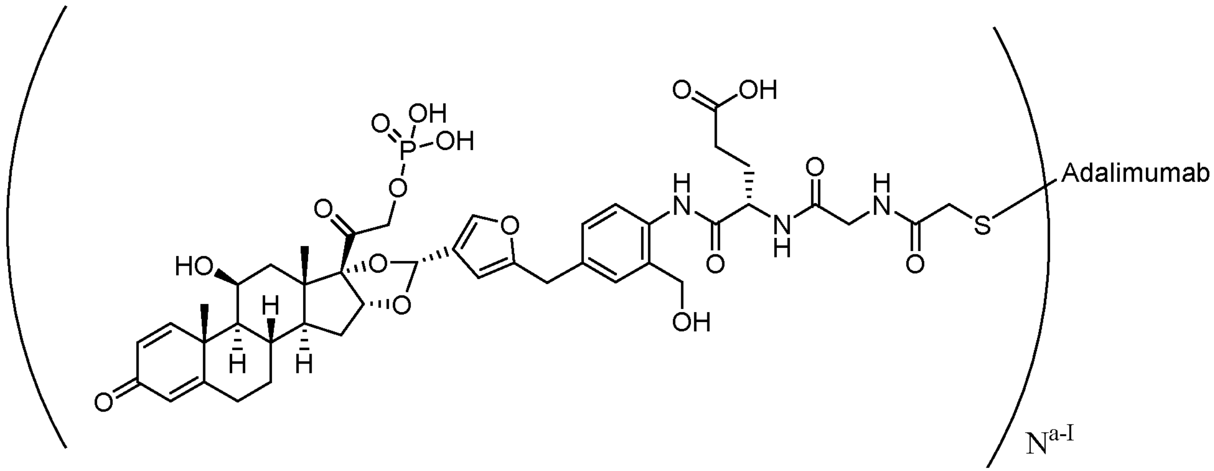 |

(continued)

| No. | Structure |
|---|---|
| V-125 | 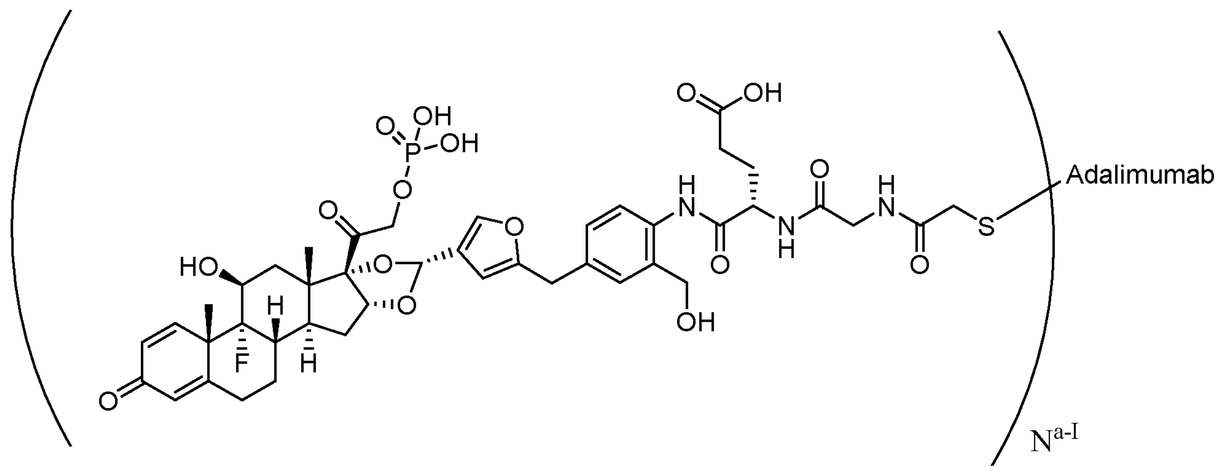 |
| V-126 | 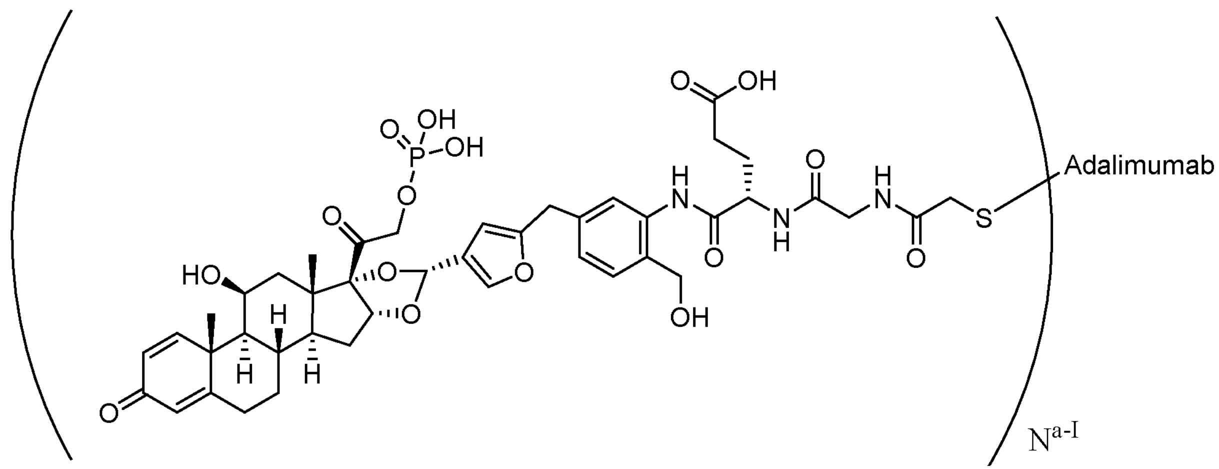 |

(continued)

| No. | Structure |
|---|---|
| V-127 | 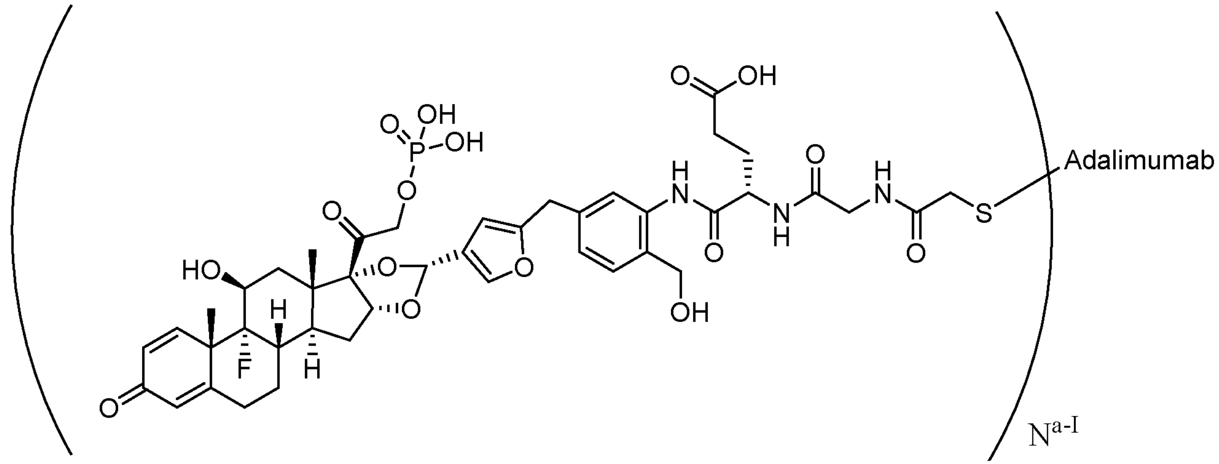 |
| V-128 | 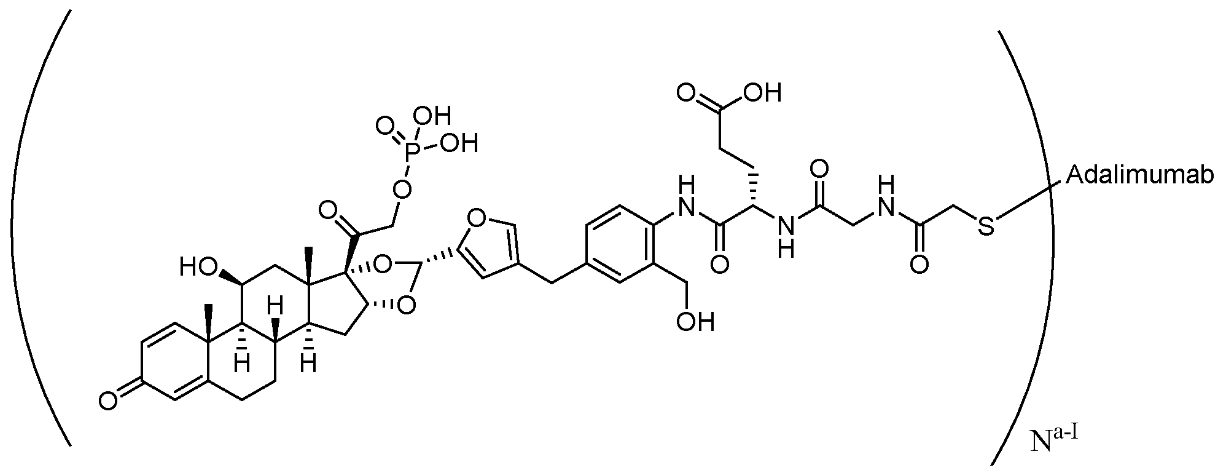 |

(continued)

| No. | Structure |
|---|---|
| V-129 | 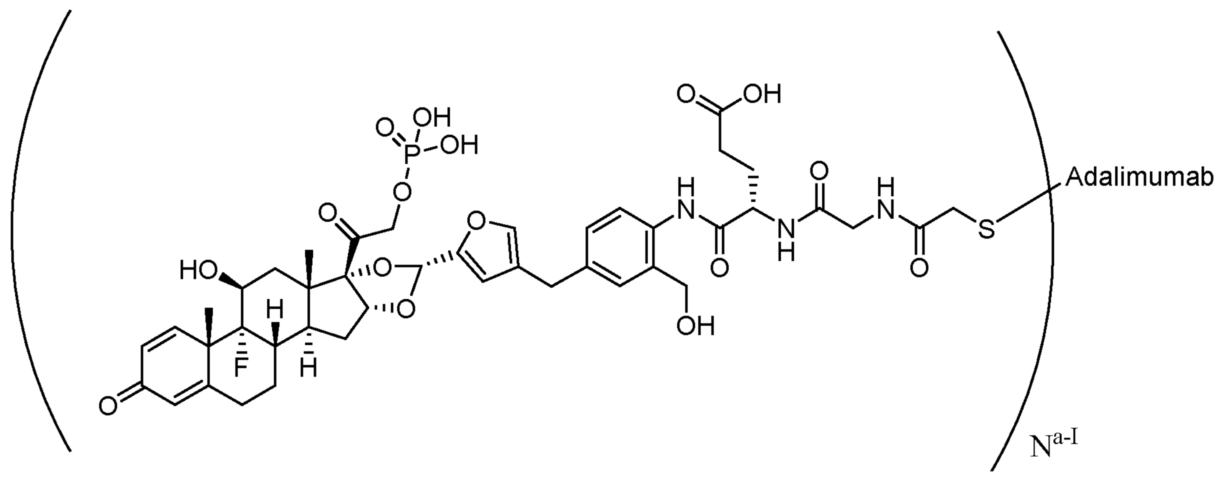 |
| V-130 | 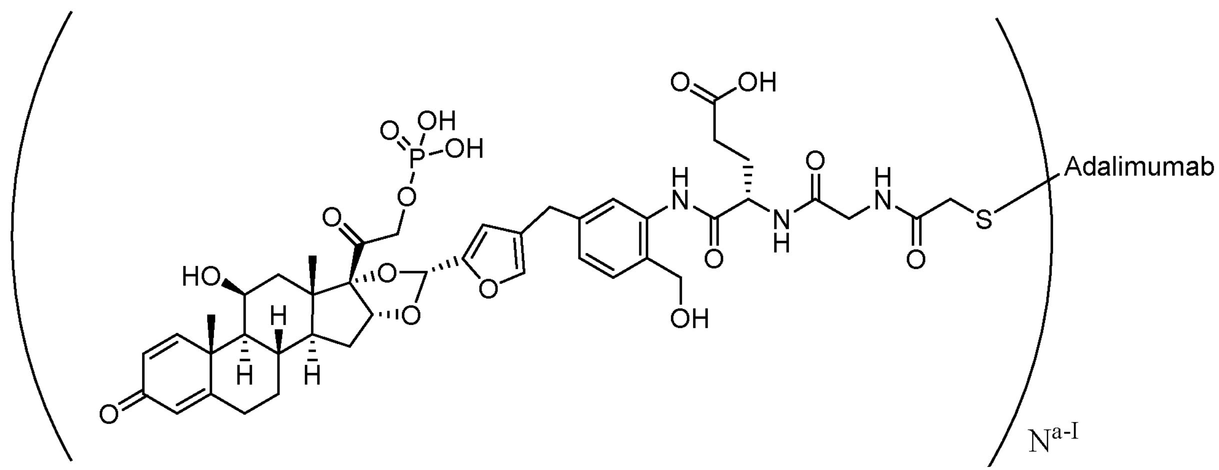 |

(continued)

| No. | Structure |
|---|---|
| V-131 | 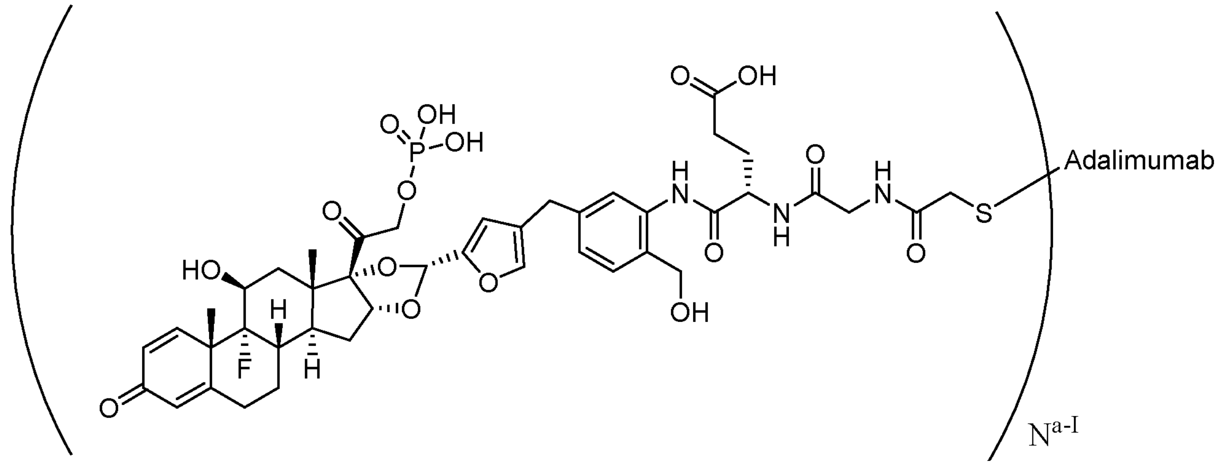 |
| V-132 | 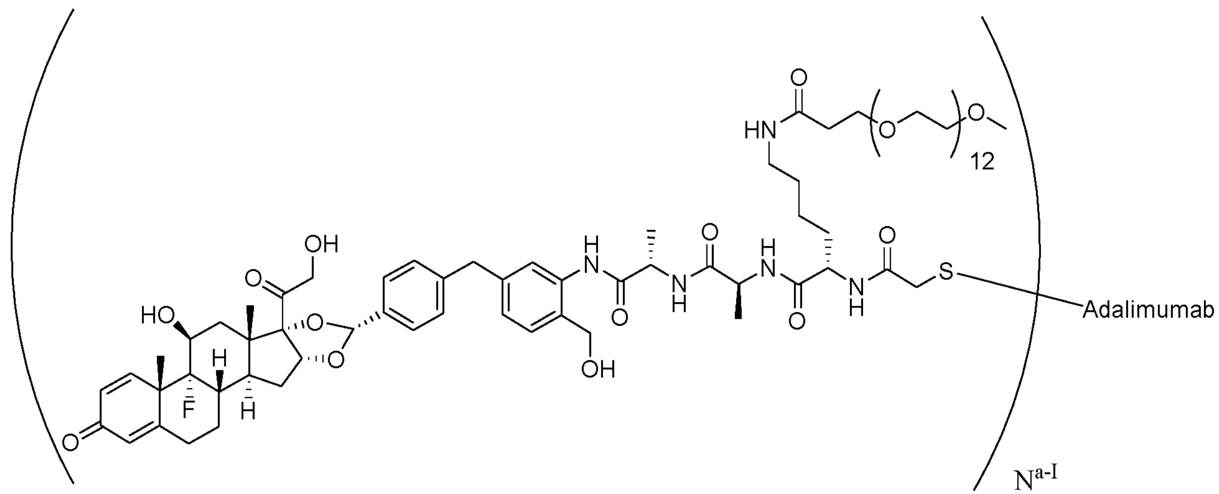 |

(continued)

| No. | Structure |
|---|---|
| V-133 | 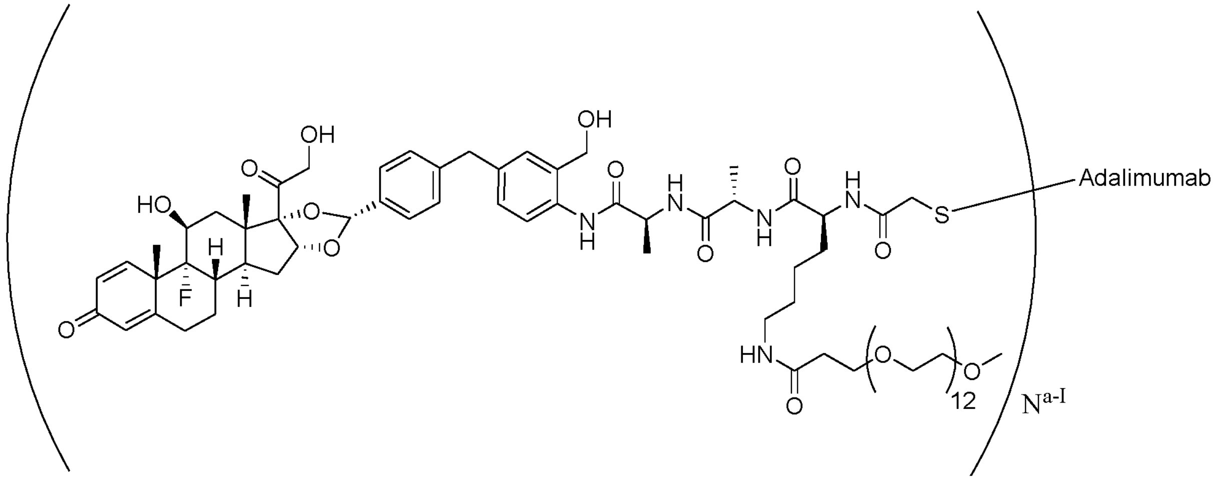 |
| V-134 | 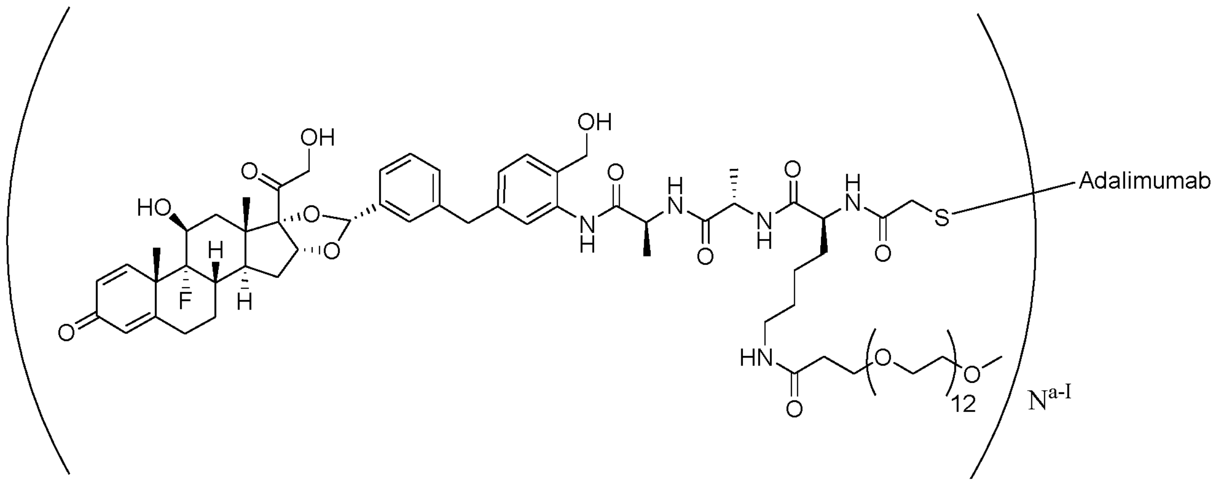 |

(continued)

| No. | Structure |
| --- | --- |
| V-135 | 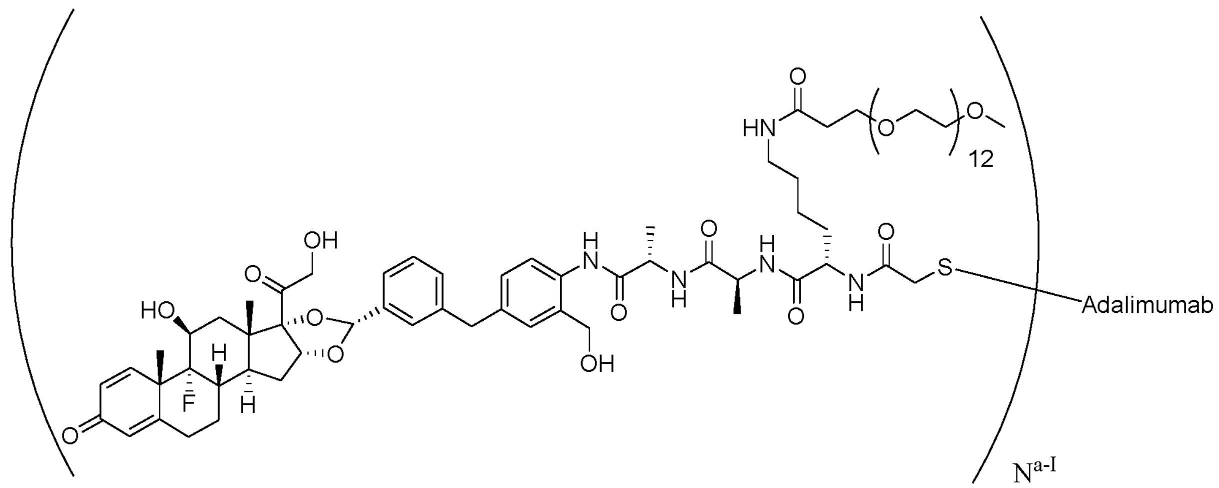 |
| V-136 | 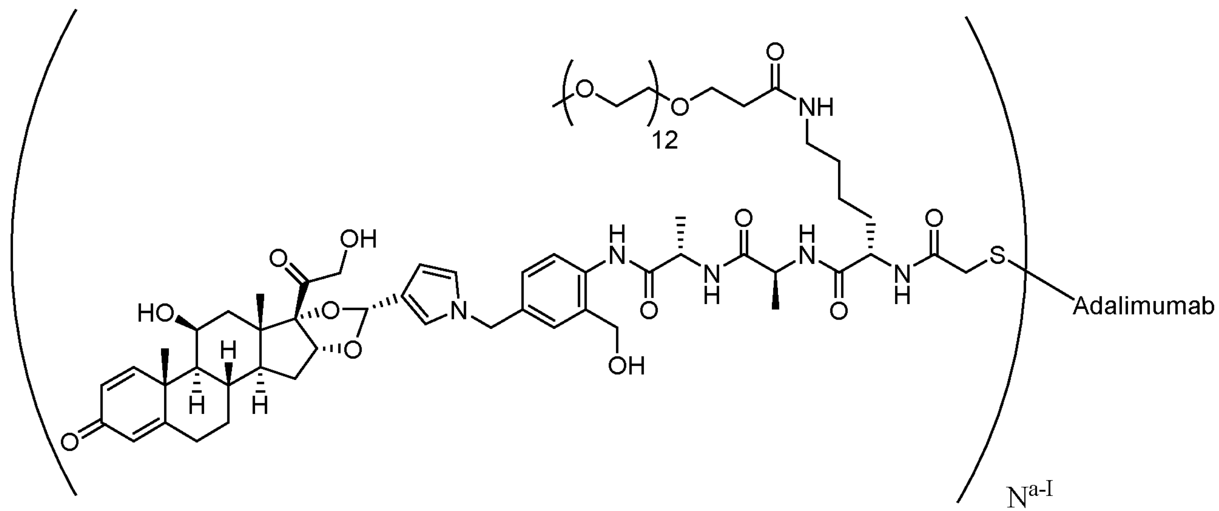 |

(continued)

| No. | Structure |
| --- | --- |
| V-137 | 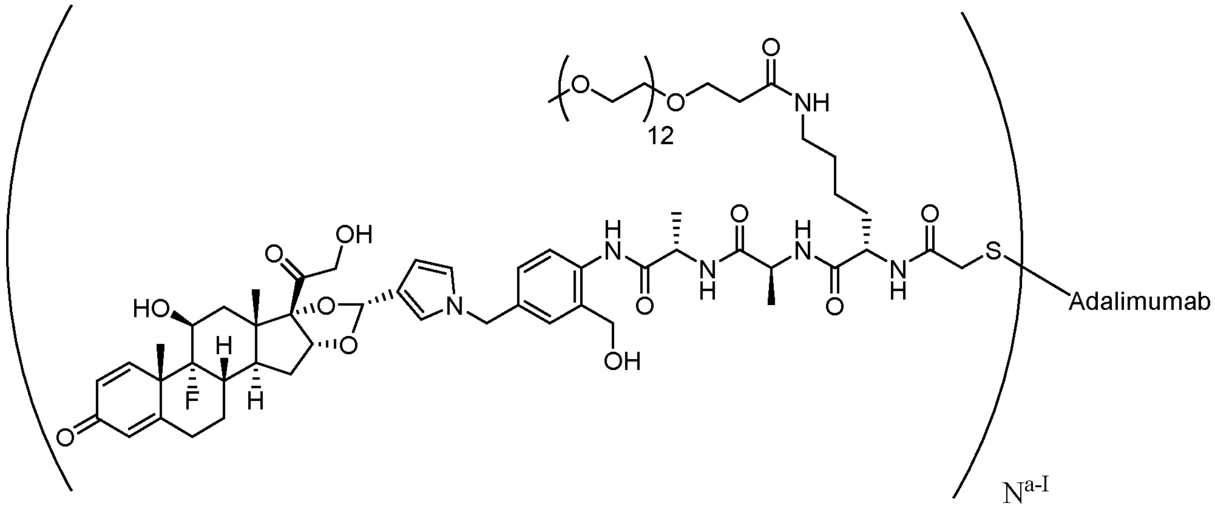 |
| V-138 | 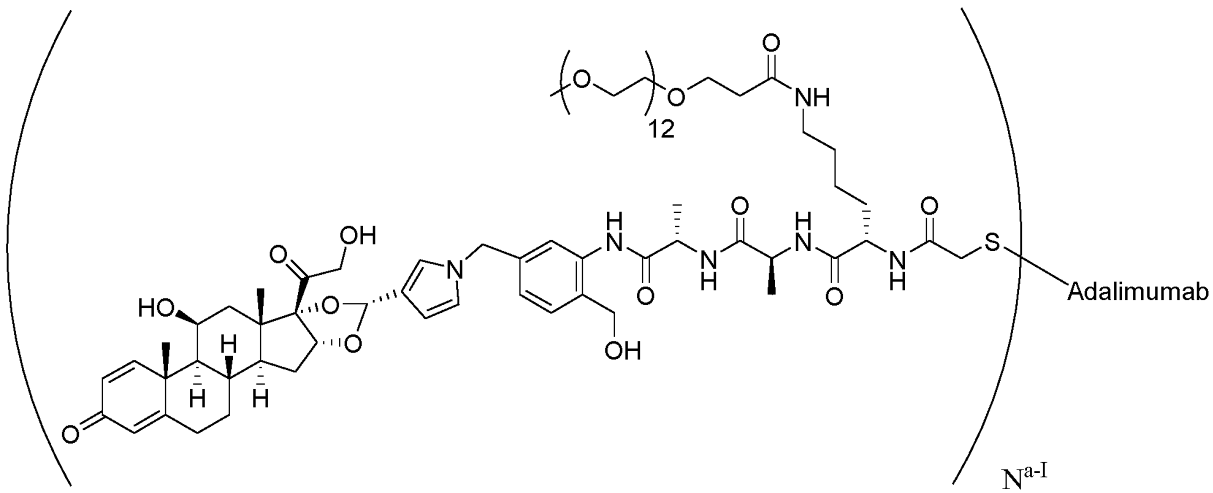 |

(continued)

| No. | Structure |
|---|---|
| V-139 | 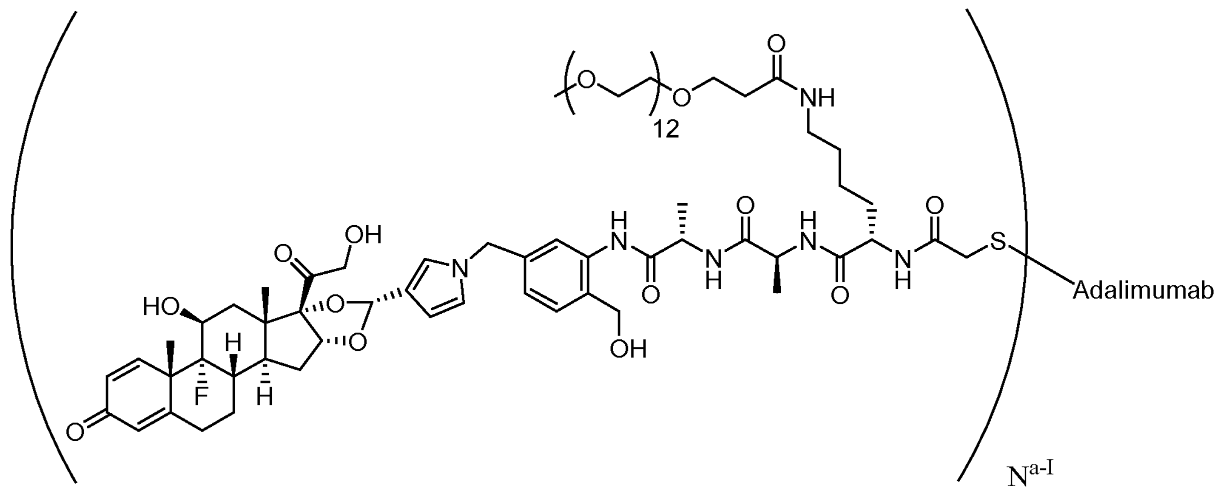 |
| V-140 | 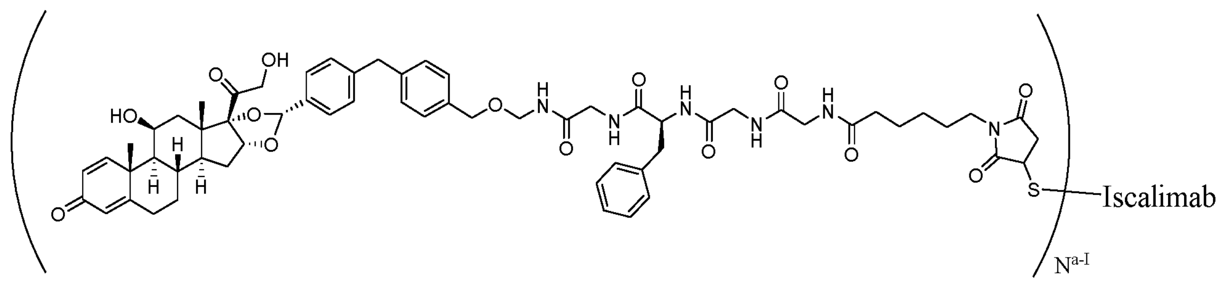 |
| V-141 | 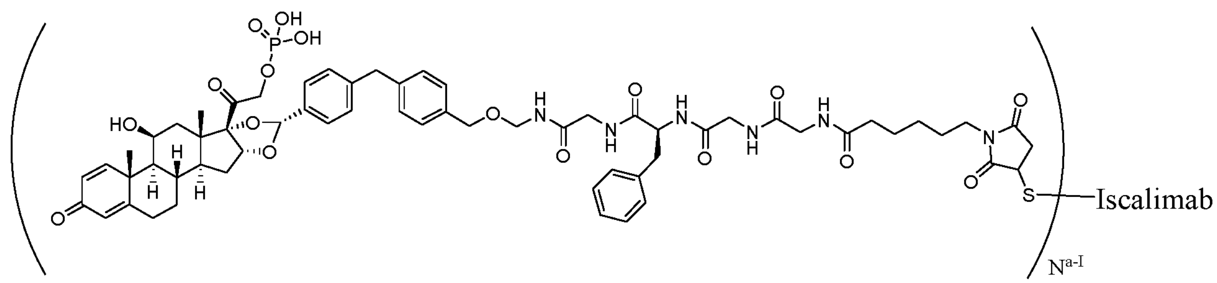 |

(continued)

| No. | Structure |
|---|---|
| V-145 | 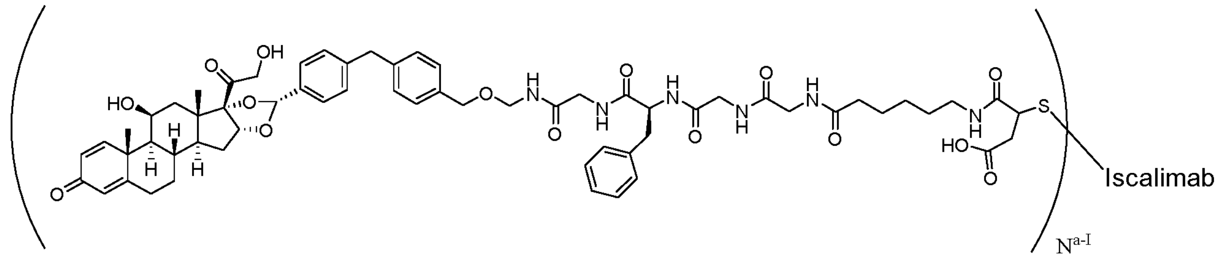 |
| V-146 | 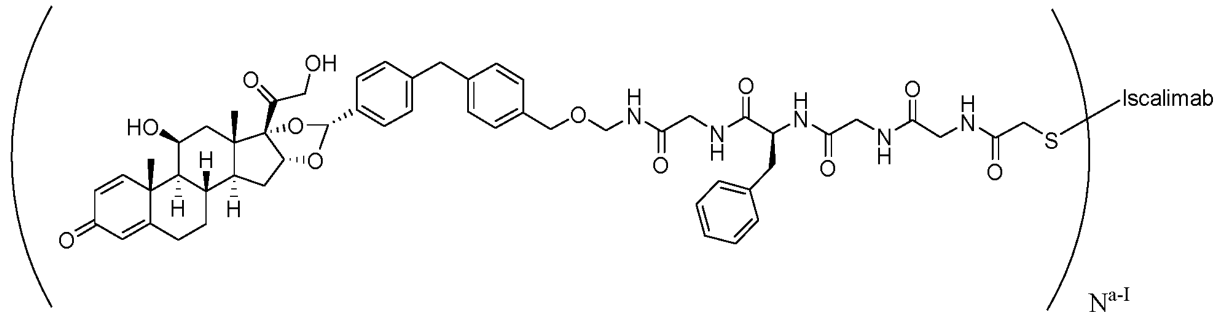 |
| V-147 | 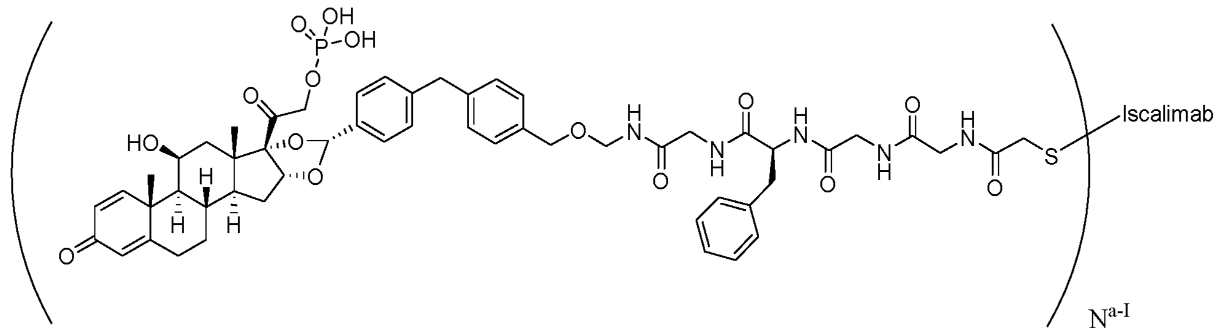 |

(continued)

| No. | Structure |
|---|---|
| V-151 | 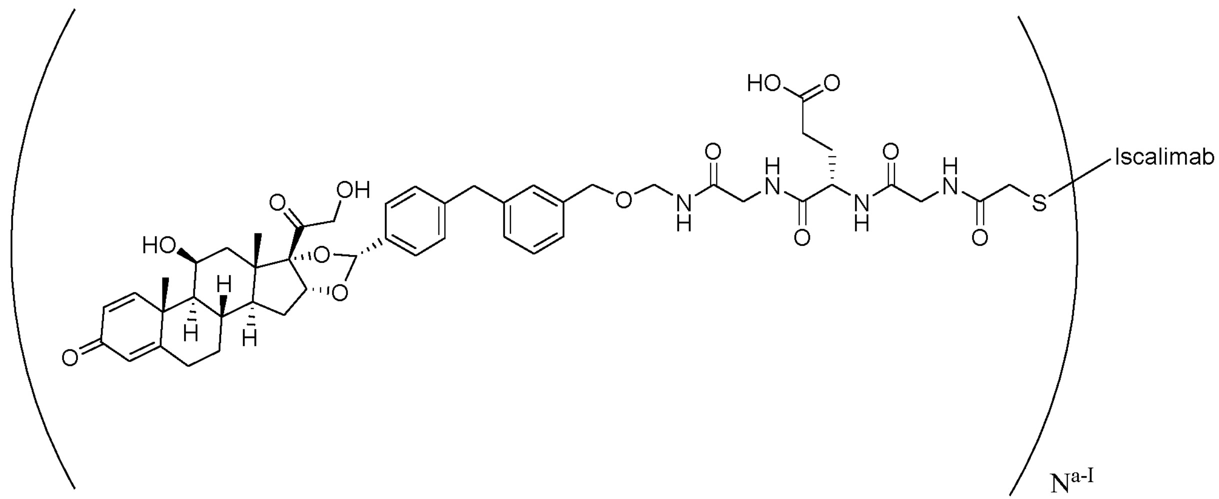 |
| V-152 | 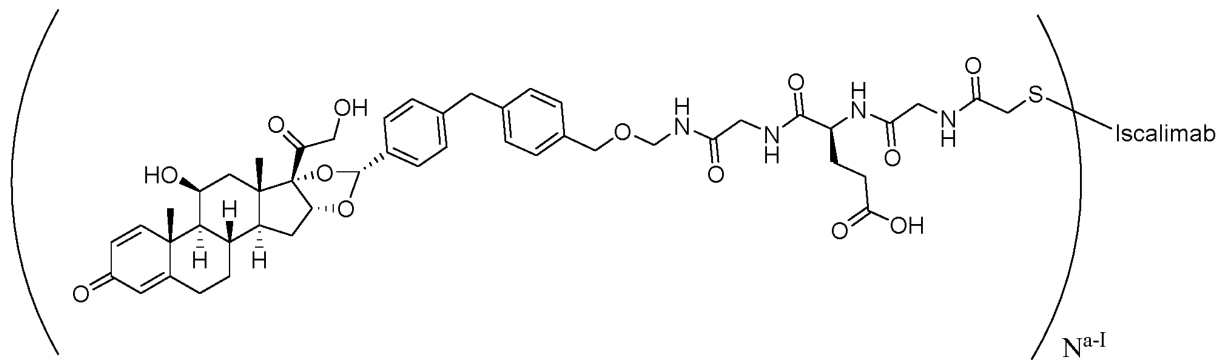 |

(continued)

| No. | Structure |
|---|---|
| V-153 | 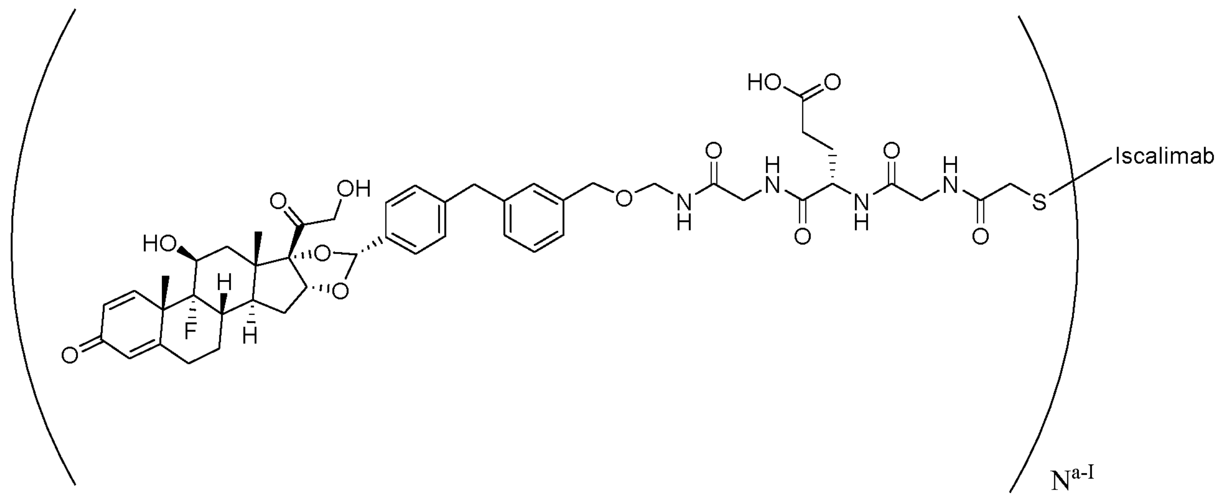 |
| V-154 | 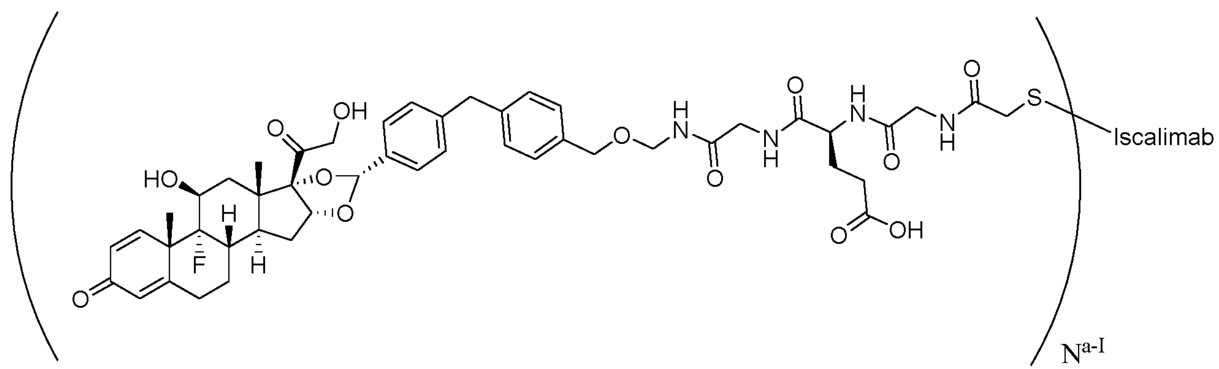 |

(continued)

| No. | Structure |
| --- | --- |
| V-155 | 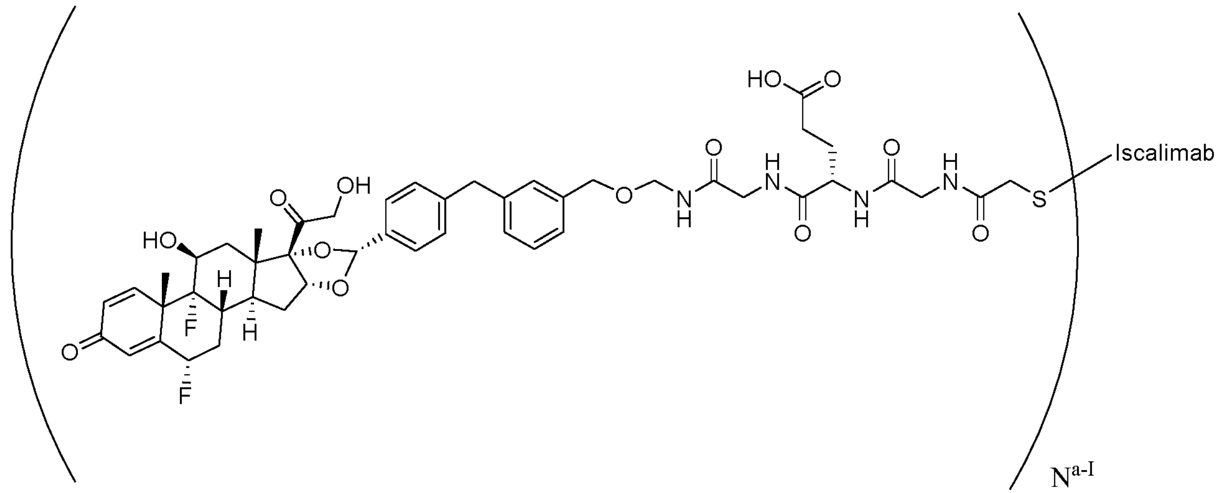 |
| V-156 | 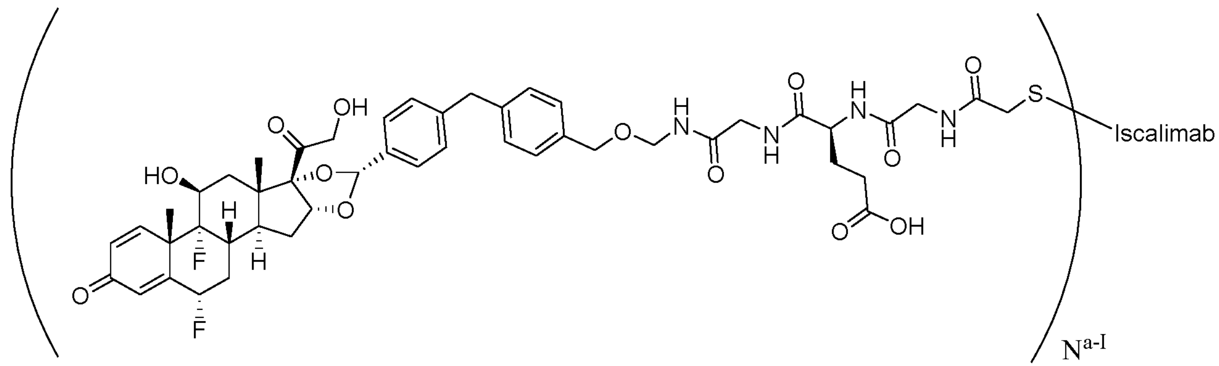 |

(continued)

| No. | Structure |
|---|---|
| V-157 | 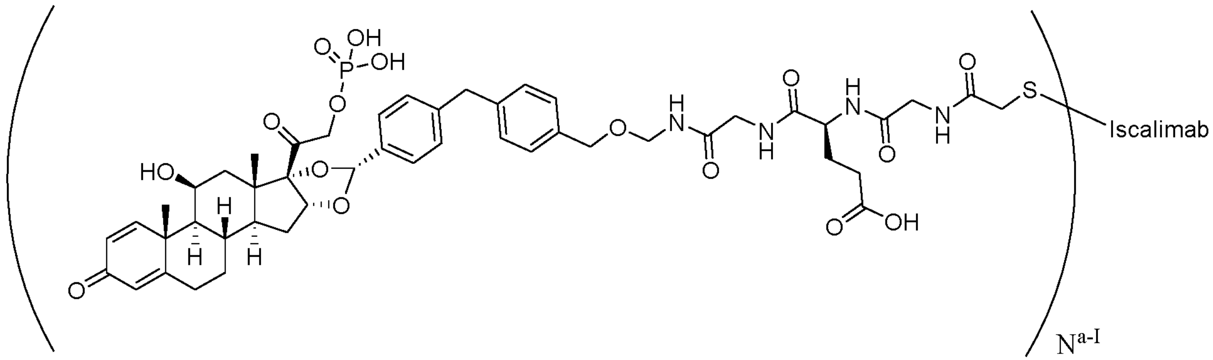 |
| V-161 | 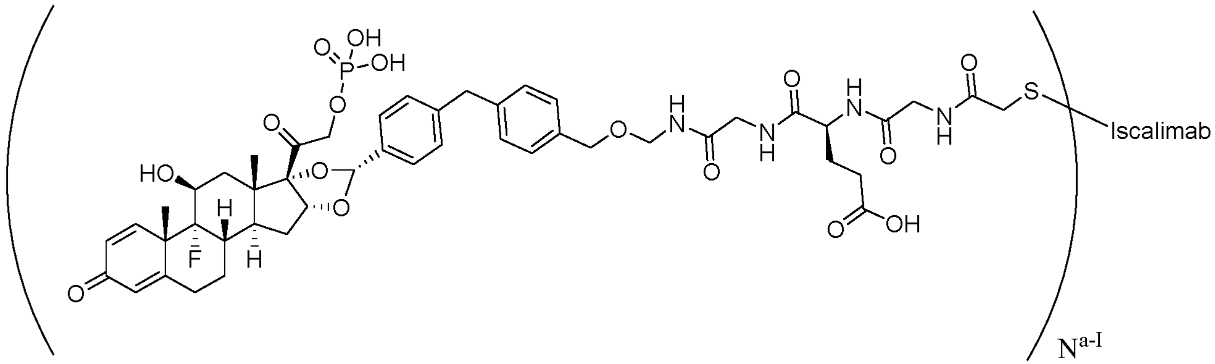 |

(continued)

| No. | Structure |
|---|---|
| V-164 | 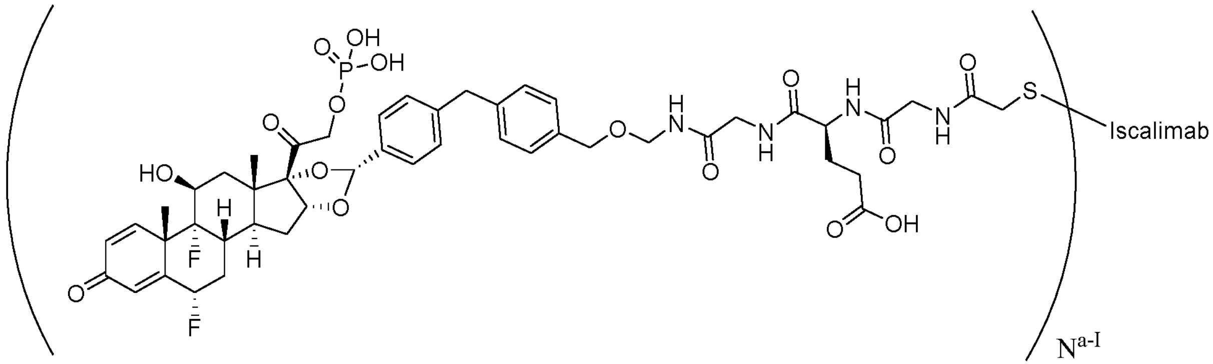 |
| V-168 | 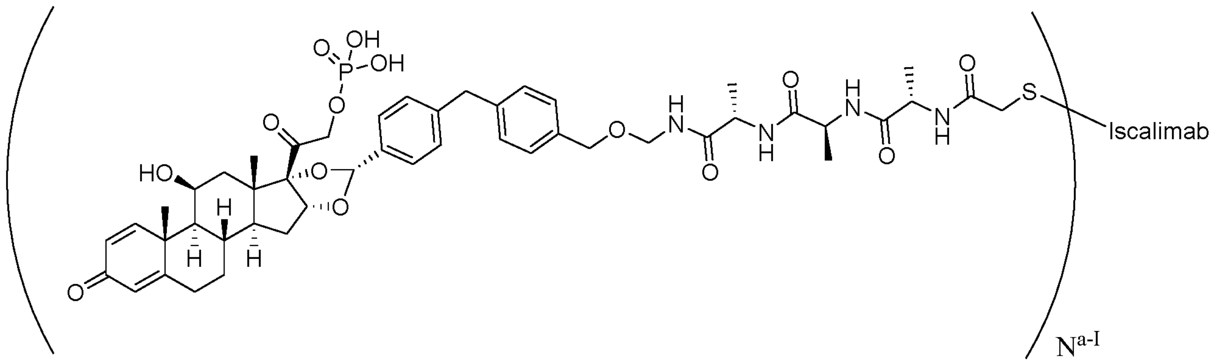 |

(continued)

| No. | Structure |
|---|---|
| V-169 | 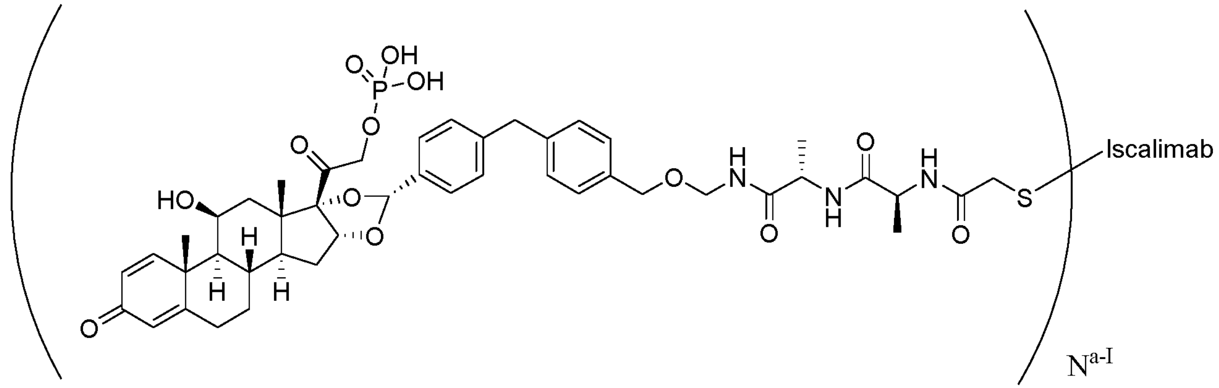 |
| V-173 | 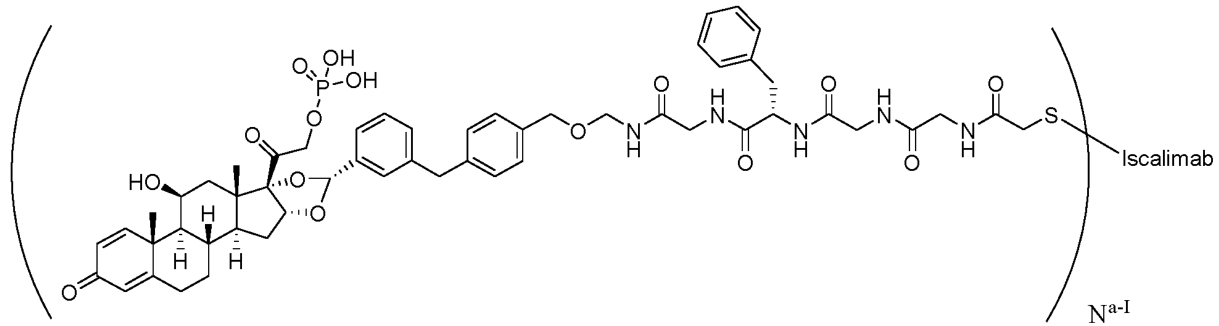 |
| V-174 | 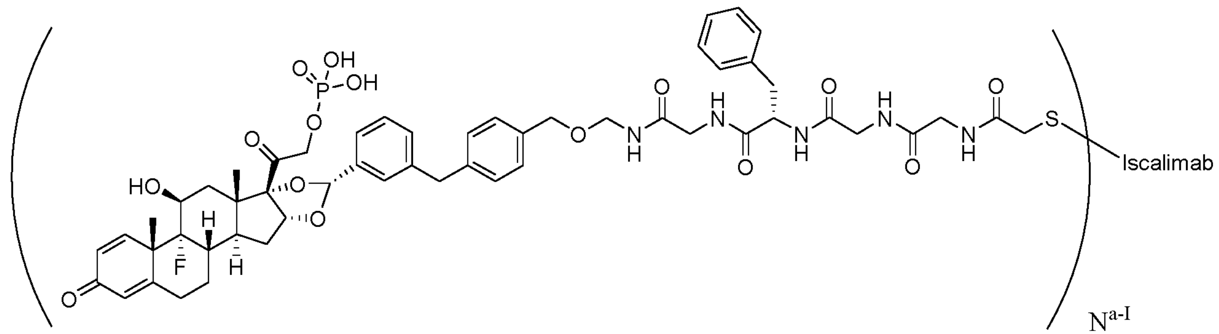 |

(continued)

| No. | Structure |
|---|---|
| V-178 | 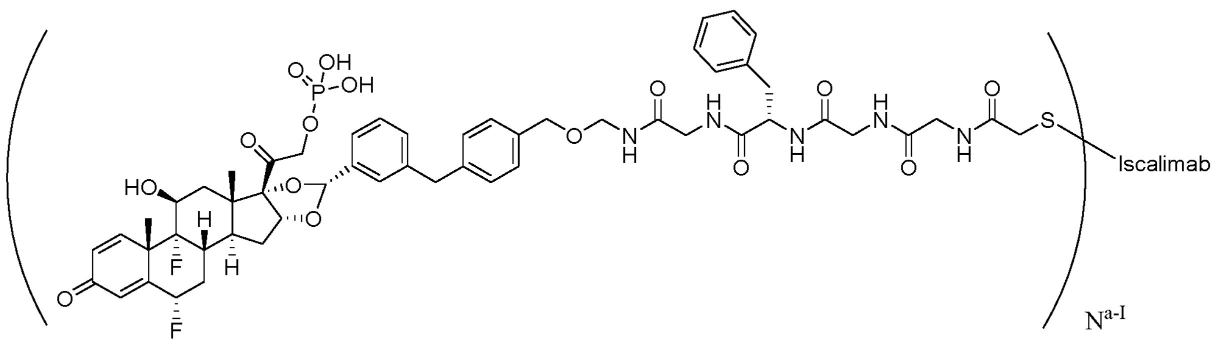 |
| V-182 | 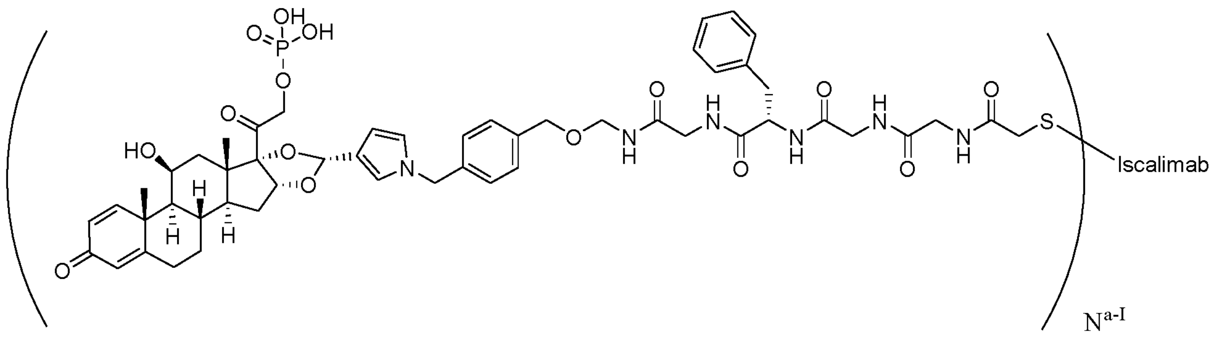 |
| V-183 | 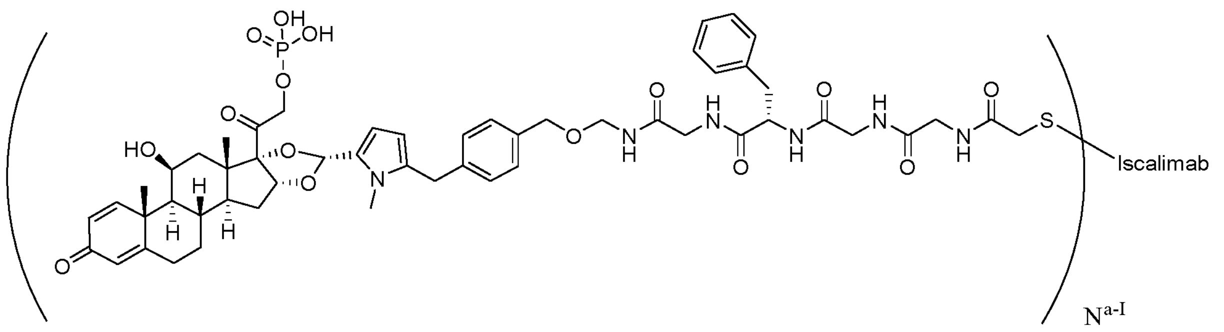 |

(continued)

| No. | Structure |
|---|---|
| V-184 | 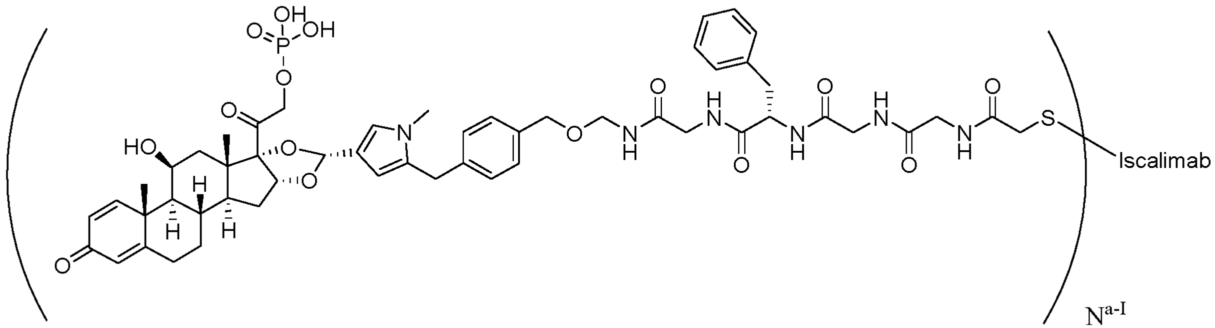 |
| V-185 | 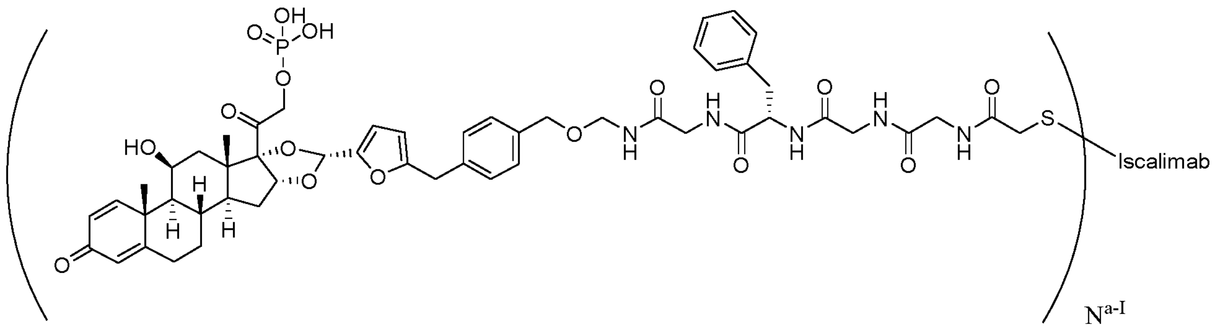 |
| V-186 | 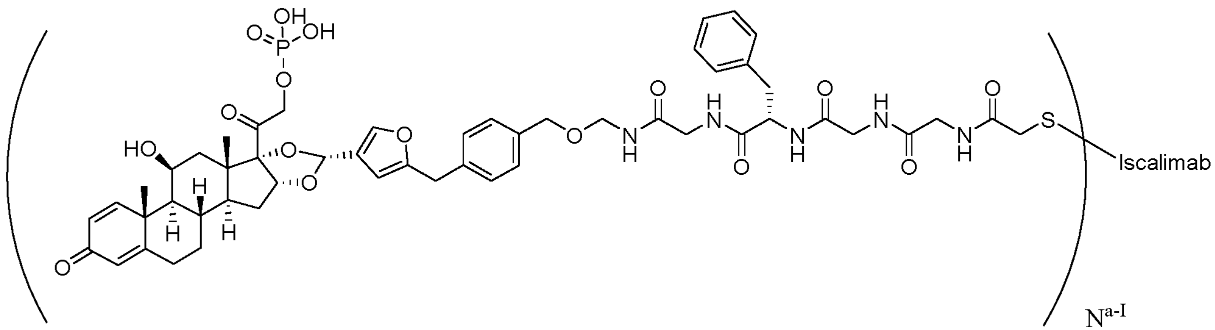 |

(continued)

| No. | Structure |
|---|---|
| V-187 | 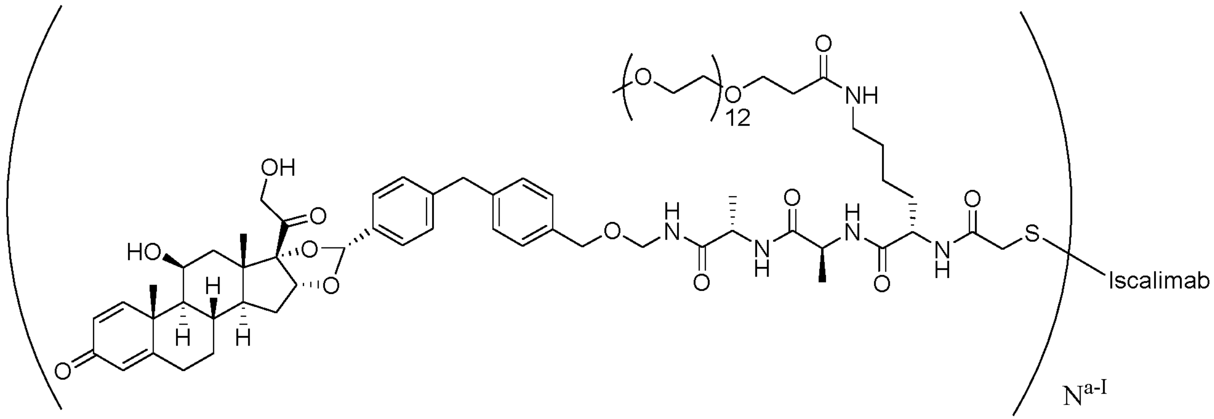 |
| V-188 | 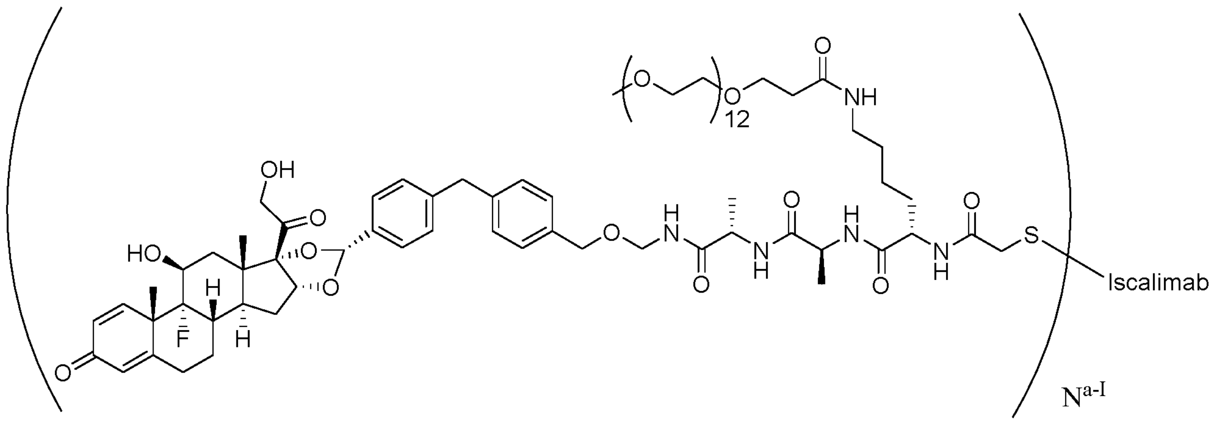 |

(continued)

| No. | Structure |
|---|---|
| V-189 | 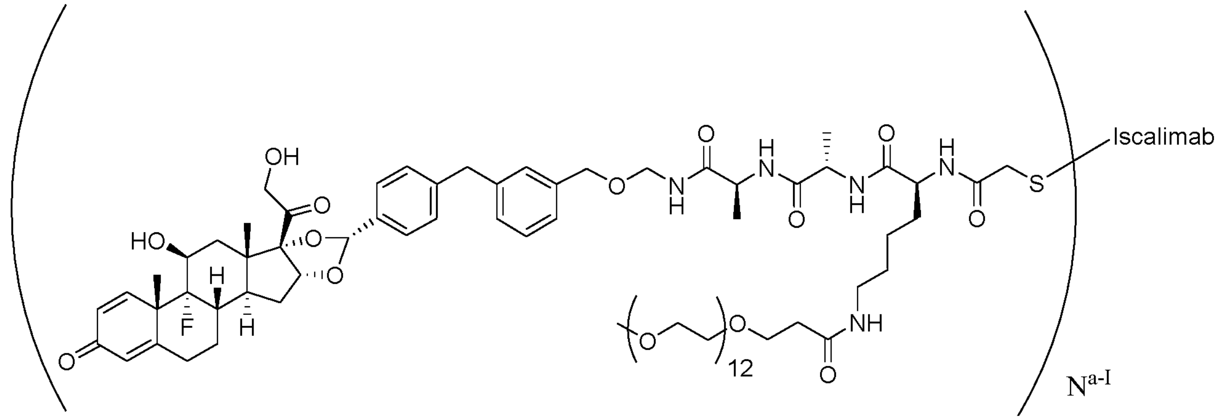 |
| V-190 | 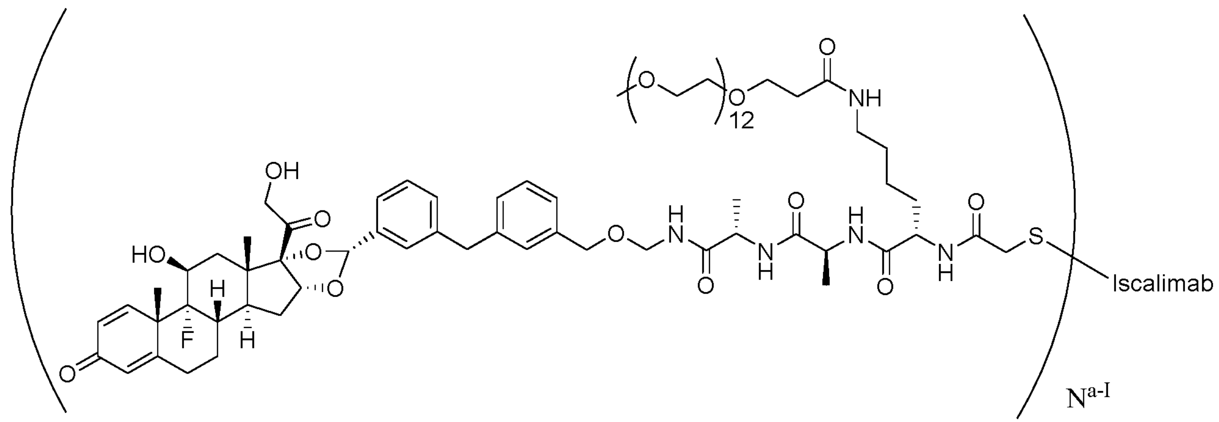 |

(continued)

| No. | Structure |
| --- | --- |
| V-191 | 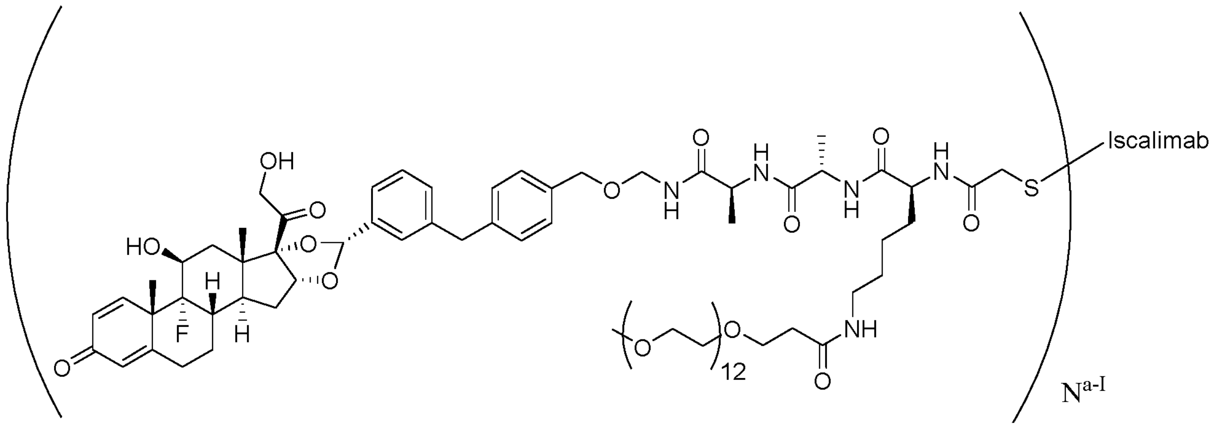 |
| V-192 | 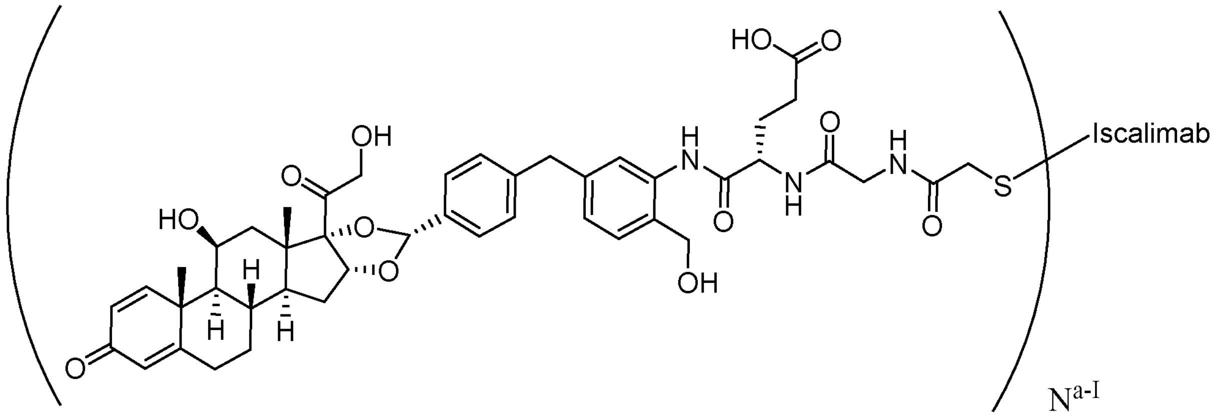 |

(continued)

| No. | Structure |
|---|---|
| V-193 | 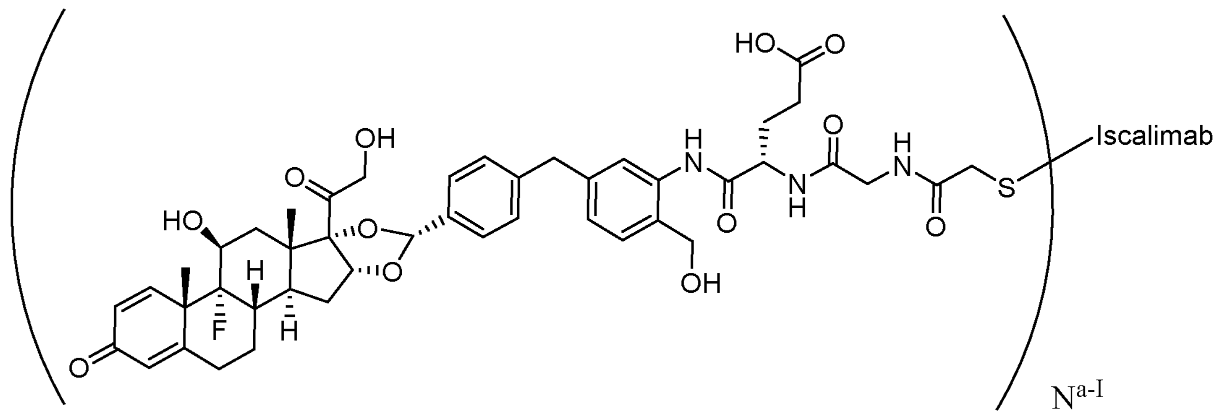 |
| V-194 | 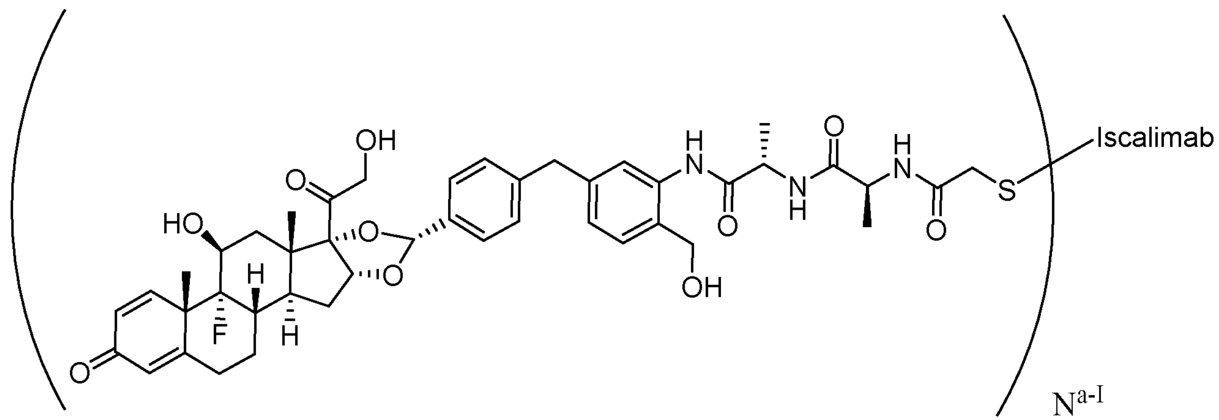 |

(continued)

| No. | Structure |
|---|---|
| V-195 | 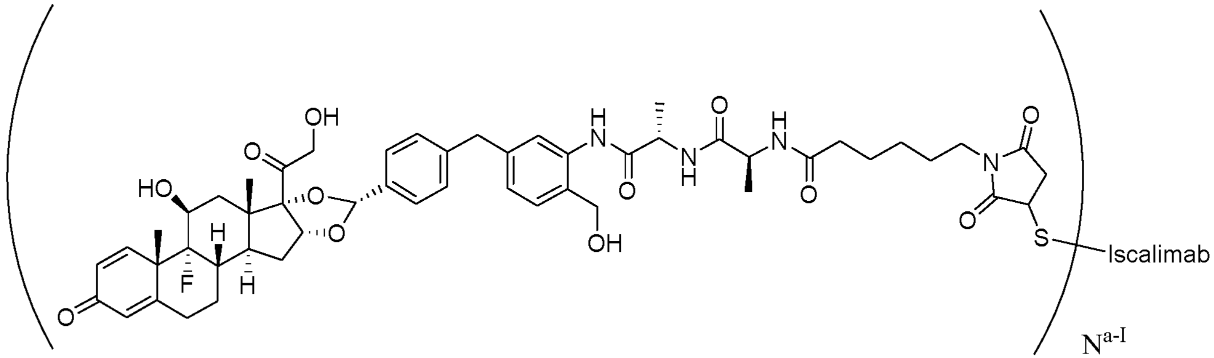 |
| V-196 | 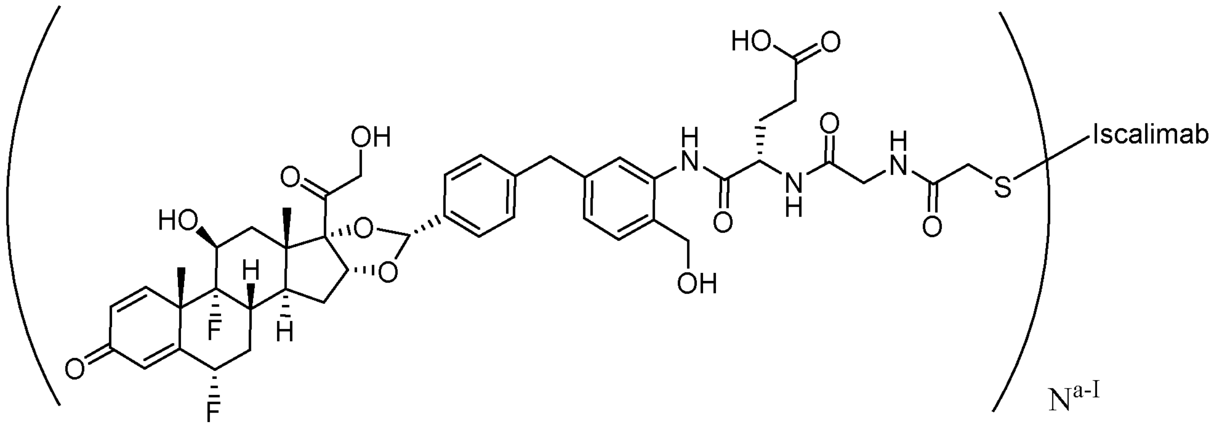 |

(continued)

| No. | Structure |
| --- | --- |
| V-197 | 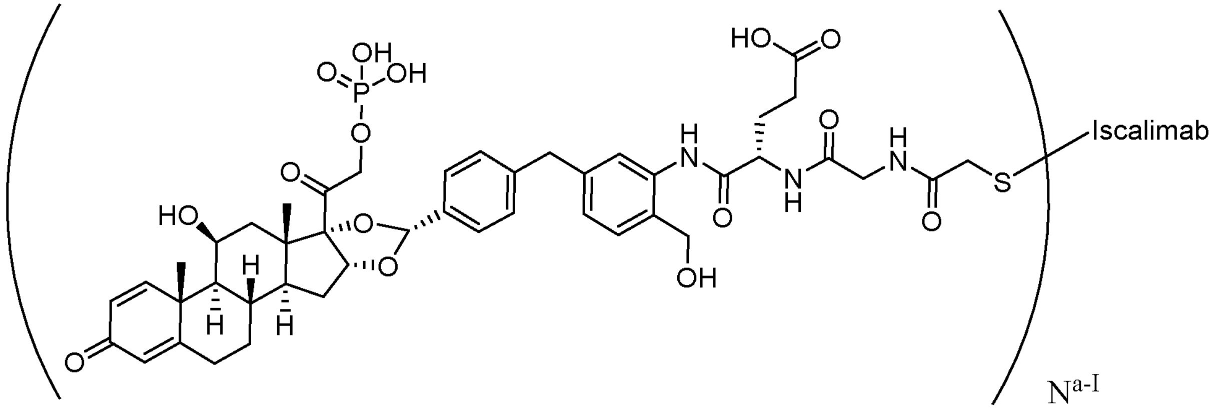 |
| V-198 | 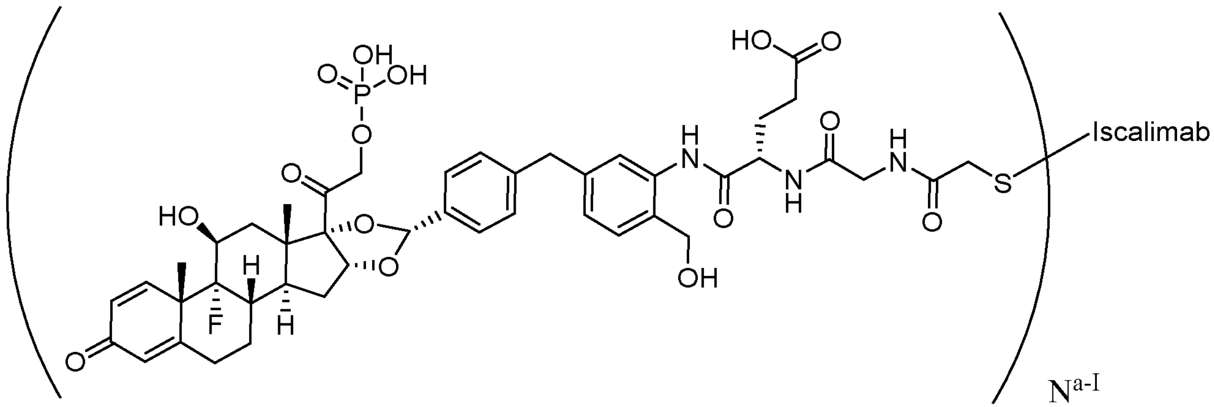 |

(continued)

| No. | Structure |
|---|---|
| V-199 | 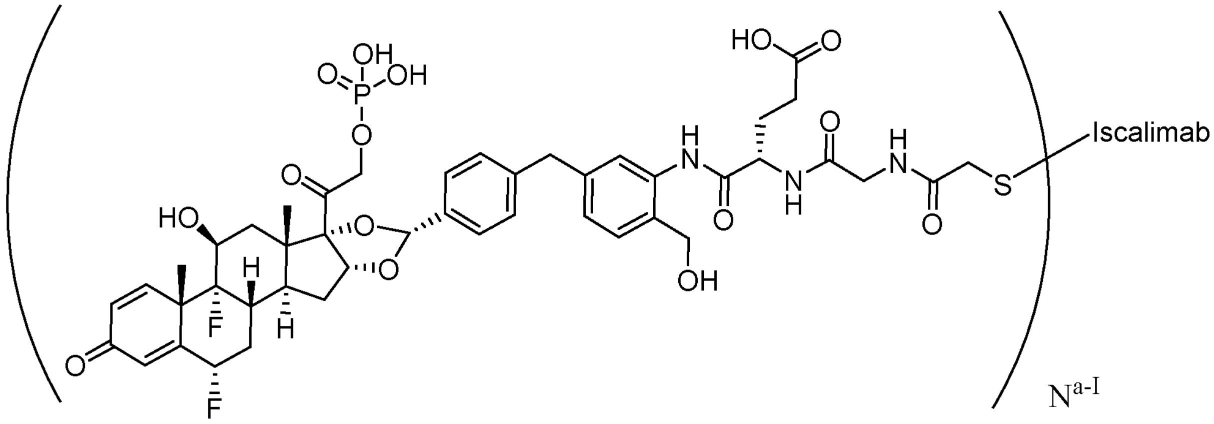 |
| V-209 | 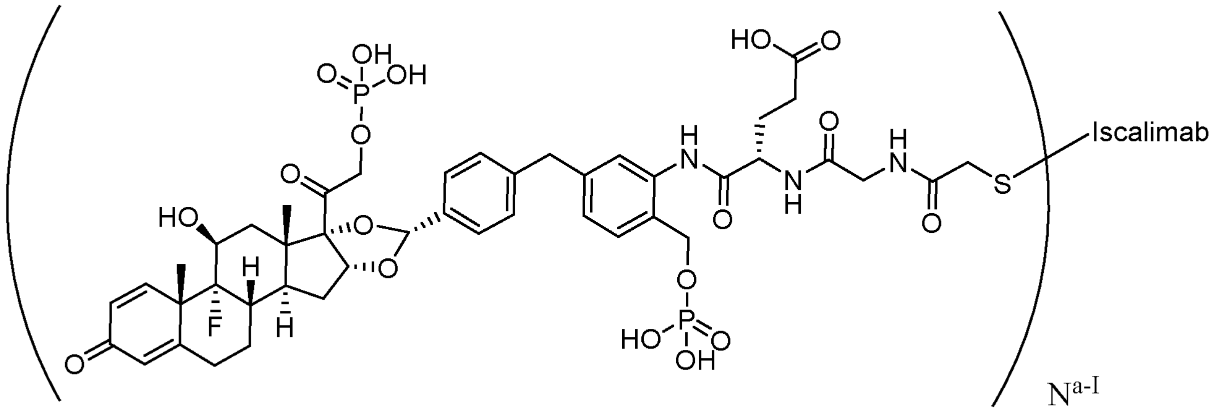 |

(continued)

| No. | Structure |
| --- | --- |
| V-213 | 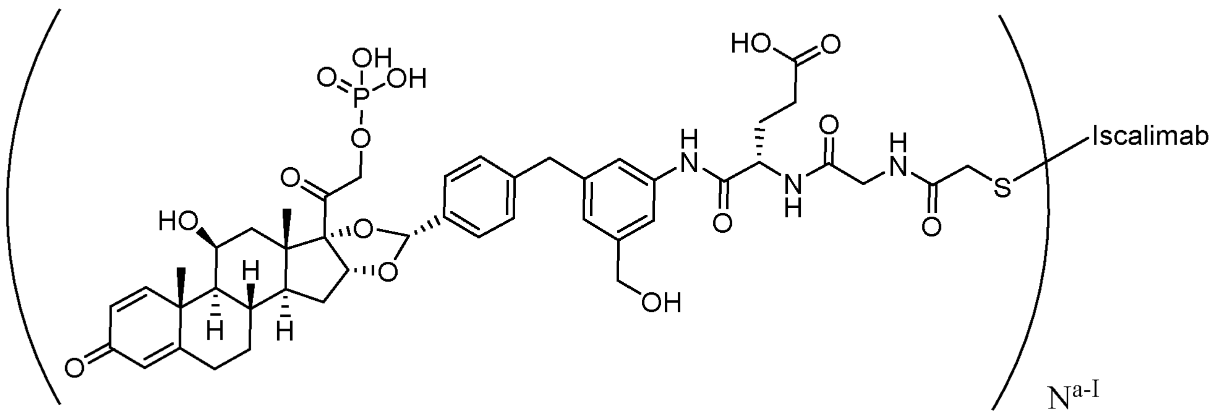 |
| V-216 | 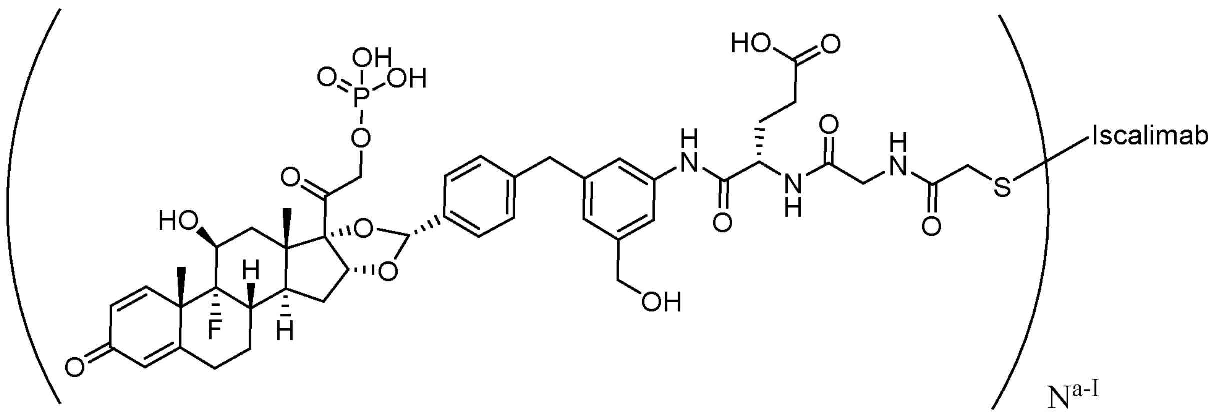 |

(continued)

| No. | Structure |
|---|---|
| V-219 | 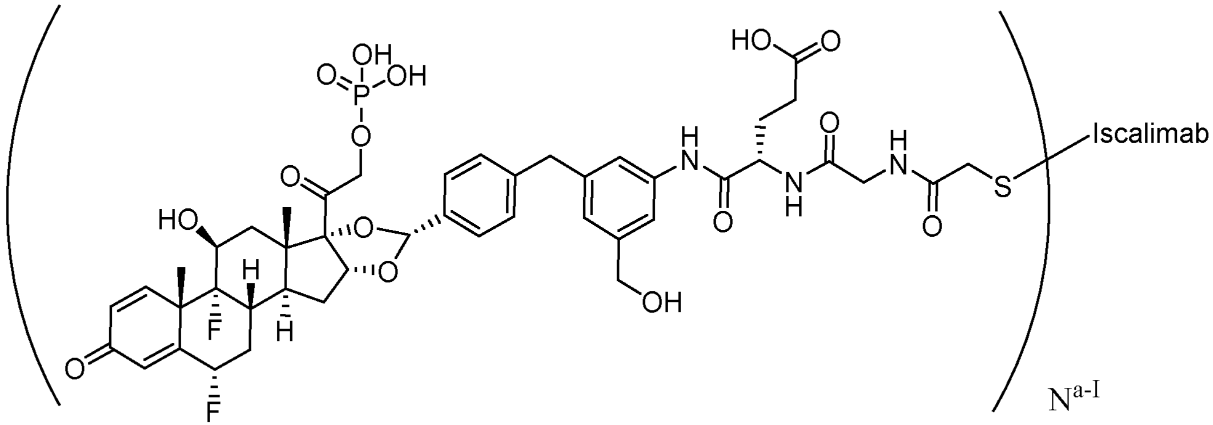 |
| V-222 | 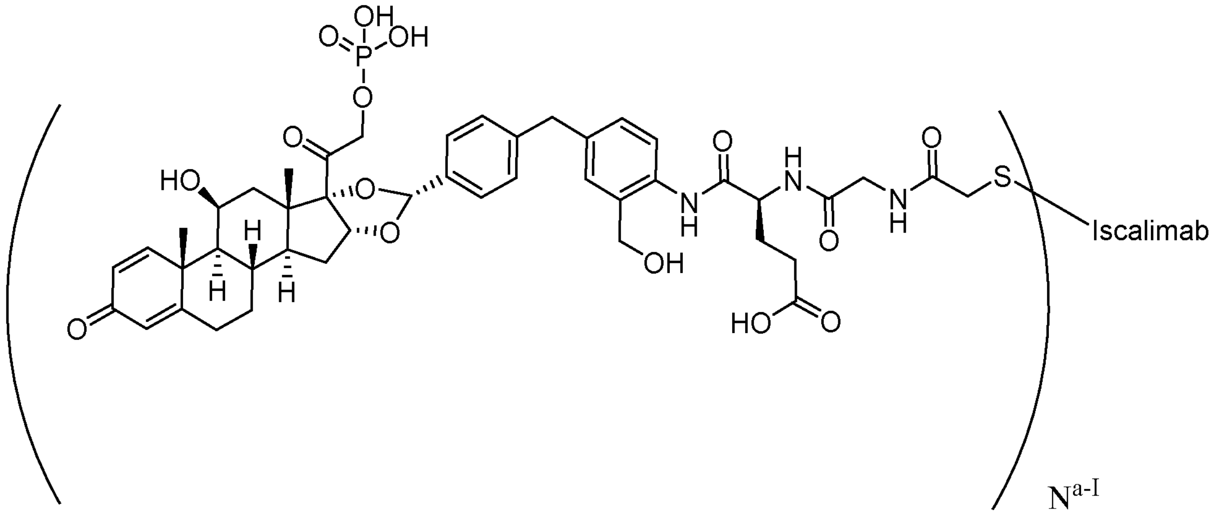 |

(continued)

| No. | Structure |
|---|---|
| V-225 | 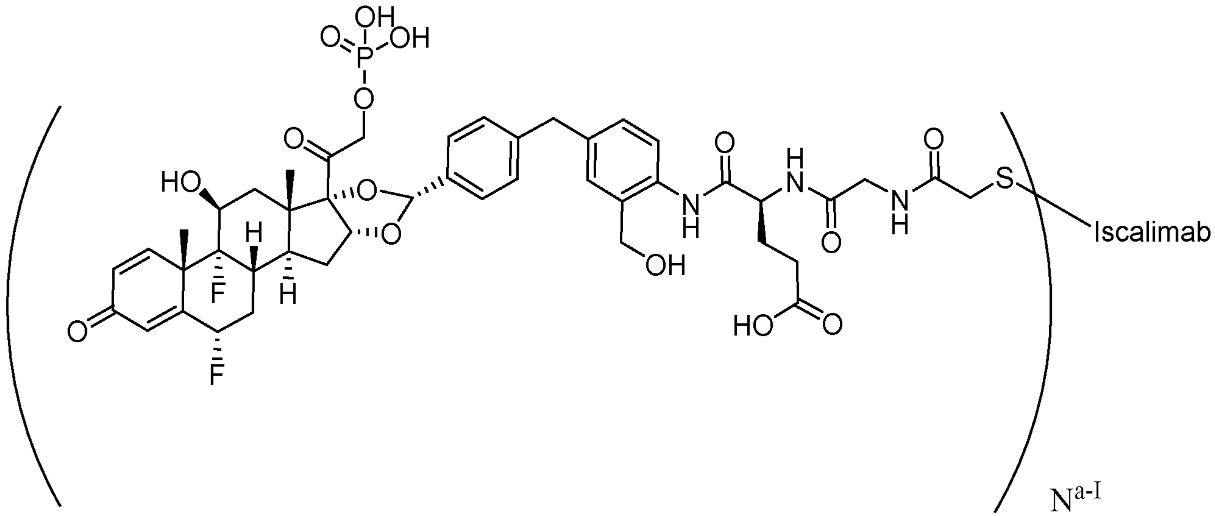 |
| V-228 | 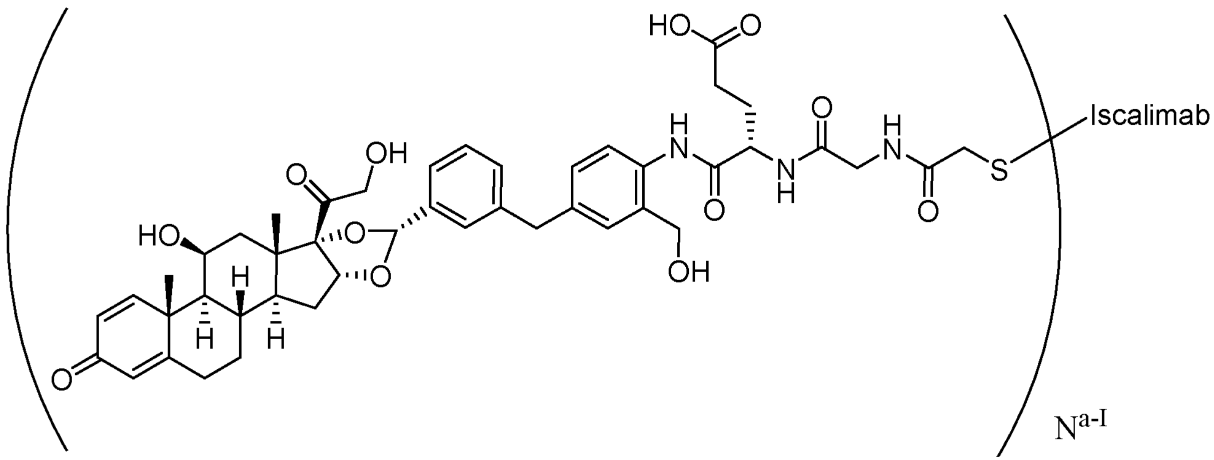 |

(continued)

| No. | Structure |
| --- | --- |
| V-229 | 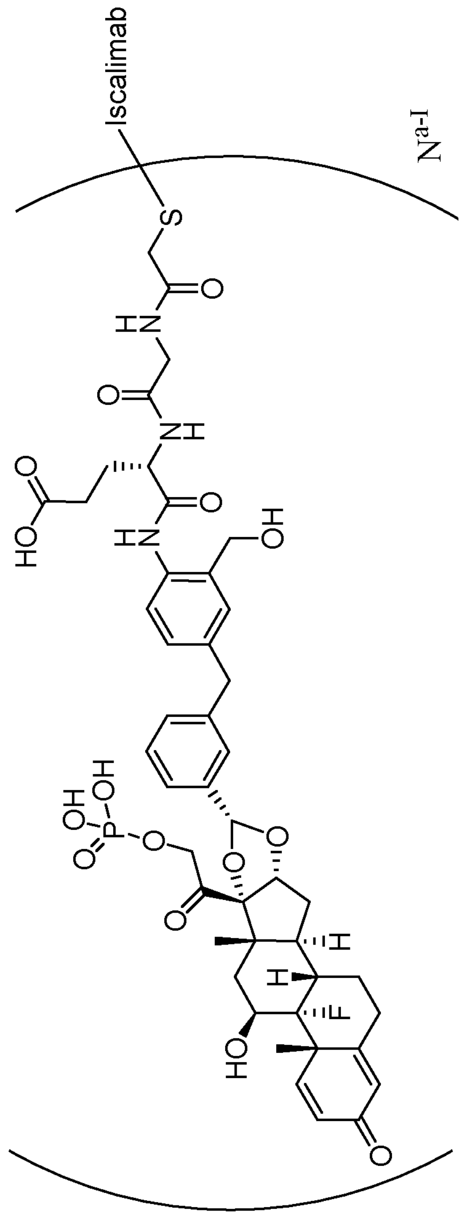 |
| V-230 | 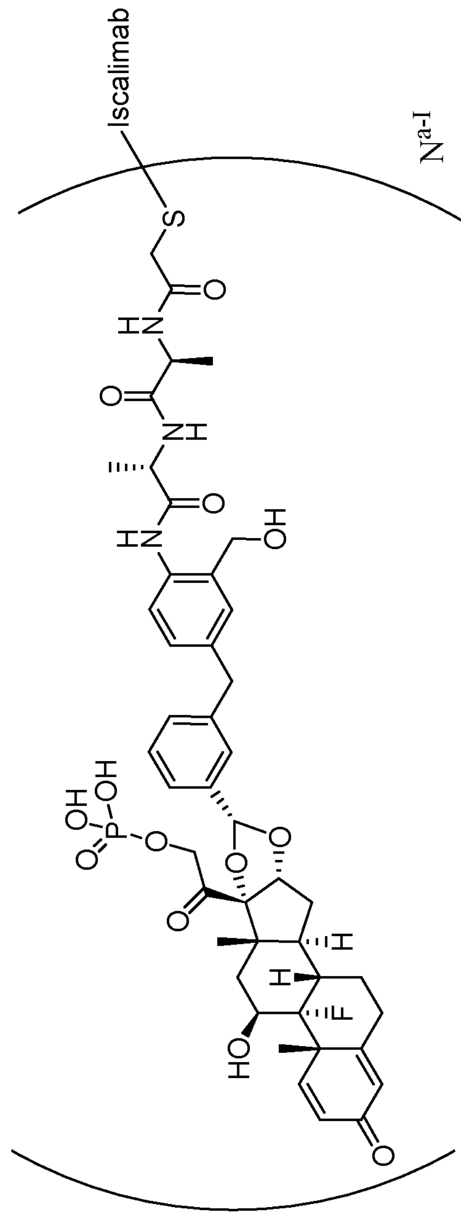 |

(continued)

| No. | Structure |
|---|---|
| V-233 | 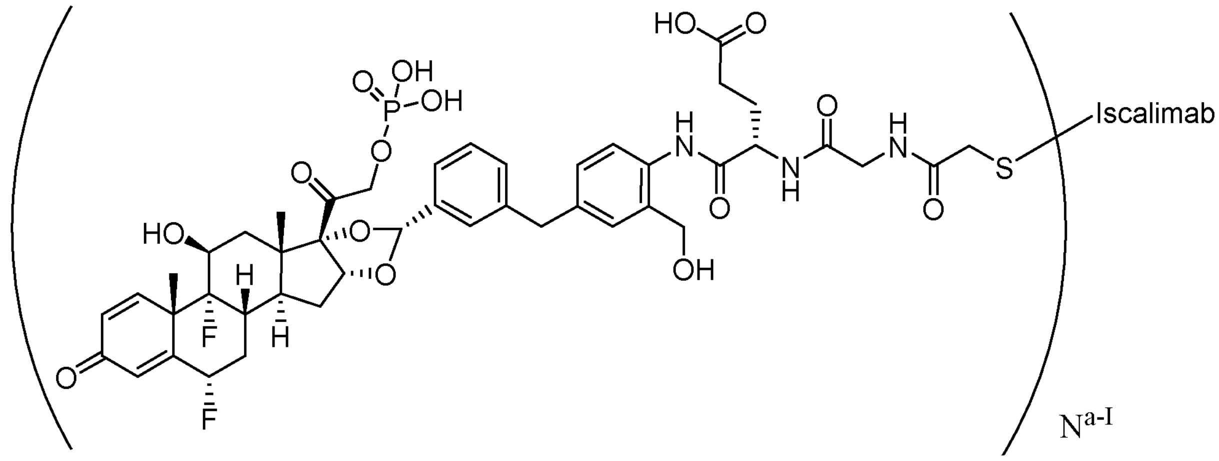 |
| V-236 | 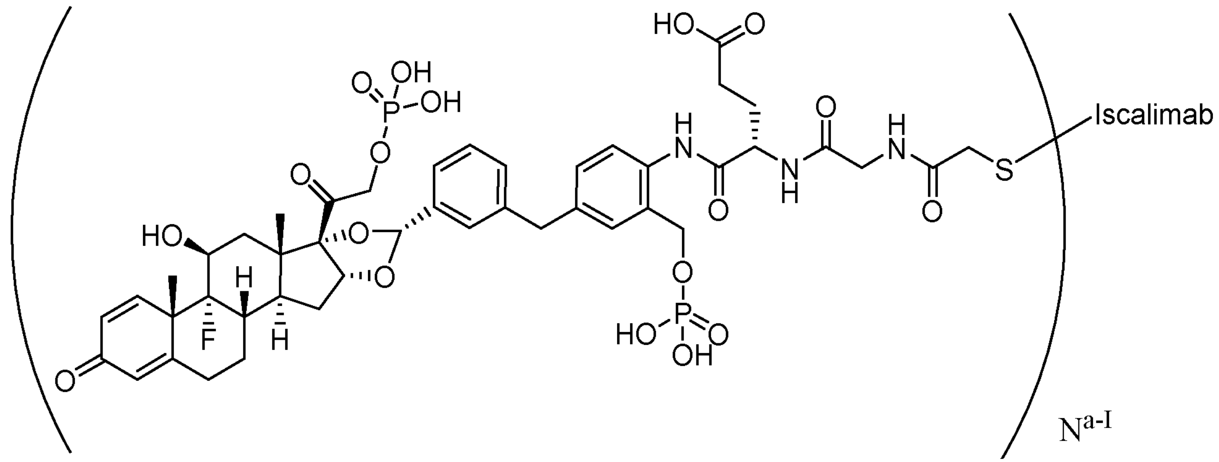 |

(continued)

| No. | Structure |
| --- | --- |
| V-240 | 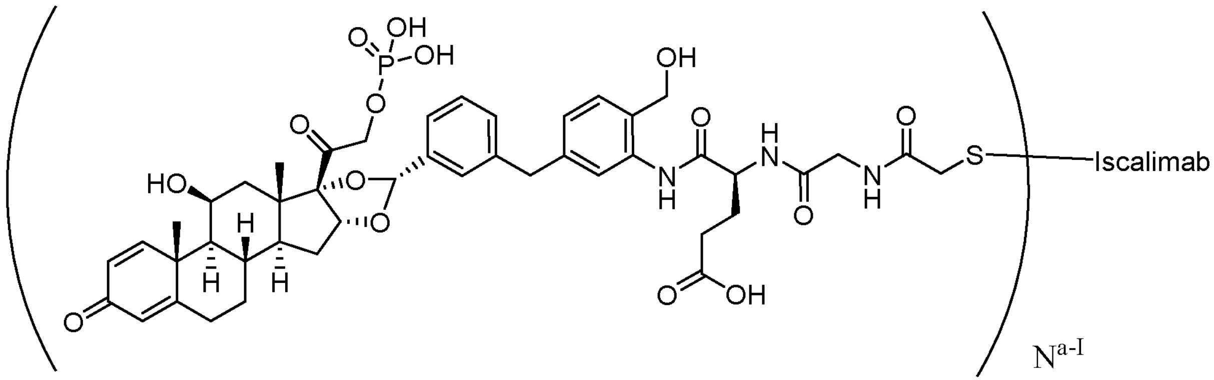 |
| V-241 | 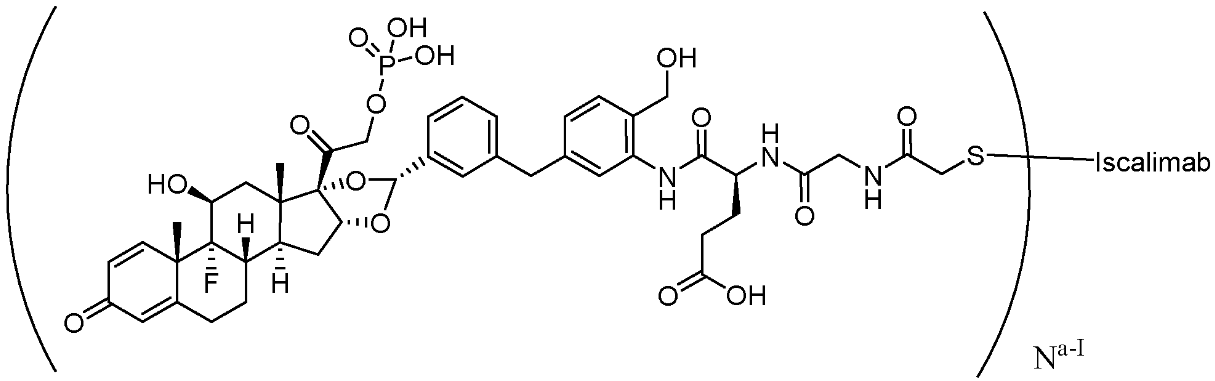 |

(continued)

| No. | Structure |
|---|---|
| V-242 | 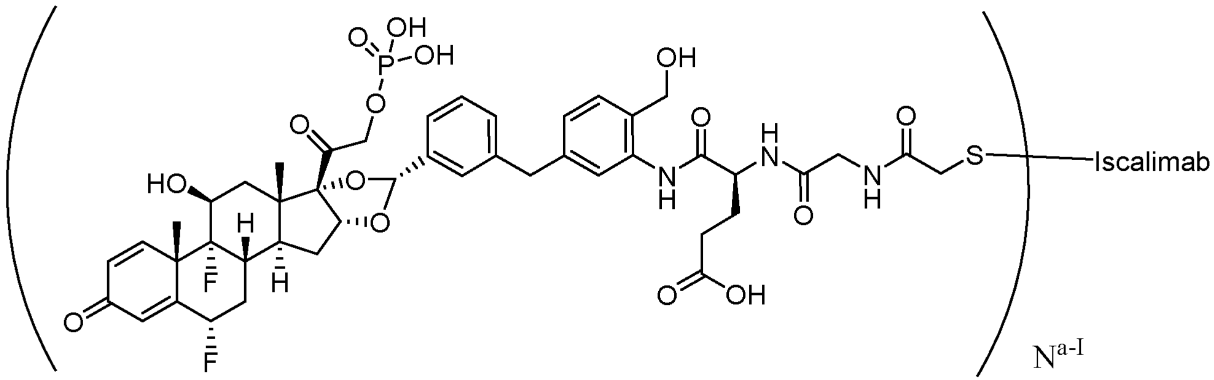 |
| V-243 | 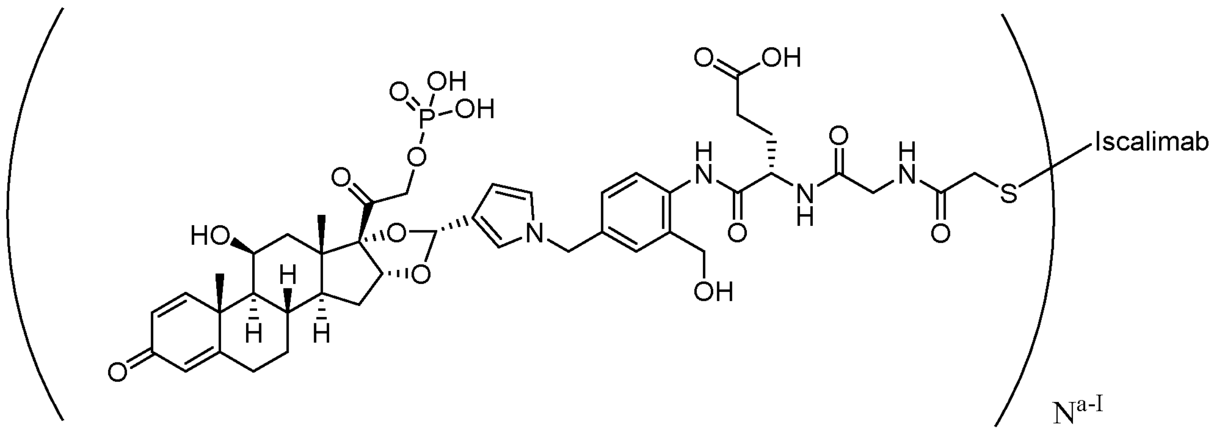 |

(continued)

| No. | Structure |
|---|---|
| V-244 | 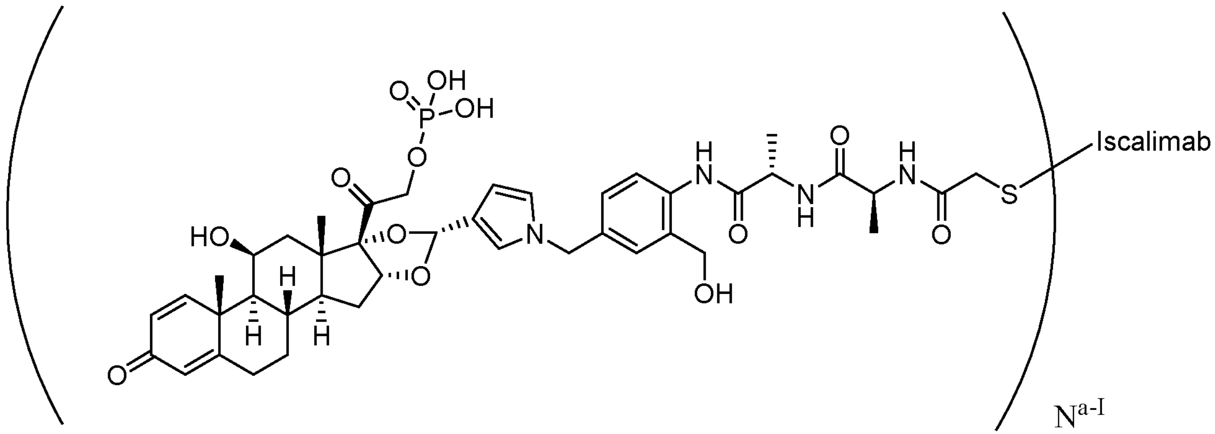 |
| V-247 | 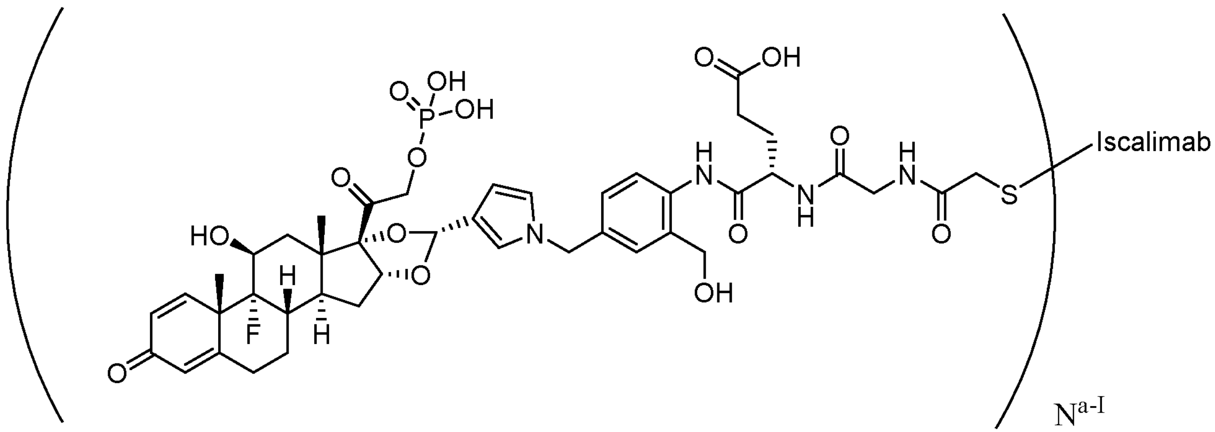 |

(continued)

| No. | Structure |
|---|---|
| V-248 | 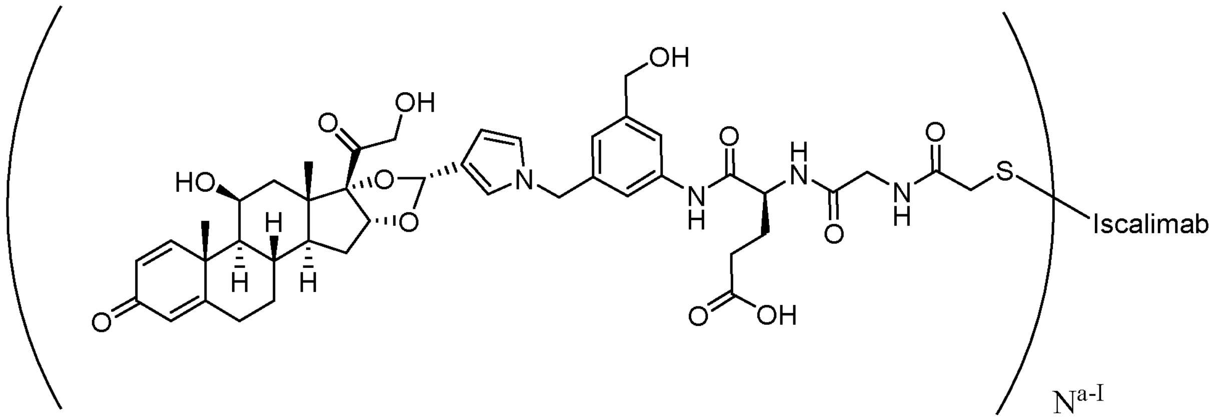 |
| V-249 | 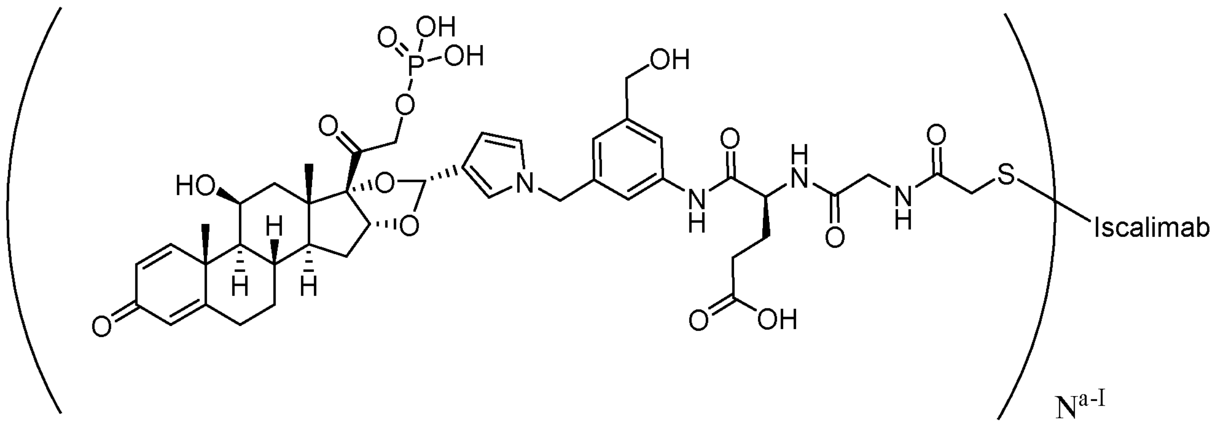 |

(continued)

| No. | Structure |
|---|---|
| V-250 | 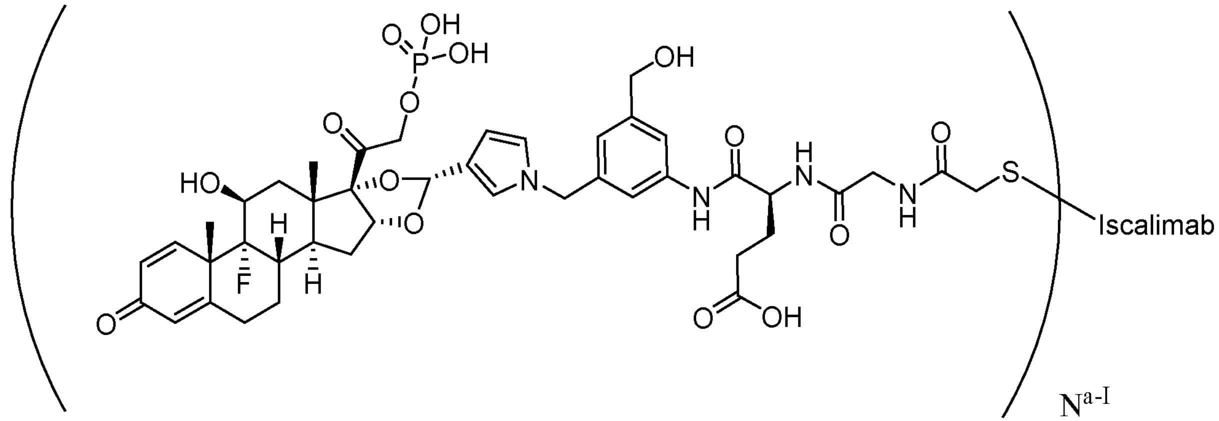 |
| V-251 | 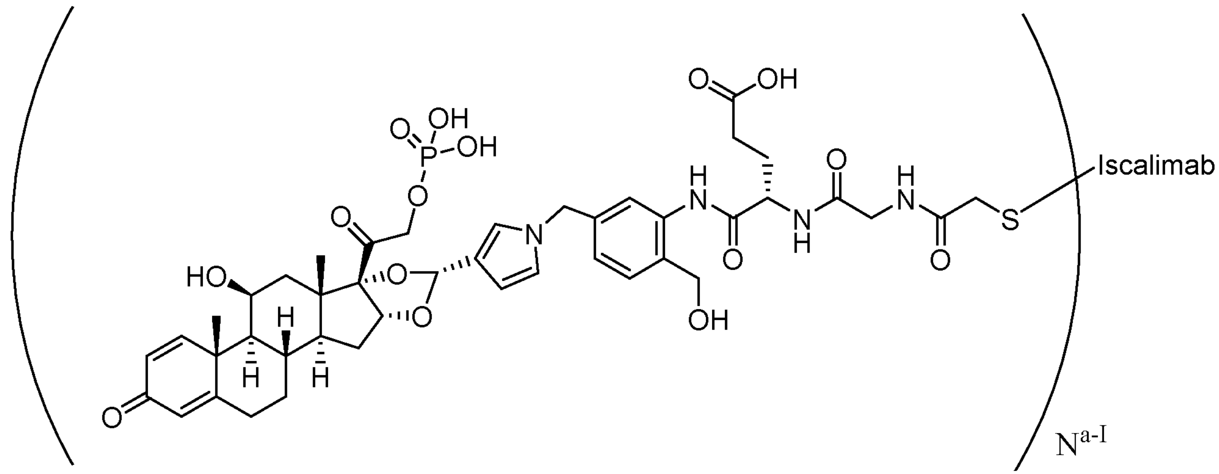 |

(continued)

| No. | Structure |
|---|---|
| V-252 | 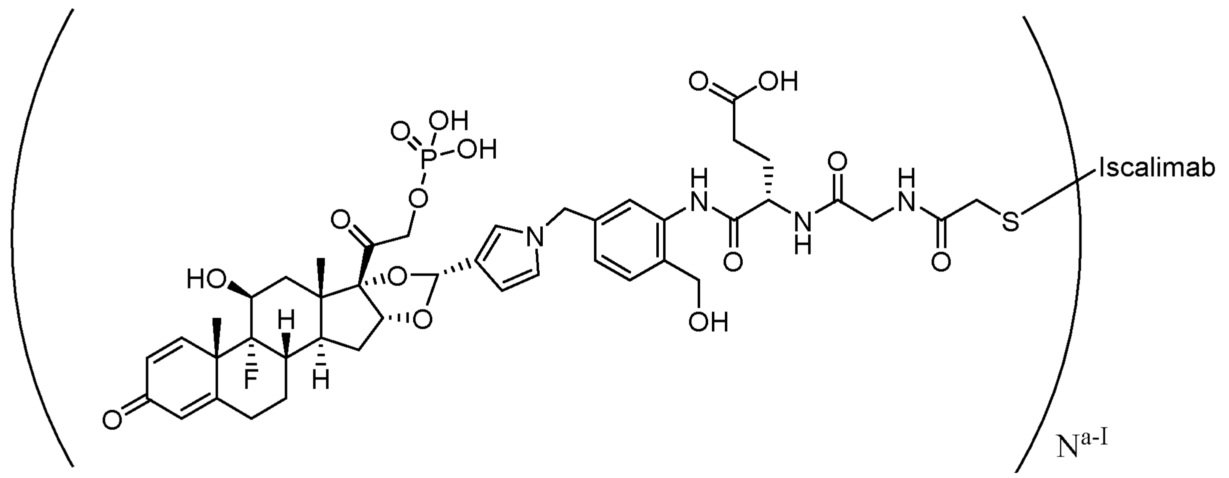 |
| V-258 | 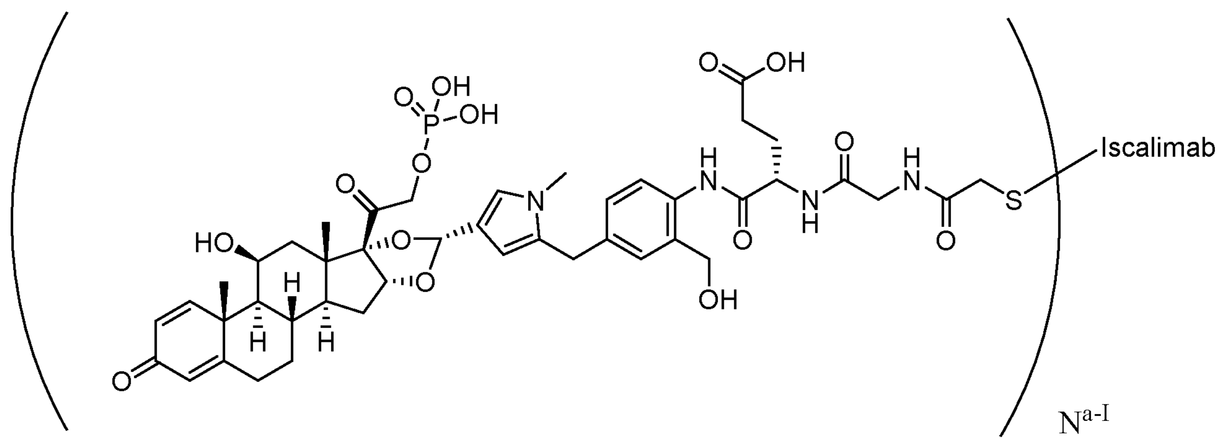 |

(continued)

| No. | Structure |
|---|---|
| V-259 | 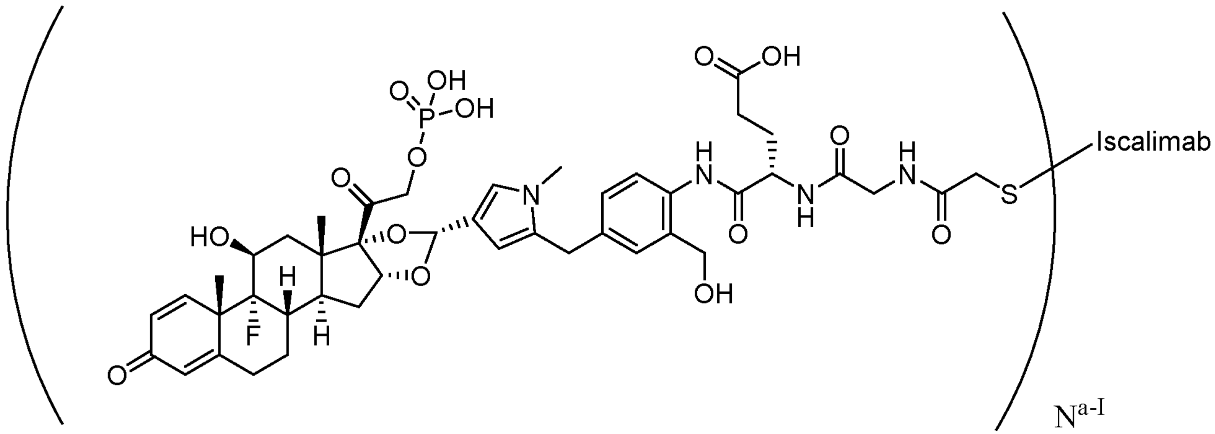 |
| V-260 | 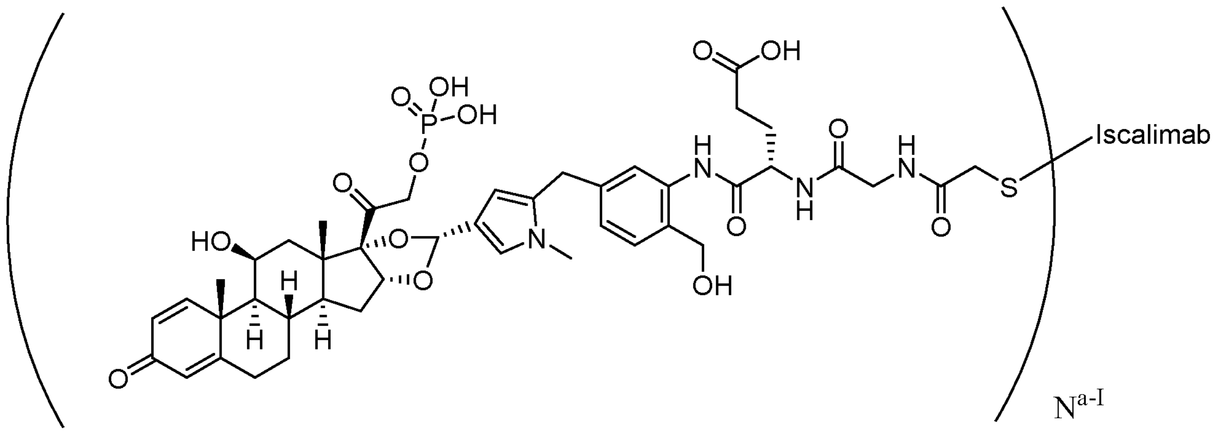 |

(continued)

| No. | Structure |
| --- | --- |
| V-261 | 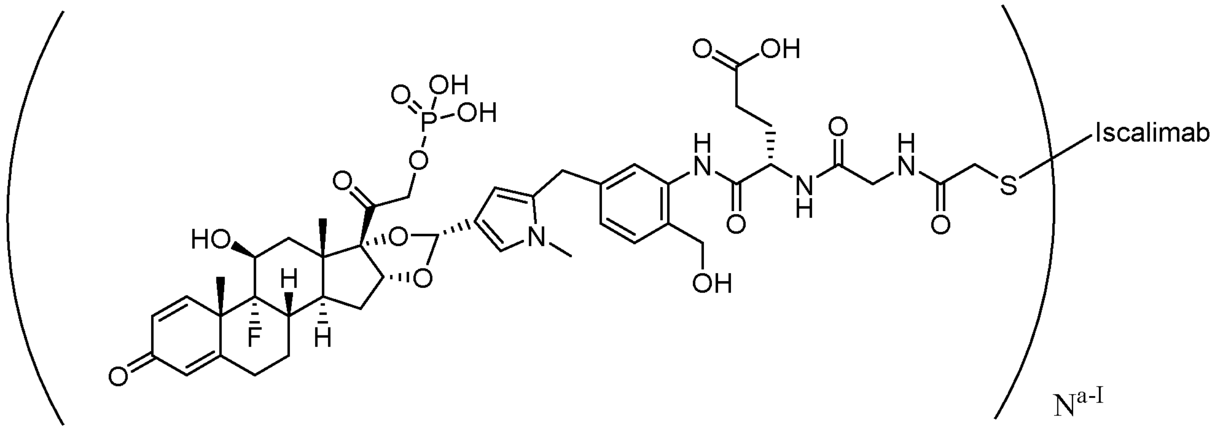 |
| V-262 | 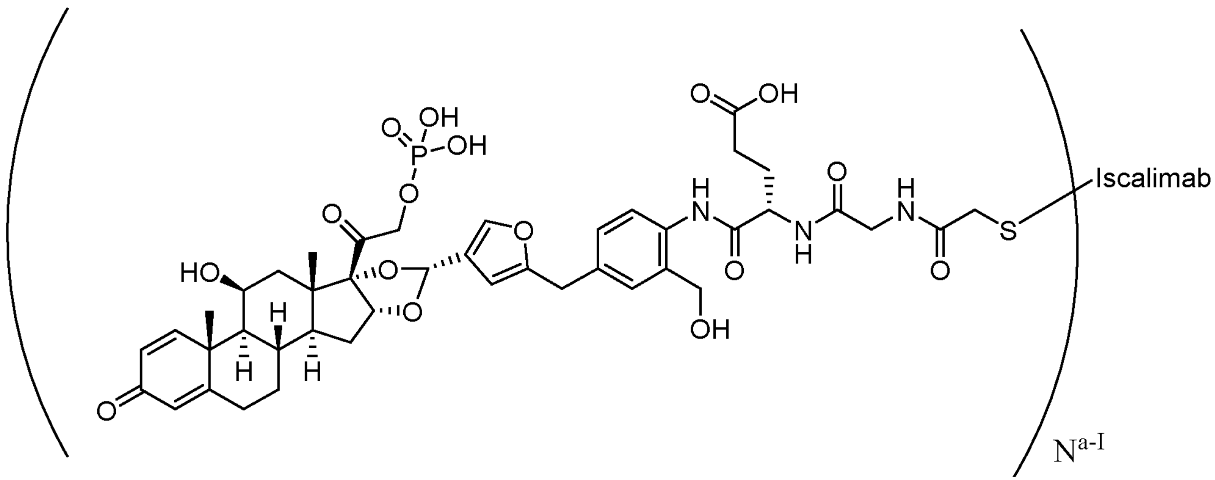 |

(continued)

| No. | Structure |
|---|---|
| V-263 | 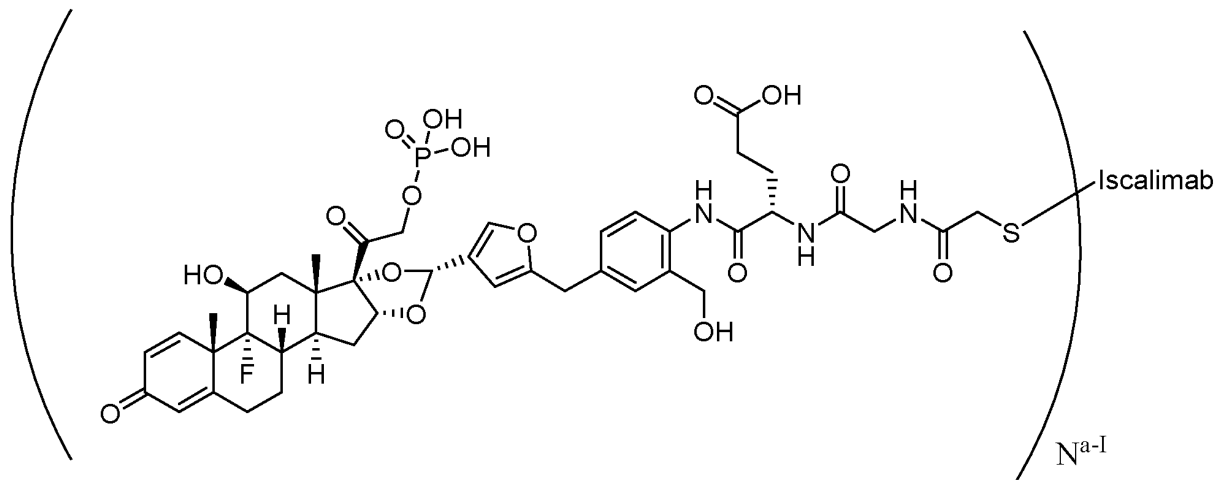 |
| V-264 | 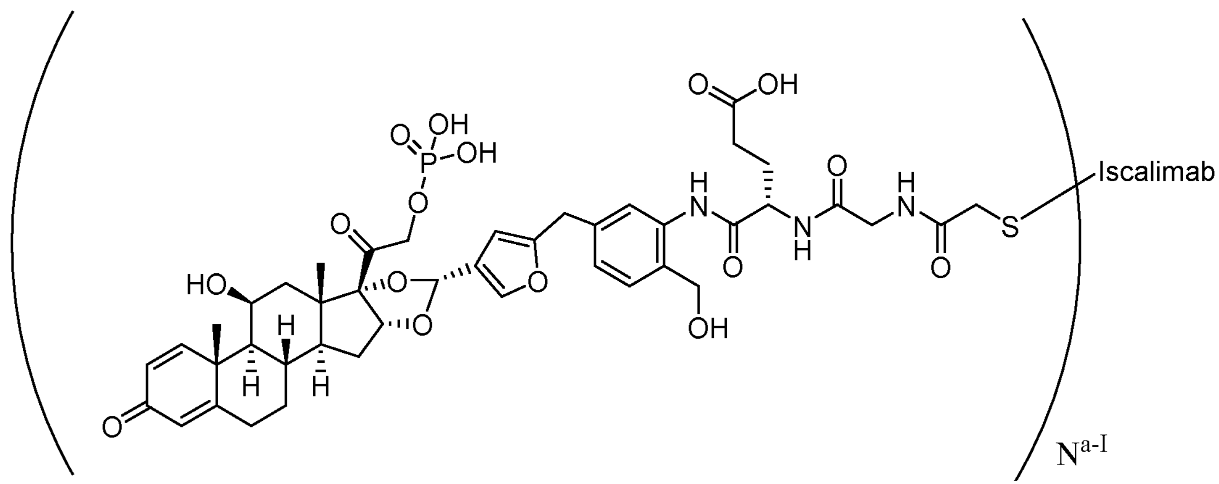 |

(continued)

| No. | Structure |
|---|---|
| V-265 | 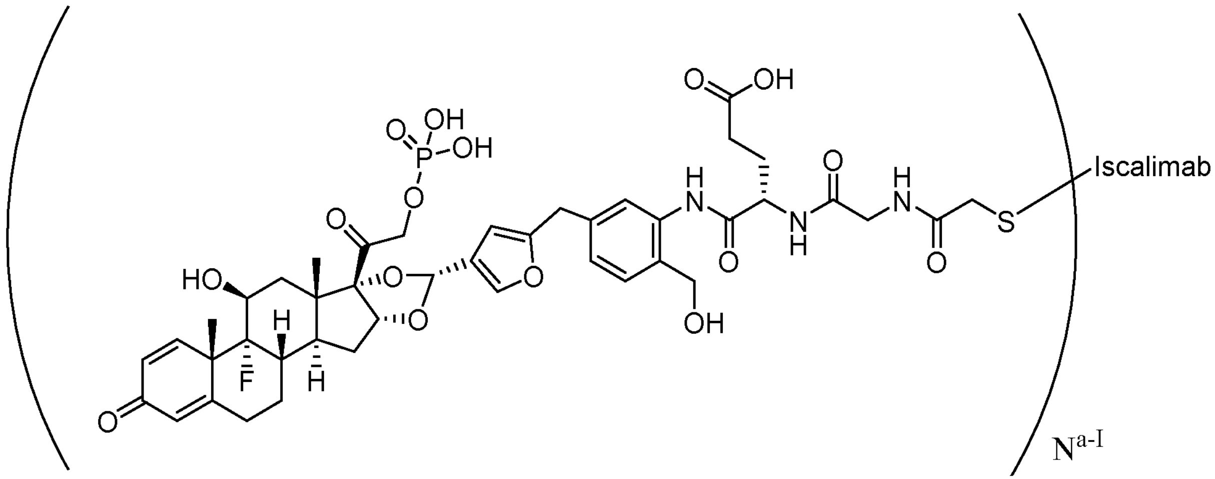 |
| V-266 | 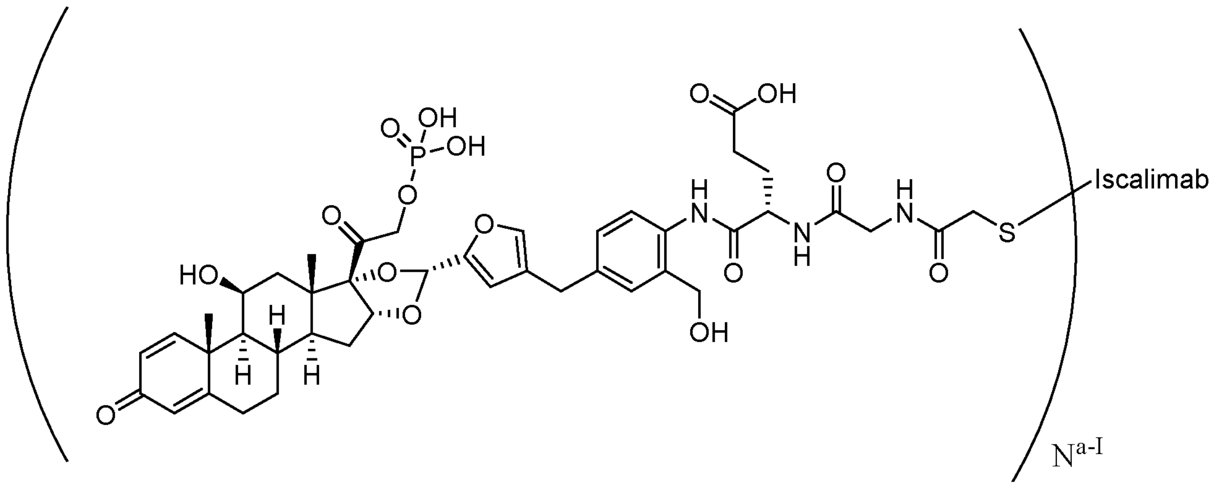 |

(continued)

| No. | Structure |
| --- | --- |
| V-267 | 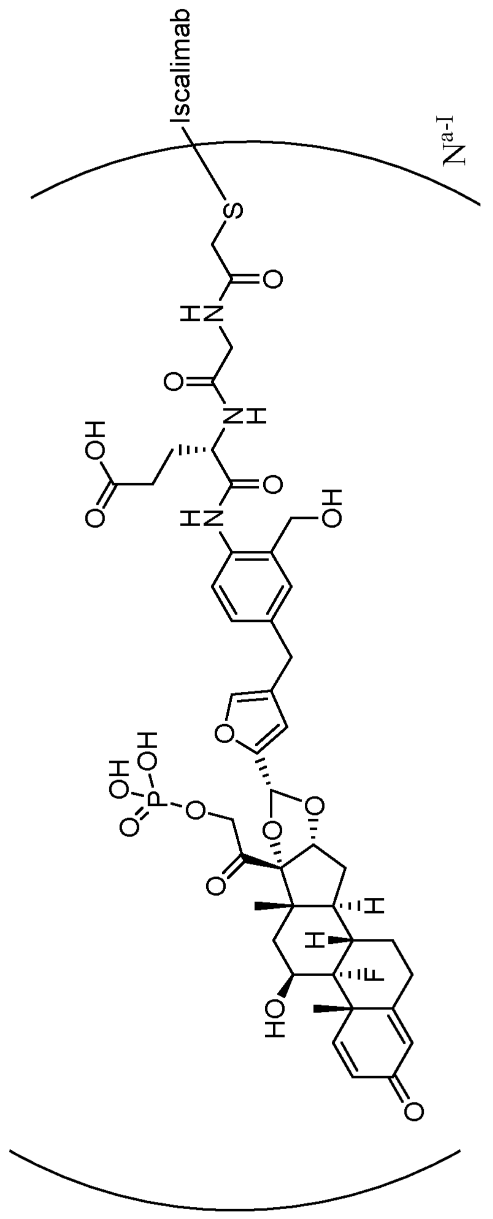 |
| V-268 | 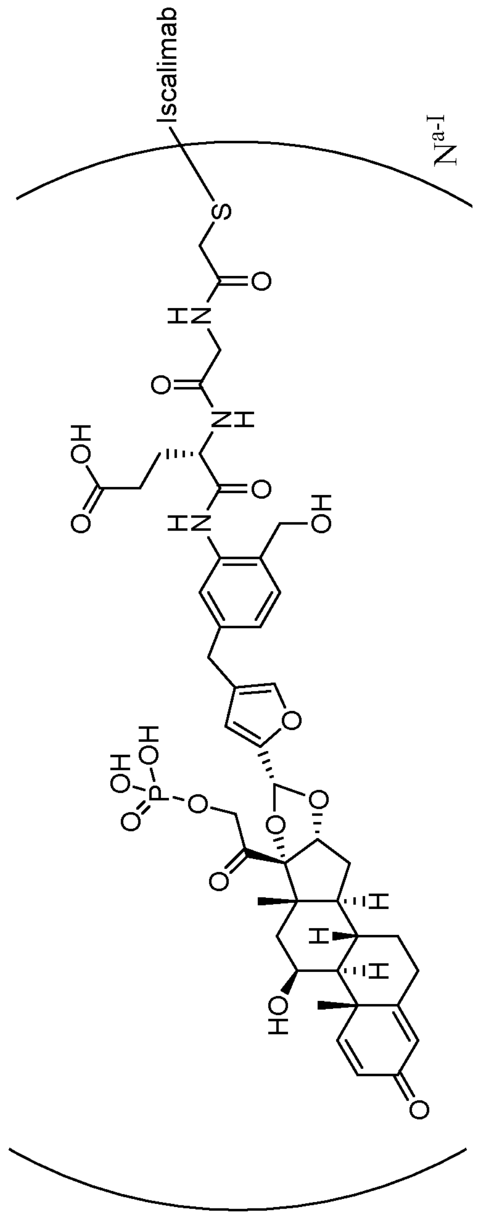 |

(continued)

| No. | Structure |
|---|---|
| V-269 | 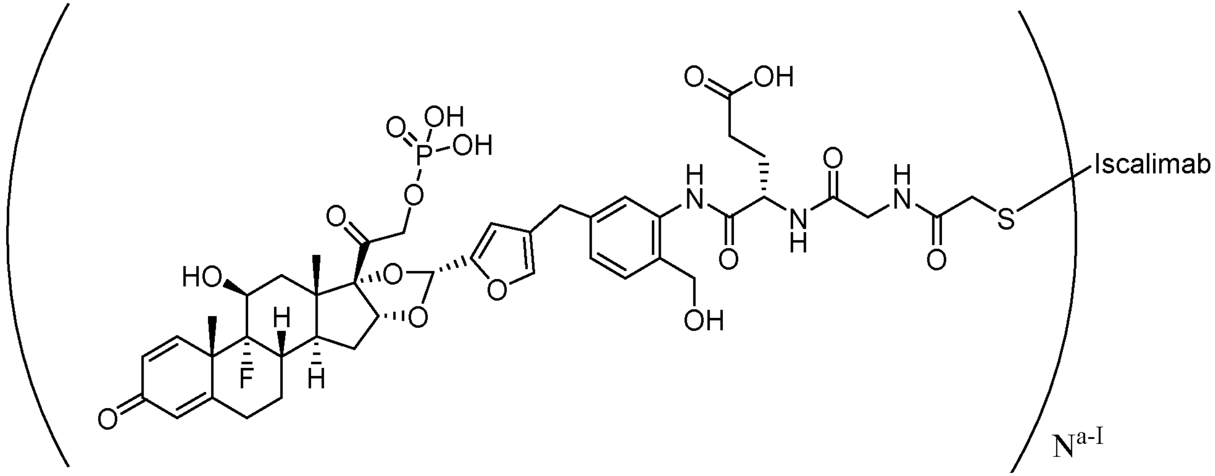 |
| V-270 | 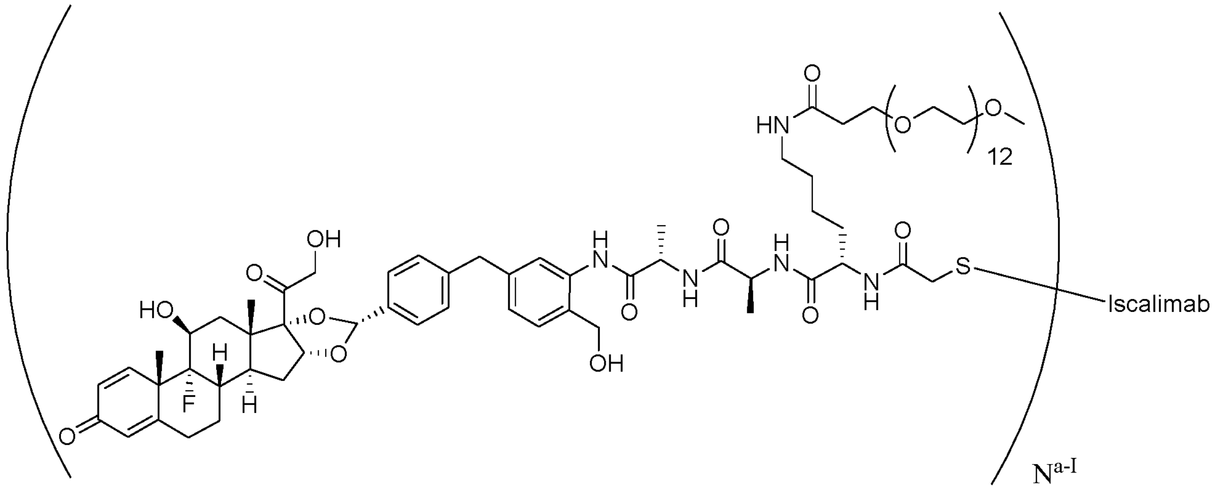 |

(continued)

| No. | Structure |
|---|---|
| V-271 | 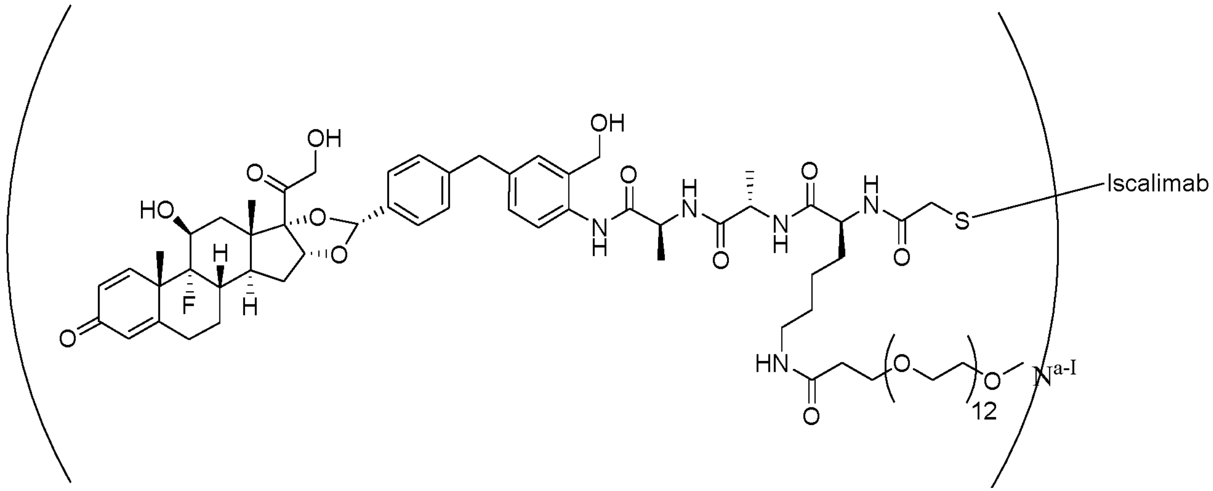 |
| V-272 | 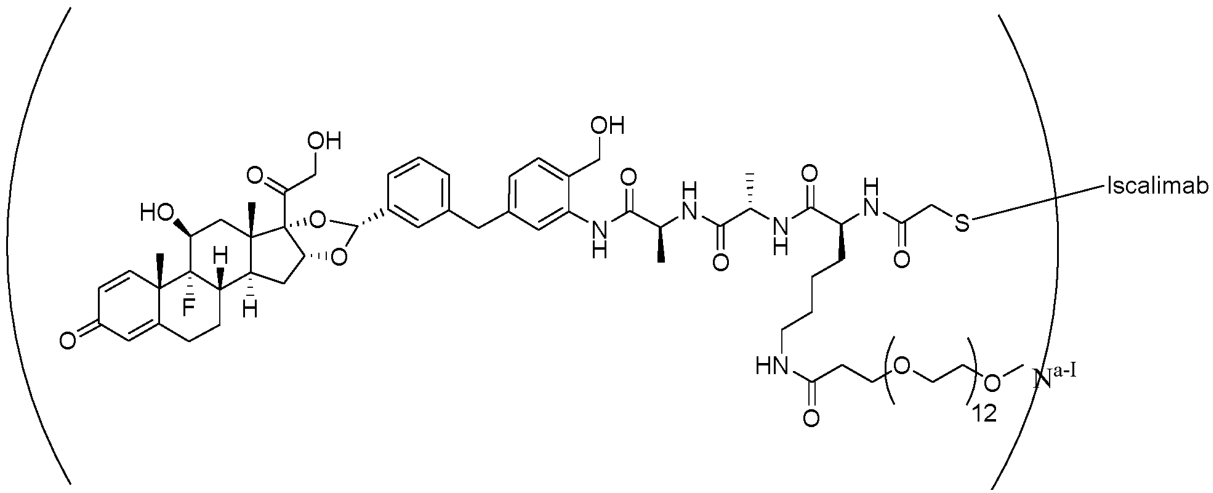 |

(continued)

| No. | Structure |
|---|---|
| V-273 | 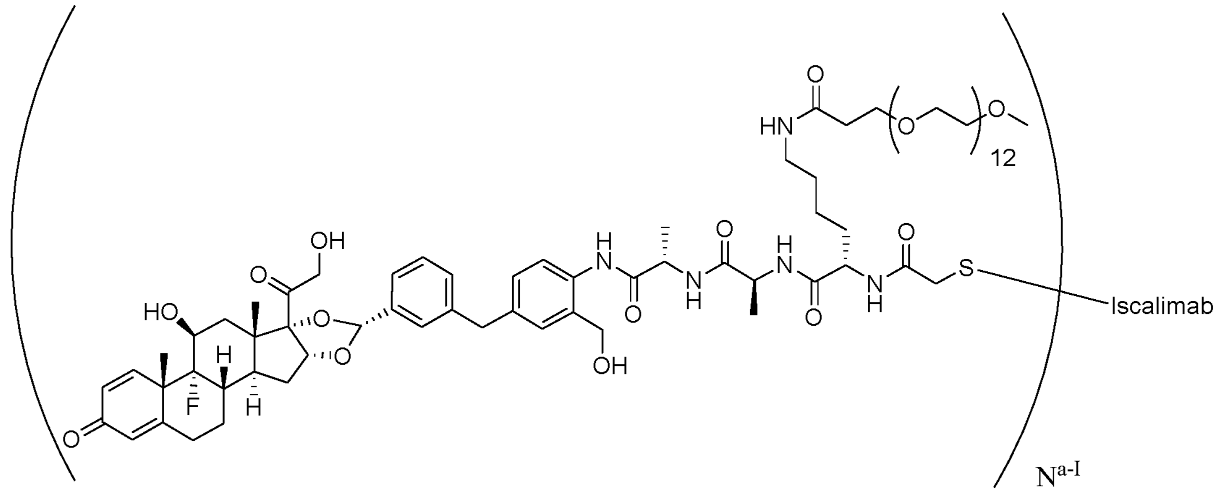 |
| V-274 | 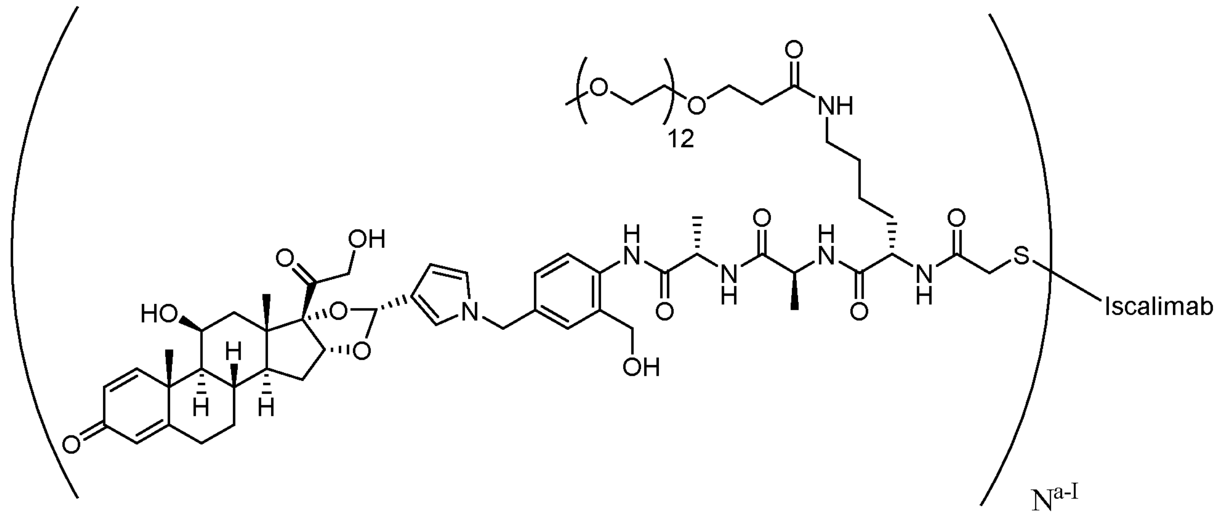 |

(continued)

| No. | Structure |
|---|---|
| V-275 | 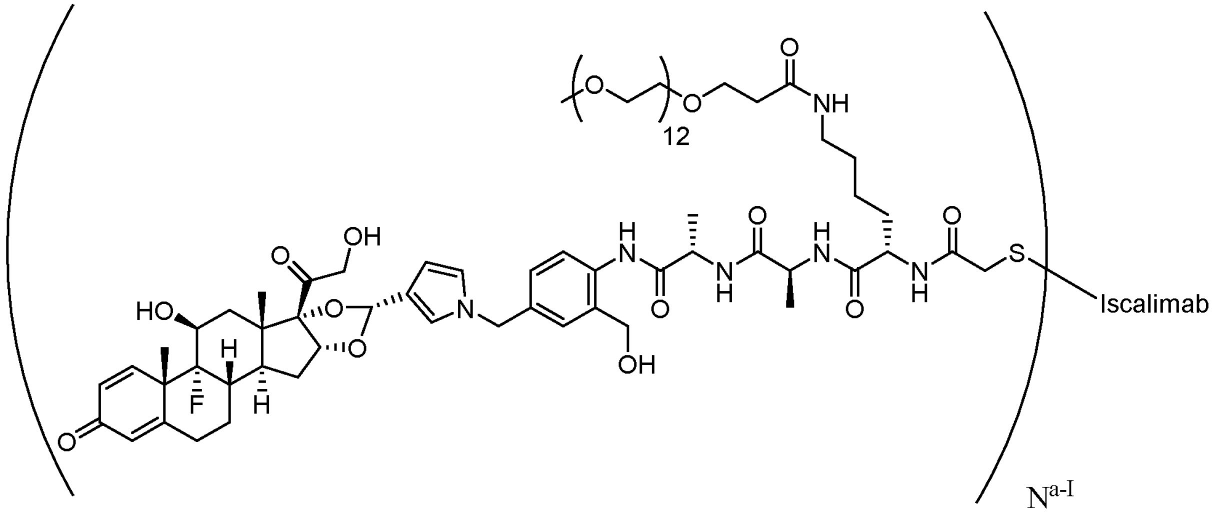 |
| V-276 | 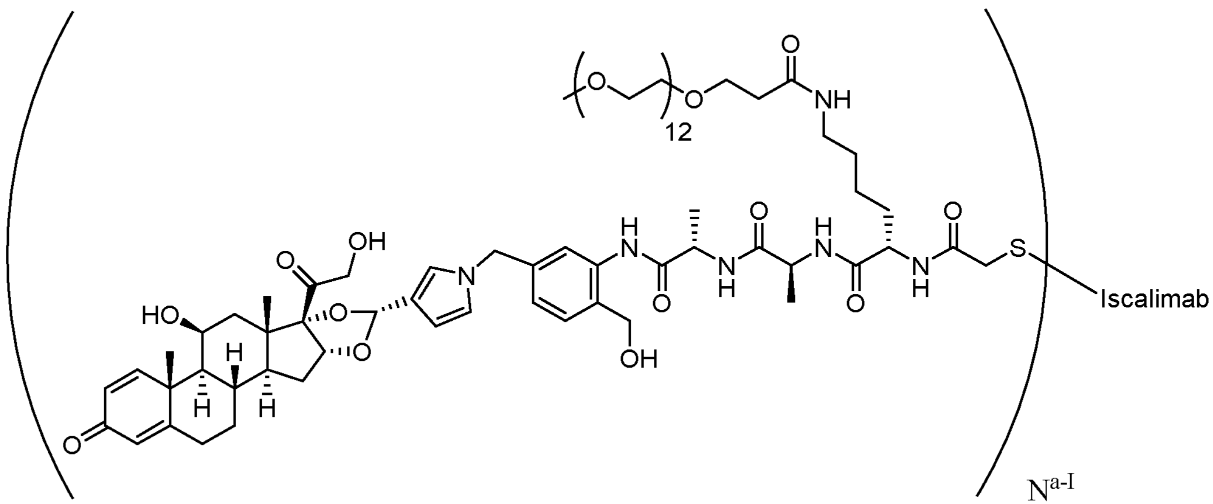 |

(continued)

| No. | Structure |
|---|---|
| V-277 | 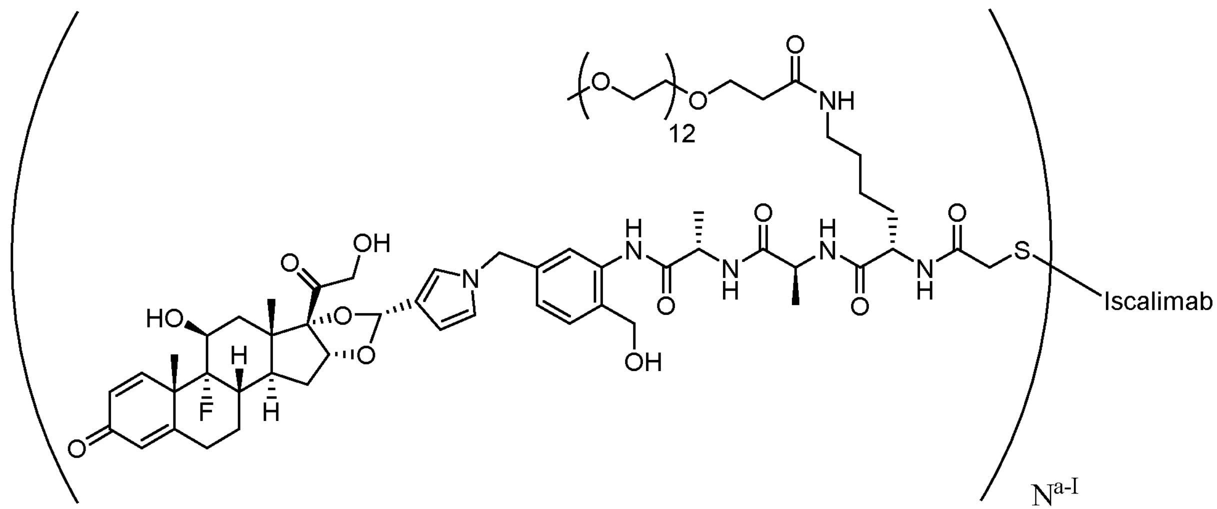 |
| V-278 | 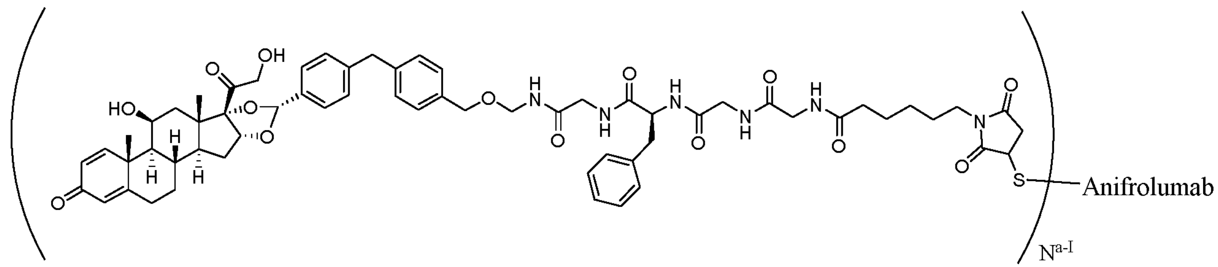 |
| V-279 | 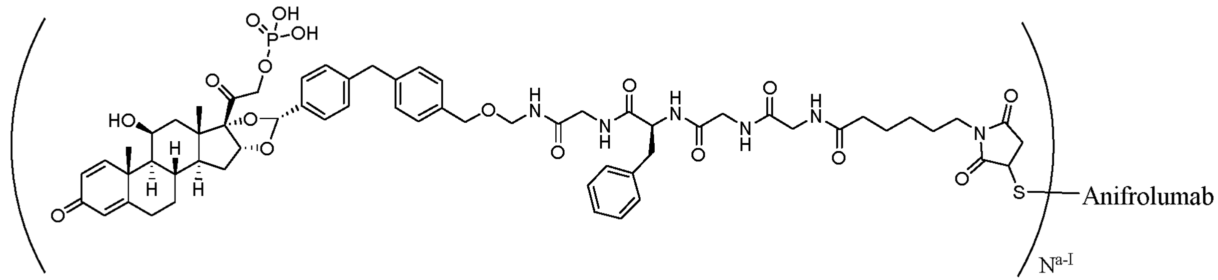 |

(continued)

| No. | Structure |
|---|---|
| V-283 | 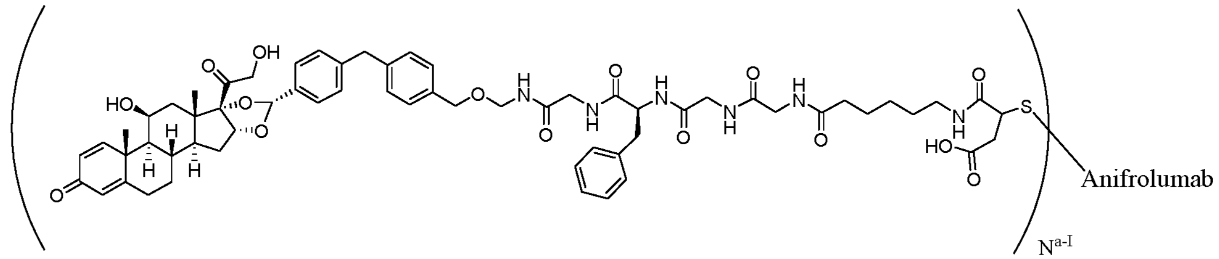 |
| V-284 | 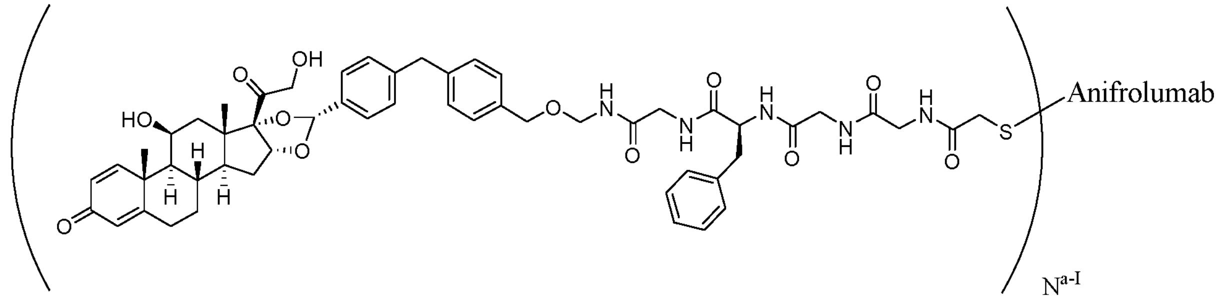 |
| V-285 | 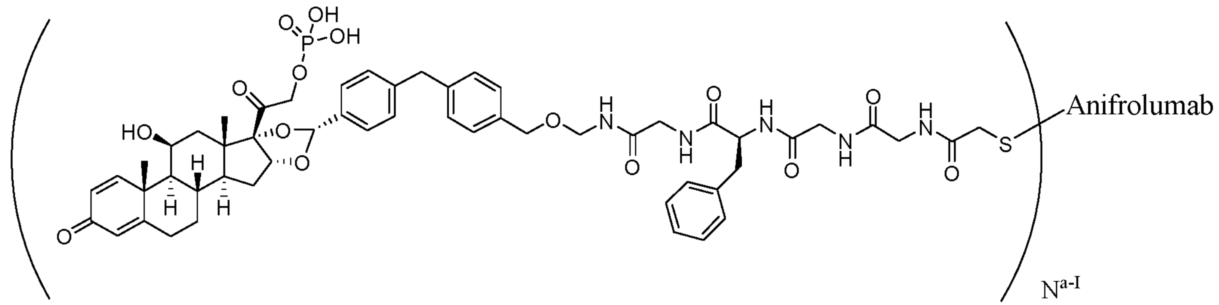 |

(continued)

| No. | Structure |
|---|---|
| V-289 | 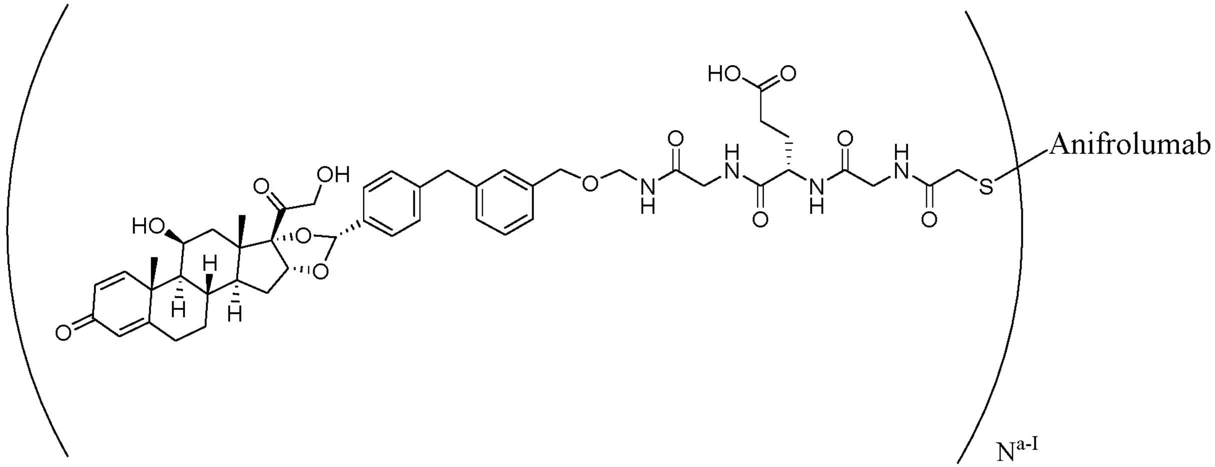 |
| V-290 | 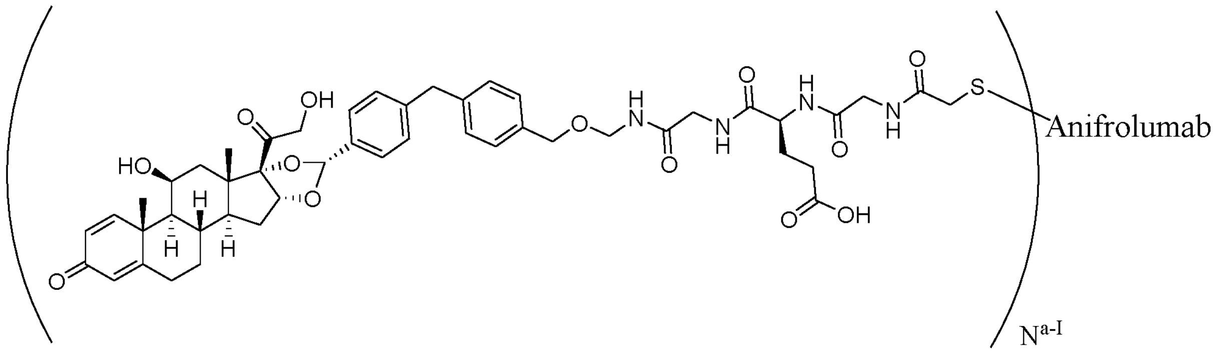 |

(continued)

| No. | Structure |
|---|---|
| V-291 | 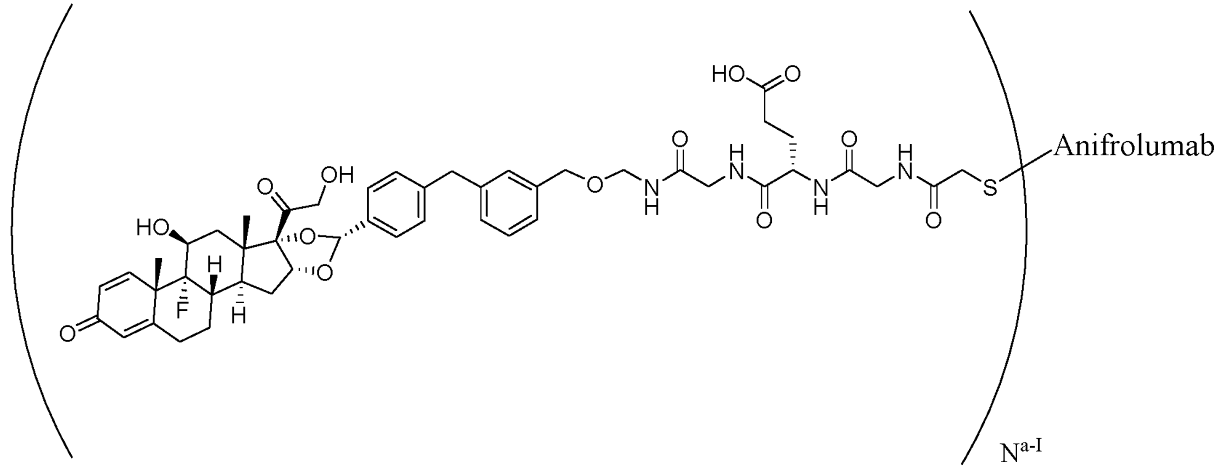 |
| V-292 | 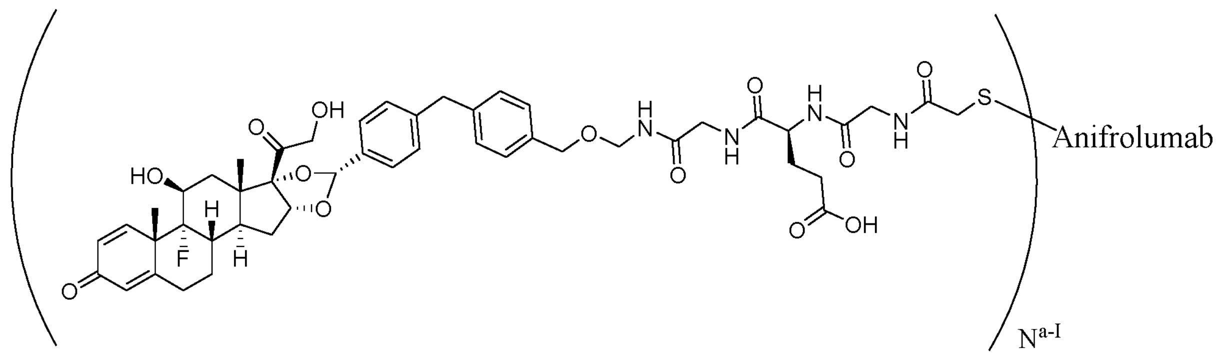 |

(continued)

| No. | Structure |
| --- | --- |
| V-293 | 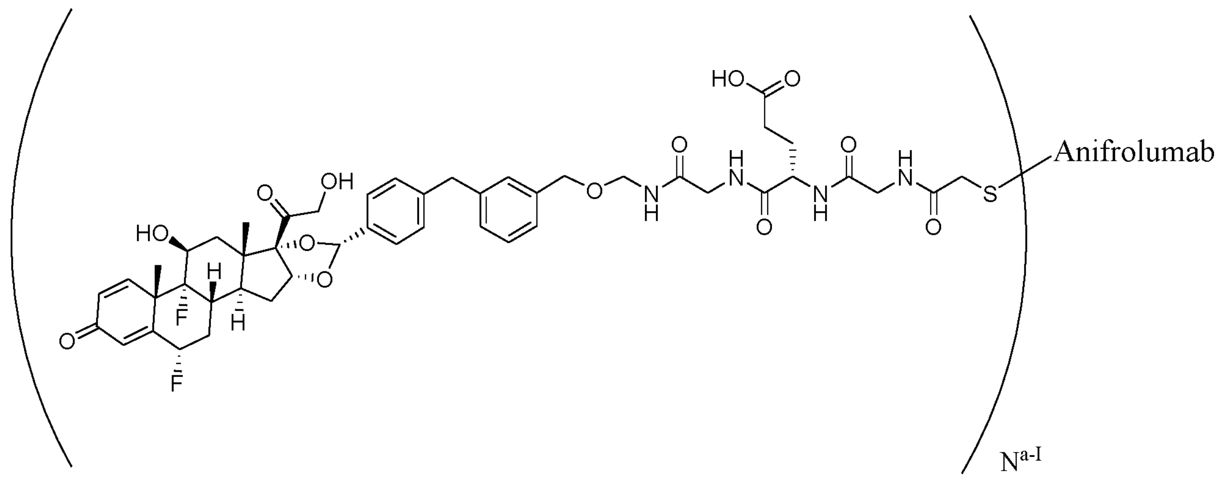 |
| V-294 | 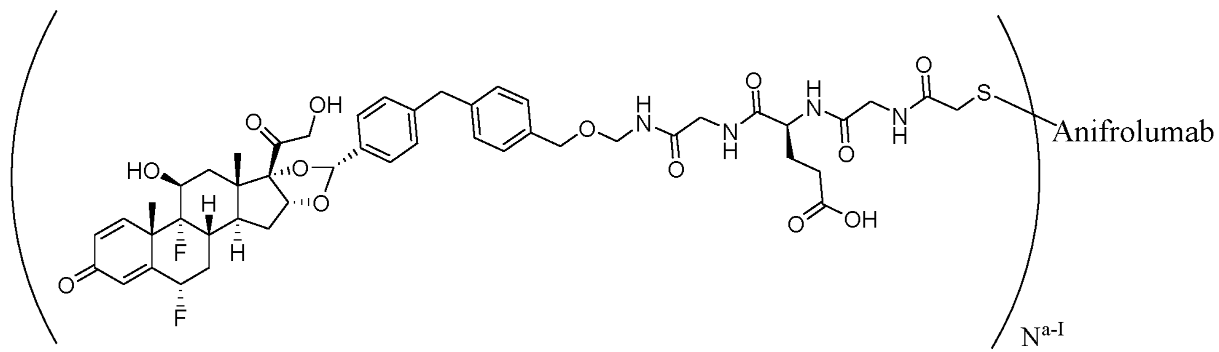 |

(continued)

| No. | Structure |
|---|---|
| V-295 | 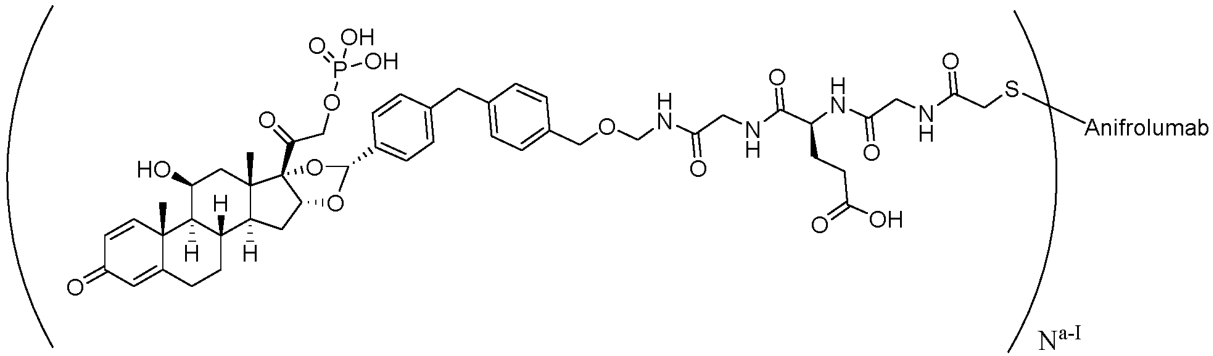 |
| V-299 | 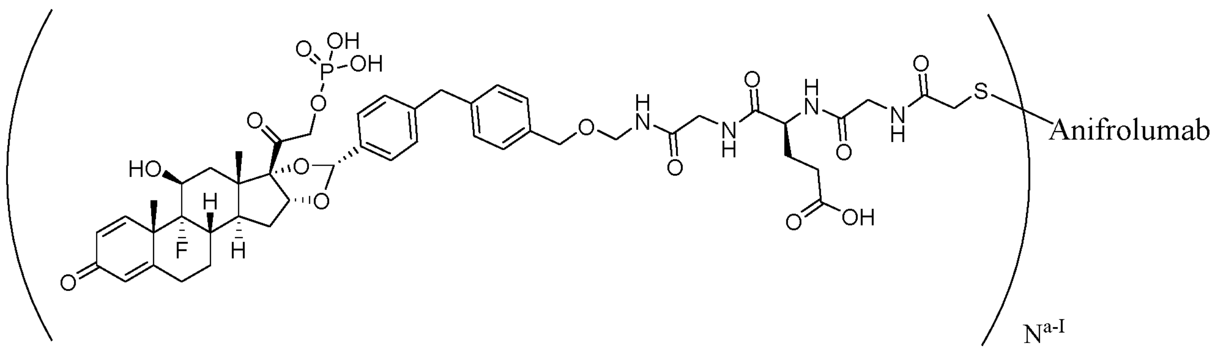 |
| V-302 | 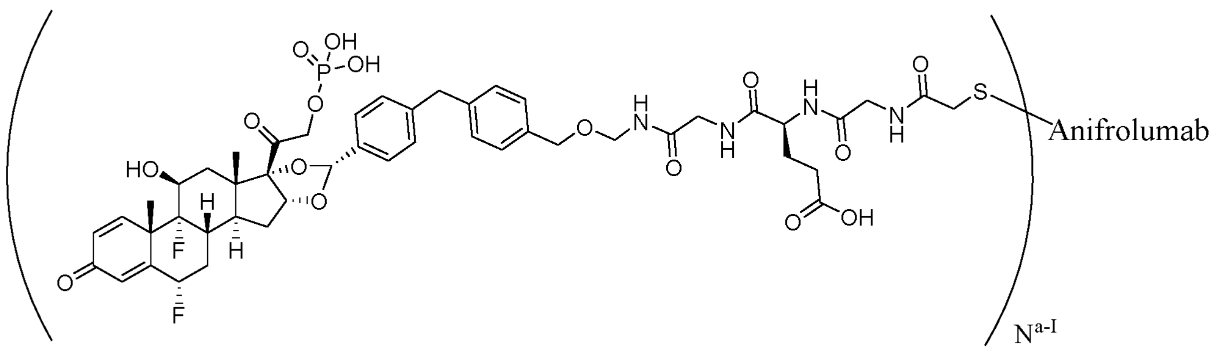 |

(continued)

| No. | Structure |
|---|---|
| V-306 | 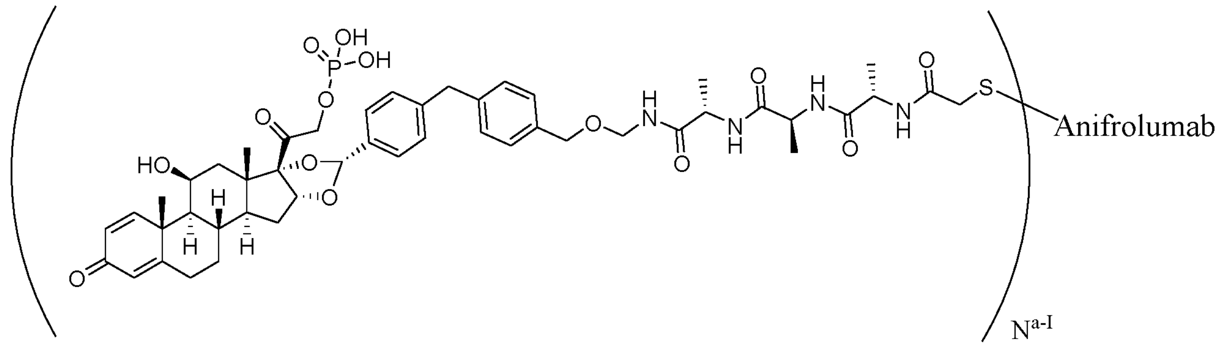 |
| V-307 | 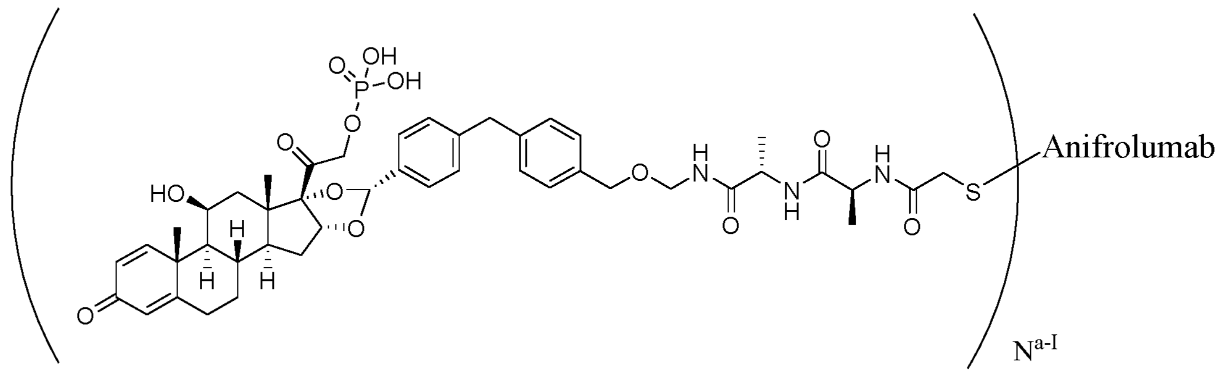 |
| V-311 | 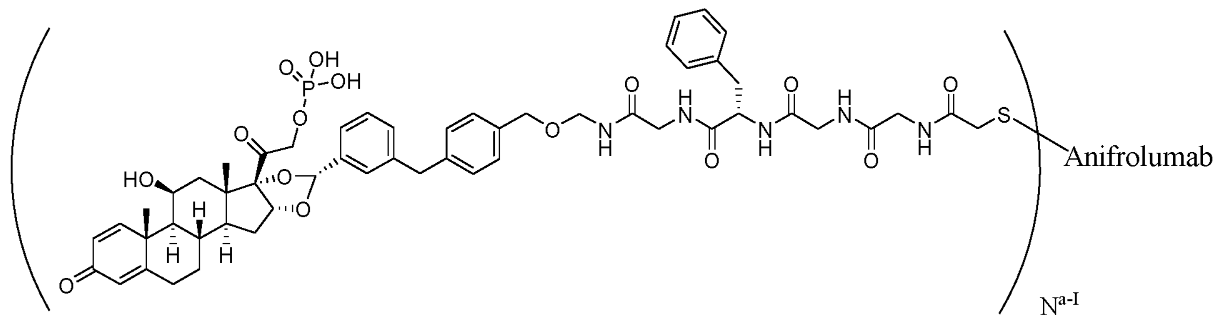 |

(continued)

| No. | Structure |
|---|---|
| V-312 | 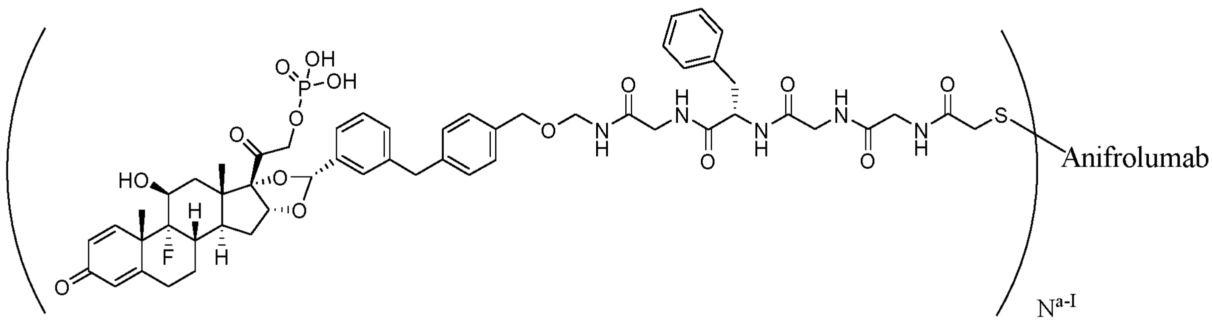 |
| V-316 | 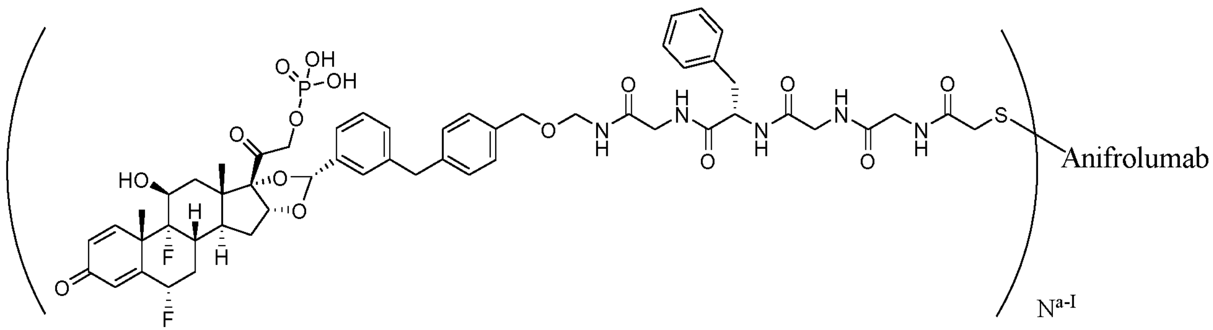 |
| V-320 | 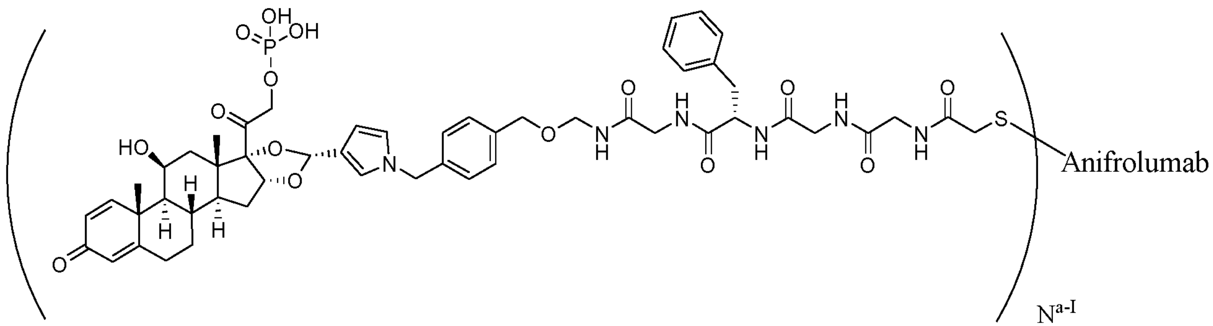 |

(continued)

| No. | Structure |
|---|---|
| V-321 | 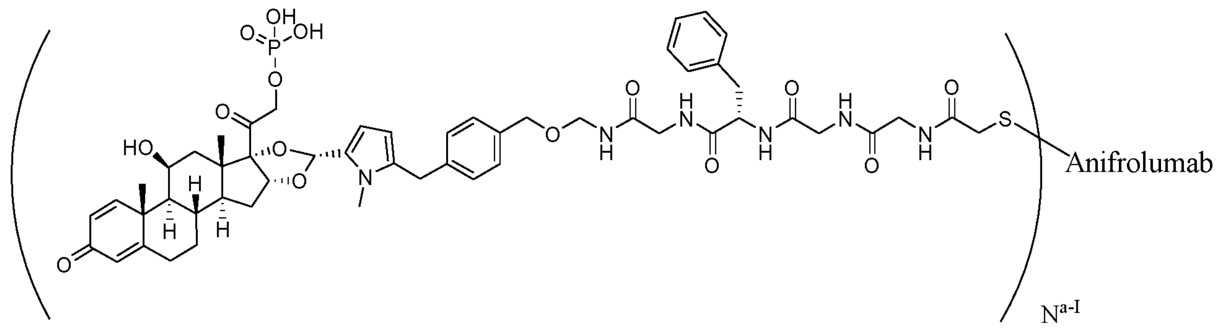 |
| V-322 | 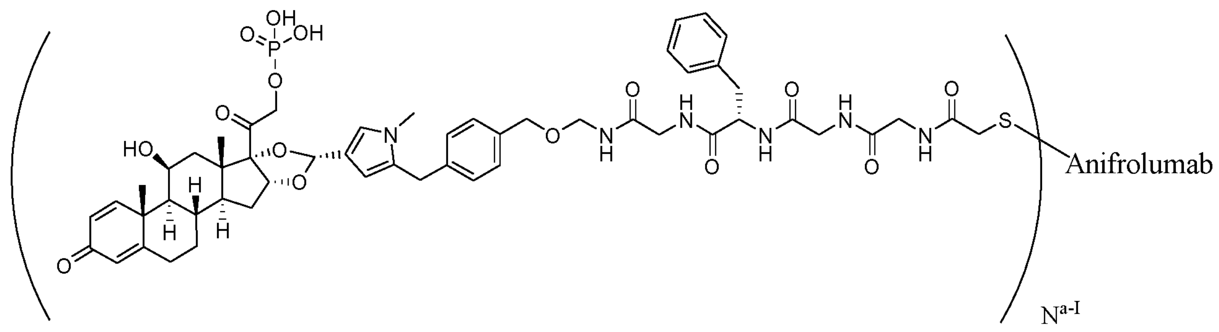 |
| V-323 | 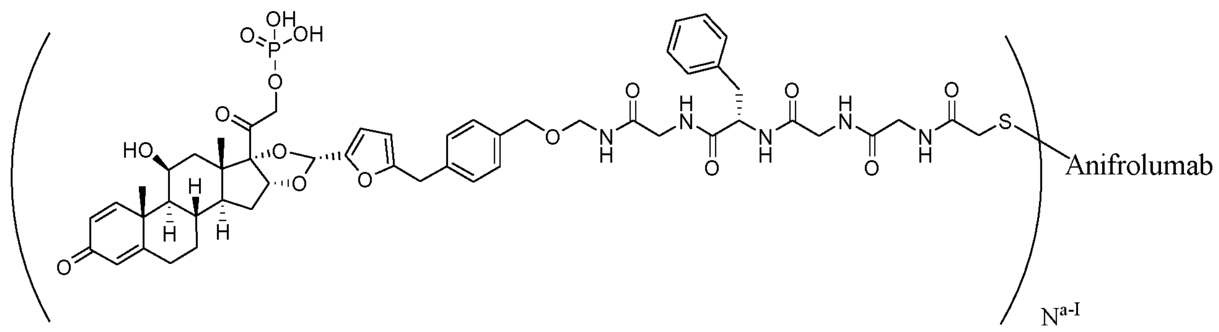 |

(continued)

| No. | Structure |
|---|---|
| V-324 | 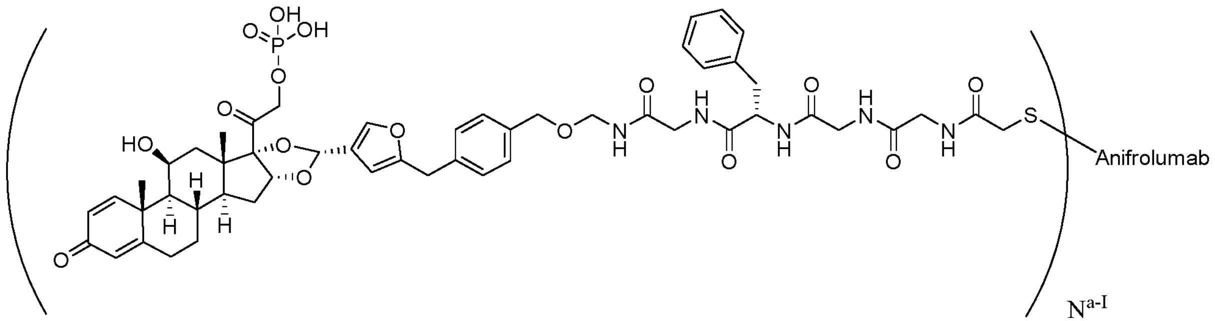 |
| V-325 | 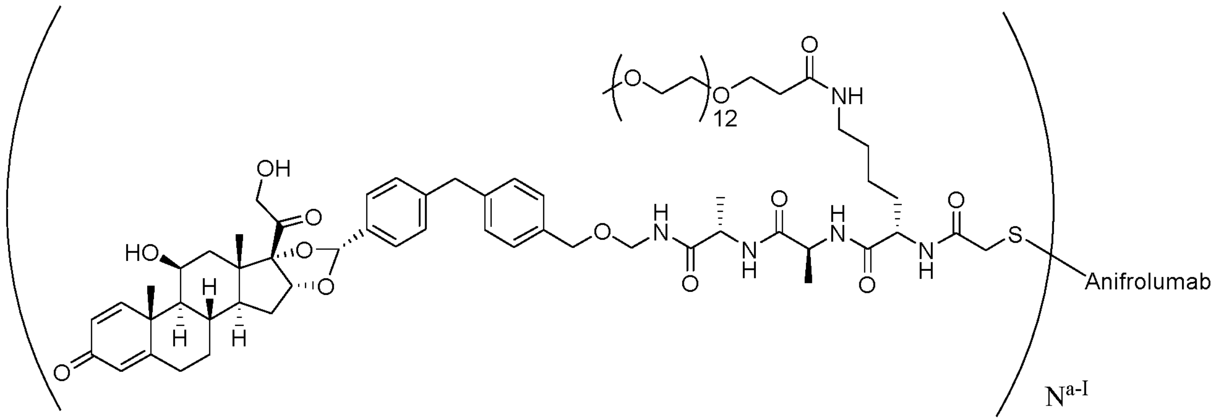 |

(continued)

| No. | Structure |
|---|---|
| V-326 | 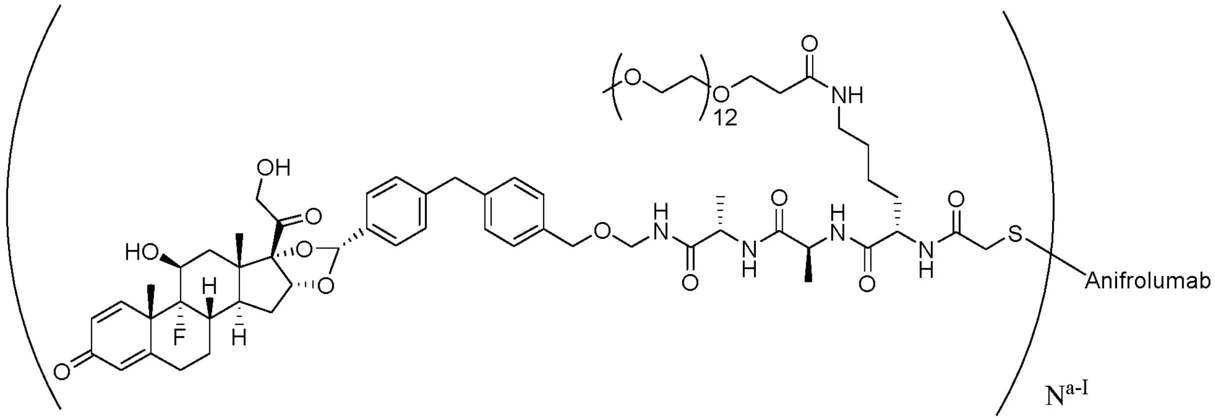 |
| V-327 | 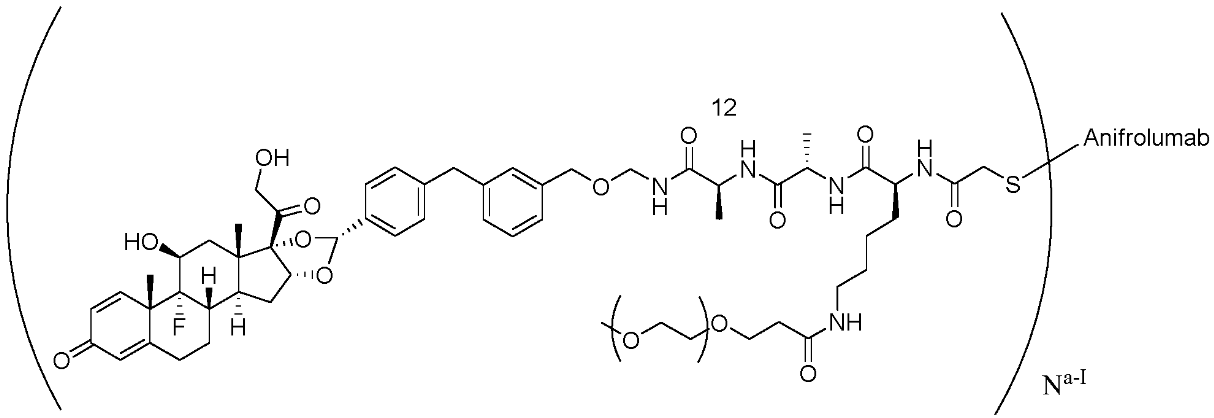 |

(continued)

| No. | Structure |
|---|---|
| V-328 | 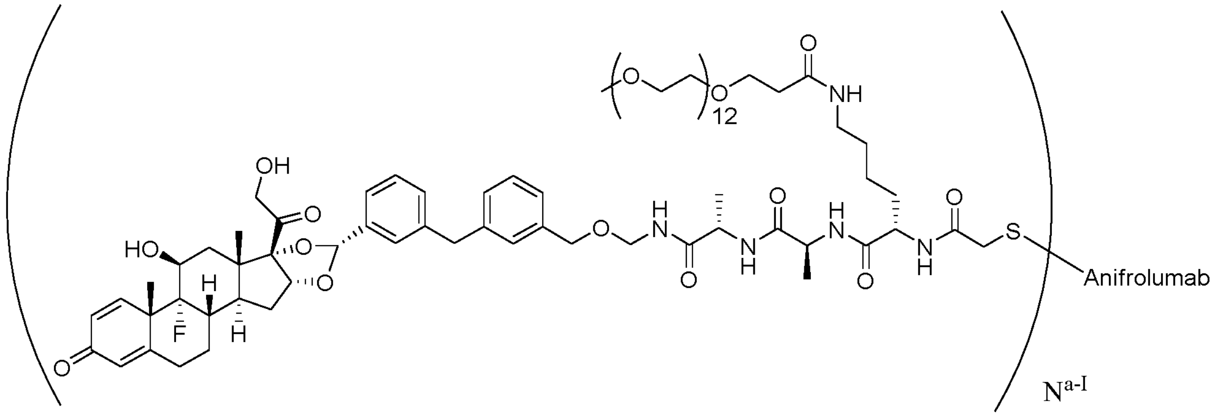 |
| V-329 | 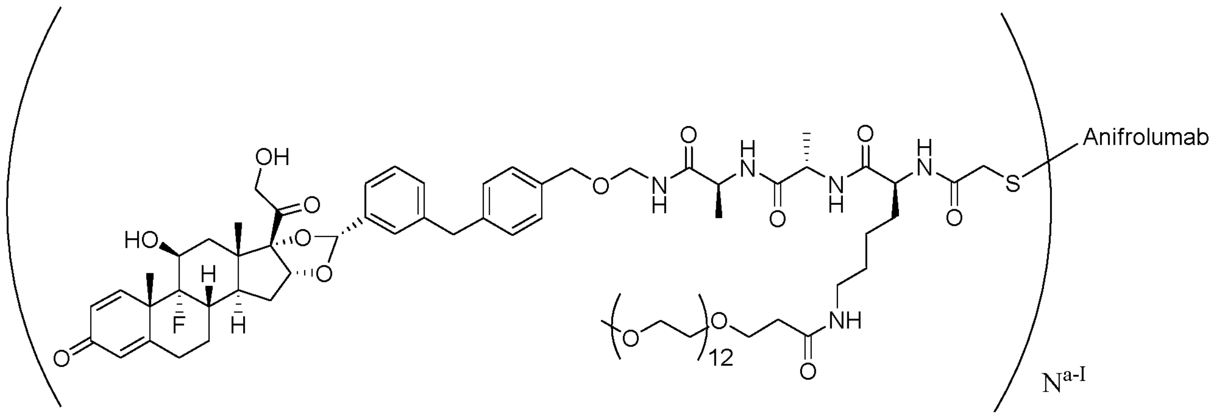 |

(continued)

| No. | Structure |
|---|---|
| V-330 | 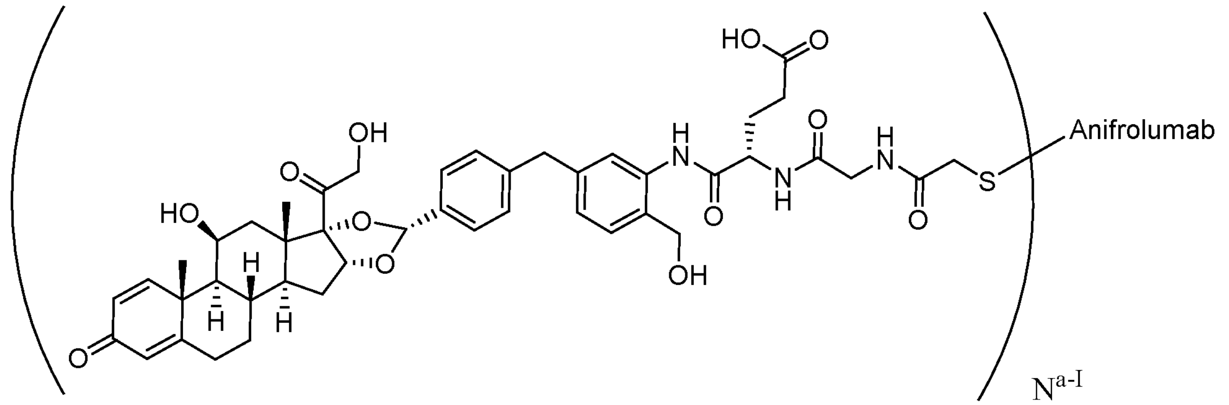 |
| V-331 | 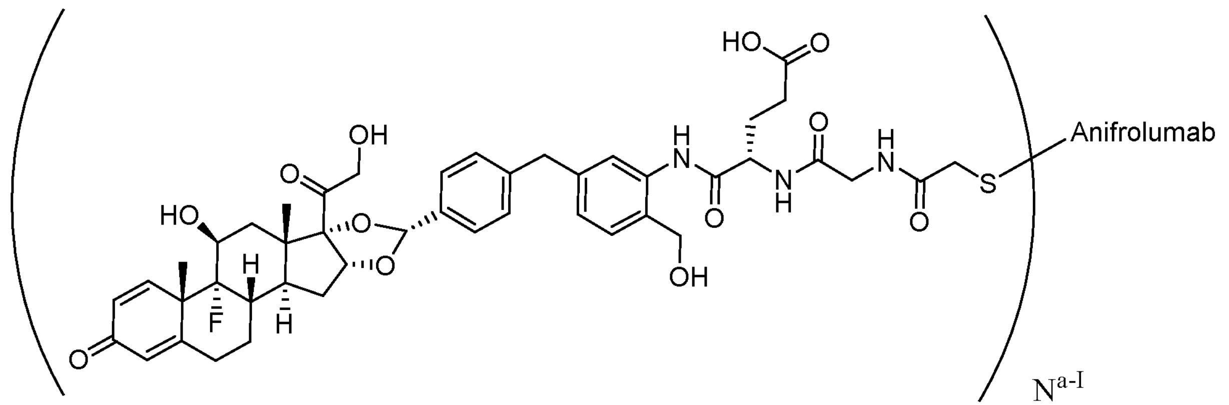 |

(continued)

| No. | Structure |
|---|---|
| V-332 | 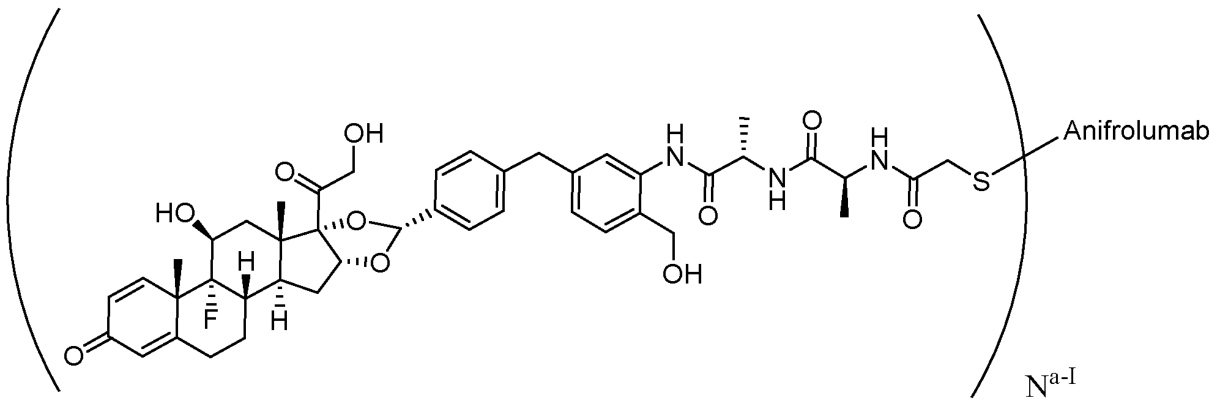 |
| V-333 | 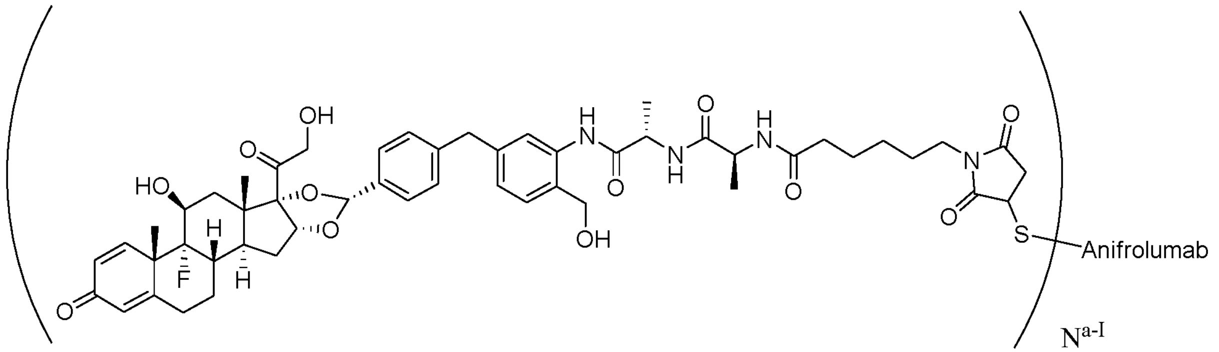 |

(continued)

| No. | Structure |
|---|---|
| V-334 | 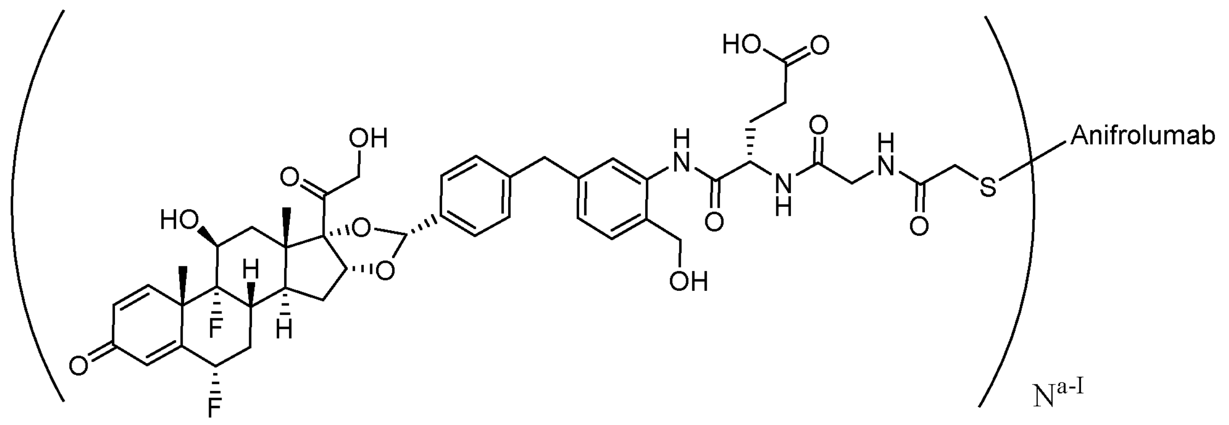 |
| V-335 | 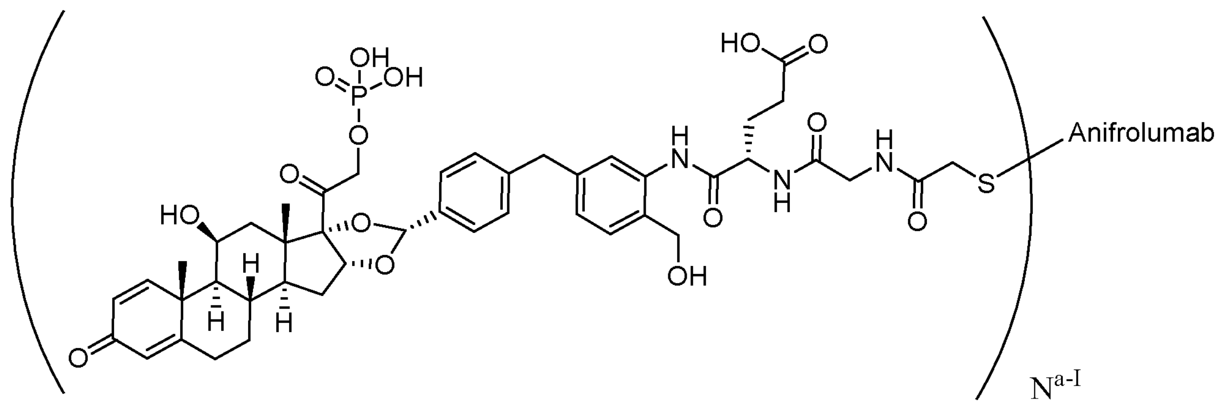 |

(continued)

| No. | Structure |
|---|---|
| V-336 | 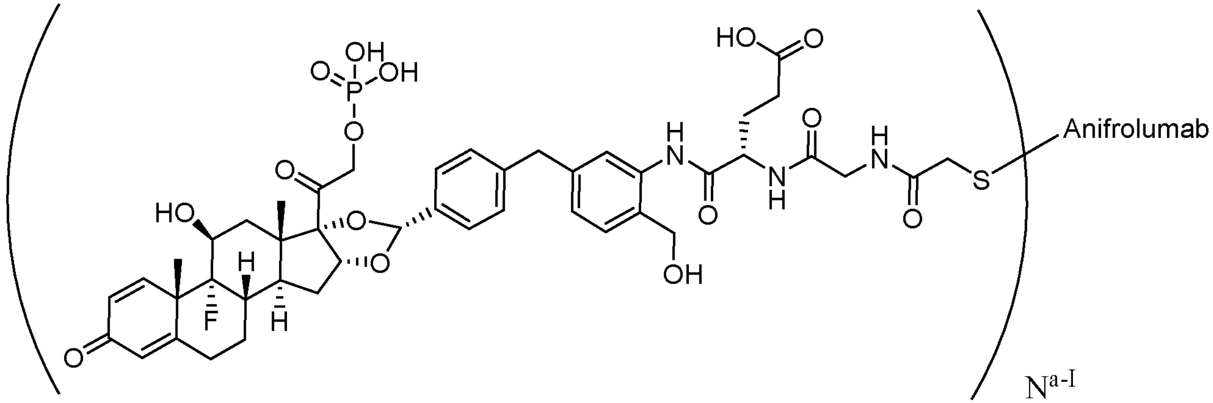 |
| V-337 | 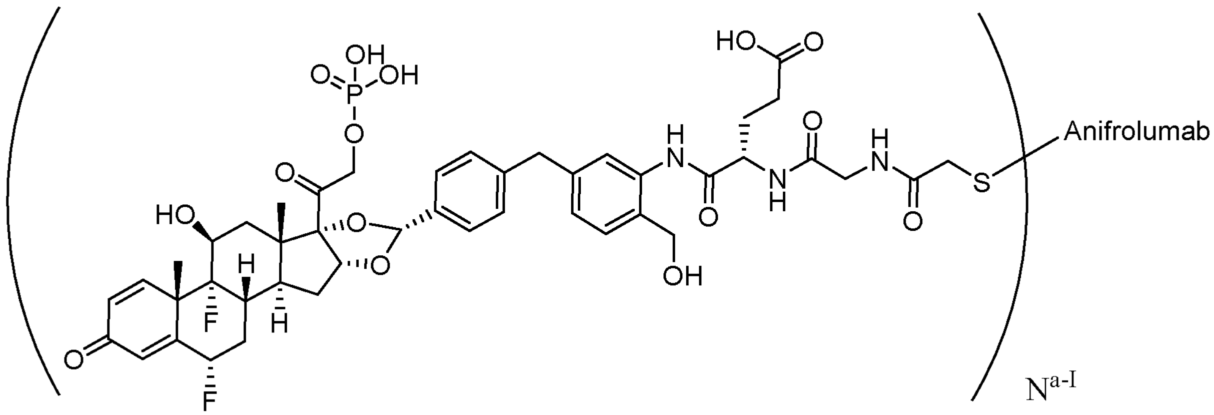 |

(continued)

| No. | Structure |
|---|---|
| V-347 | 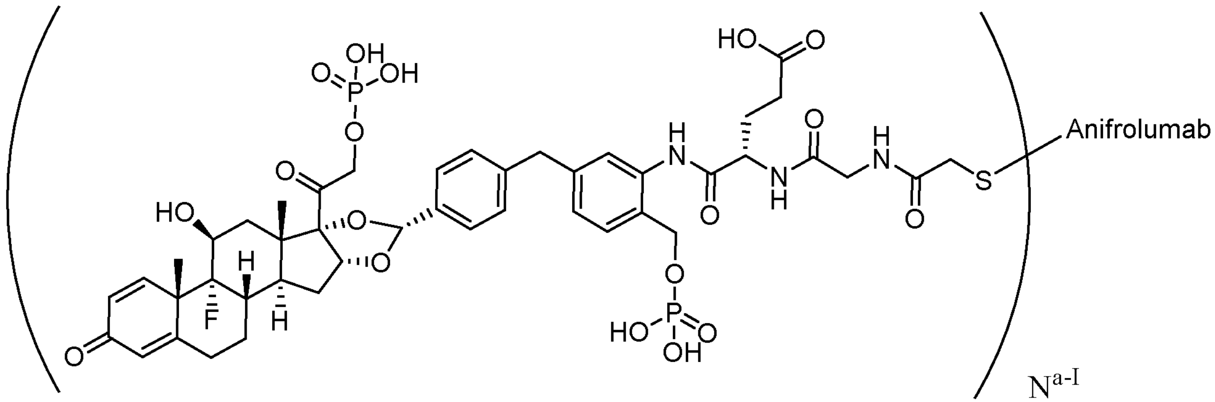 |
| V-351 | 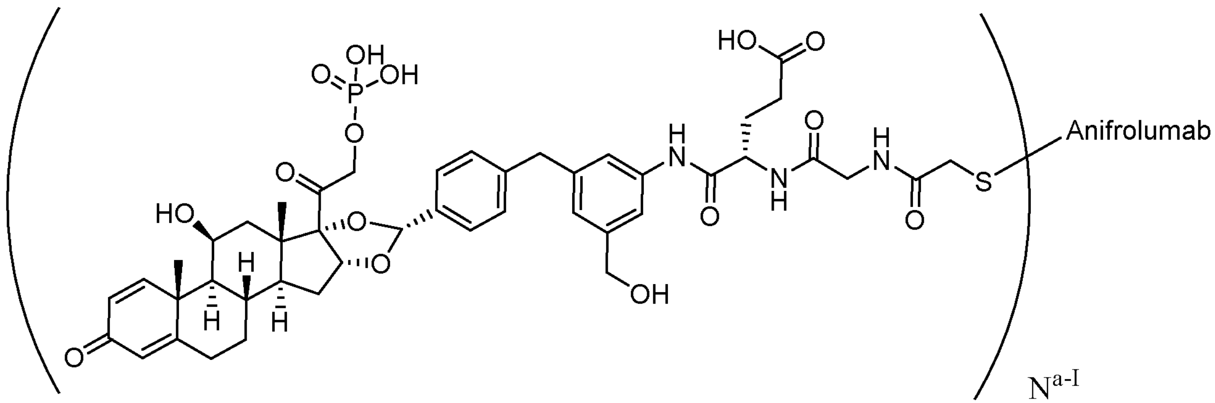 |

(continued)

| No. | Structure |
|---|---|
| V-354 | 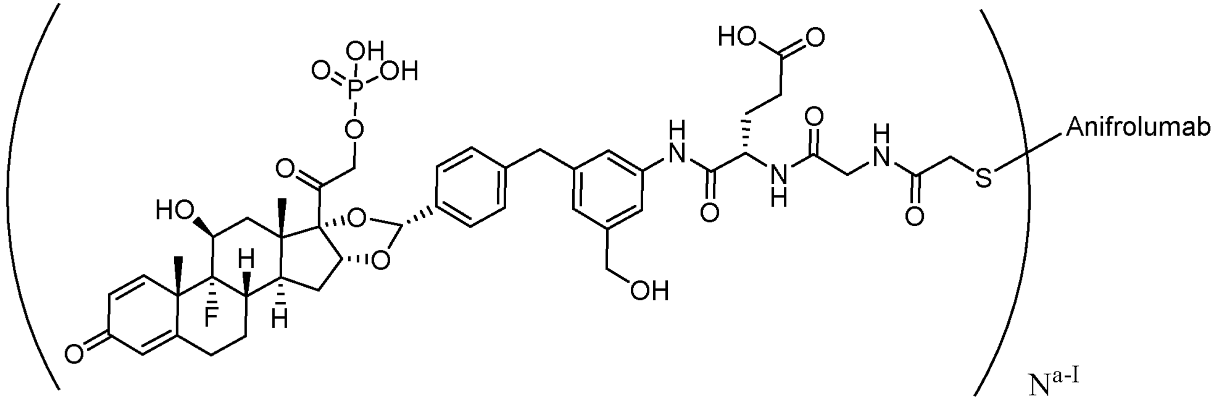 |
| V-357 | 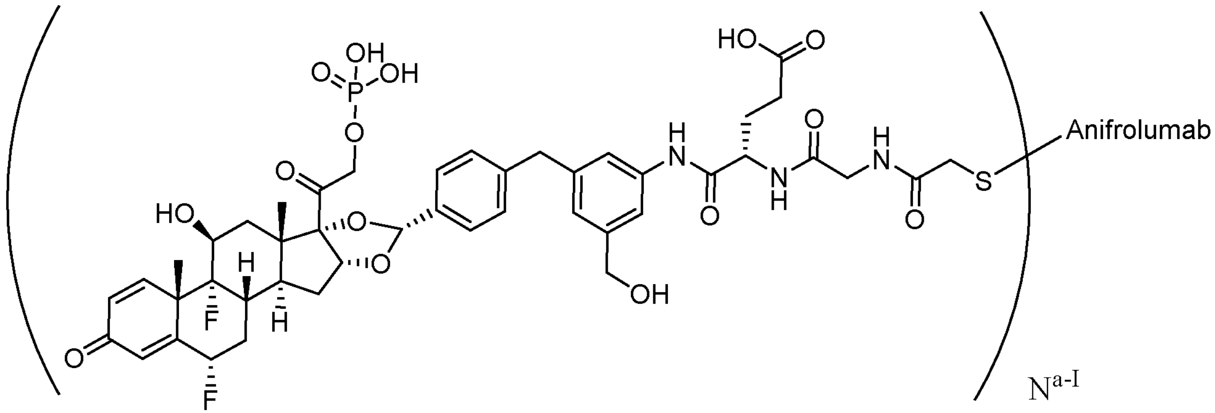 |

(continued)

| No. | Structure |
|---|---|
| V-360 | 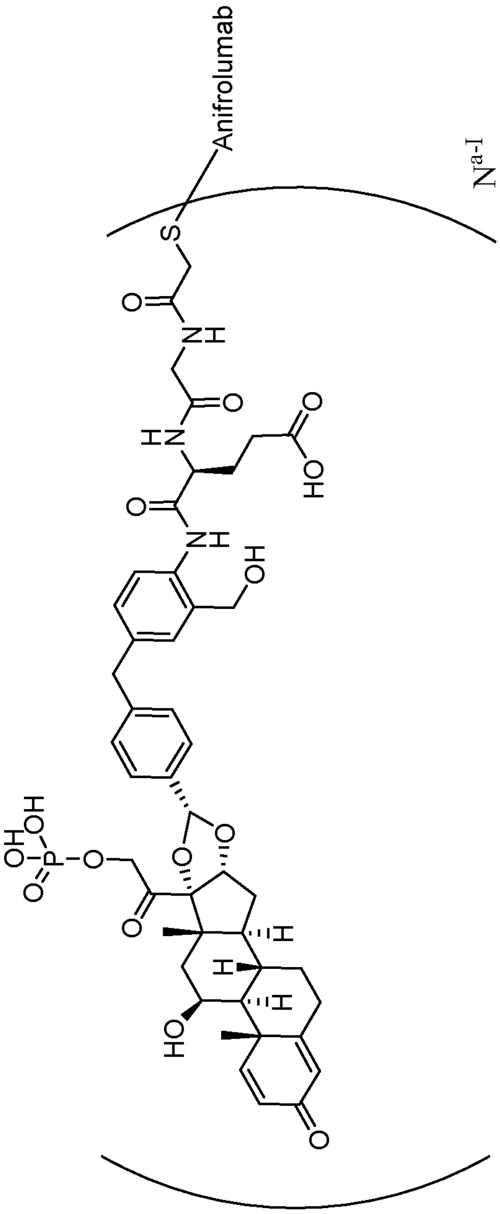 |
| V-363 | 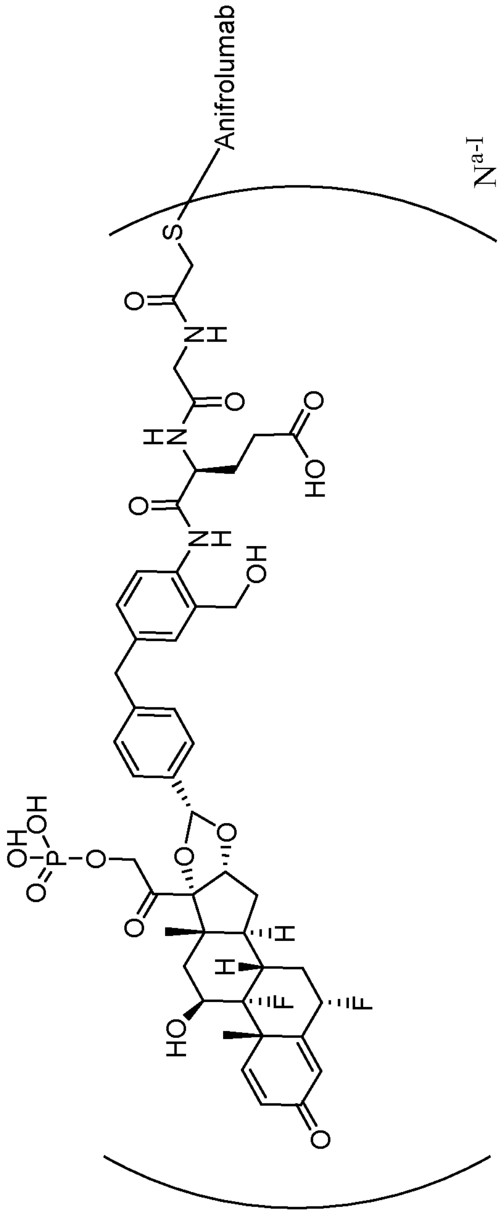 |

(continued)

| No. | Structure |
|---|---|
| V-366 | 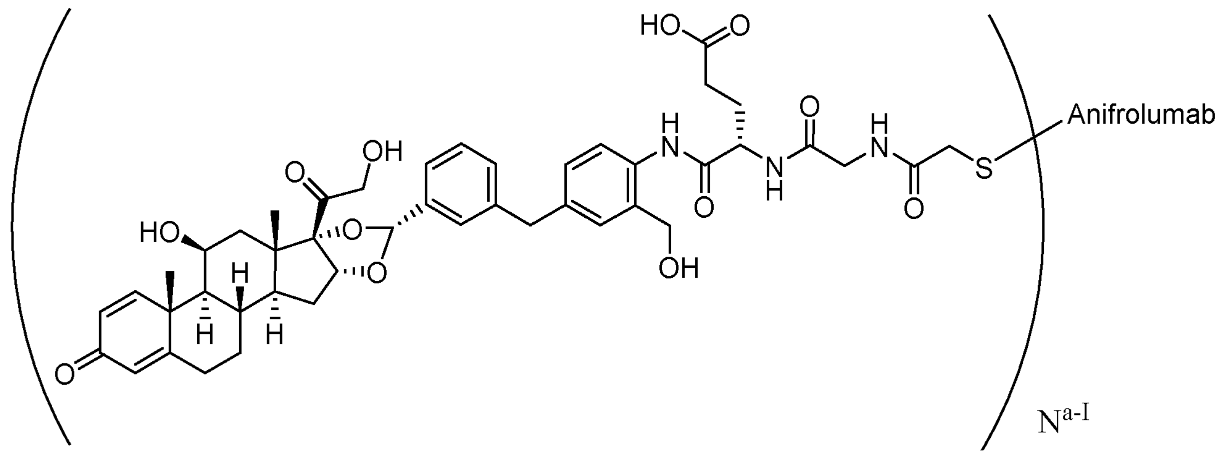 |
| V-367 | 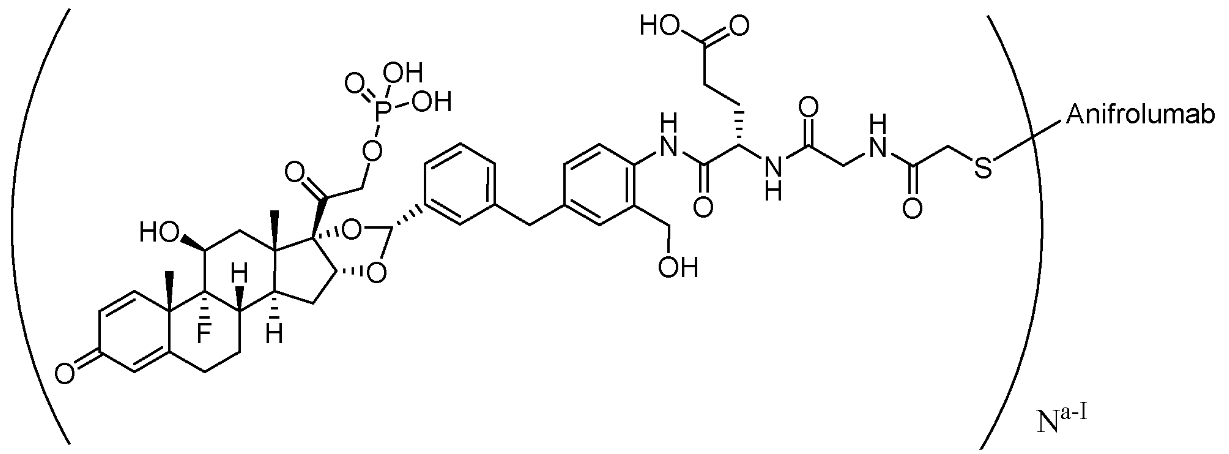 |

(continued)

| No. | Structure |
|---|---|
| V-368 | 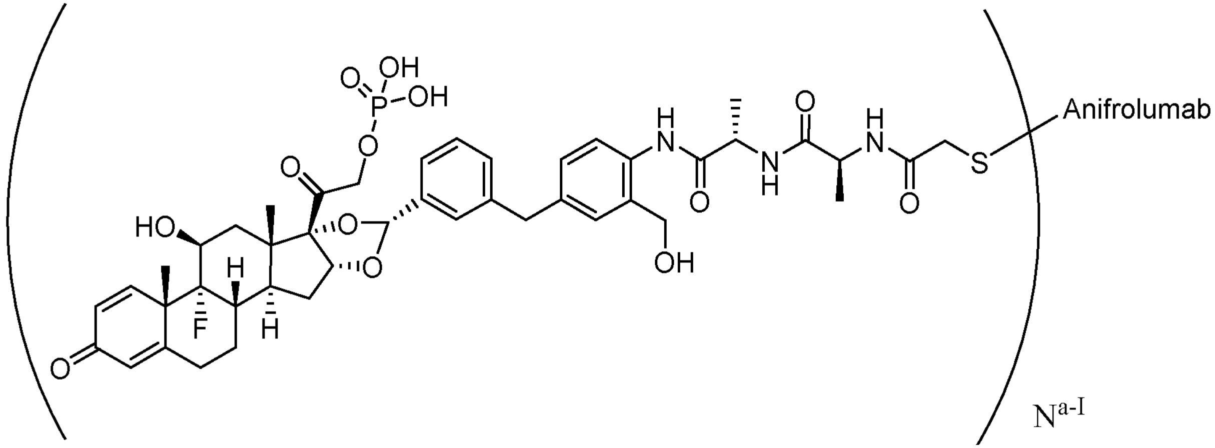 |
| V-371 | 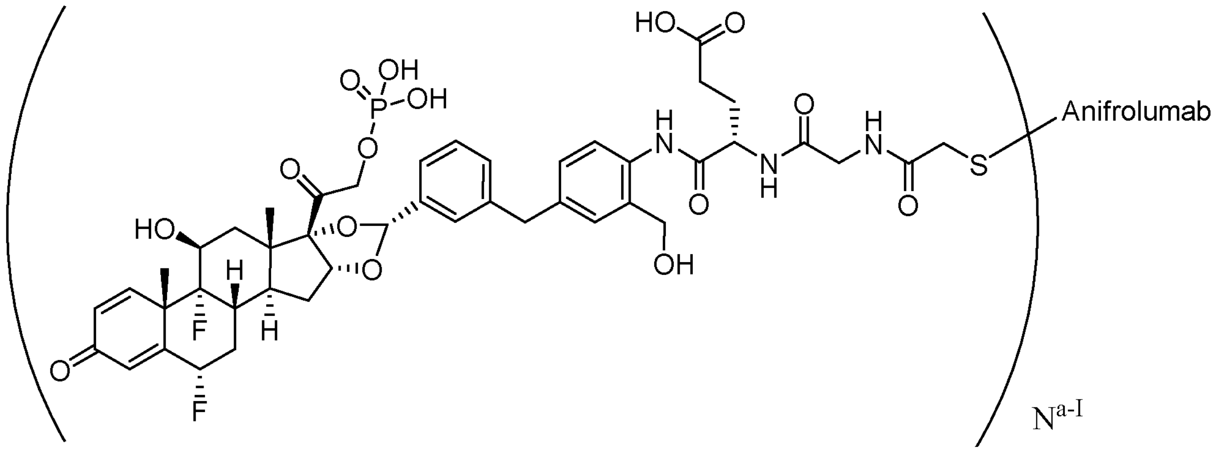 |

(continued)

| No. | Structure |
|---|---|
| V-374 | 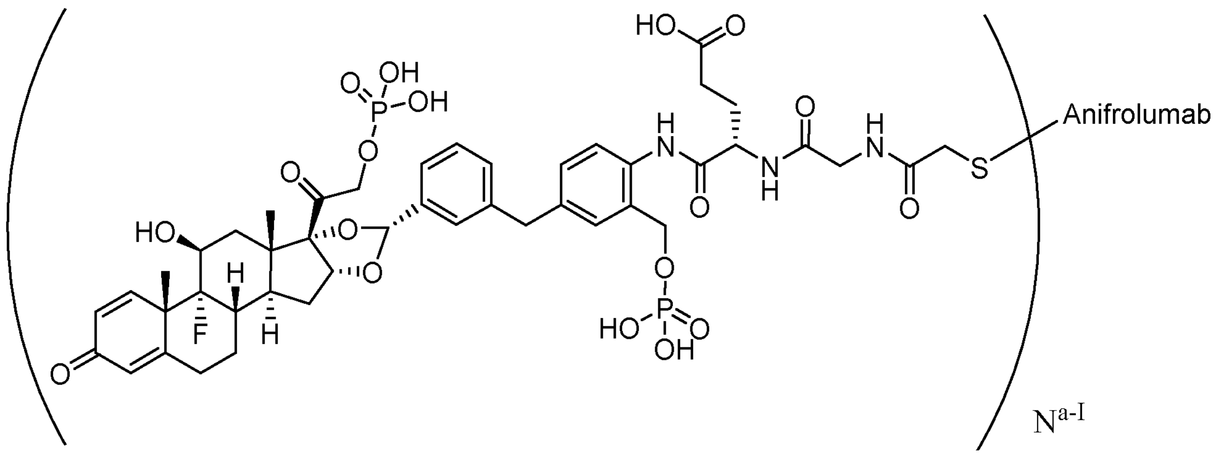 |
| V-378 | 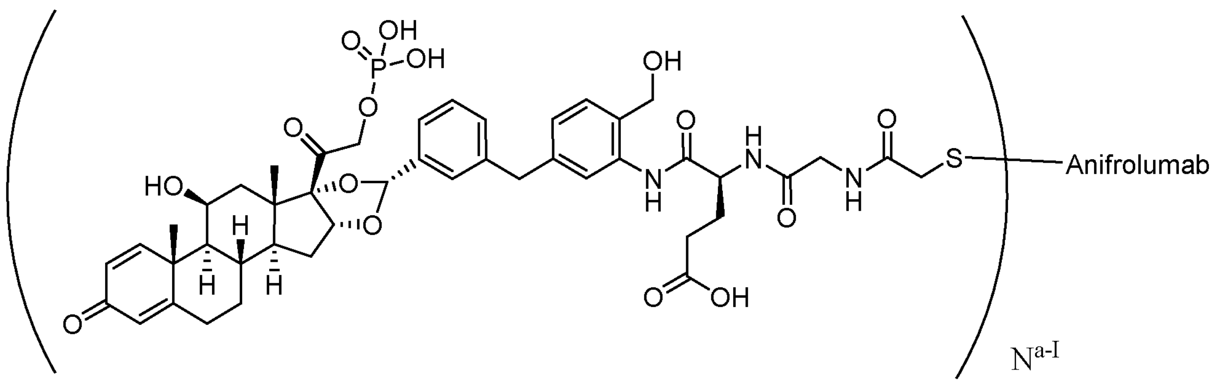 |

(continued)

| No. | Structure |
|---|---|
| V-379 | 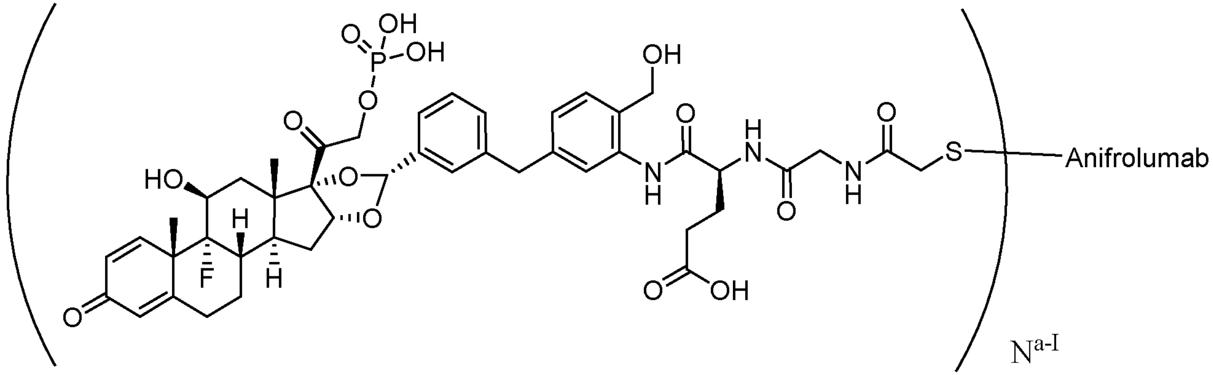 |
| V-380 | 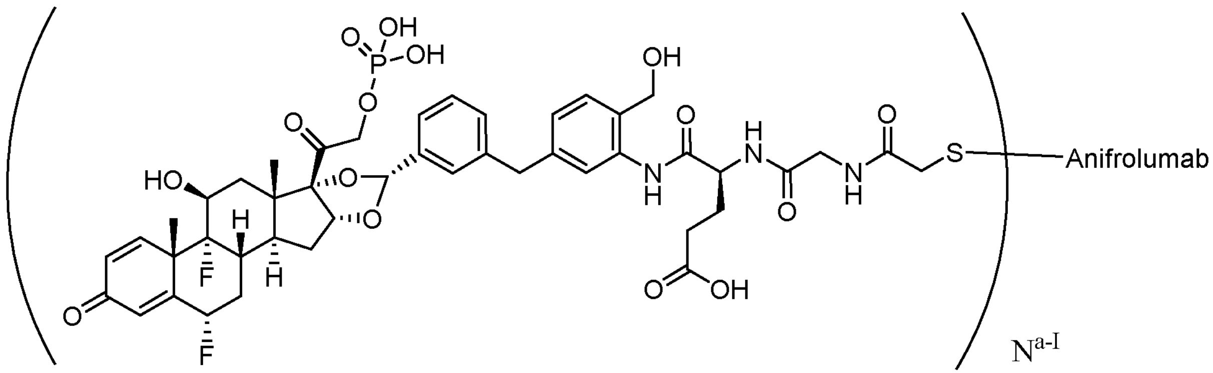 |

(continued)

| No. | Structure |
|---|---|
| V-381 | 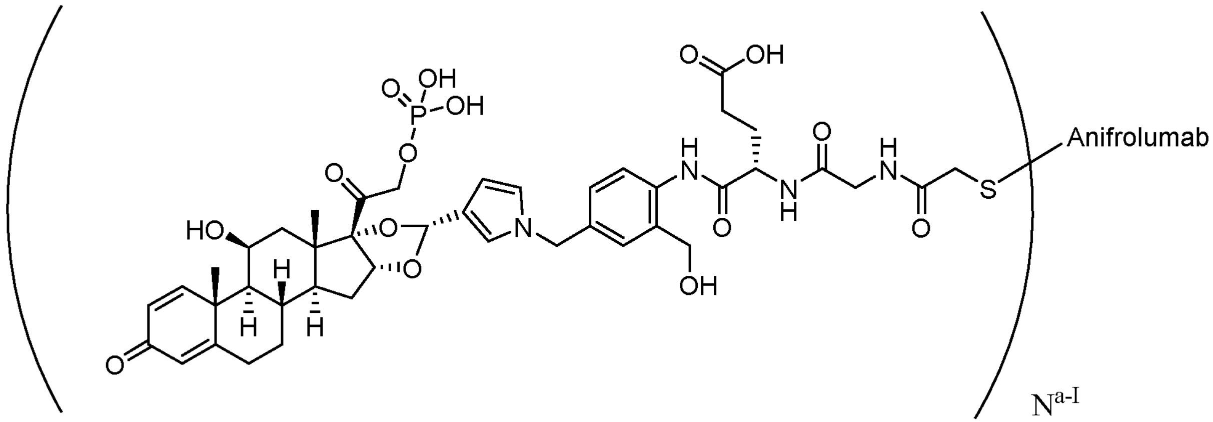 |
| V-382 | 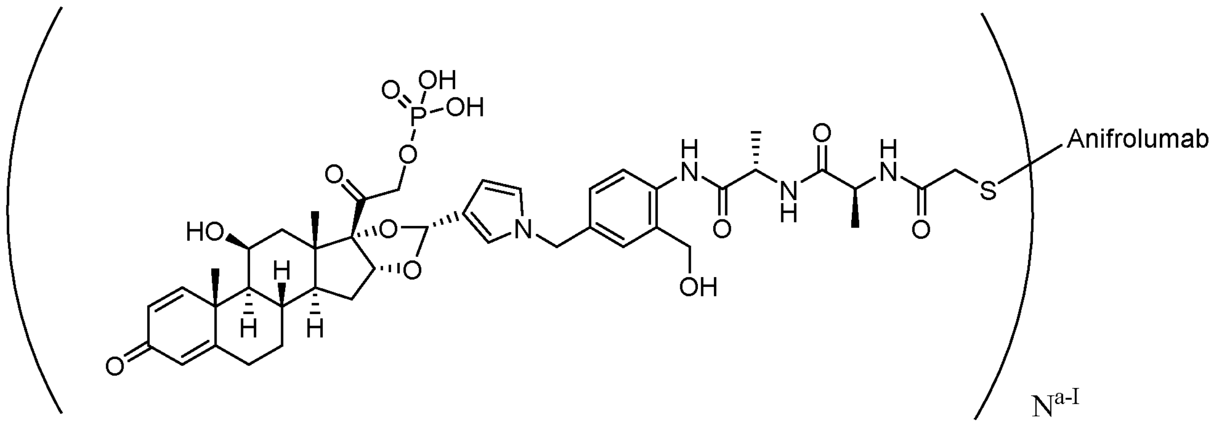 |

(continued)

| No. | Structure |
|---|---|
| V-385 | 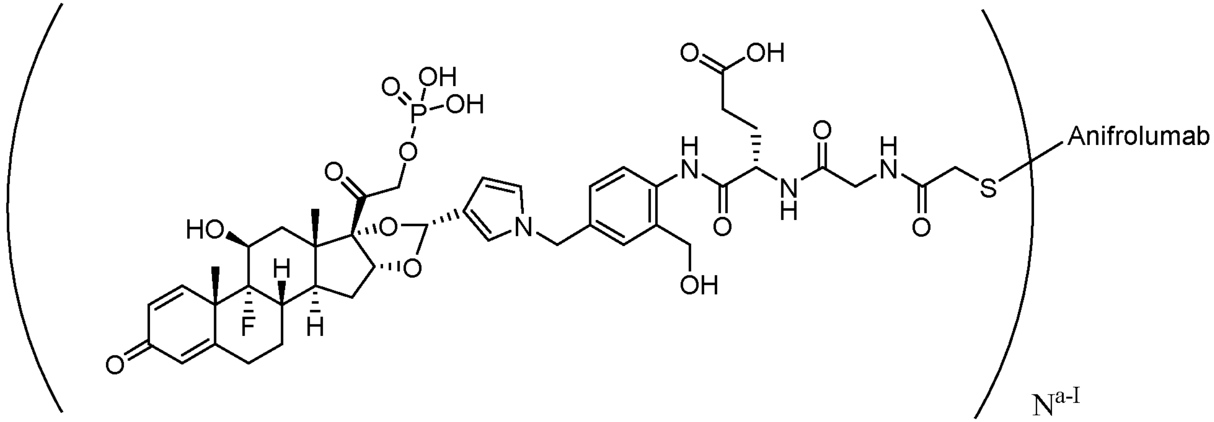 |
| V-386 | 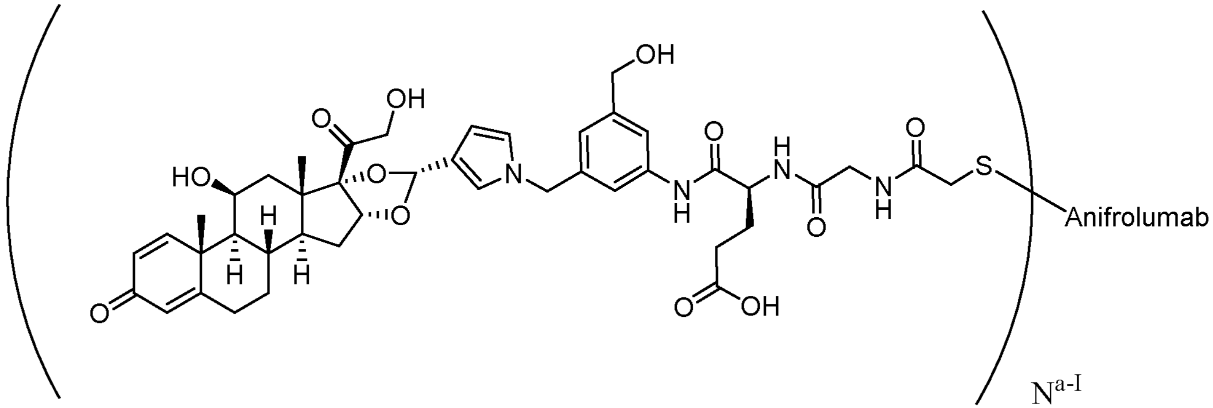 |

(continued)

| No. | Structure |
|---|---|
| V-387 | 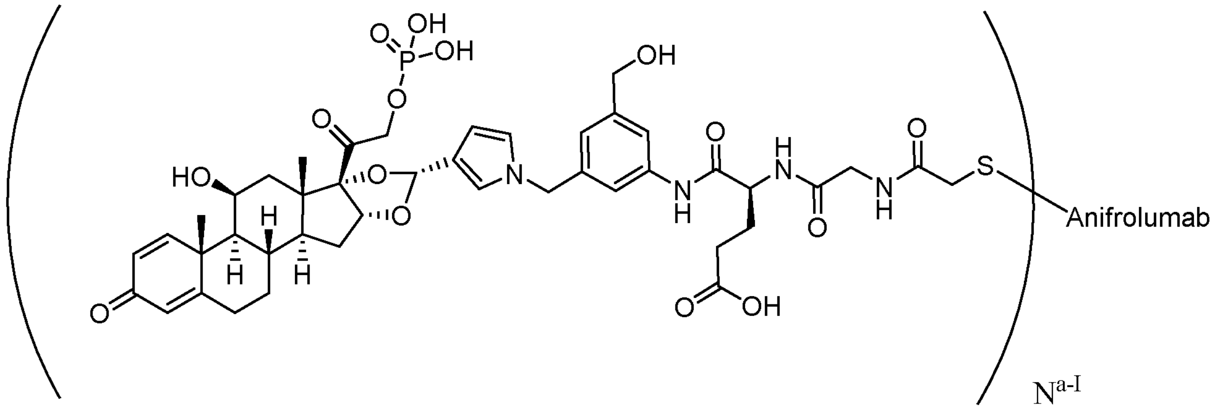 |
| V-388 | 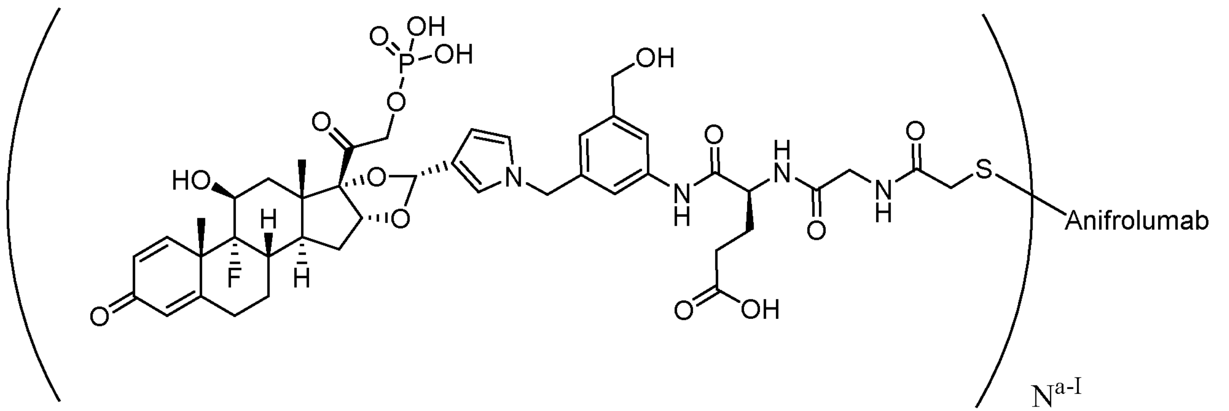 |

(continued)

| No. | Structure |
|---|---|
| V-389 | 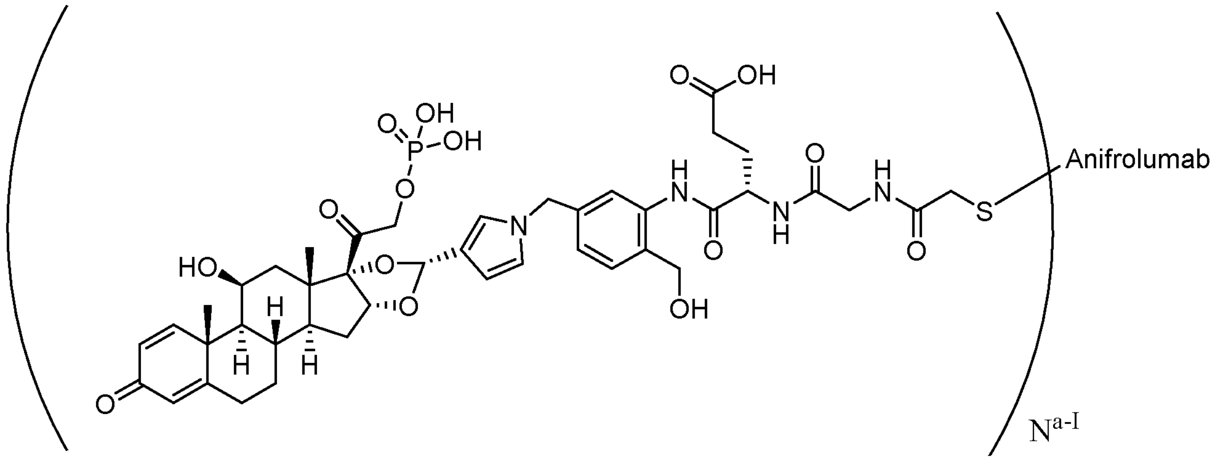 |
| V-390 | 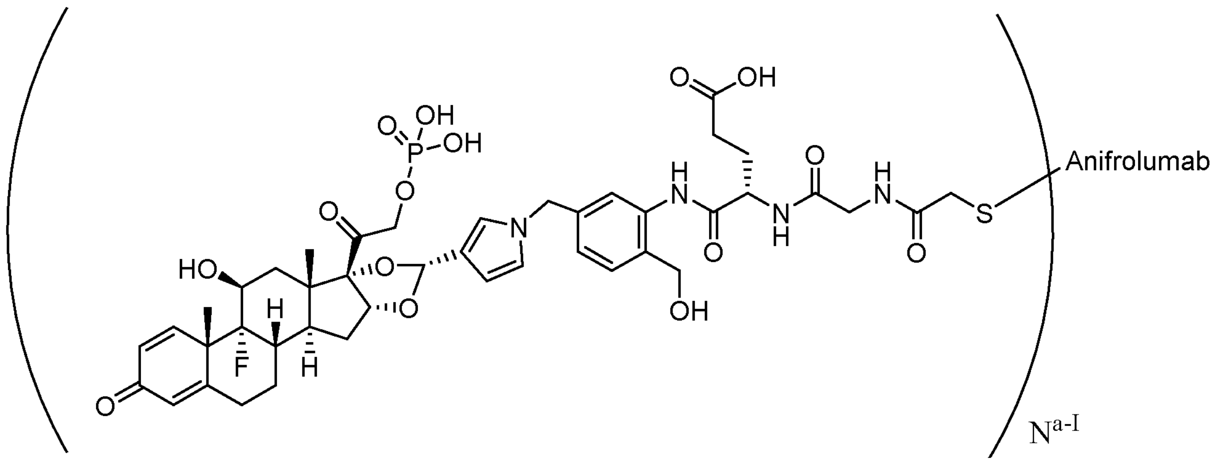 |

(continued)

| No. | Structure |
|---|---|
| V-396 | 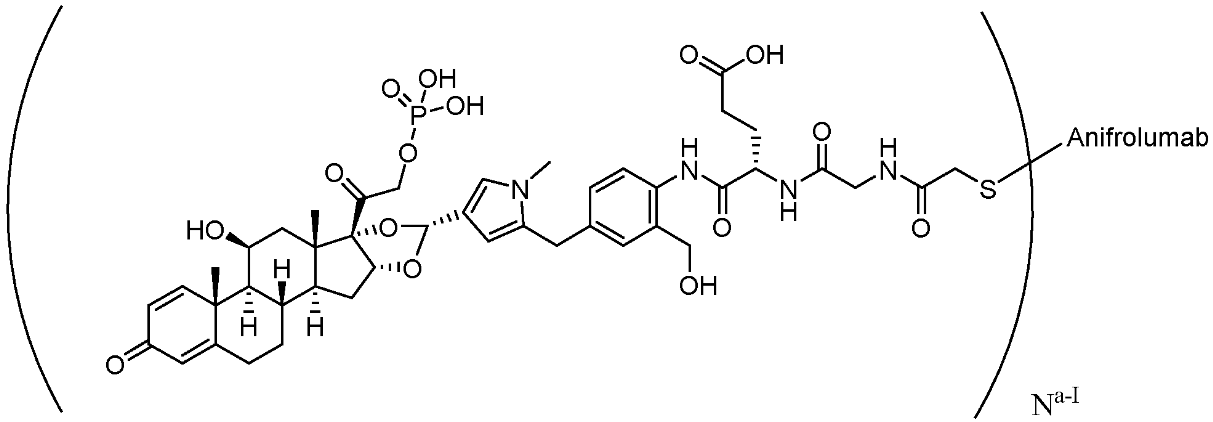 $N^{a-I}$ |
| V-397 | 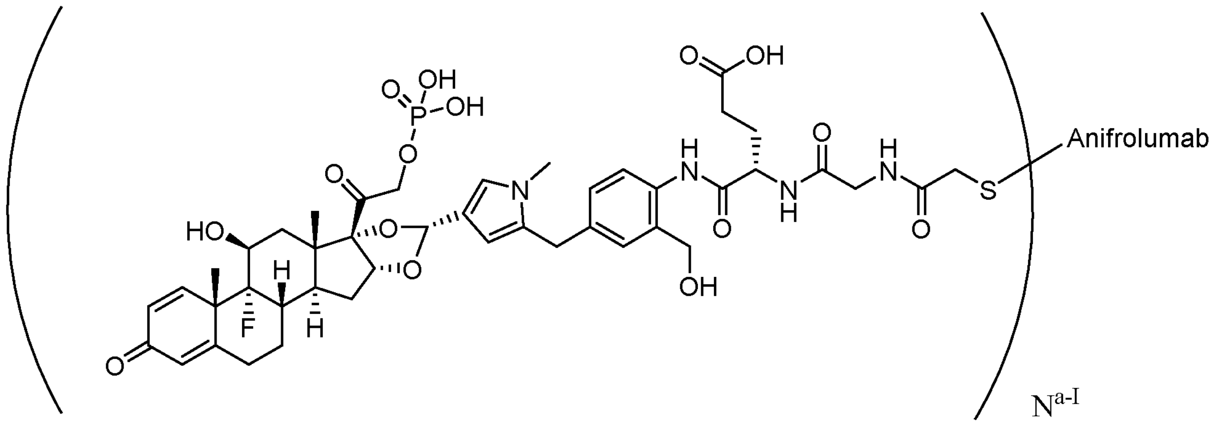 $N^{a-I}$ |

(continued)

| No. | Structure |
| --- | --- |
| V-398 | 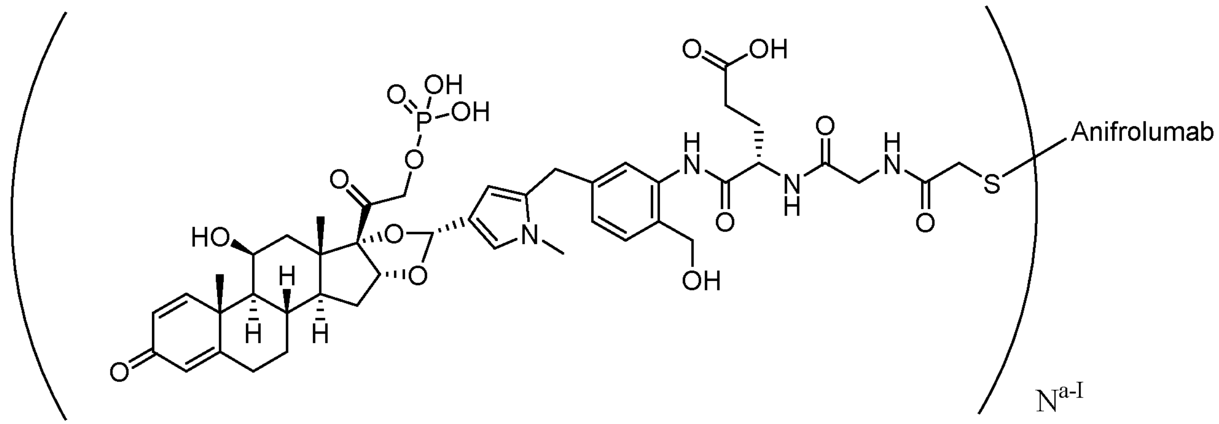 |
| V-399 | 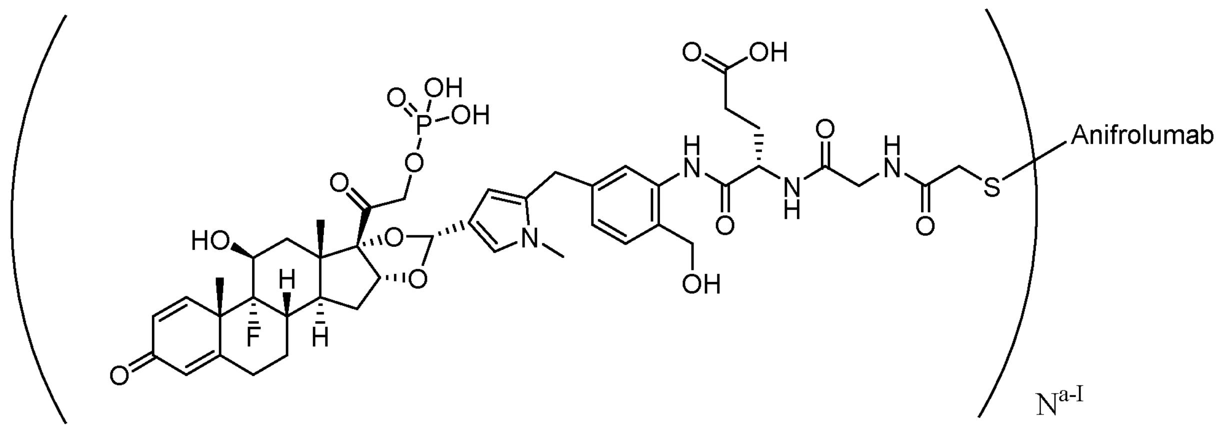 |

(continued)

| No. | Structure |
|---|---|
| V-400 | 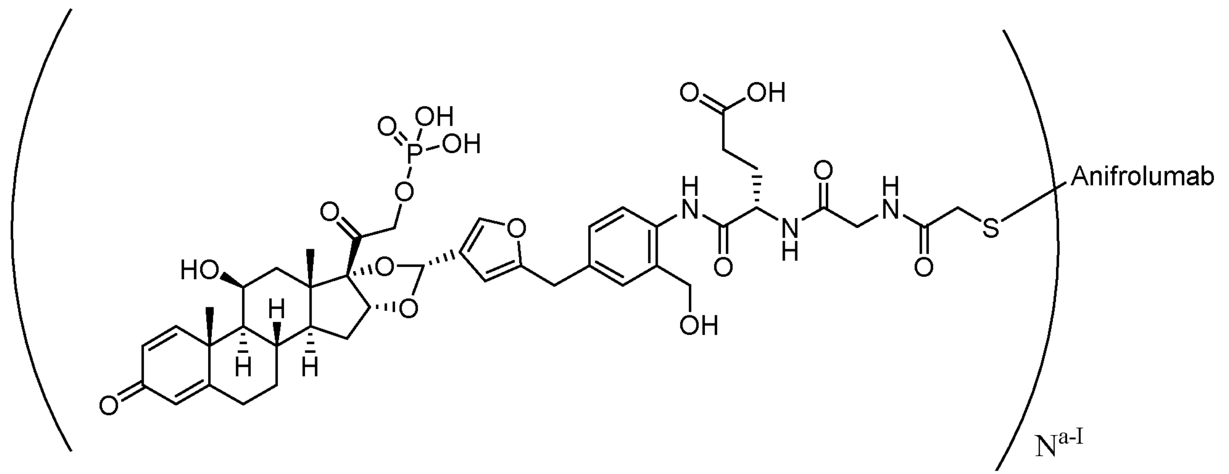 |
| V-401 | 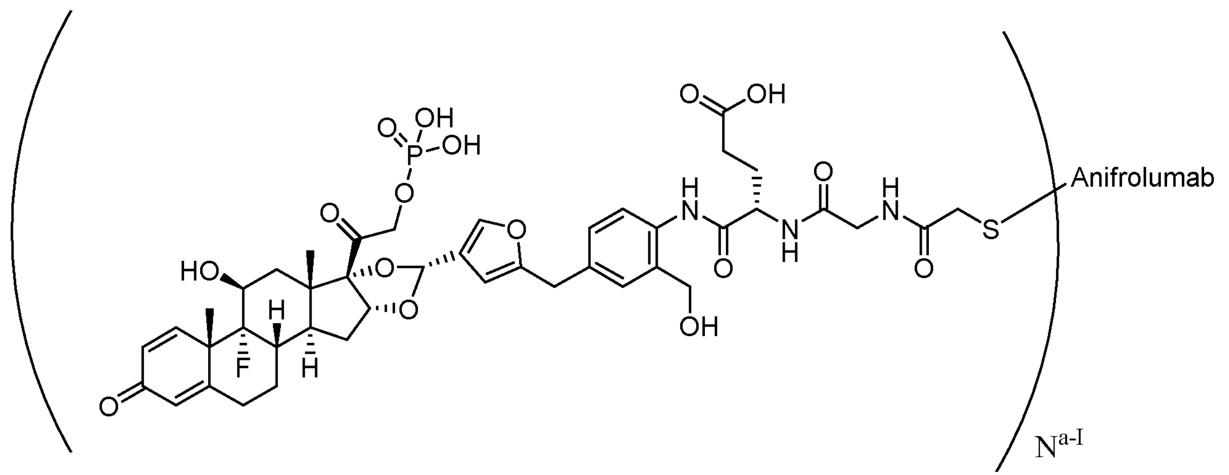 |

(continued)

| No. | Structure |
|---|---|
| V-402 | 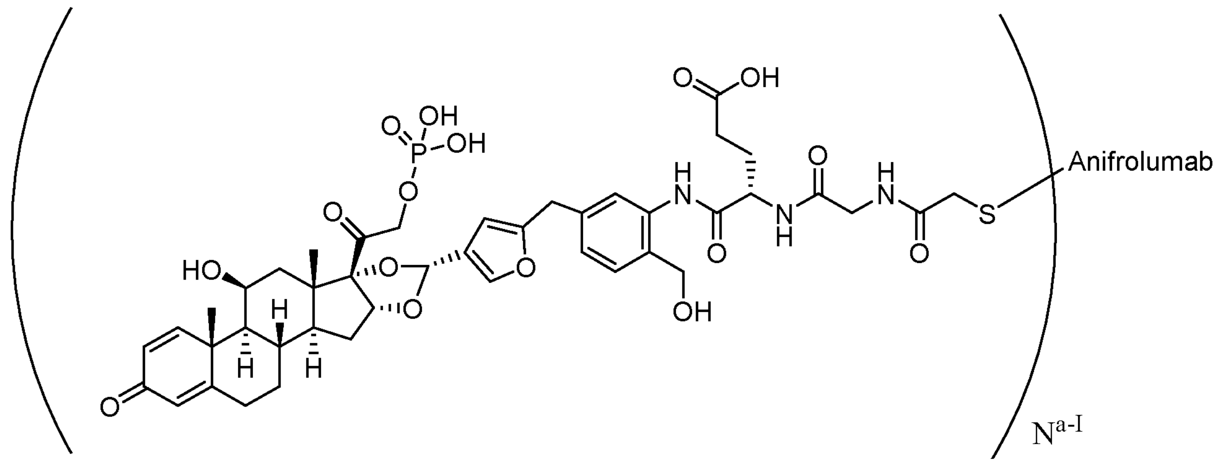 |
| V-403 | 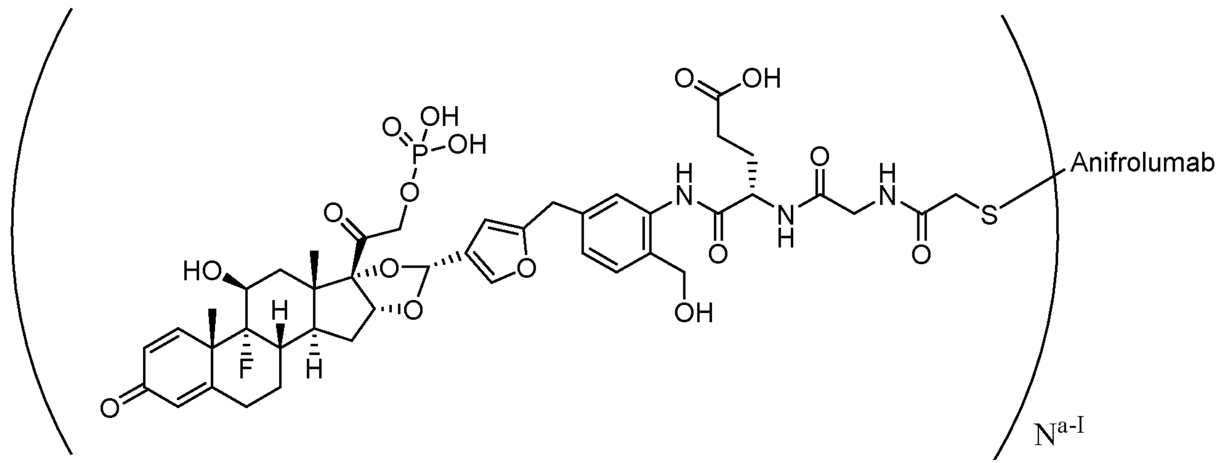 |

(continued)

| No. | Structure |
|---|---|
| V-404 | 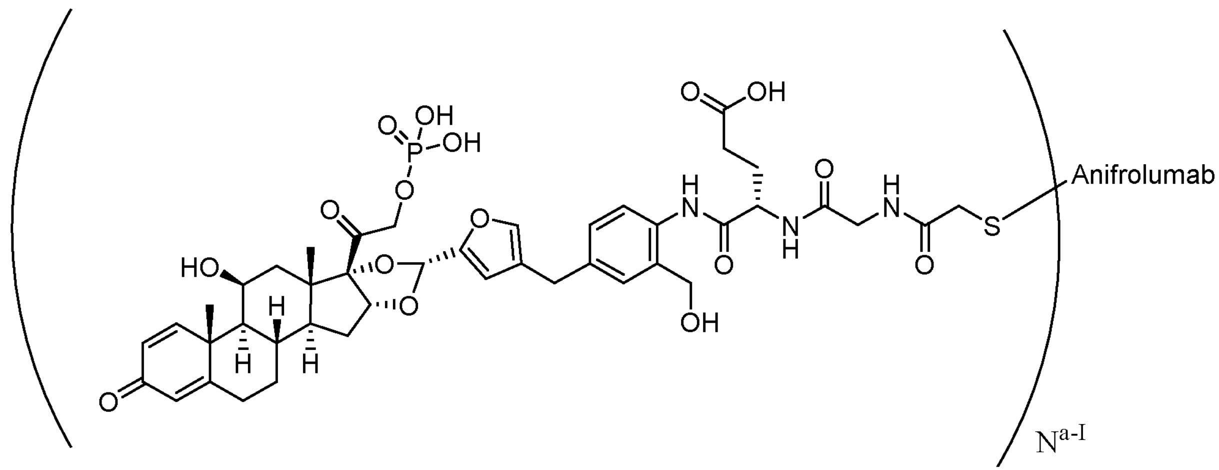 |
| V-405 | 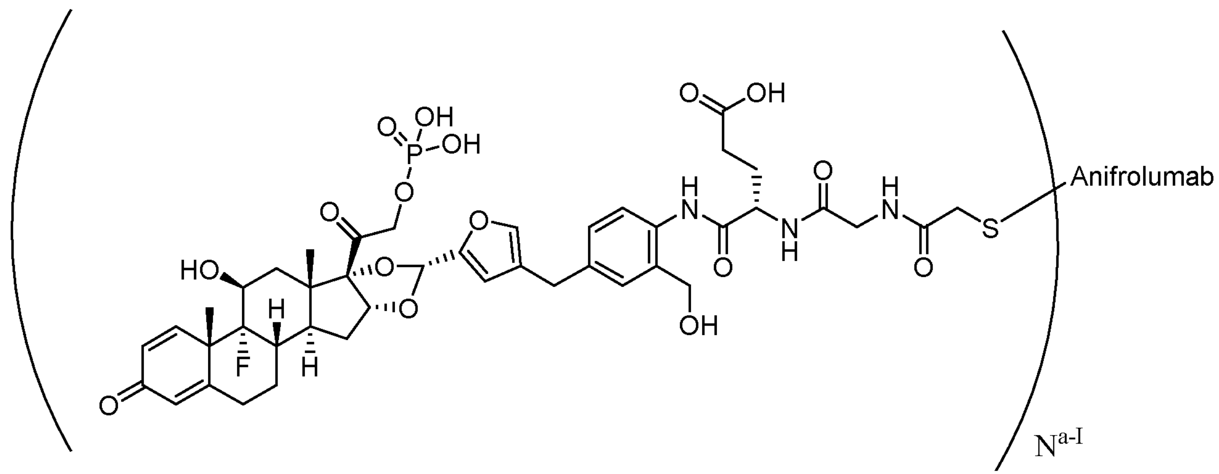 |

(continued)

| No. | Structure |
|---|---|
| V-406 | 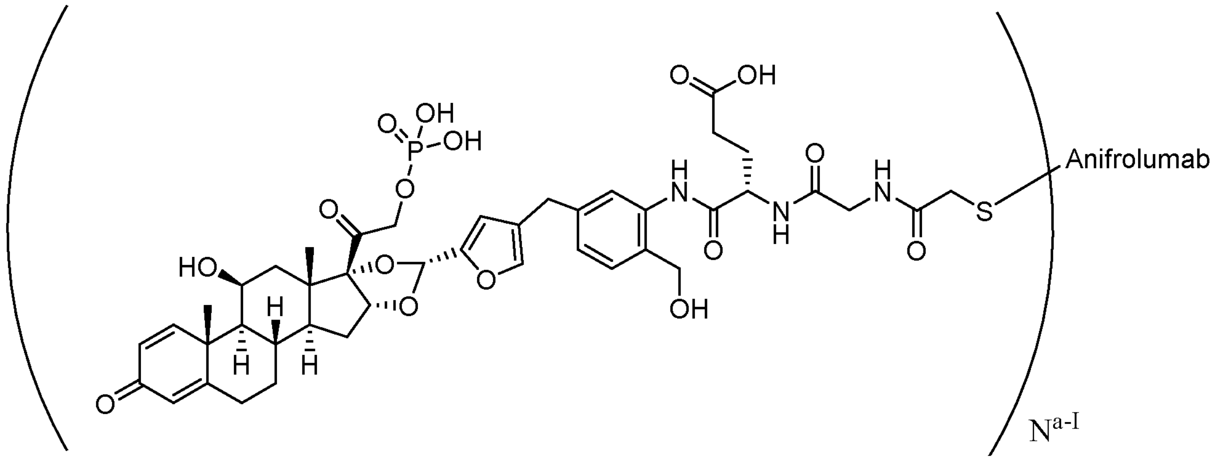 |
| V-407 | 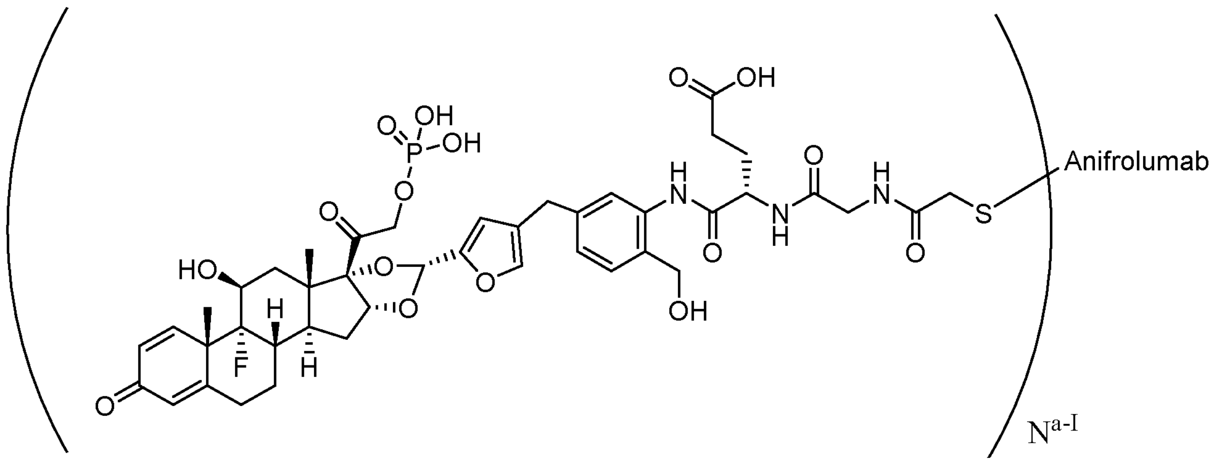 |

(continued)

| No. | Structure |
|---|---|
| V-408 | 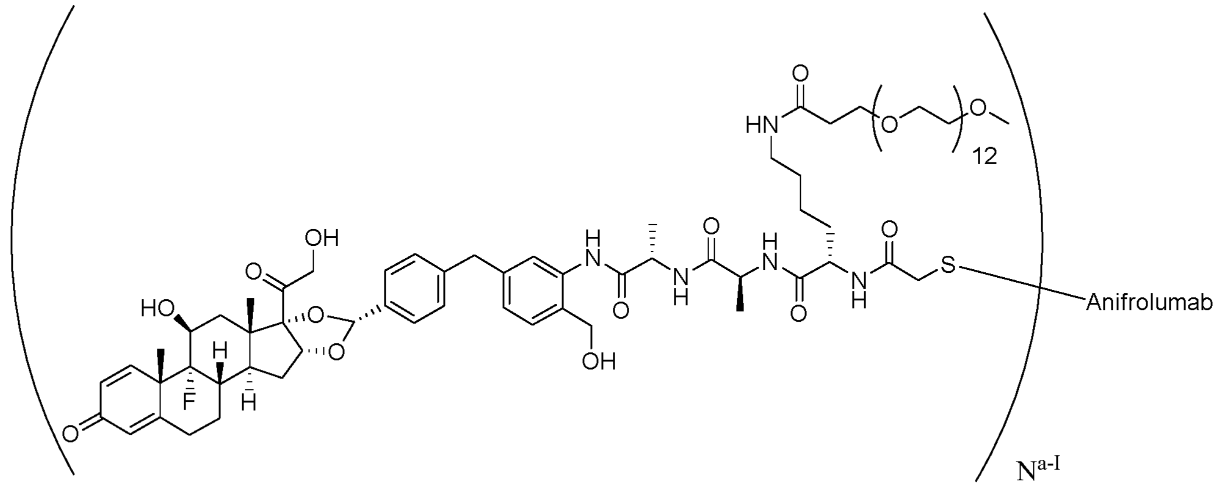 |
| V-409 | 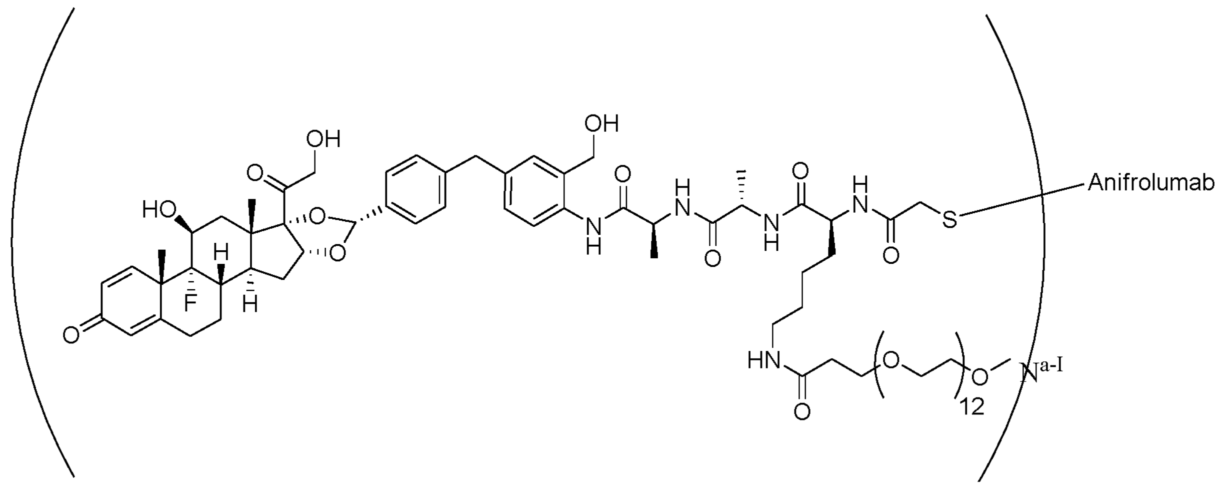 |

(continued)

| No. | Structure |
|---|---|
| V-410 | 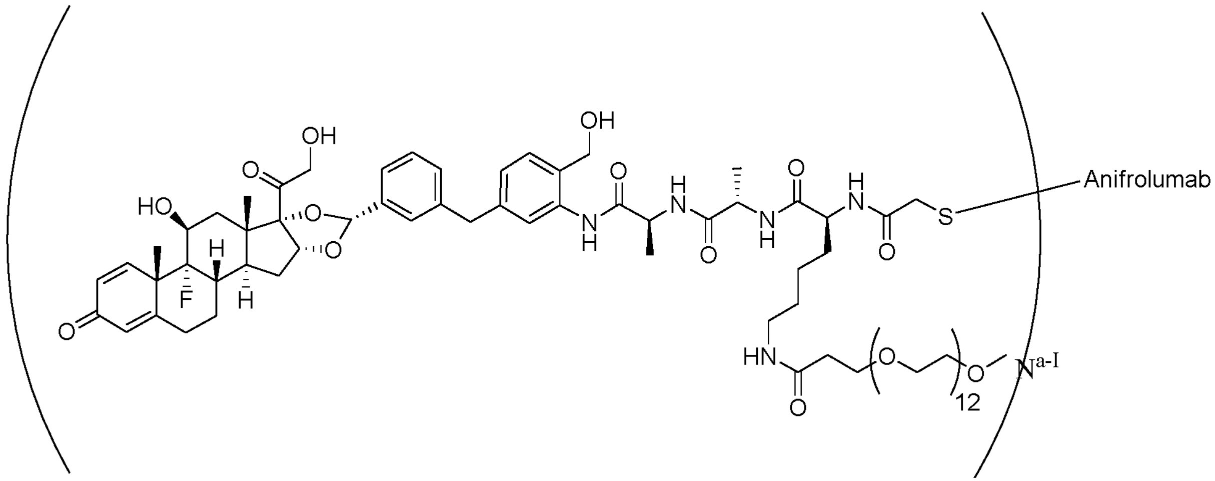 |
| V-411 | 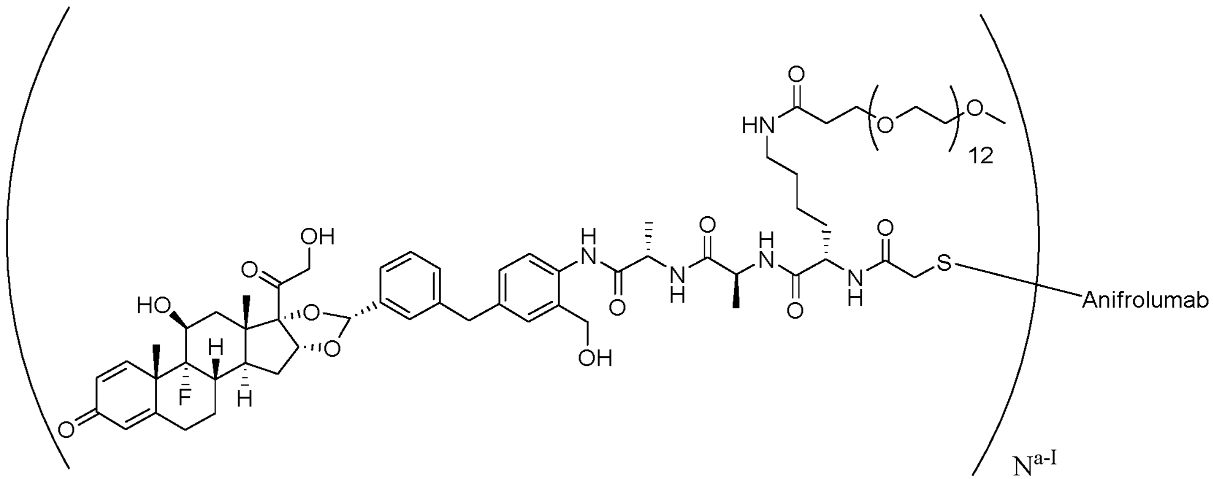 |

(continued)

| No. | Structure |
|---|---|
| V-412 | 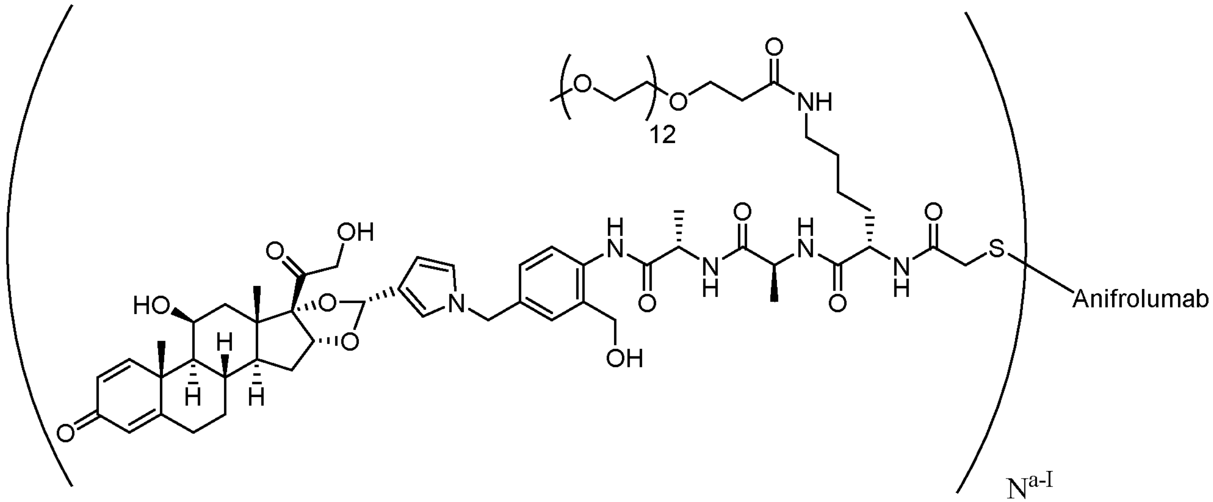 |
| V-413 | 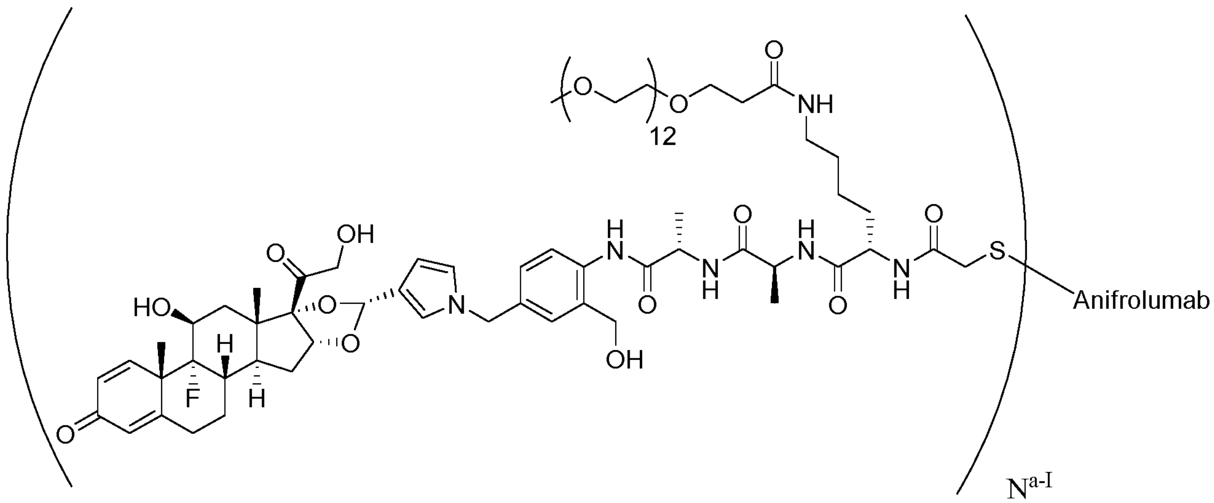 |

(continued)

| No. | Structure |
|---|---|
| V-414 | 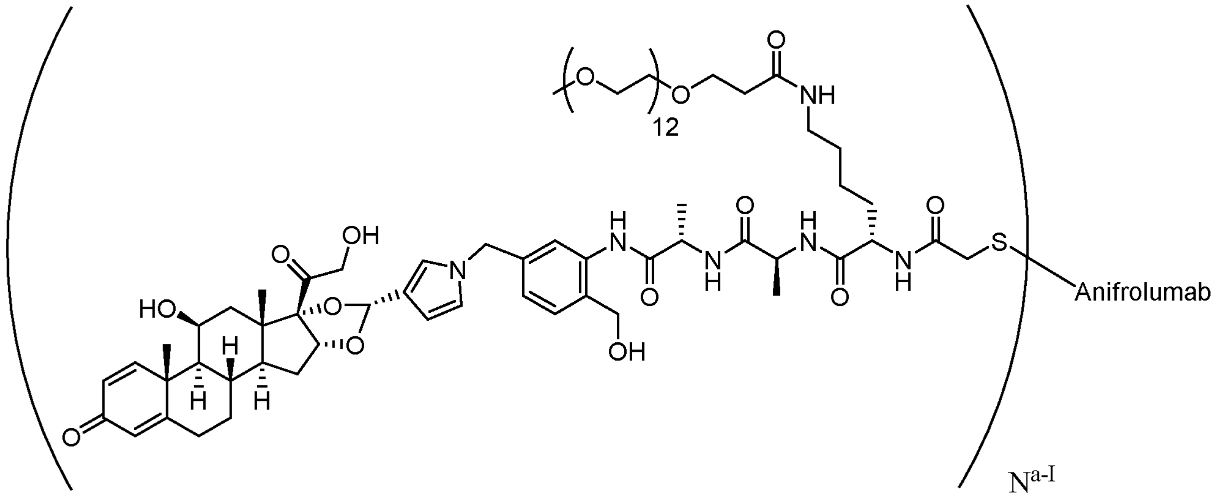 $N^{a-I}$ |
| V-415 | 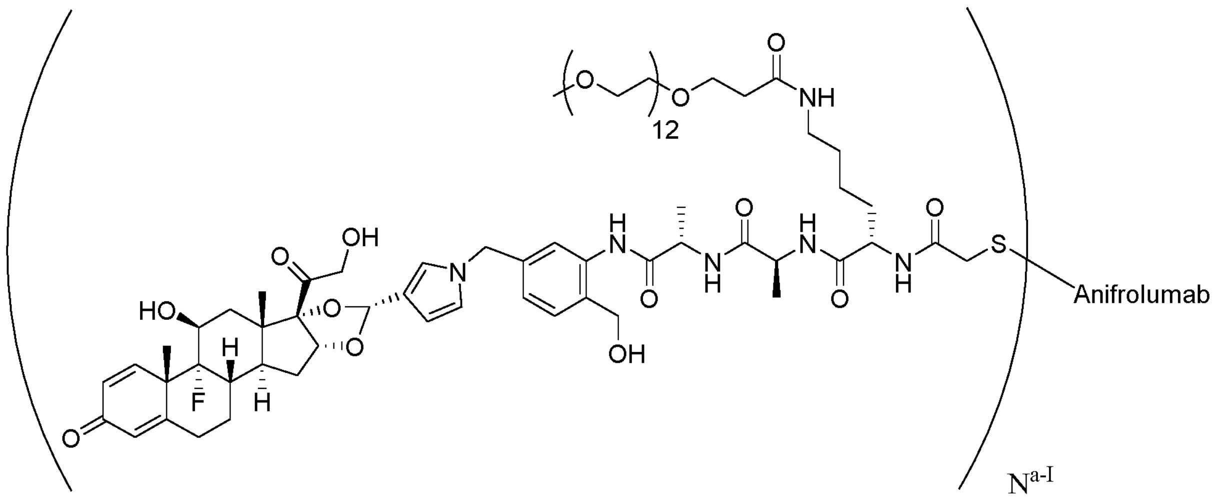 $N^{a-I}$ |

; wherein $N^{a-I}$ is any number from 1 to 10.

**67.** The conjugate according to claims 58-65, wherein the ligand-drug conjugate has the following structure:

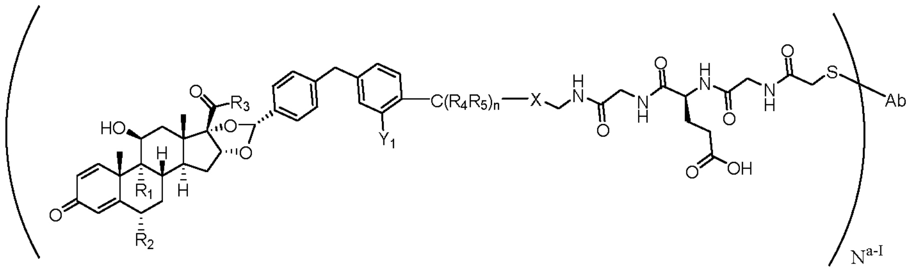

(Va-1)

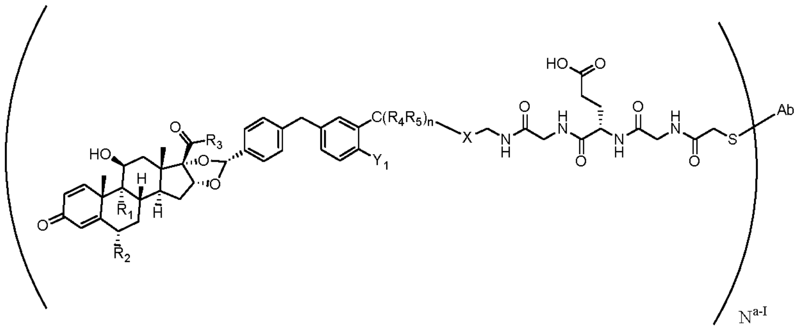

(Vb-1)

wherein,

Ab represents a ligand capable of binding to a target, and $N^{a-I}$ is any number from 1 to 10;
X is selected from the group consisting of -O-, -S- and -NH-;
$R_4$ and $R_5$ are each independently selected from the group consisting of: H, F, Cl, -OH, $-NH_2$ and $C_1$-$C_6$ alkyl;
n is selected from the group consisting of 1, 2 and 3;
$Y_1$ is absent or selected from the group consisting of: hydroxy, sulfhydryl, amino and $C_1$-$C_6$ alkyl;
$R_1$ and $R_2$ are each independently selected from the group consisting of hydrogen, fluorine, chlorine and methyl;
$R_3$ is selected from the group consisting of: $-CH_2Cl$, $-CH_2SH$, $-CH_2OH$,

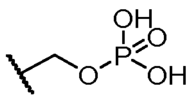,

$-OCH_3$, - $OCH_2F$, $-OCH_2Cl$, $-OCH_2CN$, $-OCH_2CH_3$, -SH, $-SCH_2F$, $-SCH_2Cl$, $-SCH_2CF_3$ and $-SCH_2CN$.

**68.** The conjugate according to claims 58-65, wherein the conjugate is selected from the group consisting of the following structures:

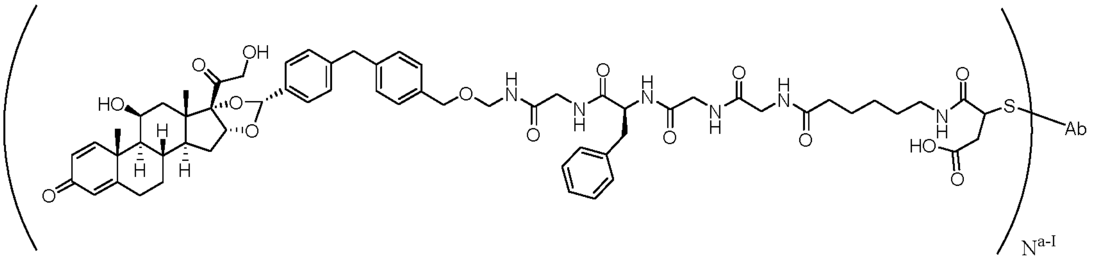

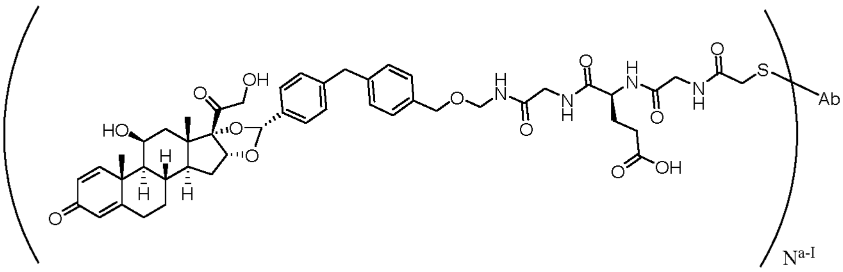

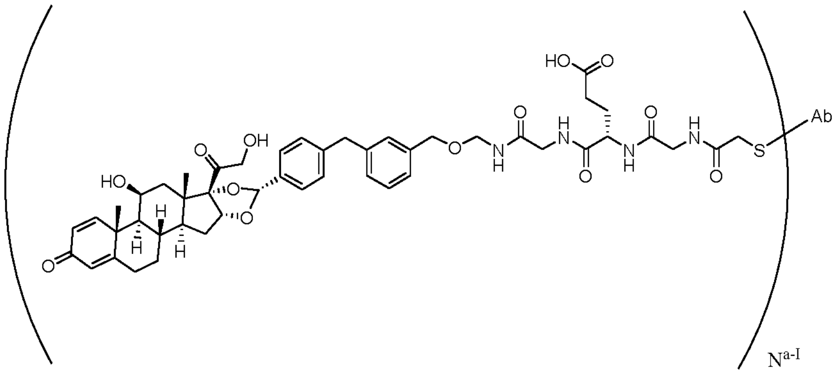

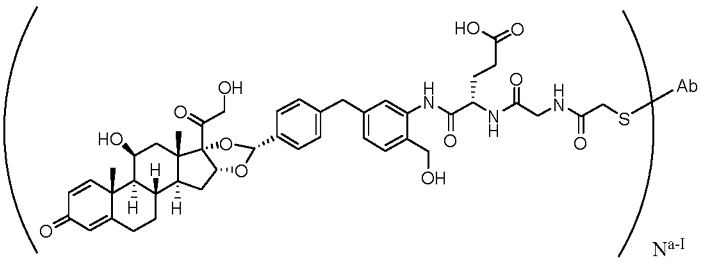

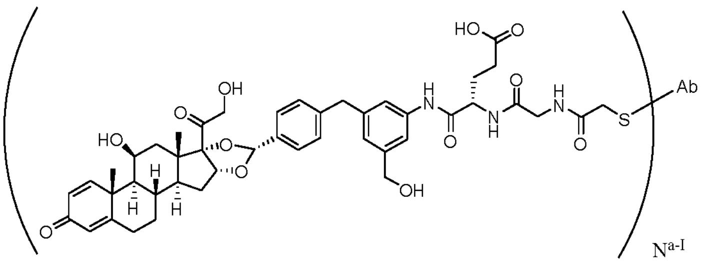

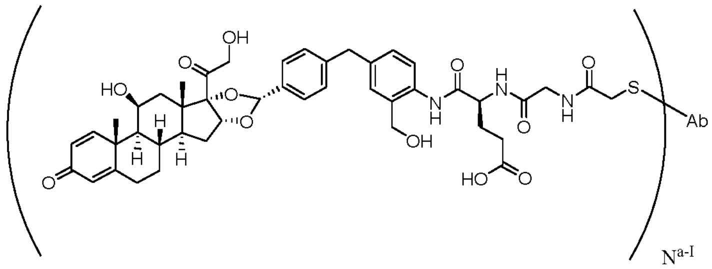

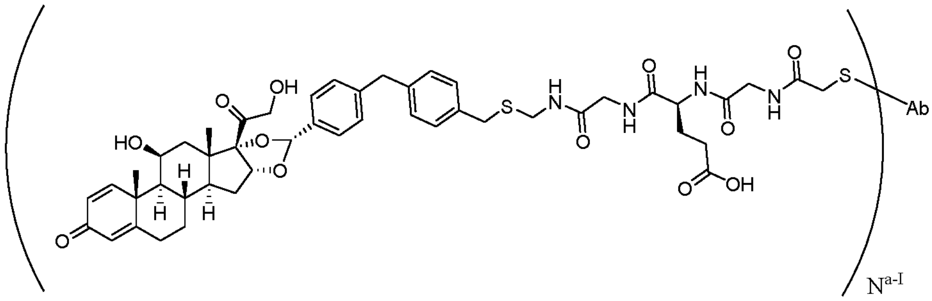

; wherein N^a-I is any number from 1 to 10, and Ab is selected from an antibody or an antigen-binding fragment thereof.

**69.** A ligand-drug conjugate of a formula below or a tautomer, an enantiomer or a diastereoisomer thereof, or a mixture of isomers thereof, or a pharmaceutically acceptable salt or a solvate thereof, wherein the ligand-drug conjugate is selected from the group consisting of the following structures:

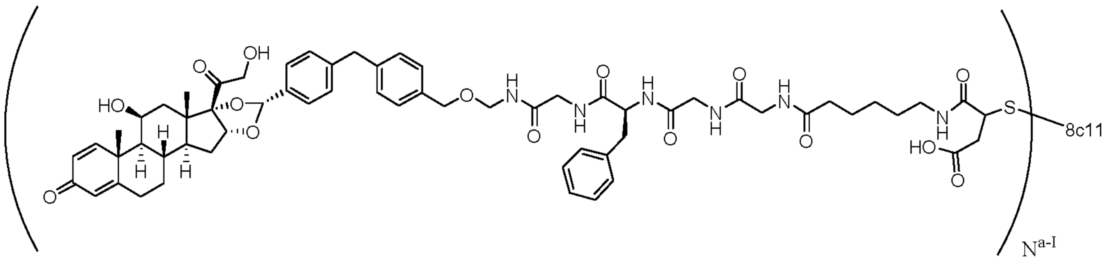

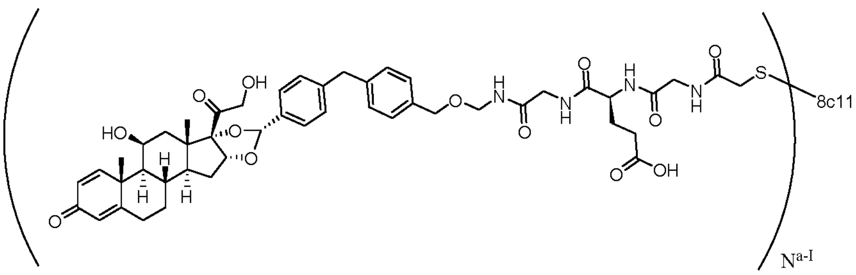

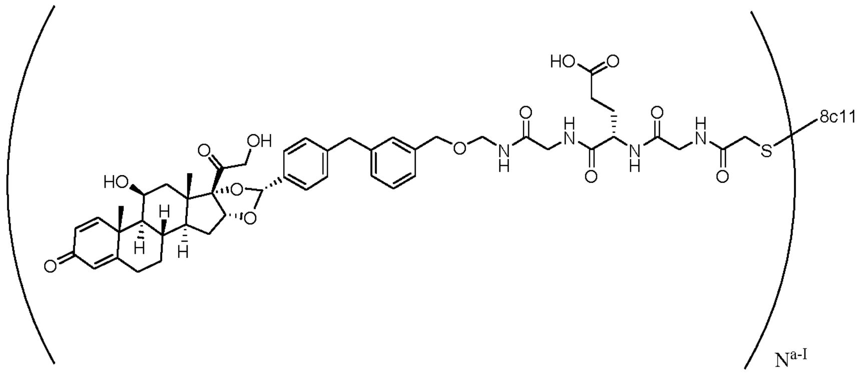

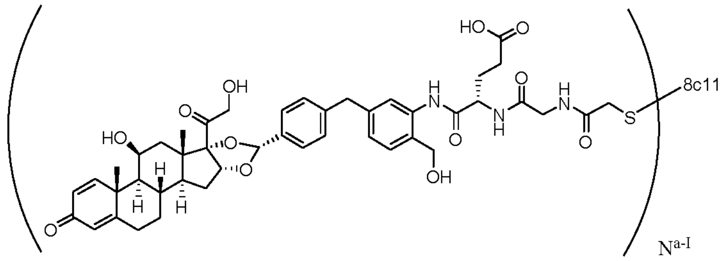

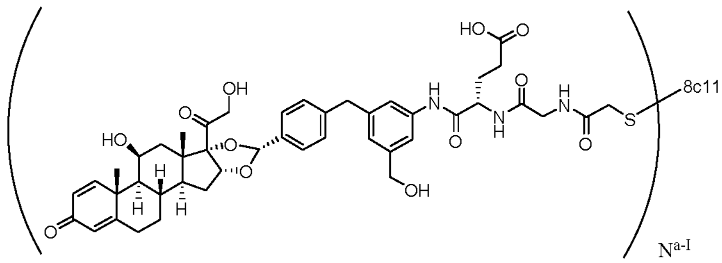

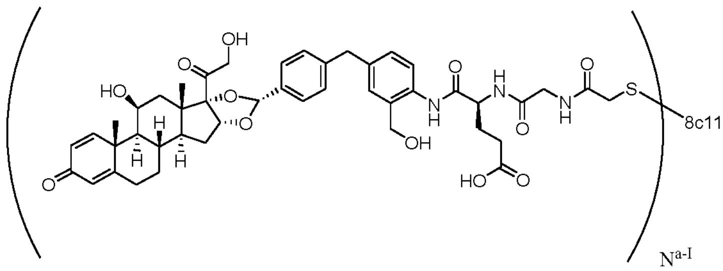

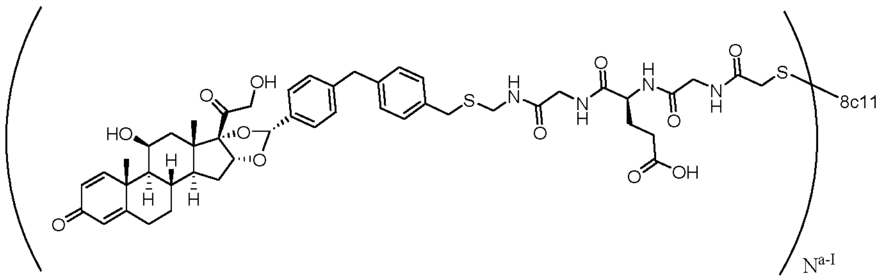

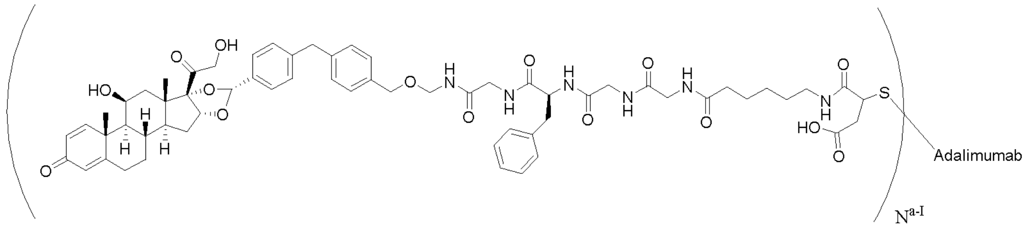

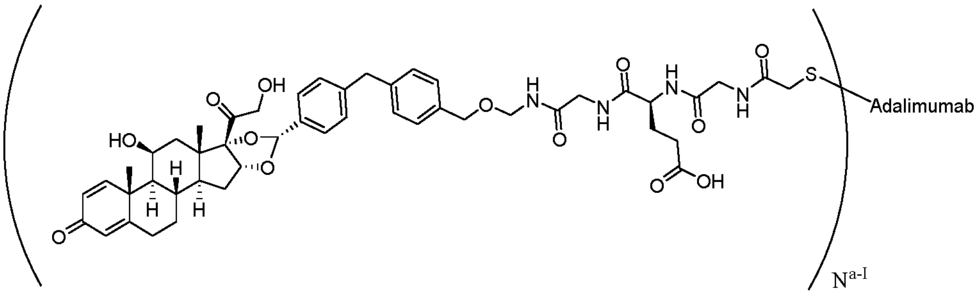

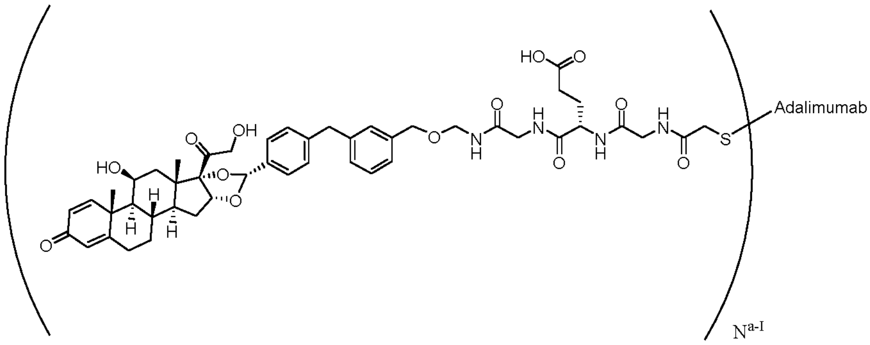

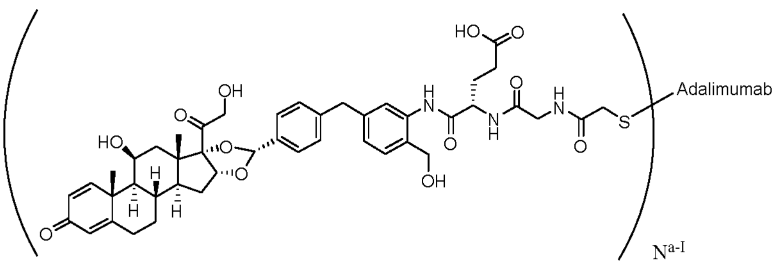

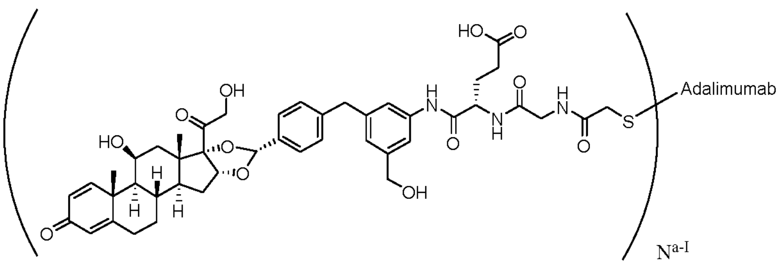

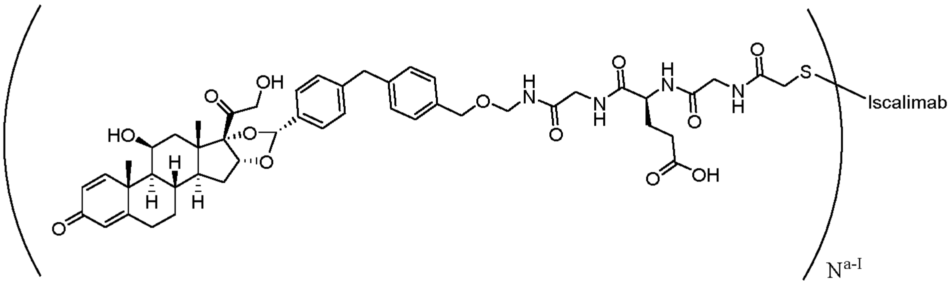

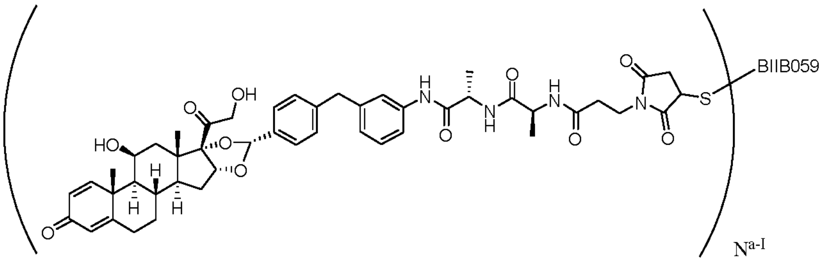

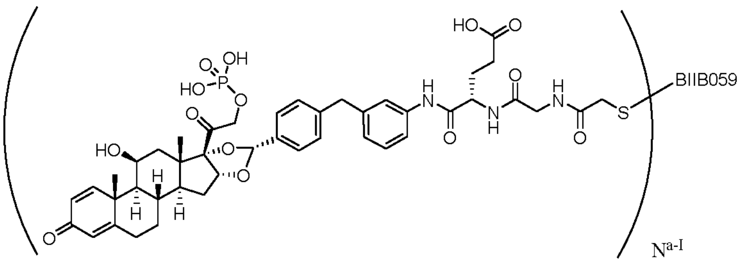

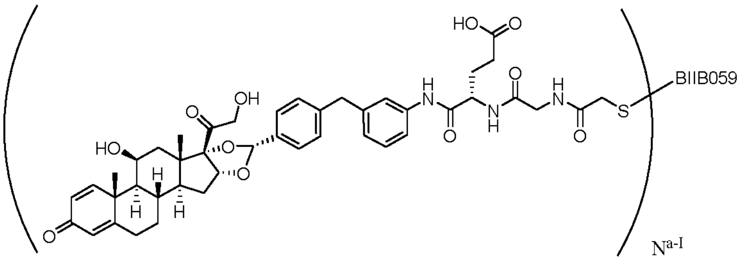

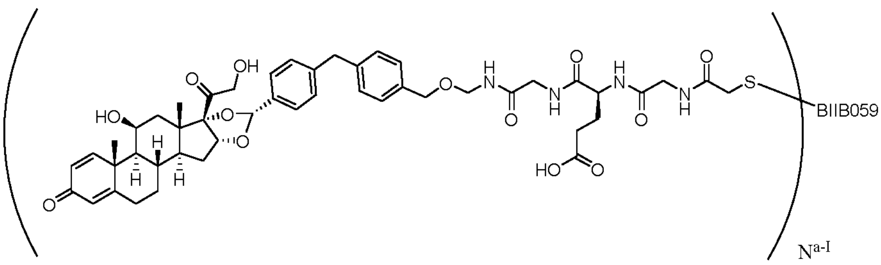

;

wherein $N^{a-I}$ is any number from 1 to 10.

**70.** A pharmaceutical composition, comprising the compound or the tautomer, the mesomer, the racemate, the enantiomer or the diastereoisomer thereof, or the mixture thereof, or the pharmaceutically acceptable salt thereof according to any one of claims 1-57 and/or the conjugate according to any one of claims 58-68, and optionally a pharmaceutically acceptable carrier.

**71.** A method for influencing immune system functions, comprising administering to a subject the compound or the tautomer, the mesomer, the racemate, the enantiomer or the diastereoisomer thereof, or the mixture thereof, or the pharmaceutically acceptable salt thereof according to any one of claims 1-57, the conjugate according to any one of claims 58-68, and/or the pharmaceutical composition according to claim 69.

**72.** Use of the compound or the tautomer, the mesomer, the racemate, the enantiomer or the diastereoisomer thereof, or the mixture thereof, or the pharmaceutically acceptable salt thereof according to any one of claims 1-57, the conjugate according to any one of claims 58-68 and/or the pharmaceutical composition according to claim 69 in the preparation of a medicament for preventing and/or treating diseases and/or conditions, wherein the diseases and/or the conditions include diseases and/or conditions associated with glucocorticoid receptor signaling.

**73.** The use according to claim 72, wherein the diseases and/or the conditions are selected from the group consisting of: rheumatoid arthritis, systemic lupus erythematosus, scleroderma, Sjogren's syndrome, ankylosing spondylitis, Wegener's granulomatosis and systemic sclerosis, autoimmune hemolytic anemia, pernicious anemia, idiopathic thrombocytopenic purpura, idiopathic thrombocytopenia and vasculitis, multiple sclerosis, myasthenia gravis and Guillain-Barre syndrome, ulcerative colitis, Crohn's disease, autoimmune diseases and atrophic gastritis, IgA nephropathy, primary nephrotic syndrome, autoimmune glomerulonephritis, Goodpasture's syndrome and lupus nephritis, type I diabetes, Grave's disease, Hashimoto's thyroiditis, primary adrenocortical atrophy and chronic thyroiditis, psoriasis, pemphigus vulgaris, cutaneous lupus erythematosus, dermatomyositis and polymyalgia rheumatica, and asthma.

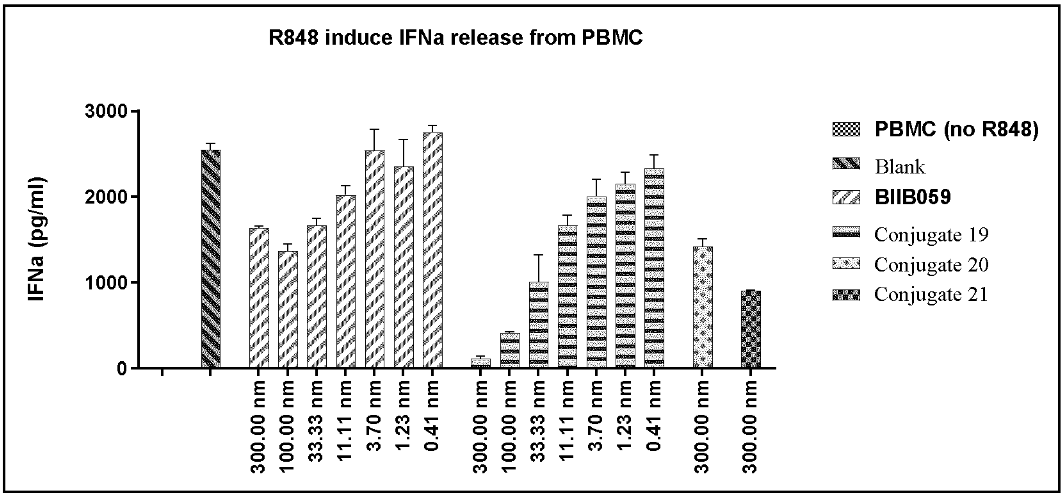


FIG. 1a

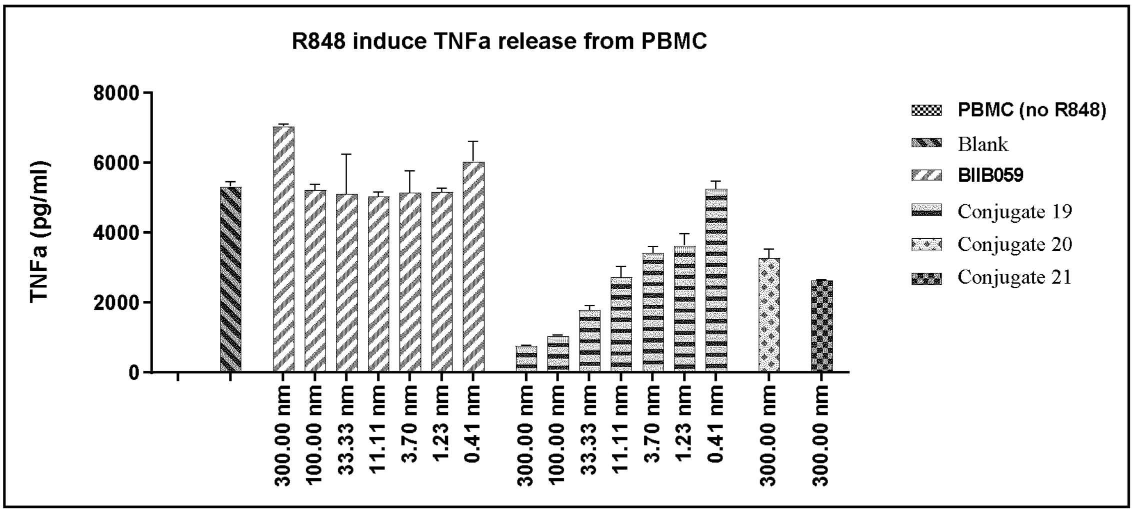


FIG. 1b

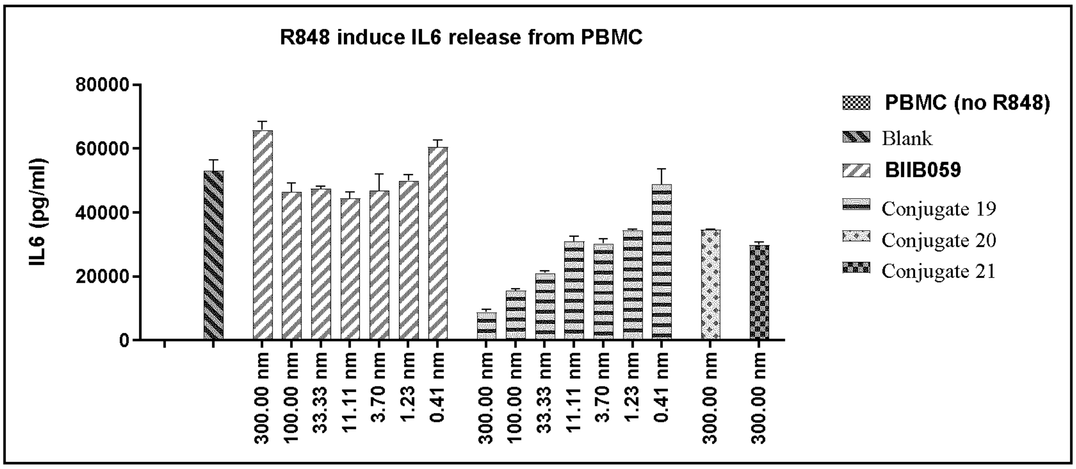


FIG. 1c

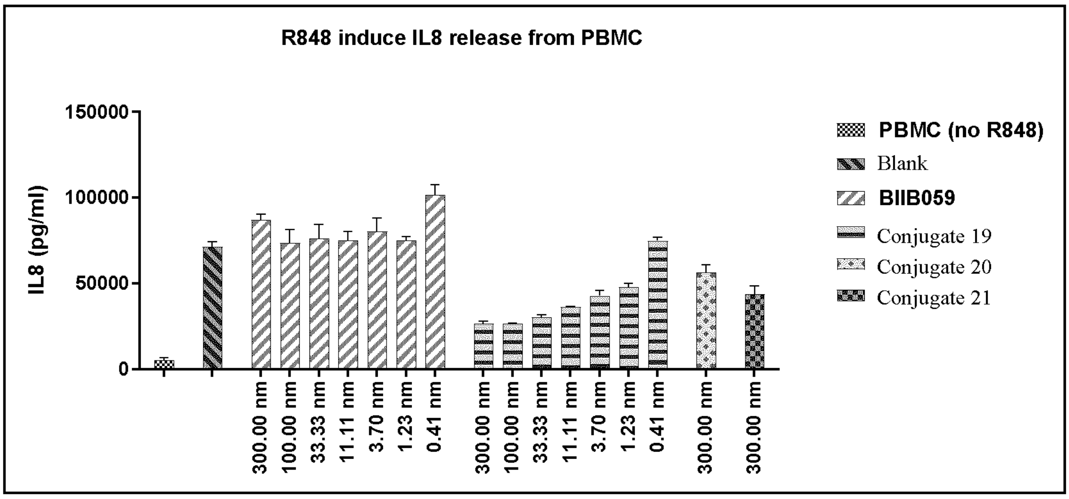


FIG. 1d

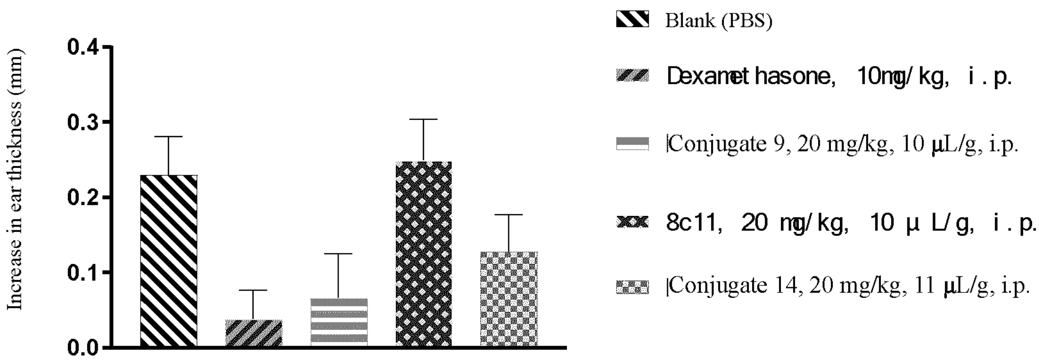


FIG. 2

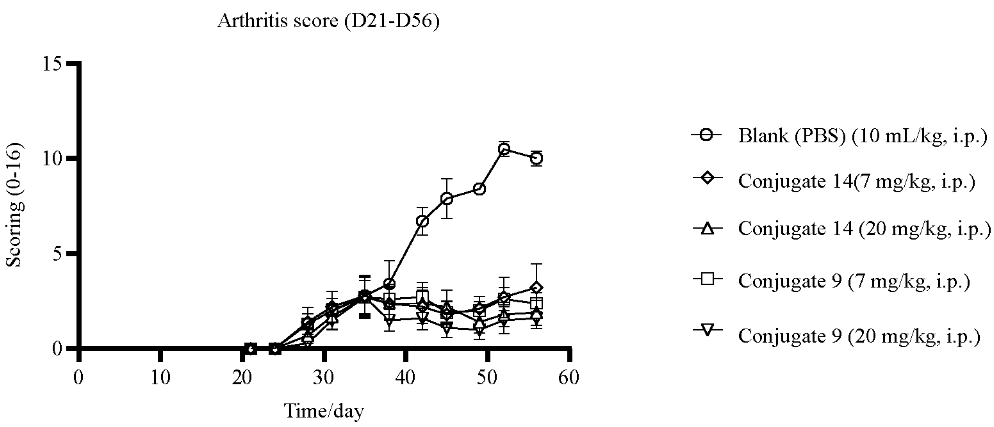


FIG. 3a

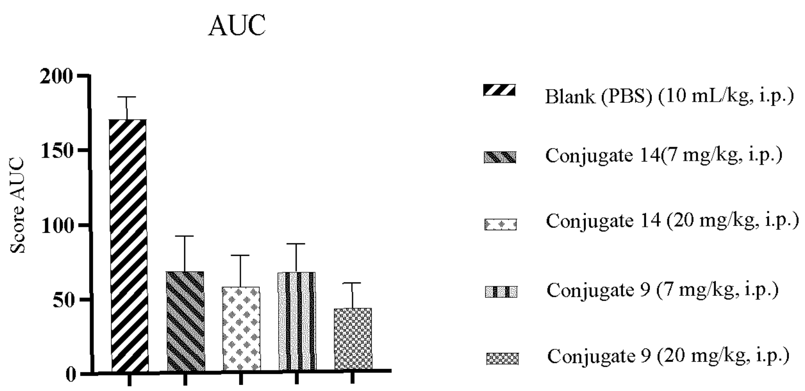
AUC
Score AUC
200
150
100
50
0
Blank (PBS) (10 mL/kg, i.p.)
Conjugate 14(7 mg/kg, i.p.)
Conjugate 14 (20 mg/kg, i.p.)
Conjugate 9 (7 mg/kg, i.p.)
Conjugate 9 (20 mg/kg, i.p.)

FIG. 3b

## INTERNATIONAL SEARCH REPORT

International application No.
**PCT/CN2022/114855**

**A. CLASSIFICATION OF SUBJECT MATTER**

C07J 71/00(2006.01)i; C07K 16/28(2006.01)i; A61K 31/585(2006.01)i; A61K 47/68(2017.01)i; A61P 37/00(2006.01)i

According to International Patent Classification (IPC) or to both national classification and IPC

**B. FIELDS SEARCHED**

Minimum documentation searched (classification system followed by classification symbols)

C07J71/-; C07K16/-; A61K31/-; A61K47/-; A61P37/-

Documentation searched other than minimum documentation to the extent that such documents are included in the fields searched

Electronic data base consulted during the international search (name of data base and, where practicable, search terms used)

WPI, EPODOC, CNPAT, CNKI, STN(REGISTRY): 甾, 类固醇, 糖皮质激素受体, 偶联物, 缀合物, 结合物, 炎症, 免疫, 靶向, 抗体, steroid, glucocorticosteroid, glucocorticoid receptor, conjugate, immunoconjugate, ADC, inflammatory, GC, target, antibody, 结构式检索, structural formula search

**C. DOCUMENTS CONSIDERED TO BE RELEVANT**

| Category* | Citation of document, with indication, where appropriate, of the relevant passages | Relevant to claim No. |
|---|---|---|
| X | CN 109476699 A (ABBVIE INC.) 15 March 2019 (2019-03-15)<br>description, paragraphs [0005]-[1308] | 1-73 |
| X | CN 110291097 A (REGENERON PHARMACEUTICALS INC.) 27 September 2019 (2019-09-27)<br>description, paragraphs [0005]-[1057] | 1-73 |
| X | CN 111417410 A (ABBVIE INC.) 14 July 2020 (2020-07-14)<br>description, paragraphs [0007]-[0362] | 1-73 |
| X | CN 111465399 A (ABBVIE INC.) 28 July 2020 (2020-07-28)<br>description, paragraphs [0009]-[0523] | 1-73 |
| PX | WO 2021216913 A1 (IMMUNEXT, INC.) 28 October 2021 (2021-10-28)<br>description, paragraphs [5]-[1024] | 1-73 |
| PX | WO 2022150637 A1 (IMMUNEXT, INC.) 14 July 2022 (2022-07-14)<br>description, paragraphs [5]-[1717] | 1-73 |
| PX | WO 2022166779 A1 (SHANGHAI SENHUI MEDICINE CO., LTD. et al.) 11 August 2022 (2022-08-11)<br>description, p. 1, line 1 to p. 68, line 5 | 1-73 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 November 2022** | **30 November 2022** |
| Name and mailing address of the ISA/CN<br>**China National Intellectual Property Administration (ISA/CN)**<br>**No. 6, Xitucheng Road, Jimenqiao, Haidian District, Beijing 100088, China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2022/114855**

**C. DOCUMENTS CONSIDERED TO BE RELEVANT**

| Category* | Citation of document, with indication, where appropriate, of the relevant passages | Relevant to claim No. |
|---|---|---|
| E | WO 2022204099 A1 (ELI LILLY AND COMPANY) 29 September 2022 (2022-09-29) description, p. 1, line 20 to p. 10, line 13, and embodiments 1, 3, 5, 7, 9, 11, and 13 | 1-5, 8-9, 11-21, 58-59, 70-73 |

Form PCT/ISA/210 (second sheet) (January 2015)

**INTERNATIONAL SEARCH REPORT**

International application No.
**PCT/CN2022/114855**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**

International application No.
**PCT/CN2022/114855**

**Box No. II Observations where certain claims were found unsearchable (Continuation of item 2 of first sheet)**

This international search report has not been established in respect of certain claims under Article 17(2)(a) for the following reasons:

1. [✓] Claims Nos.: **71**
because they relate to subject matter not required to be searched by this Authority, namely:

[1] Claim 71 sets forth a method for affecting functions of an immune system. Hence, claim 71 relates to a treatment method implemented on a human/animal body, and thus does not comply with PCT Rule 39.1. Nevertheless, a search was still conducted on the basis of the claimed effect of the compound or composition.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2022/114855**

| Patent document cited in search report | Publication date (day/month/year) | Patent family member(s) | Publication date (day/month/year) |
|---|---|---|---|
| CN 109476699 A | 15 March 2019 | RU 2018145728 A | 10 July 2020 |
| | | DK 3464318 T3 | 28 June 2021 |
| | | JP 2021088582 A | 10 June 2021 |
| | | KR 20190014542 A | 12 February 2019 |
| | | PE 20190622 A1 | 26 April 2019 |
| | | CR 20180594 A | 29 July 2019 |
| | | US 2018126000 A1 | 10 May 2018 |
| | | MX 2018014927 A | 09 April 2019 |
| | | PH 12018502539 A1 | 30 September 2019 |
| | | AU 2021269433 A1 | 16 December 2021 |
| | | PL 3464318 T3 | 08 November 2021 |
| | | TW 201801749 A | 16 January 2018 |
| | | SG 10202001787Q A | 29 April 2020 |
| | | EP 3901162 A1 | 27 October 2021 |
| | | WO 2017210471 A1 | 07 December 2017 |
| | | EC SP18094857 A | 31 January 2019 |
| | | KR 20220119529 A | 29 August 2022 |
| | | IL 263214 A | 31 December 2018 |
| | | DO P2018000261 A | 31 December 2018 |
| | | BR 112018074922 A2 | 12 March 2019 |
| | | UY 37269 A | 02 January 2018 |
| | | SG 11201810678W A | 28 December 2018 |
| | | CL 2018003406 A1 | 15 February 2019 |
| | | ZA 201808623 B | 28 August 2019 |
| | | LT 3464318 T | 25 June 2021 |
| | | JP 2019524645 A | 05 September 2019 |
| | | PT 3464318 T | 02 July 2021 |
| | | HU E054804 T2 | 28 September 2021 |
| | | CO 2018012996 A2 | 18 January 2019 |
| | | SI 3464318 T1 | 30 July 2021 |
| | | ES 2877548 T3 | 17 November 2021 |
| | | MA 51586 A | 10 April 2019 |
| | | HR P20210924 T1 | 03 September 2021 |
| | | AU 2017274442 A1 | 13 December 2018 |
| | | EP 3464318 A1 | 10 April 2019 |
| | | US 2020338208 A1 | 29 October 2020 |
| | | RS 62040 B1 | 30 July 2021 |
| | | CA 3025377 A1 | 07 December 2017 |
| | | US 2019262465 A1 | 29 August 2019 |
| CN 110291097 A | 27 September 2019 | US 2021332080 A1 | 28 October 2021 |
| | | US 2021040144 A1 | 11 February 2021 |
| | | AU 2017359043 A1 | 23 May 2019 |
| | | MX 2019005330 A | 11 September 2019 |
| | | EP 3538539 A2 | 18 September 2019 |
| | | IL 266353 A | 30 June 2019 |
| | | KR 20190074310 A | 27 June 2019 |
| | | CO 2019005966 A2 | 10 July 2019 |
| | | JP 2019536765 A | 19 December 2019 |
| | | CA 3042428 A1 | 17 May 2018 |
| | | CL 2019001259 A1 | 19 July 2019 |

Form PCT/ISA/210 (patent family annex) (January 2015)

**INTERNATIONAL SEARCH REPORT**
**Information on patent family members**

International application No.
**PCT/CN2022/114855**

| Patent document cited in search report | Publication date (day/month/year) | Patent family member(s) | Publication date (day/month/year) |
|---|---|---|---|
| | | BR 112019009019 A2 | 09 July 2019 |
| | | PH 12019501021 A1 | 24 June 2019 |
| | | SG 10202104259R A | 29 June 2021 |
| | | US 2018155389 A1 | 07 June 2018 |
| | | WO 2018089373 A2 | 17 May 2018 |
| CN 111417410 A | 14 July 2020 | LT 3658192 T | 12 July 2021 |
| | | CA 3082356 A1 | 06 June 2019 |
| | | CL 2020001452 A1 | 11 September 2020 |
| | | PT 3658192 T | 25 June 2021 |
| | | ZA 202003325 B | 28 July 2021 |
| | | UY 37991 A | 28 June 2019 |
| | | SI 3658192 T1 | 31 August 2021 |
| | | CL 2021001075 A1 | 15 October 2021 |
| | | RS 61994 B1 | 30 July 2021 |
| | | JP 2021054845 A | 08 April 2021 |
| | | MA 49796 A | 03 June 2020 |
| | | AU 2018374634 A1 | 28 May 2020 |
| | | US 2021015938 A1 | 21 January 2021 |
| | | JP 6813712 B2 | 13 January 2021 |
| | | KR 20200095477 A | 10 August 2020 |
| | | HR P20210917 T1 | 03 September 2021 |
| | | PE 20201286 A1 | 24 November 2020 |
| | | DK 3658192 T3 | 21 June 2021 |
| | | PL 3658192 T3 | 18 October 2021 |
| | | IL 274651 A | 30 June 2020 |
| | | EC SP20029001 A | 30 June 2020 |
| | | DO P2020000116 A | 31 August 2020 |
| | | CO 2020006446 A2 | 09 June 2020 |
| | | EP 3658192 A1 | 03 June 2020 |
| | | ES 2877659 T3 | 17 November 2021 |
| | | WO 2019106609 A1 | 06 June 2019 |
| | | TW 201924723 A | 01 July 2019 |
| | | BR 112020010694 A2 | 10 November 2020 |
| | | CR 20200239 A | 21 September 2020 |
| | | US 2019167804 A1 | 06 June 2019 |
| | | EP 3884962 A1 | 29 September 2021 |
| | | RU 2020117698 A3 | 04 January 2022 |
| | | HU E054428 T2 | 28 September 2021 |
| | | SG 11202004867W A | 29 June 2020 |
| CN 111465399 A | 28 July 2020 | PH 12020550551 A1 | 22 March 2021 |
| | | CL 2020001442 A1 | 11 September 2020 |
| | | BR 112020010691 A2 | 10 November 2020 |
| | | EP 3716982 A1 | 07 October 2020 |
| | | SG 11202004865S A | 29 June 2020 |
| | | CA 3081559 A1 | 06 June 2019 |
| | | JP 2021504430 A | 15 February 2021 |
| | | CR 20200285 A | 04 September 2020 |
| | | KR 20200095493 A | 10 August 2020 |
| | | EC SP20034868 A | 31 August 2020 |
| | | PE 20201464 A1 | 17 December 2020 |

Form PCT/ISA/210 (patent family annex) (January 2015)

**INTERNATIONAL SEARCH REPORT**
**Information on patent family members**

International application No.
**PCT/CN2022/114855**

| Patent document cited in search report | Publication date (day/month/year) | Patent family member(s) | Publication date (day/month/year) |
|---|---|---|---|
| | | WO 2019106608 A1 | 06 June 2019 |
| | | IL 274650 A | 30 June 2020 |
| | | US 2022265842 A1 | 25 August 2022 |
| | | AU 2018374633 A1 | 21 May 2020 |
| | | DO P2020000119 A | 31 August 2020 |
| | | RU 2020117156 A | 04 January 2022 |
| WO 2021216913 A1 | 28 October 2021 | None | |
| WO 2022150637 A1 | 14 July 2022 | None | |
| WO 2022166779 A1 | 11 August 2022 | None | |
| WO 2022204099 A1 | 29 September 2022 | US 2022306681 A1 | 29 September 2022 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20140294813 A
- WO 2013087912 A
- WO 2014152247 A
- WO 2015073884 A
- US 6258562 B
- US 5656272 A
- WO 0194585 A
- WO 2012131053 A
- US 8828396 B
- US 9221913 B
- US 8277810 B
- US 5874082 A
- US 7445780 B
- US 7193064 B
- US 6051228 A
- US 8669352 B
- US 20140093497 A
- US 8591900 B
- US 8778345 B
- US 5801227 A
- US 20120301488 A
- US 5235038 A
- US 5919453 A
- US 10831459 B
- US 10182058 B
- US 11157494 B
- US 11521102 B
- CN 109476699 A
- CN 111465399 A
- CN 111417410 A
- WO 2015191783 A2
- US 6090382 A
- CN 105452295 B
- CN 105949314 B


### Non-patent literature cited in the description


- **TROUT et al.** *Proc. Natl. Acad. Sci. USA,* 1982, 626-629
- **UMEMOTO et al.** *Int. J. Cancer,* 1989, 677-684
- **DORONINA et al.** *Nat. Biotechnol.,* 2003, vol. 21, 778-784
- **WARD et al.** Binding Activities of a Repertoire of Single Immunoglobulin Variable Domains Secreted From Escherichia coli. *Nature,* 1989, vol. 341, 544-546
- **HUSTON et al.** Protein Engineering of Antibody Binding Sites: Recovery of Specific Activity in an Anti-Digoxin Single-Chain Fv Analogue Produced in Escherichia coli. *Proc. Natl. Acad. Sci. USA,* 1988, vol. 85, 5879-5883
- **NGUYEN VK. et al.** *The EMBO Journal,* 2000, vol. 19, 921-930
- **MUYLDERMANS S.** *J Biotechnol.,* 2001, vol. 74, 277-302
- **VANL ; SCHOOT P. et al.** *Antiviral Research,* 2011, vol. 92, 389-407
- **KABAT et al.** Sequences of Proteins of Immunological Interest. National Institutes of Health, 1991
- **A1-LAZIKANI et al.** *J Mol Biol,* 1997, vol. 273, 927-48
- **MATHIEU DONDELINGER et al.** Understanding the Significance and Implications of Antibody Numbering and Antigen-Binding Surface/Residue Definition. *Front. Immunol.,* 16 October 2018
- **ALTSCHUL SF et al.** *Nucleic Acids Res.,* 1997, vol. 25, 3389-3402
- **VINCENT.** *Int.J.Clin.Pract.,* 2000, vol. 54, 190-193
- **GAY et al.** *Mabs,* 2010, vol. 2, 625-638
- **MCRAE BL et al.** *J Crohns Colitis,* 2016, vol. 10 (1), 69-76
- **PEREZC et al.** *Cell,* 1990, vol. 63 (2), 251-8